# EUROPEAN PATENT APPLICATION

(11) **EP 1 329 160 A2**
(43) Date of publication of application: **23.07.2003**
(21) Application number: 01958383.0
(22) Date of filing: 21.08.2001
(51) Int. Cl.: A01N 1/00

(54) **4-ACYLAMINOPYRAZOLE DERIVATIVES**

(30) Priority: 25.08.2000 JP 2000254809
(71) Applicant: Sankyo Company, Limited, Tokyo 103-8426 (JP)
(72) Inventor: KAJINO, Hisaki,, SANKYO COMPANY, LIMITED, Yasu-gun, Shiga 520-2342 (JP); MORIMOTO, Munetsugu, SANKYO COMPANY, LIMITED, Yasu-gun, Shiga 520-2342 (JP); FURUTA, Satoru, SANKYO COMPANY, LIMITED, Yasu-gun, Shiga 520-2342 (JP); TANAKA, Hisako, SANKYO COMPANY, LIMITED, Yasu-gun, Shiga 520-2342 (JP); TANAKA, Harukazu, SANKYO COMPANY, LIMITED, Yasu-gun, Shiga 520-2342 (JP); OHNISHI, Tohru, SANKYO COMPANY, LIMITED, Yasu-gun, Shiga 520-2342 (JP)
(74) Representative: Gibson, Christian John Robert
(86) International application number: JP0107166
(87) International publication number: WO02023986

(57) **Abstract**

A 4-acylaminopyrazole derivative represented by the following general formula: 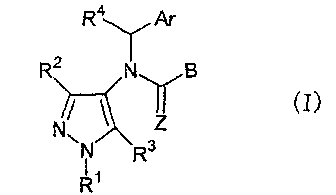 wherein R¹ is a hydrogen atom, an optionally substituted C₁-C₁₆ alkyl group or the like,
R² and R³ are independently a hydrogen atom, a halogen atom, an optionally substituted C₁-C₆ alkyl group or the like,
R⁴ is a hydrogen atom, a C₁-C₆ alkyl group or a cyano group,
Z is an oxygen atom or a sulfur atom,
Ar is an optionally substituted C₆-C₁₄ aryl group or an optionally substituted 5-6 membered unsaturated heterocyclic group,
B is a hydrogen atom, a halogen atom, an optionally substituted C₁-C₁₆ alkyl group or the like.

## Description

### [Technical field]

The present invention relates to 4-acylaminopyrazole derivatives and salts thereof, and to agricultural chemicals containing the same as an active ingredient.

### [Background Art]

Previously, 4-acylaminopyrazole derivatives, for example, N-phenylmethyl-N-1,3,5-trimethyl-4-pyrazolylheptaneamide (Example 36), N-(2-chlorophenyl)methyl-N-(1,3,5-trimethyl-4-pyrazolyl)cyclohexanecarboxamide (Example 38) and N-(4-chlorophenyl)methyl-N-(1,3,5-trimethyl-4-pyrazolyl)heptaneamide (Example 39), have been described in the publication JP-A Sho 51-146465. However, these compounds have a different structure from that of the 4-acylaminopyrazole derivatives of the present invention and, although it is described in the same publication that "they are useful for preventing and curing direct hypersensitive diseases including asthma and for alleviating the asthma condition" there is no description that they can be used as an agricultural and horticultural fungicide.

An object of the present invention is to develop novel agricultural and horticultural fungicides being capable of controlling plant diseases due to Oomycetes such as late blight on tomatoes (Phytophthora infestans), downy mildew on grape vines (Plasmopara viticola) and seedling blight on rice (Pythium graminicola), which frequently give severe damage to agricultural and horticultural crops, among plant pathogenic fungal diseases, at a low dose.

### [Disclosure of the invention]

The present inventors intensively studied 4-aminopyrazole derivatives and, as a result, found that particular 4-acylaminopyrazole derivatives have specifically extremely excellent fungicidal activity against a variety of vegetable diseases, which resulted in completion of the present invention.

The present invention comprises 4-acylaminopyrazole derivatives represented by the following general formula: 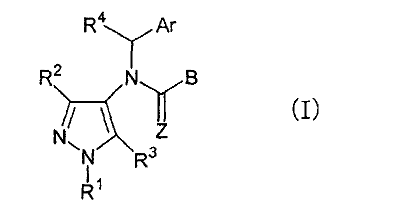 [wherein R¹ represents a hydrogen atom, an optionally substituted C₁-C₁₆ alkyl group (substituent(s) being selected from the substituent group a below), a C₃-C₈ cycloalkyl group, an optionally substituted C₂-C₆ alkenyl group (substituent(s) being selected from the substituent group f below), a C₄-C₆ cycloalkenyl group, a C₂-C₆ alkynyl group, an optionally substituted C₆-C₁₄ aryl group (substituent(s) being selected from the substituent group g below), a 4-6 membered saturated heterocyclic group (the heterocycle contains 1-2 same or different heteroatom(s) selected from the group consisting of an oxygen atom, a sulfur atom and a nitrogen atom), an optionally substituted 5-6 membered unsaturated heterocyclic group (substituent(s) being selected from the substituent group i below; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a C₂-C₇ alkylcarbonyl group, an optionally substituted benzoyl group (substituent(s) being selected from the substituent group b below), a C₂-C₇ alkoxycarbonyl group, a C₈-C₁₀ aralkyloxycarbonyl group, a C₃-C₇ alkenyloxycarbonyl group, an optionally substituted phenylsulfonyl group (substituent(s) being C₁-C₆ alkyl group) or a di(C₁-C₆ alkyl)sulfamoyl group; R² and R³ represent, independently, a hydrogen atom, a halogen atom, an optionally substituted C₁-C₆ alkyl group (substituent(s) being selected from the substituent group j below), a carboxyl group, a C₂-C₇ alkoxycarbonyl group, a C₂-C₇ alkylcarbamoyl group, a cyano group, a C₁-C₆ alkoxy group or a phenyl group;
R⁴ represents a hydrogen atom, a C₁-C₆ alkyl group or a cyano group;
Z represents an oxygen atom or a sulfur atom; Ar represents an optionally substituted C₆-C₁₄ aryl group (substituent(s) being selected from the substituent group k below) or an optionally substituted 5-6 membered unsaturated heterocyclic group (substituent(s) being selected from the substituent group b below; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s));
B represents a hydrogen atom, a halogen atom, an optionally substituted C₁-C₁₆ alkyl group (substituent(s) being selected from the substituent group m below), a C₃-C₈ cycloalkyl group, an optionally substituted C₂-C₆ alkenyl group (substituent(s) being selected from the substituent group n below), a C₄-C₈ cycloalkenyl group, a C₂-C₆ alkynyl group, an optionally substituted phenyl group (substituent(s) being selected from the substituent group b below), a 4-6 membered saturated heterocyclic group (the heterocycle contains 1-2 same or different heteroatom(s) selected from the group consisting of an oxygen atom, a sulfur atom and a nitrogen atom), an optionally substituted 5-6 membered unsaturated heterocyclic group (substituent(s) being selected from the substituent group b below; the heterocycle contains an oxygen atom, a sulfur atom or a nitrogen atom, and may further contain 1-2 nitrogen atom(s), two oxygen atoms may bind to the sulfur atom the heterocycle may be fused with a benzene ring or a pyridine ring, and the heterocycle may be substituted with 1-2 oxo group(s)), a C₁-C₆ alkoxy group, a C₇-C₉ aralkyloxy group, a C₃-C₆ cycloalkoxy group, a C₂-C₆ alkenyloxy group, an optionally substituted phenoxy group (substituent(s) being selected from the substituent group b below), an optionally substituted 5-6 membered unsaturated (heterocyclyl)oxy group (substituent(s) being selected from the substituent group b below; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a C₁-C₆ alkylthio group, a C₇-C₉ aralkylthio group, a C₃-C₆ cycloalkylthio group, a C₂-C₆ alkenylthio group, an optionally substituted phenylthio group (substituent(s) being selected from the substituent group b below) or an optionally substituted amino group (substituent(s) being selected from the substituent group d below);
or Ar and B are taken together to represent an optionally substituted o-phenylene group (substituent(s) being selected from the substituent group b below, and one methylene group may be inserted between the phenylene group and the carbon atom to which B binds);
provided that, when R¹, R² and R³ are all a methyl group, then B is neither an unsubstituted alkyl group nor a cycloalkyl group;
the substituent group a is the group consisting of a halogen atom, a C₂-C₇ alkylcarbonyl group, an optionally substituted benzoyl group (substituent(s) being selected from the substituent group b below), a carboxyl group, a C₂-C₇ alkoxycarbonyl group, a phenoxycarbonyl group, an optionally substituted carbamoyl group (substituent(s) being selected from the substituent group c below), a cyano group, a nitro group, an optionally substituted C₃-C₆ cycloalkyl group (substituent(s) being C₁-C₆ alkyl group or halogen atom), a C₇-C₈ bicycloalkyl group, an optionally substituted C₆-C₁₄ aryl group (substituent(s) being selected from the substituent group b below), an optionally substituted 4-6 membered saturated heterocyclic group (substituent(s) being C₁-C₆ alkyl group or halogen atom; the heterocycle contains 1-2 same or different heteroatom(s) selected from the group consisting of an oxygen atom, a sulfur atom and a nitrogen atom), an optionally substituted 5-6 membered unsaturated heterocyclic group (substituent(s) being selected from the substituent group b below; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo groups), a hydroxyl group, an optionally substituted C₁-C₆ alkoxy group (substituent(s) being cyano group, C₁-C₆ alkoxy group or tri(C₁-C₆ alkyl)silyl group), a C₇-C₉ aralkyloxy group, a C₂-C₆ alkenyloxy group, a C₂-C₆ alkynyloxy group, an optionally substituted phenoxy group (substituent(s) being selected from the substituent group b below), an optionally substituted 4-6 membered saturated (heterocyclyl)oxy group (substituent(s) being C₁-C₆ alkyl group; the heterocycle contains 1-2 same or different heteroatom(s) selected from the group consisting of an oxygen atom, a sulfur atom and a nitrogen atom), an optionally substituted 5-6 unsaturated (heterocyclyl)oxy group (substituent(s) being selected from the substituent group b below; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a tri(C₁-C₆ alkyl)silyloxy group, a di(C₁-C₆ alkyl)(phenyl)silyloxy group, a mono(C₁-C₆ alkyl) (diphenyl)silyloxy group, a triphenylsilyloxy group, an optionally substituted C₂-C₁₇ alkylcarbonyloxy group {substituent(s) being halogen atom, substituted C₁-C₆ alkoxy group (substituent(s) being cyano group) or substituted C₁-C₆ alkylthio group (substituent(s) being cyano group)}, a C₄-C₉ cycloalkylcarbonyloxy group, a C₃-C₇ alkenylcarbonyloxy group, a C₅-C₇ cycloalkenylcarbonyloxy group, a C₃-C₇ alkynylcarbonyloxy group, an optionally substituted benzoyloxy group (substituent(s) being selected from the substituent group b below), an optionally substituted 5-6 membered unsaturated (heterocyclyl)carbonyloxy group (substituent(s) being selected from the substituent group b below; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a C₂-C₇ alkoxycarbonyloxy group, a C₈-C₁₀ aralkyloxycarbonyloxy group, a C₃-C₇ alkenyloxycarbonyloxy group, an optionally substituted phenoxycarbonyloxy group (substituent(s) being selected from the substituent group b below), an optionally substituted carbamoyloxy group (substituent(s) being selected from the substituent group c below), a C₂-C₇ alkyl(thiocarbonyl)oxy group, a phenyl(thiocarbonyl)oxy group, an optionally substituted thiocarbamoyloxy group (substituent(s) being selected from the substituent group c below), a C₂-C₇ (alkylthio)(thiocarbonyloxy) group, an optionally substituted aminooxy group (substituent(s) being selected from the substituent group d below), an optionally substituted C₁-C₆ alkylthio group (substituent(s) being cyano group), a C₇-C₉ aralkylthio group, a C₃-C₆ cycloalkylthio group, an optionally substituted phenylthio group (substituent(s) being selected from the substituent group b below), a 5-6 membered unsaturated (heterocyclyl) thio group (the heterocycle may contain an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted C₁-C₆ alkylsulfinyl group (substituent(s) being cyano group), a C₇-C₉ aralkylsulfinyl group, a C₃-C₆ cycloalkylsulfinyl group, a phenylsulfinyl group, a 5-6 membered unsaturated (heterocyclyl)sulfinyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted C₁-C₆ alkylsulfonyl group (substituent(s) being cyano group), a C₇-C₉ aralkylsulfonyl group, a C₃-C₆ cycloalkylsulfonyl group, a phenylsulfonyl group, a 5-6 membered unsaturated (heterocyclyl)sulfonyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted amino group (substituent(s) being selected from the substituent group d below), a substituted imino group {substituent(s) being optionally substituted C₁-C₆ alkyl group (substituent(s) being halogen atom or cyano group), cyano group, nitro group, hydroxyl group, C₂-C₇ alkoxycarbonyloxy group, optionally substituted C₁-C₆ alkoxy group (substituent(s) being cyano group or C₂-C₇ alkoxycarbonyl group), C₇-C₉ aralkyloxy group or C₂-C₇ alkylcarbonyloxy group}, a C₁-C₆ alkylhydrazono group and a tri(C₁-C₆alkyl)silyl group; the substituent group b is the group consisting of a halogen atom, an optionally substituted C₁-C₆ alkyl group
(substituent(s) being halogen atom), a hydroxyl group, an optionally substituted C₁-C₆ alkoxy group (substituent(s) being halogen atom), a benzoyloxy group, a carboxyl group, a C₂-C₇ alkoxycarbonyl group, a cyano group, a nitro group, an optionally substituted amino group (substituent(s) being C₁-C₆ alkyl group, C₂-C₇ alkylcarbonyl group or benzoyl group) and a phenyl group; the substituent group c is the group consisting of an optionally substituted C₁-C₆ alkyl group {substituent(s) being halogen atom, C₂-C₇ (alkylthio)carbonyloxy group, (phenylthio)carbonyloxy group, C₂-C₇ alkyl (thiocarbonyl) oxy group, phenyl(thiocarbonyl)oxy group, cyano group or C₁-C₆ alkoxyimino group}, an optionally substituted C₂-C₇ alkylcarbonyl group (substituent(s) being halogen atom, cyano group or C₁-C₆ alkoxyimino group), a C₂-C₇ alkoxycarbonyl group, a benzoyl group, a benzoylamino group and an optionally substituted phenyl group (substituent(s) being selected from the above substituent group b); the substituent group d is the group consisting of an optionally substituted C₁-C₆ alkyl group {substituent(s) being cyano atom, optionally substituted C₆-C₁₄ aryl group (substituent(s) being selected from the above substituent group b), carboxyl group, C₂-C₇ alkoxycarbonyl group, C₈-C₁₀ aralkyloxycarbonyl group, phenoxycarbonyl group or optionally substituted carbamoyl group (substituent(s) being selected from the above substituent group c)}, a C₃-C₆ cycloalkyl group, a C₂-C₆ alkenyl group, a formyl group, an optionally substituted C₂-C₁₇ alkylcarbonyl group (substituent(s) being halogen atom, C₂-C₇ alkoxycarbonylamino group, cyano group or C₁-C₆ alkoxyimino group), a C₄-C₉ cycloalkylcarbonyl group, a C₃-C₇ alkenylcarbonyl group, a C₅-C₇ cycloalkenylcarbonyl group, a C₃-C₇ alkynylcarbonyl group, a C₂-C₇ alkoxycarbonyl group, a C₈-C₁₀ aralkyloxycarbonyl group, a C₃-C₇ alkenyloxycarbonyl group, a phenoxycarbonyl group, a C₂-C₇ (alkylthio)carbonyl group, a C₂-C₇ (alkylthio)(thiocarbonyl) group, a phenyl(thiocarbonyl) group, an optionally substituted benzoyl group (substituent(s) being selected from the above substituent group b), an optionally substituted 5-6 membered unsaturated (heterocyclyl)carbonyl group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted carbamoyl group (substituent(s) being selected from the above substituent group c), a C₂-C₇ alkyl(thiocarbonyl)group, an optionally substituted thiocarbamoyl group (substituent(s) being selected from the above substituent group c), an optionally substituted C₁-C₆ alkylidene group (substituent(s) being selected from the substituent group e below), a C₄-C₆ cycloalkylidene group (an alkylene chain of the cycloalkylidene group may be interrupted with one oxygen atom), a phenyl group, a C₁-C₆ alkylsulfonyl group and a phenylsulfonyl group; the substituent group e is the group consisting of a C₃-C₆ cycloalkyl group, a C₂-C₇ alkoxycarbonyl group, an optionally substituted 5-6 membered unsaturated (heterocyclyl)carbonyl group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted phenyl group (substituent(s) being selected from the above substituent group b), an optionally substituted 5-6 membered unsaturated heterocyclic group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)) and a C₁-C₆ alkoxy group; the substituent group f is the group consisting of a halogen atom, a hydroxyl group, a C₂-C₇ alkoxycarbonyl group and a C₁-C₆ alkoxy group;
the substituent group g is the group consisting of a halogen atom, an optionally substituted C₁-C₆ alkyl group (substituent(s) being selected from the substituent group h), an optionally substituted C₃-C₆ cycloalkyl group (substituent(s) being halogen atom or C₁-C₆ alkyl group), an optionally substituted C₂-C₆ alkenyl group (substituent(s) being phenyl group, C₁-C₆ alkoxy group or C₂-C₇ alkoxycarbonyl group), a C₂-C₆ alkynyl group, a C₂-C₇ alkylcarbonyl group, an optionally substituted benzoyl group (substituent(s) being selected from the above substituent group b), a carboxyl group, a C₂-C₇ alkoxycarbonyl group, an optionally substituted carbamoyl group (substituent(s) being selected from the above substituent group c), an optionally substituted thiocarbamoyl group (substituent(s) being selected from the above substituent group c), a cyano group, an optionally substituted phenyl group (substituent(s) being selected from the above substituent group b), a hydroxyl group, an optionally substituted C₁-C₆ alkoxy group (substituent(s) being halogen atom), an optionally substituted C₂-C₆ alkenyloxy group (substituent(s) being halogen atom), an optionally substituted phenoxy group (substituent(s) being selected from the above substituent group b), a C₂-C₇ alkylcarbonyloxy group, a benzoyloxy group, a C₂-C₇ alkoxycarbonyloxy group, a phenoxycarbonyloxy group, a mercapto group, a C₁-C₆ alkylthio group, a phenylthio group, a C₁-C₆ alkylsulfinyl group, a phenylsulfinyl group, a C₁-C₆ alkylsulfonyl group, a phenylsulfonyl group, a C₂-C₇ alkylcarbonylthio group, a benzoylthio group, a C₂-C₇ (alkylthio)(thiocarbonylthio) group, an azido group, an optionally substituted amino group (substituent(s) being selected from the above substituent group d), an isocyano group, a nitro group, a tri(C₁-C₆ alkyl)silyl group and an optionally substituted 5-6 membered unsaturated heterocyclic group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s));
the substituent group h is the group consisting of a halogen atom, an optionally substituted C₁-C₆ alkylimino group (substituent(s) being C₁-C₆ alkylthio group, optionally substituted C₇-C₉ aralkylthio group (substituent(s) being selected from the above substituent group b) or phenylthio group} and an optionally substituted C₁-C₆ alkoxyimino group (substituent(s) being C₁-C₆ alkoxycarbonyl group, cyano group, amino group or phenyl group);
the substituent group i is the group consisting of the above substituent group b and an optionally substituted 1-pyrazolyl group {substituent(s) being C₁-C₆ alkyl group or N-acetyl-N-(cyanobenzyl)amino group};
the substituent group j is the group consisting of a halogen atom, a hydroxyl group, a C₁-C₆ alkoxy group, a C₂-C₇ alkylcarbonyloxy group, a C₂-C₇ alkoxycarbonyloxy group, a C₂-C₇ alkylcarbamoyloxy group and a C₁-C₆ alkylsulfonyloxy group; the substituent group k is the group consisting of a halogen atom, an optionally substituted C₁-C₆ alkyl group (substituent(s) being selected from the substituent group 1 below), an optionally substituted C₃-C₆ cycloalkyl group (substituent(s) being halogen atom or C₁-C₆ alkyl group), an optionally substituted C₂-C₆ alkenyl group (substituent(s) being phenyl group, C₁-C₆ alkoxy group or C₂-C₇ alkoxycarbonyl group), a C₂-C₆ alkynyl group, a C₂-C₇ alkylcarbonyl group, an optionally substituted benzoyl group (substituent(s) being selected from the above substituent group b), a carboxyl group, a C₂-C₇ alkoxycarbonyl group, an optionally substituted carbamoyl group (substituent(s) being selected from the above substituent group c), an optionally substituted thiocarbamoyl group (substituent(s) being selected from the above substituent group c), a cyano group, an optionally substituted phenyl group (substituent(s) being selected from the above substituent group b), an optionally substituted 5-6 membered unsaturated heterocyclic group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a hydroxyl group, an optionally substituted C₁-C₆ alkoxy group (substituent(s) being halogen atom), an optionally substituted C₂-C₆ alkenyloxy group (substituent(s) being halogen atom), an optionally substituted phenoxy group (substituent(s) being selected from the above substituent group b), a C₂-C₇ alkylcarbonyloxy group, a benzoyloxy group, a C₂-C₇ alkoxycarbonyloxy group, a phenoxycarbonyloxy group, a mercapto group, an optionally substituted C₁-C₆ alkylthio group (substituent(s) being halogen atom), a phenylthio group, an optionally substituted C₁-C₆ alkylsulfinyl group (substituent(s) being halogen atom), a phenylsulfinyl group, an optionally substituted C₁-C₆ alkylsulfonyl group (substituent(s) being halogen atom), a phenylsulfonyl group, a C₂-C₇ alkylcarbonylthio group, a benzoylthio group, a C₂-C₇ (alkylthio)(thiocarbonylthio) group, an azido group, an isocyano group, a nitro group, an optionally substituted amino group (substituent(s) being selected from the above substituent group d) and a tri(C₁-C₆ alkyl)silyl group;
the substituent group 1 is the group consisting of a halogen atom, a cyano group, a hydroxyl group, a C₁-C₆ alkoxy group, a C₁-C₆ alkylthio group, a C₁-C₆ alkylsulfinyl group, a C₁-C₆ alkylsulfonyl group, an optionally substituted amino group {substituent(s) being phenoxy(C₂-C₇ alkylcarbonyl) group or optionally substituted 4-pyrazolyl group (substituent(s) being C₁-C₆ alkyl group)}, an imino group, an optionally substituted C₁-C₆ alkylimino group {substituent(s) being C₁-C₆ alkylthio group, optionally substituted C₇-C₉ aralkylthio group (substituent(s) being selected from the above substituent group b) or phenylthio group}, a hydroxyimino group, a C₁-C₆ alkoxyimino group, a C₂-C₇ alkoxycarbonyl group and a phenyl group;
the substituent group m is the group consisting of a halogen atom, a carboxyl group, a C₂-C₇ alkoxycarbonyl group, a phenoxycarbonyl group, an optionally substituted carbamoyl group (substituent(s) being selected from the above substituent group c), a cyano group, a C₃-C₆ cycloalkyl group, a C₄-C₆ cycloalkenyl group, an optionally substituted C₆-C₁₄ aryl group (substituent(s) being selected from the above substituent group b), a 4-6 membered saturated heterocyclic group (the heterocycle contains 1-2 same or different heteroatom(s) selected from the group consisting of an oxygen atom, a sulfur atom and a nitrogen atom), an optionally substituted 5-6 membered unsaturated heterocyclic group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a hydroxyl group, an optionally substituted C₁-C₆ alkoxy group {substituent(s) being optionally substituted 5-6 membered unsaturated heterocyclic group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), cyano group, optionally substituted C₁-C₆ alkoxy group (substituent(s) being C₁-C₆ alkoxy group or triC₁-C₆ alkyl)silyl group) or C₇-C₉ aralkyloxy group}, an optionally substituted C₇-C₉ aralkyloxy group (substituent(s) being selected from the above substituent group b), a C₂-C₆ alkenyloxy group, a C₂-C₆ alkynyloxy group, an optionally substituted phenoxy group (substituent(s) being selected from the above substituent group b), a 4-6 membered saturated (heterocyclyl)oxy group (the heterocycle contains 1-2 same or different heteroatom(s) selected from the group consisting of an oxygen atom, a sulfur atom and a nitrogen atom), an optionally substituted 5-6 membered unsaturated (heterocyclyl)oxy group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a tri(C₁-C₆ alkyl)silyloxy group, a di(C₁-C₆ alkyl) (monophenyl)silyloxy group, a mono(C₁-C₆ alkyl)(diphenyl)silyloxy group, a triphenylsilyloxy group, a formyloxy group, an optionally substituted C₂-C₁₇ alkylcarbonyloxy group {substituent(s) being halogen atom, C₄-C₆ cycloalkenyl group, optionally substituted 5-6 membered unsaturated heterocyclic group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), optionally substituted C₁-C₆ alkoxy group (substituent(s) being cyano group), phenoxy group, C₂-C₇ alkylcarbonyloxy group or optionally substituted C₁-C₆ alkylthio group (substituent(s) being cyano group)}, an optionally substituted C₈-C₁₀ aralkylcarbonyloxy group (substituent(s) being selected from the above substituent group b), a C₄-C₉ cycloalkylcarbonyloxy group, an optionally substituted C₃-C₇ alkenylcarbonyloxy group (substituent(s) being phenyl group), a C₅-C₇ cycloalkenylcarbonyloxy group, an optionally substituted cyclohexadienylcarbonyloxy group (substituent(s) being C₁-C₆ alkyl group), a C₃-C₇ alkynylcarbonyloxy group, an optionally substituted benzoyloxy group (substituent(s) being selected from the above substituent group b), an optionally substituted 5-6 membered unsaturated hetero carbonyloxy group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a C₂-C₇ alkoxycarbonyloxy group, a C₃-C₇ alkenyloxycarbonyloxy group, a C₈-C₁₀ aralkyloxycarbonyloxy group, a phenoxycarbonyloxy group, an optionally substituted carbamoyloxy group (substituent(s) being selected from the above substituent group c), an optionally substituted thiocarbamoyloxy group (substituent(s) being selected from the above substituent group c), a C₂-C₇ (alkylthio)carbonyloxy group, a C₂-C₇ (alkylthio)(thiocarbonyloxy) group, an optionally substituted aminooxy group (substituent(s) being selected from the above substituent group d), a di(C₁-C₆ alkoxy)phosphoryloxy group, a di(C₁-C₆ alkoxy)thiophosphoryloxy group, a C₁-C₆ alkylsulfonyloxy group, an optionally substituted phenylsulfonyloxy group (substituent(s) being selected from the above substituent group b), an optionally substituted C₁-C₆ alkylthio group (substituent(s) being C₂-C₇ alkoxycarbonyl group or cyano group), a C₇-C₉ aralkylthio group, a phenylthio group, an optionally substituted 5-6 membered unsaturated (heterocyclyl)thio group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted C₁-C₆ alkylsulfinyl group (substituent(s) being cyano group), a C₇-C₉ aralkylsulfinyl group, a phenylsulfinyl group, a 5-6 membered unsaturated (heterocyclyl)sulfinyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted C₁-C₆ alkylsulfonyl group (substituent(s) being cyano group), a C₇-C₉ aralkylsulfonyl group, a phenylsulfonyl group, a 5-6 membered unsaturated (heterocyclyl) sulfonyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a di (C₁-C₆ alkoxy)thiophosphorylthio group, an optionally substituted amino group (substituent(s) being selected from the above substituent group d) and an optionally substituted C₁-C₆ alkoxyimino group (substituent(s) being phenyl group or cyano group);
the substituent group n is the group consisting of a phenyl group and an optionally substituted N-aralkyl-N-(5-6 membered unsaturated heterocyclyl)aminocarbonyl group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))], or salts thereof, and agricultural chemicals containing the same as an active ingredient.

In the present invention, the "C₁-C₁₆ alkyl group" is a straight or branched alkyl group having a carbon number of 1-16 and, for example, can be a methyl group, an ethyl group, a propyl group, an isopropyl group, a butyl group, an isobutyl group, a s-butyl group, a tert-butyl group, a pentyl group, an isopentyl group, a 2-methylbutyl group, a neopentyl group, a 1-ethylpropyl group, a hexyl group, an isohexyl group, a 4-methylpentyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, a 2-ethylbutyl group, a heptyl group, a 1-methylhexyl group, a 2-methylhexyl group, a 3-methylhexyl group, a 4-methylhexyl group, a 5-methylhexyl group, a 1-propylbutyl group, a 4,4-dimethylpentyl group, an octyl group, a 1-methylheptyl group, a 2-methylheptyl group, a 3-methylheptyl group, a 4-methylheptyl group, a 5-methylheptyl group, a 6-methylheptyl group, a 1-propylpentyl group, a 2-ethylhexyl group, a 5,5-dimethylhexyl group, a nonyl group, a 3-methyloctyl group, a 4-methyloctyl group, a 5-methyloctyl group, a 6-methyloctyl group, a 1-propylhexyl group, a 2-ethylheptyl group, a 6,6-dimethylheptyl group, a decyl group, a 1-methylnonyl group, a 3-methylnonyl group, a 8-methylnonyl group, a 3-ethyloctyl group, a 3,7-dimethyloctyl group, a 7,7-dimethyloctyl group, an undecyl group, a 4,8-dimethylnonyl group, a dodecyl group, a tridecyl group, a tetradecyl group, a pentadecyl group, a 3,7,11-trimethyldodecyl group, a hexadecyl group, a 4,8,12-trimethyltridecyl group, a 1-methylpentadecyl group, a 14-methylpentadecyl group or a 13,13-dimethyltetradecyl group and, in R¹, is preferably a straight or branched alkyl group having a carbon number of 1-10 (C₁-C₁₀ alkyl group), more preferably a straight or branched alkyl group having a carbon number of 2-8, further more preferably a straight or branched alkyl group having a carbon number of 2-6, particularly preferably a straight or branched alkyl group having a carbon number of 3-6, and most preferably a branched alkyl group having a carbon number of 4-5; and, in other substituents, is preferably a straight or branched alkyl group having a carbon number of 1-15, more preferably a straight or branched alkyl group having a carbon number of 1-7, further more preferably a straight or branched alkyl group having a carbon number of 1-6, further more preferably a straight or branched alkyl group having a carbon number of 1-4, further more preferably a straight or branched alkyl group having a carbon number of 1-3, particularly preferably an alkyl group having a carbon number of 1-2, and most preferably a methyl group.

In the present invention, the "halogen atom" is, for example, a fluorine atom, a chlorine atom, a bromine atom or an iodine atom, preferably a fluorine atom, a chlorine atom or a bromine atom and, in B, is more preferably a chlorine atom or a bromine atom, further more preferably a chlorine atom; and, in other substituents, is more preferably a fluorine atom or a chlorine atom.

In the present invention, the "C₂-C₁₇ alkylcarbonyl group" is a carbonyl group to which the above "C₁-C₁₆ alkyl group" binds, and, for example, can be an acetyl group, a propionyl group, a butyryl group, an isobutyryl group, a valeryl group, an isovaleryl group, a pivaloyl group, a laurolyl group, a myristoyl group, a palmitoyl group or a stearoyl group, preferably, a carbonyl group to which a straight or branched alkyl group having a carbon number of 1-6 binds (C₂-C₇ alkylcarbonyl group), more preferably a carbonyl group to which a straight or branched alkyl group having a carbon number of 1-5 binds and, in R¹, is further more preferably a carbonyl group to which a straight or branched alkyl group having a carbon number of 2-5 binds, particularly preferably a carbonyl group to which a straight or branched alkyl group having a carbon number of 3-5 binds; and, in other substituents, is futher more preferably a carbonyl group to which a straight or branched alkyl group having a carbon number of 1-3 binds, particularly preferably an acetyl group.

In the present invention, the "substituted C₁-C₆ alkyl group (substituent(s) being halogen atom)" is the above "C₁-C₆ alkyl group" substituted with 1-9 same or different "halogen atom(s)" described above, and, for example, can be a trifluoromethyl group, a trichloromethyl group, a difluoromethyl group, a dichloromethyl group, a dibromomethyl group, a fluoromethyl group, a trifluoroethyl group, a trichloroethyl group, a bromoethyl group, a chloroethyl group, a fluoroethyl group, an iodoethyl group, a chloropropyl group, a fluorobutyl group, an iodohexyl group, a dibromoethyl group, a pentafluoroethyl group, a pentafluoropropyl group, a heptafluoropropyl group, a 3,3,3-trifluoroisobutyl group, a 3,3,3,3',3',3'-hexafluoroisobutyl group, a nonafluorobutyl group, a fluoropentyl group, a fluorohexyl group, a fluoroheptyl group, a fluorooctyl group, a fluorononyl group or a fluorodecyl group, and is preferably a straight or branched alkyl group having a carbon number of 1-8 substituted with 1-8 same or different halogen atom(s) selected from the group consisting of a fluorine atom, a chlorine atom and a bromine atom, more preferably a straight or branched alkyl group having a carbon number of 1-6 substituted with 1-6 same or different halogen atom(s) selected from the group consisting of a fluorine atom, a chlorine atom and a bromine atom and, in R¹, is further more preferably a straight or branched alkyl group having a carbon number of 3-6 substituted with 3-6 fluorine atoms or chlorine atoms, particularly preferably a straight or branched alkyl group having a carbon number of 3-6 substituted with 3-6 fluorine atoms; and, in R² and R³, is further more preferably a straight or branched alkyl group having a carbon number of 1-3 substituted with 1-3 same or different halogen atom(s) selected from the group consisting of a fluorine atom, a chlorine atom and a bromine atom, particularly preferably an alkyl group having a carbon number of 1-2 substituted with 1-3 fluorine atom(s), most preferably a fluoromethyl group; and, in other substituents, is further more preferably a straight or branched alkyl group having a carbon number of 1-3 substituted with 1-3 same or different halogen atom(s) selected from the group consisting of a fluorine atom, a chlorine atom and a bromine atom, particularly preferably an alkyl group having a carbon number of 1-2 substituted with 1-2 same or different halogen atom(s) selected from the group consisting of a fluorine atom and a chlorine atom, most preferably a chloromethyl group.

In the present invention, the "C₁-C₆ alkoxy group" is a straight or branched alkoxy group having a carbon number of 1-6, and, for example, can be a methoxy group, an ethoxy group, a propoxy group, an isopropoxy group, a butoxy group, an isobutoxy group, a s-butoxy group, a t-butoxy group, a pentoxy group, an isopentoxy group, a 2-methylbutoxy group, a neopentoxy group, a hexyloxy group, a 4-methylpentoxy group, a 3-methylpentoxy group, a 2-methylpentoxy group, a 3,3-dimethylbutoxy group, a 2,2-dimethylbutoxy group, a 1,1-dimethylbutoxy group, a 1,2-dimethylbutoxy group, a 1,3-dimethylbutoxy group or a 2,3-dimethylbutoxy group, and is preferably a straight or branched alkoxy group having a carbon number of 1-4, more preferably a straight or branched alkoxy group having a carbon number of 1-3, further more preferably an alkoxy group having a carbon number of 1-2, particularly preferably a methoxy group.

In the present invention, the "substituted C₁-C₆ alkoxy group (substituent(s) being halogen atom)" is the above "C₁-C₆ alkoxy group" substituted with 1-5 same or different "halogen atom(s)" described above, and, for example, can be a trifluoromethoxy group, a trichloromethoxy group, a difluoromethoxy group, a dichloromethoxy group, a dibromomethoxy group, a fluoromethoxy group, a trifluoroethoxy group, a trichloroethoxy group, a bromoethoxy group, a chloroethoxy group, a fluoroethoxy group, an iodoethoxy group, a chloropropoxy group, a fluorobutoxy group, an iodohexyloxy group, a dibromoethoxy group, a pentafluoroethoxy group, a pentafluoropropoxy group, a fluoropentoxy group or a fluorohexyloxy group, and is preferably a straight or branched alkoxy group having a carbon number of 1-3 substituted with 1-5 same or different substituent(s) selected from the group consisting of a fluorine atom, a chlorine atom and a bromine atom, more preferably an alkoxy group having a carbon number of 1-2 substituted with 1-3 fluorine atom(s), further more preferably a trifluoromethoxy group.

In the present invention, the "C₂-C₇ alkoxycarbonyl group" is a carbonyl group to which the above "C₁-C₆ alkoxy group" binds, and, for example, can be a methoxycarbonyl group, an ethoxycarbonyl group, a propoxycarbonyl group, an isopropoxycarbonyl group, a butoxycarbonyl group, an isobutoxycarbonyl group, a s-butoxycarbonyl group, a t-butoxycarbonyl group, a pentoxycarbonyl group, an isopentoxycarbonyl group, a 2-methylbutoxycarbonyl group, a neopentoxycarbonyl group, a hexyloxycarbonyl group, a 4-methylpentoxycarbonyl group, a 3-methylpentoxycarbonyl group, a 2-methylpentoxycarbonyl group, a 3,3-dimethylbutoxycarbonyl group, a 2,2-dimethylbutoxycarbonyl group, a 1,1-dimethylbutoxycarbonyl group, a 1,2-dimethylbutoxycarbonyl group, a 1,3-dimethylbutoxycarbonyl group or a 2,3-dimethylbutoxycarbonyl group, and is preferably a carbonyl group to which a straight or branched alkoxy group having a carbon number of 1-4 binds, more preferably a carbonyl group to which a straight or branched alkoxy group having a carbon number of 1-3 binds, further more preferably a carbonyl group to which an alkoxy group having a carbon number of 1-2 binds, particularly preferably a methoxycarbonyl group.

In the present invention, the "substituted amino group (substituent(s) being C₁-C₆ alkyl group, C₂-C₇ alkylcarbonyl group or benzoyl group)" is a "C₁-C₆ alkylamino group", a "C₂-C₇ alkylcarbonylamino group", or a benzoylamino group, preferably an alkylcarbonylamino group or a benzoylamino group; the "C₁-C₆ alkylamino group" is an amino group substituted with one "C₁-C₆ alkyl group" described above, and, for example, can be a methylamino group, an ethylamino group, a propylamino group, an isopropylamino group, a butylamino group, a t-butylamino group, a pentylamino group or a hexylamino group, and is preferably an amino group to which one straight or branched alkyl group having a carbon number of 1-3 binds, more preferably an amino group to which one alkyl group having a carbon number of 1-2 binds, further more preferably a methylamino group; the "C₂-C₇ alkylcarbonylamino group" is an amino group substituted with one "C₂-C₇ alkylcarbonyl group" described above, and, for example, can be an acetylamino group, a propionylamino group, a butyrylamino group, an isobutyrylamino group, a valerylamino group, an isovalerylamino group or a pivaloylamino group, and is preferably an amino group to which a straight or branched alkylcarbonyl group having a carbon number of 2-4 binds, more preferably an amino group to which one alkylcarbonyl group having a carbon number of 2-3 binds, further more preferably an acetylamino group.

In the present invention, the "substituted benzoyl group (substituent(s) being selected from the substituent group b)" is a benzoyl group substituted with 1-3 same or different substituent(s) selected from the substituent group b, and, for example, can be a fluorobenzoyl group, a chlorobenzoyl group, a bromobenzoyl group, a difluorobenzoyl group, a dichlorobenzoyl group, a methylbenzoyl group, an ethylbenzoyl group, a dimethylbenzoyl group, a trimethylbenzoyl group, a trifluoromethylbenzoyl group, a hydroxybenzoyl group, a methoxybenzoyl group, an ethoxybenzoyl group, a dimethoxybenzoyl group, a trifluoromethoxybenzoyl group, a benzoyloxybenzoyl group, a carboxybenzoyl group, a methoxycarbonylbenzoyl group, an ethoxycarbonylbenzoyl group, a cyanobenzoyl group, a nitrobenzoyl group, an aminobenzoyl group, a methylaminobenzoyl group, an acetylaminobenzoyl group, a benzoylaminobenzoyl group or a phenylbenzoyl group, and is preferably a benzoyl group substituted with one substituent selected from a fluorine atom, a chlorine atom, a bromine atom, a cyano group or a straight or branched haloalkoxy group having a carbon number of 1-3 (the halogen substituent(s) is (are) 1-3 same or different halogen atom(s) selected from the group consisting of a fluorine atom, a chlorine atom and a bromine atom), more preferably a benzoyl group substituted with one substituent selected from a fluorine atom, a chlorine atom and a bromine atom, more preferably a fluorobenzoyl group. In the present invention, the "C₁-C₆ alkylthio group" is a sulfur atom to which the above "C₁-C₆ alkyl group" binds, and, for example, can be a methylthio group, an ethylthio group, a propylthio group, an isopropylthio group, a butylthio group, a pentylthio group or a hexylthio group; and, in an alkylthio group in the substituent group a, is preferably a straight or branched alkylthio group having a carbon number of 1-4, more preferably a straight or branched alkylthio group having a carbon number of 3-4; and, in the other substituents, is preferably a straight or branched alkylthio group having a carbon number of 1-3, more preferably an alkylthio group having a carbon number of 1-2, further more preferably a methylthio group.

In the present invention, the "C₂-C₇(alkylthio)carbonyl group" is a carbonyl group to which the above "C₁-C₆ alkylthio group" binds, and, for example, can be a (methylthio)carbonyl group, an (ethylthio)carbonyl group, a (propylthio)carbonyl group, an (isopropylthio)carbonyl group, a (butylthio)carbonyl group, a (pentylthio)carbonyl group or a (hexylthio)carbonyl group, and is preferably a carbonyl group to which one straight or branched alkylthio group having a carbon number of 1-3 binds, more preferably an (alkylthio)carbonyl group having a carbon number of 2-3, further more preferably a (methylthio)carbonyl group.

In the present invention, the "C₂-C₇(alkylthio)carbonyloxy group" is an oxygen atom to which the "C₂-C₇(alkylthio)carbonyl group" binds, and, for example, can be a (methylthio)carbonyloxy group, an (ethylthio)carbonyloxy group, a (propylthio)carbonyloxy group, an (isopropylthio)carbonyloxy group, a (butylthio)carbonyloxy group, a (pentylthio)carbonyloxy group or a (hexylthio)carbonyloxy group, and is preferably a straight or branched (alkythio)carbonyloxy group having a carbon number of 2-5, more preferably an (alkylthio)carbonyloxy group having a carbon number of 2-3, further more preferably a (methylthio)carbonyloxy group.

In the present invention, the "C₂-C₇ alkyl(thiocarbonyl) group" is a thiocarbonyl group to which the above "C₁-C₆ alkyl group" binds, and, for example, can be a methyl(thiocarbonyl) group, an ethyl(thiocarbonyl) group, a propyl(thiocarbonyl) group, an isopropyl(thiocarbonyl) group, a butyl(thiocarbonyl) group, a pentyl(thiocarbonyl) group or a hexyl(thiocarbonyl) group, and is preferably a thiocarbonyl group to which a straight or branched alkyl group having a carbon number of 1-3 binds, more preferably a thiocarbonyl group to which an alkyl group having a carbon number of 1-2 binds, further more preferably a methyl(thiocarbonyl) group.

In the present invention, the "C₂-C₇ alkyl(thiocarbonyl)oxy group" is an oxygen atom to which the above "C₂-C₇ alkyl(thiocarbonyl) group" binds, and, for example, can be a methyl(thiocarbonyl)oxy group, an ethyl(thiocarbonyl)oxy group, a propyl(thiocarbonyl)oxy group, an isopropyl(thiocarbonyl)oxy group, a butyl(thiocarbonyl)oxy group, a pentyl(thiocarbonyl)oxy group or a hexyl(thiocarbonyl)oxy group, and is preferably a straight or branched alkyl(thiocarbonyl)oxy group having a carbon number of 2-4, more preferably an alkyl(thiocarbonyl)oxy group having a carbon number of 2-3, further more preferably a methyl(thiocarbonyl)oxy group.

In the present invention, the "C₁-C₆ alkoxyimino group" is an imino group substituted with one "C₁-C₆ alkoxy group" described above, and, for example, can be a methoxyimino group, an ethoxyiminomethyl group, a propoxyiminomethyl group, an isopropoxyiminomethyl group, a butoxyiminomethyl group, a pentoxyiminomethyl group or a hexyloxyiminomethyl group, and is preferably a straight or branched alkoxyimino group having a carbon number of 1-3, more preferably an alkoxyimino group having a carbon number of 1-2, further more preferably a methoxyimino group.

In the present invention, the "substituted C₁-C₆ alkyl group {substituent(s) being halogen atom, C₂-C₇ (alkylthio)carbonyloxy group, (phenylthio)carbonyloxy group, C₂-C₇ alkyl (thiocarbonyl)oxy group, phenyl(thiocarbonyl)oxy group, cyano group or C₁-C₆ alkoxyimino group}" is the above "substituted C₁-C₆ alkyl group (substituent(s) being halogen atom)", a " [C₂-C₇ (alkylthio) carbonyloxy] (C₁-C₆ alkyl) group", a "(phenylthio) carbonyloxy(C₁-C₆ alkyl) group", a "cyano (C₁-C₆ alkyl) group" or a "(C₁-C₆ alkoxyimino) (C₁-C₆ alkyl) group", preferably a "cyano(C₁-C₆ alkyl) group"; the "[C₂-C₇ (alkylthio) carbonyloxy] (C₁-C₆ alkyl) group" is the above "C₁-C₆ alkyl group" to which one "C₂-C₇ (alkylthio)carbonyloxy group" described above binds, and, for example, can be a (methylthio)carbonyloxymethyl group, a (methylthio)carbonyloxyethyl group, a (methylthio)carbonyloxypropyl group, a (methylthio)carbonyloxybutyl group, a (methylthio)carbonyloxypentyl group, a (methylthio)carbonyloxyhexyl group, an (ethylthio)carbonyloxymethyl group, a (propylthio)carbonyloxymethyl group, an (isopropylthio)carbonyloxymethyl group, a (butylthio)carbonyloxymethyl group, a (pentylthio)carbonyloxymethyl group or a (hexylthio)carbonyloxymethyl group, and is preferably a straight or branched alkyl group having a carbon number of 1-3 to which one straight or branched (alkylthio)carbonyloxy group having a carbon number of 2-5 binds, more preferably an alkyl group having a carbon number of 1-2 substituted with one (alkylthio)carbonyloxy group having a carbon number of 2-3, further more preferably a (methylthio)carbonyloxymethyl group; the "(phenylthio)carbonyloxy(C₁-C₆ alkyl) group" is the above "C₁-C₆ alkyl group" substituted with one (phenylthio)carbonyloxy group, and, for example, can be a (phenylthio)carbonyloxymethyl group, a (phenylthio)carbonyloxyethyl group, a (phenylthio)carbonyloxypropyl group, a (phenylthio)carbonyloxybutyl group, a (phenylthio)carbonyloxypentyl group or a (phenylthio)carbonyloxyhexyl group, is preferably a straight or branched alkyl group having a carbon number of 1-3 substituted with one (phenylthio)carbonyloxy group, more preferably an alkyl group having a carbon number of 1-2 substituted with one (phenylthio)carbonyloxy group, further more preferably a (phenylthio)carbonyloxymethyl group; the "cyano(C₁-C₆ alkyl) group" is the above "C₁-C₆ alkyl group" substituted with 1-2 cyano group(s), and, for example, can be a cyanomethyl group, a dicyanomethyl group, a cyanoethyl group, a dicyanoethyl group, a cyanopropyl group, a cyanoisopropyl group, a cyanobutyl group, a 3-cyanoisobutyl group, a 3,3'-dicyanoisobutyl group, a cyanopentyl group, a cyanohexyl group and, in R¹, is preferably a 2-cyanoethyl group, a 2,2-dicyanoethyl group, a cyanopropyl group, a cyanoisopropyl group, a cyanobutyl group, a 3-cyanoisobutyl group, a 3/3'-dicyanoisobutyl group or a cyanopentyl group; and, in the other substituents, is preferably an alkyl group having a carbon number of 1-2 substituted with one cyano group, more preferably a cyanomethyl group; and the "(C₁-C₆ alkoxyimino) (C₁-C₆ alkyl) group" is the above "C₁-C₆ alkyl group" substituted with one "C₁-C₆ alkoxyimino group" described above, and, for example, can be a methoxyiminomethyl group, a methoxyiminoethyl group, a methoxyiminopropyl group, a methoxyiminobutyl group, a methoxyiminopentyl group, a methoxyiminohexyl group, an ethoxyiminomethyl group, a propoxyiminomethyl group, an isopropoxyiminomethyl group, a butoxyiminomethyl group, a pentoxyiminomethyl group or a hexyloxyiminomethyl group, and is preferably a straight or branched alkyl group having a carbon number of 1-3 substituted with one straight or branched alkoxyimino group having a carbon number of 1-3, more preferably an alkyl group having a carbon number of 1-2 substituted with one alkoxyimino group having a carbon number of 1-2, further more preferably a methoxyiminomethyl group.

In the present invention, the "optionally substituted C₂-C₇ alkylcarbonyl group (substituent(s) being halogen atom, cyano group or C₁-C₆ alkoxyimino group)" is a "halo(C₂-C₇ alkylcarbonyl) group" or a " (C₁-C₆ alkoxyimino) (C₂-C₇ alkylcarbonyl) group substituted with a cyano", preferably a "(C₁-C₆ alkoxyimino) (C₂-C₇ alkylcarbonyl) group substituted with a cyano"; the "halo (C₂-C₇ alkylcarbonyl) group" is the above "C₂-C₇ alkylcarbonyl group" substituted with 1-3 same or different "halogen atom(s)" described above, and, for example, can be a fluoroacetyl group, a chloroacetyl group, a bromoacetyl group, an iodoacetyl group, a difluoroacetyl group, a dichloroacetyl group, a trifluoroacetyl group, a trichloroacetyl group, a fluoropropionyl group, a chloropropionyl group, a fluorobutyryl group, a chlorobutyryl group, a fluoroisobutyryl group, a chloroisobutyryl group, a fluorovaleryl group, a chlorovaleryl group, a fluoroisovaleryl group, a chloroisovaleryl group, a fluoropivaloyl group or a chloropivaloyl group, and is preferably a straight or branched alkylcarbonyl group having a carbon number of 2-4 substituted with 1-3 same or different halogen atom(s) selected from the group consisting of a fluorine atom, a chlorine atom and a bromine atom, more preferably an alkylcarbonyl group having a carbon number of 2-3 substituted with one fluorine atom or chlorine atom, further more preferably a chloroacetyl group; and
the "(C₁-C₆ alkoxyimino) (C₂-C₇ alkylcarbonyl) group substituted with a cyano" is the above "C₂-C₇ alkylcarbonyl group" substituted with one cyano group and one "C₁-C₆ alkoxyimino group" described above, and, for example, can be a 2-cyano-2-methoxyiminoacetyl group, a 2-cyano-2-ethoxyiminoacetyl group, a 2-cyano-2-propoxyiminoacetyl group, a 2-cyano-2-isopropoxyiminoacetyl group, a 2-cyano-2-butoxyiminoacetyl group, a 2-cyano-2-pentoxyiminoacetyl group, a 2-cyano-2-hexyloxyiminoacetyl group, a 2-cyano-2-methoxyiminopropionyl group, a 2-cyano-2-methoxyiminobutyryl group, a 2-cyano-2-methoxyimino-3-methylbutyryl group, a 2-cyano-2-ethoxyimino-3-methylbutyryl group or a 2-cyano-2-methoxyiminovaleryl group, and is preferably a straight or branched alkylcarbonyl group having a carbon number of 2-5 substituted with one cyano group and one straight or branched alkoxyimino group having a carbon number of 1-3, more preferably a straight or branched alkylcarbonyl group having a carbon number of 2-5 substituted with one cyano group and one alkoxyimino group having a carbon number of 1-2, further more preferably a 2-cyano-2-methoxyiminoacetyl group or a 2-cyano-2-methoxyiminoacetyl-3-methylbutyryl group.

In the present invention, the "C₆-C₁₄ aryl group" can be, for example, a phenyl group, a naphthyl group, an anthryl group or a ferrocenyl group, preferably a phenyl group.

In the present invention, the "optionally substituted C₆-C₁₄ aryl group (substituent(s) being selected from the substituent group b)" is preferably an "optionally substituted phenyl group (substituent(s) being selected from the substituent group b)", and the "optionally substituted phenyl group (substituent(s) being selected from the substituent group b)" is a phenyl group optionally substituted with 1-3 same or different substituent(s) selected from the substituent group b, and, for example, can be a phenyl group, a fluorophenyl group, a chlorophenyl group, a bromophenyl group, a difluorophenyl group, a dichlorophenyl group, a tolyl group, an ethylphenyl group, a dimethylphenyl group, a trimethylphenyl group, a trifluoromethylphenyl group, a hydroxyphenyl group, a methoxyphenyl group, an ethoxyphenyl group, a dimethoxyphenyl group, a trifluoromethoxyphenyl group, a benzoyloxyphenyl group, a carboxylphenyl group, a methoxycarbonylphenyl group, an ethoxycarbonylphenyl group, a cyanophenyl group, a nitrophenyl group, an aminophenyl group, a methylaminophenyl group, an acetylaminophenyl group, a benzoylaminophenyl group or a phenylphenyl group, and is preferably a phenyl group optionally substituted with 1-2 same or different substituent(s) selected from the group consisting of a fluorine atom, a chlorine atom and a bromine atom, more preferably a phenyl group optionally substituted with 1-2 same or different substituent(s) selected from the group consisting of a fluorine atom and a chlorine atom, further more preferably fluorophenyl, chlorophenyl, difluorophenyl or dichlorophenyl, particularly preferably a 3-fluorophenyl group or a 3,5-difluorophenyl group.

In the present invention, the "optionally substituted carbamoyl group (substituent(s) being selected from the substituent group c)" is a carbamoyl group optionally substituted with 1-2 same or different substituent(s) selected from the substituent group c, and, for example, can be a carbamoyl group, a methylcarbamoyl group, a dimethylcarbamoyl group, an ethylcarbamoyl group, a propylcarbamoyl group, an isopropylcarbamoyl group, a butylcarbamoyl group, a pentylcarbamoyl group, a hexylcarbamoyl group, a fluoromethylcarbamoyl group, a chloromethylcarbamoyl group, a difluoromethylcarbamoyl group, a dichloromethylcarbamoyl group, a trifluoromethylcarbamoyl group, a (methylthio)carbonyloxymethylcarbamoyl group, a (methylthio)carbonyloxyethylcarbamoyl group, an (ethylthio)carbonyloxymethylcarbamoyl group, a (phenylthio) carbonyloxymethylcarbamoyl group, a methyl (thiocarbonyl) oxymethylcarbamoyl group, an ethyl(thiocarbonyl)oxymethylcarbamoyl group, a phenyl(thiocarbonyl)oxymethylcarbamoyl group, a 1-cyano-1-methoxyiminomethylcarbamoyl group, a 1-cyano-1-ethoxyiminomethylcarbamoyl group, a 1-cyano-1-methoxyimino-3-methylbutylcarbamoyl group, an acetylcarbamoyl group, a propionylcarbamoyl group, a butyrylcarbamoyl group, an isobutyrylcarbamoyl group, a valerylcarbamoyl group, an isovalerylcarbamoyl group, a pivaloylcarbamoyl group, a fluoroacetylcarbamoyl group, a chloroacetylcarbamoyl group, a difluoroacetylcarbamoyl group, a dichloroacetylcarbamoyl group, a trifluoroacetylcarbamoyl group, a 2-cyano-2-methoxyiminoacetylcarbamoyl group, a 2-cyano-2-ethoxyiminoacetylcarbamoyl group, a 2-cyano-2-methoxyimino-3-methylbutyrylcarbamoyl group, a methoxycarbonylcarbamoyl group, an ethoxycarbonylcarbamoyl group, a benzoylcarbamoyl group, a benzoylaminocarbamoyl group, a phenylcarbamoyl group, a fluorophenylcarbamoyl group, a chlorophenylcarbamoyl group, a difluorophenylcarbamoyl group or a dichlorophenylcarbamoyl group, and is preferably a carbamoyl group optionally substituted with a straight or branched alkyl group having a carbon number of 1-3, a straight or branched alkyl group having a carbon number of 1-3 substituted with one cyano group, an alkylcarbonyl group having a carbon atom of 2-4, or a phenyl group optionally substituted with one straight or branched alkoxy group having a carbon number of 1-3 , more preferably a carbamoyl group optionally substituted with a methyl group, a cyanomethyl group, a methoxycarbonyl group, a phenyl group or a methoxyphenyl group, further more preferably a carbamoyl group.

In the present invention, the "C₃-C₈ cycloalkyl group" is a cyclopropyl group, a cyclobutyl group, a cyclopentyl group, a cyclohexyl group, a cycloheptyl group or a cyclooctyl group, is preferably a cycloalkyl group having a carbon number of 3-6 (C₃-C₆ cycloalkyl group) and, in R¹, is more preferably a cycloalkyl group having a carbon number of 4-6, further more preferably a cyclobutyl group; and, in B, is more preferably a cycloalkyl group having a carbon number of 3-5, further more preferably a cyclopropyl group and, in the other substituents, is more preferably a cycloalkyl group having a carbon number of 5-6, further more preferably a cyclohexyl group.

In the present invention, the "optionally substituted C₃-C₆ cycloalkyl group (substituent(s) being C₁-C₆ alkyl group or halogen atom)" is the above "C₃-C₆ cycloalkyl group" optionally substituted with one "C₁-C₆ alkyl group" described above or 1-4 same or different "halogen atom(s)" described above, and, for example, can be a cyclopropyl group, a methylcyclopropyl group, an ethylcyclopropyl group, a propylcyclopropyl group, an isopropylcyclopropyl group, a butylcyclopropyl group, a pentylcyclopropyl group, a hexylcyclopropyl group, a fluorocyclopropyl group, a chlorocyclopropyl group, a bromocyclopropyl group, an iodocyclopropyl group, a cyclobutyl group, a methylcyclobutyl group, a fluorocyclobutyl group, a chlorocyclobutyl group, a cyclopentyl group, a methylcyclopentyl group, a fluorocyclopentyl group, a difluorocyclopentyl group, a tetrafluorocyclopentyl group, a chlorocyclopentyl group, a cyclohexyl group, a methylcyclohexyl group, a fluorocyclohexyl group or a chlorocyclohexyl group, and is preferably a cycloalkyl group having a carbon number of 3-6 substituted with one straight or branched alkyl group having a carbon number of 1-3 or a cycloalkyl group having a carbon number of 5-6 substituted with 1-4 fluorine atoms, more preferably a cycloalkyl group having a carbon number of 3-6 optionally substituted with one methyl group, further more preferably a cycloalkyl group having a carbon number of 4-6 optionally substituted with one methyl group, particularly preferably a cyclobutyl group or a cyclopentyl group.

In the present invention, the "C₇-C₈ bicycloalkyl group" is preferably a norbonyl group.

In the present invention, the "4-6 membered saturated heterocyclic group (the heterocycle contains 1-2 same or different heteroatom(s) selected from the group consisting of an oxygen atom, a sulfur atom and a nitrogen atom)" can be, for example, a tetrahydrofuranyl group, a tetrahydropyranyl group, a dioxanyl group or a morpholyl group, and is preferably a 5-6 membered saturated heterocyclic group wherein the heteroatom(s) for the heterocycle include(s) 1-2 same or different heteroatom(s) selected from the group consisting of an oxygen atom and a nitrogen atom, and, in the substituent group a, is more preferably a 5-6 membered saturated heterocyclic group wherein the heteroatom for the heterocycle is one oxygen atom, further more preferably a tetrahydropyranyl group; and, in the other substituents, is more preferably a morpholyl group, further more preferably a morpholino group.

In the present invention, the "optionally substituted 4-6 membered saturated heterocyclic group (substituent(s) being C₁-C₆ alkyl group or halogen atom; the heterocycle contains 1-2 same or different heteroatom(s) selected from the group consisting of an oxygen atom, a sulfur atom and a nitrogen atom)" is the above "4-6 membered saturated heterocyclic group (the heterocycle contains 1-2 same or different heteroatom(s) selected from the group consisting of an oxygen atom, a sulfur atom and a nitrogen atom)" optionally substituted with 1-2 same or different substituent(s) selected from the group consisting of the "C₁-C₆ alkyl group" described above, and the "halogen atom" described above, and, for example, can be a tetrahydrofuranyl group, a tetrahydropyranyl group, a dioxanyl group or a morpholylmethyl group, and is preferably a 5-6 membered saturated heterocyclic group optionally substituted with 1-2 same or different straight or branched alkyl group(s) having a carbon atom of 1-3 wherein the heteroatom(s) for the heterocycle include(s) 1-2 same or different heteroatom(s) selected from the group consisting of an oxygen atom and a nitrogen atom and, in R¹, is more preferably a 5-6 membered saturated heterocyclic group wherein the heteroatom for the heterocycle is one oxygen atom, further more preferably a tetrahydropyranyl group; and, in the other substituents, is more preferably a 5-6 membered saturated heterocyclic group wherein the heteroatom(s) for the heterocycle include(s) 1-2 same or different heteroatom(s) selected from the group consisting of an oxygen atom and a nitrogen atom, further more preferably a morpholyl group.

In the present invention, the "5-6 membered unsaturated heterocyclic group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" can be, for example, a pyrazolyl group, a pyridyl group, an oxopyridyl group, a thienyl group, a benzooxazolyl group, a benzothiazolyl group, a furyl group, a thienyl group, an oxazolyl group, an isooxazolyl group, a thiazolyl group, an oxadiazolyl group, a thiadiazolyl group, a pyridazyl group, a pyrimidinyl group, a triazinyl group, a quinolyl group, a phthalazyl group or a benzothiadiazolyl group and, in the substituent group a, is preferably a 5-6 membered unsaturated heterocyclic group wherein the heteroatom for the heterocycle is an oxygen atom, a sulfur atom or a nitrogen atom, and may be 1-2 further nitrogen atom(s), and which may be fused with a benzene ring; and, in R¹, is preferably a 5-6 membered unsaturated heterocyclic group wherein the heteroatom for the heterocycle is a sulfur atom or a nitrogen atom, and may be 1-2 further nitrogen atom(s), and which may be fused with a benzene ring; and, in B, is preferably a 5-6 membered unsaturated heterocyclic group wherein the heteroatom for the heterocycle is an oxygen atom, a sulfur atom or a nitrogen atom, and may be 1-2 further nitrogen atom(s), and which may be fused with a benzene ring and may be substituted with one oxo group, more preferably a 5-6 membered unsaturated heterocycle wherein the heteroatom for the heterocycle is an oxygen atom or a nitrogen atom, and may be 1-2 further nitrogen atom(s), and which may be fused with a benzene ring and may be substituted with one oxo group, further more preferably a pyrazol-1-yl group, an isooxazol-3-yl group, an imidazol-1-yl group, an imidazol-1-yl group, a 2-oxo-1-pyridyl group, a 2-oxo-1-pyridyl group, a 6-oxo-pyrimidin-1-yl group, a 4-oxo-quinazolin-3-yl group, a 3-oxo-pyrazol-1-yl group or a 2-pyridyl group, particularly preferably a 1-pyrazolyl group; and, in Ar, is preferably a 5 membered unsaturated heterocyclic group wherein the heteroatom for the heterocycle is a sulfur atom or a nitrogen atom, and may be one further nitrogen atom and which may be fused with a benzene ring.

In the present invention, the "optionally substituted 5-6 membered unsaturated heterocyclic group (substituent(s) being selected from the substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" is the above "5-6 membered unsaturated heterocyclic group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" optionally substituted with 1-3 same or different substituent(s) selected from the substituent group b, and, for example, can be a pyrazolyl group, a fluoropyrazolyl group, a chloropyrazolyl group, a bromopyrazolyl group, a methylpyrazolyl group, an ethylpyrazolyl group, a dimethylpyrazolyl group, a trimethylpyrazolyl group, a hydroxypyrazolyl group, a methoxypyrazolyl group, a trifluoromethoxypyrazolyl group, a benzoyloxypyrazolyl group, a carboxylpyrazolyl group, a methoxycarbonylpyrazolyl group, an ethoxycarbonylpyrazolyl group, a cyanopyrazolyl group, a nitropyrazolyl group, an aminopyrazolyl group, a methylaminopyrazolyl group, an acetylaminopyrazolyl group, a benzoylaminopyrazolyl group, a phenylpyrazolyl group, a pyridyl group, an oxopyridyl group, a fluoropyridyl group, a dichloropyridyl group, a trifluoromethylpyridyl group, a chlorothienyl group, a benzooxazolyl group, a benzothiazolyl group, a methylbenzothiazolyl group, an ethylbenzothiazolyl group, a t-butylbenzothiazolyl group, a furyl group, a methylfuryl group, a methoxycarbonylfuryl group, a cyanofuryl group, a thienyl group, a methylthienyl group, a cyanothienyl group, a methoxycarbonylthienyl group, a nitrothienyl group, an oxazolyl group, a methyloxazolyl group, an isooxazolyl group, a methylisooxazolyl group, a thiazolyl group, a methylthiazolyl group, a phenylthiazolyl group, an oxadiazolyl group, a methyloxadiazolyl group, a thiadiazolyl group, a methylthiadiazolyl group, a pyridazyl group, a pyrimidinyl group, a methylpyrimidinyl group, a dimethylpyrimidinyl group, a dimethoxypyrimidinyl group, a dichloropyrimidinyl group, a chloro-methoxypyrimidinyl group, a triazinyl group, a dimethoxytriazinyl group, a quinolyl group, a phthalazinyl group or a benzothiadiazolyl group and, in the substituent group a, is preferably a 5-6 membered unsaturated heterocyclic group which may be substituted with one of a fluorine atom, a chlorine atom or a bromine atom and wherein the heteroatom for the heterocycle is an oxygen atom, a sulfur atom or a nitrogen atom, and may be 1-2 further nitrogen atom(s) and which may be fused with a benzene ring; and, in R¹, is preferably a 5-6 membered unsaturated heterocyclic group which may be substituted with 1-2 same or different substituent(s) selected from the group consisting of a fluorine atom, a chlorine atom, a bromine atom, a straight or branched alkyl group having a carbon number of 1-3, a straight or branched haloalkyl group having a carbon number of 1-3 (the halogen substituent(s) is (are) 1-3 same or different halogen atom(s) selected from the group consisting of a fluorine atom, a chlorine atom and a bromine atom) and a straight or branched alkoxy group having a carbon number of 1-3, and wherein the heteroatom for the heterocycle is a sulfur atom or a nitrogen atom, and may be 1-2 further nitrogen atom(s), and which may be fused with a benzene ring; and, in the substituent group m, is preferably a 5-6 membered unsaturated heterocyclic group which may be substituted with 1-3 substituent(s) selected from the group consisting of a fluorine atom, a chlorine atom, a bromine atom, a straight or branched alkyl group having a carbon number of 1-3, a straight or branched haloalkyl group having a carbon number of 1-3 (the substituent(s) is (are) 1-3 same or different halogen atom(s) selected from the group consisting of a fluorine atom, a chlorine atom and a bromine atom), a straight or branched alkoxycarbonyl group having a carbon number of 2-4, a cyano group, a nitro group and an amino group, and wherein the heteroatom for the heterocycle is an oxygen atom, a sulfur atom or a nitrogen atom, and may be 1-2 further nitrogen atom(s), and which may be fused with a benzene ring and may be substituted with one oxo group, more preferably a 5-6 membered unsaturated heterocyclic group which may be substituted with 1-2 same or different substituent(s) selected from the group consisting of a chlorine atom, a bromine atom;
a methyl group, an alkyl group having a carbon number of 1-2 substituted with 1-3 fluorine atom(s), an alkoxycarbonyl group having a carbon number of 2-3 and a nitro group, and wherein the heteroatom for the heterocycle is an oxygen atom or a nitrogen atom, and may be 1-2 further nitrogen atom(s), and which may be fused with a benzene ring and may be substituted with one oxo group, further more preferably a pyrazol-1-yl group optionally substituted with 1-2 same or different substituent(s) selected from the group consisting of a chlorine atom, a bromine atom, a methyl group, an ethoxycarbonyl group and a nitro group, a methyl-isooxazol-3-yl group, an imidazol-1-yl group, a dichloro-imidazol-1-yl group, a 2-oxo-1-pyridyl group, a trifluoromethyl-2-oxo-1-pyridyl group, a 6-oxo-pyrimidin-1-yl group, a 4-oxo-quinazolin-3-yl group, a 3-oxo-pyrazol-1-yl group optionally substituted with 1-2 methyl group(s), or a 2-pyridyl group, particularly preferably a 4-chloro-1-pyrazolyl group or a 4-bromo-1-pyrazolyl group; and, in B, is preferably a 5-6 membered unsaturated heterocyclic group optionally substituted with 1-2 same or different substituent(s) selected from the group consisting of a fluorine atom, a chlorine atom, a bromine atom and a straight or branched alkyl group having a carbon number of 1-6, and wherein the heteroatom for the heterocycle is an oxygen atom, a sulfur atom or a nitrogen atom, and may be 1-2 further nitrogen atom(s), and which may be fused with a benzene ring, more preferably a 5-6 membered unsaturated heterocyclic group wherein the heteroatom(s) is (are) 1-2 nitrogen atom(s) and, in Ar, is preferably a 5 membered unsaturated heterocyclic group optionally substituted with 1-2 same or different substituent(s) selected from the group consisting of a fluorine atom, a chlorine atom, a bromine atom, an iodine atom, a straight or branched alkyl group having a carbon number of 1-3, a hydroxyl group, a straight or branched alkoxy group having a carbon number of 1-3, a cyano group, a nitro group and an amino group, and wherein the heteroatom for the heterocycle is a sulfur atom or a nitrogen atom, and may be one further nitrogen atom, and which may be fused with a benzene ring.

In the present invention, the "tri(C₁-C₆ alkyl)silyl group" is a silicon atom to which three same or different "C₁-C₆ alkyl groups" described above bind, and, for example, can be a trimethylsilyl group, a triethylsilyl group, a triisopropylsilyl group, an isopropyldimethylsilyl group, a t-butyldimethylsilyl group or a hexyldimethylsilyl group, and is preferably a silyl group to which 1-3 straight or branched alkyl group(s) having a carbon number of 1-4 bind, more preferably a trimethylsilyl group or a t-butyldimethylsilyl group. In the present invention, the "optionally substituted C₁-C₆ alkoxy group (substituent(s) being cyano group, C₁-C₆ alkoxy group or tri(C₁-C₆ alkyl)silyl group)" is a "substituted C₁-C₆ alkoxy group (substituent(s) being cyano group)", a "(C₁-C₆ alkoxy)(C₁-C₆ alkoxy) group" or a "tri(C₁-C₆ alkyl)silyl(C₁-C₆ alkoxy) group", and is preferably a "substituted C₁-C₆ alkoxy group (substituent(s) being cyano group)" or a "(C₁-C₆ alkoxy) (C₁-C₆ alkoxy) group";
the "substituted C₁-C₆ alkoxy group (substituent(s) being cyano group)" is the "C₁-C₆ alkoxy group" described above substituted with 1-2 cyano group(s), and, for example, can be a cyanomethoxyacetoxymethyl group, a cyanomethoxyacetoxyethyl group, a cyanomethoxypropionyloxymethyl group, a cyanomethoxybutyryloxymethyl group, a cyanoethoxyacetoxymethyl-group, a cyanopropoxyacetoxymethyl group, a cyanoisopropoxyacetoxymethyl group or a cyanobutoxyacetoxymethyl group, and is preferably a straight or branched alkyl group having a carbon number of 1-3 substituted with one straight or branched alkylcarbonyloxy group having a carbon number of 2-4 substituted with one straight or branched alkoxy group having a carbon number of 1-3 substituted with one cyano group, more preferably an alkyl group having a carbon number of 1-2 substituted with one alkylcarbonyloxy group having a carbon number of 2-3 substituted with one alkoxy group having a carbon number of 1-2 substituted with one cyano group, more preferably a cyanomethoxyacetoxymethyl group;
the "(C₁-C₆ alkoxy) (C₁-C₆ alkoxy) group" is the "C₁-C₆ alkoxy group" described above substituted with one "C₁-C₆ alkoxy group" described above, and, for example, can be a methoxymethoxymethyl group, a methoxymethoxyethyl group, a methoxymethoxypropyl group, a methoxymethoxybutyl group, a methoxymethoxypentyl group, a methoxymethoxyhexyl group, a methoxyethoxymethyl group, a methoxyethoxyethyl group, a methoxymethoxypropyl group, a methoxypropoxymethyl group, a methoxypropoxyethyl group, an ethoxymethoxymethyl group, an ethoxymethoxyethyl group, an ethoxyethoxymethyl group, an ethoxyethoxyethyl group, a propoxymethoxymethyl group, a propoxymethoxyethyl group, a propoxyethoxymethyl group, a propoxyethoxyethyl group, an isopropoxymethoxymethyl group, an isopropoxymethoxyethyl group, an isopropoxyethoxymethyl group, a butoxymethoxymethyl group, a butoxymethoxyethyl group, a butoxyethoxymethyl group, a pentoxymethoxymethyl group or a hexyloxymethoxymethyl group, and is preferably a straight or branched alkyl group having a carbon number of 1-3 substituted with one straight or branched alkoxy group having a carbon number of 1-3 substituted with one straight or branched alkoxy group having a carbon number of 1-3, more preferably an alkyl group having a carbon number of 1-2 substituted with one alkoxy group having a carbon number of 1-2 substituted with one alkoxy group having a carbon number of 1-2, further more preferably a methyl group substituted with one alkoxy group having a carbon number of 1-2 substituted with one alkoxy group having a carbon number of 1-2, particularly preferably a methoxymethoxymethyl group;
the "tri(C₁-C₆ alkyl)silyl(C₁-C₆ alkoxy) group" is the "C₁-C₆ alkoxy group" described above substituted with one "tri(C₁-C₆ alkyl)silyl group" described above, and, for example, can be a trimethylsilylmethoxymethyl group, a trimethylsilylmethoxyethyl group, a trimethylsilylmethoxypropyl group, a trimethylsilylmethoxybutyl group, a trimethylsilylmethoxypentyl group, a trimethylsilylmethoxyhexyl group, a trimethylsilylethoxymethyl group, a trimethylsilylmethoxyethyl group, a trimethylsilylpropoxymethyl group, a trimethylsilylbutoxymethyl group, a trimethylsilylpentoxymethyl group, a trimethylsilylhexyloxymethyl group, a triethylsilylmethoxymethyl group, a triisopropylsilylmethoxymethyl group, an isopropyldimethylsilylmethoxymethyl group, a t-butyldimethylsilylmethoxymethyl group, a t-butyldimethylsilylmethoxyethyl group, a t-butyldimethylsilylethoxymethyl group, a t-butyldimethylsilylethoxyethyl group or a hexyldimethylsilylmethoxymethyl group, and is preferably a straight or branched alkyl group having a carbon atom of 1-3 substituted with one straight or branched alkoxy group having a carbon number of 1-3 substituted with one silyl group to which 1-3 same or different straight or branched alkyl group(s) having a carbon number of 1-4 binds, more preferably a trimethylsilylmethoxymethyl group or a t-butyldimethylsilylmethoxymethyl group.

In the present invention, the "C₇-C₉ aralkyl group" can be, for example, a benzyl group, a phenethyl group, an α-methylbenzyl group or a 3-phenylpropyl group, and is preferably an aralkyl group having a carbon number of 7-8, more preferably a benzyl group.

In the present invention, the "C₇-C₉ aralkyloxy group" is an oxygen atom to which the above "C₇-C₉ aralkyl group" binds, and, for example, can be a benzyloxy group, a phenethyloxy group, an α-methylbenzyloxy group or a 3-phenylpropoxy group, and is preferably an aralkyloxy group having a carbon number of 7-8, more preferably a benzyloxy group.

In the present invention, the "C₂-C₆ alkenyl group" is a straight or branched alkenyl group having a carbon number of 2-6, and, for example, can be a vinyl group, a 1-propenyl group, an allyl group, a 1-butenyl group, a 2-butenyl group, a 3-butenyl group, a 1-pentenyl group, a 1-isopentenyl group, a 2-pentenyl group, a 1-hexenyl group or a 2-hexenyl group, and is preferably a straight or branched alkenyl group having a carbon number of 2-4, more preferably a vinyl group.

In the present invention, the "C₂-C₆ alkenyloxy group" is an oxygen atom to which the above "C₂-C₆ alkenyl group" binds, and, for example, can be a vinyloxy group, a 1-propenyloxy group, an allyloxy group, a 2-methylallyloxy group, a 1-butenyloxy group, a 2-butenyloxy group, a 2-methyl-2-butenyloxy group, a 3-butenyloxy group, a 1-pentenyloxy group, a 1-isopentenyloxy group, a 2-pentenyloxy group, a 1-hexenyloxy group or a 2-hexenyloxy group, and is preferably a straight or branched alkenyloxy group having a carbon number of 2-5, more preferably a straight or branched alkenyloxy group having a carbon number of 3-5, further more preferably an allyloxy group or a 2-methyl-2-butenyloxy group.

In the present invention, the "C₂-C₆ alkynyl group" is a straight or branched alkynyl group having a carbon number of 2-6, and, for example, can be an ethynyl group, a propynyl group, a butynyl group, a pentynyl group or a hexynyl group, and is preferably an alkynyl group having a carbon number of 2-3, more preferably a propynyl group.

In the present invention, the "C₂-C₆ alkynyloxy group" is an oxygen atom to which the above "C₂-C₆ alkynyl group" binds, and, for example, can be an ethynyloxy group, a propynyloxy group, a butynyloxy group, a pentynyloxy group or a hexynyloxy group, and is preferably a straight or branched alkynyloxy group having a carbon number of 2-3, more preferably a propynyloxy group.

In the present invention, the "optionally substituted phenoxy group (substituent(s) being selected from the substituent group b)" is a phenoxy group optionally substituted with 1-3 same or different substituent(s) selected from the substituent group b, and, for example, can be a phenoxy group, a fluorophenoxy group, a chlorophenoxy group, a bromophenoxy group, a difluorophenoxy group, a dichlorophenoxy group, a methylphenoxy group, an ethylphenoxy group, a dimethylphenoxy group, a trimethylphenoxy group, a trifluoromethylphenoxy group, a hydroxyphenoxy group, a methoxyphenoxy group, an ethoxyphenoxy group, a dimethoxyphenoxy group, a trifluoromethoxyphenoxy group, a benzoyloxyphenoxy group, a carboxylphenoxy group, a methoxycarbonylphenoxy group, an ethoxycarbonylphenoxy group, a cyanophenoxy group, a nitrophenoxy group, an aminophenoxy group, a methylaminophenoxy group, an acetylaminophenoxy group, a benzoylaminophenoxy group or a phenylphenoxy group, and is preferably a phenoxy group optionally substituted with 1-2 same or different substituent(s) selected from the group consisting of a fluorine atom, a chlorine atom, a bromine atom, a straight or branched alkyl group having a carbon number of 1-3, a straight or branched haloalkyl group having a carbon number of 1-3 (the halogen substituent(s) is (are) 1-3 same or different halogen atom(s) selected from the group consisting of a fluorine atom, a chlorine atom and a bromine atom), an alkoxy group having a carbon number of 1-3, a cyano group and a nitro group, more preferably a phenoxy group optionally substituted with 1-2 same or different substituent(s) selected from the group consisting of a fluorine atom, a chlorine atom, a methyl group, an alkyl group having a carbon number of 1-2 substituted with 1-3 fluorine atom(s), a methoxy group and a nitro group, more preferably a phenoxy group optionally substituted with 1-2 same or different substituent(s) selected from the group consisting of a fluorine atom, a chlorine atom, a methyl group, a trifluoromethyl group, a methoxy group and a cyano group, further more preferably a phenoxy group, a fluorophenoxy group, a chlorophenoxy group, a difluorophenoxy group, a dichlorophenoxy group, a methylphenoxy group, a trifluoromethylphenoxy group, a methoxyphenoxy group, a nitrophenoxy group, a dinitrophenoxy group or a cyanophenoxy group, particularly preferably a phenoxy group, a 2-fluorophenoxy group, a 3-fluorophenoxy group, a 4-fluorophenoxy group, a 4-chlorophenoxy group, a 2-methylphenoxy group, a 2-cyanophenoxy group or a 3-cyanophenoxy group, most preferably a phenoxy group, a 2-fluorophenoxy group, a 2-cyanophenoxy group or a 3-cyanophenoxy group.

In the present invention, the "4-6 membered saturated (heterocyclyl)oxy group (the heterocycle contains 1-2 same or different heteroatom(s) selected from the group consisting of an oxygen atom, a sulfur atom and a nitrogen atom)" can be, for example, an oxetanyloxy group, a tetrahydrofuranyloxy group, a tetrahydropyranyloxy group, a morpholyloxy group, a dioxanyloxy group or a dithianyloxy group, and is preferably a 4-6 membered saturated (heterocyclyl)oxy group wherein the heteroatom(s) for the heterocycle is (are) 1-2 same or different heteroatom(s) selected from the group consisting of an oxygen atom, a sulfur atom and a nitrogen atom, more preferably a 5-6 membered saturated (heterocyclyl)oxy group wherein the heteroatom(s) for the heterocycle is (are) 1-2 same or different heteroatom(s) selected from the group consisting of an oxygen atom and a nitrogen atom, further more preferably a tetrahydropyranyloxy group.

In the present invention, the "optionally substituted 4-6 membered saturated (heterocyclyl)oxy group (substituent(s) being C₁-C₆ alkyl group; the heterocycle contains 1-2 same or different heteroatom(s) selected from the group consisting of an oxygen atom, a sulfur atom and a nitrogen atom)" is the above "4-6 membered saturated (heterocyclyl)oxy group (the heterocycle contains 1-2 same or different heteroatom(s) selected from the group consisting of an oxygen atom, a sulfur atom and a nitrogen atom)" optionally substituted with 1-2 same or different above "C₁-C₆ alkyl group(s)", and is preferably the above "5-6 membered (heterocyclyl)oxy group wherein the heteroatom(s) for the heterocycle is (are) 1-2 same or different heteroatom(s) selected from the group consisting of an oxygen atom and a nitrogen atom", optionally substituted with 1-2 methyl group(s), more preferably a tetrahydropyranyloxy group.

In the present invention, the "5-6 membered unsaturated (heterocyclyl)oxy group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)" is preferably a 5-6 membered unsaturated (heterocyclyl)oxy group wherein the heteroatom for the heterocycle is an oxygen atom, a sulfur atom or a nitrogen atom, and may be 1-2 further nitrogen atom(s), two oxygen atoms may bind to the sulfur atom, the heterocycle may be fused with a benzene ring, and may be substituted with 1-2 oxo group(s), more preferably a 5-6 membered unsaturated (heterocyclyl)oxy group wherein the heteroatom for the heterocycle is an oxygen atom, a sulfur atom or a nitrogen atom, and may be 1-2 further nitrogen atom(s), two oxygen atoms may bind to the sulfur atom, the heterocycle may be fused with a benzene ring and may be substituted with 1-2 oxo group(s), further more preferably a pyrazol-1-yloxy group; a 2-pyridyloxy group; a 3-pyridyloxy group; a pyrimidin-2-yloxy group; a pyrimidin-4-yloxy group; a benzisooxazol-3-yloxy group; an imidazol-1-yloxy group; a 1,2,4-triazol-1-yloxy group; a thiazol-2-yloxy group; a 2-quinolyloxy group; a 3-isoquinolyloxy group; a benzisothiazol-1,1-dioxide-3-yloxy group; a benzisothiazol-2-yloxy group; an N,N-phthaloyloxy group; or a 3-isooxazolyloxy group, particularly preferably a pyrazol-1-yloxy group, a 2-pyridyloxy group, a pyrimidin-2-yloxy group or a 3-isooxazolyloxy group.

In the present invention, the "optionally substituted 5-6 membered unsaturated (heterocyclyl)oxy group (substituent(s) being selected from the substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" is the above "5-6 membered unsaturated (heterocyclyl)oxy group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" optionally substituted with 1-3 same or different substituent(s) selected from the substituent group b, and is preferably a 5-6 membered unsaturated (heterocyclyl)oxy group which may be substituted with 1-4 same or different substituent(s) selected from the group consisting of a fluorine atom, a chlorine atom, a bromine atom, a straight or branched alkyl group having a carbon number of 1-3, a straight or branched haloalkyl group having a carbon number of 1-3 (the halogen substituent(s) is (are) 1-3 same or different halogen atom(s) selected from the group consisting of a fluorine atom, a chlorine atom and a bromine atom), a straight or branched alkoxy group having a carbon number of 1-3, a carbamoyl group, a straight or branched alkoxycarbonyl group having a carbon number of 2-4, a cyano group, a nitro group, an amino group and a phenyl group, and wherein the heteroatom for the heterocycle is an oxygen atom, a sulfur atom or a nitrogen atom, and may be 1-2 further nitrogen atom(s), two oxygen atoms may bind to the sulfur atom, the heterocycle may be fused with a benzene ring, and which may be substituted with 1-2 oxo group(s), more preferably a 5-6 membered unsaturated (heterocyclyl)oxy group which may be substituted with 1-4 same or different substituent(s) selected from the group consisting of a fluorine atom, a chlorine atom, a bromine atom, a straight or branched alkyl group having a carbon number of 1-3, a straight or branched haloalkyl group having a carbon number of 1-3 (the halogen substituent(s) is (are) 1-3 same or different halogen atom(s) selected from the group consisting of a fluorine atom, a chlorine atom and a bromine atom), an alkoxy group having a carbon number of 1-2, a carbamoyl group, an alkoxycarbonyl group having a carbon number of 2-3, a cyano group, a nitro group, an amino group and a phenyl group, and wherein the heteroatom for the heterocycle is an oxygen atom, a sulfur atom or a nitrogen atom, and may be 1-2 further nitrogen atom(s), two oxygen atoms may bind to the sulfur atom, and the heterocycle may be fused with a benzene ring, and which may be substituted with 1-2 oxo group(s), further more preferably a pyrazol-1-yloxy group optionally substituted with one bromine atom or methyl group; a 2-pyridyloxy group optionally substituted with 1-3 same or different substituent(s) selected from the group consisting of a fluorine atom, a chlorine atom, a methyl group, a trifluoromethyl group, a methoxy group, a carbamoyl group, a methoxycarbonyl group, a cyano group and a phenyl group, or 3-4 fluorine atoms; a 3-pyridyloxy group; a pyrimidin-2-yloxy group optionally substituted with 1-3 same or different substituent(s) selected from the group consisting of a fluorine atom, a chlorine atom, a methyl group, a trifluoromethyl group, a methoxy group, a carbamoyl group, a methoxycarbonyl group, a cyano group and a phenyl group, or 3-4 fluorine atoms; a pyrimidin-4-yloxy group; an (isopropyl-methyl-pyrimidin-4-yl)oxy group; a benzisooxazol-3-yloxy group substituted with one chlorine atom, methyl group, nitro group or amino group; an imidazol-1-yloxy group; a 1,2,4-triazol-1-yloxy group; a thiazol-2-yloxy group; a 2-quinolyloxy group; a 3-isoquinolyloxy group; a benzisothiazol-1,1-dioxide-3-yloxy group; a benzisothiazol-2-yloxy group; an N,N-phthaloyloxy group; or a 3-isooxazolyloxy group optionally substituted with one methyl group, particularly preferably a pyrazol-1-yloxy group; a 2-pyridyloxy group optionally substituted with 1-3 same or different substituent(s) selected from the group consisting of a fluorine atom, a chlorine atom, a methyl group, a trifluoromethyl group and a cyano group; a pyrimidin-2-yloxy group optionally substituted with 1-3 same or different substituent(s) selected from the group consisting of a fluorine atom, a chlorine atom, a methyl group, a trifluoromethyl group and a cyano group; or a 3-isooxazolyloxy group or a 5-methyl-3-isooxazolyloxy group, most preferably a 2-pyridyloxy group or a pyrimidin-2-yloxy group optionally substituted with 1-3 same or different substituent(s) selected from the group consisting of a chlorine atom, a methyl group, a trifluoromethyl group and a cyano group; or a 5-methyl-3-isooxazolyloxy group.

In the present invention, the "tri(C₁-C₆ alkyl)silyloxy group" is an oxygen atom to which the above "tri(C₁-C₆ alkyl)silyl group" binds, and, for example, can be a trimethylsilyloxy group, a triethylsilyloxy group, a triethylsilyloxy group, a tripropylsilyloxy group, a propyldimethylsilyloxy group, an isopropyldimethylsilyloxy group, a butyldimethylsilyloxy group, an isobutyldimethylsilyloxy group, a t-butyldimethylsilyloxy group, a pentyldimethylsilyloxy group or a hexyldimethylsilyloxy group and, in the substituent group a, is preferably a silyloxy group to which same or different straight or branched alkyl groups having a carbon number of 1-3 bind, more preferably a trimethylsilyloxy group; and, in the substituent group m, is preferably a silyloxy group to which same or different straight or branched alkyl groups having a carbon number of 1-4 bind, more preferably a silyloxy group to which one straight or branched alkyl group having a carbon number of 3-4 and two methyl groups bind.

In the present invention, the "di(C₁-C₆ alkyl)(phenyl)silyloxy group" is a silyloxy group to which two same or different "C₁-C₆ alkyl groups" described above and one phenyl group bind, and, for example, can be a dimethylmonophenylsilyloxy group, a diethylmonophenylsilyloxy group, a dipropylmonophenylsilyloxy group, a diisopropylmonophenylsilyloxy group, a dibutylmonophenylsilyloxy group, a dipentylmonophenylsilyloxy group or a dihexylmonophenylsilyloxy group, and is preferably a silyloxy group to which two same or different straight or branched alkyl groups having a carbon number of 1-3 and one phenyl group bind, more preferably a dimethylmonophenylsilyloxy group.

In the present invention, the "mono(C₁-C₆ alkyl) (diphenyl)silyloxy group" is a silyloxy group to which one "C₁-C₆ alkyl group" described above and two phenyl groups bind, and, for example, can be a monomethyldiphenylsilyloxy group, a monoethyldiphenylsilyloxy group, a monopropyldiphenylsilyloxy group, a monoisopropyldiphenylsilyloxy group, a monobutyldiphenylsilyloxy group, a monopentyldiphenylsilyloxy group or a monohexyldiphenylsilyloxy group, and is preferably a silyloxy group to which one straight or branched alkyl group having a carbon number of 1-4 and two phenyl groups bind, more preferably a monomethyldiphenylsilyloxy group.

In the present invention, the "substituted C₁-C₆ alkylthio group (substituent(s) being cyano group)" is the above "C₁-C₆ alkylthio group" substituted with 1-2 cyano group(s), and, for example, can be a cyanomethylthio group, a cyanoethylthio group, a cyanopropylthio group, a cyanoisopropylthio group, a cyanobutylthio group, a cyanoisobutylthio group, a cyano-s-butylthio group, a cyano-t-butylthio group, a cyanopentylthio group, a cyanoneopentylthio group or a cyanohexylthio group, and is preferably a straight or branched alkylthio group having a carbon number of 1-6 substituted with one cyano group, more preferably a straight or branched alkylthio group having a carbon number of 1-4 substituted with one cyano group, further more preferably a straight or branched alkylthio group having a carbon number of 3-4 substituted with one cyano group.

In the present invention, the "C₂-C₁₇ alkylcarbonyloxy group" is an oxygen atom to which the above "C₂-C₁₇ alkylcarbonyl group" binds, and, for example, can be an acetyloxy group, a propionyloxy group, a butyryloxy group, an isobutyryloxy group, a valeryloxy group, an isovaleryloxy group, a pivaloyloxy group, a lauroyloxy group, a myristoyloxy group, a palmitoyloxy group or a stearoyloxy group, and is preferably a straight or branched alkylcarbonyloxy group having a carbon number of 2-7 (C₂-C₇ alkylcarbonyloxy group) and, in the substituent group a, is preferably a straight or branched alkylcarbonyloxy group having a carbon number of 2-4, further more preferably an acetoxy group and, in the other substituents, is more preferably a straight or branched alkylcarbonyloxy group having a carbon number of 2-5, further more preferably an isovaleryloxy group or a pivaloyloxy group.

In the present invention, "the optionally substituted C₂-C₁₇ alkylcarbonyloxy group {substituent(s) being halogen atom, substituted C₁-C₆ alkoxy group (substituent(s) being cyano group) or substituted C₁-C₆ alkylthio group (substituent(s) being cyano group)}" is a "halo(C₂-C₁₇ alkylcarbonyloxy) group", a "cyano(C₁-C₆ alkoxy) (C₂-C₁₇ alkylcarbonyloxy) group" or a "cyano(C₁-C₆ alkylthio) (C₂-C₁₇ alkylcarbonyloxy) group", and is preferably a "halo(C₂-C₁₇ alkylcarbonyloxy) group" or a "cyano(C₁-C₆ alkoxy)(C₂-C₁₇ alkylcarbonyloxy) group"; the "halo(C₂-C₁₇ alkylcarbonyloxy) group" is the above "C₂-C₁₇ alkylcarbonyloxy group" substituted with 1-3 same or different "halogen atom(s)" described above, and, for example, can be a fluoroacetyloxy group, a difluoroacetyloxy group, a trifluoroacetyloxy group, a chloroacetyloxy group, a dichloroacetyloxy group, a trichloroacetyloxy group, a bromoacetyloxy group, a fluoropropionyloxy group, a chloropropionyloxy group, a fluorobutyryloxy group, a chlorobutyryloxy group, a fluoroisobutyryloxy group, a chloroisobutyryloxy group, a fluorovaleryloxy group, a chlorovaleryloxy group, a fluoroisovaleryloxy group, a chloroisovaleryloxy group, a fluoropivaloyloxy group or a chloropivaloyloxy group, and is preferably a straight or branched alkylcarbonyloxy group having a carbon number of 2-4 substituted with 1-3 same or different halogen atom(s) selected from the group consisting of a fluorine atom, a chlorine atom and a bromine atom, more preferably an acetoxy group substituted with one fluorine atom or chlorine atom, further more preferably a fluoroacetoxy group or a chloroacetoxy group;
the "cyano(C₁-C₆ alkoxy) (C₂-C₇ alkylcarbonyloxy) group" is the above "C₂-C₇ alkylcarbonyloxy group" to which the above "substituted C₁-C₆ alkoxy group (substituent(s) being cyano group)" binds, and, for example, can be a cyanomethoxyacetoxy group, a cyanomethoxypropionyloxy group, a cyanomethoxybutyryloxy group, a cyanoethoxyacetoxy group, a cyanopropoxyacetoxy group, a cyanoisopropoxyacetoxy group or a cyanobutoxyacetoxy group, and is preferably a straight or branched alkylcarbonyloxy group having a carbon number of 2-4 substituted with one straight or branched alkoxy group having a carbon number of 1-3 substituted with one cyano group, more preferably an alkylcarbonyloxy group having a carbon number of 2-3 substituted with one alkoxy group having a carbon number of 1-2 substituted with one cyano group, further more preferably a cyanomethoxyacetoxy group.

In the present invention, the "cyano(C₁-C₆ alkylthio) (C₂-C₇ alkylcarbonyloxy) group" is the above "(C₂-C₇ alkylcarbonyloxy) group" to which the above "substituted C₁-C₆ alkylthio group (substituent(s) being cyano group)" binds, and, for example, can be a (cyanomethylthio)acetoxy group, a (cyanomethylthio)propionyloxy group, a (cyanomethylthio)butyryloxy group, a (cyanoethylthio)acetoxy group, a (cyanopropylthio)acetoxy group, a (cyanoisopropylthio)acetoxy group or a (cyanobutylthio)acetoxy group, and is preferably a straight or branched alkylcarbonyloxy group having a carbon number of 2-4 substituted with one straight or branched alkylthio group having a carbon number of 1-3 substituted with one cyano group, more preferably an alkylcarbonyloxy group having a carbon number of 2-3 substituted with one alkylthio group having a carbon number of 1-2 substituted with one cyano group, further more preferably a (cyanomethylthio)acetoxy group.

In the present invention, the "C₄-C₉ cycloalkylcarbonyl group" is a carbonyl group to which the above "C₃-C₈ cycloalkyl group" binds, and, for example, can be a cyclopropylcarbonyl group, a cyclobutylcarbonyl group, a cyclopentylcarbonyl group, a cyclohexylcarbonyl group, a cycloheptylcarbonyl group or a cyclooctylcarbonyl group, and is preferably a cycloalkylcarbonyl group having a carbon number of 4-7, more preferably a cycloalkylcarbonyl group having a carbon number of 5-7, further more preferably a cyclopentylcarbonyl group or a cyclohexylcarbonyl group.

In the present invention, the "C₄-C₉ cycloalkylcarbonyloxy group" is an oxygen atom to which the above "C₄-C₉ cycloalkylcarbonyl group" binds, and is a cyclopropylcarbonyloxy group, a cyclobutylcarbonyloxy group, a cyclopentylcarbonyloxy group, a cyclohexylcarbonyloxy group, a cycloheptylcarbonyloxy group or a cyclooctylcarbonyloxy group, and is preferably a cycloalkylcarbonyloxy group having a carbon atom of 4-7, more preferably a cycloalkylcarbonyloxy group having a carbon number of 5-7, further more preferably a cyclopentylcarbonyloxy group or a cyclohexylcarbonyloxy group.

In the present invention, the "C₃-C₇ alkenylcarbonyl group" is a carbonyl group to which the above "C₂-C₆ alkenyl group" binds, and, for example, can be a vinylcarbonyl group, a 1-propenylcarbonyl group, an allylcarbonyl group, a 1-butenylcarbonyl group, a 2-butenylcarbonyl group, a 3-butenylcarbonyl group, a 1-pentenylcarbonyl group, a 1-isopentenylcarbonyl group, a 2-pentenylcarbonyl group, a 1-hexenylcarbonyl group or a 2-hexenylcarbonyl group, and is preferably a straight or branched alkenylcarbonyl group having a carbon number of 2-4, more preferably a vinylcarbonyl group.

In the present invention, the "C₃-C₇ alkenylcarbonyloxy group" is an oxygen atom to which the above "C₃-C₇ alkenylcarbonyl group" binds, and, for example, can be an acryloyloxy group, a 1-propenylcarbonyloxy group, an allylcarbonyloxy group, a 1-butenylcarbonyloxy group, a 2-butenylcarbonyloxy group, a 2-methyl-2-butenylcarbonyloxy group, a 3-butenylcarbonyloxy group, a 1-pentenylcarbonyloxy group, a 1-isopentenylcarbonyloxy group, a 2-pentenylcarbonyloxy group, a 1-hexenylcarbonyloxy group or a 2-hexenylcarbonyloxy group, and is preferably a carbonyloxy group to which a straight or branched alkenyl group having a carbon number of 3-5 binds, more preferably a straight or branched alkenylcarbonyloxy group having a carbon number of 4-5, further more preferably a pentenylcarbonyloxy group.

In the present invention, the "C₄-C₆ cycloalkenyl group" is a cyclobutenyl group, a cyclopentenyl group or a cyclohexenyl group, and is preferably a cycloalkenyl group having a carbon number of 6-7, more preferably a cyclohexenyl group.

In the present invention, the "C₅-C₇ cycloalkenylcarbonyl group" is a carbonyl group to which the above "C₄-C₆ cycloalkenyl group" binds, and is a cyclobutenylcarbonyl group, a cyclopentenylcarbonyl group or a cyclohexenylcarbonyl group, preferably a cycloalkenylcarbonyl group having a carbon number of 6-7, more preferably a cyclohexenylcarbonyl group.

In the present invention, the "C₅-C₇ cycloalkenylcarbonyloxy group" is an oxygen atom to which the above "C₅-C₇ cycloalkenylcarbonyl group" binds, and is a cyclobutenylcarbonyloxy group, a cyclopentenylcarbonyloxy group or a cyclohexenylcarbonyloxy group, preferably a cycloalkenylcarbonyloxy group having a carbon number of 6-7, more preferably a cyclohexenylcarbonyloxy group.

In the present invention, the "C₃-C₇ alkynylcarbonyl group" is a carbonyl group to which the above "C₂-C₆ alkynyl group" binds, and, for example, can be a propioloyl group, a 1-propynylcarbonyl group, a propargylcarbonyl group, a 1-butynylcarbonyl group, a 2-butynylcarbonyl group, a 3-butynylcarbonyl group, a 1-pentynylcarbonyl group or a 1-hexynylcarbonyl group, and is preferably a carbonyl group to which an alkynyl group having a carbon number of 2-4 binds, more preferably a propargylcarbonyl group.

In the present invention, the "C₃-C₇ alkynylcarbonyloxy group" is an oxygen atom to which the above "C₃-C₇ alkynylcarbonyl group" binds, and, for example, can be a propioloyloxy group, a 1-propynylcarbonyloxy group, a propargylcarbonyloxy group, a 1-butynylcarbonyloxy group, a 2-butynylcarbonyloxy group, a 3-butynylcarbonyloxy group, 1-pentynylcarbonyloxy group or a 1-hexynylcarbonyloxy group, and is preferably a carbonyloxy group to which an alkynyl group having a carbon number of 2-4 binds, more preferably a propargylcarbonyloxy group.

In the present invention, the "substituted benzoyloxy group (substituent(s) being selected from the substituent group b)" is a benzoyloxy group substituted with 1-3 same or different substituent(s) selected from the substituent group b, and, for example, can be a fluorobenzoyloxy group, a chlorobenzoyloxy group, a bromobenzoyloxy group, a difluorobenzoyloxy group, a dichlorobenzoyloxy group, a methylbenzoyloxy group, an ethylbenzoyloxy group, a dimethylbenzoyloxy group, a trimethylbenzoyloxy group, a trifluoromethylbenzoyloxy group, a hydroxybenzoyloxy group, a methoxybenzoyloxy group, an ethoxybenzoyloxy group, a dimethoxybenzoyloxy group, a trifluoromethoxybenzoyloxy group, a benzoyloxybenzoyloxy group, a carboxylbenzoyloxy group, a methoxycarbonylbenzoyloxy group, an ethoxycarbonylbenzoyloxy group, a cyanobenzoyloxy group, a nitrobenzoyloxy group, an aminobenzoyloxy group, a methylaminobenzoyloxy group, an acetylaminobenzoyloxy group, a benzoylaminobenzoyloxy group or a phenylbenzoyloxy group, and is preferably a benzoyloxy group substituted with one fluorine atom, chlorine atom or bromine atom, more preferably a benzoyloxy group substituted with one fluorine atom, further more preferably a benzoyloxy group substituted with one fluorine atom, particularly preferably a fluorobenzoyloxy group.

In the present invention, the "5-6 membered unsaturated (heterocyclyl)carbonyloxy group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)" is preferably a 5-6 membered unsaturated (heterocyclyl)carbonyloxy group wherein the heteroatom for the heterocycle is an oxygen atom, a sulfur atom or a nitrogen atom, and may be 1-2 further nitrogen atom(s), the heterocycle may be fused with a benzene ring, and which may be substituted with one oxo group, further more preferably a 2-pyridylcarbonyloxy group, a 4-pyridylcarbonyloxy group, a 3-furylcarbonyloxy group, a 2-furylcarbonyloxy group, a 2-thienylcarbonyloxy group, a 7-benzothiazolylcarbonyloxy group or a 3-pyridylcarbonyloxy group, more preferably a 2-pyridylcarbonyloxy group, a 4-pyridylcarbonyloxy group or a 7-benzothiazolylcarbonyloxy group.

In the present invention, the "optionally substituted 5-6 membered unsaturated (heterocyclyl)carbonyloxy group (substituent(s) being selected from the substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)" is the above "5-6 membered unsaturated (heterocyclyl)carbonyloxy group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)" optionally substituted with 1-3 same or different substituent(s) selected from the substituent group b, preferably a 5-6 membered unsaturated (heterocyclyl)carbonyloxy group which may be substituted with 1-3 same or different substituent(s) selected from the group consisting of a fluorine atom, a chlorine atom, a bromine atom, a straight or branched alkyl group having a carbon number of 1-4, a straight or branched haloalkyl group having a carbon number of 1-3 (the halogen substituent(s) is (are) 1-3 same or different halogen atom(s) selected from the group consisting of a fluorine atom, a chlorine atom and a bromine atom), a straight or branched alkoxy group having a carbon number of 1-3 and a phenyl group, and wherein the heteroatom for the heterocycle is an oxygen atom, a sulfur atom or a nitrogen atom, and may be 1-2 further nitrogen atom(s), the heterocycle may be fused with a benzene ring, and which may be substituted with one oxo group, further more preferably a 2-pyridylcarbonyloxy group, a 4-pyridylcarbonyloxy group, a (dichloro-4-pyridyl)carbonyloxy group, a (chloro-methoxy-4-pyridyl)carbonyloxy group, a (dimethyl-3-furyl)carbonyloxy group, a (t-butyl-methyl-3-furyl)carbonyloxy group, a (trifluoromethyl-3-furyl)carbonyloxy group, a 2-furylcarbonyloxy group, a 2-thienylcarbonyloxy group, a 7-benzothiazolylcarbonyloxy group or a 3-pyridylcarbonyloxy group, more preferably a 2-pyridylcarbonyloxy group, a 2,6-dichloro-4-pyridylcarbonyloxy group or a 7-benzothiazolylcarbonyloxy group.

In the present invention, the "C₂-C₇ alkoxycarbonyloxy group" is an oxygen atom to which the above "C₂-C₇ alkoxycarbonyl group" binds, and, for example, can be a methoxycarbonyloxy group, an ethoxycarbonyloxy group, a propoxycarbonyloxy group, an isopropoxycarbonyloxy group, a butoxycarbonyloxy group, a pentoxycarbonyloxy group or a hexyloxycarbonyloxy group, and is preferably an alkoxycarbonyloxy group having a carbon number of 2-5, more preferably a methoxycarbonyloxy group or an ethoxycarbonyloxy group.

In the present invention, the "C₈-C₁₀ aralkyloxycarbonyl group" is a carbonyl group to which the above "C₇-C₉ aralkyloxy group" binds, for example, a benzyloxycarbonyl group, a phenethyloxycarbonyl group, an α-methylbenzyloxycarbonyl group or a 3-phenylpropoxycarbonyl group, preferably an aralkyloxycarbonyl group having a carbon number of 8-9, more preferably a benzyloxycarbonyl group.

In the present invention, the "C₈-C₁₀ aralkyloxycarbonyloxy group" is an oxygen atom to which the above "C₈-C₁₀ aralkyloxycarbonyl group" binds, for example, a benzyloxycarbonyloxy group, a phenethyloxycarbonyloxy group, an α-methylbenzyloxycarbonyloxy group or a 3-phenylpropoxycarbonyloxy group, preferably a carbonyloxy group to which one aralkyloxy group having a carbon number of 7-8 binds, more preferably a benzyloxycarbonyloxy group.

In the present invention, the "C₃-C₇ alkenyloxycarbonyl group" is a carbonyl group to which the above "C₂-C₆ alkenyloxy group" binds, and, for example, can be a vinyloxycarbonyloxycarbonyl group, a 1-propenyloxycarbonyloxycarbonyl group, an allyloxycarbonyloxycarbonyl group, a 2-methylallyloxycarbonyloxycarbonyl group, a 1-butenyloxycarbonyloxycarbonyl group, a 2-butenyloxycarbonyloxycarbonyl group, a 2-methyl-2-butenyloxycarbonyloxycarbonyl group, a 3-butenyloxycarbonyloxycarbonyl group, a 1-pentenyloxycarbonyloxycarbonyl group, a 1-isopentenyloxycarbonyloxycarbonyl group, a 2-pentenyloxycarbonyloxycarbonyl group, a 1-hexenyloxycarbonyloxycarbonyl group or a 2-hexenyloxycarbonyloxycarbonyl group, and is preferably a carbonyloxycarbonyl group to which one straight or branched alkenyloxy group having a carbon number of 2-5 binds, more preferably a carbonyloxycarbonyl group to which one straight or branched alkenyloxy group having a carbon number of 3-5 binds, further more preferably an allyloxycarbonyloxy group or a 2-methyl-2-butenyloxycarbonyloxycarbonyl group.

In the present invention, the "C₃-C₇ alkenyloxycarbonyloxy group" is an oxygen atom to which the above "C₃-C₇ alkenyloxycarbonyl group" binds, and, for example, can be a vinyloxycarbonyloxy group, a 1-propenyloxycarbonyloxy group, an allyloxycarbonyloxy group, a 2-methylallyloxycarbonyloxy group, a 1-butenyloxycarbonyloxy group, a 2-butenyloxycarbonyloxy group, a 2-methyl-2-butenyloxycarbonyloxy group, a 3-butenyloxycarbonyloxy group, a 1-pentenyloxycarbonyloxy group, a 1-isopentenyloxycarbonyloxy group, a 2-pentenyloxycarbonyloxy group, a 1-hexenyloxycarbonyloxy group or a 2-hexenyloxycarbonyloxy group, and is preferably a carbonyloxy group to which one straight or branched alkenyloxy group having a carbon number of 2-5 binds, more preferably a carbonyloxy group to which one straight or branched alkenyloxy group having a carbon number of 3-5 binds, further more preferably an allyloxycarbonyloxy group or a 2-methyl-2-butenyloxycarbonyloxy group.

In the present invention, the "optionally substituted phenoxycarbonyloxy group (substituent(s) being selected from the substituent group b)" is a phenoxycarbonyloxy group optionally substituted with 1-3 same or different substituent(s) selected from the substituent group b, and, for example, can be a phenoxycarbonyloxy group, a fluorophenoxycarbonyloxy group, a chlorophenoxycarbonyloxy group, a bromophenoxycarbonyloxy group, a difluorophenoxycarbonyloxy group, a dichlorophenoxycarbonyloxy group, a methylphenoxycarbonyloxy group, an ethylphenoxycarbonyloxy group, a dimethylphenoxycarbonyloxy group, a trimethylphenoxycarbonyloxy group, a trifluoromethylphenoxycarbonyloxy group, a hydroxyphenoxycarbonyloxy group, a methoxyphenoxycarbonyloxy group, an ethoxyphenoxycarbonyloxy group, a dimethoxyphenoxycarbonyloxy group, a trifluoromethoxyphenoxycarbonyloxy group, a benzoyloxyphenoxycarbonyloxy group, a carboxylphenoxycarbonyloxy group, a methoxycarbonylphenoxycarbonyloxy group, an ethoxycarbonylphenoxycarbonyloxy group, a cyanophenoxycarbonyloxy group, a nitrophenoxycarbonyloxy group, an aminophenoxycarbonyloxy group, a methylaminophenoxycarbonyloxy group, an acetylaminophenoxycarbonyloxy group, a benzoylaminophenoxycarbonyloxy group or a phenylphenoxycarbonyloxy group, and is preferably a phenoxycarbonyloxy group.

In the present invention, the "optionally substituted carbamoyloxy group (substituent(s) being selected from the substituent group c)" is a carbamoyloxy group optionally substituted with 1-2 same or different substituent(s) selected from the substituent group c, and, for example, can be a carbamoyloxy group, a methylcarbamoyloxy group, a dimethylcarbamoyloxy group, an ethylcarbamoyloxy group, a fluoromethylcarbamoyloxy group, a chloromethylcarbamoyloxy group, a difluoromethylcarbamoyloxy group, a dichloromethylcarbamoyloxy group, a trifluoromethylcarbamoyloxy group, a (methylthio)carbonyloxymethylcarbamoyloxy group, a (methylthio)carbonyloxyethylcarbamoyloxy group, an (ethylthio)carbonyloxymethylcarbamoyloxy group, a (phenylthio)carbonyloxymethylcarbamoyloxy group, a methyl(thiocarbonyl)oxymethylcarbamoyloxy group, an ethyl(thiocarbonyl)oxymethylcarbamoyloxy group, a phenyl(thiocarbonyl)oxymethylcarbamoyloxy group, a 1-cyano-1-methoxyiminomethylcarbamoyloxy group, a 1-cyano-1-ethoxyiminomethylcarbamoyloxy group, a 1-cyano-1-methoxyimino-3-methylbutylcarbamoyloxy group, an acetylcarbamoyloxy group, a propionylcarbamoyloxy group, a butyrylcarbamoyloxy group, a fluoroacetylcarbamoyloxy group, a chloroacetylcarbamoyl group, a difluoroacetylcarbamoyloxy group, a dichloroacetylcarbamoyloxy group, a trifluoroacetylcarbamoyloxy group, a 2-cyano-2-methoxyiminoacetylcarbamoyloxy group, a 2-cyano-2-ethoxyiminoacetylcarbamoyloxy group, a 2-cyano-2-methoxyimino-3-methylbutyrylcarbamoyloxy group, a methoxycarbonylcarbamoyloxy group, an ethoxycarbonylcarbamoyloxy group, a benzoylcarbamoyloxy group, a benzoylaminocarbamoyloxy group, a phenylcarbamoyloxy group, a fluorophenylcarbamoyloxy group, a chlorophenylcarbamoyloxy group, a difluorophenylcarbamoyloxy group or a dichlorophenylcarbamoyloxy group, and is preferably a carbamoyloxy group optionally substituted with 1-2 same or different straight or branched alkyl group(s) having a carbon number of 1-3, more preferably a carbamoyloxy group optionally substituted with 1-2 alkyl group(s) having a carbon number of 1-2, further more preferably a carbamoyloxy group.

In the present invention, the "optionally substituted thiocarbamoyloxy group (substituent(s) being selected from the substituent group c)" is a thiocarbamoyl group optionally substituted with 1-2 same or different substituent(s) selected from the substituent group c, and, for example, can be a thiocarbamoyloxy group, a methyl(thiocarbamoyl)oxy group, a dimethyl(thiocarbamoyl)oxy group, an ethyl(thiocarbamoyl)oxy group, a fluoromethyl(thiocarbamoyl)oxy group, a chloromethyl(thiocarbamoyl)oxy group, a difluoromethyl(thiocarbamoyl)oxy group, a dichloromethyl(thiocarbamoyl)oxy group, a trifluoromethyl(thiocarbamoyl)oxy group, a (methylthio)carbonyloxymethyl(thiocarbamoyl)oxy group, a (methylthio)carbonyloxyethyl(thiocarbamoyl)oxy group, an (ethylthio)carbonyloxymethyl(thiocarbamoyl)oxy group, a (phenylthio)carbonyloxymethyl(thiocarbamoyl)oxy group, a methyl (thiocarbonyl)oxymethyl(thiocarbamoyl)oxy group, an ethyl(thiocarbonyl)oxymethyl(thiocarbamoyl)oxy group, a phenyl(thiocarbonyl)oxymethyl(thiocarbamoyl)oxy group, a 1-cyano-1-methoxyiminomethyl(thiocarbamoyl)oxy group, a 1-cyano-1-ethoxyiminomethyl(thiocarbamoyl)oxy group, a 1-cyano-1-methoxyimino-3-methylbutyl(thiocarbamoyl)oxy group, an acetyl(thiocarbamoyl)oxy group, a propionyl(thiocarbamoyl)oxy group, a butyryl(thiocarbamoyl)oxy group, a fluoroacetyl(thiocarbamoyl)oxy group, a chloroacetyl(thiocarbamoyl)oxy group, a difluoroacetyl(thiocarbamoyl)oxy group, a dichloroacetyl (thiocarbamoyl)oxy group, a trifluoroacetyl(thiocarbamoyl)oxy group, a 2-cyano-2-methoxyiminoacetyl(thiocarbamoyl)oxy group, a 2-cyano-2-ethoxyiminoacetyl(thiocarbamoyl)oxy group, a 2-cyano-2-methoxyimino-3-methylbutyryl(thiocarbamoyl)oxy group, a methoxycarbonyl(thiocarbamoyl)oxy group, an ethoxycarbonyl(thiocarbamoyl)oxy group, a benzoyl(thiocarbamoyl)oxy group, a benzoylamino(thiocarbamoyl)oxy group, a phenyl(thiocarbamoyl)oxy group, a fluorophenyl(thiocarbamoyl)oxy group, a chlorophenyl(thiocarbamoyl)oxy group, a difluorophenyl(thiocarbamoyl)oxy group or a dichlorophenyl(thiocarbamoyl)oxy group, and is preferably a thiocarbamoyloxy group optionally substituted with 1-2 same or different straight or branched alkyl group (s) having a carbon number of 1-3, more preferably a thiocarbamoyloxy group optionally substituted with 1-2 alkyl group(s) having a carbon number of 1-2, further more preferably a thiocarbamoyloxy group.

In the present invention, the "C₂-C₇ (alkylthio)(thiocarbonyl) group" is a thiocarbonyl group to which the above "C₁-C₆ alkylthio group" binds, and, for example, can be a (methylthio)(thiocarbonyl) group, an (ethylthio)(thiocarbonyl) group, a (propylthio)(thiocarbonyl) group, an (isopropylthio)(thiocarbonyl) group, a (butylthio)(thiocarbonyl) group, a (t-butylthio)(thiocarbonyl) group, a (pentylthio)(thiocarbonyl) group or a (hexylthio)(thiocarbonyl) group, and is preferably a thiocarbonyl group to which one straight or branched alkylthio group having a carbon number of 1-3 binds, more preferably a (methylthio)(thiocarbonyl) group or an (ethylthio)(thiocarbonyl) group.

In the present invention, the "C₂-C₇ (alkylthio)(thiocarbonyloxy) group" is an oxygen atom to which the above "C₂-C₇ (alkylthio)(thiocarbonyl) group" binds, and, for example, can be a (methylthio)(thiocarbonyloxy) group, an (ethylthio)(thiocarbonyloxy) group, a (propylthio)(thiocarbonyloxy) group, an (isopropylthio)(thiocarbonyloxy) group, a (butylthio)(thiocarbonyloxy) group, a (t-butylthio)(thiocarbonyloxy) group, a (pentylthio)(thiocarbonyloxy) group or a (hexylthio)(thiocarbonyloxy) group, and is preferably a thiocarbonyloxy group to which one straight or branched alkylthio group having a carbon number of 1-3 binds, more preferably a (methylthio) (thiocarbonyloxy) group or an (ethylthio) (thiocarbonyloxy) group.

In the present invention, the "optionally substituted C₁-C₆ alkyl group {substituent(s) being cyano group, optionally substituted C₆-C₁₄ aryl group (substituent(s) being selected from the substituent group b), carboxyl group, C₂-C₇ alkoxycarbonyl group, C₈-C₁₀ aralkyloxycarbonyl group, phenoxycarbonyl group or optionally substituted carbamoyl group (substituent(s) being selected from the substituent group c)}" is the above "cyano(C₁-C₆ alkyl) group", an "optionally substituted (C₆-C₁₄ aryl) (C₁-C₆ alkyl) group (substituent(s) being selected from the substituent group b)", a "carboxyl(C₁-C₆ alkyl) group", a "(C₂-C₇ alkoxycarbonyl) (C₁-C₆ alkyl) group", a "(C₈-C₁₀ aralkyloxycarbonyl) (C₁-C₆ alkyl) group", a "phenoxycarbonyl(C₁-C₆ alkyl) group" or an "optionally substituted carbamoyl(C₁-C₆ alkyl) group (substituent(s) being selected from the substituent group c)", and is preferably a C₁-C₆ alkyl group;
the "optionally substituted (C₆-C₁₄ aryl) (C₁-C₆ alkyl) group (substituent(s) being selected from the substituent group b)" is a "C₁-C₆ alkyl group" substituted with one "C₆-C₁₄ aryl group" optionally substituted with 1-3 substituent(s) selected from the substituent group b, and is preferably the above "C₇-C₉ aralkyl group"; the "carboxyl(C₁-C₆ alkyl) group" is the above "C₁-C₆ alkyl group" substituted with one carboxyl group, and, for example, can be a carboxylmethyl group, a carboxylethyl group, a carboxylpropyl group, a carboxylisopropyl group, a carboxylbutyl group, a carboxylpentyl group or a carboxylhexyl group, and is preferably a straight or branched alkyl group having a carbon number of 1-4 substituted with one carboxyl group, more preferably a straight or branched alkyl group having a carbon number of 1-3 substituted with one carboxyl group, further more preferably a straight or branched alkyl group having a carbon number of 1-2 substituted with one carboxyl group, particularly preferably a carboxylmethyl group; the "(C₂-C₇ alkoxycarbonyl)(C₁-C₆ alkyl) group" is the above "C₁-C₆ alkyl group" substituted with one "C₂-C₇ alkoxycarbonyl group" described above, and, for example, can be a methoxycarbonylmethyl group, a methoxycarbonylethyl group, a methoxycarbonylpropyl group, a methoxycarbonylbutyl group, a methoxycarbonylpentyl group, a methoxycarbonylhexyl group, an ethoxycarbonylmethyl group, an ethoxycarbonylethyl group, an ethoxycarbonylpropyl group, an ethoxycarbonylbutyl group, an ethoxycarbonylpentyl group, an ethoxycarbonylhexyl group, a propoxycarbonylmethyl group, a propoxycarbonylethyl group, an isopropoxycarbonylmethyl group, an isopropoxycarbonylethyl group, a butoxycarbonylmethyl group, a pentoxycarbonylmethyl group or a hexyloxycarbonylmethyl group, and is preferably a straight or branched alkyl group having a carbon number of 1-3 substituted with one alkoxycarbonyl group having a carbon number of 2-5, more preferably an alkyl group having a carbon number of 1-2 substituted with one alkoxycarbonyl group having a carbon number of 2-5, further more preferably a methyl group substituted with one alkoxycarbonyl group having a carbon number of 2-5, particularly preferably a methoxycarbonylmethyl group or an ethoxycarbonylmethyl group;
the "(C₈-C₁₀ aralkyloxycarbonyl)(C₁-C₆ alkyl) group" is the above "C₁-C₆ alkyl group" substituted with one "C₈-C₁₀ aralkyloxycarbonyl group" described above, and, for example, can be a benzyloxycarbonylmethyl group, a benzyloxycarbonylethyl group, a benzyloxycarbonylpropyl group, a benzyloxycarbonylbutyl group, a benzyloxycarbonylpentyl group, a benzyloxycarbonylhexyl group, a phenethyloxycarbonylmethyl group, a phenethyloxycarbonylethyl group, an α-methylbenzyloxycarbonylmethyl group, an α-methylbenzyloxycarbonylethyl group or a 3-phenylpropoxycarbonylmethyl group, and is preferably a straight or branched alkyl group having a carbon number of 1-3 substituted with one aralkyloxycarbonyl group having a carbon number of 8-9, more preferably a benzyloxycarbonylmethyl group; the "phenoxycarbonyl(C₁-C₆ alkyl) group" is the above "C₁-C₆ alkyl group" substituted with one phenoxycarbonyl group, and, for example, can be a phenoxycarbonylmethyl group, a phenoxycarbonylethyl group, a phenoxycarbonylpropyl group, a phenoxycarbonylbutyl group, a phenoxycarbonylpentyl group or a phenoxycarbonylhexyl group, and is preferably a straight or branched alkyl group having a carbon number of 1-3 substituted with one phenoxycarbonyl group, more preferably an alkyl group having a carbon number of 1-2 substituted with one phenoxycarbonyl group, further more preferably a phenoxycarbonylmethyl group;
the "optionally substituted carbamoyl(C₁-C₆ alkyl) group (substituent(s) being selected from the substituent group c)" is the above "C₁-C₆ alkyl group" substituted with one "optionally substituted carbamoyloxy group (substituent(s) being selected from the substituent group c)" described above, and, for example, can be a carbamoylmethyl group, a carbamoylethyl group, a carbamoylpropyl group, a carbamoylisopropyl group, a carbamoylbutyl group, a carbamoylpentyl group, a carbamoylhexyl group, a methylcarbamoylmethyl group, a dimethylcarbamoylmethyl group, an ethylcarbamoylmethyl group, a fluoromethylcarbamoylmethyl group, a chloromethylcarbamoylmethyl group, a difluoromethylcarbamoylmethyl group, a dichloromethylcarbamoylmethyl group, a trifluoromethylcarbamoylmethyl group, a (methylthio)carbonyloxymethylcarbamoylmethyl group, a (methylthio)carbonyloxyethylcarbamoylmethyl group, an (ethylthio)carbonyloxymethylcarbamoylmethyl group, a (phenylthio)carbonyloxymethylcarbamoylmethyl group, a methyl(thiocarbonyl)oxymethylcarbamoylmethyl group, an ethyl(thiocarbonyl)oxymethylcarbamoylmethyl group, a phenyl(thiocarbonyl)oxymethylcarbamoylmethyl group, a 1-cyano-1-methoxyiminomethylcarbamoylmethyl group, a 1-cyano-1-ethoxyiminomethylcarbamoylmethyl group, a 1-cyano-1-methoxyimino-3-methylbutylcarbamoylmethyl group, an acetylcarbamoylmethyl group, a propionylcarbamoylmethyl group, a butyrylcarbamoylmethyl group, a fluoroacetylcarbamoylmethyl group, a chloroacetylcarbamoylmethyl group, a difluoroacetylcarbamoylmethyl group, a dichloroacetylcarbamoylmethyl group, a trifluoroacetylcarbamoylmethyl group, a 2-cyano-2-methoxyiminoacetylcarbamoylmethyl group, a 2-cyano-2-ethoxyiminoacetylcarbamoylmethyl group, a 2-cyano-2-methoxyimino-3-methylbutyrylcarbamoylmethyl group, a methoxycarbonylcarbamoylmethyl group, an ethoxycarbonylcarbamoylmethyl group, a benzoylcarbamoylmethyl group, a benzoylaminocarbamoylmethyl group, a phenylcarbamoylmethyl group, a fluorophenylcarbamoylmethyl group, a chlorophenylcarbamoylmethyl group, a difluorophenylcarbamoylmethyl group or a dichlorophenylcarbamoylmethyl group, and is preferably a straight or branched alkyl group having a carbon number of 1-3 substituted with one carbamoyl group optionally substituted with a straight or branched alkyl group having a carbon number of 1-3, a straight or branched alkyl group having a carbon number of 1-3 substituted with one cyano group, an alkylcarbonyl group having a carbon number of 2-4 or a phenyl group optionally substituted with one straight or branched alkoxy group having a carbon number of 1-3, more preferably a carbamoylmethyl group optionally substituted with a methyl group, a cyanomethyl group, a methoxycarbonyl group, a phenyl group or a methoxyphenyl group, further more preferably a carbamoylmethyl group. In the present invention, the "C₂-C₇ alkoxycarbonylamino group" is an amino group substituted with the above "C₂-C₇ alkoxycarbonyl group", and, for example, can be a methoxycarbonylamino group, an ethoxycarbonylamino group, a propoxycarbonylamino group, an isopropoxycarbonylamino group, a butoxycarbonylamino group, a pentoxycarbonylamino group or a hexyloxycarbonylamino group, and is preferably an alkoxycarbonylamino group having a carbon number of 2-5, more preferably a methoxycarbonylamino group or an ethoxycarbonylamino group.

In the present invention, the "optionally substituted C₂-C₁₇ alkylcarbonyl group (substituent(s) being halogen atom-, C₂-C₇ alkoxycarbonylamino group, cyano group or C₁-C₆ alkoxyimino group)" is a "halo(C₂-C₁₇ alkylcarbonyl) group", a "(C₂-C₇ alkoxycarbonylamino)(C₂-C₁₇ alkylcarbonyl) group", or a "cyano-substituted (C₁-C₆ alkoxyimino)(C₂-C₁₇ alkylcarbonyl) group", and is preferably a "cyano-substituted (C₁-C₆ alkoxyimino) (C₂-C₁₇ alkylcarbonyl) group";
the "halo(C₂-C₁₇ alkylcarbonyl) group" is the above "C₂-C₁₇ alkylcarbonyl group" substituted with 1-3 same or different "halogen atom(s)" described above, and, for example, can be a fluoroacetyl group, a difluoroacetyl group, a trifluoroacetyl group, a chloroacetyl group, a dichloroacetyl group, a trichloroacetyl group, a bromoacetyl group, a fluoropropionyl group, a chloropropionyl group, a fluorobutyryl group, a chlorobutyryl group, a fluoroisobutyryl group, a chloroisobutyryl group, a fluorovaleryl group, a chlorovaleryl group, a fluoroisovaleryl group, a chloroisovaleryl group, a fluoropivaloyl group or a chloropivaloyl group, and is preferably a straight or branched alkylcarbonyl group having a carbon number of 2-4 substituted with 1-3 same or different halogen atom(s) selected from the group consisting of a fluorine atom, a chlorine atom and a bromine atom, more preferably an acetyl group substituted with one of a fluorine atom and a chlorine atom, further more preferably a fluoroacetyl group or a chloroacetyl group;
the "(C₂-C₇ alkoxycarbonylamino) (C₂-C₁₇ alkylcarbonyl) group" is the above "C₂-C₇ alkylcarbonyl group" substituted with one "C₂-C₇ alkoxycarbonylamino group" described above, and, for example, can be a methoxycarbonylaminoacetyl group, a methoxycarbonylaminopropionyl group, a methoxycarbonylaminobutyryl group, a methoxycarbonylaminoisobutyryl group, a methoxycarbonylaminovaleryl group, a methoxycarbonylaminoisovaleryl group, a methoxycarbonylaminopivaloyl group, an ethoxycarbonylaminoacetyl group, a propoxycarbonylaminoacetyl group, an isopropoxycarbonylaminoacetyl group, a butoxycarbonylaminoacetyl group, a pentoxycarbonylaminoacetyl group or a hexyloxycarbonylaminoacetyl group, and is preferably a straight or branched alkylcarbonyl group having a carbon number of 2-4 substituted with one alkoxycarbonylamino group having a carbon number of 2-5, more preferably an alkylcarbonyl group having a carbon number of 2-3 substituted with one alkoxycarbonylamino group having a carbon number of 2-5, further more preferably an acetyl group substituted with one alkoxycarbonylamino group having a carbon number of 2-5, particularly preferably methoxycarbonylaminoacetyl group or an ethoxycarbonylaminoacetyl group; the "cyano-substituted (C₁-C₆ alkoxyimino) (C₂-C₁₇ alkylcarbonyl) group" is the above "C₂-C₁₇ alkylcarbonyl group" substituted with one cyano group and one "C₁-C₆ alkoxyimino group" described above, and is preferably the above "cyano-substituted (C₁-C₆ alkoxyimino)(C₂-C₇ alkylcarbonyl) group".

In the present invention, the "5-6 membered unsaturated (heterocyclyl)carbonyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" is preferably a 5-6 membered unsaturated (heterocyclyl)carbonyl group wherein the heteroatom for the heterocycle is an oxygen atom, a sulfur atom or a nitrogen atom, and may be 1-2 further nitrogen atom(s), the heterocycle may be fused with a benzene ring, and which may be substituted with one oxo group, further more preferably a 2-pyridylcarbonyl group, a 4-pyridylcarbonyl group, a 3-furylcarbonyl group, a 2-furylcarbonyl group, a 2-thienylcarbonyl group, a 7-benzothiazolylcarbonyl group or a 3-pyridylcarbonyl group, more preferably a 2-pyridylcarbonyl group, a 4-pyridylcarbonyl group or a 7-benzothiazolylcarbonyl group.

In the present invention, the "optionally substituted 5-6 membered unsaturated (heterocyclyl)carbonyl group (substituent(s) being selected from the substituent group b; the heterocycle contains an oxygen atom, a sulfur atom or a nitrogen atom, may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" is the above "5-6 membered unsaturated (heterocyclyl)carbonyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" optionally substituted with 1-3 same or different substituent(s) selected from the substituent group b, and is preferably a 5-6 membered unsaturated (heterocyclyl)carbonyl group which may be substituted with 1-3 same or different substituent(s) selected from the group consisting of a fluorine atom, a chlorine atom, a bromine atom, a straight or branched alkyl group having a carbon number of 1-4, a straight or branched haloalkyl group having a carbon number of 1-3 (the halogen substituent(s) is (are) 1-3 same or different halogen atom(s) selected from the group consisting of a fluorine atom, a chlorine atom and a bromine atom), a straight or branched alkoxy group having a carbon number of 1-3 and a phenyl group, and wherein the heteroatom for the heterocycle is an oxygen atom, a sulfur atom or a nitrogen atom, and may be 1-2 further nitrogen atom(s), and the heterocycle may be fused with a benzene ring, and which may be substituted with one oxo group, further more preferably a 2-pyridylcarbonyl group, a 4-pyridylcarbonyl group, a (dichloro-4-pyridyl)carbonyl group, a (chloro-methoxy-4-pyridyl)carbonyl group, a (dimethyl-3-furyl)carbonyl group, a (t-butyl-methyl-3-furyl)carbonyl group, a (trifluoromethyl-3-furyl)carbonyl group, a 2-furylcarbonyl group, a 2-thienylcarbonyl group, a 7-benzothiazolylcarbonyl group or a 3-pyridylcarbonyl group, more preferably a 2-pyridylcarbonyl group, a 2,6-dichloro-4-pyridylcarbonyl group or a 7-benzothiazolylcarbonyl group.

In the present invention, the "optionally substituted thiocarbamoyl group (substituent(s) being selected from the substituent group c)" is a thiocarbamoyl group optionally substituted with 1-2 same or different substituent(s) selected from the substituent group c, and, for example, can be a thiocarbamoyl group, a methyl(thiocarbamoyl) group, a dimethyl(thiocarbamoyl) group, an ethyl(thiocarbamoyl) group, a fluoromethyl(thiocarbamoyl) group, a chloromethyl(thiocarbamoyl) group, a difluoromethyl(thiocarbamoyl) group, a dichloromethyl(thiocarbamoyl) group, a trifluoromethyl(thiocarbamoyl) group, a (methylthio)carbonyloxymethyl(thiocarbamoyl) group, a (methylthio)carbonyloxyethyl(thiocarbamoyl) group, an (ethylthio)carbonyloxymethyl(thiocarbamoyl) group, a (phenylthio)carbonyloxymethyl(thiocarbamoyl) group, a methyl(thiocarbonyl)oxymethyl(thiocarbamoyl) group, an ethyl(thiocarbonyl)oxymethyl(thiocarbamoyl) group, a phenyl(thiocarbonyl)oxymethyl(thiocarbamoyl) group, a 1-cyano-1-methoxyiminomethyl(thiocarbamoyl) group, a 1-cyano-1-ethoxyiminomethyl(thiocarbamoyl) group, a 1-cyano-1-methoxyimino-3-methylbutyl(thiocarbamoyl) group, an acetyl(thiocarbamoyl) group, a propionyl(thiocarbamoyl) group, a butyryl(thiocarbamoyl) group, a fluoroacetyl(thiocarbamoyl) group, a chloroacetyl(thiocarbamoyl) group, a difluoroacetyl(thiocarbamoyl) group, a dichloroacetyl(thiocarbamoyl) group, a trifluoroacetyl(thiocarbamoyl) group, a 2-cyano-2-methoxyiminoacetyl(thiocarbamoyl) group, a 2-cyano-2-ethoxyiminoacetyl(thiocarbamoyl) group, a 2-cyano-2-methoxyimino-3-methylbutyryl(thiocarbamoyl) group, a methoxycarbonyl(thiocarbamoyl) group, an ethoxycarbonyl(thiocarbamoyl) group, a benzoyl(thiocarbamoyl) group, a benzoylamino(thiocarbamoyl) group, a phenyl(thiocarbamoyl) group, a fluorophenyl(thiocarbamoyl) group, a chlorophenyl(thiocarbamoyl) group, a difluorophenyl(thiocarbamoyl) group or a dichlorophenyl(thiocarbamoyl) group, and is preferably a thiocarbamoyloxy group optionally substituted with 1-2 straight or branched alkyl group(s) having a carbon number of 1-3, more preferably a thiocarbamoyloxy group optionally substituted with 1-2 same or different alkyl group(s) having a carbon number of 1-2, further more preferably a thiocarbamoyl group.

In the present invention, the "C₁-C₆ alkylidene group" is a straight or branched alkylidene group having a carbon number of 1-6, preferably an alkylidene group having a carbon number of 1-3, more preferably an ethylidene group or a 2-propylidene group.

In the present invention, the "optionally substituted C₁-C₆ alkylidene group (substituent(s) being selected from the substituent group e)" is the above "C₁-C₆ alkylidene group" optionally substituted with 1-2 same or different substituent(s) selected from the substituent group e, and is preferably a straight or branched alkylidene group having a carbon number of 1-6 optionally substituted with 1-2 substituent(s) selected from the group consisting of a cycloalkyl group having a carbon number of 3-6, a straight or branched alkoxycarbonyl group having a carbon number of 2-4, an optionally substituted phenyl group {substituent(s) being straight or branched haloalkyl group having a carbon number of 1-3 (the halogen substituent(s) is (are) 1-3 same or different halogen atom(s) selected from the group consisting of a fluorine atom, a chlorine atom and a bromine atom) or straight or branched alkoxy group having a carbon number of 1-3}, a 5-6 membered unsaturated heterocyclic group optionally substituted with a straight or branched alkyl group having a carbon number of 1-3 wherein the halogen atom for the heterocycle is an oxygen atom, a sulfur atom or a nitrogen atom, and may be one further nitrogen atom, and the heterocycle may be fused with a benzene ring, and a straight or branched alkoxy group having a carbon number of 1-3, more preferably an alkylidene group having a carbon number of 1-3 optionally substituted with 1-2 same or different substituent(s) selected from the group consisting of an alkoxycarbonyl group having a carbon number of 2-3, an optionally substituted phenyl group (substituent(s) being haloalkyl group having a carbon number of 1-2 substituted with 1-3 fluorine atom(s) or methoxy group), a 5-6 membered unsaturated heterocyclic group optionally substituted with one methyl group wherein the heteroatom for the heterocycle is an oxygen atom, a sulfur atom or a nitrogen atom and the heterocycle may be fused with a benzene ring, and. a methoxy group, further more preferably an alkylidene group having a carbon number of 1-3 optionally substituted with 1-2 same or different substituent(s) selected from the group consisting of a methoxycarbonyl group, a phenyl group, a trifluoromethylphenyl group, a methoxyphenyl group, a methyl-2-furyl group and a methoxy group, particularly preferably an ethylidene group or a 2-propylidene group. In the present invention, the "C₄-C₆ cycloalkylidene group (an alkylene chain of the cycloalkylidene group may be interrupted by one oxygen atom)" can be, for example, a cyclobutylidene group, a cyclopentylidene group, a cyclohexylidene group or a tetrahydropyran-4-yl group, and is preferably a cyclopentylidene group or a tetrahydropyran-4-yl group.

In the present invention, the "C₁-C₆ alkylsulfonyl group" is an SO₂ group to which the above "C₁-C₆ alkyl group" binds, and, for example, can be a methylsulfonyl group, an ethylsulfonyl group, a propylsulfonyl group, an isopropylsulfonyl group, a butylsulfonyl group, an isobutylsulfonyl group, a s-butylsulfonyl group, a t-butylsulfonyl group, a pentylsulfonyl group, a neopentylsulfonyl group or a hexylsulfonyl group, and is preferably a straight or branched alkylsulfonyl group having a carbon number of 1-4, more preferably a straight or branched alkylsulfonyl group having a carbon number of 3-4.

In the present invention, the "optionally substituted aminooxy group (substituent(s) being selected from the substituent group d)" is an aminooxy group optionally substituted with 1-2 same or different substituent(s) selected from the substituent group d, and is preferably an aminooxy group optionally substituted with a cycloalkylcarbonyl group having a carbon number of 4-6; a straight or branched alkoxycarbonyl group having a carbon number of 2-4; a straight or branched alkenyloxycarbonyl group having a carbon number of 4-5; a carbamoyl group optionally substituted with 1-2 same or different substituent(s) selected from the group consisting of a straight or branched alkyl group having a carbon number of 1-3, and an alkylcarbonyl group having a carbon number of 2-4 substituted with one cyano group and one straight or branched alkoxyimino group having a carbon number of 1-3; a straight or branched alkylidene group having a carbon number of 1-6 optionally substituted with 1-2 same or different substituent(s) selected from the group consisting of a cycloalkyl group having a carbon number of 3-6, a straight or branched alkoxycarbonyl group having a carbon number of 2-4, an optionally substituted phenyl group {substituent(s) being straight or branched haloalkyl group having a carbon number of 1-3 (the halogen substituent(s) is (are) 1-3 same or different halogen atom(s) selected from the group consisting of a fluorine atom, a chlorine atom and a bromine atom) or straight or branched alkoxy group having a carbon number of 1-3}, a 5-6 membered unsaturated heterocyclic group optionally substituted with a straight or branched alkyl group having a carbon number of 1-3 wherein the heteroatom for the heterocycle is an oxygen atom, a sulfur atom or a nitrogen atom, and may be one further nitrogen atom, and the heterocycle may be fused with a benzene ring, and a straight or branched alkoxy group having a carbon number of 1-3; or a cycloalkylidene group having a carbon number of 5-6 wherein the carbocycle may be interrupted by one oxygen atom; more preferably an aminooxy group optionally substituted with a carbamoyl group substituted with 1-2 same or different alkyl group(s) having a carbon number of 1-2; an alkylidene group having a carbon number of 1-3 optionally substituted with 1-2 same or different substituent(s) selected from the group consisting of an alkoxycarbonyl group having a carbon number of 2-3, an optionally substituted phenyl group (substituent(s) being haloalkyl group having a carbon number of 1-2 substituted with 1-3 fluorine atom(s) or methoxy group), a 5-6 membered unsaturated heterocyclic group optionally substituted with one methyl group wherein the heteroatom for the heterocycle is an oxygen atom, a sulfur atom or a nitrogen atom and the heterocycle may be fused with a benzene ring, and a methoxy group; or a cycloalkylidene group having a carbon number of 5-6; further more preferably an aminooxy group optionally substituted with a carbamoyl group optionally substituted with 1-2 methyl group(s); an alkylidene group having a carbon number of 1-3 optionally substituted with 1-2 same or different substituent(s) selected from the group consisting of a methoxycarbonyl group, a phenyl group, a trifluoromethylphenyl group, a methoxyphenyl group, a methyl-2-furyl group and a methoxy group; or a cyclopentylidene group; particularly preferably an aminooxy group, an N-ethylideneaminooxy group or an N-(2-propylidene)aminooxy group.

In the present invention, the "C₇-C₉ aralkylthio group" is a sulfur atom to which the above "C₇-C₉ aralkyl group" binds, and, for example, can be a benzylthio group, a phenethylthio group, an α-methylbenzylthio group or a 3-phenylpropylthio group, and is preferably an aralkylthio group having a carbon number of 7-8, more preferably a benzylthio group.

In the present invention, the "C₃-C₆ cycloalkylthio group" is a sulfur atom to which the above "C₃-C₆ cycloalkyl group" binds, and, for example, can be a cyclopropylthio group, a cyclopropylthio group, a cyclobutylthio group, a cyclopentylthio group or a cyclohexylthio group, and is preferably a cycloalkylthio group having a carbon number of 3-6, more preferably a cycloalkylthio group having a carbon number of 3-6, further more preferably a cycloalkylthio group having a carbon number of 5-6, particularly preferably a cyclopentylthio group or a cyclohexylthio group.

In the present invention, the "optionally substituted phenylthio group (substituent(s) being selected from the substituent group b)" is a phenylthio group substituted with 1-3 same or different substituent(s) selected from the substituent group b, and, for example, can be a phenylthiomethyl group, a phenylthioethyl group, a phenylthiopropyl group, a phenylthiobutyl group, a phenylthiopentyl group, a phenylthiohexyl group, a fluorophenylthiomethyl group, a chlorophenylthiomethyl group, a bromophenylthiomethyl group, a difluorophenylthiomethyl group, a dichlorophenylthiomethyl group, a methylphenylthiomethyl group, an ethylphenylthiomethyl group, a dimethylphenylthiomethyl group, a trimethylphenylthiomethyl group, a trifluoromethylphenylthiomethyl group, a hydroxyphenylthiomethyl group, a methoxyphenylthiomethyl group, an ethoxyphenylthiomethyl group, a dimethoxyphenylthiomethyl group, a trifluoromethoxyphenylthiomethyl group, a benzoyloxyphenylthiomethyl group, a carboxylphenylthiomethyl group, a methoxycarbonylphenylthiomethyl group, an ethoxycarbonylphenylthiomethyl group, a cyanophenylthiomethyl group, a nitrophenylthiomethyl group, an aminophenylthiomethyl group, a methylaminophenylthiomethyl group, an acetylaminophenylthiomethyl group, a benzoylaminophenylthiomethyl group or a phenylphenylthiomethyl group, more preferably a straight or branched alkyl group having a carbon number of 1-6 substituted with one phenylthio group, more preferably a straight or branched alkyl group having a carbon number of 1-3 substituted with one phenylthio group, further more preferably an alkyl group having a carbon number of 1-2 substituted with one phenylthio group, particularly preferably a phenylthiomethyl group.

In the present invention, the "5-6 membered unsaturated (heterocyclyl)thio group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" is preferably a 5-6 membered unsaturated (heterocyclyl)thio group wherein the heteroatom for the heterocycle is an oxygen atom, a sulfur atom or a nitrogen atom, and may be 1-2 further nitrogen atom(s), and the heterocycle may be fused with a benzene ring or a pyridine ring, more preferably a 5-6 membered unsaturated (heterocyclyl)thio group wherein the heteroatom for the heterocycle is an oxygen atom, a sulfur atom or a nitrogen atom and the heterocycle may be fused with a benzene ring or a pyridine ring, further more preferably a 2-pyridylthio group, a (chloro-2-pyridyl)thio group, a (trifluoromethyl-2-pyridyl)thio group, a (nitro-2-pyridyl)thio group, a (chloro-trifluoromethyl-2-pyridyl)thio group, a pyrimidin-2-ylthio group, a (methyl-pyrimidin-2-yl)thio group, a (dimethyl-pyrimidin-2-yl)thio group, a (phenyl-pyrimidin-2-yl)thio group, an imidazol-2-ylthio group, a (methyl-imidazol-2-yl)thio group, a 1,3,4-triazol-2-ylthio group, a (methyl-1,3,4-triazol-2-yl)thio group, a (t-butyl-1,3,4-triazol-2-yl)thio group, a (cyclohexyl-1,3,4-triazol-2-yl)thio group, a (methyl-1,3,4-isothiadiazol-2-yl)thio group, a 4-pyridylthio group, a benzooxazol-2-ylthio group, a benzothiazol-2-ylthio group, a benzimidazol-2-ylthio group, a 1H-imidazo[4,5-b]pyridin-2-ylthio group or a 4,5-dihydrothiazol-2-ylthio group, particularly preferably a 2-pyridylthio group, a (3-chloro-2-pyridyl)thio group, a (4-methylpyrimidin-2-yl)thio group, an imidazol-2-ylthio group, a 4-pyridylthio group, a benzimidazol-2-ylthio group or a 1H-imidazo[4,5-b]pyridin-2-ylthio group, most preferably a 2-pyridylthio group or a 2-pyrimidylthio group.

In the present invention, the "C₁-C₆ alkylsulfinyl group" is an S(O) group to which the above "C₁-C₆ alkyl group" binds, and, for example, can be a methylsulfinyl group, an ethylsulfinyl group, a propylsulfinyl group, an isopropylsulfinyl group, a butylsulfinyl group, a pentylsulfinyl group or a hexylsulfinyl group, and is preferably a straight or branched alkylsulfinyl group having a carbon number of 1-3, more preferably an alkylsulfinyl group having a carbon number of 1-2, further more preferably a methylsulfinyl group.

In the present invention, the "substituted C₁-C₆ alkylsulfinyl group (substituent(s) being cyano group)" is the above "C₁-C₆ alkylsulfinyl group" substituted with 1-2 cyano group(s), and, for example, can be a cyanomethylsulfinyl group, a cyanoethylsulfinyl group, a cyanopropylsulfinyl group, a cyanoisopropylsulfinyl group, a cyanobutylsulfinyl group, a cyanoisobutylsulfinyl group, a cyano-s-butylsulfinyl group, a cyano-t-butylsulfinyl group, a cyanopentylsulfinyl group, a cyanoneopentylsulfinyl group or a cyanohexylsulfinyl group, and is preferably a straight or branched alkylsulfinyl group having a carbon number of 1-6 substituted with one cyano group, more preferably a straight or branched alkylsulfinyl group having a carbon number of 1-4 substituted with one cyano group, further more preferably a straight or branched alkylsulfinyl group having a carbon number of 3-4 substituted with one cyano group.

In the present invention, the "C₇-C₉ aralkylsulfinyl group" is an S(O) group to which the above "C₇-C₉ aralkyl group" binds, and, for example, can be a benzylsulfinyl group, a phenethylsulfinyl group, an α-methylbenzylsulfinyl group or a 3-phenylpropylsulfinyl group, and is preferably an aralkylsulfinyl group having a carbon number of 7-8, more preferably a benzylsulfinyl group.

In the present invention, the "C₃-C₆ cycloalkylsulfinyl group" is an S(O) group to which the above "C₃-C₆ cycloalkyl group" binds, and is a cyclopropylsulfinyl group, a cyclobutylsulfinyl group, a cyclopentylsulfinyl group or a cyclohexylsulfinyl group, more preferably a cycloalkylsulfinyl group having a carbon number of 3-6, more preferably a cycloalkylsulfinyl group having a carbon number of 5-6, further more preferably a cyclopentylsulfinyl group or a cyclohexylsulfinyl group.

In the present invention, the "5-6 membered unsaturated (heterocyclyl)sulfinyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" is preferably a 5-6 unsaturated (heterocyclyl)sulfinyl group wherein the heteroatom for the heterocycle is an oxygen atom, a sulfur atom or a nitrogen atom, and may be 1-2 further nitrogen atom(s), and the heterocycle may be fused with a benzene ring or a pyridine ring, more preferably a 5-6 membered unsaturated (heterocyclyl)sulfinyl group wherein the heteroatom(s) for the heterocycle is (are) 1-2 nitrogen atom(s), further more preferably a 2-pyridylsulfinyl group or a 2-pyrimidinylsulfinyl group.

In the present invention; the "substituted C₁-C₆ alkylsulfonyl group (substituent(s) being cyano group)" is the above "C₁-C₆ alkylsulfonyl group" substituted with 1-2 cyano group(s), and, for example, can be a cyanomethylsulfonyl group, a cyanoethylsulfonyl group, a cyanopropylsulfonyl group, a cyanoisopropylsulfonyl group, a cyanobutylsulfonyl group, a cyanoisobutylsulfonyl group, a cyano-s-butylsulfonyl group, a cyano-t-butylsulfonyl group, a cyanopentylsulfonyl group, a cyanoneopentylsulfonyl group or a cyanohexylsulfonyl group, and is preferably a straight or branched alkylsulfonyl group having a carbon number of 1-6 substituted with one cyano group, more preferably a straight or branched alkylsulfonyl group having a carbon number of 1-4 substituted with one cyano group, further more preferably an alkylsulfonyl group having a carbon number of 1-2 substituted with one straight or branched alkylsulfonyl group having a carbon number of 3-4 substituted with one cyano group, particularly preferably a methyl group substituted with one straight or branched alkylsulfonyl group having a carbon number of 3-4 substituted with one cyano group.

In the present invention, the "C₇-C₉ aralkylsulfonyl group" is an SO₂ group to which the above "C₇-C₉ aralkyl group" binds, and, for example, can be a benzylsulfonyl group, a phenethylsulfonyl group, an α-methylbenzylsulfonyl group or a 3-phenylpropylsulfonyl group, and is preferably an aralkylsulfonyl group having a carbon number of 7-8, more preferably a benzylsulfonyl group.

In the present invention, the "C₃-C₆ cycloalkylsulfonyl group" is an SO₂ group to which the above "C₃-C₆ cycloalkyl group" binds, and is an cyclopropylsulfonyl group, a cyclobutylsulfonyl group, a cyclopentylsulfonyl group or a cyclohexylsulfonyl group, and is preferably a cycloalkylsulfonyl group having a carbon number of 3-6, more preferably a cycloalkylsulfonyl group having a carbon number of 5-6, further more preferably a cyclopentylsulfonyl group or a cyclohexylsulfonyl group.

In the present invention, the "5-6 membered unsaturated (heterocyclyl)sulfonyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" is preferably a 5-6 membered unsaturated (heterocyclyl)sulfonyl group wherein the heteroatom for the heterocycle is an oxygen atom, a sulfur atom or a nitrogen atom, and may be 1-2 further nitrogen atom(s), and the heterocycle may be fused with a benzene ring or a pyridine ring, more preferably a 5-6 membered unsaturated (heterocyclyl)sulfonyl group wherein the heteroatom(s) for the heterocycle is (are) 1-2 nitrogen atom(s), further more preferably a 2-pyridylsulfonyl group or a 2-pyrimidinylsulfonyl group.

In the present invention, the "optionally substituted amino group (substituent(s) being selected from the substituent group d)" is an amino group optionally substituted with 1-2 same or different substituent(s) selected from the substituent group d, and is preferably an amino group optionally substituted with a cycloalkylcarbonyl group having a carbon number of 4-6; a straight or branched alkoxycarbonyl group having a carbon number of 2-4; a straight or branched alkenyloxycarbonyl group having a carbon number of 4-5; a carbamoyl group optionally substituted with 1-2 same or different substituent(s) selected from the group consisting of a straight or branched alkyl group having a carbon number of 1-3, and an alkylcarbonyl group having a carbon number of 2-4 substituted with one cyano group and one straight or branched alkoxyimino group having a carbon number of 1-3; a straight or branched alkylidene group having a carbon number of 1-6 optionally substituted with 1-2 same or different substituent(s) selected from the group consisting of a cycloalkyl group having a carbon number of 3-6, a straight or branched alkoxycarbonyl group having a carbon number of 2-4, an optionally substituted phenyl group {substituent(s) being straight or branched haloalkyl group having a carbon number of 1-3 (the halogen substituent(s) is (are) 1-3 same or different halogen atom(s) selected from the group consisting of a fluorine atom, a chlorine atom and a bromine atom) or straight or branched alkoxy group having a carbon number of 1-3}, a 5-6 membered unsaturated heterocyclic group optionally substituted with a straight or branched alkyl group having a carbon number of 1-3 wherein the heteroatom for the heterocycle is an oxygen atom, a sulfur atom or a nitrogen atom, and may be one further nitrogen atom, and the heterocycle may be fused with a benzene ring, and a straight or branched alkoxy group having a carbon number of 1-3; or a cycloalkylidene group having a carbon number of 5-6 wherein the carbocycle may be interrupted by one oxygen atom; more preferably an amino group optionally substituted with a carbamoyl group substituted with 1-2 same or different alkyl group(s) having a carbon number of 1-2; an alkylidene group having a carbon number of 1-3 optionally substituted with 1-2 same or different substituent(s) selected from the group consisting of an alkoxycarbonyl group having a carbon number of 2-3, an optionally substituted phenyl group (substituent(s) being haloalkyl group having a carbon number of 1-2 substituted with 1-3 fluorine atom(s) or methoxy group), a 5-6 membered unsaturated heterocyclic group optionally substituted with one methyl group wherein the heteroatom for the heterocycle is an oxygen atom, a sulfur atom or a nitrogen atom, and the heterocycle may be fused with a benzene ring, or a methoxy group; or a cycloalkylidene group having a carbon number of 5-6; further more preferably an amino group optionally substituted with a carbamoyl group optionally substituted with 1-2 methyl group(s); an alkylidene group having a carbon number of 1-3 optionally substituted with 1-2 same or different substituent(s) selected from the group consisting of a methoxycarbonyl group, a phenyl group, a trifluoromethylphenyl group, a methoxyphenyl group, a methyl-2-furyl group and a methoxy group; or a cyclopentylidene group; particularly preferably an amino group, an N-ethylideneamino group or an N-(2-propylidene)amino group.

In the present invention, the "optionally substituted C₁-C₆ alkyl group (substituent(s) being halogen atom or cyano group)" is the above "substituted C₁-C₆ alkyl group (substituent(s) being halogen atom)" or the above "cyano(C₁-C₆ alkyl) group".

In the present invention, the "substituted C₁-C₆ alkoxy group (substituent(s) being cyano group or a C₂-C₇ alkoxycarbonyl group)" is the above "substituted C₁-C₆ alkoxy group (substituent(s) being cyano group)" or the above "(C₂-C₇ alkoxycarbonyl) (C₁-C₆ alkoxy) group".

In the present invention, the "substituted imino group {substituent(s) being optionally substituted C₁-C₆ alkyl group (substituent(s) being halogen atom or cyano group), cyano group, nitro group, hydroxyl group, C₂-C₇ alkoxycarbonyloxy group, optionally substituted C₁-C₆ alkoxy group (substituent(s) being cyano group or C₂-C₇ alkoxycarbonyl group), C₇-C₉ aralkyloxy group or C₂-C₇ alkylcarbonyloxy group}" is a "substituted imino group {substituent(s) being halo(C₁-C₆ alkyl) group (the halogen substituent(s) is (are) 1-3 same or different halogen atom(s)), cyano group or nitro group}", a "C₁-C₆ alkylimino group", a hydroxyimino group, a "C₂-C₇ alkoxycarbonyloxyimino group", a "C₇-C₉ aralkyloxyimino group" or a "C₂-C₇ alkylcarbonyloxyimino group", and is preferably a hydroxyimino group, a "C₂-C₇ alkoxycarbonyloxyimino group", a "C₇-C₉ aralkyloxyimino group" or a "C₂-C₇ alkylcarbonyloxyimino group"; the "substituted imino group {substituent(s) being halo(C₁-C₆ alkyl) group (the halogen substituent(s) is (are) 1-3 same or different halogen atom(s)), cyano group or nitro group}" can be, for example, a fluoromethylimino group, a difluoromethylimino group, a trifluoromethylimino group, a fluoroethylimino group, a trifluoroethylimino group, a trifluoropropylimino group, a trifluorobutylimino group, a chloromethylimino group, a dichloromethylimino group, a trichloromethylimino group, a chloroethylimino group, a bromomethylimino group, a cyanoimino group or a nitroimino group, and is preferably an imino group substituted with one of a straight or branched alkyl group having a carbon number of 1-3 substituted with 1-3 same or different halogen atom(s) selected from the group consisting of a fluorine atom, a chlorine atom and a bromine atom, a cyano group and a nitro group, more preferably a fluoromethylimino group, a chloromethylimino group, a cyanoimino group or a nitroimino group; the "C₁-C₆ alkylimino group" is an imino group substituted with the above "C₁-C₆ alkyl group" and can be, for example, a methylimino group, an ethylimino group, a propylimino group, an isopropylimino group, a butylimino group, an isobutylimino group, a pentylimino group or a hexylimino group, and is preferably an imino group substituted with one straight or branched alkyl group having a carbon number of 1-3, more preferably a methylimino group or a propylimino group, the "C₂-C₇ alkoxycarbonyloxyimino group" is an imino group substituted with the above "C₂-C₇ alkoxycarbonyloxy group" and can be, for example, a methoxycarbonyloxyimino group, an ethoxycarbonyloxyimino group, a propoxycarbonyloxyimino group, an isopropoxycarbonyloxyimino group, a butoxycarbonyloxyimino group, a pentoxycarbonyloxyimino group or a hexyloxycarbonyloxyimino group, and is preferably a straight or branched alkoxycarbonyloxyimino group having a carbon number of 2-4, more preferably an alkoxycarbonyloxyimino group having a carbon number of 2-3, further more preferably a methoxycarbonyloxyimino group or an ethoxycarbonyloxyimino group; the "C₇-C₉ aralkyloxyimino group" is an imino group substituted with the above "C₇-C₉ aralkyloxy group" and can be, for example, a benzyloxyimino group, a phenethyloxyimino group or a 3-phenylpropoxyimino group, and is preferably an aralkyloxyimino group having a carbon number of 7-8, more preferably a benzyloxyimino group;
the "C₂-C₇ alkylcarbonyloxyimino group" is an imino group substituted with the above "C₂-C₇ alkylcarbonyloxy group" and can be, for example, an acetyloxyimino group, a propionyloxyimino group, a butyryloxyimino group, an isobutyryloxyimino group, a valeryloxyimino group, an isovaleryloxyimino group or a pivaloyloxyimino group, and is preferably a straight or branched alkylcarbonyloxyimino group having a carbon number of 2-4, more preferably an acetoxyimino group.

In the present invention, the "C₁-C₆ alkylhydrazono group" is a hydrazono group substituted with one above "C₁-C₆ alkyl group", and, for example, can be a methylhydrazono group, an ethylhydrazono group, a propylhydrazono group, an isopropylhydrazono group, a butylhydrazono group, a pentylhydrazono group or a hexylhydrazono group, and is preferably a straight or branched alkylhydrazono group having a carbon number of 1-3, more preferably an alkylhydrazono group having a carbon number of 1-2, further more preferably a methylhydrazono group.

In the present invention, the "optionally substituted C₁-C₁₆ alkyl group (substituent(s) being selected from the substituent group a)" is preferably a C₁-C₁₀ alkyl group, a halo(C₁-C₈ alkyl) group (the halogen substituent(s) is (are) 1-3 same or different substituent(s) selected from the group consisting of a fluorine atom, a chlorine atom and a bromine atom), a (C₂-C₄ alkylcarbonyl)(C₁-C₃ alkyl) group, a benzoyl(C₁-C₄ alkyl) group wherein the phenyl part may be substituted (substituent(s) being fluorine atom, chlorine atom or bromine atom), a (C₂-C₄ alkoxycarbonyl)(C₁-C₃ alkyl) group, a phenoxycarbonyl(C₁-C₃ alkyl) group, a cyano(C₁-C₃ alkyl) group, a (C₃-C₆ cycloalkyl) (C₁-C₃ alkyl) group wherein the cycloalkyl part may be substituted (substituent(s) being C₁-C₃ alkyl group, fluorine atom or chlorine atom), a norbornyl(C₁-C₃ alkyl) group, a benzyl group wherein the phenyl part may be substituted {substituent(s) being fluorine atom, chlorine atom, bromine atom, cyano group or halo(C₁-C₃ alkoxy) group (the halogen substituent(s) is (are) 1-3 same or different fluorine atom(s), chlorine atom(s) or bromine atom(s))}, a (5-6 membered saturated heterocyclyl) (C₁-C₃ alkyl) group wherein the heterocycle part may be substituted (the substituent(s) is (are) 1-2 same or different C₁-C₃ alkyl group(s), and the heterocycle contains 1-2 same or different heteroatom(s) selected from the group consisting of an oxygen atom and a nitrogen atom), a (5-6 membered unsaturated heterocyclyl)(C₁-C₃ alkyl) group wherein the heterocycle part may be substituted (substituent(s) being fluorine atom, chlorine atom or bromine atom; the heterocycle contains an oxygen atom, a sulfur atom or a nitrogen atom, and may contain one further nitrogen atom, and the heterocycle may be fused with a benzene ring), a hydroxy(C₁-C₄ alkyl) group, a (C₁-C₃ alkoxy) (C₁-C₄ alkyl) group, a (C₁-C₃ alkoxy) (C₁-C₃ alkoxy) (C₁-C₃ alkyl) group, a phenoxy(C₁-C₃ alkyl) group, a (5-6 membered saturated heterocyclyl)oxy(C₁-C₃ alkyl) group (the heterocycle contains 1-2 same or different heteroatom(s) selected from the group consisting of an oxygen atom and a nitrogen atom), a tri(C₁-C₃ alkyl)silyloxy(C₁-C₄ alkyl) group, a (C₂-C₄ alkylcarbonyloxy) (C₁-C₄ alkyl) group (the alkylcarbonyloxy substituent(s) is (are) 1-2 same or different alkylcarbonyloxy group(s)), a benzoyloxy(C₁-C₃ alkyl) group wherein the phenyl part may be substituted (the substituent(s) is (are) 1-2 same or different substituent(s) selected from the group consisting of a fluorine atom, a chlorine atom and a bromine atom), a (C₁-C₄ alkylthio)(C₁-C₃ alkyl) group, a (C₃-C₆ cycloalkylthio)(C₁-C₃ alkyl) group, a (C₁-C₄ alkylsulfonyl)(C₁-C₃ alkyl) group, a (C₃-C₆ cycloalkylsulfonyl)(C₁-C₃ alkyl) group, a hydroxyimino(C₁-C₃ alkyl) group, a (C₂-C₄ alkoxycarbonyloxyimino)(C₁-C₃ alkyl) group, a (C₁-C₃ alkoxyimino)(C₁-C₃ alkyl) group wherein the alkyl part may be substituted (substituent(s) being C₂-C₄ alkoxycarbonyl group), a (C₇-C₈ aralkyloxyimino) (C₁-C₃ alkyl) group, a (C₂-C₄ alkylcarbonyloxyimino)(C₁-C₃ alkyl) group; more preferably a tri(C₁-C₃ alkyl)silyl(C₁-C₃ alkyl) group; further more preferably a C₂-C₈ alkyl group, a halo(C₃-C₆ alkyl) group (halogen substituent(s) being fluorine atom or chlorine atom), a (C₃-C₆ cycloalkyl)(C₁-C₂ alkyl) group wherein the cycloalkyl part may be substituted (substituent(s) being methyl group, fluorine atom or chlorine atom), a norbornyl(C₁-C₂ alkyl) group, a benzyl group wherein the phenyl part may be substituted (substituent(s) being fluorine atom, chlorine atom, bromine atom or cyano group), a (5-6 membered saturated heterocyclyl)(C₁-C₂ alkyl) group (the heterocycle contains one oxygen atom), a methoxy(C₃-C₄ alkyl) group, a trimethylsilyloxy(C₃-C₄ alkyl) group, an acetoxy(C₃-C₄ alkyl) group (the number of acetoxy substituents is 1-2), a (C₃-C₄ alkylthio)(C₁-C₂ alkyl) group, a (C₅-C₆ cycloalkylthio) (C₁-C₂ alkyl) group, a (C₃-C₄ alkylsulfonyl)(C₁-C₂ alkyl) group or a trimethylsilyl(C₁-C₂ alkyl) group; further still more preferably a C₂-C₆ alkyl group, a halo(C₃-C₄ alkyl) group (halogen substituent(s) being fluorine atom or chlorine atom), a (C₄-C₆ cycloalkyl)methyl group wherein the cycloalkyl part may be substituted (substituent(s) being methyl group or fluorine atom), a norbornylmethyl group, a benzyl group, a 3-fluorobenzyl group, a tetrahydropyranylmethyl group, a (C₃-C₄ alkylthio)methyl group, a (C₃-C₄ alkylsulfonyl)methyl group or a trimethylsilylmethyl group; particularly preferably a C₃-C₆ alkyl group, a cyclobutylmethyl group or a cyclopentylmethyl group; most preferably a C₄-C₅ branched alkyl group or a cyclobutylmethyl group.

In the present invention, the "optionally substituted C₂-C₆ alkenyl group (substituent(s) being selected from the substituent group f)" is preferably a C₂-C₆ alkenyl group, a halo(C₂-C₄ alkenyl) group (the halogen substituent(s) is (are) 1-2 same or different substituent(s) selected from the group consisting of a fluorine atom, a chlorine atom, a bromine atom and an iodine atom), a hydroxy(C₂-C₃ alkenyl) group wherein the alkenyl part may be substituted (substituent(s) being C₂-C₄ alkoxycarbonyl group) or a (C₁-C₃ alkoxy) (C₂-C₃ alkenyl) group wherein the alkenyl part may be substituted (substituent(s) being C₂-C₄ alkoxycarbonyl group), more preferably a C₂-C₆ alkenyl group, a halo(C₂-C₄ alkenyl) group (the halogen substituent(s) is (are) 1-2 same or different substituent(s) selected from the group consisting of a fluorine atom, a chlorine atom, a bromine atom and an iodine atom), a hydroxy(C₂-C₃ alkenyl) group wherein the alkenyl part may be substituted (substituent(s) being C₂-C₄ alkoxycarbonyl group) or a (C₁-C₃ alkoxy) (C₂-C₃ alkenyl) group wherein the alkenyl part may be substituted (substituent(s) being C₂-C₄ alkoxycarbonyl group), further more preferably a C₄-C₅ alkenyl group.

In the present invention, the "optionally substituted C₇-C₉ aralkylthio group (substituent(s) being selected from the substituent group b)" is the above "C₇-C₉ aralkylthio group" optionally substituted 1-3 same or different substituent(s) selected from the substituent group b and can be, for example, a benzylthio group, a fluorobenzylthio group, a chlorobenzylthio group, a bromobenzylthio group, a difluorobenzylthio group, a dichlorobenzylthio group, a methylbenzylthio group, an ethylbenzylthio group, a dimethylbenzylthio group, a trimethylbenzylthio group, a trifluoromethylbenzylthio group, a hydroxybenzylthio group, a methoxybenzylthio group, an ethoxybenzylthio group, a dimethoxybenzylthio group, a trifluoromethoxybenzylthio group, a benzoyloxybenzylthio group, a carboxylbenzylthio group, a methoxycarbonylbenzylthio group, an ethoxycarbonylbenzylthio group, a cyanobenzylthio group, a nitrobenzylthio group, an aminobenzylthio group, a methylaminobenzylthio group, an acetylaminobenylthio group, a benzoylaminobenzylthio group, a phenylbenzylthio group, a phenethylthio group, a fluorophenethylthio group, a chlorophenethylthio group, a methylphenethylthio group, an α-methylbenzylthio group or a 3-phenylpropylthio group, and is preferably an aralkylthio group having a carbon number of 7-8 substituted with one fluorine atom, chlorine atom or methyl group, more preferably a benzylthio group.

In the present invention, the "substituted C₁-C₆ alkylimino group {substituent(s) being C₁-C₆ alkylthio group, optionally substituted C₇-C₉ aralkylthio group (substituent(s) being selected from the substituent group b) or phenylthio group}" is the above "C₁-C₆ alkylimino group" substituted with the above "C₁-C₆ alkylthio group", the above "optionally substituted C₇-C₉ aralkylthio group (substituent(s) being selected from the substituent group b)" or a phenylthio group, and is preferably a "(C₁-C₆ alkylthio)(C₁-C₆ alkylimino) group".

In the present invention, the "optionally substituted C₁-C₆ alkoxyimino group (substituent(s) being C₁-C₆ alkoxycarbonyl group, cyano group, amino group or phenyl group)" is the above "C₁-C₆ alkoxyimino group" optionally substituted with the above "C₁-C₆ alkoxycarbonyl group", a cyano group, an amino group or a phenyl group, and is preferably a methoxyimino group.

In the present invention, the "optionally substituted C₁-C₆ alkyl group (substituent(s) being selected from the substituent group h)" is preferably a C₁-C₃ alkyl group or a halo(C₁-C₃ alkyl) group (the halogen substituent(s) is (are) 1-3 same or different substituent(s) selected from the group consisting of a fluorine atom, a chlorine atom and a bromine atom), more preferably a methyl group or a halo(C₁-C₂ alkyl) group (the halogen substituent(s) is (are) 1-3 fluorine atom(s)), further more preferably a methyl group.

In the present invention, the "optionally substituted C₂-C₆ alkenyl group (substituent(s) being phenyl group, C₁-C₆ alkoxy group or C₂-C₇ alkoxycarbonyl group)" is preferably the above "C₂-C₆ alkenyl group" optionally substituted with 1-2 different substituent(s) selected from the group consisting of a phenyl group, the above "C₁-C₆ alkoxy group" and the above "C₂-C₇ alkoxycarbonyl group".

In the present invention, the "substituted C₂-C₆ alkenyloxy group (substituent(s) being halogen atom)" is the above "C₂-C₆ alkenyloxy group" substituted with 1-2 same or different "halogen atom(s)" described above, and, for example, can be a fluorovinyloxy group, a difluorovinyloxy group, a trifluorovinyloxy group, a chlorovinyloxy group, a dichlorovinyloxy group, a trichlorovinyloxy group, a chlorofluorovinyloxy group, a bromovinyloxy group, an iodovinyloxy group, a fluoroiodovinyloxy group, a chloroiodovinyloxy group, a fluoro-1-propenyloxy group, a chloro-1-propenyloxy group, a bromo-1-propenyloxy group, an iodo-1-propenyloxy group, a fluoroallyloxy group, a chloroallyloxy group, a bromoallyloxy group, an iodoallyloxy group, a fluoro-1-butenyloxy group, a fluoro-2-butenyloxy group, a fluoro-2-methyl-2-butenyloxy group, a fluoro-3-butenyloxy group, a fluoro-1-pentenyloxy group, a fluoro-1-isopentenyloxy group, a fluoro-2-pentenyloxy group, a fluoro-1-hexenyloxy group or a fluoro-2-hexenyloxy group, and is preferably a straight or branched alkenyloxy group having a carbon number of 2-4 substituted with 1-2 same or different halogen atom(s) selected from the group consisting of a fluorine atom, a chlorine atom, a bromine atom and an iodine atom, more preferably a vinyloxy group substituted with 1-2 same or different halogen atom(s) selected from the group consisting of a fluorine atom and an iodine atom.

In the present invention, the "C₂-C₇ alkylcarbonylthio group" can be, for example, an acetylthio group, a propionylthio group, a butyrylthio group, an isobutyrylthio group, a valerylthio group, an isovalerylthio group or a pivaloylthio group, and is preferably a straight or branched alkylcarbonylthio group having a carbon number of 2-4, more preferably an alkylcarbonylthio group having a carbon number of 2-3, further more preferably an acetylthio group.

In the present invention, the "C₂-C₇ (alkylthio)(thiocarbonylthio) group" can be, for example, a (methylthio)(thiocarbonylthio) group, an (ethylthio)(thiocarbonylthio) group, a (propylthio)(thiocarbonylthio) group, a (butylthio)(thiocarbonylthio) group, a (pentylthio)(thiocarbonylthio) group or a (hexylthio)(thiocarbonylthio) group, and is preferably a straight or branched (alkylthio)(thiocarbonylthio) group having a carbon number of 2-4, more preferably an (alkylthio)(thiocarbonylthio) group having a carbon number of 2-3, further more preferably a (methylthio)(thiocarbonylthio) group.

In the present invention, the "optionally substituted C₆-C₁₄ aryl group (substituent(s) being selected from the substituent group g)" is the above "C₆-C₁₄ aryl group" optionally substituted with 1-5 same or different substituent(s) selected from the substituent group g, and, for example, can be a phenyl group, a fluorophenyl group, a difluorophenyl group, a trifluorophenyl group, a tetrafluorophenyl group, a pentafluorophenyl group, a chlorophenyl group, a bromophenyl group, a tolyl group, a dimethylphenyl group, a trimethylphenyl group, an ethylphenyl group, a fluoromethylphenyl group, a difluoromethylphenyl group, a trifluoromethylphenyl group, a chloromethylphenyl group, a carboxylphenyl group, a methoxycarbonylphenyl group, an ethoxycarbonylphenyl group, a carbamoylphenyl group, a cyanophenyl group, a methoxyphenyl group, a dimethoxyphenyl group, an ethoxyphenyl group, an isopropoxyphenyl group, a trifluoromethoxyphenyl group, a (fluoro-iodovinyl)phenyl group, a nitrophenyl group, a dinitrophenyl group, a naphthyl group, an anthryl or a ferrocenyl group, and is preferably a phenyl group optionally substituted with 1-3 same or different substitutent(s) selected from the group consisting of a fluorine atom, a chlorine atom, a bromine atom, a straight or branched alkyl group having a carbon number of 1-3, a straight or branched haloalkyl group having a carbon number of 1-3 (the halogen substituent(s) is (are) 1-3 same or different halogen atom(s) selected from the group consisting of a fluorine atom, a chlorine atom and a bromine atom), a carboxyl group, a straight or branched alkoxycarbonyl group having a carbon number of 2-4, a carbamoyl group, a cyano group, a straight or branched alkoxy group having a carbon number of 1-3, a straight or branched haloalkoxy group having a carbon number of 1-3 (the halogen substituent(s) is (are) 1-3 same or different halogen atom(s) selected from the group consisting of a fluorine atom, a chlorine atom and a bromine atom), a haloalkenyloxy group having a carbon number of 2-3 (the halogen substituent(s) is (are) 1-2 same or different halogen atom(s) selected from the group consisting of a fluorine atom, a chlorine atom, a bromine atom and an iodine atom) and a nitro group; more preferably a phenyl group optionally substituted with 1-2 same or different substituent(s) selected from the group consisting of a fluorine atom, a chlorine atom, a methyl group, an alkyl group having a carbon number of 1-2 substituted with 1-3 fluorine atom(s), a carbamoyl group, and a cyano group; further more preferably a phenyl group, a 3-fluorophenyl group, a 4-fluorophenyl group, a 3,5-difluorophenyl group, a 2-cyanophenyl group, a 3-cyanophenyl group, a 4-cyanophenyl group, a 5-fluoro-3-cyanophenyl group or a 3-nitrophenyl group.

In the present invention, the "substituted 1-pyrazolyl group {substituent(s) being C₁-C₆ alkyl group or N-acetyl-N-(cyanobenzyl)amino group}" is a 1-pyrazolyl group substituted with 1-3 same or different substituent(s) selected from the group consisting of a C₁-C₆ alkyl group and an N-acetyl-N-(cyanobenzyl)amino group, and is preferably a 1-pyrazolyl group substituted with 1-3 same or different substituent(s) selected from the group consisting of a methyl group and an N-acetyl-N-(cyanobenzyl)amino group.

In the present invention, the "optionally substituted 5-6 membered unsaturated heterocyclic group (substituent(s) being selected from the substituent group i; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" is the above "5-6 membered unsaturated heterocyclic group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" substituted with 1-3 same or different substituent(s) selected from the substituent group b or the above "substituted 1-pyrazolyl group {substituent(s) being C₁-C₆ alkyl group or N-acetyl-N-(cyanobenzyl)amino group}", and is preferably an optionally substituted 5-6 membered unsaturated heterocyclic group {substituent(s) being fluorine atom, chlorine atom, bromine atom, C₁-C₃ alkyl group, or halo(C₁-C₃ alkyl) group (the halogen substituent(s) is (are) 1-3 same or different substituent(s) selected from the group consisting of a fluorine atom, a chlorine atom and a bromine atom)}.

In the present invention, the "optionally substituted phenylsulfonyl group (substituent(s) being C₁-C₆ alkyl group)" is a phenylsulfonyl group optionally substituted with 1-5 same or different "C₁-C₆ alkyl group(s)" described above, and, for example, can be a phenylsulfonyl group, a tolylsulfonyl group, an ethyl-phenylsulfonyl group, a propyl-phenylsulfonyl group, an isopropyl-phenylsulfonyl group, a butyl-phenylsulfonyl group, an isobutyl-phenylsulfonyl group, a s-butyl-phenylsulfonyl group, a t-butyl-phenylsulfonyl group, a pentyl-phenylsulfonyl group or a hexyl-phenylsulfonyl group, and is preferably a phenylsulfonyl group optionally substituted with one straight or branched alkyl group having a carbon number of 1-3, more preferably a phenylsulfonyl group or a tolylsulfonyl group, further more preferably a 4-tolylsulfonyl group.

In the present invention, the "di(C₁-C₆ alkyl)sulfamoyl group" can be, for example, a dimethylsulfamoyl group, a diethylsulfamoyl group, an ethylmethylsulfamoyl group, a dipropylsulfamoyl group, a diisopropylsulfamoyl group, a dibutylsulfamoyl group, a dipentylsulfamoyl group or a dihexylsulfamoyl group, and is preferably a sulfamoyl group substituted with two same or different straight or branched alkyl groups having a carbon number of 1-3, more preferably a sulfamoyl group substituted with two same alkyl groups having a carbon number of 1-2, further more preferably a dimethylsulfamoyl group.

In the present invention, the "C₂-C₇ alkylcarbamoyloxy group" can be, for example, a methylcarbamoyloxy group, an ethylcarbamoyloxy group, a propylcarbamoyloxy group, an isopropylcarbamoyloxy group, a butylcarbamoyloxy group, a pentylcarbamoyloxy group or a hexylcarbamoyloxy group, and is preferably a carbamoyloxy group to which one straight or branched alkyl group having a carbon number of 1-3 binds, more preferably a carbamoyloxy group to which one alkyl group having a carbon number of 1-2 binds, further more preferably a methylcarbamoyloxy group. In the present invention, the "C₁-C₆ alkylsulfonyloxy group" can be, for example, a methylsulfonyloxy group, an ethylsulfonyloxy group, a propylsulfonyloxy group, an isopropylsulfonyloxy group, a butylsulfonyloxy group, a pentylsulfonyloxy group or a hexylsulfonyloxy group, and is preferably a straight or branched alkylsulfonyloxy group having a carbon number of 2-4, more preferably an alkylsulfonyloxy group having a carbon number of 2-3, further more preferably a methylsulfonyl group.

In the present invention, the "optionally substituted C₁-C₆ alkyl group (substituent(s) being selected from the substituent group j)" is preferably a C₁-C₆ alkyl group, a halo(C₁-C₆ alkyl) group (the halogen substituent(s) is (are) 1-3 same or different halogen atom(s)), a hydroxy(C₁-C₆ alkyl) group, a (C₁-C₆ alkoxy) (C₁-C₆ alkyl) group, a (C₂-C₇ alkylcarbonyloxy) (C₁-C₆ alkyl) group, a (C₂-C₇ alkoxycarbonyloxy) (C₁-C₆ alkyl) group, a (C₂-C₇ alkylcarbamoyloxy) (C₁-C₆ alkyl) group or a (C₁-C₆ alkylsulfonyloxy) (C₁-C₆ alkyl) group, more preferably a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a hydroxy(C₁-C₃ alkyl) group or a (C₁-C₃ alkoxy) (C₁-C₃ alkyl) group, further more preferably a methyl group.

In the present invention, the "C₂-C₇ alkylcarbamoyl group" can be, for example, a methylcarbamoyl group, an ethylcarbamoyl group, a propylcarbamoyl group, an isopropylcarbamoyl group, a butylcarbamoyl group, a pentylcarbamoyl group or a hexylcarbamoyl group, and is preferably a carbamoyl group to which one straight or branched alkyl group having a carbon number of 1-3 binds, more preferably a carbamoyl group to which one alkyl group having a carbon number of 1-2 binds, further more preferably a methylcarbamoyl group.

In the present invention, the "phenoxy(C₂-C₇ alkylcarbonyl) group" can be, for example, a phenoxyacetyl group, a phenoxypropionyl group, a phenoxybutyryl group, a phenoxyisobutyryl group, a phenoxyvaleryl group, a phenoxyisovaleryl group or a phenoxypivaloyl group, and is preferably a straight or branched alkyl group having a carbon number of 2-4 substituted with one phenoxy group, more preferably an alkyl group having a carbon number of 2-3 substituted with one phenoxy group, further more preferably a phenoxyacetyl group.

In the present invention, the "optionally substituted 4-pyrazolyl group (substituent(s) being C₁-C₆ alkyl group)" is a 4-pyrazolyl group optionally substituted with 1-2 same or different above "C₁-C₆ alkyl group(s)", and is preferably a 4-pyrazolyl group substituted with two same or different C₁-C₄ alkyl groups, more preferably a 1-isobutyl-5-methyl-4-pyrazolyl group.

In the present invention, the "optionally substituted amino group {substituent(s) being phenoxy(C₂-C₇ alkylcarbonyl) group or optionally substituted 4-pyrazolyl group (substituent(s) being C₁-C₆ alkyl group)}" is preferably a substituted amino group {the substituent(s) is (are) 1-2 same or different substituent(s) selected from the group consisting of a phenoxy(C₂-C₇ alkylcarbonyl) group and an optionally substituted 4-pyrazolyl group (the substituent(s) is (are) 1-2 same or different C₁-C₆ alkyl group(s))}, and is preferably an N-(cyano-aralkyl)-N-{1,5-di(C₁-C₄ alkyl)-4-pyrazolyl}amino group, further more preferably an N-(3-cyanobenzyl)-N-(1-isobutyl-5-methyl-4-pyrazolyl)amino group.

In the present invention, the "optionally substituted C₁-C₆ alkyl group (substituent(s) being selected from the substituent group 1)" is preferably a C₂-C₆ alkenyl group, a halo(C₂-C₆ alkenyl) group (the halogen substituent(s) is (are) 1-2 same or different halogen atom(s)), a hydroxy(C₂-C₆ alkenyl) group wherein the alkenyl part may be substituted (substituent(s) being C₂-C₇ alkoxycarbonyl group) or a (C₁-C₆ alkoxy)(C₂-C₆ alkenyl) group wherein the alkenyl part may be substituted (substituent(s) being C₂-C₇ alkoxycarbonyl group), more preferably a C₂-C₆ alkenyl group, a halo(C₂-C₄ alkenyl) group (the halogen substituent(s) is (are) 1-2 same or different substituent(s) selected from the group consisting of a fluorine atom, a chlorine atom, a bromine atom and an iodine atom), a hydroxy(C₂-C₃ alkenyl) group wherein the alkenyl part may be substituted (substituent (s) being C₂-C₄ alkoxycarbonyl group) or a (C₁-C₃ alkoxy) (C₂-C₃ alkenyl) group wherein the alkenyl part may be substituted (substituent(s) being C₂-C₄ alkoxycarbonyl group), further more preferably a C₄-C₅ alkenyl group.

In the present invention, the "substituted C₁-C₆ alkylthio group (substituent(s) being halogen atom)" is the above "C₁-C₆ alkylthio group" substituted with 1-3 same or different "halogen atom(s)" described above, and, for example, can be a trifluoromethylthio group, a trichloromethylthio group, a difluoromethylthio group, a dichloromethylthio group, a dibromomethylthio group, a fluoromethylthio group, a trifluoroethylthio group, a trichloroethylthio group, a bromoethylthio group, a chloroethylthio group, a fluoroethylthio group, an iodoethylthio group, a chloropropylthio group, a fluorobutylthio group, an iodohexylthio group, a dibromoethylthio group, a pentafluoroethylthio group, a pentafluoropropylthio group, a fluoropentylthio group or a fluorohexylthio group, and is preferably a straight or branched alkylthio group having a carbon number of 1-3 substituted with 1-5 same or different substituent(s) selected from the group consisting of a fluorine atom, a chlorine atom and a bromine atom, more preferably an alkylthio group having a carbon number of 1-2 substituted with 1-3 fluorine atom(s), more preferably a trifluoromethylthio group.

In the present invention, the "optionally substituted C₁-C₆ alkylsulfinyl group (substituent(s) being halogen atom)" is the above "C₁-C₆ alkylsulfinyl group" substituted with 1-3 same or different "halogen atom(s)" defined above, and, for example, can be a trifluoromethylsulfinyl group, a trichloromethylsulfinyl group, a difluoromethylsulfinyl group, a dichloromethylsulfinyl group, a dibromomethylsulfinyl group, a fluoromethylsulfinyl group, a trifluoroethylsulfinyl group, a trichloroethylsulfinyl group, a bromoethylsulfinyl group, a chloroethylsulfinyl group, a fluoroethylsulfinyl group, an iodoethylsulfinyl group, a chloropropylsulfinyl group, a fluorobutylsulfinyl group, an iodohexylsulfinyl group, a dibromoethylsulfinyl group, a pentafluoroethylsulfinyl group, a pentafluoropropylsulfinyl group, a fluoropentylsulfinyl group or a fluorohexylsulfinyl group, and is preferably a straight or branched alkylsulfinyl group having a carbon number of 1-3 substituted with 1-5 same or different substituent(s) selected from the group consisting of a fluorine atom, a chlorine atom and a bromine atom, more preferably an alkylsulfinyl group having a carbon number of 1-2 substituted with 1-3 fluorine atom(s), further more preferably a trifluoromethylsulfinyl group.

In the present invention, the "optionally substituted C₁-C₆ alkylsulfonyl group (substituent(s) being halogen atom)" is the above "C₁-C₆ alkylsulfonyl group" substituted with 1-3 same or different "halogen atom(s)" described above, and, for example, can be a trifluoromethylsulfonyl group, a trichloromethylsulfonyl group, a difluoromethylsulfonyl group, a dichloromethylsulfonyl group, a dibromomethylsulfonyl group, a fluoromethylsulfonyl group, a trifluoroethylsulfonyl group, a trichloroethylsulfonyl group, a bromoethylsulfonyl group, a chloroethylsulfonyl group, a fluoroethylsulfonyl group, an iodoethylsulfonyl group, a chloropropylsulfonyl group, a fluorobutylsulfonyl group, an iodohexylsulfonyl group, a dibromoethylsulfonyl group, a pentafluoroethylsulfonyl group, a pentafluoropropylsulfonyl group, a fluoropentylsulfonyl group or a fluorohexylsulfonyl group, and is preferably a straight or branched alkylsulfonyl group having a carbon number of 1-3 substituted with 1-5 same or different substituent(s) selected from the group consisting of a fluorine atom, a chlorine atom and a bromine atom, more preferably an alkylsulfonyl group having a carbon number of 1-2 substituted with 1-3 fluorine atom(s), further more preferably a trifluoromethylsulfonyl group.

In the present invention, the "optionally substituted C₆-C₁₄ aryl group (substituent(s) being selected from the substituent group k)" is the above "C₆-C₁₄ aryl group" optionally substituted with 1-5 same or different substituent(s) selected from the substituent group k, and is preferably an optionally substituted C₆-C₁₀ aryl group [the substituent(s) is (are) 1-3 same or different substituent(s) selected from the group consisting of a fluorine atom, a chlorine atom, a bromine atom, an iodine atom, a C₁-C₃ alkyl group, a halo(C₁-C₃ alkyl) group (the halogen substituent(s) is (are) 1-2 same or different substituent(s) selected from the group consisting of a fluorine atom, a chlorine atom and a bromine atom), a cyano(C₁-C₃ alkyl) group, a hydroxy(C₁-C₃ alkyl) group, a (C₁-C₃ alkylthio) (C₁-C₃ alkyl) group, a (C₁-C₃ alkylsulfonyl) (C₁-C₃ alkyl), an imino(C₁-C₃ alkyl) group wherein the alkyl part is substituted (substituent(s) being C₁-C₃ alkoxy group), a (C₁-C₃ alkylimino) (C₁-C₃ alkyl) group wherein the alkyl part is substituted {substituent(s) being C₁-C₃ alkylthio group or C₇-C₈ aralkylthio group wherein the aryl part is substituted (substituent(s) being cyano group)}, a hydroxyimino(C₁-C₃ alkyl) group wherein the alkyl part is substituted (substituent(s) being amino group), an optionally substituted C₂-C₅ alkenyl group (the substituent(s) is (are) 1-2 different substituent(s) selected from the group consisting of a C₁-C₃ alkoxy group and a C₂-C₄ alkoxycarbonyl group), a C₂-C₄ alkylcarbonyl group, a carboxyl group, a C₂-C₄ alkoxycarbonyl group, an optionally substituted carbamoyl group {substituent(s) being C₁-C₃ alkyl group, cyano(C₁-C₃ alkyl) group, C₂-C₄ alkylcarbonyl group or optionally substituted phenyl group (substituent(s) being C₁-C₃ alkoxy group)}, an optionally substituted thiocarbamoyl group (substituent(s) being C₁-C₃ alkyl group), a cyano group, a phenyl group, an optionally substituted 5 membered unsaturated heterocyclic group {substituent(s) being C₁-C₃ alkyl group or halo(C₁-C₃ alkyl) group (the halogen substituent(s) is (are) 1-3 same or different substituent(s) selected from the group consisting of a fluorine atom, a chlorine atom and a bromine atom), the heterocycle contains an oxygen atom, a sulfur atom or a nitrogen atom, and contains 1-2 further nitrogen atom(s)}, a hydroxyl group, a C₁-C₃ alkoxy group, a halo(C₁-C₃ alkoxy) group (the halogen substituent(s) is (are) 1-5 same or different substituent (s) selected from the group consisting of a fluorine atom, a chlorine atom and a bromine atom), a halo(C₂-C₄ alkenyloxy) group (the halogen substituent(s) is (are) 1-2 substituent(s) selected from the group consisting of a fluorine atom, a chlorine atom, a bromine atom and an iodine atom), an optionally substituted phenoxy group (the substituent is a C₁-C₃ alkyl group), a C₁-C₃ alkylthio group, a halo(C₁-C₃ alkylthio) group (the halogen substituent(s) is (are) 1-3 same or different substituent(s) selected from the group consisting of a fluorine atom, a chlorine atom and a bromine atom), a phenylthio group, a C₁-C₃ alkylsulfinyl group, a phenylsulfinyl group, a C₁-C₃ alkylsulfonyl group, a halo(C₁-C₃ alkylsulfonyl) group (the halogen substituent(s) is (are) 1-3 same or different substituent(s) selected from the group consisting of a fluorine atom, a chlorine atom, and a bromine atom), a phenylsulfonyl group, an azido group, a nitro group and an optionally substituted amino group {the substituent(s) is (are) 1-2 same-or different substituent(s) selected from the group consisting of a C₁-C₃ alkyl group, a C₇-C₈ aralkyl group, a formyl group, a C₂-C₅ alkylcarbonyl group, a (C₂-C₄ alkoxycarbonylamino)(C₂-C₅ alkylcarbonyl) group, a C₂-C₄ alkoxycarbonyl group, a C₂-C₄ alkyl(thiocarbonyl) group, an optionally substituted carbamoyl group (substituent(s) being C₁-C₃ alkyl group) and a C₁-C₃ alkylsulfonyl group}]; more preferably an optionally substituted phenyl group {the substituent(s) is (are) 1-2 same or different substituent(s) selected from the group consisting of a fluorine atom, a chlorine atom, a bromine atom, an iodine atom, a methyl group, a (C₁-C₂ alkylthio)methyl group, an iminomethyl group wherein the alkyl part is substituted (substituent(s) being C₁-C₂ alkoxy group), a carbamoyl group, a cyano group, a 1,2,4-oxadiazolyl group, a C₁-C₂ alkoxy group, a halo(C₁-C₂ alkoxy) group (the halogen substituent(s) is (are) 1-3 fluorine atom(s)), an optionally substituted phenoxy group (substituent(s) being methyl group), a C₁-C₂ alkylthio group, a C₁-C₂ alkylsulfinyl group, a phenylsulfinyl group, a C₁-C₂ alkylsulfonyl group, a phenylsulfonyl group, an azido group, a nitro group and an optionally substituted amino group (substituent(s) being methyl group or C₇-C₈ aralkyl group)}; further more preferably an optionally substituted phenyl group {the substituent(s) is (are) 1-2 same or different substituent(s) selected from the group consisting of a fluorine atom, a bromine atom, an iodine atom, a methyl group, a methylthiomethyl group, a cyano group, a 1,2,4-oxadiazolyl group, a methoxy group, a trifluoromethoxy group, a phenoxy group, a methylthio group, a phenylsulfinyl group, a phenylsulfonyl group, a nitro group and a dimethylamino group}; particularly preferably a 3-cyanophenyl group, a 3-nitrophenyl group or a 2-fluoro-5-cyanophenyl group; most preferably a 3-cyanophenyl group.

In the present invention, the "optionally substituted C₁-C₆ alkoxy group (substituent(s) being C₁-C₆ alkoxy group or tri(C₁-C₆ alkyl)silyl group)" is the above "C₁-C₆ alkoxy group" optionally substituted with the above "C₁-C₆ alkoxy group" or the above "tri(C₁-C₆ alkyl)silyl group", and is preferably a (C₁-C₆ alkoxy) (C₁-C₆ alkoxy) group.

In the present invention, the "optionally substituted C₁-C₆ alkoxy group {substituent(s) being optionally substituted 5-6 membered unsaturated heterocyclic group (substituents being selected from the substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), cyano group, optionally substituted C₁-C₆ alkoxy group (substituent(s) being C₁-C₆ alkoxy group or tri(C₁-C₆ alkyl)silyl group) or C₇-C₉ aralkyloxy group}" is preferably a (5-6 membered unsaturated heterocyclyl)(C₁-C₆ alkoxy) group wherein the heterocycle part may be substituted (the substituent(s) is (are) 1-2 same or different substituent(s) selected from the group consisting of a fluorine atom, a chlorine atom, a bromine atom and a C₁-C₃ alkyl group, the heterocycle contains an oxygen atom, a sulfur atom or a nitrogen atom, and may contain one further nitrogen atom); more preferably a (5-6 membered unsaturated heterocyclyl) (C₁-C₆ alkoxy) group wherein the heterocycle part may be substituted (the substituent(s) is (are) 1-2 same or different substituent(s) selected from the group consisting of a fluorine atom, a chlorine atom, a bromine atom and a methyl group, and the heterocycle contains an oxygen atom or a nitrogen atom, and may contain one further nitrogen atom); further more preferably a (dichloro-4-pyridyl)methoxy group, a (methyl-isooxazol-3-yl)methoxy group or a 3-pyridylmethoxy group.

In the present invention, the "optionally substituted C₇-C₉ aralkyloxy group (substituent(s) being selected from the substituent group b)" is the above "C₇-C₉ aralkyloxy group" optionally substituted with 1-3 same or different substituent(s) selected from the substituent group b, and, for example, can be a benzyloxy group, a trifluoromethylbenzyloxy group, a difluoromethylbenzyloxy group, a fluoromethylbenzyloxy group, a dichloromethylbenzyloxy group, a chloromethylbenzyloxy group, a cyanobenzyloxy group, a dicyanobenzyloxy group, a nitrobenzyloxy group, a dinitrobenzyloxy group, a phenethyloxy group or a 3-phenylpropoxy group, and is preferably an aralkyloxy group having a carbon number of 7-8 optionally substituted with one of a straight or branched alkyl group having a carbon number of 1-3 substituted with 1-3 same or different halogen atom(s) selected from the group consisting of a fluorine atom, a chlorine atom and a bromine atom; a cyano group and a nitro group; more preferably a benzyloxy group optionally substituted with one of an alkyl group having a carbon number of 1-2 substituted with 1-3 fluorine atom(s); a cyano group and a nitro group; further more preferably a nitrobenzyloxymethyl group or a cyanobenzyloxy group, particularly preferably a 3-nitrobenzyloxy group.

In the present invention, the "optionally substituted C₂-C₁₇ alkylcarbonyloxy group {substituent(s) being halogen atom, C₄-C₆ cycloalkenyl group, optionally substituted 5-6 membered unsaturated heterocyclic group (substituent(s) being selected from the substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), optionally substituted C₁-C₆ alkoxy group (substituent(s) being cyano group), phenoxy group, C₂-C₇ alkylcarbonyloxy group or substituted C₁-C₆ alkylthio group (substituent(s) being cyano group)}" is the above "C₂-C₁₇ alkylcarbonyloxy group" substituted with 1-3 same or different "halogen atom(s)" described above, one "C₄-C₆ cycloalkenyl group" described above, one "optionally substituted 5-6 membered unsaturated heterocyclic group (substituent(s) being selected from the substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" described above, one "optionally substituted C₁-C₆ alkoxy group (substituent(s) being cyano group)" described above, one phenoxy group, one "C₂-C₇ alkylcarbonyloxy group" or one "substituted C₁-C₆ alkylthio group (substituent(s) being cyano group)", and is preferably a C₂-C₅ alkylcarbonyloxy group.

In the present invention, the "C₈-C₁₀ aralkylcarbonyloxy group" is a carbonyloxy group to which the above "C₇-C₉ aralkyl group" binds, and, for example, can be a benzylcarbonyl group, a phenethylcarbonyl group, an α-methylbenzylcarbonyl group or a 3-phenylpropylcarbonyl group, and is preferably an aralkylcarbonyl group having a carbon number of 7-8, more preferably a benzylcarbonyl group. In the present invention, the "optionally substituted C₈-C₁₀ aralkylcarbonyloxy group (substituent(s) being selected from the substituent group b)" is the above "C₈-C₁₀ aralkylcarbonyloxy group" optionally substituted with 1-3 same or different substituent(s) selected from the substituent group b, and, for example, can be a benzylcarbonyloxy group, a fluorobenzylcarbonyloxy group, a chlorobenzylcarbonyloxy group, a bromobenzylcarbonyloxy group, a difluorobenzylcarbonyloxy group, a dichlorobenzylcarbonyloxy group, a methylbenzylcarbonyloxy group, an ethylbenzylcarbonyloxy group, a dimethylbenzylcarbonyloxy group, a trimethylbenzylcarbonyloxy group, a trifluoromethylbenzylcarbonyloxy group, a hydroxybenzylcarbonyloxy group, a methoxybenzylcarbonyloxy group, an ethoxybenzylcarbonyloxy group, a dimethoxybenzylcarbonyloxy group, a trifluoromethoxybenzylcarbonyloxy group, a benzoyloxybenzylcarbonyloxy group, a carboxylbenzylcarbonyloxy group, a methoxycarbonylbenzylcarbonyloxy group, an ethoxycarbonylbenzylcarbonyloxy group, a cyanobenzylcarbonyloxy group, a nitrobenzylcarbonyloxy group, an aminobenzylcarbonyloxy group, a methylaminobenzylcarbonyloxy group, an acetylaminobenzylcarbonyloxy group, a benzoylaminobenzylcarbonyloxy group, a phenylbenzylcarbonyloxy group, a phenethylcarbonyloxy group, a fluorophenethylcarbonyloxy group, a chlorophenethylcarbonyloxy group, a methylphenethylcarbonyloxy group, an α-methylbenzylcarbonyloxy group or a 3-phenylpropylcarbonyloxy group, and is preferably an aralkylcarbonyloxy group having a carbon number of 8-9, more preferably a benzylcarbonyloxy group. In the present invention, the "optionally substituted C₃-C₇ alkenylcarbonyloxy group (substitutent(s) being phenyl group)" is the above "C₃-C₇ alkenylcarbonyloxy group" substituted with one phenyl group, and, for example, can be a phenylacryloyloxy group, a phenyl-1-propenylcarbonyloxy group, a phenylallylcarbonyloxy group, a phenyl-1-butenylcarbonyloxy group, a phenyl-2-butenylcarbonyloxy group, a phenyl-2-methyl-2-butenylcarbonyloxy group, a phenyl-3-butenylcarbonyloxy group, a phenyl-1-pentenylcarbonyloxy group, a phenyl-1-isopentenylcarbonyloxy group, a phenyl-2-pentenylcarbonyloxy group, a phenyl-1-hexenylcarbonyloxy group or a phenyl-2-hexenylcarbonyloxy group, and is preferably a straight or branched alkenylcarbonyloxy group having a carbon number of 4-6 substituted with one phenyl group, more preferably a phenylpentenylcarbonyloxy group.

In the present invention, the "optionally substituted cyclohexadienylcarbonyloxy group (substitutent(s) being C₁-C₆ alkyl group)" is a cyclohexadienylcarbonyloxy group optionally substituted with one "C₁-C₆ alkyl group" described above, and, for example, can be a cyclohexadienylcarbonyloxymethyl group, a methyl-cyclohexadienylcarbonyloxymethyl group, an ethyl-cyclohexadienylcarbonyloxymethyl group, a propyl-cyclohexadienylcarbonyloxymethyl group, a butyl-cyclohexadienylcarbonyloxymethyl group, a pentyl-cyclohexadienylcarbonyloxymethyl group, a hexyl-cyclohexadienylcarbonyloxymethyl group, a cyclohexadienylcarbonyloxyethyl group, a methyl-cyclohexadienylcarbonyloxyethyl group, a cyclohexadienylcarbonyloxypropyl group, a cyclohexadienylcarbonyloxybutyl group, a cyclohexadienylcarbonyloxypentyl group or a cyclohexadienylcarbonyloxyhexyl group, and is preferably a straight or a branched alkyl group having a carbon number of 1-3 substituted with one cyclohexadienylcarbonyloxy group optionally substituted with one straight or branched alkyl group having a carbon number of 1-3, more preferably an alkyl group having a carbon number of 1-2 substituted with one 1,4-cyclohexadienylcarbonyloxy group optionally substituted with one methyl group.

In the present invention, the "5-6 membered unsaturated hetero carbonyloxy group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" is the above "5-6 membered unsaturated hetero carbonyloxy group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))".

In the present invention, the "optionally substituted 5-6 membered unsaturated hetero carbonyloxy group (substituent(s) being selected from the substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" is preferably a 5-6 membered unsaturated hetero carbonyloxy group wherein the heterocycle part may be substituted {the substituent(s) is (are) 1-3 same or different substituent(s) selected from the group consisting of a fluorine atom, a chlorine atom, a bromine atom, a C₁-C₄ alkyl group, a halo(C₁-C₃ alkyl) group (the halogen substituent(s) is (are) 1-3 same or different substituent(s) selected from the group consisting of a fluorine atom, a chlorine atom and a bromine atom), a C₁-C₃ alkoxy group and a phenyl group, the heterocycle contains an oxygen atom, a sulfur atom or a nitrogen atom, and may contain 1-2 further nitrogen atom(s), the heterocycle may be fused with a benzene ring, and the heterocycle may be substituted with one oxo group}, more preferably a (5-6 membered unsaturated heterocyclyl)carbonyloxy group wherein the heterocycle part may be substituted {substituent(s) being fluorine atom, chlorine atom, C₁-C₄ alkyl group, halo (C₁-C₂ alkyl) group (the halogen substituent(s) is (are) 1-3 fluorine atom(s)), further more preferably a 4-pyridylcarbonyloxy group, a (dichloro-4-pyridyl)carbonyloxy group, a (chloro-methoxy-4-pyridyl)carbonyloxy group, a (trimethyl-pyrrol-3-yl)carbonyloxy group, a (dimethyl-3-furyl)carbonyloxy group, a (t-butyl-methyl-3-furyl)carbonyloxy group, a (trifluoromethyl-3-furyl)carbonyloxy group, a 2-furylcarbonyloxy group, a 2-thienylcarbonyloxy group or a 3-pyridylcarbonyloxy group, particularly preferably a 4-pyridylcarbonyloxy group or a 2-thienylcarbonyloxy group.

In the present invention, the "di(C₁-C₆ alkoxy)phosphoryloxy group" can be, for example, a dimethoxyphosphoryloxy group, a diethoxyphosphoryloxy group, a methoxy-ethoxy-phosphoryloxy group, a dipropoxyphosphoryloxy group, a diisopropoxyphosphoryloxy group, a dibutoxyphosphoryloxy group, a dipentoxyphosphoryloxy group or a dihexyloxyphosphoryloxy group, and is preferably a phosphoryloxy group to which two same or different straight or branched alkoxy groups having a carbon number of 1-3 bind, more preferably a phosphoryloxy group to which two same or different alkoxy groups having a carbon number of 1-2 bind, further more preferably a diethoxyphosphoryloxy group.

In the present invention, the "di(C₁-C₆ alkoxy)thiophosphoryloxy group" can be, for example, a dimethoxythiophosphoryloxy group, a diethoxythiophosphoryloxy group, a methoxy-ethoxy-thiophosphoryloxy group, a dipropoxythiophosphoryloxy group, a diisopropoxythiophosphoryloxy group, a dibutoxythiophosphoryloxy group, a dipentoxythiophosphoryloxy group or a dihexyloxythiophosphoryloxy group, and is preferably a thiophosphoryloxy group to which two same or different straight or branched alkoxy groups having a carbon number of 1-3 bind, more preferably a thiophosphoryloxy group to which two same or different alkoxy groups having a carbon number of 1-2 bind, further more preferably a diethoxythiophosphoryloxy group. In the present invention, the "optionally substituted phenylsulfonyloxy group (substituent(s) being selected from the substituent group b)" is a phenylsulfonyloxy group optionally substituted with 1-3 same or different substituent(s) selected from the substituent group b, and, for example, can be a phenylsulfonyloxy group, a fluorophenylsulfonyloxy group, a chlorophenylsulfonyloxy group, a bromophenylsulfonyloxy group, a difluorophenylsulfonyloxy group, a dichlorophenylsulfonyloxy group, a tolylsulfonyloxy group, an ethylphenylsulfonyloxy group, a dimethylphenylsulfonyloxy group, a trimethylphenylsulfonyloxy group, a trifluoromethylphenylsulfonyloxy group, a hydroxyphenylsulfonyloxy group, a methoxyphenylsulfonyloxy group, an ethoxyphenylsulfonyloxy group, a dimethoxyphenylsulfonyloxy group, a trifluoromethoxyphenylsulfonyloxy group, a benzoyloxyphenylsulfonyloxy group, a carboxylphenylsulfonyloxy group, a methoxycarbonylphenylsulfonyloxy group, an ethoxycarbonylphenylsulfonyloxy group, a cyanophenylsulfonyloxy group, a nitrophenylsulfonyloxy group, an aminophenylsulfonyloxy group, a methylaminophenylsulfonyloxy group, an acetylaminophenylsulfonyloxy group, a benzoylaminophenylsulfonyloxy group or a phenylphenylsulfonyloxy group, and is preferably a phenylsulfonyloxy group optionally substituted with one straight or branched alkyl group having a carbon number of 1-3, more preferably a phenylsulfonyloxy group or a tolylsulfonyloxy group.

In the present invention, the "optionally substituted C₁-C₆ alkylthio group (substituents(s) being C₂-C₇ alkoxycarbonyl group or cyano group)" is preferably a straight or branched alkylthio group having a carbon number of 1-6 to which one straight or branched alkoxycarbonyl group having a carbon number of 2-4 or cyano group may bind, more preferably a straight or branched alkylthio group having a carbon number of 1-3 to which one alkoxycarbonyl group having a carbon number of 2-3 or cyano group may bind, further more preferably a straight or branched alkylthio group having a carbon number of 1-3, a straight or branched alkylthio group having a carbon number of 1-3 substituted with one cyano group, or a methylthio group substituted with one alkoxycarbonyl group having a carbon number of 2-3, particularly preferably a cyanomethylthio group.

In the present invention, the "optionally substituted 5-6 membered unsaturated (heterocyclyl)thio group (substituent(s) being selected from the substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" is preferably a 5-6 membered unsaturated (heterocyclyl)thio group wherein the heterocycle part may be substituted {the substituent(s) is (are) 1-2 same or different substituent(s) selected from the group consisting of a fluorine atom, a chlorine atom, a bromine atom, a C₁-C₄ alkyl group, a halo(C₁-C₃ alkyl) group (the halogen substituent(s) is (are) 1-3 same or different substituent(s) selected from the group consisting of a fluorine atom, a chlorine atom, and a bromine atom), a C₃-C₆ cycloalkyl group, a nitro group and a phenyl group; the heterocycle contains an oxygen atom, a sulfur atom, or a nitrogen atom, may contain 1-2 further nitrogen atom(s), and the heterocycle may be fused with a benzene ring or a pyridine ring}, more preferably a 5-6 membered unsaturated (heterocyclyl)thio group wherein the heterocycle part may be substituted {the substituent(s) is (are) 1-2 same or different substituent(s) selected from the group consisting of a fluorine atom, a chlorine atom, a C₁-C₄ alkyl group, a halo(C₁-C₂ alkyl) group (the halogen substituent(s) is (are) 1-3 fluorine atom(s)), a cyclohexyl group, a nitro group and a phenyl group; the heterocycle contains an oxygen atom, a sulfur atom or a nitrogen atom, and the heterocycle may be fused with a benzene ring or a pyridine ring}, further more preferably a 2-pyridylthio group, a (chloro-2-pyridyl)thio group, a (trifluoromethyl-2-pyridyl)thio group, a (nitro-2-pyridyl)thio group, a (chloro-trifluoromethyl-2-pyridyl)thio group, a pyrimidin-2-ylthio group, a (methyl-pyrimidin-2-yl)thio group, a (dimethyl-pyrimidin-2-yl)thio group, a (phenyl-pyrimidin-2-yl)thio group, an imidazol-2-ylthio group, a (methyl-imidazol-2-yl)thio group, a 1,3,4-triazol-2-ylthio group, a (methyl-1,3,4-triazol-2-yl)thio group, a (t-butyl-1,3,4-triazol-2-yl)thio group, a (cyclohexyl-1,3,4-triazol-2-yl)thio group, a (methyl-1,3,4-isothiadiazol-2-yl)thio group, a 4-pyridylthio group, a benzooxazol-2-ylthio group, a benzothiazol-2-ylthio group, a benzimidazol-2-ylthio group, a 1H-imidazo[4,5-b]pyridin-2-ylthio group or a 4,5-dihydrothiazol-2-ylthio group, particularly preferably a (3-chloro-2-pyridyl)thio group, a (4-methyl-pyrimidin-2-yl)thio group, an imidazol-2-ylthio group, a 4-pyridylthio group, a benzimidazol-2-ylthio group, a 1H-imidazo[4,5-b]pyridin-2-ylthio group or a 2-pyrimidinylthio group, most preferably a 2-pyrimidinylthio group.

In the present invention, the "optionally substituted C₁-C₁₆ alkyl group (substituent(s) being selected from the substituent group m)" is preferably a C₁-C₁₅ alkyl group, a halo(C₁-C₃ alkyl) group (the halogen substituent(s) is (are) 1-3 same or different substituent(s) selected from the group consisting of a fluorine atom, a chlorine atom, and a bromine atom), a (C₂-C₄ alkoxycarbonyl) (C₁-C₃ alkyl) group, a cyano(C₁-C₃ alkyl) group, a (C₃-C₆ cycloalkyl) (C₁-C₃ alkyl) group, a (C₄-C₆ cycloalkenyl) (C₁-C₃ alkyl) group, a C₇-C₈ aralkyl group, a (5-6 membered saturated heterocyclyl) (C₁-C₃ alkyl) group (the heterocycle contains 1-2 same or different heteroatom(s) selected from the group consisting of an oxygen atom and a nitrogen atom), a (5-6 membered unsaturated heterocyclyl) (C₁-C₃ alkyl) group wherein the heterocycle part may be substituted {the substituent(s) is (are) 1-3 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a C₂-C₄ alkoxycarbonyl group, a cyano group, a nitro group and an amino group, and the heterocycle contains an oxygen atom, a sulfur atom or a nitrogen atom, and may contain 1-2 further nitrogen atom(s), the heterocycle may be fused with a benzene ring, and the heterocycle may be substituted with one oxo group}, a hydroxy(C₁-C₃ alkyl) group, a (C₁-C₃ alkoxy) (C₁-C₃ alkyl) group, a (C₇-C₈ aralkyloxy) (C₁-C₃ alkyl) group wherein the aryl part may be substituted {substituent(s) being halo(C₁-C₃ alkyl) group (the halogen substituent(s) is (are) 1-3 same or different substituent(s) selected from the group consisting of a fluorine atom, a chlorine atom and a bromine atom), cyano group or nitro group}, a (5-6 membered unsaturated heterocyclyl) (C₁-C₆ alkoxy) (C₁-C₆ alkyl) group wherein the heterocycle part may be substituted (the substituent(s) is (are) 1-2 same or different substituent(s) selected from the group consisting of a fluorine atom, a chlorine atom, a bromine atom and a C₁-C₃ alkyl group, and the heterocycle contains an oxygen atom, a sulfur atom or a nitrogen atom, and may contain one further nitrogen atom), a cyano (C₁-C₃ alkoxy) (C₁-C₃ alkyl) group, a (C₁-C₃ alkoxy) (C₁-C₃ alkoxy) (C₁-C₃ alkyl) group, a (C₇-C₈ aralkyloxy) (C₁-C₃ alkoxy) (C₁-C₃ alkyl) group, a (C₁-C₆ alkoxy) (C₁-C₆ alkoxy) (C₁-C₆ alkoxy) (C₁-C₆ alkyl) group, a tri(C₁-C₃ alkyl)silyl(C₁-C₃ alkoxy) (C₁-C₃ alkoxy) (C₁-C₃ alkyl) group, a (C₂-C₅ alkenyloxy) (C₁-C₃ alkyl) group, a (C₂-C₃ alkynyloxy) (C₁-C₃ alkyl) group, a phenoxy(C₁-C₃ alkyl) group wherein the phenyl part may be substituted {the substituent(s) is (are) 1-2 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom, a bromine atom), a C₁-C₃ alkoxy group, a cyano group and a nitro group}, a (5-6 membered saturated heterocyclyl)oxy(C₁-C₃ alkyl) group (the heterocycle contains 1-2 same or different heteroatom(s) selected from the group consisting of an oxygen atom and a nitrogen atom), a (5-6 membered unsaturated heterocyclyl)oxy(C₁-C₃ alkyl) group wherein the heterocycle part may be substituted {the substituent(s) is (are) 1-4 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a C₂-C₄ alkoxycarbonyl group, a cyano group, a nitro group, an amino group and a phenyl group, the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring, and the heterocycle may be substituted with 1-2 oxo group(s)}, a tri(C₁-C₄ alkyl)silyloxy(C₁-C₃ alkyl) group, a di(C₁-C₃ alkyl)(monophenyl)silyloxy(C₁-C₃ alkyl) group, a mono(C₁-C₄ alkyl)(diphenyl)silyloxy(C₁-C₃ alkyl) group, a formyloxy(C₁-C₃ alkyl) group, a (C₂-C₅ alkylcarbonyloxy)(C₁-C₃ alkyl) group, a (C₅-C₆ cycloalkenyl) (C₂-C₄ alkylcarbonyloxy) (C₁-C₃ alkyl) group, a (C₈-C₉ aralkylcarbonyloxy)(C₁-C₃ alkyl) group, a (5-6 membered unsaturated heterocyclyl) (C₂-C₇ alkylcarbonyloxy)(C₁-C₆ alkyl) group wherein the heterocycle part may be substituted (substituent(s) being C₁-C₃ alkyl group; the heterocycle contains an oxygen atom, a sulfur atom or a nitrogen atom, and may contain one further nitrogen atom, and the heterocycle may be fused with a benzene ring), a (C₁-C₃ alkoxy) (C₂-C₄ alkylcarbonyloxy) (C₁-C₃ alkyl) group, a phenoxy(C₂-C₄ alkylcarbonyloxy) (C₁-C₃ alkyl) group, a (C₂-C₄ alkylcarbonyloxy) (C₂-C₄ alkylcarbonyloxy) (C₁-C₃ alkyl) group, a (C₄-C₇ cycloalkylcarbonyloxy) (C₁-C₃ alkyl) group, a (C₄-C₆ alkenylcarbonyloxy) (C₁-C₃ alkyl) group, a phenyl(C₄-C₆ alkenylcarbonyloxy) (C₁-C₃ alkyl) group, a (C₆-C₇ cycloalkenylcarbonyloxy) (C₁-C₃ alkyl) group, a cyclohexadienylcarbonyloxy(C₁-C₃ alkyl) group wherein the cyclohexadiene part may be substituted (substituent(s) being C₁-C₃ alkyl group), a benzoyloxy(C₁-C₃ alkyl) group wherein the phenyl part may be substituted (the substituent(s) is (are) 1-2 same or different substituent(s) selected from the group consisting of a fluorine atom, a chlorine atom, a bromine atom, a C₁-C₃ alkyl group, a C₁-C₃ alkoxy group and a cyano group), a (5-6 membered unsaturated heterocyclyl)carbonyloxy(C₁-C₃ alkyl) group wherein the heterocycle part may be substituted {the substituent(s) is (are) 1-3 same or different substituent(s) selected from the group consisting of a fluorine atom, a chlorine atom, a bromine atom, a C₁-C₄ alkyl group, a halo(C₁-C₃ alkyl) group (the halogen substituent(s) is (are) 1-3 same or different substituent(s) selected from the group consisting of a fluorine atom, chlorine atom and a bromine atom), a C₁-C₃ alkoxy group and a phenyl group, the heterocycle contains an oxygen atom, a sulfur atom or a nitrogen atom, and may contain 1-2 further nitrogen atom(s), the heterocycle may be fused with a benzene ring, and the heterocycle may be substituted with one oxo group), a (C₂-C₅ alkoxycarbonyloxy) (C₁-C₃ alkyl) group, a (C₄-C₅ alkenyloxycarbonyloxy) (C₁-C₃ alkyl) group, a phenoxycarbonyloxy(C₁-C₃ alkyl) group, a carbamoyloxy(C₁-C₃ alkyl) group wherein the carbamoyl part may be substituted (the substituent(s) is (are) 1-2 same or different C₁-C₃ alkyl group(s)), a thiocarbamoyloxy(C₁-C₃ alkyl) group wherein the thiocarbamoyl part may be substituted (the substituent(s) is (are) 1-2 same or different C₁-C₃ alkyl group(s)), a [C₂-C₅ (alkylthio)carbonyloxy] (C₁-C₃ alkyl) group, an aminooxy(C₁-C₃ alkyl) group wherein the amino part may be substituted [substituent(s) being C₄-C₆ cycloalkylcarbonyl group, C₂-C₄ alkoxycarbonyl group, C₄-C₅ alkenyloxycarbonyl group, optionally substituted carbamoyl group (the substituent(s) is (are) 1-2 same or different substituent(s) selected from the group consisting of a C₁-C₃ alkyl group and a (C₁-C₃ alkoxyimino) (C₂-C₄ alkylcarbonyl) group wherein the alkyl part is cyano-substituted), optionally substituted C₁-C₆ alkylidene group <<the substituent(s) is (are) 1-2 same or different substituent(s) selected from the group consisting of a C₃-C₆ cycloalkyl group, a C₂-C₄ alkoxycarbonyl group, an optionally substituted phenyl group {substituent(s) being halo(C₁-C₃ alkyl) group (the halogen substituent(s) is (are) 1-3 same or different substituent(s) selected from the group consisting of a fluorine atom, a chlorine atom and a bromine atom) or C₁-C₃ alkoxy group}, an optionally substituted 5-6 membered unsaturated heterocyclic group (substituent(s) being C₁-C₃ alkyl group; the heterocycle contains an oxygen atom, a sulfur atom or a nitrogen atom, and may contain one further nitrogen atom, and the heterocycle may be fused with a benzene ring) and a C₁-C₃ alkoxy group» or C₅-C₆ cycloalkylidene group (an alkylene chain of the cycloalkylidene group may be interrupted by one oxygen atom)}, a di(C₁-C₃ alkoxy)phosphoryloxy(C₁-C₃ alkyl) group, a di(C₁-C₃ alkoxy)thiophosphoryloxy(C₁-C₃ alkyl) group, a (C₁-C₃ alkylsulfonyloxy) (C₁-C₃ alkyl) group, a phenylsulfonyloxy(C₁-C₃ alkyl) group wherein the phenyl part may be substituted (substituent(s) being C₁-C₃ alkyl group), a (C₁-C₆ alkylthio)(C₁-C₃ alkyl) group wherein the alkyl part of the alkylthio part may be substituted (substituent(s) being C₂-C₄ alkoxycarbonyl group or cyano group), a phenylthio(C₁-C₃ alkyl) group, a (5-6 membered unsaturated heterocyclyl)thio(C₁-C₃ alkyl) group wherein the heterocycle part may be substituted {the substituent(s) is (are) 1-2 same or different substituent(s) selected from the group consisting of a fluorine atom, a chlorine atom, a bromine atom, a C₁-C₄ alkyl group, a halo(C₁-C₃ alkyl) group (the halogen substituent(s) is (are) 1-3 same or different substituent(s) selected from the group consisting of a fluorine atom, a chlorine atom and a bromine atom), a C₃-C₆ cycloalkyl group, a nitro group and a phenyl group, the heterocycle contains an oxygen atom, a sulfur atom or a nitrogen atom, and may contain 1-2 further nitrogen atom(s), and the heterocycle may be fused with a benzene ring or a pyridine ring}, a (C₁-C₆ alkylsulfinyl) (C₁-C₃ alkyl) group, a phenylsulfinyl(C₁-C₃ alkyl) group, a (5-6 membered unsaturated heterocyclyl)sulfinyl(C₁-C₃ alkyl) group (the heterocycle contains an oxygen atom, a sulfur atom or a nitrogen atom, and may contain 1-2 further nitrogen atom(s), and the heterocycle may be fused with a benzene ring or a pyridine ring), a (C₁-C₆ alkylsulfonyl) (C₁-C₃ alkyl) group, a phenylsulfonyl(C₁-C₃ alkyl) group, a (5-6 membered unsaturated heterocyclyl)sulfonyl(C₁-C₃ alkyl) group (the heterocycle contains an oxygen atom, a sulfur atom or a nitrogen atom, may contain 1-2 further nitrogen atom(s), and the heterocycle may be fused with a benzene ring or a pyridine ring), a di(C₁-C₃ alkoxy)thiophosphorylthio(C₁-C₃ alkyl) group, an amino(C₁-C₄ alkyl) group wherein the amino part may be substituted {substituent (s) are C₁-C₃ alkyl group, carboxyl (C₁-C₄ alkyl) group, (C₂-C₄ alkoxycarbonyl) (C₁-C₄ alkyl) group, (C₈-C₉ aralkyloxycarbonyl) (C₁-C₄ alkyl) group, formyl group, (C₁-C₃ alkoxyimino) (C₂-C₄ alkylcarbonyl) group wherein the alkyl part is cyano-substituted, C₂-C₅ alkoxycarbonyl group, C₈-C₁₀ aralkyloxycarbonyl group or optionally substituted carbamoyl group (substituent(s) being C₁-C₃ alkyl group or (C₁-C₃ alkoxyimino) (C₂-C₄ alkylcarbonyl) group wherein the alkyl part is cyano-substituted)}, an anilino(C₁-C₃ alkyl) group or a (C₁-C₃ alkoxyimino) (C₁-C₃ alkyl) group wherein the alkyl part may be substituted (substituent(s) being phenyl group or cyano group); more preferably a C₁-C₇ alkyl group, a halo(C₁-C₂ alkyl) group (halogen substituent(s) being fluorine atom or chlorine atom), a (C₂-C₃ alkoxycarbonyl) (C₁-C₂ alkyl) group, a benzyl group, a morpholyl(C₁-C₂ alkyl) group, a (5-6 membered unsaturated heterocyclyl)(C₁-C₃ alkyl) group wherein the heterocycle part may be substituted {the substituent(s) is (are) 1-2 same or different substituent(s) selected from the group consisting of a chlorine atom, a bromine atom, a methyl group, a halo(C₁-C₂ alkyl) group (the halogen substituent(s) is (are) 1-3 fluorine atom(s)), a C₂-C₃ alkoxycarbonyl group and a nitro group, the heterocycle contains an oxygen atom or a nitrogen atom, and may contain 1-2 further nitrogen atom(s), the heterocycle may be fused with a benzene ring, and the heterocycle may be substituted with one oxo group}, a hydroxy(C₁-C₃ alkyl) group, a (C₁-C₃ alkoxy) (C₁-C₂ alkyl) group, a benzyloxy(C₁-C₂ alkyl) group wherein the phenyl part may be substituted {substituent(s) being halo(C₁-C₂ alkyl) group (the halogen substituent(s) is (are) 1-3 fluorine atom(s)), cyano group or nitro group}, a (5-6 membered unsaturated heterocyclyl) (C₁-C₆ alkoxy) (C₁-C₆ alkyl) group wherein the heterocycle part may be substituted (the substituent(s) is (are) 1-2 same or different substituent(s) selected from the group consisting of a fluorine atom, a chlorine atom, a bromine atom and a methyl group, the heterocycle contains an oxygen atom or a nitrogen atom, and may contain one further nitrogen atom), a cyano(C₁-C₃ alkoxy)(C₁-C₂ alkyl) group, a (C₁-C₂ alkoxy) (C₁-C₂ alkoxy) (C₁-C₂ alkyl) group, a benzyloxy(C₁-C₂ alkoxy) (C₁-C₂ alkyl) group, a trimethylsilyl(C₁-C₂ alkoxy) (C₁-C₂ alkoxy) (C₁-C₂ alkyl) group, a (C₃-C₅ alkenyloxy) (C₁-C₂ alkyl) group, a propynyloxy(C₁-C₂ alkyl) group, a phenoxy(C₁-C₂ alkyl) group wherein the phenyl part may be substituted {the substituent(s) is (are) 1-2 same or different substituent(s) selected from the group consisting of a fluorine atom, a chlorine atom, a methyl group, a halo(C₁-C₂ alkyl) group (the halogen substituent(s) is (are) 1-3 fluorine atom(s)), a methoxy group and a nitro group}, a (5-6 membered unsaturated heterocyclyl)oxy(C₁-C₂ alkyl) group wherein the heterocycle part may be substituted {the substituent(s) is (are) 1-4 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same substituent(s) selected from the group consisting of a fluorine atom, a chlorine atom and a bromine atom), a nitro group and an amino group, the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring, and the heterocycle may be substituted with 1-2 oxo group(s)}, a tri(C₁-C₄ alkyl) silyloxy(C₁-C₂ alkyl) group, a (dimethyl) (monophenyl)silyloxy(C₁-C₂ alkyl) group, a (C₂-C₅ alkylcarbonyloxy) (C₁-C₃ alkyl) group, a cyclopentenyl (C₂-C₃ alkylcarbonyloxy) (C₁-C₂ alkyl) group, a benzylcarbonyloxy (C₁-C₂ alkyl) group, a (5-6 membered unsaturated heterocyclyl) (C₂-C₇ alkylcarbonyloxy) (C₁-C₆ alkyl) group wherein the heterocycle part may be substituted (substituent(s) being methyl group;
the heterocycle contains an oxygen atom, a sulfur atom or a nitrogen atom, and may contain one further nitrogen atom, and the heterocycle may be fused with a benzene ring), a (C₁-C₂ alkoxy) (C₂-C₃ alkylcarbonyloxy) (C₁-C₂ alkyl) group, a phenoxy(C₂-C₃ alkylcarbonyloxy) (C₁-C₂ alkyl) group, a (C₂-C₃ alkylcarbonyloxy)(C₂-C₃ alkylcarbonyloxy) (C₁-C₂ alkyl) group, a (C₄-C₇ cycloalkylcarbonyloxy) (C₁-C₂ alkyl) group, a (C₄-C₆ alkenylcarbonyloxy) (C₁-C₂ alkyl) group, a phenyl (C₄-C₆ alkenylcarbonyloxy) (C₁-C₂ alkyl) group, a 1,4-cyclohexadienylcarbonyloxy (C₁-C₂ alkyl) group wherein the cyclohexadiene part may be substituted (substituent(s) being methyl group), a benzoyloxy(C₁-C₂ alkyl) group wherein the phenyl part may be substituted (the substituent(s) is (are) 1-2 same or different substituent(s) selected from the group consisting of a fluorine atom, a chlorine atom, a methyl group, a methoxy group and a cyano group), a (5-6 membered unsaturated heterocyclyl)carbonyloxy(C₁-C₃ alkyl) group wherein the heterocycle part may be substituted {substituent(s) is (are) 1-3 same or different substituent(s) selected from the group consisting of a fluorine atom, a chlorine atom, a C₁-C₄ alkyl group, a halo(C₁-C₂ alkyl) group (the halogen substituent(s) is (are) 1-3 fluorine atom(s)) and a methoxy group, the heterocycle contains an oxygen atom, a sulfur atom or a nitrogen atom, and the heterocycle may be fused with a benzene ring}, a (C₂-C₅ alkoxycarbonyloxy) (C₁-C₂ alkyl) group, an allyloxycarbonyloxy(C₁-C₂ alkyl) group, a phenoxycarbonyloxy(C₁-C₂ alkyl) group, a carbamoyloxy (C₁-C₂ alkyl) group wherein the carbamoyl part may be substituted (the substituent(s) is (are) 1-2 same or different C₁-C₂ alkyl group(s)), a thiocarbamoyloxy(C₁-C₂ alkyl) group wherein the thiocarbamoyl part may be substituted (the substituent(s) is (are) 1-2 same or different C₁-C₂ alkyl group(s)), a [C₂-C₃ (alkylthio)carbonyloxy] (C₁-C₂ alkyl) group, an aminooxy(C₁-C₂ alkyl) group wherein the amino part may be substituted [substituent(s) being optionally substituted carbamoyl group (the substituent(s) is (are) 1-2 same or different C₁-C₂ alkyl group(s)), optionally substituted C₁-C₃ alkylidene group «the substituent(s) is (are) 1-2 same or different substituent(s) selected from the group consisting of a C₂-C₃ alkoxycarbonyl group, an optionally substituted phenyl group {substituent(s) being halo(C₁-C₂ alkyl) group (the halogen substituent(s) is (are) 1-3 fluorine atom(s)) or methoxy group}, an optionally substituted 5-6 membered unsaturated heterocyclic group (substituent(s) being methyl group; the heterocycle contains an oxygen atom, a sulfur atom or a nitrogen atom, and the heterocycle may be fused with a benzene ring) and a methoxy group» or C₅-C₆ cycloalkylidene group], a di(C₁-C₂ alkoxy)phosphoryloxy(C₁-C₂ alkyl) group, a di(C₁-C₂ alkoxy)thiophosphoryloxy(C₁-C₂ alkyl) group, a C₁-C₂ alkylsulfonyloxy(C₁-C₂ alkyl) group, a phenylsulfonyloxy(C₁-C₂ alkyl) group wherein the phenyl part may be substituted (substituent(s) being methyl group), a (C₁-C₃ alkylthio) (C₁-C₂ alkyl) group wherein an alkyl part of the alkylthio part may be substituted (substituent(s) being C₂-C₃ alkoxycarbonyl group or cyano group), a phenylthio(C₁-C₂ alkyl) group, a (5-6 membered unsaturated heterocyclyl)thio(C₁-C₂ alkyl) group wherein the heterocycle part may be substituted {the substituent(s) is (are) 1-2 same or different substituent(s) selected from the group consisting of a fluorine atom, a chlorine atom, a C₁-C₄ alkyl group, a halo(C₁-C₂ alkyl) group (the halogen substituent(s) is (are) 1-3 fluorine atom(s)), a cyclohexyl group, a nitro group and a phenyl group, the heterocycle contains an oxygen atom, a sulfur atom or a nitrogen atom, and the heterocycle may be fused with a benzene ring or a pyridine ring}, a (C₁-C₃ alkylsulfinyl)(C₁-C₂ alkyl) group, a phenylsulfinyl(C₁-C₂ alkyl) group, a (6 membered unsaturated heterocyclyl)sulfinyl(C₁-C₂ alkyl) group (the heterocycle contains 1-2 nitrogen atom(s)), a (C₁-C₃ alkylsulfonyl)(C₁-C₂ alkyl) group, a phenylsulfonyl(C₁-C₂ alkyl) group, a (6 membered unsaturated heterocyclyl)sulfonyl(C₁-C₂ alkyl) group (the heterocycle contains 1-2 nitrogen atom(s)), a di(C₁-C₂ alkoxy)thiophosphorylthio(C₁-C₂ alkyl) group, an amino(C₁-C₂ alkyl) group wherein the amino part may be substituted {substituent(s) being methyl group, carboxyl(C₁-C₄ alkyl) group, (C₂-C₃ alkoxycarbonyl) (C₁-C₄ alkyl) group, benzyloxycarbonyl (C₁-C₄ alkyl) group, (C₁-C₂ alkoxyimino)methylcarbonyl group wherein the methyl part is cyano-substituted, C₂-C₃ alkoxycarbonyl group, benzyloxycarbonyl group or optionally substituted carbamoyl group (substituent(s) being C₁-C₂ alkyl group or (C₁-C₂ alkoxyimino)methylcarbonyl group wherein the methyl part is cyano-substituted)}, an anilino(C₁-C₂ alkyl) group or a (C₁-C₂ alkoxyimino)methyl group wherein the alkyl part may be substituted (substituent(s) being phenyl group or cyano group); further more preferably a C₁-C₇ alkyl group, a chloromethyl group, a (C₂-C₃ alkoxycarbonyl)methyl group, a benzyl group, a morpholinomethyl group, a pyrazol-1-ylmethyl group wherein the pyrazole part may be substituted (the substituent(s) is (are) 1-2 same or different substituent(s) selected from the group consisting of a chlorine atom, a bromine atom, a methyl group, an ethoxycarbonyl group and a nitro group), a (methyl-isooxazol-3-yl)methyl group, an imidazol-1-ylmethyl group, a (dichloro-imidazol-l-yl)methyl group, a 2-oxo-1-pyridylmethyl group, a (trifluoromethyl-2-oxo-1-pyridyl)methyl group, a 6-oxo-pyrimidin-1-ylmethyl group, a 4-oxo-quinazolin-3-ylmethyl group, a 3-oxo-pyrazol-1-ylmethyl group wherein the pyrazole part may be substituted (the substituent(s) is (are) 1-2 methyl group(s)), a 2-pyridylmethyl group, a hydroxy(C₁-C₃ alkyl) group, a (C₁-C₃ alkoxy) (C₁-C₂ alkyl) group, a nitrobenzyloxymethyl group, a cyanobenzyloxymethyl group, a (dichloro-4-pyridyl)methoxymethyl group, a (methyl-isooxazol-3-yl)methoxymethyl group, a 3-pyridylmethoxymethyl group, a cyano(C₁-C₃ alkoxy) (C₁-C₂ alkyl) group, a (C₁-C₂ alkoxy) (C₁-C₂ alkoxy)methyl group, a benzyloxymethoxymethyl group, a trimethylsilylethoxymethoxymethyl group, a (C₃-C₅ alkenyloxy)(C₁-C₂ alkyl) group, a propionyloxymethyl group, a phenoxy(C₁-C₂ alkyl) group wherein the phenyl part may be substituted (the substituent(s) is (are) 1-2 same substituent(s) selected from the group consisting of a fluorine atom, a chlorine atom, a methyl group, a trifluoromethyl group, a methoxy group and a nitro group), a (methyl-isooxazol-5-yl)oxymethyl group, a pyrazol-1-yloxymethyl group wherein the pyrazole part may be substituted (substituent(s) being bromine atom or methyl group), a 2-pyridyloxymethyl group wherein the pyridine part may be substituted (the substituent(s) is (are) 1-2 same or different substituent(s) selected from the group consisting of a fluorine atom, a chlorine atom and a trifluoromethyl group, or 3-4 fluorine atom(s)), a 3-pyridyloxymethyl group, a pyrimidin-2-yloxymethyl group, a pyrimidin-4-yloxymethyl group, an (isopropyl-methyl-pyrimidin-4-yl)oxymethyl group, a benzisooxazol-3-yloxymethyl group wherein the benzisooxazole part may be substituted (substituent(s) being chlorine atom, methyl group, nitro group or amino group), an imidazol-1-yloxymethyl group, a 1,2,4-triazol-1-yloxymethyl group, a thiazol-2-yloxymethyl group, a 2-quinolyloxymethyl group, a 3-isoquinolyloxymethyl group, a benzisothiazol-1,1-dioxide-3-yloxymethyl group, a benzothiazol-2-yloxymethyl group, an N,N-phthaloyloxymethyl group, a mono(C₃-C₄ alkyl)dimethylsilyloxymethyl group, a dimethylmonophenylsilyloxymethyl group, a (C₂-C₅ alkylcarbonyloxy)(C₁-C₂ alkyl) group, a cyclopentenylacetoxymethyl group, a benzylcarbonyloxymethyl group, a 2-thienylacetoxymethyl group, a 3-thienylacetoxymethyl group, a 3-indolylacetoxymethyl group, a 3-benzothienylacetoxymethyl group, a 2-pyridylacetoxymethyl group, a 3-pyridylacetoxymethyl group, a 4-pyridylacetoxymethyl group, a (methyl-isooxazol-5-yl)acetoxymethyl group, a methoxyacetoxymethyl group, a phenoxyacetoxymethyl group, an acetoxyacetoxymethyl group, a (C₄-C₇ cycloalkylcarbonyloxy)methyl group, a (C₄-C₆ alkenylcarbonyloxy)methyl group, a 3-ene-4-phenylbutyryloxymethyl group, a methyl-1,4-cyclohexadienylcarbonyloxymethyl group, a benzoyloxymethyl group wherein the phenyl part may be substituted (the substituent(s) is (are) 1-2 fluorine atom(s), 1-2 chlorine atom(s), one methyl group, one methoxy group or one cyano group), a 4-pyridylcarbonyloxymethyl group, a (dichloro-4-pyridyl)carbonyloxymethyl group, a (chloro-methoxy-4-pyridyl)carbonyloxymethyl group, a (trimethyl-pyrrol-3-yl)carbonyloxymethyl group, a (dimethyl-3-furyl)carbonyloxymethyl group, a (t-butyl-methyl-3-furyl)carbonyloxymethyl group, a (trifluoromethyl-3-furyl)carbonyloxymethyl group, a 2-furylcarbonyloxymethyl group, a 2-thienylcarbonyloxymethyl group, a 3-pyridylcarbonyloxymethyl group, a (C₂-C₅ alkoxycarbonyloxy)methyl group, an allyloxycarbonyloxymethyl group, a phenoxycarbonyloxymethyl group, a carbamoyloxymethyl group wherein the carbamoyl part is substituted (the substituent(s) is (are) 1-2 same or different C₁-C₂ alkyl group(s)), a thiocarbamoyloxymethyl group wherein the thiocarbamoyl part is substituted (the substituent(s) is (are) 1-2 methyl group(s)), a (methylthio)carbonyloxymethyl group, an aminooxymethyl group wherein the amino part may be substituted [substituent(s) being optionally substituted carbamoyl group (the substituent(s) is (are) 1-2 methyl group(s)), optionally substituted C₁-C₃ alkylidene group {the substituent(s) is (are) 1-2 same or different substituent(s) selected from the group consisting of a methoxycarbonyl group, an optionally substituted phenyl group (substituent(s) being trifluoromethyl group or methoxy group), a methyl-2-furyl group and a methoxy group} or cyclopentylidene group], a diethoxyphosphoryloxymethyl group, a diethoxy(thiophosphoryl)oxymethyl group, a methylsulfonyloxymethyl group, a phenylsulfonyloxymethyl group, a tolylsulfonyloxymethyl group, a (C₁-C₃ alkylthio)methyl group, a cyano(C₁-C₃ alkylthio) methyl group, a (C₂-C₃ alkoxycarbonyl)methylthiomethyl group, a phenylthiomethyl group, a 2-pyridylthiomethyl group, a (chloro-2-pyridyl)thiomethyl group, a (trifluoromethyl-2-pyridyl)thiomethyl group, a (nitro-2-pyridyl)thiomethyl group, a (chloro-trifluoromethyl-2-pyridyl)thiomethyl group, a pyrimidin-2-ylthiomethyl group, a (methyl-pyrimidin-2-yl)thiomethyl group, a (dimethyl-pyrimidin-2-yl)thiomethyl group, a (phenyl-pyrimidin-2-yl)thiomethyl group, an imidazol-2-ylthiomethyl group, a (methyl-imidazol-2-yl)thiomethyl group, a 1,3,4-triazol-2-ylthiomethyl group, a (methyl-1,3,4-triazol-2-yl)thiomethyl group, a (t-butyl-1,3,4-triazol-2-yl)thiomethyl group, a (cyclohexyl-1,3,4-triazol-2-yl)thiomethyl group, a (methyl-1,3,4-isothiadiazol-2-yl)thiomethyl group, a 4-pyridylthiomethyl group, a benzooxazol-2-ylthiomethyl group, a benzothiazol-2-ylthiomethyl group, a benzimidazol-2-ylthiomethyl group, a 1H-imidazo[4,5-b]pyridin-2-ylthiomethyl group, a 4,5-dihydrothiazol-2-ylthiomethyl group, a (C₁-C₃ alkylsulfinyl)methyl group, a phenylsulfinylmethyl group, a 2-pyridylsulfinylmethyl group, a (C₁-C₃ alkylsulfonyl)methyl group, a phenylsulfonylmethyl group, a 2-pyridylsulfonylmethyl group, a diethoxy(thiophosphoryl)thiomethyl group, an aminomethyl group wherein the amino part may be substituted (substituent(s) being methyl group, methoxycarbonyl(C₁-C₄ alkyl) group, benzyloxycarbonyl(C₁-C₄ alkyl) group, methoxyiminomethylcarbonyl group wherein the methyl part is cyano-substituted, methoxycarbonyl group, benzyloxycarbonyl group or methoxyiminomethylcarbonylcarbamoyl group wherein the methyl part is cyano-substituted), an anilinomethyl group or a methoxyiminomethyl group wherein the methyl part may be substituted (the substituent is a phenyl group or a cyano group), particularly preferably a methyl group, a 4-chloro-1-pyrazolylmethyl group, a 4-bromo-1-pyrazolylmethyl group, a hydroxymethyl group, a methoxymethyl group, a 3-nitrobenzyloxymethyl group, a 3-pyridylmethoxymethyl group, a cyanomethoxymethyl group, an allyloxymethyl group, a 2-methyl-2-butenyloxymethyl group, a phenoxymethyl group, a (fluorophenoxy)methyl group, a (chlorophenoxy)methyl group, a (difluorophenoxy)methyl group, a (dichlorophenoxy)methyl group, a (methylphenoxy)methyl group, a (trifluoromethylphenoxy)methyl group, a (methoxyphenoxy)methyl group, a (nitrophenoxy)methyl group, a (dinitrophenoxy)methyl group, a (cyanophenoxy)methyl group, a 2-(phenoxy)ethyl group, a 3-pyridyloxymethyl group, a 3-isooxazolyloxymethyl group, a 5-methyl-3-isooxazolyloxymethyl group, an isovaleryloxymethyl group, a pivaloyloxymethyl group, a (C₅-C₇ cycloalkylcarbonyloxy)methyl group, a (fluorobenzoyl)oxymethyl group, a (chlorobenzoyl)oxymethyl group, a (difluorobenzoyl)oxymethyl group, a (methylbenzoyl)oxymethyl group, a (methoxybenzoyl)oxymethyl group, a (cyanobenzoyl)oxymethyl group, a 4-pyridylcarbonyloxymethyl group, a 2-thienylcarbonyloxymethyl group, a carbamoyloxymethyl group, a cyanomethylthiomethyl group, a phenylthiomethyl group, a (3-chloro-2-pyridyl)thiomethyl group, a (4-methyl-pyrimidin-2-yl)thiomethyl group, an imidazol-2-ylthiomethyl group, a 4-pyridylthiomethyl group, a benzimidazol-2-ylthiomethyl group, a 1H-imidazo[4,5-b]pyridin-2-ylthiomethyl group, a 2-pyrimidinylthiomethyl group, an aminomethyl group wherein the amino part is substituted (substituent(s) being methoxyiminomethylcarbonyl group or methoxycarbonyl group wherein the methyl part is cyano-substituted) or an anilinomethyl group; most preferably a methyl group, a 4-chloro-1-pyrazolylmethyl group, a 4-bromo-1-pyrazolylmethyl group, a hydroxymethyl group, a methoxymethyl group, a 3-pyridylmethoxymethyl group, a cyanomethoxymethyl group, a phenoxymethyl group, a (4-fluorophenoxy)methyl group, a (4-chlorophenoxy)methyl group, a 3-isooxazolyloxymethyl group, a 5-methyl-3-isooxazolyloxymethyl group, a carbamoyloxymethyl group, a cyanomethylthiomethyl group or a 2-pyrimidinylthiomethyl group.

In the present invention, the "N-aralkyl-N-(5-6 membered unsaturated heterocyclyl)aminocarbonyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" is preferably an aminocarbonyl group substituted with one aralkyl group and one 4-pyrazolyl group wherein two same or different straight or branched alkyl groups having a carbon number of 1-4 are substituted at the 1- and 5-positions, more preferably an N-benzyl-N-(4-pyrazolyl)aminocarbonyl group.

In the present invention, the "optionally substituted N-aralkyl-N-(5-6 membered unsaturated heterocyclyl)aminocarbonyl group (substituent(s) being selected from the substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" is the above "N-aralkyl-N-(5-6 membered unsaturated heterocyclyl)aminocarbonyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s))" optionally substituted with 1-3 same or different substituent(s) selected from the substituent group b, and is preferably an aminocarbonyl group substituted with one cyano-aralkyl group and one 4-pyrazolyl group wherein two same or different straight or branched alkyl groups having a carbon number of 1-4 are substituted at the 1- and 5-positions, more preferably an N-(3-cyanobenzyl)-N-(1-isobutyl-5-methyl-4-pyrazolyl)aminocarbonyl group.

In the present invention, the "optionally substituted C₂-C₆ alkenyl group (substitutent(s) being selected from the substituent group n)" is the above "C₂-C₆ alkenyl group" optionally substituted with one substituent selected from the substituent group n, and is preferably a C₃-C₅ alkenyl group, a phenyl(C₃-C₅ alkenyl) group or an N-(cyano-aralkyi)-N-{1,5-di(C₁-C₄ alkyl)-4-pyrazolyl}aminocarbonyl(C₂-C₆ alkenyl) group, more preferably a C₃-C₅ alkenyl group or an N-(3-cyanobenzyl)-N-(1-isobutyl-5-methyl-4-pyrazolyl)aminocarbonyl(C₂-C₆ alkenyl) group, more preferably a C₃-C₅ alkenyl group. In the present invention, the "C₃-C₆ cycloalkoxy group" can be a cyclopropoxy group, a cyclobutoxy group, a cyclopentoxy group or a cyclohexyloxy group, and is preferably a cyclobutoxy group or a cyclopentyloxy group.

In the present invention, the "C₂-C₆ alkenylthio group" can be, for example, a vinylthio group, a 1-propenylthio group, an allylthio group, a 2-methylallylthio group, a 1-butenylthio group, a 2-butenylthio group, a 2-methyl-2-butenylthio group, a 3-butenylthio group, a 1-pentenylthio group, a 1-isopentenylthio group, a 2-pentenylthio group, a 1-hexenylthio group or a 2-hexenylthio group, and is preferably a straight or branched alkenylthio group having a carbon number of 2-5, more suitably a straight or branched alkenylthio group having a carbon number of 3-5, further more preferably an allylthio group or a 2-methyl-2-butenylthio group.

In the present invention, the "o-phenylene group (one methylene group may be inserted between the phenylene group and the carbon atom to which B binds)" is an o-phenylene group wherein one methylene group may be inserted between the phenylene group and the carbon atom to which B binds, and is preferably an o-phenylene group wherein one methylene group is inserted between the phenylene group and the carbon atom to which B binds.

In the present invention, the "optionally substituted o-phenylene group (substituent(s) being selected from the substituent group b; one methylene group may be inserted between the phenylene group and the carbon atom to which B binds)" is the above "o-phenylene group (one methylene group may be inserted between the phenylene group and the carbon atom to which B binds)" optionally substituted with 1-3 same or different substituent(s) selected from the substituent group b, and, for example, can be 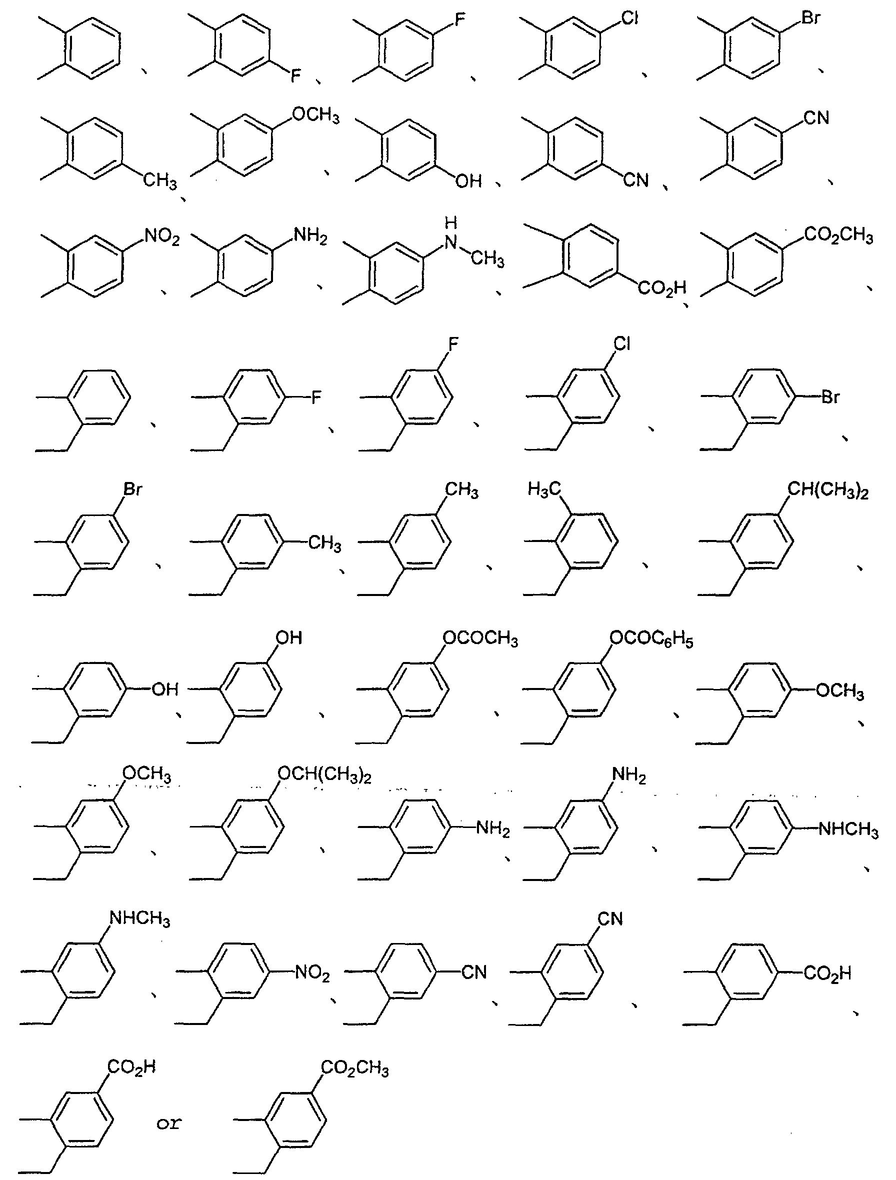 and is preferably an o-phenylene group wherein the phenyl part may be substituted with one substituent selected from the group consisting of a fluorine atom, a chlorine atom, a bromine atom, a hydroxyl group, a cyano group, a nitro group and an amino group, and one methylene group may be inserted between the phenylene group and the carbon atom to which B binds, more preferably an o-phenylene group optionally substituted with one substituent selected from the group consisting of a bromine atom, a cyano group and a nitro group, and one methylene group is inserted between the phenylene group and the carbon atom to which B binds.
(1) In the present invention, R¹ can be, for example, a hydrogen atom, a methyl group, an ethyl group, a propyl group, an isopropyl group, a butyl group, an isobutyl group, a s-butyl group, a t-butyl group, a pentyl group, a s-pentyl group, a t-pentyl group, a neopentyl group, a hexyl group, a heptyl group, an octyl group, a nonyl group, a decyl group, an undecyl group, a dodecyl group, a tridecyl group, a tetradecyl group, a pentadecyl group, a hexadecyl group, a cyclopropyl group, a cyclobutyl group, a cyclopentyl group, a cyclohexyl group, a cycloheptyl group, a cyclooctyl group, a chloromethyl group, a 2-fluoro-2-iodoethylene group, an allyl group, a 2-butenyl group, a 3-methyl-2-butenyl group, a methallyl group, a 2-methyl-2-butenyl group, a 2,3-dimethyl-2-butenyl group, a 3-cyclobutenyl group, a 3-cyclopentenyl group, a 3-cyclohexenyl group, a 3-cyclohexen-1-yl group, a propargyl group, a 2-butynyl group, a 1-pyrazolylmethyl group, a 2-(1-pyrazolyl)ethyl group, a 3-methylpyrazol-1-ylmethyl group, a 5-methylpyrazol-1-ylmethyl group, a 3,5-dimethylpyrazol-1-ylmethyl group, a 4-fluoropyrazol-1-ylmethyl group, a 4-chloropyrazol-1-ylmethyl group, a 2-(4-chloropyrazol-1-yl)ethyl group, a 4-bromopyrazol-1-ylmethyl group, a 2-(4-bromopyrazol-1-yl)ethyl group, a 4-nitropyrazol-1-ylmethyl group, a 4-cyanopyrazol-1-ylmethyl group, a 4-carboxylpyrazol-1-ylmethyl group, a 4-methoxycarbonylpyrazol-1-ylmethyl group, a 4-ethoxycarbonylpyrazol-1-ylmethyl group, a 4-aminopyrazol-1-ylmethyl group, a 4-acetylaminopyrazol-1-ylmethyl group, a 4-benzoylaminopyrazol-1-ylmethyl group, a 2-oxo-1(2H)-pyridylmethyl group, a 2-(2-oxo-1(2H)-pyridyl)ethyl group, a 2-pyridyl group, a 2-pyridylmethyl group, a 3-pyridyl group, a 3-pyridylmethyl group, a 4-pyridyl group, a 2,6-dichloro-4-pyridyl group, a 5-chloro-2-thienylmethyl group, a 2-benzooxazolylmethyl group, a 2-benzothiazolylmethyl group, a carboxylmethyl group, a methoxycarbonylmethyl group, a 2-methoxycarbonylethyl group, an ethoxycarbonylmethyl group, a phenoxycarbonylmethyl group, a carbamoylmethyl group, a 2-carbamoylethyl group, a methylcarbamoylmethyl group, a phenylcarbamoylmethyl group, an N,N-dimethylcarbamoylmethyl group, a cyanomethyl group, a 2-cyanoethyl group, a 3-cyanopropyl group, a 4-cyanobutyl group, a 5-cyanopentyl group, a cyclopropylmethyl group, a 2-cyclopropylethyl group, a 3-cyclopropylpropyl group, a cyclobutylmethyl group, a 2-cyclobutylethyl group, a cyclopentylmethyl group, a 2-cyclopentylethyl group, a 3-cyclopentylpropyl group, a cyclohexylmethyl group, a 2-norbornylmethyl group, a benzyl group, a phenethyl group, a 3-phenylpropyl group, a 4-phenylbutyl group, a 2-fluorobenzyl group, a 3-fluorobenzyl group, a 4-fluorobenzyl group, a 3-chlorobenzyl group, a 4-bromobenzyl group, a 3-cyanobenzyl group, a 3-cyanophenethyl group, a 3-trifluoromethoxybenzyl group, a hydroxymethyl group, a 2-hydroxyethyl group, a 3-hydroxypropyl group, a 4-hydroxybutyl group, a 5-hydroxypentyl group, a methoxymethyl group, a 2-methoxyethyl group, a 3-methoxpropyl group, a 4-methoxybutyl group, an isopropoxymethyl group, an ethoxymethyl group, a 2-ethoxymethyl group, a 3-ethoxypropyl group, a propoxymethyl group, a butoxymethyl group, a s-butoxymethyl group, a t-butoxymethyl group, an isobutyloxymethyl group, a pentyloxymethyl group, a hexyloxymethyl group, a benzyloxymethyl group, a 2-methoxyethoxymethyl group, a cyanomethoxymethyl group, a 2-(cyanomethoxy)ethyl group, an allyloxymethyl group, a 2-allyloxyethyl group, a 3-allyloxypropyl group, a 2-butenyloxymethyl group, a 3-methyl-2-butenyloxymethyl group, a 2-(3-methyl-2-butenyloxy)ethyl group, a methallyloxymethyl group, a 2-methyl-2-butenyloxymethyl group, a 2,3-dimethyl-2-butenyloxymethyl group, a propargyloxymethyl group, a 2-propargyloxyethyl group, a 2-butynyloxymethyl group, a phenoxymethyl group, a 2-phenoxyethyl group, a 3-phenoxypropyl group, a 4-phenoxybutyl group, a 5-phenoxypentyl group, a 2-fluorophenoxymethyl group, a 3-fluorophenoxymethyl group, a 2-(3-fluorophenoxy)ethyl group, a 4-fluorophenoxymethyl group, a 2-chlorophenoxymethyl group, a 3-chlorophenoxymethyl group, a 4-chlorophenoxymethyl group, a 3-(4-chlorophenoxy)propyl group, a 2-bromophenoxymethyl group, a 3-bromophenoxymethyl group, a 4-bromophenoxymethyl group, a 2-iodophenoxymethyl group, a 3-iodophenoxymethyl group, a 4-iodophenoxymethyl group, a 2-methylphenoxymethyl group, a 3-methylphenoxymethyl group, a 2-(3-methylphenoxy)ethyl group, a 4-methylphenoxymethyl group, a 2-methoxyphenoxymethyl group, a 3-methoxyphenoxymethyl group, a 4-methoxyphenoxymethyl group, a 2-(4-methoxyphenoxy)ethyl group, a 2-trifluoromethylphenoxymethyl group, a 3-trifluoromethylphenoxymethyl group, a 4-trifluoromethylphenoxymethyl group, a 2-nitrophenoxymethyl group, a 3-nitrophenoxymethyl group, a 2-(3-nitrophenoxy)ethyl group, a 4-nitrophenoxymethyl group, a 2-(4-nitrophenoxy)ethyl group, a 3-(nitrophenoxy)propyl group, a 2-cyanophenoxymethyl group, a 3-cyanophenoxymethyl group, a 2-(3-cyanophenoxy)ethyl group, a 4-cyanophenoxymethyl group, a 2-(4-cyanophenoxy)ethyl group, a 2,4-difluorophenoxymethyl group, a 3,5-difluorophenoxymethyl group, a 2,4-dichlorophenoxymethyl group, a 3,5-dichlorophenoxymethyl group, a 2,4-dinitrophenoxymethyl group, a 2-furyloxymethyl group, a 2-(2-furyloxy)ethyl group, a 3-furyloxymethyl group, a 2-thienyloxymethyl group, a 3-thienyloxymethyl group, a 2-(3-thienyloxy)ethyl group, a 2-oxazolyloxymethyl group, a 4-oxazolyloxymethyl group, a 2-thiazolyloxymethyl group, a 4-thiazolyloxymethyl group, a 3-isooxazolyloxymethyl group, a 5-methylisooxazolyl-3-yloxymethyl group, a 2-(5-methylisooxazolyl-3-yloxy)ethyl group, a 5-phenylisooxazolyl-3-yloxymethyl group, a 1,2,3-thiadiazol-5-yloxymethyl group, a 1,2,3-benzothiadiazol-7-yloxymethyl group, a trimethylsilyloxymethyl group, a 2-trimethylsilyloxyethyl group, a triethylsilyloxymethyl group; a t-butyldimethylsilyloxymethyl group, a 2-(t-butyldimethylsilyloxy)ethyl group, a 3-(t-butyldimethylsilyloxy)propyl group, a 4-(t-butyldimethylsilyloxy)butyl group, a triphenylsilyloxymethyl group, an acetoxymethyl group, a 2-acetoxyethyl group, a 3-acetoxypropyl group, a 4-acetoxybutyl group, a propionyloxymethyl group, a 2-propionyloxyethyl group, a 3-propionyloxypropyl group, a propylcarbonyloxymethyl group, a 2-propylcarbonyloxyethyl group, an isopropylcarbonyloxymethyl group, a butylcarbonyloxymethyl group, a s-butylcarbonyloxymethyl group, a t-butylcarbonyloxymethyl group, a pentylcarbonyloxymethyl group, a s-pentylcarbonyloxymethyl group, a t-pentylcarbonyloxymethyl group, a neopentylcarbonyloxymethyl group, a hexylcarbonyloxymethyl group, a heptylcarbonyloxymethyl group, an octylcarbonyloxymethyl group, a nonylcarbonyloxymethyl group, a decylcarbonyloxymethyl group, an undecylcarbonyloxymethyl group, a dodecylcarbonyloxymethyl group, a tridecylcarbonyloxymethyl group, a tetradecylcarbonyloxymethyl group, a pentadecylcarbonyloxymethyl group, a hexadecylcarbonyloxymethyl group, a cyclohexylcarbonyloxymethyl group, a cyclobutylcarbonyloxymethyl group, a cyclopentylcarbonyloxymethyl group, a cyclohexylcarbonyloxymethyl group, a cycloheptylcarbonyloxymethyl group, a cyclooctylcarbonyloxymethyl group, a trifluoroacetoxymethyl group, a 2-trifluoroacetoxyethyl group, a chloroacetoxymethyl group, a dichloroacetoxymethyl group, a trichloroacetoxymethyl group, a bromoacetoxymethyl group, a cyanomethoxyacetoxymethyl group, a 2-(cyanomethoxyacetoxy)ethyl group, a cyano(methylthio)acetoxymethyl group, an acryloyloxymethyl group, a 1-propenylcarbonyloxymethyl group, a 2-methyl-1-propenylcarbonyloxymethyl group, a 2-(2-methyl-1-propenylcarbonyloxy)ethyl group, a 2-methylacryloyloxymethyl group, a 1-methyl-1-propenylcarbonyloxymethyl group, a 1,2-dimethyl-1-propenylcarbonyloxymethyl group, a 2-propenylcarbonyloxymethyl group, a 2-butenylcarbonyloxymethyl group, a 3-methyl-2-butenylcarbonyloxymethyl group, a 2-(3-methyl-2-butenylcarbonyloxy)ethyl group, a 2-methyl-2-propenyloxymethyl group, a 2-methyl-2-butenyloxymethyl group, a 2,3-dimethyl-2-butenyloxymethyl group, a 1-cyclobutenylcarbonyloxymethyl group, a 3-cyclobutenylcarbonyloxymethyl group, a 1-cyclopentenylcarbonyloxymethyl group, a 2-(1-cyclopentenylcarbonyloxy)ethyl group, a 3-cyclopentenylcarbonyloxymethyl group, a 1-cyclohexenylcarbonyloxymethyl group, a 3-cyclohexenylcarbonyloxymethyl group, a 4-cyclohexenylcarbonyloxymethyl group, a propioloyloxymethyl group, a 1-propynylcarbonyloxymethyl group, a 2-propynylcarbonyloxymethyl group, a 2-butynylcarbonyloxymethyl group, a 2-(2-butynylcarbonyloxy)ethyl group, a benzoyloxymethyl group, a 2-benzoyloxyethyl group, a 2-fluorobenzoyloxymethyl group, a 3-fluorobenzoyloxymethyl group, a 2-(3-fluorobenzoyloxy)ethyl group, a 4-fluorobenzoyloxymethyl group, a 2-chlorobenzoyloxymethyl group, a 3-chlorobenzoyloxymethyl group, a 4-chlorobenzoyloxymethyl group, a 2-(4-chlorobenzoyloxy)ethyl group, a 2-bromobenzoyloxymethyl group, a 3-bromobenzoyloxymethyl group, a 4-bromobenzoyloxymethyl group, a 2-iodobenzoyloxymethyl group, a 3-iodobenzoyloxymethyl group, a 4-iodobenzoyloxymethyl group, a 2-methylbenzoyloxymethyl group, a 3-methylbenzoyloxymethyl group, a 2-(3-methylbenzoyloxy)ethyl group, a 4-methylbenzoyloxymethyl group, a 2-methoxybenzoyloxymethyl group, a 3-methoxybenzoyloxymethyl group, a 4-methoxybenzoyloxymethyl group, a 2-(4-methoxybenzoyloxy)ethyl group, a 2-trifluoromethylbenzoyloxymethyl group, a 3-trifluoromethylbenzoyloxymethyl group, a 4-trifluoromethylbenzoyloxymethyl group, a 2-nitrobenzoyloxymethyl group, a 3-nitrobenzoyloxymethyl group, a 2-(3-nitrobenzoyloxy)ethyl group, a 4-nitrobenzoyloxymethyl group, a 2-cyanobenzoyloxymethyl group, a 3-cyanobenzoyloxymethyl group, a 4-cyanobenzoyloxymethyl group, a 2-(4-cyanobenzoyloxy)ethyl group, a 2,4-difluorobenzoyloxymethyl group, a 3,5-difluorobenzoyloxymethyl group, a 2,4-dichlorobenzoyloxymethyl group, a 3,5-dichlorobenzoyloxymethyl group, a 2,4-dinitrobenzoyloxymethyl group, a 2-furylcarbonyloxymethyl group, a 2-(2-furylcarbonyloxy)ethyl group, a 3-furylcarbonyloxymethyl group, a 2-thienylcarbonyloxymethyl group, a 3-thienylcarbonyloxymethyl group, a 2-oxazolylcarbonyloxymethyl group, a 4-oxazolylcarbonyloxymethyl group, a 2-thiazolylcarbonyloxymethyl group, a 4-thiazolylcarbonyloxymethyl group, a 3-isooxazolylcarbonyloxymethyl group, a 5-methylisooxazolyl-3-ylcarbonyloxymethyl group, a 2-(5-methylisooxazolyl-3-ylcarbonyloxy)ethyl group, a 5-phenylisooxazolyl-3-ylcarbonyloxymethyl group, a 1,2,3-thiadiazol-5-ylcarbonyloxymethyl group, a 1,2,3-benzothiadiazol-7-ylcarbonyloxymethyl group, a 2-pyridylcarbonyloxymethyl group, a 2-(2-pyridylcarbonyloxy)ethyl group, a 3-pyridylcarbonyloxymethyl group, a 2-(3-pyridylcarbonyloxy)ethyl group, a 4-pyridylcarbonyloxymethyl group, a 2,6-dichloro-4-pyridylcarbonyloxymethyl group, a 2-(2,6-dichloro-4-pyridylcarbonyloxy)ethyl group, a methoxycarbonyloxymethyl group, a 2-methoxycarbonyloxyethyl group, a 3-methoxycarbonyloxyethyl group, an ethoxycarbonyloxymethyl group, an allyloxycarbonyloxymethyl group, a benzyloxycarbonyloxymethyl group, a phenoxycarbonyloxymethyl group, a carbamoyloxymethyl group, a 2-carbamoyloxyethyl group, a 3-carbamoyloxypropyl group, a 4-carbamoyloxybutyl group, a methylcarbamoyloxymethyl group, an ethylcarbamoyloxymethyl group, a phenylcarbamoyloxymethyl group, a chloroacetylcarbamoyloxymethyl group, a trichloroacetylcarbamoyloxymethyl group, a benzoylcarbamoyloxymethyl group, an ethoxycarbonylcarbamoyloxymethyl group, a (2-cyano-2-methoxyiminoacetyl)carbamoyloxymethyl group, a 2-(2-cyano-2-methoxyiminoacetylcarbamoyloxy)ethyl group, a methyl(thiocarbonyl)oxymethyl group, a 2-methyl(thiocarbonyl)oxyethyl group, a 3-methyl(thiocarbonyl)oxypropyl group, an ethyl(thiocarbonyl)oxymethyl group, a phenyl(thiocarbonyl)oxymethyl group, a methyl(thiocarbamoyl)oxymethyl group, a phenyl(thiocarbamoyl)oxymethyl group, a (methylthio)(thiocarbonyl)oxymethyl group, an aminomethyl group, a 2-aminoethyl group, a 3-aminopropyl group, a methylaminomethyl group, an ethylaminomethyl group, a 3,4-dimethoxyphenethylaminomethyl group, a 1-carboxylethylaminomethyl group, a 1-methoxycarbonylethylaminomethyl group, a 1-benzyloxycarbonylethylaminomethyl group, a 1-carboxyl-2-methylpropylaminomethyl group, a 1-methoxycarbonyl-2-methylpropylaminomethyl group, a 1-benzyloxycarbonyl-2-methylpropylaminomethyl group, an anilinomethyl group, an acetylaminomethyl group, a 2-acetylaminoethyl group, a propionylaminomethyl group, a propylcarbonylaminomethyl group, an isopropylcarbonylaminomethyl group, a butylcarbonylaminomethyl group, a s-butylcarbonylaminomethyl group, a t-butylcarbonylaminomethyl group, a pentylcarbonylaminomethyl group, a s-pentylcarbonylaminomethyl group, a t-pentylcarbonylaminomethyl group, a neopentylcarbonylaminomethyl group, a hexylcarbonylaminomethyl group, a heptylcarbonylaminomethyl group, an octylcarbonylaminomethyl group, a nonylcarbonylaminomethyl group, a decylcarbonylaminomethyl group, an undecylcarbonylaminomethyl group, a dodecylcarbonylaminomethyl group, a tridecylcarbonylaminomethyl group, a tetradecylcarbonylaminomethyl group, a pentadecylcarbonylaminomethyl group, a hexadecylcarbonylaminomethyl group, a cyclopropylcarbonylaminomethyl group, a cyclobutylcarbonylaminomethyl group, a cyclopentylcarbonylaminomethyl group, a cyclohexylcarbonylaminomethyl group, a cycloheptylcarbonylaminomethyl group, a cyclooctylcarbonylaminomethyl group, a trifluoroacetylaminomethyl group, a chloroacetylaminomethyl group, a dichloroacetylaminomethyl group, a trichloroacetylaminomethyl group, a bromoacetylaminomethyl group, an acryloylaminomethyl group, a 1-propenylcarbonylaminomethyl group, a 2-methyl-1-propenylcarbonyl)aminomethyl group, a 1-methyl-1-acryloylaminomethyl group, a 1-methyl-1-propenylcarbonylaminomethyl group, a 1,2-dimethyl-1-propenylcarbonylaminomethyl group, a 2-propenylcarbonylaminomethyl group, a 2-butenylcarbonylaminomethyl group, a 3-methyl-2-butenylcarbonylaminomethyl group, a 2-methyl-2-propenylcarbonylaminomethyl group, a 2,3-dimethyl-2-butenylcarbonylaminomethyl group, a 1-cyclobutenylcarbonylaminomethyl group, a 3-cyclobutenylcarbonylaminomethyl group, a 1-cyclopentenylcarbonylaminomethyl group, a 2-cyclopenten-1-ylcarbonylaminomethyl group, a 1-cyclohexenylcarbonylaminomethyl group, a 3-cyclohexenylcarbonylaminomethyl group, a 4-cyclohexenylcarbonylaminomethyl group, a propioloylaminomethyl group, a 1-propynylcarbonylaminomethyl group, a 2-propynylcarbonylaminomethyl group, a 2-butynylcarbonylaminomethyl group, a 2-(2-butynylcarbonylamino)ethyl group, a benzoylaminomethyl group, a 2-benzoylaminoethyl group, a 3-benzoylaminopropyl group, a 2-fluorobenzoylaminomethyl group, a 3-fluorobenzoylaminomethyl group, a 2-(3-fluorobenzoylamino)ethyl group, a 4-fluorobenzoylamino group, a 2-chlorobenzoylaminomethyl group, a 3-chlorobenzoylaminomethyl group, a 4-chlorobenzoylaminomethyl group, a 2-bromobenzoylaminomethyl group, a 3-bromobenzoylaminomethyl group, a 4-bromobenzoylaminomethyl group, a 2-iodobenzoylaminomethyl group, a 3-iodobenzoylaminomethyl group, a 4-iodobenzoylaminomethyl group, a 2-methylbenzoylaminomethyl group, a 3-methylbenzoylaminomethyl group, a 4-methylbenzoylaminomethyl group, a 2-methoxybenzoylaminomethyl group, a 3-methoxybenzoylaminomethyl group, a 4-methoxybenzoylaminomethyl group, a 2-(4-methoxybenzoylamino)ethyl group, a 2-trifluoromethylbenzoylaminomethyl group, a 3-trifluoromethylbenzoylaminomethyl group, a 4-trifluoromethylbenzoylaminomethyl group, a 2-nitrobenzoylaminomethyl group, a 3-nitrobenzoylaminomethyl group, a 4-nitrobenzoylaminomethyl group, a 2-(4-nitrobenzoylamino)ethyl group, a 2-cyanobenzoylaminomethyl group, a 3-cyanobenzoylaminomethyl group, a 2-(3-cyanobenzoylamino)ethyl group, a 4-cyanobenzoylaminomethyl group, a 2,4-difluorobenzoylaminomethyl group, a 3,5-difluorobenzoylaminomethyl group, a 2,4-dichlorobenzoylaminomethyl group, a 3,5-dichlorobenzoylaminomethyl group, a 2,4-dinitrobenzoylaminomethyl group, a 2-furylcarbonylaminomethyl group, a 2-(2-furylcarbonylamino)ethyl group, a 3-furylcarbonylaminomethyl group, a 2-thienylcarbonylaminomethyl group, a 3-thienylcarbonylaminomethyl group, a 2-oxazolylcarbonylaminomethyl group, a 4-oxazolylcarbonylaminomethyl group, a 2-thiazolylcarbonylaminomethyl group, a 4-thiazolylcarbonylaminomethyl group, a 2-isooxazolylcarbonylaminomethyl group, a 5-methylisooxazol-3-ylcarbonylaminomethyl group, a 2-(5-methyl-3-isooxazolylcarbonylamino)ethyl group, a 5-phenyl-3-isooxazolylcarbonylaminomethyl group, a 1,2,3-thiadiazol-5-ylcarbonylaminomethyl group, a 1,2,3-benzothiadiazol-7-ylcarbonylaminomethyl group, a 2-pyridylcarbonylaminomethyl group, a 3-pyridylcarbonylaminomethyl group, a 2-(3-pyridylcarbonylamino)ethyl group, a 4-pyridylcarbonylaminomethyl group, a 2,6-dichloro-4-pyridylcarbonylaminomethyl group, a 2-(2,6-dichloro-4-pyridylcarbonylamino)ethyl group, a methoxycarbonylaminomethyl group, a 2-(methoxycarbonylamino)ethyl group, a 3-(methoxycarbonylamino)propyl group, an ethoxycarbonylaminomethyl group, an allyloxycarbonylaminomethyl group, a benzyloxycarbonylaminomethyl group, a phenoxycarbonylaminomethyl group, a ureidomethyl group, a 2-ureidoethyl group, a 3-ureidopropyl group, a 3-methylureidomethyl group, a 3-ethylureidomethyl group, a 3-phenylureidomethyl group, a 3-(chloroacetyl)ureidomethyl group, a 3-(trichloroacetyl)ureidomethyl group, a 3-benzoylureidomethyl group, a 3-ethoxycarbonylureidomethyl group, a 3-(2-cyano-2-methoxyiminoacetyl)ureidomethyl group, a 2-{3-(2-cyano-2-methoxyiminoacetyl)ureido}ethyl group, a methyl(thiocarbonyl)aminomethyl group, a 2-{methyl(thiocarbonyl)amino}ethyl group, an ethyl(thiocarbonyl)aminomethyl group, a phenyl(thiocarbonyl)aminomethyl group, a 3-methyl(thioureido)methyl group, a 3-phenyl(thioureido)methyl group, a (methylthio)(thiocarbonyl)aminomethyl group, a 2-[(methylthio)(thiocarbonyl)amino]ethyl group, an aminooxymethyl group, an acetylaminooxymethyl group, a propionylaminooxymethyl group, a cyclopropylcarbonylaminooxymethyl group, a benzoylaminooxymethyl group, a 2-cyano-2-methoxyiminoacetylaminooxymethyl group, a methoxycarbonylaminooxymethyl group, an allyloxycarbonylaminooxymethyl group, a ureidooxymethyl group, a 3-methylureidooxymethyl group, a 3,3-dimethylureidooxymethyl group, a thioacetylaminooxymethyl group, a 3-methyl(thioureido)oxymethyl group, a 3-acetylureidooxymethyl group, a 3-(chloroacetyl)ureidooxymethyl group, a 3-(trichloroacetyl)ureidooxymethyl group, a 3-(2-cyano-2-methoxyiminoacetyl)ureidooxymethyl group, an ethylideneiminooxymethyl group, an isopropylideneiminooxymethyl group, a 1-cyclopropylethylideneiminooxymethyl group, an α-methylbenzylideneiminooxymethyl group, an α-methyl-2-trifluoromethylbenzylideneiminooxymethyl group, an α-methyl-3-trifluoromethylbenzylideneiminooxymethyl group, a 1α-methyl-4-trifluoromethylbenzylideneiminooxymethyl group, an α-methyl-2-methoxybenzylideneiminooxymethyl group, an α-methyl-3-methoxybenzylideneiminooxymethyl group, an α-methyl-4-methoxybenzylideneiminooxymethyl group, a 1-(5-methyl-2-furyl)ethylideneiminooxymethyl group, a 1-methoxyethylideneiminooxymethyl group, a 1-methoxycarbonylbenzylideneiminooxycarbonyl group, a cyclopentylideneiminooxymethyl group, a tetrahydropyran-4-ylidineiminooxymethyl group, a methylthiomethyl group, a 2-methylthioethyl group, an ethylthiomethyl group, a propylthiomethyl group, an isopropylthiomethyl group, a butylthiomethyl group, an isobutylthiomethyl group, a s-butylthiomethyl group, a t-butylthiomethyl group, a pentylthiomethyl group, a cyclopentylthiomethyl group, a hexylthiomethyl group, a cyclohexylthiomethyl group, a benzylthiomethyl group, a phenylthiomethyl group, a cyanomethylthiomethyl group, a 2-pyridylthiomethyl group, a 2-pyrimidylthiomethyl group, a methylsulfinylmethyl group, a 2-methylsulfinylethyl group, an ethylsulfinylmethyl group, a propylsulfinylmethyl group, an isopropylsulfinylmethyl group, a butylsulfinylmethyl group, an isobutylsulfinylmethyl group, a s-butylsulfinylmethyl group, a t-butylsulfinylmethyl group, a pentylsulfinylmethyl group, a cyclopentylsulfinylmethyl group, a hexylsulfinylmethyl group, a cyclohexylsulfinylmethyl group, a benzylsulfinylmethyl group, a phenylsulfinylmethyl group, a cyanomethylsulfinylmethyl group, a 2-pyridylsulfinylmethyl group, a 2-pyrimidylsulfinylmethyl group, a methylsulfonylmethyl group, a 2-methylsulfonylethyl group, an ethylsulfonylmethyl group, a propylsulfonylmethyl group, an isopropylsulfonylmethyl group, a butylsulfonylmethyl group, an isobutylsulfonylmethyl group, a s-butylsulfonylmethyl group, a t-butylsulfonylmethyl group, a pentylsulfonylmethyl group, a cyclopentylsulfonylmethyl group, a hexylsulfonylmethyl group, a cyclohexylsulfonylmethyl group, a benzylsulfonylmethyl group, a phenylsulfonylmethyl group, a cyanomethylsulfonylmethyl group, a 2-pyridylsulfonylmethyl group, a 2-pyrimidylsulfonylmethyl group, a trimethylsilylmethyl group, a 2-trimethylsilylethyl group, a triethylsilylmethyl group, a 2-tetrahydropyranyloxymethyl group, a 2-tetrahydropyranyl group, a 3-methyloxetan-3-ylmethyl group, 2-tetrahydrofuranylmethyl group, a 2-tetrahydropyranylmethyl group, a morpholinomethyl group, a 3,5-difluorophenethyl group, a 2-fluoroethyl group, a 2-chloroethyl group, a pentafluoroethyl group, a 2,2,2-trifluoroethyl group, a 2-aminoethyl group, a 2-acetylaminoethyl group, a 2-(2-tetrahydropyranyloxy)ethyl group, a 2-chlorophenethyl group, a 3-fluorophenethyl group, a 4-nitrophenethyl group, a 3,5-difluorophenethyl group, a 2,2-dimethoxyethyl group, a 2-(2-tetrahydrofuranyl)ethyl group, a 2-(2-tetrahydropyranyl)ethyl group, a 2-morpholinoethyl group, a 1,3-dioxolan-2-ylmethyl group, a 1,3-dioxan-2-ylmethyl group, a 5,5-dimethyl-1,3-dioxan-2-ylmethyl group, a 1,3-dithiolan-2-ylmethyl group, a 1,3-dithian-2-ylmethyl group, a 2-nitroiminoethyl group, a 2-cyanoiminoethyl group, a 2-trifluoromethyliminoethyl group, a 3-nitroiminopropyl group, a 3-cyanoiminopropyl group, a 3-trifluoromethyliminopropyl group, a 2-nitroethyl group, a 2,2-dicyanoethyl group, a 2,2-dinitroethyl group, a 2-cyano-2-methyliminoethyl group, a 2-cyano-2-methoxyiminoethyl group, a 2-nitro-2-methyliminoethyl group, a 3,3,3-trifluoropropyl group, a 2,2,3,3,3-pentafluoropropyl group, a heptafluoropropyl group, an acetylmethyl group, a 2-hydroxypropyl group, a 2-methoxypropyl group, a 2-acetoxypropyl group, a 2-trimethylsilyloxypropyl group, a 2-fluoropropyl group, a 2-chloropropyl group, a 2-hydroxyisobutyl group, a 2-methoxyisobutyl group, a 2-acetoxyisobutyl group, a 2-trimethylsilyloxyisobutyl group, a 2-fluoroisobutyl group, a 2-chloroisobutyl group, a 2,2,2-trifluoroisopropyl group, a 2,2,2,2',2',2'-hexafluoroisopropyl group, a 2-hydroxyiminopropyl group, a 2-acetoxyiminopropyl group, a 2-methoxyiminopropyl group, a 2-benzyloxyiminopropyl group, a 2-methyliminopropyl group, a 2-methylhydrazonopropyl group, a pivaloylmethyl group, a 2-fluorobenzoylmethyl group, a 4-fluorobenzoylmethyl group, a 4-fluorobutyl group, a 4,4,4-trifluorobutyl group, a 3,3,4,4-tetrafluorobutyl group, a 3,3,4,4,4-pentafluorobutyl group, a 2,2,3,3,4,4,4-heptafluorobutyl group, a nonafluorobutyl group, a propionylmethyl group, a 2-hydroxybutyl group, a 1-acetylethyl group, a 2-fluoroisopropyl group, a 3,3,3-trifluoroisobutyl group, a 3,3,3,3',3',3'-hexafluoroisobutyl group, a 3,3,3',3'-tetrafluoroisobutyl group, a 3,3'-difluoroisobutyl group, a 3,3,3-trifluoro-t-butyl group, a 3,3,3,3',3',3'-hexafluoro-t-butyl group, a 3,3,3',3'-tetrafluoro-t-butyl group, a 3,3'-difluoro-t-butyl group, a 2,2-dimethylpropyl group, a 2-cyanopropyl group, a 2-nitropropyl group, a 2,2-dicyanopropyl group, a 2,2-dinitropropyl group, a 2-cyanoisobutyl group, a 2-nitroisobutyl group, a 3-fluoro-t-butyl group, a 3-hydroxy-t-butyl group, a 3-methoxy-t-butyl group, a 3,3'-dihydroxyisobutyl group, a 3,3'-dimethoxyisobutyl group, a 3,3'-diacetoxyisobutyl group, a 1,3-dioxan-5-ylmethyl group, a 2,2-dimethyl-1,3-dioxan-5-ylmethyl group, a 3,3'-dihydroxy-t-butyl group, a 3,3'-dimethoxy-t-butyl group, a 5-methyl-1,3-dioxan-5-ylmethyl group, a 2,2,5-trimethyl-1,3-dioxan-5-ylmethyl group, a 3,3'-di(methylthio)isobutyl group, a 1,3-dithian-5-ylmethyl group, a 2,2-dimethyl-1,3-dithian-5-ylmethyl group, a 3,3'-di(methylthio)-t-butyl group, a 5-methyl-1,3-dithian-5-ylmethyl group, a 2,2,5-trimethyl-1,3-dithian-5-ylmethyl group, a 3,3',3"-trifluoro-t-butyl group, a 3,3',3''-trihydroxy-t-butyl group, a 3,3',3"-trimethoxy-t-butyl group, a 2-methylbutyl group, a 2-ethylbutyl group, a t-hexyl group, a 2-isohexyl group, a 3-pentyl group, a 2-hydroxy-1-methoxycarbonylvinyl group, a 2-methoxy-1-methoxycarbonylvinyl group, a 1-hydroxyimino-1-methoxycarbonylmethyl group, a 1-methoxyimino-1-methoxycarbonylmethyl group, an acetyl group, a propionyl group, a benzoyl group, a 2-fluorobenzoyl group, a 3-fluorobenzoyl group, a 4-fluorobenzoyl group, a 3-cyanobenzoyl group, a methoxycarbonyl group, an ethoxycarbonyl group, an allyloxycarbonyl group, a t-butoxycarbonyl group, a benzyloxycarbonyl group, a phenylsulfonyl group, a tolylsulfonyl group, a dimethylsulfamoyl group, a phenyl group, a 2-fluorophenyl group, a 3-fluorophenyl group, a 4-fluorophenyl group, a 2-chlorophenyl group, a 3-chlorophenyl group, a 4-chlorophenyl group, a 2-bromophenyl group, a 3-bromophenyl group, a 4-bromophenyl group, a 3-iodophenyl group, a 4-iodophenyl group, a 2-methylphenyl group, a 3-methylphenyl group, a 4-methylphenyl group, a 2-ethylphenyl group, a 3-ethylphenyl group, a 4-ethylphenyl group, a 2-propylphenyl group, a 3-isopropylphenyl group, a 4-butylphenyl group, a 2-s-butylphenyl group, a 3-t-butylphenyl group, a 4-neopentylphenyl group, a 3-t-pentylphenyl group, a 4-hexylphenyl group, a 2-fluoromethylphenyl group, a 3-fluoromethylphenyl group, a 4-fluoromethylphenyl group, a 2-difluoromethylphenyl group, a 3-difluoromethylphenyl group, a 4-difluoromethylphenyl group, a 2-trifluoromethylphenyl group, a 3-trifluoromethylphenyl group, a 4-trifluoromethylphenyl group, a 2-chloromethylphenyl group, a 3-chloromethylphenyl group, a 4-chloromethylphenyl group, a 3-dichloromethylphenyl group, a 4-trichloromethylphenyl group, a 2-bromomethylphenyl group, a 3-bromomethylphenyl group, a 4-bromomethylphenyl group, a 3-dibromomethylphenyl group, a 4-dibromomethylphenyl group, a 3-iodomethylphenyl group, a 4-iodomethylphenyl group, a 3-cyclopropylphenyl group, a 4-cyclopropylphenyl group, a 2-cyclobutylphenyl group, a 3-cyclopentylphenyl group, a 4-cyclohexylphenyl group, a 2-(1-chlorocyclopropyl)phenyl group, a 3-(2,2-dichlorocyclopropyl)phenyl group, a 4-(2,2-dimethylcyclopropyl)phenyl group, a 2-vinylphenyl group, a 3-(1-propenyl)phenyl group, a 4-allylphenyl group, a 2-(1-methylvinyl)phenyl group, a 3-(2-methyl-1-propenyl)phenyl group, a 4-(1-pentyl)pheny group, a 3-(2-phenylvinyl)phenyl group, a 2-ethynylphenyl group, a 3-(1-propynyl)phenyl group, a 4-propargylphenyl group, a 4-phenylphenyl group, a 3-(2-fluorophenyl)phenyl group, a 4-(3-fluorophenyl)phenyl group, a 2-(4-fluorophenyl)phenyl group, a 4-(4-chlorophenyl)phenyl group, a 3-(4-nitrophenyl)phenyl group, a 4-(4-tolyl)phenyl group, a 3-(4-methoxyphenyl)phenyl group, a 2-acetylphenyl group, a 3-acetylphenyl group, a 4-acetylphenyl group, a 3-propionylphenyl group, a 4-propionylphenyl group, a 3-benzoylphenyl group, a 3-(2-fluorobenzoyl)phenyl group, a 3-(3-fluorobenzoyl)phenyl group, a 4-(4-fluorobenzoyl)phenyl group, a 4-(4-methylbenzoyl)phenyl group, a 3-(4-methoxybenzoyl)phenyl group, a 3-(1-amino-1-methoxyiminomethyl)phenyl group, a 3-(1-methyl-1-methoxyiminomethyl)phenyl group, a 3-(α-methoxyiminobenzyl)phenyl group, a 3-(1-methylimino-1-methylthiomethyl)phenyl group, a 3-(1-ethylthio-1-methyliminomethyl)phenyl group, a 4-(1-ethylthio-1-methyliminomethyl)phenyl group, a 3-(1-propylimino-1-methylthiomethyl)phenyl group, a 3-(1-propylthio-1-methyliminomethyl)phenyl group, a 3-(1-benzylthio-1-methyliminomethyl)phenyl group, a 3-{1-(3-cyanophenylthio)-1-methyliminomethyl}phenyl group, a 2-cyanophenyl group, a 3-cyanophenyl group, a 4-cyanophenyl group, a 2-carboxylphenyl group, a 3-carboxylphenyl group, a 4-carboxylphenyl group, a 2-methoxycarbonylphenyl group, a 3-methoxycarbonylphenyl group, a 3-ethoxycarbonylphenyl group, a 4-ethoxycarbonylphenyl group, a 3-propoxycarbonylphenyl group, a 3-isopropoxycarbonylphenyl group, a 2-carbamoylphenyl group, a 3-carbamoylphenyl group, a 4-carbamoylphenyl group, a 2-(methylcarbamoyl)phenyl group, a 3-(methylcarbamoyl)phenyl group, a 4-(methylcarbamoyl)phenyl group, a 3-(dimethylcarbamoyl)phenyl group, a 4-(dimethylcarbamoyl)phenyl group, a 3-(phenylcarbamoyl)phenyl group, a 3-(4-methoxyphenyl-carbamoyl)phenyl group, a 3-(propionylcarbamoyl)phenyl group, a 3-thiocarbamoylphenyl group, a 4-thiocarbamoylphenyl group, a 2-hydroxyphenyl group, a 3-hydroxyphenyl group, a 4-hydroxyphenyl group, a 2-methoxyphenyl group, a 3-methoxyphenyl group, a 4-methoxyphenyl group, a 3-ethoxyphenyl group, a 4-ethoxyphenyl group, a 3-propoxyphenyl group, a 4-propoxyphenyl group, a 3-isopropoxyphenyl group, a 4-butoxyphenyl group, a 3-s-butoxyphenyl group, a 3-t-butoxyphenyl group, a 4-isobutoxyphenyl group, a 3-fluoromethoxyphenyl group, a 3-chloromethoxyphenyl group, a 3-difluoromethoxyphenyl group, a 3-trifluoromethoxyphenyl group, a 4-trifluoromethoxyphenyl group, a 3-(2,2,2-trifluoroethoxy)phenyl group, a 3-(pentafluoroethoxy)phenyl group, a 3-(2-fluoro-2-iodovinyloxy)phenyl group, a 2-acetoxyphenyl group, a 3-acetoxyphenyl group, a 4-acetoxyphenyl group, a 3-propionyloxyphenyl group, a 3-benzoyloxyphenyl group, a 2-methoxycarbonyloxyphenyl group, a 3-methoxycarbonyloxyphenyl group, a 4-methoxycarbonyloxyphenyl group, a 3-ethoxycarbonyloxyphenyl group, a 3-phenoxycarbonyloxyphenyl group, a 2-aminophenyl group, a 3-aminophenyl group, a 4-aminophenyl group, a 2-methylaminophenyl group, a 3-methylaminophenyl group, a 4-methylaminophenyl group, a 3-dimethylaminophenyl group, a 3-benzylaminophenyl group, a 3-phenylaminophenyl group, a 3-formylaminophenyl group, a 2-acetylaminophenyl group, a 3-acetylaminophenyl group, a 4-acetylaminophenyl group, a 3-benzoylaminophenyl group, a 3-(N-acetyl-N-methylamino)phenyl group, a 2-methoxycarbonylaminophenyl group, a 3-ethoxycarbonylaminophenyl group, a 4-phenoxycarbonylaminophenyl group, a 3-(N-methyl-N-methoxycarbonylamino)phenyl group, a 2-(3-methylureido)phenyl group, a 3-(3-ethylureido)phenyl group, a 4-(3-phenylureido)phenyl group, a 3-{3-(2-cyano-2-methoxyiminoacetyl)ureido}phenyl group, a 3-(N-methyl-N-methylcarbamoylamino)phenyl group, a 2-thioacetylaminophenyl group, a 3-thioacetylaminophenyl group, a 4-thioacetylaminophenyl group, a 2-thioacetylaminophenyl group, a 2-(3-methyl(thioureido)}phenyl group, a 3-{3-ethyl(thioureido)}phenyl group, a 4-{3-phenyl(thioureido)}phenyl group, a 2-isocyanophenyl group, a 3-isocyanophenyl group, a 4-isocyanophenyl group, a 2-nitrophenyl group, a 3-nitrophenyl group, a 4-nitrophenyl group, a 2-mercaptophenyl group, a 3-mercaptophenyl group, a 4-mercaptophenyl group, a 2-methylthiophenyl group, a 3-methylthiophenyl group, a 4-methylthiophenyl group, a 3-ethylthiophenyl group, a 3-phenylthiophenyl group, a 2-methylsulfinylphenyl group, a 3-methylsulfinylphenyl group, a 4-methylsulfinylphenyl group, a 3-phenylsulfinylphenyl group, a 2-methylsulfonylphenyl group, a 3-methylsulfonylphenyl group, a 4-methylsulfonylphenyl group, a 3-phenylsulfonylphenyl group, a 2-acetylthiophenyl group, a 3-acetylthiophenyl group, a 4-acetylthiophenyl group, a 3-benzoylthiophenyl group, a 2-[(methylthio)(thiocarbonyl)thio]phenyl group, a 3-[(methylthio)(thiocarbonyl)thio]phenyl group, a 2-trimethylsilylphenyl group, a 3-trimethylsilylphenyl group, a 4-trimethylsilylphenyl group, a 3-(2-thienyl)phenyl group, a 4-(2-thienyl)phenyl group, a 3-(1,2,4-oxadiazol-3-yl)phenyl group, a 4-(1,2,4-oxadiazol-3-yl)phenyl group, a 3-(5-methyl-1,2,4-oxazol-3-yl)phenyl group, a 4-(5-methyl-1,2,4-oxazol-3-yl)phenyl group, a 3-(5-trifluoromethyl-1,2,4-oxazol-3-yl)phenyl group, a 2,3-difluorophenyl group, a 2,4-difluorophenyl group, a 2,5-difluorophenyl group, a 3,5-difluorophenyl group, a 2,4-dichlorophenyl group, a 3,5-dichlorophenyl group, a 2-fluoro-5-bromophenyl group, a 5-fluoro-3-bromophenyl group, a 3-fluoro-4-bromophenyl group, a 3-bromo-4-methoxyphenyl group, a 2,4-dimethylphenyl group, a 3,5-dimethylphenyl group, a 2,4-di(trifluoromethyl)phenyl group, a 3,5-di(trifluoromethyl)phenyl group, a 2,4-dimethoxyphenyl group, a 3,5-dimethoxyphenyl group, a 2,4-dinitrophenyl group, a 2,6-dinitrophenyl group, a 2,4-dicyanophenyl group, a 3,4-dicyanophenyl group, a 2,6-dicyanophenyl group, a 4-amino-4-fluorophenyl group, a 4-nitro-2-fluorophenyl group, a 4-nitro-2-methylsulfonyl *group,* a 2-nitro-4-phenylsulfonyl group, a 4-cyano-2-fluorophenyl group, a 2-cyano-4-methylsulfonyl group, a 4-cyano-2-phenylsulfonyl group, a 3-nitro-5-fluorophenyl group, a 4-azido-3-nitrophenyl group, a 4-ethoxy-3-nitrophenyl group, a 3-nitro-4-methylaminophenyl group, a 3-nitro-4-phenethylaminophenyl group, a 3-nitro-4-methylthiophenyl group, a 3-nitro-4-phenylthiophenyl group, a 3-nitro-4-methylsulfinylphenyl group, a 3-nitro-4-phenylsulfinylphenyl group, a 3-nitro-4-methylsulfonyl group, a 3-nitro-4-phenylsulfonyl group, a 3-cyano-5-fluorophenyl group, a 3-cyano-4-methoxyphenyl group, a 4-ethoxy-3-cyanophenyl group, a 3-cyano-4-phenoxyphenyl group, a 3-cyano-4-(4-tolyloxy)phenyl group, a 3-cyano-4-methylsulfonylphenyl group, a 3-cyano-4-phenylsulfonylphenyl group, a 2,6-dichloro-4-trifluoromethylphenyl group, a 2,3,4,6-tetrafluorophenyl group, a pentafluorophenyl group, a 1-naphthyl group, a 3-fluoro-1-naphthyl group, a 4-cyano-1-naphthyl group, a 5-nitro-1-naphthyl group, a 2-naphthyl group, a 4-chloro-2-naphthyl group, a 5-cyano-2-naphthyl group, a 1-anthryl group, a 2-anthryl group, a 9-anthryl group, a ferrocenyl group, a 2-furyl group, a 5-methyl-2-furyl group, a 4-methoxycarbonyl-2-furyl group, a 4-cyano-2-furyl group, a 5-cyano-2-furyl group, a 3-furyl group, a 4-cyano-3-furyl group, a 2-cyano-3-furyl group, a 5-cyano-3-furyl group, a 2-thienyl group, a 4-nitro-2-thienyl group, a 4-cyano-2-thienyl group, a 5-cyano-2-thienyl group, a 5-methoxycarbonyl-2-thienyl group, a 4-methyl-2-thienyl group, a 3-thienyl group, a 2-methyl-3-thienyl group, a 5-nitro-3-thienyl group, a 2-cyano-3-thienyl group, a 5-cyano-3-thienyl group, a 2-oxazolyl group, a 5-methyl-2-oxazolyl group, a 4-oxazolyl group, a 3-isooxazolyl group, a 5-methyl-3-isooxazolyl group, a 2-thiazolyl group, a 4-methyl-2-thiazolyl group, a 4-thiazolyl group, a 1-methyl-4-pyrazolyl group, a 1-phenyl-4-pyrazolyl group, a 1,3,5-trimethyl-4-pyrazolyl group, a 1,2,4-oxadiazol-3-yl group, a 5-methyl-1,2,4-oxadiazol-3-yl group, a 1,2,4-thiadiazol-3-yl group, a 5-methyl-1,2,4-thiadiazol-3-yl group, a 2-pyridyl group, a 4-trifluoromethyl-2-pyridyl group, a 6-fluoro-2-pyridyl group, a 6-chloro-2-pyridyl group, a 6-trifluoromethyl-2-pyridyl group, a 3-pyridyl group, a 4-pyridyl group, a 2,6-dichloro-4-pyridyl group, a 3-pyridazinyl group, a 4-pyridazinyl group, a 2-pyrimidinyl group, a 4-methyl-2-pyrimidinyl group, a 4,6-dimethyl-2-pyrimidinyl group, a 4,6-dimethoxy-2-pyrimidinyl group, a 4,6-dichloro-2-pyrimidinyl group, a 4-chloro-6-methoxy-2-pyrimidinyl group, a 4-pyrimidinyl group, a 2,6-dimethyl-4-pyrimidinyl group, a 2,6-dichloro-4-pyrimidinyl group, a 1,3,5-triazinyl group, a 4,6-dimethoxy-1,3,5-triazinyl group, a 4,6-dichloro-1,3,5-triazinyl group, a 2-benzooxazolyl group, a 2-benzothiazolyl group, a 4-benzothiazolyl group, a 7-benzothiazolyl group, a 2-quinolyl group or a 3-phthalazyl group; and is preferably a C₁-C₁₀ alkyl group, a halo(C₁-C₈ alkyl) group (the halogen substituent(s) is (are) 1-3 same or different substituent(s) selected from the group consisting of a fluorine atom, a chlorine atom and a bromine atom), a (C₂-C₄ alkylcarbonyl) (C₁-C₃ alkyl) group, a benzoyl (C₁-C₄ alkyl) group wherein the phenyl part may be substituted (substituent(s) being fluorine atom, chlorine atom or bromine atom), a (C₂-C₄ alkoxycarbonyl) (C₁-C₃ alkyl) group, a phenoxycarbonyl(C₁-C₃ alkyl) group, a cyano(C₁-C₃ alkyl) group, a (C₃-C₆ cycloalkyl) (C₁-C₃ alkyl) group wherein the cycloalkyl part may be substituted (substituent(s) being C₁-C₃ alkyl group, fluorine atom or chlorine atom), a norbornyl (C₁-C₃ alkyl) group, a benzyl group wherein the phenyl part may be substituted {substituent(s) being fluorine atom, chlorine atom, bromine atom, cyano group or halo(C₁-C₃ alkoxy) group (the halogen substituent(s) is (are) 1-3 same or different fluorine atom(s), chlorine atom(s) or bromine atom(s))}, a (5-6 membered saturated heterocyclyl)(C₁-C₃ alkyl) group wherein the heterocycle part may be substituted (the substituent(s) is (are) 1-2 same or different C₁-C₃ alkyl group(s), and the heterocycle contains 1-2 same or different heteroatom(s) selected from the group consisting of an oxygen atom and a nitrogen atom), a (5-6 membered unsaturated heterocyclyl)(C₁-C₃ alkyl) group wherein the heterocycle part may be substituted (substituent(s) being fluorine atom, chlorine atom or bromine atom; the heterocycle contains an oxygen atom, a sulfur atom or a nitrogen atom, and may contain one further nitrogen atom, and the heterocycle may be fused with a benzene ring), a hydroxy(C₁-C₄ alkyl) group, a (C₁-C₃ alkoxy) (C₁-C₄ alkyl) group, a (C₁-C₃ alkoxy) (C₁-C₃ alkoxy) (C₁-C₃ alkyl) group, a phenoxy(C₁-C₃ alkyl) group, a (5-6 membered saturated heterocyclyl)oxy(C₁-C₃ alkyl) group (the heterocycle contains 1-2 same or different heteroatom(s) selected from the group consisting of an oxygen atom and a nitrogen atom), a tri(C₁-C₃ alkyl)silyloxy(C₁-C₄ alkyl) group, a (C₂-C₄ alkylcarbonyloxy) (C₁-C₄ alkyl) group (the alkylcarbonyloxy substituent(s) is (are) 1-2 same or different alkylcarbonyloxy group(s)), a benzoyloxy(C₁-C₃ alkyl) group wherein the phenyl part may be substituted (the substituent(s) is (are) 1-2 same or different substituent(s) selected from the group consisting of a fluorine atom, a chlorine atom and a bromine atom), a (C₁-C₄ alkylthio)(C₁-C₃ alkyl) group, a (C₃-C₆ cycloalkylthio)(C₁-C₃ alkyl) group, a (C₁-C₄ alkylsulfonyl)(C₁-C₃ alkyl) group, a (C₃-C₆ cycloalkylsulfonyl)(C₁-C₃ alkyl) group, a hydroxyimino(C₁-C₃ alkyl) group, a (C₂-C₄ alkoxycarbonyloxyimino)(C₁-C₃ alkyl) group, a (C₁-C₃ alkoxyimino)(C₁-C₃ alkyl) group wherein the alkyl part may be substituted (substituent(s) being C₂-C₄ alkoxycarbonyl group), a (C₇-C₈ aralkyloxyimino)(C₁-C₃ alkyl) group, a (C₂-C₄ alkylcarbonyloxyimino) (C₁-C₃ alkyl) group, a tri(C₁-C₃ alkyl)silyl(C₁-C₃ alkyl) group, a C₃-C₆ cycloalkyl group, a C₂-C₆ alkenyl group, a halo(C₂-C₄ alkenyl) group (the halogen substituent(s) is (are) 1-2 same or different substituent(s) selected from the group consisting of a fluorine atom, a chlorine atom, a bromine atom and an iodine atom), a hydroxy(C₂-C₃ alkenyl) group wherein the alkenyl part may be substituted (substituent(s) being C₂-C₄ alkoxycarbonyl group), a (C₁-C₃ alkoxy) (C₂-C₃ alkenyl) group wherein the alkenyl part may be substituted (substituent(s) being C₂-C₄ alkoxycarbonyl group), a C₂-C₃ alkynyl group, an optionally substituted phenyl group {the substituent(s) is (are) 1-3 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a carboxyl group, a C₂-C₄ alkoxycarbonyl group, a carbamoyl group, a cyano group, a C₁-C₃ alkoxy group, a halo(C₁-C₃ alkoxy) group (the halogen substituent(s) is (are) 1-3 same or different substituent(s) selected from the group consisting of a fluorine atom, a chlorine atom and a bromine atom), a halo(C₂-C₃ alkenyloxy) group (the halogen substituent(s) is (are) 1-2 same or different halogen atom(s) selected from the group consisting of a fluorine atom, a chlorine atom, a bromine atom and an iodine atom) and a nitro group}, a 5-6 membered saturated heterocyclic group (the heterocycle contains 1-2 same or different heteroatom(s) selected from the group consisting of an oxygen atom and a nitrogen atom), an optionally substituted 5-6 membered unsaturated heterocyclic group {the substituent(s) is (are) 1-2 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom) and a C₁-C₃ alkoxy group; the heterocycle contains a sulfur atom or a nitrogen atom, and may contain 1-2 further nitrogen atom(s), and the heterocycle may be fused with a benzene ring}, a C₂-C₆ alkylcarbonyl group, an optionally substituted benzoyl group {substituent(s) being fluorine atom, chlorine atom, bromine atom, cyano group or halo(C₁-C₃ alkoxy) group (the halogen substituent(s) is (are) 1-3 same or different substituent(s) selected from the group consisting of a fluorine atom, a chlorine atom and a bromine atom)}, a C₂-C₅ alkoxycarbonyl group, an optionally substituted phenylsulfonyl group (substituent(s) being C₁-C₃ alkyl group) or a di(C₁-C₃ alkyl)sulfamoyl group;
   more preferably a C₂-C₈ alkyl group, a halo(C₃-C₆ alkyl) group (halogen substituent(s) being fluorine atom or chlorine atom), a (C₃-C₆ cycloalkyl) (C₁-C₂ alkyl) group wherein the cycloalkyl part may be substituted (substituent(s) being methyl group, fluorine atom or chlorine atom), a norbornyl(C₁-C₂ alkyl) group, a benzyl group wherein the phenyl part may be substituted (substituent(s) being fluorine atom, chlorine atom, bromine atom or cyano group), a (5-6 membered saturated heterocyclyl)(C₁-C₂ alkyl) group (the heterocycle contains one oxygen atom), a methoxy (C₃-C₄ alkyl) group, a trimethylsilyloxy(C₃-C₄ alkyl) group, an acetoxy(C₃-C₄ alkyl) group (the number of acetoxy substituent(s) is 1-2), a (C₃-C₄ alkylthio) (C₁-C₂ alkyl) group, a (C₅-C₆ cycloalkylthio)(C₁-C₂ alkyl) group, a (C₃-C₄ alkylsulfonyl)(C₁-C₂ alkyl) group, a trimethylsilyl(C₁-C₂ alkyl) group, a C₅-C₆ cycloalkyl group, a C₄-C₅ alkenyl group, a C₂-C₃ alkynyl group, an optionally substituted phenyl group {the substituent(s) is (are) 1-2 same or different substituent(s) selected from the group consisting of a fluorine atom, a chlorine atom, a methyl group, a halo(C₁-C₂ alkyl) group (the halogen substituent(s) is (are) 1-3 fluorine atom(s)), a carbamoyl group and a cyano group}, a C₃-C₆ alkylcarbonyl group, an optionally substituted benzoyl group (substituent(s) being fluorine atom) or a dimethylsulfamoyl group;
   further more preferably a C₂-C₆ alkyl group, a halo(C₃-C₄ alkyl) group (halogen substituent(s) being fluorine atom or chlorine atom), a (C₄-C₆ cycloalkyl)methyl group wherein the cycloalkyl part may be substituted (substituent(s) being methyl group or fluorine atom), a norbornylmethyl group, a benzyl group, a 3-fluorobenzyl group, a tetrahydropyranylmethyl group, a (C₃-C₄ alkylthio)methyl group, a (C₃-C₄ alkylsulfonyl)methyl group, a trimethylsilylmethyl group, a cyclohexyl group, a pentenyl group, a phenyl group, a 2-fluorophenyl group, a 3-fluorophenyl group, a 4-fluorophenyl group, a 2-chlorophenyl group, a 3-chlorophenyl group, a 2,3-difluorophenyl group, a 2,5-difluorophenyl group, a 3,5-difluorophenyl group, a 2-methylphenyl group, a 3-methylphenyl group, a 4-methylphenyl group or a C₄-C₆ alkylcarbonyl group; particularly preferably a C₃-C₆alkyl group, a cyclobutylmethyl group, a cyclopentylmethyl group, a phenyl group, a 3-fluorophenyl group, a 3,5-difluorophenyl group or a 3-methylphenyl group; most preferably a C₄-C₅ branched alkyl group, a cyclobutylmethyl group, a phenyl group, a 3-fluorophenyl group or a 3-methylphenyl group.
(2) In the present invention, R² can be, for example, a hydrogen atom, a fluorine atom, a chlorine atom, a bromine atom, a methyl group, an ethyl group, a propyl group, an isopropyl group, a butyl group, a s-butyl group, a t-butyl group, an isobutyl group, a pentyl group, a carboxyl group, a methoxycarbonyl group, a methylcarbamoyl group, a cyano group, a trifluoromethyl group, a fluoromethyl group, a chloromethyl group, a hydroxymethyl group, an acetoxymethyl group, a methoxycarbonyloxymethyl group, a methoxymethyl group, an isopropoxymethyl group, a mesyloxymethyl group, a methoxy group, an ethoxy group, a propoxy group, an isopropoxy group or a phenyl group;
   and is preferably a hydrogen atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a hydroxy(C₁-C₃ alkyl) group, a (C₁-C₃ alkoxy) (C₁-C₃ alkyl) group, a carboxyl group, a C₂-C₄ alkoxycarbonyl group or a cyano group;
   more preferably a hydrogen atom or a methyl group.
(3) In the present invention, R³ can be, for example, a hydrogen atom, a fluorine atom, a chlorine atom, a bromine atom, a methyl group, an ethyl group, a propyl group, an isopropyl group, a butyl group, a s-butyl group, a t-butyl group, an isobutyl group, a pentyl group, a carboxyl group, a methoxycarbonyl group, a methylcarbamoyl group, a cyano group, a trifluoromethyl group, a fluoromethyl group, a chloromethyl group, a hydroxymethyl group, an acetoxymethyl group, a methoxycarbonyloxymethyl group, a methoxymethyl group, an isopropoxymethyl group, a mesyloxymethyl group, a methoxy group, an ethoxy group, a propoxy group, an isopropoxy group or a phenyl group;
   and is preferably a hydrogen atom, a fluorine atom, a chlorine atom, a bromine atom, a C₁-C₄ alkyl group, a halo(C₁-C₃ alkyl) group (the halogen substituent(s) is (are) 1-3 same or different substituent(s) selected from the group consisting of a fluorine atom, a'chlorine atom and a bromine atom), a hydroxy(C₁-C₃ alkyl) group, a (C₁-C₃ alkoxy) (C₁-C₃ alkyl) group, a (C₁-C₃ alkylsulfonyloxy) (C₁-C₃ alkyl) group, a carboxyl group, a C₂-C₄ alkoxycarbonyl group, a cyano group or a C₁-C₃ alkoxy group; more preferably a hydrogen atom, a chlorine atom, a C₁-C₂ alkyl group, a halo(C₁-C₂ alkyl) group (the halogen substituent(s) is (are) 1-3 fluorine atom(s)) or a cyano group; further more preferably a hydrogen atom, a methyl group or a fluoromethyl group;
   particularly preferably a hydrogen atom or a methyl group.
(4) In the present invention, R⁴ can be, for example, a hydrogen atom, a methyl group, an ethyl group, a propyl group, an isopropyl group, a butyl group, a s-butyl group, a t-butyl group, an isobutyl group or a cyano group; and is preferably a hydrogen atom, a C₁-C₃ alkyl group or a cyano group;
   more preferably a hydrogen atom or a C₁-C₂ alkyl group; further more preferably a hydrogen atom or a methyl group; particularly preferably a hydrogen atom.
(5) In the present invention, Z is preferably an oxygen atom.
(6) In the present invention, Ar can be, for example, a phenyl group, a 2-fluorophenyl group, a 3-fluorophenyl group, a 4-fluorophenyl group, a 2-chlorophenyl group, a 3-chlorophenyl group, a 4-chlorophenyl group, a 2-bromophenyl group, a 3-bromophenyl group, a 4-bromophenyl group, a 3-iodophenyl group, a 4-iodophenyl group, a 2-methylphenyl group, a 3-methylphenyl group, a 4-methylphenyl group, a 2-ethylphenyl group, a 3-ethylphenyl group, a 4-ethylphenyl group, a 2-propylphenyl group, a 3-isopropylphenyl group, a 4-butylphenyl group, a 2-s-butylphenyl group, a 3-t-butylphenyl group, a 4-neopentylphenyl group, a 3-t-pentylphenyl group, a 4-hexylphenyl group, a 2-fluoromethylphenyl group, a 3-fluoromethylphenyl group, a 4-fluoromethylphenyl group, a 2-difluoromethylphenyl group, a 3-difluoromethylphenyl group, a 4-difluoromethylphenyl group, a 2-trifluoromethylphenyl group, a 3-trifluoromethylphenyl group, a 4-trifluoromethylphenyl group, a 2-chloromethylphenyl group, a 3-chloromethylphenyl group, a 4-chloromethylphenyl group, a 3-dichloromethylphenyl group, a 4-trichloromethylphenyl group, a 2-bromomethylphenyl group, a 3-bromomethylphenyl group, a 4-bromomethylphenyl group, a 3-dibromomethylphenyl group, a 4-dibromomethylphenyl group, a 3-iodomethylphenyl group, a 4-iodomethylphenyl group, a 3-cyclopropylphenyl group, a 4-cyclopropylphenyl group, a 2-cyclobutylphenyl group, a 3-cyclopentylphenyl group, a 4-cyclohexylphenyl group, a 2-(1-chlorocyclopropyl)phenyl group, a 3-(2,2-dichlorocyclopropyl)phenyl group, a 4-(2,2-dimethylcyclopropyl)phenyl group, a 2-vinylphenyl group, a 3-(1-propenyl)phenyl group, a 4-(2-propenyl)phenyl group, a 2-(1-methylvinyl)phenyl group, a 3-(2-methyl-1-propenyl)phenyl group, a 4-(1-pentenyl)phenyl group, a 3-styrylphenyl group, a 2-ethynylphenyl group, a 3-(1-propynyl)phenyl group, a 4-propargylphenyl group, a 2-cyanomethylphenyl group, a 3-cyanomethylphenyl group, a 4-cyanomethylphenyl group, a 2-phenylphenyl group, a 3-phenylphenyl group, a 4-phenylphenyl group, a 3-(2-fluorophenyl)phenyl group, a 4-(3-fluorophenyl)phenyl group, 2-(4-fluorophenyl)phenyl group, a 4-(4-chlorophenyl)phenyl group, a 3-(4-nitrophenyl)phenyl group, a 4-(4-methylphenyl)phenyl group, a 3-(4-methoxyphenyl)phenyl group, a 2-acetylphenyl group, a 3-acetylphenyl group, a 4-acetylphenyl group, a 3-propionylphenyl group, a 4-propionylphenyl group, a 3-benzoylphenyl group, a 3-(2-fluorobenzoyl)phenyl group, a 3-(3-fluorobenzoyl)phenyl group, a 4-(4-fluorobenzoyl)phenyl group, a 4-(4-methylbenzoyl)phenyl group, a 3-(4-methoxybenzoyl)phenyl group, a 2-(2-methoxy-1-methoxycarbonylvinyl)phenyl group, a 3-(2-methoxy-1-methoxycarbonylvinyl)phenyl group, a 4-(2-methoxy-1-methoxycarbonylvinyl)phenyl group, a 3-(1-amino-1-hydroxyiminomethyl)phenyl group, a 3-(1-amino-1-methoxyiminomethyl)phenyl group, a 3-(1-methoxyiminoethyl)phenyl group, a 3-(α-methoxyiminobenzyl)phenyl group, a 2-(1-methoxycarbonyl-1-methoxyiminomethyl)phenyl group, a 3-(1-methoxycarbonyl-1-methoxyiminomethyl)phenyl group, a 4-(1-methoxycarbonyl-1-methoxyiminomethyl)phenyl group, a 3-(1-methoxy-1-iminomethyl)phenyl group, a 3-(1-ethoxy-1-iminomethyl)phenyl group, a 4-(1-ethoxy-1-iminomethyl)phenyl group, a 3-(1-methylthio-1-methyliminomethyl)phenyl group, a 3-(1-ethylthio-1-methyliminomethyl)phenyl group, a 4-(1-ethylthio-1-methyliminomethyl)phenyl group, a 3-(1-methylthio-1-propyliminomethyl)phenyl group, a 3-(1-propylthio-1-methyliminomethyl)phenyl group, a 3-(1-isopropylthio-1-methyliminomethyl)phenyl group, a 3-(1-benzylthio-1-methyliminomethyl)phenyl group, a 3-{1-(3-cyanobenzylthio)-1-methyliminomethyl}phenyl group, a 2-cyanophenyl group, a 3-cyanophenyl group, a 4-cyanophenyl group, a 2-carboxylphenyl group, a 3-carboxylphenyl group, a 4-carboxylphenyl group, a 2-methoxycarbonylphenyl group, a 3-methoxycarbonylphenyl group, a 3-ethoxycarbonylphenyl group, a 4-ethoxycarbonylphenyl group, a 3-propoxycarbonylphenyl group, a 3-isopropoxycarbonylphenyl group, a 2-carbamoylphenyl group, a 3-carbamoylphenyl group, a 4-carbamoylphenyl group, a 2-methylcarbamoylphenyl group, a 3-methylcarbamoylphenyl group, a 4-methylcarbamoylphenyl group, a 3-ethylcarbamoylphenyl group, a 4-ethylcarbamoylphenyl group, a 3-propylcarbamoylphenyl group, a 4-propylcarbamoylphenyl group, a 3-cyanomethylcarbamoylphenyl group, a 4-cyanomethylcarbamoylphenyl group, a 3-dimethylcarbamoylphenyl group, a 4-dimethylcarbamoylphenyl group, a 3-phenylcarbamoylphenyl group, a 3-(4-methoxyphenyl)carbamoylphenyl group, a 3-propionylcarbamoylphenyl group, a 3-thiocarbamoylphenyl group, 4-thiocarbamoylphenyl group, a 3-methyl(thiocarbamoyl)phenyl group, a 4-methyl(thiocarbamoyl)phenyl group, a 3-ethyl(thiocarbamoyl)phenyl group, a 4-ethyl(thiocarbamoyl)phenyl group, a 3-propyl (thiocarbamoyl)phenyl group, a 4-propyl(thiocarbamoyl)phenyl group, a 3-isopropyl(thiocarbamoyl)phenyl group, a 2-hydroxyphenyl group, a 3-hydroxyphenyl group, a 4-hydroxyphenyl group, a 2-methoxyphenyl group, a 3-methoxyphenyl group, a 4-methoxyphenyl group, a 3-ethoxyphenyl group, a 4-ethoxyphenyl group, a 3-propoxyphenyl group, a 4-propoxyphenyl group, a 3-isopropyloxyphenyl group, a 4-butoxyphenyl group, a 3-s-butoxyphenyl group, a 3-t-butoxyphenyl group, a 4-isobutyloxyphenyl group, a 3-fluoromethoxyphenyl group, a 3-chloromethoxyphenyl group, a 3-difluoromethoxyphenyl group, a 3-trifluoromethoxyphenyl group, a 3-(2,2,2-trifluoroethoxy)phenyl group, a 3-pentafluoroethoxyphenyl group, a 3-(2-fluoro-2-iodovinyloxy)phenyl group, a 3-(2,2,3,3-tetrafluoropropoxy)phenyl group, a 3-phenoxyphenyl group, a 2-acetoxyphenyl group, a 3-acetoxyphenyl group, a 4-acetoxyphenyl group, a 3-propionyloxyphenyl group, a 3-benzoyloxyphenyl group, a 2-methoxycarbonyloxyphenyl group, a 3-methoxycarbonyloxyphenyl group, a 4-methoxycarbonyloxyphenyl group, a 3-ethoxycarbonyloxyphenyl group, a 3-phenoxycarbonyloxyphenyl group, a 2-aminophenyl group, a 3-aminophenyl group, a 4-aminophenyl group, a 2-methylaminophenyl group, a 3-methylaminophenyl group, a 4-methylaminophenyl group, a 3-dimethylaminophenyl group, a 3-benzylaminophenyl group, a 3-anilinophenyl group, a 3-formylaminophenyl group, a 2-acetylaminophenyl group, a 3-acetylaminophenyl group, a 4-acetylaminophenyl group, a 3-benzoylaminophenyl group, a 3-(N-acetyl-N-methylamino)phenyl group, a 3-{2-(methoxycarbonylaminopropionyl)amino}phenyl group, 3-{2-(methoxycarbonylamino-3-methylbutyryl)amino}phenyl group, a 3-{2-(isopropoxycarbonylamino-3-methylbutyryl)amino}phenyl group, a 2-methoxycarbonylaminophenyl group, a 3-methoxycarbonylaminophenyl group, a 4-methoxycarbonylaminophenyl group, a 3-ethoxycarbonylaminophenyl group, a 4-phenoxycarbonylaminophenyl group, a 3-(N-methoxycarbonyl-N-methylamino)phenyl group, a 2-(3-methylureido)phenyl group, a 3-(3-ethylureido)phenyl group, a 4-(3-phenylureido)phenyl group, a 3-(3-(2-cyano-2-methoxyiminoacetyl)ureido}phenyl group, a 3-(1,3-dimethylureido)phenyl group, a 2-{methyl(thiocarbonyl)amino}phenyl group, a 3-{methyl(thiocarbonyl)amino}phenyl group, a 4-{methyl(thiocarbonyl)amino}phenyl group, a 2-{3-methyl(thioureido)}phenyl group, a 3-{3-methyl(thioureido)}phenyl group, a 4-{3-phenyl(thioureido)}phenyl group, a 2-methylsulfonylaminophenyl group, a 3-methylsulfonylaminophenyl group, a 4-methylsulfonylaminophenyl group, a 3-ethylsulfonylaminophenyl group, a 4-ethylsulfonylaminophenyl group, a 3-phenylsulfonylaminophenyl group, a 4-phenylsulfonylaminophenyl group, a 2-isocyanophenyl group, a 3-isocyanophenyl group, a 4-isocyanophenyl group, a 2-nitrophenyl group, a 3-nitrophenyl group, a 4-nitrophenyl group, a 2-mercaptophenyl group, a 3-mercaptophenyl group, a 4-mercaptophenyl group, a 2-methylthiophenyl group, a 3-methylthiophenyl group, a 4-methylthiophenyl group, a 3-ethylthiophenyl group, a 3-phenylthiophenyl group, a 3-trifluoromethylthiophenyl group, a 2-methylsulfinylphenyl group, a 3-methylsulfinylphenyl group, a 4-methylsulfinylphenyl group, a 3-phenylsulfinylphenyl group, a 3-trifluoromethylsulfinylphenyl group, a 2-methylsulfonylphenyl group, a 3-methylsulfonylphenyl group, a 4-methylsulfonylphenyl group, a 3-phenylsulfonylphenyl group, a 3-trifluoromethylsulfonylphenyl group, a 2-acetylthiophenyl group, a 3-acetylthiophenyl group, a 4-acetylthiophenyl group, a 3-benzoylthiophenyl group, a 2-[(methylthio)(thiocarbonyl)thio]phenyl group, a 3-[(methylthio)(thiocarbonyl)thio]phenyl group, a 2-trimethylsilylphenyl group, a 3-trimethylsilylphenyl group, a 4-trimethylsilylphenyl group, a 3-(2-thienyl)phenyl group, a 4-(2-thienyl)phenyl group, a 3-(3-methylpyrazol-5-yl)phenyl group, a 3-(thiazol-2-yl)phenyl group, a 4-(thiazol-2-yl)phenyl group, a 3-(4-methylthiazol-2-yl)phenyl group, a 4-(4-methylthiazol-2-yl)phenyl group, a 3-(1,2,4-oxadiazol-3-yl)phenyl group, a 4-(1,2,4-oxadiazol-3-yl)phenyl group, a 3-(5-methyl-1,2,4-oxadiazol-3-yl)phenyl group, a 4-(5-methyl-1,2,4-oxadiazol-3-yl)phenyl group, a 4-(5-trifluoromethyl-1,2,4-oxadiazol-3-yl)phenyl group, a 2,4-difluorophenyl group, a 3,5-difluorophenyl group, a 2,4-dichlorophenyl group, a 3,5-dichlorophenyl group, a 2,4-dibromophenyl group, a 3,5-dibromophenyl group, a 5-bromo-2-fluorophenyl group, a 3-bromo-4-fluorophenyl group, a 3-bromo-5-fluorophenyl group, a 4-bromo-3-fluorophenyl group, a 3-bromo-4-methoxyphenyl group, a 5-bromo-2-methoxyphenyl group, a 3-bromo-4-ethoxyphenyl group, a 5-bromo-2-ethoxyphenyl group, a 2,4-dimethylphenyl group, a 3,5-dimethylphenyl group, a 2,4-dimethoxyphenyl group, a 3,5-dimethoxyphenyl group, a 2,4-dinitrophenyl group, a 3,5-dinitrophenyl group, a 2,4-dicyanophenyl group, a 2,5-dicyanophenyl group, a 3,4-dicyanophenyl group, a 3,5-dicyanophenyl group, a 3-amino-4-fluorophenyl group, a 2-fluoro-5-nitrophenyl group, a 5-nitro-2-phenoxyphenyl group, a 2-tolyloxy-5-nitrophenyl group, a 5-nitro-2-methylthiophenyl group, a 5-nitro-2-phenylthiophenyl group, a 5-nitro-2-methylsulfinylphenyl group, a 5-nitro-2-phenylsulfinylphenyl group, a 5-nitro-2-methylsulfonylphenyl group, a 5-nitro-2-phenylsulfonylphenyl group, a 5-cyano-2-fluorophenyl group, a 5-cyano-2-bromophenyl group, a 5-cyano-2-methoxyphenyl group, a 2-ethoxy-5-cyanophenyl group, a 5-cyano-2-phenoxyphenyl group, a 5-cyano-2-tolyloxyphenyl group, a 5-cyano-2-nitrophenyl group, a 5-cyano-2-methylthiophenyl group, a 5-cyano-2-phenylthiophenyl group, 5-cyano-2-methylsulfinylphenyl group, a 5-cyano-2-phenylsulfinylphenyl group, a 5-cyano-2-methylsulfonylphenyl group, a 5-cyano-2-phenylsulfonylphenyl group, a 3-nitro-5-fluorophenyl group, a 4-azido-3-nitrophenyl group, a 4-ethoxy-3-nitrophenyl group, a 3-nitro-4-methylaminophenyl group, a 3-nitro-4-phenethylaminophenyl group, a 3-nitro-4-methylthiophenyl group, a 3-nitro-4-phenylthiophenyl group, a 3-nitro-4-methylsulfinylphenyl group, a 3-nitro-4-phenylsulfinylphenyl group, a 3-nitro-4-methylsulfonylphenyl group, a 3-nitro-4-phenylsulfonylphenyl group, a 3-cyano-4-fluorophenyl group, a 3-cyano-5-fluorophenyl group, a 3-cyano-4-methoxyphenyl group, a 4-ethoxy-3-cyanophenyl group, a 3-cyano-4-phenoxyphenyl group, a 3-cyano-4-tolyloxyphenyl group, a 4-azido-3-cyanophenyl group, a 4-amino-3-cyanophenyl group, a 3-cyano-4-methylaminophenyl group, a 3-cyano-4-phenethylaminophenyl group, a 3-cyano-4-methylthiophenyl group, a 3-cyano-4-phenylthiophenyl group, a 3-cyano-4-methylsulfinylphenyl group, a 3-cyano-4-phenylsulfinylphenyl group, a 3-cyano-4-methylsulfonylphenyl group, a 3-cyano-4-phenylsulfonylphenyl group, a 4-ethoxy-3-cyanophenyl group, a 3-cyano-5-bromomethylphenyl group, a 3-cyano-5-cyanomethylphenyl group, a 3-cyano-5-methylthiomethylphenyl group, a 3-cyano-5-methylphenyl group, a 3-cyano-5-methylsulfonylmethylphenyl group, a 2-cyano-5-fluorophenyl group, a 4-cyano-4-dimethylamino-2-fluorophenyl group, a 2-chloro-3-nitro-4-methylaminophenyl group, a 2,3,4,6-tetrafluorophenyl group, a pentafluorophenyl group, a 1-naphthyl group, a 3-fluoro-1-naphthyl group, a 4-cyano-1-naphthyl group, a 5-nitro-1-naphthyl group, a 2-naphthyl group, a 4-chloro-2-naphthyl group, a 5-cyano-2-naphthyl group, a 1-anthryl group, a 2-anthryl group, a 9-anthryl group, a ferrocenyl group, a 2-furyl group, a 5-methyl-2-furyl group, a 4-methoxycarbonyl-2-furyl group, a 4-cyano-2-furyl group, a 5-cyano-2-furyl group, a 3-furyl group, a 4-cyano-3-furyl group, a 2-cyano-3-furyl group, a 2-thienyl group, a 5-chloro-2-thienyl group, a 2,3-dichloro-5-thienyl group, a 4-nitro-2-thienyl group, a 5-nitro-2-thienyl group, a 4-cyano-2-thienyl group, a 5-cyano-2-thienyl group, a 5-methoxycarbonyl-2-thienyl group, a 4-methyl-2-thienyl group, a 3-methoxy-2-thienyl group, a 4-methoxy-2-thienyl group, a 5-methoxy-2-thienyl group, a 3-thienyl group, a 2-methyl-3-thienyl group, a 5-nitro-3-thienyl group, a 2-cyano-3-thienyl group, a 5-cyano-3-thienyl group, a 2-oxazolyl group, a 5-methyl-2-oxazolyl group, a 4-oxazolyl group, a 3-isooxazolyl group, a 5-methyl-3-isooxazolyl group, a 2-thiazolyl group, a 4-methyl-2-thiazolyl group, a 4-thiazolyl group, a 1-methyl-4-pyrazolyl group, a 1-phenyl-4-pyrazolyl group, a 1,3,5-trimethyl-4-pyrazolyl group, a 1,2,4-oxadiazol-3-yl group, a 5-methyl-1,2,4-oxadiazol-3-yl group, a 1,2,4-thiadiazol-3-yl group, a 5-methyl-1,2,4-thiadiazol-3-yl group, a 2-pyridyl group, a 6-chloro-2-pyridyl group, a 3-pyridyl group, a 4-pyridyl group, a 2-chloro-5-pyridyl group, a 2,6-dichloro-4-pyridyl group, a 3-pyridazyl group, a 4-pyridazyl group, a 2-pyrimidinyl group, a 4-methyl-2-pyrimidinyl group, a 4,6-dimethyl-2-pyrimidinyl group, a 4,6-dimethoxy-2-pyrimidinyl group, a 4,6-dichloro-2-pyrimidinyl group, a 4-pyrimidinyl group, a 2,6-dimethoxy-4-pyrimidinyl group, a 2,6-dichloro-4-pyrimidinyl group, a 1,3,5-triazinyl group, a 4,6-dimethoxy-1,3,5-triazinyl group, a 4,6-dichloro-1,3,5-triazinyl group, a 2-benzooxazolyl group, a 2-benzothiazolyl group, a 4-benzothiazolyl group, a 5-benzothiazolyl group, a 6-benzothiazolyl group, a 7-benzothiazolyl group, a 2-methyl-5-benzothiazolyl group, a 2-ethyl-5-benzothiazolyl group, a 2-t-butyl-5-benzothiazolyl group, a 2-methyl-7-benzothiazolyl group, a 2-ethyl-7-benzothiazolyl group or a 1,2,3-benzothiadiazol-7-yl group; B can be a hydrogen atom, a methyl group, an ethyl group, a propyl group, an isopropyl group, a butyl group, an isobutyl group, a s-butyl group, a t-butyl group, a pentyl group, a s-pentyl group, a t-pentyl group, a neopentyl group, a hexyl group, a heptyl group, an octyl group, a nonyl group, a decyl group, an undecyl group, a dodecyl group, a tridecyl group, a tetradecyl group, a pentadecyl group, a hexadecyl group, a cyclopropyl group, a cyclobutyl group, a cyclopentyl group, a cyclohexyl group, a cycloheptyl group, a cyclooctyl group, a trifluoromethyl group, a chloromethyl group, a dichloromethyl group, a trichloromethyl group, a bromomethyl group, a vinyl group, a 1-propenyl group, a 2-methyl-1-propenyl group, a 1-methylvinyl group, a 1-methyl-1-propenyl group, a 1,2-dimethyl-1-propenyl group, a 2-propenyl group, a 2-butenyl group, a 2-pentenyl group, a 3-methyl-2-butenyl group, a 2-methyl-2-propenyl group, a 2-methyl-2-butenyl group, a 2,3-dimethyl-2-butenyl group, a 2-methyl-3-butenyl group, a 1-cyclobutenyl group, a 3-cyclobutenyl group, a 1-cyclopentenyl group, a 3-cyclopentenyl group, a 1-cyclohexenyl group, a 3-cyclohexenyl group, a 4-cyclohexenyl group, an ethynyl group, a 1-propynyl group, a propargyl group, a 2-butynyl group, a phenyl group, a 2-fluorophenyl group, a 3-fluorophenyl group, a 4-fluorophenyl group, a 2-chlorophenyl group, a 3-chlorophenyl group, a 4-chlorophenyl group, a 2-bromophenyl group, a 3-bromophenyl group, a 4-bromophenyl group, a 3-iodophenyl group, a 4-iodophenyl group, a 2-methylphenyl group, a 3-methylphenyl group, a 4-methylphenyl group, a 2-methoxyphenyl group, a 3-methoxyphenyl group, a 4-methoxyphenyl group, a 2-trifluoromethylphenyl group, a 3-trifluoromethylphenyl group, a 4-trifluoromethylphenyl group, a 2-nitrophenyl group, a 3-nitrophenyl group, a 4-nitrophenyl group, a 2-cyanophenyl group, a 3-cyanophenyl group, a 4-cyanophenyl group, a 2,4-difluorophenyl group, a 3,5-difluorophenyl group, a 2,4-dichlorophenyl group, a 3,5-dichlorophenyl group, a 2,4-dinitrophenyl group, a 2-furyl group, a 3-furyl group, a 2-thienyl group, a 3-thienyl group, a 2-oxazolyl group, a 4-oxazolyl group, a 2-thiazolyl group, a 4-thiazolyl group, a 2-isooxazolyl group, a 5-methylisooxazol-3-yl group, a 5-phenylisooxazol-3-yl group, a 1,2,3-thiadiazol-5-yl group, a 4-methyl-1,2,3-thiadiazol-5-yl group, a 1,2,3-benzothiadiazol-7-yl group, a 2-furylmethyl group, a 3-furylmethyl group, a 2-thienylmethyl group, a 3-thienylmethyl group, a 2-oxazolylmethyl group, a 4-oxazolylmethyl group, a 2-thiazolylmethyl group, a 4-thiazolylmethyl group, a 5-methylisooxazol-3-ylmethyl group, a 5-phenylisooxazol-3-ylmethyl group, a 1-pyrazolylmethyl group, a 2-(1-pyrazolyl)ethyl group, a 3-methylpyrazol-1-ylmethyl group, a 5-methylpyrazol-1-ylmethyl group, a 3,5-dimethylpyrazol-1-ylmethyl group, a 4-fluoropyrazol-1-ylmethyl group, a 4-chloropyrazol-1-ylmethyl group, a 2-(4-chloropyrazol-1-yl)ethyl group, a 4-bromopyrazol-1-ylmethyl group, a 2-(4-bromopyrazol-1-yl)ethyl group, a 4-iodopyrazol-1-ylmethyl group, a 4-nitropyrazol-1-ylmethyl group, a 4-cyanopyrazol-1-ylmethyl group, a 4-carboxylpyrazol-1-ylmethyl group, a 4-methoxycarbonylpyrazol-1-ylmethyl group, a 4-ethoxycarbonylpyrazol-1-ylmethyl group, a 4-aminopyrazol-1-ylmethyl group, a 4-acetylaminopyrazol-1-ylmethyl group, a 4-benzoylaminopyrazol-1-ylmethyl group, a 4-nitro-3-methylpyrazol-1-ylmethyl group, a 4-nitro-5-methylpyrazol-1-ylmethyl group, a 4-nitro-3,5-dimethylpyrazol-1-ylmethyl group, a 4-amino-3-methylpyrazol-1-ylmethyl group, a 4-amino-5-methylpyrazol-1-ylmethyl group, a 4-amino-3,5-dimethylpyrazol-1-ylmethyl group, a 1-imidazolylmethyl group, a 4,5-dichloro-1-imidazolylmethyl group, a 1,2,4-triazol-1-ylmethyl group, a 2-methyl-3-oxo-1(3H)-pyrazolylmethyl group, a 2-methyl-5-oxo-3(5H)-pyrazolylmethyl group, a 2,5-dimethyl-3-oxo-1(3H)-pyrazolylmethyl group, a 2-oxo-1(2H)-pyrazolylmethyl group, a 2-(2-oxo-1(2H)-pyrazolyl)ethyl group, a 3-methyl-2-oxo-1(2H)-pyrazolylmethyl group, a 5-trifluoromethyl-2-oxo-1(2H)-pyrazolylmethyl group, a 4-oxo-3(4H)-quinoxanylmethyl group, a 2-pyridyl group, a 2-pyridylmethyl group, a 3-pyridyl group, a 3-pyridylmethyl group, a 4-pyridyl group, a 2-chloro-4-pyridyl group, a 2,6-dichloro-4-pyridyl group, a 4-pyridylmethyl group, a carboxylmethyl group, a methoxycarbonylmethyl group, a 2-methoxycarbonylethyl group, an ethoxycarbonylmethyl group, a phenoxycarbonylmethyl group, a carbamoylmethyl group, a 2-carbamoylethyl group, a methylcarbamoylmethyl group, a phenylcarbamoylmethyl group, a dimethylcarbamoylmethyl group, a cyanomethyl group, a cyclopropylmethyl group, a 2-cyclopropylethyl group, a 3-cyclopropylpropyl group, a cyclobutylmethyl group, a 2-cyclobutylethyl group, a cyclopentylmethyl group, a 2-cyclopentylethyl group, a 3-cyclopentylpropyl group, a 1-cyclopentenylmethyl group, a cyclohexylmethyl group, a benzyl group, a phenethyl group, a 3-phenylpropyl group, a 4-phenylbutyl group, a 3-phenyl-2-propenyl group, a 2-fluorobenzyl group, a 3-chlorobenzyl group, a 4-bromobenzyl group, a 3-cyanobenzyl group, a 3-cyanophenethyl group, a 3-trifluoromethoxybenzyl group, a hydroxymethyl group, a 2-hydroxyethyl group, a 3-hydroxypropyl group, a 4-hydroxybutyl group, a 5-hydroxypentyl group, a 1-hydroxyethyl group, a 1-hydroxypropyl group, a 2-hydroxypropan-2-yl group, 3-hydroxypentan-3-yl group, a methoxymethyl group, a 2-methoxyethyl group, a 3-methoxypropyl group, a 4-methoxybutyl group, an ethoxymethyl group, a 2-ethoxyethyl group, a 3-ethoxypropyl group, a propoxymethyl group, a 2-propoxyethyl group, an isopropoxymethyl group, a butoxymethyl group, a s-butoxymethyl group, a t-butoxymethyl group, an isobutoxymethyl group, a pentoxymethyl group, a hexyloxymethyl group, a benzyloxymethyl group, a 2-fluorobenzyloxymethyl group, a 3-fluorobenzyloxymethyl group, a 4-fluorobenzyloxymethyl group, a 3-cyanobenzyloxymethyl group, a 3-nitrobenzyloxymethyl group, a 3-trifluoromethoxybenzyloxymethyl group, a 2,6-dichloro-4-pyridylmethoxymethyl group, a methoxymethoxymethyl group, an ethoxymethoxymethyl group, a 2-methoxyethoxymethoxymethyl group, a 2-tetrahydropyranyloxymethyl group, a benzyloxymethoxymethyl group, a 2-methoxyethoxymethyl group, a cyanomethoxymethyl group, a 2-(cyanomethoxy)ethyl group, an allyloxymethyl group, a 2-allyloxymethyl group, a 3-allyloxymethyl group, a 2-butenyloxymethyl group, a 3-methyl-2-butenyloxymethyl group, a 2-(3-methyl-2-butenyloxy)ethyl group, a methallyloxymethyl group, a 2-methyl-2-butenyloxymethyl group, a 2,3-dimethyl-2-butenyloxymethyl group, a propargyloxymethyl group, a 2-butynyloxymethyl group, a 2-(2-butynyloxy)ethyl group, a phenoxymethyl group, a 2-phenoxyethyl group, a 3-phenoxypropyl group, a 4-phenoxybutyl group, a 5-phenoxypentyl group, a 2-fluorophenoxymethyl group, a 3-fluorophenoxymethyl group, a 2-(3-fluorophenoxy)ethyl group, a 4-fluorophenoxymethyl group, a 2-chlorophenoxymethyl group, a 3-chlorophenoxymethyl group, a 4-chlorophenoxymethyl group, a 3-(4-chlorophenoxy)propyl group, a 2-bromophenoxymethyl group, a 3-bromophenoxymethyl group, a 4-bromophenoxymethyl group, a 2-iodophenoxymethyl group, a 3-iodophenoxymethyl group, a 4-iodophenoxymethyl group, a 2-methylphenoxymethyl group, a 3-methylphenoxymethyl group, a 2-(3-methylphenoxy)ethyl group, a 4-methylphenoxymethyl group, a 2-methoxyphenoxymethyl group, a 3-methoxyphenoxymethyl group, a 4-methoxyphenoxymethyl group, a 2-(4-methoxyphenoxy)ethyl group, a 2-trifluoromethylphenoxymethyl group, a 3-trifluoromethylphenoxymethyl group, a 4-trifluoromethylphenoxymethyl group, a 2-nitrophenoxymethyl group, a 3-nitrophenoxymethyl group, a 2-(3-nitrophenoxy)ethyl group, a 4-nitrophenoxymethyl group, a 2-(4-nitrophenoxy)ethyl group, a 2-cyanophenoxymethyl group, a 3-cyanophenoxymethyl group, a 2-(3-cyanophenoxy)ethyl group, a 4-cyanophenoxymethyl group, a 2-(4-cyanophenoxy)ethyl group, a 2,4-difluorophenoxymethyl group, a 3,5-difluorophenoxymethyl group, a 2,4-dichlorophenoxymethyl group, a 3,5-dichlorophenoxymethyl group, a 2,4-dinitrophenoxymethyl group, a 2-furyloxymethyl group, a 2-(2-furyloxy)ethyl group, a 3-furyloxymethyl group, a 2-thienyloxymethyl group, a 3-thienyloxymethyl group, a 2-(3-thienyloxy)ethyl group, a 2-oxazolyloxymethyl group, a 4-oxazolyloxymethyl group, a 2-benzooxazolyloxymethyl group, a 2-thiazolyloxymethyl group, a 4-thiazolyloxymethyl group, a 2-benzothiazolyloxymethyl group, a 3-isooxazolyloxymethyl group, a 5-methylisooxazol-3-yloxymethyl group, a 2-(5-methylisooxazol-3-yloxy)ethyl group, a 4,5-dimethylisooxazol-3-yloxymethyl group, a 5-isopropylisooxazol-3-yloxymethyl group, a 4-chloro-5-methylisooxazol-3-yloxymethyl group, a 5-phenylisooxazol-3-yloxymethyl group, a 4-chloro-5-phenylisooxazol-3-yloxymethyl group, a 5-methoxycarbonylisooxazol-3-yloxymethyl group, a 3-benzisooxazolyloxymethyl group, a 7-methylbenzisooxazol-3-yloxymethyl group, a 6-chlorobenzisooxazol-3-yloxymethyl group, a 7-aminobenzisooxazol-3-yloxymethyl group, a 7-nitrobenzisoxazol-3-yloxymethyl group, a benzisothiazol-1,1-dioxide-3-yloxymethyl group, a 1-pyrazolyloxymethyl group, a 4-methylpyrazol-1-yloxymethyl group, a 1,2,3-thiadiazol-5-yloxymethyl group, a 1,2,3-benzothiadiazol-7-yloxymethyl group, a 2-pyridyloxymethyl group, a 3-methyl-2-pyridyloxymethyl group, a 3,4,5,6-tetrafluoro-2-pyridyloxymethyl group, a 5-trifluoromethyl-2-pyridyloxymethyl group, a 3-fluoro-5-trifluoromethyl-2-pyridyloxymethyl group, a 3-chloro-5-trifluoromethyl-2-pyridyloxymethyl group, a 3-pyridyloxymethyl group, a 4-pyridyloxymethyl group, a 2-pyrazinyloxymethyl group, a 2-pyrimidinyloxymethyl group, a 4-pyrimidinyloxymethyl group, a 2-isopropyl-6-methyl-4-pyrimidinyloxymethyl group, a 3-pyridazinyloxymethyl group, a 6-chloro-3-pyridazinyloxymethyl group, a trimethylsilyloxymethyl group, a 2-trimethylsilyloxyethyl group, a triethylsilyloxymethyl group, a triisopropylsilyloxymethyl group, an isopropyldimethylsilyloxymethyl group, a t-butyldimethylsilyloxymethyl group, a 2-(t-butyldimethylsilyloxy)ethyl group, a 3-(t-butyldimethylsilyloxy)propyl group, a 4-(t-butyldimethylsilyloxy)butyl group, a triphenylsilyloxymethyl group, a t-butyldiphenylsilyloxymethyl group, a 2-trimethylsilylethoxymethoxymethyl group, a formyloxymethyl group, an acetoxymethyl group, a 2-acetoxyethyl group, a 3-acetoxypropyl group, a 4-acetoxybutyl group, a propionyloxymethyl group, a 2-propionyloxyethyl group, a 3-propionyloxypropyl group, a butyryloxymethyl group, a 2-butyryloxyethyl group, an isobutyryloxymethyl group, a valeryloxymethyl group, an isovaleryloxymethyl group, a 2-methylbutyryloxymethyl group, a pivaloyloxymethyl group, a pentylcarbonyloxymethyl group, a s-pentylcarbonyloxymethyl group, a t-pentylcarbonyloxymethyl group, a neopentylcarbonyloxymethyl group, a hexylcarbonyloxymethyl group, a heptylcarbonyloxymethyl group, an octylcarbonyloxymethyl group, a nonylcarbonyloxymethyl group, a decylcarbonyloxymethyl group, an undecylcarbonyloxymethyl group, a dodecylcarbonyloxymethyl group, a tridecylcarbonyloxymethyl group, a tetradecylcarbonyloxymethyl group, a pentadecylcarbonyloxymethyl group, a hexadecylcarbonyloxymethyl group, a cyclopropylcarbonyloxymethyl group, a cyclobutylcarbonyloxymethyl group, a cyclopentylcarbonyloxymethyl group, a cyclohexylcarbonyloxymethyl group, a cycloheptylcarbonyloxymethyl group, a cyclooctylcarbonyloxymethyl group, a 1-acetoxyethyl group, a 1-acetoxypropyl group, a 2-acetoxypropan-2-yl group, a 3-acetoxypentan-3-yl group, a trifluoroacetoxymethyl group, a 2-trifluoroacetoxyethyl group, a chloroacetoxymethyl group, a dichloroacetoxymethyl group, a trichloroacetoxymethyl group, a bromoacetoxymethyl group, a methoxyacetoxymethyl group, an ethoxyacetoxymethyl group, a cyanomethoxyacetoxymethyl group, a 2-(cyanomethoxyacetoxy)ethyl group, a phenoxyacetoxymethyl group, an acetoxyacetoxymethyl group, a cyano(methylthio)acetoxymethyl group, an acryloyloxymethyl group, a 1-propynylcarbonyloxymethyl group, a 2-methyl-1-propenylcarbonyloxymethyl group, a 2-(2-methyl-1-propenylcarbonyloxy)ethyl group, a 2-methylacryloyloxymethyl group, a 1-methyl-1-propenylcarbonyloxymethyl group, a 1,2-dimethyl-1-propenylcarbonyloxymethyl group, a 2-propenylcarbonyloxymethyl group, a 2-butenylcarbonyloxymethyl group, a 2-pentenylcarbonyloxymethyl group, a 3-methyl-2-butenylcarbonyloxymethyl group, a 2-(3-methyl-2-butenylcarbonyloxy)ethyl group, a 2-methyl-2-propenylcarbonyloxymethyl group, a 2-methyl-2-butenylcarbonyloxymethyl group, a 2,3-dimethyl-2-butenylcarbonyloxymethyl group, a 3-butenylcarbonyloxymethyl group, a 3-pentenylcarbonyloxymethyl group, a 4-pentenylcarbonyloxymethyl group, a 1-cyclobutenylcarbonyloxymethyl group, a 3-cyclobutenylcarbonyloxymethyl group, a 1-cyclopentenylcarbonyloxymethyl group, a 2-(1-cyclopentenylcarbonyloxy)ethyl group, a 3-cyclopentenylcarbonyloxymethyl group, a 1-cyclohexenylcarbonyloxymethyl group, a 3-cyclohexenylcarbonyloxymethyl group, a 4-cyclohexenylcarbonyloxymethyl group, a 2-methyl-1,4 cyclohexadien-1-ylcarbonyloxymethyl group, a 1-cyclopentenylacetoxymethyl group, a 3-cyclopentenylacetoxymethyl group, a 1-cyclohexenylacetoxymethyl group, a propioloyloxymethyl group, a 1-propynylcarbonyloxymethyl group, a 2-propynylcarbonyloxymethyl group, a 2-butynylcarbonyloxymethyl group, a 2-(2-butynylcarbonyloxy)ethyl group, a phenylacetoxymethyl group, a 3-phenylpropionyloxymethyl group, a 4-phenylbutyryloxymethyl group, a 5-phenylvaleryloxymethyl group, a 3-phenyl-2-propenylcarbonyloxymethyl group, a (2-fluorophenyl)acetoxymethyl group, a (3-chlorophenyl)acetoxymethyl group, a (4-bromophenyl)acetoxymethyl group, a benzoyloxymethyl group, a 2-benzoyloxyethyl group, a 2-fluorobenzoyloxymethyl group, a 3-fluorobenzoyloxymethyl group, a 2- (3-fluorobenzoyloxy) ethyl group, a 4-fluorobenzoyloxymethyl group, a 2-chlorobenzoyloxymethyl group, a 3-chlorobenzoyloxymethyl group, a 4-chlorobenzoyloxymethyl group, a 2-(4-chlorobenzoyloxy)ethyl group, a 2-bromobenzoyloxymethyl group, a 3-bromobenzoyloxymethyl group, a 4-bromobenzoyloxymethyl group, a 2-iodobenzoyloxymethyl group, a 3-iodobenzoyloxymethyl group, a 4-iodobenzoyloxymethyl group, a 2-methylbenzoyloxymethyl group, a 3-methylbenzoyloxymethyl group, a 2-(3-methylbenzoyloxy)ethyl group, a 4-methylbenzoyloxymethyl group, a 2-methoxybenzoyloxymethyl group, a 3-methoxybenzoyloxymethyl group, a 4-methoxybenzoyloxymethyl group, a 2-(4-methoxybenzoyloxy)ethyl group, a 2-trifluoromethylbenzoyloxymethyl group, a 3-trifluoromethylbenzoyloxymethyl group, a 4-trifluoromethylbenzoyloxymethyl group, a 2-nitrobenzoyloxymethyl group, a 3-nitrobenzoyloxymethyl group, a 2-(3-nitrobenzoyloxy)ethyl group, a 4-nitrobenzoyloxymethyl group, a 2-cyanobenzoyloxymethyl group, a 3-cyanobenzoyloxymethyl group, a 4-cyanobenzoyloxymethyl group, a 2-(4-cyanobenzoyloxy)ethyl group, a 2,4-difluorobenzoyloxymethyl group, a 3,5-difluorobenzoyloxymethyl group, a 2,4-dichlorobenzoyloxymethyl group, a 3,5-dichlorobenzoyloxymethyl group, a 2,4-dinitrobenzoyloxymethyl group, a 2-furylacetoxymethyl group, a 2-(2-furylacetoxy)ethyl group, a 3-furylacetoxymethyl group, a 2-thienylacetoxymethyl group, a 3-thienylacetoxymethyl group, a 2-oxazolylacetoxymethyl group, a 4-oxazolylacetoxymethyl group, a 2-thiazolylacetoxymethyl group, a 4-thiazolylacetoxymethyl group, a 2-isooxazolylacetoxymethyl group, a 5-methylisooxazol-3-ylacetoxymethyl group, a 2-(5-methylisooxazol-3-ylacetoxy)ethyl group, a 5-phenylisooxazol-3-ylacetoxymethyl group, a 3-indolylacetoxymethyl group, a 3-benzothiophenylacetoxymethyl group, a 2-furylacetoxymethyl group, a 2-(2-furylacetoxy)ethyl group, a 3-furylacetoxymethyl group, a 2,5-dimethyl-3-furylacetoxymethyl group, a 5-t-butyl-2-methyl-3-furylacetoxymethyl group, a 2-trifluoromethyl-5-methyl-3-furylacetoxymethyl group, a 2-thienylcarbonyloxymethyl group, a 3-thienylcarbonyloxymethyl group, a 1,2,5-trimethyl-3-pyrrolylcarbonyloxymethyl group, a 2-oxazolylcarbonyloxymethyl group, a 4-oxazolylcarbonyloxymethyl group, a 2-thiazolylcarbonyloxymethyl group, a 4-thiazolylcarbonyloxymethyl group, a 2-isooxazolylcarbonyloxymethyl group, a 5-methyl-3-oxo-2(3H)-isooxazolylcarbonyloxymethyl group, a 5-methylisooxazol-3-ylcarbonyloxymethyl group, a 2-(5-methylisooxazol-3-ylcarbonyloxy)ethyl group, a 5-phenylisooxazol-3-ylcarbonyloxymethyl group, a 1,2,3-thiadiazol-5-ylcarbonyloxymethyl group, a 4-methyl-1,2,3-thiadiazol-5-ylcarbonyloxymethyl group, a 1,2,3-benzothiadiazol-7-ylcarbonyloxymethyl group, a 2-pyridylcarbonyloxymethyl group, a 2-(2-pyridylcarbonyloxy)ethyl group, a 2-pyridylacetoxymethyl group, a 3-pyridylcarbonyloxymethyl group, a 2-(3-pyridylcarbonyloxy)ethyl group, a 3-pyridylacetoxymethyl group, a 4-pyridylcarbonyloxymethyl group, a 2,6-dichloro-4-pyridylcarbonyloxymethyl group, a 2-chloro-6-methoxy-4-pyridylcarbonyloxymethyl group, a 2-(2,6-dichloro-2-pyridylcarbonyloxy)ethyl group, a 2-pyridylacetoxymethyl group, a methoxycarbonyloxymethyl group, a 2-methoxycarbonyloxyethyl group, a 3-methoxycarbonyloxypropyl group, an ethoxycarbonyloxymethyl group, a propoxycarbonyloxymethyl group, an isopropoxycarbonyloxymethyl group, an allyloxycarbonyloxymethyl group, a butoxycarbonyloxymethyl group, an isobutoxycarbonyloxymethyl group, a s-butoxycarbonyloxymethyl group, a t-butoxycarbonyloxymethyl group, a benzyloxycarbonyloxymethyl group, a phenoxycarbonyloxymethyl group, a *carbamoyloxymethyl group, a* 2-carbamoyloxyethyl group, a 3-carbamoyloxypropyl group, a 4-carbamoyloxybutyl group, a methylcarbamoyloxymethyl group, an ethylcarbamoyloxymethyl group, a phenylcarbamoyloxymethyl group, a dimethylcarbamoyloxymethyl group, a diethylcarbamoyloxymethyl group, a chloroacetylcarbamoyloxymethyl group, a trichloroacetylcarbamoyloxymethyl group, a benzoylcarbamoyloxymethyl group, an ethoxycarbonylcarbamoyloxymethyl group, a 2-cyano-2-methoxyiminoacetylcarbamoyloxymethyl group, a 2-(2-cyano-2-methoxyiminoacetylcarbamoyloxy)ethyl group, a (methylthio)carbonyloxymethyl group, a (phenylthio)carbonyloxymethyl group, a methyl(thiocarbonyl)oxymethyl group, a 2-{methyl(thiocarbonyl)oxy}ethyl group, a 3-{methyl(thiocarbonyl)oxy}propyl group, an ethyl(thiocarbonyl)oxymethyl group, a phenyl(thiocarbonyl)oxymethyl group, a methyl(thiocarbamoyl)oxymethyl group, a phenyl(thiocarbamoyl)oxymethyl group, a dimethyl(thiocarbamoyl)oxymethyl group, a (methylthio)(thiocarbonyl)oxymethyl group, a dimethoxyphosphoryloxymethyl group, a diethoxyphosphoryloxymethyl group, a dimethoxythiophosphoryloxymethyl group, a diethoxythiophosphoryloxymethyl group, a methylsulfonyloxymethyl group, an ethylsulfonyloxymethyl group, a phenylsulfonyloxymethyl group, a tolylsulfonyloxymethyl group, an aminomethyl group, a 2-aminoethyl group, a 3-aminopropyl group, a methylaminomethyl group, an ethylaminomethyl group, a 3,4-dimethoxyphenethylaminomethyl group, a (1-carboxylethyl)aminomethyl group, a (1-methoxycarbonylethyl)aminomethyl group, a (1-benzyloxycarbonylethyl)aminomethyl group, a (1-carboxyl-2-methylpropyl) aminomethyl group, a (1-methoxycarbonyl-2-methylpropyl) aminomethyl group, a (1-benzyloxycarbonyl-2-methylpropyl)aminomethyl group, an anilinomethyl group, a formylaminomethyl group, an acetylaminomethyl group, a 2-acetylaminoethyl group, a propionylaminomethyl group, a butyrylaminomethyl group, an isobutyrylaminomethyl group, a valerylaminomethyl group, a 2-methylbutyrylaminomethyl group, a pivaloylaminomethyl group, a pentylcarbonylaminomethyl group, a s-pentylcarbonylaminomethyl group, a t-pentylcarbonylaminomethyl group, a neopentylcarbonylaminomethyl group, a hexylcarbonylaminomethyl group, a heptylcarbonylaminomethyl group, an octylcarbonylaminomethyl group, a nonylcarbonylaminomethyl group, a decylcarbonylaminomethyl group, an undecylcarbonylaminomethyl group, a dodecylcarbonylaminomethyl group, a tridecylcarbonylaminomethyl group, a tetradecylcarbonylaminomethyl group, a pentadecylcarbonylaminomethyl group, a hexadecylcarbonylaminomethyl group, a cyclopropylcarbonylaminomethyl group, a cyclobutylcarbonylaminomethyl group, a cyclopentylcarbonylaminomethyl group, a cyclohexylcarbonylaminomethyl group, a cycloheptylcarbonylaminomethyl group, a cyclooctylcarbonylaminomethyl group, a trifluoroacetylaminomethyl group, a chloroacetylaminomethyl group, a dichloroacetylaminomethyl group, a trichloroacetylaminomethyl group, a bromoacetylaminomethyl group, an acryloylaminomethyl group, a 1-propenylcarbonylaminomethyl group, a 2-methyl-1-propenylcarbonylaminomethyl group, a 2-methylacryloylaminomethyl group, a 1-methyl-1-propenylcarbonylaminomethyl group, a 1,2-dimethyl-1-propenylcarbonylaminomethyl group, a 2-propenylcarbonylaminomethyl group, a 2-butenylcarbonylaminomethyl group, a 3-methyl-2-butenylcarbonylaminomethyl group, a 2-methyl-2-propenylcarbonylaminomethyl group, a 2-methyl-2-butenylcarbonylaminomethyl group, a 2,3-dimethyl-2-butenylcarbonylaminomethyl group, a 1-cyclobutenylcarbonylaminomethyl group, a 3-cyclobutenylcarbonylaminomethyl group, a 1-cyclopentenylcarbonylaminomethyl group, a 3-cyclopentenylcarbonylaminomethyl group, a 1-cyclohexenylcarbonylaminomethyl group, a 3-cyclohexenylcarbonylaminomethyl group, a 4-cyclohexenylcarbonylaminomethyl group, a propioloylaminomethyl group, a 1-propynylcarbonylaminomethyl group, a propargylcarbonylaminomethyl group, a 2-propargylcarbonylaminoethyl group, a 2-cyano-2-methoxyiminoacetylaminomethyl group, a benzoylaminomethyl group, a 2-benzoylaminoethyl group, a 3-benzoylaminopropyl group, a 2-fluorobenzoylaminomethyl group, a 3-fluorobenzoylaminomethyl group, a 2-(3-fluorobenzoylamino)ethyl group, a 4-fluorobenzoylaminomethyl group, a 2-chlorobenzoylaminomethyl group, a 3-chlorobenzoylaminomethyl group, a 4-chlorobenzoylaminomethyl group, a 2-bromobenzoylaminomethyl group, a 3-bromobenzoylaminomethyl group, a 4-bromobenzoylaminomethyl group, a 2-iodobenzoylaminomethyl group, a 3-iodobenzoylaminomethyl group, a 4-iodobenzoylaminomethyl group, a 2-methylbenzoylaminomethyl group, a 3-methylbenzoylaminomethyl group, a 4-methylbenzoylaminomethyl group, a 2-methoxybenzoylaminomethyl group, a 3-methoxybenzoylaminomethyl group, a 4-methoxybenzoylaminomethyl group, a 2-(4-methoxybenzoylamino)ethyl group, a 2-trifluoromethylbenzoylaminomethyl group, a 3-trifluoromethylbenzoylaminomethyl group, a 4-trifluoromethylbenzoylaminomethyl group, a 2-nitrobenzoylaminomethyl group, a 3-nitrobenzoylaminomethyl group, a 4-nitrobenzoylaminomethyl group, a 2-(4-nitrobenzoylamino)ethyl group, a 2-cyanobenzoylaminomethyl group, a 3-cyanobenzoylaminomethyl group, a 2-(3-cyanobenzoylamino)ethyl group, a 4-cyanobenzoylaminomethyl group, a 2,4-difluorobenzoylaminomethyl group, a 3,5-difluorobenzoylaminomethyl group, a 2,4-dichlorobenzoylaminomethyl group, a 3,5-dichlorobenzoylaminomethyl group, a 2,4-dinitrobenzoylaminomethyl group, a 2-furylcarbonylaminomethyl group, a 2-(2-furylcarbonylamino)ethyl group, a 3-furylcarbonylaminomethyl group, a 2-thienylcarbonylaminomethyl group, a 3-thienylcarbonylaminomethyl group, a 2-oxazolylcarbonylaminomethyl group, a 4-oxazolylcarbonylaminomethyl group, a 2-thiazolylcarbonylaminomethyl group, a 4-thiazolylcarbonylaminomethyl group, a 2-isooxazolylcarbonylaminomethyl group, a 5-methylisooxazol-3-ylcarbonylaminomethyl group, a 2-(5-methylisooxazol-3-ylcarbonylamino)ethyl group, a 5-phenylisooxazol-3-ylcarbonylaminomethyl group, a 1,2,3-thiadiazol-5-ylcarbonylaminomethyl group, a 1,2,3-benzothiadiazol-7-ylcarbonylaminomethyl group, a 2-pyridylcarbonylaminomethyl group, a 3-pyridylcarbonylaminomethyl group, a 2-(3-pyridylcarbonylamino)ethyl group, a 4-pyridylcarbonylaminomethyl group, a 2,6-dichloro-4-pyridylcarbonylaminomethyl group, a 2-(2,6-dichloro-4-pyridylcarbonylamino)ethyl group, a methoxycarbonylaminomethyl group, a 2-methoxycarbonylaminoethyl group, an ethoxycarbonylaminomethyl group, an allyloxycarbonylaminomethyl group, a benzyloxycarbonylaminomethyl group, a phenoxycarbonylaminomethyl group, an ureidomethyl group, a 2-ureidoethyl group, a 3-methylureidomethyl group, a 3-ethylureidomethyl group, a 3-phenylureidomethyl group, a 3-chloroacetylureidomethyl group, a 3-trichloroacetylureidomethyl group, a 3-benzoylureidomethyl group, a 3-ethoxycarbonylureidomethyl group, a 3-(2-cyano-2-methoxyiminoacetyl)ureidomethyl group, a 2-{3-(2-cyano-2-methoxyiminoacetyl)ureido}ethyl group, a methyl(thiocarbonyl)aminomethyl group, a 2-{methyl(thiocarbonyl)amino}ethyl group, an ethyl(thiocarbonyl)aminomethyl group, a phenyl(thiocarbonyl)aminomethyl group, a (methylthio)(thiocarbonyl)aminomethyl group, a 1-(methoxycarbonylamino)ethyl group, a 2-methyl-1-(methoxycarbonylamino)propyl group, a 2-methyl-1-(isopropoxycarbonylamino)propyl group, an α-methoxyiminobenzyl group, a 1-cyano-1-methoxyiminomethyl group, an aminooxymethyl group, an acetylaminooxymethyl group, a propionylaminooxymethyl group, a cyclopropylcarbonylaminooxymethyl group, a benzoylaminooxymethyl group, a 2-cyano-2-methoxyiminoacetylaminooxymethyl group, a methoxycarbonylaminooxymethyl group, an allyloxycarbonylaminooxymethyl group, an ureidooxymethyl group, an N'-methylureidooxymethyl group, an N',N'-dimethylureidooxymethyl group, a methyl(thiocarbonyl)aminooxymethyl group, an N'-methyl(thioureido)oxymethyl group, an N'-acetylureidooxymethyl group, an N'-chloroacetylureidooxymethyl group, an N'-trichloroacetylureidooxymethyl group, an N'-(2-cyano-2-methoxyiminoacetyl)ureidooxymethyl group, an ethylideneiminooxymethyl group, an isopropylideneiminooxymethyl group, a 1-cyclopropylethylideneiminooxymethyl group, an α-methylbenzylideneiminooxymethyl group, a 2-trifluoromethyl-α-methylbenzylideneiminooxymethyl group, a 3-trifluoromethyl-α-methylbenzylideneiminooxymethyl group, a 4-trifluoromethyl-α-methylbenzylideneiminooxymethyl group, an α-methyl-2-methoxybenzylideneiminooxymethyl group, an α-methyl-3-methoxybenzylideneiminoxoymethyl group, an α-methyl-4-methoxybenzylideneiminooxymethyl group, a 1-(5-methyl-2-furyl)ethylideneiminooxymethyl group, a 1-methoxyethylideneiminooxymethyl group, an α-methoxycarbonylbenzylideneiminooxymethyl group, a cyclopentylideneiminooxymethyl group, a tetrahydropyran-4-ylideneiminooxymethyl group, an N,N-phthaloyloxymethyl group, a methylthiomethyl group, a 2-methylthioethyl group, an ethylthiomethyl group, a benzylthiomethyl group, a phenylthiomethyl group, a methoxycarbonylmethylthiomethyl group, an ethoxycarbonylmethylthiomethyl group, a cyanomethylthiomethyl group, a 2-oxazolinylthiomethyl group, a 2-oxazolylthiomethyl group, a 2-benzooxazolylthiomethyl group, a 2-thiazolinylthiomethyl group, a 2-thiazolylthiomethyl group, a 2-benzothiazolylthiomethyl group, a 2-imidazolylthiomethyl group, a 1-methylimidazol-2-ylthiomethyl group, a 2-benzimidazolylthiomethyl group, an imidazo[4,5-b]pyrimidin-2-ylthiomethyl group, a 1,3,4-triazol-2-ylthiomethyl group, a 1-methyl-1,3,4-triazol-2-ylthiomethyl group, a 5-methyl -1,3,4-triazol-2-ylthiomethyl group, a 5-t-butyl-1,3,4-triazol-2-ylthiomethyl group, a 5-cyclohexyl-1,3,4-triazol-2-ylthiomethyl group, a 5-methyl-1,3,4-thiadiazol-2-ylthiomethyl group, a 2-pyridylthiomethyl group, a 3-chloro-2-pyridylthiomethyl group, a 3-trifluoromethyl-2-pyridylthiomethyl group, a 3-nitro-2-pyridylthiomethyl group, a 5-trifluoromethyl-2-pyridylthiomethyl group, a 3-chloro-5-trifluoromethyl-2-pyridylthiomethyl group, a 3-pyridylthiomethyl group, a 4-pyridylthiomethyl group, a 2-pyrimidinylthiomethyl group, a 4-methyl-2-pyrimidinylthiomethyl group, a 4,6-dimethyl-2-pyrimidinylthiomethyl group, a 5-phenyl-2-pyrimidinylthiomethyl group, a methylsulfinylmethyl group, a 2-methylsulfinylethyl group, an ethylsulfinylmethyl group, a benzoylsulfinylmethyl group, a phenylsulfinylmethyl group, a cyanomethylsulfinylmethyl group, a 2-pyridylsulfinylmethyl group, a 3-pyridylsulfinylmethyl group, a 4-pyridylsulfinylmethyl group, a 2-pyrimidinylsulfinylmethyl group, a methylsulfonylmethyl group, a 2-methylsulfonylethyl group, an ethylsulfonylmethyl group, a benzylsulfonylmethyl group, a phenylsulfonylmethyl group, a cyanomethylsulfonylmethyl group, a 2-pyridylsulfonylmethyl group, a 3-pyridylsulfonylmethyl group, a 4-pyridylsulfonylmethyl group, a 2-pyrimidinylsulfonylmethyl group, a dimethoxythiophosphorylthiomethyl group, a diethoxythiophosphorylthiomethyl group, a methoxy group, an ethoxy group, a propoxy group, an isopropoxy group, a butoxy group, an isobutoxy group, a s-butoxy group, a t-butoxy group, a pentoxy group, a s-pentoxy group, a hexyloxy group, an octyloxy group, a cyclopentoxy group, a cyclohexyloxy group, an allyloxy group, a phenoxy group, a 3-fluorophenoxy group, a 4-fluorophenoxy group, a 3-chlorophenoxy group, a 4-chlorophenoxy group, a 3-bromophenoxy group, a 4-bromophenoxy group, a 3-methylphenoxy group, a 4-methylphenoxy group, a 3-methoxyphenoxy group, a 4-methoxyphenoxy group, a 3-trifluoromethylphenoxy group, a 4-trifluoromethylphenoxy group, a 2-nitrophenoxy group, a 3-nitrophenoxy group, a 4-nitrophenoxy group, a 2-cyanophenoxy group, a 3-cyanophenoxy group, a 4-cyanophenoxy group, a 2,4-difluorophenoxy group, a 3,5-difluorophenoxy group, a 2,4-dichlorophenoxy group, a 3,5-dichlorophenoxy group, a 4,6-dimethylpyrimidin-2-yloxy group, a 3-isooxazolyloxy group, a 5-methylisooxazol-3-yloxy group, a 3-oxo-5-methyl-2(3H)-isooxazolyloxy group, a benzyloxy group, an amino group, a methylamino group, an ethylamino group, a propylamino group, an isopropylamino group, a butylamino group, an isobutylamino group, a s-butylamino group, a t-butylamino group, a pentylamino group, a s-pentylamino group, a hexylamino group, an octylamino group, a cyclopentylamino group, a cyclohexylamino group, an allylamino group, a carboxylmethylamino group, a methoxycarbonylmethylamino group, an ethoxycarbonylmethylamino group, a carbamoylmethylamino group, a cyanomethylamino group, a 1-carboxyl-2-methylpropylamino group, a 1-methoxycarbonyl -2-methylpropylamino group, a 1-phenoxycarbonyl-2-methylpropylamino group, a 1-benzyloxycarbonyl-2-methylpropylamino group, a 1-(α-methylbenzylcarbamoyl)-2-methylpropylamino group, a 1-(3,4-dimethoxyphenethylcarbamoyl)-2-methylpropylamino group, an anilino group, a 3-fluoroanilino group, a 4-fluoroanilino group, a 3-chloroanilino group, a 4-chloroanilino group, a 3-bromoanilino group, a 4-bromoanilino group, a 3-methylanilino group, a 4-methylanilino group, a 3-methoxyanilino group, a 4-methoxyanilino group, a 3-trifluoromethylanilino group, a 4-trifluoromethylanilino group, a 2-nitroanilino group, a 3-nitroanilino group, a 4-nitroanilino group, a 2-cyanoanilino group, a 3-cyanoanilino group, a 4-cyanoanilino group, a 2,4-difluoroanilino group, a 3,5-difluoroanilino group, a 2,4-dichloroanilino group, a 3,5-dichloroanilino group, a benzylamino group, a dimethylamino group, a diisopropylamino group, an N-methylanilino group, a 1-pyrrolidinyl group, a 1-imidazolyl group, a 1-pyrazolyl group, a piperidino group, a morpholino group, an acetylamino group, a chloroacetylamino group, a trichloroacetylamino group, a benzoylamino group, a 2-cyano-2-methoxyiminoacetylamino group, a bis(2-cyano-2-methoxyiminoacetyl)amino group, a methylthio group, an ethylthio group, a propylthio group, an isopropylthio group, a butylthio group, an isobutylthio group, a s-butylthio group, a t-butylthio group, a cyclopentylthio group, a cyclohexylthio group, an allylthio group, a phenylthio group, a 4-fluorophenylthio group, a 4-chlorophenylthio group, a 4-methylphenylthio group, a 3-methoxyphenylthio group, a 4-trifluoromethylphenylthio group, a 3-nitrophenylthio group, a 4-nitrophenylthio group, a 2-cyanophenylthio group, a 3-cyanophenylthio group, a 4-cyanophenylthio group, a 3,5-difluorophenylthio group, or a benzylthio group, or Ar and B can be taken together to be 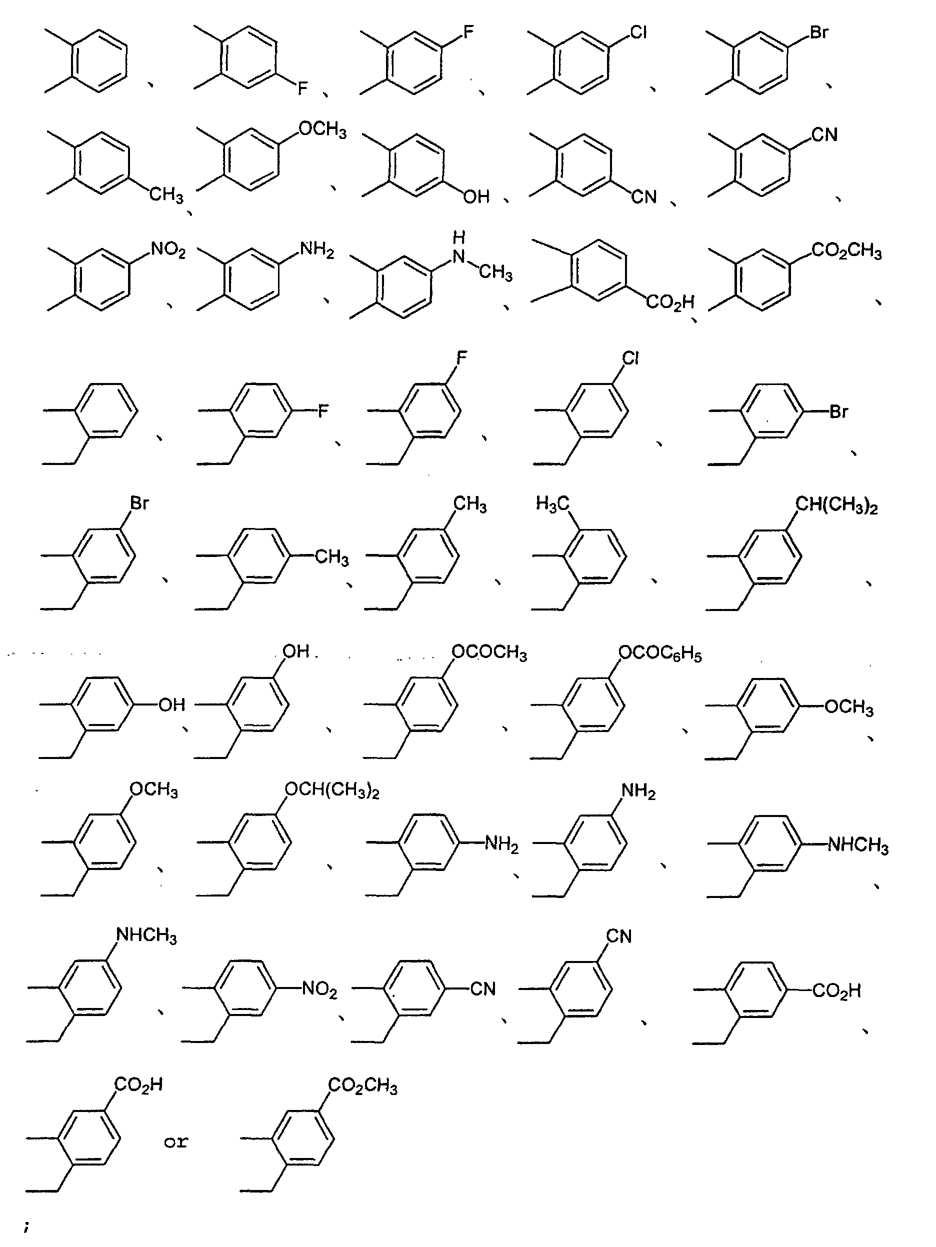 more preferably, Ar is an optionally substituted C₆-C₁₀ aryl group [the substituent(s) is (are) 1-3 same or different substituent(s) selected from the group consisting of a fluorine atom, a chlorine atom, a bromine atom, an iodine atom, a C₁-C₃ alkyl group, a halo(C₁-C₃ alkyl) group (the halogen substituent(s) is (are) 1-2 same or different substituent(s) selected from the group consisting of a fluorine atom, a chlorine atom and a bromine atom), a cyano(C₁-C₃ alkyl) group, a hydroxy(C₁-C₃ alkyl) group, a (C₁-C₃ alkylthio)(C₁-C₃ alkyl) group, a (C₁-C₃ alkylsulfonyl) (C₁-C₃ alkyl), an imino(C₁-C₃ alkyl) group wherein the alkyl part is substituted (substituent(s) being C₁-C₃ alkoxy group), a (C₁-C₃ alkylimino) (C₁-C₃ alkyl) group wherein the alkyl part is substituted {substituent(s) being C₁-C₃ alkylthio group or C₇-C₈ aralkylthio group wherein the aryl part may be substituted (substituent(s) being cyano group)}, a hydroxyimino(C₁-C₃ alkyl) group wherein the alkyl part is substituted (substituent(s) being amino group), an optionally substituted C₂-C₅ alkenyl group (the substitutent(s) is (are) 1-2 different substituent(s) selected from the group consisting of a C₁-C₃ alkoxy group and a C₂-C₄ alkoxycarbonyl group), a C₂-C₄ alkylcarbonyl group, a carboxyl group, a C₂-C₄ alkoxycarbonyl group, an optionally substituted carbamoyl group {substituent(s) being C₁-C₃ alkyl group, cyano(C₁-C₃ alkyl) group, C₂-C₄ alkylcarbonyl group or optionally substituted phenyl group (substituent(s) being C₁-C₃ alkoxy group)}, an optionally substituted thiocarbamoyl group (substituent(s) being C₁-C₃ alkyl group), a cyano group, a phenyl group, an optionally substituted 5 membered unsaturated heterocyclic group {substituent(s) being C₁-C₃ alkyl group or halo(C₁-C₃ alkyl) group (the halogen substituent(s) is (are) 1-3 same or different substituent(s) selected from the group consisting of a fluorine atom, a chlorine atom and a bromine atom); the heterocycle contains an oxygen atom, a sulfur atom or a nitrogen atom, and contains 1-2 further nitrogen atom(s)}, a hydroxyl group, a C₁-C₃ alkoxy group, a halo(C₁-C₃ alkoxy) group (the halogen substituent(s) is (are) 1-5 same or different substituent(s) selected from the group consisting of a fluorine atom, a chlorine atom and a bromine atom), a halo(C₂-C₄ alkenyloxy) group (the halogen substituent(s) is (are) 1-2 same or different substituent(s) selected from the group consisting of a fluorine atom, a chlorine atom, a bromine atom and an iodine atom), an optionally substituted phenoxy group (substituent(s) being C₁-C₃ alkyl group), a C₁-C₃ alkylthio group, a halo(C₁-C₃ alkylthio) group (the halogen substituent(s) is (are) 1-3 same or different substituent(s) selected from the group consisting of a fluorine atom, a chlorine atom and a bromine atom), a phenylthio group, a C₁-C₃ alkylsulfinyl group, a phenylsulfinyl group, a C₁-C₃ alkylsulfonyl group, a halo(C₁-C₃ alkylsulfonyl) group (the halogen substituent(s) is (are) 1-3 same or different substituent(s) selected from the group consisting of a fluorine atom, a chlorine atom and a bromine atom), a phenylsulfonyl group, an azido group, a nitro group and an optionally substituted amino group {the substituent(s) is (are) 1-2 same or different substituent(s) selected from the group consisting of a C₁-C₃ alkyl group, a C₇-C₈ aralkyl group, a formyl group, a C₂-C₅ alkylcarbonyl group, a (C₂-C₄ alkoxycarbonylamino) (C₂-C₅ alkylcarbonyl) group, a C₂-C₄ alkoxycarbonyl group, a C₂-C₄ alkyl (thiocarbonyl) group, an optionally substituted carbamoyl group (substituent(s) being C₁-C₃ alkyl group) and a C₁-C₃ alkylsulfonyl group}] or an optionally substituted 5 membered unsaturated heterocyclic group (the substituent(s) is (are) 1-2 same or different substituent(s) selected from the group consisting of a fluorine atom, a chlorine atom, a bromine atom, an iodine atom, a C₁-C₃ alkyl group, a hydroxyl group, a C₁-C₃ alkoxy group, a cyano group, a nitro group and an amino group; the heterocycle contains a sulfur atom or a nitrogen atom, and may contain one further nitrogen atom, and the heterocycle may be fused with a benzene ring);
   B is a chlorine atom, a bromine atom, a C₁-C₁₅ alkyl group, a halo(C₁-C₃ alkyl) group (the halogen substituent(s) is (are) 1-3 same or different substituent(s) selected from the group consisting of a fluorine atom, a chlorine atom and a bromine atom), a (C₂-C₄ alkoxycarbonyl)(C₁-C₃ alkyl) group, a cyano(C₁-C₃ alkyl) group, a (C₃-C₆ cycloalkyl)(C₁-C₃ alkyl) group, a (C₄-C₆ cycloalkenyl) (C₁-C₃ alkyl) group, a C₇-C₈ aralkyl group, a (5-6 membered saturated heterocyclyl) (C₁-C₃ alkyl) group (the heterocycle contains 1-2 same or different heteroatom(s) selected from the group consisting of an oxygen atom and a nitrogen atom), a (5-6 membered unsaturated heterocyclyl) (C₁-C₃ alkyl) group wherein the heterocycle part may be substituted {the substituent(s) is (are) 1-3 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a C₂-C₄ alkoxycarbonyl group, a cyano group, a nitro group and an amino group; the heterocycle contains an oxygen atom, a sulfur atom or a nitrogen atom, and may contain 1-2 further nitrogen atom(s), the heterocycle may be fused with a benzene ring, and the heterocycle may be substituted with one oxo group}, a hydroxy(C₁-C₃ alkyl) group, a (C₁-C₃ alkoxy) (C₁-C₃ alkyl) group, a (C₇-C₈ aralkyloxy) (C₁-C₃ alkyl) group wherein the aryl part may be substituted {substituent(s) being halo(C₁-C₃ alkyl) group (the halogen substituent(s) is (are) 1-3 same or different substituent(s) selected from the group consisting of a fluorine atom, a chlorine atom and a bromine atom), cyano group or nitro group}, a (5-6 membered unsaturated heterocyclyl) (C₁-C₆ alkoxy) (C₁-C₆ alkyl) group
   wherein the heterocycle part may be substituted (the substitutent(s) is (are) 1-2 same or different substituent(s) selected from the group consisting of a fluorine atom, a chlorine atom, a bromine atom and a C₁-C₃ alkyl group; the heterocycle contains an oxygen atom, a sulfur atom or a nitrogen atom, and may contain one further nitrogen atom), a cyano(C₁-C₃ alkoxy) (C₁-C₃ alkyl) group, a (C₁-C₃ alkoxy) (C₁-C₃ alkoxy) (C₁-C₃ alkyl) group, a (C₇-C₈ aralkyloxy) (C₁-C₃ alkoxy) (C₁-C₃ alkyl) group, a (C₁-C₆ alkoxy) (C₁-C₆ alkoxy) (C₁-C₆ alkoxy) (C₁-C₆ alkyl) group, a tri (C₁-C₃ alkyl)silyl(C₁-C₃ alkoxy) (C₁-C₃ alkoxy) (C₁-C₃ alkyl) group, a (C₂-C₄ alkenyloxy) (C₁-C₃ alkyl) group, a (C₂-C₃ alkynyloxy)(C₁-C₃ alkyl) group, a phenoxy(C₁-C₃ alkyl) group wherein the phenyl part may be substituted {the substituent(s) is (are) 1-2 same or different substituent(s) selected from the group consisting of a flue atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a C₁-C₃ alkoxy group, a cyano group and a nitro group}, a (5-6 membered saturated heterocyclyl)oxy(C₁-C₃ alkyl) group (the heterocycle contains 1-2 same or different heteroatom(s) selected from the group consisting of an oxygen atom and a nitrogen atom), a (5-6 membered unsaturated heterocyclyl)oxy(C₁-C₃ alkyl) group wherein the heterocycle part may be substituted {the substituent(s) is (are) 1-4 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a C₂-C₄ alkoxycarbonyl group, a cyano group, a nitro group, an amino group and a phenyl group; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring, and the heterocycle may be substituted with 1-2 oxo group(s)}, a tri(C₁-C₄ alkyl)silyloxy (C₁-C₃ alkyl) group, a di(C₁-C₃ alkyl)(monophenyl)silyloxy(C₁-C₃ alkyl) group, a mono(C₁-C₄ alkyl)(diphenyl)silyloxy(C₁-C₃ alkyl) group, a formyloxy(C₁-C₃ alkyl) group, a (C₂-C₅ alkylcarbonyloxy)(C₁-C₃ alkyl) group, a (C₅-C₆ cycloalkenyl)(C₂-C₄ alkylcarbonyloxy) (C₁-C₃ alkyl) group, a (C₈-C₉ aralkylcarbonyloxy)(C₁-C₃ alkyl) group, a (5-6 membered unsaturated heterocyclyl)(C₁-C₆ alkylcarbonyloxy)(C₁-C₆ alkyl) group wherein the heterocycle part may be substituted (substituent(s) being C₁-C₃ alkyl group; the heterocycle contains an oxygen atom, a sulfur atom or a nitrogen atom, and may contain one further nitrogen atom, and the heterocycle may be fused with a benzene ring), a C₁-C₃ alkoxy (C₂-C₄ alkylcarbonyloxy) (C₁-C₃ alkyl) group, a phenoxy(C₂-C₄ alkylcarbonyloxy) (C₁-C₃ alkyl) group, a (C₂-C₄ alkylcarbonyloxy) (C₂-C₄ alkylcarbonyloxy) (C₁-C₃ alkyl) group, a (C₄-C₇ cycloalkylcarbonyloxy) (C₁-C₃ alkyl) group, a (C₄-C₆ alkenylcarbonyloxy) (C₁-C₃ alkyl) group, a phenyl(C₄-C₆ alkenylcarbonyloxy) (C₁-C₃ alkyl) group, a (C₆-C₇ cycloalkenylcarbonyloxy)(C₁-C₃ alkyl) group, a cyclohexadienylcarbonyloxy(C₁-C₃ alkyl) group wherein the cyclohexadiene part may be substituted (substituent(s) being C₁-C₃ alkyl group), a benzoyloxy(C₁-C₃ alkyl) group wherein the phenyl part may be substituted (the substituent(s) is (are) 1-2 substituent(s) selected from the group consisting of a fluorine atom, a chlorine atom, a bromine atom, a C₁-C₃ alkyl group, a C₁-C₃ alkoxy group and a cyano group), a (5-6 membered unsaturated heterocyclyl)carbonyloxy(C₁-C₃ alkyl) group wherein the heterocycle part may be substituted {the substituent(s) is (are) 1-3 same or different substituent(s) selected from the group consisting of a fluorine atom, a chlorine atom, a bromine atom, a C₁-C₄ alkyl group, a halo(C₁-C₃ alkyl) group (the halogen substituent(s) is (are) 1-3 same or different substituent(s) selected from the group consisting of a fluorine atom, a chlorine atom and a bromine atom), a C₁-C₃ alkoxy group and a phenyl group; the heterocycle contains an oxygen atom, a sulfur atom or a nitrogen atom, and may contain 1-2 further nitrogen atom(s), the heterocycle may be fused with a benzene ring, and the heterocycle may be substituted with one oxo group}, a (C₂-C₅ alkoxycarbonyloxy) (C₁-C₃ alkyl) group, a (C₄-C₅ alkenyloxycarbonyloxy)(C₁-C₃ alkyl) group, a phenoxycarbonyloxy(C₁-C₃ alkyl) group, a carbamoyloxy(C₁-C₃ alkyl) group wherein the carbamoyl part may be substituted (the substituent(s) is (are) 1-2 same or different C₁-C₃ alkyl group(s)), a thiocarbamoyloxy(C₁-C₃ alkyl) group wherein the thiocarbamoyl group may be substituted (the substituent(s) is (are) 1-2 same or different C₁-C₃ alkyl group(s)), a [C₂-C₅ (alkylthio)carbonyloxy] (C₁-C₃ alkyl) group, an aminooxy(C₁-C₃ alkyl) group wherein the amino part may be substituted [substituent(s) being C₄-C₆ cycloalkylcarbonyl group, C₂-C₄ alkoxycarbonyl group, C₄-C₅ alkenyloxycarbonyl group, optionally substituted carbamoyl group (the substituent(s) is (are) 1-2 same or different substituent(s) selected from the group consisting of a C₁-C₃ alkyl group and a (C₁-C₃ alkoxyimino) (C₂-C₄ alkylcarbonyl) group wherein the alkyl part is cyano-substituted), optionally substituted C₁-C₆ alkylidene group << the substituent(s) is (are) 1-2 same or different substituent(s) selected from the group consisting of a C₃-C₆ cycloalkyl group, a C₂-C₄ alkoxycarbonyl group, an optionally substituted phenyl group {substituent(s) being halo(C₁-C₃ alkyl) group (the halogen substituent(s) is (are) 1-3 same or different substituent(s) selected from the group consisting of fluorine atom, a chlorine atom and a bromine atom) or C₁-C₃ alkoxy group}, an optionally substituted 5-6 membered unsaturated heterocyclic group (substituent(s) being C₁-C₃ alkyl group; the heterocycle contains an oxygen atom, a sulfur atom or a nitrogen atom, and may contain one further nitrogen atom, and the heterocycle may be fused with a benzene ring) and a C₁-C₃ alkoxy group » or C₅-C₆ cycloalkylidene group (an alkylene chain of the cycloalkylidene group may be interrupted by one oxygen atom)], a di (C₁-C₃ alkoxy) phosphoryloxy (C₁-C₃ alkyl) group, a di (C₁-C₃ alkoxy) thiophosphoryloxy(C₁-C₃ alkyl) group, a (C₁-C₃ alkylsulfonyloxy) (C₁-C₃ alkyl) group, a phenylsulfonyloxy(C₁-C₃ alkyl) group wherein the phenyl part may be substituted (substituent (s) being C₁-C₃ alkyl group), a (C₁-C₆ alkylthio) (C₁-C₃ alkyl) group wherein the alkyl part of the alkylthio part may be substituted (substituent(s) being C₂-C₄ alkoxycarbonyl group or cyano group), a phenylthio(C₁-C₃ alkyl) group, a (5-6 membered unsaturated heterocyclyl)thio(C₁-C₃ alkyl) group wherein the heterocycle part may be substituted {the substituent(s) is (are) 1-2 same or different substituent(s) selected from a fluorine atom, a chlorine atom, a bromine atom, a C₁-C₄ alkyl group, a halo(C₁-C₃ alkyl) group (the halogen substituent(s) is (are) 1-3 same or different substituent(s) selected from the group consisting of a fluorine atom, a chlorine atom and a bromine atom), a C₃-C₆ cycloalkyl group, a nitro group and a phenyl group; the heterocycle contains an oxygen atom, a sulfur atom or a nitrogen atom, and may contain 1-2 further nitrogen atom(s), and the heterocycle may be fused with a benzene ring or a pyridine ring}, a (C₁-C₆ alkylsulfinyl) (C₁-C₃ alkyl) group, a phenylsulfinyl(C₁-C₃ alkyl) group, a (5-6 membered unsaturated heterocyclyl)sulfinyl(C₁-C₃ alkyl) group (the heterocycle contains an oxygen atom, a sulfur atom or a nitrogen atom, and may contain 1-2 further nitrogen atom(s), and the heterocycle may be fused with a benzene ring or a pyridine ring), a (C₁-C₆ alkylsulfonyl) (C₁-C₃ alkyl) group, a phenylsulfonyl(C₁-C₃ alkyl) group, a (5-6 membered unsaturated heterocyclyl)sulfonyl(C₁-C₃ alkyl) group (the heterocycle contains an oxygen atom, a sulfur atom or a nitrogen atom, and may contain 1-2 further nitrogen atom(s), and the heterocycle may be fused with a benzene ring or a pyridine ring), a di(C₁-C₃ alkoxy)thiophosphorylthio(C₁-C₃ alkyl) group, an amino(C₁-C₄ alkyl) group wherein the amino part may be substituted {substituent(s) being C₁-C₃ alkyl group, carboxyl(C₁-C₄ alkyl) group, (C₂-C₄ alkoxycarbonyl)(C₁-C₄ alkyl) group, (C₈-C₉ aralkyloxycarbonyl) (C₁-C₄ alkyl) group, formyl group, (C₁-C₃ alkoxyimino) (C₂-C₄ alkylcarbonyl) group wherein the alkyl part is cyano-substituted, C₂-C₅ alkoxycarbonyl group, C₈-C₁₀ aralkyloxycarbonyl group or optionally substituted carbamoyl group (substituent(s) being C₁-C₃ alkyl group or (C₁-C₃ alkoxyimino)(C₂-C₄ alkylcarbonyl) group wherein the alkyl part is cyano-substituted)}, an anilino(C₁-C₃ alkyl) group, a (C₁-C₃ alkoxyimino)(C₁-C₃ alkyl) group wherein the alkyl part may be substituted (substituent(s) being phenyl group or cyano group), a C₃-C₆ cycloalkyl group, a C₃-C₅ alkenyl group, a phenyl(C₃-C₅ alkenyl) group, an N-(cyano-aralkyl)-N-{1,5-di(C₁-C₄ alkyl)-4-pyrazolyl}aminocarbonyl(C₂-C₆ alkenyl) group, a C₅-C₆ cycloalkenyl group, an optionally substituted phenyl group (the substituent(s) is (are) 1-2 same or different substituent(s) selected from the group consisting of a fluorine atom, a chlorine atom and a bromine atom), a 5-6 membered saturated heterocyclic group (the heterocycle contains 1-2 same or different heteroatom(s) selected from the group consisting of an oxygen atom and a nitrogen atom), an optionally substituted 5-6 membered unsaturated heterocyclic group (the substituent(s) is (are) 1-2 same or different substituent(s) selected from the group consisting of a fluorine atom, a chlorine atom, a bromine atom, and a C₁-C₆ alkyl group; the heterocycle contains an oxygen atom, a sulfur atom or a nitrogen atom, and may contain 1-2 further nitrogen atom(s), and the heterocycle may be fused with a benzene ring), a C₁-C₄ alkoxy group, an optionally substituted phenoxy group {the substituent(s) is (are) 1-2 same or different substituent(s) selected from the group consisting of a fluorine atom, a chlorine atom, a bromine atom, a straight or branched alkyl group having a carbon number of 1-3, a straight or branched haloalkyl group having a carbon number of 1-3 (the halogen substituent(s) is (are) 1-3 same or different halogen atom(s) selected from the group consisting of a fluorine atom, a chlorine atom and a bromine atom), an alkoxy group having a carbon number of 1-3, a cyano group and a nitro group}, an optionally substituted 5-6 membered unsaturated (heterocyclyl)oxy group {the substituent(s) is (are) 1-4 same or different substituent(s) selected from the group consisting of a fluorine atom, a chlorine atom, a bromine atom, a straight or branched alkyl group having a carbon number of 1-3, a straight or branched haloalkyl group having a carbon number of 1-3 (the halogen substituent(s) is (are) 1-3 same or different halogen atom(s) selected from the group consisting of a fluorine atom, a chlorine atom and a bromine atom), a straight or branched alkoxy group having a carbon number of 1-3, a carbamoyl group, a straight or branched alkoxycarbonyl group having a carbon number of 2-4, a cyano group, a nitro group, an amino group and a phenyl group; the heteroatom of the heterocycle is an oxygen atom, a sulfur atom or a nitrogen atom, and may be 1-2 further nitrogen atom(s), two oxygen atoms may bind to the sulfur atom, and the heterocycle may be fused with a benzene ring, and may be substituted with 1-2 oxo group(s)}, a C₁-C₃ alkylthio group, an optionally substituted amino group [the substituent(s) is (are) 1-2 same or different substituent(s) selected from the group consisting of a C₁-C₃ alkyl group, a cyano(C₁-C₃ alkyl) group, a carboxyl (C₁-C₄ alkyl) group, a (C₂-C₄ alkoxycarbonyl) (C₁-C₄ alkyl) group, a (C₈-C₉ aralkyloxycarbonyl) (C₁-C₄ alkyl) group, a carbamoyl (C₁-C₆ alkyl) group wherein the carbamoyl part may be substituted {substituent(s) being optionally substituted C₇-C₈ aralkyl group (the substituent(s) is (are) 1-2 same or different C₁-C₃ alkoxy group(s))} and a (C₁-C₆ alkoxyimino) (C₂-C₇ alkylcarbonyl) group wherein the alkyl part is cyano-substituted] or an anilino group; or Ar and B are taken together to be an optionally substituted o-phenylene group (substituent(s) being fluorine atom, chlorine atom, bromine atom, hydroxyl group, cyano group, nitro group or amino group, and one methylene group is inserted between the phenylene group and the carbon atom to which B binds); more preferably Ar is an optionally substituted phenyl group {the substituent(s) is (are) 1-2 same or different substituent(s) selected from the group consisting of a fluorine atom, a chlorine atom, a bromine atom, an iodine atom, a methyl group, a C₁-C₂ alkylthiomethyl group, an iminomethyl group wherein the alkyl part is substituted (substituent(s) being C₁-C₂ alkoxy group), a carbamoyl group, a cyano group, a 1,2,4-oxadiazolyl group, a C₁-C₂ alkoxy group, a halo(C₁-C₂ alkoxy) group (the halogen substituent(s) is (are) 1-3 fluorine atom(s)), an optionally substituted phenoxy group (substituent(s) being methyl group), a C₁-C₂ alkylthio group, a C₁-C₂ alkylsulfinyl group, a phenylsulfinyl group, a C₁-C₂ alkylsulfonyl group, a phenylsulfonyl group, an azido group, a nitro group and an optionally substituted amino group (substituent(s) being methyl group or C₇-C₈ aralkyl group)}; B is a chlorine atom, a C₁-C₇ alkyl group, a halo(C₁-C₂ alkyl) group (halogen substituent(s) being fluorine atom or chlorine atom), a (C₂-C₃ alkoxycarbonyl)(C₁-C₂ alkyl) group, a benzyl group, a morpholyl(C₁-C₂ alkyl) group, a (5-6 membered unsaturated heterocyclyl)(C₁-C₃ alkyl) group wherein the heterocycle part may be substituted (the substituent(s) is (are) 1-2 same or different substituent(s) selected from the group consisting of a chlorine atom, a bromine atom, a methyl group, a halo(C₁-C₂ alkyl) group (the halogen substituent(s) is (are) 1-3 fluorine atom(s)), a C₂-C₃ alkoxycarbonyl group and a nitro group; the heterocycle contains an oxygen atom or a nitrogen atom, and may contain 1-2 further nitrogen atom(s), the heterocycle may be fused with a benzene ring, and the heterocycle may be substituted with one oxo group), a hydroxy(C₁-C₃ alkyl) group, a (C₁-C₃ alkoxy) (C₁-C₂ alkyl) group, a benzyloxy(C₁-C₂ alkyl) group wherein the phenyl part may be substituted {substituent(s) being halo(C₁-C₂ alkyl) group (the halogen substituent(s) is (are) 1-3 fluorine atom(s)), cyano group or nitro group}, a (5-6 membered unsaturated heterocyclyl) (C₁-C₆ alkoxy) (C₁-C₆ alkyl) group wherein the heterocycle part may be substituted (the substituent(s) is (are) 1-2 same or different substituent(s) selected from the group consisting of a fluorine atom, a chlorine atom, a bromine atom and a methyl group; the heterocycle contains an oxygen atom or a nitrogen atom, and may contain one further nitrogen atom), a cyano(C₁-C₃ alkoxy) (C₁-C₂ alkyl) group, a (C₁-C₂ alkoxy) (C₁-C₂ alkoxy) (C₁-C₂ alkyl) group, a benzyloxy(C₁-C₂ alkoxy) (C₁-C₂ alkyl) group, a trimethylsilyl (C₁-C₂ alkoxy) (C₁-C₂ alkoxy) (C₁-C₂ alkyl) group, a (C₃-C₄ alkenyloxy) (C₁-C₂ alkyl) group, a propynyloxy(C₁-C₂ alkyl) group, a phenoxy(C₁-C₂ alkyl) group wherein the phenyl part may be substituted {the substituent(s) is (are) 1-2 same or different substituent(s) selected from the group consisting of a fluorine atom, a chlorine atom, a methyl group, a halo(C₁-C₂ alkyl) group (the halogen substituent(s) is (are) 1-3 fluorine atom(s)), a methoxy group and a nitro group}, a (5-6 membered unsaturated heterocyclyl)oxy(C₁-C₂ alkyl) group wherein the heterocycle part may be substituted (the substituent(s) is (are) 1-4 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same substituent(s) selected from the group consisting of a fluorine atom, a chlorine atom and a bromine atom), a nitro group and an amino group; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring, and the heterocycle may be substituted with 1-2 oxo group(s)), a tri(C₁-C₄ alkyl)silyloxy(C₁-C₂ alkyl) group, a dimethylmonophenylsilyloxy(C₁-C₂ alkyl) group, a (C₂-C₅ alkylcarbonyloxy) (C₁-C₃ alkyl) group, a cyclopentenyl(C₂-C₃ alkylcarbonyloxy) (C₁-C₂ alkyl) group, a benzylcarbonyloxy(C₁-C₂ alkyl) group, a (5-6 membered unsaturated heterocyclyl)(C₁-C₆ alkylcarbonyloxy)(C₁-C₆ alkyl) group wherein the heterocycle part may be substituted (substituent(s) being methyl group; the heterocycle contains an oxygen atom, a sulfur atom or a nitrogen atom, and may contain one further nitrogen atom, and the heterocycle may be fused with a benzene ring), a (C₁-C₂ alkoxy) (C₂-C₃ alkylcarbonyloxy) (C₁-C₂ alkyl) group, a phenoxy(C₂-C₃ alkylcarbonyloxy) (C₁-C₂ alkyl) group, a (C₂-C₃ alkylcarbonyloxy) (C₂-C₃ alkylcarbonyloxy) (C₁-C₂ alkyl) group, a (C₄-C₇ cycloalkylcarbonyloxy) (C₁-C₂ alkyl) group, a (C₄-C₆ alkenylcarbonyloxy) (C₁-C₂ alkyl) group, a phenyl(C₄-C₆ alkenylcarbonyloxy) (C₁-C₂ alkyl) group, a 1,4-cyclohexadienylcarbonyloxy(C₁-C₂ alkyl) group wherein the cyclohexadiene part may be substituted (substituent(s) being methyl group), a benzoyloxy(C₁-C₂ alkyl) group wherein the phenyl part may be substituted (the substituent(s) is (are) 1-2 same or different substituent(s) selected from the group consisting of a fluorine atom, a chlorine atom, a methyl group, a methoxy group and a cyano group), a (5-6 membered unsaturated heterocyclyl)carbonyloxy(C₁-C₃ alkyl) group wherein the heterocycle part may be substituted {the substituent(s) is (are) 1-3 same or different substituent(s) selected from the group consisting of a fluorine atom, a chlorine atom, a C₁-C₄ alkyl group, a halo(C₁-C₂ alkyl) group (the halogen substituent(s) is (are) 1-3 fluorine atom(s)) and a methoxy group; the heterocycle contains an oxygen atom, a sulfur atom or a nitrogen atom, and the heterocycle may be fused with a benzene ring}, a (C₂-C₅ alkoxycarbonyloxy) (C₁-C₂alkyl) group, an allyloxycarbonyloxy(C₁-C₂ alkyl) group, a phenoxycarbonyloxy(C₁-C₂ alkyl) group, a carbamoyloxy(C₁-C₂ alkyl) group wherein the carbamoyl part may be substituted (the substituent(s) is (are) 1-2 same or different C₁-C₂ alkyl group(s)), a thiocarbamoyloxy(C₁-C₂ alkyl) group wherein the thiocarbamoyl part may be substituted (the substituent(s) is (are) 1-2 same or different C₁-C₂ alkyl group(s)), a C₂-C₃ (alkylthio) carbonyloxy (C₁-C₂ alkyl) group, an aminooxy(C₁-C₂ alkyl) group wherein the amino part may be substituted [substituent(s) being optionally substituted carbamoyl group (the substituent(s) is (are) 1-2 same or different C₁-C₂ alkyl group(s)), optionally substituted C₁-C₃ alkylidene group «the substituent(s) is (are) 1-2 same or different substituent(s) selected from the group consisting of a C₂-C₃ alkoxycarbonyl group, an optionally substituted phenyl group {substituent(s) being halo (C₁-C₂ alkyl) group (the halogen substituent(s) is (are) 1-3 fluorine atom(s)) or methoxy group}, an optionally substituted 5-6 membered unsaturated heterocyclic group (substituent(s) being methyl group; the heterocycle contains an oxygen atom, a sulfur atom or a nitrogen atom, and the heterocycle may be fused with a benzene ring) and a methoxy group>> or C₅-C₆ cycloalkylidene group], a di (C₁-C₂ alkoxy)phosphoryloxy(C₁-C₂ alkyl) group, a di (C₁-C₂ alkoxy) thiophosphoryloxy (C₁-C₂ alkyl) group, a (C₁-C₂ alkylsulfonyloxy) (C₁-C₂ alkyl) group, a phenylsulfonyloxy(C₁-C₂ alkyl) group wherein the phenyl part may be substituted (substituent(s) being methyl group), a (C₁-C₃ alkylthio) (C₁-C₂ alkyl) group wherein an alkyl part of the alkylthio part may be substituted (substituent(s) being C₂-C₃ alkoxycarbonyl group or cyano group), a phenylthio(C₁-C₂ alkyl) group, a (5-6 membered unsaturated heterocyclyl)thio(C₁-C₂ alkyl) group wherein the heterocycle part may be substituted {the substituent(s) is (are) 1-2 same or different substituent(s) selected from the group consisting of a fluorine atom, a chlorine atom, a C₁-C₄ alkyl group, a halo(C₁-C₂ alkyl) group (the halogen substituent(s) is (are) 1-3 fluorine atom(s)), a cyclohexyl group, a nitro group and a phenyl group; the heterocycle contains an oxygen atom, a sulfur atom or a nitrogen atom, and the heterocycle may be fused with a benzene ring or a pyridine ring}, a (C₁-C₃ alkylsulfinyl) (C₁-C₂ alkyl) group, a phenylsulfinyl(C₁-C₂ alkyl) group, a (6 membered unsaturated heterocyclyl) sulfinyl(C₁-C₂ alkyl) group (the heterocycle contains 1-2 nitrogen atom(s)), a (C₁-C₃ alkylsulfonyl) (C₁-C₂ alkyl) group, a phenylsulfonyl(C₁-C₂ alkyl) group, a (6 membered unsaturated heterocyclyl)sulfonyl(C₁-C₂ alkyl) group (the heterocycle contains 1-2 nitrogen atom(s)), a di(C₁-C₂ alkoxy)thiophosphorylthio(C₁-C₂ alkyl) group, an amino(C₁-C₂ alkyl) group wherein the amino part may be substituted {substituent(s) being methyl group, carboxyl(C₁-C₄ alkyl) group, (C₂-C₃ alkoxycarbonyl) (C₁-C₄ alkyl) group, benzyloxycarbonyl (C₁-C₄ alkyl) group, (C₁-C₂ alkoxyimino)methylcarbonyl group wherein the methyl part is cyano-substituted, C₂-C₃ alkoxycarbonyl group, benzyloxycarbonyl group or optionally substituted carbamoyl group (substituent(s) being C₁-C₂ alkyl group or (C₁-C₂ alkoxyimino)methylcarbonyl group wherein the methyl part is cyano-substituted)}, an anilino(C₁-C₂ alkyl) group, a (C₁-C₂ alkoxyimino)methyl group wherein the alkyl part may be substituted (substituent(s) being phenyl group or cyano group) a C₃-C₅ cycloalkyl group, a C₃-C₅ alkenyl group, an N-(3-cyanobenzyl)-N-(1-isobutyl-5-methyl-4-pyrazolyl)aminocarbonyl(C₂-C₆ alkenyl) group, a cyclohexenyl group, an optionally substituted phenyl group (the substituent(s) is (are) 1-2 same or different substituent(s) selected from the group consisting of a fluorine atom and a chlorine atom), a 5-6 membered unsaturated heterocyclic group (the heterocycle contains 1-2 nitrogen atom(s)), a C₁-C₃ alkoxy group, an optionally substituted phenoxy group (the substituent(s) is (are) 1-2 same or different substituent(s) selected from the group consisting of a fluorine atom, a chlorine atom, a methyl group, an alkyl group having a carbon number of 1-2 substituted with 1-3 fluorine atom(s), a methoxy group and a nitro group), an optionally substituted 5-6 membered unsaturated (heterocyclyl)oxy group {the substituent(s) is (are) 1-4 same or different substituent(s) selected from the group consisting of a fluorine atom, a chlorine atom, a bromine atom, a straight or branched alkyl group having a carbon number of 1-3, a straight or branched haloalkyl group having a carbon number of 1-3 (the halogen substituent(s) is (are) 1-3 same or different halogen atom(s) selected from the group consisting of a fluorine atom, a chlorine atom and a bromine atom), an alkoxy group having a carbon number of 1-2, a carbamoyl group, an alkoxycarbonyl group having a carbon number of 2-3, a cyano group, a nitro group, an amino group and a phenyl group; the heteroatom for the heterocycle is an oxygen atom, a sulfur atom or a nitrogen atom, and may be 1-2 further nitrogen atom(s), two oxygen atoms may bind to the sulfur atom, and the heterocycle may be fused with a benzene ring and may be substituted with 1-2 oxo groups)}, a C₁-C₂ alkylthio group, an optionally substituted amino group {the substituent(s) is (are) 1-2 same or different substituent(s) selected from the group consisting of a C₁-C₂ alkyl group, a cyano(C₁-C₂ alkyl) group, a (C₂-C₃ alkoxycarbonyl) (C₁-C₄ alkyl) group, a benzyloxycarbonyl (C₁-C₄ alkyl) group, a carbamoyl(C₁-C₄ alkyl) group wherein the carbamoyl part may be substituted (substituent (s) being phenethyl group) and a (C₁-C₂ alkoxyimino)methylcarbonyl group wherein the alkyl part is cyano-substituted} or an anilino group;
   or Ar and B are taken together to be an optionally substituted o-phenylene group (substituent(s) being bromine atom, cyano group or nitro group, and one methylene group is inserted between the phenylene group and the carbon atom to which B binds);
   further more preferably Ar is an optionally substituted phenyl group {the substituent(s) is (are) 1-2 same or different substituent(s) selected from the group consisting of a fluorine atom, a bromine atom, an iodine atom, a methyl group, a methylthiomethyl group, a cyano group, a 1,2,4-oxadiazolyl group, a methoxy group, a trifluoromethoxy group, a phenoxy group, a methylthio group, a phenylsulfinyl group, a phenylsulfonyl group, a nitro group and a dimethylamino group};
   B is a C₁-C₇ alkyl group, a chloromethyl group, a (C₂-C₃ alkoxycarbonyl)methyl group, a benzyl group, a morpholinomethyl group, a pyrazol-1-ylmethyl group wherein the pyrazole part may be substituted (the substituent(s) is (are) 1-2 same or different substituent(s) selected from the group consisting of a chlorine atom, a bromine atom, a methyl group, an ethoxycarbonyl group and a nitro group), a (methyl-isooxazol-3-yl)methyl group, an imidazol-1-ylmethyl group, a (dichloro-imidazol-1-yl)methyl group, a 2-oxo-1-pyridylmethyl group, a (trifluoromethyl-2-oxo-1-pyridyl)methyl group, a 6-oxo-pyrimidin-1-ylmethyl group, a 4-oxo-quinazolin-3-ylmethyl group, a 3-oxo-pyrazol-1-ylmethyl group wherein the pyrazole part may be substituted (the substituent(s) is (are) 1-2 methyl group(s)), a 2-pyridylmethyl group, a hydroxy(C₁-C₃ alkyl) group, a (C₁-C₃ alkoxy) (C₁-C₂ alkyl) group, a nitrobenzyloxymethyl group, a cyanobenzyloxymethyl group, a (dichloro-4-pyridyl)methoxymethyl group, a (methyl-isooxazol-3-yl)methoxymethyl group, a 3-pyridylmethoxymethyl group, a cyano (C₁-C₃ alkoxy) (C₁-C₂ alkyl) group, a (C₁-C₂ alkoxy) (C₁-C₂ alkoxy) methyl group, a benzyloxymethoxymethyl group, a trimethylsilylethoxymethoxymethyl group, a (C₃-C₄ alkenyloxy) (C₁-C₂ alkyl) group, a propynyloxymethyl group, a phenoxy(C₁-C₂ alkyl) group wherein the phenyl part may be substituted (the substituent(s) is (are) 1-2 same substituent(s) selected from the group consisting of a fluorine atom, a chlorine atom, a methyl group, a trifluoromethyl group, a methoxy group and a nitro group), a (methyl-isooxazol-5-yl)oxymethyl group, a pyrazol-1-yloxymethyl group wherein the pyrazole part may be substituted (substituent(s) being bromine atom or methyl group), a 2-pyridyloxymethyl group wherein the pyridine part may be substituted (the substituent(s) is (are) 1-3 same or different substituent(s) selected from the group consisting of a fluorine atom, a chlorine atom and a trifluoromethyl group, or 3-4 fluorine atom(s)), a 3-pyridyloxymethyl group, a pyrimidin-2-yloxymethyl group, a pyrimidin-4-yloxymethyl group, an (isopropyl-methyl-pyrimidin-4-yl)oxymethyl group, a benzisooxazol-3-yloxymethyl group wherein the benzisooxazole part is substituted (substituent(s) being chlorine atom, methyl group, nitro group or amino group), an imidazol-1-yloxymethyl group, a 1,2,4-triazol-1-yloxymethyl group, a thiazol-2-yloxymethyl group, a 2-quinolyloxymethyl group, a 3-isoquinolyloxymethyl group, a benzisothiazol-1,l-dioxide-3-yloxymethyl group, a benzothiazol-2-yloxymethyl group, an N,N-phthaloyloxymethyl group, a dimethylmonophenylsilyloxymethyl group, a mono(C₃-C₄ alkyl)dimethylsilyloxymethyl group, a (C₂-C₅ alkylcarbonyloxy) (C₁-C₂ alkyl) group, a cyclopentenylacetoxymethyl group, a benzylcarbonyloxymethyl group, a 2-thienylacetoxymethyl group, a 3-thienylacetoxymethyl group, a 3-indolylacetoxymethyl group, a 3-benzothienylacetoxymethyl group, a 2-pyridylacetoxymethyl group, a 3-pyridylacetoxymethyl group, a 4-pyridylacetoxymethyl group, a (methyl-isooxazol-5-yl)acetoxymethyl group, a methoxyacetoxymethyl group, a phenoxyacetoxymethyl group, an acetoxyacetoxymethyl group, a (C₄-C₇ cycloalkylcarbonyloxy)methyl group, a (C₄-C₆ alkenylcarbonyloxy)methyl group, a 3-ene-4-phenylbutyryloxymethyl group, a methyl-1,4-cyclohexadienylcarbonyloxymethyl group, a benzoyloxymethyl group wherein the phenyl part may be substituted (the substituent(s) is (are) 1-2 fluorine atom(s), 1-2 chlorine atom(s), one methyl group, one methoxy group or one cyano group), a 4-pyridylcarbonyloxymethyl group, a (dichloro-4-pyridyl)carbonyloxymethyl group, a (chloro-methoxy-4-pyridyl)carbonyloxymethyl group, a (trimethyl-pyrrol-3-yl)carbonyloxymethyl group, a (dimethyl-3-furyl)carbonyloxymethyl group, a (t-butyl-methyl-3-furyl)carbonyloxymethyl group, a (trifluoromethyl-3-furyl)carbonyloxymethyl group, a 2-furylcarbonyloxymethyl group, a 2-thienylcarbonyloxymethyl group, a 3-pyridylcarbonyloxymethyl group, a (C₂-C₅ alkoxycarbonyloxy)methyl group, an allyloxycarbonyloxymethyl group, a phenoxycarbonyloxymethyl group, a carbamoyloxymethyl group wherein the carbamoyl part is substituted (the substituent(s) is (are) 1-2 same or different C₁-C₂ alkyl group(s)), a thiocarbamoyloxymethyl group wherein the thiocarbamoyl part is substituted (the substituent(s) is (are) 1-2 methyl group(s)), a (methylthio)carbonyloxymethyl group, an aminooxymethyl group wherein the amino part may be substituted [substituent(s) being optionally substituted carbamoyl group (the substituent(s) is (are) 1-2 methyl groups), optionally substituted C₁-C₃ alkylidene group {the substituent(s) is (are) 1-2 same or different substituent(s) selected from the group consisting of a methoxycarbonyl group, an optionally substituted phenyl group (substituent(s) being trifluoromethyl group or methoxy group), a methyl-2-furyl group and a methoxy group} or cyclopentylidene group], a diethoxyphosphoryloxymethyl group, a diethoxy(thiophosphoryl)oxymethyl group, a methylsulfonyloxymethyl group, a phenylsulfonyloxymethyl group, a tolylsulfonyloxymethyl group, a (C₁-C₃ alkylthio)methyl group, a cyano (C₁-C₃ alkyl) thiomethyl group, a (C₂-C₃ alkoxycarbonyl)methylthiomethyl group, a phenylthiomethyl group, a 2-pyridylthiomethyl group, a (chloro-2-pyridyl)thiomethyl group, a (trifluoromethyl-2-pyridyl)thiomethyl group, a (nitro-2-pyridyl) thiomethyl group, a (chloro-trifluoromethyl-2-pyridyl)thiomethyl group, a pyrimidin-2-ylthiomethyl group, a (methyl-pyrimidin-2-yl)thiomethyl group, a (dimethyl-pyrimidin-2-yl)thiomethyl group, a (phenyl-pyrimidin-2-yl)thiomethyl group, an imidazol-2-ylthiomethyl group, a (methyl-imidazol-2-yl)thiomethyl group, a 1,3,4-triazol-2-ylthiomethyl group, a (methyl-1,3,4-triazol-2-yl)thiomethyl group, a (t-butyl-1,3,4-triazol-2-yl)thiomethyl group, a (cyclohexyl-1,3,4-triazol-2-yl)thiomethyl group, a (methyl-1,3,4-isothiadiazol-2-yl)thiomethyl group, a 4-pyridylthiomethyl group, a benzooxazol-2-ylthiomethyl group, a benzothiazol-2-ylthiomethyl group, a benzimidazol-2-ylthiomethyl group, a 1H-imidazo[4,5-b]pyridin-2-ylthiomethyl group, a 4,5-dihydrothiazol-2-ylthiomethyl group, a (C₁-C₃ alkylsulfinyl)methyl group, a phenylsulfinylmethyl group, a 2-pyridylsulfinylmethyl group, a (C₁-C₃ alkylsulfonyl)methyl group, a phenylsulfonylmethyl group, a 2-pyridylsulfonylmethyl group, a diethoxy(thiophosphoryl)thiomethyl group, an aminomethyl group wherein the amino part may be substituted (substituent(s) being methyl group, methoxycarbonyl(C₁-C₄ alkyl) group, benzyloxycarbonyl (C₁-C₄ alkyl) group, methoxyiminomethylcarbonyl group wherein the methyl part is cyano-substituted, methoxycarbonyl group, benzyloxycarbonyl group or methoxyiminomethylcarbonylcarbamoyl group wherein the methyl part is cyano-substituted), an anilinomethyl group, a methoxyiminomethyl group wherein the methyl part may be substituted (substituent(s) being phenyl group or cyano group), a C₃-C₅ cycloalkyl group, a C₃-C₅ alkenyl group, a cyclohexenyl group, a fluorophenyl group, a chlorophenyl group, a difluorophenyl group, a dichlorophenyl group, a C₁-C₃ alkoxy group, an optionally substituted phenoxy group (the substituent(s) is (are) 1-2 same or different substituent(s) selected from the group consisting of a fluorine atom, a chlorine atom, a methyl group, a trifluoromethyl group, a methoxy group and a cyano group), an optionally substituted pyrazol-1-yloxy group (the substituent is one bromine atom or methyl group), an optionally substituted 2-pyridyloxy group (the substituent(s) is (are) 1-3 same or different substituent(s) selected from the group consisting of a fluorine atom, a chlorine atom, a methyl group, a trifluoromethyl group, a methoxy group, a carbamoyl group, a methoxycarbonyl group, a cyano group and a phenyl group, or 3-4 fluorine atom(s)), a 3-pyridyloxy group, an optionally substituted pyrimidin-2-yloxy group (the substituent(s) is (are) 1-3 same or different substituent(s) selected from the group consisting of a fluorine atom, a chlorine atom, a methyl group, a trifluoromethyl group, a methoxy group, a carbamoyl group, a methoxycarbonyl group, a cyano group and a phenyl group, or 3-4 fluorine atom(s)), a pyrimidin-4-yloxy group, an (isopropyl-methyl-pyrimidin-4-yl)oxy group, a substituted benzisooxazol-3-yloxy group (the substituent is one chlorine atom, methyl group, nitro group or amino group), an imidazol-1-yloxy group, a 1,2,4-triazol-1-yloxy group, a thiazol-2-yloxy group, a 2-quinolyloxy group, a 3-isoquinolyloxy group, a benzisothiazole-1,1-dioxide-3-yloxy group, a benzisothiazol-3-yloxy group, an N,N-phthaloyloxy group, an optionally substituted 3-isooxazolyloxy group (the substituent is one methyl group), a methylthio group, an optionally substituted amino group (substituent(s) being C₁-C₂ alkyl group, cyanomethyl group, (C₂-C₃ alkoxycarbonyl)methyl group or methoxyiminomethylcarbonyl group wherein the methyl part is cyano-substituted) or an anilino group; particularly preferably Ar is a 3-cyanophenyl group, a 3-nitrophenyl group or a 2-fluoro-5-cyanophenyl group; B is a methyl group, a 4-chloro-1-pyrazolylmethyl group, a 4-bromo-1-pyrazolylmethyl group, a hydroxymethyl group, a methoxymethyl group, a 3-nitrobenzyloxymethyl group, a 3-pyridylmethoxymethyl group, a cyanomethoxymethyl group, an allyloxymethyl group, a 2-methyl-2-butenyloxymethyl group, a phenoxymethyl group, a (fluorophenoxy)methyl group, a (chlorophenoxy)methyl group, a (difluorophenoxy)methyl group, a (dichlorophenoxy)methyl group, a (methylphenoxy)methyl group, a (trifluoromethylphenoxy)methyl group, a (methoxyphenoxy)methyl group, a (nitrophenoxy)methyl group, a (dinitrophenoxy)methyl group, a (cyanophenoxy)methyl group, a 2-(phenoxy)ethyl group, a 3-pyridyloxymethyl group, a 3-isooxazolyloxymethyl group, a 5-methyl-3-isooxazolyloxymethyl group, an isovaleryloxymethyl group, a pivaloyloxymethyl group, a (C₅-C₇ cycloalkylcarbonyloxy)methyl group, a (fluorobenzoyl)oxymethyl group, a (chlorobenzoyl)oxymethyl group, a (difluorobenzoyl)oxymethyl group, a (methylbenzoyl)oxymethyl group, a (methoxybenzoyl)oxymethyl group, a (cyanobenzoyl)oxymethyl group, a 4-pyridylcarbonyloxymethyl group, a 2-thienylcarbonyloxymethyl group, a carbamoyloxymethyl group, a cyanomethylthiomethyl group, a phenylthiomethyl group, a (3-chloro-2-pyridyl)thiomethyl group, a (4-methyl-pyrimidin-2-yl)thiomethyl group, an imidazol-2-ylthiomethyl group, a 4-pyridylthiomethyl group, a benzimidazol-2-ylthiomethyl group, a 1H-imidazo[4,5-b]pyridin-2-ylthiomethyl group, a 2-pyrimidinylthiomethyl group, an aminomethyl group wherein the amino part is substituted (substituent(s) being methoxyiminomethylcarbonyl group wherein the methyl part is cyano-substituted, or methoxycarbonyl group), an anilinomethyl group, a cyclopropyl group, a 2-chlorophenyl group, a methoxy group, a phenoxy group, a fluorophenoxy group, a chlorophenoxy group, a difluorophenoxy group, a dichlorophenoxy group, a methylphenoxy group, a trifluoromethylphenoxy group, a methoxyphenoxy group, a nitrophenoxy group, a dinitrophenoxy group, a cyanophenoxy group, an optionally substituted 2-pyridyloxy group (the substituent(s) is (are) 1-3 same or different substituent(s) selected from the group consisting of a fluorine atom, a chlorine atom, a methyl group, a trifluoromethyl group and a cyano group), an optionally substituted pyrimidin-2-yloxy group (the substituent(s) is (are) 1-3 same or different substituent(s) selected from the group consisting of a fluorine atom, a chlorine atom, a methyl group, a trifluoromethyl group and a cyano group), a 3-isooxazolyloxy group, a 5-methyl-3-isooxazolyloxy group or an amino group;
   most preferably Ar is a 3-cyanophenyl group; B is a methyl group, a 4-chloro-1-pyrazolylmethyl group, a 4-bromo-1-pyrazolylmethyl group, a hydroxymethyl group, a methoxymethyl group, a 3-pyridylmethoxymethyl group, a cyanomethoxymethyl group, a phenoxymethyl group, a (4-fluorophenoxy)methyl group, a (4-chlorophenoxy)methyl group, a 3-isooxazolyloxymethyl group, a 5-methyl-3-isooxazolyloxymethyl group, a carbamoyloxymethyl group, a cyanomethylthiomethyl group, a 2-pyrimidinylthiomethyl group, a cyclopropyl group, a methoxy group, a phenoxy group, a 2-fluorophenoxy group, a 3-fluorophenoxy group, a 4-fluorophenoxy group, a 4-chlorophenoxy group, a 2-methylphenoxy group, a 2-cyanophenoxy group, a 3-cyanophenoxy group, an optionally substituted 2-pyridyloxy group (the substituent(s) is (are) 1-3 same or different substituent(s) selected from the group consisting of a chlorine atom, a methyl group, a trifluoromethyl group and a cyano group), an optionally substituted pyrimidin-2-yloxy group (the substituent(s) is (are) 1-3 same or different substituent(s) selected from the group consisting of a chlorine atom, a methyl group, a trifluoromethyl group and a cyano group), a 5-methyl-3-isooxazolyloxy group or an amino group.

The compound (I) of the present invention, when it has a hydroxyl group in the molecule, can be converted into, for example, an alkali metal salt, an alkaline earth metal salt or an ammonium salt, or, when it has a basic part in the molecule, can be converted into salts such as sulfate, hydrochloride, nitrate and phosphate. Those salts are included in the present invention insofar as they can be used as an agricultural or horticultural fungicide.

In the present invention, the "alkali metal salt" includes a sodium salt, a potassium salt and a lithium salt, preferably, a sodium salt or a potassium salt.

In the present invention, the "alkaline earth metal salt" includes a calcium salt and a magnesium salt, preferably, a calcium salt.

Hydrates of the present compounds are included in the present invention.

Some of the present compounds have an asymmetric carbon and, in this case, the present invention includes both single optically active compounds and mixtures of several optically active compounds in arbitrary ratio.

The compound (I) of the present invention is preferably a compound wherein
(a1) R¹ is a C₁-C₁₀ alkyl group, a halo(C₁-C₈ alkyl) group (the halogen substituent(s) is (are) 1-3 same or different substituent(s) selected from the group consisting of a fluorine atom, a chlorine atom and a bromine atom), a (C₂-C₄ alkylcarbonyl) (C₁-C₃ alkyl) group, a benzoyl (C₁-C₄ alkyl) group wherein the phenyl part may be substituted (substituent(s) being fluorine atom, chlorine atom or bromine atom), a (C₂-C₄ alkoxycarbonyl) (C₁-C₃ alkyl) group, a phenoxycarbonyl (C₁-C₃ alkyl) group, a cyano (C₁-C₃ alkyl) group, a (C₃-C₆ cycloalkyl) (C₁-C₃ alkyl) group wherein the cycloalkyl part may be substituted (substituent(s) being C₁-C₃ alkyl group, fluorine atom or chlorine atom), a norbornyl (C₁-C₃ alkyl) group, a benzyl group wherein the phenyl part may be substituted {substituent(s) being fluorine atom, chlorine atom, bromine atom, cyano group or halo(C₁-C₃ alkoxy) group (the halogen substituent(s) is (are) 1-3 same or different fluorine atom(s) chlorine atom(s) or bromine atom(s))}, a (5-6 membered saturated heterocyclyl) (C₁-C₃ alkyl) group wherein the heterocycle part may be substituted (the substituent(s) is (are) 1-2 same or different C₁-C₃ alkyl group(s); the heterocycle contains 1-2 same or different heteroatom(s) selected from the group consisting of an oxygen atom and a nitrogen atom), a (5-6 membered unsaturated heterocyclyl) (C₁-C₃ alkyl) group wherein the heterocycle part may be substituted (substituent(s) being fluorine atom, chlorine atom or bromine atom; the heterocycle contains an oxygen atom, a sulfur atom or a nitrogen atom, and may contain one further nitrogen atom, and the heterocycle may be fused with a benzene ring), a hydroxy(C₁-C₄ alkyl) group, a (C₁-C₃ alkoxy) (C₁-C₄ alkyl) group, a (C₁-C₃ alkoxy) (C₁-C₃ alkoxy) (C₁-C₃ alkyl) group, a phenoxy(C₁-C₃ alkyl) group, a (5-6 membered saturated heterocyclyl)oxy(C₁-C₃ alkyl) group (the heterocycle contains 1-2 same or different heteroatom(s) selected from the group consisting of an oxygen atom and a nitrogen atom), a tri(C₁-C₃ alkyl)silyloxy(C₁-C₄ alkyl) group, a (C₂-C₄ alkylcarbonyloxy) (C₁-C₄ alkyl) group (the alkylcarbonyloxy substituent(s) is (are) 1-2 same or different alkylcarbonyloxy group(s)), a benzoyloxy(C₁-C₃ alkyl) group wherein the phenyl part may be substituted (the substituent(s) is (are) 1-2 same or different substituent(s) selected from the group consisting of a fluorine atom, a chlorine atom and a bromine atom), a (C₁-C₄ alkylthio) (C₁-C₃ alkyl) group, a (C₃-C₆ cycloalkylthio) (C₁-C₃ alkyl) group, a (C₁-C₄ alkylsulfonyl) (C₁-C₃ alkyl) group, a (C₃-C₆ cycloalkylsulfonyl) (C₁-C₃ alkyl) group, a hydroxyimino(C₁-C₃ alkyl) group, a (C₂-C₄ alkoxycarbonyloxyimino) (C₁-C₃ alkyl) group, a (C₁-C₃ alkoxyimino) (C₁-C₃ alkyl) group wherein the alkyl part may be substituted (substituent(s) being C₂-C₄ alkoxycarbonyl group), a (C₇-C₈ aralkyloxyimino) (C₁-C₃ alkyl) group, a (C₂-C₄ alkylcarbonyloxyimino) (C₁-C₃ alkyl) group, a tri(C₁-C₃ alkyl)silyl(C₁-C₃ alkyl) group, a C₃-C₆ cycloalkyl group, a C₂-C₆ alkenyl group, a halo(C₂-C₄ alkenyl) group (the halogen substituent(s) is (are) 1-2 same or different substituent(s) selected from the group consisting of a fluorine atom, a chlorine atom, a bromine atom and an iodine atom), a hydroxy(C₂-C₃ alkenyl) group wherein the alkenyl part may be substituted (substituent(s) being C₂-C₄ alkoxycarbonyl group), a (C₁-C₃ alkoxy) (C₂-C₃ alkenyl) group wherein the alkenyl part may be substituted (substituent(s) being C₂-C₄ alkoxycarbonyl group), a C₂-C₃ alkynyl group, an optionally substituted phenyl group {the substituent(s) is (are) 1-3 same or different substituent(s) selected from the group consisting of a
   fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a carboxyl group, a C₂-C₄ alkoxycarbonyl group, a carbamoyl group, a cyano group, a C₁-C₃ alkoxy group, a halo(C₁-C₃ alkoxy) group (the halogen substituent(s) is (are) 1-3 same or different substituent(s) selected from the group consisting of a fluorine atom, a chlorine atom and a bromine atom), a halo(C₂-C₃ alkenyloxy) group (the halogen substituent(s) is
   (are) 1-2 same or different halogen atom(s) selected from the group consisting of a fluorine atom, a chlorine atom, a
   bromine atom and an iodine atom) and a nitro group}, a 5-6 membered saturated heterocyclic group (the heterocycle contains 1-2 same or different heteroatom(s) selected from the group consisting of an oxygen atom and a nitrogen atom), an optionally substituted 5-6 membered unsaturated heterocyclic group {the substituent(s) is (are) 1-2 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom) and a C₁-C₃ alkoxy group; the heterocycle contains a sulfur atom or a nitrogen atom, and may contain 1-2 further nitrogen atom(s), and the heterocycle may be fused with a benzene ring}, a C₂-C₆ alkylcarbonyl group, an optionally substituted benzoyl group {substituent(s) being fluorine atom, chlorine atom, bromine atom, cyano group or halo(C₁-C₃ alkoxy) group (the halogen substituent(s) is (are) 1-3 same or different substituent(s) selected from the group consisting of a fluorine atom, a chlorine atom and a bromine atom)}, a C₂-C₅ alkoxycarbonyl group, an optionally substituted phenylsulfonyl group (substituent(s) being C₁-C₃ alkyl group) or a di(C₁-C₃ alkyl)sulfamoyl group;
(a2) R² is a hydrogen atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a hydroxy(C₁-C₃ alkyl) group, a (C₁-C₃ alkoxy) (C₁-C₃ alkyl) group, a carboxyl group, a C₂-C₄ alkoxycarbonyl group or a cyano group;
(a3) R³ is a hydrogen atom, a fluorine atom, a chlorine atom, a bromine atom, a C₁-C₄ alkyl group, a halo(C₁-C₃ alkyl) group (the halogen substituent(s) is (are) 1-3 same or different substituent(s) selected from the group consisting of a fluorine atom, a chlorine atom and a bromine atom), a hydroxy(C₁-C₃ alkyl) group, a (C₁-C₃ alkoxy) (C₁-C₃ alkyl) group, a (C₁-C₃ alkylsulfonyloxy) (C₁-C₃ alkyl) group, a carboxyl group, a C₂-C₄ alkoxycarbonyl group, a cyano group or a C₁-C₃ alkoxy group;
(a4) R⁴ is a hydrogen atom, a C₁-C₃ alkyl group or a cyano group;
(a5) Z is an oxygen atom or a sulfur atom;
(a6) Ar is an optionally substituted C₆-C₁₀ aryl group [the substituent(s) is (are) 1-3 same or different substituent(s) selected from the group consisting of a fluorine atom, a chlorine atom, a bromine atom, an iodine atom, a C₁-C₃ alkyl group, a halo(C₁-C₃ alkyl) group (the halogen substituent(s) is (are) 1-2 same or different substituent(s) selected from the group consisting of a fluorine atom, a chlorine atom, a bromine atom), a cyano(C₁-C₃ alkyl) group, a hydroxy(C₁-C₃ alkyl) group, a (C₁-C₃ alkylthio) (C₁-C₃ alkyl) group, a (C₁-C₃ alkylsulfonyl) (C₁-C₃ alkyl), an imino(C₁-C₃ alkyl) group wherein the alkyl part is substituted (substituent(s) being C₁-C₃ alkoxy group), a (C₁-C₃ alkylimino) (C₁-C₃ alkyl) group wherein the alkyl part is substituted {substituent(s) being C₁-C₃ alkylthio group or C₇-C₈ aralkylthio group wherein the aryl part may be substituted (substituent(s) being cyano group)}, a hydroxyimino(C₁-C₃ alkyl) group wherein the alkyl part is substituted (substituent(s) being amino group), an optionally substituted C₂-C₅ alkenyl group (the substituent(s) is (are) 1-2 different substituent(s) selected from the group consisting of a C₁-C₃ alkoxy group and a C₂-C₄ alkoxycarbonyl group), a C₂-C₄ alkylcarbonyl group, a carboxyl group, a C₂-C₄ alkoxycarbonyl group, an optionally substituted carbamoyl group {substituent(s) being C₁-C₃ alkyl group, cyano(C₁-C₃ alkyl) group, C₂-C₄ alkylcarbonyl group or optionally substituted phenyl group (substituent(s) being C₁-C₃ alkoxy group)}, an optionally substituted thiocarbamoyl group (substituent(s) being C₁-C₃ alkyl group), a cyano group, a phenyl group, an optionally substituted 5 membered unsaturated heterocyclic group {substituent(s) being C₁-C₃ alkyl group or halo(C₁-C₃ alkyl) group (the halogen substituent(s) is (are) 1-3 same or different substituent(s) selected from the group consisting of a fluorine atom, a chlorine atom and a bromine atom); the heterocycle contains an oxygen atom, a sulfur atom or a nitrogen atom, and contains 1-2 further nitrogen atom(s)}, a hydroxyl group, a C₁-C₃ alkoxy group, a halo(C₁-C₃ alkoxy) group (the halogen substituent(s) is (are) 1-5 same or different substituent(s) selected from the group consisting of a fluorine atom, a chlorine atom and a bromine atom), a halo(C₂-C₄ alkenyloxy) group (the halogen substituent(s) is (are) 1-2 same or different substituent(s) selected from the group consisting of a fluorine atom, a chlorine atom, a bromine atom and an iodine atom), an optionally substituted phenoxy group (substituent(s) being C₁-C₃ alkyl group), a C₁-C₃ alkylthio group, a halo(C₁-C₃ alkylthio) group (the halogen substituent(s) is (are) 1-3 same or different substituent(s) selected from the group consisting of a fluorine atom, a chlorine atom and a bromine atom), a phenylthio group, a C₁-C₃ alkylsulfinyl group, a phenylsulfinyl group, a C₁-C₃ alkylsulfonyl group, a halo(C₁-C₃ alkylsulfonyl) group (the halogen substituent(s) is (are) 1-3 same or different substituent(s) selected from the group consisting of a fluorine atom, a chlorine atom and a bromine atom), a phenylsulfonyl group, an azido group, a nitro group and an optionally substituted amino group {the substituent(s) is (are) 1-2 same or different substituent(s) selected from the group consisting of a C₁-C₃ alkyl group, a C₇-C₈ aralkyl group, a formyl group, a C₂-C₅ alkylcarbonyl group, a (C₂-C₄ alkoxycarbonylamino) (C₂-C₅ alkylcarbonyl) group, a C₂-C₄ alkoxycarbonyl group, a C₂-C₄ alkyl(thiocarbonyl) group, an optionally substituted carbamoyl group (substituent(s) being C₁-C₃ alkyl group) and a C₁-C₃ alkylsulfonyl group}] or an optionally substituted 5 membered unsaturated heterocyclic group (the substituent(s) is (are) 1-2 same or different substituent(s) selected from the group consisting of a fluorine atom, a chlorine atom, a bromine atom, an iodine atom, a C₁-C₃ alkyl group, a hydroxyl group, a C₁-C₃ alkoxy group, a cyano group, a nitro group and an amino group; the heterocycle contains a sulfur atom or a nitrogen atom, and may contain one further nitrogen atom, and the heterocycle may be fused with a benzene ring);
B is a chlorine atom, a bromine atom, a C₁-C₁₅ alkyl group, a halo(C₁-C₃ alkyl) group (the halogen substituent(s) is (are) 1-3 same or different substituent(s) selected from the group consisting of a fluorine atom, a chlorine atom and a bromine atom), a (C₂-C₄ alkoxycarbonyl) (C₁-C₃ alkyl) group, a cyano(C₁-C₃ alkyl) group, a (C₃-C₆ cycloalkyl) (C₁-C₃ alkyl) group, a (C₄-C₆ cycloalkenyl) (C₁-C₃ alkyl) group, a C₇-C₈ aralkyl group, a (5-6 membered saturated heterocyclyl) (C₁-C₃ alkyl) group (the heterocycle contains 1-2 same or different heteroatom(s) selected from the group consisting of an oxygen atom and a nitrogen atom), a (5-6 membered unsaturated heterocyclyl) (C₁-C₃ alkyl) group wherein the heterocycle part may be substituted {the substituent(s) is (are) 1-3 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a C₂-C₄ alkoxycarbonyl group, a cyano group, a nitro group and an amino group; the heterocycle contains an oxygen atom, a sulfur atom or a nitrogen atom, and may contain 1-2 further nitrogen atom(s), the heterocycle may be fused with a benzene ring, and the heterocycle may be substituted with one oxo group}, a hydroxy(C₁-C₃ alkyl) group, a (C₁-C₃ alkoxy) (C₁-C₃ alkyl) group, a (C₇-C₈ aralkyloxy) (C₁-C₃ alkyl) group wherein the aryl part may be substituted {substituent(s) being halo(C₁-C₃ alkyl) group (the halogen substituent(s) is (are) 1-3 same or different substituent(s) selected from the group consisting of a fluorine atom, a chlorine atom and a bromine atom), cyano group or nitro group}, a (5-6 membered unsaturated heterocyclyl) (C₁-C₆ alkoxy) (C₁-C₆ alkyl) group wherein the heterocycle part may be substituted (the substituent(s) is (are) 1-2 same or different substituent(s) selected from the group consisting of a fluorine atom, a chlorine atom, a bromine atom and a C₁-C₃ alkyl group; the heterocycle contains an oxygen atom, a sulfur atom or a nitrogen atom, and may contain one further nitrogen atom), a cyano(C₁-C₃ alkoxy) (C₁-C₃ alkyl) group, a (C₁-C₃ alkoxy) (C₁-C₃ alkoxy) (C₁-C₃ alkyl) group, a (C₇-C₈ aralkyloxy) (C₁-C₃ alkoxy) (C₁-C₃ alkyl) group, a (C₁-C₆ alkoxy) (C₁-C₆ alkoxy) (C₁-C₆ alkoxy) (C₁-C₆ alkyl) group, a tri(C₁-C₃ alkyl)silyl(C₁-C₃ alkoxy) (C₁-C₃ alkoxy) (C₁-C₃ alkyl) group, a (C₂-C₅ alkenyloxy) (C₁-C₃ alkyl) group, a (C₂-C₃ alkynyloxy) (C₁-C₃ alkyl) group, a phenoxy(C₁-C₃ alkyl) group wherein the phenyl part may be substituted {the substituent(s) is (are) 1-2 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a C₁-C₃ alkoxy group, a cyano group and a nitro group}, a (5-6 membered saturated heterocyclyl)oxy(C₁-C₃ alkyl) group (the heterocycle contains 1-2 same or different heteroatom(s) selected from the group consisting of an oxygen atom and a nitrogen atom), a (5-6 membered unsaturated heterocyclyl)oxy(C₁-C₃ alkyl) group wherein the heterocycle part may be substituted {the substituent(s) is (are) 1-4 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom, and a bromine atom), a C₂-C₄ alkoxycarbonyl group, a cyano group, a nitro group, an amino group and a phenyl group; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring, and the heterocycle may be substituted with 1-2 oxo group(s)}, a tri(C₁-C₄ alkyl)silyloxy(C₁-C₃ alkyl) group, a di(C₁-C₃ alkyl) (monophenyl)silyloxy(C₁-C₃ alkyl) group, a mono(C₁-C₄ alkyl) (diphenyl)silyloxy(C₁-C₃ alkyl) group, a formyloxy(C₁-C₃ alkyl) group, a (C₂-C₅ alkylcarbonyloxy) (C₁-C₃ alkyl) group, a (C₅-C₆ cycloalkenyl)(C₂-C₄ alkylcarbonyloxy) (C₁-C₃ alkyl) group, a (C₈-C₉ aralkylcarbonyloxy) (C₁-C₃ alkyl) group, a (5-6 membered unsaturated heterocyclyl) (C₁-C₆ alkylcarbonyloxy) (C₁-C₆ alkyl) group wherein the heterocycle part may be substituted (substituent(s) being C₁-C₃ alkyl group; the heterocycle contains an oxygen atom, a sulfur atom or a nitrogen atom, and may contain one further nitrogen atom, and the heterocycle may be fused with a benzene ring), a (C₁-C₃ alkoxy)(C₂-C₄ alkylcarbonyloxy) (C₁-C₃ alkyl) group, a phenoxy(C₂-C₄ alkylcarbonyloxy) (C₁-C₃ alkyl) group, a (C₂-C₄ alkylcarbonyloxy) (C₂-C₄ alkylcarbonyloxy) (C₁-C₃ alkyl) group, a (C₄-C₇ cycloalkylcarbonyloxy) (C₁-C₃ alkyl) group, a (C₄-C₆ alkenylcarbonyloxy) (C₁-C₃ alkyl) group, a phenyl (C₄-C₆ alkenylcarbonyloxy) (C₁-C₃ alkyl) group, a (C₆-C₇ cycloalkenylcarbonyloxy) (C₁-C₃ alkyl) group, a cyclohexadienylcarbonyloxy (C₁-C₃ alkyl) group wherein the cyclohexadiene part may be substituted (substituent(s) being C₁-C₃ alkyl group), a benzoyloxy(C₁-C₃ alkyl) group wherein the phenyl part may be substituted (the substituent(s) is (are) 1-2 same or different substituent(s) selected from the group consisting of a fluorine atom, a chlorine atom, a bromine atom, a C₁-C₃ alkyl group, a C₁-C₃ alkoxy group and a cyano group), a (5-6 membered unsaturated heterocyclyl)carbonyloxy(C₁-C₃ alkyl) group wherein the heterocycle part may be substituted {the substituent(s) is (are) 1-3 same or different substituent(s) selected from the group consisting of a fluorine atom, a chlorine atom, a bromine atom, a C₁-C₄ alkyl group, a halo(C₁-C₃ alkyl) group (the halogen substituent(s) is (are) 1-3 same or different substituent(s) selected from the group consisting of a fluorine atom, a chlorine atom and a bromine atom), a C₁-C₃ alkoxy group and a phenyl group; the heterocycle contains an oxygen atom, a sulfur atom or a nitrogen atom, and may contain 1-2 further nitrogen atom(s), the heterocycle may be fused with a benzene ring, and the heterocycle may be substituted with one oxo group}, a (C₂-C₅ alkoxycarbonyloxy) (C₁-C₃ alkyl) group, a (C₄-C₅ alkenyloxycarbonyloxy)(C₁-C₃ alkyl) group, a phenoxycarbonyloxy(C₁-C₃ alkyl) group, a carbamoyloxy(C₁-C₃ alkyl) group wherein the carbamoyl part may be substituted (the substituent(s) is (are) 1-2 same or different C₁-C₃ alkyl group(s)), a thiocarbamoyloxy(C₁-C₃ alkyl) group wherein the thiocarbamoyl part may be substituted (the substituent(s) is (are) 1-2 same or different C₁-C₃ alkyl group(s)), a [C₂-C₅ (alkylthio)carbonyloxy](C₁-C₃ alkyl) group, an aminooxy(C₁-C₃ alkyl) group wherein the amino part may be substituted [substituent(s) being C₄-C₆ cycloalkylcarbonyl group, C₂-C₄ alkoxycarbonyl group, C₄-C₅ alkenyloxycarbonyl group, optionally substituted carbamoyl group (the substituent(s) is (are) 1-2 same or different substituent(s) selected from the group consisting of a C₁-C₃ alkyl group and a (C₁-C₃ alkoxyimino) (C₂-C₄ alkylcarbonyl) group wherein the alkyl part is cyano-substituted), optionally substituted C₁-C₆ alkylidene group «the substituent(s) is (are) 1-2 same or different substituent(s) selected from the group consisting of a C₃-C₆ cycloalkyl group, a C₂-C₄ alkoxycarbonyl group, an optionally substituted phenyl group {substituent(s) being halo(C₁-C₃ alkyl) group (the halogen substituent(s) is (are) 1-3 same or different substituent(s) selected from the group consisting of a fluorine atom, a chlorine atom and a bromine atom) or C₁-C₃ alkoxy group}, an optionally substituted 5-6 membered unsaturated heterocyclic group (substituent(s) being C₁-C₃ alkyl group; the heterocycle contains an oxygen atom, a sulfur atom or a nitrogen atom, and may contain one further nitrogen atom, and the heterocycle may be fused with a benzene ring) and a C₁-C₃ alkoxy group» or C₅-C₆ cycloalkylidene group (an alkylene chain of the cycloalkylidene group may be interrupted by one oxygen atom)], a di(C₁-C₃ alkoxy)phosphoryloxy(C₁-C₃ alkyl) group, a di(C₁-C₃alkoxy)thiophosphoryloxy(C₁-C₃ alkyl) groups a (C₁-C₃ alkylsulfonyloxy)(C₁-C₃ alkyl) group, a phenylsulfonyloxy(C₁-C₃ alkyl) group wherein the phenyl part may be substituted (substituent(s) being C₁-C₃ alkyl group), a (C₁-C₆ alkylthio) (C₁-C₃ alkyl) group wherein the alkyl part of the alkylthio part may be substituted (substituent(s) being C₂-C₄ alkoxycarbonyl group or cyano group), a phenylthio(C₁-C₃ alkyl) group, a (5-6 membered unsaturated heterocyclyl)thio(C₁-C₃ alkyl) group wherein the heterocycle part may be substituted {the substituent(s) is (are) 1-2 same or different substituent(s) selected from the group consisting of a fluorine atom, a chlorine atom, a bromine atom, a C₁-C₄ alkyl group, a halo(C₁-C₃ alkyl) group (the halogen substituent(s) is (are) 1-3 same or different substituent(s) selected from the group consisting of a fluorine atom, a chlorine atom and a bromine atom), a C₃-C₆ cycloalkyl group, a nitro group and a phenyl group; the heterocycle contains an oxygen atom, a sulfur atom or a nitrogen atom, and may contain 1-2 further nitrogen atom(s), and the heterocycle may be fused with a benzene ring or a pyridine ring), a (C₁-C₆ alkylsulfinyl) (C₁-C₃ alkyl) group, a phenylsulfinyl(C₁-C₃ alkyl) group, a (5-6 membered unsaturated heterocyclyl)sulfinyl(C₁-C₃ alkyl) group (the heterocycle contains an oxygen atom, a sulfur atom or a nitrogen atom, and may contain 1-2 further nitrogen atom(s), and the heterocycle may be fused with a benzene ring or a pyridine ring), a (C₁-C₆ alkylsulfonyl) (C₁-C₃ alkyl) group, a phenylsulfonyl(C₁-C₃ alkyl) group, a (5-6 membered unsaturated heterocyclyl)sulfonyl(C₁-C₃ alkyl) group (the heterocycle contains an oxygen atom, a sulfur atom or a nitrogen atom, and may contain 1-2 further nitrogen atom(s), and the heterocycle may be fused with a benzene ring or a pyridine ring), a di(C₁-C₃ alkoxy)thiophosphorylthio(C₁-C₃ alkyl) group, an amino(C₁-C₄ alkyl) group wherein the amino part may be substituted {substituent(s) being C₁-C₃ alkyl group, carboxyl(C₁-C₄ alkyl) group, (C₂-C₄ alkoxycarbonyl) (C₁-C₄ alkyl) group, (C₈-C₉ aralkyloxycarbonyl) (C₁-C₄ alkyl) group, formyl group, (C₁-C₃ alkoxyimino)(C₂-C₄ alkylcarbonyl) group wherein the alkyl part is cyano-substituted, C₂-C₅ alkoxycarbonyl group, C₈-C₁₀ aralkyloxycarbonyl group or optionally substituted carbamoyl group (substituent(s) being C₁-C₃ alkyl group or (C₁-C₃ alkoxyimino)(C₂-C₄ alkylcarbonyl) group wherein the alkyl part is cyano-substituted)}, an anilino(C₁-C₃ alkyl) group, a (C₁-C₃ alkoxyimino)(C₁-C₃ alkyl) group wherein the alkyl part may be substituted (substituent(s) being phenyl group or cyano group), a C₃-C₆ cycloalkyl group, a C₃-C₅ alkenyl group, a phenyl(C₃-C₅ alkenyl) group, an N-(cyano-aralkyl)-N-{1,5-di(C₁-C₄ alkyl)-4-pyrazolyl}aminocarbonyl(C₂-C₆ alkenyl) group, a C₅-C₆ cycloalkenyl group, an optionally substituted phenyl group (the substituent(s) is (are) 1-2 same or different substituent(s) selected from the group consisting of a
fluorine atom, a chlorine atom and a bromine atom), a 5-6 membered saturated heterocyclic group (the heterocycle contains 1-2 same or different heteroatom(s) selected from the group consisting of an oxygen atom and a nitrogen atom), an optionally substituted 5-6 membered unsaturated heterocyclic group (the substituent(s) is (are) 1-2 same or different substituent(s) selected from the group consisting of a
fluorine atom, a chlorine atom, a bromine atom and a C₁-C₆ alkyl group; the heterocycle contains an oxygen atom, a sulfur atom or a nitrogen atom, and may contain 1-2 further nitrogen atom(s), and the heterocycle may be fused with a benzene ring) a C₁-C₄ alkoxy group, an optionally substituted phenoxy group {the substituent(s) is (are) 1-2 same or different substituent(s) selected from the group consisting of a
fluorine atom, a chlorine atom, a bromine atom, a straight or branched alkyl group having a carbon number of 1-3, a straight or branched haloalkyl group having a carbon number of 1-3 (the halogen substituent(s) is (are) 1-3 same or different halogen atom(s) selected from the group consisting of a fluorine atom, a chlorine atom and a bromine atom), an alkoxy group having a carbon number of 1-3, a cyano group and a nitro group}, an optionally substituted 5-6 membered unsaturated (heterocyclyl)oxy group {the substituent(s) is (are) 1-4 same or different substituent(s) selected from the group consisting of a fluorine atom, a chlorine atom, a bromine atom, a
straight or branched alkyl group having a carbon number of 1-3, a straight or branched haloalkyl group having a carbon number of 1-3 (the halogen substituent(s) is (are) 1-3 same or different halogen atom(s) selected from the group consisting
of a fluorine atom, a chlorine atom and a bromine atom), a straight or branched alkoxy group having a carbon number of 1-3, a carbamoyl group, a straight or branched alkoxycarbonyl group having a carbon number of 2-4, a cyano group, a nitro group, an amino group and a phenyl group; the heteroatom for the heterocycle is an oxygen atom, a sulfur atom or a nitrogen atom, and may be 1-2 further nitrogen atom(s), two oxygen atoms may bind to the sulfur atom, the heterocycle may be fused with a benzene ring, and may be substituted with 1-2 oxo group(s)}, a C₁-C₃ alkylthio group, an optionally substituted amino group [the substituent(s) is (are) 1-2 same or different substituent(s) selected from the group consisting of a C₁-C₃ alkyl group, a cyano(C₁-C₃ alkyl) group, a carboxyl(C₁-C₄ alkyl) group, a (C₂-C₄ alkoxycarbonyl)(C₁-C₄ alkyl) group, a (C₈-C₉ aralkyloxycarbonyl) (C₁-C₄ alkyl) group, a carbamoyl (C₁-C₆ alkyl) group wherein the carbamoyl group may be substituted {substituent(s) being optionally substituted C₇-C₈ aralkyl group (the substituent(s) is (are) 1-2 same or different C₁-C₃ alkoxy group(s)) } and a (C₁-C₆ alkoxyimino) (C₂-C₇ alkylcarbonyl) group wherein the alkyl part is cyano-substituted] or an anilino group; or Ar and B are taken together to be an optionally substituted o-phenylene group (substituent(s) being fluorine atom, chlorine atom, bromine atom, hydroxyl group, cyano group, nitro group or amino group, and one methylene group is inserted between the phenylene group and the carbon atom to which B binds);
more preferably a compound wherein
(b1) R¹ is a C₂-C₈ alkyl group, a halo(C₃-C₆ alkyl) group (halogen substituent(s) being fluorine atom or chlorine atom), a (C₃-C₆ cycloalkyl) (C₁-C₂ alkyl) group wherein the cycloalkyl part may be substituted (substituent(s) being methyl group, fluorine atom or chlorine atom), a norbornyl(C₁-C₂ alkyl) group, a benzyl group wherein the phenyl part may be substituted (substituent(s) being fluorine atom, chlorine atom, bromine atom or cyano group), a (5-6 membered saturated heterocyclyl) (C₁-C₂ alkyl) group (the heterocycle contains one oxygen atom), a methoxy(C₃-C₄ alkyl) group, a trimethylsilyloxy(C₃-C₄ alkyl) group, an acetoxy(C₃-C₄ alkyl) group (the number of acetoxy substituent(s) is 1-2), a (C₃-C₄ alkylthio)(C₁-C₂ alkyl) group, a (C₅-C₆ cycloalkylthio) (C₁-C₂ alkyl) group, a (C₃-C₄ alkylsulfonyl) (C₁-C₂ alkyl) group, a trimethylsilyl(C₁-C₂ alkyl) group, a C₅-C₆ cycloalkyl group, a C₄-C₅ alkenyl group, a C₂-C₃ alkynyl group, an optionally substituted phenyl group {the substituent(s) is (are) 1-2 same or different substituent(s) selected from the group consisting of a fluorine atom, a chlorine atom, a methyl group, a halo(C₁-C₂ alkyl) group (the halogen substituent(s) is (are) 1-3 fluorine atom(s)), a carbamoyl group and a cyano group}, a C₃-C₆ alkylcarbonyl group, an optionally substituted benzoyl group (substituent(s) being fluorine atom) or a dimethylsulfamoyl group;
(b2) R² is a hydrogen atom or a methyl group;
(b3) R³ is a hydrogen atom, a chlorine atom, a C₁-C₂ alkyl group, a halo(C₁-C₂ alkyl) group (the halogen substituent(s) is (are) 1-3 fluorine atom(s)) or a cyano group;
(b4) R⁴ is a hydrogen atom or a C₁-C₂ alkyl group;
(b5) Z is an oxygen atom or a sulfur atom;
(b6) Ar is an optionally substituted phenyl group {the substituent(s) is (are) 1-2 same or different substituent(s) selected from the group consisting of a fluorine atom, a chlorine atom, a bromine atom, an iodine atom, a methyl group, a (C₁-C₂ alkylthio)methyl group, an iminomethyl group wherein the alkyl part is substituted (substituent(s) being C₁-C₂ alkoxy group), a carbamoyl group, a cyano group, a 1,2,4-oxadiazolyl group, a C₁-C₂ alkoxy group, a halo(C₁-C₂ alkoxy) group (the halogen substituent(s) is (are) 1-3 fluorine atom(s)), an optionally substituted phenoxy group (substituent(s) being methyl group), a C₁-C₂ alkylthio group, a C₁-C₂ alkylsulfinyl group, a phenylsulfinyl group, a C₁-C₂ alkylsulfonyl group, a phenylsulfonyl group, an azido group, a nitro group and an optionally substituted amino group (substituent(s) being methyl group or C₇-C₈ aralkyl group)};
B is a chlorine atom, a C₁-C₇ alkyl group, a halo(C₁-C₂ alkyl) group (halogen substituent(s) being fluorine atom or chlorine atom), a (C₂-C₃ alkoxycarbonyl)(C₁-C₂ alkyl) group, a benzyl group, a morpholyl(C₁-C₂ alkyl) group, a (5-6 membered unsaturated heterocyclyl)(C₁-C₃ alkyl) group wherein the heterocycle part may be substituted {the substituent(s) is (are) 1-2 same or different substituent(s) selected from the group consisting of a chlorine atom, a bromine atom, a methyl group, a halo(C₁-C₂ alkyl) group (the halogen substituent(s) is (are) 1-3 fluorine atom(s)), a C₂-C₃ alkoxycarbonyl group and a nitro group; the heterocycle contains an oxygen atom or a nitrogen atom, and may contain 1-2 further nitrogen atom(s), the heterocycle may be fused with a benzene ring, and the heterocycle may be substituted with one oxo group}, a hydroxy(C₁-C₃ alkyl) group, a (C₁-C₃ alkoxy) (C₁-C₂ alkyl) group, a benzyloxy(C₁-C₂ alkyl) group wherein the phenyl part may be substituted {substituent(s) being halo(C₁-C₂ alkyl) group (the halogen substituent(s) is (are) 1-3 fluorine atom(s)), cyano group or nitro group}, a (5-6 membered unsaturated heterocyclyl) (C₁-C₆ alkoxy) (C₁-C₆ alkyl) group wherein the heterocycle part may be substituted (the substituent(s) is (are) 1-2 same or different substituent(s) selected from the group consisting of a fluorine atom, a chlorine atom, a bromine atom and a methyl group; the heterocycle contains an oxygen atom or a nitrogen atom, and may contain one further nitrogen atom), a cyano(C₁-C₃ alkoxy)(C₁-C₂ alkyl) group, a (C₁-C₂ alkoxy)(C₁-C₂ alkoxy)(C₁-C₂ alkyl) group, a benzyloxy(C₁-C₂ alkoxy)(C₁-C₂ alkyl) group, a trimethylsilyl(C₁-C₂ alkoxy)(C₁-C₂ alkoxy)(C₁-C₂ alkyl) group, a (C₃-C₅ alkenyloxy)(C₁-C₂ alkyl) group, a propynyloxy(C₁-C₂ alkyl) group, a phenoxy(C₁-C₂ alkyl) group wherein the phenyl part may be substituted {the substituent(s) is (are) 1-2 same or different substituent(s) selected from the group consisting of a fluorine atom, a chlorine atom, a methyl group, a halo(C₁-C₂ alkyl) group (the halogen substituent(s) is (are) 1-3 fluorine atom(s)), a methoxy group and a nitro group}, a (5-6 membered unsaturated heterocyclyl)oxy(C₁-C₂ alkyl) group wherein the heterocycle part may be substituted {the substituent(s) is (are) 1-4 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a nitro group and an amino group; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring, and the heterocycle may be substituted with 1-2 oxo groups)}, a tri(C₁-C₄ alkyl)silyloxy(C₁-C₂ alkyl) group, a dimethylmonophenylsilyloxy(C₁-C₂ alkyl) group, a (C₂-C₅ alkylcarbonyloxy)(C₁-C₃ alkyl) group, a cyclopentenyl(C₂-C₃ alkylcarbonyloxy)(C₁-C₂ alkyl) group, a benzylcarbonyloxy(C₁-C₂ alkyl) group, a (5-6 membered unsaturated heterocyclyl)(C₁-C₆ alkylcarbonyloxy)(C₁-C₆ alkyl) group wherein the heterocycle part may be substituted (substituent(s) being methyl group; the heterocycle contains an oxygen atom, a sulfur atom or a nitrogen atom, and may contain one further nitrogen atom, and the heterocycle may be fused with a benzene ring), a (C₁-C₂ alkoxy)(C₂-C₃ alkylcarbonyloxy)(C₁-C₂ alkyl) group, a phenoxy(C₂-C₃ alkylcarbonyloxy)(C₁-C₂ alkyl) group, a (C₂-C₃ alkylcarbonyloxy)(C₂-C₃ alkylcarbonyloxy)(C₁-C₂ alkyl) group, a (C₄-C₇ cycloalkylcarbonyloxy)(C₁-C₂ alkyl) group, a (C₄-C₆ alkenylcarbonyloxy)(C₁-C₂ alkyl) group, a phenyl(C₄-C₆ alkenylcarbonyloxy)(C₁-C₂ alkyl) group, a 1,4-cyclohexadienylcarbonyloxy(C₁-C₂ alkyl) group wherein the cyclohexadiene part may be substituted (substituent(s) being methyl group), a benzoyloxy(C₁-C₂ alkyl) group wherein the phenyl part may be substituted (the substituent(s) is (are) 1-2 same or different substituent(s) selected from the group consisting of a fluorine atom, a chlorine atom, a methyl atom, a methoxy group and a cyano group), a (5-6 membered unsaturated heterocyclyl)carbonyloxy(C₁-C₃ alkyl) group wherein the heterocycle part may be substituted {the substituent(s) is (are) 1-3 same or different substituent(s) selected from the group consisting of a fluorine atom, a chlorine atom, a C₁-C₄ alkyl group, a halo(C₁-C₂ alkyl) group (the halogen substituent(s) is (are) 1-3 fluorine atom(s)) and a methoxy group; the heterocycle contains an oxygen atom, a sulfur atom or a nitrogen atom, and the heterocycle may be fused with a benzene ring}, a (C₂-C₅ alkoxycarbonyloxy)(C₁-C₂ alkyl) group, an allyloxycarbonyloxy(C₁-C₂ alkyl) group, a phenoxycarbonyloxy(C₁-C₂ alkyl) group, a carbamoyloxy(C₁-C₂ alkyl) group wherein the carbamoyl part may be substituted (the substituent(s) is (are) 1-2 same or different C₁-C₂ alkyl group(s)), a thiocarbamoyloxy(C₁-C₂ alkyl) group wherein the thiocarbamoyl part may be substituted (the substituent(s) is (are) 1-2 same or different C₁-C₂ alkyl group(s)), a [C₂-C₃(alkylthio)carbonyloxy](C₁-C₂ alkyl) group, an aminooxy(C₁-C₂ alkyl) group wherein the amino part may be substituted [substituent(s) being optionally substituted carbamoyl group (the substituent(s) is (are) 1-2 same or different C₁-C₂ alkyl group(s)), optionally substituted C₁-C₃ alkylidene group «the substituent(s) is (are) 1-2 same or different substituent(s) selected from the group consisting of a C₂-C₃ alkoxycarbonyl group, an optionally substituted phenyl group {substituent(s) being halo(C₁-C₂ alkyl) group (the halogen substituent(s) is (are) 1-3 fluorine atom(s)) or methoxy group}, an optionally substituted 5-6 membered unsaturated heterocyclic group (substituent(s) being methyl group; the heterocycle contains an oxygen atom, a sulfur atom or a nitrogen atom, and the heterocycle may be fused with a benzene ring) and a methoxy group» or C₅-C₆ cycloalkylidene group], a di(C₁-C₂ alkoxy)phosphoryloxy(C₁-C₂ alkyl) group, a di(C₁-C₂ alkoxy)thiophosphoryloxy(C₁-C₂ alkyl) group, a (C₁-C₂ alkylsulfonyloxy)(C₁-C₂ alkyl) group, a phenylsulfonyloxy(C₁-C₂ alkyl) group wherein the phenyl part may be substituted (substituent(s) being methyl group), a (C₁-C₃ alkylthio) (C₁-C₂ alkyl) group wherein the alkyl part of the alkylthio part may be substituted (substituent(s) being C₂-C₃ alkoxycarbonyl group or cyano group), a phenylthio(C₁-C₂ alkyl) group, a (5-6 membered unsaturated heterocyclyl)thio(C₁-C₂ alkyl) group wherein the heterocycle part may be substituted {the substituent(s) is (are) 1-2 same or different substituent(s) selected from the group consisting of a fluorine atom, a chlorine atom, a C₁-C₄ alkyl group, a halo (C₁-C₂ alkyl) group (the halogen substituent(s) is (are) 1-3 fluorine atom(s)), a cyclohexyl group, a nitro group and a phenyl group; the heterocycle contains an oxygen atom, a sulfur atom or a nitrogen atom, and the heterocycle may be fused with a benzene ring or a pyridine ring}, a (C₁-C₃ alkylsulfinyl) (C₁-C₂ alkyl) group, a phenylsulfinyl(C₁-C₂ alkyl) group, a (6 membered unsaturated heterocyclyl)sulfinyl(C₁-C₂ alkyl) group (the heterocycle contains 1-2 nitrogen atom(s)), a (C₁-C₃ alkylsulfonyl) (C₁-C₂ alkyl) group, a phenylsulfonyl(C₁-C₂ alkyl) group, a (6 membered unsaturated heterocyclyl)sulfonyl(C₁-C₂ alkyl) group (the heterocycle contains 1-2 nitrogen atom(s)), a di (C₁-C₂ alkoxy)thiophosphorylthio(C₁-C₂ alkyl) group, an amino(C₁-C₂ alkyl) group wherein the amino part may be substituted {substituent(s) being methyl group, carboxyl(C₁-C₄ alkyl) group, (C₂-C₃ alkoxycarbonyl) (C₁-C₄ alkyl) group, benzyloxycarbonyl(C₁-C₄ alkyl) group, (C₁-C₂ alkoxyimino)methylcarbonyl group wherein the methyl part is cyano-substituted, C₂-C₃ alkoxycarbonyl group, benzyloxycarbonyl group or optionally substituted carbamoyl group (substituent(s) being C₁-C₂ alkyl group or (C₁-C₂ alkoxyimino)methylcarbonyl group wherein the methyl part is cyano-substituted)}, an anilino(C₁-C₂ alkyl) group, a (C₁-C₂ alkoxyimino)methyl group wherein the alkyl part may be substituted (substituent(s) being phenyl group or cyano group), a C₃-C₅ cycloalkyl group, a C₃-C₅ alkenyl group, an N- (3-cyanobenzyl)-N-(1-isobutyl-5-methyl-4-pyrazolyl)aminocarbonyl(C₂-C₆ alkenyl) group, a cyclohexenyl group, an optionally substituted phenyl group (the substituent(s) is (are) 1-2 same or different substituent(s) selected from the group consisting of a fluorine atom and a chlorine atom), a 5-6 membered unsaturated heterocyclic group (the heterocycle contains 1-2 nitrogen atom(s)), a C₁-C₃ alkoxy group, an optionally substituted phenoxy group (the substituent(s) is (are) 1-2 same or different substituent(s) selected from the group consisting of a fluorine atom, a chlorine atom, a methyl group, an alkyl group having a carbon number of 1-2 substituted with 1-3 fluorine atom(s), a methoxy group and a nitro group), an optionally substituted 5-6 membered unsaturated (heterocyclyl)oxy group {the substituent(s) is (are) 1-4 same or different substituent(s) selected from the group consisting of a fluorine atom, a chlorine atom, a bromine atom, a straight or branched alkyl group having a carbon number of 1-3, a straight or branched haloalkyl group having a carbon number of 1-3 (the halogen substituent(s) is (are) 1-3 halogen atom(s) selected from the group consisting of a fluorine atom, a chlorine atom and a bromine atom), an alkoxy group having a carbon number of 1-2, a carbamoyl group, an alkoxycarbonyl group having a carbon number of 2-3, a cyano group, a nitro group, an amino group and a phenyl group; the heteroatom for the heterocycle is an oxygen atom, a sulfur atom or a nitrogen atom, and may be 1-2 further nitrogen atom(s), two oxygen atoms may bind to the sulfur atom, and the heterocycle may be fused with a benzene ring and may be substituted with 1-2 oxo group(s)}, a C₁-C₂ alkylthio group, an optionally substituted amino group {the substituent(s) is (are) 1-2 same or different substituent(s) selected from the group consisting of a C₁-C₂ alkyl group, a cyano(C₁-C₂ alkyl) group, a (C₂-C₃ alkoxycarbonyl) (C₁-C₄ alkyl) group, a benzyloxycarbonyl(C₁-C₄ alkyl) group, a carbamoyl(C₁-C₄ alkyl) group wherein the carbamoyl part may be substituted (substituent(s) being phenethyl group) and a (C₁-C₂ alkoxyimino)methylcarbonyl group wherein the alkyl part is cyano-substituted} or an anilino group; or Ar and B are taken together to be an optionally substituted o-phenylene group (substituent(s) being bromine atom, cyano group or nitro group, and one methylene group is inserted between the phenylene group and the carbon atom to which B binds) ;
further more preferably a compound wherein
(c1) R¹ is a C₂-C₆ alkyl group, a halo(C₃-C₄ alkyl) group (halogen substituent(s) being fluorine atom or chlorine atom), a (C₄-C₆ cycloalkyl)methyl group wherein the cycloalkyl part may be substituted (substituent(s) being methyl group or fluorine atom), a norbornylmethyl group, a benzyl group, a 3-fluorobenzyl group, a tetrahydropyranylmethyl group, a (C₃-C₄ alkylthio)methyl group, a (C₃-C₄ alkylsulfonyl)methyl group, a trimethylsilylmethyl group, a cyclohexyl group, a pentenyl group, a phenyl group, a 2-fluorophenyl group, a 3-fluorophenyl group, a 4-fluorophenyl group, a 2-chlorophenyl group, a 3-chlorophenyl group, a 2,3-difluorophenyl group, a 2,5-difluorophenyl group, a 3,5-difluorophenyl group, a 2-methylphenyl group, a 3-methylphenyl group, a 4-methylphenyl group or a C₄-C₆ alkylcarbonyl group;
(c2) R² is a hydrogen atom or a methyl group;
(c3) R³ is a hydrogen atom, a methyl group or a fluoromethyl group;
(c4) R⁴ is a hydrogen atom or a methyl group;
(c5) Z is an oxygen atom or a sulfur atom;
(c6) Ar is an optionally substituted phenyl group {the substituent(s) is (are) 1-2 same or different substituent(s) selected from the group consisting of a fluorine atom, a bromine atom, an iodine atom, a methyl group, a methylthiomethyl group, a cyano group, a 1,2,4-oxadiazolyl group, a methoxy group, a trifluoromethoxy group, a phenoxy group, a methylthio group, a phenylsulfinyl group, a phenylsulfonyl group, a nitro group and a dimethylamino group};
B is a C₁-C₇ alkyl group, a chloromethyl group, a (C₂-C₃ alkoxycarbonyl)methyl group, a benzyl group, a morpholinomethyl group, a pyrazol-1-ylmethyl group wherein the pyrazole part may be substituted (the substituent(s) is (are) 1-2 same or different substituent(s) selected from the group consisting of a chlorine atom, a bromine atom, a methyl group, an ethoxycarbonyl group and a nitro group), a (methyl-isooxazol-3-yl)methyl group, an imidazol-1-ylmethyl group, a (dichloro-imidazol-1-yl)methyl group, a 2-oxo-1-pyridylmethyl group, a (trifluoromethyl-2-oxo-1-pyridyl)methyl group, a 6-oxo-pyrimidin-1-ylmethyl group, a 4-oxo-quinazolin-3-ylmethyl group, a 3-oxo-pyrazol-1-ylmethyl group wherein the pyrazole part may be substituted (the substituent(s) is (are) 1-2 methyl group(s)), a 2-pyridylmethyl group, a hydroxy(C₁-C₃ alkyl) group, a (C₁-C₃ alkoxy)(C₁-C₂ alkyl) group, a nitrobenzyloxymethyl group, a cyanobenzyloxymethyl group, a (dichloro-4-pyridyl)methoxymethyl group, a (methyl-isooxazol-3-yl)methoxymethyl group, a 3-pyridylmethoxymethyl group, a cyano(C₁-C₃ alkoxy)(C₁-C₂ alkyl) group, a (C₁-C₂ alkoxy)(C₁-C₂ alkoxy)methyl group, a benzyloxymethoxymethyl group, a trimethylsilylethoxymethoxymethyl group, a (C₃-C₅ alkenyloxy)(C₁-C₂ alkyl) group, a propynyloxymethyl group, a phenoxy(C₁-C₂ alkyl) group wherein the phenyl part may be substituted (the substituent(s) is (are) 1-2 same substituent(s) selected from the group consisting of a fluorine atom, a chlorine atom, a methyl group, a trifluoromethyl group, a methoxy group and a nitro group), a (methyl-isooxazol-5-yl)oxymethyl group, a pyrazol-1-yloxymethyl group wherein the pyrazole part may be substituted (substituent(s) being bromine atom or methyl group), a 2-pyridyloxymethyl group wherein the pyridine part may be substituted (the substituent(s) is (are) 1-2 same or different substituent(s) selected from the group consisting of a fluorine atom, a chlorine atom, and a trifluoromethyl group, or 3-4 fluorine atom(s)), a 3-pyridyloxymethyl group, a pyrimidin-2-yloxymethyl group, a pyrimidin-4-yloxymethyl group, an (isopropyl-methyl-pyrimidin-4-yl)oxymethyl group, a benzisooxazol-3-yloxymethyl group wherein the benzisooxazole part is substituted (substituent(s) being chlorine atom, methyl group, nitro group or amino group), an imidazol-1-yloxymethyl group, a 1,2,4-triazol-1-yloxymethyl group, a thiazol-2-yloxymethyl group, a 2-quinolyloxymethyl group, a 3-isoquinolyloxymethyl group, a benzisothiazol-1,1-dioxide-3-yloxymethyl group, a benzothiazol-2-yloxymethyl group, an N,N-phthaloyloxymethyl group, a mono(C₃-C₄ alkyl)dimethylsilyloxymethyl group, a dimethylmonophenylsilyloxymethyl group, a (C₂-C₅ alkylcarbonyloxy)(C₁-C₂ alkyl) group, a cyclopentenylacetoxymethyl group, a benzylcarbonyloxymethyl group, a 2-thienylacetoxymethyl group, a 3-thienylacetoxymethyl group, a 3-indolylacetoxymethyl group, a 3-benzothienylacetoxymethyl group, a 2-pyridylacetoxymethyl group, a 3-pyridylacetoxymethyl group, a 4-pyridylacetoxymethyl group, a (methyl-isooxazol-5-yl)acetoxymethyl group, a methoxyacetoxymethyl group, a phenoxyacetoxymethyl group, an acetoxyacetoxymethyl group, a (C₄-C₇ cycloalkylcarbonyloxy)methyl group, a (C₄-C₆ alkenylcarbonyloxy)methyl group, a 3-ene-4-phenylbutyryloxymethyl group, a methyl-1,4-cyclohexadienylcarbonyloxymethyl group, a benzoyloxymethyl group wherein the phenyl part may be substituted (the substituent(s) is (are) 1-2 fluorine atom(s), 1-2 chlorine atom(s), one methyl group, one methoxy group or one cyano group), a 4-pyridylcarbonyloxymethyl group, a (dichloro-4-pyridyl)carbonyloxymethyl group, a (chloro-methoxy-4-pyridyl)carbonyloxymethyl group, a (trimethyl-pyrrol-3-yl)carbonyloxymethyl group, a (dimethyl-3-furyl)carbonyloxymethyl group, a (t-butyl-methyl-3-furyl)carbonyloxymethyl group, a (trifluoromethyl-3-furyl)carbonyloxymethyl group, a 2-furylcarbonyloxymethyl group, a 2-thienylcarbonyloxymethyl group, a 3-pyridylcarbonyloxymethyl group, a (C₂-C₅ alkoxycarbonyloxy)methyl group, an allyloxycarbonyloxymethyl group, a phenoxycarbonyloxymethyl group, a carbamoyloxymethyl group wherein the carbamoyl part is substituted (the substituent(s) is (are) 1-2 same or different C₁-C₂ alkyl group(s)), a thiocarbamoyloxymethyl group wherein the thiocarbamoyl part is substituted (the substituent(s) is (are) 1-2 methyl group(s)), a (methylthio)carbonyloxymethyl group, an aminooxymethyl group wherein the amino part may be substituted [substituent(s) being optionally substituted carbamoyl group (the substituent(s) is (are) 1-2 methyl group(s)), optionally substituted C₁-C₃ alkylidene group {the substituent(s) is (are) 1-2 same or different substituent(s) selected from the group consisting of a methoxycarbonyl group, an optionally substituted phenyl group (substituent(s) being trifluoromethyl group or methoxy group), a methyl-2-furyl group and a methoxy group} or cyclopentylidene group], a diethoxyphosphoryloxymethyl group, a diethoxy(thiophosphoryl)oxymethyl group, a methylsulfonyloxymethyl group, a phenylsulfonyloxymethyl group, a tolylsulfonyloxymethyl group, a (C₁-C₃ alkylthio)methyl group, a cyano(C₁-C₃ alkylthio)methyl group, a (C₂-C₃ alkoxycarbonyl)methylthiomethyl group, a phenylthiomethyl group, a 2-pyridylthiomethyl group, a (chloro-2-pyridyl)thiomethyl group, a (trifluoromethyl-2-pyridyl)thiomethyl group, a (nitro-2-pyridyl)thiomethyl group, a (chloro-trifluoromethyl-2-pyridyl)thiomethyl group, a pyrimidin-2-ylthiomethyl group, a (methyl-pyrimidin-2-yl)thiomethyl group, a (dimethyl-pyrimidin-2-yl)thiomethyl group, a (phenyl-pyrimidin-2-yl)thiomethyl group, an imidazol-2-ylthiomethyl group, a (methyl-imidazol-2-yl)thiomethyl group, a 1,3,4-triazol-2-ylthiomethyl group, a (methyl-1,3,4-triazol-2-yl)thiomethyl group, a (t-butyl-1,3,4-triazol-2-yl)thiomethyl group, a (cyclohexyl-1,3,4-triazol-2-yl)thiomethyl group, a (methyl-1,3,4-isothiadiazol-2-yl)thiomethyl group, a 4-pyridylthiomethyl group, a benzooxazol-2-ylthiomethyl group, a benzothiazol-2-ylthiomethyl group, a benzimidazol-2-ylthiomethyl group, a 1H-imidazo[4,5-b]pyridin-2-ylthiomethyl group, a 4,5-dihydrothiazol-2-ylthiomethyl group, a (C₁-C₃ alkylsulfinyl)methyl group, a phenylsulfinylmethyl group, a 2-pyridylsulfinylmethyl group, a (C₁-C₃ alkylsulfonyl)methyl group, a phenylsulfonylmethyl group, a 2-pyridylsulfonylmethyl group, a diethoxy(thiophosphoryl)thiomethyl group, an aminomethyl group wherein the amino part may be substituted (substituent(s) being methyl group, methoxycarbonyl(C₁-C₄ alkyl) group, benzyloxycarbonyl(C₁-C₄ alkyl) group, methoxyiminomethylcarbonyl group wherein the methyl part is cyano-substituted, methoxycarbonyl group, benzyloxycarbonyl group or methoxyiminomethylcarbonylcarbamoyl group wherein the methyl part is cyano-substituted), an anilinomethyl group, a methoxyiminomethyl group wherein the methyl part may be substituted (substituent(s) being phenyl group or cyano group), a C₃-C₅ cycloalkyl group, a C₃-C₅ alkenyl group, a cyclohexenyl group, a fluorophenyl group, a chlorophenyl group, a difluorophenyl group, a dichlorophenyl group, a C₁-C₃ alkoxy group, an optionally substituted phenoxy group (the substituent(s) is (are) 1-2 same or different substituent(s) selected from the group consisting of a fluorine atom, a chlorine atom, a methyl group, a trifluoromethyl group, a methoxy group and a cyano group), an optionally substituted pyrazol-1-yloxy group (the substituent is one bromine atom or methyl group), an optionally substituted 2-pyridyloxy group (the substituent(s) is (are) 1-3 same or different substituent(s) selected from the group consisting of a fluorine atom, a chlorine atom, a methyl group, a trifluoromethyl group, a methoxy group, a carbamoyl group, a methoxycarbonyl group, a cyano group and a phenyl group, or 3-4 fluorine atom(s)), a 3-pyridyloxy group, an optionally substituted pyrimidin-2-yloxy group (the substituent(s) is (are) 1-3 same or different substituent(s) selected from the group consisting of a fluorine atom, a chlorine atom, a methyl group, a trifluoromethyl group, a methoxy group, a carbamoyl group, a methoxycarbonyl group, a cyano group and a phenyl group, or 3-4 fluorine atom(s)), a pyrimidin-4-yloxy group, an (isopropyl-methyl-pyrimidin-4-yl)oxy group, a substituted benzisooxazol-3-yloxy group (the substituent is one chlorine atom, methyl group, nitro group or amino group), an imidazol-1-yloxy group, a 1,2,4-triazol-1-yloxy group, a thiazol-2-yloxy group, a 2-quinolyloxy group, a 3-isoquinolyloxy group, a benzisothiazol-1,1-dioxide-3-yloxy group, a benzisothiazol-2-yloxy group, an N,N-phthaloyloxy group, an optionally substituted 3-isooxazolyloxy group (the substituent is one methyl group), a methylthio group, an optionally substituted amino group (substituent(s) being C₁-C₂ alkyl group, cyanomethyl group, (C₂-C₃ alkoxycarbonyl)methyl group or methoxyiminomethylcarbonyl group wherein the methyl part is cyano-substituted) or an anilino group;
particularly preferably
(d1) R¹ is a C₃-C₆ alkyl group, a cyclobutylmethyl group, a cyclopentylmethyl group, a phenyl group, a 3-fluorophenyl group, a 3,5-difluorophenyl group or a 3-methylphenyl group;
(d2) R² is a hydrogen atom or a methyl group;
(d3) R³ is a hydrogen atom or a methyl group;
(d4) R⁴ is a hydrogen atom;
(d5) Z is an oxygen atom;
(d6) Ar is a 3-cyanophenyl group, a 3-nitrophenyl group or a 2-fluoro-5-cyanophenyl group;
B is a methyl group, a 4-chloro-1-pyrazolylmethyl group, a 4-bromo-1-pyrazolylmethyl group, a hydroxymethyl group, a methoxymethyl group, a 3-nitrobenzyloxymethyl group, a 3-pyridylmethoxymethyl group, a cyanomethoxymethyl group, an allyloxymethyl group, a 2-methyl-2-butenyloxymethyl group, a phenoxymethyl group, a (fluorophenoxy)methyl group, a (chlorophenoxy)methyl group, a (difluorophenoxy)methyl group, a (dichlorophenoxy)methyl group, a (methylphenoxy)methyl group, a (trifluoromethylphenoxy)methyl group, a (methoxyphenoxy)methyl group, a (nitrophenoxy)methyl group, a (dinitrophenoxy)methyl group, a (cyanophenoxy)methyl group, a 2-(phenoxy)ethyl group, a 3-pyridyloxymethyl group, a 3-isooxazolyloxymethyl group, a 5-methyl-3-isooxazolyloxymethyl group, an isovaleryloxymethyl group, a pivaloyloxymethyl group, a (C₅-C₇ cycloalkylcarbonyloxy)methyl group, a (fluorobenzoyl)oxymethyl group, a (chlorobenzoyl)oxymethyl group, a (difluorobenzoyl)oxymethyl group, a (methylbenzoyl)oxymethyl group, a (methoxybenzoyl)oxymethyl group, a (cyanobenzoyl)oxymethyl group, a 4-pyridylcarbonyloxymethyl group, a 2-thienylcarbonyloxymethyl group, a carbamoyloxymethyl group, a cyanomethylthiomethyl group, a phenylthiomethyl group, a (3-chloro-2-pyridyl)thiomethyl group, a (4-methyl-pyrimidin-2-yl)thiomethyl group, an imidazol-2-ylthiomethyl group, a 4-pyridylthiomethyl group, a benzimidazol-2-ylthiomethyl group, a 1H-imidazo[4,5-b]pyridin-2-ylthiomethyl group, a 2-pyrimidinylthiomethyl group, an aminomethyl group wherein the amino part may be substituted (substituent(s) being methoxyiminomethylcarbonyl group wherein the methyl part is cyano-substituted, or methoxycarbonyl group), an anilinomethyl group, a cyclopropyl group, a 2-chlorophenyl group, a methoxy group, a phenoxy group, a fluorophenoxy group, a chlorophenoxy group, a difluorophenoxy group, a dichlorophenoxy group, a methylphenoxy group, a trifluoromethylphenoxy group, a methoxyphenoxy group, a nitrophenoxy group, a dinitrophenoxy group, a cyanophenoxy group, an optionally substituted 2-pyridyloxy group (the substituent(s) is (are) 1-3 same or different substituent(s) selected from the group consisting of a fluorine atom, a chlorine atom, a methyl group, a trifluoromethyl group and a cyano group), an optionally substituted pyrimidin-2-yloxy group (the substituent(s) is (are) 1-3 same or different substituent(s) selected from the group consisting of a fluorine atom, a chlorine atom, a methyl group, a trifluoromethyl group and a cyano group), a 3-isooxazolyloxy group, a 5-methyl-3-isooxazolyloxy group or an amino group;
most preferably
(e1) R¹ is a C₄-C₅ branched alkyl group, a cyclobutylmethyl group, a phenyl group, a 3-fluorophenyl group or a 3-methylphenyl group;
(e2) R² is a hydrogen atom or a methyl group;
(e3) R³ is a hydrogen atom or a methyl group;
(e4) R⁴ is a hydrogen atom;
(e5) Z is an oxygen atom;
(e6) Ar is a 3-cyanophenyl group;
B is a methyl group, a 4-chloro-1-pyrazolylmethyl group, a 4-bromo-1-pyrazolylmethyl group, a hydroxymethyl group, a methoxymethyl group, a 3-pyridylmethoxymethyl group, a cyanomethoxymethyl group, a phenoxymethyl group, a (4-fluorophenoxy)methyl group, a (4-chlorophenoxy)methyl group, a 3-isooxazolyloxymethyl group, a 5-methyl-3-isooxazolyloxymethyl group, a carbamoyloxymethyl group, a cyanomethylthiomethyl group, a 2-pyrimidinylthiomethyl group, a cyclopropyl group, a methoxy group, a phenoxy group, a 2-fluorophenoxy group, a 3-fluorophenoxy group, a 4-fluorophenoxy group, a 4-chlorophenoxy group, a 2-methylphenoxy group, a 2-cyanophenoxy group, a 3-cyanophenoxy group, an optionally substituted 2-pyridyloxy group (the substituent(s) is (are) 1-3 same or different substituent(s) selected from the group consisting of a chlorine atom, a methyl group, a trifluoromethyl group and a cyano group), an optionally substituted pyrimidin-2-yloxy group (the substituent(s) is (are) 1-3 same or different substituent(s) selected from the group consisting of a chlorine atom, a methyl group, a trifluoromethyl group and a cyano group), a 5-methyl-3-isooxazolyloxy group or an amino group.

Representative compounds of the present invention will be exemplified in the following Table 1, but the present invention is not limited to these compounds.

Hereinafter, "Me" denotes a methyl group, "Et" denotes an ethyl group, "Pr" denotes a propyl group, "iPr" denotes an isopropyl group, "cPr" denotes a cyclopropyl group, "Bu" denotes a butyl group, "iBu" denotes an isobutyl group, "sBu" denotes a sec-butyl group, "tBu" denotes a tert-butyl group, "cBu" denotes a cyclobutyl group, "neoPen" denotes a neopentyl group, "cPen" denotes a cyclopentyl group, "Hex" denotes a hexyl group, "cHex" denotes a cyclohexyl group, "Hep" denotes a heptyl group, "Ph" denotes a phenyl group, "C₆H₄-3-CN" denotes a 3-cyanophenyl group, "C₆H₃-2,4-F₂" denotes a 2,4-difluorophenyl group, "Ac" denotes an acetyl group, "3-oxo" denotes a 3-oxo group, "p-Tol" denotes a p-tolyl group, "3-Isox" denotes a 3-isooxazolyl group, "3-Pyr" denotes a 3-pyridyl group, "3-Pyzn" denotes a 3-pyridazinyl group, "2-Pym" denotes a 2-pyrimidinyl group, "1-Pyza" denotes a 1-pyrazolyl group, "4-Mor" denotes a morpholino group, "2-Thi" denotes a 2-thienyl group, "2-Fur" denotes a 2-furyl group, "3-Pyrr" denotes a 3-pyrrolyl group, "3-Ind" denotes a 3-indolyl group, "2-Pyz" denotes a 2-pyrazinyl group, "2-Thiz" denotes a 2-thiazolyl group, "2-Quin" denotes a 2-quinolyl group, "3-iQuin" denotes a 3-isoquinolyl group, "Phtl" denotes an N,N-phthaloyl group, "2-Imid" denotes a 2-imidazolyl group, "2-Np" denotes a 2-naphthyl group, "TBDMS" denotes a tert-butyl dimethylsilyl group, "TIPS" denotes a triisopropylsilyl group, "TBDPS" denotes a t-butyldiphenylsilyl group, "SEM" denotes a trimethylsilylethoxymethyl group, "THP" denotes a 2-tetrahydropyranyl group, "2-THF" denotes a 2-tetrahydrofuranyl group, "G1" denotes a 4-cyclohexenyl group, "4-Me-G2" denotes a 4-methyl-1,2,3-thiadiazol-5-yl group, "G3" denotes a 3-cyclopentenyl group, "G4" denotes a group represented by the formula: 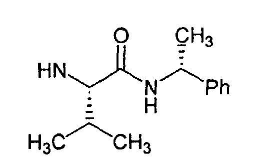 "G5" denotes a group represented by the formula: 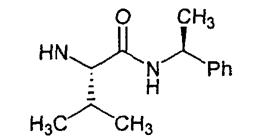 , "G6" denotes a 2-methyl-1,4-cyclohexadien-3-yl group, "5-G7" denotes a 1,3-dioxan-5-yl group, "G8" denotes a 3-benzothiophenyl group, "G9" denotes a 3-benzisooxazolyl group, "G10" denotes a benzisothiazol-1,1-dioxide-3-yl group, "2-G11" denotes a 2-benzothiazolyl group, "G12" denotes a 4,5-dihydrothiazol-2-yl group, "G13" denotes a 1,3,4-triazol-2-yl group, "5-Me-G14" denotes a 5-methyl-1,3,4-thiadiazol-2-yl group, "G15" denotes a 2-benzooxazolyl group, "G16" denotes a 2-benzimidazolyl group, "G17" denotes a 1H-imidazo[4,5-b]pyridin-2-yl group, "G18" denotes a 4-oxo-3-quinazolinyl group, "G19" denotes a 1,2,4-triazol-1-yl group, "G20" denotes a [2] norbornyl group, "G21" denotes a 1,2,4-oxadiazol-3-yl group, "G22" denotes a 1-phthalazinyl group, "4,6-(OMe)₂-G23" denotes a 4,6-dimethoxy-1,3,5-triazin-2-yl group, "G24" denotes a 1,2,3-benzothiadiazol-7-yl group, "G25" denotes an N-(1-isobutyl-5-methyl-4-pyrazolyl)-N-(phenoxyacetyl)aminomethyl group, "G26" denotes a pentadecyl group, "3-Me-G27" denotes a 3-methyl-3-oxetanyl group, "(Z)" denotes that a geometrical isomer is a Z compound, "(E)" denotes that a geometrical isomer is an E compound, and "(S)" denotes that an optical isomer is a S compound, respectively.

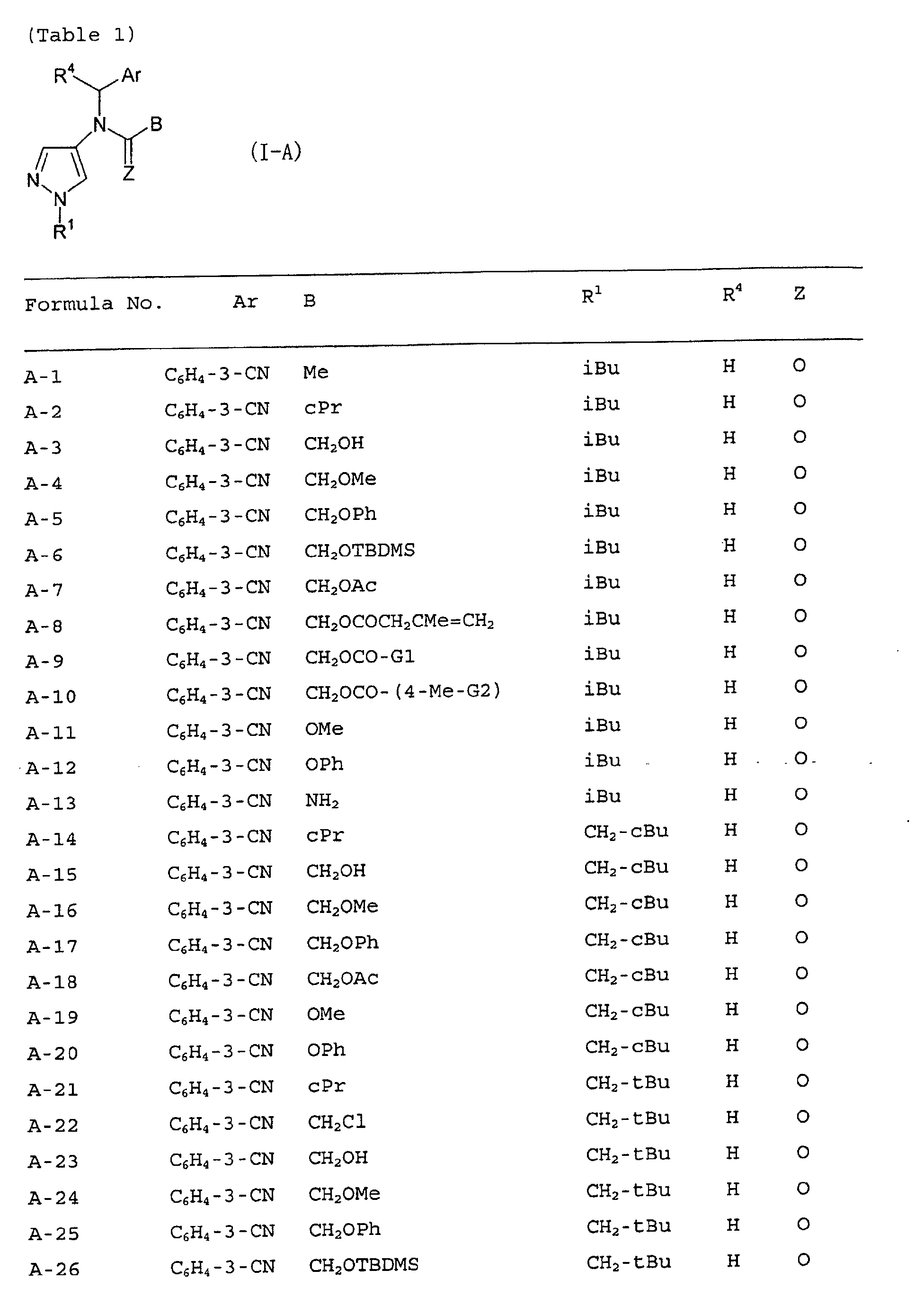

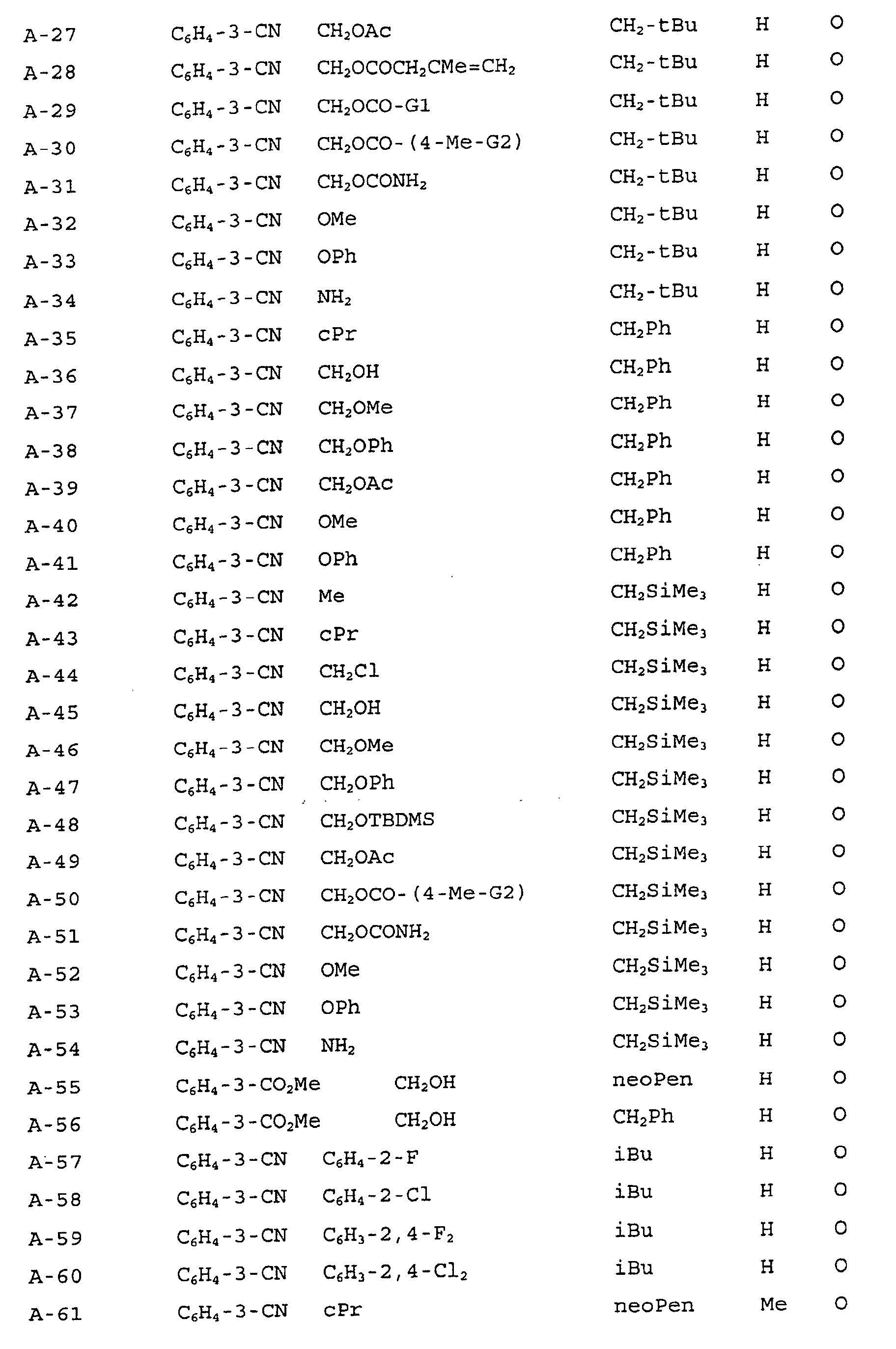

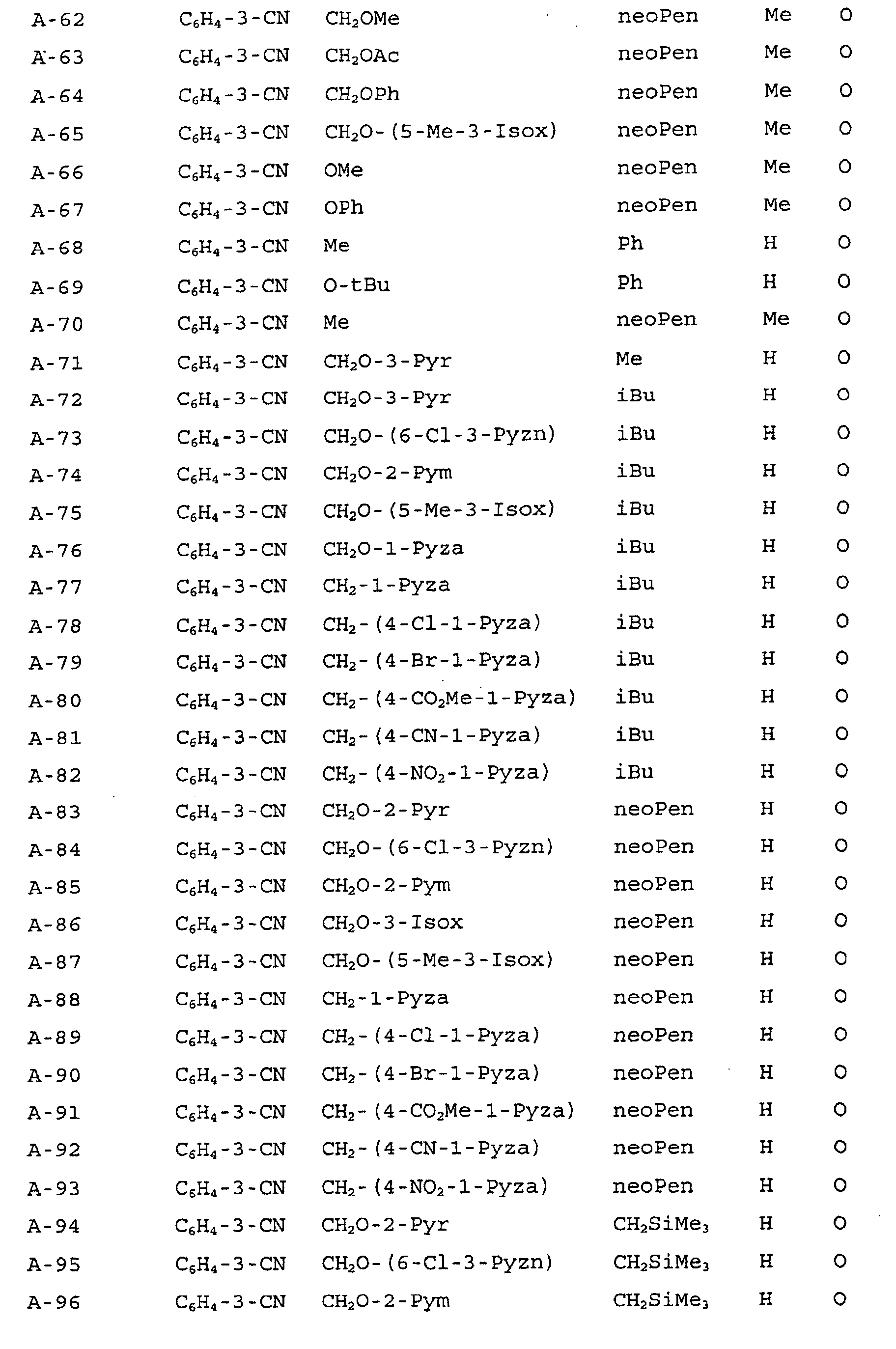

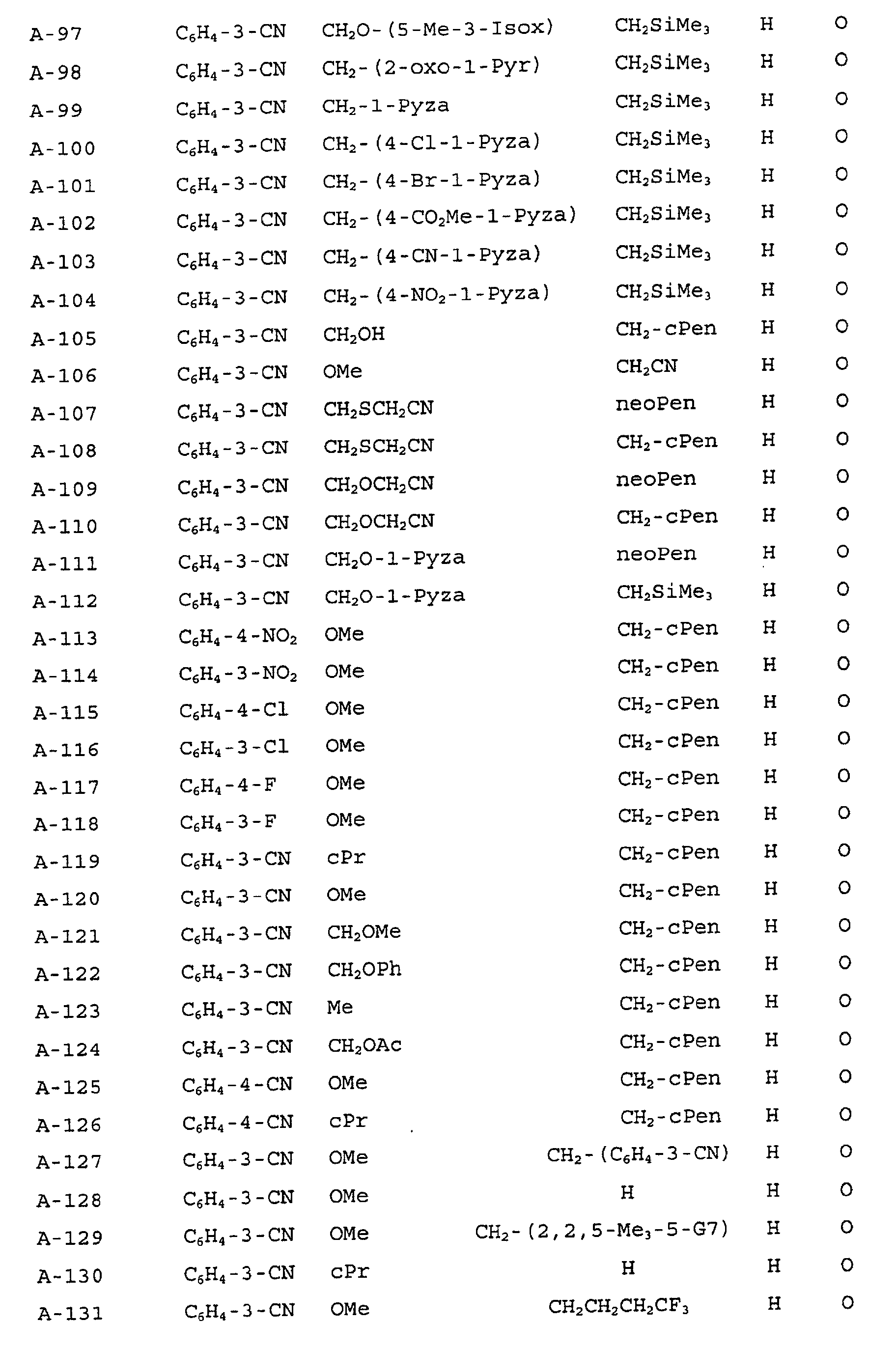

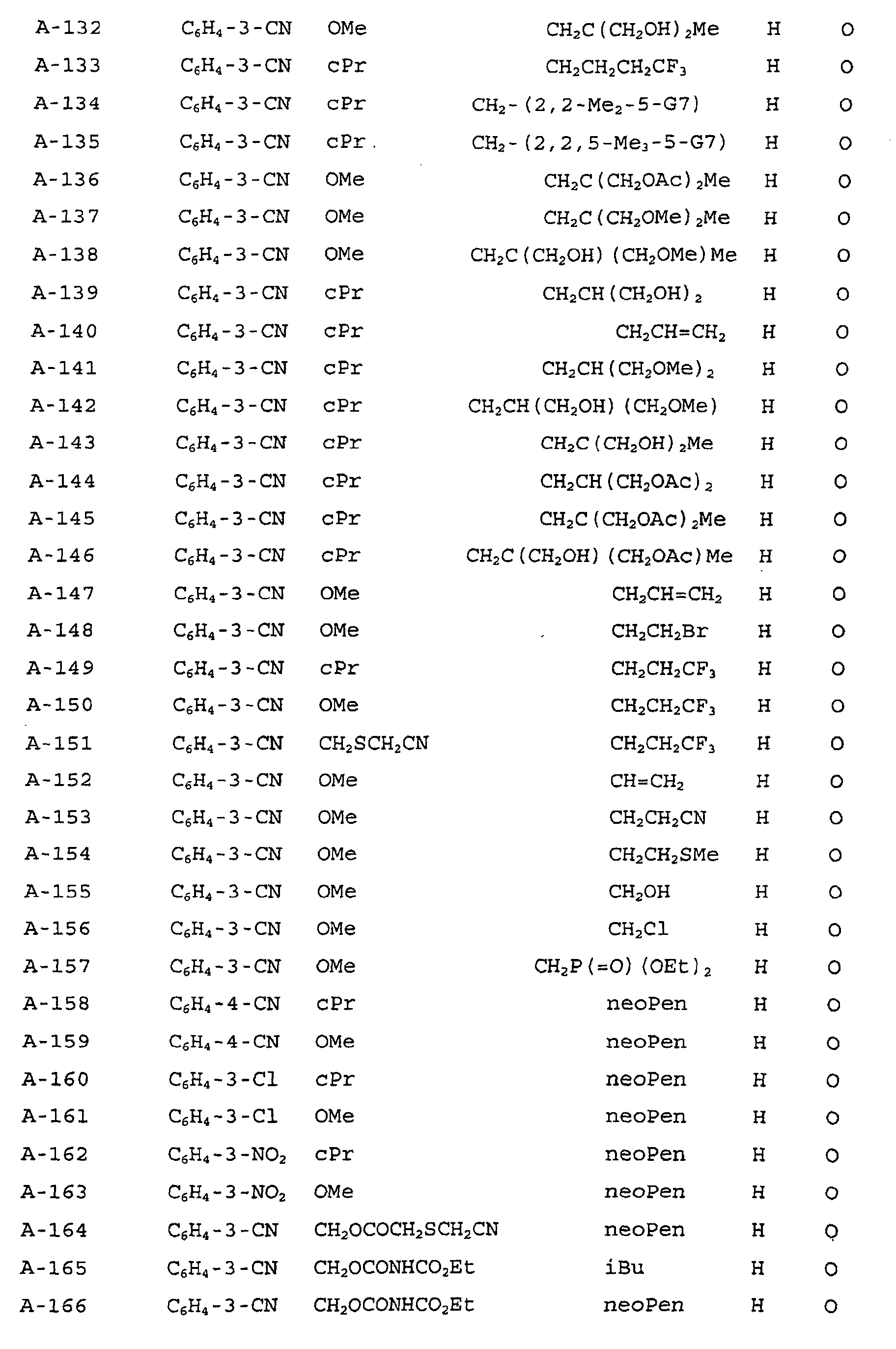

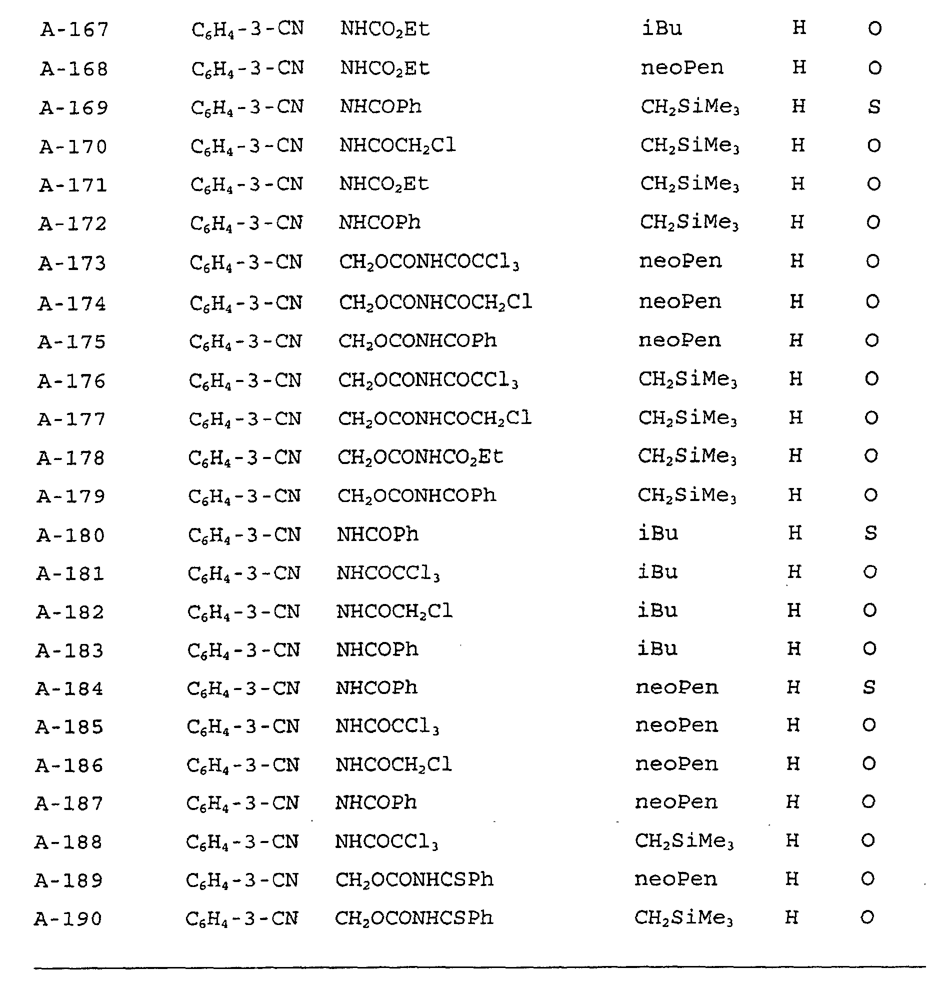

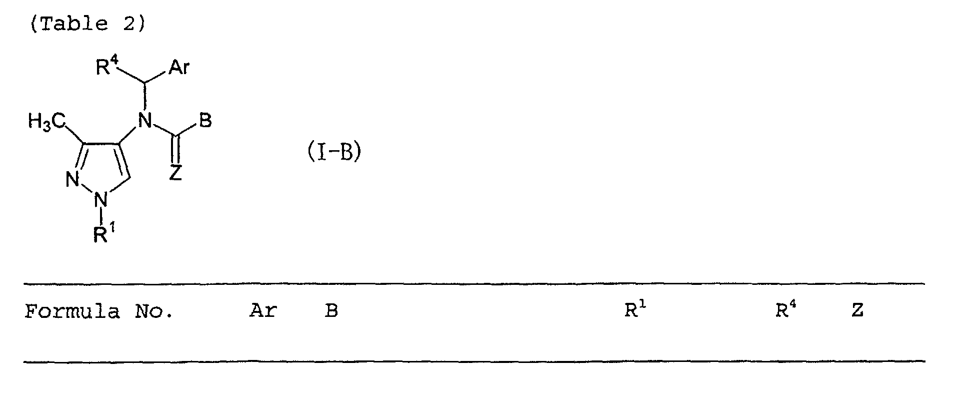

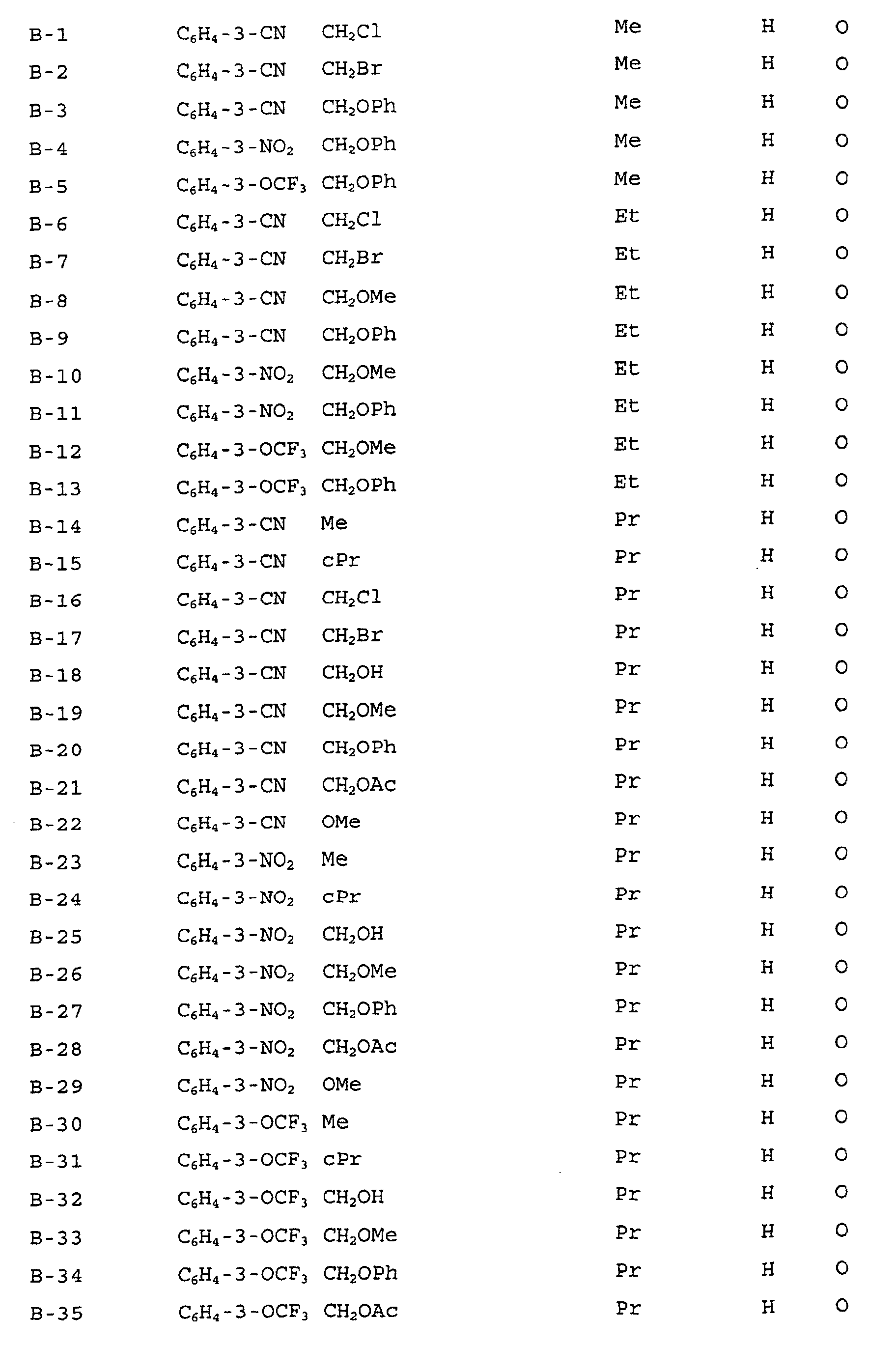

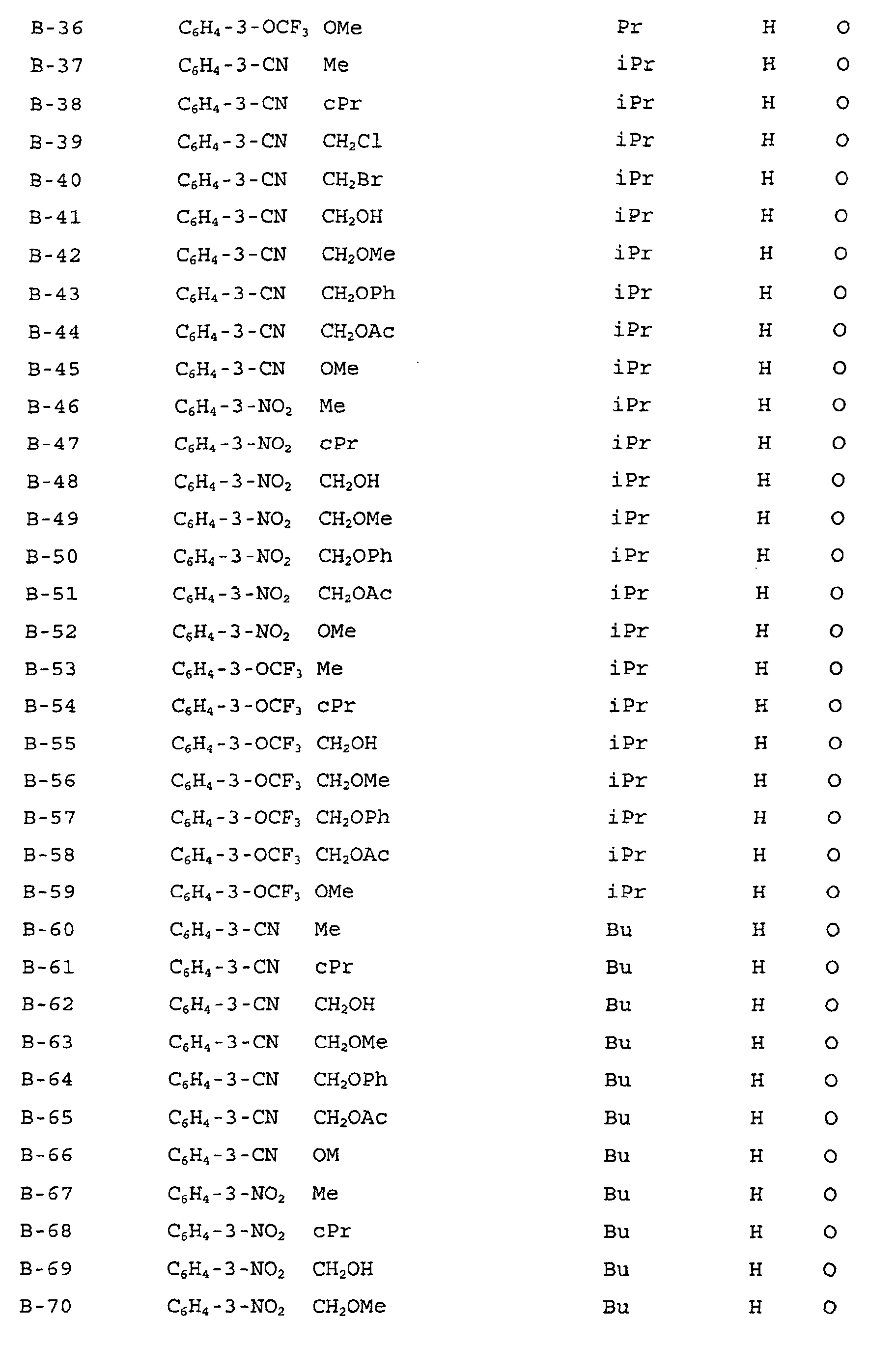

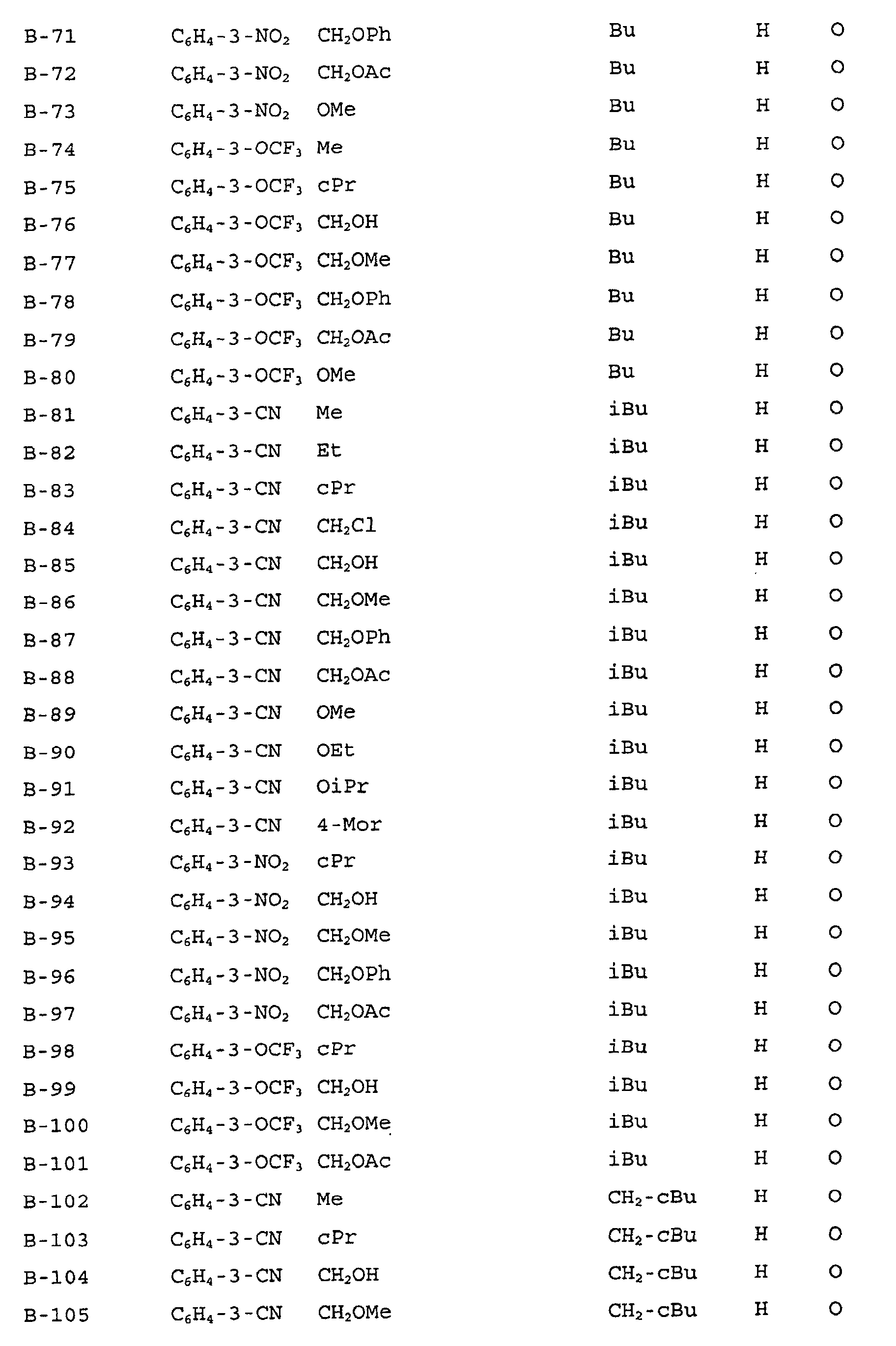

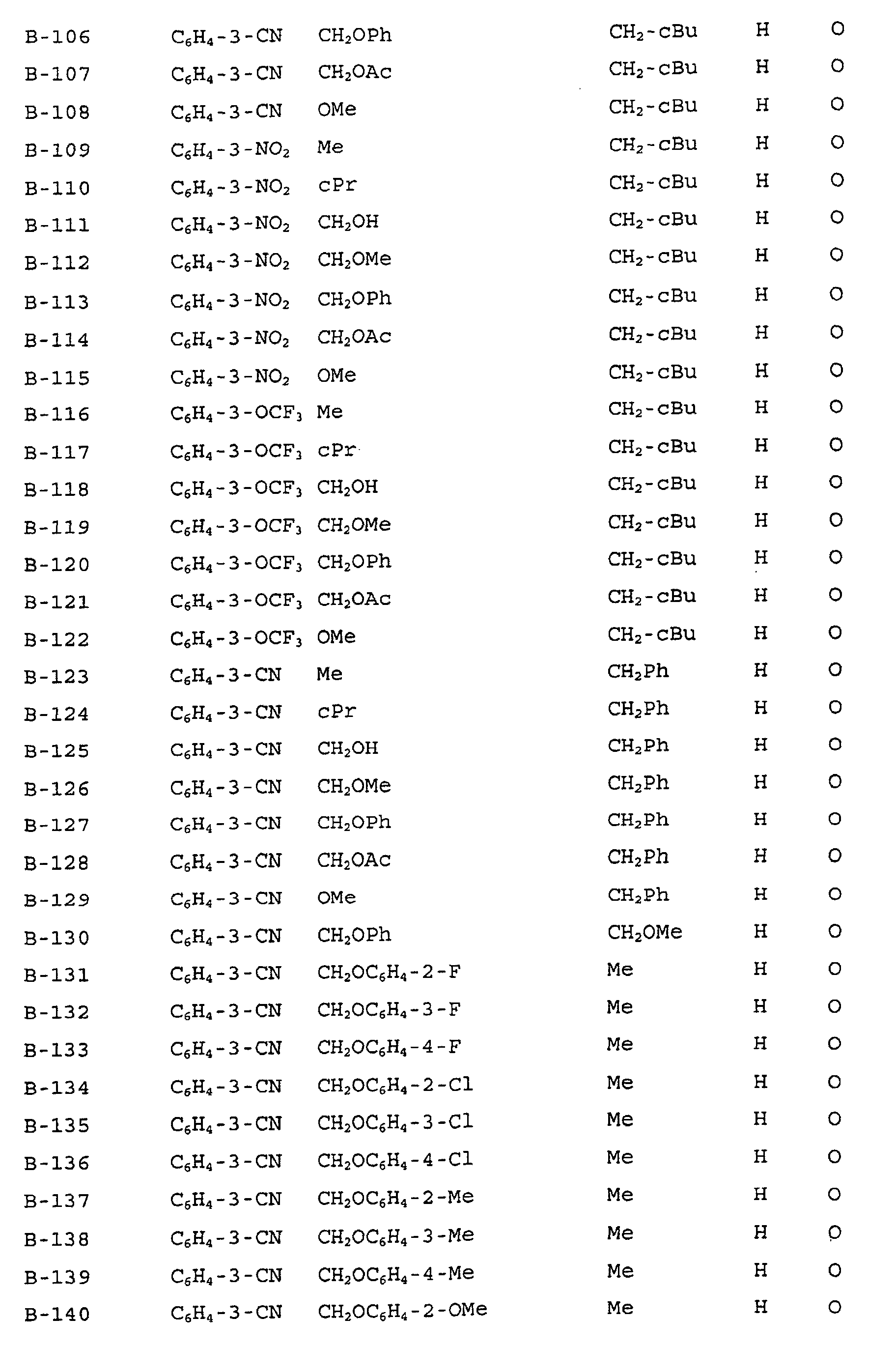

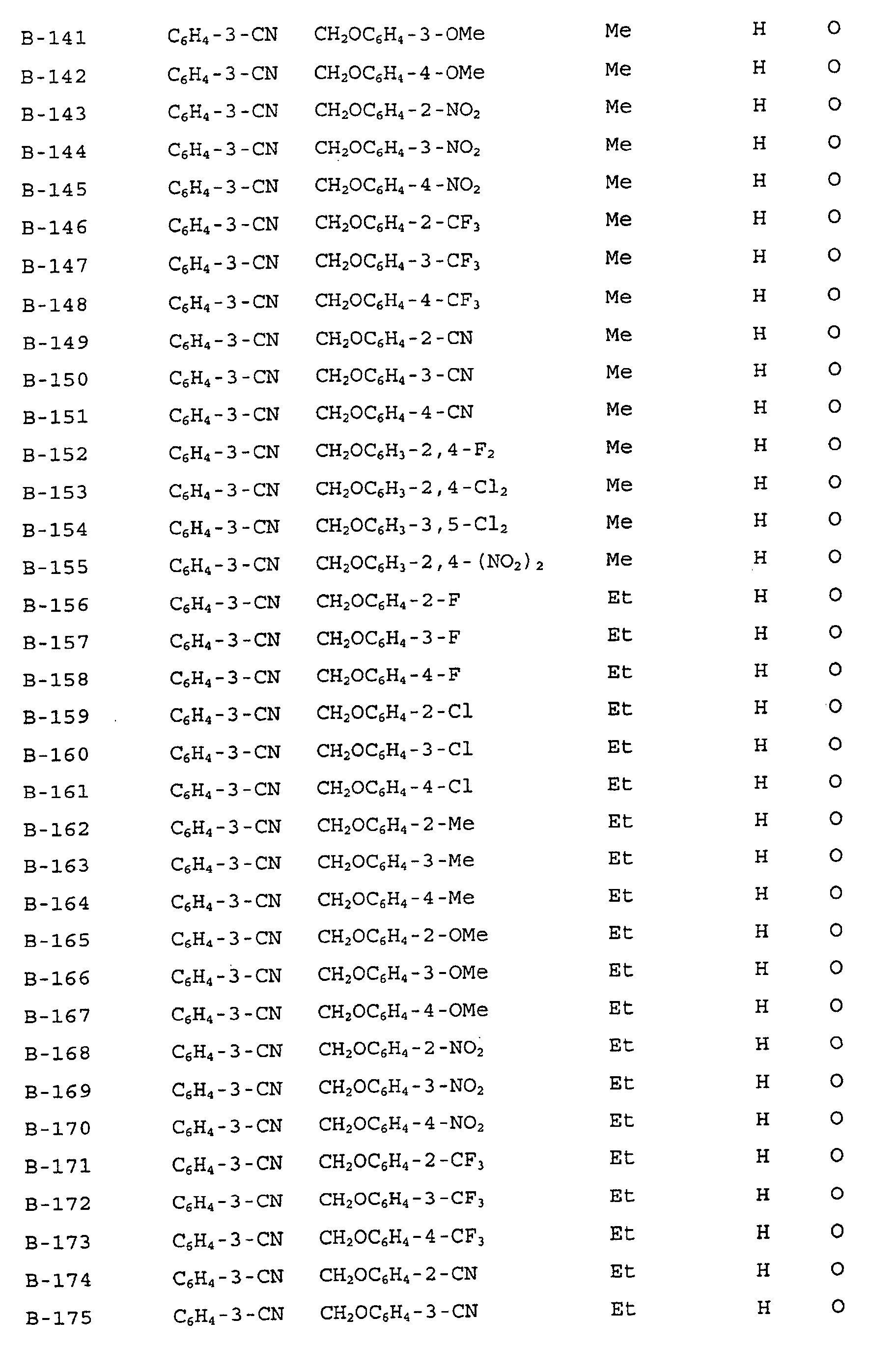

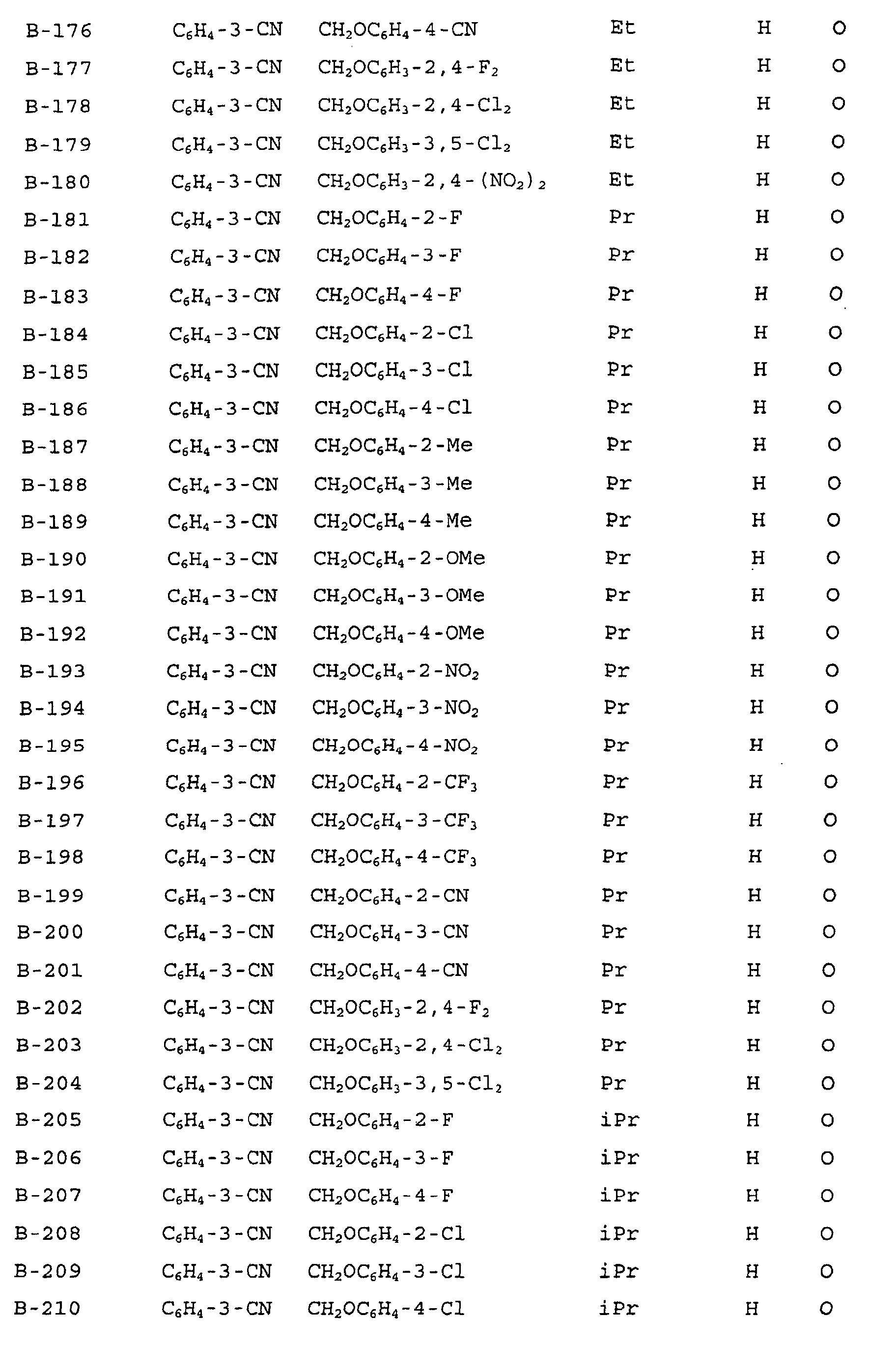

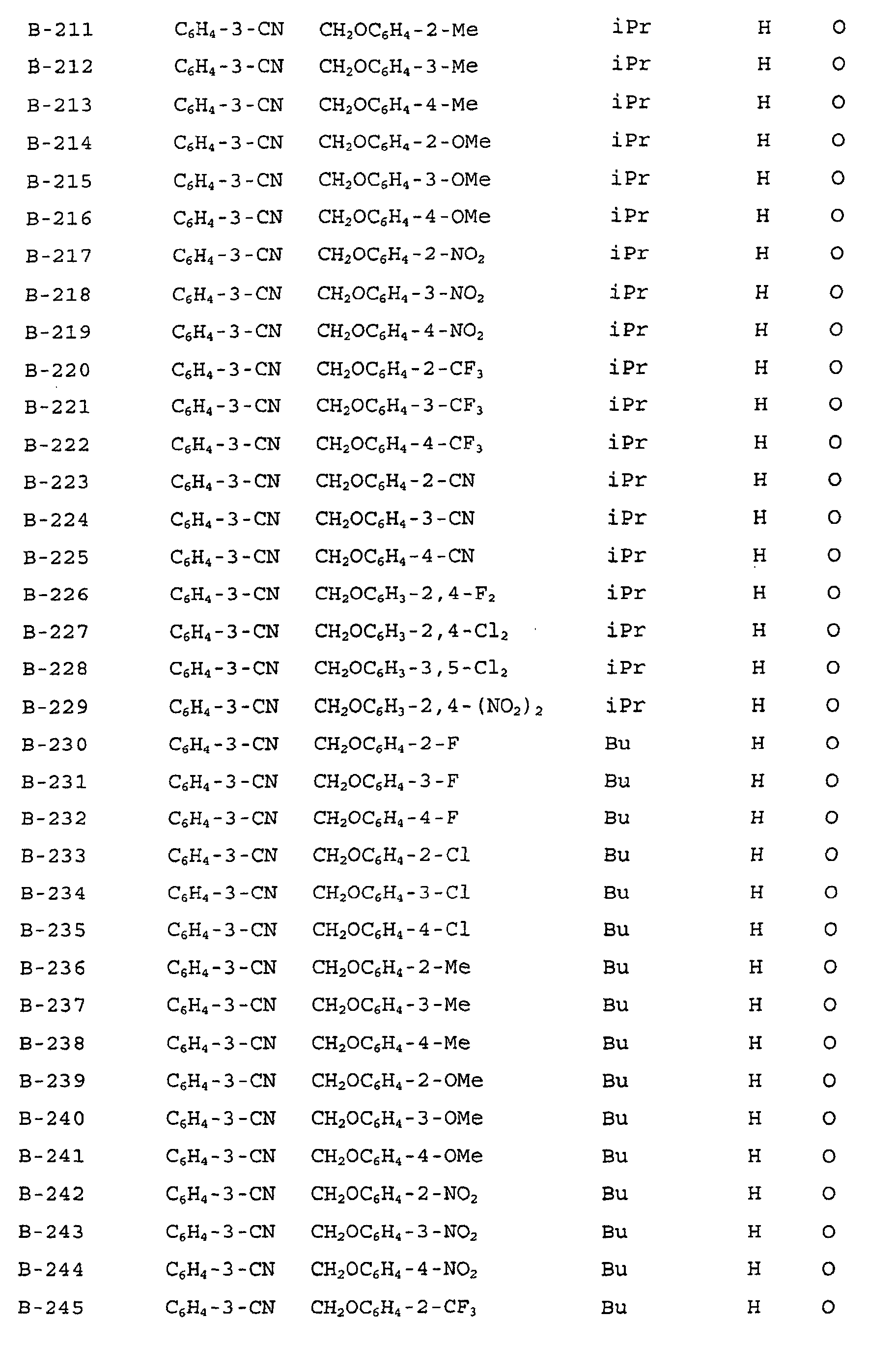

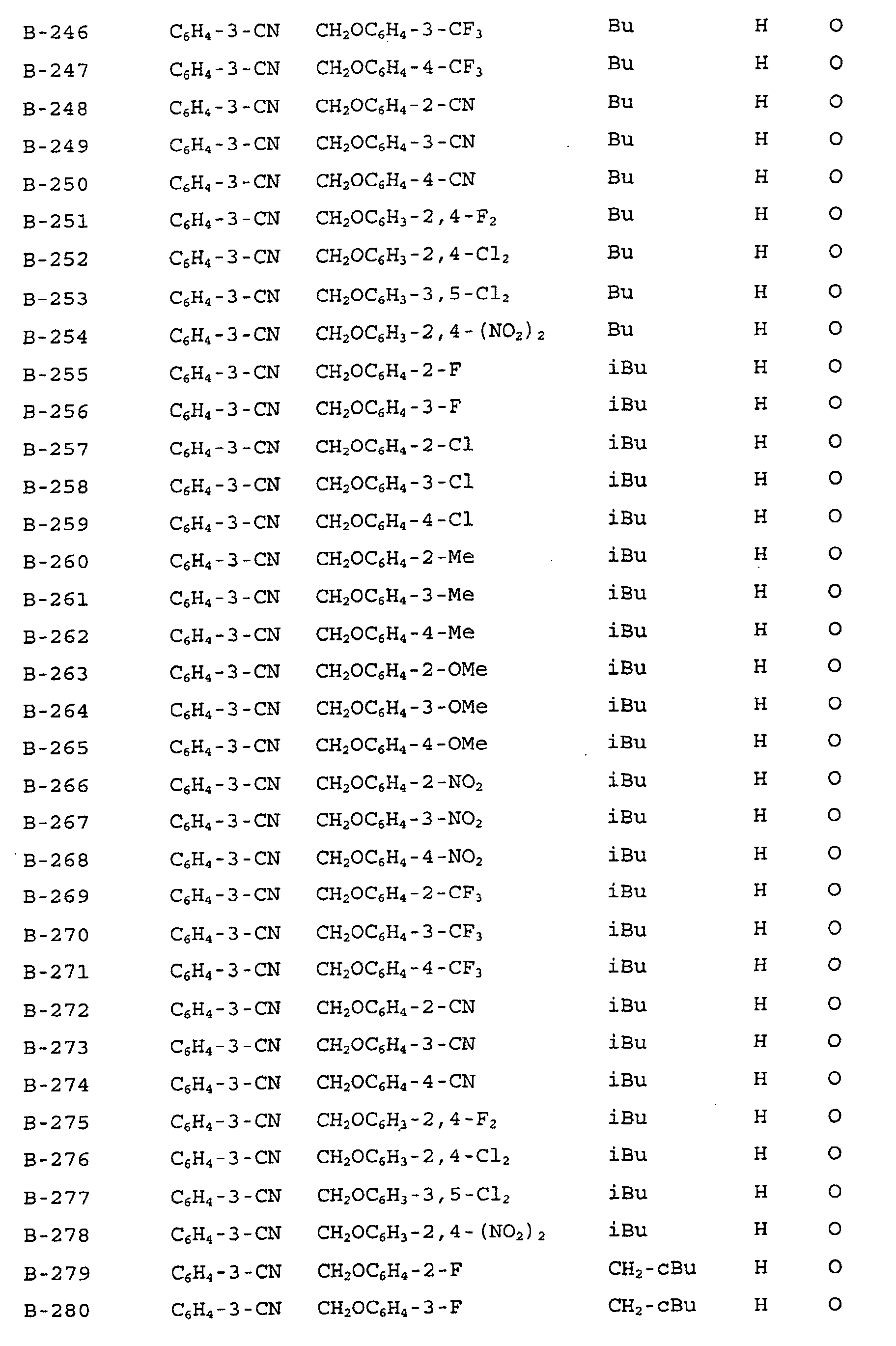

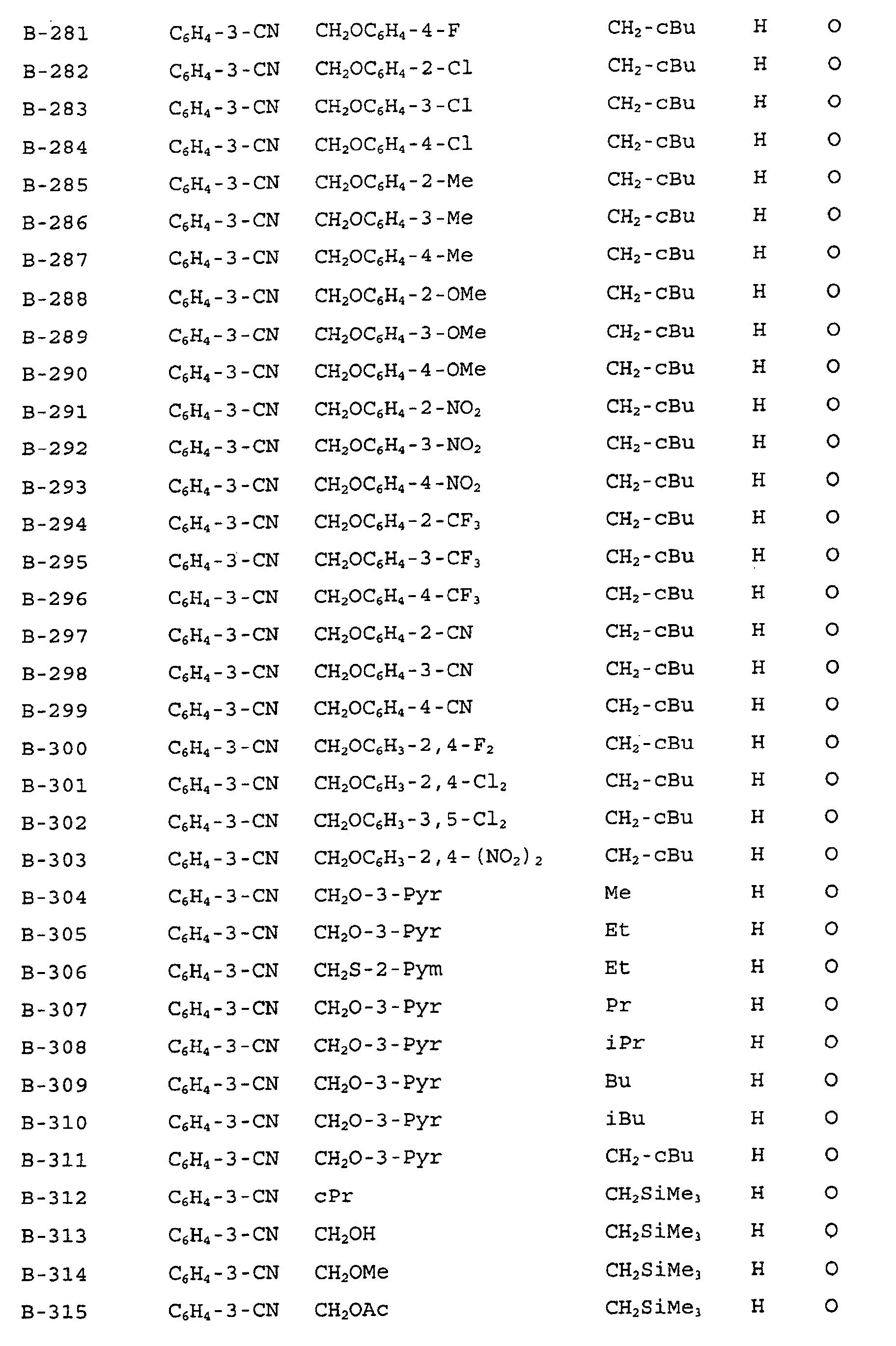

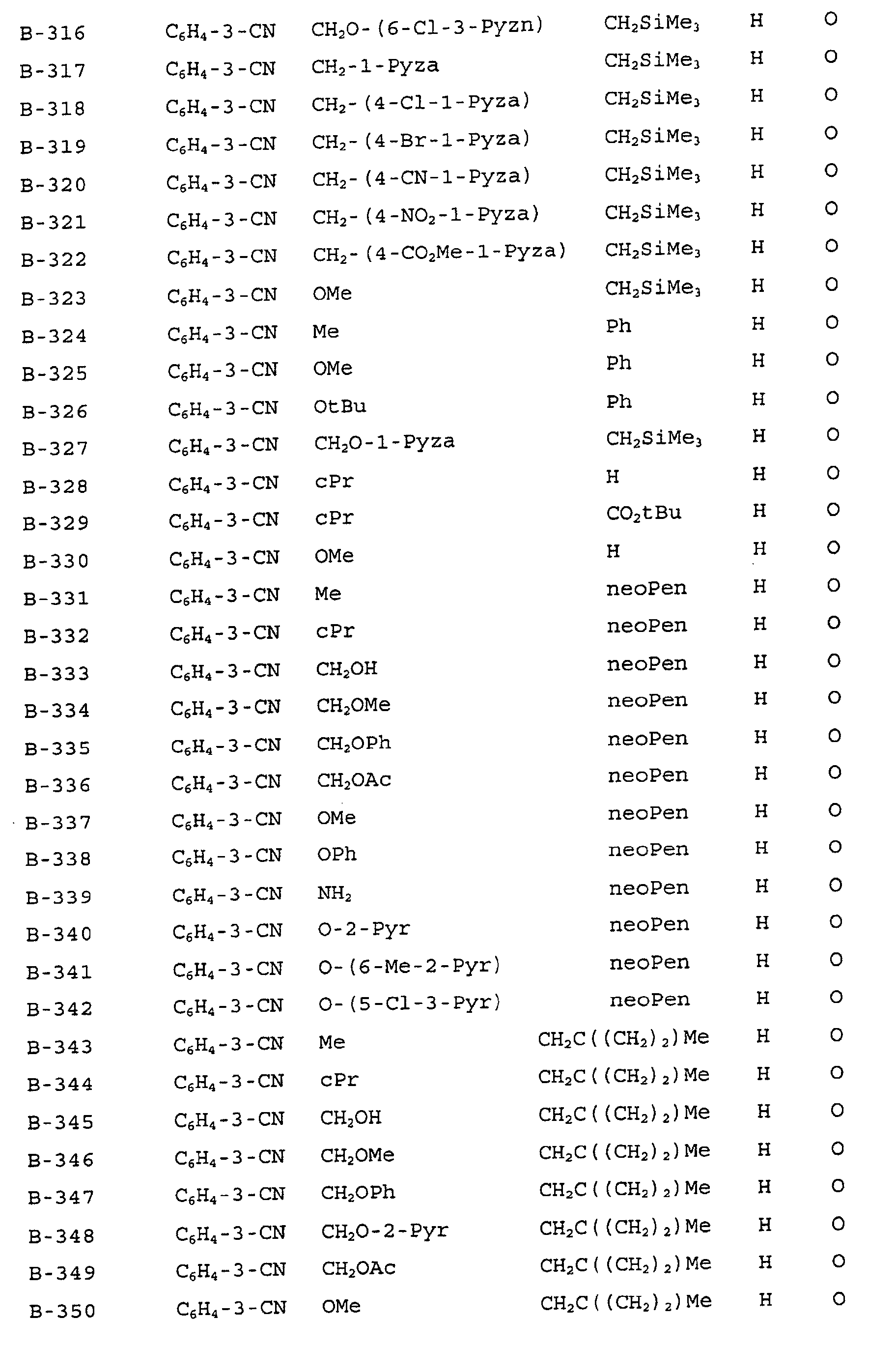

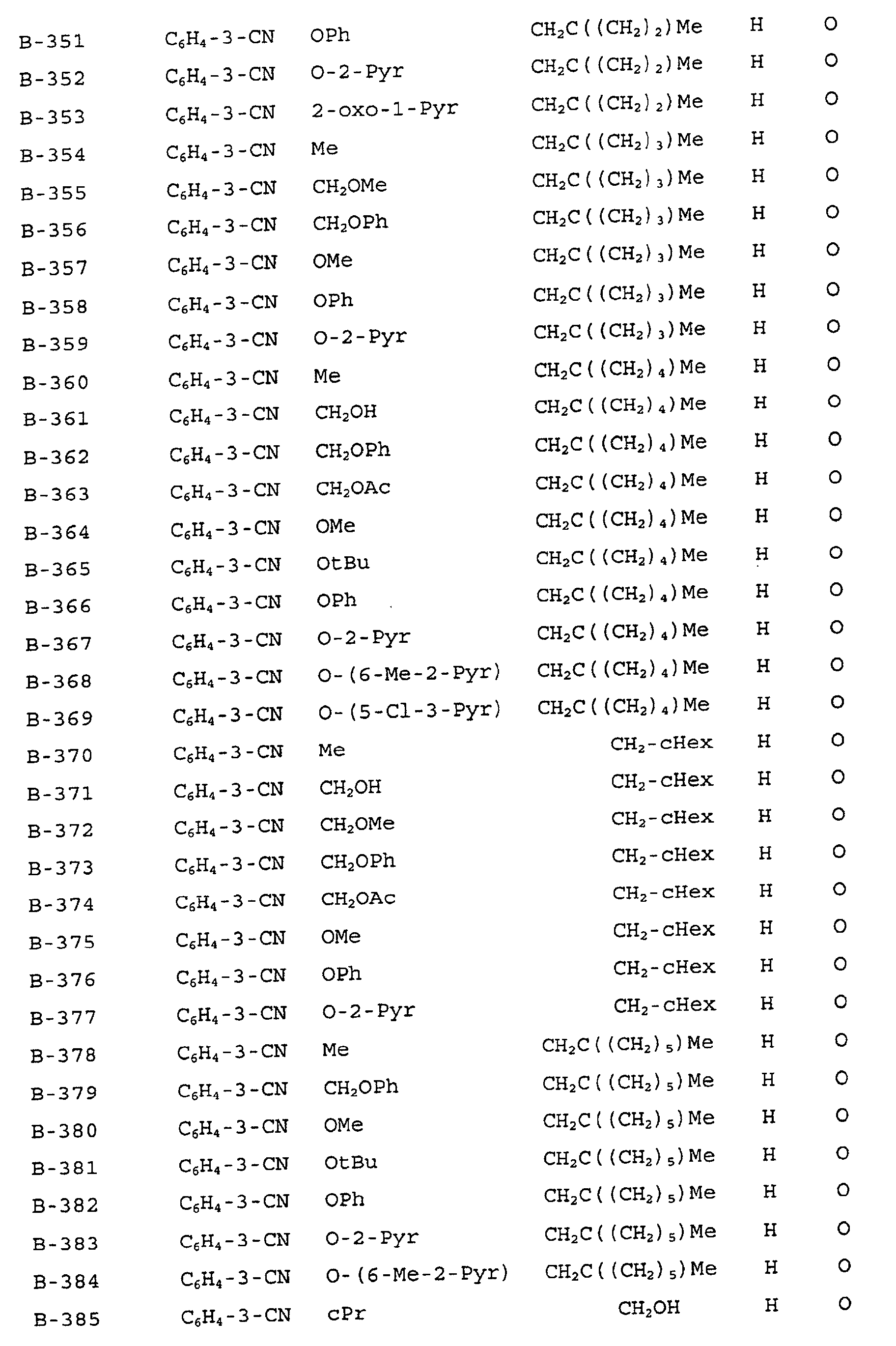

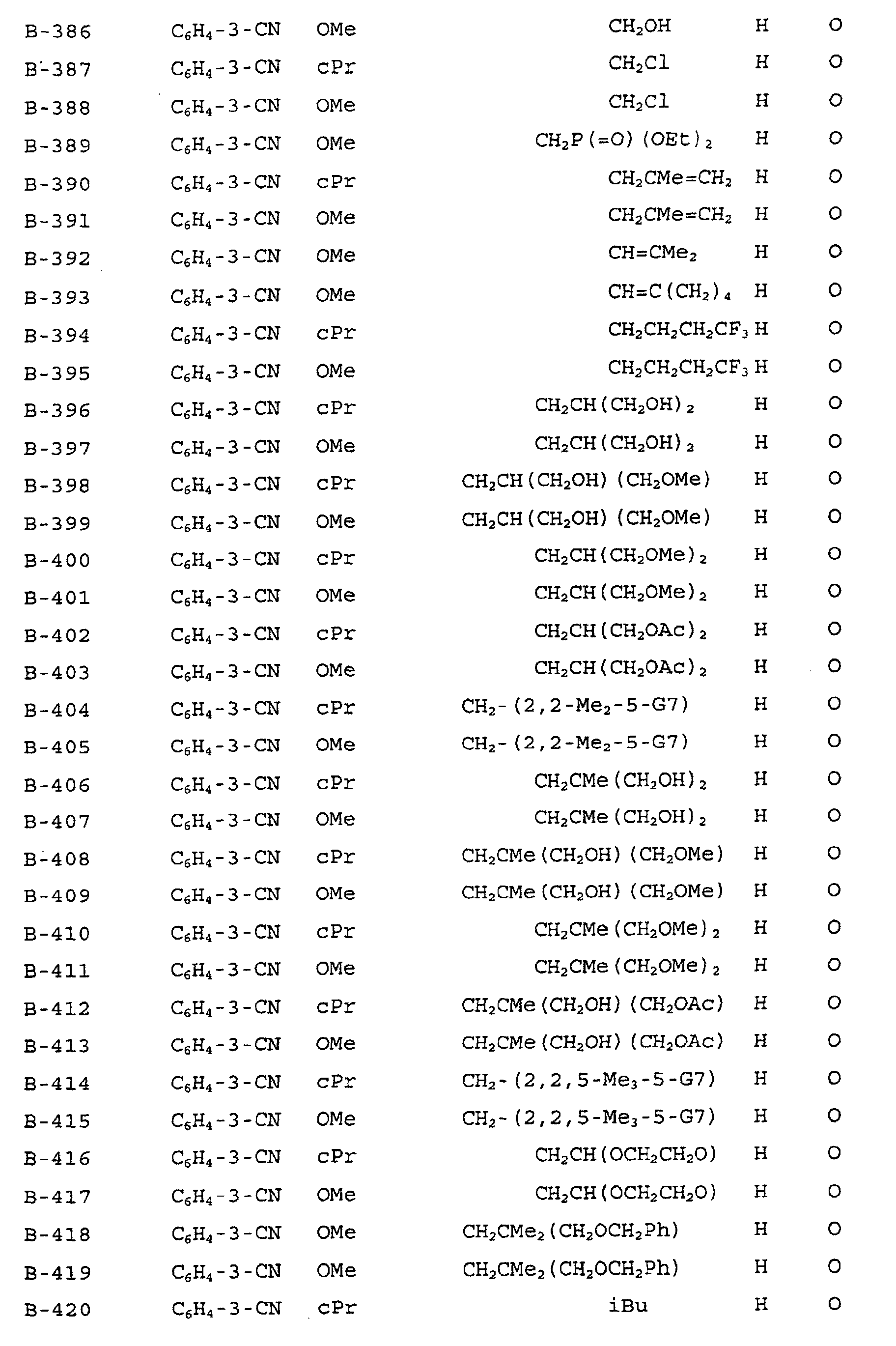

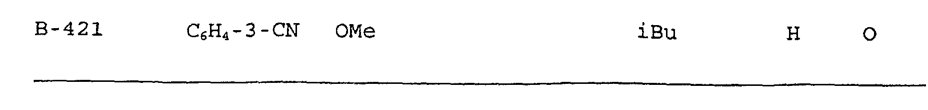

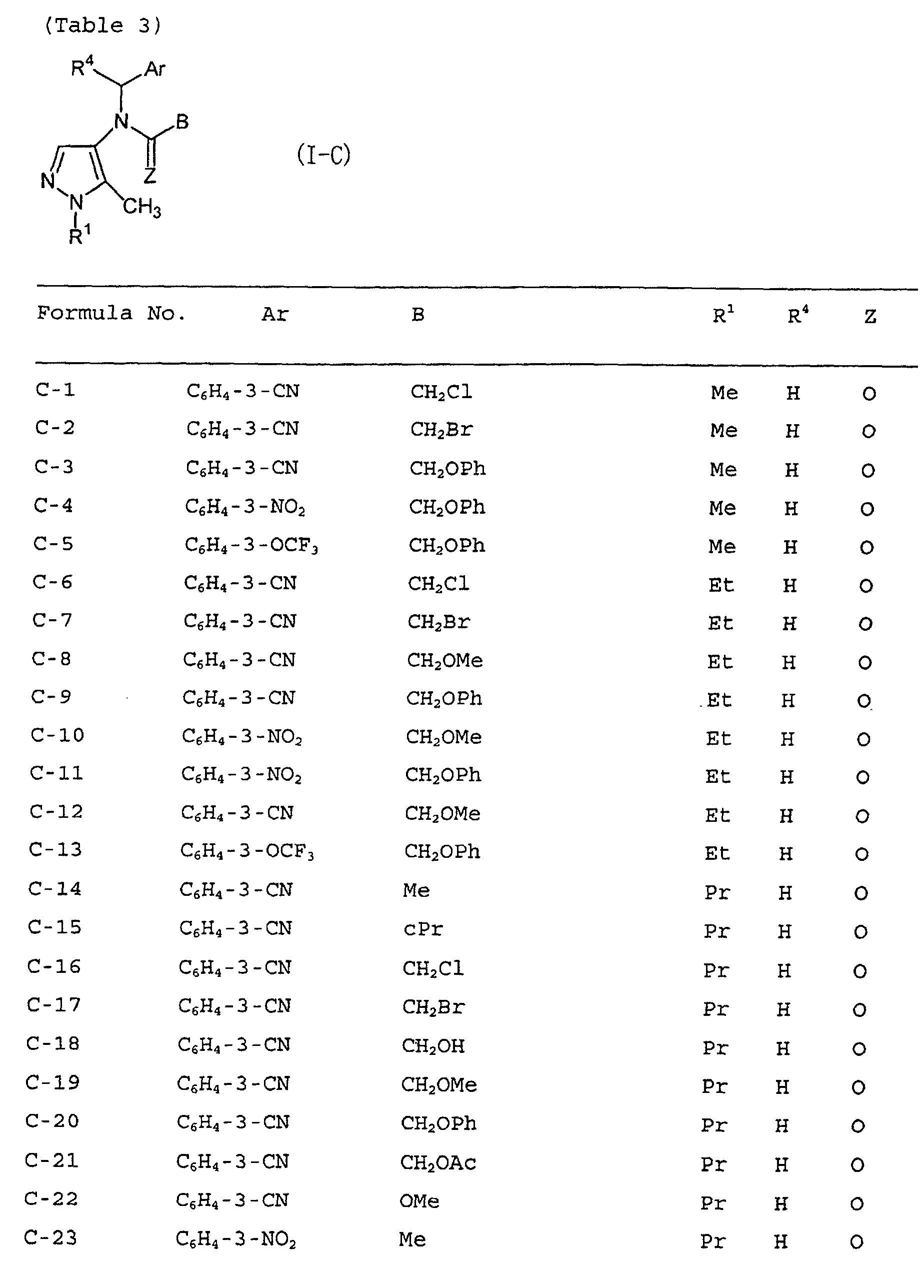

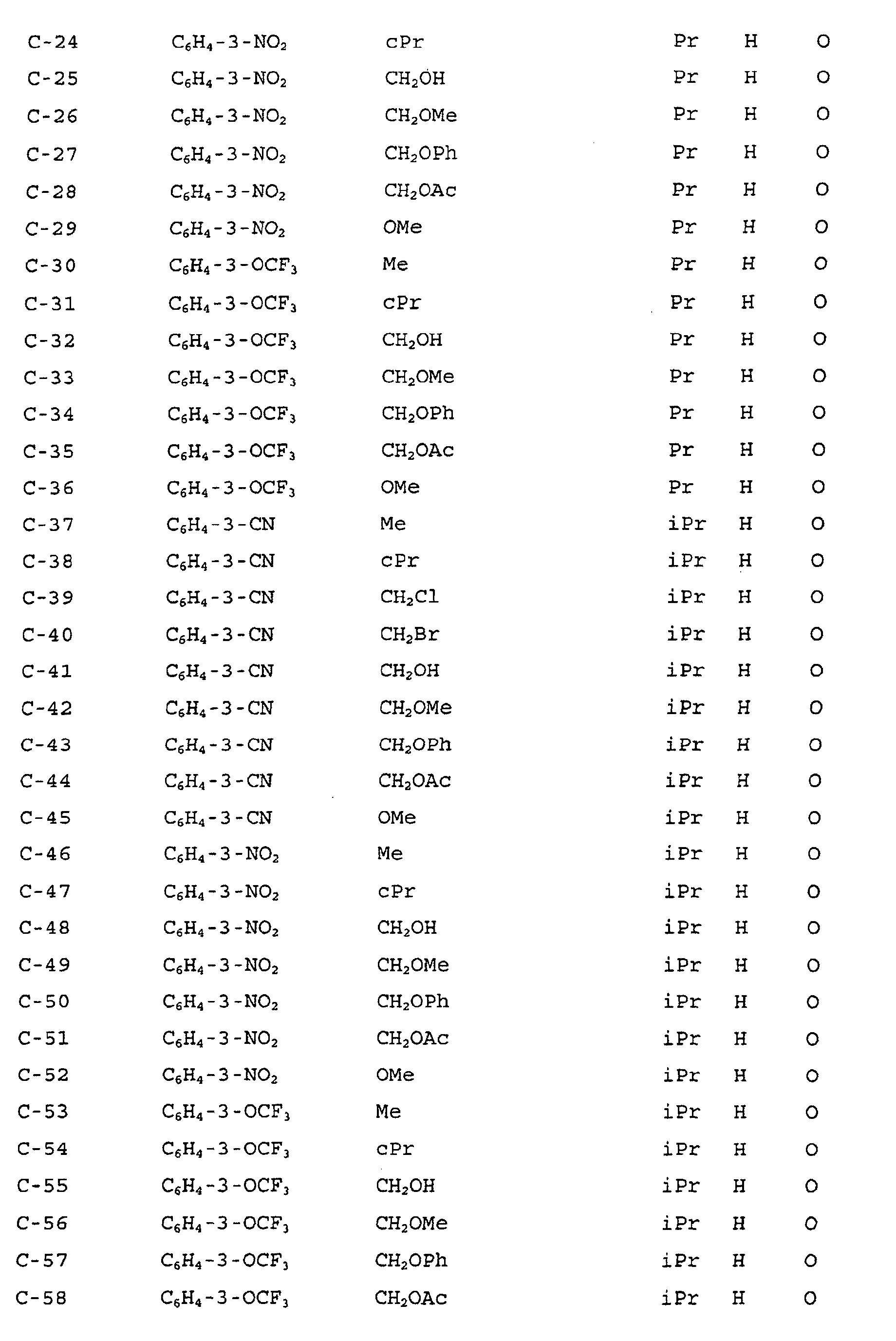

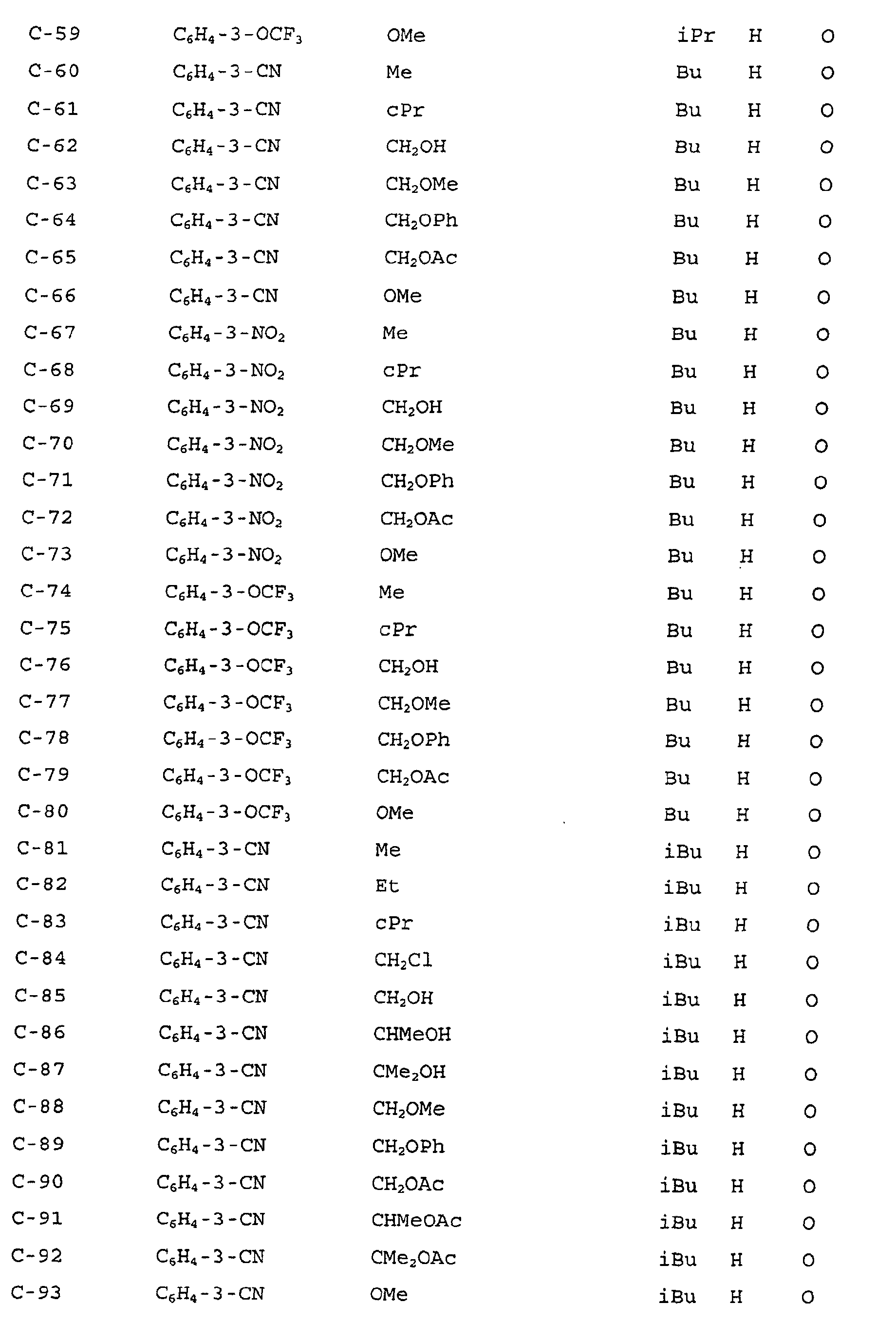

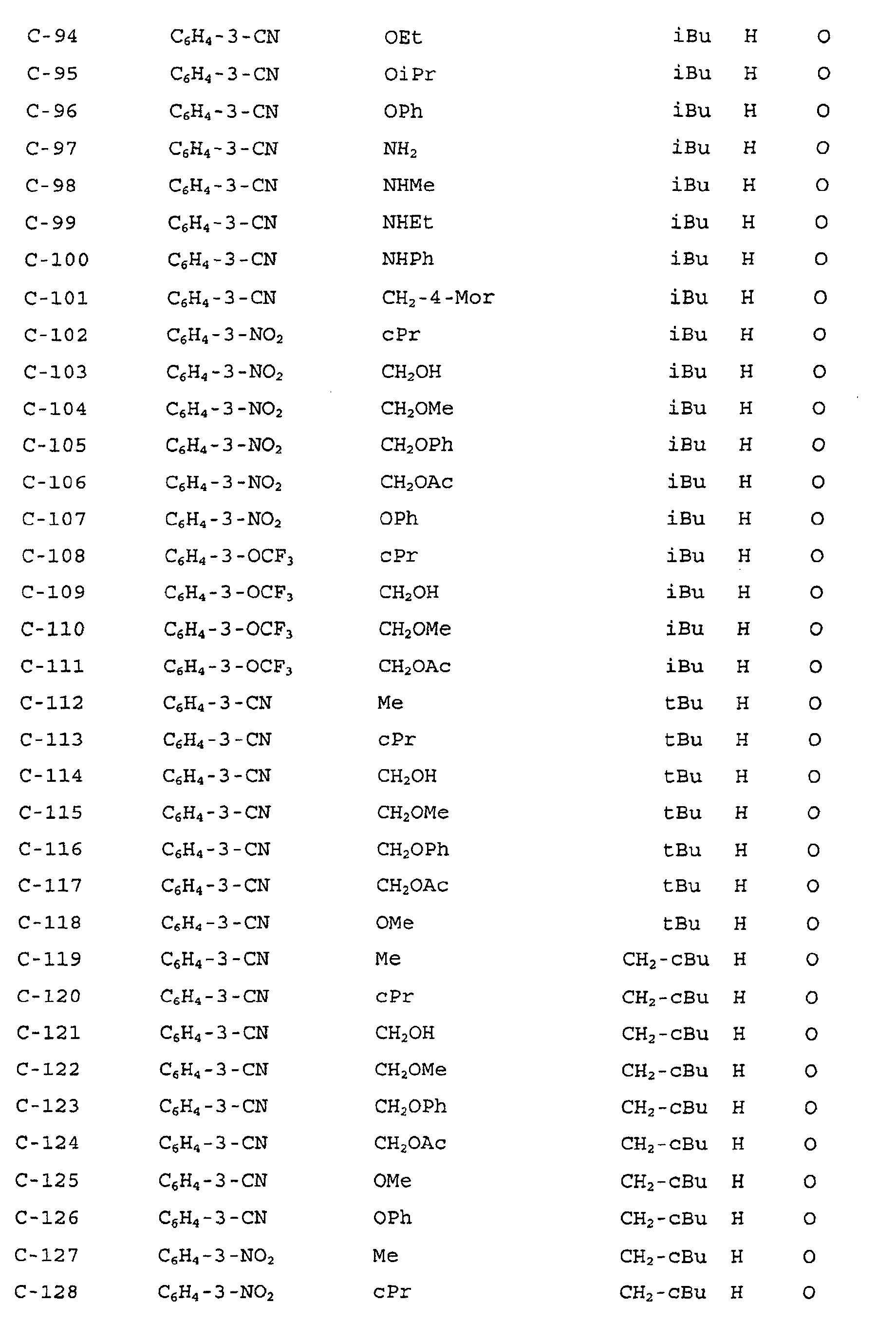

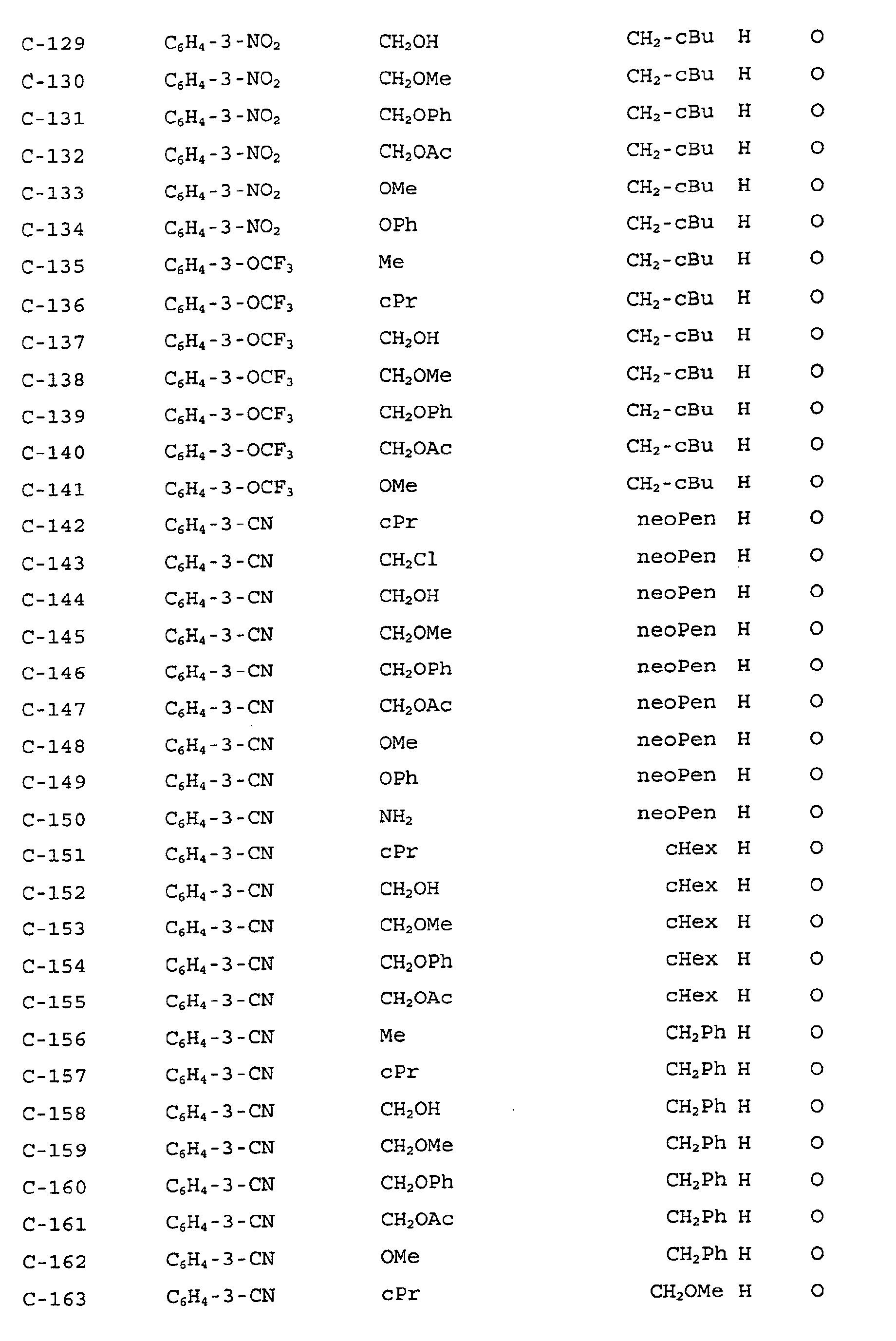

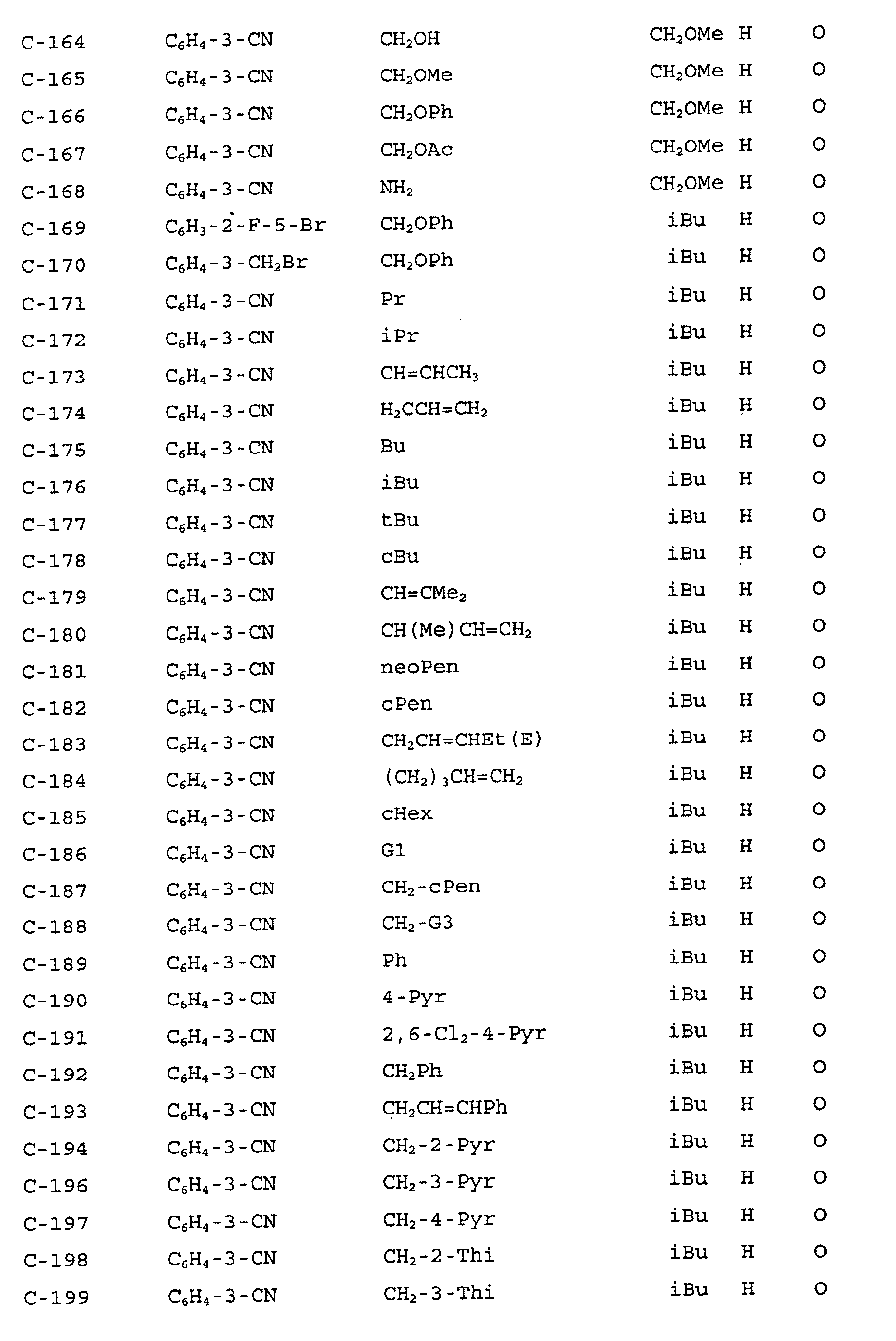

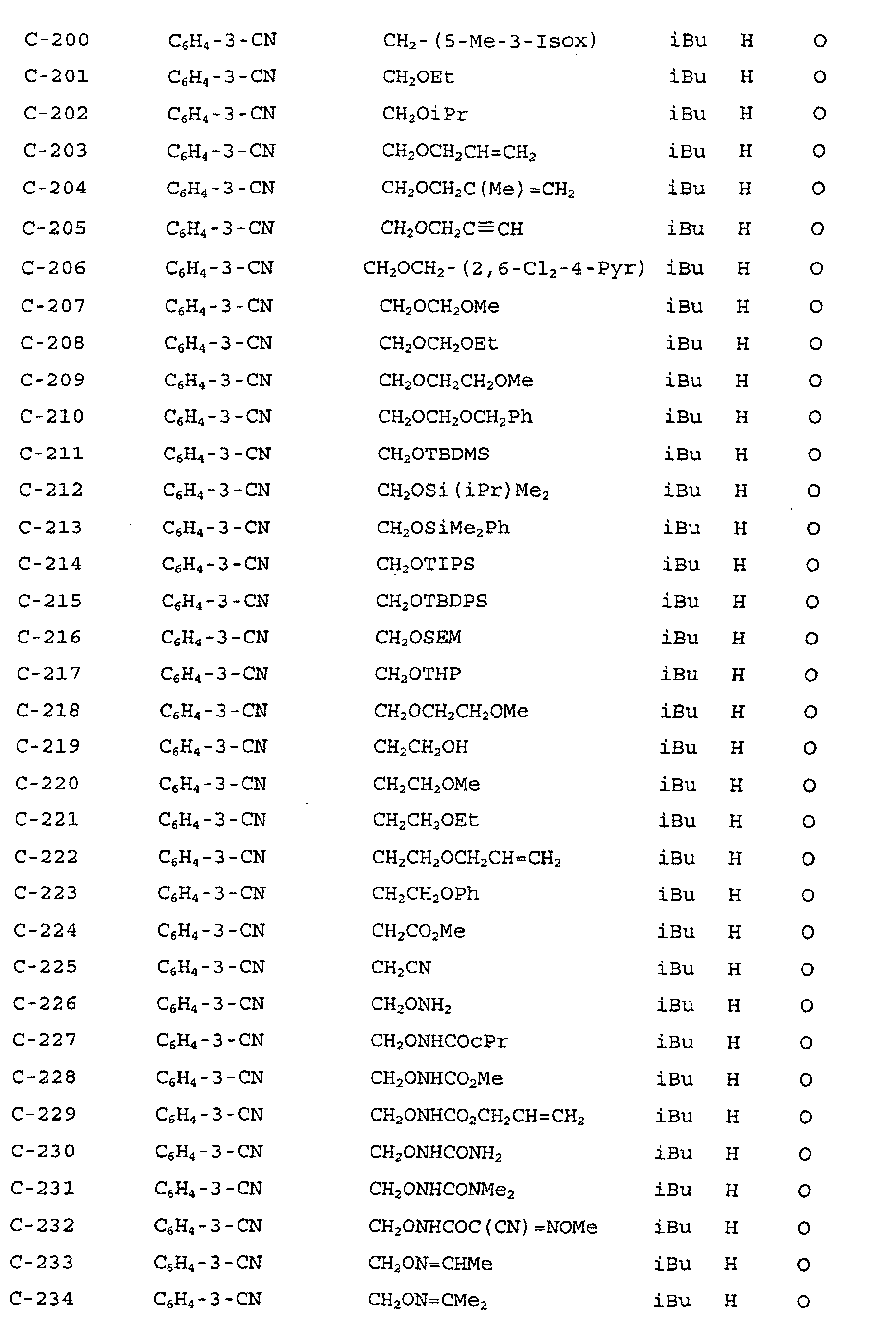

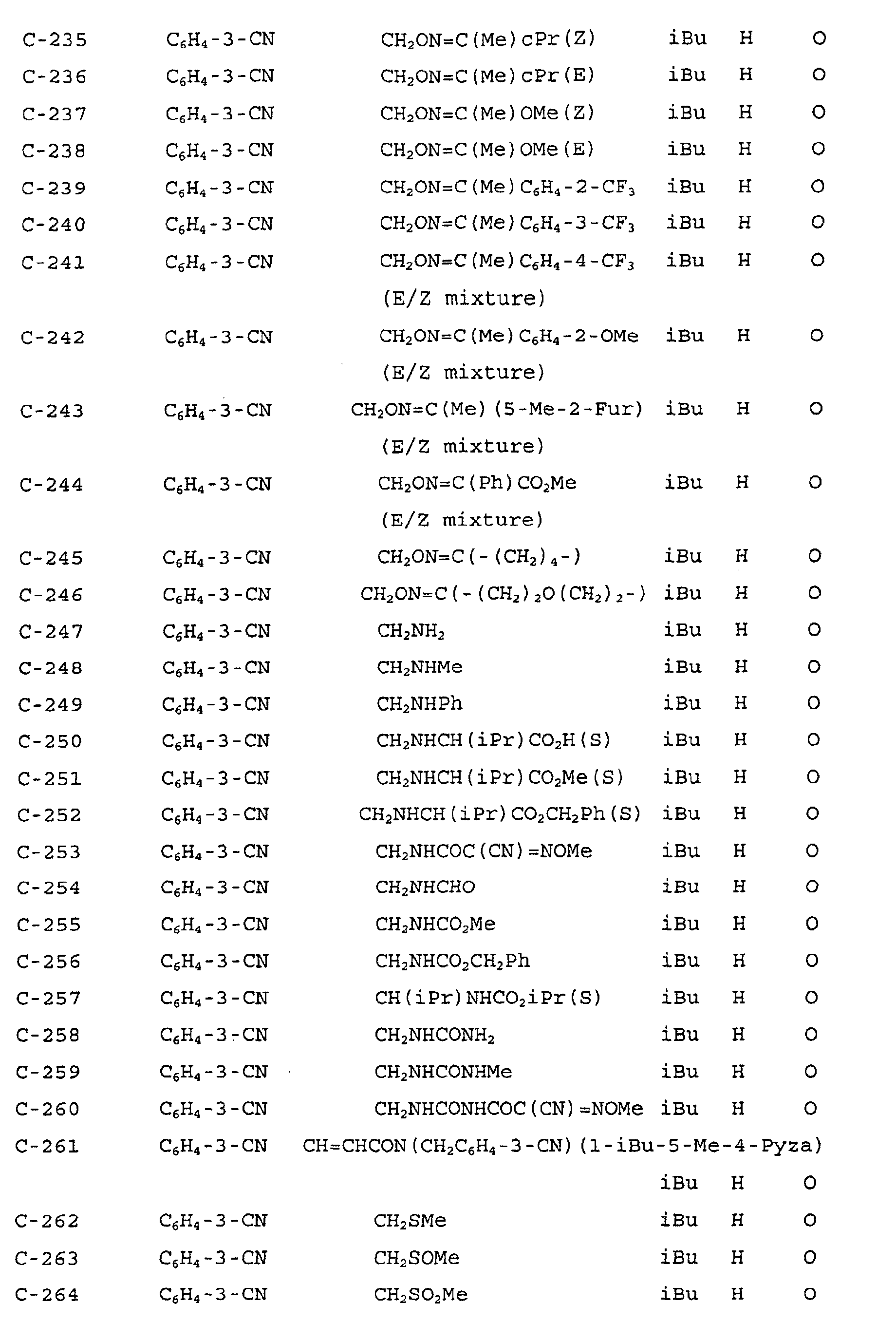

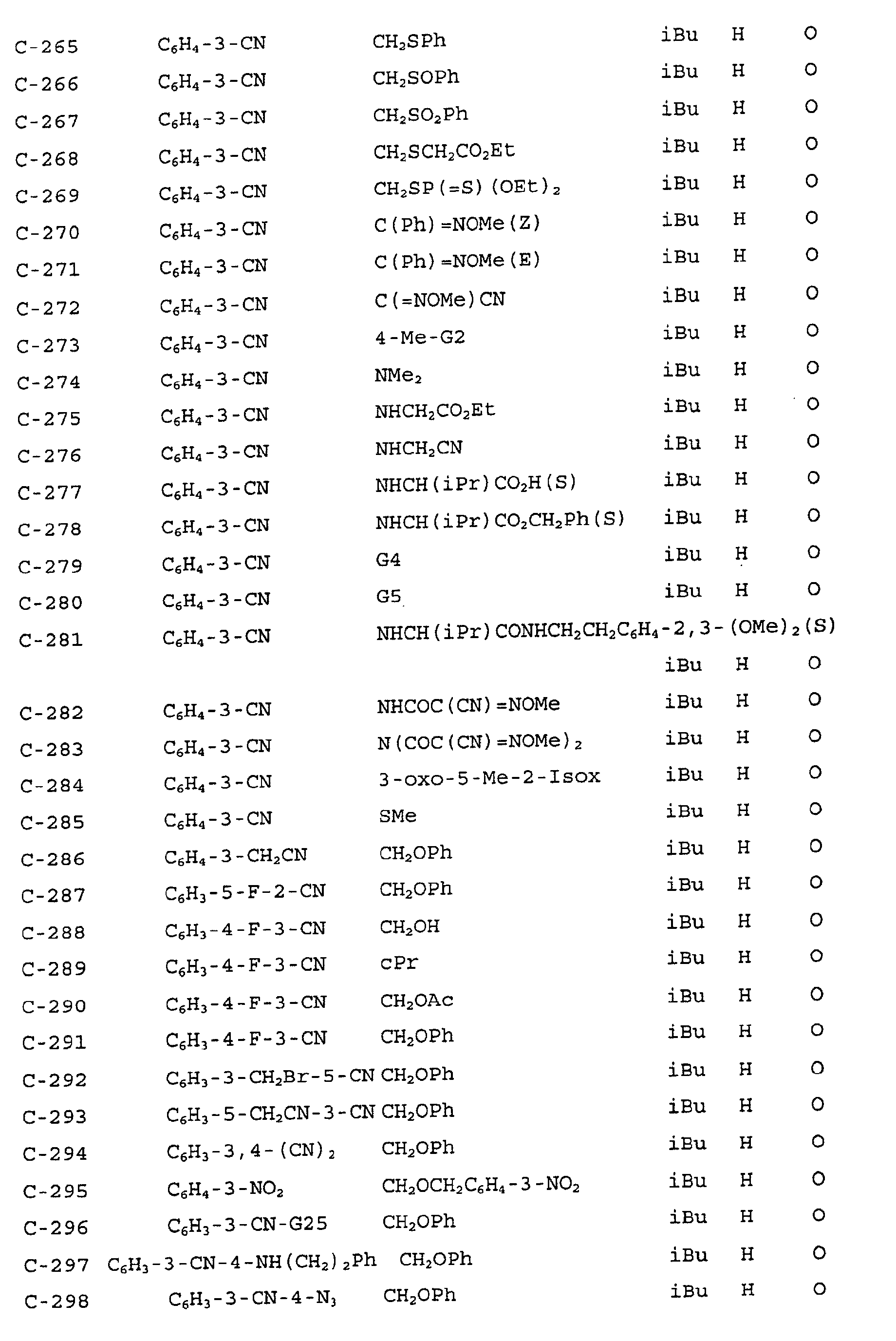

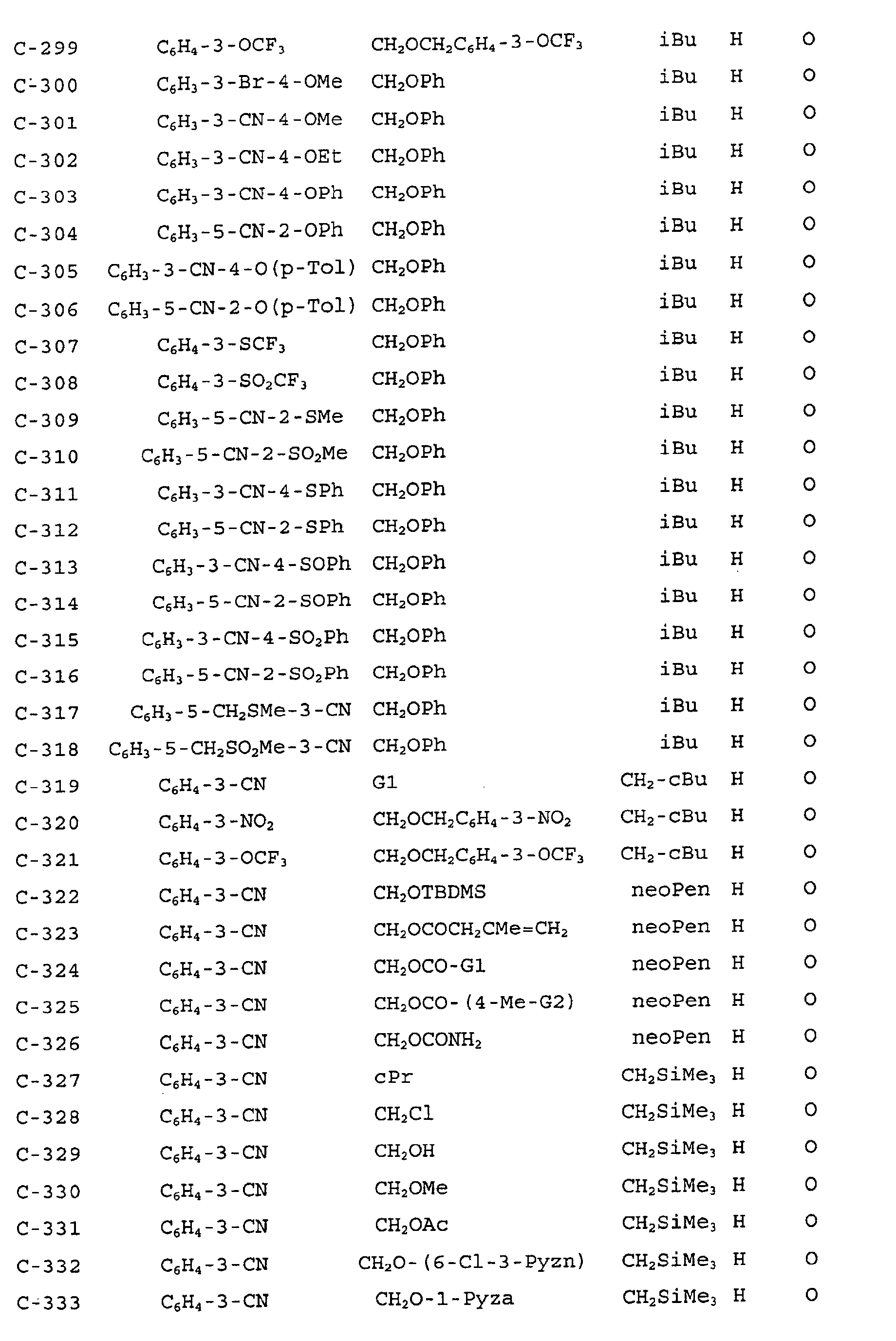

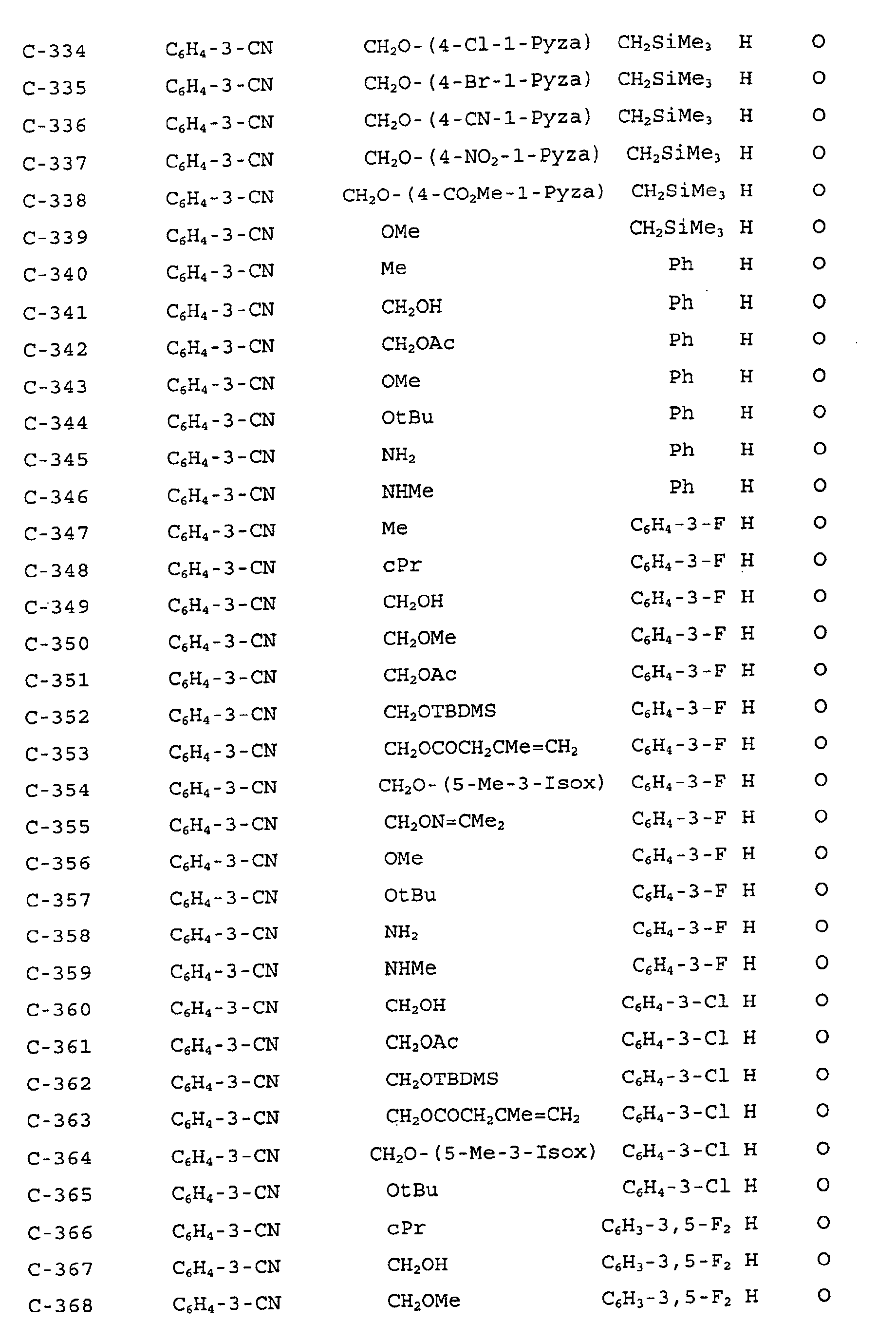

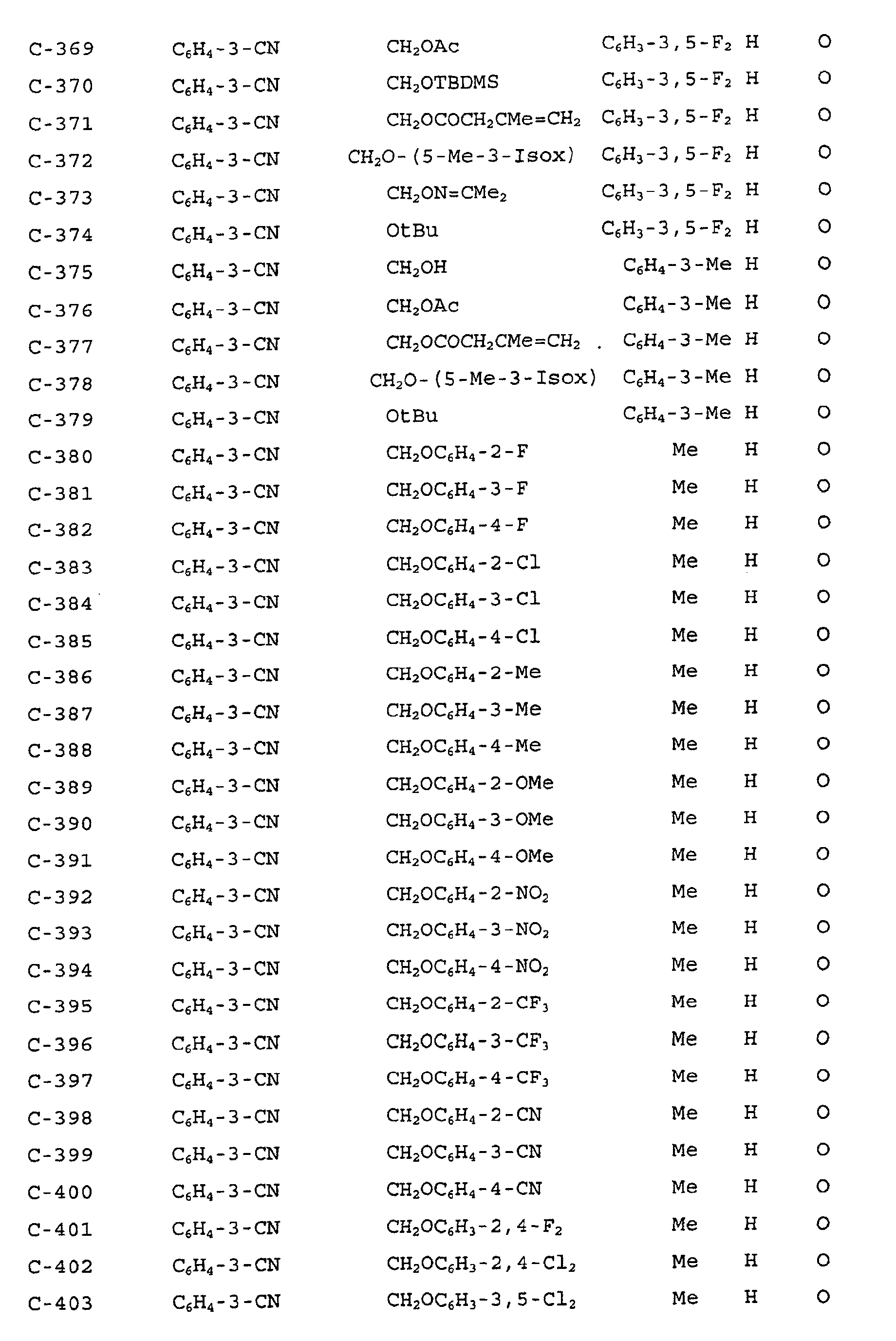

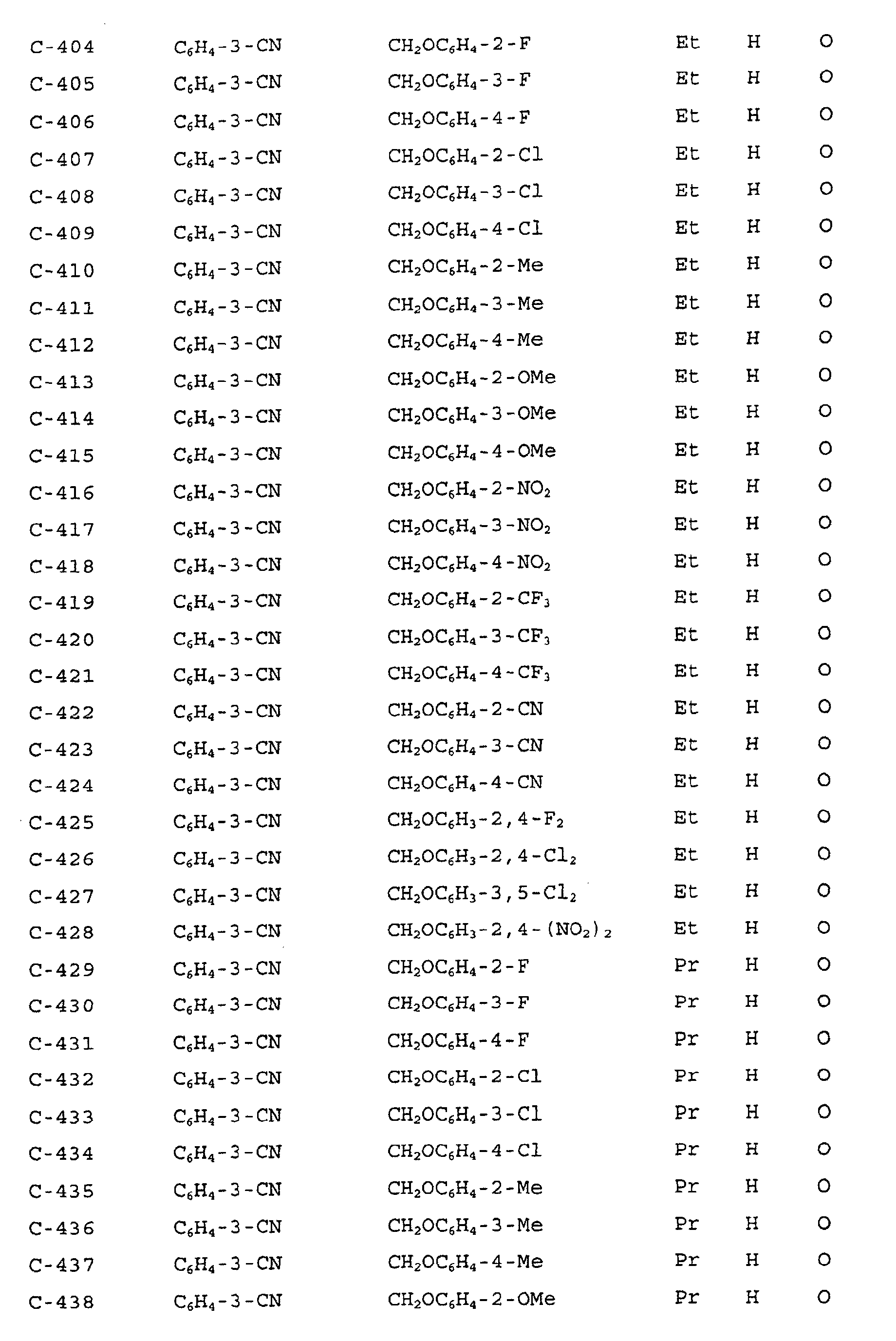

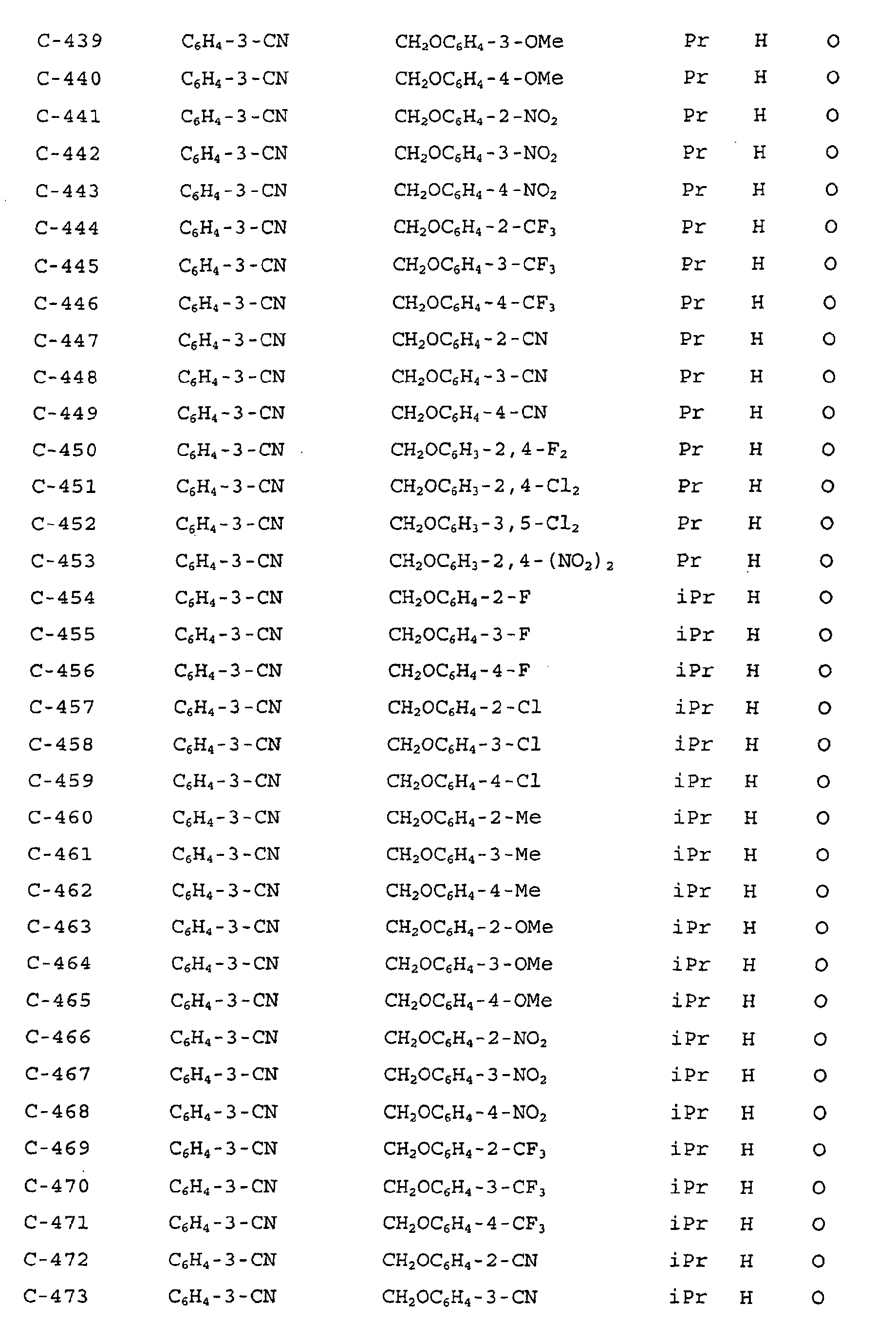

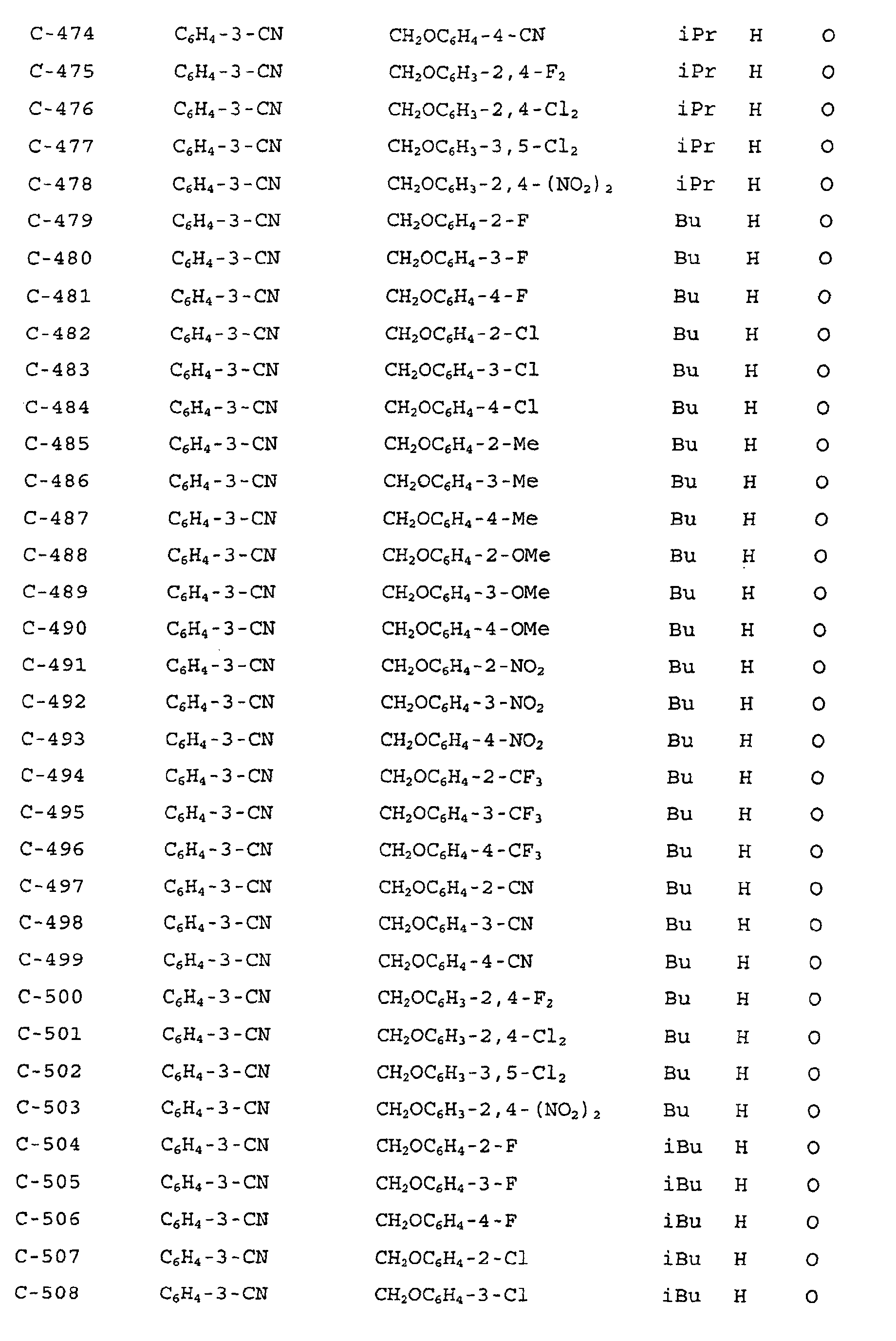

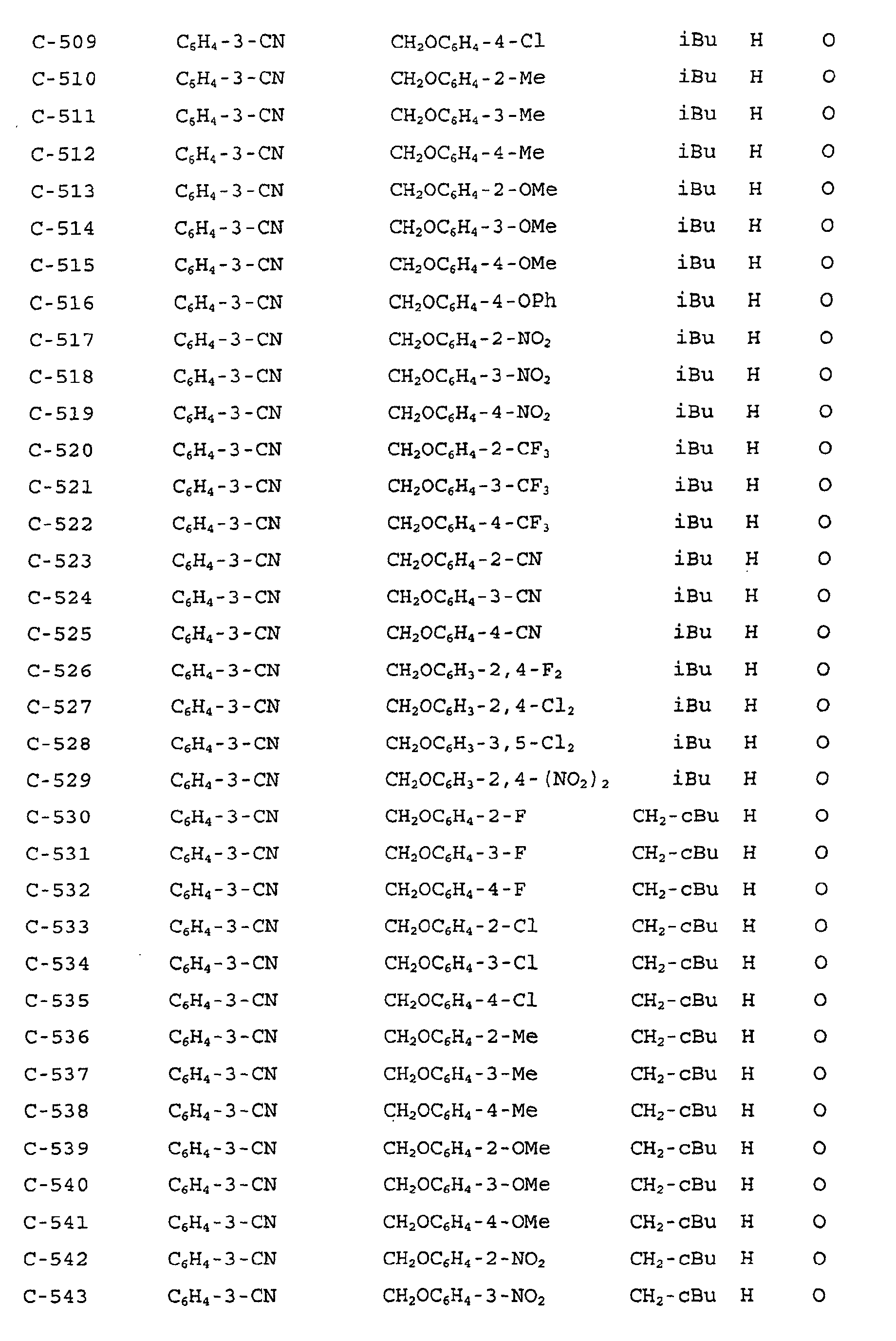

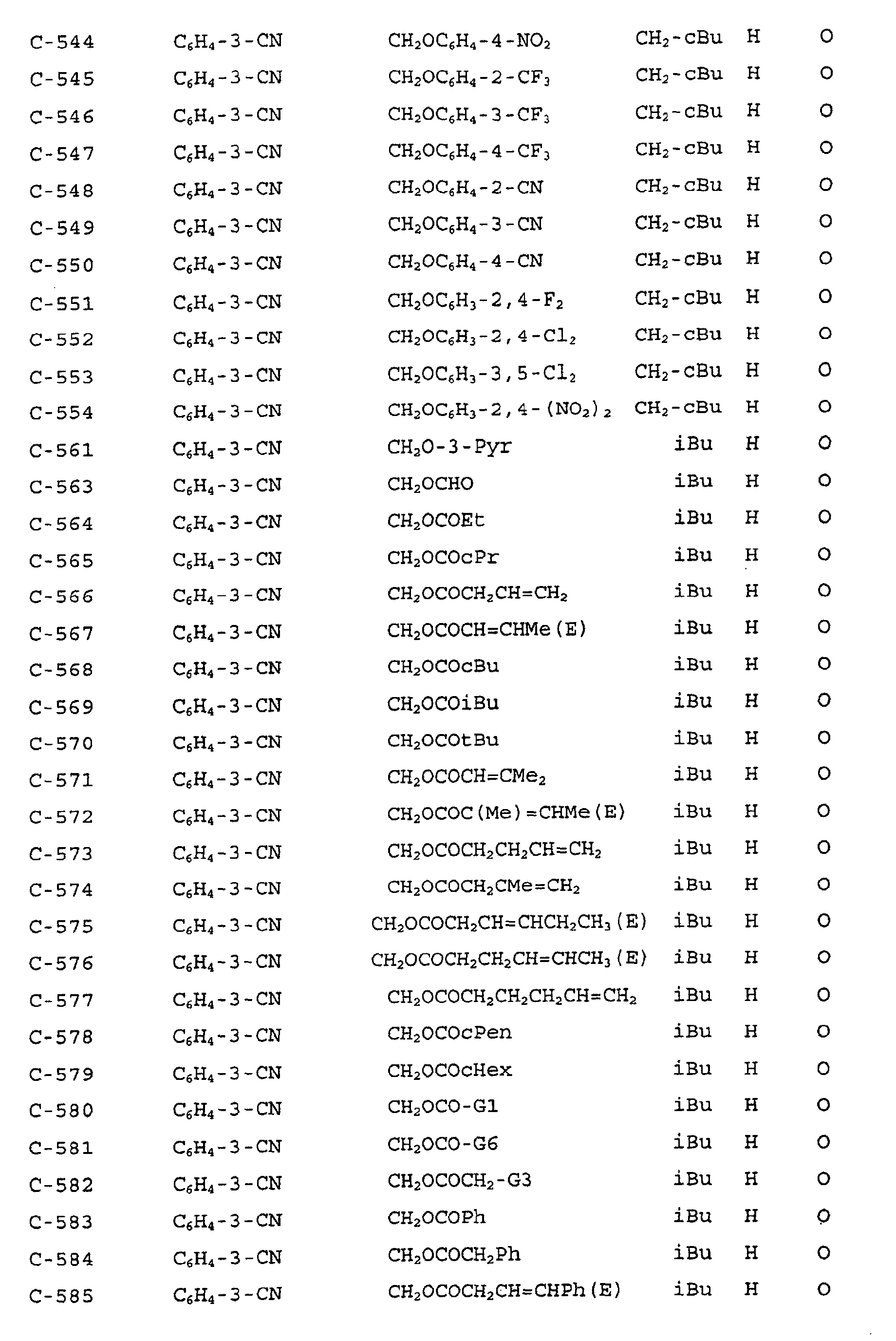

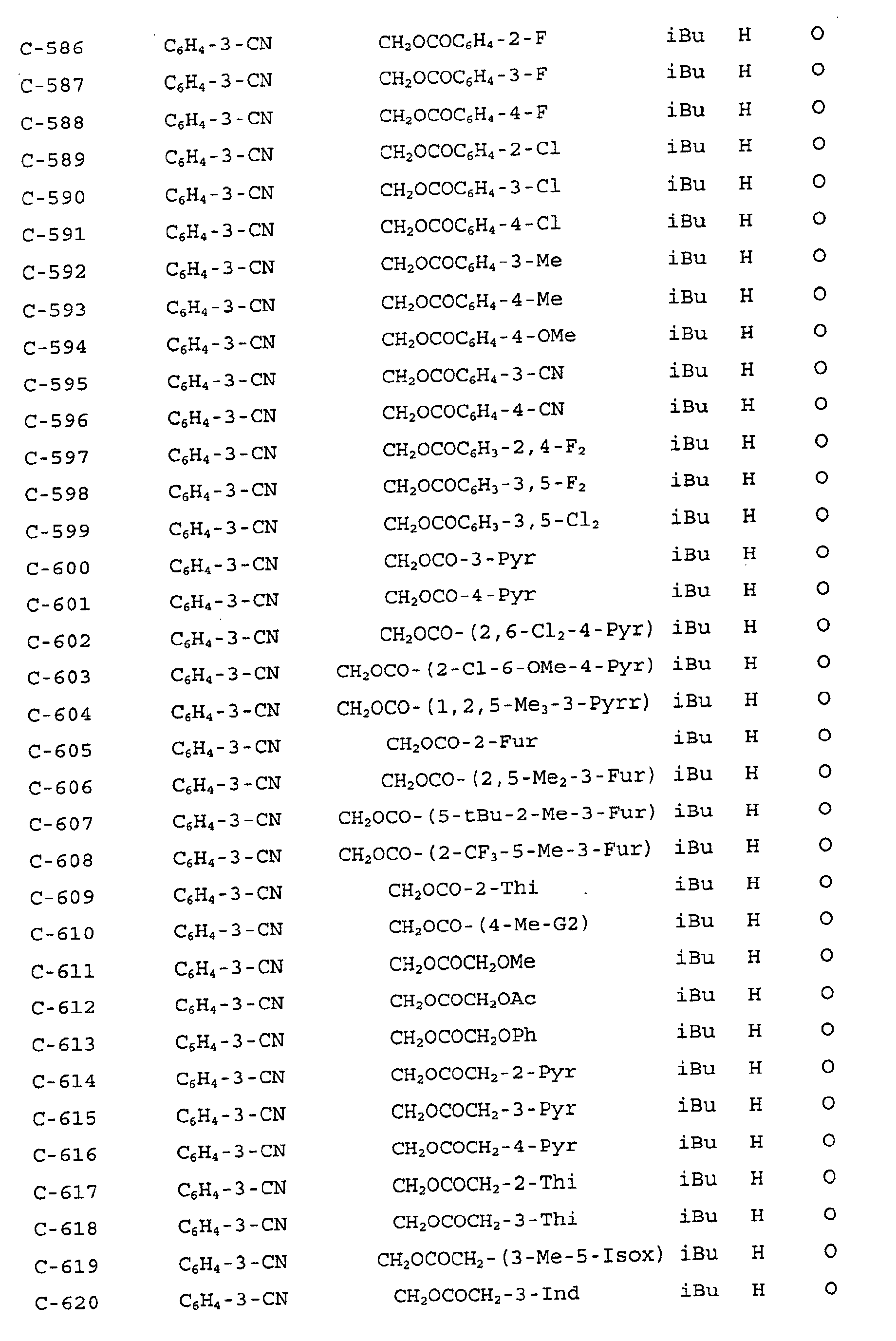

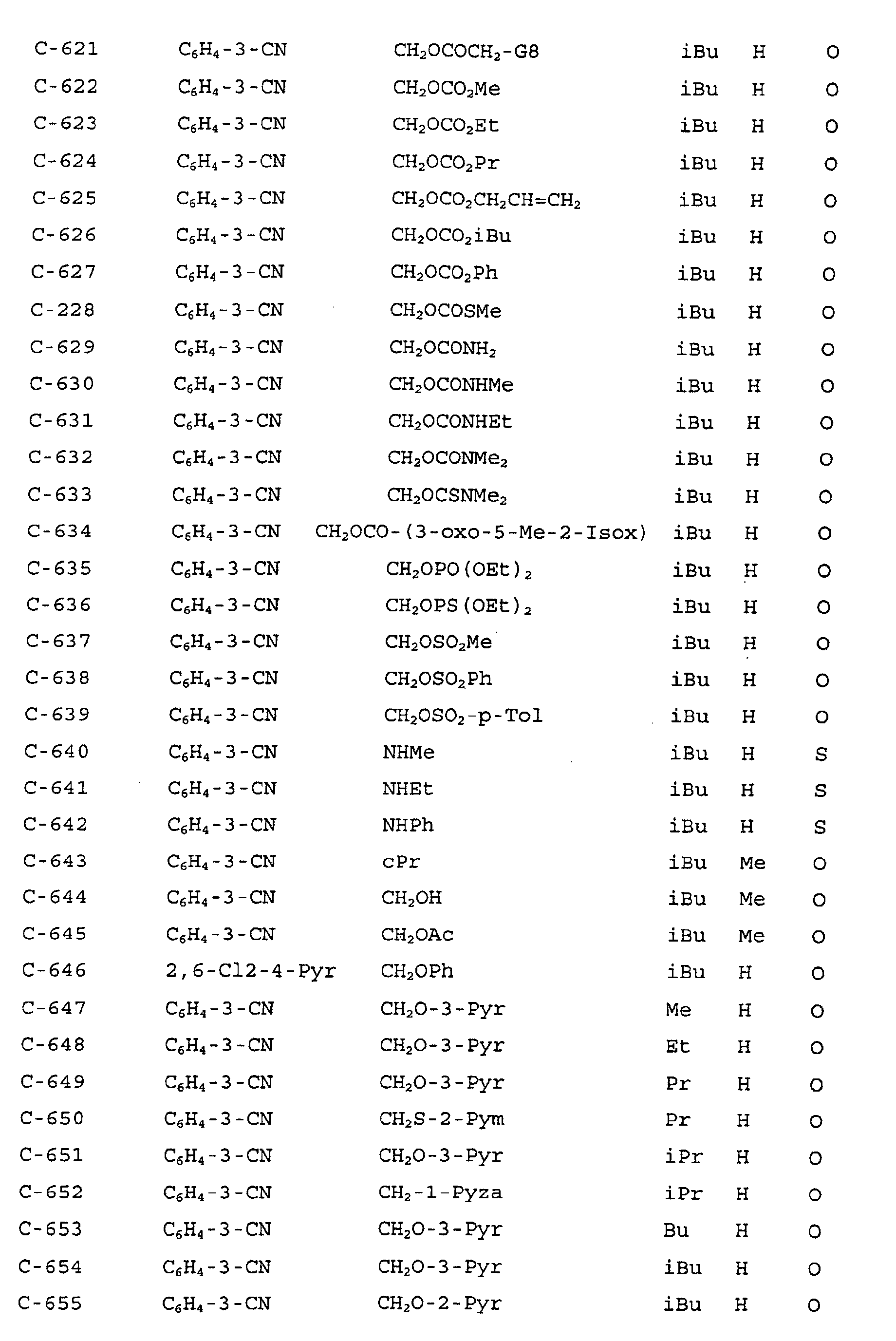

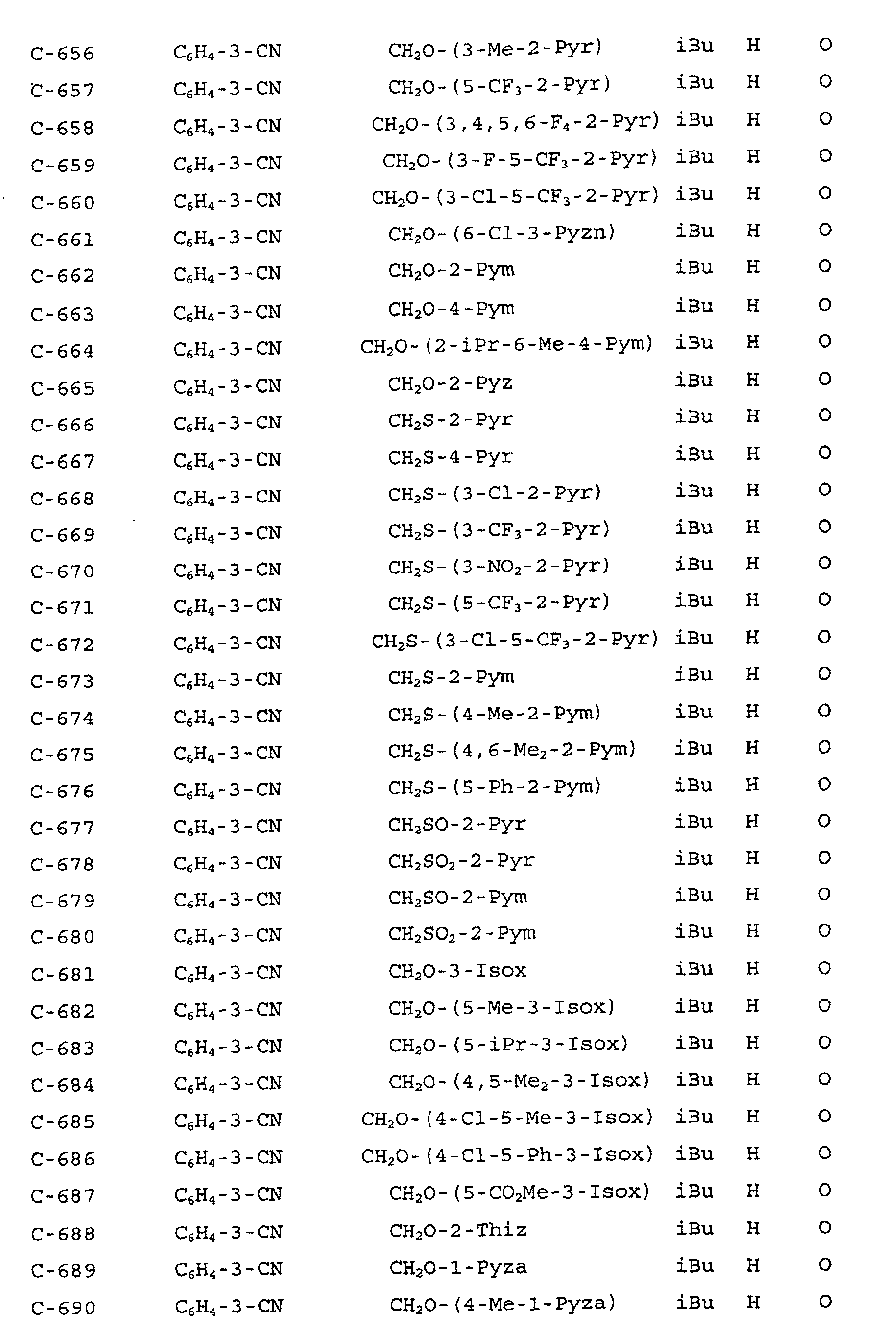

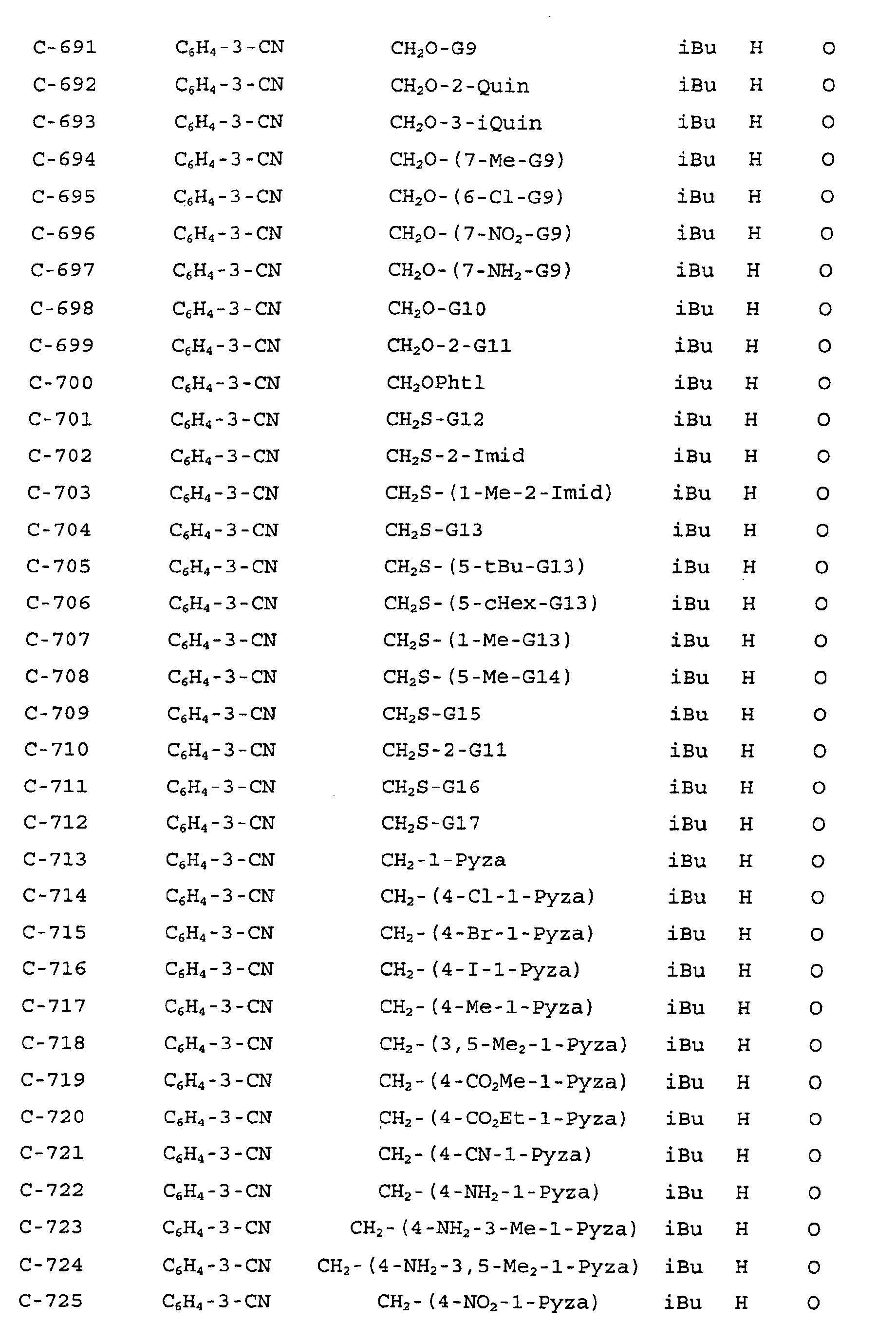

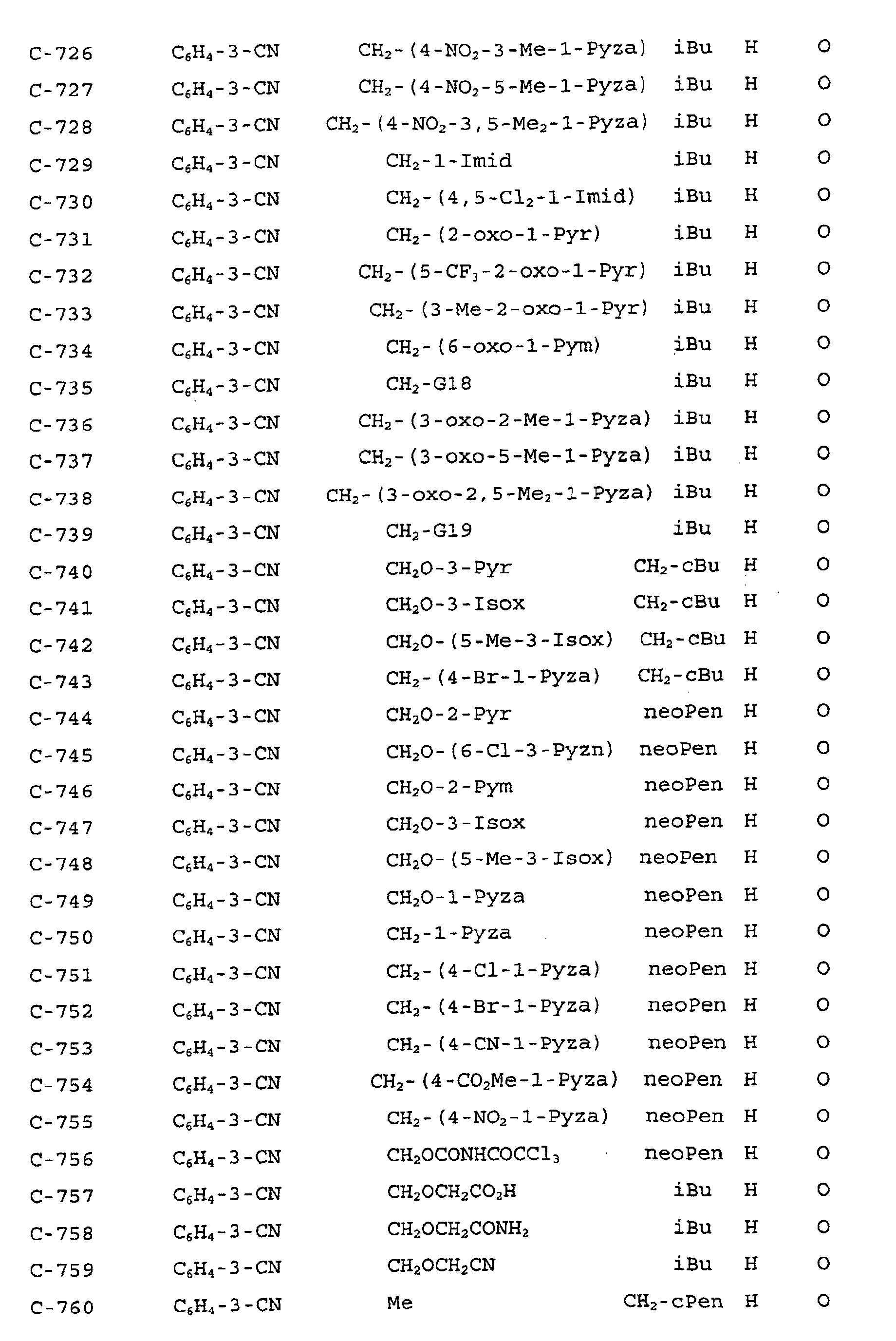

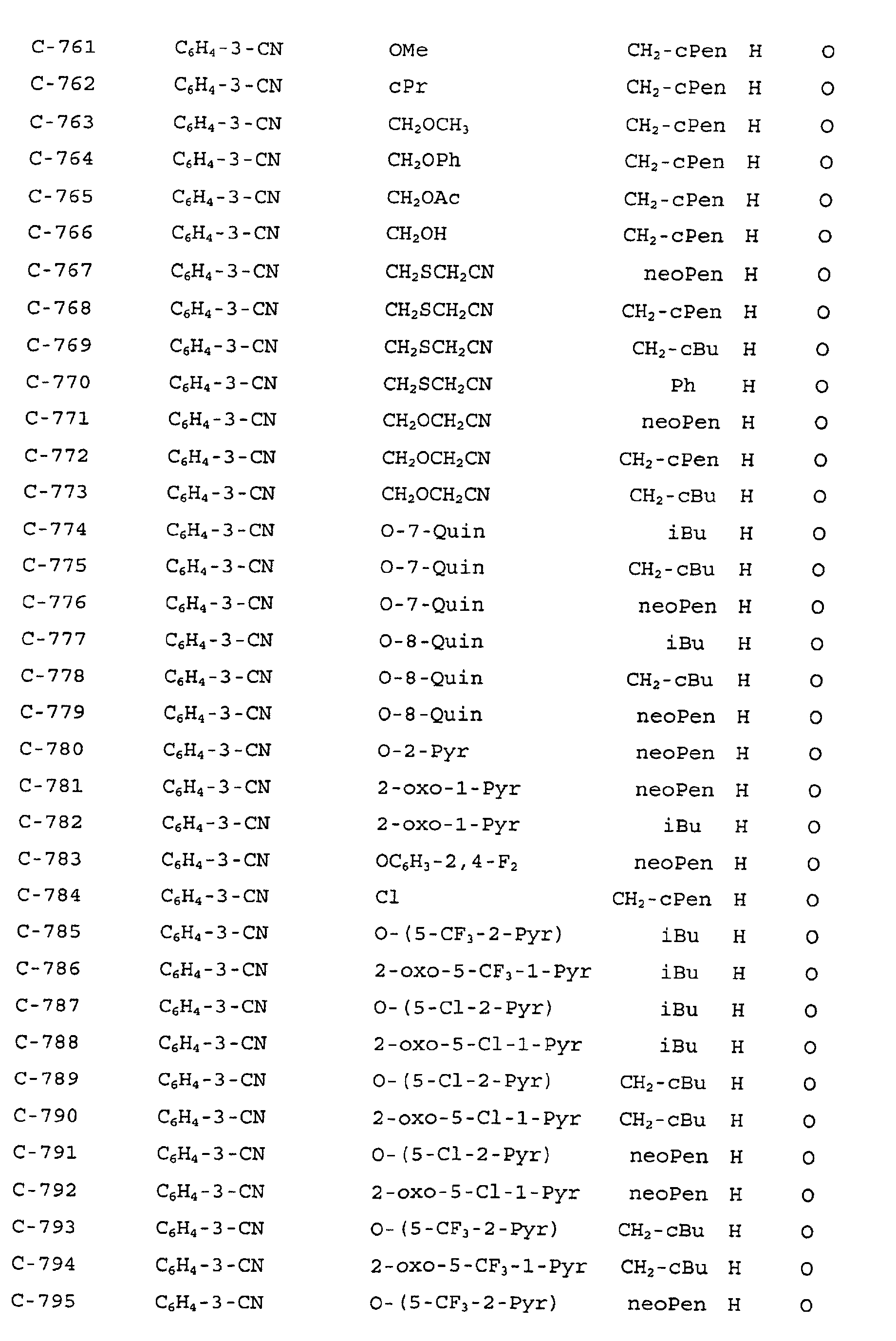

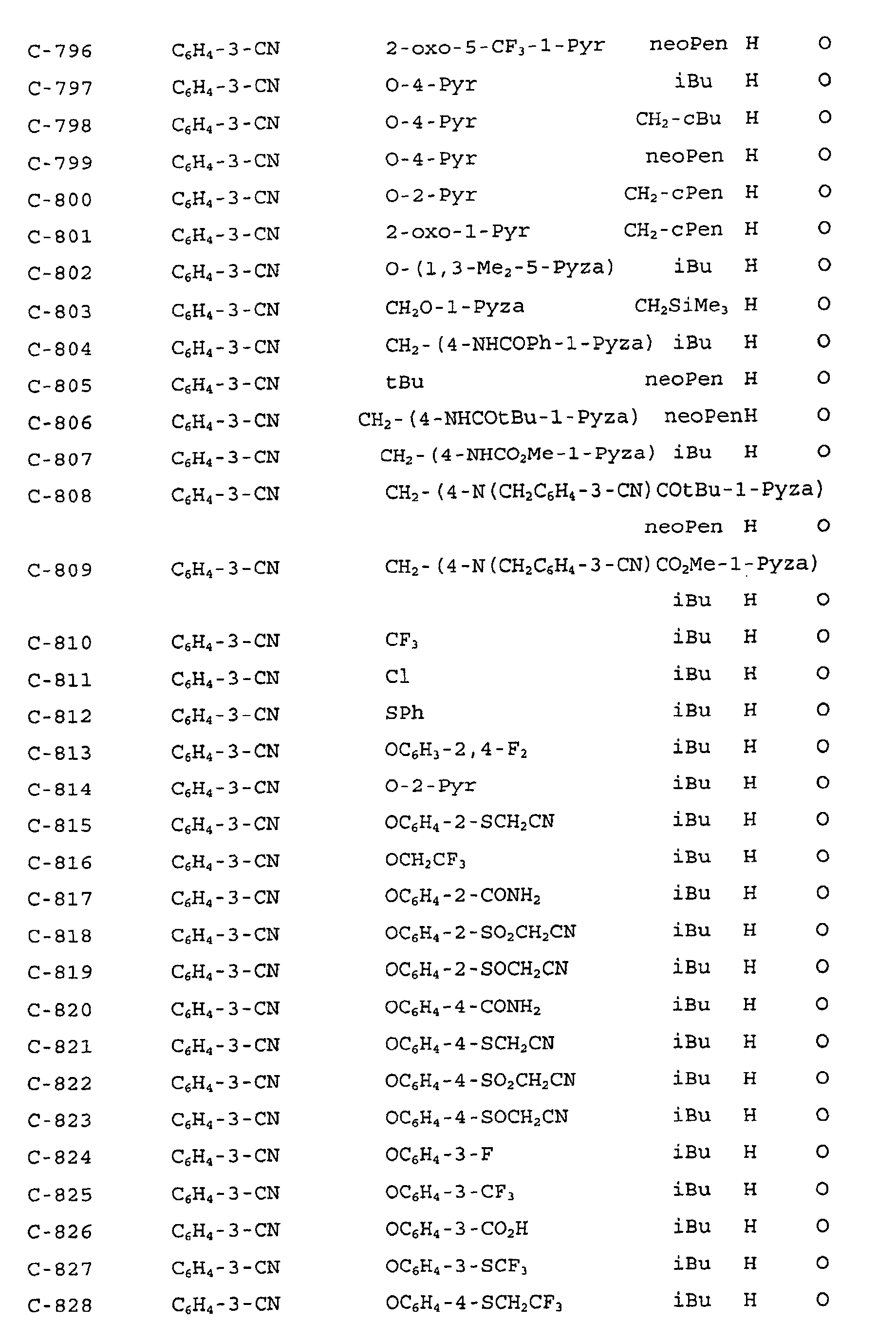

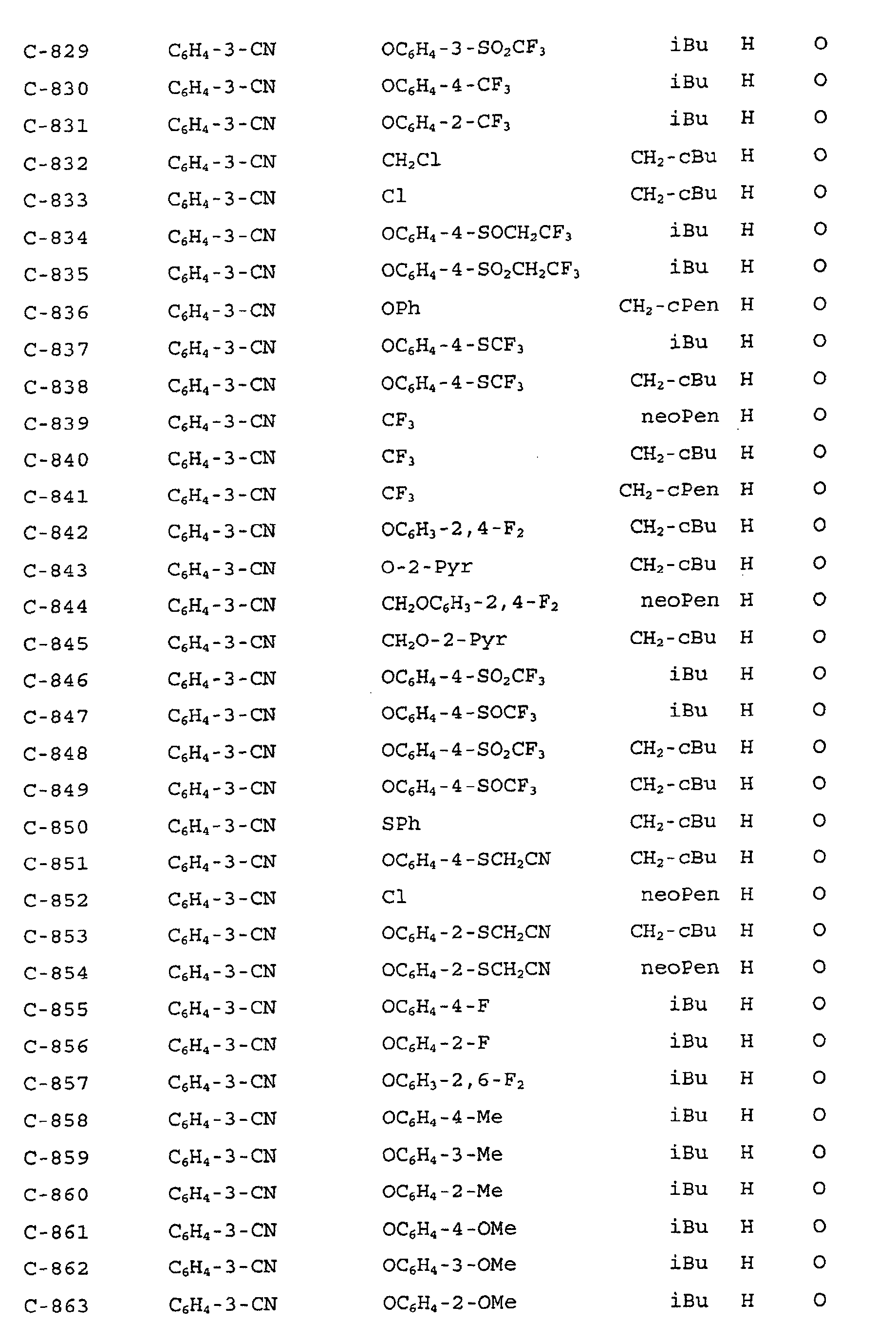

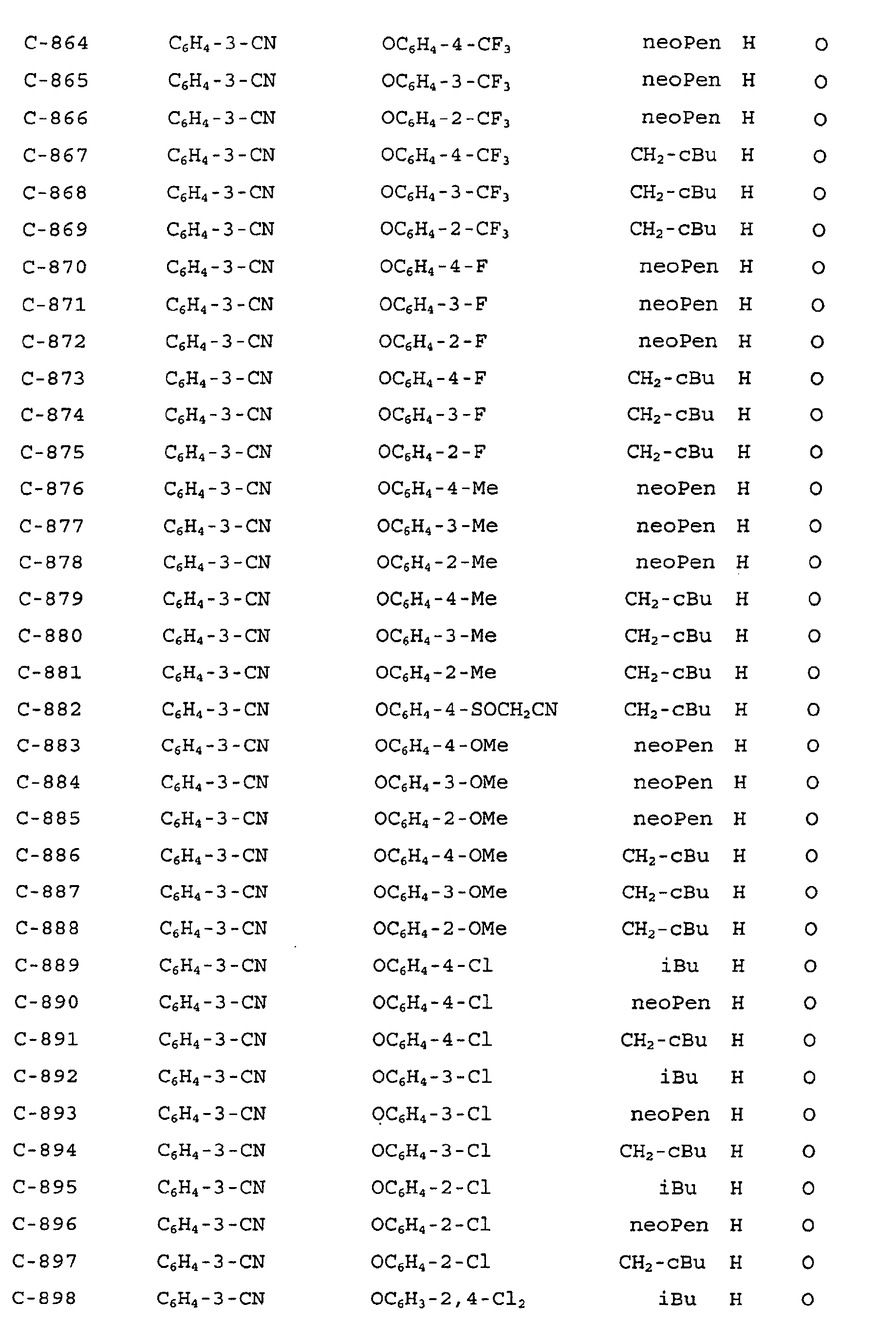

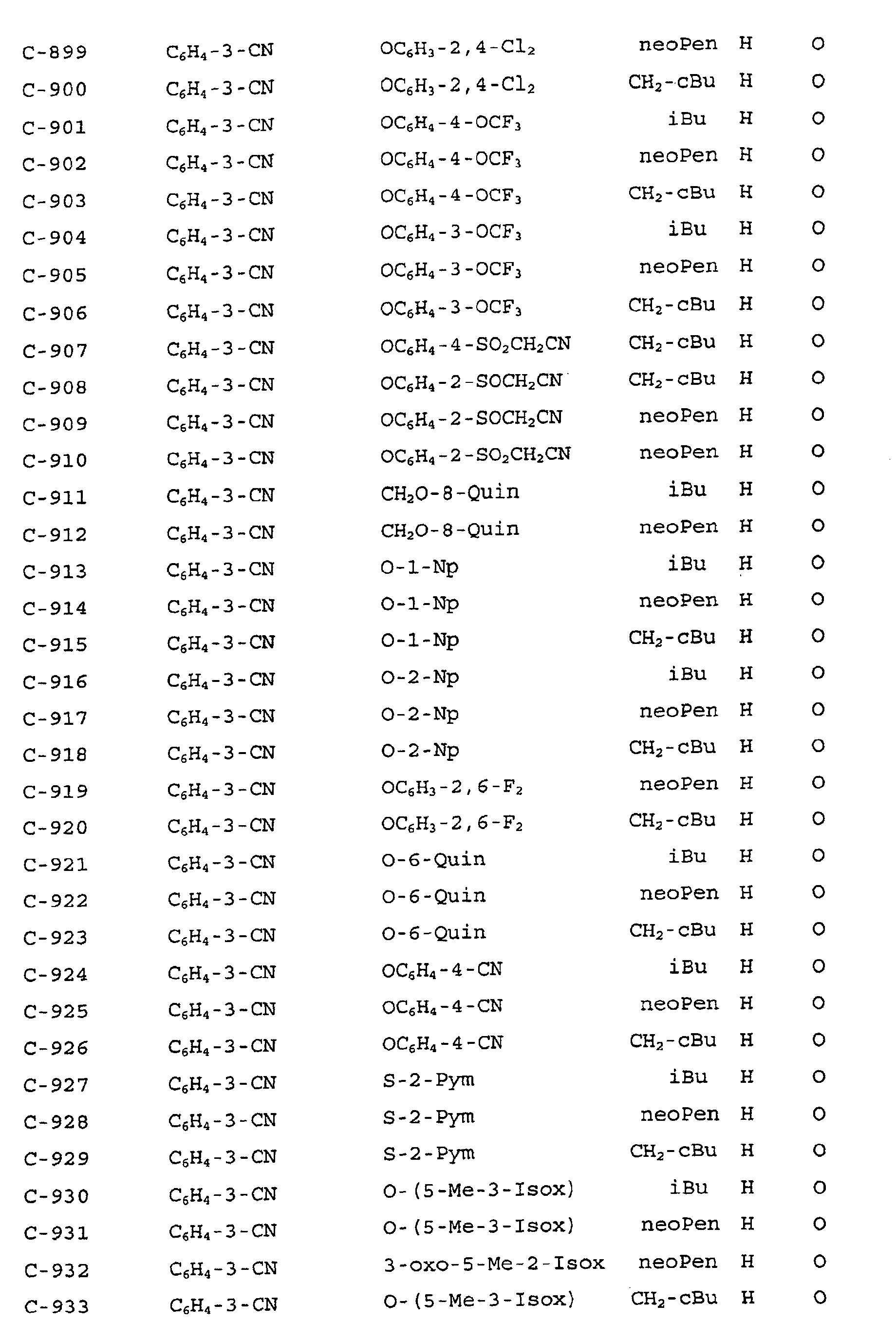

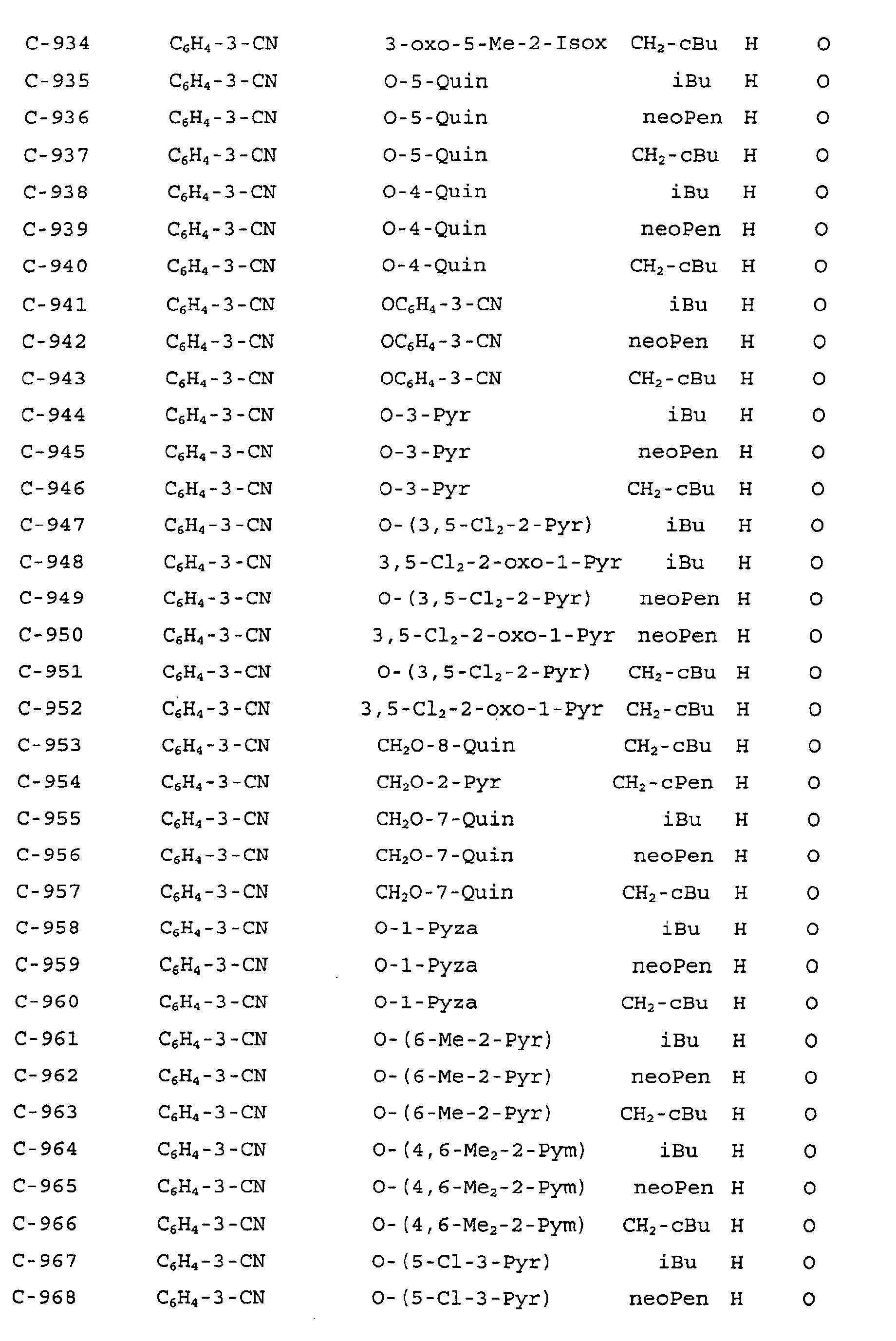

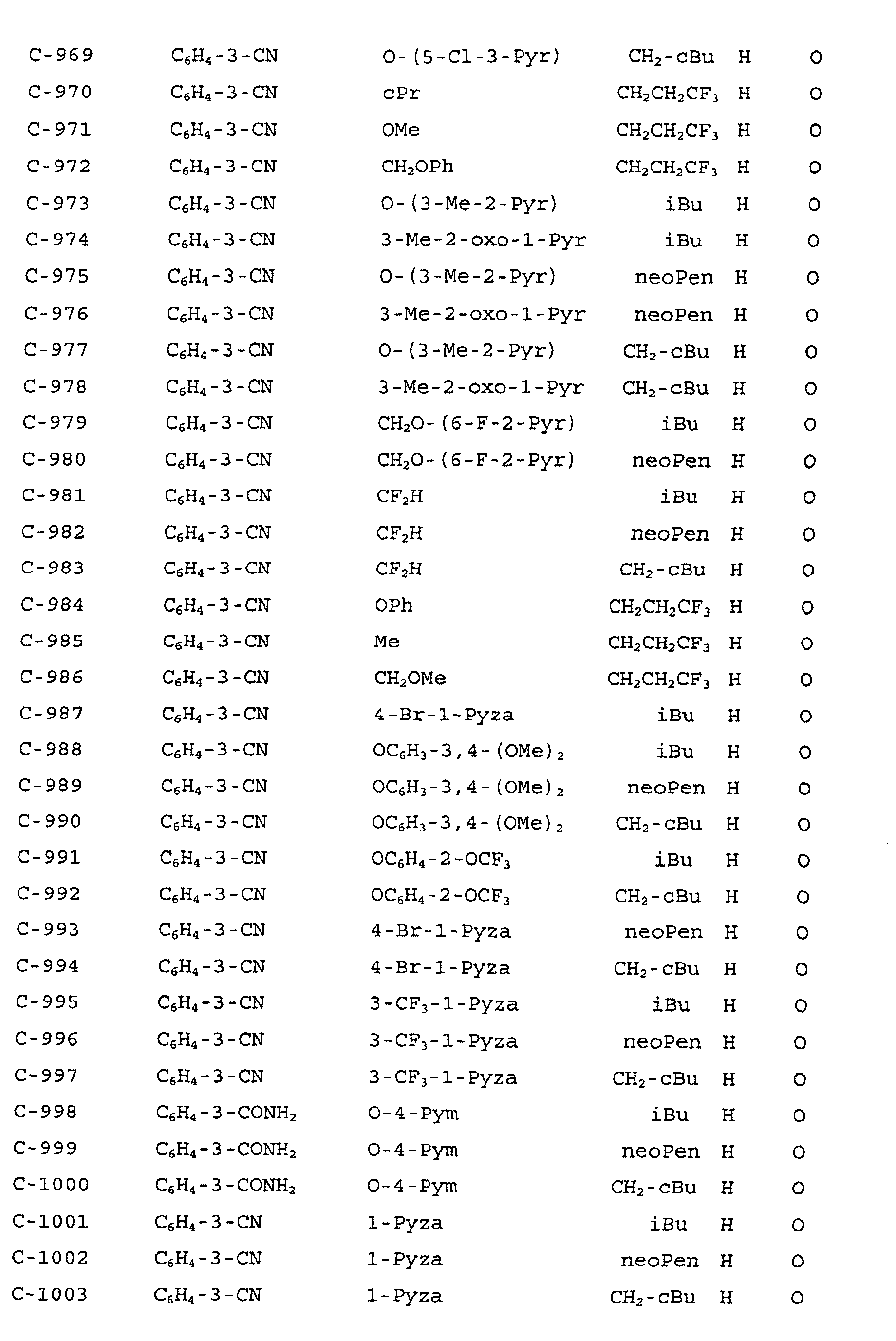

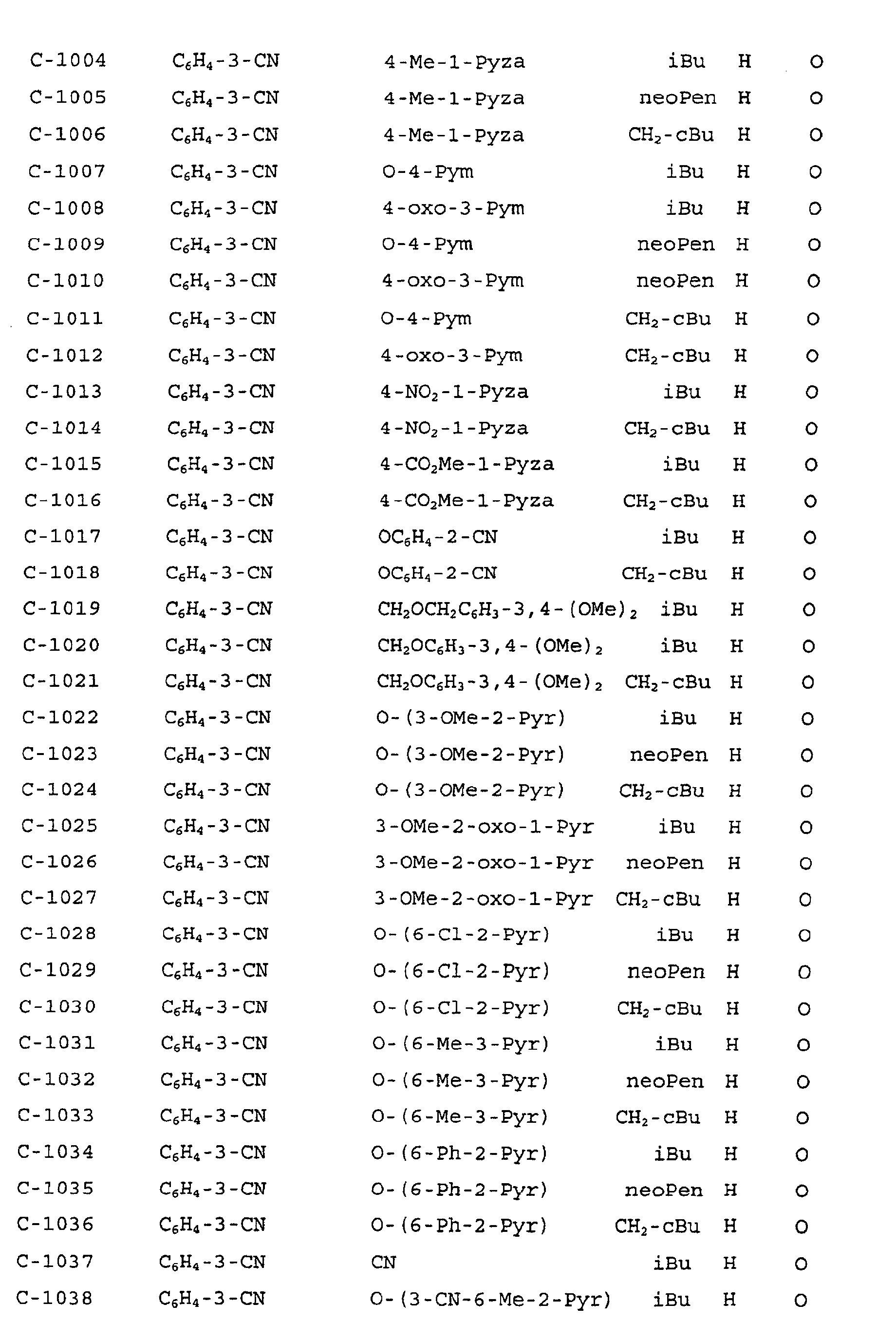

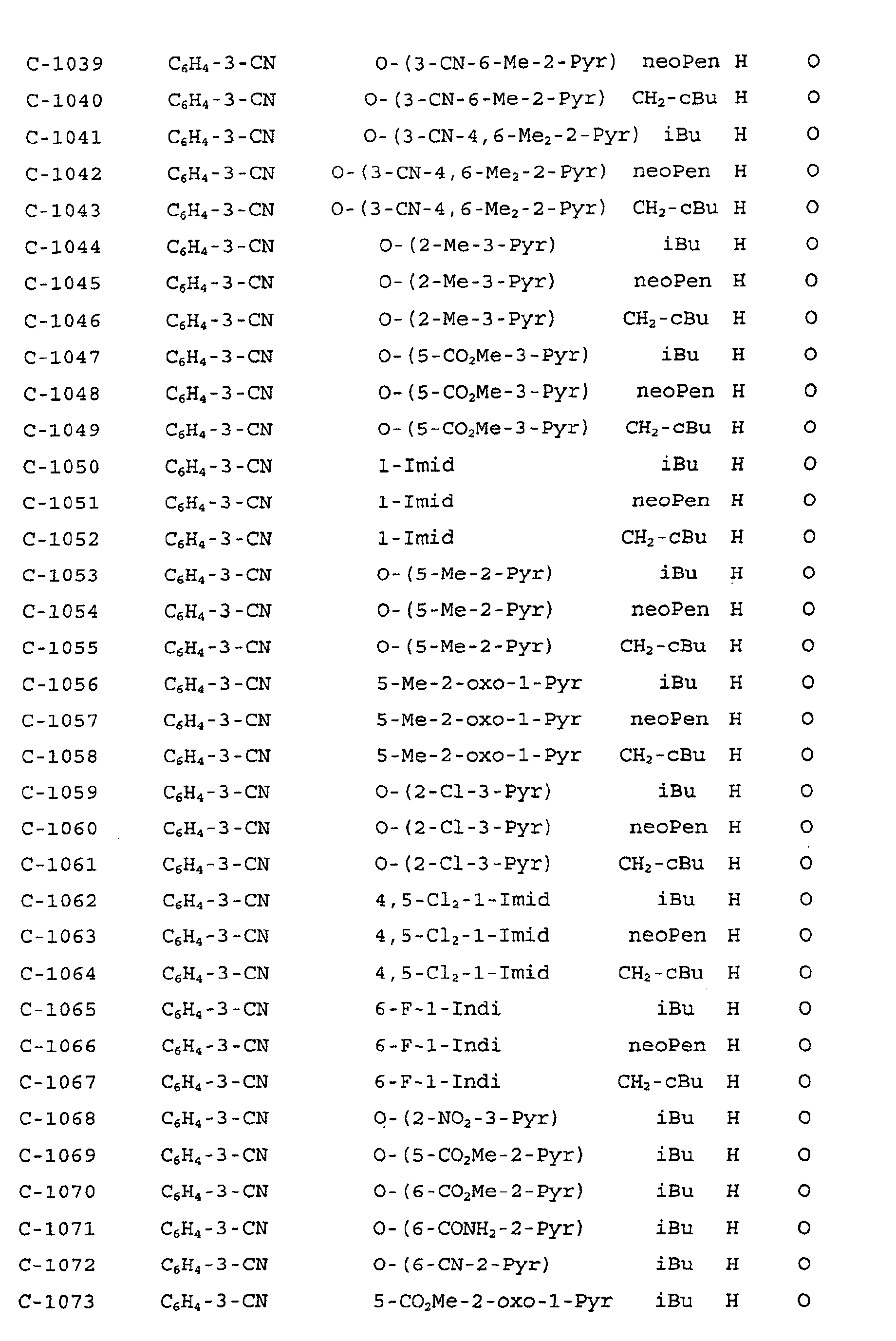

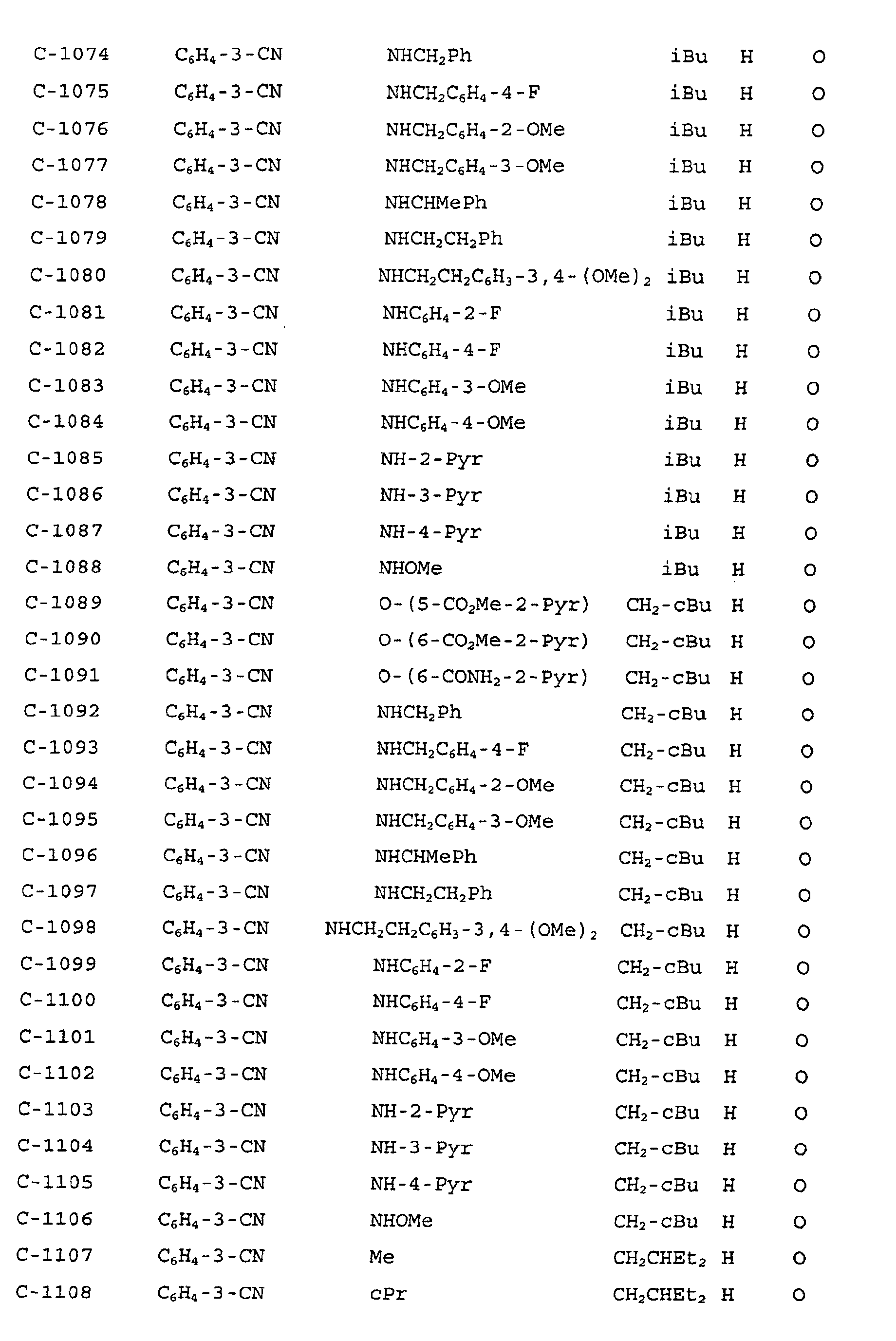

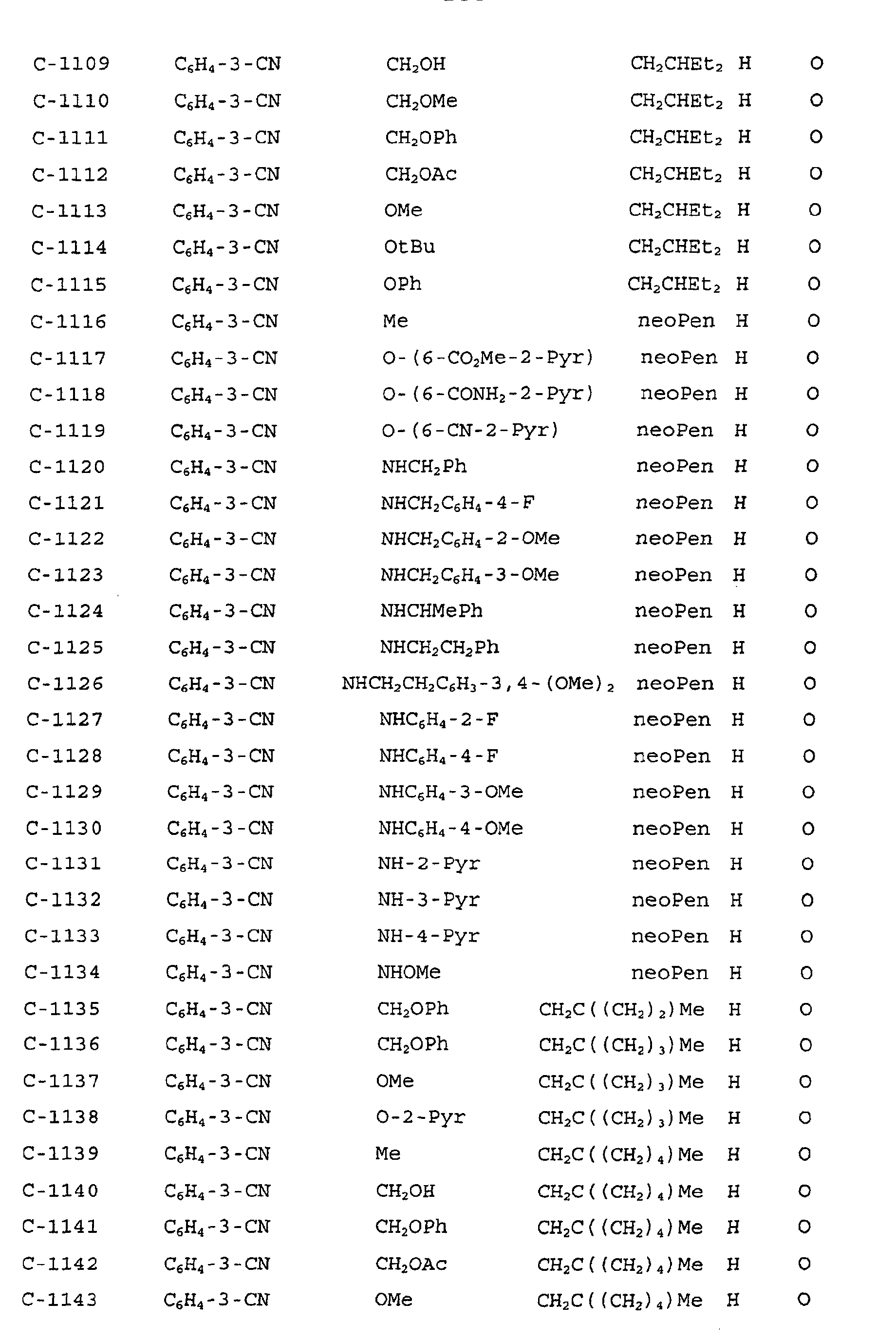

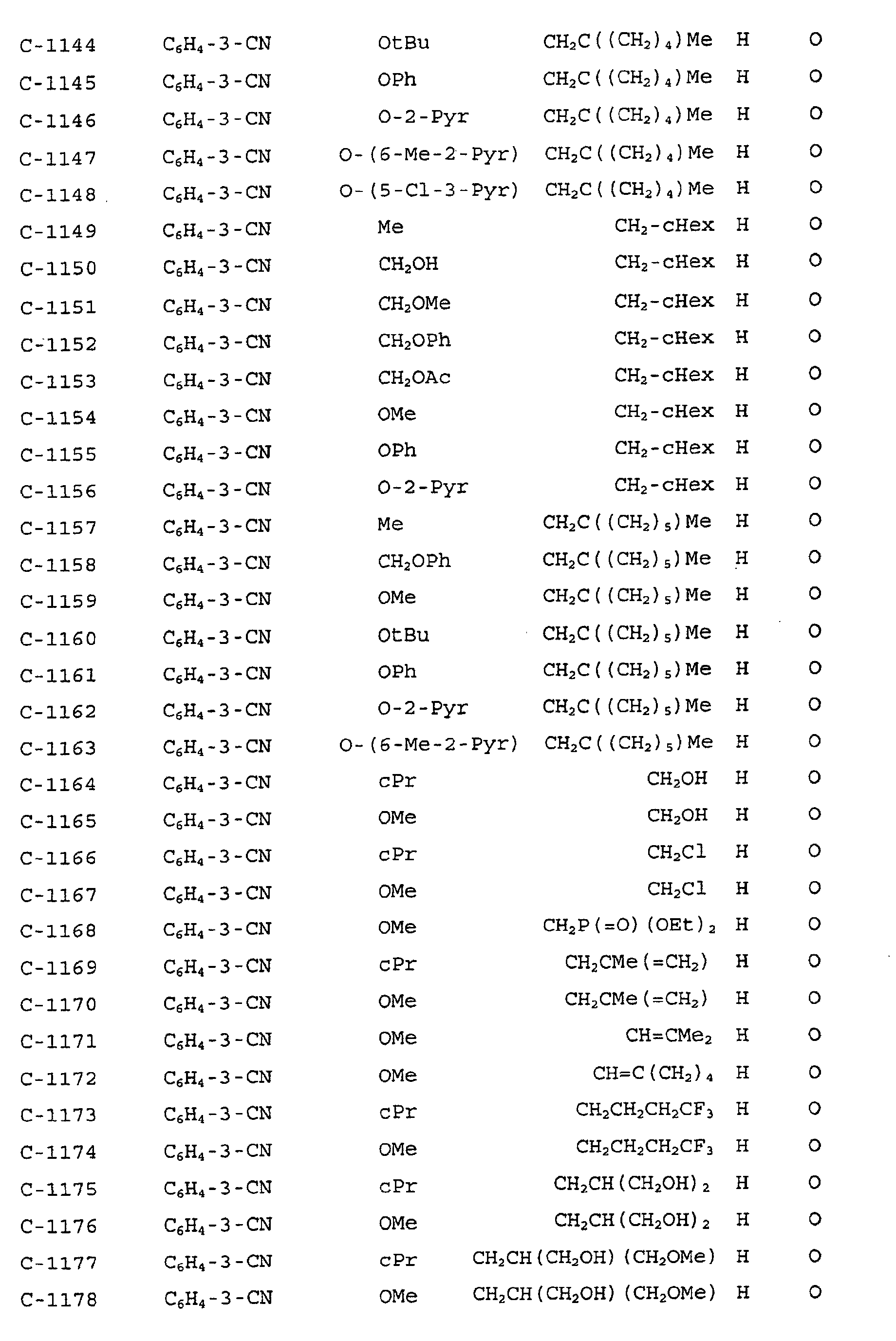

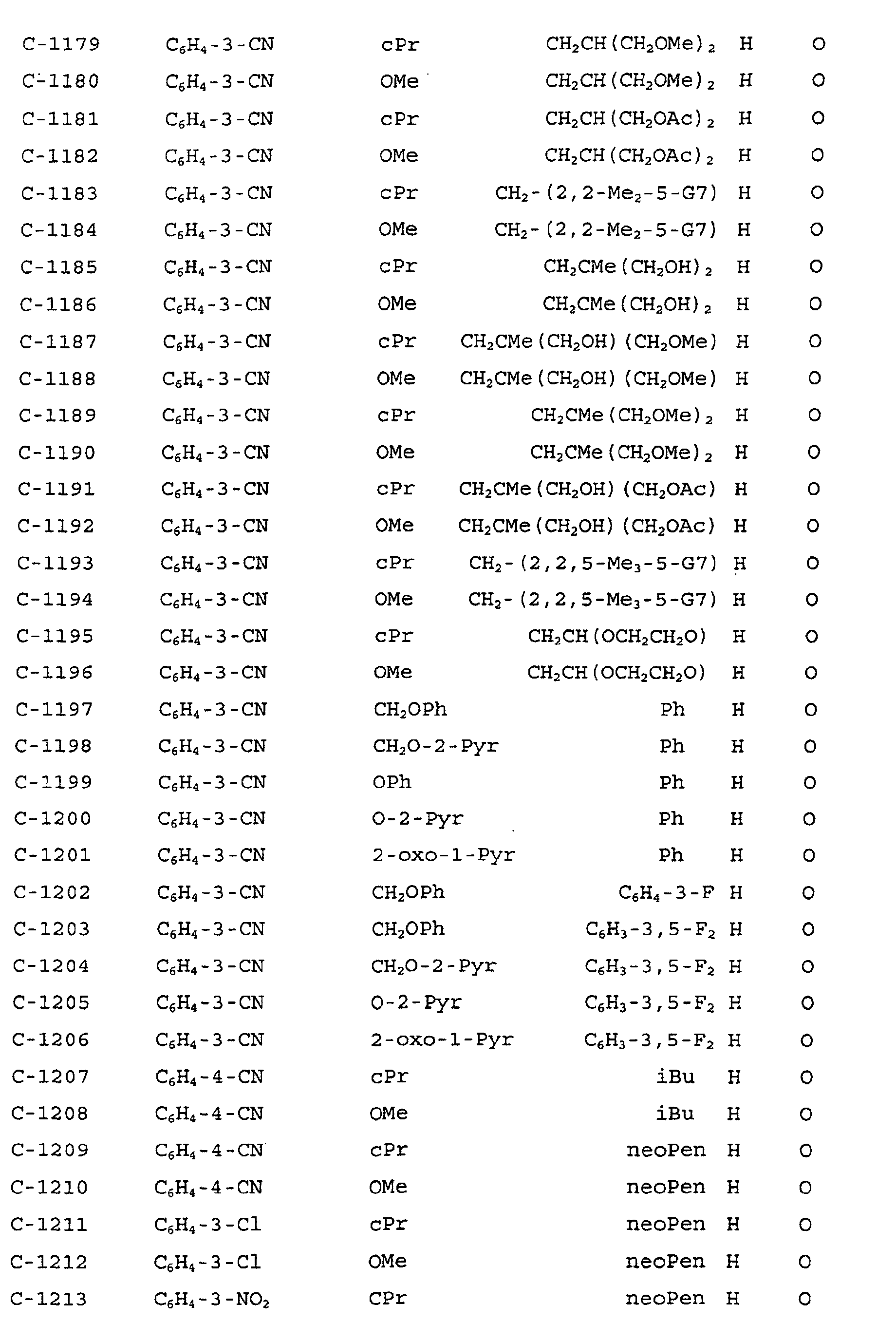

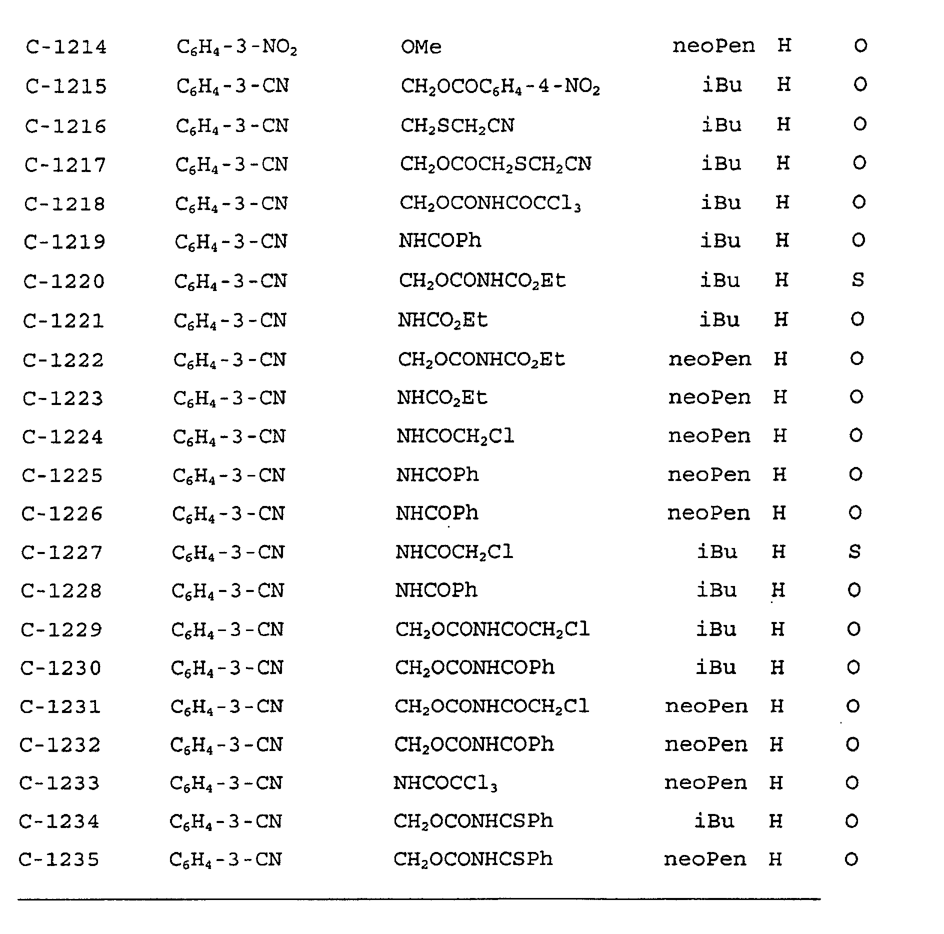

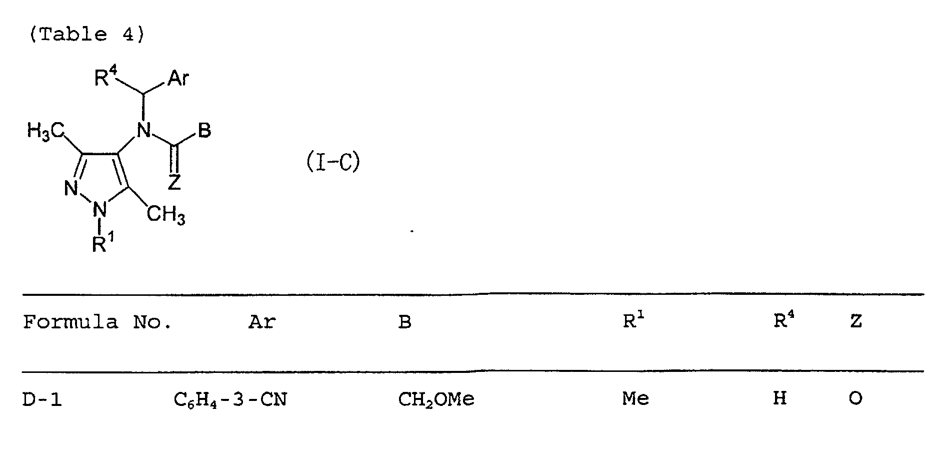

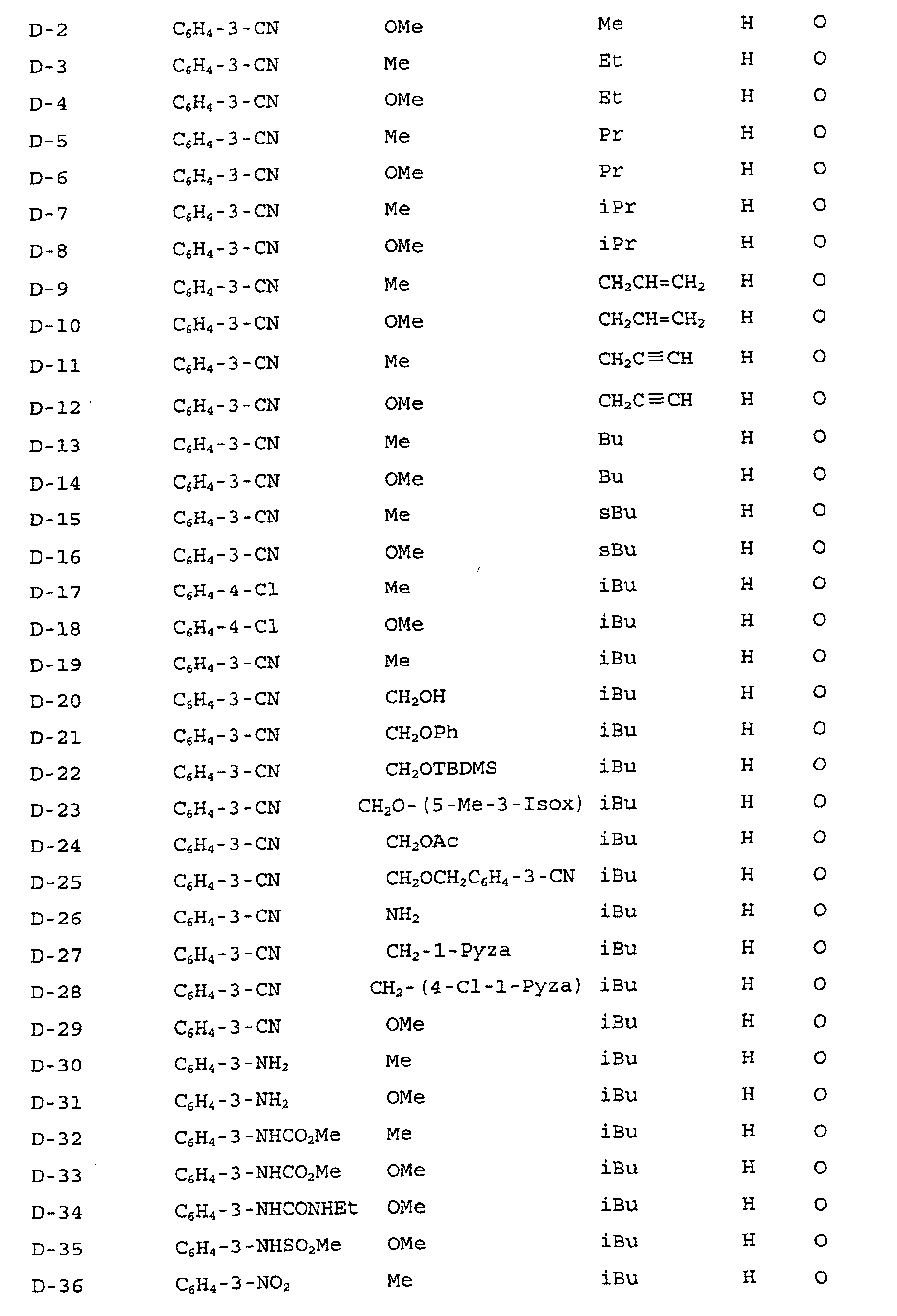

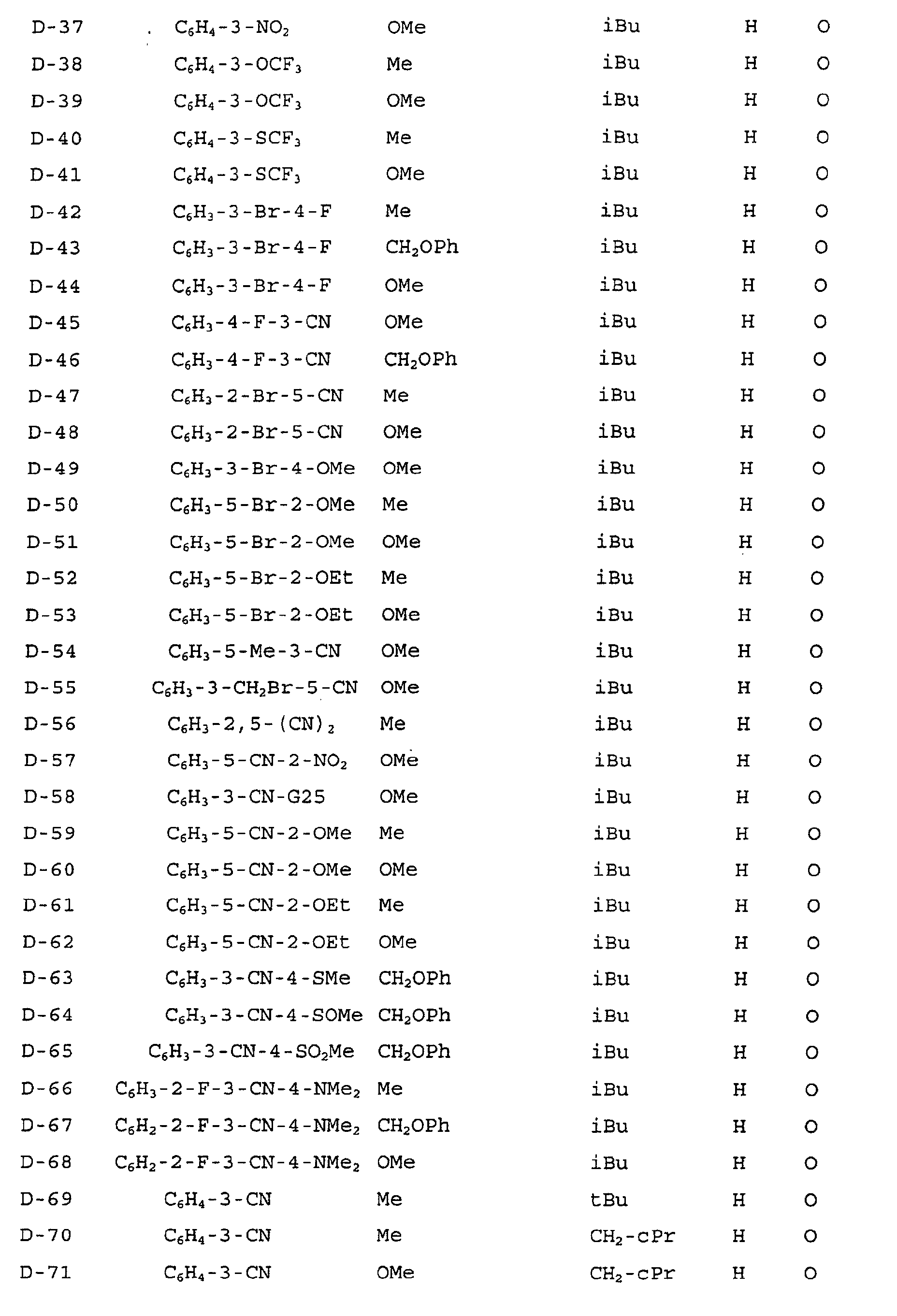

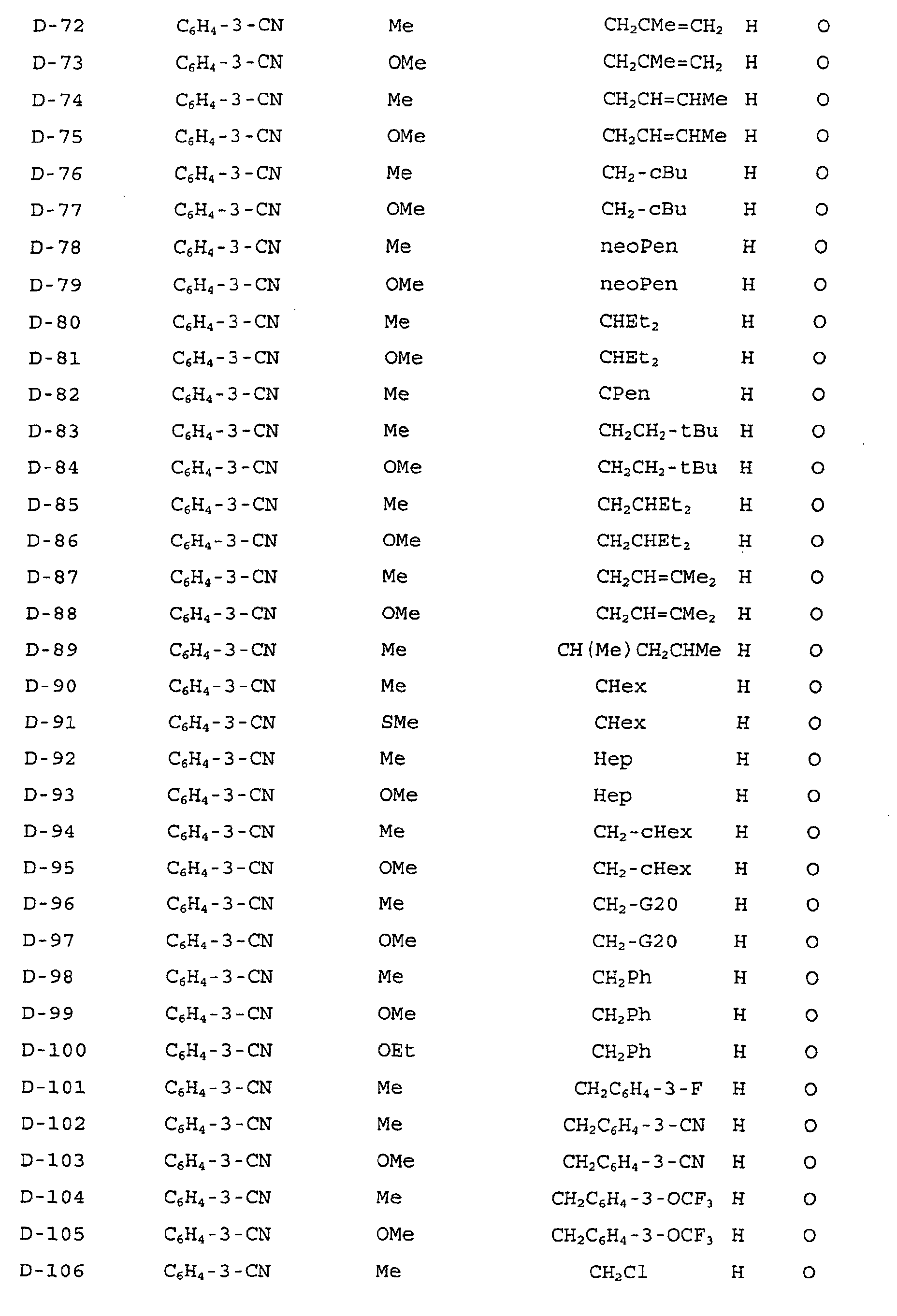

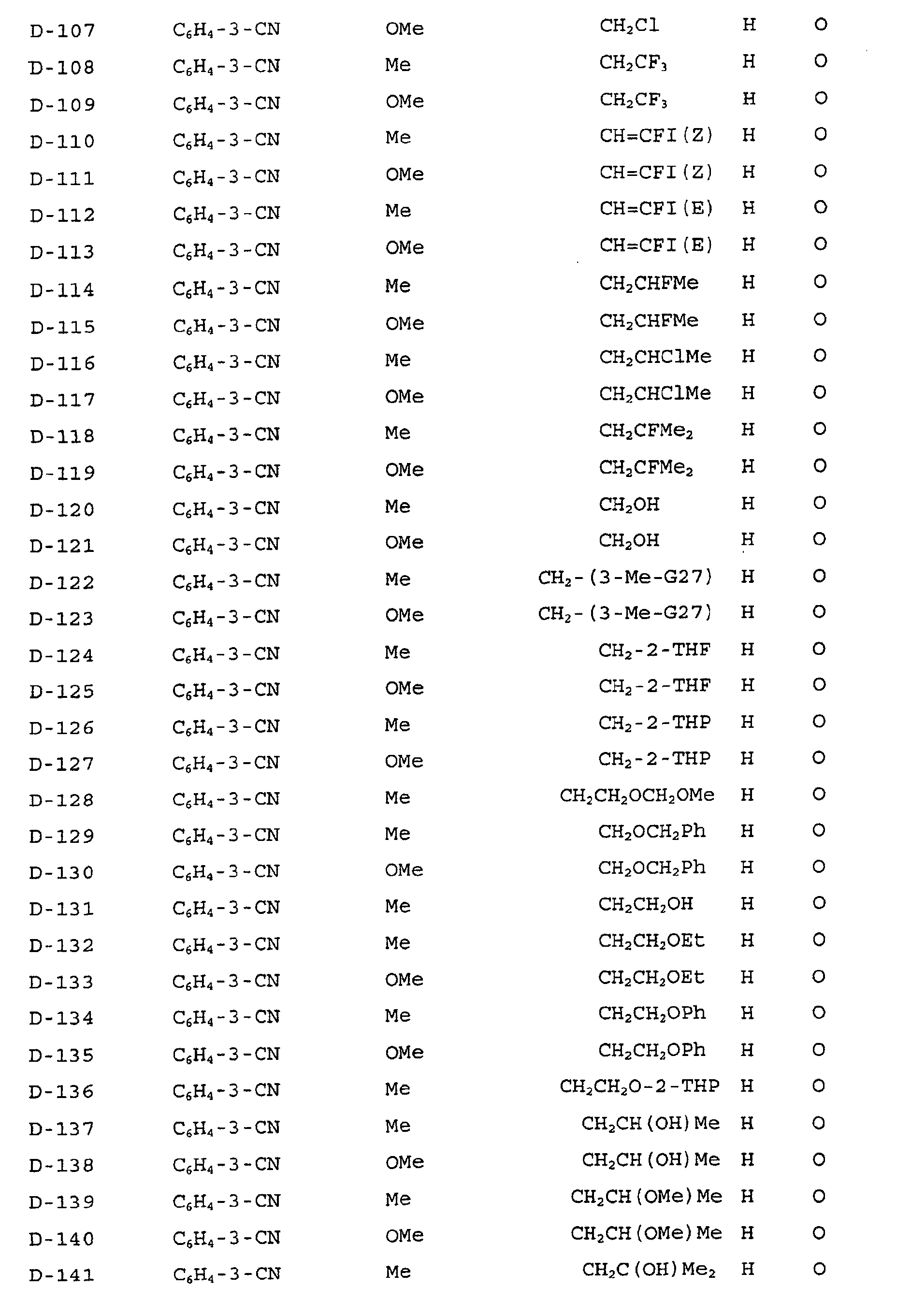

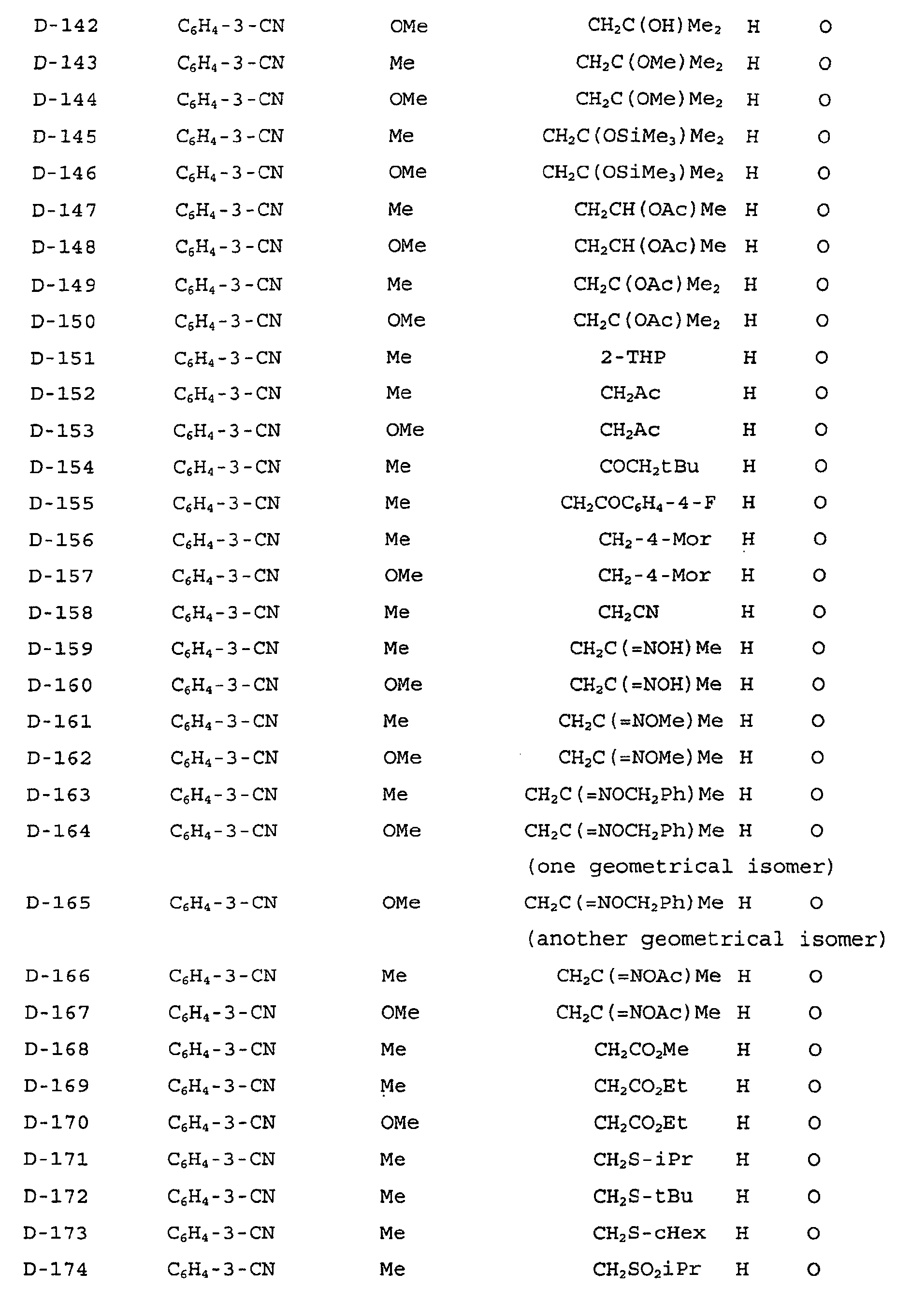

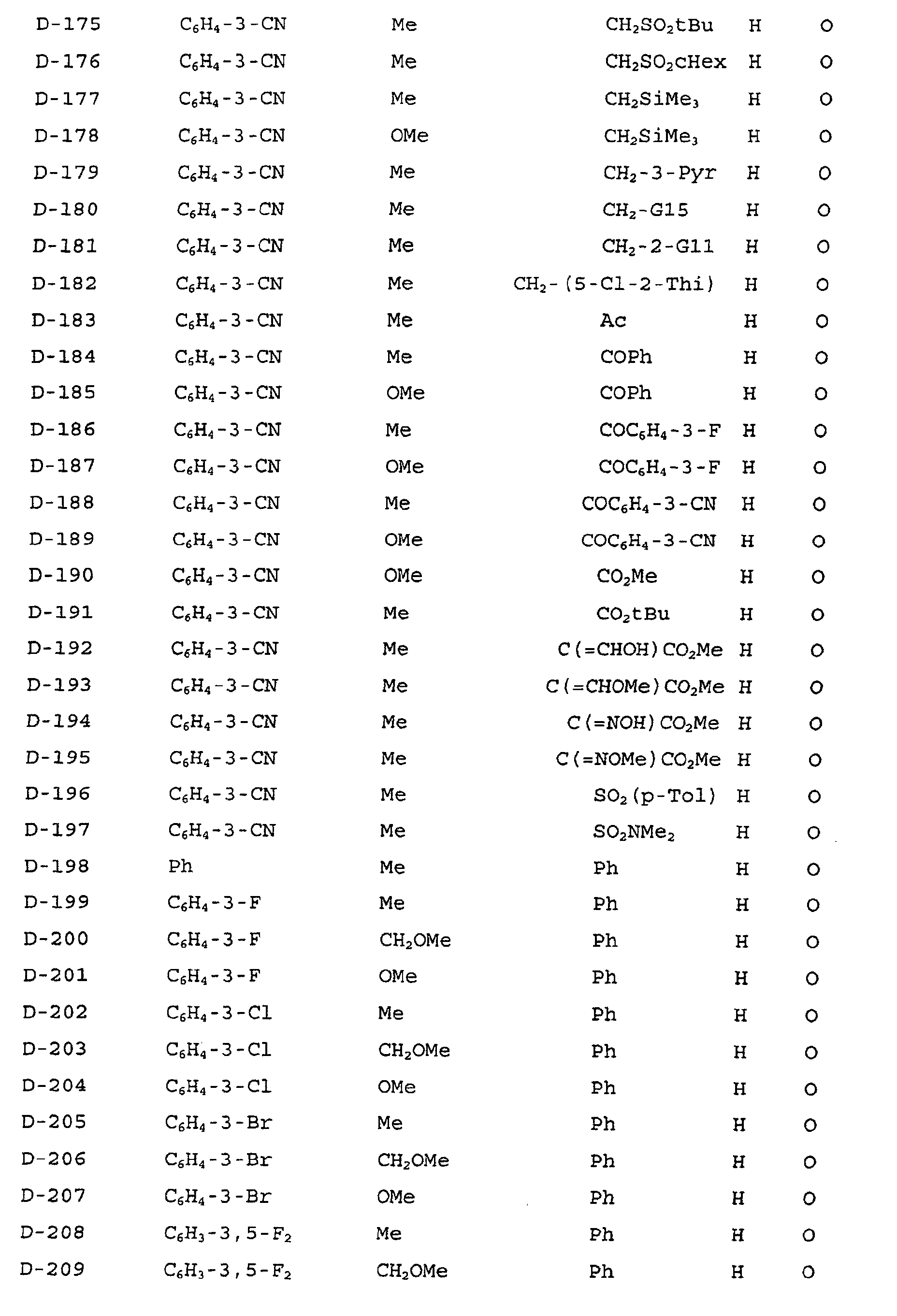

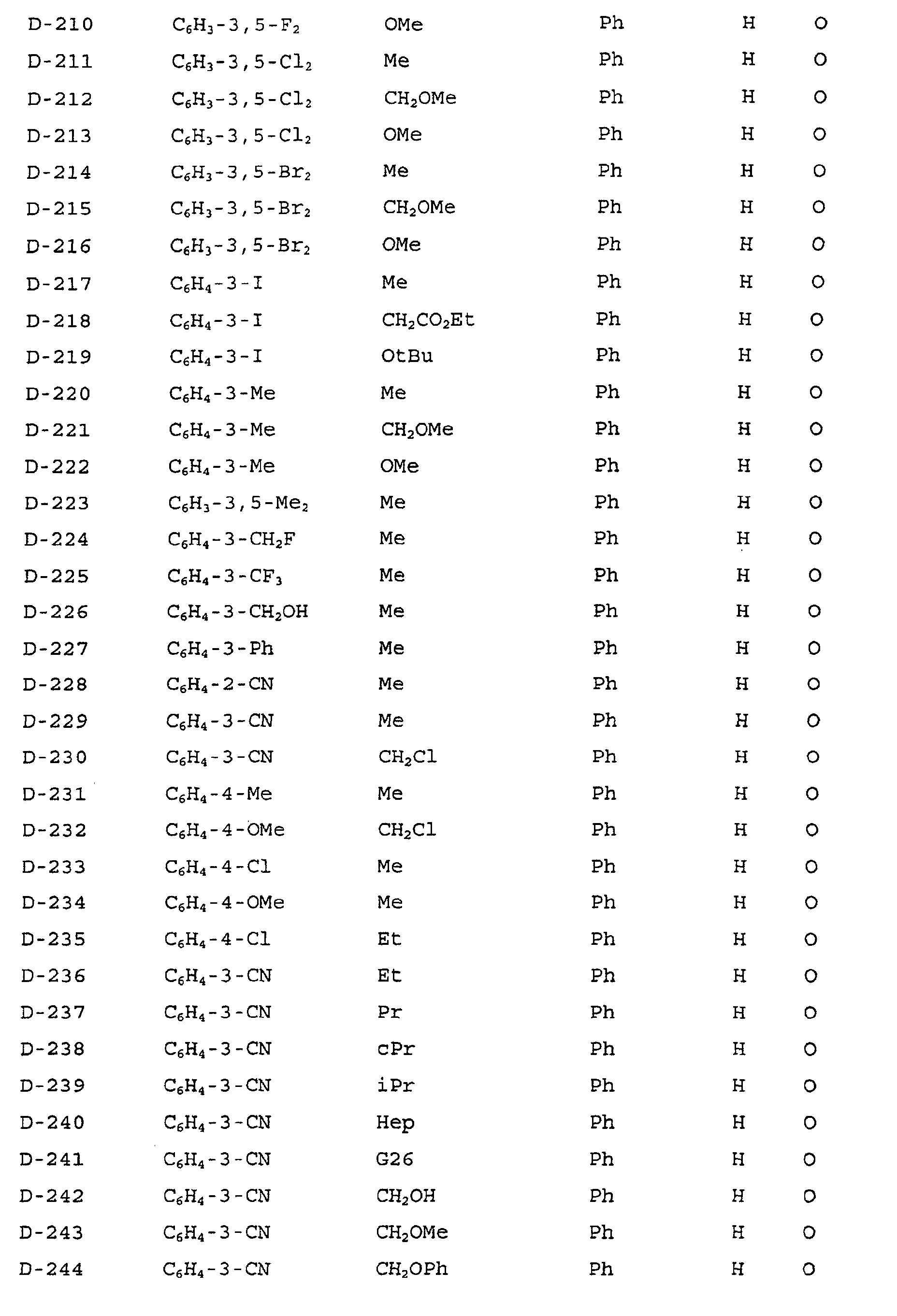

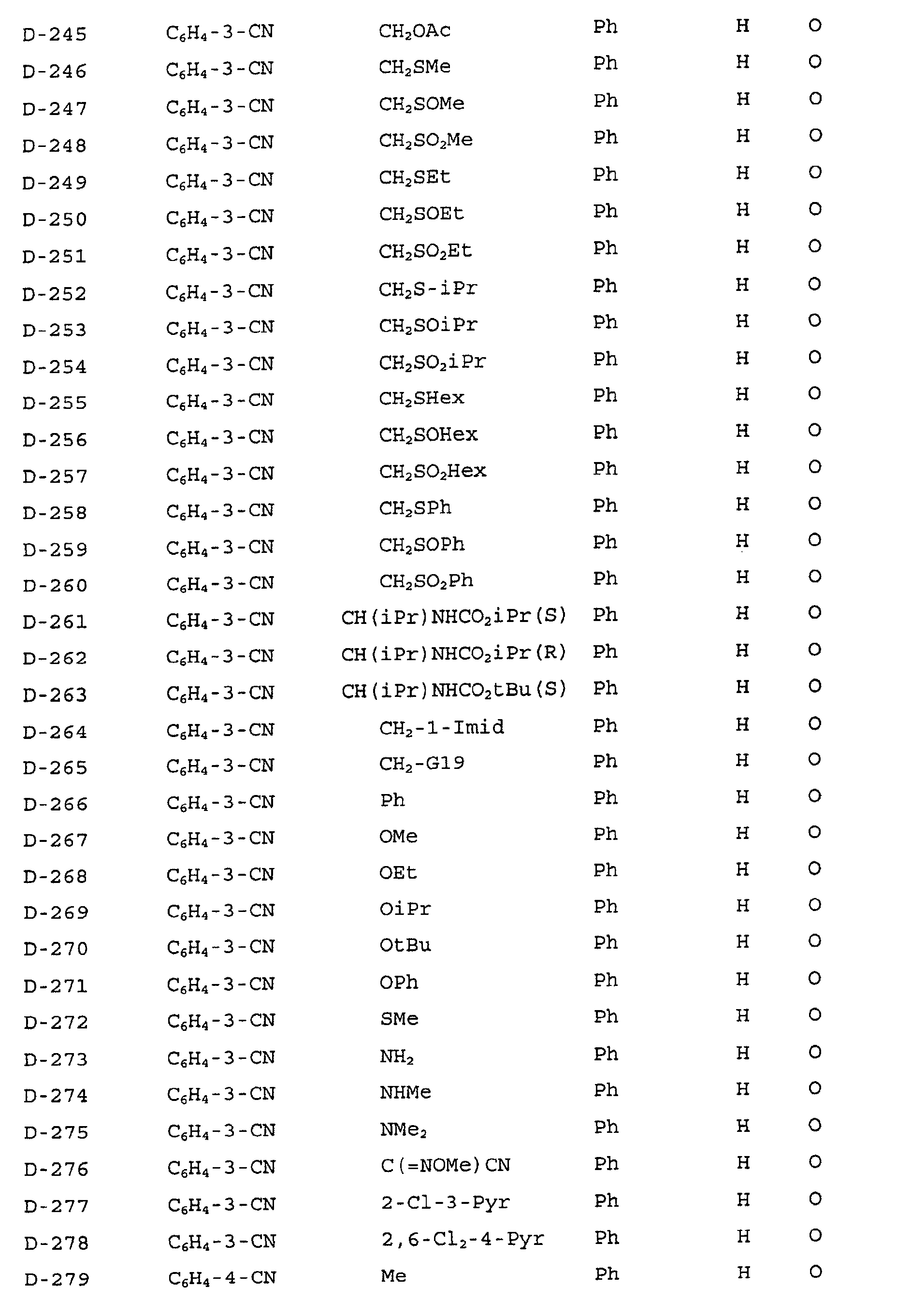

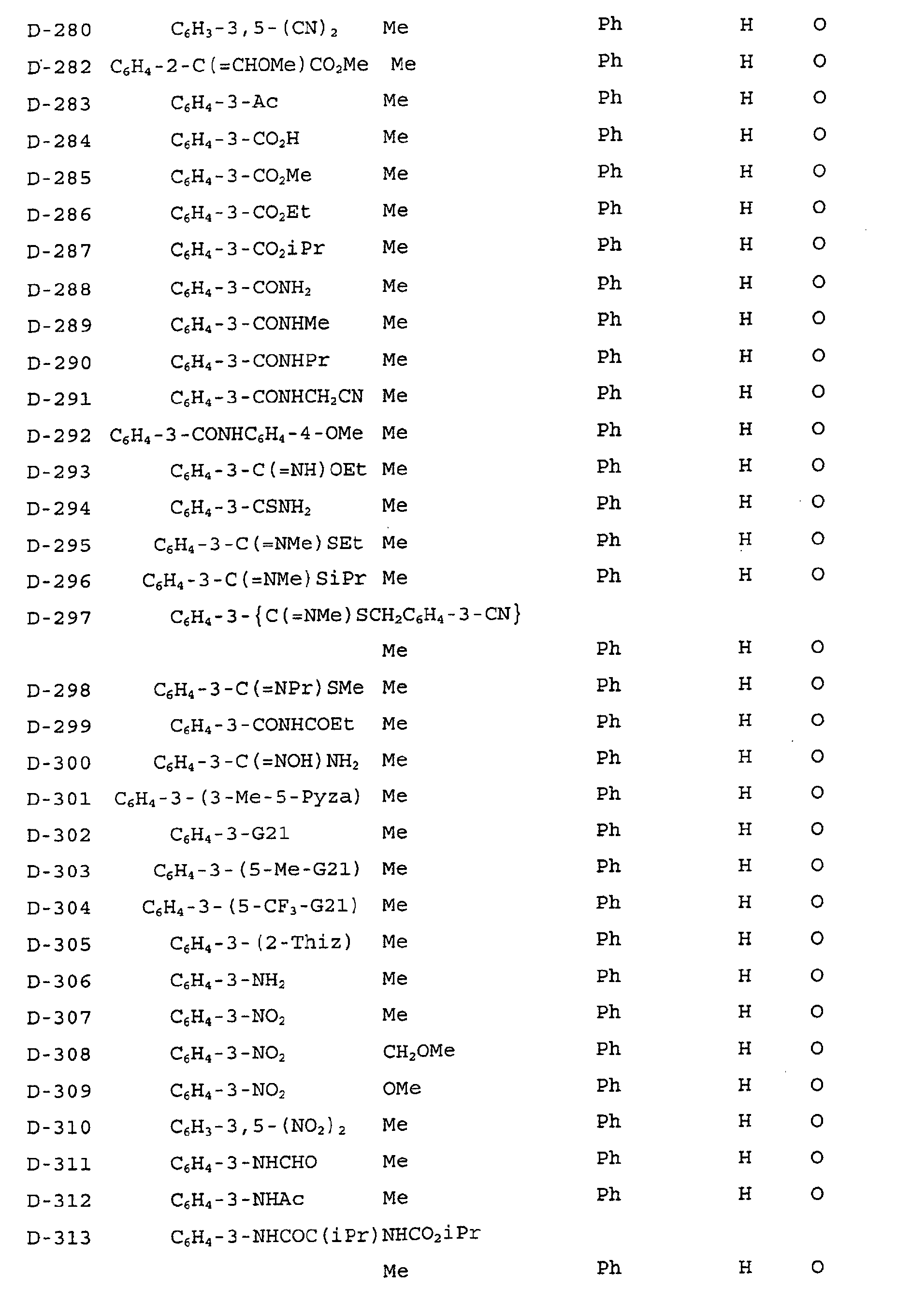

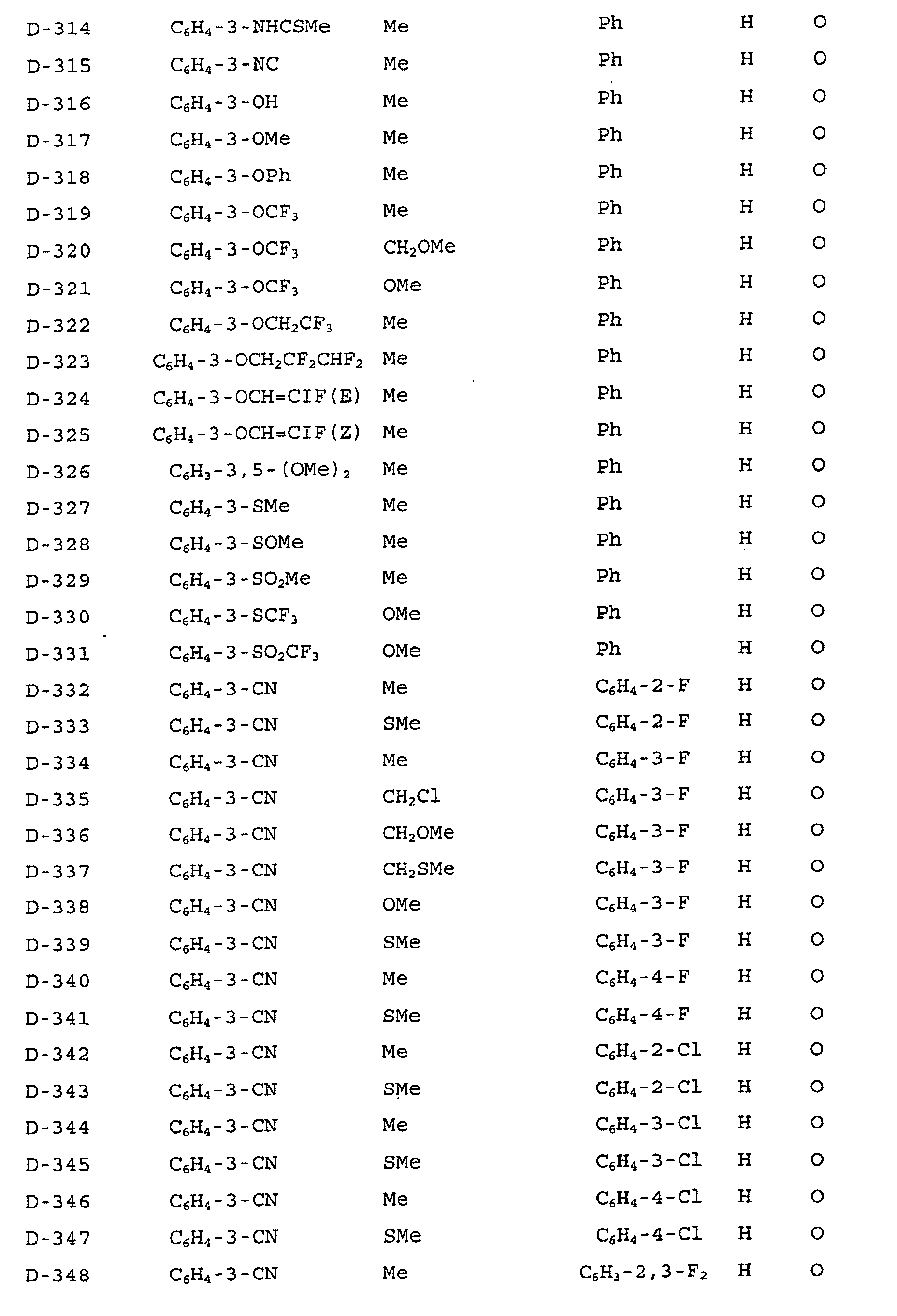

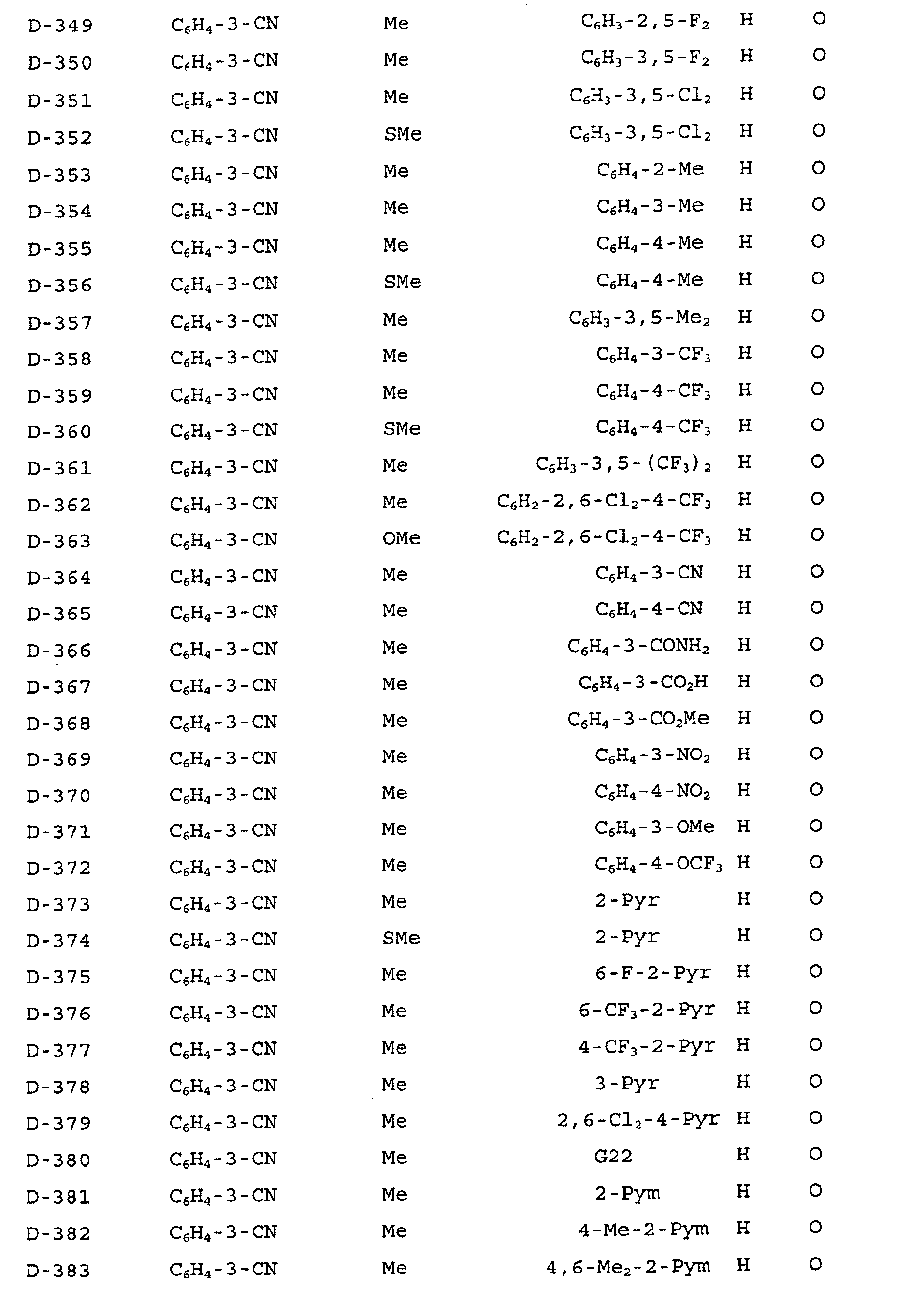

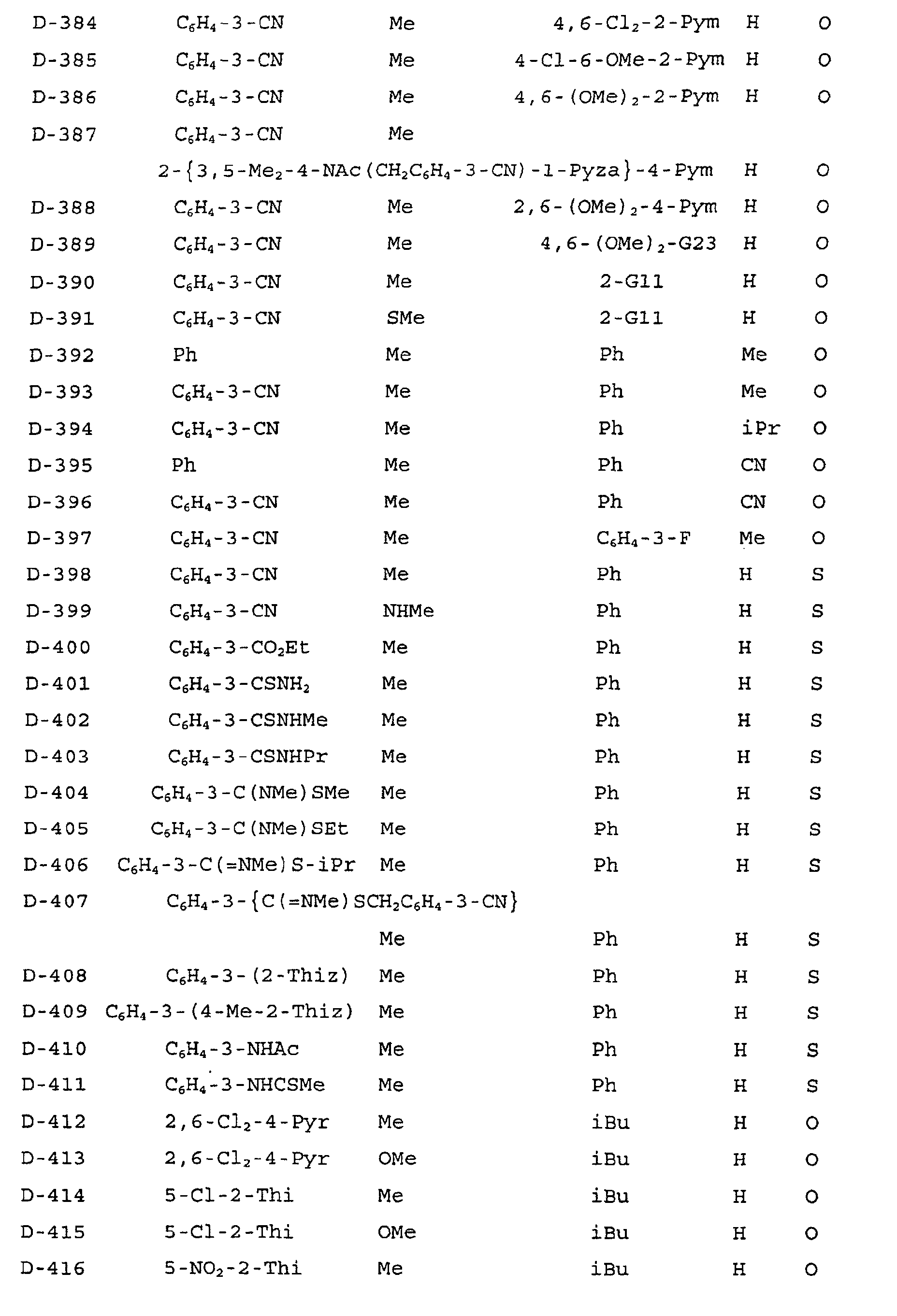

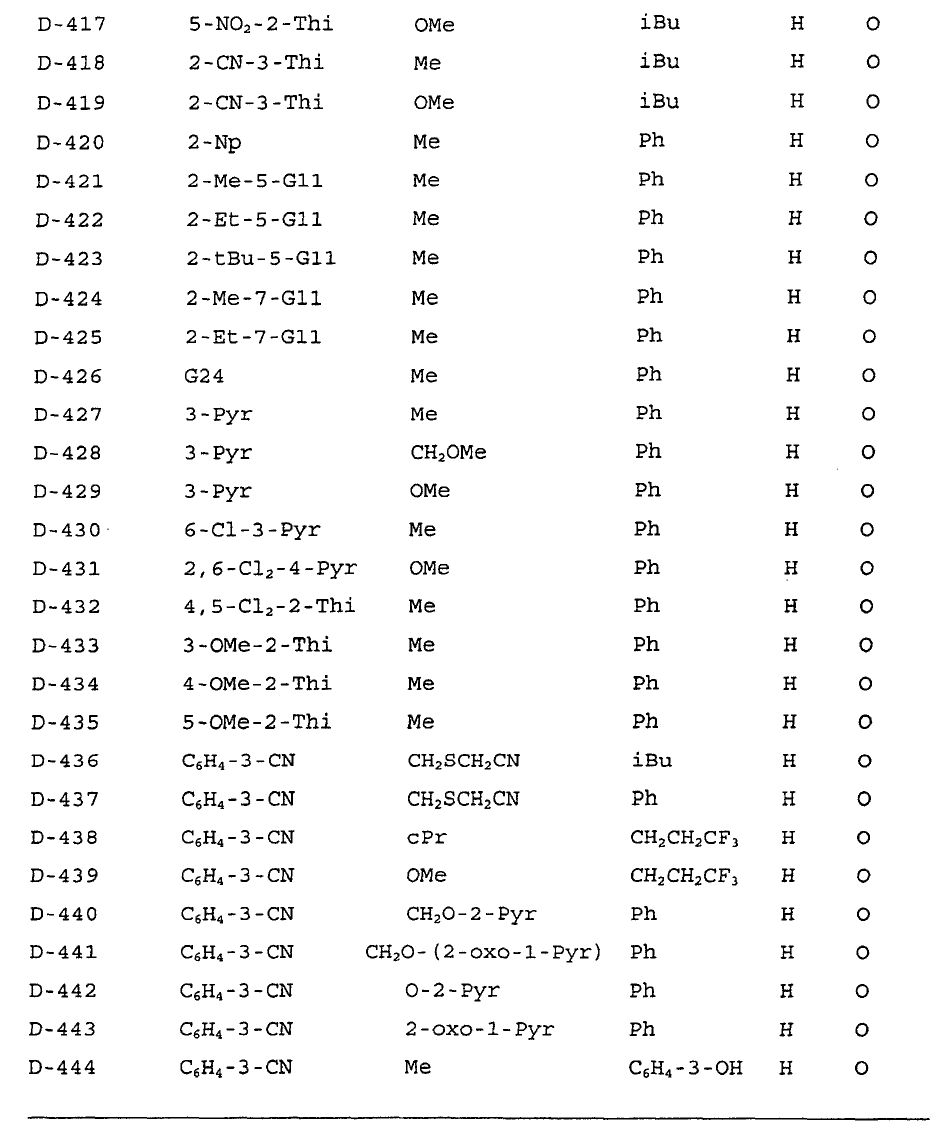

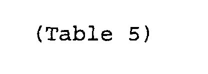

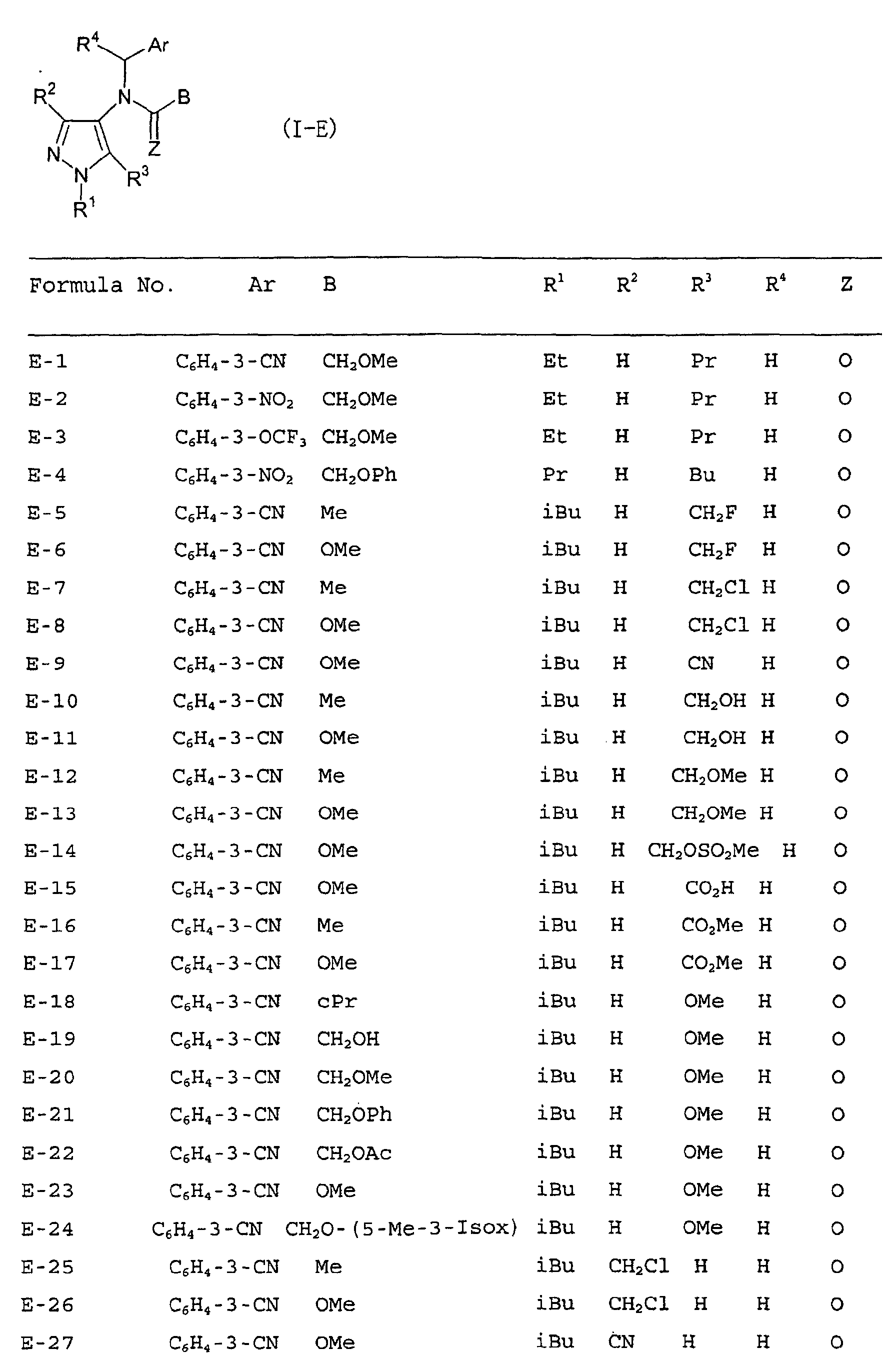

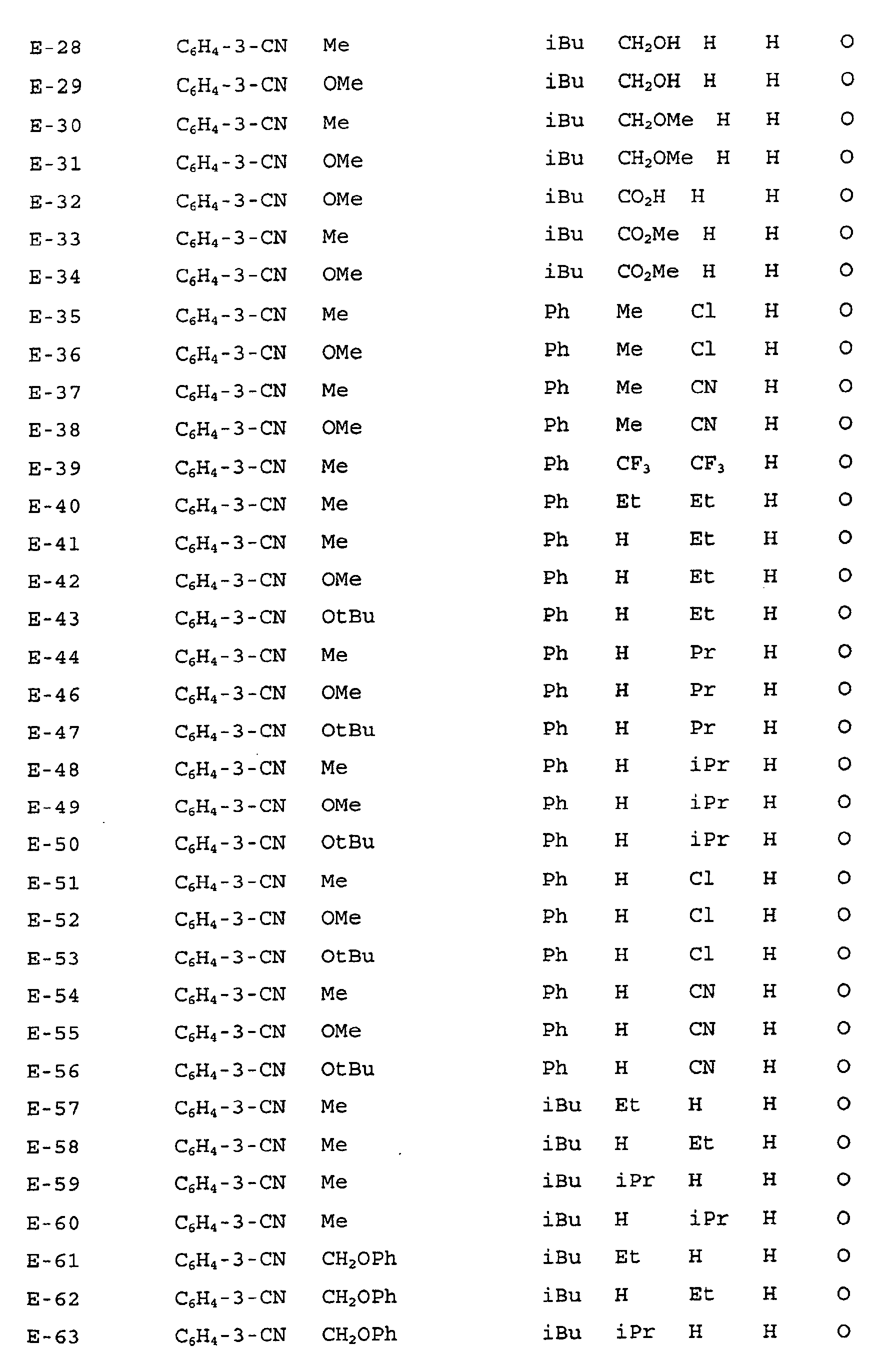

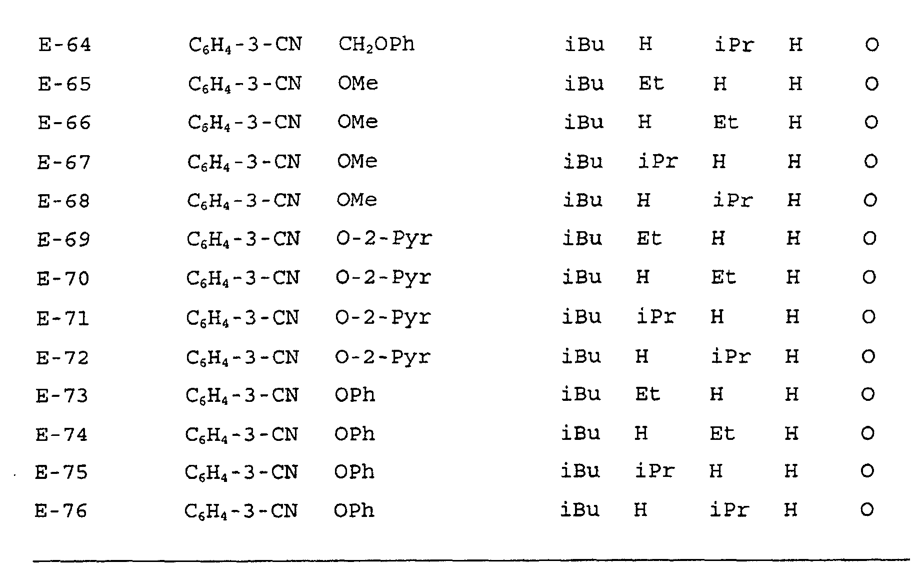

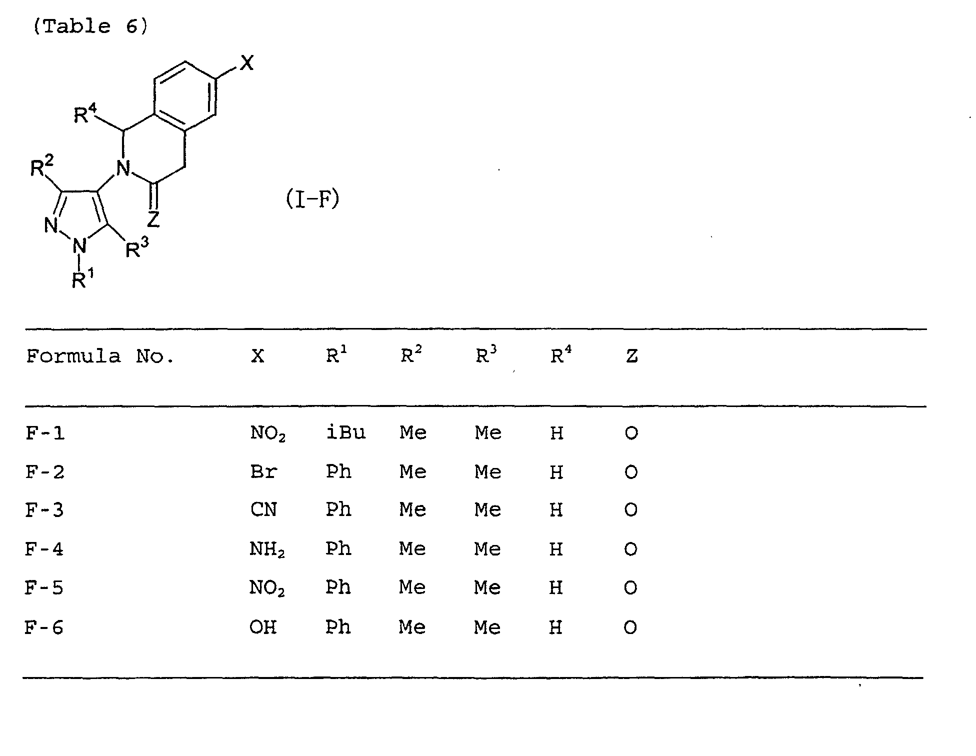

Among the aforementioned exemplified compounds, preferred compounds are compounds of Formula No. A-5, Formula No. A-12, Formula No. A- 17, Formula No. A-18, Formula No. A-19, Formula No. A-21, Formula No. A-23, Formula No. A-25, Formula No. A-27, Formula No. A-32, Formula No. A-33, Formula No. A-57, Formula No. A-58, Formula No. A-59, Formula No. A-60, Formula No. A-87, Formula No. A-90, Formula No. B-18, Formula No. B-20, Formula No. B-25, Formula No. B-27, Formula No. B-32, Formula No. B-61, Formula No. B-62, Formula No. B-63, Formula No. B-64, Formula No. B-65, Formula No. B-69, Formula No. B-71, Formula No. B-72, Formula No. B-79, Formula No. B-81, Formula No. B-83, Formula No. B-84, Formula No. B-85, Formula No. B-86, Formula No. B-87, Formula No. B-88, Formula No. B-89, Formula No. B-90, Formula No. B-91, Formula No. B-93, Formula No. B-94, Formula No. B-95, Formula No. B-96, Formula No. B-97, Formula No. B-102, Formula No. B-103, Formula No. B-104, Formula No. B-105, Formula No. B-106, Formula No. B-107, Formula No. B-108, Formula No. B-109, Formula No. B-110, Formula No. B-111, Formula No. B-112, Formula No. B-113, Formula No. B-114, Formula No. B-115, Formula No. B-120, Formula No. B-127, Formula No. B-128, Formula No. B-168, Formula No. B-171, Formula No. B-181, Formula No. B-182, Formula No. B-183, Formula No. B-184, Formula No. B-185, Formula No. B-186, Formula No. B-187, Formula No. B-188, Formula No. B-189, Formula No. B-190, Formula No. B-191, Formula No. B-192, Formula No. B-193, Formula No. B-194, Formula No. B-195, Formula No. B-196, Formula No. B-197, Formula No. B-199, Formula No. B-200, Formula No. B-201, Formula No. B-202, Formula No. B-203, Formula No. B-208, Formula No. B-212, Formula No. B-215, Formula No. B-220, Formula No. B-227, Formula No. B-228, Formula No. B-229, Formula No. B-230, Formula No. B-231, Formula No. B-232, Formula No. B-233, Formula No. B-234, Formula No. B-235, Formula No. B-236, Formula No. B-237, Formula No. B-238, Formula No. B-239, Formula No. B-240, Formula No. B-241, Formula No. B-242, Formula No. B-243, Formula No. B-244, Formula No. B-245, Formula No. B-246, Formula No. B-247, Formula No. B-248, Formula No. B-249, Formula No. B-250, Formula No. B-251, Formula No. B-252, Formula No. B-253, Formula No. B-254, Formula No. B-255, Formula No. B-256, Formula No. B-257, Formula No. B-258, Formula No. B-259, Formula No. B-260, Formula No. B-261, Formula No. B-262, Formula No. B-263, Formula No. B-264, Formula No. B-265, Formula No. B-266, Formula No. B-267, Formula No. B-268, Formula No. B-269, Formula No. B-270, Formula No. B-271, Formula No. B-272, Formula No. B-273, Formula No. B-274, Formula No. B-275, Formula No. B-276, Formula No. B-277, Formula No. B-278, Formula No. B-279, Formula No. B-280, Formula No. B-281, Formula No. B-282, Formula No. B-283, Formula No. B-284, Formula No. B-285, Formula No. B-286, Formula No. B-287, Formula No. B-288, Formula No. B-289, Formula No. B-290, Formula No. B-291, Formula No. B-292, Formula No. B-293, Formula No. B-294, Formula No. B-295, Formula No. B-296, Formula No. B-297, Formula No. B-298, Formula No. B-299, Formula No. B-300, Formula No. B-301, Formula No. B-302, Formula No. B-303, Formula No. B-306, Formula No. B-307, Formula No. B-309, Formula No. B-310, Formula No. B-311, Formula No. B-368, Formula No. C-15, Formula No. C-18, Formula No. C-19, Formula No. C-20, Formula No. C-21, Formula No. C-27, Formula No. C-28, Formula No. C-55, Formula No. C-60, Formula No. C-61, Formula No. C-62, Formula No. C-63, Formula No. C-64, Formula No. C-65, Formula No. C-66, Formula No. C-68, Formula No. C-69, Formula No. C-70, Formula No. C-71, Formula No. C-72, Formula No. C-79, Formula No. C-81, Formula No. C-82, Formula No. C-83, Formula No. C-84, Formula No. C-85, Formula No. C-86, Formula No. C-87, Formula No. C-88, Formula No. C-89, Formula No. C-90, Formula No. C-91, Formula No. C-92, Formula No. C-93, Formula No. C-94, Formula No. C-95, Formula No. C-96, Formula No. C-97, Formula No. C-99, Formula No. C-100, Formula No. C-101, Formula No. C-102, Formula No. C-103, Formula No. C-104, Formula No. C-105, Formula No. C-106, Formula No. C-107, Formula No. C-111, Formula No. C-116, Formula No. C-117, Formula No. C-120, Formula No. C-122, Formula No. C-123, Formula No. C-124, Formula No. C-125, Formula No. C-126, Formula No. C-127, Formula No. C-128, Formula No. C-129, Formula No. C-130, Formula No. C-131, Formula No. C-132, Formula No. C-133, Formula No. C-134, Formula No. C-136, Formula No. C-137, Formula No. C-138, Formula No. C-139, Formula No. C-140, Formula No. C-142, Formula No. C-144, Formula No. C-145, Formula No. C-146, Formula No. C-147, Formula No. C-148, Formula No. C-149, Formula No. C-151, Formula No. C-152, Formula No. C-153, Formula No. C-154, Formula No. C-155, Formula No. C-157, Formula No. C-159, Formula No. C-160, Formula No. C-161, Formula No. C-169, Formula No. C-172, Formula No. C-173, Formula No. C-176, Formula No. C-177, Formula No. C-178, Formula No. C-179, Formula No. C-180, Formula No. C-181, Formula No. C-182, Formula No. C-184, Formula No. C-185, Formula No. C-186, Formula No. C-190, Formula No. C-192, Formula No. C-194, Formula No. C-200, Formula No. C-201, Formula No. C-202, Formula No. C-203, Formula No. C-204, Formula No. C-205, Formula No. C-206, Formula No. C-207, Formula No. C-208, Formula No. C-209, Formula No. C-210, Formula No. C-211, Formula No. C-212, Formula No. C-213, Formula No. C-216, Formula No. C-217, Formula No. C-218, Formula No. C-219, Formula No. C-220, Formula No. C-221, Formula No. C-222, Formula No. C-223, Formula No. C-224, Formula No. C-226, Formula No. C-227, Formula No. C-228, Formula No. C-230, Formula No. C-231, Formula No. C-232, Formula No. C-233, Formula No. C-234, Formula No. C-237, Formula No. C-238, Formula No. C-239, Formula No. C-240, Formula No. C-241, Formula No. C-242, Formula No. C-243, Formula No. C-244, Formula No. C-245, Formula No. C-248, Formula No. C-249, Formula No. C-250, Formula No. C-251, Formula No. C-252, Formula No. C-253, Formula No. C-255, Formula No. C-256, Formula No. C-259, Formula No. C-260, Formula No. C-261, Formula No. C-262, Formula No. C-263, Formula No. C-264, Formula No. C-265, Formula No. C-266, Formula No. C-267, Formula No. C-268, Formula No. C-269, Formula No. C-270, Formula No. C-271, Formula No. C-272, Formula No. C-275, Formula No. C-276, Formula No. C-278, Formula No. C-279, Formula No. C-280, Formula No. C-281, Formula No. C-282, Formula No. C-283, Formula No. C-285, Formula No. C-287, Formula No. C-290, Formula No. C-295, Formula No. C-298, Formula No. C-299, Formula No. C-301, Formula No. C-304, Formula No. C-306, Formula No. C-309, Formula No. C-312, Formula No. C-313, Formula No. C-314, Formula No. C-315, Formula No. C-316, Formula No. C-317, Formula No. C-319, Formula No. C-320, Formula No. C-321, Formula No. C-341, Formula No. C-342, Formula No. C-343, Formula No. C-347, Formula No. C-348, Formula No. C-349, Formula No. C-350, Formula No. C-351, Formula No. C-353, Formula No. C-354, Formula No. C-355, Formula No. C-356, Formula No. C-363, Formula No. C-364, Formula No. C-366, Formula No. C-368, Formula No. C-371, Formula No. C-372, Formula No. C-373, Formula No. C-377, Formula No. C-378, Formula No. C-404, Formula No. C-405, Formula No. C-410, Formula No. C-416, Formula No. C-419, Formula No. C-421, Formula No. C-429, Formula No. C-430, Formula No. C-431, Formula No. C-432, Formula No. C-433, Formula No. C-434, Formula No. C-435, Formula No. C-436, Formula No. C-438, Formula No. C-439, Formula No. C-440, Formula No. C-441, Formula No. C-442, Formula No. C-443, Formula No. C-444, Formula No. C-446, Formula No. C-447, Formula No. C-448, Formula No. C-449, Formula No. C-450, Formula No. C-451, Formula No. C-453, Formula No. C-456, Formula No. C-457, Formula No. C-458, Formula No. C-460, Formula No. C-462, Formula No. C-463, Formula No. C-466, Formula No. C-468, Formula No. C-469, Formula No. C-472, Formula No. C-474, Formula No. C-476, Formula No. C-478, Formula No. C-479, Formula No. C-480, Formula No. C-481, Formula No. C-482, Formula No. C-483, Formula No. C-484, Formula No. C-485, Formula No. C-486, Formula No. C-487, Formula No. C-488, Formula No. C-489, Formula No. C-490, Formula No. C-491, Formula No. C-492, Formula No. C-493, Formula No. C-494, Formula No. C-496, Formula No. C-497, Formula No. C-498, Formula No. C-499, Formula No. C-500, Formula No. C-501, Formula No. C-503, Formula No. C-505, Formula No. C-506, Formula No. C-507, Formula No. C-508_{,} Formula No. C-509, Formula No. C-510, Formula No. C-511, Formula No. C-512, Formula No. C-513, Formula No. C-514, Formula No. C-515, Formula No. C-516, Formula No. C-517, Formula No. C-518, Formula No. C-519, Formula No. C-520, Formula No. C-521, Formula No. C-522, Formula No. C-523, Formula No. C-524, Formula No. C-525, Formula No. C-526, Formula No. C-527, Formula No. C-528, Formula No. C-529, Formula No. C-530, Formula No. C-531, Formula No. C-532, Formula No. C-533, Formula No. C-534, Formula No. C-535, Formula No. C-536, Formula No. C-537, Formula No. C-538, Formula No. C-539, Formula No. C-540, Formula No. C-541, Formula No. C-542, Formula No. C-543, Formula No. C-544, Formula No. C-545, Formula No. C-546, Formula No. C-547, Formula No. C-548, Formula No. C-549, Formula No. C-550, Formula No. C-551, Formula No. C-552, Formula No. C-553, Formula No. C-554, Formula No. C-561, Formula No. C-564, Formula No. C-565, Formula No. C-566, Formula No. C-567, Formula No. C-568, Formula No. C-569, Formula No. C-570, Formula No. C-571, Formula No. C-572, Formula No. C-573, Formula No. C-574, Formula No. C-575, Formula No. C-576, Formula No. C-577, Formula No. C-578, Formula No. C-579, Formula No. C-580, Formula No. C-581, Formula No. C-582, Formula No. C-583, Formula No. C-584, Formula No. C-585, Formula No. C-586, Formula No. C-587, Formula No. C-588, Formula No. C-589, Formula No. C-590, Formula No. C-591, Formula No. C-592, Formula No. C-593, Formula No. C-594, Formula No. C-595, Formula No. C-596, Formula No. C-597, Formula No. C-598, Formula No. C-599, Formula No. C-600, Formula No. C-601, Formula No. C-602, Formula No. C-603, Formula No. C-604, Formula No. C-605, Formula No. C-606, Formula No. C-607, Formula No. C-608, Formula No. C-609, Formula No. C-611, Formula No. C-612, Formula No. C-613, Formula No. C-614, Formula No. C-615, Formula No. C-616, Formula No. C-617, Formula No. C-618, Formula No. C-619, Formula No. C-620, Formula No. C-621, Formula No. C-622, Formula No. C-623, Formula No. C-624, Formula No. C-625, Formula No. C-626, Formula No. C-627, Formula No. C-628, Formula No. C-629, Formula No. C-630, Formula No. C-631, Formula No. C-632, Formula No. C-633, Formula No. C-635, Formula No. C-636, Formula No. C-637, Formula No. C-638, Formula No. C-639, Formula No. C-640, Formula No. C-641, Formula No. C-642, Formula No. C-645, Formula No. C-649, Formula No. C-650, Formula No. C-651, Formula No. C-653, Formula No. C-654, Formula No. C-655, Formula No. C-657, Formula No. C-658, Formula No. C-659, Formula No. C-660, Formula No. C-661, Formula No. C-662, Formula No. C-663, Formula No. C-664, Formula No. C-665, Formula No. C-666, Formula No. C-667, Formula No. C-668, Formula No. C-669, Formula No. C-670, Formula No. C-671, Formula No. C-672, Formula No. C-673, Formula No. C-674, Formula No. C-675, Formula No. C-676, Formula No. C-677, Formula No. C-678, Formula No. C-680, Formula No. C-682, Formula No. C-686, Formula No. C-688, Formula No. C-689, Formula No. C-690, Formula No. C-692, Formula No. C-693, Formula No. C-694, Formula No. C-695, Formula No. C-696, Formula No. C-697, Formula No. C-698, Formula No. C-699, Formula No. C-700, Formula No. C-701, Formula No. C-702, Formula No. C-703, Formula No. C-704, Formula No. C-705, Formula No. C-706, Formula No. C-707, Formula No. C-708, Formula No. C-709, Formula No. C-710, Formula No. C-711, Formula No. C-712, Formula No. C-713, Formula No. C-715, Formula No. C-717, Formula No. C-718, Formula No. C-720, Formula No. C-725, Formula No. C-726, Formula No. C-729, Formula No. C-730, Formula No. C-731, Formula No. C-732, Formula No. C-734, Formula No. C-735, Formula No. C-736, Formula No. C-737, Formula No. C-738, Formula No. C-739, Formula No. C-740, Formula No. C-742, Formula No. C-743, Formula No. C-748, Formula No. C-750, Formula No. C-752, Formula No. C-774, Formula No. C-775, Formula No. C-776, Formula No. C-777, Formula No. C-780, Formula No. C-785, Formula No. C-787, Formula No. C-792, Formula No. C-800, Formula No. C-801, Formula No. C-814, Formula No. C-843, Formula No. C-872, Formula No. C-925, Formula No. C-930, Formula No. C-931, Formula No. C-934, Formula No. C-936, Formula No. C-942, Formula No. C-943, Formula No. C-945, Formula No. C-947, Formula No. C-949, Formula No. C-950, Formula No. C-951, Formula No. C-953, Formula No. C-962, Formula No. C-963, Formula No. C-964, Formula No. C-965, Formula No. C-966, Formula No. C-977, Formula No. C-1017, Formula No. C-1018, Formula No. C-1028, Formula No. C-1029, Formula No. C-1030, Formula No. C-1031, Formula No. C-1032, Formula No. C-1033, Formula No. C-1038, Formula No. C-1039, Formula No. C-1040, Formula No. C-1041, Formula No. C-1043, Formula No. C-1045, Formula No. C-1053, Formula No. C-1054, Formula No. C-1055, Formula No. C-1060, Formula No. C-1072, Formula No. C-1118, Formula No. C-1119, Formula No. C-1140, Formula No. C-1142, Formula No. C-1147, Formula No. C-1162, Formula No. D-12, Formula No. D-13, Formula No. D-14, Formula No. D-19, Formula No. D-20, Formula No. D-21, Formula No. D-24, Formula No. D-25, Formula No. D-26, Formula No. D-29, Formula No. D-36, Formula No. D-37, Formula No. D-43, Formula No. D-45, Formula No. D-46, Formula No. D-47, Formula No. D-48, Formula No. D-51, Formula No. D-53, Formula No. D-54, Formula No. D-57, Formula No. D-63, Formula No. D-64, Formula No. D-67, Formula No. D-70, Formula No. D-71, Formula No. D-72, Formula No. D-73, Formula No. D-76, Formula No. D-77,. Formula No. D-78, Formula No. D-79, Formula No. D-80, Formula No. D-81, Formula No. D-82, Formula No. D-83, Formula No. D-84, Formula No. D-85, Formula No. D-86, Formula No. D-87, Formula No. D-88, Formula No. D-90, Formula No. D-91, Formula No. D-94, Formula No. D-95, Formula No. D-96, Formula No. D-97, Formula No. D-98, Formula No. D-99, Formula No. D-100, Formula No. D-101, Formula No. D-102, Formula No. D-114, Formula No. D-116, Formula No. D-117, Formula No. D-118, Formula No. D-119, Formula No. D-122, Formula No. D-123, Formula No. D-125, Formula No. D-126, Formula No. D-127, Formula No. D-140, Formula No. D-143, Formula No. D-144, Formula No. D-146, Formula No. D-147, Formula No. D-150, Formula No. D-154, Formula No. D-171, Formula No. D-172, Formula No. D-173, Formula No. D-174, Formula No. D-175, Formula No. D-177, Formula No. D-178, Formula No. D-187, Formula No. D-197, Formula No. D-203, Formula No. D-204, Formula No. D-205, Formula No. D-207, Formula No. D-209, Formula No. D-210, Formula No. D-215, Formula No. D-218, Formula No. D-236, Formula No. D-237, Formula No. D-238, Formula No. D-239, Formula No. D-240, Formula No. D-242, Formula No. D-243, Formula No. D-244, Formula No. D-245, Formula No. D-246, Formula No. D-248, Formula No. D-252, Formula No. D-253, Formula No. D-254, Formula No. D-256, Formula No. D-260, Formula No. D-264, Formula No. D-265, Formula No. D-268, Formula No. D-269, Formula No. D-271, Formula No. D-272, Formula No. D-274, Formula No. D-275, Formula No. D-288, Formula No. D-293, Formula No. D-302, Formula No. D-307, Formula No. D-308, Formula No. D-309, Formula No. D-315, Formula No. D-319, Formula No. D-320, Formula No. D-321, Formula No. D-328, Formula No. D-329, Formula No. D-332, Formula No. D-334, Formula No. D-335, Formula No. D-336, Formula No. D-337, Formula No. D-338, Formula No. D-339, Formula No. D-340, Formula No. D-341, Formula No. D-342, Formula No. D-343, Formula No. D-344, Formula No. D-348, Formula No. D-349, Formula No. D-350, Formula No. D-353, Formula No. D-354, Formula No. D-355, Formula No. D-358, Formula No. D-365, Formula No. D-366, Formula No. D-393, Formula No. D-397, Formula No. E-6, Formula No. E-36, Formula No. E-38, Formula No. E-43 and Formula No. F-2;
more preferably compounds of Formula No. A-12, Formula No. A-19, Formula No. A-21, Formula No. A-23, Formula No. A-25, Formula No. A-27, Formula No. A-32, Formula No. A-33, Formula No. A-57, Formula No. A-58, Formula No. A-59, Formula No. A-60, Formula No. A-87, Formula No. A-90, Formula No. B-18, Formula No. B-20, Formula No. B-25, Formula No. B-32, Formula No. B-64, Formula No. B-65, Formula No. B-71, Formula No. B-81, Formula No. B-83, Formula No. B-85, Formula No. B-86, Formula No. B-87, Formula No. B-88, Formula No. B-89, Formula No. B-90, Formula No. B-96, Formula No. B-102, Formula No. B-103, Formula No. B-104, Formula No. B-105, Formula No. B-106, Formula No. B-107, Formula No. B-108, Formula No. B-109, Formula No. B-110, Formula No. B-111, Formula No. B-112, Formula No. B-113, Formula No. B-114, Formula No. B-127, Formula No. B-171, Formula No. B-181, Formula No. B-182, Formula No. B-183, Formula No. B-184, Formula No. B-185, Formula No. B-186, Formula No. B-187, Formula No. B-188, Formula No. B-190, Formula No. B-191, Formula No. B-193, Formula No. B-195, Formula No. B-196, Formula No. B-197, Formula No. B-199, Formula No. B-201, Formula No. B-202, Formula No. B-203, Formula No. B-208, Formula No. B-212, Formula No. B-220, Formula No. B-227, Formula No. B-230, Formula No. B-231, Formula No. B-232, Formula No. B-233, Formula No. B-234, Formula No. B-235, Formula No. B-236, Formula No. B-237, Formula No. B-238, Formula No. B-239, Formula No. B-240, Formula No. B-241, Formula No. B-242, Formula No. B-243, Formula No. B-244, Formula No. B-245, Formula No. B-246, Formula No. B-247, Formula No. B-248, Formula No. B-249, Formula No. B-250, Formula No. B-251, Formula No. B-252, Formula No. B-254, Formula No. B-255, Formula No. B-256, Formula No. B-257, Formula No. B-258, Formula No. B-259, Formula No. B-260, Formula No. B-261, Formula No. B-262, Formula No. B-263, Formula No. B-264, Formula No. B-265, Formula No. B-266, Formula No. B-267, Formula No. B-268, Formula No. B-269, Formula No. B-270, Formula No. B-271, Formula No. B-272, Formula No. B-273, Formula No. B-274, Formula No. B-275, Formula No. B-276, Formula No. B-277, Formula No. B-278, Formula No. B-279, Formula No. B-280, Formula No. B-281, Formula No. B-282, Formula No. B-283, Formula No. B-284, Formula No. B-285, Formula No. B-286, Formula No. B-287, Formula No. B-288, Formula No. B-289, Formula No. B-290, Formula No. B-291, Formula No. B-292, Formula No. B-293, Formula No. B-294, Formula No. B-295, Formula No. B-296, Formula No. B-297, Formula No. B-298, Formula No. B-299, Formula No. B-300, Formula No. B-301, Formula No. B-302, Formula No. B-303, Formula No. B-306, Formula No. B-307, Formula No. B-309, Formula No. B-310, Formula No. B-311, Formula No. B-368, Formula No. C-15, Formula No. C-18, Formula No. C-20, Formula No. C-21, Formula No. C-28, Formula No. C-61, Formula No. C-62, Formula No. C-63, Formula No. C-64, Formula No. C-65, Formula No. C-72, Formula No. C-79, Formula No. C-81, Formula No. C-82, Formula No. C-83, Formula No. C-84, Formula No. C-85, Formula No. C-86, Formula No. C-87, Formula No. C-88, Formula No. C-89, Formula No. C-90, Formula No. C-91, Formula No. C-92, Formula No. C-93, Formula No. C-94, Formula No. C-95, Formula No. C-96, Formula No. C-97, Formula No. C-99, Formula No. C-100, Formula No. C-101, Formula No. C-102, Formula No. C-104, Formula No. C-105, Formula No. C-106, Formula No. C-107, Formula No. C-116, Formula No. C-117, Formula No. C-120, Formula No. C-122, Formula No. C-123, Formula No. C-124, Formula No. C-125, Formula No. C-126, Formula No. C-127, Formula No. C-128, Formula No. C-129, Formula No. C-130, Formula No. C-131, Formula No. C-132, Formula No. C-133, Formula No. C-134, Formula No. C-138, Formula No. C-139, Formula No. C-140, Formula No. C-142, Formula No. C-144, Formula No. C-145, Formula No. C-146, Formula No. C-147, Formula No. C-148, Formula No. C-149, Formula No. C-151, Formula No. C-152, Formula No. C-153, Formula No. C-154, Formula No. C-155, Formula No. C-157, Formula No. C-159, Formula No. C-161, Formula No. C-169, Formula No. C-172, Formula No. C-173, Formula No. C-176, Formula No. C-177, Formula No. C-178, Formula No. C-180, Formula No. C-181, Formula No. C-182, Formula No. C-184, Formula No. C-185, Formula No. C-186, Formula No. C-192, Formula No. C-194, Formula No. C-200, Formula No. C-201, Formula No. C-202, Formula No. C-203, Formula No. C-204, Formula No. C-205, Formula No. C-206, Formula No. C-207, Formula No. C-208, Formula No. C-209, Formula No. C-210, Formula No. C-211, Formula No. C-212, Formula No. C-213, Formula No. C-216, Formula No. C-217, Formula No. C-218, Formula No. C-219, Formula No. C-220, Formula No. C-221, Formula No. C-222, Formula No. C-223, Formula No. C-224, Formula No. C-226, Formula No. C-227, Formula No. C-228, Formula No. C-230, Formula No. C-231, Formula No. C-232, Formula No. C-233, Formula No. C-234, Formula No. C-237, Formula No. C-238, Formula No. C-239, Formula No. C-240, Formula No. C-241, Formula No. C-242, Formula No. C-243, Formula No. C-244, Formula No. C-245, Formula No. C-248, Formula No. C-249, Formula No. C-251, Formula No. C-252, Formula No. C-253, Formula No. C-255, Formula No. C-256, Formula No. C-260, Formula No. C-262, Formula No. C-263, Formula No. C-264, Formula No. C-265, Formula No. C-266, Formula No. C-267, Formula No. C-268, Formula No. C-269, Formula No. C-271, Formula No. C-275, Formula No. C-276, Formula No. C-282, Formula No. C-283, Formula No. C-285, Formula No. C-287, Formula No. C-295, Formula No. C-301, Formula No. C-304, Formula No. C-309, Formula No. C-312, Formula No. C-313, Formula No. C-314, Formula No. C-315, Formula No. C-317, Formula No. C-319, Formula No. C-320, Formula No. C-341, Formula No. C-342, Formula No. C-343, Formula No. C-347, Formula No. C-348, Formula No. C-349, Formula No. C-350, Formula No. C-351, Formula No. C-353, Formula No. C-354, Formula No. C-355, Formula No. C-356, Formula No. C-363, Formula No. C-364, Formula No. C-366, Formula No. C-368, Formula No. C-371, Formula No. C-372, Formula No. C-373, Formula No. C-377, Formula No. C-378, Formula No. C-404, Formula No. C-405, Formula No. C-410, Formula No. C-416, Formula No. C-421, Formula No. C-429, Formula No. C-430, Formula No. C-431, Formula No. C-432, Formula No. C-434, Formula No. C-435, Formula No. C-436, Formula No. C-438, Formula No. C-439, Formula No. C-440, Formula No. C-441, Formula No. C-443, Formula No. C-444, Formula No. C-447, Formula No. C-448, Formula No. C-449, Formula No. C-450, Formula No. C-451, Formula No. C-453, Formula No. C-457, Formula No. C-460, Formula No. C-463, Formula No. C-466, Formula No. C-468, Formula No. C-469, Formula No. C-472, Formula No. C-478, Formula No. C-479, Formula No. C-480, Formula No. C-481, Formula No. C-482, Formula No. C-483, Formula No. C-484, Formula No. C-485, Formula No. C-486, Formula No. C-487, Formula No. C-488, Formula No. C-489, Formula No. C-490, Formula No. C-491, Formula No. C-492, Formula No. C-493, Formula No. C-494, Formula No. C-496, Formula No. C-497, Formula No. C-498, Formula No. C-499, Formula No. C-500, Formula No. C-501, Formula No. C-503, Formula No. C-505, Formula No. C-506, Formula No. C-507, Formula No. C-508, Formula No. C-509, Formula No. C-510, Formula No. C-511, Formula No. C-512, Formula No. C-513, Formula No. C-514, Formula No. C-515, Formula No. C-516, Formula No. C-517, Formula No. C-518, Formula No. C-519, Formula No. C-520, Formula No. C-521, Formula No. C-522, Formula No. C-523, Formula No. C-524, Formula No. C-525, Formula No. C-526, Formula No. C-527, Formula No. C-528, Formula No. C-529, Formula No. C-530, Formula No. C-531, Formula No. C-532, Formula No. C-533, Formula No. C-534, Formula No. C-535, Formula No. C-536, Formula No. C-537, Formula No. C-538, Formula No. C-539, Formula No. C-540, Formula No. C-541, Formula No. C-542, Formula No. C-543, Formula No. C-544, Formula No. C-545, Formula No. C-546, Formula No. C-547, Formula No. C-548, Formula No. C-549, Formula No. C-550, Formula No. C-551, Formula No. C-552, Formula No. C-553, Formula No. C-554, Formula No. C-561, Formula No. C-564, Formula No. C-565, Formula No. C-566, Formula No. C-567, Formula No. C-568, Formula No. C-569, Formula No. C-570, Formula No. C-571, Formula No. C-572, Formula No. C-573, Formula No. C-574, Formula No. C-575, Formula No. C-576, Formula No. C-577, Formula No. C-578, Formula No. C-579, Formula No. C-580, Formula No. C-581, Formula No. C-582, Formula No. C-583, Formula No. C-584, Formula No. C-585, Formula No. C-586, Formula No. C-587, Formula No. C-588, Formula No. C-589, Formula No. C-590, Formula No. C-591, Formula No. C-592, Formula No. C-593, Formula No. C-594, Formula No. C-595, Formula No. C-596, Formula No. C-597, Formula No. C-598, Formula No. C-599, Formula No. C-600, Formula No. C-601, Formula No. C-602, Formula No. C-603, Formula No. C-604, Formula No. C-605, Formula No. C-606, Formula No. C-607, Formula No. C-608, Formula No. C-609, Formula No. C-611, Formula No. C-612, Formula No. C-613, Formula No. C-614, Formula No. C-615, Formula No. C-616, Formula No. C-617, Formula No. C-618, Formula No. C-619, Formula No. C-620, Formula No. C-621, Formula No. C-622, Formula No. C-623, Formula No. C-624, Formula No. C-625, Formula No. C-626, Formula No. C-627, Formula No. C-628, Formula No. C-629, Formula No. C-630, Formula No. C-631, Formula No. C-632, Formula No. C-633, Formula No. C-635, Formula No. C-636, Formula No. C-637, Formula No. C-639, Formula No. C-642, Formula No. C-649, Formula No. C-650, Formula No. C-653, Formula No. C-654, Formula No. C-655, Formula No. C-657, Formula No. C-658, Formula No. C-659, Formula No. C-660, Formula No. C-661, Formula No. C-662, Formula No. C-663, Formula No. C-664, Formula No. C-665, Formula No. C-666, Formula No. C-667, Formula No. C-668, Formula No. C-669, Formula No. C-670, Formula No. C-671, Formula No.. C-672, Formula No. C-673, Formula No. C-674, Formula No. C-675, Formula No. C-676, Formula No. C-677, Formula No. C-678, Formula No. C-682, Formula No. C-686, Formula No. C-688, Formula No. C-689, Formula No. C-690, Formula No. C-692, Formula No. C-693, Formula No. C-694, Formula No. C-695, Formula No. C-696, Formula No. C-697, Formula No. C-698, Formula No. C-699, Formula No. C-700, Formula No. C-701, Formula No. C-702, Formula No. C-703, Formula No. C-704, Formula No. C-705, Formula No. C-706, Formula No. C-707, Formula No. C-708, Formula No. C-709, Formula No. C-710, Formula No. C-711, Formula No. C-712, Formula No. C-713, Formula No. C-715, Formula No. C-717, Formula No. C-718, Formula No. C-720, Formula No. C-725, Formula No. C-726, Formula No. C-729, Formula No. C-730, Formula No. C-731, Formula No. C-732, Formula No. C-734, Formula No. C-735, Formula No. C-736, Formula No. C-737, Formula No. C-738, Formula No. C-740, Formula No. C-742, Formula No. C-743, Formula No. C-748, Formula No. C-750, Formula No. C-752, Formula No. C-774, Formula No. C-775, Formula No. C-776, Formula No. C-777, Formula No. C-780, Formula No. C-785, Formula No. C-787, Formula No. C-792, Formula No. C-800, Formula No. C-801, Formula No. C-814, Formula No. C-843, Formula No. C-872, Formula No. C-925, Formula No. C-930, Formula No. C-931, Formula No. C-934, Formula No. C-936, Formula No. C-942, Formula No. C-943, Formula No. C-945, Formula No. C-947, Formula No. C-949, Formula No. C-950, Formula No. C-951, Formula No. C-953, Formula No. C-962, Formula No. C-963, Formula No. C-964, Formula No. C-965, Formula No. C-966, Formula No. C-977, Formula No. C-1017, Formula No. C-1018, Formula No. C-1028, Formula No. C-1029, Formula No. C-1030, Formula No. C-1031, Formula No. C-1032, Formula No. C-1033, Formula No. C-1038, Formula No. C-1039, Formula No. C-1040, Formula No. C-1041, Formula No. C-1043, Formula No. C-1045, Formula No. C-1053, Formula No. C-1054, Formula No. C-1055, Formula No. C-1060, Formula No. C-1072, Formula No. C-1118, Formula No. C-1119, Formula No. C-1140, Formula No. C-1142, Formula No. C-1147, Formula No. C-1162, Formula No. D-13, Formula No. D-14, Formula No. D-19, Formula No. D-20, Formula No. D-21, Formula No. D-24, Formula No. D-25, Formula No. D-26, Formula No. D-29, Formula No. D-36, Formula No. D-37, Formula No. D-43, Formula No. D-45, Formula No. D-46, Formula No. D-47, Formula No. D-48, Formula No. D-51, Formula No. D-54, Formula No. D-63, Formula No. D-67, Formula No. D-72, Formula No. D-73, Formula No. D-76, Formula No. D-77, Formula No. D-79, Formula No. D-82, Formula No. D-83, Formula No. D-84, Formula No. D-85, Formula No. D-86, Formula No. D-87, Formula No. D-88, Formula No. D-90, Formula No. D-91, Formula No. D-94, Formula No. D-95, Formula No. D-96, Formula No. D-97, Formula No. D-98, Formula No. D-99, Formula No. D-100, Formula No. D-101, Formula No. D-116, Formula No. D-117, Formula No. D-118, Formula No. D-119, Formula No. D-123, Formula No. D-126, Formula No. D-127, Formula No. D-147, Formula No. D-154, Formula No. D-171, Formula No. D-172, Formula No. D-174, Formula No. D-177, Formula No. D-178, Formula No. D-205, Formula No. D-218, Formula No. D-236, Formula No. D-237, Formula No. D-238, Formula No. D-239, Formula No. D-240, Formula No. D-242, Formula No. D-243, Formula No. D-244, Formula No. D-245, Formula No. D-246, Formula No. D-248, Formula No. D-252, Formula No. D-254, Formula No. D-260, Formula No. D-264, Formula No. D-265, Formula No. D-268, Formula No. D-269, Formula No. D-271, Formula No. D-272, Formula No. D-274, Formula No. D-275, Formula No. D-302, Formula No. D-307, Formula No. D-308, Formula No. D-309, Formula No. D-315, Formula No. D-332, Formula No. D-334, Formula No. D-335, Formula No. D-336, Formula No. D-337, Formula No. D-338, Formula No. D-339, Formula No. D-340, Formula No. D-342, Formula No. D-343, Formula No. D-344, Formula No. D-348, Formula No. D-349, Formula No. D-353, Formula No. D-354, Formula No. D-355, Formula No. D-393, Formula No. D-397 and Formula No. E-6; further more preferably compounds of Formula No. A-32, Formula No. A-58, Formula No. B-87, Formula No. B-96, Formula No. B-106, Formula No. B-110, Formula No. B-113, Formula No. B-184, Formula No. B-188, Formula No. B-203, Formula No. B-232, Formula No. B-233, Formula No. B-236, Formula No. B-237, Formula No. B-240, Formula No. B-242, Formula No. B-243, Formula No. B-244, Formula No. B-245, Formula No. B-251, Formula No. B-255, Formula No. B-256, Formula No. B-257, Formula No. B-258, Formula No. B-259, Formula No. B-260, Formula No. B-261, Formula No. B-262, Formula No. B-263, Formula No. B-264, Formula No. B-265, Formula No. B-266, Formula No. B-267, Formula No. B-268, Formula No. B-269, Formula No. B-270, Formula No. B-271, Formula No. B-272, Formula No. B-274, Formula No. B-275, Formula No. B-276, Formula No. B-278, Formula No. B-279, Formula No. B-280, Formula No. B-281, Formula No. B-282, Formula No. B-283, Formula No. B-284, Formula No. B-285, Formula No. B-286, Formula No. B-287, Formula No. B-288, Formula No. B-289, Formula No. B-290, Formula No. B-291, Formula No. B-292, Formula No. B-293, Formula No. B-297, Formula No. B-298, Formula No. B-299, Formula No. B-300, Formula No. B-310, Formula No. B-368, Formula No. C-89, Formula No. C-105, Formula No. C-120, Formula No. C-123, Formula No. C-126, Formula No. C-131, Formula No. C-148, Formula No. C-149, Formula No. C-203, Formula No. C-204, Formula No. C-222, Formula No. C-223, Formula No. C-249, Formula No. C-253, Formula No. C-255, Formula No. C-265, Formula No. C-320, Formula No. C-432, Formula No. C-435, Formula No. C-441, Formula No. C-444, Formula No. C-479, Formula No. C-481, Formula No. C-482, Formula No. C-485, Formula No. C-488, Formula No. C-489, Formula No. C-491, Formula No. C-493, Formula No. C-494, Formula No. C-497, Formula No. C-499, Formula No. C-500, Formula No. C-505, Formula No. C-506, Formula No. C-507, Formula No. C-508, Formula No. C-509, Formula No. C-510, Formula No. C-511, Formula No. C-512, Formula No. C-513, Formula No. C-514, Formula No. C-515, Formula No. C-518, Formula No. C-519, Formula No. C-521, Formula No. C-522, Formula No. C-524, Formula No. C-525, Formula No. C-526, Formula No. C-527, Formula No. C-528, Formula No. C-529, Formula No. C-530, Formula No. C-531, Formula No. C-532, Formula No. C-533, Formula No. C-534, Formula No. C-535, Formula No. C-536, Formula No. C-537, Formula No. C-538, Formula No. C-539, Formula No. C-540, Formula No. C-541, Formula No. C-542, Formula No. C-543, Formula No. C-544, Formula No. C-545, Formula No. C-546, Formula No. C-548, Formula No. C-549, Formula No. C-550, Formula No. C-551, Formula No. C-561, Formula No. C-568, Formula No. C-569, Formula No. C-570, Formula No. C-578, Formula No. C-579, Formula No. C-586, Formula No. C-587, Formula No. C-588, Formula No. C-589, Formula No. C-591, Formula No. C-593, Formula No. C-594, Formula No. C-595, Formula No. C-596, Formula No. C-597, Formula No. C-598, Formula No. C-601, Formula No. C-609, Formula No. C-654, Formula No. C-667, Formula No. C-668, Formula No. C-674, Formula No. C-702, Formula No. C-711, Formula No. C-712, Formula No. C-740, Formula No. C-774, Formula No. C-775, Formula No. C-776, Formula No. C-777, Formula No. C-780, Formula No. C-785, Formula No. C-787, Formula No. C-792, Formula No. C-800, Formula No. C-801, Formula No. C-814, Formula No. C-843, Formula No. C-872, Formula No. C-925, Formula No. C-930, Formula No. C-931, Formula No. C-934, Formula No. C-936, Formula No. C-942, Formula No. C-943, Formula No. C-945, Formula No. C-947, Formula No. C-949, Formula No. C-950, Formula No. C-951, Formula No. C-953, Formula No. C-962, Formula No. C-963, Formula No. C-964, Formula No. C-965, Formula No. C-966, Formula No. C-977, Formula No. C-1017, Formula No. C-1018, Formula No. C-1028, Formula No. C-1029, Formula No. C-1030, Formula No. C-1031, Formula No. C-1032, Formula No. C-1033, Formula No. C-1038, Formula No. C-1039, Formula No. C-1040, Formula No. C-1041, Formula No. C-1043, Formula No. C-1045, Formula No. C-1053, Formula No. C-1054, Formula No. C-1055, Formula No. C-1060, Formula No. C-1072, Formula No. C-1118, Formula No. C-1119, Formula No. C-1140, Formula No. C-1142, Formula No. C-1147, Formula No. C-1162 and Formula No. D-48; particularly preferably compounds of Formula No. B-255, Formula No. B-256, Formula No. B-258, Formula No. B-261, Formula No. B-262, Formula No. B-267, Formula No. B-268, Formula No. B-269, Formula No. B-270, Formula No. B-271, Formula No. B-368, Formula No. C-148, Formula No. C-255, Formula No. C-505, Formula No. C-507, Formula No. C-508, Formula No. C-511, Formula No. C-512, Formula No. C-518, Formula No. C-519, Formula No. C-528, Formula No. C-529, Formula No. C-530, Formula No. C-531, Formula No. C-533, Formula No. C-534, Formula No. C-535, Formula No. C-536, Formula No. C-537, Formula No. C-539, Formula No. C-540, Formula No. C-541, Formula No. C-542, Formula No. C-543, Formula No. C-544, Formula No. C-548, Formula No. C-549, Formula No. C-550, Formula No. C-551, Formula No. C-740, Formula No. C-774, Formula No. C-775, Formula No. C-776, Formula No. C-777, Formula No. C-780, Formula No. C-785, Formula No. C-787, Formula No. C-792, Formula No. C-800, Formula No. C-801, Formula No. C-814, Formula No. C-843, Formula No. C-872, Formula No. C-925, Formula No. C-930, Formula No. C-931, Formula No. C-934, Formula No. C-936, Formula No. C-942, Formula No. C-943, Formula No. C-945, Formula No. C-947, Formula No. C-949, Formula No. C-950, Formula No. C-951, Formula No. C-953, Formula No. C-962, Formula No. C-963, Formula No. C-964, Formula No. C-965, Formula No. C-966, Formula No. C-977, Formula No. C-1017, Formula No. C-1018, Formula No. C-1028, Formula No. C-1029, Formula No. C-1030, Formula No. C-1031, Formula No. C-1032, Formula No. C-1033, Formula No. C-1038, Formula No. C-1039, Formula No. C-1040, Formula No. C-1041, Formula No. C-1043, Formula No. C-1045, Formula No. C-1053, Formula No. C-1054, Formula No. C-1055, Formula No. C-1060, Formula No. C-1072, Formula No. C-1118, Formula No. C-1119, Formula No. C-1140, Formula No. C-1142, Formula No. C-1147 and Formula No. C-1162.

The 4-acylaminopyrazole derivatives of the present invention can be prepared by the methods A to M described below.

Method A is a process for preparing a 4-(N-acyl-N-substituted amino)pyrazole derivative represented by the general formula (Ia) of the present invention by acylating a 4-aminopyrazole derivative represented by the general formula (II), and then performing a substitution reaction such as benzylation or the like.

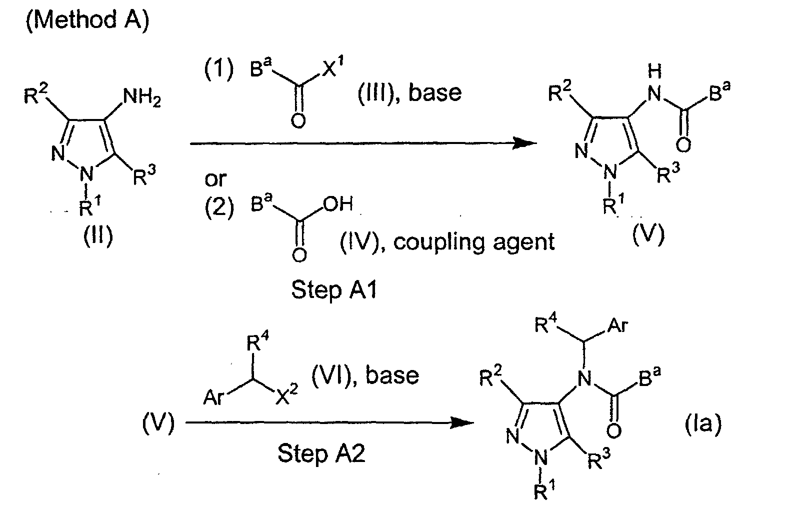

In the above formulae, R¹, R², R³, R⁴ and Ar have the same meanings as those described above; B^{a} has the same meaning as B described above, except for a primary amino group and a secondary amino group; X¹ represents a halogen atom (preferably a chlorine atom or a bromine atom), a group represented by the formula OC(=O)B^{a} (wherein B^{a} has the same meaning as described above) or a group represented by the formula OC(=O)R⁷ {wherein R⁷ represents a C₁-C₆ alkyl group, a C₁-C₆ alkoxy group, a phenyl group or an optionally substituted phenoxy group (the substituent(s) is (are) 1-5 same or different substituent(s) selected from the above substituent group a), preferably a methyl group, an ethyl group, a methoxy group, an ethoxy group, a propoxy group, a phenyl group or a phenoxy group};

X² represents a halogen atom (preferably a chlorine atom, a bromine atom or an iodine atom), a C₁-C₆ alkylsulfonyloxy group (preferably a methylsulfonyloxy group) or an optionally substituted phenylsulfonyloxy group (substituent(s) being C₁-C₆ alkyl group; preferably a phenylsulfonyloxy group or a p-tolylsulfonyloxy group).

### (Step A1)

Step A1 is a process for preparing an N-acylaminopyrazole derivative represented by the general formula (V) by (i) reacting compound (II) with an acid chloride, an acid anhydride or a mixed acid anhydride represented by the general formula (III) in an inert solvent in the presence of a base, or (ii) reacting compound (II) with a carboxylic acid represented by the general formula (IV) in the presence of a coupling agent. (i) When compound (III) is used in this step, the amount of compound (III) to be used is usually 1-10 equivalents, preferably 1.04-3 equivalents, relative to compound (II).

The base used in this step is not particularly limited, but examples thereof are hydroxides such as lithium hydroxide, sodium hydroxide, potassium hydroxide, calcium hydroxide and the like; carbonates such as lithium carbonate, sodium carbonate, potassium carbonate, magnesium carbonate, calcium carbonate, lithium bicarbonate, sodium bicarbonate, potassium bicarbonate and the like; or organic bases such as trimethylamine, triethylamine, triisopropylamine, diisopropylethylamine, N-methylmorpholine, pyridine, N,N-dimethylaminopyridine and the like; preferably carbonates or organic bases; more preferably potassium carbonate, potassium bicarbonate, triethylamine or pyridine.

The amount of base to be used in this step is usually 1-10 equivalents, preferably 1.04-3 equivalents, relative to compound (II).

The solvent used in this step is not particularly limited as long as it does not inhibit the reaction, and examples thereof are protonic solvents such as water, methanol, ethanol, butanol and the like; hydrocarbon solvents such as pentane, hexane, octane, petroleum ether, ligroin, benzene, toluene, xylene and the like; ethers such as diethyl ether, diisobutyl ether, tetrahydrofuran (THF), dioxane and the like; esters such as methyl acetate, ethyl acetate, methyl propionate and the like; halogenated solvents such as dichloromethane, chloroform, dichloroethane, tetrachloroethane and the like; amides such as N,N-dimethylformamide (DMF), N,N-dimethylacetamide (DMA), N,N-dimethyltetrahydroimidazolinone (DMI), hexamethylphosphoric triamide (HMPA) and the like; or mixed solvents thereof; preferably hydrocarbon solvents, ethers, esters or halogenated solvents; more preferably toluene, xylene, THF, ethyl acetate, dichloromethane or dichloroethane.

The reaction temperature is not particularly limited, but is usually -78°C to 150°C, preferably -20°C to 70°C. (ii) When compound (IV) is used in this step, the amount of compound (IV) to be used is usually 1-10 equivalents, preferably 1.04-3 equivalents relative to compound (II).

The coupling agent used in this step is not particularly limited as long as it is usually used as a coupling agent in a reaction to form an amide linkage by reaction with a carboxylic acid, and examples thereof are dicyclohexylcarbodiimide (DCC), 2-chloro-1-methylpyridinium iodide, polyphosphoric acid, polyphosphoric acid ethyl ester (PPE), polyphosphoric acid trimethylsilyl ester (PPSE), 1-p-toluenesulfonylimidazole, 1-p-toluenesulfonyltriazole or 1-p-mesitylsulfonylimidazole, preferably 2-chloro-1-methylpyridinium iodide.

The amount of coupling agent to be used is usually 1-10 equivalents, preferably 1.04-3 equivalents relative to compound (II).

In this step, when a base or an acid is also present in the reaction mixture, the reaction is promoted in some cases.

The base to be used in this step can be an organic base such as trimethylamine, triethylamine, triisopropylamine, diisopropylethylamine, N-methylmorpholine, imidazole, triazole, pyridine, N,N-dimethylaminopyridine and the like, preferably triethylamine or pyridine.

The amount of base to be used is usually 0.001-5 equivalents, preferably 0.01-2 equivalents relative to compound (II).

Examples of the acid to be used in this step are inorganic acids such as hydrogen chloride, hydrogen bromide and the like; organic acids such as p-toluenesulfonic acid, trifluoroacetic acid, trifluoromethanesulfonic acid and the like; preferably hydrogen chloride or p-toluenesulfonic acid; more preferably p-toluenesulfonic acid.

The amount of acid to be used is usually 0.001-5 equivalents, preferably 0.01-2 equivalents relative to compound (II).

The solvent used in this step is not particularly limited as long as it does not inhibit the reaction, and examples thereof are hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably ethers or halogenated solvents; more preferably THF, dichloromethane or dichloroethane.

The reaction temperature is not particularly limited, but is usually -78°C to 150°C, preferably -20°C to 70°C.

Compound (II) used in the present step can be prepared, for example, by method E described below.

### (Step A2)

Step A2 is a process for preparing compound (Ia) of the present invention by reacting compound (V) with compound (VI) in an inert solvent in the presence of a base.

The amount of compound (VI) used in this step is usually 1-10 equivalents, preferably 1.04-5 equivalents relative to compound (v).

The base used in this step is not particularly limited, but examples thereof are carbonates such as lithium carbonate, sodium carbonate, potassium carbonate, magnesium carbonate, calcium carbonate, lithium bicarbonate, sodium bicarbonate, potassium bicarbonate and the like; hydrides such as lithium hydride, sodium hydride, potassium hydride and the like; or organometallic compounds such as methylmagnesium bromide, ethylmagnesium bromide, butyllithium and the like; preferably carbonates or hydrides; more preferably potassium carbonate or sodium hydride.

The amount of base to be used is usually 1-10 equivalents, preferably 1.04-5 equivalents relative to compound (V).

In this step, when a metal iodide or metal bromide is also present in the reaction mixture, the reaction is promoted in some cases.

The metal iodide or metal bromide used in this step can be, for example, lithium iodide, sodium iodide, potassium iodide, magnesium iodide, zinc iodide, lithium bromide, sodium bromide, potassium bromide, magnesium bromide or zinc bromide, preferably lithium iodide or sodium iodide.

The amount of metal iodide or metal bromide to be used is usually 0.001-5 equivalents, preferably 0.01-2 equivalents relative to compound (V).

The solvent used in this step is not particularly limited as long as it does not inhibit the reaction, but can be, for example, hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably ethers or amides; more preferably THF, DMF or DMA.

The reaction temperature is not particularly limited, but is usually -78°C to 150°C, preferably -20°C to 70°C.

Method B is a process for preparing a 4-(N-acyl-N-substituted amino)pyrazole derivative represented by the general formula (Ib) or (Ic) of the present invention by first performing a substitution reaction such as benzylation or the like on a 4-aminopyrazole derivative represented by the general formula (II), and then acylating the product.

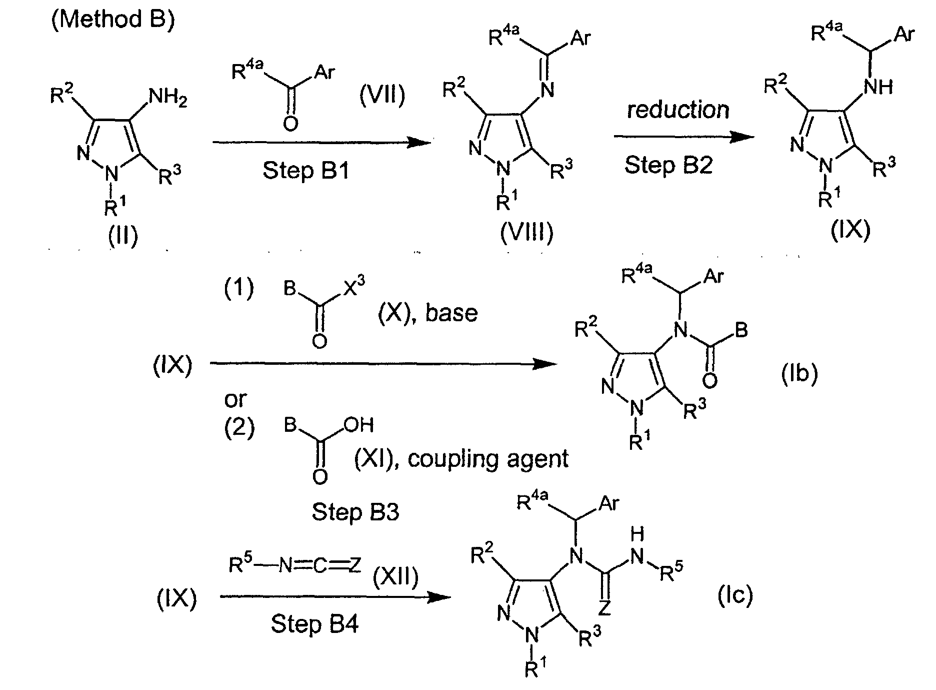

In the above formulae, R¹, R², R³, Ar, B and Z have the same meanings as those described above;
R^{4a} has the same meaning as R⁴ described above, except for a cyano group;
X³ represents a halogen atom (preferably a chlorine atom or bromine atom), a group represented by the formula OC(=O)B (wherein B has the same meaning as described above) or a group represented by the formula OC(=O)R⁷ (wherein R⁷ has the same meaning as described above),
R⁵ represents a substituent selected from the above substituent group d or a substituted phenyl group (the substituent(s) is (are) 1-3 same or different substitutent(s) selected from the above substituent group b).

### (Step B1)

Step B1 is a process for preparing imines represented by the general formula (VIII) by dehydration-condensing compound (II) with an aldehyde or ketone represented by the general formula (VII) in an inert solvent.

The amount of compound (VII) used in this step is usually 1-10 equivalents, preferably 1.04-3 equivalents relative to compound (II).

In this step, when an acid is also present in the reaction mixture, the reaction is promoted in some cases.

Examples of the acid used in this step are inorganic acids such as hydrogen chloride, hydrogen bromide and the like; organic acids such as p-toluenesulfonic acid, acetic acid, trifluoroacetic acid, trifluoromethanesulfonic acid and the like; preferably organic acids; more preferably p-toluenesulfonic acid or acetic acid; further more preferably p-toluenesulfonic acid.

The amount of acid to be used is usually 0.001-5 equivalents, more preferably 0.01-2 equivalents relative to compound (II).

The solvent used in this step is not particularly limited as long as it does not inhibit the reaction, but examples thereof are protonic solvents such as water, methanol, ethanol, butanol and the like; hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably protonic solvents or hydrocarbon solvents; more preferably methanol, toluene or xylene.

The reaction temperature is not particularly limited, but is usually -78°C to 300°C, preferably 0°C to 150°C.

### (Step B2)

Step B2 is a process for preparing an amine represented by the general formula (IX) by reducing compound (VIII) with a reducing agent in an inert solvent.

The reducing agent used in this step is not particularly limited as long as it can reduce normal imines, but examples thereof are hydrides such as borane, sodium tetrahydroborate, sodium trihydrocyanoborate, alane, diisobutylaluminum hydride, lithium aluminum hydride and the like, or hydrogen, preferably sodium tetrahydroborate or sodium trihydrocyanoborate. When hydrogen is used in this step, transition metal catalysts such as palladium-carbon, platinum-carbon, Raney nickel and the like are usually used (preferably palladium-carbon).

The amount of reducing agent to be used is not particularly limited, but when the reducing agent is a hydride, the amount is usually 1-10 equivalents, preferably 1.04-5 equivalents relative to compound (VIII), and, when the reducing agent is hydrogen, the amount is usually 1-100 atm, preferably 1-20 atm. When the reducing agent is hydrogen and a transition metal catalyst is used, the amount of transition metal catalyst is usually 0.00001-1 equivalents, preferably 0.001-0.5 equivalents relative to compound (VIII).

The solvent used in this step is not particularly limited as long as it does not inhibit the reaction, but examples thereof are protonic solvents such as water, methanol, ethanol, butanol and the like; hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably protonic solvents or ethers; more preferably methanol or THF.

The reaction temperature is not particularly limited, but is usually -78°C to 150°C, preferably -20°C to 100°C.

### (Step B3)

Step B3 is a process for preparing compound (Ib) of the present invention by reacting compound (IX) with a compound represented by the general formula (X) in an inert solvent in the presence of a base, or reacting compound (IX) with a carboxylic acid represented by the general formula (XI) in the presence of a coupling agent.

This step can be performed in the same manner as step A1.

### (Step B4)

Step B4 is a process for preparing compound (Ic) of the present invention by reacting compound (IX) with an isocyanate or isothiocyanate represented by the general formula (XII) in an inert solvent.

The amount of compound (XII) used in this step is usually 1-10 equivalents, preferably 1.04-3 equivalents relative to compound (IX).

In this step, when a base or an acid is also present in the reaction mixture, the reaction is promoted in some cases.

The base used in this step can be, for example, an organic base such as trimethylamine, triethylamine, triisopropylamine, diisopropylethylamine, N-methylmorpholine, imidazole, triazole, pyridine, N,N-dimethylaminopyridine and the like, preferably triethylamine, pyridine or N,N-dimethylaminopyridine.

The amount of base to be used is usually 0.001-5 equivalents, preferably 0.01-2 equivalents relative to compound (IX).

The acid used in this step can be, for example, an inorganic acid such as hydrogen chloride, hydrogen bromide and the like; or an organic acid such as p-toluenesulfonic acid, trifluoroacetic acid, trifluoromethanesulfonic acid and the like; preferably hydrogen chloride or p-toluenesulfonic acid; more preferably p-toluenesulfonic acid.

The amount of acid to be used is usually 0.001-5 equivalents, preferably 0.01-2 equivalents relative to compound (IX).

The solvent used in this step is not particularly limited as long as it does not inhibit the reaction, but examples thereof are hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably ethers, esters or halogenated solvents; more preferably THF, ethyl acetate, dichloromethane or dichloroethane.

The reaction temperature is not particularly limited, but is usually -78°C to 150°C, preferably -20°C to 70°C.

Method C is a process for preparing a 4-(N-acyl-N-substituted amino)pyrazole derivative represented by the general formula (Id) or (Ie) by first performing substitution reaction such as benzylation or the like on a 4-aminopyrazole derivative represented by the general formula (II), and then acylating the product.

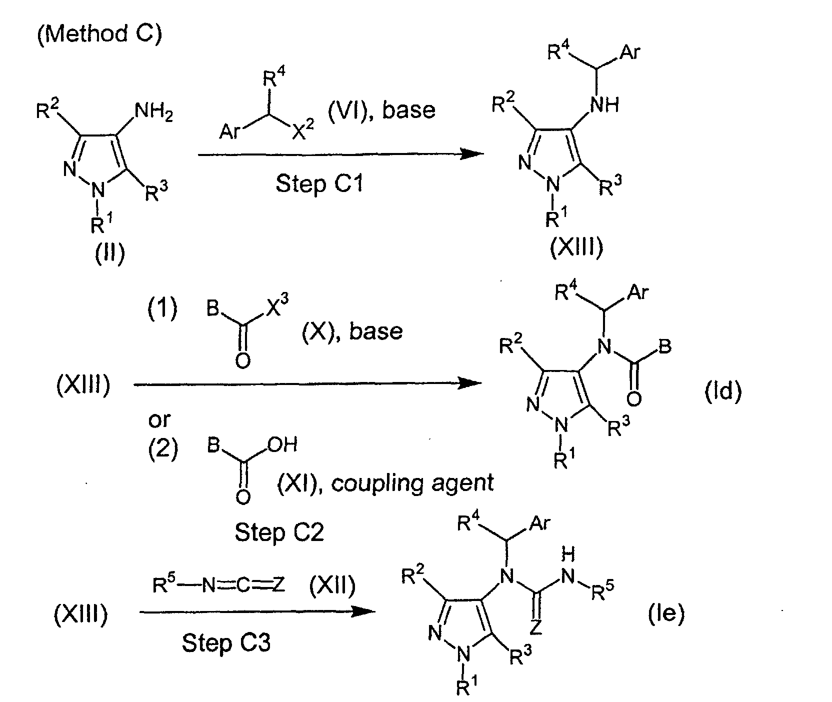

In the above formulae, R¹, R², R³, R⁴, Ar, B, Z, R⁵, X² and X³ have the same meanings as those described above.

### (Step C1).

Step C1 is a process for preparing a 4-(N-substituted amino)pyrazole derivative represented by the general formula (XIII) by reacting compound (II) with compound (VI) in an inert solvent.

The amount of compound (VI) used in the present step is usually 1-5 equivalents, preferably 1.04-2 equivalents relative to compound (V).

In this step, when a base is also present in the reaction mixture, the reaction is promoted in some cases.

The base used in this step is not particularly limited, but can be, for example, a carbonate such as lithium carbonate, sodium carbonate, potassium carbonate, magnesium carbonate, calcium carbonate, lithium bicarbonate, sodium bicarbonate, potassium bicarbonate and the like; a hydride such as lithium hydride, sodium hydride, potassium hydride and the like; an organometallic compound such as methylmagnesium bromide, ethylmagnesium bromide, butyllithium and the like; preferably a carbonate; more preferably sodium bicarbonate or potassium bicarbonate.

The amount of base to be used is usually 1-5 equivalents, preferably 1.04-2 equivalents relative to compound (V).

In this step, when a metal iodide or metal bromide is also present in the reaction mixture, the reaction is promoted in some cases.

The metal iodide or metal bromide used in the present step can be, for example, lithium iodide, sodium iodide, potassium iodide, magnesium iodide, zinc iodide, lithium bromide, sodium bromide, potassium bromide, magnesium bromide or zinc bromide, preferably lithium iodide or sodium iodide.

The amount of metal iodide or metal bromide to be used is usually 0.001-5 equivalents, preferably 0.01-2 equivalents relative to compound (V).

The solvent used in this step is not particularly limited as long as it does not inhibit the reaction, but examples thereof are hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably hydrocarbon solvents or ethers; more preferably toluene, xylene or THF.

The reaction temperature is not particularly limited, but is usually -78°C to 150°C, preferably -20°C to 70°C.

### (Step C2)

Step C2 is a process for preparing compound (Id) of the present invention by reacting compound (XIII) with compound (X) in an inert solvent in the presence of a base, or reacting compound (XIII) with compound (XI) in the presence of a coupling agent.

This step can be performed in the same manner as step A1.

### (Step C3)

Step C3 is a process for preparing compound (Ie) of the present invention by reacting compound (XIII) with compound (XII) in an inert solvent.

This step can be performed in the same manner as step B4.

Method D is a process for preparing a compound represented by the general formula (If) of the present invention by reductive acylation of the hydroxyimino group of a hydroxyimino-1,3-diketone derivative represented by the general formula (XIV), and, after cyclization, performing a substitution reaction such as acylation, benzylation or the like.

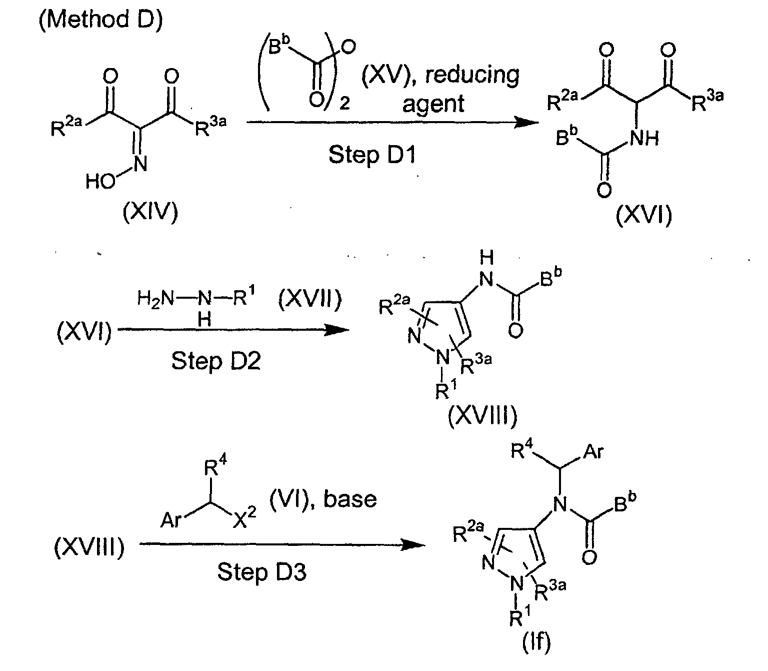

In the above formulae, R¹, R⁴, Ar and X² have the same meanings as those described above;
R^{2a} and R^{2b} independently represent a C₁-C₆ alkyl group or a phenyl group;
B^{b} has the same meaning as B described above except for a C₁-C₆ alkylthio group, a C₇-C₉ aralkylthio group, a C₃-C₆ cycloalkylthio group, a C₂-C₆ alkenylthio group, an optionally substituted phenylthio group (substituent(s) being selected from the above substituent group b) or an optionally substituted amino group (substituent(s) being selected from the above substituent group d).

### (Step D1)

Step D1 is a process for preparing an amido-1,3-diketone derivative represented by the general formula (XVI) by reacting compound (XIV) with a compound represented by the general formula (XV) in an inert solvent in the presence of a reducing agent.

The amount of compound (XV) used in this step is usually 1-20 equivalents, preferably 2-10 equivalents relative to compound (XIV).

The reducing agent used in this step is usually hydrogen and, when reduction is performed using hydrogen, a transition metal catalyst is used.

The transition metal catalyst to be used is not particularly limited as long as it has the ability to catalyze hydrogenation, and can be, for example, palladium, palladium-carbon, platinum, platinum-carbon, platinum oxide, nickel, Raney nickel, rhodium or Wilkinson's complex, preferably palladium-carbon.

The amount of hydrogen to be used is usually 1-30 atm, preferably 1-10 atm. The amount of transition metal catalyst to be used is usually 0.000001-1 equivalents, preferably 0.0001-0.2 equivalents relative to compound (XIV).

The solvent used in this step is not particularly limited as long as it dissolves compound (XIV) and compound (XV) at least to some extent and it does not inhibit the reaction, but examples thereof are hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably ethers or esters; more preferably THF or ethyl acetate.

The reaction temperature is not particularly limited, but is usually -78°C to 150°C, preferably -20°C to 70°C.

Compound (XIV) used in this step can be prepared by the methods of, for example, Liebigs Ann. Chem., 325, 139(1902), J. Chem. Soc. A, 1971, 233 or J. Heterocyclic Chem., 17, 1723(1980).

### (Step D2)

Step D2 is a process for preparing a pyrazole represented by the general formula (XVIII) by reacting compound (XVI) with a hydrazine represented by the general formula (XVII) in an inert solvent.

The amount of compound (XVII) to be used in this step is usually 1-5 equivalents, preferably 1.04-2 equivalents relative to compound (XVI).

The solvent used in this step is not particularly limited as long as it dissolves compound (XVI) at least to some extent but examples thereof are protonic solvents such as water, methanol, ethanol, butanol and the like; hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably protonic solvents; more preferably methanol or ethanol.

The reaction temperature is not particularly limited, but is usually -78°C to 150°C, preferably -20°C to 70°C.

### (Step D3)

Step D3 is a process for preparing compound (If) of the present invention by reacting compound (XVIII) with compound (VI) in an inert solvent in the presence of a base.

This step can be performed in the same manner as step A2.

Method E is a process for preparing compound (II) which is an intermediate for compound (I) of the present invention and for preparing a compound represented by the general formula (Ig) of the present invention, by azidation of a pyrazolyl-4-carboxylic acid derivative represented by the general formula (VIII) and then converting the product into an isocyanate compound.

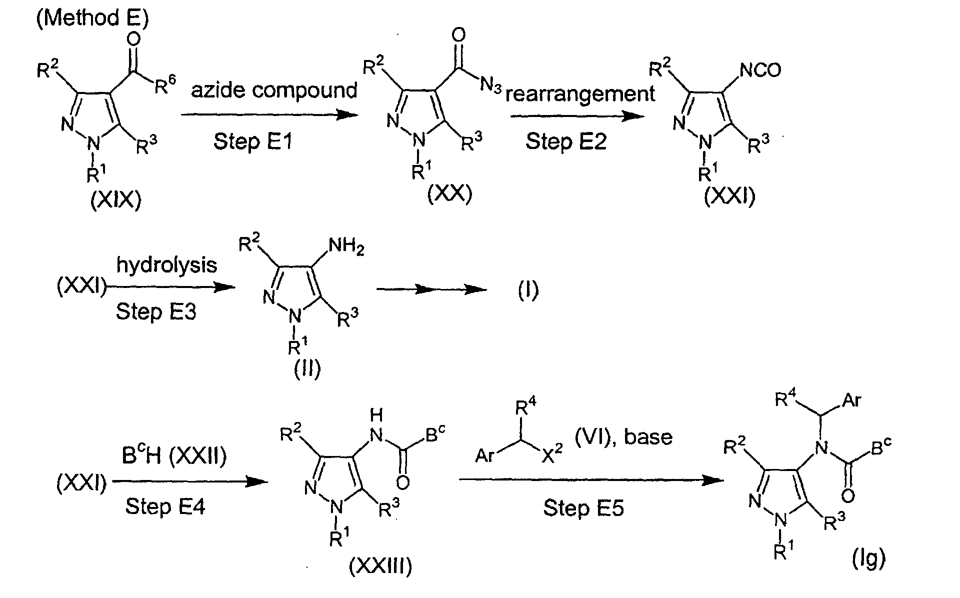

In the above formulae, R¹, R², R³, R⁴, Ar and X² have the same meanings as those described above; R⁶ represents a hydroxyl group, a halogen atom (preferably a chlorine atom), a group represented by the formula: 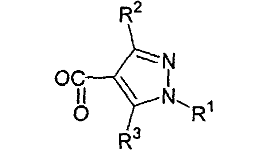 (wherein R¹, R² and R³ have the same meanings as those described above), a C₂-C₇ alkylcarbonyloxy group, a C₂-C₇ alkoxycarbonyloxy group, a benzoyloxy group or a phenoxycarbonyloxy group; B^{c} represents a C₁-C₆ alkoxy group, a C₇-C₉ aralkyloxy group, a C₃-C₆ cycloalkoxy group, a C₂-C₆ alkenyloxy group, an optionally substituted phenoxy group (substituent(s) being selected from the above substituent group b), an optionally substituted 5-6 membered unsaturated (heterocyclyl)oxy group (substituent(s) being selected from the above substituent group b; the heterocycle contains an oxygen atom, a sulfur atom or a nitrogen atom, may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a C₁-C₆ alkylthio group, a C₇-C₉ aralkylthio group, a C₃-C₆ cycloalkylthio group, a C₂-C₆ alkenylthio group, an optionally substituted phenylthio group (substituent(s) being selected from the above substituent group b) or an optionally substituted amino group (substituent(s) being selected from the above substituent group d) (preferably a methoxy group, an ethoxy group, a phenoxy group, a dimethylamino group, diethylamino group, a diisopropylamino group, an N-methylanilino group, an N-ethylanilino group or an N-phenylanilino group).

### (Step E1)

Step E1 is a process for preparing an acylazide derivative represented by the general formula (XX) by reacting compound (XIX) with an azide compound in an inert solvent.

The azide compound used in this step is not particularly limited as long as it can react with a carboxylic acid or a derivative thereof, but can be, for example, hydrogen azide, lithium azide, sodium azide, potassium azide or diphenylphosphoryl azide (DPPA), preferably sodium azide or DPPA.

The amount of azide compound to be used is usually 1-10 equivalents, preferably 1-3 equivalents relative to compound (XIX).

In this step, it is necessary in some cases, for example, when R⁶ is a hydroxyl group and the azide compound is DPPA, that a base is also present in the reaction mixture.

The base used in this step can be, for example, a hydroxide such as lithium hydroxide, sodium hydroxide, potassium hydroxide, calcium hydroxide and the like; a carbonate such as lithium carbonate, sodium carbonate, potassium carbonate, magnesium carbonate, calcium carbonate, lithium bicarbonate, sodium bicarbonate, potassium bicarbonate and the like; or an organic base such as trimethylamine, triethylamine, triisopropylamine, diisopropylethylamine, N-methylmorpholine, pyridine, N,N-dimethylaminopyridine and the like; preferably an organic base; more preferably triethylamine.

The amount of base to be used is usually 1-10 equivalents, preferably 1.04-5 equivalents relative to compound (XIX).

The solvent used in this step is not particularly limited as long as it dissolves compound (XIX) and the azide compound at least to some extent, but examples thereof are protonic solvents such as water, methanol, ethanol, butanol, t-butyl alcohol and the like; hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably protonic solvents, ethers or halogenated solvents; more preferably methanol, ethanol, dichloromethane or dichloroethane.

The reaction temperature is not particularly limited, but is usually -78°C to 200°C, preferably -20°C to 120°C.

Compound (XIX) used in the present step can be prepared, for example, by the methods of J. Heterocyclic Chem., 24, 1669(1987), Aust. J. Chem., 36, 135(1983), Agric, Biol. Chem., 45, 2769(1981), J. Heterocyclic Chem., 28, 1545(1991), J. Heterocyclic Chem., 24, 267(1987), J. Heterocyclic Chem., 25, 555(1988) or J. Heterocyclic Chem., 30, 865(1993).

### (Step E2)

Step E2 is a process for preparing a pyrazolyl-4-isocyanate derivative represented by the general formula (XXI) by subjecting compound (XX) to a rearrangement reaction in an inert solvent.

The solvent used in this *step* is not particularly limited as long as it does not inhibit the reaction, but examples thereof are protonic solvents such as water, methanol, ethanol, butanol, t-butyl alcohol and the like; hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably protonic solvents, ethers or halogenated solvents; more preferably water, methanol, ethanol, t-butyl alcohol, THF, dichloromethane or dichloroethane.

The reaction temperature is not particularly limited, but is usually -20°C to 200°C, preferably 20°C to 150°C.

### (Step E3)

Step E3 is a process for preparing compound (II) by hydrolyzing compound (XXI) in water in the presence of an acid.

The acid used in this step is usually a protonic acid, and can be, for example, an inorganic acid such as hydrochloric acid, hydrobromic acid, hydroiodic acid, sulfuric acid, nitric acid and the like; or an organic acid such as acetic acid, methanesulfonic acid, p-toluenesulfonic acid, camphorsulfonic acid and the like; preferably an inorganic acid; more preferably hydrochloric acid.

The amount of acid to be used is usually an excess amount relative to compound (XXI).

The reaction temperature is not particularly limited, but is usually -20°C to 200°C, preferably 20°C to 150°C.

Compound (II) obtained in this step can be converted into compound (I) of the present invention, for example, by methods A to C.

### (Step E4)

Step E4 is a process for preparing a 4-(N-acylamino)pyrazole derivative represented by the general formula (XXIII) by reacting a compound (XXI) with an alcohol or a secondary amine represented by the general formula (XXII) in an inert solvent.

This step can be performed in the same manner as step B4, provided that, when compound (XXII) is an alcohol, the compound (XXII) can be used as the solvent.

### (Step E5)

Step E5 is a process for preparing compound (Ig) of the present invention by reacting compound (XXIII) with compound (VI) in an inert solvent in the presence of a base.

This step can be performed in the same manner as step A2.

Method F is a process for preparing a 1-substituted-4-(N-acyl-N-substituted amino)pyrazole derivative represented by the general formula (Ih) of the present invention by diacylating a 4-amino-1H-pyrazole derivative represented by the general formula (XXIV), performing substitution reaction such as benzylation or the like, hydrolyzing the 1-acyl group, and introducing a substituent at the 1-position of the resulting 4-(N-acyl-N-substituted amino)-1H-pyrazole.

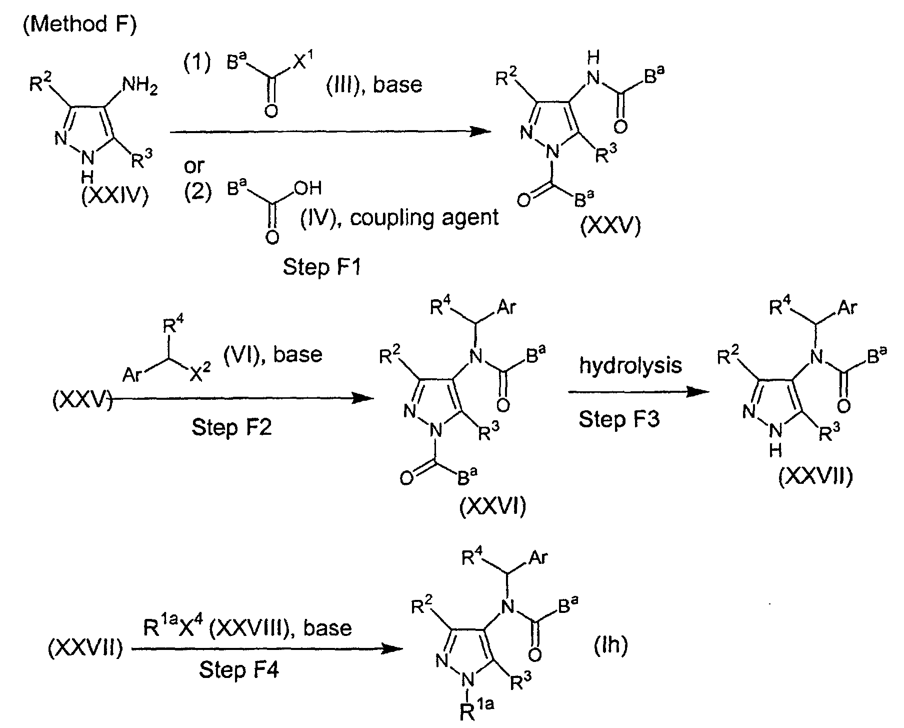

In the above formulae, R², R³, R⁴, Ar, B^{a}, X¹ and X² have the same meanings as those described above; R^{1a} has the same meaning as R¹ described above except for a hydrogen atom, an optionally substituted C₆-C₁₄ aryl group (substituent(s) being selected from the above substituent group g), a 4-6 membered saturated heterocyclic group (the heterocycle contains 1-2 same or different heteroatom(s) selected from the group consisting of an oxygen atom, a sulfur atom and a nitrogen atom), an optionally substituted 5-6 membered unsaturated heterocyclic group (substituent(s) being selected from the above substituent group i; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a C₂-C₇ alkylcarbonyl group, an optionally substituted benzoyl group (substituent(s) being selected from the above substituent group b), a C₂-C₇ alkoxycarbonyl group, a C₈-C₁₀ aralkyloxycarbonyl group, a C₃-C₇ alkenyloxycarbonyl group, an optionally substituted phenylsulfonyl group (substituent(s) being C₁-C₆ alkyl group) or a di(C₁-C₆ alkyl)sulfamoyl group; when R^{1a} is a phenyl group substituted with an electron withdrawing group at the 2-position or 4-position, or an optionally substituted 2-pyridyl group, 2-pyrimidinyl group, 3-pyridazinyl group, 2-thiazolyl group or 2-benzothiazolyl group (substituent(s) being selected from the above substituent group i), X⁴ represents a halogen atom, a C₁-C₆ alkylsulfonyl group, an optionally substituted phenylsulfonyl group (substituent(s) being C₁-C₆ alkyl group), a C₁-C₆ alkylsulfonyloxy group, or an optionally substituted phenylsulfonyloxy group (substituent(s) being C₁-C₆ alkyl group) (preferably a chlorine atom, a bromine atom, a methylsulfonyl group, a phenylsulfonyl group, a methylsulfonyloxy group, a phenylsulfonyloxy group or a p-tolylsulfonyloxy group) and, when R^{1a} is any other group, X⁴ represents a halogen atom, a C₁-C₆ alkylsulfonyloxy group, or an optionally substituted phenylsulfonyloxy group (substituent(s) being C₁-C₆ alkyl group)(preferably a chlorine atom, a bromine atom, a methylsulfonyloxy group, a phenylsulfonyloxy group or a p-tolylsulfonyloxy group).

### (Step F1)

Step F1 is a process for preparing a 1-acyl-4-(N-acylamino)pyrazole derivative represented by the general formula (XXV) by reacting a 4-amino-1H-pyrazole derivative represented by the general formula (XXIV) with an acid chloride, an acid anhydride or a mixed acid anhydride represented by the general formula (III) in an inert solvent in the presence of a base, or by reacting the derivative with a carboxylic acid represented by the general formula (IV) in the presence of a coupling agent.

This step can be performed in the same manner as step A1 except that the amounts of compound (III) and of the base, or the amounts of compound (IV) and of the coupling agent are twice the respective amounts for the step A1.

Compound (XXIV) used in the present step can be prepared, for example, by the methods of Tetrahedron Lett., 25, 8585(1997) or J. Org. Chem., 38, 2958(1973).

### (Step F2)

Step F2 is a process for preparing a 1-acyl-4-(N-acyl-N-substituted amino)pyrazole derivative represented by the general formula (XXVI) by reacting compound (XXV) with compound (VI) in an inert solvent in the presence of a base.

This step can be performed in the same manner as step A2.

### (Step F3)

Step F3 is a process for preparing a 4-(N-acyl-N-substituted amino)-1H-pyrazole derivative represented by the general formula (XXVII) with compound (XXVI) in an inert solvent in the presence of an acid or a base.

The acid used in this step can be, for example, an inorganic acid such as hydrochloric acid, hydrobromic acid, hydroiodic acid, sulfuric acid, nitric acid, silicic acid, silica gel and the like; or an organic acid such as acetic acid, trichloroacetic acid, trifluoroacetic acid, methanesulfonic acid, p-toluenesulfonic acid, camphorsulfonic acid and the like; preferably hydrochloric acid or trifluoroacetic acid; more preferably hydrochloric acid.

The amount of acid to be used is usually 0.1-100 equivalents, preferably 1-50 equivalents relative to compound (XXVI).

The base used in this step can be, for example, a hydroxide such as lithium hydroxide, sodium hydroxide, potassium hydroxide and the like; a carbonate such as lithium carbonate, sodium carbonate, potassium carbonate, lithium bicarbonate, sodium bicarbonate, potassium bicarbonate and the like; or an amine such as ammonia, methylamine, dimethylamine, ethylamine and the like; preferably an amine; more preferably ammonia.

The amount of base to be used is usually 0.1-100 equivalents, preferably 1-50 equivalents relative to compound (XXVI).

The solvent used in this step is not particularly limited as long as it dissolves compound (XXVI) and water at least to some extent, but can be, for example, an alcohol such as methanol, ethanol, propanol and the like; an ether such as THF, dioxane and the like; or an amide such as DMF and the like; preferably an alcohol or an ether; more preferably methanol, ethanol, THF or dioxane.

The reaction temperature is not particularly limited, but is usually -78°C to 150°C, preferably -20°C to 70°C.

### (Step F4)

Step F4 is a process for preparing compound (Ih) of the present invention by reacting compound (XXVII) with a compound represented by the general formula (XXVIII) in an inert solvent in the presence of a base.
(i) When R^{1a} is a C₂-C₇ alkylcarbonyl group, an optionally substituted benzoyl group (substituent(s) being selected from the above substituent group b), a C₂-C₇ alkoxycarbonyl group, a C₈-C₁₀ aralkyloxycarbonyl group, or a C₃-C₇ alkenyloxycarbonyl group, this step can be performed in the same manner as step A1.
(ii) In this step, when R^{1a} is a phenyl group substituted with an electron withdrawing group at the 2-position or 4-position, or an optionally substituted 2-pyridyl group, 2-pyrimidinyl group, 3-pyridazinyl group, 2-thiazolyl group or 2-benzothiazolyl group (substituent(s) being selected from the above substituent group i), then, when a fluoride is also present in the reaction mixture, the reaction is promoted in some cases.
   The fluoride used in this step can be, for example, lithium fluoride, sodium fluoride, potassium fluoride, cesium fluoride, magnesium fluoride or tetrabutylammonium fluoride, preferably potassium fluoride or a cesium fluoride.
   The amount of fluoride to be used is usually 0.001-20 equivalents, preferably 0.01-10 equivalents relative to compound (XXVII).
   The base used in this step can be, for example, a carbonate such as lithium carbonate, sodium carbonate, potassium carbonate, magnesium carbonate, calcium carbonate, lithium bicarbonate, sodium bicarbonate, potassium bicarbonate and the like; a hydride such as lithium hydride, sodium hydride, potassium hydride and the like; or an organometallic compound such as methylmagnesium bromide, ethylmagnesium bromide, butyllithium and the like; preferably a hydride; more preferably sodium hydride.
   The amount of base to be used is usually 1-10 equivalents, preferably 1.04-5 equivalents relative to compound (XXVII).
   The solvent used in this step is not particularly limited as long as it does not inhibit the reaction, but examples thereof are hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably ethers or amides; more preferably THF, DMF or DMA.
   The reaction temperature is not particularly limited, but is usually -78°C to 150°C, preferably -20°C to 70°C.
(iii) In this step, when R^{1a} is another substituent, then, when a metal iodide or metal bromide is also present in the reaction mixture, the reaction is promoted in some cases.

The metal iodide or metal bromide used in this step can be, for example, lithium iodide, sodium iodide, potassium iodide, magnesium iodide, zinc iodide, lithium bromide, sodium bromide, potassium bromide, magnesium bromide or zinc bromide, preferably lithium iodide or sodium iodide.

The amount of metal iodide or metal bromide to be used is usually 0.001-5 equivalents, preferably 0.01-2 equivalents relative to compound (XXVII).

The base used in this step can be, for example, a carbonate such as lithium carbonate, sodium carbonate, potassium carbonate, magnesium carbonate, calcium carbonate, lithium bicarbonate, sodium bicarbonate, potassium bicarbonate and the like; a hydride such as lithium hydride, sodium hydride, potassium hydride and the like; or an organometallic compound such as methylmagnesium bromide, ethylmagnesium bromide, butyllithium and the like; preferably a hydride; more preferably sodium hydride.

The amount of base to be used is usually 1-10 equivalents, preferably 1.04-5 equivalents relative to compound (XXVII).

The solvent used in the present step is not particularly limited as long as it does not inhibit the reaction, but examples thereof are hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably ethers or amides; more preferably THF, DMF or DMA.

The reaction temperature is not particularly limited, but is usually -78°C to 150°C, preferably- -20°C to 70°C.

Method G is a process for preparing a compound represented by the general formula (Ii) or (Ij) of the present invention by acylating the 1-position of a 4-nitroso-1H-pyrazole derivative represented by the general formula (XXIX), reducing the 4-position, performing a substitution reaction such as acylation, benzylation or the like, and then hydrolyzing the acyl group at the 1-position and introducing a substituent.

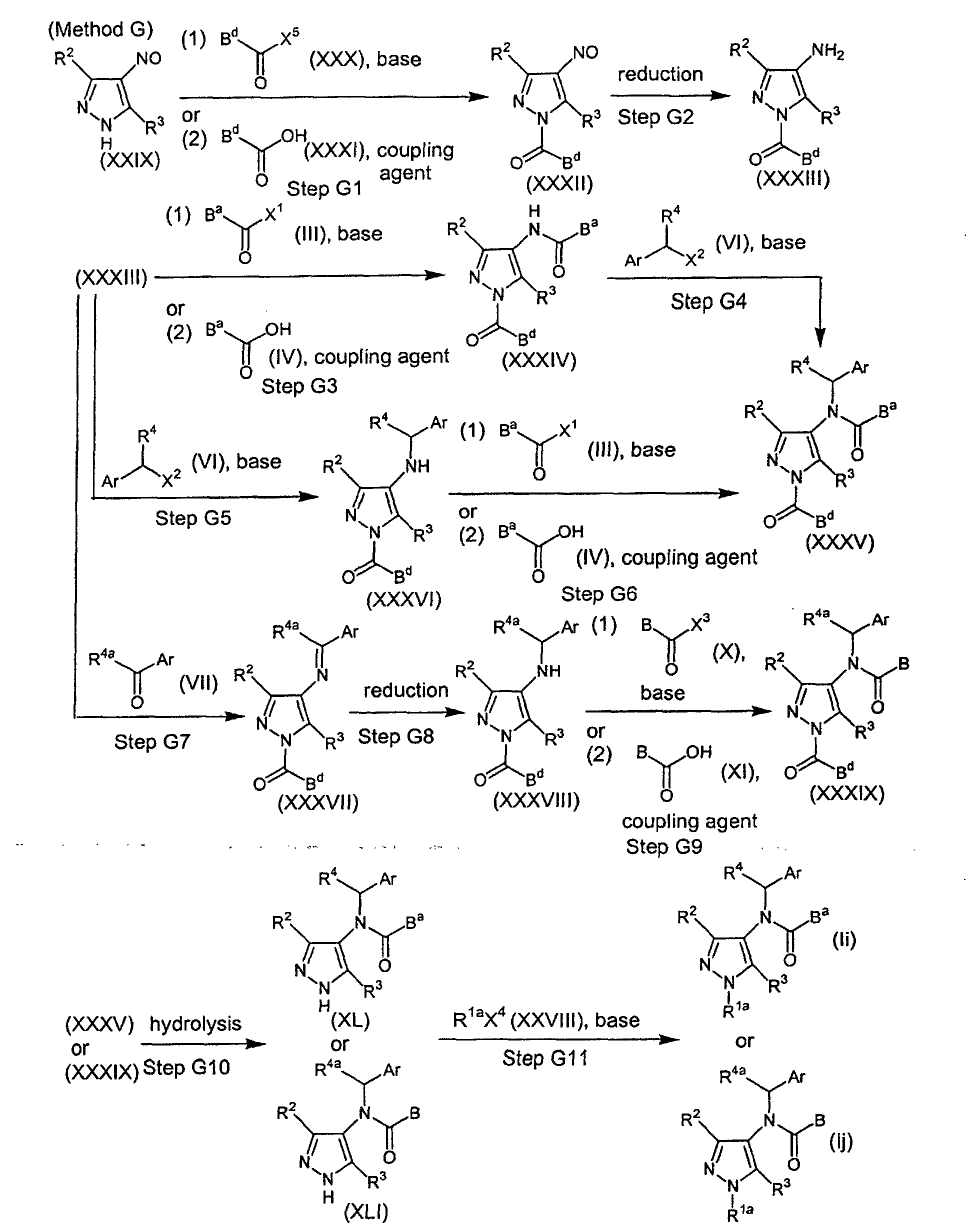

In the above formulae, R^{1a}, R², R³, R⁴, R^{4a}, Ar, B, B^{a}, Z, X¹, X², X³ and X⁴ have the same meanings as those described above; B^{d} has the same meaning as B described above except for a primary amino group and a secondary amino group;
X⁵ represents a halogen atom (preferably a chlorine atom or a bromine atom), a group represented by the formula OC(=O)B^{a} (wherein B^{a} has the same meaning as described above) or a group represented by the formula OC(=O)R⁷ {wherein R⁷ represents a C₁-C₆ alkyl group, a C₁-C₆ alkoxy group, a phenyl group or an optionally substituted phenoxy group (the substituent(s) is (are) 1-5 same or different substituent(s) selected from the above substituent group a), preferably a methyl group, an ethyl group, a methoxy group, an ethoxy group, a propoxy group, a phenyl group or a phenoxy group}.

### (Step G1)

Step G1 is a process for preparing a 1-acylaminopyrazole derivative represented by the general formula (XXXII) by reacting compound (XXIX) with an acid chloride, an acid anhydride or a mixed acid anhydride represented by the general formula (XXX) in an inert solvent in the presence of a base, or reacting compound (XXIX) with a carboxylic acid represented by the general formula (XXXI) in the presence of a coupling agent.

This step can be performed in the same manner as step A1.

Compound (XXIX) used in the present step can be prepared, for example, by the method of Liebigs Ann. Chem., 325, 193(1902).

### (Step G2)

Step G2 is a process for preparing a 4-aminopyrazole derivative represented by the general formula (XXXIII) by reducing compound (XXXII) in an inert solvent.

The reduction method used in this step is not particularly a limited as long as it is a method capable of reducing a nitroso group, but is usually hydrogenation using a transition metal catalyst.

The amount of hydrogen to be used is usually 1-30 atm, preferably 1-10 atm.

The transition metal catalyst used in this step is not particularly limited as long as it has the ability to catalyze hydrogenation, but can be, for example, palladium, palladium-carbon, platinum, platinum-carbon, platinum oxide, nickel, Raney nickel, rhodium or Wilkinson's complex, preferably palladium-carbon.

The amount of transition metal catalyst to be used is usually 0.000001-1 equivalents, preferably 0.0001-0.2 equivalents relative to compound (XXXII).

The solvent used in this step is not particularly limited as long as it dissolves compound (XXXII) at least to some extent, but examples thereof are protonic solvents such as water, methanol, ethanol, isopropyl alcohol, butanol, t-butyl alcohol and the like; hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably protonic solvents or esters; more preferably methanol, ethanol or ethyl acetate.

The reaction temperature is not particularly limited, but is usually -78°C to 200°C, preferably -20°C to 120°C.

### (Step G3)

Step G3 is a process for preparing a 1-acyl-4-(N-acylamino)pyrazole derivative represented by the general formula (XXXIV) by reacting compound (XXXIII) with compound (III) in an inert solvent in the presence of a base, or reacting compound (XXXIII) with compound (IV) in the presence of a coupling agent.

This step can be performed in the same manner as step A1.

### (Step G4)

Step G4 is a process for preparing a compound represented by the general formula (XXXV) by reacting compound (XXXIV) with compound (VI) in an inert solvent in the presence of a base.

This step can be performed in the same manner as step A2.

### (Step G5)

Step G5 is a process for preparing a compound represented by the general formula (XXXVI) by reacting compound (XXXIII) with compound (VI) in an inert solvent in the presence of a base.

This step can be performed in the same manner as step A2.

### (Step G6)

Step G6 is a process for preparing compound (XXXV) by reacting compound (XXXVI) with compound (III) in an inert solvent in the presence of a base, or reacting compound (XXXVI) with compound (IV) in the presence of a coupling agent.

This step can be performed in the same manner as step A1.

### (Step G7)

Step G7 is a process for preparing an imine represented by the general formula (XXXVII) by dehydration-condensing compound (XXXIII) with compound (VII) in an inert solvent.

This step can be performed in the same manner as step B1.

### (Step G8)

Step G8 is a process for preparing an amine represented by the general formula (XXXVIII) by reducing compound (XXXVII) with a reducing agent in an inert solvent.

This step can be performed in the same manner as step B2.

### (Step G9)

Step G9 is a process for preparing a compound represented by the general formula (XXXIX) by reacting compound (XXXVIII) with a compound represented by the general formula (X) in an inert solvent in the presence of a base, or reacting a compound (XXXVIII) with a carboxylic acid represented by the general formula (XI) in the presence of a coupling agent.

This step can be performed in the same manner as step A1.

### (Step G10)

Step G10 is a process for preparing a 4-(N-acyl-N-substituted amino)-1H-pyrazole derivative represented by the general formula (XL) or the general formula (XLI) by reacting compound (XXXV) or compound (XXXIX) with an excess amount of water in an inert solvent in the presence of an acid or a base, respectively.

This step can be performed in the same manner as step F3.

### (Step G11)

Step G11 is a process for preparing compound (Ii) of the present invention or compound (Ij) of the present invention by reacting compound (XL) or compound (XLI) with a compound represented by the general formula (XXVIII) in an inert solvent in the presence of a base, respectively.

This step can be performed in the same manner as step F4.

Method H is a process for preparing a sulfur-containing compound represented by the general formula (Il) by converting the carbonyl group of a compound represented by the general formula (Ik) into a thiocarbonyl group.

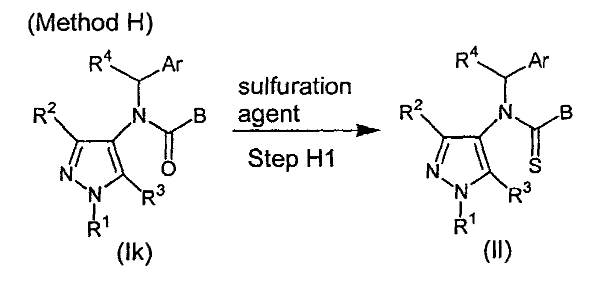

In the above formulae, R¹, R², R³, R⁴, Ar and B have the same meanings as those described above.

### (H1 step)

Step H1 is a process for preparing compound (Il) of the present invention by reacting compound (Ik) with a sulfuration agent in an inert solvent.

The sulfuration agent used in this step can be, for example, phosphorus pentasulfide or a 2,4-bisaryl-1,3-dithia-2,4-diphosphetane-2,4-disulfide, preferably phosphorus pentasulfide or 2,4-bis(4-methoxyphenyl)-1,3-dithia-2,4-diphosphetane-2,4-disulfide (Lawesson's Reagent).

The amount of sulfuration agent to be used is usually 1-10 equivalents, preferably 1-5 equivalents relative to compound (Ik).

In this step, when a base is also present in the reaction mixture, the reaction is promoted in some cases.

The base used in this step can be, for example, an organic base such as triethylamine, pyridine, N,N-dimethylaminopyridine and the like, preferably pyridine.

The amount of base to be used is usually 0.001-20 equivalents, preferably 0.01-10 equivalents relative to compound (Ik).

The solvent used in this step is not particularly limited as long as it does not inhibit the reaction, and examples thereof are hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably hydrocarbon solvents or ethers; more preferably toluene, xylene or THF.

The reaction temperature is not particularly limited, but is usually -78°C to 300°C, preferably -20°C to 200°C.

Method I is a process for preparing a compound represented by the general formula (In) of the present invention by converting a specified functional group in Ar in a compound represented by the general formula (Im) into another functional group.

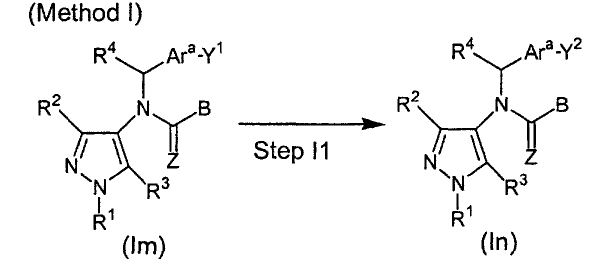

In the above formulae, R¹, R², R³, R⁴, B and Z have the same meanings as those described above (provided that, when substituents to be converted by the present step are included, substituents after conversion are included);
Ar^{a}-Y¹ represents the above Ar which include Y¹;
Ar^{a}-Y² represents the above Ar which include Y²;
Y¹ represents a halogen atom, a halomethyl group (halogen substituent(s) being chlorine atom or bromine atom), a hydroxyl group, a carboxyl group, a C₂-C₇ alkoxycarbonyl group, a cyano group, a nitro group, an amino group, an amido group, a C₂-C₆ alkylcarbonyl group, a carbamoyl group, a thiocarbamoyl group, a C₂-C₇ alkylcarbonyloxy group, a benzoyloxy group, a C₂-C₇ alkoxycarbonyloxy group, a phenoxycarbonyloxy group, a carbamate group or a sulfinyl group;
Y² represents a halogen atom, a (C₁-C₆ alkoxy)methyl group, a (C₆-C₁₀ aryloxy)methyl group, a cyanomethyl group, a sulfinylmethyl group, an aminomethyl group, a hydroxyl group, a carboxyl group, a C₂-C₇ alkoxycarbonyl group, a C₂-C₇ alkoxy(thiocarbonyl) group, a cyano group, an amino group, an amido group, a thioamido group, a carbamoyl group, a thiocarbamoyl group, a C₁-C₆ alkoxy group, a C₂-C₇ alkylcarbonyloxy group, a C₂-C₇ alkyl(thiocarbonyl)oxy group, a C₁-C₆ alkylsulfonyloxy group, a C₆-C₁₀ arylsulfonyloxy group, a carbamate group, a thiocarbamate group, a urea group, a mercapto group, a thio group, a sulfinyl group, an amidine group, an imino group, an oxyimino group, an O-(C₁-C₆ alkyl)oxyimino group, an N-(C₁-C₆ alkyl)-S-(C₁-C₆ alkyl)thioxoimino group, a tri(C₁-C₆ alkyl)phosphoryl group or a tri(C₆-C₁₀ aryl)phosphoryl group.

### (Step I1)

Step Il is a process for preparing compound (In) of the present invention by converting a functional group of compound (Im).

For the conversion of a functional group in this step, methods normally known as functional group conversion reactions can be used and, for example, can be from a halogen atom to a cyano group, from a halogen atom to an amino group, from a halogen atom to an alkoxy group, from a halogen atom to a mercapto group, from a halogen atom to a sulfinyl group, from a hydroxyl group to an alkoxy group, from a hydroxyl group to an alkylcarbonyloxy group, from a hydroxyl group to an alkylsulfonyloxy group, from a hydroxyl group to an arylsulfonyloxy group, from a hydroxyl group to a trialkylphosphoryl group, from a hydroxyl group to a triarylphosphoryl group, from an amino group to an amido group, from an amino group to a carbamate group, from an amino group to an urea group, from an amino group to a halogen atom via a diazonium salt, from an amino group to a cyano group via a diazonium salt, from an amino group to a hydroxyl group via a diazonium salt, from an amino group to a mercapto group via a diazonium salt, from an amino group to a sulfinyl group via a diazonium salt, from a nitro group to an amino group, from a carboxyl group to an alkoxycarbonyl group, from a carboxyl group to a carbamoyl group, from an alkoxycarbonyl group to a carboxyl group, from an alkoxycarbonyl group to an alkoxy(thiocarbonyl) group, from a carbamoyl group to a thiocarbamoyl group, from an alkylcarbonyloxy group to an alkyl(thiocarbonyl)oxy group, from an amido group to a thioamido group, from a carbamate group to a thiocarbamate group, from a cyano group to a carboxyl group, from a cyano group to an alkoxycarbonyl group, from a cyano group to a carbamoyl group, from a cyano group to an amidine group, from a cyano group to an aminomethyl group, from a chloromethyl group or a bromomethyl group to a cyanomethyl group, from a chloromethyl group or a bromomethyl group to an alkoxymethyl group, from a chloromethyl group or a bromomethyl group to an aryloxymethyl group, from a chloromethyl group or a bromomethyl group to a sulfinylmethyl group, from a sulfinyl group to a sulfenyl group or a sulfonyl group, from an acyl group to a secondary alcohol, from an acyl group to an imino group, from an acyl group to an oxyimino group, from an acyl group to an O-alkyloxyimino group, or from a thiocarbamoyl group to an N-alkyl-s-alkylthioxoimino group.

This step can be performed, for example, according to New Experimental Chemistry Course, vol. 14, Synthesis and Reaction of Organic Compounds, edited by Japan Chemistry Association (1978), Maruzen, Tokyo.

Method J is a process for preparing a compound represented by the general formula (Ip) of the present invention by converting a specified functional group in R¹ of a compound represented by the general formula (Io) into another functional group.

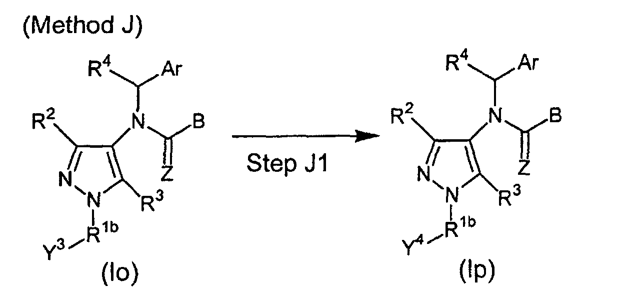

In the above formulae, R², R³, R⁴, Ar, B and Z have the same meanings as those described above (provided that, when substituents to be converted in the present step are included, substituents after conversion are included);
R^{1b}-Y³ represents the above R¹ which include Y³;
R^{1b}-Y⁴ represents the above R¹ which include Y⁴;
Y³ represents a halogen atom, a halomethyl group (halogen substituent(s) being chlorine atom or bromine atom), a hydroxyl group, a carboxyl group, a C₂-C₇ alkoxycarbonyl group, a cyano group, a nitro group, an amino group, an amido group, a C₂-C₆ alkylcarbonyl group, a carbamoyl group, a thiocarbamoyl group, a C₂-C₇ alkylcarbonyloxy group, a benzoyloxy group, a C₂-C₇ alkoxycarbonyloxy group, a phenoxycarbonyloxy group, a carbamate group or a sulfinyl group;
Y⁴ represents a halogen atom, a (C₁-C₆ alkoxy)methyl group, a (C₆-C₁₀ aryloxy)methyl group, a cyanomethyl group, a sulfinylmethyl group, an aminomethyl group, a hydroxyl group,. a carboxyl group, a C₂-C₇ alkoxycarbonyl group, a C₂-C₇ alkoxy(thiocarbonyl) group, a cyano group, an amino group, an amido group, a thioamido group, a carbamoyl group, a thiocarbamoyl group, a C₁-C₆ alkoxy group, a C₂-C₇ alkylcarbonyloxy group, a C₂-C₇ alkyl(thiocarbonyl)oxy group, a C₁-C₆ alkylsulfonyloxy group, a C₆-C₁₀ arylsulfonyloxy group, a carbamate group, a thiocarbamate group, a urea group, a mercapto group, a thio group, a sulfinyl group, an amidine group, an imino group, an oxyimino group, an O-(C₁-C₆ alkyl)oxyimino group, an N- (C₁-C₆ alkyl)-S-(C₁-C₆ alkyl)thioxoimino group, a tri(C₁-C₆ alkyl)phosphoryl group or a tri(C₆-C₁₀ aryl)phosphoryl group.

### (Step J1)

Step J1 is a process for preparing compound (Ip) of the present invention by converting a functional group of compound (Io).

This step can be performed in the same manner as step I1.

Method K is a process for preparing a 4-(N-substituted amino)pyrazole derivative represented by the general formula (XIII) which is an intermediate for compound (I) of the present invention by deacylating a compound represented by the general formula (Iq).

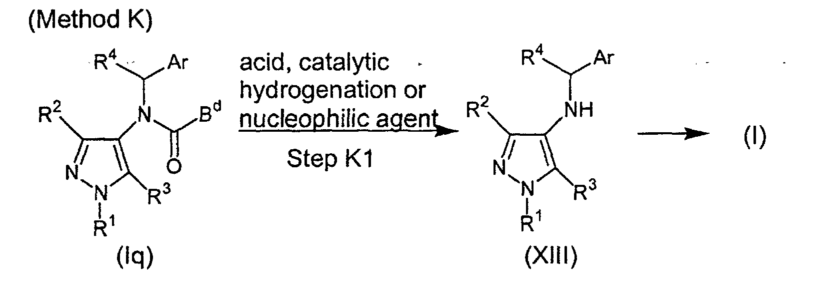

In the above formulae, R¹, R², R³, R⁴ and Ar have the same meanings as those described above;
B^{d} represents a t-butoxy group, a benzyloxy group or an optionally substituted C₆-C₁₀ allyloxy group (substituent(s) being C₁-C₃ alkyl group or phenyl group).

### (Step K1)

This step is a process for preparing compound (XIII) by (i) when B^{d} is a t-butoxy group, reacting compound (Iq) with an acid in an inert solvent, (ii) when B^{d} is a benzyloxy group, hydrogenating compound (Iq) in an inert solvent, (iii) when B^{d} is an optionally substituted phenoxy group (the substituent(s) is (are) 1-3 same or different substituent(s) selected from the above substituent group a), reacting compound (Iq) with a nucleophilic agent in an inert solvent in the presence of a transition metal catalyst.
(i) When B^{d} is a t-butoxy group, the acid used in this step is not particularly limited as long as it is an acid usually employed for deprotecting a t-butoxycarbonyl group, and examples thereof are protonic acids such as hydrochloric acid, hydrobromic acid, hydroiodic acid, sulfuric acid, nitric acid, acetic acid, trichloroacetic acid, trifluoroacetic acid, silicic acid, silica gel, methanesulfonic acid, p-toluenesulfonic acid, camphorsulfonic acid and the like; or Lewis acids such as boron trifluoride, boron trichloride, boron tribromide, boron trifluoride diethyl ether complex, aluminum chloride, titanium tetrachloride, trimethylsilyl trifluoromethanesulfonate, zinc chloride and the like; preferably protonic acids; more preferably hydrochloric acid, sulfuric acid or trifluoroacetic acid.
   The solvent used in this step is not particularly limited as long as it does not inhibit the reaction, and can be, for example, a protonic solvent such as water, methanol, ethanol, isopropyl alcohol, butanol, t-butyl alcohol and the like; a hydrocarbon solvent such as pentane, hexane, octane, petroleum ether, ligroin, benzene, toluene, xylene and the like; an ether such as diethyl ether, diisobutyl ether, THF, dioxane and the like; an ester such as methyl acetate, ethyl acetate, methyl propionate and the like; a halogenated solvent such as dichloromethane, chloroform, dichloroethane, tetrachloroethane and the like; an amide such as DMF, DMA, DMI, HMPA and the like; or mixed solvents thereof; preferably a protonic solvent or a halogenated solvent; more preferably methanol, dichloromethane and dichloroethane.
   The reaction temperature is not particularly limited, but is usually -78°C-200°C, preferably -20°C-120°C.
(ii) When B^{d} is a benzyloxy group, the transition metal catalyst used in the hydrogenation in this step is not particularly limited as long as it has the ability to catalyze hydrogenation, and can be, for example, palladium, palladium-carbon, platinum, platinum-carbon, platinum oxide, nickel, Raney nickel, rhodium or Wilkinson's complex, preferably palladium-carbon.
   The amount of transition metal catalyst used is usually 0.000001-1 equivalents, preferably 0.0001-0.2 equivalents relative to compound (Iq).
   The amount of hydrogen to be used in this step is usually 1-30atm, preferably 1-10atm.
   The solvent used in this step is not particularly limited as long as it dissolves compound (Iq) at least to some extent, and examples thereof are protonic solvents such as water, methanol, ethanol, isopropyl alcohol, butanol, t-butyl alcohol and the like; hydrocarbon solvents such as pentane, hexane, octane, petroleum ether, ligroin, benzene, toluene, xylene and the like; ethers such as diethyl ether, diisobutyl ether, THF, dioxane and the like; esters such as methyl acetate, ethyl acetate, methyl propionate and the like; halogenated solvents such as dichloromethane, chloroform, dichloroethane, tetrachloroethane and the like; amides such as DMF, DMA, DMI, HMPA and the like; or mixed solvents thereof; preferably protonic solvents or esters; more preferably methanol, ethanol or ethyl acetate.
   The reaction temperature is not particularly limited, but is usually -78°C to 200°C, preferably -20°C to 120°C.
(iii) When B^{d} is an optionally substituted C₆-C₁₀ allyloxy group (substituent(s) being C₁-C₃ alkyl group or phenyl group), the nucleophilic agent used in this step can be, for example, a silane such as phenylsilane, diphenylsilane, triphenylsilane and the like; an amine such as ammonia, methylamine, dimethylamine, ethylamine, diethylamine, propylamine, butylamine and the like; a malonic acid ester such as dimethyl malonate, diethyl malonate and the like; or malononitrile; preferably a silane, an amine or a malonic acid ester; more preferably phenylsilane, butylamine or dimethyl malonate.

The amount of nucleophilic agent to be used is usually 1-30 equivalents, preferably 1.04-10 equivalents relative to compound (Iq).

The transition metal catalyst used in this step is not particularly limited as long as it can form a π-allyl metal complex, and can be, for example, a palladium complex, preferably tetrakistriphenylphosphinepalladium.

The amount of transition metal catalyst to be used is usually 0.000001-1 equivalents, preferably 0.0001-0.2 equivalents relative to compound (Iq).

The solvent used in this step is not particularly limited as long as it dissolves compound (Iq) at least to some extent, but examples thereof are alcohols such as methanol, ethanol, isopropyl alcohol and the like; hydrocarbon solvents such as pentane, hexane, octane, benzene, toluene, xylene and the like; ethers such as diethyl ether, diisobutyl ether, THF and the like; esters such as methyl acetate, ethyl acetate, methyl propionate and the like; halogenated solvents such as dichloromethane, chloroform, dichloroethane and the like; amides such as DMF, DMA, DMI, and the like; or mixed solvents thereof; preferably ethers; more preferably THF.

The reaction temperature is not particularly limited, but is usually -78C° to 200°C, preferably -20°C to 70°C. (XIII) obtained in this step can be converted into compound (I) of the present invention, for example, by method C.

Method L is a process for preparing a compound represented by the general formula (Is) of the present invention by converting a specified functional group in B of a compound represented by the general formula (Ir) into another functional group.

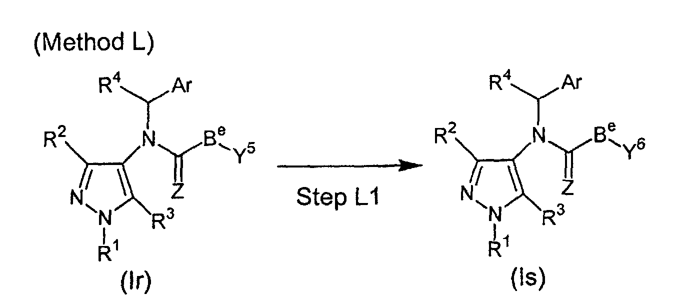

In the above formulae, R¹, R², R³, R⁴, Ar and Z have the same meanings as those described above (provided that, when substituents to be converted in the present step are included, substituents after conversion are included);
B^{e}-Y⁵ represents the above B which include Y⁵;
B^{e}-Y⁶ represents the above B which include Y⁶;
Y⁵ represents a halogen atom, a halomethyl group (halogen substituent(s) being chlorine atom or bromine atom), a hydroxyl group, a carboxyl group, a C₂-C₇ alkoxycarbonyl group, a cyano group, a nitro group, an amino group, an amido group, a C₂-C₆ alkylcarbonyl group, a carbamoyl group, a thiocarbamoyl group, a C₂-C₇ alkylcarbonyloxy group, a benzoyloxy group, a C₂-C₇ alkoxycarbonyloxy group, a phenoxycarbonyloxy group, a carbamate group or a sulfinyl-group;
Y⁶ represents a halogen atom, a (C₁-C₆ alkoxy)methyl group, a (C₆-C₁₀ aryloxy)methyl group, a cyanomethyl group, a sulfinylmethyl group, an aminomethyl group, a hydroxyl group, a carboxyl group, a C₂-C₇ alkoxycarbonyl group, a C₂-C₇ alkoxy(thiocarbonyl) group, a cyano group, an amino group, an amido group, a thioamido group, a carbamoyl group, a thiocarbamoyl group, a C₁-C₆ alkoxy group, a C₂-C₇ alkylcarbonyloxy group, a C₂-C₇ alkyl(thiocarbonyl)oxy group, a C₁-C₆ alkylsulfonyloxy group, a C₆-C₁₀ arylsulfonyloxy group, a carbamate group, a thiocarbamate group, a urea group, a mercapto group, a thio group, a sulfinyl group, an amidine group, an imino group, an oxyimino group, an O-(C₁-C₆ alkyl)oxyimino group, an N-(C₁-C₆ alkyl)-S-(C₁-C₆ alkyl)thioxoimino group, a tri(C₁-C₆ alkyl)phosphoryl group or a tri(C₆-C₁₀ aryl)phosphoryl group.

### (Step L1)

Step L1 is a process for preparing compound (Is) of the present invention by converting a functional group of compound (Ir).

This step can be performed in the same manner as step I1.

Method M is a process for preparing a compound represented by the general formula (Iu) of the present invention by halogenocarbamoylation of the amino group of a compound represented by the general formula (XIII), and then performing a nucleophilic reaction.

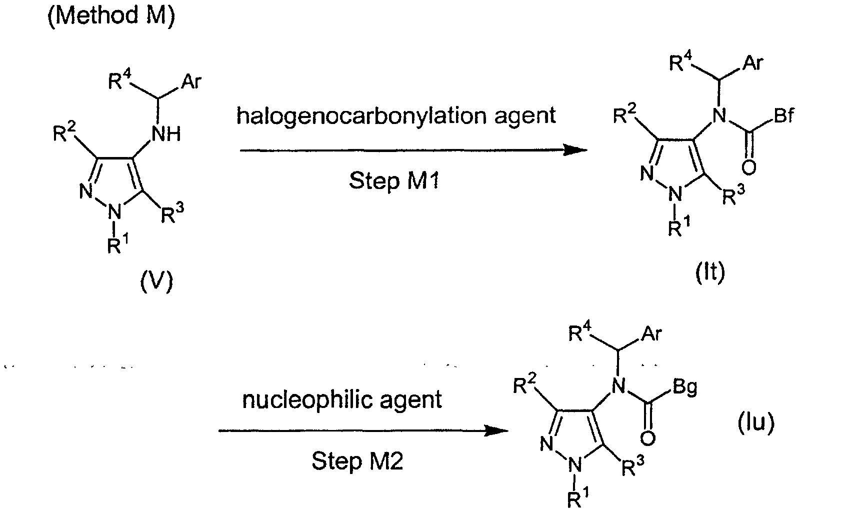

In the above formulae, R¹, R², R³, R⁴, Ar and Z have the same meanings as those described above;
Bf represents a chlorine atom or a bromine atom;
Bg represents a C₁-C₆ alkoxy group, a C₇-C₉ aralkyloxy group, a C₃-C₆ cycloalkoxy group, a C₂-C₆ alkenyloxy group, an optionally substituted phenoxy group (substituent(s) being selected from the above substituent group b), an optionally substituted 5-6 membered unsaturated (heterocyclyl)oxy group (substituent(s) being selected from the above substituent group b; the heterocycle contains an oxygen atom, a sulfur atom or a nitrogen atom, and may contain 1-2 further nitrogen atoms, two oxygen atoms may bind to the sulfur atom, the heterocycle may be fused with a benzene ring or a pyridine ring, and the heterocycle may be substituted with 1-2 oxo groups), a C₁-C₆ alkylthio group, a C₇-C₉ aralkylthio group, a C₃-C₆ cycloalkylthio group, a C₂-C₆ alkenylthio group, an optionally substituted phenylthio group (substituent(s) being selected from the above substituent group b) or an optionally substituted amino group (substituent(s) being selected from the above substituent group d).

### (Step M1)

Step M1 is a process for preparing compound (It) of the present invention by reacting an amino group of compound (XIII) with a halogenocarbonylation agent in an inert solvent.

The halogenocarbonylation agent used in this step can be, for example, carbonyl chloride (phosgene), carbonyl bromide (bromophosgene), trichloromethyl chlorocarbonate (diphosgene) or bis(trichloromethyl) carbonate (triphosgene).

When the halogenocarbonylation agent used in the present step is phosgene or carbonyl bromide, the amount of halogenocarbonylation agent to be used is usually 1-10 equivalents, preferably 1.04-3 equivalents relative to compound (XIII) and, when the halogenocarbonylation agent used in the present step is trichloromethyl chlorocarnobate (diphosgene), the amount of halogenocarbonylation agent to be used is usually 0.5-5 equivalents, preferably 0.502-2 equivalents relative to compound (XIII) and, when the halogenocarbonylation agent used in the present step is bis(trichloromethyl) carbonate (triphosgene), the amount of halogenocarbonylation agent to be used is usually 1/3-3 equivalents, preferably 0.34-1 equivalents relative to a compound (XIII).

In this step, when a base is used, the reaction is promoted in some cases.

The base used in this step is not particularly limited as long as it can neutralize hydrogen chloride or hydrogen bromide, and can be, for example, a carbonate such as lithium carbonate, sodium carbonate, potassium carbonate, magnesium carbonate, calcium carbonate, lithium bicarbonate, sodium bicarbonate, potassium bicarbonate and the like; or an organic base such as trimethylamine, triethylamine, triisopropylamine, diisopropylethylamine, N-methylmorpholine, pyridine, N,N-dimethylaminopyridine and the like; preferably sodium bicarbonate, triethylamine or pyridine; more preferably triethylamine.

The amount of base to be used is usually 0.001-1.5 equivalents, preferably 0.95-1.04 equivalents relative to compound (XIII).

The solvent used in this step is not particularly limited as long as it does not inhibit the reaction, and can be, for example, a hydrocarbon solvent such as pentane, hexane, octane, petroleum ether, ligroin, benzene, toluene, xylene and the like; an ether such as diethyl ether, diisobutyl ether; tetrahydrofuran (THF), dioxane and the like; an ester such as methyl acetate, ethyl acetate, methyl propionate and the like; a halogenated solvent such as dichloromethane, chloroform, dichloroethane, tetrachloroethane and the like; preferably a hydrocarbon solvent, an ether or a halogenated solvent; more preferably toluene, diethyl ether, dichloromethane or chloroform.

The reaction temperature is not particularly limited, but is usually -150°C to 100°C, preferably -78°C to room temperature, more preferably -20°C to 10°C.

### (Step M2)

Step M2 is a process for preparing compound (Iu) of the present invention by reacting halogenocarbamoyl (It) of the present invention obtained in step M1 with a nucleophilic agent.

As the nucleophilic agent used in this step, a compound represented by the formula Bg-H (wherein Bg has the same meaning as described above) is used.

This step can be performed in the same manner as step A1.

After completion of each reaction described above, the product of each reaction can be isolated from the reaction mixture by conventional methods. For example, the product can be obtained by neutralizing the reaction mixture (or, when insoluble material is present, after the insoluble material is removed by filtration), adding water and an immiscible organic solvent such as ethyl acetate, and, after washing with water, separating the organic layer containing the product, drying with anhydrous magnesium sulfate or the like, and distilling off the solvent.

If necessary, the resulting product can be further purified by conventional methods, for example, recrystallization, reprecipitation, chromatography or the like. Of course, purification may be stopped at an arbitrary stage of purification to obtain an active ingredient as a crude product, or a raw material compound which is to be fed through to the next reaction.

A step of preparing a salt of compound (I) of the present invention is performed by adding an acid to the extraction concentrate of the reaction mixture containing the desired compound (I) prepared in each step, or to a solution in which compound (I) is dissolved in a suitable solvent.

Examples of the acid to be used in the reaction include hydrohalic acids such as hydrofluoric acid, hydrochloric acid, hydrobromic acid, hydroiodic acid and the like; inorganic acids such as nitric acid, perchloric acid, sulfuric acid, phosphoric acid and the like; lower alkylsulfonic acids such as methanesulfonic acid, trifluoromethanesulfonic acid, ethanesulfonic acid and the like; arylsulfonic acids such as benzenesulfonic acid, p-toluenesulfonic acid and the like; organic acids such as succinic acid, oxalic acid and the like; and organic acid amide compounds such as saccharin. The acid is usually used at an amount of 1 equivalent-10 equivalents, preferably 1 equivalent-5 equivalents.

The solvent used in the reaction is not particularly limited as long as it does not inhibit the reaction, and examples thereof preferably include ethers such as ether, diisopropyl ether, tetrahydrofuran (THF), dioxane and the like; and alcohols such as methanol, ethanol and the like.

The reaction temperature is usually -20°C to 50°C, preferably -10°C to 30°C.

The reaction time varies depending on the temperature and the kind of solvent used, and is usually 10 minutes to 1 hour.

The resulting salt is isolated by conventional methods. That is, when the salt is precipitated as crystals, the salt is isolated by filtration and, in the case of a water-soluble salt, the salt is isolated as an aqueous solution by partition between an organic solvent and water.

The compounds of the present invention are used as an active ingredient in an agent for the control of harmful organisms. For example, as an agricultural or horticultural fungicide, the present compounds exhibit excellent controlling effects against diseases caused by various plant pathogens. In particular, the present compounds exhibit excellent controlling effects against various diseases due to Oomycetes such as downy mildew of cucumber and grape, phytophthora disease of tomato and potato, Pythium, Aphanomyces and the like. The compounds of the present invention have long residual effects, and not only exhibit excellent preventive effects, but also have excellent curative effects, and can control diseases by treatment after infection. In addition, the compounds of the present invention can control diseases by soil treatment. The compounds of the present invention are effective against not only sensitive fungi but also various resistant fungi (metalaxyl-resistant downy mildew fungus, strobilurin fungicide-resistant downy mildew fungus and the like).

Upon use, in the same manner as previous agricultural chemical formulations, the compounds of the present invention and an additive can be prepared into a variety of forms such as emulsions, powders, wettable powders, liquids, granules and suspensions. Upon actual use of these formulations, they can be used as they are, or can be used by diluting with a diluent such as water to a prescribed concentration.

Examples of additives to be used include carriers, emulsifiers, suspending agents, dispersants, developers, penetrants, wetting agents, thickeners, stabilizers and the like, and they can be appropriately added if necessary.

Carriers to be used are classified into solid carriers and liquid carriers, and solid carriers can be animal and vegetable powders such as starch, sugar, cellulose powder, cyclodextrin, activated carbon, soybean powder, wheat flour, hull powder, woodmeal, fish powder and milk powder; or mineral powders such as talc, kaolin, bentonite, organic bentonite, potassium carbonate, calcium sulfate, sodium bicarbonate, zeolite, diatomaceous earth, white carbon, clay, alumina, silica and sulfur powder; and liquid carriers can be water; animal or vegetable oils such as soybean oil, cottonseed oil and corn oil; alcohols such as ethyl alcohol and ethylene glycol; ketones such as acetone and methyl ethyl ketone; ethers such as dioxane and tetrahydrofuran; aliphatic hydrocarbons such as kerosine, kerosene and liquid paraffin; aromatic hydrocarbons such as xylene, trimethylbenzene, tetramethylbenzene, cyclohexane and solvent naphtha; halogenated hydrocarbons such as chloroform and chlorobenzene; acid amides such as dimethylformamide; esters such as ethyl acetate and glycerin esters of fatty acids; nitriles such as acetonitrile; sulfur-containing compounds such as dimethyl sulfoxide; or N-methylpyrrolidone.

The ratio by mass of incorporation of the present compound and an additive is usually 0.05:99.95-90:10, preferably 0.2:99.8-80:20.

The concentration and amount used of the compounds of the present invention are different depending on the subject crop, the method of use, the formulation form and the application dose, and, in the case of foliar treatment, are usually 0.1-10000ppm, preferably 1-1000ppm per active ingredient and, in the case of soil treatment, are usually 10-100000g/ha, preferably 200-20000g/ha.

If necessary, the compounds of the present invention can be mixed with or used together with other agricultural chemicals, for example, insecticides, acaricides, attractants, nematicides, fungicides, bactericides, herbicides and plant growth regulators, preferably insecticides, acaricides, nematicides or fungicides.

The insecticides, acaricides or nematicides to be used can be, for example, organic phosphate ester compounds such as O-(4-bromo-2-chlorophenyl)O-ethyl-S-propylphosphorothioate (common name: profenofos), O-(2,2-dichlorovinyl) O,O-dimethylphosphate (common name: dichlorvos), O-ethyl O-[3-methyl-4-(methylthio)phenyl] N-isopropylphosphoroamidate (common name: fenamiphos), O,O-dimethyl O-(4-nitro-m-tolyl)phosphorothioate (common name: fenitrothion), O-ethyl O-(4-nitrophenyl)phenylphosphonothioate (common name: EPN), O,O-diethyl O-(2-isopropyl-6-methylpyrimidin-4-yl)phosphorothioate (common name: diazinon), O,O-dimethyl O-(3,5,6-trichloro-2-pyridyl)phosphorothioate (common name: chlorpyrifos-methyl), O,S-dimethyl N-acetylphosphoroamidothioate (common name: acephate), O-(2,4-dichlorophenyl)O-ethyl S-propylphosphorodithioate (common name: prothiofos); carbamate compounds such as 1-naphthyl N-methylcarbamate (common name: carbaryl), 2-isopropoxyphenyl N-methylcarbamate (common name: propoxur), 2-methyl-2-(methylthio)propionaldehyde O-methylcarbamoyloxime (common name: aldicarb), 2,3-dihydro-2,2-dimethylbenzofuran-7-yl N-methylcarbamate (common name: carbofuran), dimethyl N,N'-[thiobis{(methylimino)carbonyloxy}]bisethaneimidothioate (common name: thiodicarb), S-methyl N-(methylcarbamoyloxy)thioacetoimidate (common name: methomyl), N,N-dimethyl-2-methylcarbamoyloxyimino-2-(methylthio)acetamide (common name: oxamyl), 2-(ethylthiomethyl)phenyl N-methylcarbamate (common name: ethiofencarb), 2-dimethylamino-5,6-dimethylpyrimidin-4-yl N,N-dimethylcarbamate (common name: pirimicarb), 2-sec-butylphenyl N-methylcarbamate (common name: fenobucarb); nereistoxin oxalate derivatives such as S,S'-2-dimethylaminotrimethylenebis(thiocarbamate) (common name: cartap) and N,N-dimethyl-1,2,3-trithian-5-ylamine (common name: thiocyclam); organochlorine compounds such as 2,2,2-trichloro-1,1-bis(4-chlorophenyl)ethanol (common name: dicofol) and 4-chlorophenyl-2,4,5-trichlorophenylsulfone (common name: tetradifon); organometallic compounds such as bis[tris(2-methyl-2-phenylpropyl)tin]oxide (common name: fenbutatin oxide); pyrethroid compounds such as (RS)-α-cyano-3-phenoxybenzyl (RS)-2-(4-chlorophenyl)-3-methylbutyrate (common name: fenvalerate), 3-phenoxybenzyl (1RS)-cis,trans-3-(2,2-dichlorovinyl)-2,2-dimethylcyclopropanecarboxylate (common name: permethrin), (RS)-α-cyano-3-phenoxybenzyl (1RS)-cis, trans-3-(2,2-dichlorovinyl)-2,2-dimethylcyclopropanecarboxylate (common name: cypermethrin), (S)-α-cyano-3-phenoxybenzyl (1R)-cis-3-(2,2-dibromovinyl)-2,2-dimethylcyclopropanecarboxylate (common name: deltamethrin), (RS)- α-cyano-3-phenoxybenzyl (1RS)-cis, trans-3-(2-chloro-3,3,3-trifluoropropenyl)-2,2-dimethylcyclopropanecarboxylate (common name: cyhalothrin), 4-methyl-2,3,5,6-tetrafluorobenzyl 3-(2-chloro-3,3,3-trifluoro-1-propenyl)-2,2-dimethylcyclopropanecarboxylate (common name: teflufchrin) and 2-(4-ethoxyphenyl)-2-methylpropyl 3-phenoxybenzyl ether (common name: etofenprox); benzoylurea compounds such as 1-(4-chlorophenyl)-3-(2,6-difluorobenzoyl)urea (common name: diflubenzuron), 1-[3,5-dichloro-4-(3-chloro-5-trifluoromethyl-2-pyridyloxy)phenyl]-3-(2,6-difluorobenzoyl)urea (common name: chlorfluazuron) and 1-(3,5-dichloro-2,4-difluorophenyl)-3-(2,6-difluorobenzoyl)urea (common name: teflubenzuron); juvenile hormone-like compounds such as isopropyl (2E,4E)-ll-methoxy-3,7,11-trimethyl-2,4-dodecadienoate (common name: methoprene); pyridazinone compounds such as 2-t-butyl-5-(4-t-butylbenzylthio)-4-chloro-3(2H)-pyridazinone (common name: pyridaben): pyrazole compounds such as t-butyl 4-[(1,3-dimethyl-5-phenoxypyrazol-4-yl)methyleneaminooxymethyl]benzoate (common name: fenpyroximate); nitro compounds such as 1-(6-chloro-3-pyridylmethyl)-N-nitro-imidazolidin-2-ylideneamine (common name: imidacloprid); or dinitro compounds, organosulfur compounds, urea compounds, triazine compounds, hydrazine compounds, and other compounds such as 2-tert-butylimino-3-isopropyl-5-phenyl-3,4,5,6-tetrahydro-2H-1,3,5-thiadiazin-4-one (common name: buprofezin), trans-(4-chlorophenyl)-N-cyclohexyl-4-methyl-2-oxothiazolidinon-3-carboxamide (common name: hexathizox), N-methylbis(2,4-xylyliminomethyl)amine (common name: amitraz), N'-(4-chloro-o-tolyl)-N,N-dimethylformamidine (common name: chlordimeform) and (4-ethoxyphenyl)-[3-(4-fluoro-3-phenoxyphenyl)propyl](dimethyl)silane (common name: silafluofen). Further, the compounds of the present invention can be mixed with or can be used together with microorganism agricultural chemicals such as BT agent and insect pathogen virus agent; or antibiotics such as avermectins and milbemycins.

Fungicides which can be used are, for example, pyrimidinamine compounds such as 2-anilino-4-methyl-6-(1-propynyl)pyrimidine (common name: mepanipyrim) and 4,6-dimethyl-N-phenyl-2-pyrimidinamine (common name: pyrimethanil); azole compounds such as 1-(4-chlorophenoxy)-3,3-dimethyl-1-(1H-1,2,4-triazol-1-yl)butanone (common name: triadimefon), 1-(biphenyl-4-yloxy)-3,3-dimethyl-1-(1H-1,2,4-triazol-1-yl)butan-2-ol (common name: bitertanol), 1-[N-(4-chloro-2-trifluoromethylphenyl)-2-propoxyacetoimidoyl]imidazole (common name: triflumizole), 1-[2-(2,4-dichlorophenyl)-4-ethyl-1,3-dioxolan-2-ylmethyl]-1H-1,2,4-triazole (common name: etaconazole), 1-[2-(2,4-dichlorophenyl)-4-propyl-1,3-dioxoran-2-ylmethyl]-1H-1,2,4-triazole (common name: propiconazole), 1-[2-(2,4-dichlorophenyl)pentyl]-1H-1,2,4-triazole (common name: penconazole), bis(4-fluorophenyl)(methyl)(1H-1,2,4-triazol-1-ylmethyl)silane (common name: flusilazole), 2-(4-chlorophenyl)-2-(1H-1,2,4-triazol-1-ylmethyl)hexanenitrile (common name: myclobutanil), (2RS,3RS)-2-(4-chlorophenyl)-3-cyclopropyl-1-(1H-1,2,4-triazol-1-yl)butan-2-ol (common name: cyproconazole), (RS)-1-(4-chlorophenyl)-4,4-dimethyl-3-(1H-l,2,4-triazol-1-ylmethyl)pentan-3-ol (common name: tebuconazole), (RS)-2-(2,4-dichlorophenyl)-1-(1H-1,2,4-triazol-1-yl)hexan-2-ol (common name: hexaconazole), (2RS,5RS)-5-(2,4-dichlorophenyl)tetrahydro-5-(1H-1,2,4-triazol-1-ylmethyl)-2-furyl 2,2,2-trifluoroethyl ether (common name: furconazole-cis), N-propyl-N-[2-(2,4,6-trichlorophenoxy)ethyl]imidazole-1-carboxamide (common name: prochloraz) and 2-(4-fluorophenyl)-1-(1H-1,2,4-triazol-1-yl)-3-trimethylsilylpropan-2-ol (common name: simeconazole); quinoxaline compounds such as 6-methyl-1,3-dithioro[4,5-b]quinoxalin-2-one (common name: chinomethionat); dithiocarbamate compounds such as polymer of manganese ethylenebis(dithiocarbamate) (common name: maneb), polymer of zinc ethylenebis(dithiocarbamate) (common name: zineb), a complex compound of zinc and manganese ethylenebis(dithiocarbamate)(maneb) (common name: mancozeb), dizinc bis(dimethyldithiocarbamate)ethylenebis(dithiocarbamate) (common name: polycarbamate) and polymer of zinc propylenebis(dithiocarbamate)(common name: propinab); organochlorine compounds such as 4,5,6,7-tetrachlorophthalide (common name: fthalide), tetrachloroisophthalonitrile (common name: chlorothalonil) and pentachloronitrobenzene (common name: quintozene); benzimidazole compound such as methyl 1-(butylcarbamoyl)benzimidazol-2-ylcarbamate (common name: benomyl), dimethyl 4,4'-(o-phenylene)bis(3-thioallophanate)(common name: thiophanate-methyl) and methyl benzimidazol-2-ylcarbamate (common name: carbendazim); pyridinamine compounds such as 3-chloro-N-(3-chloro-2,6-dinitro-4-α, α, α-trifluorotolyl)-5-trifluoromethyl-2-pyrimidinamine (common name: fluazinam); cyanoacetamide compounds such as 1-(2-cyano-2-methoxyiminoacetyl)-3-ethylurea (common name: cymoxanil); phenylamide compounds such as methyl N-(2-methoxyacetyl)-N-(2,6-xylyl)-DL-alaninate (common name: metalaxyl), 2-methoxy-N-(2-oxo-1,3-oxazolydin-3-yl)aceto-2',6'-xylidide (common name: oxadixyl), (±)-α-2-chloro-N-(2,6-xylylacetamido)-γ-butyrolactone (common name: ofurace), methyl N-phenylacetyl-N-(2,6-xylyl)-DL-alaninate (common name: benalaxyl), methyl N-(2-furoyl)-N-(2,6-xylyl)-DL-alaninate (common name: furalaxyl) and (±)-α-[N-(3-chlorophenyl)cyclopropanecarboxamide]- γ-butyrolactone (common name: cyprofuran); sulfenic acid compounds such as N-dichlorofluoromethylthio-N',N'-dimethyl-N-phenylsulfamide (common name: dichlofluanid); copper compounds such as cupric hydroxide (common name: cupric hydroxide) and copper 8-quinolinolate (common name: oxine-copper); isooxazole compounds such as 5-methylisoxazol-3-ol (common name: hydroxyisoxazole); organophosphorus compounds such as aluminium tris(ethylphosphonate)(common name: fosetyl-aluminium), O-2,6-dichloro-p-tolyl-O,O-dimethylphosphorothioate (common name: tolclofos-methyl), S-benzyl O,O-diisopropylphosphorothioate, O-ethyl S,S-diphenylphosphorodithioate and aluminium ethyl hydrogen phosphonate; N-halogenothioalkyl compounds such as N-(trichloromethylthio)cyclohex-4-ene-1,2-dicarboximide (common name: captan), N-(1,1,2,2-tetrachloroethylthio)cyclohex-4-ene-1,2-dicarboximide (common name:captafol) and N-(trichloromethylthio)phthalimide (common name: folpet); dicarboximide compounds such as N-(3,5-dichlorophenyl)-1,2-dimethylcyclopropane-1,2-dicarboximide (common name: procymidone), 3-(3,5-dichlorophenyl)-N-isopropyl-2,4-dioxoimidazolidine-1-carboxamide (common name: iprodione) and (RS)-3-(3,5-dichlorophenyl)-5-methyl-5-vinyl-1,3-oxazolidine-2,4-dione (common name: vinclozolin); benzanilide compounds such as α,α,α-trifluoro-3'-isopropoxy-o-toluanilide (common name: flutolanil) and 3'-isopropoxy-o-toluanilide (common name: mepronil); piperazine compounds such as N,N'-[piperadin-1,4-diylbis[(trichloromethyl)methylene]]diformamide (common name: triforine); pyridine compounds such as 2',4'-dichloro-2-(3-pyridyl)acetophenone O-methyloxime (common name: pyrifenox); carbinol compounds such as (±)-2,4'-dichloro-α-(pyrimidin-5-yl)benzhydrylalcohol (common name: fenarimol) and (±)-2,4'-difluoro-α-(1H-1,2,4-triazol-1-ylmethyl)benzhydrylalcohol (common name: flutriafol); piperidine compounds such as (RS)-1-[3-(4-tertiary-butylphenyl)-2-methylpropyl]piperidine (common name: fenpropidin); morpholine compounds such as (±)-cis-4-[3-(4-tertiary-butylphenyl)-2-methylpropyl]-2,6-dimethylmorpholine (common name: fenpropimorph); organotin compounds such as triphenyltin hydroxide (common name: fentin hydroxide) and triphenyltin acetate (common name: fentin acetate); urea compounds such as 1-(4-chlorobenzyl)-1-cyclopentyl-3-phenylurea (common name: pencycuron); cinnamic acid compounds such as (E,Z)4-[3-(4-chlorophenyl)-3-(3,4-dimethoxyphenyl)acryloyl]morpholine (common name: dimethomorph); phenylcarbamate compounds such as isopropyl 3,4-diethoxycarbanilate (common name: diethofencarb); or cyanopyrrole compounds such as 3-cyano-4-(2,2-difluoro-1,3-benzodioxol-4-yl)pyrrole (common name: fludioxonil) and 3-(2',3'-dichlorophenyl)-4-cyano-pyrrole (common name: fenpiclonil).

### [Best mode for carrying out the invention]

The present invention will be explained in detail below by showing Examples, Formulation Examples and Test Examples of compounds of the present invention.

### (Example 1)

### N-(3-Cyanobenzyl)-N-(3,5-dimethyl-1-phenyl-lH-pyrazol-4-yl)acetamide (Exemplification compound number D-229)

(1) N-(3,5-Dimethyl-1-phenyl-1H-pyrazol-4-yl)acetamide (Step A1)
   To a solution of 4-amino-3,5-dimethyl-1-phenylpyrazole (18.2 g, 98.2 mmol) in dichloromethane (250 ml) was added pyridine (20 ml, 247.3 mmol) at 0°C with stirring. Then, acetic anhydride (20 ml, 212.0 mmol) was added dropwise to the resulting mixture with stirring at 0°C over 3 minutes, and the resulting mixture was stirred for 1 hr at the same temperature. After the reaction was completed, the reaction mixture was washed successively with saturated sodium hydrogen carbonate solution and 0.5-mol/L aqueous hydrochloric acid solution. The organic layer obtained by partition was dried over anhydrous sodium sulfate and concentrated after removing the drying agent by filtration. The residue obtained was purified by chromatography on a silica gel column (eluent: elution was performed using hexane, ethyl acetate/hexane (1/2), ethyl acetate and methanol/ethyl acetate (1/20) successively) to afford N-(3,5-dimethyl-1-phenyl-1H-pyrazol-4-yl)acetamide (16.81 g, 73.32 mmol) in 75% yield. ¹H-NMR (270 MHz, CDCl₃) δ (ppm): 2.20(s); 2.21(s); 2.22(s) (2.20-2.22, 6H), 2.251(s); 2.255(s) (2.251-2.25, 3H), 6.48(brs); 6.65(brs) (6.48-6.65, 1H), 7.30-7.52(m, 5H). MASS (EI) m/z: 229(M⁺), 187, 145, 131, 118, 104, 77.
(2) N-(3-Cyanobenzyl)-N-(3,5-dimethyl-1-phenyl-1H-pyrazol-4-yl)acetamide (Exemplification compound number D-229, Step A2)

To a solution of N-(3,5-dimethyl-1-phenyl-1H-pyrazol-4-yl)acetamide (10.22 g, 44.57 mmol) obtained in (1) in dimethylformamide (120 ml) was added 60% sodium hydride (1.81 g, 45.25 mmol) at 0°C with stirring, and the resulting mixture was stirred for 20 min at the same temperature. At the end of this time, to the reaction mixture was further added 95% 3-cyanobenzyl bromide (9.7 g, 47.00 mmol) at 0°C with stirring, and the resulting mixture was stirred for 1.5 hr at the same temperature. After the reaction was completed, water was added gradually to the reaction mixture, and the resulting mixture was extracted with a mixed solvent of ethyl acetate and hexane (1:1, v/v). The extract was washed successively with water for 5 times and aqueous sodium chloride solution once and dried over anhydrous sodium sulfate. After removal of the drying agent by filtration, the solvent was evaporated *in vacuo.* The residue obtained was purified by chromatography on *a* silica gel column (eluent: elution was performed using hexane and ethyl acetate/hexane (1/1) successively) to afford the title compound of the present invention (15.21 g, 44.16 mmol) in 99% yield.
¹H-NMR (200 MHz, CDCl₃) δ (ppm) : 1.81(s, 3H), 1.90(s, 3H), 1.97(s, 3H), 4.57(d, J=12.8Hz, 1H), 4.99(d, J=12.8Hz, 1H), 7.32-8.62(m, 9H).
MASS (EI) m/z: 344(M⁺), 301, 228, 186, 172, 145, 118, 89, 77.

### (Example 2)

### Cyclopropanecarboxylic acid (3-cyanobenzyl)(1-isobutyl-5-methyl-1H-pyrazol-4-yl)amide (Exemplification compound number C-83)

(1) Cyclopropanecarboxylic acid (1-isobutyl-3-methyl-1H-pyrazol-4-yl)amide and cyclopropanecarboxylic acid (1-isobutyl-5-methyl-1H-pyrazol-4-yl)amide (Step A1)
   To a solution of a mixture (22.51 g, 146.91 mmol) of 4-amino-1-isobutyl-3-methyl-1H-pyrazole and 4-amino-1-isobutyl-5-methyl-1H-pyrazole in ethyl acetate (270 ml) was added pyridine (14.5 ml, 179.28 mmol) at 0°C with stirring. Then, cyclopropanecarbonyl chloride (16.0 ml, 176.32 mmol) was added dropwise to the resulting mixture with stirring over 5 minutes at the same temperature, and the resulting mixture was stirred at room temperature for 60 min. After the reaction was completed, saturated aqueous sodium hydrogen carbonate solution was added to the reaction mixture, and the resulting mixture was extracted with a mixed solvent of ethyl acetate and hexane (1:1, v/v). The extract was dried over anhydrous sodium sulfate and concentrated by evaporation after removal of the drying agent by filtration. The residue obtained was purified by chromatography on a silica gel column (eluent: elution was performed using hexane, ethyl acetate/hexane (1/2) and ethyl acetate successively) to afford the title compound, cyclopropanecarboxylic acid (1-isobutyl-3-methyl-1H-pyrazol-4-yl)amide (15.75 g, 71.22 mmol, yield: 48%), and cyclopropanecarboxylic acid (1-isobutyl-5-methyl-1H-pyrazol-4-yl)amide (12.67 g, 57.25 mmol, yield: 40%).
   Cyclopropanecarboxylic acid (1-isobutyl-3-methyl-1H-pyrazol-4-yl)amide Rf (Development solvent : ethyl acetate/hexane = 1/1): 0.29 ¹H-NMR (200 MHz, CDCl₃) δ (ppm): 0.80-0.95(m, 2H), 0.89(d, 6H, J=7.0Hz), 1.05-1.12(m, 2H), 1.53(m, 1H), 2.15(hep, 1H, J=7.0Hz), 2.24(s, 3H), 3.78(d, 2H, J=7.0Hz), 7.0(brs, 1H), 7.82(s, 1H).
   Cyclopropanecarboxylic acid (1-isobutyl-5-methyl-1H-pyrazol-4-yl)amide (mixture of two atropisomers) Rf (Development solvent : ethyl acetate/hexane = 1/1): 0.16 ¹H-NMR (200 MHz, CDCl₃) δ (ppm): 0.69-0.78 and 0.80-0.95(m, 2H), 0.88 and 0.90(d, 6H, J=7.0Hz), 1.02-1.11(m, 2H), 1.53(m, 1H), 2.15(brs, 1H, J=7.0Hz), 2.17 and 2.22(s, 3H), 3.79 and 3.81(d, 2H, J=7.0Hz), 6.47 and 6.78(brs, 1H), 7.42 and 7.54(s, 1H).
(2) Cyclopropanecarboxylic acid (3-cyanobenzyl)(1-isobutyl-5-methyl-1H-pyrazol-4-yl)amide (Exemplification compound number C-83, Step A2)
   To a solution of cyclopropanecarboxylic acid (1-isobutyl-5-methyl-1H-pyrazol-4-yl)amide (12.67 g, 57.25 mmol) obtained in (1) in dimethylformamide (DMF)(400 ml) was added 60% sodium hydride (1.54 g, 64.00 mmol) at 0°C with stirring, and the resulting mixture was stirred for 30 min at room temperature. After cooling to 0°C once again, to the reaction mixture was further added 3-cyanobenzyl bromide (12.39 g, 63.20 mmol) with stirring at 0°C, and the resulting mixture was stirred for 40 min at room temperature. After the reaction was completed, water (200 ml) was added to the reaction mixture, and the resulting mixture was concentrated until the volume was reduced to 300 ml. Then, water (500 ml) was added to the concentrated reaction mixture thus obtained, and the resulting mixture was extracted with a mixed solvent of ethyl acetate and hexane (1:1, v/v). The extract was washed successively with water and saturated aqueous sodium chloride solution and dried over anhydrous sodium sulfate. After removal of the drying agent by filtration, the filtrate was concentrated. The residue obtained was purified by chromatography on a silica gel column (eluent: elution was performed using hexane, ethyl acetate/hexane (1/2) and ethyl acetate successively) to afford the title compound of the present invention (20.00 g, 57.25 mmol) in 100% yield.
   ¹H-NMR (200 MHz, CDCl₃) δ (ppm): 0.69-0.63(m, 2H), 0.87(d, 6H, J=7Hz), 0.92-1.08(brm, 2H), 1.38-1.58(m, 1H), 1.80(s, 3H), 2.18(brm, 2H), 3.78(d, 2H, J=7Hz), 4.78(brs, 2H), 7.27(s, 3H), 7.34-7.58(m, 4H).
   MASS (EI) m/z: 336(M⁺), 268, 220, 152, 116, 69, 57.

### (Example 3)

### Methyl N-(3-cyanobenzyl)-N-(1-isobutyl-3-methyl-1H-pyrazole)carbamate (Exemplification compound number B-89)

(1) 3-{(1-Isobutyl-3-methyl-1H-pyrazol-4-ylamino)methyl}benzonitrile and 3-{(1-isobutyl-5-methyl-1H-pyrazol-4-ylamino)methyl}benzonitrile (Steps B1 and B2)
   To a solution of a mixture (3.08 g) of 1-isobutyl-3-methylpyrazole and 1-isobutyl-5-methylpyrazole (mixing ratio: about 1:1) in ethanol (30 ml) was added 3-cyanobenzaldehyde (content: 95%, 3.05 g), and the resulting mixture was refluxed for 3 hr. After the starting materials were confirmed to be consumed, to the reaction mixture were added acetic acid (5.7 ml) and sodium cyanoborohydride (content: 95%, 2.66 g) successively with stirring under cooling in ice-water bath, and the resulting mixture was stirred at room temperature for 1 hr. After the reaction was completed, water was added to the reaction mixture, and the resulting mixture was basified slightly with aqueous sodium hydrogen carbonate solution and extracted with ethyl acetate. The extract was washed with saturated sodium chloride solution and dried over anhydrous sodium sulfate. After removal of the drying agent by filtration, the solvent was evaporated *in vacuo*, and the residue obtained was purified by chromatography on a silica gel column (eluent: from hexane/ethyl acetate (2/8) to ethyl acetate) to afford 3-{(1-isobutyl-3-methyl-1H-pyrazol-4-ylamino)methyl}benzonitrile (2.63 g, yield: 49%) which is the title compound and 3-{(1-isobutyl-5-methyl-1H-pyrazol-4-ylamino)methyl}benzonitrile (1.92 g, yield: 36%).
   3-{(1-Isobutyl-3-methyl-1H-pyrazol-4-ylamino)methyl}benzonitrile
   ¹H-NMR (200 MHz, CDCl₃) δ (ppm): 7.68 (1H, s), 7.61 (1H, d, J=8Hz), 7.57 (1H, d, J=8Hz), 7.44 (1H, t, J=8Hz), 6.68 (1H, s), 4.17 (2H, s), 3.69 (2H, d, J=7Hz), 2.76 (1H, brs), 2.17 (3H, s), 2.08 (1H, brhept, J=7Hz), 0.85 (6H, d, J=7Hz).
   ¹³C-NMR (67.9 MHz, CDCl₃) δ (ppm) : 141.3, 137.3, 133.3, 132.1, 131.2, 130.9, 129.7, 129.3, 117.1, 112.6, 59.7, 51.7, 29.7, 19.9, 10.9. MASS (EI) m/z: 268(M⁺), 225, 212, 157, 152, 116, 110, 96, 57. 3-{(1-Isobutyl-5-methyl-1H-pyrazol-4-ylamino)methyl}benzonitrile ¹H-NMR (200 MHz, CDCl₃) δ (ppm): 7.67 (1H, s), 7.58 (1H, d, J=8Hz), 7.55 (1H, d, J=8Hz), 7.42 (1H, t, J=8Hz), 7.09 (1H, s), 4.19 (2H, s), 3.76 (2H, d, J=7Hz), 2.83 (1H, brs), 2.15 (1H, brhept, J=7Hz), 2.10 (3H, s), 0.88 (6H, d, J=7Hz). ¹³C-NMR (67.9 MHz, CDCl₃) δ (ppm): 141.7, 132.3, 131.4, 130.8, 129.6, 129.1, 128.4, 127.3, 118.8, 112.5, 56.7, 52.3, 29.6, 19.9, 8.8. MASS (EI) m/z: 268(M⁺), 253, 225, 212, 152, 116, 96, 57.
(2) Methyl N-(3-cyanobenzyl)-N-(1-isobutyl-3-methyl-1H-pyrazole)carbamate (Exemplification compound number B-89, Step B3)

To a solution of 3-{(1-isobutyl-3-methyl-1H-pyrazol-4-ylamino)methyl}benzonitrile (106 mg) obtained in (1) in dichloromethane (2ml) were added pyridine (64 µl) and methyl chlorocarbonate (61 µl) successively under cooling in an ice-water bath, and the resulting mixture was stirred for 15 min under the same reaction conditions. After the reaction was completed, the reaction mixture was acidified slightly with aqueous ammonium chloride solution and extracted with ethyl acetate. The extract was washed with saturated aqueous sodium chloride solution and dried over anhydrous sodium sulfate. After removal of the drying agent by filtration, the solvent was evaporated *in vacuo,* and the residue obtained was purified by chromatography on a silica gel column (eluent: hexane/ethyl acetate = 4/6) to afford the title compound of the present invention (110 mg) in 85% yield. ¹H-NMR (200 MHz, CDCl₃) δ (ppm): 7.60-7.36 (4H, m), 6.89 (1H, s), 4.69 (2H, s), 3.72 (2H, d, J=7Hz), 3.72 (3H, brs), 2.10 (1H, brhept, J=7Hz), 1.99 (3H, s), 0.84 (6H, d, J=7Hz). MASS (EI) m/z: 326(M⁺), 311, 283, 270, 210, 168, 154, 116, 57.

### (Example 4)

Methyl N-(3-cyanobenzyl)-N-(1-isobutyl-5-methyl-1H-pyrazole)carbamate (Exemplification compound number B-93, Step B3)

To a solution of 3-{(1-isobutyl-5-methyl-1H-pyrazol-4-ylamino)methyl}benzonitrile (97.8 mg) obtained in step (1) of Example 3 in dichloromethane (2ml) were added pyridine (59 µl) and methyl chlorocarbonate (56 µl) successively under cooling in an ice-water bath, and the resulting mixture was stirred for 15 min under the same reaction conditions. After the reaction was completed, the reaction mixture was acidified slightly with aqueous ammonium chloride solution and extracted with ethyl acetate. The extract was washed with saturated sodium chloride solution and dried over anhydrous sodium sulfate. After removal of the drying agent by filtration, the solvent was evaporated *in vacuo,* and the residue obtained was purified by chromatography on a silica gel column (eluent: hexane/ethyl acetate = 4/6) to afford the title compound of the present invention (95.8 mg) in 81% yield.
¹H-NMR (200 MHz, CDCl₃) δ (ppm): 7.60-7.36 (4H, m), 7.17 (1H, s), 4.71 (2H, s), 3.75 (2H, d, J=7Hz), 3.70 (3H, brs), 2.16 (1H, brhept, J=7Hz), 1.80 (3H, s), 0.86 (6H, d, J=7Hz).
MASS (EI) m/z: 326(M⁺), 310, 261, 234, 210, 188, 149, 91, 57. HRMS m/z: 326.1741 (326.1743 calcd. for C₁₈H₂₂N₄O₂).

### (Example 5)

### N-[1-(3-Cyanophenyl)ethyl]-N-(1-isobutyl-3-methyl-1H-pyrazol-4-yl)-2-acetoxyacetamide (Exemplification compound number C-645)

(1) 3-[1-(1-Isobutyl-5-methyl-1H-pyrazol-4-ylamino)ethyl]benzonitrile (Steps B1 and B2) To a solution of 4-amino-1-isobutylpyrazole (506.3 mg, 3.304 mmol) and 3-cyanoacetophenone (503.6 mg, 3.469 mmol) in xylene (35 ml) was added p-toluenesulfonic acid monohydrate (62 mg, 0.326 mmol), and the resulting mixture was refluxed for 1 hr. After cooling to room temperature, saturated aqueous sodium hydrogen carbonate solution was added to the reaction mixture, and the resulting mixture was extracted with ethyl acetate and dried over anhydrous magnesium sulfate. After removal of the drying agent by filtration, the filtrate was concentrated *in vacuo,* and the residue obtained was purified by chromatography on a silica gel column (eluent: from hexane to ethyl acetate/hexane (4/1)) to afford the imine derivative. To a solution of the obtained imine derivative in ethanol (20 ml) were added acetic acid (1.90 ml, 33.191 mmol) and 95% sodium cyanotrihydroborate (645.0 mg, 9.751 mmol) successively at 0°C with stirring, and the resulting mixture was stirred at room temperature for 1 hr. After stirring, the reaction mixture was poured into saturated aqueous sodium hydrogen carbonate solution, extracted with ethyl acetate and dried over anhydrous magnesium sulfate. After removal of the drying agent by filtration, the filtrate was concentrated *in vacuo,* and the residue obtained was purified by chromatography on a silica gel column (eluent: from hexane to ethyl acetate/hexane (1/11)) to afford the title compound (588.0 mg, 2.082 mmol) in 63% yield.
   ¹H-NMR (270 MHz, CDCl₃) δ (ppm): 0.83(3H, d, J=7.0Hz), 0.85(3H, d, J=7.0Hz), 1.45(3H, d, J=6.4Hz), 2.07(3H, s), 2.09(1H, m), 3.72(2H, d, J=7.5Hz), 4.22(1H, q, J=6.5Hz), 6.91(1H, s), 7.35-7.72(4H,m). MASS (EI) m/z: 282(M⁺), 267, 152, 130, 103, 96, 57.
(2) N-[1-(3-Cyanophenyl)ethyl]-N-(1-isobutyl-3-methyl-1H-pyrazol-4-yl)-2-acetoxyacetamide (Exemplification compound number C-121, Step B3)

To a solution of 3-[1-(1-isobutyl-5-methyl-1H-pyrazol-4-ylamino)ethyl]benzonitrile (210.3 mg, 0.7447 mmol) obtained in (1) in dichloromethane (10ml) were added pyridine (0.10 ml, 1.2092 mmol) and acetoxyacetyl chloride (0.13 ml, 1.2093 mmol) successively at 0°C with stirring, and the resulting mixture was stirred for 75 min at room temperature. After the reaction was completed, saturated sodium hydrogen carbonate solution was added to the reaction mixture, and the resulting mixture was extracted with dichloromethane and dried over anhydrous magnesium sulfate. After removal of the drying agent by filtration, the filtrate was concentrated *in vacuo.* The residue obtained was purified by chromatography on a silica gel column (eluent: from hexane to ethyl acetate/hexane (1/1)) to afford the title compound of the present invention (222.8 mg, 0.5825 mmol) in 78% yield.
¹H-NMR (270 MHz, CDCl₃) δ (ppm): 0.8-0.95(6H, m), [1.33(d, J=7.5Hz) and 1.50(d, J=7.0Hz)](3H), 2.06-2.28(5H, m), 3.6-3.8(2H, m), [3.83(d, J=7.0Hz) and 4.20(d, J=10Hz)](2H), [6.14(q, J=7.0Hz) and 6.19(q, J=7.0Hz)] (1H), 7.32-7.65(5H, m). MASS (EI) m/z: 382(M⁺), 280, 252, 210, 197, 180, 149, 130, 103, 73, 57.

### (Example 6)

### N-Cyano(3-cyanophenyl)methyl-N-[3,5-dimethyl-1-phenyl-1H-pyrazol-4-yl]acetamide (Exemplification compound number C-484)

(1) 3-[Cyano(3,5-dimethyl-1-phenyl-1H-pyrazol-4-ylamino)methyl]benzonitrile (Step C1)
   To a solution of 4-amino-3,5-dimethyl-1-phenylpyrazole (445.8 mg, 2.381 mmol) in xylene (8 ml) was added 3-(bromocyanomethyl)benzonitrile (0.54 ml, c.a. 2.5 mmol), and the resulting mixture was heated at 100°C in an oil bath for 2 hr. After the reaction was completed, the reaction mixture was cooled to room temperature and purified by preparative thin layer chromatography (hereinafter referred to as preparative TLC. Development solvent: ethyl acetate/hexane = 2/1) to afford the title compound (304.4 mg, 0.930 mmol) in 39% yield. ¹H-NMR (270 MHz, CDCl₃) δ (ppm): 2.32(3H, s), 2.35(3H, s) , 5.05(1H, s), 7.29-7.58(5H, m), 7.66(1H, dd, J=8.1Hz, J=8.1Hz), 7.89(1H, d, J=8.1Hz), 7.95(1H, d, J=8.1Hz), 8.03(1H, s). MASS (EI) m/z: 327(M⁺), 186, 149, 141, 130, 118, 77.
(2) N-Cyano(3-cyanophenyl)methyl-N-[3,5-dimethyl-1-phenyl-1H-pyrazol-4-yl]acetamide (Exemplification compound number C-484, Step C2)
   To a solution of 3-[cyano(3,5-dimethyl-l-phenyl-1H-pyrazol-4-ylamino)methyl]benzonitrile (70.8 mg, 0.216 mmol) obtained in (1) in dichloromethane (5ml) were added pyridine (0.12 ml, 1.484 mmol) and acetyl chloride (0.1 ml, 1.406 mmol) successively at 0°C with stirring, and the resulting mixture was stirred for 4 hr at the same temperature and then kept standing overnight. After the reaction was completed, to the reaction mixture was added saturated sodium hydrogen carbonate solution under stirring, and the resulting mixture was extracted with dichloromethane and dried over anhydrous magnesium sulfate. After removal of the drying agent by filtration, the filtrate was concentrated *in vacuo,* and the residue obtained was purified by preparative TLC (development solvent: ethyl acetate/hexane = 2/1) to afford the title compound of the present invention (72.9 mg, 0.197 mmol) in 91% yield.
   ¹H-NMR (270 MHz, CDCl₃) δ (ppm): [1.21(s) and 1.22(s)](3H), [1.90(s) and 1.91(s)](3H), [2.43(s) and 2.44(s)](3H), 7.28-7.84(10H, m).
   MASS (EI) m/z: 369(M⁺), 326, 300, 228, 186, 145, 118, 145, 118, 77.

### (Example 7)

### N-(3-Cyanobenzyl)-N-[3,5-dimethyl-1-(2,5-difluorophenyl)-1H-pyrazol-4-yl]acetamide (Exemplification compound number D-349)

(1) N-(1-Acetyl-2-oxopropyl)acetamide (Step D1)
   To a solution of 3-hydroxyimino-2,4-pentanedione (13.0 g, 101 mmol) in ethyl acetate (130 ml) were added successively 10% palladium on carbon (1.00 g, 9.40 mmol) and acetic anhydride (28.5 ml, 302 mmol) in a nitrogen atmosphere under stirring. After degassing with nitrogen and replacing the nitrogen with hydrogen successively, the resulting mixture was stirred at room temperature for 3 hr under atmospheric pressure. After stirring, the reaction mixture was degassed with nitrogen once again and filtered with Celite to remove the palladium on carbon catalyst, which was washed thoroughly with ethyl acetate. The filtrate and the washings were combined and concentrated *in vacuo*, and the residue obtained was purified by chromatography on a silica gel column (eluent: ethyl acetate) to afford the title compound (15.16 g, 96.4 mmol) in 96% yield. MASS (EI); m/z: 157(M⁺), 115, 100, 97, 87, 72.
(2) N-[3,5-Dimethyl-1-(2,5-difluorophenyl)-1H-pyrazol-4-yl]acetamide (Step D2)
   To a solution of N-(1-acetyl-2-oxopropyl)acetamide (318.0 mg, 2.02 mmol) obtained in (1) in water (10ml) was added 2,5-difluorophenylhydrazine hydrochloride (452 mg, 2.43 mmol) with stirring at room temperature, and the resulting mixture was stirred for 3 hr. After the reaction was completed, water was added to the reaction mixture, and the resulting mixture was extracted with ethyl acetate and dried over anhydrous sodium sulfate. After removal of the solvent by evaporation, the residue obtained was purified by chromatography on a silica gel column (eluent: ethyl acetate) to afford the title compound (447.0 mg, 1.69 mmol) in 83% yield.
   ¹H-NMR (200 MHz, CDCl₃) 8 (ppm): 1.59(s), 2.12(d, J=2.24Hz), 2.16(d, J=2.11Hz), 2.21(d, J=2.48Hz), 6.45(brs), 6.58(brs), 7.05-7.35(m).
   MASS (EI) m/z: 265(M⁺), 223, 205, 187, 182, 154, 140, 127, 118, 113, 93,83, 72, 63.
(3) N-(3-Cyanobenzyl)-N-[3,5-dimethyl-1-(2,5-difluorophenyl)-1H-pyrazol-4-yl]acetamide (Exemplification compound number D-349, Step D3) To a solution of M-[3,5-dimethyl-1-(2,5-difluorophenyl)-1H-pyrazol-4-yl]acetamide (100.6 mg, 0.379 mmol) obtained in (2) in N,N-dimethylformamide (DMF, 3 ml) was added 60% sodium hydride (23 mg, 0.569 mmol) at 0°C with stirring, and the resulting mixture was stirred for 5 min at the same temperature. Subsequently, to the reaction mixture was further added 3-cyanobenzyl bromide (117 mg, 0.569 mmol) with stirring at the same temperature, and the resulting mixture was stirred for 1 hr. After the reaction was completed, aqueous sodium chloride solution was added to the reaction mixture, and the resulting mixture was extracted with ethyl acetate. The extract was washed with aqueous sodium chloride solution and dried over anhydrous sodium sulfate. After removal of the drying agent by filtration, the filtrate was concentrated in *vacuo,* and the residue obtained was purified by chromatography on a silica gel column (eluent: ethyl acetate) to afford the title compound of the present invention (137.6 mg, 0.362 mmol) in 95% yield.
   ¹H-NMR (200 MHz, CDCl₃) δ (ppm): 1.67(3H, d, J=2.12Hz), 1.91(3H, s), 2.01(3H, s), 4.49(1H, d, J=13.84Hz), 5.08(1H, d, J=13.84Hz), 7.05-7.35(3H, m), 7.36-7.67(4H, m).

MASS (EI) m/z: 387(M⁺), 337, 264, 222, 208, 181, 154, 113, 89,83, 63.

### (Example 8)

### Methyl (3-cyanobenzyl)-[1-(3-fluorophenyl)-3,5-dimethyl-1H-pyrazol-4-yl]carbamate (Exemplification compound number D-338)

(1) Methyl (1-acetyl-2-oxopropyl)carbamate (Step D1)
   3-Hydroxyimino-2,4-pentanedione (25.03 g, 193.85 mmol) was dissolved in ethyl acetate (50 ml), and the resulting solution was degassed with nitrogen. Subsequently, to the solution was added 10% palladium on carbon (2.22 g, 2.07 mmol) under stirring. After degassing with nitrogen and replacing the nitrogen with hydrogen successively, to the resulting mixture was further added dimethylpyrocarbonate (60.0 ml, 558.49 mmol), and the resulting mixture was stirred successively for 2 hr at room temperature and for 5 hr at 50°C. After the reaction was completed, the reaction mixture was degassed with nitrogen once again and filtered with Celite to remove the palladium on carbon catalyst, which was washed with ethyl acetate. The filtrate and the washings were combined and concentrated *in vacuo,* and the residue obtained was purified by chromatography on a silica gel column (eluent: from hexane to ethyl acetate/hexane (1/2)) to afford the title compound (8.05 g, 46.49 mmol) in 24% yield.
   ¹H-NMR (200 MHz, CDCl₃, 70°C) δ (ppm): 2.10(6H, s), 5.54(1H, brs), 15.71(1H, s).
   MASS (EI) m/z: 173(M⁺), 155, 141, 131, 126, 114, 99, 87, 72, 59, 54.
(2) Methyl [1-(3-fluorophenyl)-3,5-dimethyl-1H-pyrazol-4-yl]carbamate (Step D2)
   To a solution of methyl (1-acetyl-2-oxopropyl)carbamate-(6.60 g, 38.11 mmol) obtained in (1) in methanol (30ml) was added an aqueous solution (50 ml) of 3-fluorophenylhydrazine (6.90 g, 42.44 mmol) at room temperature, and the resulting mixture was stirred for 2.5 hr. After the reaction was completed, water (250 ml) was added gradually to the reaction mixture, and the resulting mixture was extracted with ethyl acetate and dried over anhydrous magnesium sulfate. After removal of the drying agent by filtration, the filtrate was concentrated to afford a mixture (10.1 g) of methyl (1-acetyl-2-oxopropyl)carbamate and the title compound with a mixing ratio of 1:7.
   ¹H-NMR (200 MHz, CDCl₃) δ (ppm): 1.6(3H, brs), 2.23(3H, s), 2.26(3H, s), 3.78(3H, s), 5.88(1H, s), 7.06(1H, brt, J=7.15Hz), 7.17-7.27(2H, m), 7.36-7.47(1H, m).
(3) Methyl (3-cyanobenzyl)-[1-(3-fluorophenyl)-3,5-dimethyl-1H-pyrazol-4-yl]carbamate (Exemplification compound number D-338, Step D3)
   To a solution of a mixture (10.1 g) of methyl (1-acetyl-2-oxopropyl)carbamate and methyl [1-(3-fluorophenyl)-3,5-dimethyl-1H-pyrazol-4-yl]carbamate (mixing ratio of 1:7) obtained in (2) in DMF (110 ml) was added 60% sodium hydride (1.86 g, 46.5 mmol) at 0°C with stirring, and the resulting mixture was stirred for 15 min at room temperature. After cooling to 0°C once again, 3-cyanobenzyl bromide (9.18 g, 46.82 mmol) was added to the reaction mixture with stirring, and the resulting mixture was stirred for 1.5 hr at room temperature. After stirring, 60% sodium hydride (342.6 mg, 8.57 mmol) was further added to the reaction mixture with stirring, and the resulting mixture was stirred for 5 min, and then 3-cyanobenzyl bromide (2.76 g, 14.08 mmol) was further added with stirring, and the resulting mixture was stirred for 45 min. After the reaction was completed, water (300 ml) was added gradually to the reaction mixture, and the resulting mixture was extracted with a mixed solvent of ethyl acetate and hexane (1:1, v/v). The extract was washed with water and aqueous sodium chloride solution successively, dried over anhydrous magnesium sulfate and concentrated *in vacuo* after removal of the drying agent by filtration. The residue obtained was purified by chromatography on a silica gel column (eluent: from hexane to ethyl acetate/hexane (1/1)) to afford the title compound of the present invention (11.15 g, 29.47 mmol). This compound was obtained in 77% yield from methyl (1-acetyl-2-oxopropyl)carbamate.
   ¹H-NMR (200 MHz, CDCl₃) δ (ppm): 1.62(3H, s), 1.90(3H, s), 1.97(3H, s), 3.72(3H, s), 4.60(1H, d, J=13.95Hz), 4.82(1H, d, J=13.95Hz), 7.07(1H, brt, J=6.97Hz), 7.15-7.25(2H, m), 7.36-7.68(5H, m).
   MASS (EI) m/z: 378(M⁺), 347, 319, 262, 230, 190, 149, 136, 116, 95, 75, 59.

### (Example 9)

### t-Butyl N-(3-Cyanobenzyl)-N-(5-methyl-1-phenyl-1H-pyrazol-4-yl)carbamate (Exemplification compound number C-344)

(1) t-Butyl N-(5-methyl-1-phenyl-1H-pyrazol-4-yl)carbamate (Steps E1, E2 and E4)
   To a suspension of 5-methyl-1-phenyl-1H-pyrazol-4-carboxylic acid (973 mg) in t-butyl alcohol (20 ml) were added successively triethylamine (0.67 ml) and diphenylphosphoryl azide (DPPA, 1.04 ml), and the resulting mixture was refluxed for 90 min. After the reaction was completed, aqueous sodium hydrogen carbonate solution was added to the reaction mixture, and the resulting mixture was extracted with ethyl acetate. The extract was washed with saturated aqueous sodium chloride solution and dried over anhydrous sodium sulfate. After removal of the drying agent by filtration, the solvent was evaporated *in vacuo,* and the residue obtained was purified by chromatography on a silica gel column (eluent: hexane/ethyl acetate (7/3)) to afford the title compound (434 mg) in 33% yield.
   ¹H-NMR (200 MHz, CDCl₃) δ (ppm): 7.71 (1H, br,s), 7.52-7.32 (5H, m), 5.91 (1H, br,s), 2.25 (3H, s), 1.52 (9H, s). MASS (EI) m/z: 273 (M⁺), 217, 200, 173, 145, 118, 77, 57.
(2) t-Butyl N-(3-cyanobenzyl)-N-(5-methyl-1-phenyl-1H-pyrazol-4-yl)carbamate (Exemplification compound number C-344, Step E5)
   To a solution of t-butyl N-(5-methyl-1-phenyl-1H-pyrazol-4-yl)carbamate (318 mg) obtained in (1) in dimethylformamide (5ml) were added sodium hydride (content: 60%, 70 mg) and 3-cyanobenzyl bromide (content: 95%, 360 mg) successively under cooling in an ice-water bath, and the resulting mixture was stirred for 3 hr at room temperature. After the reaction was completed, saturated aqueous sodium chloride solution was added to the reaction mixture, and the resulting mixture was extracted with ethyl acetate. The extract was washed with saturated aqueous sodium chloride solution and dried over anhydrous sodium sulfate. After removal of the drying agent by filtration, the solvent was evaporated *in vacuo*, and the residue obtained was purified by chromatography on a silica gel column (eluent: hexane/ethyl acetate = 7/3) to afford the title compound of the present invention (448 mg) in 99% yield. ¹H-NMR (200 MHz, CDCl₃) δ (ppm): 7.67-7.34 (10H, m), 4.74 (2H, s), 2.01 (3H, s), 1.46 (9H, s).
   MASS (EI) m/z: 388(M⁺), 332, 315, 288, 216, 186, 172, 118, 77.

### (Example 10)

### N-(3-Cyanobenzyl)-N-(5-methyl-1-phenyl-1H-pyrazol-4-yl)acetamide (Exemplification compound number C-340)

(1) 3-{(5-Methyl-1-phenyl-1H-pyrazol-4-ylamino)methyl}benzonitrile (Step K1)
   To a solution of t-butyl N-(5-methyl-1-phenyl-1H-pyrazol-4-yl)carbamate (310 mg) in dichloromethane (5 ml) was added trifluoroacetic acid (0.61 ml) with stirring under cooling in an ice-water bath, and the resulting mixture was stirred for 7 hr at room temperature. After the reaction was completed, water was added to the reaction mixture, and the resulting mixture was basified by addition of 10% aqueous sodium hydroxide solution and extracted with dichloromethane. The extract was washed with saturated aqueous sodium chloride solution and dried over anhydrous sodium sulfate. After removal of the drying agent by filtration, the solvent was evaporated *in vacuo,* and the residue obtained was purified by chromatography on a silica gel column (eluent: hexane/ethyl acetate = 4/6) to afford the title compound (198 mg) in 86% yield.
   ¹H-NMR (200 MHz, CDCl₃) δ (ppm): 7.74 (1H, s), 7.64 (1H, d, J=7Hz), 7.58 (1H, d, J=7Hz), 7.51-7.30 (6H, m), 7.28 (1H, s), 4.28 (2H, s), 3.04 (1H, br,s), 2.23 (3H, s). MASS (EI) m/z: 288(M⁺), 172, 118, 77.
(2) N-(3-Cyanobenzyl)-N-(5-methyl-1-phenyl-1H-pyrazol-4-yl)acetamide (Exemplification compound number C-340, Step B3)
   To a solution of 3-{(5-methyl-1-phenyl-1H-pyrazol-4-ylamino)methyl}benzonitrile (60.9 mg) obtained in (1) in dichloromethane (2ml) were added pyridine (34 µl) and acetyl chloride (30 µl) successively under cooling in an ice-water bath, and the resulting mixture was stirred for 20 min under the same reaction conditions. After the reaction was completed, the reaction mixture was acidified slightly by addition of dilute hydrochloric acid and extracted with ethyl acetate. The extract was washed with aqueous sodium hydrogen carbonate solution and saturated sodium chloride solution successively and dried over anhydrous sodium sulfate. After removal of the drying agent by filtration, the solvent was evaporated *in vacuo*, and the residue obtained was purified by chromatography on a silica gel column (eluent: hexane/ethyl acetate = 3/7) to afford the title compound of the present invention (62.7 mg) in 90% yield.
   ¹H-NMR (200 MHz, CDCl₃) δ (ppm): 7.61-7.35 (10H, m), 4.83 (2H, s), 1.97 (3H, s), 1.95 (3H, s).
   MASS (EI) m/z: 330(M⁺), 288, 214, 186, 172, 158, 118, 77.

### (Example 11)

### t-Butyl N-(3-cyanobenzyl)-N-(3-methyl-1-phenyl-1H-pyrazol-4-yl)carbamate (Exemplification compound number B-326)

(1) t-Butyl N-(3-methyl-1-phenyl-1H-pyrazol-4-yl)carbamate (Steps E1, E2 and E4)
   To a suspension of 3-methyl-1-phenyl-1H-pyrazol-4-carboxylic acid (228 mg) in t-butyl alcohol (5 ml) were added triethylamine (0.24 ml) and diphenylphosphoryl azide (DPPA, 0.36 ml) successively, and the resulting mixture was refluxed for 4 hr. After the reaction was completed, aqueous sodium hydrogen carbonate solution was added to the reaction mixture, and the resulting mixture was extracted with ethyl acetate. The extract was washed with saturated aqueous sodium chloride solution and dried over anhydrous sodium sulfate. After removal of the drying agent by filtration, the solvent was evaporated *in vacuo*, and the residue obtained was purified by chromatography on a silica gel column (eluent: hexane/ethyl acetate = 8/2) to afford the title compound (110 mg) in 36% yield.
   ¹H-NMR (200 MHz, CDCl₃) δ (ppm): 8.22 (1H, br,s), 7.63 (2H, d, J=8Hz), 7.40 (2H, t, J=8Hz), 7.22 (1H, d, J=8Hz), 6.08 (1H, br,s), 2.29 (3H, s), 1.54 (9H, s). MASS (EI) m/z: 273(M⁺), 217, 199, 173, 131, 104, 77, 57.
(2) t-Butyl N-(3-cyanobenzyl)-N-(3-methyl-1-phenyl-1H-pyrazol-4-yl)carbamate (Exemplification compound number B-326, Step E5)
   To a solution of t-butyl N-(3-methyl-1-phenyl-1H-pyrazol-4-yl)carbamate (107 mg) obtained in (1) in dimethylformamide (2ml) were added sodium hydride (content: 60%, 23 mg) and 3-cyanobenzyl bromide (content: 95%, 121 mg) successively under cooling in an ice-water bath, and the resulting mixture was stirred for 1 hr at room temperature. After the reaction was completed, saturated aqueous sodium chloride solution was added to the reaction mixture, and the resulting mixture was extracted with ethyl acetate. The extract was washed with saturated aqueous sodium chloride solution and dried over anhydrous sodium sulfate. After removal of the drying agent by filtration, the solvent was evaporated *in vacuo*, and the residue obtained was purified by chromatography on a silica gel column (eluent: hexane/ethyl acetate = 8/2) to afford the title compound of the present invention (147 mg) in 96% yield. ¹H-NMR (200 MHz, CDCl₃) δ (ppm): 7.60-7.37 (9H, m), 7.28-7.20 (1H, m), 4.72 (2H, s), 2.09 (3H, br,s), 1.45 (9H, s). MASS (EI) m/z: 388(M⁺), 332, 315, 288, 216, 186, 172, 116, 104, 77, 57.

### (Example 12)

### N-(3-Cyanobenzyl)-N-(3-methyl-1-phenyl-1H-pyrazol-4-yl)acetamide (Exemplification compound number B-324)

(1) 3-{(3-Methyl-1-phenyl-1H-pyrazol-4-ylamino)methyl}benzonitrile (Step K1
   To a solution of t-butyl N-(3-cyanobenzyl)-N-(3-methyl-1-phenyl-1H-pyrazol-4-yl)carbamate (136 mg) obtained in Example 11 in dichloromethane (2 ml) was added trifluoroacetic acid (0.54 ml) with stirring under cooling in an ice-water bath, and the resulting mixture was stirred for 6 hr at room temperature. After the reaction was completed, water was added to the reaction mixture, and the resulting mixture was basified by addition of 10% aqueous sodium hydroxide solution and extracted with dichloromethane. The extract was washed with saturated aqueous sodium chloride solution and dried over anhydrous sodium sulfate. After removal of the drying agent by filtration, the solvent was evaporated *in vacuo,* and the residue obtained was purified by chromatography on a silica gel column (eluent: hexane/ethyl acetate = 6/4) to afford the title compound (92.1 mg) in 92% yield.
   ¹H-NMR (200 MHz, CDCl₃) δ (ppm) : 7.73 (1H, s), 7.66 (1H, d, J=7Hz), 7.59 (1H, d, J=7Hz), 7.58-7.33 (5H, m), 7.18 (1H, s), 7.16 (1H, t, J=7Hz), 4.28 (2H, s), 3.22 (1H, br,s), 2.29 (3H, s) . MASS (EI) m/z: 288(M⁺), 172, 131, 116, 104, 77.
(2) N-(3-Cyanobenzyl)-N-(3-methyl-1-phenyl-1H-pyrazol-4-yl)acetamide (Exemplification compound number B-324, Step B3)
   To a solution of 3-{(3-methyl-1-phenyl-1H-pyrazol-4-ylamino)methyl}benzonitrile (41.0 mg) obtained in (1) in dichloromethane (2ml) were added pyridine (23 µl) and acetyl chloride (20 µl) successively under cooling in an ice-water bath, and the resulting mixture was stirred for 10 min under the same reaction conditions. After the reaction was completed, the reaction mixture was acidified slightly by addition of dilute hydrochloric acid and extracted with dichloromethane. The extract was washed with aqueous sodium hydrogen carbonate solution and saturated aqueous sodium chloride solution successively and dried over anhydrous sodium sulfate. After removal of the drying agent by filtration, the solvent was evaporated *in vacuo*, and the residue obtained was purified by chromatography on a silica gel column (eluent: hexane/ethyl acetate = 6/4) to afford the title compound of the present invention (43.9 mg) in 94% yield.
   ¹H-NMR (200 MHz, CDCl₃) δ (ppm): 7.61-7.38 (9H, m), 7.30 (1H, t, J=7Hz), 4.81 (2H, s), 2.06 (3H, s), 1.98 (3H, s).
   MASS (EI) m/z: 330(M⁺), 288, 214, 172, 158, 131, 116, 104, 77.

### (Example 13)

### t-Butyl N-(5-chloro-1-phenyl-1H-pyrazol-4-yl)-N-(3-cyanobenzyl)carbamate (Exemplification compound number E-53)

(1) t-Butyl N-(5-chloro-1-phenyl-1H-pyrazol-4-yl)carbamate (Steps E1, E2 and E4)
   To a suspension of 5-chloro-1-phenyl-1H-pyrazol-4-carboxylic acid (0.98 g) in t-butyl alcohol (20 ml) were added triethylamine (0.73 ml) and diphenylphosphoryl azide (DPPA, 1.13 ml) successively, and the resulting mixture was refluxed for 1 hr. After the reaction was completed, saturated aqueous sodium chloride solution was added to the reaction mixture, and the resulting mixture was extracted with ethyl acetate. The extract was washed with aqueous sodium hydrogen carbonate solution and saturated aqueous sodium chloride solution successively and dried over anhydrous sodium sulfate. After removal of the drying agent by filtration, the solvent was evaporated *in vacuo*, and the residue obtained was purified by chromatography on a silica gel column (eluent: from hexane/ethyl acetate (9/1) to hexane/ethyl acetate (7/3)) to afford the title compound (548 mg) in 42% yield. ¹H-NMR (200 MHz, CDCl₃) δ (ppm): 8.07 (1H, br,s), 7.59-7.36 (5H, m), 6.13 (1H, br,s), 1.54 (9H, s).
   MASS (EI) m/z: 293(M⁺), 237, 220, 193, 166, 131, 104, 77, 57.
(2) t-Butyl N-(5-chloro-1-phenyl-1H-pyrazol-4-yl)-N-(3-cyanobenzyl)carbamate (Exemplification compound number E-53, Step E5)
   To a solution of t-butyl N-(5-chloro-1-phenyl-1H-pyrazol-4-yl)carbamate (331 mg) obtained in (1) in dimethylformamide (5ml) were added sodium hydride (content: 60%, 68 mg) and 3-cyanobenzyl bromide (content: 95%, 348 mg) successively under cooling in an ice-water bath, and the resulting mixture was stirred for 2 hr at room temperature. After the reaction was completed, saturated aqueous sodium chloride solution was added to the reaction mixture, and the resulting mixture was extracted with ethyl acetate. The extract was washed with saturated aqueous sodium chloride solution and dried over anhydrous sodium sulfate. After removal of the drying agent by filtration, the solvent was evaporated *in vacuo*, and the residue obtained was purified by chromatography on a silica gel column (eluent: hexane/ethyl acetate = 8/2) to afford the title compound of the present invention (462 mg) in 100% yield. ¹H-NMR (200 MHz, CDCl₃) δ (ppm): 7.63-7.44 (10H, m), 4.81 (2H, s), 1.49 (9H, s). MASS (EI) m/z: 408(M⁺), 352, 308, 273, 236, 192, 144, 116, 104, 77, 57.

### (Example 14)

### Methyl N-(5-chloro-1-phenyl-1H-pyrazol-4-yl)-M-(3-cyanobenzyl)carbamate (Exemplification compound number E-52)

(1) 3-{(5-Chloro-l-phenyl-1H-pyrazol-4-ylamino)methyl}benzonitrile (Step K1)
   To a solution of t-butyl N-(5-chloro-1-phenyl-1H-pyrazol-4-yl)-N-(3-cyanobenzyl)carbamate (374 mg) obtained in Example 13 in dichloromethane (5 ml) was added trifluoroacetic acid (0.70 ml) with stirring under cooling in an ice-water bath, and the resulting mixture was stirred for 2 days at room temperature. After the reaction was completed, water was added to the reaction mixture, and the resulting mixture was basified by addition of 10% aqueous sodium hydroxide solution and extracted with dichloromethane. The extract was washed with saturated aqueous sodium chloride solution and dried over anhydrous sodium sulfate. After removal of the drying agent by filtration, the solvent was evaporated *in vacuo*, and the residue obtained was purified by chromatography on a silica gel column (eluent: hexane/ethyl acetate = 8/2) to afford the title compound (173 mg) in 61% yield.
   ¹H-NMR (200 MHz, CDCl₃) δ (ppm): 7.73-7.38 (10H, m), 4.35 (2H, s) .
   MASS (EI) m/z: 308(M⁺), 192, 165, 138, 116, 104, 77.
(2) Methyl N-(5-chloro-1-phenyl-1H-pyrazol-4-yl)-N-(3-cyanobenzyl)carbamate (Exemplification compound number E-52, Step B3)
   To a solution of 3-{(5-chloro-1-phenyl-1H-pyrazol-4-ylamino)methyl}benzonitrile (79.0 mg) obtained in (1) in dichloromethane (2ml) were added pyridine (41 µl) and methyl chlorocarbonate (40 µl) successively under cooling in an ice-water bath, and the resulting mixture was stirred for 10 min under the same reaction conditions. After the reaction was completed, the reaction mixture was acidified slightly with dilute hydrochloric acid and extracted with dichloromethane. The extract was washed with aqueous sodium hydrogen carbonate solution and saturated aqueous sodium chloride solution successively and dried over anhydrous sodium sulfate. After removal of the drying agent by filtration, the solvent was evaporated *in vacuo,* and the residue obtained was purified by chromatography on a silica gel column (eluent: hexane/ethyl acetate = 6/4) to afford the title compound of the present invention (87.2 mg) in 93% yield.
   ¹H-NMR (200 MHz, CDCl₃) δ (ppm): 7.62-7.40 (10H, m), 4.81 (2H, s), 3.78 (3H, s).
   MASS (EI) m/z: 366(M⁺), 307, 250, 178, 138, 131, 116, 104, 77, 59.

### (Example 15)

### t-Butyl N-(5-cyano-1-phenyl-1H-pyrazol-4-yl)-N-(3-cyanobenzyl)carbamate (Exemplification compound number E-56)

(1) t-Butyl N-(5-cyano-1-phenyl-1H-pyrazol-4-yl)carbamate (Steps E1, E2 and E4)
   To a suspension of 5-cyano-1-phenyl-1H-pyrazol-4-carboxylic acid (485 mg) in t-butyl alcohol (10 ml) were added triethylamine (0.38 ml) and diphenylphosphoryl azide (DPPA, 0.59 ml) successively, and the resulting mixture was refluxed for 3 hr. After the reaction was completed, aqueous sodium hydrogen carbonate solution was added to the reaction mixture, and the resulting mixture was extracted with ethyl acetate. The extract was washed with saturated aqueous sodium chloride solution and dried over anhydrous sodium sulfate. After removal of the drying agent by filtration, the solvent was evaporated *in vacuo*, and the residue obtained was purified by chromatography on a silica gel column (eluent: hexane/ethyl acetate = 8/2) to afford the title compound (336 mg) in 52% yield.
   ¹H-NMR (200 MHz, CDCl₃) δ (ppm): 8.19 (1H, br,s), 7.72-7.65 (2H, m), 7.56-7.36 (3H, m), 6.64 (1H, br,s), 1.55 (9H, s).
   MASS (EI) m/z: 284(M⁺), 228, 211, 184, 157, 129, 104, 77, 57.
(2) t-Butyl N-(5-cyano-1-phenyl-1H-pyrazol-4-yl)-N-(3-cyanobenzyl)carbamate (Exemplification compound number E-56, Step E5)
   To a solution of t-butyl N-(5-cyano-1-phenyl-1H-pyrazol-4-yl)carbamate (239 mg) obtained in (1) in dimethylformamide (5ml) were added sodium hydride (content: 60%, 50 mg) and 3-cyanobenzyl bromide (content: 95%, 260 mg) successively under cooling in an ice-water bath, and the resulting mixture was stirred for 2 hr at room temperature. After the reaction was completed, saturated aqueous sodium chloride solution was added to the reaction mixture, and the resulting mixture was extracted with ethyl acetate. The extract was washed with saturated aqueous sodium chloride solution and dried over anhydrous sodium sulfate. After removal of the drying agent by filtration, the solvent was evaporated *in vacuo*, and the residue obtained was purified by chromatography on a silica gel column (eluent: hexane/ethyl acetate = 8/2) to afford the title compound of the present invention (282 mg) in 84% yield. ¹H-NMR (200 MHz, CDCl₃) δ (ppm): 7.68-7.42 (10H, m), 4.93 (2H, s), 1.52 (9H, s).
   MASS (EI) m/z: 399(M⁺), 343, 326, 299, 197, 183, 170, 129, 116, 77, 57.

### (Example 16)

### Methyl N-(5-cyano-1-phenyl-1H-pyrazol-4-yl)-N-(3-cyanobenzyl)carbamate (Exemplification compound number E-55)

(1) 4-(3-Cyanobenzylamino)-1-phenyl-1H-pyrazol-5-carbonitrile (Step K)
   To a solution of t-butyl N-(5-cyano-1-phenyl-1H-pyrazol-4-yl)-N-(3-cyanobenzyl)carbamate (226 mg) obtained in Example 15 in dichloromethane (3 ml) was added trifluoroacetic acid (0.87 ml) with stirring under cooling in an ice-water bath, and the resulting mixture was stirred for 4 hr at room temperature. After the reaction was completed, water was added to the reaction mixture, and the resulting mixture was basified by addition of 10% aqueous sodium hydroxide solution and extracted with dichloromethane. The extract was washed with saturated aqueous sodium chloride solution and dried over anhydrous sodium sulfate. After removal of the drying agent by filtration, the solvent was evaporated *in vacuo*, and the residue obtained was purified by chromatography on a silica gel column (eluent: hexane/ethyl acetate = 7/3) to afford the title compound (135 mg) in 80% yield.
   ¹H-NMR (200 MHz, CDCl₃) δ (ppm): 7.73-7.38 (10H, m), 4.35 (2H, s). MASS (EI) m/z: 299(M⁺), 183, 170, 129, 116, 77.
(2) Methyl N-(5-cyano-1-phenyl-1H-pyrazol-4-yl)-N-(3-cyanobenzyl)carbamate (Exemplification compound number E-55, Step B3)
   To a solution of 4-(3-cyanobenzylamino)-1-phenyl-1H-pyrazol-5-carbonitrile (70.0 mg) obtained in (1) in dichloromethane (2ml) were added pyridine (38 µl) and methyl chlorocarbonate (36 µl) successively under cooling in an ice-water bath, and the resulting mixture was stirred for 10 min under the same reaction conditions. After the reaction was completed, the reaction mixture was acidified slightly with dilute hydrochloric acid and extracted with dichloromethane. The extract was washed with aqueous sodium hydrogen carbonate solution and saturated aqueous sodium chloride solution successively and dried over anhydrous sodium sulfate. After removal of the drying agent by filtration, the solvent was evaporated *in vacuo*, and the residue obtained was purified by chromatography on a silica gel column (eluent: hexane/ethyl acetate = 7/3) to afford the title compound of the present invention (68.4 mg) in 82% yield.
   ¹H-NMR (200 MHz, CDCl₃) δ (ppm): 7.70-7.44 (10H, m), 4.96 (2H, s), 3.86 (3H, s).
   MASS (EI) m/z: 357(M⁺), 298, 241, 210, 184, 169, 116, 77, 59.

### (Example 17)

### t-Butyl N-(3-cyanobenzyl)-N-(1-phenyl-1H-pyrazol-4-yl)carbamate (Exemplification compound number A-69)

(1) t-Butyl N-(1-phenyl-1H-pyrazol-4-yl)carbamate (Steps E1, E2 and E4)
   To a suspension of 1-phenyl-1H-pyrazol-4-carboxylic acid (566 mg) in t-butyl alcohol (15 ml) were added triethylamine (0.50 ml) and diphenylphosphoryl azide (DPPA, 0.78 ml) successively, and the resulting mixture was refluxed for 4 hr. After the reaction was completed, saturated aqueous sodium chloride solution was added to the reaction mixture, and the resulting mixture was extracted with ethyl acetate. The extract was washed with aqueous sodium hydrogen carbonate solution and saturated aqueous sodium chloride solution successively and dried over anhydrous sodium sulfate. After removal of the drying agent by filtration, the solvent was evaporated *in vacuo*, and the residue obtained was purified by chromatography on a silica gel column (eluent: hexane/ethyl acetate = 8/2) to afford the title compound (695 mg) in 90% yield.
   ¹H-NMR (200 MHz, CDCl₃) δ (ppm): 8.22 (1H, br,s), 7.64 (2H, d, J=8Hz), 7.54 (1H, s), 7.46-7.36 (2H, m), 7.29-7.21 (1H, m), 6.38 (1H, br,s), 1.53 (9H, s).
(2) t-Butyl N-(3-cyanobenzyl)-N-(1-phenyl-1H-pyrazol-4-yl)carbamate (Exemplification compound number A-69, Step E5)
   To a solution of t-butyl N-(1-phenyl-1H-pyrazol-4-yl)carbamate (400 mg) obtained in (1) in dimethylformamide (5ml) were added sodium hydride (content: 60%, 74 mg) and 3-cyanobenzyl bromide (content: 95%, 382 mg) successively under cooling in an ice-water bath, and the resulting mixture was stirred for 30 min at 0°C. After the reaction was completed, saturated aqueous sodium chloride solution was added to the reaction mixture, and the resulting mixture was extracted with ethyl acetate. The extract was washed with saturated aqueous sodium chloride solution and dried over anhydrous sodium sulfate. After removal of the drying agent by filtration, the solvent was evaporated *in vacuo,* and the residue obtained was purified by chromatography on a silica gel column (eluent: hexane/ethyl acetate = 8/2) to afford the title compound of the present invention (434 mg) in 67% yield.
   ¹H-NMR (200 MHz, CDCl₃) δ (ppm): 7.64-7.23 (11H, m), 4.90 (2H, s), 1.52 (9H, s).
   MASS (EI) m/z: 374(M⁺), 318, 274, 202, 158, 131, 116, 104, 77, 57.

### (Example 18)

### N-(3-Cyanobenzyl)-N-(1-phenyl-1H-pyrazol-4-yl)acetamide (Exemplification compound number A-68)

(1) 3-{(1-Phenyl-1H-pyrazol-4-ylamino)methyl}benzonitrile (Step K1)
   To a solution of t-butyl N-(1-phenyl-1H-pyrazol-4-yl)carbamate (331 mg) obtained in Example 17 (1) in dichloromethane (5 ml) was added trifluoroacetic acid (0.68 ml) with stirring under cooling in an ice-water bath, and the resulting mixture was stirred for 3 hr at room temperature. After the reaction was completed, water was added to the reaction mixture, and the resulting mixture was basified by addition of 10% aqueous sodium hydroxide solution and extracted with dichloromethane. The extract was washed with saturated aqueous sodium chloride solution and dried over anhydrous sodium sulfate. After removal of the drying agent by filtration, the solvent was evaporated *in vacuo*, and the residue obtained was purified by chromatography on a silica gel column (eluent: hexane/ethyl acetate = 6/4) to afford the title compound (218 mg) in 90% yield. ¹H-NMR (200 MHz, CDCl₃) δ (ppm): 7.72-7.18 (11H, m), 4.29 (2H, s), 3.58 (1H, br,s).
   MASS (EI) m/z: 274(M⁺), 158, 131, 116, 104, 77.
(2) N-(3-Cyanobenzyl)-N-(1-phenyl-1H-pyrazol-4-yl)acetamide (Exemplification compound number A-68, Step B3)
   To a solution of 3-{(1-phenyl-1H-pyrazol-4-ylamino)methyl}benzonitrile (115 mg) obtained in (1) in dichloromethane (2ml) were added pyridine (120 µl) and acetyl chloride (60 µl) successively under cooling in an ice-water bath, and the resulting mixture was stirred for 20 min under the same reaction conditions. After the reaction was completed, saturated aqueous sodium chloride solution was added to the reaction mixture, and the resulting mixture was extracted with dichloromethane. The extract was washed with saturated aqueous sodium chloride solution and dried over anhydrous sodium sulfate. After removal of the drying agent by filtration, the solvent was evaporated *in vacuo*, and the residue obtained was purified by chromatography on a silica gel column (eluent: hexane/ethyl acetate = 5/5) to afford the title compound (127 mg) in 96% yield.
   ¹H-NMR (200 MHz, CDCl₃) δ (ppm): 7.71-7.28 (11H, m), 4.87 (2H, s), 2.07 (3H, s).
MASS (EI) m/z: 316(M⁺), 274, 200, 158, 116, 104, 77.

### (Example 19)

### Methyl (3-cyanobenzyl) (1-cyclobutylmethyl-3,5-dimethyl-1H-pyrazol-4-yl)carbamate (Exemplification compound number D-77)

(1) 4-Methoxycarbonylamino-3,5-dimethyl-pyrazol-1-carboxylic acid methyl ester (Step F1)
   To a solution of 4-amino-3,5-dimethyl-1H-pyrazole (37.60 g, 338.30 mmol) in dichloromethane (600 ml) was added pyridine (69.44 ml, 877.88 mmol) at 0°C with stirring. Then, methyl chlorocarbonate (68.0 ml, 880.04 mmol) was added dropwise to the reaction mixture at 0°C over 15 minutes, and the resulting mixture was stirred for 2 hr at room temperature. After stirring, to the reaction mixture were further added pyridine (30.00 ml, 370.92 mmol) and methyl chlorocarbonate (20 ml, 258.83 mmol), and the resulting mixture was stirred for 2 hr and kept standing overnight. After standing, to the reaction mixture were further added pyridine (10.00 ml, 123.64 mmol) and methyl chlorocarbonate (10.00 ml, 129.41 mmol), and the resulting mixture was stirred for 1.5 hr. After the reaction was completed, the reaction mixture was adjusted to pH 12 with saturated aqueous sodium hydrogen carbonate solution and extracted with dichloromethane. The extract was dried over anhydrous sodium sulfate, and after removal of the drying agent by filtration, the filtrate was concentrated *in vacuo*. The residue obtained was dissolved in toluene, and pyridine remaining in the residue was removed by azeotroping with toluene in vacuo to afford the title compound (71.88 g, 315.99 mmol) in 89% yield.
   ¹H-NMR (270 MHz, CDCl₃) δ (ppm): 2.22(s, 1H), 2.45(s, 3H), 3.76(brs, 3H), 4.03(s, 3H), 5.81(brs, 1H). MASS (EI) m/z: 227(M⁺), 195, 169, 150, 136, 124, 82, 67, 59,56.
(2) Methyl (3-cyanobenzyl)-(3,5-dimethyl-1H-pyrazol-4-yl)carbamate (Steps F2 and F3)
   To a solution of 4-methoxycarbonylamino-3,5-dimethyl-pyrazol-1-carboxylic acid methyl ester (71.8 g, 316.0 mmol) obtained in (1) in dimethylformamide (DMF, 200 ml) was added dropwise 60% sodium hydride (8.46 g, 352.5 mmol) with stirring at 0°C over 10 minutes, and the resulting mixture was stirred for 30 min at room temperature. After cooling to 0°C once again, to the reaction mixture was added portionwise 3-cyanobenzyl bromide (67.55 g, 344.5 mmol) over 15 minutes, and the resulting mixture was stirred for 4 hr at room temperature. After the reaction was completed, water (500 ml) was added to the reaction mixture, and the resulting mixture was extracted with a mixed solvent of ethyl acetate and hexane (3:2, v/v). The extract was washed with water and saturated aqueous sodium chloride solution successively and dried over anhydrous sodium sulfate. After removal of the drying agent by filtration, the filtrate was concentrated *in vacuo* to afford 4-[(3-cyanobenzyl)-methoxycarbonyl-amino]-3,5-dimethylpyrazol-1-carboxylic acid methyl ester. Then, to a solution of 4-[(3-cyanobenzyl)-methoxycarbonyl-amino]-3,5-dimethylpyrazol-1-carboxylic acid methyl ester obtained above in methanol (500 ml) was added dilute hydrochloric acid, which was prepared from concentrated hydrochloric acid (100 ml) and water (300 ml), at 0°C with stirring, and the resulting mixture was stirred for 2 hr at room temperature. After stirring, to the reaction mixture was added dilute hydrochloric acid prepared from concentrated hydrochloric acid (100 ml) and water (200 ml), with stirring, and the resulting mixture was stirred for 3 hr and then kept standing for 10 days. After the reaction was completed, the reaction mixture was adjusted to pH 8 with aqueous sodium hydroxide solution and extracted with ethyl acetate. The extract was dried over anhydrous sodium sulfate, and after removal of the drying agent by filtration, the filtrate was concentrated *in vacuo*. The residue obtained was purified by chromatography on a silica gel column (eluent: elution was performed using hexane, ethyl acetate/hexane (1/2) and ethyl acetate successively) to afford a mixture (36.0 g) of 4-[(3-cyanobenzyl)-methoxycarbonyl-amino]-3,5-dimethylpyrazole-1-carboxylic acid methyl ester derivatives and the title compound (53.3 g, 187.5 mmol, 59% yield). ¹H-NMR (270 MHz, CDCl₃) δ (ppm): 1.87(s, 6H), 3.69(s, 3H), 4.66(s, 2H), 7.35-7.65(m, 4H), 10.00(brs, 1H). MASS (EI) m/z: 284(M⁺), 253, 225, 168, 136, 116, 96, 89, 59.
(3) Methyl (3-cyanobenzyl)(1-cyclobutylmethyl-3,5-dimethyl-1H-pyrazol-4-yl)carbamate (Exemplification compound number D-77, Step F4)
   To a solution of methyl (3-cyanobenzyl)-(3,5-dimethyl-1H-pyrazol-4-yl)carbamate (50.7 mg, 0.1783 mmol) obtained in (2) in DMF (1 ml) was added 60% sodium hydride (7.8 mg, 0.195 mmol) at 0°C with stirring, and the resulting mixture was stirred for 5 min. Then, to the reaction mixture was added dropwise cyclobutylmethyl bromide (24.8 µl, 0.2206 mmol), and the resulting mixture was stirred for 3 hr at the same temperature. After the reaction was completed, water was added to the reaction mixture, and the resulting mixture was extracted with ethyl acetate. The extract was dried over anhydrous sodium sulfate, and after removal of the drying agent by filtration, the filtrate was concentrated *in vacuo*. The residue obtained was purified by preparative TLC over silica gel plate (development solvent: ethyl acetate) to afford the title compound of the present invention (51.9 mg, 0.1473 mmol) in 83% yield.
   ¹H-NMR (200 MHz, CDCl₃) δ (ppm): 1.70 (s, 3H), 1.92(s, 3H), 1.60-2.10(m, 6H), 2.74(brhpt, 2H, J=7.3Hz), 3.67(s, 3H), 3.90(dd, 2H, J=7.3Hz, 3.7Hz), 4.42(d, 1H, J=14.3Hz), 4.86(d, 1H, J=14.3Hz), 7.30-7.65(m, 4H).
   MASS (EI) m/z: 352(M⁺), 337, 324, 297, 284, 236, 208, 182, 168, 150, 136, 116, 96, 83, 69, 55.

### (Example 20)

### Methyl (3-cyanobenzyl)[3,5-dimethyl-1-(2-oxopropyl)-1H-pyrazol-4-yl]carbamate (Exemplification compound number D-153, Step F4)

To a solution of methyl (3-cyanobenzyl)-(3,5-dimethyl-1H-pyrazol-4-yl)carbamate (5.00 g, 17.59 mmol) obtained in step (1) of Example 19 in acetonitrile (22 ml) were added potassium carbonate (2.67 g, 19.32 mmol) and 96% chloroacetone (2.51 ml, 29.94 mmol) at room temperature, and the resulting mixture was stirred for 1.5 hr. Then, to the reaction mixture was added sodium iodide (30 mg), and the resulting mixture was stirred for 2 hr. Then, further 96% chloroacetone (2.51 ml, 29.94 mmol) was added, and the resulting mixture was stirred for 50 min, and after further addition of 96% chloroacetone (2.51 ml, 29.94 mmol), the resulting mixture was stirred for 2 hr. After the reaction was completed, water was added to the reaction mixture, and the resulting mixture was extracted with ethyl acetate. The extract was washed with aqueous sodium chloride solution and dried over anhydrous sodium sulfate, and after removal of the drying agent by filtration, the filtrate was concentrated *in vacuo*. The residue obtained was purified by chromatography on a silica gel column (eluent: elution was performed using hexane, ethyl acetate/hexane (1/100), ethyl acetate/hexane (2/1), ethyl acetate and methanol/ethyl acetate (1/10) successively) to afford the title compound of the present invention (5.22 g, 15.57 mmol) in 87% yield. ¹H-NMR (200 MHz, CDCl₃) δ (ppm): 1.66(brs, 3H), 1.78(brs, 3H), 2.10(s, 3H), 3.69(brs, 3H), 4.51(brd, 1H, J=13.9Hz), 4.66(brd, 1H, J=17.9Hz), 4.77(brd, 1H, J=17.9Hz), 4.83(brd, 1H, J=13.9Hz), 7.35-7.65(m, 4H).
MASS (EI) m/z: 340(M⁺), 297, 281, 224, 150, 116, 89, 81, 55.

### (Example 21)

### Methyl (3-cyanobenzyl)[1-(2-hydroxypropyl)-3,5-dimethyl-1H-pyrazol-4-yl]carbamate (Exemplification compound number D-138, Step J)

To a solution of methyl (3-cyanobenzyl)[3,5-dimethyl-1-(2-oxopropyl)-1H-pyrazol-4-yl]carbamate (472.0 mg, 1.387 mmol) obtained in Example 20 in methanol (8 ml) was added sodium tetrahydroborate (59.2 mg, 1.549 mmol) at room temperature, and the resulting mixture was stirred for 30 min. After the reaction was completed, water was added to the reaction mixture, and the resulting mixture was extracted with ethyl acetate. The extract was washed with water and aqueous sodium chloride solution successively and dried over anhydrous sodium sulfate, and after removal of the drying agent by filtration, the filtrate was concentrated *in vacuo*. The residue obtained was purified by chromatography on a silica gel column (eluent: elution was performed using ethyl acetate/hexane (1/10), ethyl acetate/hexane (1/3) and ethyl acetate successively) to afford the title compound of the present invention (400.2 mg, 1.169 mmol) in 84% yield.
¹H-NMR (200 MHz, CDCl₃) δ (ppm): [1.18(d, J=6.2Hz) and 1.20(d, J=6.2Hz), 3H], 1.73(s) and 1.75(s), 3H], 1.91(s, 3H), 3.68(s, 3H), 3.6-4.0(m, 2H), 4.18(brm, 1H), [4.44(d, J=14.0Hz) and 4.53(d, J=14.0Hz), 1H], [4.77(d, J=14.0Hz) and 4.83(d, J=14.0Hz), 1H], 7.35-764 (m, 4H).
MASS (EI) m/z: 342(M⁺), 327, 297, 284, 257, 226, 194, 182, 168, 150, 141, 116, 55.

### (Example 22)

### Methyl (3-cyanobenzyl)[1-(2-fluoropropyl)-3,5-dimethyl-1H-pyrazol-4-yl]carbamate (Exemplification compound number D-115, Step J1)

To a solution of N-(3-cyanobenzyl)-N-[1-(2-hydroxypropyl)-3,5-dimethyl-1H-pyrazol-4-yl]acetamide (54.7 mg, 0.160 mmol) obtained in Example 21 in dichloromethane (2 ml) was added (dimethylamino)sulfur trifluoride (42.2 µl, 0.320 mmol) at -17°C, and the resulting mixture was stirred for 75 min at the same temperature. After the reaction was completed, ice water was added to the reaction mixture, and the resulting mixture was extracted with ethyl acetate. The extract was washed with water and aqueous sodium chloride solution successively and dried over anhydrous sodium sulfate, and after removal of the drying agent by filtration, the filtrate was concentrated *in vacuo.* The residue obtained was purified by preparative TLC (development solvent: ethyl acetate) to afford the title compound of the present invention (31.0 mg, 0.090 mmol) in 56% yield.
¹H-NMR (200 MHz, CDCl₃) δ (ppm): [1.29(dd, J=5.9Hz, 8.8Hz) and 1.41(dd, J=5.9Hz, 8.8Hz), 3H], [1.75(s) and 1.78(s), 3H], 1.89(s, 3H), 3.68(s, 3H), 3.90-4.16(m, 2H), [4.50(d, J=14.0Hz) and 4.51(d, J=14.0Hz), 1H], 4.79(d, J=14.0Hz, 1H), [4.88(m), and 5.09(m), 1H], 7.30-7.61(m, 4H).
MASS (EI) m/z: 344(M⁺), 297, 228, 196, 116, 102, 81, 61.

### (Example 23)

### Methyl [1-(2-chloropropyl)-3,5-dimethyl-1H-pyrazol-4-yl](3-cyanobenzyl)carbamate (Exemplification compound number D-117, Step J1)

To a solution of N-(3-cyanobenzyl)-N-[1-(2-hydroxypropyl)-3,5-dimethyl-1H-pyrazol-4-yl]acetamide (51.8 mg, 0.151 mmol) obtained in Example 21 in toluene (2 ml) was added thionyl chloride (122 µl, 1.512 mmol) at 0°C, and the resulting mixture was stirred successively for 5 hr at room temperature and for 30 min at 100°C. After the reaction was completed, saturated sodium hydrogen carbonate solution was added to the reaction mixture, and the resulting mixture was extracted with ethyl acetate. The extract was washed with water and aqueous sodium chloride solution successively and dried over anhydrous sodium sulfate, and after removal of the drying agent by filtration, the filtrate was concentrated *in vacuo*. The residue obtained was purified by preparative TLC (development solvent: ethyl acetate) to afford the title compound of the present invention (33.1 mg, 0.092 mmol) in 61% yield.
¹H-NMR (200 MHz, CDCl₃) δ (ppm): [1.46(d, J=6.6Hz) and 1.53(d, J=6.5Hz), 3H], [1.75(s) and 1.78(s), 3H], 1.91(s, 3H), 3.68(s, 3H), 3.85-4.51(m, 4H), 4.85(d, J=14.0Hz), 7.30-7.60(m,4H). MASS (EI) m/z: 360(M⁺),325, 297, 284, 244, 208, 168, 149, 136, 116, 96, 81, 55.

### (Example 24)

### 2-{4-[(3-Cyanobenzyl)(methoxycarbonyl)amino]-3,5-dimethyl-1H-pyrazol-1-yl}-1-methylethyl acetate (Exemplification compound number D-148, Step J1)

To a solution of N-(3-cyanobenzyl)-N-[1-(2-hydroxypropyl)-3,5-dimethyl-1H-pyrazol-4-yl]acetamide (59.0 mg, 0.172 mmol) obtained in Example 21 in dichloromethane (2 ml) were added N,N-dimethylaminopyridine (23 mg, 0.188 mmol) and triethylamine (53 µl, 0.380 mmol) at 0°C under stirring, and then acetic anhydride (81 µl, 0.858 mmol) was added at the same temperature, and the resulting mixture was stirred for 2 hr at room temperature. After the reaction was completed, saturated sodium hydrogen carbonate solution was added to the reaction mixture, and the resulting mixture was extracted with ethyl acetate. The extract was washed with water and aqueous sodium chloride solution successively and dried over anhydrous sodium sulfate, and after removal of the drying agent by filtration, the filtrate was concentrated *in vacuo*. The residue obtained was purified by preparative TLC (development solvent: ethyl acetate/hexane = 1/2) to afford the title compound of the present invention (57.7 mg, 0.150 mmol) in 87% yield. ¹H-NMR (200 MHz, CDCl₃) δ (ppm) : 1.25(d, J=6.2Hz, 3H), 1.82(s, 3H), [1.82(s) and 1.88(s), 3H], 2.06(s, 3H), 3.67(brs, 3H), 4.01(d. J=6.2Hz, 2H), [4.49(d, J=14.0Hz) and 4.70(d, J=14.0Hz), 1H], [4.56(d, J=14.0Hz) and 4.79(d, J=14.0Hz), 1H], 5.19(dq, J=6.2Hz, 6.2Hz), 7.33-7.61(m, 4H).
MASS (EI) m/z: 384(M⁺), 353, 341, 324, 297, 284, 268, 226, 208, 182, 168, 150, 136, 116, 101, 89, 82, 55.

### (Example 25)

### Methyl (3-cyanobenzyl)[1-(2-methoxypropyl)-3,5-dimethyl-1H-pyrazol-4-yl]carbamate (Exemplification compound number D-140, Step J1)

To a solution of N-(3-cyanobenzyl)-N-[1-(2-hydroxypropyl)-3,5-dimethyl-1H-pyrazol-4-yl]acetamide (49.0 mg, 0.143 mmol) obtained in Example 21 in dichloroethane (3 ml) were added silver oxide (1658 mg, 7.15 mmol) and methyl iodide (0.45 ml, 7.15 mmol) with stirring at room temperature, and the resulting mixture was stirred for 3 hr. After the reaction was completed, the reaction mixture was filtered using Celite, and ice-water was added to the filtrate, and the resulting mixture was extracted with ethyl acetate. The extract was washed with water and aqueous sodium chloride solution successively and dried over anhydrous sodium sulfate, and after removal of the drying agent by filtration, the filtrate was concentrated *in vacuo.* The residue obtained was purified by preparative TLC (development solvent: ethyl acetate) to afford the title compound of the present invention (30.3 mg, 0.085 mmol) in 59% yield.
¹H-NMR (200 MHz, CDCl₃) δ (ppm): [1.08(d, J=6.3Hz) and 1.13(d, J=5.9Hz), 3H], [1.76(s) and 1.78(s), 3H], [1.89(s) and 1.91(s), 3H], 3.20(s, 3H), 3.68(s, 3H), 3.55-3.95(m, 1H), 3.86Z(d, J=5.2Hz, 2H), [4.48(d, J=14.0Hz) and 4.51(d, J=14.0Hz), 1H], [4.81(d, J=14.0Hz) and 4.83(d, J=14.0Hz), 1H], 7.40(t, J=8.5Hz, 1H), 7.45-7.53 (m, 3H).
MASS (EI) m/z: 356(M⁺), 325, 297, 284, 240, 182, 168, 150, 141, 116, 96, 81, 73, 59.

### (Example 26)

### Methyl (3-cyanobenzyl){1-[2-(hydroxyimino)propyl]-3,5-dimethyl-1H-pyrazol-4-yl}carbamate (exemplification compound number D-160, Step J)

In a mixture of water (0.5 ml) and dioxane (1 ml) was dissolved 97% hydroxylamine hydrochloride (85.0 mg, 1.185 mmol), and a dioxane (2.5 ml) solution of methyl (3-cyanobenzyl) [3,5-dimethyl-1-(2-oxopropyl)-1H-pyrazol-4-yl]carbamate (193.6 mg, 0.569 mmol) obtained in Example 20 was added thereto at room temperature followed by stirring for 3 hours. After the end of the reaction, water was poured into the reaction mixture, and then the resulting solution was extracted with ethyl acetate. The organic layer was washed with water and then with aqueous sodium chloride solution, dried over anhydrous sodium sulfate, and after the drying agent was removed by filtration, the solution was concentrated. The residue obtained was purified by chromatography on a silica gel column (elution solvent: hexane only - ethyl acetate / hexane = 1 / 2 - ethyl acetate only) to afford the title compound of the present invention (166.4 mg, 0.468 mmol, 82% yield).
¹H-NMR (200 MHz, CDCl₃) δ (ppm): 1.68(s, 3H), 1.79(s, 3H), 1.93(s, 3H), 3.67(s, 3H), 4.43(d, J=14.0Hz, 1H), 4.63(d, J=4.0Hz, 2H), 4.86(d, J=14.0Hz, 1H), 7.34-7.61(m, 4H), 7.76(brs, 1H).
MASS (EI) m/z: 355(M⁺), 338, 297, 284, 239, 224, 182, 168, 149, 136, 127, 116, 72, 55.

### (Example 27)

### Methyl (3-cyanobenzyl) [1-(2-hydroxy-2-methylpropyl)-3,5-dimethyl-1H-pyrazol-4-yl] carbamate (exemplification compound number D-142, Step J1)

Methyl (3-cyanobenzyl)[3,5-dimethyl-1-(2-oxopropyl)-1H-pyrazol-4-yl]carbamate (48.0 mg, 0.148 mmol) obtained in Example 20 was dissolved in dichloromethane (3 ml), and a solution of 3M magnesium bromide in diethylether (0.8 ml, 2.4 mmol) was added thereto at 0°C, and the resulting mixture was stirred for 1 hour at 0°C and then for 5 hours at room temperature. After the reaction was completed, the reaction mixture was poured into saturated aqueous ammonium chloride solution, and then extracted with ethyl acetate. The organic layer was washed with water and then with aqueous sodium chloride solution, dried over anhydrous sodium sulfate, and after the drying agent was removed by filtration, the solution was concentrated. The residue obtained was purified by preparative thin layer chromatography (developing solvent: ethyl acetate) to afford the title compound of the present invention (24.9 mg, 0.073 mmol, 50% yield).
¹H-NMR (200 MHz, CDCl₃) δ (ppm): 1.06(s, 3H), 1.14(s, 3H), 1.69(s, 3H), 1.94(s, 3H), 3.68(s, 3H), 3.80(s, 1H), 3.82(s, 1H), 4.43(d, J=14.0Hz, 1H), 4.46(s, 1H), 4.89(d, J=14.0Hz, 1H), 7.32-7.61(m, 4H).
MASS (EI) m/z: 356(M⁺), 341, 298, 257, 222, 182, 150, 141, 116, 109, 55.

### (Example 28)

### Methyl (3-cyanobenzyl)(3,5-dimethyl-1-{2-methyl-2-[(trimethylsilyl)oxy]propyl}-1H-pyrazol-4-yl)carbamate (exemplification compound number D-146, Step J1)

Methyl (3-cyanobenzyl)[1-(2-hydroxy-2-methylpropyl)-3,5-dimethyl-1H-pyrazol-4-yl]carbamate (45.5 mg, 0.128 mmol) obtained in Example 27 and imidazole (86.9 mg, 1.276 mmol) were dissolved in DMF (2 ml), and chlorotrimethylsilane (81 µl, 6.382 mmol) was added thereto at 0°C followed by stirring for 2 hours at the same temperature. After the reaction was completed, water was poured into the reaction mixture, and then the resulting solution was extracted with ethyl acetate. The organic layer was washed with water and then with aqueous sodium chloride solution, dried over anhydrous sodium sulfate, and after the drying agent was removed by filtration, the solution was concentrated. The residue obtained was purified by preparative thin layer chromatography (developing solvent: ethyl acetate / hexane = 2 / 1) to afford the title compound of the present invention (44.1 mg, 0.103 mmol, 81% yield). ¹H-NMR (200 MHz, CDCl₃) δ (ppm): 0.00(s, 9H), 1.21(s, 3H), 1.28(s, 3H), 1.82(s, 3H), 1.84(s, 3H), 3.65(s, 3H), 3.81(s, 2H), 4.58(d, J=14.0Hz, 1H), 4.69(d, J=14.0Hz, 1H), 7.37(t, J=7.7Hz, 1H), 7.43-7.58(m, 3H).
MASS (EI) m/z: 428(M⁺), 413, 370, 298, 150, 131, 116, 73, 55.

### (Example 29)

### N-(3-cyanobenzyl)-N-[1-(3-fluorobenzyl)-3,5-dimethyl-1H-pyrazol-4-yl]acetamide (exemplification compound number D-101)

(1) 1-(t-Butoxycarbonyl)-3,5-dimethyl-4-nitroso-1H-pyrazole (Step G1)
   3,5-Dimethyl-4-nitroso-1H-pyrazole (11.6 g) was dissolved in tetrahydrofuran (120 ml), and di-t-butyldicarbonate (content 97%, 23.8 ml) and 10% aqueous sodium hydroxide solution (40 ml) were added thereto in an ice-water bath followed by stirring for 40 minutes at room temperature. After the reaction was completed, this reaction solution was diluted with water, and extracted with dichloromethane. The extract was washed with saturated aqueous sodium chloride solution, and dried over anhydrous sodium sulfate. After the drying agent was removed by filtration, the solvent was evaporated under reduced pressure to afford the desired title compound (23.6 g). This compound was utilized in the next reaction without further purification. ¹H-NMR (200 MHz, CDCl₃) δ (ppm): 3.38 (3H, s), 2.20 (3H, s), 1.71 (9H, s).
   MASS (EI) m/z: 225(M⁺), 210, 166, 125, 57.
(2) N-{1-(t-Butoxycarbonyl)-3,5-dimethyl-1H-pyrazole-4-yl}acetamide (Step G2 and Step G3)
   1-(t-Butoxycarbonyl)-3,5-dimethyl-4-nitroso-lH-pyrazole (23.6 g) obtained in (1) was dissolved in a mixture of ethyl acetate (220 ml) and acetic anhydride (14.3 ml), and 10% palladium-charcoal catalyst (2.0 g) was added thereto, followed by stirring for 4 hours at room temperature in a hydrogen atmosphere. After the reaction was completed, the insoluble materials were removed using Celite, and the filtrate was washed with aqueous sodium hydrogencarbonate solution and then with saturated aqueous sodium chloride solution, and dried over anhydrous sodium sulfate. After the drying agent was removed by filtration, the solvent was evaporated *in vacuo*, and the residue obtained was purified by chromatography on a silica gel column (elution solvent: ethyl acetate) to afford the desired title compound (10.5 g, ca. 41% yield).
   ¹H-NMR (200 MHz, CDCl₃) δ (ppm) : [6.66 (br,s) & 6.43 (br,s), 1H], [2.43 (s) & 2.37 (s), 3H], [2.23 (s) & 2.18 (s), 3H], [2.18 (s) & 1.81 (s), 3H], [1.66 (s) & 1.63 (s), 9H].
   MASS (EI) m/z: 253(M⁺), 194, 153, 111, 70, 57.
(3) N-{1-(t-Butoxycarbonyl)-3,5-dimethyl-1H-pyrazol-4-yl}-N-(3-cyanobenzyl)acetamide (Step G4)
   N-{1-(t-Butoxycarbonyl)-3,5-dimethyl-1H-pyrazol-4-yl}acetamide (10.5 g) obtained in (2) was dissolved in dimethylformamide (100 ml), and 60% sodium hydride (1.82 g) and 95% 3-cyanobenzyl bromide (9.41 g) were successively added thereto in an ice-water bath followed by stirring for 45 minutes. After the reaction was completed, saturated aqueous sodium chloride solution was added thereto, and the resulting solution was extracted with ethyl acetate. The extract was washed with saturated aqueous sodium chloride solution, and dried over anhydrous sodium sulfate. After the drying agent was removed by filtration, the solvent was evaporated *in vacuo,* and the obtained residue was purified by chromatography on a silica gel column (elution solvent: hexane / ethyl acetate = 3 / 7) to afford the desired title compound (12.0 g, 79% yield). ¹H-NMR (200 MHz, CDCl₃) δ (ppm): 7.59 (1H, td, J=8, 2Hz), 7.57 (1H, d, J=2Hz), 7.50 (1H, td, J=8Hz, 2Hz), 7.40 (1H, t, J=8Hz), 4.71 (2H, Abq, J=14Hz, Δµ=47Hz), 2.05 (3H, s), 1.92 (3H, s), 1.83 (3H, s), 1.66 (9H, s).
   MASS (EI) m/z: 368(M⁺), 309, 268, 226, 152, 110, 96, 57.
(4) N-(3-Cyanobenzyl)-N-(3,5-dimethyl-1H-pyrazol-4-yl)acetamide (Step G10)
   N-{1-(t-Butoxycarbonyl)-3,5-dimethyl-1H-pyrazol-4-yl}-N-(3-cyanobenzyl)acetamide (12.0 g) obtained in (3) was dissolved in dichloromethane (120 ml), and trifluoroacetic acid (12.5 ml) was added thereto in an ice-water bath followed by stirring for 5 hours. After the reaction was completed, water was added to this reaction solution, and then 10% aqueous sodium hydroxide solution was added thereto to make the solution alkaline, and the resulting solution was extracted with dichloromethane. The extract was washed with saturated aqueous sodium chloride solution, and dried over anhydrous sodium sulfate. After the drying agent was removed by filtration, the solvent was evaporated *in vacuo*, and the residue obtained was purified by chromatography on a silica gel column (elution solvent: ethyl acetate) to afford the desired title compound (7.10 g, 81% yield).
   ¹H-NMR (200 MHz, CDCl₃) δ (ppm): 7.61-7.52 (3H, m), 7.42 (1H, dd, J=9Hz, 7Hz), 4.73 (2H, s), 1.87 (6H, s), 1.85 (3H, s). [NH: not detected]
   MASS (EI) m/z: 268(M⁺), 225, 152, 116, 110, 96.
(5) N-(3-Cyanobenzyl)-N-[1-(3-fluorobenzyl)-3,5-dimethyl-1H-pyrazol-4-yl]acetamide (exemplification compound number D-101, Step G11)
   N-(3-Cyanobenzyl)-N-(3,5-dimethyl-1H-pyrazol-4-yl)acetamide (108.1 mg, 0.403 mmol) obtained in (4) was dissolved in DMF (2 ml), and 60% sodium hydride (24 mg, 0.604 mmol) was added thereto at 0°C followed by stirring for 15 minutes at the same temperature. To this reaction solution was added dropwise 3-fluorobenzyl bromide (79 µl, 0.604 mmol) followed by stirring for 1 hour at room temperature. After the reaction was completed, saturated aqueous sodium chloride solution was added thereto, and the resulting solution was extracted with ethyl acetate. The organic layer was washed with aqueous sodium chloride solution, dried over anhydrous sodium sulfate, and after the drying agent was removed by filtration, the filtrate was concentrated, and the residue obtained was purified by chromatography on a silica gel column (elution solvent: ethyl acetate) to afford the title compound of the present invention (125.5 mg, 0.333 mmol, 83% yield). ¹H-NMR (200 MHz, CDCl₃) δ (ppm): 1.61 (s, 3H), 1.85(s, 3H), 1.97(s, 3H), 4.42(d, 1H, J=13.9Hz), 5.05(d, 1H, J=13.9Hz), 5.17(brs, 2H), 6.64(d, 1H, J=9.5Hz), 6.79(d, 1H, J=7.7Hz), 6.99(dt, 1H, J=1.8Hz, 7.7Hz), 7.25-7.60(m, 5H).
   MASS (EI) m/z: 376(M⁺), 333, 260, 218, 204, 189, 177, 157, 116, 109, 95, 83.

### (Example 30)

### N-(3-Cyanobenzyl)-N-[1-(3,5-dimethyl-2-oxobutyl)-3,5-dimethyl-1H-pyrazol-4-yl]acetamide (exemplification compound number D-154, Step G11)

N-(3-Cyanobenzyl)-N-(3,5-dimethyl-1H-pyrazol-4-yl) acetamide (536.8 mg, 2.00 mmol) obtained in Example 29-(4) was dissolved in acetonitrile (5 ml), and potassium carbonate (359.0 mg, 2.60 mmol) and 1-chloro-3,3-dimethyl-2-butanone (0.34 ml, 2.60 mmol) were added thereto at room temperature followed by stirring for 6 hours at the same temperature. After the reaction was completed, water was added to the reaction solution, and the resulting solution was extracted with ethyl acetate. The organic layer was washed with aqueous sodium chloride solution, and dried over anhydrous sodium sulfate. After the drying agent was removed by filtration, the filtrate was concentrated, and the obtained residue was purified by chromatography on a silica gel column (elution solvent: ethyl acetate) to afford the title compound of the present invention (660.0 mg, 1.801 mmol, 98% yield).
¹H-NMR (200 MHz, CDCl₃) δ (ppm): 1.25(s, 9H), 1.56 (s, 3H), 1.87(s, 3H), 1.89(s, 3H), 4.46(d, 1H, J=13.6Hz), 4.96(d, 1H, J=17.6Hz), 4.98(d, 1H, J=17.6Hz), 4.99(d, 1H, J=13.6Hz), 7.32-7.63 (m, 4H).
MASS (EI) m/z: 366(M⁺), 323, 293, 281, 250, 239, 166, 157, 149, 125, 116, 89, 81, 71, 57.

### (Example 31)

### N-(3-Carboxylbenzyl)-N-[3,5-dimethyl-1-phenyl-1H-pyrazol-4-yl]acetamide (exemplification compound number D-284, Step I1)

N-(3-Cyanobenzyl)-N-(3,5-dimethyl-1-phenyl-1H-pyrazol-4-yl)acetamide (1.07 g, 3.10 mmol) obtained in Example 1-(2) was dissolved in concentrated hydrochloric acid (52 ml, 630 mmol) followed by heating under reflux for 30 minutes. After the reaction was completed, the reaction solution was cooled to room temperature, and further cooled to 0°C. Water was added to the reaction solution at 0°C, and the resulting solution was extracted with ethyl acetate. The extract was washed with saturated aqueous sodium chloride solution, and dried over anhydrous magnesium sulfate, and after the drying agent was removed by filtration, the solvent was evaporated *in vacuo* to afford the title compound of the present invention (0.65 g, 1.8 mmol, 58% yield).
¹H-NMR (200 MHz, CDCl₃) δ (ppm) : 9.03 (1H, brs), 8.03-7.34 (9H, m), 5.10 (1H, d, J=14Hz), 4.57 (1H, d, J=14Hz), 2.01 (3H, s), 1.93 (3H, s), 1.78 (3H, s) .
MASS (EI) m/z: 363 (M⁺), 319, 228, 172, 118.

### (Example 32)

### N-(3-Methylcarbamoylbenzyl)-N-[3,5-dimethyl-1-phenyl-1H-pyrazol-4-yl]acetamide (exemplification compound number D-289, Step I1)

N-(3-Carboxybenzyl)-N-[3,5-dimethyl-1-phenyl-1H-pyrazol-4-yl]acetamide (0.48 g, 1.3 mmol) obtained in Example 31 was dissolved in tetrahydrofuran (8 ml), and N-methylmorpholine (0.18 ml, 1.6 mmol) was added thereto at -10°C, and then ethyl chlorocarbonate (0.15 ml, 1.6 mmol) was added thereto followed by stirring for 30 minutes. To the resulting reaction solution was added 40% aqueous methylamine solution, and then the temperature of the solution was returned to room temperature followed by stirring for 30 minutes. After the reaction was completed, water was added thereto, and the resulting solution was extracted with ethyl acetate. The extract was washed with saturated aqueous sodium chloride solution, and dried over anhydrous sodium sulfate, and after the drying agent was removed by filtration, the solvent was evaporated *in vacuo.* The residue was purified by chromatography on a silica gel column to afford the title compound of the present invention (0.40 g, 1.1 mmol, 80% yield).
¹H-NMR (200 MHz, CDCl₃) δ (ppm): 7.79-7.34 (9H, m), 6.24-6.13 (1H, m), 5.07 (1H, d, J=14Hz), 4.52 (1H, d, J=14Hz), 2.99 (3H, d, J=5Hz), 1.99 (3H, s), 1.90 (3H, s), 1.76 (3H, s).
MASS (EI) m/z: 376(M⁺), 333, 228, 118.

### (Example 33)

### N-(3-Methylthiocarbamoylbenzyl)-N-[3,5-dimethyl-1-phenyl-1H-pyrazole-4-yl]thioacetamide (exemplification compound number D-402, Step JH1 and Step I1)

N-(3-Methylcarbamoylbenzyl)-N-(3,5-dimethyl-1-phenyl-1H-pyrazol-4-yl]acetamide (0.12 g, 0.31 mmol) obtained in Example 32 was dissolved in xylene (6 ml), and Lawesson's reagent (0.62 g, 1.5 mmol) was added thereto followed by heating under reflux for 4 hours. After the reaction was completed, the reaction solution was cooled down to room temperature, and saturated aqueous sodium hydrogencarbonate solution was added thereto, and the resulting solution was extracted with ethyl acetate. The extract was washed with saturated aqueous sodium chloride solution, and dried over anhydrous magnesium sulfate. After the drying agent was removed by filtration, the solvent was evaporated under reduced pressure. The residue was purified by preparative thin layer chromatography (developing solvent: ethyl acetate / hexane = 1 / 1) to give the title compound of the present invention (0.10 g, 0.25 mmol, 82% yield). ¹H-NMR (200 MHz, CDCl₃) δ (ppm) : 7.77-7.29 (10H, m), 5.99 (1H, d, J=13Hz), 5.02 (1H, d, J=13Hz), 3.31 (1H, d, J=5Hz), 2.43 (3H, s), 2.02 (3H, s), 1.73 (3H, s).
MASS (EI) m/z: 408(M⁺), 392, 349, 164.

### (Example 34)

### N-(3-Cyanobenzyl)-N-[1-isobutyl-5-methoxy-1H-pyrazol-4-yl]-2-cyclopropanecarboxamide (exemplification compound number E-18)

(1) 3-[(1-Isobutyl-5-methoxy-1H-pyrazol-4-ylamino)methyl]benzonitrile (Step B1 and Step B2)
   4-Amino-1-isobutyl-5-methoxypyrazole (638.7 mg, 3.777 mmol) was dissolved in ethanol (10 ml), and 95% 3-cyanobenzaldehyde (632.8 mg, 4.584 mmol) was added thereto at room temperature followed by stirring for 1 hour and then allowing to stand for 12 hours. After the resulting solution was cooled to 0°C, acetic acid (2.2 ml, 38.431 mmol) was added thereto, and then 95% sodium cyanotrihydroborate (750.3 mg, 11.343 mmol) was added thereto over 5 minutes followed by stirring for 1 hour at room temperature. After the reaction was completed, the reaction mixture was poured into saturated aqueous sodium hydrogencarbonate solution, and the resulting solution was extracted with ethyl acetate. The organic layer was dried over anhydrous sodium sulfate, and the drying agent was removed by filtration, and then the filtrate was concentrated. The residue obtained was purified by chromatography on a silica gel column (elution solvent: hexane only - ethyl acetate / hexane = 1 / 2 - ethyl acetate only) to afford the desired title compound (928.3 mg, 3.267 mmol, 86% yield).
   ¹H-NMR (270 MHz, CDCl₃) δ (ppm): 0.87(3H, d, J=7.0Hz), 2.15(1H, dhep, J=7.0Hz, 7.0Hz), 3.68(2H, d, J=7.0Hz), 3.94(3H, s), 4.17(2H, brs), 7.04(1H, s), 7.43(1H, dd, J=7.5Hz, 7.5Hz), 7.51-7.61(2H, brm), 7.67(1H, brs). MASS (EI) m/z: 284(M⁺), 269, 228, 168, 141, 116, 117, 89, 57.
(2) N-(3-Cyanobenzyl)-N-[1-isobutyl-5-methoxy-1H-pyrazol-4-yl]-2-cyclopropanecarboxamide (exemplification compound number E-18, Step B3)
   3-[(1-Isobutyl-5-methoxy-1H-pyrazol-4-ylamino)methyl]benzonitrile (34.2mg, 0.1204 mmol) obtained in (1) was dissolved in dichloromethane (3 ml), and pyridine (0.011 ml, 0.1360 mmol) and cyclopropanecarbonyl chloride (0.013 ml, 0.1433 ml) were successively added thereto at 0°C followed by stirring for 1 hour at room temperature and then allowing to stand for 12 hours. After the reaction was completed, saturated aqueous sodium hydrogencarbonate solution was added thereto, and the resulting solution was extracted with dichloromethane. The organic layer was dried over anhydrous sodium sulfate, and the drying agent was removed by filtration, and then the solvent was evaporated in *vacuo.* The residue obtained was purified by preparative thin layer chromatography (developing solvent: ethyl acetate / hexane = 2 / 1) to afford the title compound of the present invention (32.9 mg, 0.0934 mmol, 78% yield).
   ¹H-NMR (270 MHz, CDCl₃) δ (ppm): 0.70-0.81(2H, m), 0.87(3H, d, J=7.0Hz), 1.01-1.09(2H, m), 1.71(1H, m), 2.15(1H, brhep, J=7.0Hz), 3.72(2H, d, J=7.0Hz), 3.76(3H, s), 4.80(2H, brs), 7.02(1H, s), 7.40(1H, dd, J=7.7Hz, 7.7Hz), 7.45-7.58(3H, brm). MASS (EI) m/z: 352(M⁺), 284, 269, 236, 228, 180, 168, 116, 117, 98, 89, 69, 58.

### (Example 35)

### Methyl N-(5-cyano-2-methoxybenzyl)-N-[1-isobutyl-3,5-dimethyl-1H-pyrazol-4-yl]carbamate (exemplification compound number D-60, Step I1)

Methyl N-(5-bromo-2-methoxybenzyl)-N-[1-isobutyl-3,5-dimethyl-1H-pyrazol-4-yl]carbamate (0.11 g, 0.26 mmol) synthesized according to Example 3 was dissolved in DMF (3 ml), and copper cyanide (60 mg, 0.67 mmol) was added thereto at room temperature followed by heating under reflux at 210°C for 5 hours. After the reaction was completed, the temperature of the reaction solution was returned to room temperature, and to the reaction solution were added water and ethyl acetate, and then the resulting solution was filtered with Celite, and the filtrate was extracted with ethyl acetate. The extract was washed with saturated aqueous sodium chloride solution, and dried over anhydrous magnesium sulfate, and after the drying agent was removed by filtration, the solvent was evaporated in vacuo. The residue was purified by preparative thin layer chromatography (developing solvent: ethyl acetate / hexane = 2 / 1) to give the title compound of the present invention (15 mg, 0.04 mmol, 16% yield).
¹H-NMR (200 MHz, CDCl₃) δ (ppm) : 7.58-7.42 (2H, m), 6.83 (1H, d, J=9Hz), 4.84 (1H, d, J=15Hz), 4.64 (1H, d, J=15Hz), 3.80-3.54 (2H, m), 3.76 (3H, s), 3.68 (3H, s), 2.23-2.00 (1H, m), 1.95 (3H, s), 1.82 (3H, s), 0.86 (3H, d, J=7Hz), 0.79 (3H, d, J=7Hz)
MASS (EI) m/z: 370(M⁺), 355, 327, 224.

### (Example 36)

### N-(3-Cyano-4-methylsulfanylbenzyl)-N-[1-isobutyl-3,5-dimethyl-1H-pyrazol-4-yl]-2-phenoxyacetamide (exemplification compound number D-63, Step I1)

N-(3-Cyano-4-fluoro)-N-[1-isobutyl-3,5-dimethyl-1H-pyrazol-4-yl]-2-phenoxyacetamide (0.14 g, 0.32 mmol) synthesized according to Example 3 was dissolved in THF (8 ml), and sodium methylthiolate (44 mg, 0.63 mmol) was added thereto at 0°C followed by stirring for 1.5 hours at room temperature. After the reaction was completed, water was added to the reaction solution, and the resulting solution was extracted with ethyl acetate. The extract was washed with saturated aqueous sodium chloride solution, and dried over anhydrous magnesium sulfate, and after the drying agent was removed by filtration, the solvent was evaporated *in vacuo.* The residue was purified by chromatography on a silica gel column (elution solvent: ethyl acetate / hexane = 1 / 5 - 1 / 1) to give the title compound of the present invention (0.15 g, 0.32 mmol, 100% yield). ¹H-NMR (200 MHz, CDCl₃) δ (ppm): 7.54-7.16 (5H, m), 6.96 (1H, t, J=7Hz), 6.79 (2H, d, J=8Hz), 5.02 (1H, d, J=14Hz), 4.38 (1H, d, J=14Hz), 4.30 (1H, d, J=15Hz), 4.25 (1H, d, J=15Hz), 3.71 (2H, d, J=7Hz), 2.55 (3H, s), 2.25-2.11 (1H, m), 1.98 (3H, s), 1.73 (3H, s), 0.86 (6H, d, J=7Hz).
MASS (EI) m/z: 462(M⁺), 434, 300, 107, 57.

### (Example 37)

### N-(3-Cyano-4-methylsulfonylbenzyl)-N-[1-isobutyl-3,5-dimethyl-1H-pyrazol-4-yl]-2-phenoxyacetamide (exemplification compound number D-65, Step I1)

N-(3-Cyano-4-methylsulfanylbenzyl)-N-[1-isobutyl-3,5-dimethyl-1H-pyrazol-4-yl]-2-phenoxyacetamide (47 mg, 0.10 mmol) obtained in Example 36 was dissolved in dichloromethane (4 ml), and 80% mCPBA (88 mg, 0.41 mmol) was added thereto at 0°C followed by stirring for 1 hour at room temperature, and then after the reaction solution was cooled to 0°C, 80% mCPBA (44 mg, 0.20 mmol) was added thereto followed by stirring for 1 hour at room temperature. After the reaction was completed, saturated aqueous sodium hydrogencarbonate solution was added to the reaction solution, and the resulting solution was extracted with ethyl acetate. The extract was washed with saturated aqueous sodium chloride solution, and dried over anhydrous sodium sulfate, and then after the drying agent was removed by filtration, the solvent was evaporated under reduced pressure. The residue was purified by preparative thin layer chromatography (developing solvent: ethyl acetate / hexane = 1 / 2) to afford the title compound of the present invention (29 mg, 0.06 mmol, 58% yield).
¹H-NMR (200 MHz, CDCl₃) δ (ppm): 8.13 (1H, d, J=8Hz), 7.83-7.74 (2H, m), 7.30-7.22 (2H, m), 6.97 (1H, t, J=7Hz), 6.79 (2H, d, J=8Hz), 5.11 (1H, d, J=14Hz), 4.55 (1H, d, J=14Hz), 4.32 (1H, d, J=15Hz), 4.31 (1H, d, J=15Hz), 3.72 (2H, d, J=7Hz), 3.27 (3H, s), 2.25-2.08 (1H, m), 2.00 (3H, s), 1.79 (3H, s), 0.87 (3H, d, J=7Hz), 0.85 (3H, d, J=7Hz).
MASS (EI) m/z: 494(M⁺), 438, 401, 300, 107.

### (Example 38)

### N-(3-Cyano-4-methylsulfinylbenzyl)-N- [1-isobutyl-3,5-dimethyl-1H-pyrazol-4-yl]-2-phenoxyacetamide (exemplification compound number D-64, Step I1)

N-(3-Cyano-4-methylsulfanylbenzyl)-N-[1-isobutyl-3,5-dimethyl-1H-pyrazol-4-yl]-2-phenoxyacetamide (51 mg, 0.11 mmol) obtained in Example 36 was dissolved in dichloromethane (4 ml), and 80% mCPBA (26 mg, 0.12 mmol) was added thereto at 0°C followed by stirring for 30 minutes at room temperature. After the reaction was completed, saturated aqueous sodium hydrogencarbonate solution was added to the reaction solution, and the resulting solution was extracted with ethyl acetate. The extract was washed with saturated aqueous sodium chloride solution, and dried over anhydrous sodium sulfate, and then after the drying agent was removed by filtration, the solvent was evaporated under reduced pressure. The residue was purified by preparative thin layer chromatography (developing solvent: ethyl acetate / hexane = 3 / 1) to afford the title compound of the present invention (25 mg, 0.05 mmol, 48% yield).
¹H-NMR (200 MHz, CDCl₃) δ (ppm): 8.09-7.63 (3H, m), 7.30-7.22 (2H, m), 6.97 (1H, t, J=7Hz), 6.79 (2H, d, J=8Hz), 5.08 (1H, d, J=14Hz), 4.54 (1H, d, J=14Hz), 4.31 (1H, d, J=15Hz), 4.30 (1H, d, J=15Hz), 3.72 (2H, d, J=8Hz), 2.86 (3H, s), 2.30-2.06 (1H, m), 1.97 (3H, s), 1.76 (3H, s), 0.86 (6H, d, J=7Hz).
MASS (EI) m/z: 478(M⁺), 462, 447, 300, 107.

### (Example 39)

### N-(3-Cyano-5-methylsulfanylmethylbenzyl)-N-[1-isobutyl-5-methyl-1H-pyrazol-4-yl]-2-phenoxyacetamide (exemplification compound number C-317, Step I1)

N-(5-Bromomethyl-3-cyanobenzyl)-N-[1-isobutyl-5-methyl-1H-pyrazol-4-yl]-2-phenoxyacetamide (65 mg, 0.13 mmol) synthesized according to Example 3 was dissolved in DMF (3 ml), and sodium methylthiolate (18 mg, 0.26 mmol) was added thereto at 0°C followed by stirring for 2 hours at room temperature. After the reaction was completed, water was added to the reaction solution, and the resulting solution was extracted with ethyl acetate. The extract was washed with saturated aqueous sodium chloride solution, and dried over anhydrous magnesium sulfate, and then after the drying agent was removed by filtration, the solvent was evaporated under reduced pressure. The residue was purified by preparative thin layer chromatography (developing solvent: ethyl acetate / hexane = 1 / 1) to afford the title compound of the present invention (41 mg, 0.09 mmol, 67% yield).
¹H-NMR (200 MHz, CDCl₃) δ (ppm): 7.52-7.23 (5H, m), 7.22 (1H, s), 6.96 (1H, t, J=7Hz), 6.81 (2H, d, J=8Hz), 4.78 (2H, br s), 4.37 (2H, br s), 3.78 (2H, d, J=7Hz), 3.62 (2H, s), 2.25-2.11 (1H, m), 1.97 (3H, s), 1.85 (3H, s), 0.86 (6H, d, J=7Hz). MASS (EI) m/z: 462(M⁺), 416, 360, 327, 107.

### (Example 40)

### N-(3-Cyanobenzyl)-N-[1-isobutyl-5-methyl-1H-pyrazol-4-yl]-2-hydroxyacetamide (exemplification compound number D-214, Step L1)

N-(3-Cyanobenzyl)-N-[1-isobutyl-5-methyl-1H-pyrazol-4-yl]-2-acetoxyacetamide (35.2 g, 95.54 mmol) synthesized according to Example 3 was dissolved in methanol (200 ml), and 10% aqueous sodium hydroxide solution (46 ml) was poured into this solution in an ice bath followed by stirring for 10 minutes at 0°C. After the reaction was completed, the reaction solution was diluted with water, and extracted with chloroform. The extract was washed with saturated aqueous sodium chloride solution, and dried over anhydrous sodium sulfate. After the drying agent was removed by filtration, the solvent was evaporated in *vacuo,* and the residue obtained was purified by chromatography on a silica gel column (elution solvent: hexane / ethyl acetate = 4 / 6 - 2 / 8) to afford the title compound of the present invention (27.6 g, 84.07 mmol, 88% yield). ¹H-NMR (200 MHz, CDCl₃) δ (ppm): 7.62-7.39 (4H, m), 7.16 (1H, s), 4.80 (2H, brs), 3.78 (2H, d, J=5Hz), 3.77 (2H, d, J=7Hz), 3.25 (1H, t, J=5Hz), 2.18 (1H, brhept, J=7Hz), 1.77 (3H, s), 0.87 (6H, d, J=7Hz).
MASS (EI) m/z: 326(M⁺), 310, 267, 180, 152, 116, 57.

### (Example 41)

### N-(3-Cyanobenzyl)-N-[1-isobutyl-5-methyl-1H-pyrazol-4-yl]-2-phthalimidoxyacetamide (exemplification compound number C-700, Step L1)

N-(3-Cyanobenzyl)-N-[1-isobutyl-5-methyl-1H-pyrazol-4-yl]-2-hydroxyacetamide (1.80 g, 5.51 mmol) obtained in Example 40 was dissolved in tetrahydrofuran (20 ml), and triphenylphosphine (1.74 g, 6.63 mmol), N-hydroxyphthalimide (1.08 g, 6.62 mmol), and diethyl azodicarboxylate (1.04 ml, 6.60 mmol) were successively added thereto followed by stirring for 3 hours at room temperature. After the reaction was completed, the solvent was evaporated *in vacuo*, and the residue obtained was purified by chromatography on a silica gel column (elution solvent: hexane / ethyl acetate = 3 / 7) to afford the title compound of the present invention (2.40 g, 5.07 mmol, 92% yield).
¹H-NMR (200 MHz, CDCl₃) δ (ppm): 7.87-7.73 (4H, m), 7.61-7.37 (4H, m), 7.25 (1H, s), 4.85 (2H, brs), 4.55 (2H, s), 4.12 (2H, d, J=7Hz), 2.19 (1H, brhept, J=7Hz), 1.81 (3H, s), 0.87 (6H, d, J=7Hz).
MASS (EI) m/z: 471(M⁺), 383, 324, 309, 295, 267, 227, 116.

### (Example 42)

### 2-Aminooxy-N-(3-cyanobenzyl)-N-(1-isobutyl-5-methyl-1H-pyrazol-4-yl)acetamide (exemplification compound number C-226, Step L1)

N-(3-Cyanobenzyl)-N-(1-isobutyl-5-methyl-1H-pyrazol-4-yl)-2-phthalimidoxyacetamide (2.40 g, 5.09 mmol) obtained in Example 41 was dissolved in tetrahydrofuran (25 ml), and hydrazine monohydrate (1.0 ml) was added dropwise thereto followed by stirring for 30 hours at room temperature. After the reaction was completed, water was poured into the reaction solution, and the resulting solution was extracted with chloroform, and the organic layer was dried over anhydrous sodium sulfate. After the drying agent was removed by filtration, the solvent was evaporated *in vacuo*, and the residue obtained was purified by chromatography on a silica gel column (elution solvent: chloroform / methanol = 95 / 5) to afford the title compound of the present invention (1.66 g, 4.88 mmol, 96% yield).
¹H-NMR (200 MHz, CDCl₃) δ (ppm): 7.60-7.30 (4H, m), 7.18 (1H, s), 5.98 (2H, brs), 4.77 (2H, brs), 3.99 (2H, brs), 3.76 (2H, d, J=7Hz), 2.18 (1H, brhept, J=7Hz), 1.77 (3H, s), 0.87 (6H, d, J=7Hz).
MASS (EI) m/z: 341(M⁺), 324, 309, 295, 267, 180, 116.

### (Example 43)

### N-(3-Cyanobenzyl)-N-(1-isobutyl-5-methyl-1H-pyrazol-4-yl)-2-isopropylideneaminooxyacetamide (exemplification compound number C-234, Step L1)

2-Aminooxy-N-(3-cyanobenzyl)-N-(1-isobutyl-5-methyl-1H-pyrazol-4-yl)acetamide (31.4 mg, 0.092 mmol) obtained in Example 42 was dissolved in chloroform (1 ml), and acetone (0.2 ml) was poured thereto followed by stirring for 2 hours at room temperature. After the reaction was completed, the solvent was evaporated *in vacuo*, and the residue obtained was purified by thin layer chromatography on a silica gel plate (developing solvent: ethyl acetate) to afford the title compound of the present invention (29.7 mg, 0.078 mmol, 85% yield).
¹H-NMR (200 MHz, CDCl₃) δ (ppm): 7.59-7.50 (3H, m), 7.40 (1H, t, J=8Hz), 7.19 (1H, s), 4.77 (2H, brs), 4.34 (2H, brs), 3.76 (2H, d, J=7Hz), 2.18 (1H, brhept, J=7Hz), 1.93 (3H, s), 1.87 (3H, s), 1.79 (3H, s), 0.86 (6H, d, J=7Hz).
MASS (EI) m/z : 381(M⁺), 325, 281, 266, 210, 116.

### (Example 44)

### Benzyl [(3-cyanobenzyl)(1-isobutyl-5-methyl-1H-pyrazol-4-yl)carbamoylmethyl]carbamate (exemplification compound number C-256, Step B3)

3-[(1-Isobutyl-5-methyl-1H-pyrazol-4-ylamino)methyl]benzonitrile (543.5 mg, 2.025 mmol) obtained in Example 3(1), N-benzyloxycarbonylglycine (466.0 mg, 2.228 mmol), and 2-chloro-N-methylpyridinium iodide (569.0 mg, 2.227 mmol) were dissolved in dichloromethane (10 ml), and triethylamine (0.31 ml, 2.224 mmol) was added thereto at room temperature followed by stirring for 5 hours. After the reaction was completed, the reaction mixture was concentrated, and water was added thereto, and then the resulting solution was extracted with ethyl acetate. The organic layer was washed with water, and then with aqueous sodium chloride solution, and dried over anhydrous sodium sulfate, and after the drying agent was removed by filtration, the solution was concentrated. The residue obtained was purified by chromatography on a silica gel column (elution solvent: hexane only - ethyl acetate / hexane = 1 / 1 - ethyl acetate only) to give the title compound of the present invention (432.6 mg, 0.941 mmol, 65% yield).
¹H-NMR (200 MHz, CDCl₃) δ (ppm): 0.87(6H, d, J=7.1Hz), 1.77(3H, s), 2.18(1H, dhep, J=7.1Hz, J=7.1Hz), 3.66(2H, brd, J=3.9Hz), 3.75 (2H, d, J=7.1Hz), 4.76(2H, brs), 5.09(2H, s), 5.59(1H, brt, J=3.9Hz), 7.18(1H, s), 7.28-7.62(9H,m).
MASS (EI) m/z: 459(M⁺), 351, 267, 256, 235, 192, 152, 136.

### (Example 45)

### 2-Amino-N-(3-cyanobenzyl)-N-(1-isobutyl-5-methyl-1H-pyrazol-4-yl)acetamide (exemplification compound number C-247, Step L1)

Benzyl [(3-cyanobenzyl)(1-isobutyl-5-methyl-1H-pyrazol-4-yl)carbamoylmethyl]carbamate (569 mg, 1.24 mmol) obtained in Example 44 was dissolved in methanol (3 ml), and after degassing the solution, nitrogen gas was introduced thereto, and then 3.6% hydrous palladium catalyst (175 mg, 0.06 mmol) was added thereto. The mixture was degassed, and hydrogen gas was introduced thereto followed by stirring for 3 hours at room temperature and allowing to stand overnight. After the reaction was completed, the reaction mixture was degassed, and nitrogen gas was introduced thereto, and then the solution was filtered using Celite to remove the catalyst, and the filtrate was concentrated to give the title compound of the present invention (406.9 mg, 1.24 mmol, 100% yield).
¹H-NMR (200 MHz, CDCl₃) δ (ppm): 0.87(6H, d, J=6.1Hz), 1.85(3H, s), 2.18(1H, dhep, J=7.1Hz, J=6.1Hz), 3.11(2H, brs), 3.78(2H, d, J=7.1Hz), 4.76(2H, brs), 7.15(1H, s), 7.50(1H, dd, J=7.1Hz, J=7.1Hz), 7.49(1H, brs), 7.50(1H, ddd, J=1.5Hz, J=1.5Hz, J=7.1Hz), 7.57(1H, ddd, J=1.5Hz, J=1.5Hz, J=7.1Hz).
MASS (EI) m/z: 325(M⁺), 296, 268, 225, 209, 180, 166, 149, 116, 98, 70, 57.

### (Example 46)

### Benzyl 2- [3-(3-cyanobenzyl)-3-(1-isobutyl-5-methyl-1H-pyrazol-4-yl)ureido] -3-methylbutanoate (exemplification compound number C-278, Step B4)

3-[(1-Isobutyl-5-methyl-1H-pyrazol-4-ylamino)methyl]benzonitrile (272.9 mg, 1.017 mmol) obtained in Example 3 (1) was dissolved in chloroform (10 ml), and pyridine (0.16 ml, 1.978 mmol) and 2-isocyanato-3-methylbutanoic acid benzyl ester (0.60 mg, 2.23 mmol) in chloroform (5 ml) were successively added thereto at room temperature followed by stirring for 4 hours. After the reaction was completed, the reaction mixture was washed with 0.5 mol/L hydrochloric acid, and then extracted with dichloromethane. The organic layer was washed with saturated aqueous sodium hydrogencarbonate solution, and dried over anhydrous magnesium sulfate, and after the drying agent was removed by filtration, the filtrate was concentrated. The residue obtained was purified by chromatography on a silica gel column (elution solvent: hexane - ethyl acetate / hexane = 1/1) to afford the title compound of the present invention (486.7 mg, 0.970 mmol, 95% yield).
¹H-NMR (270 MHz, CDCl₃) δ (ppm): 0.67(3H, d, J=7.0Hz), 0.82-0.89(9H, m), 1.80(3H, s), 2.02-2.15(2H, m), 3.76(1H, d, J=7.6Hz), 3.77(1H, d, J=3.77Hz), 4.46(1H, dd, J=4.1Hz, J=8.7Hz), 4.70(1H, brd, J=15.0Hz), 4.74(1H, brd, J=15.0Hz), 4.89(1H, d, J=8.7Hz), 5.08(1H, d, J=12.2Hz), 7.17(1H, d, J=12.2Hz), 7.21(1H, s), 7.27-7.57(9H, m).
MASS (EI) m/z: 501(M⁺), 486, 458, 410, 366, 295, 268, 239, 212, 178, 152, 136, 116, 91, 57.

### (Example 47)

### 2-[3-(3-Cyanobenzyl)-3-(1-isobutyl-5-methyl-1H-pyrazol-4-yl)ureido]-3-methylbutanoic acid (exemplification compound number C-277, Step L1)

Benzyl 2-[3-(3-cyanobenzyl)-3-(1-isobutyl-5-methyl-1H-pyrazol-4-yl)ureido]-3-methylbutanoate (418.4 mg, 0.834 mmol) obtained in Example 46 was dissolved in methanol (10 ml), and after degassing the solution, nitrogen gas was introduced thereto, and then 3.6% hydrous palladium catalyst (254.5 mg, 0.086 mmol) was added thereto. The mixture was degassed, and hydrogen gas was introduced thereto followed by stirring for 2 hours at room temperature. After the reaction was completed, the reaction mixture was degassed, and nitrogen gas was introduced thereto, and then the solution was filtered using Celite to remove the catalyst, and the filtrate was concentrated to give the title compound of the present invention (299.1 mg, 0.727 mmol, 87% yield).
¹H-NMR (270 MHz, DMSO-d₆) δ (ppm): 0.63(3H, d, J=6.4Hz), 0.74-0.85(9H, m), 1.98(3H, s), 1.95-2.15(2H, m), 3.75-3.95(3H, brm), 4.29(1H, brd, J=13.7Hz), 4.96(1H, brd, J=13.7Hz), 5.11(1H, d, J=8.7Hz), 7.04-7.57(5H, m).

### (Example 48)

### N-(3-Cyanobenzyl)-N-[3,5-dimethyl-1-phenyl-1H-pyrazol-4-yl]-N'-methylthiourea (exemplification compound number D-399, Step B4)

3-(3,5-Dimethyl-1-phenyl-1H-pyrazol-4-ylaminomethyl)benzonitrile (29 mg, 0.10 mmol) synthesized according to Example 3(1) was dissolved in dichloromethane (2 ml), and pyridine (0.09 ml, 0.11 mmol) and methylthioisocyanate (8 mg, 0.11 mmol) were added thereto at 0°C followed by stirring for 1.5 hours at room temperature and then heating under reflux for 4 hours. Pyridine (large excess) and methylthioisocyanate (large excess) were added to the resulting solution, and then after chloroform (2 ml) was added thereto, the reaction solution was heated under reflux for 3 hours. After the reaction was completed, the reaction solution was cooled to room temperature, and saturated aqueous sodium hydrogencarbonate solution was added thereto, and the resulting solution was extracted with ethyl acetate. The extract was washed with saturated aqueous sodium chloride solution, and dried over anhydrous sodium sulfate, and after the drying agent was removed by filtration, the solvent was evaporated under reduced pressure. The residue was purified by preparative thin layer chromatography (developing solvent: ethyl acetate) to afford the title compound of the present invention (28 mg, 0.08 mmol, 78% yield).
¹H-NMR (200 MHz, CDCl₃) δ (ppm): 7.78-7.35 (9H, m), 5.90 (1H, q, J=4Hz), 5.67 (1H, d, J=14Hz), 5.21 (1H, d, J=14Hz), 3.14 (1H, d, J=4Hz), 1.94 (3H, s), 1.80 (3H, s).
MASS (EI) m/z: 375(M⁺), 301, 204, 172.

### (Example 49)

### 1-(3-Cyanobenzyl)-1-(1-isobutyl-5-methyl-1H-pyrazol-4-yl)urea (exemplification compound number C-97, Step B4 and Step L1)

3-[(1-Isobutyl-5-methyl-1H-pyrazol-4-ylamino)methyl]benzonitrile (1.44 g, 5.37 mmol) obtained in Example 3 (1) was dissolved in tetrahydrofuran (15 ml), and trimethylsilylisocyanate (1.5 ml, 11.08 mmol) and triethylamine (1 drop) were added thereto followed by stirring for 5 days at room temperature. After the reaction was completed, the solvent was evaporated in vacuo, and the obtained residue was purified by chromatography on a silica gel column (elution solvent: ethyl acetate only - hexane / acetone = 2 / 8) to give the title compound of the present invention (1.07 g, 3.44 mmol, 64% yield).
¹H-NMR (200 MHz, CDCl₃) δ (ppm): 7.59-7.53 (3H, m), 7.40 (1H, td, J=7Hz, 1Hz), 7.23 (1H, s), 4.72 (2H, s), 4.60 (2H, s), 3.77 (2H, d, J=7Hz), 2.17 (1H, brhept, J=7Hz), 1.84 (3H, s), 0.87 (6H, d, J=7Hz).
MASS (EI) m/z: 311(M+), 267, 212, 195, 178, 152, 116.

### (Examples 50 and 51)

### 1-(2-Cyano-2-methoxyiminoacetyl)-3-(3-cyanobenzyl)-3-(1-isobutyl-5-methyl-1H-pyrazol-4-yl)urea (exemplification compound number C-282) and 1,1-Bis(2-cyano-2-methoxyiminoacetyl)-3-(3-cyanobenzyl)-3-(1-isobutyl-5-methyl-1H-pyrazol-4-yl)urea (exemplification compound number C-283, Step L1)

1-(3-Cyanobenzyl)-1-(1-isobutyl-5-methyl-1H-pyrazol-4-yl)urea (191 mg, 0.613 mmol) obtained in Example 49 was dissolved in chloroform (3 ml), and pyridine (0.15 ml, 1.85 mmol) and 2-cyano-2-(methoxyimino)acetyl chloride (0.21 ml, 1.3 mmol) were successively added thereto in an ice bath followed by stirring for 2 hours at room temperature. After the reaction was completed, saturated aqueous sodium chloride solution was added to the reaction solution, and the resulting solution was extracted with ethyl acetate. The extract was washed with saturated aqueous sodium hydrogencarbonate solution and then with saturated aqueous sodium chloride solution, and dried over anhydrous sodium sulfate. After the drying agent was removed by filtration, the solvent was evaporated under reduced pressure, and then the residue was purified by chromatography on a silica gel column (elution solvent: hexane / ethyl acetate = 5 / 5 - ethyl acetate only) to afford the compound of the present invention, 1-(2-cyano-2-methoxyiminoacetyl) -3-(3-cyanobenzyl)-3-(1-isobutyl-5-methyl-1H-pyrazol-4-yl)urea (92.3 mg, 0.221 mmol, 36% yield) and also the compound of the present invention, 1,1-bis(2-cyano-2-methoxyiminoacetyl)-3-(3-cyanobenzyl)-3-(1-isobutyl-5-methyl-1H-pyrazol-4-yl)urea (163 mg, 0.307 mmol, 50% yield).

1-(2-Cyano-2-methoxyiminoacetyl)-3-(3-cyanobenzyl)-3-(1-isobutyl-5-methyl-1H-pyrazol-4-yl)urea (exemplification compound number C-282, Example 50)
¹H-NMR (200 MHz, CDCl₃) δ (ppm): 8.47 (1H, s), 7.62-7.40 (4H, m), 7.28 (1H, s), 4.76 (2H, brs), 4.08 (3H, s), 3.81 (2H, d, J=7Hz), 2.17 (1H, brhept, J=7Hz), 1.84 (3H, s), 0.89 (6H, d, J=7Hz).
MASS (EI) m/z: 421(M⁺), 337, 305, 268, 212, 152, 116

1,1-Bis(2-cyano-2-methoxyiminoacetyl)-3-(3-cyanobenzyl)-3-(1-isobutyl-5-methyl-1H-pyrazol-4-yl)urea (exemplification compound number C-283, Example 51)
¹H-NMR (200 MHz, CDCl₃) δ (ppm): 7.65-7.44 (4H, m), 7.15 (1H, s), 4.78 (2H, brd), 4.16 (6H, s), 3.72 (2H, d, J=7Hz), 2.10 (1H, brhept, J=7Hz), 1.98 (3H, s), 0.86 (6H, d, J=7Hz). MASS (EI) m/z: 531(M⁺), 378, 347, 279, 267, 149, 116.

### (Example 52)

### 1,4-Dihydro-2-(3,5-dimethyl-1-phenyl-1H-pyrazol-4-yl)-7-nitro-2H-isoquinolin-3-one (exemplification compound number F-5)

(1) N-(3,5-Dimethyl-1-phenyl-1H-pyrazol-4-yl)-2-(2-chloromethyl-4-nitrophenyl)acetamide (Step A1)
   4-Amino-3,5-dimethyl-1-phenyl-1H-pyrazole (230 mg, 1.229 mmol) was dissolved in dichloromethane (25 ml), and pyridine (0.12 ml, 1.485 mmol) and 2-(2-chloromethyl-4-nitrophenyl)acetyl chloride (0.5 g, 2.0 mmol) were successively added thereto at 0°C followed by stirring for 2 hours at room temperature. After the reaction was completed, saturated aqueous sodium hydrogencarbonate solution was added to the reaction solution, and the resulting solution was extracted with dichloromethane. The organic layer was dried over anhydrous magnesium sulfate, and after the drying agent was removed by filtration, the filtrate was concentrated. The obtained residue was purified by chromatography on a silica gel column (elution solvent: hexane - ethyl acetate / hexane = 2/1) to afford the desired title compound (387.2 mg, 0.993 mmol, 79% yield).
   ¹H-NMR (270 MHz, CDCl₃) δ (ppm): [2.14(s), 2.16(s) and 2.20(s)](6H), [2.47(t-like, J=8.1Hz), 2.79(t-like, J=8.1Hz), 3.16(t-like, J=8.1Hz), and 3.31(t-like, J=8.1Hz))(4H), [6.49(brs) and 6.57(brs)](1H), 7.3-7.6(6H, m), 8.05(1H, dd, J=2.7Hz, J=10.8Hz), [8.21(d, J=2.7Hz) and 8.24(d, J=2.7Hz)](1H).
   MASS (EI) m/z: 398(M⁺), 363, 333, 187, 145, 118, 104, 77.
(2) 1,4-Dihydro-2-(3,5-dimethyl-1-phenyl-1H-pyrazol-4-yl)-7-nitro-2H-isoquinolin-3-one (exemplification compound number F-5, Step A2)
   N-(3,5-Dimethyl-1-phenyl-1H-pyrazol-4-yl)-2-(2-chloromethyl-4-nitrophenyl)acetamide (539.4 mg, 1.352 mmol) obtained in (1) was dissolved in DMF (25 ml), and 60% sodium hydride (67 mg, 1.68 mmol) was added thereto at 0°C followed by stirring for 3 hours at room temperature. After the reaction was completed, aqueous sodium chloride solution was added to the reaction solution, and the resulting solution was extracted with dichloromethane. The organic layer was dried over anhydrous sodium sulfate, and after the drying agent was removed by filtration, the filtrate was concentrated to afford the title compound of the present invention (462.7 mg, 1.227 mmol, 94% yield).
   ¹H-NMR (270 MHz, CDCl₃) δ (ppm): 2.08(3H, s), 2.11(3H, s), 2.90-297(2H, m), 3.17-3.24(2H, m), [6.72(s) and 6.75(s)] (1H), 7.35-7.55(6H, m), 8.04(1H, dd, J=2.7Hz, J=10.8Hz), 8.16(1H, d, J=2.7Hz).
   MASS (EI) m/z: 362(M⁺), 346, 333, 319, 171, 149, 118, 77.

### (Example 53)

### 7-Amino-1,4-dihydro-2-(3,5-dimethyl-1-phenyl-1H-pyrazol-4-yl)-2H-isoquinolin-3-one (exemplification compound number F-4, Step I1)

1,4-Dihydro-2-(3,5-dimethyl-1-phenyl-1H-pyrazol-4-yl)-7-nitro-2H-isoquinolin-3-one (462.7 mg, 1.227 mmol) obtained in Example 52 was dissolved in methanol (25 ml), and this solution was degassed three times, and nitrogen gas was introduced thereto. To the reaction solution was added 10% palladium-charcoal catalyst, and the reaction mixture was degassed twice, and after nitrogen gas was introduced thereto, the resulting solution was degassed, and then hydrogen gas was introduced thereto followed by stirring for 5.5 hours. Then, the reaction mixture was degassed three times, and then after nitrogen gas was introduced, the catalyst was removed using Celite. The organic layer was concentrated to give the title compound of the present invention (407 mg, 0.13 mmol, 100% yield).
MASS (EI) m/z: 332(M⁺), 304, 289, 275, 171, 152, 145, 118, 77. ¹H-NMR (270 MHz, DMSO-d₆) δ (ppm): 1.93(3H, s), 2.06(3H, s), 2.63-2.70(2H, m), 2.84-2.94(2H, m), 4.87(2H, brs), 6.21(1H, d, J=8.1Hz), 6..35(1H, dd, J=2.3Hz, J=8.1Hz), 6.49(1H, d, J=2.3Hz), 7.41(1H, t, J=7.0Hz), 7.40-7.52(4H, m).

### (Examples 54 and 55)

### 7-Bromo-1,4-dihydro-2-(3,5-dimethyl-1-phenyl-1H-pyrazol-4-yl)-2H-isoquinolin-3-one (exemplification compound number F-2) and 1,4-Dihydro-2-(3,5-dimethyl-1-phenyl-1H-pyrazol-4-yl)-7-hydroxy-2H-isoquinolin-3-one (exemplification compound number F-6) (Step I1)

To 7-amino-1,4-dihydro-2-(3,5-dimethyl-1-phenyl-1H-pyrazol-4-yl)-2H-isoquinolin-3-one (393.1 mg, 1.2582 mmol) obtained in Example 53 were added successively 47% hydrobromic acid (1.0 ml, 8.597 mmol) and sodium nitrite (88.5 mg, 1.283 mmol) at 0°C followed by stirring for 40 minutes to give a precipitate of the diazonium salt.

To copper bromide (156.8 mg, 1.0931 mmol) was added 47% hydrobromic acid (0.5 ml, 4.299 mmol), and the mixture was heated at 110°C in a bath, and then the diazonium salt described above was added thereto in portions using a pipette, followed by stirring for 30 minutes at 110°C in the bath. After the reaction was completed, the reaction mixture was poured into a mixture of saturated aqueous sodium hydrogencarbonate and acetic acid in an ice bath followed by stirring for 4 hours. After the precipitated green solid was filtered off, the filtrate was separated into two layers (organic and aqueous layers), and the aqueous layer was extracted with ethyl acetate. This extract was added to the organic layer, and dried over anhydrous magnesium sulfate, and then after the drying agent was removed by filtration, the filtrate was concentrated. The residue obtained was purified by chromatography on a silica gel column (elution solvent: hexane - ethyl acetate / hexane = 3 / 1) to afford the compound of the present invention, 7-bromo-1,4-dihydro-2-(3,5-dimethyl-1-phenyl-1H-pyrazol-4-yl)-2H-isoquinolin-3-one (360.0 mg, 0.9084 mmol, 72% yield).

On the other hand, to the remaining part of the diazonium salt described above, which could not be taken up by the pipette, were added successively water (3 ml) and concentrated sulfuric acid (0.7 ml) followed by stirring for 20 minutes at 110°C in the bath. After the reaction was completed, water was added to the reaction solution, and then the resulting solution was extracted with dichloromethane. The extract was dried over anhydrous sodium sulfate, and after the drying agent was removed by filtration, the filtrate was concentrated to give the compound of the present invention, 1,4-dihydro-2-(3,5-dimethyl-1-phenyl-1H-pyrazol-4-yl)-7-hydroxy-2H-isoquinolin-3-one (7.3 mg, 0.22 mmol, 1.7% yield).

7-Bromo-1,4-dihydro-2-(3,5-dimethyl-1-phenyl-1H-pyrazol-4-yl)-2H-isoquinolin-3-one (exemplification compound number F-2, Example 54)
¹H-NMR (270 MHz, DMSO-d₆) δ (ppm): 1.94(3H, s), 2.07(3H, s), 2.73-2.82(2H, m), 3.04-3.12(2H, m), 6.47(1H, d, J=8.7Hz), 7.33(1H, dd, J=2.3Hz, J=8.7Hz), 7.38-7.47(1H, m), 7.50-7.632(5H, m).
MASS (EI) m/z: 395(M⁺), 352, 317, 279, 171, 149, 129, 118, 77, 57.

1,4-Dihydro-2-(3,5-dimethyl-1-phenyl-1H-pyrazol-4-yl)-7-hydroxy-2H-isoquinolin-3-one (exemplification compound number F-6, Example 55)
¹H-NMR (270 MHz, CDCl₃) δ (ppm): 2.07(3H, s), 2.11(3H, s), 2.67-2.76(2H, m), 2.95-3.06(2H, m), 6.43(1H, d, J=8.7Hz), 6.57(1H, dd, J=2.7Hz, J=8.7Hz), 6.70(1H, d, J=2.7Hz), 7.33-7.55(5H, m).
MASS (EI) m/z: 333(M⁺), 304, 290, 275, 171, 152, 118, 77.

### (Example 56)

### 3-Cyanobenzyl [1- (cyclopentylmethyl) -5-methyl-1H-pyrazol-4-yl]carbamoyl chloride (exemplification compound number C-784, Step M1)

3-({[1-(Cyclopentylmethyl)-5-methyl-1H-pyrazol-4-yl]amino}methyl)benzonitrile (1.12 g, 3.804 mmol) was dissolved in dichloromethane (25 ml), and triethylamine (0.53 ml, 0.3803 mmol) and bis(trichloromethyl)carbonate (0.3918 g, 1.320 mmol) were successively added thereto followed by stirring for 3 hours at room temperature. After the reaction was completed, water was added to the reaction mixture, and the resulting solution was extracted with dichloromethane. The organic layer was dried and concentrated, and the residue obtained was purified by chromatography on a silica gel column (elution solvent: hexane - ethyl acetate / hexane = 1 / 1) to afford the title compound of the present invention (1.27 g, 3.664 mmol, 96% yield).
¹H-NMR (270 MHz, CDCl₃) δ (ppm): 7.63(1H, dd, J=1.8Hz, J=7.6Hz), 7.52(1H, ddd, J=1.8Hz, J=1.8Hz, J=7.6Hz), 7.48(1H, brs), 7.21(1H, s), 4.78(2H, brs), 3.90(2H, d, J=7.6Hz), 2.38(1H, ddd, J=7.6Hz, J=7.6Hz, J=7.6 Hz), 1.80(3H, s), 1.50-1.75(6H, brm), 1.13-1.30(2H, brm).
MASS (EI) m/z: 356(M⁺), 341, 321, 298, 288, 274, 253, 240, 225, 211, 190, 172, 158, 136, 131, 123, 116, 109, 90, 83, 67, 55.

### (Examples 57 and 58)

### 2-Pyridyl 3-cyanobenzyl[1-(cyclopentylmethyl)-5-methyl-1H-pyrazol-4-yl]carbamate (exemplification compound number C-800) and N-(3-Cyanomethyl)-N-[1-(cyclopentylmethyl)-5-methyl-1H-pyrazol-4-yl]-2-oxo-1(2H)-pyridinecarboxamide (exemplification compound number C-801) (Step M2)

3-Cyanobenzyl[1-(cyclopentylmethyl)-5-methyl-1H-pyrazol-4-yl]carbamoyl chloride (112.4 mg, 0.3150 mmol) obtained in Example 56 was dissolved in chloroform (3 ml), and N,N-dimethylaminopyridine (63.6 mg, 0.5206 mmol) and 2-hydroxypyridine (46.6 mg, 0.4900 mmol) were successively added thereto followed by stirring for 4 hours at 50°C. After the reaction solution was cooled to room temperature, water was added to the reaction mixture, and the resulting solution was extracted with chloroform. The organic layer was dried and concentrated, and the obtained residue was purified by preparative thin layer chromatography (developing solvent: ethyl acetate / hexane = 1 / 2) to afford the compound of the present invention, 2-pyridyl 3-cyanobenzyl[1-(cyclopentylmethyl)-5-methyl-1H-pyrazol-4-yl] carbamate (77.0 mg, 0.1853 mmol, 59% yield) and the compound of the present invention, N-(3-cyanomethyl)-N-[1-(cyclopentylmethyl)-5-methyl-1H-pyrazol-4-yl]-2-oxo-1(2H)-pyridinecarboxamide (31.6 mg, 0.076 mmol, 24% yield).

2-Pyridyl 3-cyanobenzyl[1-(cyclopentylmethyl)-5-methyl-1H-pyrazol-4-yl]carbamate (exemplification compound number C-800, Example 57)
¹H-NMR (270 MHz, CDCl₃) δ (ppm): 8.37(1H, d, J=4.1Hz), 7.75(1H, t, J=8.1Hz), 7.51-7.68(3H, m), 7.43(1H, t, J=7.6Hz), 7.29(1H, s), 7.18(1H, brm), 7.00(1H, d, J=8.1Hz), 4.80(2H, s), 3.88(2H, s), 2.39(1H, ddd, J=7.6Hz, J=7.6Hz, J=7.6Hz), 1.96(3H, s), 1.50-1.78(6H, brm), 1.14-1.33(2H, brm).
MASS (EI) m/z: 415(M⁺), 293, 239, 204, 136, 124, 116, 96, 89, 83, 82, 78, 67, 55.

N-(3-Cyanomethyl)-N-[1-(cyclopentylmethyl)-5-methyl-1H-pyrazol-4-yl]-2-oxo-1(2H)-pyridinecarboxamide (exemplification compound number C-801, Example 58)
¹H-NMR (270 MHz, CDCl₃) δ (ppm): 7.73(1H, d, J=6.27Hz), 7.61(1H, d, J=7.6Hz), 7.57(1H, brs), 7.48(1H, dd, J=7.6Hz, J=7.6Hz), 7.10-7.21(2H, m), 6.25(1H, d, J=9.3Hz), 6.06(1H, dt, J=1.2Hz, J=7.0Hz), 5.01(1H, brd, J=14.0Hz), 4.82(1H, brd, J=14.0Hz), 3.80(2H, d, J=7.6Hz), 2.22(1H, m), 1.92(3H, s), 1.41-1.65(6H, m), 0.99-1.15 (2H, brm).
MASS (EI) m/z: 415(M⁺), 321, 292, 239, 211, 204, 136, 123, 116, 96, 82, 78, 67, 55.

The following compounds were synthesized according to similar procedures to those described in Examples 1 - 58. The following ¹H-NMR data show only their characteristic peaks measured by 200 MHz or 270 MHz NMR instruments. The symbols, ┌ 3-H┘, ┌ 5-H┘, ┌ 3-CH₃┘, and ┌ 5-CH₃┘ indicate the peaks due to the proton or methyl protons at the 3- or 5-positions in the pyrazole ring, respectively.

### (Example 59)

### Compound of Formula No. A-1

¹H-NMR (CDCl₃) δ(ppm) : 7.25 (1H, s, 3-H), 7.03 (1H, s, 5-H), 4.80 (2H, s, NCH₂Ar), 3.84 (2H, d, J=7Hz, NCH₂iPr), 1.98(3H, s, COCH₃).
MASS (EI) m/z : 296(M⁺), 254, 211, 198, 180, 138, 116.

### (Example 60)

### Compound of Formula No. A-2

¹H-NMR (CDCl₃) δ(ppm) : 7.33 (1H, s, 3-H), 7.09 (1H, s, 5-H), 4.82 (2H, s, NCH₂Ar), 3.84 (2H, d, J=7Hz, NCH₂iPr), 1.67-1.54 (1H, m, COCH(CH₂)₂).
MASS (EI) m/z : 322(M⁺), 254, 198, 138, 116.

### (Example 61)

### Compound of Formula No. A-3

¹H-NMR (CDCl₃) δ(ppm) : 7.24 (1H, s, 3-H), 7.03 (1H, s, 5-H), 4.83 (2H, s, NCH₂Ar), 3.94 (2H, d, J=4Hz, COCH₂O), 3.84 (2H, d, J=7Hz, NCH₂iPr).
MASS (EI) m/z : 312(M⁺), 281, 254, 211, 196, 166, 116.

### (Example 62)

### Compound of Formula No. A-4

¹H-NMR (CDCl₃) δ(ppm) : 7.24 (1H, s, 3-H), 7.04 (1H, s, 5-H), 4.80 (2H, s, NCH₂Ar), 3.90 (2H, s, COCH₂O), 3.84 (2H, d, J=7Hz, NCH₂iPr).
MASS (EI) m/z : 326(M⁺), 311, 296, 281, 267, 253, 116.

### (Example 63)

### Compound of Formula No. A-5

¹H-NMR (CDCl₃) δ(ppm) : 7.29 (1H, s, 3-H), 7.09 (1H, s, 5-H), 4.82 (2H, s, NCH₂Ar), 4.50 (2H, s, COCH₂O), 3.84 (2H, d, J=7Hz, NCH₂iPr).
MASS (EI) m/z : 388(M⁺), 295, 281, 267, 253, 211, 197.

### (Example 64)

### Compound of Formula No. A-6

¹H-NMR (CDCl₃) δ(ppm) : 7.23 (1H, s, 3-H), 7.01 (1H, s, 5-H), 4.79 (2H, s, NCH₂Ar), 4.10 (2H, s, COCH₂O), 3.83 (2H, d, J=7Hz, NCH₂iPr).
MASS (EI) m/z : 425(M-1), 411, 369, 210, 149, 116.

### (Example 65)

### Compound of Formula No. A-7

¹H-NMR (CDCl₃) δ(ppm) : 7.29 (1H, s, 3-H), 7.10 (1H, s, 5-H), 4.78 (2H, s, NCH₂Ar), 4.48 (2H, s, COCH₂O), 3.84 (2H, d, J=7Hz, NCH₂iPr).
MASS (EI) m/z : 354(M⁺), 311, 254, 211, 198, 166, 116.

### (Example 66)

### Compound of Formula No. A-8

¹H-NMR (CDCl₃) δ(ppm) : 7.29 (1H, s, 3-H), 7.11 (1H, s, 5-H), 4.79 (2H, s, NCH₂Ar), 4.51 (2H, s, COCH₂O), 3.84 (2H, d, J=7Hz, NCH₂iPr).
MASS (EI) m/z : 394(M⁺), 313, 254, 198, 166, 116.

### (Example 67)

### Compound of Formula No. A-9

¹H-NMR (CDCl₃) δ(ppm) : 7.30 (1H, s, 3-H), 7.11 (1H, s, 5-H), 4.79 (2H, s, NCH₂Ar), 4.50 (2H, s, COCH₂O), 3.84 (2H, d, J=7Hz, NCH₂iPr).
MASS (EI) m/z : 420(M⁺), 313, 254, 167, 138, 116.

### (Example 68)

### Compound of Formula No. A-10

¹H-NMR (CDCl₃) δ(ppm) : 7.34 (1H, s, 3-H), 7.16 (1H, s, 5-H), 4.81 (2H, S, NCH₂Ar), 4.74 (2H, s, COCH₂O), 3.87 (2H, d, J=7Hz, NCH₂iPr).
MASS (EI) m/z : 438(M⁺), 313, 295, 279, 254, 239, 211.

### (Example 69)

### Compound of Formula No. A-11

¹H-NMR (CDCl₃) δ(ppm) : 7.27 (1H, s, 3-H), 7.11 (1H, brs, 5-H), 4.85 (2H, brs, NCH₂Ar), 3.81 (2H, d, J=7Hz, NCH₂iPr), 3.79(3H, s, COOCH₃).
MASS (EI) m/z : 312(M⁺), 297, 269, 256, 196, 154, 122.

### (Example 70)

### Compound of Formula No. A-12

¹H-NMR (CDCl₃) δ(ppm) : 7.38 (1H, s, 3-H), 7.11 (1H, brs, 5-H), 5.08 (1H, brs, NCH₂Ar), 4.88 (1H, brs, NCH₂Ar), 3.84 (2H, d, J=7Hz, NCH₂iPr).
MASS (EI) m/z : 374(M⁺), 281, 225, 116.

### (Example 71)

### Compound of Formula No. A-13

¹H-NMR (CDCl₃) δ(ppm) : 7.32 (1H, s, 3-H), 7.10 (1H, s, 5-H), 4.76 (2H, s, NCH₂Ar), 4.67 (2H, s, CONH₂), 3.83 (2H, d, J=7Hz, NCH₂iPr).
MASS (EI) m/z : 297(M⁺), 254, 211, 198, 181, 138, 116.

### (Example 72)

### Compound of Formula No. A-14

¹H-NMR (CDCl₃) δ(ppm) : 7.31 (1H, s, 3-H), 7.12 (1H, s, 5-H), 4.81 (2H, s, NCH₂Ar), 4.06 (2H, d, J=7Hz, NCH₂cBu), 1.67-1.54 (1H, m, COCH(CH₂)₂).
MASS (EI) m/z : 334(M⁺), 226, 198, 150, 116.

### (Example 73)

### Compound of Formula No. A-15

¹H-NMR (CDCl₃) δ(ppm) : 7.22 (1H, s, 3-H), 7.04 (1H, s, 5-H), 4.85 (2H, s, NCH₂Ar), 4.06 (2H, d, J=7Hz, NCH₂cBu), 3.92 (2H, d, J=4Hz, COCH₂O).
MASS (EI) m/z : 324(M⁺), 266, 225, 208, 198, 178.

### (Example 74)

### Compound of Formula No. A-16

¹H-NMR (CDCl₃) δ(ppm) : 7.22 (1H, s, 3-H), 7.04 (1H, s, 5-H), 4.79 (2H, s, NCH₂Ar), 4.06 (2H, d, J=7Hz, MCH₂cBu), 3.88 (2H, s, COCH₂O).
MASS (EI) m/z : 338(M⁺), 323, 310, 279, 265, 211, 197.

### (Example 75)

### Compound of Formula No. A-17

¹H-NMR (CDCl₃) δ(ppm) : 7.27 (1H, s, 3-H), 7.12 (1H, S, 5-H), 4.81 (2H, s, NCH₂Ar), 4.49 (2H, s, COCH₂O), 4.05 (2H, d, J=7Hz, NCH₂cBu).
MASS (EI) m/z : 400(M⁺), 307, 279, 265, 227, 211, 167.

### (Example 76)

### Compound of Formula No. A-18

¹H-NMR (CDCl₃) δ(ppm) : 7.47 (1H, s, 3-H), 7.10 (1H, s, 5-H), 4.78 (2H, s, NCH₂Ar), 4.47 (2H, s, COCH₂O), 4.06 (2H, d, J=7Hz, NCH₂cBu).
MASS (EI) m/z : 366(M⁺), 266, 238, 198, 178, 116.

### (Example 77)

### Compound of Formula No. A-19

¹H-NMR (CDCl₃) δ(ppm) : 7.26 (1H, s, 3-H), 7.12 (1H, brs, 5-H), 4.84 (2H, brs, NCH₂Ar), 3.79 (3H, s, COOCH₃), 4.04 (2H, d, J=7Hz, NCH₂cBu).
MASS (EI) m/z : 324(M⁺), 296, 269, 256, 227, 208, 180.

### (Example 78)

### Compound of Formula No. A-20

¹H-NMR (CDCl₃) δ(ppm) : 7.36 (1H, s, 3-H), 7.11 (1H, brs, 5-H), 5.07 (1H, brs, NCH₂Ar), 4.87 (1H, brs, NCH₂Ar), 4.05 (2H, d, J=7Hz, NCH₂cBu).
MASS (EI) m/z : 386(M⁺), 293, 225, 116.

### (Example 79)

### Compound of Formula No. A-21

¹H-NMR (CDCl₃) δ(ppm) : 7.33 (1H, s, 3-H), 7.07 (1H, s, 5-H), 4.82 (2H, s, NCH₂Ar), 3.82 (2H, s, NCH₂tBu), 1.66-1.54 (1H, m, COCH(CH₂)₂).
MASS (EI) m/z : 336(M⁺), 321, 268, 211, 152, 116.

### (Example 80)

### Compound of Formula No. A-22

¹H-NMR (CDCl₃ δ(ppm) : 7.30 (1H, s, 3-H), 7.09 (1H, s, 5-H), 4.82 (2H, s, NCH₂Ar), 3.95 (2H, s, NCH₂tBu), 3.83 (2H, s, COCH₂Cl).
MASS (EI) m/z : 344(M⁺), 329, 287, 265, 228, 172, 116.

### (Example 81)

### Compound of Formula No. A-23

¹H-NMR (CDCl₃) δ(ppm) : 7.25 (1H, s, 3-H), 7.04 (1H, s, 5-H), 4.85 (2H, s, NCH₂Ar), 3.95 (2H, d, J=4Hz, COCH₂O), 3.83 (2H, s, NCH₂tBu).
MASS (EI) m/z : 326(M⁺), 311, 268, 210, 197, 154, 116.

### (Example 82)

### Compound of Formula No. A-24

¹H-NMR (CDCl₃) δ(ppm) : 7.25 (1H, s, 3-H), 6.98 (1H, s, 5-H), 4.81 (2H, brs, NCH₂Ar), 3.89 (2H, s, COCH₂O), 3.81 (2H, s, NCH₂tBu).
MASS (EI) m/z : 340(M⁺), 325, 281, 267, 224, 211.

### (Example 83)

### Compound of Formula No. A-25

¹H-NMR (CDCl₃) δ(ppm) : 7.30 (1H, s, 3-H), 7.08 (1H, s, 5-H), 4.83 (2H, s, NCH₂Ar), 4.51 (2H, s, COCH₂O), 3.82 (2H, s, NCH₂tBu).
MASS (EI) m/z : 402(M⁺), 387, 309, 281, 267, 239, 211.

### (Example 84)

### Compound of Formula No. A-26

¹H-NMR (CDCl₃) δ(ppm) : 7.23 (1H, s, 3-H), 6.98 (1H, s, 5-H), 4.79 (2H, s, NCH₂Ar), 4.10 (2H, s, COCH₂O), 3.82 (2H, s, NCH₂tBu).
MASS (EI) m/z : 439(M-1), 425, 383, 210, 149, 116.

### (Example 85)

### Compound of Formula No. A-27

¹H-NMR (CDCl₃) δ(ppm) : 7.30 (1H, s, 3-H), 7.07 (1H, s, 5-H), 4.80 (2H, s, NCH₂Ar), 4.49 (2H, s, COCH₂O), 3.84 (2H, s, NCH₂tBu).
MASS (EI) m/z : 368(M⁺), 353, 311, 268, 211, 196, 152.

### (Example 86)

### Compound of Formula No. A-28

¹H-NMR (CDCl₃) δ(ppm) : 7.29 (1H, s, 3-H), 7.08 (1H, s, 5-H), 4.80 (2H, s, NCH₂Ar), 4.51 (2H, s, COCH₂O), 3.82 (2H, s, NCH₂tBu).
MASS (EI) m/z : 408(M⁺), 327, 268, 211, 180, 116.

### (Example 87)

### Compound of Formula No. A-29

¹H-NMR (CDCl₃) δ(ppm) : 7.31 (1H, s, 3-H), 7.09 (1H, s, 5-H), 4.80 (2H, s, NCH₂Ar), 4.51 (2H, s, COCH₂O), 3.83 (2H, s, NCH₂tBu).
MASS (EI) m/z : 434(M⁺), 419, 341, 327, 311, 281, 268.

### (Example 88)

### Compound of Formula No. A-30

¹H-NMR (CDCl₃) δ(ppm) : 7.35 (1H, s, 3-H), 7.13 (1H, s, 5-H), 4.81 (2H, s, NCH₂Ar), 4.74 (2H, s, COCH₂O), 3.85 (2H, s, NCH₂tBu).
MASS (EI) m/z : 452(M⁺), 437, 309, 268, 239, 211, 157.

### (Example 89)

### Compound of Formula No. A-31

¹H-NMR (CDCl₃) δ(ppm) : 7.29 (1H, s, 3-H), 7.07 (1H, s, 5-H), 4.86 (2H, brs, NCH₂Ar), 4.47 (2H, s, COCH₂O), 3.82 (2H, s, NCH₂tBu).
MASS (EI) m/z : 369(M⁺), 326, 311, 268, 211, 197, 154.

### (Example 90)

### Compound of Formula No. A-32

¹H-NMR (CDCl₃) δ(ppm) : 7.28 (1H, s, 3-H), 7.09 (1H, brs, 5-H), 4.85 (2H, brs, NCH₂Ar), 3.81 (2H, s, NCH₂tBu), 3.80 (3H, s, COOCH₃).
MASS (EI) m/z : 326(M⁺), 311, 269, 210, 154, 116.

### (Example 91)

### Compound of Formula No. A-33

¹H-NMR (CDCl₃) δ(ppm) : 7.37 (1H, s, 3-H), 7.11 (1H, brs, 5-H), 5.09 (1H, brs, NCH₂Ar), 4.89 (1H, brs, NCH₂Ar), 3.81 (2H, s, NCH₂tBu).
MASS (EI) m/z : 326(M⁺), 311, 269, 210, 154, 116.

### (Example 92)

### Compound of Formula No. A-34

¹H-NMR (CDCl₃) δ(ppm) : 7.32 (1H, s, 3-H), 7.07 (1H, s, 5-H), 4.81 (2H, brs, NCH₂Ar), 4.77 (2H, s, CONH₂), 3.81 (2H, s, NCH₂tBu).
MASS (EI) m/z : 311(M⁺), 296, 268, 254, 211, 152, 139.

### (Example 93)

### Compound of Formula No. A-35

¹H-NMR (CDCl₃) δ(ppm) : 7.37 (1H, s, 3-H), 7.11 (1H, s, 5-H), 5.24 (2H, s, NCH₂Ph), 4.79 (2H, s, NCH₂Ar), 1.66-1.54 (1H, m, COCH(CH₂)₂).
MASS (EI) m/z : 356(M⁺), 288, 172, 116.

### (Example 94)

### Compound of Formula No. A-36

¹H-NMR (CDCl₃) δ(ppm) : 7.27 (1H, s, 3-H)-H), 7.04 (1H, s, 5-H), 5.23 (2H, s, NCH₂Ph), 4.80 (2H, s, NCH₂Ar), 3.92 (2H, d, J=4Hz, COCH₂O).
MASS (EI) m/z : 346(M⁺), 288, 172, 157, 116.

### (Example 95)

### Compound of Formula No. A-37

¹H-NMR (CDCl₃) δ(ppm) : 7.36 (1H, s, 3-H), 7.05 (1H, s, 5-H), 5.24 (2H, s, NCH₂Ph), 4.77 (2H, s, NCH₂Ar), 3.87 (2H, s, COCH₂O). MASS (EI) m/z : 360(M⁺), 301, 287, 157, 116.

### (Example 96)

### Compound of Formula No. A-38

¹H-NMR (CDCl₃) δ(ppm) : 7.32 (1H, s, 3-H), 7.10 (1H, s, 5-H)-H), 5.22 (2H, s, NCH₂Ph), 4.79 (2H, s, NCH₂Ar), 4.47 (2H, s, COCH₂O). MASS (EI) m/z : 422(M⁺), 301, 287, 227, 121.

### (Example 97)

### Compound of Formula No. A-39

¹H-NMR (CDCl₃) δ(ppm) : 7.32 (1H, s, 3-H), 7.10 (1H, s, 5-H), 5.23 (2H, s, NCH₂Ph), 4.76 (2H, s, NCH₂Ar), 4.47 (2H, s, COCH₂O). MASS (EI) m/z : 388(M⁺), 288, 200, 172, 116.

### (Example 98)

### Compound of Formula No. A-40

¹H-NMR (CDCl₃) δ(ppm) : 7.31 (1H, s, 3-H), 7.18 (1H, brs, 5-H), 5.21 (2H, s, NCH₂Ph), 4.83 (2H, brs, NCH₂Ar), 3.78 (3H, s, COOCH₃).
MASS (EI) m/z : 346(M⁺).

### (Example 99)

### Compound of Formula No. A-41

¹H-NMR (CDCl₃) δ(ppm) : 7.33 (1H, s, 3-H), 7.09 (1H, brs, 5-H), 5.22 (2H, s, NCH₂Ph), 5.06 (1H, brs, NCH₂Ar), 4.85 (1H, brs, NCH₂Ar).
MASS (EI) m/z : 408(M⁺), 315, 206, 198, 116.

### (Example 100)

### Compound of Formula No. A-42

¹H-NMR (CDCl₃) δ(ppm) : 7.17 (1H, s, 3-H), 6.95 (1H, s, 5-H), 4.79 (2H, s, NCH₂Ar), 3.66 (2H, s, NCH₂SiMe₃), 1.98 (3H, s, COCH₃).
MASS (EI) m/z : 326(M⁺), 311, 283, 210, 184, 168, 139.

### (Example 101)

### Compound of Formula No. A-43

¹H-NMR (CDCl₃) δ(ppm) : 7.26 (1H, s, 3-H), 7.01 (1H, s, 5-H), 4.81 (2H, s, NCH₂Ar), 3.67 (2H, s, NCH₂SiMe₃), 1.68-1.56 (1H, m, COCH(CH₂)₂).
MASS (EI) m/z : 352(M⁺), 337, 284, 269, 236, 168, 139.

### (Example 102)

### Compound of Formula No. A-44

¹H-NMR (CDCl₃) δ(ppm) : 7.21 (1H, s, 3-H), 7.05 (1H, s, 5-H), 4.80 (2H, s, NCH₂Ar), 3.96 (2H, s, NCH₂tBu), 3.67 (2H, s, COCH₂Cl).
MASS (EI) m/z : 360(M⁺), 345, 283, 244, 189, 168, 116, 73.

### (Example 103)

### Compound of Formula No. A-45

¹H-NMR (CDCl₃) δ(ppm) : 7.16 (1H, s, 3-H), 6.94 (1H, s, 5-H), 4.82 (2H, s, NCH₂Ar), 3.93 (2H, d, J=4.7Hz, COCH₂O), 3.67 (2H, s, NCH₂tBu).
MASS (EI) m/z : 342(M⁺), 327, 311, 283, 226, 116, 73.

### (Example 104)

### Compound of Formula No. A-46

¹H-NMR (CDCl₃) δ(ppm) : 7.17 (1H, s, 3-H), 6.94 (1H, s, 5-H), 4.79 (2H, s, NCH₂Ar), 3.89 (2H, s, COCH₂O), 3.67 (2H, s, NCH₂SiMe₃).
MASS (EI) m/z : 356(M⁺), 341, 311, 297, 283, 240, 180.

### (Example 105)

### Compound of Formula No. A-47

¹H-NMR (CDCl₃) δ(ppm) : 7.22 (1H, s, 3-H), 7.02 (1H, s, 5-H), 4.81 (2H, s, NCH₂Ar), 4.50 (2H, s, COCH₂O), 3.67 (2H, s, NCH₂SiMe₃).
MASS (EI) m/z : 418(M⁺), 403, 325, 311, 283, 267, 209.

### (Example 106)

### Compound of Formula No. A-48

¹H-NMR (CDCl₃) δ(ppm) : 7.15 (1H, s, 3-H), 6.94 (1H, s, 5-H), 4.78 (2H, s, NCH₂Ar), 4.11 (2H, S, COCH₂O), 3.67 (2H, S, NCH₂SiMe₃).
MASS (EI) m/z : 455(M-1), 441, 399, 282, 268, 210, 116.

### (Example 107)

### Compound of Formula No. A-49

¹H-NMR (CDCl₃) δ(ppm) : 7.21 (1H, s, 3-H), 7.01 (1H, s, 5-H), 4.77 (2H, s, NCH₂Ph), 4.48 (2H, s, NCH₂Ar), 3.66 (2H, s, COCH₂O). MASS (EI) m/z : 384(M⁺), 369, 309, 283, 268, 196, 189, 180.

### (Example 108)

### Compound of Formula No. A-50

¹H-NMR (CDCl₃) δ(ppm) : 7.27 (1H, s, 3-H), 7.07 (1H, s, 5-H), 4.80 (2H, s, NCH₂Ar), 4.74 (2H, s, COCH₂O), 3.69 (2H, s, NCH₂SiMe₃).
MASS (EI) m/z : 468(M⁺), 453, 352, 309, 283, 180, 157.

### (Example 109)

### Compound of Formula No. A-51

¹H-NMR (CDCl₃) δ(ppm) : 7.21 (1H, s, 3-H), 7.02 (1H, s, 5-H), 4.88 (2H, brs, NCH₂Ar), 4.47 (2H, s, COCH₂O), 3.66 (2H, s, NCH₂SiMe₃).
MASS (EI) m/z : 385(M⁺), 370, 342, 327, 309, 284, 269.

### (Example 110)

### Compound of Formula No. A-52

¹H-NMR (CDCl₃) δ(ppm) : 7.21 (1H, s, 3-H), 7.03 (1H, brs, 5-H), 4.84 (2H, brs, NCH₂Ar), 3.79 (3H, brs, COOCH₃), 3.65 (2H, s, NCH₂SiMe₃).
MASS (EI) m/z : 342(M⁺), 327, 283, 226, 153, 122.

### (Example 111)

### Compound of Formula No. A-53

¹H-NMR (CDCl₃) δ(ppm) : 7.26 (1H, s, 3-H), 7.14 (1H, brs, 5-H), 5.08 (1H, brs, NCH₂Ar), 4.88 (1H, brs, NCH₂Ar), 3.67 (2H, s, NCH₂SiMe₃).
MASS (EI) m/z : 404(M⁺), 389, 311, 295, 283, 194, 116.

### (Example 112)

### Compound of Formula No. A-54

¹H-NMR (CDCl₃) δ(ppm) : 7.22 (1H, s, 3-H), 6.99 (1H, s, 5-H), 4.78 (2H, brs, NCH₂Ar), 4.73 (2H, s, CONH₂), 3.62 (2H, s, NCH₂SiMe₃).
MASS (EI) m/z : 327(M⁺), 312, 284, 269, 211, 184, 153.

### (Example 113)

### Compound of Formula No. A-55

¹H-NMR (CDCl₃) δ(ppm) : 7.23 (1H, s, 3-H), 6.94 (1H, s, 5-H), 4.85 (2H, s, NCH₂Ar), 3.91 (2H, d, J=4Hz, COCH₂O), 3.79 (2H, s, NCH₂tBu).
MASS (EI) m/z : 359(M⁺), 344, 326, 300, 285, 268, 210.

### (Example 114)

### Compound of Formula No. A-56

¹H-NMR (CDCl₃) δ(ppm) : 7.27 (1H, s, 3-H), 6.98 (1H, s, 5-H), 5.21 (2H, s, NCH₂Ph), 4.83 (2H, s, NCH₂Ar), 3.91 (2H, brs, COCH₂O).
MASS (EI) m/z : 359(M⁺), 344, 326, 300, 285, 268, 210.

### (Example 115)

### Compound of Formula No. A-57

¹H-NMR (CDCl₃) δ(ppm) : 7.28 (1H, s, 3-H), 7.09 (1H, s, 5-H), 4.82 (2H, s, NCH₂Ar), 4.57 (2H, s, ArOCH₂CO), 3.83 (2H, d, J=7Hz, NCH₂iPr).
MASS (EI) m/z : 406(M⁺), 295, 267, 253, 125, 116.

### (Example 116)

### Compound of Formula No. A-58

¹H-NMR (CDCl₃) δ(ppm) : 7.30 (1H, s, 3-H), 7.14 (1H, s, 5-H), 4.83 (2H, s, NCH₂Ar), 4.57 (2H, s, ArOCH₂CO), 3.82 (2H, d, J=7Hz, NCH₂iPr).
MASS (EI) m/z : 422(M⁺), 387, 295, 267, 253, 141, 116.

### (Example 117)

### Compound of Formula No. A-59

¹H-NMR (CDCl₃) δ(ppm) : 7.27 (1H, s, 3-H), 7.07 (1H, s, 5-H), 4.81 (2H, s, NCH₂Ar), 4.53 (2H, s, ArOCH₂CO), 3.83 (2H, d, J=7Hz, NCH₂iPr).
MASS (EI) m/z : 424(M⁺), 295, 267, 253, 143, 116.

### (Example 118)

### Compound of Formula No. A-60

¹H-NMR (CDCl₃) δ(ppm) : 7.30 (1H, s, 3-H), 7.12 (1H, s, 5-H), 4.82 (2H, s, NCH₂Ar), 4.55 (2H, s, ArOCH₂CO), 3.83 (2H, d, J=7Hz, NCH₂iPr).
MASS (EI) m/z : 456(M⁺), 421, 295, 267, 253, 175, 116.

### (Example 119)

### Compound of Formula No. A-61

¹H-NMR (CDCl₃) δ(ppm) : 7.12 (1H, s, 3-H), 6.82 (1H, s, 5-H), 6.20 (1H, q, J=7Hz, NCH(Me)Ar), 3.82 (1H, d, J=14Hz, NCH₂tBu), 3.77 (1H, d, J=14Hz, NCH₂tBu), 1.51-1.39 (1H, m, COCH(CH₂)₂). MASS (EI) m/z : 350(M⁺), 335, 282, 267, 257, 220.

### (Example 120)

### Compound of Formula No. A-62

¹H-NMR (CDCl₃) δ(ppm) : 7.04 (1H, s, 3-H), 6.78 (1H, s, 5-H), 6.21 (1H, q, J=7Hz, NCH(Me)Ar), 3.82 (1H, d, J=14Hz, COCH₂O), 3.79 (1H, d, J=14Hz, COCH₂O), 3.79 (1H, d, J=15Hz, NCH₂tBu), 3.70 (1H, d, J=15Hz, NCH₂tBu).
MASS (EI) m/z : 354(M⁺), 339, 281, 225, 210, 195.

### (Example 121)

### Compound of Formula No. A-63

¹H-NMR (CDCl₃) δ(ppm) : 7.11 (1H, s, 3-H), 6.88 (1H, s, 5-H), 6.14 (1H, q, J=7Hz, NCH(Me)Ar), 4.40 (1H, d, J=15Hz, COCH₂O), 4.35 (1H, d, J=15Hz, COCH₂O), 3.83 (1H, d, J=14Hz, NCH₂tBu), 3.78 (1H, d, J=14Hz, NCH₂tBu).
MASS (EI) m/z : 382(M⁺), 367, 325, 280, 267, 253, 238.

### (Example 122)

### Compound of Formula No. A-64

¹H-NMR (CDCl₃) δ(ppm) : 7.07 (1H, s, 3-H), 6.84 (1H, s, 5-H), 6.18 (1H, q, J=7Hz, NCH(Me)Ar), 4.40 (1H, d, J=14Hz, COCH₂O), 4.37 (1H, d, J=14Hz, COCH₂O), 3.82 (1H, d, J=14Hz, NCH₂tBu), 3.76 (1H, d, J=14Hz, NCH₂tBu).
MASS (EI) m/z : 416(M⁺), 401, 323, 287, 257, 231, 166.

### (Example 123)

### Compound of Formula No. A-65

¹H-NMR (CDCl₃) δ(ppm) : 7.11 (1H, s, 3-H), 6.88 (1H, s, 5-H), 6.19 (1H, q, J=7Hz, NCH(Me)Ar), 4.59 (1H, d, J=15Hz, COCH₂O), 4.54 (1H, d, J=15Hz, COCH₂O), 3.83 (1H, d, J=14Hz, NCH₂tBu), 3.77 (1H, d, J=14Hz, NCH₂tBu).
MASS (EI) m/z : 421(M⁺), 406, 322, 292, 280, 263, 235.

### (Example 124)

### Compound of Formula No. A-66

¹H-NMR (CDCl₃) δ(ppm) : 7.04 (1H, s, 3-H), 6.78 (1H, s, 5-H), 5.77 (1H, q, J=7Hz, NCH(Me)Ar), 3.81 (1H, d, J=14Hz, NCH₂tBu), 3.74 (1H, d, J=14Hz, NCH₂tBu), 3.69 (3H, brs, COOCH₃).
MASS (EI) m/z : 340(M⁺), 325, 211, 154, 140, 130.

### (Example 125)

### Compound of Formula No. A-67

¹H-NMR (CDCl₃) δ(ppm) : 7.16 (1H, s, 3-H), 6.92 (1H, brs, 5-H), 5.81 (1H, q, J=7Hz, NCH(Me)Ar), 3.81 (1H, d, J=14Hz, NCH₂tBu), 3.76 (1H, d, J=14Hz, NCH₂tBu).
MASS (EI) m/z : 402(M⁺), 387, 309, 180, 130.

### (Example 126)

### Compound of Formula No. A-70

¹H-NMR (CDCl₃) δ(ppm) : 7.04(1H, s, 3-H), 6.77(1H, s, 5-H), 6.21(1H, q, J=7Hz, NCH(Me)Ar), 3.81(1H, d, J=14Hz, NCH₂CH), 3.78(1H, d, J=14Hz, NCH₂CH), 1.89(3H, s, COMe), 1.43(3H, d, J=7Hz, NCH(Me)Ar).
MASS (EI) m/z : 324(M⁺), 309, 282, 267, 211, 194, 180, 152.

### (Example 127)

### Compound of Formula No. A-71

¹H-NMR (CDCl₃) δ(ppm) : 7.34 (1H, s, 3-H), 7.26 (1H, s, 5-H), 4.82 (2H, s, NCH₂Ar), 4.81 (2H, s, COCH₂O), 3.88 (3H, s, NCH₃). MASS (EI) m/z : 347(M⁺), 210, 136, 116.

### (Example 128)

### Compound of Formula No. A-72

¹H-NMR (CDCl₃) δ(ppm) : 7.37 (1H, s, 3-H), 7.18 (1H, s, 5-H), 4.82 (2H, s, NCH₂Ar), 4.79 (2H, s, COCH₂O), 3.85 (2H, d, J=7Hz, NCH₂iPr).
MASS (EI) m/z : 389(M⁺), 282, 254, 239, 209, 136, 116.

### (Example 129)

### Compound of Formula No. A-73

¹H-NMR (CDCl₃) δ(ppm) : 7.24 (1H, s, 3-H), 7.15 (1H, s, 5-H), 4.95 (2H, s, NCH₂Ar), 4.80 (2H, s, COCH₂O), 3.85 (2H, d, J=7Hz, NCH₂iPr).
MASS (EI) m/z : 424(M⁺), 254, 171.

### (Example 130)

### Compound of Formula No. A-74

¹H-NMR (CDCl₃) δ(ppm) : 7.36 (1H, s, 3-H), 7.18 (1H, s, 5-H), 4.85 (2H, s, NCH₂Ar), 4.82 (2H, s, COCH₂O), 3.86 (2H, d, J=7Hz, NCH₂iPr).
MASS (EI) m/z : 390(M⁺), 254, 137.

### (Example 131)

### Compound of Formula No. A-75

¹H-NMR (CDCl₃) δ(ppm) : 7.31 (1H, s, 3-H), 7.16 (1H, s, 5-H), 4.82 (2H, s, NCH₂Ar), 4.69 (2H, s, COCH₂O), 3.84 (2H, d, J=7Hz, NCH₂iPr).
MASS (EI) m/z : 393(M⁺), 294, 252, 196, 140, 122.

### (Example 132)

### Compound of Formula No. A-76

¹H-NMR (CDCl₃) δ(ppm) : 7.21 (1H, s, 3-H), 6.99 (1H, s, 5-H), 4.79 (2H, s, NCH₂Ar), 4.78 (2H, s, COCH₂O), 3.82 (2H, d, J=7Hz, NCH₂iPr).
MASS (EI) m/z : 378(M⁺), 310, 254, 116.

### (Example 133)

### Compound of Formula No. A-77

¹H-NMR (CDCl₃) δ(ppm) : 7.33 (1H, s, 3-H), 7.14 (1H, s, 5-H), 4.82 (2H, s, NCH₂Ar), 4.80 (2H, s, COCH₂N), 3.85 (2H, d, J=7Hz, NCH₂iPr).
MASS (EI) m/z : 362(M⁺), 294, 252.

### (Example 134)

### Compound of Formula No. A-78

¹H-NMR (CDCl₃) δ(ppm) : 7.33 (1H, s, 3-H), 7.13 (1H, s, 5-H), 4.80 (2H, s, NCH₂Ar), 4.75 (2H, s, COCH₂N), 3.86 (2H, d, J=5.8Hz, NCH₂iPr), 3.82(3H, s, OMe).
MASS (EI) m/z : 396(M⁺), 294, 252.

### (Example 135)

### Compound of Formula No. A-79

¹H-NMR (CDCl₃) δ(ppm) : 7.33 (1H, s, 3-H), 7.10 (1H, s, 5-H), 4.80 (2H, s, NCH₂Ar), 4.76 (2H, s, COCH₂N), 3.86 (2H, d, J=7Hz, NCH₂iPr).
MASS (EI) m/z : 440(M⁺), 294, 252, 196, 159, 116.

### (Example 136)

### Compound of Formula No. A-80

¹H-NMR (CDCl₃) δ(ppm) : 7.37 (1H, s, 3-H), 7.13 (1H, s, 5-H), 4.80 (2H, s, NCH₂Ar), 4.79 (2H, s, COCH₂N), 3.86 (2H, d, J=7Hz, NCH₂iPr).
MASS (EI) m/z : 420(M⁺), 389, 294, 266, 252, 209, 196, 139.

### (Example 137)

### Compound of Formula No. A-81

¹H-NMR (CDCl₃) δ(ppm) : 7.36 (1H, s, 3-H), 7.15 (1H, s, 5-H), 4.83 (2H, S, NCH₂Ar), 4.81 (2H, s, COCH₂N), 3.88 (2H, d, J=7Hz, NCH₂iPr).
MASS (EI) m/z : 387(M⁺), 252, 196, 116.

### (Example 138)

### Compound of Formula No. A-82

¹H-NMR (CDCl₃) δ(ppm) : 7.36 (1H, s, 3-H), 7.16 (1H, s, 5-H), 4.83 (2H, s, NCH₂Ar), 4.81 (2H, s, COCH₂N), 3.88 (2H, d, J=7Hz, NCH₂iPr).
MASS (EI) m/z : 407(M⁺), 291, 252, 209, 196, 178, 126, 116.

### (Example 139)

### Compound of Formula No. A-83

¹H-NMR (CDCl₃) δ(ppm) : 7.34 (1H, s, 3-H), 7.14 (1H, s, 5-H), 4.81 (2H, s, NCH₂Ar), 4.77 (2H, s, COCH₂O), 3.81 (2H, s, NCH₂tBu).
MASS (EI) m/z : 403(M⁺), 388, 268, 239, 209, 197, 136.

### (Example 140)

### Compound of Formula No. A-84

¹H-NMR (CDCl₃) δ(ppm) : 7.21 (1H, s, 3-H), 7.11 (1H, s, 5-H), 4.95 (2H, s, NCH₂Ar), 4.80 (2H, s, COCH₂O), 3.84 (2H, s, NCH₂tBu).
MASS (EI) m/z : 438(M⁺), 423, 326, 268, 171.

### (Example 141)

### Compound of Formula No. A-85

¹H-NMR (CDCl₃) δ(ppm) : 7.36 (1H, s, 3-H), 7.20 (1H, s, 5-H), 4.85 (2H, s, NCH₂Ar), 4.83 (2H, s, COCH₂O), 3.85 (2H, s, NCH₂tBu).
MASS (EI) m/z : 393(M⁺), 378, 309, 266, 209, 116.

### (Example 142)

### Compound of Formula No. A-86

¹H-NMR (CDCl₃) δ(ppm) : 7.32 (1H, s, 3-H), 7.11 (1H, s, 5-H), 4.83 (2H, s, NCH₂Ar), 4.79 (2H, s, COCH₂O), 3.83 (2H, s, NCH₂tBu).
MASS (EI) m/z : 404(M⁺), 389, 268, 137.

### (Example 143)

### Compound of Formula No. A-87

¹H-NMR (CDCl₃) δ(ppm) : 7.31 (1H, s, 3-H), 7.13 (1H, s, 5-H), 4.82 (2H, s, NCH₂Ar), 4.69 (2H, s, COCH₂O), 3.83 (2H, s, NCH₂tBu).
MASS (EI) m/z : 407(M⁺), 392, 326, 266, 228, 197.

### (Example 144)

### Compound of Formula No. A-88

¹H-NMR (CDCl₃) δ(ppm) : 7.33 (1H, s, 3-H), 7.10 (1H, s, 5-H), 4.81 (2H, s, NCH₂Ar), 4.81 (2H, s, COCH₂N), 3.84 (2H, s, NCH₂tBu).
MASS (EI) m/z : 376(M⁺), 361, 308, 266.

### (Example 145)

### Compound of Formula No. A-89

¹H-NMR (CDCl₃) δ(ppm) : 7.33 (1H, s, 3-H), 7.10 (1H, s, 5-H), 4.80 (2H, s, NCH₂Ar), 4.74 (2H, s, COCH₂N), 3.85 (2H, s, NCH₂tBu).
MASS (EI) m/z : 410(M⁺), 395, 266, 116.

### (Example 146)

### Compound of Formula No. A-90

¹H-NMR (CDCl₃) δ(ppm) : 7.33 (1H, s, 3-H), 7.09 (1H, s, 5-H), 4.80 (2H, s, NCH₂Ar), 4.76 (2H, s, COCH₂N), 3.84 (2H, s, NCH₂tBu).
MASS (EI) m/z : 454(M⁺), 280, 266, 209, 167, 149.

### (Example 147)

### Compound of Formula No. A-91

¹H-NMR (CDCl₃) δ(ppm) : 7.35 (1H, s, 3-H), 7.13 (1H, s, 5-H), 4.81 (2H, s, NCH₂Ar), 4.81(2H, s, COCH₂N), 3.85 (2H, d, J=7Hz, NCH₂tBu), 3.82 (3H, s, OMe).
MASS (EI) m/z : 434(M⁺), 403, 280, 266, 209, 139, 116.

### (Example 148)

### Compound of Formula No. A-92

¹H-NMR (CDCl₃) δ(ppm) : 7.36 (1H, s, 3-H), 7.12 (1H, s, 5-H), 4.82 (2H, s, NCH₂Ar), 4.81 (2H, s, COCH₂N), 3.86 (2H, s, NCH₂tBu).
MASS (EI) m/z : 401(M⁺), 386, 285, 266, 229, 116.

### (Example 149)

### Compound of Formula No. A-93

¹H-NMR (CDCl₃) δ(ppm) : 7.36 (1H, s, 3-H), 7.12 (1H, s, 5-H), 4.82 (2H, s, NCH₂Ar), 4.81 (2H, s, COCH₂N), 3.86 (2H, d, J=7Hz, NCH₂tBu).
MASS (EI) m/z : 421(M⁺), 406, 391, 305, 266, 249, 209, 126.

### (Example 150)

### Compound of Formula No. A-94

¹H-NMR (CDCl₃) δ(ppm) : 7.29 (1H, s, 3-H), 7.09 (1H, s, 5-H), 4.82 (2H, s, NCH₂Ar), 4.79 (2H, s, COCH₂O), 3.68 (2H, s, NCH₂Si). MASS (EI) m/z : 419(M⁺), 404, 284, 136, 116.

### (Example 151)

### Compound of Formula No. A-95

¹H-NMR (CDCl₃) δ(ppm) : 7.30 (1H, s, 3-H), 7.11 (1H, s, 5-H), 4.95 (2H, s, NCH₂Ar), 4.80 (2H, s, COCH₂O), 3.68 (2H, s, NCH₂Si). MASS (EI) m/z : 454(M⁺), 439, 284, 171.

### (Example 152)

### Compound of Formula No. A-96

¹H-NMR (CDCl₃) δ(ppm) : 7.29 (1H, s, 3-H), 7.11 (1H, s, 5-H), 4.85 (2H, s, NCH₂Ar), 4.81 (2H, s, COCH₂O), 3.69 (2H, s, NCH₂Si). MASS (EI) m/z : 420(M⁺), 405, 284, 137.

### (Example 153)

### Compound of Formula No. A-97

¹H-NMR (CDCl₃) δ(ppm) : 7.25 (1H, s, 3-H), 7.10 (1H, s, 5-H), 4.82 (2H, s, NCH₂Ar), 4.70 (2H, s, COCH₂O), 3.68 (2H, s, NCH₂Si). MASS (EI) m/z : 423(M⁺), 408, 309, 282, 208, 180, 168.

### (Example 154)

### Compound of Formula No. A-98

¹H-NMR (CDCl₃) δ(ppm) : 7.30 (1H, s, 3-H), 7.24 (1H, s, 5-H), 4.82 (2H, s, NCH₂Ar), 4.50 (2H, s, COCH₂N), 3.68 (2H, s, NCH₂Si). MASS (EI) m/z : 353(M⁺), 295, 267, 239, 225, 195.

### (Example 155)

### Compound of Formula No. A-99

¹H-NMR (CDCl₃) δ(ppm) : 7.26 (1H, s, 3-H), 7.05 (1H, s, 5-H), 4.81 (2H, s, NCH₂Ar), 4.80 (2H, s, COCH₂N), 3.67 (2H, s, NCH₂Si). MASS (EI) m/z : 392(M⁺), 377, 324, 283.

### (Example 156)

### Compound of Formula No. A-100

¹H-NMR (CDCl₃) δ(ppm) : 7.20 (1H, s, 3-H), 7.00 (1H, s, 5-H), 4.74 (2H, s, NCH₂Ar), 4.69 (2H, s, COCH₂N), 3.63 (2H, s, NCH₂Si). MASS (EI) m/z : 426(M⁺), 411, 383, 283.

### (Example 157)

### Compound of Formula No. A-101

¹H-NMR (CDCl₃) δ(ppm) : 7.25 (1H, s, 3-H), 7.03 (1H, s, 5-H), 4.79 (2H, s, NCH₂Ar), 4.76 (2H, s, COCH₂N), 3.68 (2H, s, NCH₂Si). MASS (EI) m/z : 472(M⁺), 457, 354, 324, 283, 159, 116.

### (Example 158)

### Compound of Formula No. A-102

¹H-NMR (CDCl₃) δ(ppm) : 7.26 (1H, s, 3-H), 7.06 (1H, s, 5-H), 4.81 (2H, s, NCH₂Ar), 4.80(2H, s, COCH₂N), 3.82(3H, s, OMe), 3.68(2H, d, J=7Hz, NCH₂Si).
MASS (EI) m/z : 450(M⁺), 435, 419, 345, 334, 283, 267, 230.

### (Example 159)

### Compound of Formula No. A-103

¹H-NMR (CDCl₃) δ(ppm) : 7.28 (1H, s, 3-H), 7.06 (1H, s, 5-H), 4.82 (2H, s, NCH₂Ar), 4.80 (2H, s, COCH₂N), 3.69 (2H, s, NCH₂Si). MASS (EI) m/z : 417(M⁺), 402, 301, 283, 195, 116.

### (Example 160)

### Compound of Formula No. A-104

¹H-NMR (CDCl₃) δ(ppm) : 7.28 (1H, s, 3-H), 7.08 (1H, s, 5-H), 4.81 (2H, s, NCH₂Ar), 4.80 (2H, s, COCH₂N), 3.70 (2H, d, J=7Hz, NCH₂Si).

### (Example 161)

### Compound of Formula No. A-105

¹H-NMR (CDCl₃) δ(ppm) : 7.23 (1H, s, 3-H), 7.07 (1H, s, 5-H), 4.83 (2H, s, NCH₂Ar), 3.97 (2H, d, J = 7.6 Hz, NCH₂CH), 3.94 (2H, d, J = 4.6 Hz, CH₂O), 3.30 (1H, t, J = 4.6 Hz, OH).
MASS (EI) m/z : 338(M⁺), 307, 280, 270, 256, 222, 211, 192, 154, 116, 98, 82.

### (Example 162)

### Compound of Formula No. A-106

¹H-NMR (CDCl₃) δ(ppm) : 5.01 (2H, s, CH₂CN), 4.88 (2H, brs, NCH₂Ar), 3.83 (3H, s, OCH₃).
MASS (EI) m/z : 295(M⁺), 236, 197, 179, 148.

### (Example 163)

### Compound of Formula No. A-107

¹H-NMR (CDCl₃) δ(ppm) : 7.30 (1H, s, 3-H), 7.11 (1H, s, 5-H), 4.81 (2H, s, NCH₂Ar), 3.83 (2H, s, MCH₂-tBu), 3.58 (2H, s, COCH₂), 3.37 (2H, s, SCH₂CN), 0.90 (9H, s, tBu).
MASS (EI) m/z : 381(M⁺), 366, 341, 324, 309, 268, 239, 209, 197, 180, 116, 86.

### (Example 164)

### Compound of Formula No. A-108

¹H-NMR (CDCl₃) δ(ppm) : 7.28 (1H, s, 3-H), 7.14 (1H, s, 5-H), 4.80 (2H, s, NCH₂Ar), 3.97 (2H, d, J = 7.6 Hz, NCH₂CH), 3.58 (2H, S, COCH₂), 3.37 (2H, s, SCH₂CN).
MASS (EI) m/z : 393(M⁺), 353, 321, 279, 239, 211, 197, 116, 86.

### (Example 165)

### Compound of Formula No. A-109

¹H-NMR (CDCl₃) δ(ppm) : 7.27 (1H, s, 3-H), 7.05 (1H, s, 5-H), 4.80 (2H, s, NCH₂Ar), 4.50 (2H, s, COCH₂), 4.10 (2H, s, OCH₂CN), 3.84 (2H, s, NCH₂-tBu), 0.90 (9H, s, tBu).
MASS (EI) m/z : 365(M⁺), 350, 310, 296, 268, 249, 239, 211, 193, 116, 82, 71.

### (Example 166)

### Compound of Formula No. A-110

¹H-NMR (CDCl₃) δ(ppm) : 7.25 (1H, s, 3-H), 7.10 (1H, s, 5-H), 4.79 (2H, s, NCH₂Ar), 4.49 (2H, s, COCH₂), 4.10 (2H, s, SCH₂CN), 3.97 (2H, d, J = 7.6 Hz, NCH₂CH).
MASS (EI) m/z : 377(M⁺),337, 321, 309, 279, 261, 193, 116.

### (Example 167)

### Compound of Formula No. A-111

¹H-NMR (CDCl₃) δ(ppm) : 7.22 (1H, s, 3-H), 6.97 (1H, s, 5-H), 4.80 (2H, s, NCH₂Ar), 4.79 (2H, s, COCH₂O), 3.80 (2 H, s, NCH₂-tBu).
MASS (EI) m/z : 392(M⁺), 377, 309, 125.

### (Example 168)

### Compound of Formula No. A-112

¹H-NMR (CDCl₃) δ(ppm) : 7.14 (1H, s, 3-H), 6.92 (1H, s, 5-H), 4.80 (2H, s, NCH₂Ar), 4.77 (2H, s, COCH₂O), 3.64 (2 H, s, NCH₂Si).
MASS (EI) m/z : 408(M⁺), 393, 340, 73.

### (Example 169)

### Compound of Formula No. A-113

¹H-NMR (CDCl₃) δ(ppm) : 7.65 (1H, s, 3-H), 7.27 (1H, s, 5-H), 4.92 (2H, brs, NCH₂Ar), 3.94 (2H, d, J=7Hz, NCH₂-cPent), 3.80 (s, 3H, COOCH₃).
MASS (EI) m/z : 358(M⁺), 290, 276, 222, 154.

### (Example 170)

### Compound of Formula No. A-114

¹H-NMR (CDCl₃) δ(ppm) : 7.50 (1H, s, 3-H), 7.28 (1H, s, 5-H), 4.93 (2H, brs, NCH₂Ar), 3.94 (2H, d, J=8Hz, NCH₂-cPent), 3.80 (s, 3H, COOCH₃).
MASS (EI) m/z : 358(M⁺), 289, 276, 259, 222.

### (Example 171)

### Compound of Formula No. A-115

¹H-NMR (CDCl₃) δ(ppm) : 7.26 (1H, s, 3-H), 7.18 (1H, s, 5-H), 4.78 (2H, brs, NCH₂Ar), 3.92 (2H, d, J=8Hz, NCH₂-cPent), 3.78 (s, 3H, COOCH₃).
MASS (EI) m/z : 347(M⁺), 279, 265, 222, 154, 125.

### (Example 172)

### Compound of Formula No. A-116

¹H-NMR (CDCl₃) δ(ppm) : 7.22 (1H, s, 3-H), 7.11 (1H, s, 5-H), 4.79 (2H, brs, NCH₂Ar), 3.93 (2H, d, J=8Hz, NCH₂-cPent), 3.79 (s, 3H, COOCH₃).
MASS (EI) m/z : 347(M⁺), 279, 265, 222, 154, 125.

### (Example 173)

### Compound of Formula No. A-117

¹H-NMR (CDCl₃) δ(ppm) : 7.28 (1H, s, 3-H), 6.99 (1H, s, 5-H), 4.78 (2H, brs, NCH₂Ar), 3.92 (2H, d, J=8Hz, NCH₂-cPent), 3.78 (s, 3H, COOCH₃).
MASS (EI) m/z : 331(M⁺), 263, 249, 222, 154, 109.

### (Example 174)

### Compound of Formula No. A-118

¹H-NMR (CDCl₃) δ(ppm) : 7.27 (1H, s, 3-H), 6.96 (1H, s, 5-H), 4.81 (2H, brs, NCH₂Ar), 3.93 (2H, d, J=8Hz, NCH₂-cPent), 3.79 (s, 3H, COOCH₃).
MASS (EI) m/z : 331(M⁺), 263, 249, 222, 154, 109.

### (Example 175)

### Compound of Formula No. A-119

¹H-NMR (CDCl₃) δ(ppm) : 7.32 (1H, s, 3-H), 7.12 (1H, s, 5-H), 4.81 (2H, brs, NCH₂Ar), 3.96 (2H, d, J=8Hz, NCH₂-cPent), 1.69-1.55 (m, 1H, COCH(CH₂)₂).
MASS (EI) m/z : 348(M⁺), 280, 232, 198.

### (Example 176)

### Compound of Formula No. A-120

¹H-NMR (CDCl₃) δ(ppm) : 7.47 (1H, s, 3-H), 7.26 (1H, s, 5-H), 4.85 (2H, brs, NCH₂Ar), 3.94 (2H, d, J=8Hz, NCH₂-cPent), 3.80 (s, 3H, COOCH₃).
MASS (EI) m/z : 338(M⁺), 312, 284, 256, 57.

### (Example 177)

### Compound of Formula No. A-121

¹H-NMR (CDCl₃) δ(ppm) : 7.49 (1H, s, 3-H), 7.06 (1H, s, 5-H), 4.80 (2H, s, NCH₂Ar), 3.96 (2H, d, J=8Hz, NCH₂-cPent), 3.89 (2 H, s, COCH₂O).
MASS (EI) m/z : 352(M⁺), 337, 293, 236, 116.

### (Example 178)

### Compound of Formula No. A-122

¹H-NMR (CDCl₃) δ(ppm) : 7.50 (1H, s, 3-H), 7.13 (1H, s, 5-H), 4.82 (2H, s, NCH₂Ar), 4.50 (2H, s, COCH₂O), 3.96 (2 H, d, J=8Hz, NCH₂-cPent).
MASS (EI) m/z : 414(M⁺), 321, 293, 116.

### (Example 179)

### Compound of Formula No. A-123

¹H-NMR (CDCl₃) δ(ppm) : 7.24 (1H, s, 3-H), 7.06 (1H, s, 5-H), 4.80 (2H, s, NCH₂Ar), 3.96 (2H, d, J=8Hz, NCH₂-cPent), 1.99 (3 H, s, COCH₃).
MASS (EI) m/z : 322(M⁺), 280, 254, 206, 116.

### (Example 180)

### Compound of Formula No. A-124

¹H-NMR (CDCl₃) δ(ppm) : 7.30 (1H, s, 3-H), 7.21 (1H, s, 5-H), 4.80 (2H, s, NCH₂Ar), 4.51 (2 H, s, COCH₂O), 3.98 (2H, d, J=7Hz, NCH₂-cPent).
MASS (EI) m/z : 380(M⁺), 338, 312, 280, 116.

### (Example 181)

### Compound of Formula No. A-125

¹H-NMR (CDCl₃) δ(ppm) : 7.26 (1H, s, 3-H), 7.14 (1H, s, 5-H), 4.87 (2H, s, NCH₂Ar), 3.94 (2H, d, J=8Hz, NCH₂-cPent), 3.79 (s, 3H, COOCH₃).
MASS (EI) m/z : 338(M⁺), 279, 270, 256, 154.

### (Example 182)

### Compound of Formula No. A-126

¹H-NMR (CDCl₃) δ(ppm) : 7.34 (1H, s, 3-H), 7.13 (1H, s, 5-H), 4.84 (2H, s, NCH₂Ar), 3.96 (2H, d, J=8Hz, NCH₂-cPent), 1.68-1.52 (m, 1H, COCH(CH₂)₂).
MASS (EI) m/z : 348(M⁺), 280, 232, 198.

### (Example 183)

### Compound of Formula No. A-127

¹H-NMR (CDCl₃) δ(ppm) : 7.47 (1H, s, 3-H), 7.35 (1H, s, 5-H), 5.26 (2H, s, 1-NCH₂Ar), 4.88 (2H, s, NCH₂Ar), 3.81 (s, 3H, COOCH₃).
MASS (EI) m/z : 371(M⁺), 312, 255, 116.

### (Example 184)

### Compound of Formula No. A-128

¹H-NMR (CDCl₃) δ(ppm) : 7.55 (1H, s, 3-H), 7.47 (1H, s, 5-H), 4.87 (2H, s, NCH₂Ar), 3.81 (s, 3H, COOCH₃).
MASS (EI) m/z : 256(M⁺), 225, 189, 116.

### (Example 185)

### Compound of Formula No. A-129

¹H-NMR (CDCl₃) δ(ppm) : 7.51 (1H, s, 3-H), 7.29 (1H, s, 5-H), 4.84 (2H, S, NCH₂Ar), 4.22 (2H, s, 1-NCH₂C), 3.79 (s, 3H, COOCH₃).
MASS (EI) m/z : 398(M⁺), 383, 311, 270, 256.

### (Example 186)

### Compound of Formula No. A-130

¹H-NMR (CDCl₃) δ(ppm) : 7.59 (1H, s, 3-H), 7.44 (1H, s, 5-H), 5.27 (2H, s, NCH₂Ar), 1.87 (1H, brs, NH), 1.53-1.46 (m, 1H, COCH(CH₂)₂).
MASS (EI) m/z : 266(M⁺), 198, 116, 69.

### (Example 187)

### Compound of Formula No. A-131

¹H-NMR (CDCl₃) δ(ppm) : 7.46 (1H, s, 3-H), 7.29 (1H, s, 5-H), 4.86 (2H, s, NCH₂Ar), 4.11 (2H, t, J=6Hz, NCH₂(CH₂)₂CF₃), 3.81 (s, 3H, COOCH₃).
MASS (EI) m/z : 366(M⁺), 347, 307, 250.

### (Example 188)

### Compound of Formula No. A-132

¹H-NMR (CDCl₃) δ(ppm) : 7.48 (1H, s, 3-H), 7.30 (1H, s, 5-H), 4.86 (2H, s, NCH₂Ar), 4.15 (2H, s, 1-NCH₂C), 3.80 (s, 3H, COOCH₃)
MASS (EI) m/z : 358(M⁺), 327, 310, 270, 116.

### (Example 189)

### Compound of Formula No. A-133

¹H-NMR (CDCl₃) δ(ppm) : 7.52 (1H, s, 3-H), 7.49 (1H, s, 5-H), 5.35 (2H, s, NCH₂Ar), 3.68 (2H, t, J=7Hz, NCH₂(CH₂)₂CF₃), 1.58-1.46 (m, 1H, COCH(CH₂)₂).
MASS (EI) m/z : 376(M⁺), 357, 308, 211, 69.

### (Example 190)

### Compound of Formula No. A-134

¹H-NMR (CDCl₃) δ(ppm) : 7.55 (1H, s, 3-H), 7.51 (1H, s, 5-H), 5.33 (2H, s, NCH₂Ar), 3.72 (2H, d, J=7Hz, 1-NCH₂C), 1.63-1.51 (m, 1H, COCH(CH₂)₂).
MASS (EI) m/z : 395((M+1)⁺), 380, 312, 266, 69.

### (Example 191)

### Compound of Formula No. A-135

¹H-NMR (CDCl₃) δ(ppm) : 7.59 (1H, s, 3-H), 7.45 (1H, s, 5-H), 5.35 (2H, s, NCH₂Ar), 3.82 (2H, s, 1-NCH₂C), 1.72-1.60 (m, 1H, COCH(CH₂)₂).
MASS (EI) m/z : 408(M⁺), 393, 325, 266, 69.

### (Example 192)

### Compound of Formula No. A-136

¹H-NMR (CDCl₃) δ(ppm) : 7.47 (1H, s, 3-H), 7.27 (1H, s, 5-H), 4.85 (2H, s, NCH₂Ar), 4.05 (2H, s, 1-NCH₂C), 3.80 (s, 3H, COOCH₃).
MASS (EI) m/z : 442(M⁺), 399, 357, 269, 116.

### (Example 193)

### Compound of Formula No. A-137

¹H-NMR (CDCl₃) δ(ppm) : 7.52 (1H, s, 3-H), 7.46 (1H, s, 5-H), 4.85 (2H, s, NCH₂Ar), 4.00 (2H, s, 1-NCH₂C), 3.80 (s, 3H, COOCH₃).
MASS (EI) m/z : 386(M⁺), 371, 339, 270, 85.

### (Example 194)

### Compound of Formula No. A-138

¹H-NMR (CDCl₃) δ(ppm) : 7.52 (1H, s, 3-H), 7.47 (1H, s, 5-H), 4.85 (2H, s, NCH₂Ar), 4.15 (1H, d, J=14Hz, 1-NCH₂C), 4.03 (1 H, d, J=14Hz, 1-NCH₂C), 3.80 (s, 3H, COOCH₃).
MASS (EI) m/z : 372(M⁺), 357, 339, 270, 116.

### (Example 195)

### Compound of Formula No. A-139

¹H-NMR (CDCl₃) δ(ppm) : 7.55 (1H, s, 3-H), 7.52 (1H, s, 5-H), 5.34 (2H, s, NCH₂Ar), 3.78 (2H, d, J=8Hz, 1-NCH₂C), 1.64-1.52 (m, 1H, COCH(CH₂)₂).
MASS (EI) m/z : 354(M⁺), 286, 266, 211.

### (Example 196)

### Compound of Formula No. A-140

¹H-NMR (CDCl₃) δ(ppm) : 7.54 (1H, s, 3-H), 7.46 (1H, s, 5-H), 5.33 (2H, s, NCH₂Ar), 4.22 (2H, d, J=6Hz, NCH₂CH=CH₂), 1.65-1.52 (m, 1H, COCH(CH₂)₂).
MASS (EI) m/z : 306(M⁺), 238, 211, 197.

### (Example 197)

### Compound of Formula No. A-141

¹H-NMR (CDCl₃) δ(ppm) : 7.50 (1H, s, 3-H), 7.47 (1H, s, 5-H), 5.32 (2H, s, NCH₂Ar), 3.68 (2H, d, J=7Hz, 1-NCH₂C), 1.63-1.47 (m, 1H, COCH(CH₂)₂).
MASS (EI) m/z : 382(M⁺), 335, 311, 211, 97.

### (Example 198)

### Compound of Formula No. A-142

¹H-NMR (CDCl₃) δ(ppm) : 7.52 (1H, s, 3-H), 7.49 (1H, s, 5-H), 5.33 (2H, s, NCH₂Ar), 3.81 (1 H, d, J=7Hz, 1-NCH₂C), 3.78 (1 H, d, J=7Hz, 1-NCH₂C), 1.65-1.51 (m, 1H, COCH(CH₂)₂).
MASS (EI) m/z : 368(M⁺), 349, 311, 211, 97.

### (Example 199)

### Compound of Formula No. A-143

¹H-NMR (CDCl₃) δ(ppm) : 7.55 (1H, s, 3-H), 7.47 (1H, s, 5-H), 5.35 (2H, s, NCH₂Ar), 3.81 (2H, s, 1-NCH₂C), 1.69-1.57 (m, 1H, COCH(CH₂)₂).
MASS (EI) m/z : 368(M⁺), 320, 300, 211, 69.

### (Example 200)

### Compound of Formula No. A-144

¹H-NMR (CDCl₃) δ(ppm) : 7.65 (1H, s, 3-H), 7.52 (1H, s, 5-H), 5.34 (2H, s, NCH₂Ar), 3.70 (2H, d, J=8Hz, 1-NCH₂C), 1.59-1.46 (m, 1H, COCH(CH₂)₂).
MASS (EI) m/z : 438(M⁺), 370, 211, 69.

### (Example 201)

### Compound of Formula No. A-145

¹H-NMR (CDCl₃) δ(ppm) : 7.55 (1H, s, 3-H), 7.48 (1H, s, 5-H), 5.31 (2H, s, NCH₂Ar), 3.76 (2H, s, 1-NCH₂C), 1.62-1.49 (m, 1H, COCH(CH₂)₂).
MASS (EI) m/z : 452(M⁺), 384, 279, 211, 69.

### (Example 202)

### Compound of Formula No. A-146

¹H-NMR (CDCl₃) δ(ppm) : 7.52 (1H, s, 3-H), 7.49 (1H, s, 5-H), 5.32 (2H, s, NCH₂Ar), 3.64 (2H, s, 1-NCH₂C), 1.64-1.52 (m, 1H, COCH(CH₂)₂).
MASS (EI) m/z : 410(M⁺), 342, 279, 211, 69.

### (Example 203)

### Compound of Formula No. A-147

¹H-NMR (CDCl₃) δ(ppm) : 7.47 (1H, s, 3-H), 7.28 (1H, s, 5-H), 4.85 (2H, s, NCH₂Ar), 4.66 (2H, d, J=6Hz, NCH₂CH=CH₂), 3.80 (s, 3H, COOCH₃).
MASS (EI) m/z : 296(M⁺), 237, 180, 116.

### (Example 204)

### Compound of Formula No. A-148

¹H-NMR (CDCl₃) δ(ppm) : 7.47 (1H, s, 3-H), 7.35 (1H, s, 5-H), 4.86 (2H, s, NCH₂Ar), 4.41 (2H, t, J=6Hz, NCH₂CH₂Br), 3.80 (s, 3H, COOCH₃).
MASS (EI) m/z : 362(M⁺), 248, 246, 116.

### (Example 205)

### Compound of Formula No. A-149

¹H-NMR (CDCl₃ δ(ppm) : 7.45 (1H, s, 3-H), 7.23 (1H, s, 5-H), 4.81 (2H, s, NCH₂Ar), 4.32 (2H, t, J=7Hz, NCH₂CH₂CF₃), 1.63-1.51 (m, 1H, COCH(CH₂)₂).
MASS (EI) m/z : 362(M⁺), 294, 178, 116, 69.

### (Example 206)

### Compound of Formula No. A-150

¹H-NMR (CDCl₃) δ(ppm) : 7.47 (1H, s, 3-H), 7.31 (1H, s, 5-H), 4.85 (2H, s, NCH₂Ar), 4.28 (2H, t, J=7Hz, NCH₂CH₂CF₃), 3.81 (s, 3H, COOCH₃).
MASS (EI) m/z : 352(M⁺), 293, 269, 236, 116.

### (Example 207)

### Compound of Formula No. A-151

¹H-NMR (CDCl₃) δ(ppm) : 7.47 (1H, s, 3-H), 7.33 (1H, s, 5-H), 4.80 (2H, s, NCH₂Ar), 4.32 (2H, t, J=7Hz, NCH₂CH₂CF₃), 3.36 (s, 2H, COCH₂S).
MASS (EI) m/z : 407(M⁺), 367, 335, 294, 116.

### (Example 208)

### Compound of Formula No. A-152

¹H-NMR (CDCl₃) δ(ppm) : 7.47 (1H, s, 3-H), 7.41 (1H, s, 5-H), 6.94 (1H, dd, J=16, 9Hz, CH=CH₂), 4.89 (2H, s, NCH₂Ar), 3.83 (s, 3H, COOCH₃).
MASS (EI) m/z : 282(M⁺), 223, 166, 116.

### (Example 209)

### Compound of Formula No. A-153

¹H-NMR (CDCl₃) δ(ppm) : 7.47 (1H, s, 3-H), 7.35 (1H, s, 5-H), 4.86 (2H, s, NCH₂Ar), 4.31 (2H, t, J=7Hz, NCH₂CH₂CN), 3.81 (s, 3H, COOCH₃).
MASS (EI) m/z : 309(M⁺), 269, 193,149, 116.

### (Example 210)

### Compound of Formula No. A-154

¹H-NMR (CDCl₃) δ(ppm) : 7.47 (1H, s, 3-H), 7.30 (1H, s, 5-H), 4.85 (2H, s, NCH₂Ar), 4.23 (2H, t, J=7Hz, NCH₂CH₂SCH₃), 3.80 (s, 3H, COOCH₃).
MASS (EI) m/z : 330(M⁺), 269, 256, 116.

### (Example 211)

### Compound of Formula No. A-155

¹H-NMR (CDCl₃) δ(ppm) : 7.47 (1H, s, 3-H), 7.40 (1H, s, 5-H), 5.41 (2H, d, J=6Hz, NCH₂OH), 4.86 (2H, s, NCH₂Ar), 3.81 (s, 3H, COOCH₃).
MASS (EI) m/z : 256((M-30)⁺), 189, 116.

### (Example 212)

### Compound of Formula No. A-156

¹H-NMR (CDCl₃) δ(ppm) : 7.48 (1H, s, 3-H), 7.43 (1H, s, 5-H), 5.79 (2H, s, NCH₂Cl), 4.89 (2H, s, NCH₂Ar), 3.83 (s, 3H, COOCH₃). MASS (EI) m/z : 304(M⁺), 269, 188, 116.

### (Example 213)

### Compound of Formula No. A-157

¹H-NMR (CDCl₃) δ(ppm) : 7.48 (1H, s, 3-H), 7.32 (1H, s, 5-H), 4.87 (2H, s, NCH₂Ar), 4.48 (2H, d, J=12Hz, NCH₂P(O)(OEt)₂), 3.80 (s, 3H, COOCH₃).
MASS (EI) m/z : 406(M⁺), 391, 347, 290, 269.

### (Example 214)

### Compound of Formula No. A-158

¹H-NMR (CDCl₃) δ(ppm) : 7.33 (1H, s, 3-H), 7.06 (1H, s, 5-H), 4.85 (2H, s, NCH₂Ar), 3.82 (2H, s, NCH₂-t-Bu), 1.67-1.54 (1H, m, COCH(CH₂)₂).
MASS (EI) m/z : 336(M⁺), 321, 268, 220, 152, 116.

### (Example 215)

### Compound of Formula No. A-159

¹H-NMR (CDCl₃) δ(ppm) : 7.27 (1H, s, 3-H), 7.06 (1H, S, 5-H), 4.87 (2H, s, NCH₂Ar), 3.80 (2H, s, NCH₂-t-Bu), 3.78 (3H, s, COOCH₃).
MASS (EI) m/z : 326(M⁺), 311, 269, 210, 154, 116.

### (Example 216)

### Compound of Formula No. A-160

¹H-NMR (CDCl₃) δ(ppm) : 7.33 (1H, s, 3-H), 7.03 (1H, s, 5-H), 4.77 (2H, s, NCH₂Ar), 3.81 (2H, s, NCH₂-t-Bu), 1.65-1.54 (1H, m, COCH(CH₂)₂).
MASS (EI) m/z : 345(M⁺), 330, 277, 220, 152, 125.

### (Example 217)

### Compound of Formula No. A-161

¹H-NMR (CDCl₃) δ(ppm) : 7.30 (1H, s, 3-H), 7.11 (1H, s, 5-H), 4.79 (2H, s, NCH₂Ar), 3.79 (5H, s, NCH₂-t-Bu & COOCH₃).
MASS (EI) m/z : 335(M⁺), 320, 278, 210, 154, 125.

### (Example 218)

### Compound of Formula No. A-162

¹H-NMR (CDCl₃) δ(ppm) : 7.34 (1H, s, 3-H), 7.11 (1H, s, 5-H), 4.90 (2H, s, NCH₂Ar), 3.82 (2H, s, NCH₂-t-Bu), 1.67-1.55 (1H, m, COCH(CH₂)₂).
MASS (EI) m/z : 356(M⁺), 341, 326, 288, 231, 149.

### (Example 219)

### Compound of Formula No. A-163

¹H-NMR (CDCl₃) δ(ppm) : 7.29 (1H, s, 3-H), 7.11 (1H, s, 5-H), 4.92 (2H, s, NCH₂Ar), 3.80 (5H, s, NCH₂-t-Bu & COOCH₃).
MASS (EI) m/z : 346(M⁺), 331, 316, 289, 273, 210, 154.

### (Example 220)

### Compound of Formula No. A-164

¹H-NMR (CDCl₃) δ(ppm) : 7.31 (1H, s, 3-H), 7.10 (1H, s, 5-H), 4.80 (2H, brs, NCH₂Ar), 4.58 (2H, s, COCH₂O), 3.83 (2H, s, 1-NCH₂), 3.70 (2H, s, CH₂CN), 3.55 (2H, s, COCH₂S).
MASS (EI) m/z : 439(M⁺), 424, 368, 325, 309, 266, 211, 197.

### (Example 221)

### Compound of Formula No. A-165

¹H-NMR (CDCl₃) δ(ppm) : 8.05 (1H, brs, NH), 7.30 (1H, s, 3-H), 7.17 (1H, s, 5-H), 4.76 (2H, brs, NCH₂Ar), 4.53 (2H, s, COCH₂O), 4.20 (2H, q, J=7Hz, OCH₂CH₃), 3.80 (2H, d, J=7Hz, 1-NCH₂-i-Pr), 1.26 (3H, t, J=7Hz, OCH₂CH₃).
MASS (EI) m/z : 427(M'), 381, 312, 297, 281, 254, 225, 211.

### (Example 222)

### Compound of Formula No. A-166

¹H-NMR (CDCl₃) δ(ppm) : 7.92 (1H, brs, NH), 7.30 (1H, s, 3-H), 7.12 (1H, s, 5-H), 4.77 (2H, .s, NCH₂Ar), 4.53 (2H, s, COCH₂O), 4.21 (2H, q, J=7Hz, OCH₂CH₃), 3.80 (2H, s, 1-NC^{H}₂), 1.26 (3H, t, J=7Hz, OCH₂CH₃).
MASS (EI) m/z : 441(M⁺), 395, 340, 326, 311, 268, 225, 210.

### (Example 223)

### Compound of Formula No. A-167

¹H-NMR (CDCl₃) δ(ppm) : 7.33 (1H, s, 3-H), 7.14 (1H, s, 5-H), 6.98 (1H, brs, NH), 4.78 (2H, s, NCH₂Ar), 4.19 (2H, q, J=7Hz, OCH₂CH₃), 3.87 (2H, d, J=7Hz, 1-NCH₂-i-Pr), 1.25 (3H, t, J=7Hz, OCH₂CH₃).
MASS (EI) m/z : 369(M⁺), 323, 280, 267, 254, 207, 198, 165.

### (Example 224)

### Compound of Formula No. A-168

¹H-NMR (CDCl₃) δ(ppm) : 7.33 (1H, s, 3-H), 7.11 (1H, s, 5-H), 6.98 (1H, brs, NH), 4.78 (2H, s, NCH₂Ar), 4.18 (2H, q, J=7Hz, OCH₂CH₃), 3.85 (2H, s, 1-NCH₂), 1.25 (3H, t, J=7Hz, OCH₂CH₃). MASS (EI) m/z : 383(M⁺), 337, 322, 281, 268, 221, 211, 197.

### (Example 225)

### Compound of Formula No. A-169

¹H-NMR (CDCl₃) δ(ppm) : 8.57 (1H, brs, NH), 7.39 (1H, s, 3-H), 7.19 (1H, s, 5-H), 5.59 (2H, brs, NCH₂Ar), 3.67 (2H, s, 1-NCH₂). MASS (EI) m/z : 447(M⁺), 389, 357, 284, 269, 211, 182, 168.

### (Example 226)

### Compound of Formula No. A-170

¹H-NMR (CDCl₃) δ(ppm) : 7.86 (1H, brs, NH), 7.20 (1H, s, 3-H), 6.98 (1H, s, 5-H), 4.69 (2H, s, NCH₂Ar), 4.52 (2H, s, COCH₂Cl), 3.61 (2H, s, 1-NCH₂).
MASS (EI) m/z : 431(M⁺), 417, 357, 285, 269, 211, 195, 182.

### (Example 227)

### Compound of Formula No. A-171

¹H-NMR (CDCl₃) δ(ppm) : 7.18 (1H, s, 3-H), 7.01 (1H, s, 5-H), 4.71 (2H, s, NCH₂Ar), 4.12 (2H, q, J=7Hz, OCH₂CH₃), 3.64 (2H, s, 1-NCH₂), 1.18 (3H, t, J=7Hz, OCH₂CH₃).
MASS (EI) m/z : 399(M⁺), 353, 338, 283, 237, 168, 116, 100.

### (Example 228)

### Compound of Formula No. A-172

¹H-NMR (CDCl₃) δ(ppm) : 7.43 (1H, s, 3-H), 7.25 (1H, s, 5-H), 7.13 (1H, brs, NH), 4.80 (2H, brs, NCH₂Ar), 3.64 (2H, s, 1-NCH₂).
MASS (EI) m/z : 431(M⁺), 365, 343, 285, 240, 105.

### (Example 229)

### Compound of Formula No. A-173

¹H-NMR (CDCl₃) δ(ppm) : 8.92 (1H, brs, NH), 7.32 (1H, s, 3-H), 7.12 (1H, s, 5-H), 4.81 (2H,s, NCH₂Ar), 4.64 (2H, s, COCH₂O), 3.84 (2H, s, 1-NCH₂).
MASS (EI) m/z : 513(M⁺), 472, 436, 326, 268.

### (Example 230)

### Compound of Formula No. A-174

¹H-NMR (CDCl₃) δ(ppm) : 8.80 (1H, brs, NH), 7.34 (1H, s, 3-H), 7.14 (1H, s, 5-H), 4.81 (2H,s, NCH₂Ar), 4.59 (2H, s, COCH₂O), 4.52 (2H, s, COCH₂Cl), 3.84 (2H, s, 1-NCH₂).
MASS (EI) m/z : 445(M⁺), 404, 395, 369, 326, 311, 268, 210.

### (Example 231)

### Compound of Formula No. A-175

¹H-NMR (CDCl₃) δ(ppm) : 7.31 (1H, s, 3-H), 7.17 (1H, s, 5-H), 6.34 (1H, brs, NH), 4.78 (2H,s, NCH₂Ar), 4.63 (2H, s, COCH₂O), 3.82 (2H, s, 1-NCH₂).
MASS (EI) m/z : 473(M⁺), 442, 403, 326, 311, 295, 268, 225.

### (Example 232)

### Compound of Formula No. A-176

¹H-NMR (CDCl₃) δ(ppm) : 9.26 (1H, brs, NH), 7.25 (1H, s, 3-H), 7.10 (1H, s, 5-H), 4.80 (2H,s, NCH₂Ar), 4.65 (2H, s, COCH₂O), 3.69 (2H, s, 1-NCH₂).
MASS (EI) m/z : 529(M⁺), 488, 473, 452.

### (Example 233)

### Compound of Formula No. A-177

¹H-NMR (CDCl₃) δ(ppm) : 8.62 (1H, brs, NH), 7.25 (1H, s, 3-H), 7.07 (1H, s, 5-H), 4.80 (2H,s, NCH₂Ar), 4.58 (2H, s, COCH₂O), 4.51 (2H, s, COCH₂Cl), 3.68 (2H, s, 1-NCH₂).
MASS (EI) m/z : 461(M⁺), 342, 327, 311, 283, 226, 180.

### (Example 234)

### Compound of Formula No. A-178

¹H-NMR (CDCl₃ δ(ppm) : 7.22 (1H, s, 3-H), 7.03 (1H, s, 5-H), 4.77 (2H, s, NCH₂Ar), 4.54 (2H, s, COCH₂O), 4.26 (2H, q, J=7Hz, OCH₂CH₃), 3.66 (2H, s, 1-NCH₂), 1.31 (3H, t, J=7Hz, OCH₂CH₃). MASS (EI) m/z : 457(M⁺), 414, 400, 342, 327, 311, 283, 226.

### (Example 235)

### Compound of Formula No. A-179

¹H-NMR (CDCl₃) δ(ppm) : 7.23 (1H, s, 3-H), 7.11 (1H, s, 5-H), 4.76 (2H,s, NCH₂Ar), 4.63 (2H, s, COCH₂O), 3.66 (2H, s, 1-NCH₂). MASS (EI) m/z : 489(M⁺), 458, 415, 342, 327, 311, 283, 269.

### (Example 236)

### Compound of Formula No. A-180

¹H-NMR (CDCl₃) δ(ppm) : 8.62 (1H, brs, NH), 7.35 (1H, s, 3-H), 7.23 (1H, s, 5-H), 5.49 (2H, brs, NCH₂Ar), 3.74 (2H, d, J=7Hz, 1-NCH₂-i-Pr).
MASS (EI) m/z : 417(M⁺), 369, 358, 254, 211, 198, 138, 116.

### (Example 237)

### Compound of Formula No. A-181

¹H-NMR (CDCl₃) δ(ppm) : 8.65 (1H, brs, NH), 7.38 (1H, s, 3-H), 7.25 (1H, s, 5-H), 4.82 (2H, s, NCH₂Ar), 3.89 (2H, d, J=7Hz, 1-NCH₂-i-Pr).
MASS (EI) m/z : 441(M⁺), 407, 297, 254.

### (Example 238)

### Compound of Formula No. A-182

¹H-NMR (CDCl₃) δ(ppm) : 7.94 (1H, brs, NH), 7.33 (1H, s, 3-H), 7.14 (1H, s, 5-H), 4.77 (2H, s, NCH₂Ar), 4.58 (2H, s, COCH₂Cl), 3.87 (2H, d, J=7Hz, 1-NCH₂-i-Pr).
MASS (EI) m/z : 373(M⁺), 337, 294, 254, 221, 211, 198.

### (Example 239)

### Compound of Formula No. A-183

¹H-NMR (CDCl₃) δ(ppm) : 7.37 (1H, s, 3-H), 7.32 (1H, s, 5-H), 4.85 (2H, brs, NCH₂Ar), 3.84 (2H, d, J=7Hz, 1-NCH₂-i-Pr).
MASS (EI) m/z : 401(M⁺), 386, 368, 356, 337, 323, 254, 225.

### (Example 240)

### Compound of Formula No. A-184

¹H-NMR (CDCl₃) δ(ppm) : 8.46 (1H, brs, NH), 7.35 (1H, s, 3-H), 7.16 (1H, s, 5-H), 5.50 (2H, s, NCH₂Ar), 3.72 (2H, s, 1-NCH₂). MASS (EI) m/z : 431(M⁺), 372, 268, 253, 211, 198, 184, 166.

### (Example 241)

### Compound of Formula No. A-185

¹H-NMR (CDCl₃) δ (ppm) : 8.69 (1H, brs, NH), 7.38 (1H, s, 3-H), 7.24 (1H, s, 5-H), 4.82 (2H, s, NCH₂Ar), 3.88 (2H, s, 1-NCH₂). MASS (EI) m/z : 455(M⁺), 441, 394, 311.

### (Example 242)

### Compound of Formula No. A-186

¹H-NMR (CDCl₃) δ(ppm) : 8.00 (1H, brs, NH), 7.34 (1H, s, 3-H), 7.13 (1H, s, 5-H), 4.78 (2H, s, NCH₂Ar), 4.56 (2H, s, COCH₂Cl), 3.85 (2H, s, 1-NCH₂).
MASS (EI) m/z : 387(M⁺), 351, 336, 294, 268, 253, 235.

### (Example 243)

### Compound of Formula No. A-187

¹H-NMR (CDCl₃) δ(ppm) : 7.39 (1H, s, 3-H), 7.24 (1H, s, 5-H), 4.84 (2H, s, NCH₂Ar), 3.85 (2H, s, 1-NCH₂).
MASS (EI) m/z : 415(M⁺), 325, 268, 253, 211, 198, 147, 133.

### (Example 244)

### Compound of Formula No. A-188

¹H-NMR (CDCl₃) δ(ppm) : 7.32 (1H, s, 3-H), 7.21 (1H, s, 5-H), 4.83 (2H, brs, NCH₂Ar), 3.71 (2H, s, 1-NCH₂).
MASS (EI) m/z : 471(M⁺), 456, 399, 355.

### (Example 245)

### Compound of Formula No. A-189

¹H-NMR (CDCl₃) δ(ppm) : 7.34 (1H, s, 3-H), 7.21 (1H, s, 5-H), 6.25 (1H, brs, NH), 5.04 (2H,s, NCH₂Ar), 4.82 (2H, s, COCH₂O), 3.85 (2H, s, 1-NCH₂).
MASS (EI) m/z : 446((M-43)⁺), 430, 415, 373, 342, 326, 309.

### (Example 246)

### Compound of Formula No. A-190

¹H-NMR (CDCl₃) δ(ppm) : 7.43 (1H, s, 3-H), 7.16 (1H, s, 5-H), 6.19 (1H, brs, NH), 5.04 (2H,s, NCH₂Ar), 4.80 (2H, s, COCH₂O), 3.69 (2H, s, 1-NCH₂).
MASS (EI) m/z : 462((M-43)⁺), 446, 431, 384, 358, 342, 327.

### (Example 247)

### Compound of Formula No. B-1

¹H-NMR (CDCl₃) δ(ppm) : 7.06 (1H, s, 5-H), 4.76 (2H, brs, NCH₂Ar), 3.89 (2H, s, COCH₂Cl), 3.78 (3H, s, NCH₃), 1.96 (3H, s, 3-CH₃).
MASS (EI) m/z : 302(M⁺).

### (Example 248)

### Compound of Formula No. B-2

¹H-NMR (CDCl₃) δ(ppm) : 7.10 (1H, s, 5-H), 4.76 (2H, brs, NCH₂Ar), 3.78 (3H, s, NCH₃), 3.71 (2H, s, COCH₂Br), 1.99 (3H, s, 3-CH₃).
MASS (EI) m/z : 346(M⁺).

### (Example 249)

### Compound of Formula No. B-3

¹H-NMR (CDCl₃) δ(ppm) : 7.04 (1H, s, 5-H), 4.77 (2H, brs, NCH₂Ar), 4.42 (2H, s, COCH₂O), 3.80 (3H, s, NCH₃), 1.98 (3H, s, 3-CH₃).
MASS (EI) m/z : 360(M⁺), 281, 267, 253, 225.

### (Example 250)

### Compound of Formula No. B-4

¹H-NMR (CDCl₃) δ(ppm) : 7.08 (1H, s, 5-H), 4.86 (2H, brs, NCH₂Ar), 4.44 (2H, s, COCH₂O), 3.81 (3H, s, NCH₃), 2.02 (3H, s, 3-CH₃).
MASS (EI) m/z : 380(M⁺), 287, 273, 245, 199, 149.

### (Example 251)

### Compound of Formula No. B-5

¹H-NMR (CDCl₃) δ(ppm) : 6.97 (1H, s, 5-H), 4.77 (2H, brs, NCH₂Ar), 4.41 (2H, s, COCH₂O), 3.77 (3H, s, NCH₃), 1.98 (3H, s, 3-CH₃).
MASS (EI) m/z : 419(M⁺), 326, 312, 284, 244, 175.

### (Example 252)

### Compound of Formula No. B-6

¹H-NMR (CDCl₃) δ(ppm) : 7.04 (1H, s, 5-H), 4.75 (2H, brs, NCH₂Ar), 4.04 (2H, q, J=7Hz, NCH₂Me), 3.88(2H, s, COCH₂Cl), 1.97 (3H, s, 3-CH₃).
MASS (EI) m/z : 316(M⁺).

### (Example 253)

### Compound of Formula No. B-7

¹H-NMR (CDCl₃) δ(ppm) : 7.09 (1H, s, 5-H), 4.75 (2H, brs, NCH₂Ar), 4.04 (2H, q, J=7Hz, NCH₂Me), 3.70(2H, s, COCH₂Br), 1.97 (3H, s, 3-CH₃).
MASS (EI) m/z : 360(M⁺).

### (Example 254)

### Compound of Formula No. B-8

¹H-NMR (CDCl₃) δ(ppm) : 6.99 (1H, s, 5-H), 4.75 (2H, brs, NCH₂Ar), B-9H4.05 (2H, q, J=7Hz, NCH₂Me), 3.40 (2H, s, CHOCH₂O), 1.95 (3H, s, 3-CH₃).
MASS (EI) m/z : 312(M⁺), 297, 282, 267, 253, 239, 168.

### (Example 255)

### Compound of Formula No. B-9

¹H-NMR (CDCl₃) δ(ppm) : 7.0H2 (1H, s, 5-H), 4.77 (2H, brs, NCH₂Ar), 4.36 H(2H, brs, COCH₂O), 4.02 (2H, q, J=7HzH, NCH₂Me), 2.00 (3H, s, 3-CH₃).
MASS (EI) m/z : 374(M⁺), 281, 267, 253, 239, 152.

### (Example 256)

### Compound of Formula No. B-10

¹H-NMR (CDCl₃) δ(ppm) : 7.03 (1HH, s, 5-H), 4.83 (2H, brs, NCH₂Ar), 4.04 (2H, Hq, J=7Hz, NCH₂Me), 3.40 (2H, s, COCH₂O), 1H.98 (3H, S, 3-CH₃).
MASS (EI) m/z : 332(M⁺), 317, 302, 287, 273, 259, 213.

### (Example 257)

### Compound of Formula No. B-11

¹H-NMR (CDCl₃) δ(ppm) : 7.05 (1H, s, 5H-H), 4.86 (2H, brs, NCH₂Ar), 4.43 (2H, s, COhCH₂O), 4.04 (2H, q, J=7Hz, NCH₂Me), 2.04 (3H, s, 3-CH₃).
MASS (EI) m/z : 394(M⁺), 301, 287, 259, 152, 123.

### (Example 258)

### Compound of Formula No. B-12

¹H-NMR (CDCl₃) δ(ppm) : 6.94(1H, s, 5-H), 4.74 (2H, brs, NCH₂Ar), 4.02 (2H, q, J=7Hz, NCH₂Me), 3.79 (2H, s, COCH₂O), 1.95 (3H, s, 3-CH₃).
MASS (EI) m/z : 371(M⁺), 356, 326, 312, 298, 196, 175, 168.

### (Example 259)

### Compound of Formula No. B-13

¹H-NMR (CDCl₃) δ(ppm) : 6.97 (1H, s, 5-H), 4.77 (2H, brs, NCH₂Ar), 4.41 (2H, brs, COCH₂O), 4.02 (2H, q, J=7Hz, NCH₂Me), 2.00 (3H, s, 3-CH₃).
MASS (EI) m/z : 433 (M⁺).

### (Example 260)

### Compound of Formula No. B-14

¹H-NMR (CDCl₃) δ(ppm) : 6.93 (1H, s, 5-H), 4.75 (2H, brs, NCH₂Ar), 3.93 (2H, t, J=7Hz, NCH₂Et), 1.97 (3H, s, 3-CH₃), 1.91 (3H, s, COCH₃).
MASS (EI) m/z : 296(M⁺), 254, 180, 138, 124, 116, 96, 82.

### (Example 261)

### Compound of Formula No. B-15

¹H-NMR (CDCl₃) δ(ppm) : 7.01 (1H, s, 5-H), 4.78 (2H, brs, NCH₂Ar), 3.94 (2H, t, J=7Hz, NCH₂Et), 2.02 (3H, s, 3-CH₃), 1.58-1.45 (1H, m, COCH(CH₂)₂).
MASS (EI) m/z : 322(M⁺), 254, 206, 138, 116.

### (Example 262)

### Compound of Formula No. B-16

¹H-NMR (CDCl₃) δ(ppm) : 7.01 (1H, s, 5-H), 4.78 (2H, brs, NCH₂Ar), 3.95 (2H, t, J=7Hz, NCH₂Et), 3.89 (2H, s, COCH₂Cl), 2.00 (3H, s, 3-CH₃).
MASS (EI) m/z : 330(M+).

### (Example 263)

### Compound of Formula No. B-17

¹H-NMR (CDCl₃) δ(ppm) : 7.06 (1H, s, 5-H), 4.78 (2H, brs, NCH₂Ar), 3.95 (2H, t, J=7Hz, NCH₂Et), 3.70 (2H, s, COCH₂Br), 2.02 (3H, s, 3-CH₃).
MASS (EI) m/z : 374(M⁺).

### (Example 264)

### Compound of Formula No. B-18

¹H-NMR (CDCl₃) δ(ppm) : 6.94 (1H, s, 5-H), 4.79 (2H, brs, NCH₂Ar), 3.94 (2H, t, J=7Hz, NCH₂Et), 3.83 (2H, d, J=5Hz, COCH₂O), 1.95 (3H, s, 3-CH₃).
MASS (EI) m/z : 312(M⁺), 281, 253, 166, 136, 124, 116.

### (Example 265)

### Compound of Formula No. B-19

¹H-NMR (CDCl₃) δ(ppm) : 6.96 (1H, s, 5-H), 4.76 (2H, brs, NCH₂Ar), 3.95 (2H, t, J=7Hz, NCH₂Et), 3.80 (2H, s, COCH₂O), 1.96 (3H, s, 3-CH₃).
MASS (EI) m/z : 326(M⁺), 311, 281, 267, 253, 210, 182.

### (Example 266)

### Compound of Formula No. B-20

¹H-NMR (CDCl₃) δ(ppm) : 7.00 (1H, s, 5-H), 4.78 (2H, brs, NCH₂Ar), 4.41 (2H, s, COCH₂O), 3.94 (2H, t, J=7Hz, NCH₂Et), 2.02 (3H, s, 3-CH₃).
MASS (EI) m/z : 388(M⁺), 295, 281, 267, 253, 166, 124.

### (Example 267)

### Compound of Formula No. B-21

¹H-NMR (CDCl₃) δ(ppm) : 7.01 (1H, s, 5-H), 4.75 (2H, brs, NCH₂Ar), 4.38 (2H, s, COCH₂O), 3.94 (2H, t, J=7Hz, NCH₂Et), 2.01 (3H, s, 3-CH₃).
MASS (EI) m/z : 354(M⁺), 311, 252, 238, 210, 166, 116.

### (Example 268)

### Compound of Formula No. B-22

¹H-NMR (CDCl₃) δ(ppm) : 6.93 (1H, brs, 5-H), 4.69 (2H, s, NCH₂Ar), 3.91 (2H, t, J=7Hz, NCH₂Et), 3.72 (3H, brs, COOCH₃), 1.98 (3H, s, 3-CH₃).
MASS (EI) m/z : 312(M⁺), 196, 154, 122, 116.

### (Example 269)

### Compound of Formula No. B-23

¹H-NMR (CDCl₃) δ(ppm) : 6.98 (1H, s, 5-H), 4.83 (2H, s, NCH₂Ar), 3.93 (2H, t, J=7Hz, NCH₂Et), 2.00 (3H, s, 3-CH₃), 1.93 (3H, s, COCH₃).
MASS (EI) m/z : 316(M⁺), 274, 180, 152, 138.

### (Example 270)

### Compound of Formula No. B-24

¹H-NMR (CDCl₃) δ(ppm) : 7.05 (1H, s, 5-H), 4.85 (2H, brs, NCH₂Ar), 3.93 (2H, t, J=7Hz, NCH₂Et), 2.04 (3H, s, 3-CH₃), 1.60-1.47 (1H, m, COCH(CH₂)₂).
MASS (EI) m/z : 342(M⁺), 274, 257, 227, 206, 138.

### (Example 271)

### Compound of Formula No. B-25

¹H-NMR (CDCl₃) δ(ppm) : 6.99 (1H, s, 5-H), 4.87 (2H, brs, NCH₂Ar), 3.94 (2H, t, J=7Hz, NCH₂Et), 3.85 (2H, d, J=5Hz, COCH₂O), 1.98 (3H, s, 3-CH₃).
MASS (EI) m/z : 332(M⁺), 301, 273, 257, 196, 166, 136, 124.

### (Example 272)

### Compound of Formula No. B-26

¹H-NMR (CDCl₃) δ(ppm) : 6.97 (1H, s, 5-H), 4.83 (2H, brs, NCH₂Ar), 3.93 (2H, t, J=7Hz, NCH₂Et), 3.80 (2H, s, COCH₂O), 1.99 (3H, s, 3-CH₃).
MASS (EI) m/z : 346(M⁺), 331, 301, 287, 273, 210, 182.

### (Example 273)

### Compound of Formula No. B-27

¹H-NMR (CDCl₃) δ(ppm) : 7.03 (1H, s, 5-H), 4.86 (2H, brs, NCH₂Ar), 4.43 (2H, s, COCH₂O), 3.93 (2H, t, J=7Hz, NCH₂Et), 2.05 (3H, s, 3-CH₃).
MASS (EI) m/z : 408(M⁺), 315, 301, 287, 273, 166, 150, 136.

### (Example 274)

### Compound of Formula No. B-28

¹H-NMR (CDCl₃) δ(ppm) : 7.05 (1H, s, 5-H), 4.82 (2H, brs, NCH₂Ar), 4.40 (2H, s, COCH₂O), 3.93 (2H, t, J=7Hz, NCH₂Et), 2.04 (3H, s, 3-CH₃).
MASS (EI) m/z : 374(M⁺), 331, 272, 257, 166, 136.

### (Example 275)

### Compound of Formula No. B-29

¹H-NMR (CDCl₃) δ(ppm) : 6.98 (1H, s, 5-H), 4.77 (2H, s, NCH₂Ar), 3.90 (2H, t, J=7Hz, NCH₂Et), 3.73 (3H, brs, COOCH₃), 2.01 (3H, s, 3-CH₃).
MASS (EI) m/z : 332(M⁺), 196, 136.

### (Example 276)

### Compound of Formula No. B-30

¹H-NMR (CDCl₃) δ(ppm) : 6.90 (1H, s, 5-H), 4.74 (2H, brs, NCH₂Ar), 3.91 (2H, t, J=7Hz, NCH₂Et), 1.97 (3H, s, 3-CH₃), 1.91 (3H, s, COCH₃).
MASS (EI) m/z : 355(M⁺), 313, 180, 152, 138, 124.

### (Example 277)

### Compound of Formula No. B-31

¹H-NMR (CDCl₃) δ(ppm) : 6.98 (1H, s, 5-H), 4.76 (2H, brs, NCH₂Ar), 3.92 (2H, t, J=7Hz, NCH₂Et), 2.00 (3H, s, 3-CH₃), 1.58-1.45 (1H, m, COCH(CH₂)₂).
MASS (EI) m/z : 381(M⁺), 313, 206, 175, 164, 138.

### (Example 278)

### Compound of Formula No. B-32

¹H-NMR (CDCl₃) δ(ppm) : 6.92 (1H, s, 5-H), 4.78 (2H, brs, NCH₂Ar), 3.92 (2H, t, J=7Hz, NCH₂Et), 3.82 (2H, d, J=5Hz, COCH₂O), 1.94 (3H, s, 3-CH₃).
MASS (EI) m/z : 371(M⁺), 340, 312, 175, 166, 124.

### (Example 279)

### Compound of Formula No. B-33

¹H-NMR (CDCl₃) δ(ppm) : 6.90 (1H, s, 5-H), 4.74 (2H, brs, NCH₂Ar), 3.92 (2H, t, J=7Hz, NCH₂Et), 3.79 (2H, s, COCH₂O), 1.95 (3H, s, 3-CH₃).
MASS (EI) m/z : 385(M⁺), 370, 340, 326, 312, 210, 175.

### (Example 280)

### Compound of Formula No. B-34

¹H-NMR (CDCl₃) δ(ppm) : 6.95 (1H, s, 5-H), 4.77 (2H, brs, NCH₂Ar), 4.40 (2H, s, COCH₂O), 3.92 (2H, t, J=7Hz, NCH₂Et), 2.01 (3H, s, 3-CH₃).
MASS (EI) m/z : 447(M⁺), 354, 326, 312, 272, 175, 166.

### (Example 281)

### Compound of Formula No. B-35

¹H-NMR (CDCl₃) δ(ppm) : 6.99 (1H, s, 5-H), 4.73 (2H, brs, NCH₂Ar), 4.39 (2H, s, COCH₂O), 3.92 (2H, t, J=7Hz, NCH₂Et), 2.00 (3H, s, 3-CH₃).
MASS (EI) m/z : 413(M⁺), 311, 238, 175, 166.

### (Example 282)

### Compound of Formula No. B-36

¹H-NMR (CDCl₃) δ(ppm) : 6.91 (1H, brs, 5-H), 4.67 (2H, s, NCH₂Ar), 3.89 (2H, t, J=7Hz, NCH₂Et), 3.71 (3H, brs, COOCH₃), 1.98 (3H, s, 3-CH₃).
MASS (EI) m/z : 371(M⁺), 342, 196, 175, 109.

### (Example 283)

### Compound of Formula No. B-37

¹H-NMR (CDCl₃) δ(ppm) : 6.96 (1H, s, 5-H), 4.74 (2H, brs, NCH₂Ar), 4.34 (1H, hept, J=7Hz, NCHMe₂), 1.97 (3H, s, 3-CH₃), 1.91 (3H, s, COCH₃).
MASS (EI) m/z : 296(M⁺), 254, 211, 180, 138, 124, 116.

### (Example 284)

### Compound of Formula No. B-38

¹H-NMR (CDCl₃) δ(ppm) : 7.03 (1H, s, 5-H), 4.76 (2H, brs, NCH₂Ar), 4.35 (1H, hept, J=7Hz, NCHMe₂), 2.05 (3H, s, 3-CH₃), 1.57-1.42 (1H, m, COCH(CH₂)₂).
MASS (EI) m/z : 322(M⁺), 254, 212, 206, 164, 138, 124, 116.

### (Example 285)

### Compound of Formula No. B-39

¹H-NMR (CDCl₃) δ(ppm) : 7.03 (1H, s, 5-H), 4.76 (2H, brs, NCH₂Ar), 4.35 (1H, hept, J=7Hz, NCHMe₂), 3.88 (2H, s, COCH₂Cl), 1.98 (3H, s, 3-CH₃).
MASS (EI) m/z : 330(M⁺).

### (Example 286)

### Compound of Formula No. B-40

¹H-NMR (CDCl₃) δ(ppm) : 7.08 (1H, s, 5-H), 4.76 (2H, brs, NCH₂Ar), 4.35 (1H, hept, J=7Hz, NCHMe₂), 3.69 (2H, s, COCH₂Br), 2.00 (3H, s, 3-CH₃).
MASS (EI) m/z : 374(M⁺).

### (Example 287)

### Compound of Formula No. B-41

¹H-NMR (CDCl₃) δ(ppm) : 6.98 (1H, s, 5-H), 4.78 (2H, brs, NCH₂Ar), 4.35 (1H, hept, J=7Hz, NCHMe₂), 3.83 (2H, d, J=5Hz, COCH₂O), 1.94 (3H, s, 3-CH₃).
MASS (EI) m/z : 312(M⁺), 297, 253, 239, 211, 166, 116.

### (Example 288)

### Compound of Formula No. B-42

¹H-NMR (CDCl₃) δ(ppm) : 6.96 (1H, s, 5-H), 4.74 (2H, brs, NCH₂Ar), 4.34 (1H, hept, J=7Hz, NCHMe₂), 3.78 (2H, s, COCH₂O), 1.95 (3H, s, 3-CH₃).
MASS (EI) m/z : 326(M⁺), 311, 281, 267, 253, 239, 211, 182.

### (Example 289)

### Compound of Formula No. B-43

¹H-NMR (CDCl₃) δ(ppm) : 7.00 (1H, s, 5-H), 4.77 (2H, brs, NCH₂Ar), 4.41 (2H, s, COCH₂O), 4.34 (1H, hept, J=7Hz, NCHMe₂), 2.01 (3H, s, 3-CH₃).
MASS (EI) m/z : 388(M⁺), 295, 281, 267, 253, 239, 211, 166.

### (Example 290)

### Compound of Formula No. B-44

¹H-NMR (CDCl₃) δ(ppm) : 6.96 (1H, s, 5-H), 4.74 (2H, brs, NCH₂Ar), 4.38 (2H, s, COCH₂O), 4.34 (1H, hept, J=7Hz, NCHMe₂), 2.00 (3H, s, 3-CH₃).
MASS (EI) m/z : 354(M⁺), 252, 238, 211, 166, 116.

### (Example 291)

### Compound of Formula No. B-45

¹H-NMR (CDCl₃) δ(ppm) : 6.97 (1H, brs, 5-H), 4.69 (2H, s, NCH₂Ar), 4.31 (1H, hept, J=7Hz, NCHMe₂), 3.72 (3H, brs, COOCH₃), 1.98 (3H, s, 3-CH₃).
MASS (EI) m/z : 312(M⁺), 297, 196, 154, 122, 116, 82.

### (Example 292)

### Compound of Formula No. B-46

¹H-NMR (CDCl₃) δ(ppm) : 7.01 (1H, s, 5-H), 4.82 (2H, s, NCH₂Ar), 4.33 (1H, hept, J=7Hz, NCHMe₂), 2.00 (3H, s, 3-CH₃), 1.92 (3H, s, COCH₃).
MASS (EI) m/z : 316(M⁺), 274, 180, 138.

### (Example 293)

### Compound of Formula No. B-47

¹H-NMR (CDCl₃) δ(ppm) : 7.11 (1H, s, 5-H), 4.85 (2H, brs, NCH₂Ar), 4.35 (1H, hept, J=7Hz, NCHMe₂), 2.04 (3H, s, 3-CH₃), 1.61-1.48 (1H, m, COCH(CH₂)₂).
MASS (EI) m/z : 342(M⁺), 274, 257, 227, 206, 185, 136, 124.

### (Example 294)

### Compound of Formula No. B-48

¹H-NMR (CDCl₃) δ(ppm) : 7.02 (1H, s, 5-H), 4.86 (2H, brs, NCH₂Ar), 4.35 (1H, hept, J=7Hz, NCHMe₂), 3.84 (2H, d, J=5Hz, COCH₂O), 1.97 (3H, s, 3-CH₃).
MASS (EI) m/z : 332(M⁺), 274, 257, 231, 166, 136, 124.

### (Example 295)

### Compound of Formula No. B-49

¹H-NMR (CDCl₃) δ(ppm) : 7.01 (1H, s, 5-H), 4.82 (2H, brs, NCH₂Ar), 4.34 (1H, hept, J=7Hz, NCHMe₂), 3.80 (2H, s, COCH₂O), 1.99 (3H, s, 3-CH₃).
MASS (EI) m/z : 346(M⁺), 331, 287, 273, 238, 210, 136.

### (Example 296)

### Compound of Formula No. B-50

¹H-NMR (CDCl₃) δ(ppm) : 7.05 (1H, s, 5-H), 4.85 (2H, brs, NCH₂Ar), 4.42 (2H, s, COCH₂O) , 4.33 (1H, hept, J=7Hz, NCHMe₂), 2.05 (3H, s, 3-CH₃).
MASS (EI) m/z : 408(M⁺), 315, 253, 239, 259, 166, 149, 136.

### (Example 297)

### Compound of Formula No. B-51

¹H-NMR (CDCl₃) δ(ppm) : 7.09 (1H, s, 5-H), 4.83 (2H, brs, NCH₂Ar), 4.40 (2H, s, COCH₂O), 4.34 (1H, hept, J=7Hz, NCHMe₂), 2.03 (3H, s, 3-CH₃).
MASS (EI) m/z : 374(M⁺), 331, 272, 238, 166, 136.

### (Example 298)

### Compound of Formula No. B-52

¹H-NMR (CDCl₃) δ(ppm) : 7.02 (1H, s, 5-H), 4.77 (2H, s, NCH₂Ar), 4.31 (1H, hept, J=7Hz, NCHMe₂), 3.72 (3H, brs, COOCH₃), 2.01 (3H, s, 3-CH₃).
MASS (EI) m/z : 332(M⁺), 196, 154, 136, 122.

### (Example 299)

### Compound of Formula No. B-53

¹H-NMR (CDCl₃) δ(ppm) : 6.93 (1H, s, 5-H), 4.73 (2H, brs, NCH₂Ar), 4.32 (1H, hept, J=7Hz, NCHMe₂), 1.97 (3H, s, 3-CH₃), 1.90 (3H, s, COCH₃).
MASS (EI) m/z : 355(M⁺), 313, 270, 180, 149, 138, 124.

### (Example 300)

### Compound of Formula No. B-54

¹H-NMR (CDCl₃) δ(ppm) : 7.00 (1H, s, 5-H), 4.75 (2H, brs, NCH₂Ar), 4.32 (1H, hept, J=7Hz, NCHMe₂), 2.01 (3H, s, 3-CH₃), 1.58-1.45 (1H, m, COCH(CH₂)₂).

MASS (EI) m/z : 381(M⁺), 313, 270, 206, 175, 164, 138, 124.

### (Example 301)

### Compound of Formula No. B-55

¹H-NMR (CDCl₃) δ(ppm) : 6.94 (1H, s, 5-H), 4.77 (2H, brs, NCH₂Ar), 4.32 (1H, hept, J=7Hz, NCHMe₂), 3.82 (2H, d, J=5Hz, COCH₂O), 1.95 (3H, s, 3-CH₃).
MASS (EI) m/z : 371(M⁺), 312, 270, 175, 166, 124.

### (Example 302)

### Compound of Formula No. B-56

¹H-NMR (CDCl₃) δ(ppm) : 6.92 (1H, s, 5-H), 4.74 (2H, brs, NCH₂Ar), 4.32 (1H, hept, J=7Hz, NCHMe₂), 3.78 (2H, s, COCH₂O), 1.96 (3H, s, 3-CH₃).
MASS (EI) m/z : 385(M⁺), 370, 312, 270, 210, 175.

### (Example 303)

### Compound of Formula No. B-57

¹H-NMR (CDCl₃) δ(ppm) : 6.96 (1H, s, 5-H), 4.76 (2H, brs, NCH₂Ar), 4.40 (2H, s, COCH₂O), 4.31 (1H, hept, J=7Hz, NCHMe₂), 2.05 (3H, s, 3-CH₃).
MASS (EI) m/z : 447(M⁺), 354, 326, 312, 272, 175, 166.

### (Example 304)

### Compound of Formula No. B-58

¹H-NMR (CDCl₃) δ(ppm) : 7.00 (1H, s, 5-H), 4.72 (2H, brs, NCH₂Ar), 4.38 (2H, s, COCH₂O), 4.32 (1H, hept, J=7Hz, NCHMe₂), 2.00 (3H, s, 3-CH₃).
MASS (EI) m/z : 413(M⁺), 370, 311, 270, 238, 175, 166.

### (Example 305)

### Compound of Formula No. B-59

¹H-NMR (CDCl₃) δ(ppm) : 6.93 (1H, brs, 5-H), 4.67 (2H, s, NCH₂Ar), 4.30 (1H, hept, J=7Hz, NCHMe₂), 3.71 (3H, brs, COOCH₃), 1.99 (3H, s, 3-CH₃).
MASS (EI) m/z : 371(M⁺), 196, 175, 154.

### (Example 306)

### Compound of Formula No. B-60

¹H-NMR (CDCl₃) δ(ppm) : 6.93 (1H, s, 5-H), 4.75 (2H, s, NCH₂Ar), 3.97 (2H, t, J=7Hz, NCH₂-Pr), 1.98 (3H, s, 3-CH₃), 1.92 (3H, s, COCH₃).
MASS (EI) m/z : 310(M⁺), 268, 194, 152, 116.

### (Example 307)

### Compound of Formula No. B-61

¹H-NMR (CDCl₃) δ(ppm) : 7.00 (1H, s, 5-H), 4.78 (2H, brs, NCH₂Ar), 3.98 (2H, t, J=7Hz, NCH₂-Pr), 2.01 (3H, s, 3-CH₃), 1.58-1.46 (1H, m, COCH(CH₂)₂).
MASS (EI) m/z : 336(M⁺), 268, 220, 152, 116.

### (Example 308)

### Compound of Formula No. B-62

¹H-NMR (CDCl₃) δ(ppm) : 6.94 (1H, s, 5-H), 4.79 (2H, brs, NCH₂Ar), 3.96 (2H, t, J=7Hz, NCH₂-Pr), 3.83 (2H, d, J=5Hz, HOCH₂CO), 1.95 (3H, s, 3-CH₃).
MASS (EI) m/z : 326(M⁺), 295, 267, 210, 180, 116.

### (Example 309)

### Compound of Formula No. B-63

¹H-NMR (CDCl₃) δ(ppm) : 6.92 (1H, s, 5-H), 4.75 (2H, brs, NCH₂Ar), 3.97 (2H, t, J=7Hz, NCH₂-Pr), 3.79 (2H, s, COCH₂O), 1.96 (3H, s, 3-CH₃).
MASS (EI) m/z : 340(M⁺), 325, 267, 224, 136, 116.

### (Example 310)

### Compound of Formula No. B-64

¹H-NMR (CDCl₃) δ(ppm) : 6.97 (1H, s, 5-H), 4.78 (2H, brs, NCH₂Ar), 4.41 (2H, s, COCH₂O), 3.98 (2H, t, J=7Hz, NCH₂-Pr), 2.02 (3H, s, 3-CH₃).
MASS (EI) m/z : 402(M⁺), 309, 267, 136, 116, 107.

### (Example 311)

### Compound of Formula No. B-65

¹H-NMR (CDCl₃) δ(ppm) : 7.01 (1H, s, 5-H), 4.75 (2H, brs, NCH₂Ar), 4.38 (2H, s, COCH₂O), 3.98 (2H, t, J=7Hz, NCH₂-Pr), 2.01 (3H, s, 3-CH₃).
MASS (EI) m/z : 368(M⁺), 325, 266, 210, 180, 116.

### (Example 312)

### Compound of Formula No. B-66

¹H-NMR (CDCl₃) δ(ppm) : 6.93 (1H, s, 5-H), 4.69 (2H, s, NCH₂Ar), 3.94 (2H, t, J=7Hz, NCH₂-Pr), 3.72 (3H, brs, COOCH₃), 1.98 (3H, s, 3-CH₃).
MASS (EI) m/z : 326(M⁺), 283, 267, 210, 168, 116.

### (Example 313)

### Compound of Formula No. B-67

¹H-NMR (CDCl₃) δ(ppm) : 6.97 (1H, s, 5-H), 4.83 (2H, brs, NCH₂Ar), 3.96 (2H, t, J=7Hz, NCH₂-Pr), 2.00 (3H, s, 3-CH₃), 1.93 (3H, s, COCH₃).
MASS (EI) m/z : 330(M⁺), 287, 271, 194, 152, 136, 110.

### (Example 314)

### Compound of Formula No. B-68

¹H-NMR (CDCl₃) δ(ppm) : 7.04 (1H, s, 5-H), 4.85 (2H, s, NCH₂Ar), 3.97 (2H, t, J=7Hz, NCH₂-Pr), 2.04 (3H, s, 3-CH₃), 1.61-1.46 (1H, m, COCH(CH₂)₂).
MASS (EI) m/z : 356(M⁺), 288, 220, 152, 136.

### (Example 315)

### Compound of Formula No. B-69

¹H-NMR (CDCl₃) δ(ppm) : 6.97 (1H, s, 5-H), 4.86 (2H, brs, NCH₂Ar), 3.97 (2H, t, J=7Hz, NCH₂-Pr), 3.85 (2H, d, J=5Hz, COCH₂O), 1.98 (3H, s, 3-CH₃).
MASS (EI) m/z : 346(M⁺), 315, 287, 180, 152, 136.

### (Example 316)

### Compound of Formula No. B-70

¹H-NMR (CDCl₃) δ(ppm) : 6.96 (1H, s, 5-H), 4.83 (2H, brs, NCH₂Ar), 3.97 (2H, t, J=7Hz, NCH₂-Pr), 3.80 (2H, s, COCH₂O), 1.99 (3H, s, 3-CH₃).
MASS (EI) m/z : 360(M⁺), 345, 315, 287, 224, 136.

### (Example 317)

### Compound of Formula No. B-71

¹H-NMR (CDCl₃) δ(ppm) : 7.01 (1H, s, 5-H), 4.85 (2H, brs, NCH₂Ar), 4.42 (2H, s, COCH₂O), 3.97 (2H, t, J=7Hz, NCH₂-Pr), 2.05 (3H, s, 3-CH₃).
MASS (EI) m/z : 422(M⁺), 329, 315, 287, 136, 107.

### (Example 318)

### Compound of Formula No. B-72

¹H-NMR (CDCl₃) δ(ppm) : 7.04 (1H, s, 5-H), 4.82 (2H, brs, NCH₂Ar), 4.39 (2H, s, COCH₂O), 3.97 (2H, t, J=7Hz, NCH₂-Pr), 2.04 (3H, s, 3-CH₃).
MASS (EI) m/z : 388(M⁺), 345, 286, 252, 180, 136.

### (Example 319)

### Compound of Formula No. B-73

¹H-NMR (CDCl₃) δ(ppm) : 6.97 (1H, s, 5-H), 4.77 (2H, s, NCH₂Ar), 3.94 (2H, t, J=7Hz, NCH₂-Pr), 3.73 (3H, s, COOCH₃), 2.01 (3H, s, 3-CH₃).
MASS (EI) m/z : 346(M⁺), 287, 210, 168, 154, 136.

### (Example 320)

### Compound of Formula No. B-74

¹H-NMR (CDCl₃) δ(ppm) : 6.89 (1H, s, 5-H), 4.74 (2H, brs, NCH₂Ar), 3.95 (2H, t, J=7Hz, NCH₂-Pr), 1.97 (3H, s, 3-CH₃), 1.90 (3H, s, COCH₃).
MASS (EI) m/z : 369(M'), 326, 194, 175, 152.

### (Example 321)

### Compound of Formula No. B-75

¹H-NMR (CDCl₃) δ(ppm) : 6.96 (1H, s, 5-H), 4.78 (2H, brs, NCH₂Ar), 3.95 (2H, t, J=7Hz, NCH₂-Pr), 2.00 (3H, s, 3-CH₃), 1.57-1.46 (1H, m, COCH(CH₂)₂).
MASS (EI) m/z : 395(M⁺), 327, 220, 175, 152, 138.

### (Example 322)

### Compound of Formula No. B-76

¹H-NMR (CDCl₃) δ(ppm) : 6.90 (1H, s, 5-H), 4.77 (2H, brs, NCH₂Ar), 3.95 (2H, t, J=7Hz, NCH₂-Pr), 3.82 (2H, d, J=5Hz, COCH₂O), 1.94 (3H, s, 3-CH₃).
MASS (EI) m/z : 385(M⁺), 326, 180, 175, 152.

### (Example 323)

### Compound of Formula No. B-77

¹H-NMR (CDCl₃) δ(ppm) : 6.89 (1H, s, 5-H), 4.74 (2H, brs, NCH₂Ar), 3.95 (2H, t, J=7Hz, NCH₂-Pr), 3.78 (2H, s, COCH₂O), 1.95 (3H, s, 3-CH₃).
MASS (EI) m/z : 399(M⁺), 354, 326, 224, 175, 138.

### (Example 324)

### Compound of Formula No. B-78

¹H-NMR (CDCl₃) δ(ppm) : 6.94 (1H, s, 5-H), 4.75 (2H, brs, NCH₂Ar), 4.40 (2H, s, COCH₂O), 3.95 (2H, t, J=7Hz, NCH₂-Pr), 2.01 (3H, s, 3-CH₃).
MASS (EI) m/z : 461(M⁺), 368, 326, 286, 175, 107.

### (Example 325)

### Compound of Formula No. B-79

¹H-NMR (CDCl₃) δ(ppm) : 6.97 (1H, s, 5-H), 4.74 (2H, brs, NCH₂Ar), 4.38 (2H, s, COCH₂O), 3.95 (2H, t, J=7Hz, NCH₂-Pr), 2.00 (3H, s, 3-CH₃).
MASS (EI) m/z : 427(M⁺), 384, 325, 252, 180, 152.

### (Example 326)

### Compound of Formula No. B-80

¹H-NMR (CDCl₃) δ(ppm) : 6.90 (1H, s, 5-H), 4.67 (2H, s, NCH₂Ar), 3.92 (2H, t, J=7Hz, NCH₂-Pr), 3.71 (3H, brs, COOCH₃), 1.98 (3H, s, 3-CH₃).
MASS (EI) m/z : 385(M⁺), 342, 210, 168, 154, 136.

### (Example 327)

### Compound of Formula No. B-81

¹H-NMR (CDCl₃) δ(ppm) : 6.89 (1H, s, 5-H), 4.75 (2H, brs, NCH₂Ar), 3.75 (2H, d, J=7Hz, NCH₂-iPr), 1.99 (3H, s, 3-CH₃), 1.91 (3H, s, COCH₃).
MASS (EI) m/z : 310(M⁺), 295, 268, 194, 152, 116.

### (Example 328)

### Compound of Formula No. B-82

¹H-NMR (CDCl₃) δ(ppm) : 6.88 (1H, s, 5-H), 4.75 (2H, brs, NCH₂Ar), 3.75 (2H, d, J=7Hz, NCH₂-iPr), 2.11 (2H, q, J=7Hz, MeCH₂-CO), 1.97 (3H, s, 3-CH₃).
MASS (EI) m/z : 324(M⁺), 268, 208, 152, 116.

### (Example 329)

### Compound of Formula No. B-83

¹H-NMR (CDCl₃) δ(ppm) : 6.96 (1H, s, 5-H), 4.76 (2H, brs, NCH₂Ar), 3.75 (2H, d, J=7Hz, NCH₂-iPr), 2.02 (3H, s, 3-CH₃), 1.57-1.41 (1H, m, COCH(CH₂)₂).
MASS (EI) m/z : 336(M⁺), 268, 220, 152, 116.

### (Example 330)

### Compound of Formula No. B-84

¹H-NMR (CDCl₃) δ(ppm) : 6.98 (1H, s, 5-H), 4.78 (2H, brs, NCH₂Ar), 3.89 (2H, s, COCH₂Cl), 3.76 (2H, d, J=7Hz, NCH₂-iPr), 2.01 (3H, s, 3-CH₃).
MASS (EI) m/z : 344(M⁺), 267, 228, 200, 179, 136, 116.

### (Example 331)

### Compound of Formula No. B-85

¹H-NMR (CDCl₃) δ(ppm) : 6.89 (1H, s, 5-H), 4.79 (2H, brs, NCH₂Ar), 3.83 (2H, d, J=5Hz, COCH₂O), 3.75 (2H, d, J=7Hz, NCH₂-iPr), 1.97 (3H, s, 3-CH₃).
MASS (EI) m/z : 326(M⁺), 310, 267, 180, 152, 116.

### (Example 332)

### Compound of Formula No. B-86

¹H-NMR (CDCl₃) δ(ppm) : 6.90 (1H, s, 5-H), 4.76 (2H, brs, NCH₂Ar), 3.79 (2H, s, COCH₂O), 3.76 (2H, d, J=7Hz, NCH₂-iPr), 1.98 (3H, s, 3-CH₃).
MASS (EI) m/z : 340(M⁺), 325, 267, 224, 152, 138, 116.

### (Example 333)

### Compound of Formula No. B-87

¹H-NMR (CDCl₃) δ(ppm) : 6.94 (1H, s, 5-H), 4.78 (2H, brs, NCH₂Ar), 4.41 (2H, s, COCH₂O), 3.76 (2H, d, J=7Hz, NCH₂-iPr), 2.03 (3H, s, 3-CH₃).
MASS (EI) m/z : 402(M⁺), 295, 267, 253, 136, 116.

### (Example 334)

### Compound of Formula No. B-88

¹H-NMR (CDCl₃) δ(ppm) : 6.96 (1H, s, 5-H), 4.75 (2H, brs, NCH₂Ar), 4.38 (2H, s, COCH₂O), 3.75 (2H, d, J=7Hz, NCH₂-iPr), 2.02 (3H, s, 3-CH₃).
MASS (EI) m/z : 368(M⁺), 325, 266, 252, 210, 180, 116.

### (Example 335)

### Compound of Formula No. B-90

¹H-NMR (CDCl₃) δ(ppm) : 6.89 (1H, s, 5-H), 4.69 (2H, brs, NCH₂Ar), 4.17 (2H, brq, J=7Hz, MeCH₂O), 3.72 (2H, d, J=7Hz, NCH₂-iPr), 1.99 (3H, s, 3-CH₃).
MASS (EI) m/z : 340(M⁺), 325, 284, 267, 224, 152, 116.

### (Example 336)

### Compound of Formula No. B-91

¹H-NMR (CDCl₃) δ(ppm) : 6.88 (1H, s, 5-H), 4.96 (1H, hept, J=7Hz, OCHMe₂), 4.68 (2H, s, NCH₂Ar), 3.72 (2H, d, J=7Hz, NCH₂-iPr), 1.98 (3H, s, 3-CH₃).

MASS (EI) m/z : 354(M⁺), 312, 267, 256, 196, 116.

### (Example 337)

### Compound of Formula No. B-92

¹H-NMR (CDCl₃) δ(ppm) : 6.93 (1H, s, 5-H), 4.62 (2H, s, NCH₂Ar), 3.73 (2H, d, J=7Hz, NCH₂-iPr), 3.25 (4H, t, J=5Hz, N(CH₂CH₂)₂O), 2.00 (3H, s, 3-CH₃).
MASS (EI) m/z : 381(M⁺), 265, 180, 149, 136, 114.

### (Example 338)

### Compound of Formula No. B-94

¹H-NMR (CDCl₃) δ(ppm) : 6.94 (1H, s, 5-H), 4.87 (2H, brs, NCH₂Ar), 3.85 (2H, d, J=4Hz, COCH₂O), 3.75 (2H, d, J=7Hz, NCH₂-iPr), 1.99 (3H, s, 3-CH₃).
MASS (EI) m/z : 346(M⁺), 326, 287, 271, 210, 180, 168, 152.

### (Example 339)

### Compound of Formula No. B-95

¹H-NMR (CDCl₃) δ(ppm) : 6.95 (1H, s, 5-H), 4.85 (2H, brs, NCH₂Ar), 3.81 (2H, s, COCH₂O), 3.75 (2H, d, J=7Hz, NCH₂-iPr), 2.00 (3H, s, 3-CH₃).
MASS (EI) m/z : 360(M⁺), 345, 315, 301, 287, 259, 224, 196.

### (Example 340)

### Compound of Formula No. B-96

¹H-NMR (CDCl₃) δ(ppm) : 7.02 (1H, s, 5-H), 4.87 (2H, brs, NCH₂Ar), 4.43 (2H, s, COCH₂O), 4.04 (2H, d, J=7Hz, NCH₂-ipr), 2.06 (3H, s, 3-CH₃).
MASS (EI) m/z : 422(M⁺), 329, 315, 301, 287, 273, 245, 136

### (Example 341)

### Compound of Formula No. B-97

¹H-NMR (CDCl₃) δ(ppm) : 7.01 (1H, s, 5-H), 4.83 (2H, brs, NCH₂Ar), 4.40 (2H, s, COCH₂O), 4.12 (2H, d, J=7Hz, NCH₂-iPr), 2.05 (3H, s, 3-CH₃).
MASS (EI) m/z : 388(M⁺), 345, 286, 271, 252, 230, 210, 180.

### (Example 342)

### Compound of Formula No. B-98

¹H-NMR (CDCl₃) δ(ppm) : 6.94 (1H, s, 5-H), 4.76 (2H, brs, NCH₂Ar), 3.73 (2H, d, J=7Hz, NCH₂-iPr), 2.00 (3H, s, 3-CH₃), 1.57-1.44 (1H, m, COCH(CH₂)₂).
MASS (EI) m/z : 395(M⁺), 380, 352, 327, 284, 220, 175, 164.

### (Example 343)

### Compound of Formula No. B-99

¹H-NMR (CDCl₃) δ(ppm) : 6.88 (1H, s, 5-H), 4.78 (2H, brs, NCH₂Ar), 3.82 (2H, d, J=5Hz, COCH₂O), 3.73 (2H, d, J=7Hz, NCH₂-iPr), 1.95 (3H, s, 3-CH₃).
MASS (EI) m/z : 385(M⁺), 354, 326, 175, 152, 138, 124.

### (Example 344)

### Compound of Formula No. B-100

¹H-NMR (CDCl₃) δ(ppm) : 6.87 (1H, s, 5-H), 4.75 (2H, brs, NCH₂Ar), 3.79 (2H, s, COCH₂O), 3.73 (2H, d, J=7Hz, NCH₂-iPr), 1.96 (3H, s, 3-CH₃).
MASS (EI) m/z : 399(M⁺), 384, 354, 326, 224, 194, 175, 138.

### (Example 345)

### Compound of Formula No. B-101

¹H-NMR (CDCl₃) δ(ppm) : 6.95 (1H, s, 5-H), 4.75 (2H, brs, NCH₂Ar), 4.38 (2H, s, COCH₂O), 3.73 (2H, d, J=7Hz, NCH₂-iPr), 2.01 (3H, s, 3-CH₃).
MASS (EI) m/z : 427(M⁺), 384, 325, 282, 269, 252, 180, 152.

### (Example 346)

### Compound of Formula No. B-102

¹H-NMR (CDCl₃) δ(ppm) : 6.91 (1H, s, 5-H), 4.74 (2H, brs, NCH₂Ar), 3.98 (2H, d, J=7Hz, NCH₂-cBu), 1.97 (3H, s, 3-CH₃), 1.90 (3H, s, COCH₃).
MASS (EI) m/z : 322(M⁺), 308, 280, 266, 225, 212, 206, 178.

### (Example 347)

### Compound of Formula No. B-103

¹H-NMR (CDCl₃) δ(ppm) : 6.96 (1H, s, 5-H), 4.76 (2H, brs, NCH₂Ar), 3.98 (2H, d, J=7Hz, NCH₂-cBu), 2.01 (3H, s, 3-CH₃), 1.55-1.45 (1H, m, COCH(CH₂)₂).
MASS (EI) m/z : 348(M⁺), 280, 232, 212, 164, 149, 116.

### (Example 348)

### Compound of Formula No. B-104

¹H-NMR (CDCl₃) δ(ppm) : 6.90 (1H, s, 5-H), 4.78 (2H, brs, NCH₂Ar), 3.98 (2H, d, J=7Hz, NCH₂-cBu), 3.81 (2H, d, J=5Hz, COCH₂O), 1.95 (3H, s, 3-CH₃).
MASS (EI) m/z : 338(M⁺), 279, 239, 222, 211, 192, 164 150.

### (Example 349)

### Compound of Formula No. B-105

¹H-NMR (CDCl₃) δ(ppm) : 6.91 (1H, s, 5-H), 4.75 (2H, brs, NCH₂Ar), 3.98 (2H, d, J=7Hz, NCH₂-cBu), 3.78 (2H, s, COCH₂O), 1.96 (3H, s, 3-CH₃).
MASS (EI) m/z : 352(M⁺), 337, 322, 307, 293, 279, 236, 225.

### (Example 350)

### Compound of Formula No. B-106

¹H-NMR (CDCl₃) δ(ppm) : 6.95 (1H, s, 5-H), 4.78 (2H, brs, NCH₂Ar), 4.39 (2H, s, COCH₂O), 3.98 (2H, d, J=7Hz, NCH₂-cBu), 2.02 (3H, s, 3-CH₃).
MASS (EI) m/z : 414(M⁺), 321, 298, 279, 253, 239, 211, 150.

### (Example 351)

### Compound of Formula No. B-107

¹H-NMR (CDCl₃) δ(ppm) : 6.97 (1H, s, 5-H), 4.74 (2H, brs, NCH₂Ar), 4.37 (2H, s, COCH₂O), 3.98 (2H, d, J=7Hz, NCH₂-cBu), 2.01 (3H, s, 3-CH₃).
MASS (EI) m/z : 380(M⁺), 337, 278, 264, 250, 210, 192, 164.

### (Example 352)

### Compound of Formula No. B-108

¹H-NMR (CDCl₃) δ(ppm) : 6.89 (1H, brs, 5-H), 4.68 (2H, brs, NCH₂Ar), 3.95 (2H, d, J=7Hz, NCH₂-cBu), 3.71 (3H, brs, COOCH₃), 1.98 (3H, s, 3-CH₃).
MASS (EI) m/z : 338(M⁺), 324, 310, 283, 270, 222, 194, 168.

### (Example 353)

### Compound of Formula No. B-109

¹H-NMR (CDCl₃) δ(ppm) : 6.93 (1H, s, 5-H), 4.82 (2H, s, NCH₂Ar), 3.97 (2H, d, J=7Hz, NCH₂-cBu), 2.00 (3H, s, 3-CH₃), 1.91 (3H, s, COCH₃).
MASS (EI) m/z : 342(M⁺), 300, 232, 206, 164, 136, 110, 69.

### (Example 354)

### Compound of Formula No. B-110

¹H-NMR (CDCl₃) δ(ppm) : 7.03 (1H, s, 5-H), 4.85 (2H, brs, NCH₂Ar), 3.98 (2H, d, J=7Hz, NCH₂-cBu), 2.03 (3H, s, 3-CH₃), 1.58-1.46 (1H, m, COCH(CH₂)₂).
MASS (EI) m/z : 368(M⁺), 300, 283, 232, 164, 150, 136, 122.

### (Example 355)

### Compound of Formula No. B-111

¹H-NMR (CDCl₃) δ(ppm) : 6.95 (1H, s, 5-H), 4.85 (2H, brs, NCH₂Ar), 3.97 (2H, d, J=7Hz, NCH₂-cBu), 3.83 (2H, d, J=5Hz, COCH₂O), 1.97 (3H, s, 3-CH₃).
MASS (EI) m/z : 358(M⁺), 327, 299, 283, 259, 232, 222, 192.

### (Example 356)

### Compound of Formula No. B-112

¹H-NMR (CDCl₃) δ(ppm) : 6.97 (1H, s, 5-H), 4.84 (2H, brs, NCH₂Ar), 3.98 (2H, d, J=7Hz, NCH₂-cBu), 3.80 (2H, s, COCH₂O), 1.99 (3H, s, 3-CH₃).
MASS (EI) m/z : 372(M⁺), 357, 327, 313, 299, 259, 236, 208.

### (Example 357)

### Compound of Formula No. B-113

¹H-NMR (CDCl₃) δ(ppm) : 6.98 (1H, s, 5-H), 4.85 (2H, brs, NCH₂Ar), 4.41 (2H, s, COCH₂O), 3.97 (2H, d, J=7Hz, NCH₂-cBu), 2.04 (3H, s, 3-CH₃).
MASS (EI) m/z : 434(M⁺), 341, 313, 299, 273, 259, 231, 150.

### (Example 358)

### Compound of Formula No. B-114

¹H-NMR (CDCl₃) δ(ppm) : 7.00 (1H, s, 5-H), 4.82 (2H, brs, NCH₂Ar), 4.38 (2H, s, COCH₂O), 3.97 (2H, d, J=7Hz, NCH₂-cBu), 2.03 (3H, s, 3-CH₃).
MASS (EI) m/z : 400(M⁺), 298, 264, 230, 222, 214, 192, 164.

### (Example 359)

### Compound of Formula No. B-115

¹H-NMR (CDCl₃) δ(ppm) : 6.93 (1H, s, 5-H), 4.76 (2H, s, NCH₂Ar), 3.95 (2H, d, J=7Hz, NCH₂-cBu), 3.72 (3H, brs, COOCH₃), 2.00 (3H, s, 3-CH₃).
MASS (EI) m/z : 358(M⁺), 343, 299, 290, 222, 154, 136.

### (Example 360)

### Compound of Formula No. B-116

¹H-NMR (CDCl₃) δ(ppm) : 6.86 (1H, s, 5-H), 4.73 (2H, brs, NCH₂Ar), 3.95 (2H, t, J=7Hz, NCH₂-cBu), 1.96 (3H, s, 3-CH₃), 1.89 (3H, s, COCH₃).
MASS (EI) m/z : 381(M⁺), 338, 271, 206, 175, 164, 150.

### (Example 361)

### Compound of Formula No. B-117

¹H-NMR (CDCl₃) δ(ppm) : 6.94 (1H, s, 5-H), 4.75 (2H, brs, NCH₂Ar), 3.96 (2H, d, J=7Hz, NCH₂-cBu), 2.00 (3H, s, 3-CH₃), 1.56-1.43 (1H, m, COCH(CH₂)₂).
MASS (EI) m/z : 407(M⁺), 339, 232, 175, 164, 150.

### (Example 362)

### Compound of Formula No. B-118

¹H-NMR (CDCl₃) δ(ppm) : 6.87 (1H, s, 5-H), 4.76 (2H, brs, NCH₂Ar), 3.94 (2H, d, J=7Hz, NCH₂-cBu), 3.81 (2H, d, J=5Hz, COCH₂O), 1.94 (3H, s, 3-CH₃).
MASS (EI) m/z: 397(M⁺), 366, 338, 270, 222, 192, 175, 164.

### (Example 363)

### Compound of Formula No. B-119

¹H-NMR (CDCl₃) δ(ppm) : 6.86 (1H, s, 5-H), 4.73 (2H, brs, NCH₂Ar), 3.96 (2H, d, J=7Hz, NCH₂-cBu), 3.77 (2H, s, COCH₂O), 1.95 (3H, s, 3-CH₃).
MASS (EI) m/z : 411(M⁺), 338, 270, 236, 206, 175, 136.

### (Example 364)

### Compound of Formula No. B-120

¹H-NMR (CDCl₃) δ(ppm) : 6.91 (1H, s, 5-H), 4.77 (2H, brs, NCH₂Ar), 4.39 (2H, s, COCH₂O), 3.96 (2H, d, J=7Hz, NCH₂-cBu), 2.01 (3H, s, 3-CH₃).
MASS (EI) m/z : 473(M⁺), 380, 352, 338, 312, 298, 175.

### (Example 365)

### Compound of Formula No. B-121

¹H-NMR (CDCl₃) δ(ppm) : 6.94 (1H, s, 5-H), 4.73 (2H, brs, NCH₂Ar), 4.37 (2H, s, COCH₂O), 3.95 (2H, d, J=7Hz, NCH₂-cBu), 2.00 (3H, s, 3-CH₃).
MASS (EI) m/z : 439(M⁺), 337, 269, 192, 175, 164, 150 124.

### (Example 366)

### Compound of Formula No. B-122

¹H-NMR (CDCl₃) δ(ppm) : 6.87 (1H, s, 5-H), 4.67 (2H, brs, NCH₂Ar), 3.93 (2H, d, J=7Hz, NCH₂-cBu), 3.71 (3H, brs, COOCH₃), 1.98 (3H, s, 3-CH₃).
MASS (EI) m/z : 397(M⁺), 329, 222, 194, 175, 154, 122.

### (Example 367)

### Compound of Formula No. B-123

¹H-NMR (CDCl₃) δ(ppm) : 6.88 (1H, s, 5-H), 5.16 (2H, s, NCH₂Ph), 4.72 (2H, s, NCH₂Ar), 1.99 (3H, s, 3-CH₃), 1.90 (3H, s, COCH₃).
MASS (EI) m/z : 344(M⁺), 302, 228, 171, 116.

### (Example 368)

### Compound of Formula No. B-124

¹H-NMR (CDCl₃) δ(ppm) : 6.96 (1H, s, 5-H), 5.16 (2H, s, NCH₂Ph), 4.74 (2H, brs, NCH₂Ar), 2.02 (3H, s, 3-CH₃), 1.54-1.44 (1H, m, COCH(CH₂)₂).
MASS (EI) m/z : 370(M⁺), 302, 254, 186, 116.

### (Example 369)

### Compound of Formula No. B-125

¹H-NMR (CDCl₃) δ(ppm) : 6.89 (1H, s, 5-H), 5.16 (2H, s, NCH₂Ph), 4.76 (2H, brs, NCH₂Ar), 3.82 (2H, d, J=5Hz, COCH₂O), 1.96 (3H, s, 3-CH₃).
MASS (EI) m/z : 360(M⁺), 301, 214, 171, 116.

### (Example 370)

### Compound of Formula No. B-126

¹H-NMR (CDCl₃) δ(ppm) : 6.89 (1H, s, 5-H), 5.16 (2H, s, NCH₂Ph), 4.73 (2H, brs, NCH₂Ar), 3.77 (2H, s, COCH₂O), 1.98 (3H, s, 3-CH₃).
MASS (EI) m/z : 374(M⁺), 301, 171, 116.

### (Example 371)

### Compound of Formula No. B-127

¹H-NMR (CDCl₃) δ(ppm) : 6.93 (1H, s, 5-H), 5.16 (2H, s, NCH₂Ph), 4.75 (2H, brs, NCH₂Ar), 4.39 (2H, s, COCH₂O), 2.03 (3H, s, 3-CH₃).
MASS (EI) m/z : 436(M⁺), 301, 265, 207, 149, 116.

### (Example 372)

### Compound of Formula No. B-128

¹H-NMR (CDCl₃) δ(ppm) : 6.96 (1H, s, 5-H), 5.16 (2H, s, NCH₂Ph), 4.72 (2H, brs, NCH₂Ar), 4.37 (2H, s, COCH₂O), 2.03 (3H, s, 3-CH₃).
MASS (EI) m/z : 402(M⁺), 300, 214, 116.

### (Example 373)

### Compound of Formula No. B-129

¹H-NMR (CDCl₃) δ(ppm) : 6.88 (1H, brs, 5-H), 5.13 (2H, s, NCH₂Ph), 4.66 (2H, s, NCH₂Ar), 3.72 (3H, brs, COOCH₃), 1.99 (3H, s, 3-CH₃).
MASS (EI) m/z : 360(M⁺), 244, 116.

### (Example 374)

### Compound of Formula No. B-130

¹H-NMR (CDCl₃) δ(ppm) : 7.21 (1H, s, 5-H), 5.23 (2H, s, NCH₂OMe), 4.80 (2H, s, NCH₂Ar), 4.42 (2H, s, COCH₂O), 2.03 (3H, s, 3-CH₃).
MASS (EI) m/z : 390(M⁺), 359, 265, 255, 136, 116.

### (Example 375)

### Compound of Formula No. B-131

¹H-NMR (CDCl₃) δ(ppm) : 7.02 (1H, s, 5-H), 4.76 (2H, brs, NCH₂Ar), 4.49 (2H, s, ArOCH₂CO), 3.79 (3H, s, NCH₃), 1.97 (3H, s, 3-CH₃).
MASS (EI) m/z : 378(M⁺), 267, 239, 225, 116.

### (Example 376)

### Compound of Formula No. B-132

¹H-NMR (CDCl₃) δ(ppm) : 7.04 (1H, s, 5-H), 4.78 (2H, brs, NCH₂Ar), 4.40 (2H, s, ArOCH₂CO), 3.81 (3H, s, NCH₃), 1.98 (3H, s, 3-CH₃).
MASS (EI) m/z : 378(M⁺), 219, 149, 116.

### (Example 377)

### Compound of Formula No. B-133

¹H-NMR (CDCl₃) δ(ppm) : 7.02 (1H, s, 5-H), 4.77 (2H, brs, NCH₂Ar), 4.38 (2H, s, ArOCH₂CO), 3.81 (3H, s, NCH₃), 1.97 (3H, s, 3-CH₃).
MASS (EI) m/z : 378(M⁺), 267, 239, 225, 125, 116.

### (Example 378)

### Compound of Formula No. B-134

¹H-NMR (CDCl₃) δ(ppm) : 7.04 (1H, s, 5-H), 4.76 (2H, brs, NCH₂Ar), 4.50 (2H, s, ArOCH₂CO), 3.78 (3H, s, NCH₃), 1.98 (3H, s, 3-CH₃).
MASS (EI) m/z : 394(M⁺), 359, 267, 239, 225, 141, 116.

### (Example 379)

### Compound of Formula No. B-135

¹H-NMR (CDCl₃) δ(ppm) : 7.04 (1H, s, 5-H), 4.77 (2H, brs, NCH₂Ar), 4.40 (2H, s, ArOCH₂CO), 3.82 (3H, s, NCH₃), 1.99 (3H, s, 3-CH₃).
MASS (EI) m/z : 394(M⁺), 359, 267, 225, 141, 116.

### (Example 380)

### Compound of Formula No. B-136

¹H-NMR (CDCl₃) δ(ppm) : 7.03 (1H, s, 5-H), 4.77 (2H, brs, NCH₂Ar), 4.39 (2H, s, ArOCH₂CO), 3.81 (3H, s, NCH₃), 1.97 (3H, s, 3-CH₃).
MASS (EI) m/z : 394(M⁺), 267, 239, 225, 141, 116.

### (Example 381)

### Compound of Formula No. B-137

¹H-NMR (CDCl₃) δ(ppm) : 6.99 (1H, s, 5-H), 4.78 (2H, brs, NCH₂Ar), 4.44 (2H, s, ArOCH₂CO), 3.80 (3H, s, NCH₃), 1.98 (3H, s, 3-CH₃).
MASS (EI) m/z : 374(M⁺), 267, 239, 226, 152, 116.

### (Example 382)

### Compound of Formula No. B-138

¹H-NMR (CDCl₃) δ(ppm) : 7.02 (1H, s, 5-H), 4.77 (2H, brs, NCH₂Ar), 4.40 (2H, s, ArOCH₂CO), 3.80 (3H, s, NCH₃), 1.99 (3H, s, 3-CH₃).
MASS (EI) m/z : 374(M⁺), 267, 239, 225, 149, 116.

### (Example 383)

### Compound of Formula No. B-139

¹H-NMR (CDCl₃) δ(ppm) : 7.01 (1H, s, 5-H), 4.77 (2H, brs, NCH₂Ar), 4.38 (2H, s, ArOCH₂CO), 3.80 (3H, s, NCH₃), 1.97 (3H, s, 3-CH₃).
MASS (EI) m/z : 374(M⁺), 267, 239, 224, 149, 116.

### (Example 384)

### Compound of Formula No. B-140

¹H-NMR (CDCl₃) δ(ppm) : 7.02 (1H, s, 5-H), 4.76 (2H, brs, NCH₂Ar), 4.47 (2H, s, ArOCH₂CO), 3.79 (3H, s, NCH₃), 1.97 (3H, s, 3-CH₃).
MASS (EI) m/z : 390(M⁺), 267, 239, 225, 137, 116.

### (Example 385)

### Compound of Formula No. B-141

¹H-NMR (CDCl₃) δ(ppm) : 7.02 (1H, s, 5-H), 4.77 (2H, brs, NCH₂Ar), 4.39 (2H, s, ArOCH₂CO), 3.81 (3H, s, NCH₃), 1.98 (3H, s, 3-CH₃).
MASS (EI) m/z : 390(M⁺), 267, 239, 224, 137, 116.

### (Example 386)

### Compound of Formula No. B-142

¹H-NMR (CDCl₃) δ(ppm) : 7.01 (1H, s, 5-H), 4.77 (2H, brs, NCH₂Ar), 4.37 (2H, s, ArOCH₂CO), 3.80 (3H, s, NCH₃), 1.97 (3H, s, 3-CH₃).
MASS (EI) m/z : 390(M⁺), 267, 239, 225, 137, 116.

### (Example 387)

### Compound of Formula No. B-143

¹H-NMR (CDCl₃) δ(ppm) : 7.08 (1H, s, 5-H), 4.77 (2H, brs, NCH₂Ar), 4.58 (2H, s, ArOCH₂CO), 3.82 (3H, s, NCH₃), 1.98 (3H, s, 3-CH₃).
MASS (EI) m/z : 405(M⁺), 359, 267, 239, 224, 152, 116.

### (Example 388)

### Compound of Formula No. B-144

¹H-NMR (CDCl₃) δ(ppm) : 7.13 (1H, s, 5-H), 4.78 (2H, brs, NCH₂Ar), 4.52 (2H, s, ArOCH₂CO), 3.84 (3H, s, NCH₃), 2.02 (3H, s, 3-CH₃).
MASS (EI) m/z : 405(M⁺), 360, 289, 267, 239, 225, 152, 116.

### (Example 389)

### Compound of Formula No. B-145

¹H-NMR (CDCl₃) δ(ppm) : 7.09 (1H, s, 5-H), 4.78 (2H, brs, NCH₂Ar), 4.52 (2H, s, ArOCH₂CO), 3.84 (3H, s, NCH₃), 1.99 (3H, s, 3-CH₃).
MASS (EI) m/z : 405(M⁺), 289, 267, 225, 152, 116.

### (Example 390)

### Compound of Formula No. B-146

¹H-NMR (CDCl₃) δ(ppm) : 7.05 (1H, s, 5-H), 4.78 (2H, brs, NCH₂Ar), 4.52 (2H, s, ArOCH₂CO), 3.78 (3H, s, NCH₃), 2.01 (3H, s, 3-CH₃).
MASS (EI) m/z : 428(M⁺), 409, 312, 267, 225, 175, 116.

### (Example 391)

### Compound of Formula No. B-147

¹H-NMR (CDCl₃) δ(ppm) : 7.05 (1H, s, 5-H), 4.78 (2H, brs, NCH₂Ar), 4.46 (2H, s, ArOCH₂CO), 3.82 (3H, s, NCH₃), 1.99 (3H, s, 3-CH₃).
MASS (EI) m/z : 428(M⁺), 409, 312, 267, 225, 175, 116.

### (Example 392)

### Compound of Formula No. B-148

¹H-NMR (CDCl₃) δ(ppm) : 7.06 (1H, s, 5-H), 4.78 (2H, brs, NCH₂Ar), 4.46 (2H, s, ArOCH₂CO), 3.82 (3H, s, NCH₃), 1.99 (3H, s, 3-CH₃).
MASS (EI) m/z : 428(M⁺), 409, 312, 267, 225, 175, 116.

### (Example 393)

### Compound of Formula No. B-149

¹H-NMR (CDCl₃) δ(ppm) : 7.13 (1H, s, 5-H), 4.76 (2H, brs, NCH₂Ar), 4.58 (2H, s, ArOCH₂CO), 3.81 (3H, s, NCH₃), 1.99 (3H, s, 3-CH₃).
MASS (EI) m/z : 385(M⁺), 295, 268, 225, 132, 116.

### (Example 394)

### Compound of Formula No. B-150

¹H-NMR (CDCl₃) δ(ppm) : 7.08 (1H, s, 5-H), 4.78 (2H, brs, NCH₂Ar), 4.44 (2H, s, ArOCH₂CO), 3.84 (3H, s, NCH₃), 1.98 (3H, s, 3-CH₃).
MASS (EI) m/z : 385(M⁺), 269, 225, 132, 116.

### (Example 395)

### Compound of Formula No. B-151

¹H-NMR (CDCl₃) δ(ppm) : 7.06 (1H, s, 5-H), 4.76 (2H, brs, NCH₂Ar), 4.47 (2H, s, ArOCH₂CO), 3.82 (3H, s, NCH₃), 1.98 (3H, s, 3-CH₃).
MASS (EI) m/z : 385(M⁺), 269, 225, 132, 116.

### (Example 396)

### Compound of Formula No. B-152

¹H-NMR (CDCl₃) δ(ppm) : 7.02 (1H, s, 3-H), 4.77 (2H, brs, NCH₂Ar), 4.45 (2H, s, ArOCH₂CO), 3.80 (3H, s, NCH₃), 1.95 (3H, s, 5-CH₃).
MASS (EI) m/z : 396(M⁺), 280, 267, 239, 225, 143, 116.

### (Example 397)

### Compound of Formula No. B-153

¹H-NMR (CDCl₃) δ(ppm) : 7.05 (1H, s, 3-H), 4.76 (2H, brs, NCH₂Ar), 4.48 (2H, s, ArOCH₂CO), 3.80 (3H, s, NCH₃), 1.97 (3H, s, 5-CH₃).
MASS (EI) m/z : 428(M⁺), 393, 267, 239, 225, 175, 116.

### (Example 398)

### Compound of Formula No. B-154

¹H-NMR (CDCl₃) δ(ppm) : 7.05 (1H, s, 3-H), 4.77 (2H, brs, NCH₂Ar), 4.39 (2H, s, ArOCH₂CO), 3.83 (3H, s, NCH₃), 1.99 (3H, s, 5-CH₃).
MASS (EI) m/z : 428(M⁺), 312, 267, 239, 225, 175, 116.

### (Example 399)

### Compound of Formula No. B-155

¹H-NMR (CDCl₃) δ(ppm) : 7.13 (1H, s, 3-H), 4.76 (2H, brs, NCH₂Ar), 4.71 (2H, s, ArOCH₂CO), 3.83 (3H, s, NCH₃), 2.00 (3H, s, 5-CH₃).
MASS (EI) m/z : 450(M⁺), 408, 388, 253, 225, 116.

### (Example 400)

### Compound of Formula No. B-156

¹H-NMR (CDCl₃) δ(ppm) : 7.03 (1H, s, 5-H), 4.77 (2H, brs, NCH₂Ar), 4.49 (2H, s, ArOCH₂CO), 4.04 (2H, q, J=7Hz, NCH₂Me), 1.99 (3H, s, 3-CH₃).
MASS (EI) m/z : 392(M⁺), 281, 239, 125, 116.

### (Example 401)

### Compound of Formula No. B-157

¹H-NMR (CDCl₃) δ(ppm) : 7.03 (1H, s, 5-H), 4.78 (2H, brs, NCH₂Ar), 4.40 (2H, s, ArOCH₂CO), 4.06 (2H, q, J=7Hz, NCH₂Me), 2.00 (3H, s, 3-CH₃).
MASS (EI) m/z : 392(M⁺), 281, 267, 239, 125, 116.

### (Example 402)

### Compound of Formula No. B-158

¹H-NMR (CDCl₃) δ(ppm) : 7.02 (1H, s, 5-H), 4.77 (2H, brs, NCH₂Ar), 4.37 (2H, s, ArOCH₂CO), 4.05 (2H, q, J=7Hz, NCH₂Me), 1.99 (3H, s, 3-CH₃).
MASS (EI) m/z : 392(M⁺), 281, 267, 239, 125, 116.

### (Example 403)

### Compound of Formula No. B-159

¹H-NMR (CDCl₃) δ(ppm) : 7.06 (1H, s, 5-H), 4.78 (2H, brs, NCH₂Ar), 4.49 (2H, s, ArOCH₂CO), 4.02 (2H, q, J=7Hz, NCH₂Me), 2.01 (3H, s, 3-CH₃).
MASS (EI) m/z : 408(M⁺), 373, 281, 239, 141, 116.

### (Example 404)

### Compound of Formula No. B-160

¹H-NMR (CDCl₃) δ(ppm) : 7.03 (1H. S, 5-H), 4.78 (2H, brs, NCH₂Ar), 4.40 (2H, s, ArOCH₂CO), 4.06 (2H, q, J=7Hz, NCH₂Me), 2.01 (3H, s, 3-CH₃).
MASS (EI) m/z : 408(M⁺), 281, 267, 239, 141, 116.

### (Example 405)

### Compound of Formula No. B-161

¹H-NMR (CDCl₃) δ(ppm) : 7.02 (1H, s, 5-H), 4.77 (2H, brs, NCH₂Ar), 4.38 (2H, s, ArOCH₂CO), 4.05 (2H, q, J=7Hz, NCH₂Me), 1.99 (3H, s, 3-CH₃).
MASS (EI) m/z : 408(M⁺), 281, 267, 239, 141, 116.

### (Example 406)

### Compound of Formula No. B-162

¹H-NMR (CDCl₃) δ(ppm) : 7.01 (1H, s, 5-H), 4.78 (2H, brs, NCH₂Ar), 4.44 (2H, s, ArOCH₂CO), 4.04 (2H, q, J=7Hz, NCH₂Me), 2.00 (3H, s, 3-CH₃).
MASS (EI) m/z : 388(M⁺), 281, 267, 240, 116.

### (Example 407)

### Compound of Formula No. B-163

¹H-NMR (CDCl₃) δ(ppm) : 7.01 (1H, s, 5-H), 4.78 (2H, brs, NCH₂Ar), 4.40 (2H, s, ArOCH₂CO), 4.04 (2H, q, J=7Hz, NCH₂Me), 2.01 (3H, s, 3-CH₃).
MASS (EI) m/z : 388(M⁺), 326, 310, 281, 253, 149, 116.

### (Example 408)

### Compound of Formula No. B-164

¹H-NMR (CDCl₃) δ(ppm) : 7.01 (1H, s, 5-H), 4.77 (2H, brs, NCH₂Ar), 4.38 (2H, s, ArOCH₂CO), 4.04 (2H, q, J=7Hz, NCH₂Me), 2.00 (3H, s, 3-CH₃).
MASS (EI) m/z : 388(M⁺), 326, 310, 281, 253, 239, 116.

### (Example 409)

### Compound of Formula No. B-165

¹H-NMR (CDCl₃) δ(ppm) : 7.03 (1H, s, 5-H), 4.77 (2H, brs, NCH₂Ar), 4.47 (2H, s, ArOCH₂CO), 4.03 (2H, q, J=7Hz, NCH₂Me), 1.98 (3H, s, 3-CH₃).
MASS (EI) m/z : 404(M⁺), 281, 267, 239, 137, 116.

### (Example 410)

### Compound of Formula No. B-166

¹H-NMR (CDCl₃) δ(ppm) : 7.03 (1H, s, 5-H), 4.78 (2H, brs, NCH₂Ar), 4.40 (2H, s, ArOCH₂CO), 4.06 (2H, q, J=7Hz, NCH₂Me), 2.01 (3H, s, 3-CH₃).
MASS (EI) m/z : 404(M⁺), 281, 267, 238, 137, 116.

### (Example 411)

### Compound of Formula No. B-167

¹H-NMR (CDCl₃) δ(ppm) : 7.01 (1H, s, 5-H), 4.78 (2H, brs, NCH₂Ar), 4.37 (2H, s, ArOCH₂CO), 4.05 (2H, q, J=7Hz, NCH₂Me), 1.99 (3H, s, 3-CH₃).
MASS (EI) m/z : 404(M⁺), 281, 239, 137, 116.

### (Example 412)

### Compound of Formula No. B-168

¹H-NMR (CDCl₃) δ(ppm) : 7.09 (1H, s, 5-H), 4.77 (2H, brs, NCH₂Ar), 4.57 (2H, s, ArOCH₂CO), 4.04 (2H, q, J=7Hz, NCH₂Me), 2.01 (3H, s, 3-CH₃).
MASS (EI) m/z : 419(M⁺), 373, 281, 267, 238, 152, 116.

### (Example 413)

### Compound of Formula No. B-169

¹H-NMR (CDCl₃) δ(ppm) : 7.13 (1H, s, 5-H), 4.78 (2H, brs, NCH₂Ar), 4.52 (2H, s, ArOCH₂CO), 4.08 (2H, q, J=7Hz, NCH₂Me), 2.05 (3H, s, 3-CH₃).
MASS (EI) m/z : 419(M⁺), 303, 281, 267, 239, 152, 116.

### (Example 414)

### Compound of Formula No. B-170

¹H-NMR (CDCl₃) δ(ppm) : 7.11 (1H, s, 5-H), 4.78 (2H, brs, NCH₂Ar), 4.52 (2H, s, ArOCH₂CO), 4.08 (2H, q, J=7Hz, NCH₂Me), 2.02 (3H, S, 3-CH₃).
MASS (EI) m/z : 419(M⁺), 303, 281, 267, 239, 152, 116.

### (Example 415)

### Compound of Formula No. B-171

¹H-NMR (CDCl₃) δ(ppm) : 7.05 (1H, s, 5-H), 4.79 (2H, brs, NCH₂Ar), 4.51 (2H, s, ArOCH₂CO), 4.02 (2H, q, J=7Hz, NCH₂Me), 2.03 (3H, s, 3-CH₃).
MASS (EI) m/z : 442(M⁺), 423, 326, 281, 267, 239, 175, 116.

### (Example 416)

### Compound of Formula No. B-172

¹H-NMR (CDCl₃) δ(ppm) : 7.04 (1H, s, 5-H), 4.78 (2H, brs, NCH₂Ar), 4.46 (2H, s, ArOCH₂CO), 4.06 (2H, q, J=7Hz, NCH₂Me), 2.02 (3H, s, 3-CH₃).
MASS (EI) m/z : 442(M⁺), 423, 326, 281, 267, 239, 175, 116.

### (Example 417)

### Compound of Formula No. B-173

¹H-NMR (CDCl₃) δ(ppm) : 7.05 (1H, s, 5-H), 4.77 (2H, brs, NCH₂Ar), 4.46 (2H, s, ArOCH₂CO), 4.06 (2H, q, J=7Hz, NCH₂Me), 2.01 (3H, s, 3-CH₃).
MASS (EI) m/z : 442(M⁺), 423, 326, 281, 267, 239, 175, 116.

### (Example 418)

### Compound of Formula No. B-174

¹H-NMR (CDCl₃) δ(ppm) : 7.15 (1H, s, 5-H), 4.77 (2H, brs, NCH₂Ar), 4.58 (2H, s, ArOCH₂CO), 4.06 (2H, q, J=7Hz, NCH₂Me), 2.01 (3H, s, 3-CH₃).
MASS (EI) m/z : 399(M⁺), 283, 267, 239, 132, 116.

### (Example 419)

### Compound of Formula No. B-175

¹H-NMR (CDCl₃) δ(ppm) : 7.08 (1H, s, 5-H), 4.78 (2H, brs, NCH₂Ar), 4.44 (2H, s, ArOCH₂CO), 4.08 (2H, q, J=7Hz, NCH₂Me), 2.00 (3H, s, 3-CH₃).
MASS (EI) m/z : 399(M⁺), 283, 267, 239, 132, 116.

### (Example 420)

### Compound of Formula No. B-176

¹H-NMR (CDCl₃) δ(ppm) : 7.08 (1H, s, 5-H), 4.77 (2H, brs, NCH₂Ar), 4.48 (2H, s, ArOCH₂CO), 4.07 (2H, q, J=7Hz, NCH₂Me), 2.00 (3H, s, 3-CH₃).
MASS (EI) m/z : 399(M⁺), 283, 239, 132, 116.

### (Example 421)

### Compound of Formula No. B-177

¹H-NMR (CDCl₃) δ(ppm) : 7.02 (1H, s, 5-H), 4.76 (2H, brs, NCH₂Ar), 4.44 (2H, s, ArOCH₂CO), 4.04 (2H, q, J=7Hz, NCH₂Me), 1.97 (3H, s, 3-CH₃).
MASS (EI) m/z : 410(M⁺), 294, 281, 267, 239, 143, 116.

### (Example 422)

### Compound of Formula No. B-178

¹H-NMR (CDCl₃) δ(ppm) : 7.06 (1H, s, 5-H), 4.77 (2H, brs, NCH₂Ar), 4.48 (2H, s, ArOCH₂CO), 4.04 (2H, q, J=7Hz, NCH₂Me), 2.00 (3H, S, 3-CH₃).
MASS (EI) m/z : 442(M⁺), 407, 326, 281, 267, 239, 175, 116.

### (Example 423)

### Compound of Formula No. B-179

¹H-NMR (CDCl₃) δ(ppm) : 7.05 (1H, s, 5-H), 4.77 (2H, brs, NCH₂Ar), 4.39 (2H, s, ArOCH₂CO), 4.07 (2H, q, J=7Hz, NCH₂Me), 2.01 (3H, s, 3-CH₃).
MASS (EI) m/z : 442(M⁺), 326, 281, 267, 239, 175, 116.

### (Example 424)

### Compound of Formula No. B-180

¹H-NMR (CDCl₃) δ(ppm) : 7.13 (1H, s, 5-H), 4.77 (2H, brs, NCH₂Ar), 4.71 (2H, s, ArOCH₂CO), 4.08 (2H, q, J=7Hz, NCH₂Me), 2.02 (3H, s, 3-CH₃).
MASS (EI) m/z : 464(M⁺), 418, 348, 281, 267, 238, 167, 116.

### (Example 425)

### Compound of Formula No. B-181

¹H-NMR (CDCl₃) δ(ppm) : 7.00 (1H, s, 5-H), 4.77 (2H, brs, NCH₂Ar), 4.49 (2H, s, ArOCH₂CO), 3.93 (2H, t, J=7Hz, NCH₂Et), 2.01 (3H, s, 3-CH₃).
MASS (EI) m/z : 406(M⁺), 295, 253, 125, 116.

### (Example 426)

### Compound of Formula No. B-182

¹H-NMR (CDCl₃) δ(ppm) : 7.01 (1H, s, 5-H), 4.77 (2H, brs, NCH₂Ar), 4.40 (2H, s, ArOCH₂CO), 3.95 (2H, t, J=7Hz, NCH₂Et), 2.02 (3H, s, 3-CH₃).
MASS (EI) m/z : 406(M⁺), 295, 253, 125, 116.

### (Example 427)

### Compound of Formula No. B-183

¹H-NMR (CDCl₃) δ(ppm) : 7.01 (1H, s, 5-H), 4.78 (2H, brs, NCH₂Ar), 4.38 (2H, s, ArOCH₂CO), 3.95 (2H, t, J=7Hz, NCH₂Et), 2.01 (3H, s, 3-CH₃).
MASS (EI) m/z : 406(M⁺), 295, 253, 125, 116.

### (Example 428)

### Compound of Formula No. B-184

¹H-NMR (CDCl₃) δ(ppm) : 7.02 (1H, s, 5-H), 4.78 (2H, brs, NCH₂Ar), 4.49 (2H, s, ArOCH₂CO), 3.92 (2H, t, J=7Hz, NCH₂Et), 2.02 (3H, s, 3-CH₃).
MASS (EI) m/z : 422(M⁺), 387, 295, 253, 141, 116.

### (Example 429)

### Compound of Formula No. B-185

¹H-NMR (CDCl₃) δ(ppm) : 7.02 (1H, s, 5-H), 4.78 (2H, brs, NCH₂Ar), 4.40 (2H, s, ArOCH₂CO), 3.96 (2H, t, J=7Hz, NCH₂Et), 2.03 (3H, s, 3-CH₃).
MASS (EI) m/z : 422(M⁺), 387, 295, 267, 253, 141, 116.

### (Example 430)

### Compound of Formula No. B-186

¹H-NMR (CDCl₃) δ(ppm) : 7.01 (1H, s, 5-H), 4.78 (2H, brs, NCH₂Ar), 4.39 (2H, s, ArOCH₂CO), 3.95 (2H, t, J=7Hz, NCH₂Et), 2.01 (3H, s, 3-CH₃).
MASS (EI) m/z : 422(M⁺), 306, 295, 253, 141, 116.

### (Example 431)

### Compound of Formula No. B-187

¹H-NMR (CDCl₃) δ(ppm) : 6.99 (1H, s, 5-H), 4.78 (2H, brs, NCH₂Ar), 4.44 (2H, s, ArOCH₂CO), 3.93 (2H, t, J=7Hz, NCH₂Et), 2.01 (3H, s, 3-CH₃).
MASS (EI) m/z : 402(M⁺), 295, 254, 116.

### (Example 432)

### Compound of Formula No. B-188

¹H-NMR (CDCl₃) δ(ppm) : 7.01 (1H, s, 5-H), 4.78 (2H, brs, NCH₂Ar), 4.39 (2H, s, ArOCH₂CO), 3.95 (2H, t, J=7Hz, NCH₂Et), 2.02 (3H, s, 3-CH₃).
MASS (EI) m/z : 402(M⁺), 295, 267, 253, 121, 116.

### (Example 433)

### Compound of Formula No. B-189

¹H-NMR (CDCl₃) δ(ppm) : 6.98 (1H, s, 5-H), 4.77 (2H, brs, NCH₂Ar), 4.37 (2H, s, ArOCH₂CO), 3.94 (2H, t, J=7Hz, NCH₂Et), 2.01 (3H, s, 3-CH₃).
MASS (EI) m/z : 402(M⁺), 295, 267, 253, 116.

### (Example 434)

### Compound of Formula No. B-190

¹H-NMR (CDCl₃) δ(ppm) : 6.99 (1H, s, 5-H), 4.77 (2H, brs, NCH₂Ar), 4.47 (2H, s, ArOCH₂CO), 3.93 (2H, t, J=7Hz, NCH₂Et), 2.00 (3H, s, 3-CH₃).
MASS (EI) m/z : 418(M⁺), 295, 267, 253, 137, 116.

### (Example 435)

### Compound of Formula No. B-191

¹H-NMR (CDCl₃) δ(ppm) : 7.01 (1H, s, 5-H), 4.78 (2H, brs, NCH₂Ar), 4.39 (2H, s, ArOCH₂CO), 3.95 (2H, t, J=7Hz, NCH₂Et), 2.02 (3H, s, 3-CH₃).
MASS (EI) m/z : 418(M⁺), 295, 267, 252, 137, 116.

### (Example 436)

### Compound of Formula No. B-192

¹H-NMR (CDCl₃) δ(ppm) : 6.98 (1H, s, 5-H), 4.78 (2H, brs, NCH₂Ar), 4.36 (2H, s, ArOCH₂CO), 3.94 (2H, t, J=7Hz, NCH₂Et), 2.02 (3H, s, 3-CH₃).
MASS (EI) m/z : 418(M⁺), 295, 267, 253, 137, 116.

### (Example 437)

### Compound of Formula No. B-193

¹H-NMR (CDCl₃) δ(ppm) : 7.05 (1H, s, 5-H), 4.76 (2H, brs, NCH₂Ar), 4.56 (2H, s, ArOCH₂CO), 3.93 (2H, t, J=7Hz, NCH₂Et), 2.03 (3H, s, 3-CH₃).
MASS (EI) m/z : 433(M⁺), 387, 252, 152, 122, 116.

### (Example 438)

### Compound of Formula No. B-194

¹H-NMR (CDCl₃) δ(ppm) : 7.09 (1H, s, 5-H), 4.79 (2H, brs, NCH₂Ar), 4.52 (2H, s, ArOCH₂CO), 3.98 (2H, t, J=7Hz, NCH₂Et), 2.06 (3H, s, 3-CH₃).
MASS (EI) m/z : 433(M⁺), 317, 295, 253, 152, 116.

### (Example 439)

### Compound of Formula No. B-195

¹H-NMR (CDCl₃) δ(ppm) : 7.07 (1H, s, 5-H), 4.78 (2H, brs, NCH₂Ar), 4.52 (2H, s, ArOCH₂CO), 3.98 (2H, t, J=7Hz, NCH₂Et), 2.04 (3H, s, 3-CH₃).
MASS (EI) m/z : 433(M⁺), 368, 317, 253, 152, 116.

### (Example 440)

### Compound of Formula No. B-196

¹H-NMR (CDCl₃) δ(ppm) : 7.04 (1H, s, 5-H), 4.80 (2H, brs, NCH₂Ar), 4.51 (2H, s, ArOCH₂CO), 3.92 (2H, t, J=7Hz, NCH₂Et), 2.04 (3H, s, 3-CH₃).
MASS (EI) m/z : 456(M⁺), 437, 340, 295, 253, 175, 116.

### (Example 441)

### Compound of Formula No. B-197

¹H-NMR (CDCl₃) δ(ppm) : 7.04 (1H, s, 5-H), 4.79 (2H, brs, NCH₂Ar), 4.46 (2H, s, ArOCH₂CO), 3.96 (2H, t, J=7Hz, NCH₂Et), 2.04 (3H, s, 3-CH₃).
MASS (EI) m/z : 456(M⁺), 437, 340, 295, 253, 175, 116.

### (Example 442)

### Compound of Formula No. B-198

¹H-NMR (CDCl₃) δ(ppm) : 7.01 (1H, s, 5-H), 4.78 (2H, brs, NCH₂Ar), 4.45 (2H, s, ArOCH₂CO), 3.95 (2H, t, J=7Hz, NCH₂Et), 2.03 (3H, s, 3-CH₃).
MASS (EI) m/z : 456(M⁺), 437, 340, 295, 253, 175, 116.

### (Example 443)

### Compound of Formula No. B-199

¹H-NMR (CDCl₃) δ(ppm) : 7.11 (1H, s, 5-H), 4.78 (2H, brs, NCH₂Ar), 4.57 (2H, s, ArOCH₂CO), 3.95 (2H, t, J=7Hz, NCH₂Et), 2.03 (3H, s, 3-CH₃).
MASS (EI) m/z : 413(M⁺), 368, 297, 253, 132, 116.

### (Example 444)

### Compound of Formula No. B-200

¹H-NMR (CDCl₃) δ(ppm) : 7.04 (1H, s, 5-H), 4.79 (2H, brs, NCH₂Ar), 4.44 (2H, s, ArOCH₂CO), 3.98 (2H, t, J=7Hz, NCH₂Et), 2.02 (3H, s, 3-CH₃).
MASS (EI) m/z : 413(M⁺), 368, 297, 253, 132, 116.

### (Example 445)

### Compound of Formula No. B-201

¹H-NMR (CDCl₃) δ(ppm) : 7.02 (1H, s, 5-H), 4.77 (2H, brs, NCH₂Ar), 4.47 (2H, S, ArOCH₂CO), 3.96 (2H, t, J=7Hz, NCH₂Et), 2.02 (3H, s, 3-CH₃).
MASS (EI) m/z : 413(M⁺), 388, 297, 253, 132, 116.

### (Example 446)

### Compound of Formula No. B-202

¹H-NMR (CDCl₃) δ(ppm) : 6.99 (1H, s, 5-H), 4.77 (2H, brs, NCH₂Ar), 4.44 (2H, s, ArOCH₂CO), 3.94 (2H, t, J=7Hz, NCH₂Et), 1.99 (3H, s, 3-CH₃).
MASS (EI) m/z : 424(M⁺), 308, 295, 253, 143, 116.

### (Example 447)

### Compound of Formula No. B-203

¹H-NMR (CDCl₃) δ(ppm) : 7.02 (1H, s, 5-H), 4.77 (2H, brs, NCH₂Ar), 4.47 (2H, s, ArOCH₂CO), 3.93 (2H, t, J=7Hz, NCH₂Et), 2.01 (3H, s, 3-CH₃).
MASS (EI) m/z : 456(M⁺), 421, 340, 295, 253, 175, 116.

### (Example 448)

### Compound of Formula No. B-204

¹H-NMR (CDCl₃) δ(ppm) : 7.01 (1H, s, 5-H), 4.77 (2H, brs, NCH₂Ar), 4.38 (2H, s, ArOCH₂CO), 3.97 (2H, t, J=7Hz, NCH₂Et), 2.03 (3H, s, 3-CH₃).
MASS (EI) m/z : 456(M⁺), 422, 340, 295, 253, 175, 116.

### (Example 449)

### Compound of Formula No. B-205

¹H-NMR (CDCl₃) δ(ppm) : 7.01 (1H, s, 5-H), 4.76 (2H, brs, NCH₂Ar), 4.48 (2H, s, ArOCH₂CO), 4.33 (1H, hept, J=7Hz, NCHMe₂), 2.00 (3H, s, 3-CH₃).
MASS (EI) m/z : 406(M⁺), 295, 253, 211, 125, 116.

### (Example 450)

### Compound of Formula No. B-206

¹H-NMR (CDCl₃) δ(ppm) : 7.01 (1H, s, 5-H), 4.78 (2H, brs, NCH₂Ar), 4.39 (2H, s, ArOCH₂CO), 4.35 (1H, hept, J=7Hz, NCHMe₂), 2.01 (3H, s, 3-CH₃).
MASS (EI) m/z : 406(M⁺), 295, 253, 211, 125, 116.

### (Example 451)

### Compound of Formula No. B-207

¹H-NMR (CDCl₃) δ(ppm) : 7.01 (1H, s, 5-H), 4.77 (2H, brs, NCH₂Ar), 4.37 (2H, s, ArOCH₂CO), 4.35 (1H, hept, J=7Hz, NCHMe₂), 2.00 (3H, s, 3-CH₃).
MASS (EI) m/z : 406(M⁺), 295, 253, 211, 125, 116.

### (Example 452)

### Compound of Formula No. B-208

¹H-NMR (CDCl₃) δ(ppm) : 7.05 (1H, s, 5-H), 4.78 (2H, brs, NCH₂Ar), 4.48 (2H, s, ArOCH₂CO), 4.31 (1H, hept, J=7Hz, NCHMe₂), 2.02 (3H, s, 3-CH₃).
MASS (EI) m/z : 422(M⁺), 398, 387, 295, 253, 141, 116.

### (Example 453)

### Compound of Formula No. B-209

¹H-NMR (CDCl₃) δ(ppm) : 7.02 (1H, s, 5-H), 4.77 (2H, brs, NCH₂Ar), 4.39 (2H, s, ArOCH₂CO), 4.35 (1H, hept, J=7Hz, NCHMe₂), 2.02 (3H, s, 3-CH₃).
MASS (EI) m/z : 422(M⁺), 295, 267, 253, 141, 116.

### (Example 454)

### Compound of Formula No. B-210

¹H-NMR (CDCl₃) δ(ppm) : 7.01 (1H, s, 5-H), 4.76 (2H, brs, NCH₂Ar), 4.38 (2H, s, ArOCH₂CO), 4.31 (1H, hept, J=7Hz, NCHMe₂), 2.00 (3H, s, 3-CH₃).
MASS (EI) m/z : 422(M⁺), 306, 295, 253, 141, 116.

### (Example 455)

### Compound of Formula No. B-211

¹H-NMR (CDCl₃) δ(ppm) : 6.98 (1H, s, 5-H), 4.78 (2H, brs, NCH₂Ar), 4.43 (2H, s, ArOCH₂CO), 4.33 (1H, hept, J=7Hz, NCHMe₂), 2.00 (3H, s, 3-CH₃).
MASS (EI) m/z : 402(M⁺), 295, 253, 116.

### (Example 456)

### Compound of Formula No. B-212

¹H-NMR (CDCl₃) δ(ppm) : 7.00 (1H, s, 5-H), 4.77 (2H, brs, NCH₂Ar), 4.38 (2H, s, ArOCH₂CO), 4.34 (1H, hept, J=7Hz, NCHMe₂), 2.01 (3H, s, 3-CH₃).
MASS (EI) m/z : 402(M⁺), 295, 267, 253, 116.

### (Example 457)

### Compound of Formula No. B-213

¹H-NMR (CDCl₃) δ(ppm) : 6.99 (1H, s, 5-H), 4.77 (2H, brs, NCH₂Ar), 4.37 (2H, s, ArOCH₂CO), 4.34 (1H, hept, J=7Hz, NCHMe₂), 2.00 (3H, s, 3-CH₃).
MASS (EI) m/z : 402(M⁺), 295, 267, 253, 116.

### (Example 458)

### Compound of Formula No. B-214

¹H-NMR (CDCl₃) δ(ppm) : 7.01 (1H, s, 5-H), 4.76 (2H, brs, NCH₂Ar), 4.45 (2H, s, ArOCH₂CO), 4.32 (1H, hept, J=7Hz, NCHMe₂), 1.99 (3H, s, 3-CH₃).
MASS (EI) m/z : 418(M⁺), 295, 267, 253, 137, 116.

### (Example 459)

### Compound of Formula No. B-215

¹H-NMR (CDCl₃) δ(ppm) : 7.00 (1H, s, 5-H), 4.77 (2H, brs, NCH₂Ar), 4.38 (2H, s, ArOCH₂CO), 4.34 (1H, hept, J=7Hz, NCHMe₂), 2.01 (3H, s, 3-CH₃).
MASS (EI) m/z : 418(M⁺), 295, 267, 252, 137, 116.

### (Example 460)

### Compound of Formula No. B-216

¹H-NMR (CDCl₃) δ(ppm) : 6.98 (1H, s, 5-H), 4.77 (2H, brs, NCH₂Ar), 4.36 (1H, hept, J=7Hz, NCHMe₂), 4.35 (2H, s, ArOCH₂CO), 2.00 (3H, s, 3-CH₃).
MASS (EI) m/z : 418(M⁺), 295, 267, 253, 137, 116.

### (Example 461)

### Compound of Formula No. B-217

¹H-NMR (CDCl₃) δ(ppm) : 7.09 (1H, s, 5-H), 4.77 (2H, brs, NCH₂Ar), 4.56 (2H, s, ArOCH₂CO), 4.33 (1H, hept, J=7Hz, NCHMe₂), 2.02 (3H, s, 3-CH₃).
MASS (EI) m/z : 433(M⁺), 387, 252, 152, 122, 116.

### (Example 462)

### Compound of Formula No. B-218

¹H-NMR (CDCl₃) δ(ppm) : 7.12 (1H, s, 5-H), 4.79 (2H, brs, NCH₂Ar), 4.51 (2H, s, ArOCH₂CO), 4.38 (1H, hept, J=7Hz, NCHMe₂), 2.05 (3H, s, 3-CH₃).
MASS (EI) m/z : 433(M⁺), 387, 252, 152, 116.

### (Example 463)

### Compound of Formula No. B-219

¹H-NMR (CDCl₃) δ(ppm) : 7.06 (1H, s, 5-H), 4.77 (2H, brs, NCH₂Ar), 4.51 (2H, s, ArOCH₂CO), 4.37 (1H, hept, J=7Hz, NCHMe₂), 2.02 (3H, s, 3-CH₃).
MASS (EI) m/z : 433(M⁺), 317, 253, 152, 116.

### (Example 464)

### Compound of Formula No. B-220

¹H-NMR (CDCl₃) δ(ppm) : 7.04 (1H, s, 5-H), 4.79 (2H, brs, NCH₂Ar), 4.50 (2H, s, ArOCH₂CO), 4.32 (1H, hept, J=7Hz, NCHMe₂), 2.04 (3H, s, 3-CH₃).
MASS (EI) m/z : 456(M⁺), 340, 295, 253, 175, 116.

### (Example 465)

### Compound of Formula No. B-221

¹H-NMR (CDCl₃) δ(ppm) : 7.03 (1H, s, 5-H), 4.78 (2H, brs, NCH₂Ar), 4.45 (2H, s, ArOCH₂CO), 4.36 (1H, hept, J=7Hz, NCHMe₂), 2.02 (3H, s, 3-CH₃).
MASS (EI) m/z : 456(M⁺), 340, 295, 253, 175, 116.

### (Example 466)

### Compound of Formula No. B-222

¹H-NMR (CDCl₃) δ(ppm) : 7.03 (1H, s, 5-H), 4.78 (2H, brs, NCH₂Ar), 4.45 (2H, s, ArOCH₂CO), 4.35 (1H, hept, J=7Hz, NCHMe₂), 2.02 (3H, s, 3-CH₃).
MASS (EI) m/z : 456(M⁺), 437, 340, 295, 253, 175, 116.

### (Example 467)

### Compound of Formula No. B-223

¹H-NMR (CDCl₃) δ(ppm) : 7.13 (1H, s, 5-H), 4.77 (2H, brs, NCH₂Ar), 4.56 (2H, s, ArOCH₂CO), 4.35 (1H, hept, J=7Hz, NCHMe₂), 2.02 (3H, s, 3-CH₃).
MASS (EI) m/z : 413(M⁺), 398, 297, 253, 132, 116.

### (Example 468)

### Compound of Formula No. B-224

¹H-NMR (CDCl₃) δ(ppm) : 7.07 (1H, s, 5-H), 4.78 (2H, brs, NCH₂Ar), 4.44 (2H, s, ArOCH₂CO), 4.38 (1H, hept, J=7Hz, NCHMe₂), 2.02 (3H, s, 3-CH₃).
MASS (EI) m/z : 413(M⁺), 398, 297, 253, 132, 116.

### (Example 469)

### Compound of Formula No. B-225

¹H-NMR (CDCl₃) δ(ppm) : 7.04 (1H, s, 5-H), 4.76 (2H, brs, NCH₂Ar), 4.46 (2H, s, ArOCH₂CO), 4.36 (1H, hept, J=7Hz, NCHMe₂), 2.01 (3H, s, 3-CH₃).
MASS (EI) m/z : 413(M⁺), 398, 297, 253, 132, 116.

### (Example 470)

### Compound of Formula No. B-226

¹H-NMR (CDCl₃) δ(ppm) : 7.00 (1H, s, 5-H), 4.76 (2H, brs, NCH₂Ar), 4.44 (2H, s, ArOCH₂CO), 4.34 (1H, hept, J=7Hz, NCHMe₂), 1.98 (3H, s, 3-CH₃).
MASS (EI) m/z : 424(M⁺), 295, 253, 143, 116.
(Example 471)

### Compound of Formula No. B-227

¹H-NMR (CDCl₃) δ(ppm) : 7.05 (1H, s, 5-H), 4.77 (2H, brs, NCH₂Ar), 4.46 (2H, s, ArOCH₂CO), 4.33 (1H, hept, J=7Hz, NCHMe₂), 2.01 (3H, s, 3-CH₃).
MASS (EI) m/z : 456(M⁺), 421, 295, 253, 175, 116.

### (Example 472)

### Compound of Formula No. B-228

¹H-NMR (CDCl₃) δ(ppm) : 7.02 (1H, s, 5-H), 4.76 (2H, brs, NCH₂Ar), 4.38 (2H, s, ArOCH₂CO), 4.33 (1H, hept, J=7Hz, NCHMe₂), 2.02 (3H, s, 3-CH₃).
MASS (EI) m/z : 456(M⁺), 340, 295, 253, 175, 116.

### (Example 473)

### Compound of Formula No. B-229

¹H-NMR (CDCl₃) δ(ppm) : 7.10 (1H, s, 5-H), 4.75 (2H, brs, NCH₂Ar), 4.70 (2H, s, ArOCH₂CO), 4.37 (1H, hept, J=7Hz, NCHMe₂), 2.03 (3H, s, 3-CH₃).
MASS (EI) m/z : 478(M⁺), 362, 252, 197, 167, 116.

### (Example 474)

### Compound of Formula No. B-230

¹H-NMR (CDCl₃) δ(ppm) : 6.99 (1H, s, 5-H), 4.77 (2H, brs, NCH₂Ar), 4.48 (2H, s, ArOCH₂CO), 3.97 (2H, t, J=7Hz, NCH₂-Pr), 2.01 (3H, s, 3-CH₃).
MASS (EI) m/z : 420(M⁺), 309, 267, 125, 116.

### (Example 475)

### Compound of Formula No. B-231

¹H-NMR (CDCl₃) δ(ppm) : 6.99 (1H, S, 5-H), 4.78 (2H, brs, NCH₂Ar), 4.39 (2H, s, ArOCH₂CO), 3.99 (2H, t, J=7Hz, NCH₂-Pr), 2.02 (3H, S, 3-CH₃).
MASS (EI) m/z : 420(M⁺), 309, 267, 125, 116.

### (Example 476)

### Compound of Formula No. B-232

¹H-NMR (CDCl₃) δ(ppm) : 6.98 (1H, s, 5-H), 4.77 (2H, brs, NCH₂Ar), 4.37 (2H, s, ArOCH₂CO), 3.98 (2H, t, J=7Hz, NCH₂-Pr), 2.01 (3H, s, 3-CH₃).
MASS (EI) m/z : 420(M⁺), 309, 267, 125, 116.

### (Example 477)

### Compound of Formula No. B-233

¹H-NMR (CDCl₃) δ(ppm) : 7.01 (1H, s, 5-H), 4.77 (2H, brs, NCH₂Ar), 4.48 (2H, s, ArOCH₂CO), 3.95 (2H, t, J=7Hz, NCH₂-Pr), 2.02 (3H, s, 3-CH₃).
MASS (EI) m/z : 436(M⁺), 401, 320, 309, 267, 141, 116.

### (Example 478)

### Compound of Formula No. B-234

¹H-NMR (CDCl₃) δ(ppm) : 6.98 (1H, s, 5-H), 4.77 (2H, brs, NCH₂Ar), 4.39 (2H, s, ArOCH₂CO), 3.99 (2H, t, J=7Hz, NCH₂-Pr), 2.02 (3H, s, 3-CH₃).
MASS (EI) m/z : 436(M⁺), 320, 309, 295, 267, 141, 116.

### (Example 479)

### Compound of Formula No. B-235

¹H-NMR (CDCl₃) δ(ppm) : 6.98 (1H, s, 5-H), 4.77 (2H, brs, NCH₂Ar), 4.37 (2H, s, ArOCH₂CO), 3.98 (2H, t, J=7Hz, NCH₂-Pr), 2.01 (3H, s, 3-CH₃).
MASS (EI) m/z : 436(M⁺), 320, 309, 295, 267, 141, 116.

### (Example 480)

### Compound of Formula No. B-236

¹H-NMR (CDCl₃) δ(ppm) : 6.95 (1H, s, 5-H), 4.79 (2H, brs, NCH₂Ar), 4.43 (2H, s, ArOCH₂CO), 3.97 (2H, t, J=7Hz, NCH₂-Pr), 2.01 (3H, s, 3-CH₃).
MASS (EI) m/z : 416(M⁺), 309, 295, 268, 121, 116.

### (Example 481)

### Compound of Formula No. B-237

¹H-NMR (CDCl₃) δ(ppm) : 6.97 (1H, s, 5-H), 4.79 (2H, brs, NCH₂Ar), 4.38 (2H, s, ArOCH₂CO), 3.98 (2H, t, J=7Hz, NCH₂-Pr), 2.02 (3H, s, 3-CH₃).
MASS (EI) m/z : 416(M⁺), 309, 300, 267, 121, 116.

### (Example 482)

### Compound of Formula No. B-238

¹H-NMR (CDCl₃) δ(ppm) : 6.96 (1H, s, 5-H), 4.77 (2H, brs, NCH₂Ar), 4.37 (2H, s, ArOCH₂CO), 3.97 (2H, t, J=7Hz, NCH₂-Pr), 2.01 (3H, s, 3-CH₃).
MASS (EI) m/z : 416(M⁺), 309, 295, 267, 121, 116.

### (Example 483)

### Compound of Formula No. B-239

¹H-NMR (CDCl₃) δ(ppm) : 6.97 (1H, s, 5-H), 4.77 (2H, brs, NCH₂Ar), 4.46 (2H, s, ArOCH₂CO), 3.96 (2H, t, J=7Hz, NCH₂-Pr), 2.00 (3H, s, 3-CH₃).
MASS (EI) m/z : 432(M⁺), 309, 267, 137, 116.

### (Example 484)

### Compound of Formula No. B-240

¹H-NMR (CDCl₃) δ(ppm) : 6.97 (1H, s, 5-H), 4.78 (2H, brs, NCH₂Ar), 4.38 (2H, s, ArOCH₂CO), 3.98 (2H, t, J=7Hz, NCH₂-Pr), 2.02 (3H, s, 3-CH₃).
MASS (EI) m/z : 432(M⁺), 309, 295, 266, 137, 116.

### (Example 485)

### Compound of Formula No. B-241

¹H-NMR (CDCl₃) δ(ppm) : 6.96 (1H, s, 5-H), 4.77 (2H, brs, NCH₂Ar), 4.35 (2H, s, ArOCH₂CO), 3.97 (2H, t, J=7Hz, NCH₂-Pr), 2.00 (3H, s, 3-CH₃).
MASS (EI) m/z : 432(M⁺), 309, 267, 137, 116.

### (Example 486)

### Compound of Formula No. B-242

¹H-NMR (CDCl₃) δ(ppm) : 7.04 (1H, s, 5-H), 4.76 (2H, brs, NCH₂Ar), 4.56 (2H, s, ArOCH₂CO), 3.96 (2H, t, J=7Hz, NCH₂-Pr), 2.02 (3H, s, 3-CH₃).
MASS (EI) m/z : 447(M⁺), 401, 309, 267, 211, 152, 116.

### (Example 487)

### Compound of Formula No. B-243

¹H-NMR (CDCl₃) δ(ppm) : 7.07 (1H, s, 5-H), 4.78 (2H, brs, NCH₂Ar), 4.51 (2H, s, ArOCH₂CO), 4.01 (2H, t, J=7Hz, NCH₂-Pr), 2.06 (3H, s, 3-CH₃).
MASS (EI) m/z : 447(M⁺), 331, 295, 267, 152, 116.

### (Example 488)

### Compound of Formula No. B-244

¹H-NMR (CDCl₃) δ(ppm) : 7.02 (1H, s, 5-H), 4.77 (2H, brs, NCH₂Ar), 4.51 (2H, s, ArOCH₂CO), 4.00 (2H, t, J=7Hz, NCH₂-Pr), 2.03 (3H, s, 3-CH₃).
MASS (EI) m/z : 447(M⁺), 404, 331, 309, 267, 152, 116.

### (Example 489)

### Compound of Formula No. B-245

¹H-NMR (CDCl₃) δ(ppm) : 7.01 (1H, s, 5-H), 4.79 (2H, brs, NCH₂Ar), 4.50 (2H, s, ArOCH₂CO), 3.95 (2H, t, J=7Hz, NCH₂-Pr), 2.04 (3H, s, 3-CH₃).
MASS (EI) m/z : 470(M⁺), 451, 354, 309, 267, 175, 116.

### (Example 490)

### Compound of Formula No. B-246

¹H-NMR (CDCl₃) δ(ppm) : 6.99 (1H, s, 5-H), 4.78 (2H, brs, NCH₂Ar), 4.45 (2H, s, ArOCH₂CO), 3.99 (2H, t, J=7Hz, NCH₂-Pr), 2.03 (3H, s, 3-CH₃).
MASS (EI) m/z : 470(M⁺), 451, 354, 309, 267, 175, 116.

### (Example 491)

### Compound of Formula No. B-247

¹H-NMR (CDCl₃) δ(ppm) : 7.00 (1H, s, 5-H), 4.79 (2H, brs, NCH₂Ar), 4.45 (2H, s, ArOCH₂CO), 3.99 (2H, t, J=7Hz, NCH₂-Pr), 2.03 (3H, s, 3-CH₃).
MASS (EI) m/z : 470(M⁺), 451, 354, 309, 267, 175, 116.

### (Example 492)

### Compound of Formula No. B-248

¹H-NMR (CDCl₃) δ(ppm) : 7.09 (1H, s, 5-H), 4.78 (2H, brs, NCH₂Ar), 4.56 (2H, s, ArOCH₂CO), 3.98 (2H, t, J=7Hz, NCH₂-Pr), 2.03 (3H, s, 3-CH₃).
MASS (EI) m/z : 427(M⁺), 384, 311, 267, 132, 116.

### (Example 493)

### Compound of Formula No. B-249

¹H-NMR (CDCl₃) δ(ppm) : 7.02 (1H, s, 5-H), 4.78 (2H, brs, NCH₂Ar), 4.43 (2H, s, ArOCH₂CO), 4.01 (2H, t, J=7Hz, NCH₂-Pr), 2.02 (3H, s, 3-CH₃).
MASS (EI) m/z : 427(M⁺), 311, 267, 132, 116.

### (Example 494)

### Compound of Formula No. B-250

¹H-NMR (CDCl₃) δ(ppm) : 7.01 (1H, s, 5-H), 4.77 (2H, brs, NCH₂Ar), 4.46 (2H, s, ArOCH₂CO), 4.00 (2H, t, J=7Hz, NCH₂-Pr), 2.02 (3H, s, 3-CH₃).
MASS (EI) m/z : 427(M⁺), 311, 267, 132, 116.

### (Example 495)

### Compound of Formula No. B-251

¹H-NMR (CDCl₃) δ(ppm) : 6.97 (1H, s, 5-H), 4.77 (2H, brs, NCH₂Ar), 4.43 (2H, s, ArOCH₂CO), 3.97 (2H, t, J=7Hz, NCH₂-Pr), 1.99 (3H, s, 3-CH₃).
MASS (EI) m/z : 438(M⁺), 322, 309, 267, 143, 116.

### (Example 496)

### Compound of Formula No. B-252

¹H-NMR (CDCl₃) δ(ppm) : 7.01 (1H, s, 5-H), 4.77 (2H, brs, NCH₂Ar), 4.46 (2H, s, ArOCH₂CO), 3.96 (2H, t, J=7Hz, NCH₂-Pr), 2.01 (3H, s, 3-CH₃).
MASS (EI) m/z : 470(M⁺), 435, 354, 309, 267, 175, 116.

### (Example 497)

### Compound of Formula No. B-253

¹H-NMR (CDCl₃) δ(ppm) : 6.99 (1H, s, 5-H), 4.77 (2H, brs, NCH₂Ar), 4.38 (2H, s, ArOCH₂CO), 4.00 (2H, t, J=7Hz, NCH₂-Pr), 2.03 (3H, s, 3-CH₃).
MASS (EI) m/z : 470(M⁺), 427, 354, 309, 267, 175, 116.

### (Example 498)

### Compound of Formula No. B-254

¹H-NMR (CDCl₃) δ(ppm) : 7.07 (1H, s, 5-H), 4.77 (2H, brs, NCH₂Ar), 4.70 (2H, s, ArOCH₂CO), 4.00 (2H, t, J=7Hz, NCH₂-Pr), 2.04 (3H, s, 3-CH₃).
MASS (EI) m/z : 492(M⁺), 416, 309, 266, 223, 167, 116.

### (Example 499)

### Compound of Formula No. B-255

¹H-NMR (CDCl₃) δ(ppm) : 6.97 (1H, s, 5-H), 4.78 (2H, brs, NCH₂Ar), 4.49 (2H, s, ArOCH₂CO), 3.75 (2H, d, J=7Hz, NCH₂-iPr), 2.02 (3H, s, 3-CH₃).
MASS (EI) m/z : 420(M⁺), 309, 267, 125, 116.

### (Example 500)

### Compound of Formula No. B-256

¹H-NMR (CDCl₃) δ(ppm) : 6.97 (1H, s, 5-H), 4.78 (2H, brs, NCH₂Ar), 4.40 (2H, s, ArOCH₂CO), 3.77 (2H, d, J=7Hz, NCH₂-iPr), 2.03 (3H, s, 3-CH₃).
MASS (EI) m/z : 420(M⁺), 309, 267, 125, 116.

### (Example 501)

### Compound of Formula No. B-257

¹H-NMR (CDCl₃) δ(ppm) : 6.99 (1H, s, 5-H), 4.78 (2H, brs, NCH₂Ar), 4.48 (2H, s, ArOCH₂CO), 3.74 (2H, d, J=7Hz, NCH₂-ipr), 2.03 (3H, S, 3-CH₃).
MASS (EI) m/z : 436(M⁺), 401, 320, 267, 141, 116.

### (Example 502)

### Compound of Formula No. B-258

¹H-NMR (CDCl₃) δ(ppm) : 6.96 (1H, s, 5-H), 4.78 (2H, brs, NCH₂Ar), 4.39 (2H, s, ArOCH₂CO), 3.77 (2H, d, J=7Hz, NCH₂-iPr), 2.04 (3H, s, 3-CH₃).
MASS (EI) m/z : 436(M⁺), 320, 295, 267, 141, 116.

### (Example 503)

### Compound of Formula No. B-259

¹H-NMR (CDCl₃) δ(ppm) : 6.95 (1H, s, 5-H), 4.77 (2H, brs, NCH₂Ar), 4.38 (2H, s, ArOCH₂CO), 3.76 (2H, d, J=7Hz, NCH₂-iPr), 2.02 (3H, s, 3-CH₃).
MASS (EI) m/z : 436(M⁺), 320, 309, 295, 267, 141, 116.

### (Example 504)

### Compound of Formula No. B-260

¹H-NMR (CDCl₃) δ(ppm) : 6.93 (1H, s, 5-H), 4.79 (2H, brs, NCH₂Ar), 4.43 (2H, s, ArOCH₂CO), 3.75 (2H, d, J=7Hz, NCH₂-iPr), 2.02 (3H, s, 3-CH₃).
MASS (EI) m/z : 416 (M⁺), 309, 267, 253, 116.

### (Example 505)

### Compound of Formula No. B-261

¹H-NMR (CDCl₃) δ(ppm) : 6.95 (1H, s, 5-H), 4.77 (2H, brs, NCH₂Ar), 4.39 (2H, s, ArOCH₂CO), 3.76 (2H, d, J=7Hz, NCH₂-iPr), 2.03 (3H, s, 3-CH₃).
MASS (EI) m/z : 416(M⁺), 309, 300, 267, 253, 121.

### (Example 506)

### Compound of Formula No. B-262

¹H-NMR (CDCl₃) δ(ppm) : 6.94 (1H, s, 5-H), 4.78 (2H, brs, NCH₂Ar), 4.37 (2H, s, ArOCH₂CO), 3.75 (2H, d, J=7Hz, NCH₂-iPr), 2.02 (3H, s, 3-CH₃).
MASS (EI) m/z : 416(M⁺), 309, 300, 267, 253, 116.

### (Example 507)

### Compound of Formula No. B-263

¹H-NMR (CDCl₃) δ(ppm) : 6.95 (1H, s, 5-H), 4.77 (2H, brs, NCH₂Ar), 4.46 (2H, s, ArOCH₂CO), 3.74 (2H, d, J=7Hz, NCH₂-iPr), 2.01 (3H, s, 3-CH₃).
MASS (EI) m/z : 432(M⁺), 309, 267, 253, 137, 116.

### (Example 508)

### Compound of Formula No. B-264

¹H-NMR (CDCl₃) δ(ppm) : 6.95 (1H, s, 5-H), 4.78 (2H, brs, NCH₂Ar), 4.39 (2H, s, ArOCH₂CO), 3.76 (2H, d, J=7Hz, NCH₂-iPr), 2.03 (3H, s, 3-CH₃).
MASS (EI) m/z : 432(M⁺), 309, 295, 266, 253, 137, 116.

### (Example 509)

### Compound of Formula No. B-265

¹H-NMR (CDCl₃) δ(ppm) : 6.94 (1H, s, 5-H), 4.78 (2H, brs, NCH₂Ar), 4.36 (2H, s, ArOCH₂CO), 3.76 (2H, d, J=7Hz, NCH₂-iPr), 2.02 (3H, s, 3-CH₃).
MASS (EI) m/z : 432(M⁺), 309, 267, 253, 137, 116.

### (Example 510)

### Compound of Formula No. B-266

¹H-NMR (CDCl₃) δ(ppm) : 7.03 (1H, s, 5-H), 4.77 (2H, brs, NCH₂Ar), 4.56 (2H, s, ArOCH₂CO), 3.75 (2H, d, J=7Hz, NCH₂-iPr), 2.04 (3H, s, 3-CH₃).
MASS (EI) m/z : 447(M⁺), 401, 309, 267, 152, 116.

### (Example 511)

### Compound of Formula No. B-267

¹H-NMR (CDCl₃) δ(ppm) : 7.05 (1H, s, 5-H), 4.80 (2H, brs, NCH₂Ar), 4.51 (2H, s, ArOCH₂CO), 3.79 (2H, d, J=7Hz, NCH₂-iPr), 2.08 (3H, s, 3-CH₃).
MASS (EI) m/z : 447(M⁺), 331, 267, 152, 136, 116.

### (Example 512)

### Compound of Formula No. B-268

¹H-NMR (CDCl₃) δ(ppm) : 7.02 (1H, s, 5-H), 4.79 (2H, brs, NCH₂Ar), 4.52 (2H, s, ArOCH₂CO), 3.79 (2H, d, J=7Hz, NCH₂-iPr), 2.05 (3H, s, 3-CH₃).
MASS (EI) m/z : 447(M⁺), 331, 267, 152, 136, 116.

### (Example 513)

### Compound of Formula No. B-269

¹H-NMR (CDCl₃) δ(ppm) : 6.97 (1H, s, 5-H), 4.80 (2H, brs, NCH₂Ar), 4.50 (2H, s, ArOCH₂CO), 3.73 (2H, d, J=7Hz, NCH₂-iPr), 2.06 (3H, s, 3-CH₃).
MASS (EI) m/z : 470(M⁺), 451, 354, 309, 267, 253, 175, 116.

### (Example 514)

### Compound of Formula No. B-270

¹H-NMR (CDCl₃) δ(ppm) : 6.96 (1H, s, 5-H), 4.81 (2H, brs, NCH₂Ar), 4.44 (2H, s, ArOCH₂CO), 3.77 (2H, d, J=7Hz, NCH₂-iPr), 2.04 (3H, s, 3-CH₃).
MASS (EI) m/z : 470(M⁺), 451, 354, 309, 267, 253, 175, 116.

### (Example 515)

### Compound of Formula No. B-271

¹H-NMR (CDCl₃) δ(ppm) : 6.97 (1H, s, 5-H), 4.78 (2H, brs, NCH₂Ar), 4.45 (2H, s, ArOCH₂CO), 3.77 (2H, d, J=7Hz, NCH₂-iPr), 2.04 (3H, s, 3-CH₃).
MASS (EI) m/z : 470(M⁺), 451, 354, 309, 267, 253, 175, 116.

### (Example 516)

### Compound of Formula No. B-272

¹H-NMR (CDCl₃) δ(ppm) : 7.06 (1H, s, 5-H), 4.79 (2H, brs, NCH₂Ar), 4.56 (2H, s, ArOCH₂CO), 3.77 (2H, d, J=7Hz, NCH₂-iPr), 2.05 (3H, s, 3-CH₃).
MASS (EI) m/z : 427(M⁺), 384, 311, 267, 132, 116.

### (Example 517)

### Compound of Formula No. B-273

¹H-NMR (CDCl₃) δ(ppm) : 6.99 (1H, s, 5-H), 4.79 (2H, brs, NCH₂Ar), 4.43 (2H, s, ArOCH₂CO), 3.79 (2H, d, J=7Hz, NCH₂-iPr), 2.04 (3H, s, 3-CH₃).
MASS (EI) m/z : 427(M⁺), 384, 311, 267, 132, 116.

### (Example 518)

### Compound of Formula No. B-274

¹H-NMR (CDCl₃) δ(ppm) : 6.97 (1H, s, 5-H), 4.77 (2H, brs, NCH₂Ar), 4.46 (2H, s, ArOCH₂CO), 3.78 (2H, d, J=7Hz, NCH₂-iPr), 2.04 (3H, s, 3-CH₃).
MASS (EI) m/z : 427(M⁺), 384, 311, 267, 132, 116.

### (Example 519)

### Compound of Formula No. B-275

¹H-NMR (CDCl₃) δ(ppm) : 6.96 (1H, s, 5-H), 4.78 (2H, brs, NCH₂Ar), 4.45 (2H, s, ArOCH₂CO), 3.75 (2H, d, J=7Hz, NCH₂-iPr), 2.01 (3H, s, 3-CH₃).
MASS (EI) m/z : 438(M⁺), 322, 309, 267, 143, 116.

### (Example 520)

### Compound of Formula No. B-276

¹H-NMR (CDCl₃) δ(ppm) : 6.97 (1H, s, 5-H), 4.78 (2H, brs, NCH₂Ar), 4.46 (2H, s, ArOCH₂CO), 3.75 (2H, d, J=7Hz, NCH₂-iPr), 2.03 (3H, s, 3-CH₃).
MASS (EI) m/z : 470(M⁺), 435, 354, 309, 267, 175, 116.

### (Example 521)

### Compound of Formula No. B-277

¹H-NMR (CDCl₃) δ(ppm) : 6.98 (1H, s, 5-H), 4.77 (2H, brs, NCH₂Ar), 4.38 (2H, s, ArOCH₂CO), 3.78 (2H, d, J=7Hz, NCH₂-iPr), 2.04 (3H, s, 3-CH₃).
MASS (EI) m/z : 470(M⁺), 354, 309, 267, 175, 116.

### (Example 522)

### Compound of Formula No. B-278

¹H-NMR (CDCl₃) δ(ppm) : 7.07 (1H, s, 5-H), 4.75 (2H, brs, NCH₂Ar), 4.71 (2H, s, ArOCH₂CO), 3.78 (2H, d, J=7Hz, NCH₂-iPr), 2.06 (3H, s, 3-CH₃).
MASS (EI) m/z : 492(M⁺), 423, 376, 309, 266, 167, 116.

### (Example 523)

### Compound of Formula No. B-279

¹H-NMR (CDCl₃) δ(ppm) : 6.94 (1H, s, 5-H), 4.77 (2H, brs, NCH₂Ar), 4.47 (2H, s, ArOCH₂CO), 3.97 (2H, d, J=7Hz, NCH₂-cBu), 2.01 (3H, s, 5-CH₃).
MASS (EI) m/z : 432(M⁺), 321, 279, 253, 125, 116.

### (Example 524)

### Compound of Formula No. B-280

¹H-NMR (CDCl₃) δ(ppm) : 6.95 (1H, s, 5-H), 4.77 (2H, brs, NCH₂Ar), 4.38 (2H, s, ArOCH₂CO), 3.97 (2H, d, J=7Hz, NCH₂-cBu), 2.02 (3H, s, 5-CH₃).
MASS (EI) m/z : 432(M⁺), 321, 279, 253, 125, 116.

### (Example 525)

### Compound of Formula No. B-281

¹H-NMR (CDCl₃) δ(ppm) : 6.97 (1H, s, 5-H), 4.77 (2H, brs, NCH₂Ar), 4.36 (2H, s, ArOCH₂CO), 3.98 (2H, d, J=7Hz, NCH₂-cBu), 2.01 (3H, s, 5-CH₃).
MASS (EI) m/z : 432(M⁺), 321, 279, 253, 125, 116.

### (Example 526)

### Compound of Formula No. B-282

¹H-NMR (CDCl₃) δ(ppm) : 6.99 (1H, s, 5-H), 4.78 (2H, brs, NCH₂Ar), 4.47 (2H, s, ArOCH₂CO), 3.96 (2H, d, J=7Hz, NCH₂-cBu), 2.02 (3H, s, 5-CH₃).
MASS (EI) m/z : 448(M⁺), 413, 321, 279, 253, 141, 116.

### (Example 527)

### Compound of Formula No. B-283

¹H-NMR (CDCl₃) δ(ppm) : 6.97 (1H, s, 5-H), 4.78 (2H, brs, NCH₂Ar), 4.38 (2H, s, ArOCH₂CO), 4.00 (2H, d, J=7Hz, NCH₂-cBu), 2.03 (3H, s, 5-CH₃).
MASS (EI) m/z : 448(M⁺), 332, 321, 279, 253, 141, 116.

### (Example 528)

### Compound of Formula No. B-284

¹H-NMR (CDCl₃) δ(ppm) : 6.94 (1H, s, 5-H), 4.77 (2H, brs, NCH₂Ar), 4.36 (2H, s, ArOCH₂CO), 3.99 (2H, d, J=7Hz, NCH₂-cBu), 2.01 (3H, s, 5-CH₃).
MASS (EI) m/z : 448(M⁺), 332, 321, 279, 253, 141, 116.

### (Example 529)

### Compound of Formula No. B-285

¹H-NMR (CDCl₃) δ(ppm) : 6.92 (1H, s, 5-H), 4.78 (2H, brs, NCH₂Ar), 4.41 (2H, s, ArOCH₂CO), 3.97 (2H, d, J=7Hz, NCH₂-cBu), 2.01 (3H, s, 5-CH₃).
MASS (EI) m/z : 428(M⁺), 400, 321, 280, 253, 116.

### (Example 530)

### Compound of Formula No. B-286

¹H-NMR (CDCl₃) δ(ppm) : 6.93 (1H, s, 5-H), 4.77 (2H, brs, NCH₂Ar), 4.37 (2H, s, ArOCH₂CO), 3.98 (2H, d, J=7Hz, NCH₂-cBu), 2.02 (3H, s, 5-CH₃).
MASS (EI) m/z : 428(M⁺), 400, 321, 279, 253, 121, 116.

### (Example 531)

### Compound of Formula No. B-287

¹H-NMR (CDCl₃) δ(ppm) : 6.92 (1H, s, 5-H), 4.77 (2H, brs, NCH₂Ar), 4.36 (2H, s, ArOCH₂CO), 3.98 (2H, d, J=7Hz, NCH₂-cBu), 2.01 (3H, s, 5-CH₃).
MASS (EI) m/z : 428(M⁺), 400, 321, 279, 253, 121, 116.

### (Example 532)

### Compound of Formula No. B-288

¹H-NMR (CDCl₃) δ(ppm) : 6.93 (1H, s, 5-H), 4.76 (2H, brs, NCH₂Ar), 4.44 (2H, s, ArOCH₂CO), 3.97 (2H, d, J=7Hz, NCH₂-cBu), 2.00 (3H, s, 5-CH₃).
MASS (EI) m/z : 444(M⁺), 416, 321, 279, 253, 137, 116.

### (Example 533)

### Compound of Formula No. B-289

¹H-NMR (CDCl₃) δ(ppm) : 6.93 (1H, s, 5-H), 4.77 (2H, brs, NCH₂Ar), 4.37 (2H, s, ArOCH₂CO), 3.98 (2H, d, J=7Hz, NCH₂-cBu), 2.02 (3H, s, 5-CH₃).
MASS (EI) m/z : 444(M⁺), 416, 321, 278, 253, 137, 116.

### (Example 534)

### Compound of Formula No. B-290

¹H-NMR (CDCl₃) δ(ppm) : 6.91 (1H, s, 5-H), 4.76 (2H, brs, NCH₂Ar), 4.34 (2H, s, ArOCH₂CO), 3.98 (2H, d, J=7Hz, NCH₂-cBu), 2.00 (3H, s, 5-CH₃).
MASS (EI) m/z : 444(M⁺), 416, 321, 279, 253, 137, 116.

### (Example 535)

### Compound of Formula No. B-291

¹H-NMR (CDCl₃) δ(ppm) : 7.01 (1H, s, 5-H), 4.78 (2H, brs, NCH₂Ar), 4.55 (2H, s, ArOCH₂CO), 3.97 (2H, d, J=7Hz, NCH₂-cBu), 2.03 (3H, s, 5-CH₃).
MASS (EI) m/z : 459(M⁺), 413, 321, 278, 253, 152, 116.

### (Example 536)

### Compound of Formula No. B-292

¹H-NMR (CDCl₃) δ(ppm) : 7.04 (1H, s, 5-H), 4.79 (2H, brs, NCH₂Ar), 4.50 (2H, s, ArOCH₂CO), 4.02 (2H, d, J=7Hz, NCH₂-cBu), 2.06 (3H, s, 5-CH₃).
MASS (EI) m/z 459(M⁺), 343, 321, 279, 253, 152, 116.

### (Example 537)

### Compound of Formula No. B-293

¹H-NMR (CDCl₃) δ(ppm) : 6.99 (1H, s, 5-H), 4.76 (2H, brs, NCH₂Ar), 4.49 (2H, s, ArOCH₂CO), 4.01 (2H, d, J=7Hz, NCH₂-cBu), 2.03 (3H, s, 5-CH₃).
MASS (EI) m/z : 459(M⁺), 343, 321, 279, 253, 152, 116.

### (Example 538)

### Compound of Formula No. B-294

¹H-NMR (CDCl₃) δ(ppm) : 6.97 (1H, s, 5-H), 4.78 (2H, brs, NCH₂Ar), 4.49 (2H, s, ArOCH₂CO), 3.96 (2H, d, J=7Hz, NCH₂-cBu), 2.04 (3H, s, 5-CH₃).
MASS (EI) m/z : 482(M⁺), 366, 321, 279, 253, 175, 116.

### (Example 539)

### Compound of Formula No. B-295

¹H-NMR (CDCl₃) δ(ppm) : 6.96 (1H, s, 5-H), 4.79 (2H, brs, NCH₂Ar), 4.43 (2H, s, ArOCH₂CO), 3.99 (2H, d, J=7Hz, NCH₂-cBu), 2.03 (3H, s, 5-CH₃).
MASS (EI) m/z : 482(M⁺), 366, 321, 279, 253, 175, 116.

### (Example 540)

### Compound of Formula No. B-296

¹H-NMR (CDCl₃) δ(ppm) : 6.98 (1H, s, 5-H), 4.78 (2H, brs, NCH₂Ar), 4.44 (2H, s, ArOCH₂CO), 4.00 (2H, d, J=7Hz, NCH₂-cBu), 2.03 (3H, s, 5-CH₃).
MASS (EI) m/z : 482(M⁺), 366, 321, 279, 253, 175, 116.

### (Example 541)

### Compound of Formula No. B-297

¹H-NMR (CDCl₃) δ(ppm) : 7.06 (1H, s, 5-H), 4.76 (2H, brs, NCH₂Ar), 4.55 (2H, s, ArOCH₂CO), 3.99 (2H, d, J=7Hz, NCH₂-cBu), 2.04 (3H, s, 5-CH₃).
MASS (EI) m/z : 439(M⁺), 411, 321, 279, 253, 132, 116.

### (Example 542)

### Compound of Formula No. B-298

¹H-NMR (CDCl₃) δ(ppm) : 7.00 (1H, s, 5-H), 4.78 (2H, brs, NCH₂Ar), 4.42 (2H, s, ArOCH₂CO), 4.01 (2H, d, J=7Hz, NCH₂-cBu), 2.03 (3H, s, 5-CH₃).
MASS (EI) m/z : 439(M⁺), 411, 321, 279, 253, 132, 116.

### (Example 543)

### Compound of Formula No. B-299

¹H-NMR (CDCl₃) δ(ppm) : 6.99 (1H, s, 5-H), 4.76 (2H, brs, NCH₂Ar), 4.45 (2H, s, ArOCH₂CO), 4.00 (2H, d, J=7Hz, NCH₂-cBu), 2.02 (3H, s, 5-CH₃).
MASS (EI) m/z : 439(M⁺), 321, 279, 253, 132, 116.

### (Example 544)

### Compound of Formula No. B-300

¹H-NMR (CDCl₃) δ(ppm) : 6.93 (1H, s, 5-H), 4.75 (2H, brs, NCH₂Ar), 4.42 (2H, s, ArOCH₂CO), 3.97 (2H, d, J=7Hz, NCH₂-cBu), 1.99 (3H, s, 5-CH₃).
MASS (EI) m/z : 450(M⁺), 321, 279, 253, 116.

### (Example 545)

### Compound of Formula No. B-301

¹H-NMR (CDCl₃) δ(ppm) : 6.96 (1H, s, 5-H), 4.76 (2H, brs, NCH₂Ar), 4.44 (2H, s, ArOCH₂CO), 3.97 (2H, d, J=7Hz, NCH₂-cBu), 2.01 (3H, s, 5-CH₃).
MASS (EI) m/z : 482(M⁺), 447, 321, 279, 253, 175, 116.

### (Example 546)

### Compound of Formula No. B-302

¹H-NMR (CDCl₃) δ(ppm) : 6.98 (1H, s, 5-H), 4.78 (2H, brs, NCH₂Ar), 4.37 (2H, s, ArOCH₂CO), 4.00 (2H, d, J=7Hz, NCH₂-cBu), 2.04 (3H, s, 5-CH₃).
MASS (EI) m/z : 482(M⁺), 424, 366, 321, 279, 253, 175, 116.

### (Example 547)

### Compound of Formula No. B-303

¹H-NMR (CDCl₃) δ(ppm) : 7.05 (1H, s, 5-H), 4.78 (2H, brs, NCH₂Ar), 4.76 (2H, s, ArOCH₂CO), 4.69 (2H, d, J=7Hz, NCH₂-cBu), 2.05 (3H, s, 5-CH₃).
MASS (EI) m/z : 504(M⁺), 321, 278, 253, 210, 167, 116.

### (Example 548)

### Compound of Formula No. B-304

¹H-NMR (CDCl₃) δ(ppm) : 7.06 (1H, s, 5-H), 4.77 (2H, brs, NCH₂Ar), 4.47 (2H, s, COCH₂O), 3.82 (3H, s, NCH₃), 1.98 (3H, s, 3-CH₃).
MASS (EI) m/z : 361(M⁺), 267, 225, 116.

### (Example 549)

### Compound of Formula No. B-305

¹H-NMR (CDCl₃) δ(ppm) : 7.05 (1H, s, 5-H), 4.78 (2H, brs, NCH₂Ar), 4.46 (2H, s, COCH₂O), 4.06 (2H, q, J=7Hz, NCH₂Me), 2.00 (3H, s, 3-CH₃).
MASS (EI) m/z : 375(M⁺), 360, 281, 267, 240, 116.

### (Example 550)

### Compound of Formula No. B-306

¹H-NMR (CDCl₃) δ(ppm) : 7.08 (1H, s, 5-H), 4.79 (2H, brs, NCH₂Ar), 4.03 (2H, q, J=7Hz, NCH₂Me), 3.82 (2H, s, COCH₂S), 2.07 (3H, s, 3-CH₃).
MASS (EI) m/z : 392(M⁺), 240, 153, 125, 116.

### (Example 551)

### Compound of Formula No. B-307

¹H-NMR (CDCl₃) δ(ppm) : 7.04 (1H, s, 5-H), 4.78 (2H, brs, NCH₂Ar), 4.46 (2H, s, COCH₂O), 3.96 (2H, t, J=7Hz, NCH₂Et), 2.02 (3H, s, 3-CH₃).
MASS (EI) m/z : 389(M⁺), 374, 295, 253, 116.

### (Example 552)

### Compound of Formula No. B-308

¹H-NMR (CDCl₃) δ(ppm) : 7.04 (1H, s, 5-H), 4.77 (2H, brs, NCH₂Ar), 4.46 (2H, s, COCH₂O), 4.36 (1H, hept, J=7Hz, Me₂CH), 2.01 (3H, s, 3-CH₃).
MASS (EI) m/z : 389(M⁺), 374, 295, 253, 116.

### (Example 553)

### Compound of Formula No. B-309

¹H-NMR (CDCl₃) δ(ppm) : 7.01 (1H, s, 5-H), 4.78 (2H, brs, NCH₂Ar), 4.46 (2H, s, COCH₂O), 3.99 (2H, t, J=7Hz, NCH₂-Pr), 2.02 (3H, s, 3-CH₃).
MASS (EI) m/z : 403(M⁺), 374, 360, 287, 267, 136, 116.

### (Example 554)

### Compound of Formula No. B-310

¹H-NMR (CDCl₃) δ(ppm) : 6.99 (1H, s, 5-H), 4.79 (2H, brs, NCH₂Ar), 4.47 (2H, s, COCH₂O), 3.78 (2H, d, J=7Hz, NCH₂-iPr), 2.03 (3H, s, 3-CH₃).
MASS (EI) m/z : 403(M⁺), 388, 360, 287, 267, 136, 116.

### (Example 555)

### Compound of Formula No. B-311

¹H-NMR (CDCl₃) δ(ppm) : 6.97 (1H, s, 5-H), 4.77 (2H, brs, NCH₂Ar), 4.44 (2H, s, COCH₂O), 3.99 (2H, d, J=7Hz, NCH₂-cBu), 2.02 (3H, s, 5-CH₃).
MASS (EI) m/z : 415(M⁺), 387, 321, 279, 253, 116.

### (Example 556)

### Compound of Formula No. B-312

¹H-NMR (CDCl₃) δ(ppm) : 6.87 (1H, s, 5-H), 4.76 (2H, brs, NCH₂Ar), 3.61 (2H, s, NCH₂Si), 1.98 (3H, s, 3-CH₃), 1.55-1.40 (1H, m, COCH(CH₂)₂).
MASS (EI) m/z : 366(M⁺), 351, 297, 283, 250, 182, 116.

### (Example 557)

### Compound of Formula No. B-313

¹H-NMR (CDCl₃) δ(ppm) : 6.82 (1H, s, 5-H), 4.79 (2H, brs, NCH₂Ar), 3.83 (2H, d, J=5Hz, COCH₂O), 3.60 (2H, s, NCH₂Si), 1.93 (3H, S, 3-CH₃).
MASS (EI) m/z : 356(M⁺), 341, 325, 297, 240, 210, 182, 167, 116.

### (Example 558)

### Compound of Formula No. B-314

¹H-NMR (CDCl₃) δ(ppm) : 6.81 (1H, s, 5-H), 4.75 (2H, brs, NCH₂Ar), 3.79 (2H, s, COCH₂O), 3.60 (2H, S, NCH₂Si), 1.93 (3H, S, 3-CH₃).
MASS (EI) m/z : 370(M⁺), 355, 325, 297, 254, 208, 167, 116

### (Example 559)

### Compound of Formula No. B-315

¹H-NMR (CDCl₃) δ(ppm) : 6.89 (1H, s, 5-H), 4.64 (2H, brs, NCH₂Ar), 4.38 (2H, s, COCH₂O), 3.60 (2H, s, NCH₂Si), 1.99 (3H, s, 3-CH₃).
MASS (EI) m/z : 398(M⁺), 383, 325, 297, 282, 210, 116.

### (Example 560)

### Compound of Formula No. B-316

¹H-NMR (CDCl₃) δ(ppm) : 7.00 (1H, s, 5-H), 4.82 (2H, brs, NCH₂Ar), 4.79 (2H, brs, COCH₂O), 3.61 (2H, s, NCH₂Si), 2.07 (3H, s, 3-CH₃).
MASS (EI) m/z : 468(M⁺), 453, 323, 298.

### (Example 561)

### Compound of Formula No. B-317

¹H-NMR (CDCl₃) δ(ppm) : 6.92 (1H, s, 5-H), 4.76 (2H, brs, NCH₂Ar), 4.71 (2H, s, COCH₂N), 3.62 (2H, s, NCH₂Si), 2.01 (3H, s, 3-CH₃).
MASS (EI) m/z : 406(M⁺), 391, 325, 297.

### (Example 562)

### Compound of Formula No. B-318

¹H-NMR (CDCl₃) δ(ppm) : 6.91 (1H, s, 5-H), 4.73 (2H, brs, NCH₂Ar), 4.63 (2H, s, COCH₂N), 3.62 (2H, s, NCH₂Si), 2.00 (3H, s, 3-CH₃).
MASS (EI) m/z : 440(M⁺), 425, 324, 297.

### (Example 563)

### Compound of Formula No. B-319

¹H-NMR (CDCl₃) δ(ppm) : 6.90 (1H, s, 5-H), 4.76 (2H, brs, NCH₂Ar), 4.66 (2H, s, COCH₂N), 3.62 (2H, s, NCH₂Si), 2.00 (3H, s, 3-CH₃).
MASS (EI) m/z : 485(M⁺), 471, 368, 325, 297, 222, 194, 159

### (Example 564)

### Compound of Formula No. B-320

¹H-NMR (CDCl₃) δ(ppm) : 6.94 (1H, s, 5-H), 4.76 (2H, brs, NCH₂Ar), 4.72 (2H, s, COCH₂N), 3.63 (2H, s, NCH₂Si), 2.01 (3H, s, 3-CH₃).
MASS (EI) m/z : 431(M⁺), 416, 325, 315, 297, 222, 194.

### (Example 565)

### Compound of Formula No. B-321

¹H-NMR (CDCl₃) δ(ppm) : 6.96 (1H, s, 5-H), 4.71 (2H, brs, NCH₂Ar), 4.67 (2H, s, COCH₂N), 3.67 (2H, s, NCH₂Si), 2.01 (3H, s, 3-CH₃).
MASS (EI) m/z : 451(M⁺), 436, 335, 325, 297, 222, 194, 116.

### (Example 566)

### Compound of Formula No. B-322

¹H-NMR (CDCl₃) δ(ppm) : 6.93 (1H, s, 5-H), 4.70 (2H, brs, NCH₂Ar), 4.66 (2H, s, COCH₂N), 3.62 (2H, s, NCH₂Si), 2.01 (3H, s, 3-CH₃).
MASS (EI) m/z : 464(M⁺), 449, 433, 359, 348, 325, 297, 281.

### (Example 567)

### Compound of Formula No. B-323

¹H-NMR (CDCl₃) δ(ppm) : 6.80 (1H, s, 5-H), 4.70 (2H, brs, NCH₂Ar), 3.71 (3H, brs, COOCH₃), 3.58 (2H, s, NCH₂Si), 1.96 (3H, s, 3-CH₃).
MASS (EI) m/z : 356(M⁺), 341, 297, 240, 136, 116.

### (Example 568)

### Compound of Formula No. B-325

¹H-NMR (CDCl₃) δ(ppm) : 4.76 (2H, s, NCH₂Ar), 3.76 (3H, brs, COOCH₃), 2.07 (3H, s, 3-CH₃).
MASS (EI) m/z : 346(M⁺), 287, 230, 116.

### (Example 569)

### Compound of Formula No. B-327

¹H-NMR (CDCl₃) δ(ppm) : 6.78 (1H, s, 5-H), 4.70 (2H, brs, NCH₂Ar), 4.69 (2H, s, COCH₂O), 3.58 (2H, s, NCH₂Si), 1.88 (3H, s, 3-CH₃).
MASS (EI) m/z : 422(M⁺), 407, 339, 73.

### (Example 570)

### Compound of Formula No. B-328

¹H-NMR (CDCl₃) δ(ppm) : 7.28 (1H, s, 5-H), 4.79 (2H, s, NCH₂Ar), 1.99 (3H, s, 3-CH₃), 1.51-1.41 (1H, m, COCH(CH₂)₂).
MASS (EI) m/z : 280(M⁺), 212, 116, 69.

### (Example 571)

### Compound of Formula No. B-329

¹H-NMR (CDCl₃) δ(ppm) : 7.81 (1H, s, 5-H), 4.78 (2H, s, NCH₂Ar), 2.05 (3H, s, 3-CH₃), 1.64 (9H, brs, COOC(CH₃)₃), 1.52-1.39 (1H, m, COCH(CH₂)₂).
MASS (EI) m/z : 380(M⁺), 365, 348, 321, 280, 212, 116.

### (Example 572)

### Compound of Formula No. B-330

¹H-NMR (CDCl₃) δ(ppm) : 7.20 (1H, s, 5-H), 4.72 (2H, s, NCH₂Ar), 3.72 (3H, s, COOCH₃), 1.98 (3H, s, 3-CH₃).
MASS (EI) m/z : 270(M⁺), 239, 211, 154, 122, 116.

### (Example 573)

### Compound of Formula No. B-331

¹H-NMR (CDCl₃) δ(ppm) : 6.86 (1H, s, 5-H), 4.76 (2H, brs, NCH₂Ar), 3.74 (2H, s, NCH₂-t-Bu), 2.00 (3H, s, 3-CH₃), 1.91 (3H, s, COCH₃).
MASS (EI) m/z : 324(M⁺), 309, 282, 267, 225, 208.

### (Example 574)

### Compound of Formula No. B-332

¹H-NMR (CDCl₃) δ(ppm) : 6.93 (1H, s, 5-H), 4.82 (2H, brs, NCH₂Ar), 3.75 (2H, s, NCH₂-t-Bu), 2.03 (3H, s, 3-CH₃), 1.54-1.42 (1H, m, COCH(CH₂)₂).
MASS (EI) m/z : 350(M⁺), 335, 293, 282, 225, 116.

### (Example 575)

### Compound of Formula No. B-333

¹H-NMR (CDCl₃) δ(ppm) : 6.87 (1H, s, 5-H), 4.80 (2H, brs, NCH₂Ar), 3.83 (2H, d, J=5Hz, COCH₂O), 3.75 (2H, s, NCH₂-t-Bu), 1.98 (3H, s, 3-CH₃).
MASS (EI) m/z : 340(M⁺), 325, 283, 224, 194, 122.

### (Example 576)

### Compound of Formula No. B-334

¹H-NMR (CDCl₃) δ(ppm) : 6.86 (1H, s, 5-H), 4.76 (2H, brs, NCH₂Ar), 3.79 (2H, s, COCH₂O), 3.75 (2H, s, NCH₂-t-Bu), 1.99 (3H, s, 3-CH₃).
MASS (EI) m/z : 354(M⁺), 339, 297, 281, 238, 182, 116.

### (Example 577)

### Compound of Formula No. B-335

¹H-NMR (CDCl₃) δ(ppm) : 6.91 (1H, s, 5-H), 4.83 (2H, brs, NCH₂Ar), 4.41 (2H, s, COCH₂O), 3.75 (2H, s, NCH₂-t-Bu), 2.04 (3H, s, 3-CH₃).
MASS (EI) m/z : 416(M⁺), 401, 340, 323, 300, 281.

### (Example 578)

### Compound of Formula No. B-336

¹H-NMR (CDCl₃) δ(ppm) : 6.93 (1H, s, 5-H), 4.76 (2H, brs, NCH₂Ar), 4.38 (2H, s, COCH₂O), 3.75 (2H, s, NCH₂-t-Bu), 2.04 (3H, s, 3-CH₃).
MASS (EI) m/z : 382(M⁺), 367, 325, 280, 266, 225, 194, 116.

### (Example 579)

### Compound of Formula No. B-337

¹H-NMR (CDCl₃) δ(ppm) : 6.85 (1H, s, 5-H), 4.70 (2H, brs, NCH₂Ar), 3.72 (5H, s, COOCH₃ & NCH₂-t-Bu), 2.00 (3H, s, 3-CH₃). MASS (EI) m/z : 340(M⁺), 325, 283, 224, 168, 116.
(Example 580)

### Compound of Formula No. B-338

¹H-NMR (CDCl₃) δ(ppm) : 7.63-7.56 & 7.24-7.05 (5H, m, COOC₆H₅), 6.99 (1H, s, 5-H), 4.78 (2H, brs, NCH₂Ar), 3.75 (2H, s, NCH₂-t-Bu), 2.10 (3H, s, 3-CH₃).
MASS (EI) m/z : 402(M⁺), 387, 309, 265, 239, 116.

### (Example 581)

### Compound of Formula No. B-339

¹H-NMR (CDCl₃) δ(ppm) : 8.25 (1H, s, CONH₂), 6.92 (1H, s, 5-H), 6.81 (1H, s, CONH₂), 5.11 (2H, brs, NCH₂Ar), 3.75 (2H, s, NCH₂-t-Bu), 2.03 (3H, s, 3-CH₃).
MASS (EI) m/z : 325(M⁺), 282, 256, 225, 209.

### (Example 582)

### Compound of Formula No. B-340

¹H-NMR (CDCl₃) δ(ppm) : 8.38 (1H, d, J=5Hz, O-Pyr-6'H), 7.01 (1H, s, 5-H), 4.80 (2H, brs, NCH₂Ar), 3.73 (2H, s, NCH₂-t-Bu), 2.14 (3H, s, 3-CH₃).
MASS (EI) m/z : 403(M⁺), 388, 359, 308, 280, 192, 136, 116.

### (Example 583)

### Compound of Formula No. B-341

¹H-NMR (CDCl₃) δ(ppm) : 7.01 (1H, s, 5-H), 4.78 (2H, brs, NCH₂Ar), 3.72 (2H, s, NCH₂-t-Bu), 2.52 (3H, s, O-Pyr-6'CH₃), 2.14 (3H, s, 3-CH₃).
MASS (EI) m/z : 417(M⁺), 402, 373, 309, 280, 265.

### (Example 584)

### Compound of Formula No. B-342

¹H-NMR (CDCl₃) δ(ppm) : 8.43 (1H, d, J=2Hz, O-Pyr-6'H), 7.00 (1H, s, 5-H), 4.78 (2H, brs, NCH₂Ar), 3.77 (2H, s, NCH₂-t-Bu), 2.08 (3H, s, 3-CH₃).
MASS (EI) m/z : 437(M⁺), 422, 309, 239, 192, 136, 116.

### (Example 585)

### Compound of Formula No. B-343

¹H-NMR (CDCl₃) δ(ppm) : 6.99 (1H, s, 5-H), 4.76 (2H, brs, NCH₂Ar), 3.82 (2H, s, NCH₂C(CH₂)₂Me), 1.99 (3H, s, 3-CH₃), 1.92 (3H, s, COCH₃).
MASS (EI) m/z : 322(M⁺), 307, 280, 267, 212, 206, 116.

### (Example 586)

### Compound of Formula No. B-344

¹H-NMR (CDCl₃) δ(ppm) : 7.05 (1H, s, 5-H), 4.78 (2H, brs, NCH₂Ar) , 3.83 (2H, s, NCH₂C(CH₂)₂Me), 2.03 (3H, s, 3-CH₃), 1.57-1.45 (1H, m, COCH(CH₂)₂).
MASS (EI) m/z : 348(M⁺), 280, 232, 212, 164, 116.

### (Example 587)

### Compound of Formula No. B-345

¹H-NMR (CDCl₃) δ(ppm) : 7.00 (1H, s, 5-H), 4.80 (2H, brs, NCH₂Ar), 3.84 (2H, d, J=4Hz, COCH₂O), 3.82 (2H, s, NCH₂C(CH₂)₂Me), 1.97 (3H, s, 3-CH₃).
MASS (EI) m/z : 338(M⁺), 323, 279, 239, 222, 192, 116.

### (Example 588)

### Compound of Formula No. B-346

¹H-NMR (CDCl₃) δ(ppm) : 6.98 (1H, s, 5-H), 4.77 (2H, brs, NCH₂Ar), 3.82 (2H, s, NCH₂C(CH₂)₂Me), 3.80 (2H, s, COCH₂O), 1.99 (3H, s, 3-CH₃).
MASS (EI) m/z : 352(M⁺), 337, 279, 265, 236, 211, 149, 116.

### (Example 589)

### Compound of Formula No. B-347

¹H-NMR (CDCl₃) δ(ppm) : 7.04 (1H, s, 5-H), 4.80 (2H, brs, NCH₂Ar), 4.42 (2H, s, COCH₂O), 3.83 (2H, s, NCH₂C(CH₂)₂Me), 2.04 (3H, s, 3-CH₃).
MASS (EI) m/z : 414(M⁺), 399, 321, 293, 279, 253, 211, 116.

### (Example 590)

### Compound of Formula No. B-348

¹H-NMR (CDCl₃) δ(ppm) : 7.11 (1H, s, 5-H), 4.79 (2H, brs, NCH₂Ar), 4.70 (2H, s, COCH₂O), 3.83 (2H, s, NCH₂C (CH₂)₂Me), 2.13 (3H, S, 3-CH₃).
MASS (EI) m/z : 415(M⁺), 280, 136.

### (Example 591)

### Compound of Formula No. B-349

¹H-NMR (CDCl₃) δ(ppm) : 7.06 (1H, s, 5-H), 4.76 (2H, brs, NCH₂Ar), 4.39 (2H, s, COCH₂O), 3.83 (2H, s, NCH₂C(CH₂)₂Me), 2.03 (3H, s, 3-CH₃).
MASS (EI) m/z : 380(M⁺), 365, 325, 278, 264, 211, 192, 116.

### (Example 592)

### Compound of Formula No. B-350

¹H-NMR (CDCl₃) δ(ppm) : 6.99 (1H, s, 5-H), 4.70 (2H, s, NCH₂Ar), 3.80 (2H, s, NCH₂C(CH₂)₂Me), 3.72 (3H, s, COOCH₃), 2.00 (3H, s, 3-CH₃).
MASS (EI) m/z : 338(M⁺), 323, 283, 270, 222, 154, 116.

### (Example 593)

### Compound of Formula No. B-351

¹H-NMR (CDCl₃) δ(ppm) : 7.39-7.06 (5H, m, COOC₆H₅), 712 (1H, s, 5-H), 4.78 (2H, brs, NCH₂Ar), 3.83 (2H, s, NCH₂C(CH₂)₂Me), 2.10 (3H, s, 3-CH₃).
MASS (EI) m/z : 400(M⁺), 307, 239, 190, 116.

### (Example 594)

### Compound of Formula No. B-352

¹H-NMR (CDCl₃) δ(ppm) : 8.38 (1H, d, J=4Hz, O-Pyr-6'H), 7.13 (1H, s, 5-H), 4.80 (2H, s, NCH₂Ar), 3.81 (2H, s, NCH₂C(CH₂)₂Me), 2.14 (3H, s, 3-CH₃).
MASS (EI) m/z : 401(M⁺), 357, 306, 278, 239, 190, 116.

### (Example 595)

### Compound of Formula No. B-353

¹H-NMR (CDCl₃) δ(ppm) : 7.20 (1H, s, 5-H), 6.28 (1H, dd, J=7Hz, 1Hz, O-Pyr-3'H), 5.20 (1H, d, J=14Hz, NCH₂Ar), 4.66 (1H, d, J=14Hz, NCH₂Ar), 1.97 (3H, s, 3-CH₃).
MASS (EI) m/z : 401(M⁺), 357, 322, 278, 239, 190, 116.

### (Example 596)

### Compound of Formula No. B-354

¹H-NMR (CDCl₃) δ(ppm) : 6.88 (1H, s, 5-H), 4.75 (2H, brs, NCH₂Ar), 3.90 (2H, s, NCH₂C(CH₂)₃Me), 1.99 (3H, s, 3-CH₃), 1.91 (3H, s, COCH₃).
MASS (EI) m/z : 336(M⁺), 321, 308, 294, 267, 220.

### (Example 597)

### Compound of Formula No. B-355

¹H-NMR (CDCl₃) δ(ppm) : 6.87 (1H, s, 5-H), 4.76 (2H, brs, NCH₂Ar), 3.91 (2H, s, NCH₂C(CH₂)₃Me), 3.78 (2H, s, COCH₂O), 1.98 (3H, S, 3-CH₃).
MASS (EI) m/z : 366(M⁺), 351, 338, 293, 265, 250.

### (Example 598)

### Compound of Formula No. B-356

¹H-NMR (CDCl₃) δ(ppm) : 6.93 (1H, s, 5-H), 4.77 (2H, brs, NCH₂Ar), 4.40 (2H, s, COCH₂O), 3.91 (2H, s, NCH₂C(CH₂)₃Me), 2.04 (3H, S, 3-CH₃).
MASS (EI) m/z : 428(M⁺), 400, 359, 335, 293, 253, 116.

### (Example 599)

### Compound of Formula No. B-357

¹H-NMR (CDCl₃) δ(ppm) : 6.87 (1H, s, 5-H), 4.69 (2H, s, NCH₂Ar), 3.88 (2H, s, NCH₂C(CH₂)₃Me), 3.72 (3H, s, COOCH₃), 1.99 (3H, s, 3-CH₃).
MASS (EI) m/z : 352(M⁺), 337, 324, 283, 270, 208, 154.

### (Example 600)

### Compound of Formula No. B-358

¹H-NMR (CDCl₃) δ(ppm) : 7.40-7.05 (5H, m, COOC₆H₅), 7.01 (1H, s, 5-H), 4.77 (2H, brs, NCH₂Ar), 3.91 (2H, s, NCH₂C(CH₂)₃Me), 2.09 (3H, s, 3-CH₃).
MASS (EI) m/z : 414(M⁺), 386, 321, 293, 239.

### (Example 601)

### Compound of Formula No. B-359

¹H-NMR (CDCl₃) δ(ppm) : 8.38 (1H, d, J=5Hz, O-Pyr-6'H), 7.02 (1H, s, 5-H), 4.79 (2H, brs, NCH₂Ar), 3.89 (2H, s, NCH₂C(CH₂)₃Me), 2.13 (3H, s, 3-CH₃).
MASS (EI) m/z : 415(M⁺), 387, 371, 337, 320, 292, 204, 116.

### (Example 602)

### Compound of Formula No. B-360

¹H-NMR (CDCl₃) δ(ppm) : 6.88 (1H, s, 5-H), 4.75 (2H, brs, NCH₂Ar), 3.84 (2H, s, NCH₂-C(CH₂)₄Me), 1.99 (3H, s, 3-CH₃), 1.91 (3H, s, COCH₃).
MASS (EI) m/z : 350(M⁺), 335, 308, 265, 234, 225, 149.

### (Example 603)

### Compound of Formula No. B-361

¹H-NMR (CDCl₃) δ(ppm) : 6.89 (1H, s, 5-H), 4.79 (2H, brs, NCH₂Ar), 3.85 (2H, s, NCH₂-C(CH₂)₄Me), 3.83 (2H, d ,J=5Hz, COCH₂O), 1.97 (3H, s, 3-CH₃).
MASS (EI) m/z : 366(M⁺), 284, 265, 250, 168, 149.

### (Example 604)

### Compound of Formula No. B-362

¹H-NMR (CDCl₃) δ(ppm) : 6.94 (1H, s, 5-H), 4.80 (2H, brs, NCH₂Ar), 4.40 (2H, s, COCH₂O), 3.85 (2H, s, NCH₂-C(CH₂)₄Me), 2.04 (3H, s, 3-CH₃).
MASS (EI) m/z : 442(M⁺), 427, 360, 349, 326, 307, 244.

### (Example 605)

### Compound of Formula No. B-363

¹H-NMR (CDCl₃) δ(ppm) : 6.96 (1H, s, 5-H), 4.78 (2H, brs, NCH₂Ar), 4.38 (2H, s, COCH₂O), 3.85 (2H, s, NCH₂-C(CH₂)₄Me), 2.03 (3H, s, 3-CH₃).
MASS (EI) m/z : 408(M⁺), 393, 348, 326, 306, 220, 210, 116.

### (Example 606)

### Compound of Formula No. B-364

¹H-NMR (CDCl₃) δ(ppm) : 6.87 (1H, s, 5-H), 4.69 (2H, s, NCH₂Ar), 3.82 (2H, s, NCH₂-C(CH₂)₄Me), 3.72 (3H, s, COOCH₃), 1.99 (3H, s, 3-CH₃).
MASS (EI) m/z : 366(M⁺), 351, 284, 265, 168, 149, 116.

### (Example 607)

### Compound of Formula No. B-365

¹H-NMR (CDCl₃) δ(ppm) : 6.88 (1H, s, 5-H), 4.65 (2H, s, NCH₂Ar), 3.81 (2H, s, NCH₂-C(CH₂)₄Me), 1.99 (3H, s, 3-CH₃), 1.42 (9H, brs, COOC(CH₃)₃).
MASS (EI) m/z : 408(M⁺), 352, 335, 308, 270, 225, 154, 116.

### (Example 608)

### Compound of Formula No. B-366

¹H-NMR (CDCl₃) δ(ppm) : 7.39-7.05 (5H, m, COOC₆H₅), 7.01 (1H, s, 5-H), 4.77 (2H, s, NCH₂Ar), 3.85 (2H, s, NCH₂-C(CH₂)₄Me), 2.09 (3H, s, 3-CH₃).
MASS (EI) m/z : 428(M⁺), 413, 346, 335, 239, 136, 116.

### (Example 609)

### Compound of Formula No. B-367

¹H-NMR (CDCl₃) δ(ppm) : 8.39 (1H, d, J=3Hz, O-Pyr-6'H), 6.99 (1H, s, 5-H), 4.79 (2H, brs, NCH₂Ar), 3.83 (2H, s, NCH₂-C(CH₂)₄Me), 2.13 (3H, s, 3-CH₃).
MASS (EI) m/z : 429(M⁺), 385, 351, 306, 251, 218, 116.

### (Example 610)

### Compound of Formula No. B-368

¹H-NMR (CDCl₃) δ(ppm) : 7.02 (1H, s, 5-H), 4.80 (2H, brs, NCH₂Ar), 3.82 (2H, s, NCH₂-C(CH₂)₄Me), 2.52 (3H, s, O-Pyr-6'CH₃), 2.13 (3H, s, 3-CH₃).
MASS (EI) m/z : 443(M⁺), 399, 279, 265, 167, 149.

### (Example 611)

### Compound of Formula No. B-369

¹H-NMR (CDCl₃) δ(ppm) : 8.42 (1H, s, O-Pyr-6'H), 7.01 (1H, s, 5-H), 4.77 (2H, brs, NCH₂Ar), 3.86 (2H, s, NCH₂-C(CH₂)₄Me), 2.07 (3H, s, 3-CH₃).
MASS (EI) m/z : 463(M⁺), 381, 335, 308, 265, 239, 116.

### (Example 612)

### Compound of Formula No. B-370

¹H-NMR (CDCl₃) δ(ppm) : 6.85 (1H, s, 5-H), 4.75 (2H, brs, NCH₂Ar), 3.76 (2H, d, J=7Hz, NCH₂-c-Hex), 2.00 (3H, s, 3-CH₃), 1.91 (3H, s, COCH₃).
MASS (EI) m/z : 350(M⁺), 335, 308, 297, 268, 254, 234, 212.

### (Example 613)

### Compound of Formula No. B-371

¹H-NMR (CDCl₃) δ(ppm) : 6.86 (1H, s, 5-H), 4.79 (2H, brs, NCH₂Ar), 3.83 (2H, brs, COCH₂O), 3.77 (2H, d, J=7Hz, NCH₂-c-Hex), 1.97 (3H, s, 3-CH₃).
MASS (EI) m/z : 366(M⁺), 307, 284, 265, 250, 168, 149, 116.

### (Example 614)

### Compound of Formula No. B-372

¹H-NMR (CDCl₃) δ(ppm) : 6.85 (1H, s, 5-H), 4.76 (2H, brs, NCH₂Ar), 3.79 (2H, s, COCH₂O), 3.78 (2H, d, J=8Hz, NCH₂-c-Hex), 1.99 (3H, s, 3-CH₃).
MASS (EI) m/z : 380(M⁺), 307, 279, 264, 167, 149.

### (Example 615)

### Compound of Formula No. B-373

¹H-NMR (CDCl₃) δ(ppm) : 6.91 (1H, s, 5-H), 4.79 (2H, brs, NCH₂Ar), 4.40 (2H, s, COCH₂O), 3.77 (2H, d, J=7Hz, NCH₂-c-Hex), 2.04 (3H, s, 3-CH₃).
MASS (EI) m/z : 442(M⁺), 359, 349, 326, 307, 244, 116.

### (Example 616)

### Compound of Formula No. B-374

¹H-NMR (CDCl₃) δ(ppm) : 6.93 (1H, s, 5-H), 4.75 (2H, brs, NCH₂Ar), 4.38 (2H, s, COCH₂O), 3.77 (2H, d, J=7Hz, NCH₂-c-Hex), 2.03 (3H, s, 3-CH₃).
MASS (EI) m/z : 408(M⁺), 365, 326, 306, 271, 220, 210.

### (Example 617)

### Compound of Formula No. B-375

¹H-NMR (CDCl₃) δ(ppm) : 6.85 (1H, s, 5-H), 4.69 (2H, s, NCH₂Ar), 3.74 (2H, d, J=7Hz, NCH₂-c-Hex), 3.72 (3H, s, COOCH₃), 2.00 (3H, s, 3-CH₃).
MASS (EI) m/z : 366(M⁺), 352, 324, 284, 270, 250, 168.

### (Example 618)

### Compound of Formula No. B-376

¹H-NMR (CDCl₃) δ(ppm) : 7.36-7.06 (5H, m, COOC₆H₅), 6.98 (1H, s, 5-H), 4.77 (2H, brs, NCH₂Ar), 3.77 (2H, d, J=7Hz, NCH₂-c-Hex), 2.10 (3H, s, 3-CH₃).
MASS (EI) m/z : 428(M⁺), 346, 335, 307, 239, 218, 116.

### (Example 619)

### Compound of Formula No. B-377

¹H-NMR (CDCl₃) δ(ppm) : 8.38 (1H, d, J=3Hz, O-Pyr-6'H), 6.99 (1H, s, 5-H), 4.79 (2H, brs, NCH₂Ar), 3.74 (2H, d, J=7Hz, NCH₂-c-Hex), 2.13 (3H, s, 3-CH₃).
MASS (EI) m/z : 429(M⁺), 385, 334, 306, 239, 218, 136, 116.

### (Example 620)

### Compound of Formula No. B-378

¹H-NMR (CDCl₃) δ(ppm) : 6.84 (1H, s, 5-H), 4.76 (2H, brs, NCH₂Ar), 3.78 (2H, s, NCH₂-C(CH₂)₅Me), 2.00 (3H, s, 3-CH₃), 1.91 (3H, s, COCH₃).
MASS (EI) m/z : 364(M⁺), 349, 322, 268, 248, 225, 152, 116.

### (Example 621)

### Compound of Formula No. B-379

¹H-NMR (CDCl₃) δ(ppm) : 6.90 (1H, s, 5-H), 4.81 (2H, brs, NCH₂Ar), 4.40 (2H, s, COCH₂O), 3.79 (2H, s, NCH₂-C(CH₂)₅Me), 2.05 (3H, s, 3-CH₃).
MASS (EI) m/z : 456(M⁺), 380, 363, 340, 322, 284, 244, 116.

### (Example 622)

### Compound of Formula No. B-380

¹H-NMR (CDCl₃) δ(ppm) : 6.84 (1H, s, 5-H), 4.70 (2H, s, NCH₂Ar), 3.76 (2H, s, NCH₂-C(CH₂)₅Me), 3.72 (3H, brs, COOCH₃), 2.00 (3H, s, 3-CH₃).
MASS (EI) m/z : 380(M⁺), 365, 284, 270, 168, 154, 116.

### (Example 623)

### Compound of Formula No. B-381

¹H-NMR (CDCl₃) δ(ppm) : 6.85 (1H, s, 5-H), 4.65 (2H, s, NCH₂Ar), 3.74 (2H, s, NCH₂-C(CH₂)₅Me), 2.05 (3H, s, 3-CH₃), 1.42 (9H, brs, COOC(CH₃)₃).
MASS (EI) m/z : 422(M⁺), 366, 349, 322, 270, 225, 154.

### (Example 624)

### Compound of Formula No. B-382

¹H-NMR (CDCl₃) δ(ppm) : 7.40-7.06 (5H, m, COOC₆H₅), 6.97 (1H, s, 5-H), 4.78 (2H, brs, NCH₂Ar), 3.79 (2H, s, NCH₂-C(CH₂)₅Me), 2.10 (3H, s, 3-CH₃).
MASS (EI) m/z : 442(M⁺), 427, 332, 239, 230, 136, 116.

### (Example 625)

### Compound of Formula No. B-383

¹H-NMR (CDCl₃) δ(ppm) : 8.38 (1H, d, J=4Hz, O-Pyr-6'H), 6.99 (1H, s, 5-H), 4.80 (2H, brs, NCH₂Ar), 3.77 (2H, s, NCH₂-C(CH₂)₅Me), 2.15 (3H, s, 3-CH₃).
MASS (EI) m/z : 443(M⁺), 399, 347, 320, 232, 211, 136.

### (Example 626)

### Compound of Formula No. B-384

¹H-NMR (CDCl₃) δ(ppm) : 7.00 (1H, s, 5-H), 4.78 (2H, brs, NCH₂Ar), 3.76 (2H, s, NCH₂-C(CH₂)₅Me), 2.53 (3H, s, O-Pyr-6'CH₃), 2.14 (3H, s, 3-CH₃).
MASS (EI) m/z : 457(M⁺), 413, 361, 347, 320, 232, 136, 116.

### (Example 627)

### Compound of Formula No. B-385

¹H-NMR (CDCl₃) δ(ppm) : 7.27 (1H, s, 5-H), 5.42 (2H, s, NCH₂OH), 4.78 (2H, s, NCH₂Ar), 2.05 (3H, s, 3-CH₃), 1.52-1.41 (1H, m, COCH(CH₂)₂).
MASS (EI) m/z : 310(M⁺), 280, 212, 116.

### (Example 628)

### Compound of Formula No. B-386

¹H-NMR (CDCl₃) δ(ppm) : 7.18 (1H, s, 5-H), 5.39 (2H, s, NCH₂OH), 4.71 (2H, s, NCH₂Ar), 3.73 (3H, s, COOCH₃), 2.05 (3H, s, 3-CH₃).
MASS (EI) m/z : 270((M-CH₂O)⁺), 211, 154, 116.

### (Example 629)

### Compound of Formula No. B-387

¹H-NMR (CDCl₃) δ(ppm) : 7.27 (1H, s, 5-H), 5.74 (2H, s, NCH₂Cl), 4.78 (2H, brs, NCH₂Ar), 2.04 (3H, s, 3-CH₃), 1.52-1.40 (1H, m, COCH(CH₂)₂).
MASS (EI) m/z : 328(M⁺), 293, 260, 224, 116.

### (Example 630)

### Compound of Formula No. B-388

¹H-NMR (CDCl₃) δ(ppm) : 7.18 (1H, s, 5-H), 5.72 (2H, s, NCH₂Cl), 4.71 (2H, s, NCH₂Ar), 3.74 (3H, s, COOCH₃), 2.01 (3H, s, 3-CH₃).
MASS (EI) m/z : 318(M⁺), 283, 202, 170, 116.

### (Example 631)

### Compound of Formula No. B-389

¹H-NMR (CDCl₃) δ(ppm) : 7.15 (1H, s, 5-H), 4.70 (2H, s, NCH₂Ar), 4.41 (2H, d, J=12Hz, NCH₂PO₃Et₂), 3.71 (3H, s, COOCH₃), 2.01 (3H, s, 3-CH₃).
MASS (EI) m/z : 420(M⁺), 361, 304, 283, 216, 116.

### (Example 632)

### Compound of Formula No. B-390

¹H-NMR (CDCl₃) δ(ppm) : 7.02 (1H, s, 5-H), 4.80 (2H, brs, NCH₂Ar), 4.52 (2H, brs, NCH₂C(=CH₂)Me), 2.02 (3H, s, 3-CH₃), 1.54-1.44 (1H, m, COCH(CH₂)₂).
MASS (EI) m/z : 334(M⁺), 266, 218, 150, 116.

### (Example 633)

### Compound of Formula No. B-391

¹H-NMR (CDCl₃) δ(ppm) : 6.95 (1H, s, 5-H), 4.70 (2H, s, NCH₂Ar), 4.49 (2H, brs, NCH₂C(=CH₂)Me), 3.72 (3H, s, COOCH₃), 1.99 (3H, s, 3-CH₃).
MASS (EI) m/z : 324(M⁺), 309, 265, 208, 116.

### (Example 634)

### Compound of Formula No. B-392

¹H-NMR (CDCl₃) δ(ppm) : 7.02 (1H, s, 5-H), 6.37 (1H, s, NCH=CMe₂), 4.73 (2H, s, NCH₂Ar), 3.73 (3H, brs, COOCH₃), 2.02 (3H, s, 3-CH₃).
MASS (EI) m/z : 324(M⁺), 283, 265, 208, 176, 116.

### (Example 635)

### Compound of Formula No. B-393

¹H-NMR (CDCl₃) δ(ppm) : 7.13 (1H, s, 5-H), 6.52 (1H, s, NCH=C(CH₂)), 4.71 (2H, s, NCH₂Ar), 3.73 (3H, brs, COOCH₃), 2.00 (3H, s, 3-CH₃).
MASS (EI) m/z : 350(M⁺), 322, 309, 283, 234, 202, 116.

### (Example 636)

### Compound of Formula No. B-394

¹H-NMR (CDCl₃) δ(ppm) : 7.03 (1H, s, 5-H), 4.78 (2H, brs, NCH₂Ar), 4.05 (2H, t, J=6Hz, NCH₂(CH₂)₂CF₃), 2.02 (3H, s, 3-CH₃), 1.54-1.41 (1H, m, COCH(CH₂)₂).
MASS (EI) m/z : 390(M⁺), 371, 322, 274, 206, 116.

### (Example 637)

### Compound of Formula No. B-395

¹H-NMR (CDCl₃) δ(ppm) : 6.96 (1H, s, 5-H), 4.70 (2H, s, NCH₂Ar), 4.02 (2H, t, J=6Hz, NCH₂(CH₂)₂CF₃), 3.73 (3H, brs, COOCH₃), 1.99 (3H, s, 3-CH₃).
MASS (EI) m/z : 380(M⁺), 361, 321, 264, 232, 116.

### (Example 638)

### Compound of Formula No. B-396

¹H-NMR (CDCl₃) δ(ppm) : 7.09 (1H, s, 5-H), 4.77 (2H, brs, NCH₂Ar), 4.22 (2H, d, J=7Hz, NCH₂(C₃H₇O₂)), 2.00 (3H, s, 3-CH₃), 1.52-1.38 (1H, m, COCH(CH₂)₂).
MASS (EI) m/z : 368(M⁺), 300, 281, 252, 225, 116.

### (Example 639)

### Compound of Formula No. B-397

¹H-NMR (CDCl₃) δ(ppm) : 7.01 (1H, s, 5-H), 4.69 (2H, s, NCH₂Ar), 4.18 (2H, d, J=7Hz, NCH₂(C₃H₇O₂)), 3.72 (3H, brs, COOCH₃), 1.97 (3H, s, 3-CH₃).
MASS (EI) m/z : 358(M⁺), 327, 284, 271, 168, 116.

### (Example 640)

### Compound of Formula No. B-398

¹H-NMR (CDCl₃) δ(ppm) : 7.08 (1H, s, 5-H), 4.79 (2H, brs, NCH₂Ar), 4.16 (2H, d, J=7Hz, NCH₂(C₄H₉O₂)), 2.00 (3H, s, 3-CH₃), 1.55-1.37 (1H, m, COCH(CH₂)₂).
MASS (EI) m/z : 382(M⁺), 367, 313, 294, 281, 149.

### (Example 641)

### Compound of Formula No. B-399

¹H-NMR (CDCl₃) δ(ppm) : 7.00 (1H, s, 5-H), 4.70 (2H, s, NCH₂Ar), 4.13 (2H, d, J=7Hz, NCH₂(C₄H₉O₂)), 3.72 (3H, brs, COOCH₃), 1.97 (3H, s, 3-CH₃).
MASS (EI) m/z : 372(M⁺), 357, 311, 284, 168, 116.

### (Example 642)

### Compound of Formula No. B-400

¹H-NMR (CDCl₃) δ(ppm) : 7.05 (1H, s, 5-H), 4.79 (2H, brs, NCH₂Ar), 4.06 (2H, d, J=7Hz, NCH₂(C₅H₁₁O₂)), 2.00 (3H, s, 3-CH₃), 1.58-1.42 (1H, m, COCH(CH₂)₂).
MASS (EI) m/z : 396(M⁺), 381, 349, 327, 294, 225, 178, 116.

### (Example 643)

### Compound of Formula No. B-401

¹H-NMR (CDCl₃) δ(ppm) : 6.97 (1H, s, 5-H), 4.70 (2H, s, NCH₂Ar), 4.02 (2H, d, J=7Hz, NCH₂(C₅H₁₁O₂)), 3.72 (3H, brs, COOCH₃), 1.98 (3H, s, 3-CH₃).
MASS (EI) m/z : 386(M⁺), 371, 339, 284, 168, 116.

### (Example 644)

### Compound of Formula No. B-402

¹H-NMR (CDCl₃) δ(ppm) : 7.06 (1H, s, 5-H), 4.78 (2H, brs, NCH₂Ar), 4.07 (2H, d, J=7Hz, NCH₂ (C₇H₁₁O₄)), 1.98 (3H, s, 3-CH₃), 1.51-1.37 (1H, m, COCH(CH₂)₂).
MASS (EI) m/z : 452(M⁺), 109, 384, 323, 116.

### (Example 645)

### Compound of Formula No. B-403

¹H-NMR (CDCl₃) δ(ppm) : 6.97 (1H, s, 5-H), 4.69 (2H, s, NCH₂Ar), 4.03 (2H, d, J=7Hz, NCH₂(C₇H₁₁O₄)), 3.72 (3H, brs, COOCH₃), 1.95 (3H, s, 3-CH₃).
MASS (EI) m/z : 442(M⁺), 399, 383, 339, 283, 168, 116.

### (Example 646)

### Compound of Formula No. B-404

¹H-NMR (CDCl₃) δ(ppm) : 7.12 (1H, s, 5-H), 4.78 (2H, brs, NCH₂Ar), 4.20 (2H, d, J=8Hz, NCH₂(C₆H₁₁O₂)), 1.98 (3H, s, 3-CH₃), 1.51-1.40 (1H, m, COCH(CH₂)₂).
MASS (EI) m/z : 408(M⁺), 393, 350, 321, 281, 212, 116.

### (Example 647)

### Compound of Formula No. B-405

¹H-NMR (CDCl₃) δ(ppm) : 7.04 (1H, s, 5-H), 4.69 (2H, s, NCH₂Ar), 4.17 (2H, d, J=8Hz, NCH₂ (C₆H₁₁O₂)), 3.71 (3H, s, COOCH₃), 1.95 (3H, s, 3-CH₃).
MASS (EI) m/z : 398(M⁺), 383, 340, 311, 270, 236, 168, 116.

### (Example 648)

### Compound of Formula No. B-406

¹H-NMR (CDCl₃) δ(ppm) : 7.05 (1H, s, 5-H), 4.78 (2H, brs, NCH₂Ar), 4.11 (2H, s, NCH₂(C₄H₉O₂)), 2.02 (3H, s, 3-CH₃), 1.53-1.37 (1H, m, COCH(CH₂)₂).
MASS (EI) m/z : 382(M⁺), 313, 294, 281, 266, 225, 212, 116.

### (Example 649)

### Compound of Formula No. B-407

¹H-NMR (CDCl₃) δ(ppm) : 6.97 (1H, s, 5-H), 4.70 (2H, s, NCH₂Ar), 4.06 (2H, s, NCH₂(C₄H₉O₂)), 3.73 (3H, s, COOCH₃), 1.98 (3H, s, 3-CH₃).
MASS (EI) m/z : 372(M⁺), 341, 284, 270, 168, 116.

### (Example 650)

### Compound of Formula No. B-408

¹H-NMR (CDCl₃) δ(ppm) : 7.04 (1H, s, 5-H), 4.77 (2H, brs, NCH₂Ar), 4.10 (1H, d, J=14Hz, NCH₂(C₅H₁₁O₂)), 3.98 (1H, d, J=14Hz, NCH₂(C₅H₁₁O₂)), 2.01 (3H, s, 3-CH₃), 1.54-1.35 (1H, m, COCH(CH₂)₂).
MASS (EI) m/z : 396(M⁺), 381, 327, 294, 281, 225, 178, 116.

### (Example 651)

### Compound of Formula No. B-409

¹H-NMR (CDCl₃) δ(ppm) : 6.96 (1H, s, 5-H), 4.70 (2H, s, NCH₂Ar), 4.06 (1H, d, J=14Hz, NCH₂(C₅H₁₁O₂)), 3.93 (1H, d, J=14Hz, NCH₂(C₅H₁₁O₂)), 3.72 (3H, s, COOCH₃), 1.98 (3H, s, 3-CH₃).
MASS (EI) m/z : 386(M⁺), 371, 325, 284, 271, 168, 138.

### (Example 652)

### Compound of Formula No. B-410

¹H-NMR (CDCl₃) δ(ppm) : 7.00 (1H, s, 5-H), 4.77 (2H, brs, NCH₂Ar), 3.95 (2H, s, NCH₂(C₆H₁₃O₂)), 2.01 (3H, s, 3-CH₃), 1.55-1.40 (1H, m, COCH(CH₂)₂).
MASS (EI) m/z : 410(M⁺), 395, 363, 341, 294, 225, 178, 116.

### (Example 653)

### Compound of Formula No. B-411

¹H-NMR (CDCl₃) δ(ppm) : 6.93 (1H, s, 5-H), 4.70 (2H, s, NCH₂Ar), 3.91 (2H, s, NCH₂(C₆H₁₃O₂)), 3.72 (3H, s, COOCH₃), 1.99 (3H, s, 3-CH₃).
MASS (EI) m/z : 400(M⁺), 385, 353, 284, 168, 116.

### (Example 654)

### Compound of Formula No. B-412

¹H-NMR (CDCl₃) δ(ppm) : 7.03 (1H, s, 5-H), 4.77 (2H, brs, NCH₂Ar), 3.99 (1H, d, J=14Hz, NCH₂(C₆H₁₁O₃)), 3.92 (1H, d, J=14Hz, NCH₂(C₆H₁₁O₃)), 2.00 (3H, s, 3-CH₃), 1.51-1.35 (1H, m, COCH(CH₂)₂).
MASS (EI) m/z : 424(M⁺), 398, 381, 356, 294, 281, 225, 116.

### (Example 655)

### Compound of Formula No. B-413

¹H-NMR (CDCl₃) δ(ppm) : 6.95 (1H, s, 5-H), 4.70 (2H, s, NCH₂Ar), 3.99 (1H, d, J=14Hz, NCH₂(C₆H₁₁O₃)), 3.92 (1H, d, J=14Hz, NCH₂(C₆H₁₁O₃)), 3.72 (3H, s, COOCH₃), 1.97 (3H, s, 3-CH₃).
MASS (EI) m/z : 414(M⁺), 371, 325, 284, 271, 168, 116.

### (Example 656)

### Compound of Formula No. B-414

¹H-NMR (CDCl₃) δ(ppm) : 7.06 (1H, s, 5-H), 4.78 (2H, brs, NCH₂Ar), 4.16 (2H, s, NCH₂(C₇H₁₃O₂)), 2.00 (3H, s, 3-CH₃), 1.52-1.40 (1H, m, COCH(CH₂)₂)
MASS (EI) m/z : 422(M⁺), 407, 335, 295, 280, 212, 116.

### (Example 657)

### Compound of Formula No. B-415

¹H-NMR (CDCl₃) δ(ppm) : 7.00 (1H, s, 5-H), 4.70 (2H, s, NCH₂Ar), 4.13 (2H, s, NCH₂(C₇H₁₃O₂)), 3.71 (3H, s, COOCH₃), 1.97 (3H, s, 3-CH₃).
MASS (EI) m/z : 412(M⁺), 397, 325, 270, 116.

### (Example 658)

### Compound of Formula No. B-416

¹H-NMR (CDCl₃) δ(ppm) : 7.16 (1H, s, 5-H), 4.78 (2H, brs, NCH₂Ar), 4.17 (2H, d, J=4Hz, NCH₂(C₃H₅O₂)), 2.03 (3H, s, 3-CH₃), 1.56-1.38 (1H, m, COCH(CH₂)₂).
MASS (EI) m/z : 366(M⁺), 298, 225, 116.

### (Example 659)

### Compound of Formula No. B-417

¹H-NMR (CDCl₃) δ(ppm) : 7.09 (1H, s, 5-H), 4.69 (2H, s, NCH₂Ar), 4.15 (2H, d, J=4Hz, NCH₂(C₃H₅O₂)), 3.72 (3H, s, COOCH₃), 2.00 (3H, s, 3-CH₃).
MASS (EI) m/z : 356(M⁺), 325, 283, 168, 116.

### (Example 660)

### Compound of Formula No. B-418

¹H-NMR (CDCl₃) δ(ppm) : 6.84 (1H, s, 5-H), 4.65 (2H, s, NCH₂Ar), 4.42 (2H, s, NCH₂(C₁₁H₁₅O)), 3.72 (3H, s, COOCH₃), 1.96 (3H, s, 3-CH₃).
MASS (EI) m/z : 446(M⁺), 355, 340, 325, 283, 168, 116.

### (Example 661)

### Compound of Formula No. B-419

¹H-NMR (CDCl₃) δ(ppm) : 6.79 (1H, s, 5-H), 4.61 (2H, s, NCH₂Ar), 4.36 (2H, s, NCH₂(C₁₁H₁₅O)), 3.70 (3H, s, COOCH₃), 2.98 (3H, s, Ph-3'-CH₃), 1.98 (3H, s, 3-CH₃).
MASS (EI) m/z : 435(M⁺), 344, 329, 272, 224, 168, 105.

### (Example 662)

### Compound of Formula No. B-420

¹H-NMR (CDCl₃) δ(ppm) : 6.95 (1H, s, 5-H), 4.80 (2H, s, NCH₂Ar), 3.74 (2H, d, J=7Hz, NCH₂-i-Pr), 2.02 (3H, s, 3-CH₃), 1.55-1.44 (1H, m, COCH(CH₂)₂).
MASS (EI) m/z : 336(M⁺), 268, 220, 152, 116.

### (Example 663)

### Compound of Formula No. B-421

¹H-NMR (CDCl₃) δ (ppm) : 6.88 (1H, s, 5-H), 4.72 (2H, s, NCH₂Ar), 3.72 (3H, s, COOCH₃), 3.71 (2H, d, J=7Hz, NCH₂-i-Pr), 1.99 (3H, s, 3-CH₃).
MASS (EI) m/z : 326(M⁺), 311, 283, 270, 210, 168, 116.

### (Example 664)

### Compound of Formula No. C-1

¹H-NMR (CDCl₃) δ(ppm) : 7.13 (1H, s, 3-H), 4.76 (2H, brs, NCH₂Ar), 3.87 (2H, s, COCH₂Cl), 3.78 (3H, s, NCH₃), 1.89 (3H, s, 5-CH₃).
MASS (EI) m/z : 302(M⁺).

### (Example 665)

### Compound of Formula No. C-2

¹H-NMR (CDCl₃) δ(ppm) : 7.15 (1H, s, 3-H), 4.76 (2H, brs, NCH₂Ar), 3.78 (3H, s, NCH₃), 3.70 (2H, s, COCH₂Br), 1.93 (3H, s, 5-CH₃).
MASS (EI) m/z : 346(M⁺).

### (Example 666)

### Compound of Formula No. C-3

¹H-NMR (CDCl₃) δ(ppm) : 7.15 (1H, s, 3-H), 4.78 (2H, brs, NCH₂Ar), 4.38 (2H, s, COCH₂O), 3.77 (3H, s, NCH₃), 1.89 (3H, s, 5-CH₃).
MASS (EI) m/z : 360(M⁺), 267, 253, 225, 116.

### (Example 667)

### Compound of Formula No. C-4

¹H-NMR (CDCl₃) δ(ppm) : 7.16 (1H, s, 3-H), 4.85 (2H, brs, NCH₂Ar), 4.40 (2H, s, COCH₂O), 3.77 (3H, s, NCH₃), 1.94 (3H, s, 5-CH₃).
MASS (EI) m/z : 380(M⁺), 287, 273, 245, 199, 136.

### (Example 668)

### Compound of Formula No. C-5

¹H-NMR (CDCl₃) δ(ppm) : 7.23 (1H, s, 3-H), 4.75 (2H, brs, NCH₂Ar), 4.35 (2H, brs, COCH₂O), 3.71 (3H, s, NCH₃), 1.77 (3H, s, 5-CH₃).
MASS (EI) m/z : 419(M⁺), 326, 298, 284, 244, 175.

### (Example 669)

### Compound of Formula No. C-6

¹H-NMR (CDCl₃) δ(ppm) : 7.17 (1H, s, 3-H), 4.77 (2H, s, NCH₂Ar), 4.06 (2H, q, J=7Hz, NCH₂Me), 3.87 (2H, s, COCH₂Cl), 1.87 (3H, s, 5-CH₃).
MASS (EI) m/z : 316(M⁺).

### (Example 670)

### Compound of Formula No. C-7

¹H-NMR (CDCl₃) δ(ppm) : 7.19 (1H, s, 3-H), 4.77 (2H, s, NCH₂Ar), 4.06 (2H, q, J=7Hz, NCH₂Me), 3.70 (2H, s, COCH₂Br), 1.91 (3H, s, 5-CH₃).
MASS (EI) m/z : 360(M⁺).

### (Example 671)

### Compound of Formula No. C-8

¹H-NMR (CDCl₃) δ(ppm) : 7.14 (1H, s, 3-H), 4.76 (2H, s, NCH₂Ar), 4.05 (2H, q, J=7Hz, NCH₂Me), 3.76 (2H, s, COCH₂O), 1.83 (3H, s, 5-CH₃).
MASS (EI) m/z : 312(M⁺), 297, 282, 267, 253, 226.

### (Example 672)

### Compound of Formula No. C-9

¹H-NMR (CDCl₃) δ(ppm) : 7.19 (1H, s, 3-H), 4.78 (2H, brs, NCH₂Ar), 4.37 (2H, brs, COCH₂O), 4.05 (2H, q, J=7Hz, NCH₂Me), 1.86 (3H, s, 5-CH₃).
MASS (EI) m/z : 374(M⁺), 281, 267, 253, 239, 152, 116.

### (Example 673)

### Compound of Formula No. C-10

¹H-NMR (CDCl₃) δ(ppm) : 7.15 (1H, s, 3-H), 4.83 (2H, s, NCH₂Ar), 4.05 (2H, q, J=7Hz, NCH₂Me), 3.77 (2H, s, COCH₂O), 1.86 (3H, s, 5-CH₃).
MASS (EI) m/z : 332(M⁺), 317, 302, 287, 273, 259.

### (Example 674)

### Compound of Formula No. C-11

¹H-NMR (CDCl₃) δ(ppm) : 7.21 (1H, s, 3-H), 4.86 (2H, s, NCH₂Ar), 4.39 (2H, brs, COCH₂O), 4.04 (2H, q, J=7Hz, NCH₂Me), 1.91 (3H, s, 5-CH₃).
MASS (EI) m/z : 394(M⁺), 301, 259, 152, 136.

### (Example 675)

### Compound of Formula No. C-12

¹H-NMR (CDCl₃) δ(ppm) : 7.18 (1H, s, 3-H), 4.75 (2H, brs, NCH₂Ar), 4.02 (2H, q, J=7Hz, NCH₂Me), 3.74 (2H, s, COCH₂O), 1.72 (3H, s, 5-CH₃).
MASS (EI) m/z : 371(M⁺), 356, 326, 312, 298, 196, 175, 166.

### (Example 676)

### Compound of Formula No. C-13

¹H-NMR (CDCl₃) δ(ppm) : 7.24 (1H, s, 3-H), 4.77 (2H, brs, NCH₂Ar), 4.36 (2H, brs, COCH₂O), 4.02 (2H, q, J=7Hz, NCH₂Me), 1.77 (3H, s, 5-CH₃).
MASS (EI) m/z : 433(M⁺), 340, 326, 312, 298, 258, 175.

### (Example 677)

### Compound of Formula No. C-14

¹H-NMR (CDCl₃) δ(ppm) : 7.16 (1H, s, 3-H), 4.76 (2H, brs, NCH₂Ar), 3.95 (2H, t, J=7Hz, NCH₂Et), 1.87 (3H, s, COCH₃), 1.81 (3H, s, 5-CH₃).
MASS (EI) m/z : 296(M⁺), 254, 180, 138, 124, 116.

### (Example 678)

### Compound of Formula No. C-15

¹H-NMR (CDCl₃) δ(ppm) : 7.24 (1H, s, 3-H), 4.78 (2H, brs, NCH₂Ar), 3.95 (2H, t, J=7Hz, NCH₂Et), 1.84 (3H, s, 5-CH₃), 1.51-1.39 (1H, m, COCH(CH₂)₂).
MASS (EI) m/z : 322(M⁺), 254, 206, 149, 138, 116.

### (Example 679)

### Compound of Formula No. C-16

¹H-NMR (CDCl₃) δ(ppm) : 7.19 (1H, s, 3-H), 4.76 (2H, brs, NCH₂Ar), 3.96 (2H, t, J=7Hz, NCH₂Et), 3.86 (2H, s, COCH₂Cl), 1.84 (3H, s, 5-CH₃).
MASS (EI) m/z : 330(M⁺).

### (Example 680)

### Compound of Formula No. C-17

¹H-NMR (CDCl₃) δ(ppm) : 7.20 (1H, s, 3-H), 4.76 (2H, brs, NCH₂Ar), 3.96 (2H, t, J=7Hz, NCH₂Et), 3.69 (2H, s, COCH₂Br), 1.88 (3H, s, 3-CH₃).
MASS (EI) m/z : 374(M⁺).

### (Example 681)

### Compound of Formula No. C-18

¹H-NMR (CDCl₃) δ(ppm) : 7.15 (1H, s, 3-H), 4.79 (2H, s, NCH₂Ar), 3.95 (2H, t, J=7Hz, NCH₂Et), 3.78 (2H, d, J=5Hz, COCH₂O), 1.79 (3H, s, 5-CH₃).
MASS (EI) m/z : 312(M⁺), 279, 265, 167, 138, 124, 116.

### (Example 682)

### Compound of Formula No. C-19

¹H-NMR (CDCl₃) δ(ppm) : 7.16 (1H, s, 3-H), 4.76 (2H, brs, NCH₂Ar), 4.02 (2H, t, J=7Hz, NCH₂Et), 3.75 (2H, s, COCH₂O), 1.79 (3H, s, 5-CH₃).
MASS (EI) m/z : 326(M⁺), 311, 281, 267, 253, 210, 180.

### (Example 683)

### Compound of Formula No. C-20

¹H-NMR (CDCl₃) δ(ppm) : 7.21 (1H, s, 3-H), 4.78 (2H, brs, NCH₂Ar), 4.36 (2H, s, COCH₂O), 3.96 (2H, t, J=7Hz, NCH₂Et), 1.83 (3H, s, 5-CH₃).
MASS (EI) m/z : 388(M⁺), 295, 281, 253, 166, 124, 116.

### (Example 684)

### Compound of Formula No. C-21

¹H-NMR (CDCl₃) δ(ppm) : 7.20 (1H, s, 3-H), 4.75 (2H, s, NCH₂Ar), 4.33 (2H, s, COCH₂O), 3.96 (2H, t, J=7Hz, NCH₂Et), 1.86 (3H, s, 5-CH₃).
MASS (EI) m/z : 354(M⁺), 311, 281, 252, 238, 210, 166.

### (Example 685)

### Compound of Formula No. C-22

¹H-NMR (CDCl₃) δ(ppm) : 7.15 (1H, brs, 3-H), 4.70 (2H, s, NCH₂Ar), 3.93 (2H, t, J=7Hz, NCH₂Et), 3.70 (3H, brs, COOCH₃), 1.84 (3H, s, 5-CH₃).
MASS (EI) m/z : 312(M⁺), 253, 196, 154, 116.

### (Example 686)

### Compound of Formula No. C-23

¹H-NMR (CDCl₃ δ(ppm) : 7.17 (1H, s, 3-H), 4.83 (2H, s, NCH₂Ar), 3.95 (2H, t, J=7Hz, NCH₂Et), 1.89 (3H, s, COCH₃), 1.86 (3H, s, 5-CH₃).
MASS (EI) m/z : 316(M⁺), 274, 180, 152, 138.

### (Example 687)

### Compound of Formula No. C-24

¹H-NMR (CDCl₃) δ(ppm) : 7.26 (1H, s, 3-H), 4.86 (2H, brs, NCH₂Ar), 3.95 (2H, t, J=7Hz, NCH₂Et), 1.89 (3H, s, 5-CH₃), 1.53-1.41 (1H, m, COCH(CH₂)₂).
MASS (EI) m/z : 342(M⁺), 274, 257, 227, 206, 138.

### (Example 688)

### Compound of Formula No. C-25

¹H-NMR (CDCl₃) δ(ppm) : 7.16 (1H, s, 3-H), 4.87 (2H, s, NCH₂Ar), 3.95 (2H, t, J=7Hz, NCH₂Et), 3.80 (2H, d, J=4Hz, COCH₂O), 1.91 (3H, s, 5-CH₃).
MASS (EI) m/z : 332(M⁺), 279, 265, 167, 149, 138.

### (Example 689)

### Compound of Formula No. C-26

¹H-NMR (CDCl₃) δ(ppm) : 7.17 (1H, s, 3-H), 4.85 (2H, brs, NCH₂Ar), 3.96 (2H, t, J=7Hz, NCH₂Et), 3.77 (2H, s, COCH₂O), 1.85 (3H, s, 5-CH₃).
MASS (EI) m/z 346(M⁺), 331, 301, 287, 273, 257, 227.

### (Example 690)

### Compound of Formula No. C-27

¹H-NMR (CDCl₃) δ(ppm) : 7.22 (1H, s, 3-H), 4.87 (2H, s, NCH₂Ar), 4.38 (2H, s, COCH₂O), 3.95 (2H, t, J=7Hz, NCH₂Et), 1.89 (3H, s, 5-CH₃).
MASS (EI) m/z : 408(M⁺), 315, 301, 287, 273, 178, 166, 149.

### (Example 691)

### Compound of Formula No. C-28

¹H-NMR (CDCl₃) δ(ppm) : 7.21 (1H, s, 3-H), 4.83 (2H, s, NCH₂Ar), 4.35 (2H, s, COCH₂O), 3.96 (2H, t, J=7Hz, NCH₂Et), 1.91 (3H, s, 5-CH₃).
MASS (EI) m/z : 374(M⁺), 331, 272, 257, 238, 166, 149.

### (Example 692)

### Compound of Formula No. C-29

¹H-NMR (CDCl₃) δ(ppm) : 7.17 (1H, s, 3-H), 4.79 (2H, s, NCH₂Ar), 3.92 (2H, t, J=7Hz, NCH₂Et), 3.71 (3H, brs, COOCH₃), 1.89 (3H, s, 5-CH₃).
MASS (EI) m/z : 332(M⁺), 273, 196, 154, 136, 122.

### (Example 693)

### Compound of Formula No. C-30

¹H-NMR (CDCl₃) δ(ppm) : 7.20 (1H, s, 3-H), 4.75 (2H, s, NCH₂Ar), 3.93 (2H, t, J=7Hz, NCH₂Et), 1.89 (3H, s, COCH₃), 1.74 (3H, s, 5-CH₃).
MASS (EI) m/z : 355(M⁺), 312, 180, 152, 138, 124.

### (Example 694)

### Compound of Formula No. C-31

¹H-NMR (CDCl₃) δ(ppm) : 7.27 (1H, s, 3-H), 4.74 (2H, brs, NCH₂Ar), 3.93 (2H, t, J=7Hz, NCH₂Et), 1.77 (3H, s, 5-CH₃), 1.51-1.38 (1H, m, COCH(CH₂)₂).
MASS (EI) m/z : 381(M⁺), 313, 236, 206, 175, 138.

### (Example 695)

### Compound of Formula No. C-32

¹H-NMR (CDCl₃) δ(ppm) : 7.19 (1H, s, 3-H), 4.78 (2H, brs, NCH₂Ar), 3.93 (2H, t, J=7Hz, NCH₂Et), 3.77 (2H, d, J=4Hz, COCH₂O), 1.72 (3H, s, 5-CH₃).
MASS (EI) m/z : 371(M⁺), 340, 312, 196, 175, 166, 124.

### (Example 696)

### Compound of Formula No. C-33

¹H-NMR (CDCl₃) δ(ppm) : 7.19 (1H, s, 3-H), 4.75 (2H, brs, NCH₂Ar), 3.93 (2H, t, J=7Hz, NCH₂Et), 3.74 (2H, s, COCH₂O), 1.71 (3H, s, 5-CH₃).
MASS (EI) m/z : 385(M⁺), 370, 340, 326, 312, 210, 182.

### (Example 697)

### Compound of Formula No. C-34

¹H-NMR (CDCl₃) δ(ppm) : 7.25 (1H, s, 3-H), 4.77 (2H, brs, NCH₂Ar), 4.36 (2H, brs, COCH₂O), 3.93 (2H, t, J=7Hz, NCH₂Et), 1.76 (3H, s, 5-CH₃).
MASS (EI) m/z : 447(M⁺), 354, 340, 326, 312, 272, 229, 175.

### (Example 698)

### Compound of Formula No. C-35

¹H-NMR (CDCl₃) δ(ppm) : 7.23 (1H, s, 3-H), 4.73 (2H, brs, NCH₂Ar), 4.33 (2H, s, COCH₂O), 3.93 (2H, t, J=7Hz, NCH₂Et), 1.79 (3H, s, 5-CH₃).
MASS (EI) m/z : 413(M⁺), 370, 340, 311, 238, 175, 166.

### (Example 699)

### Compound of Formula No. C-36

¹H-NMR (CDCl₃) δ(ppm) : 7.19 (1H, brs, 3-H), 4.69 (2H, s, NCH₂Ar), 3.91 (2H, t, J=7Hz, NCH₂Et), 3.69 (3H, brs, COOCH₃), 1.79 (3H, s, 5-CH₃).
MASS (EI) m/z : 371(M⁺), 342, 196, 175, 164, 109, 59.

### (Example 700)

### Compound of Formula No. C-37

¹H-NMR (CDCl₃) δ(ppm) : 7.18 (1H, s, 3-H), 4.75 (2H, brs, NCH₂Ar), 4.33 (1H, hept, J=7Hz, NCHMe₂), 1.87 (3H, s, COCH₃), 1.81 (3H, s, 5-CH₃).
MASS (EI) m/z : 296(M⁺), 254, 211, 180, 138, 124, 116.

### (Example 701)

### Compound of Formula No. C-38

¹H-NMR (CDCl₃) δ(ppm) : 7.26 (1H, s, 3-H), 4.78 (2H, brs, NCH₂Ar), 4.34 (1H, hept, J=7Hz, NCHMe₂), 1.85(3H, s, 5-CH₃), 1.51-1.37 (1H, m, COCH(CH₂)₂).
MASS (EI) m/z : 322(M⁺), 283, 254, 236, 206, 164, 138, 116.

### (Example 702)

### Compound of Formula No. C-39

¹H-NMR (CDCl₃) δ(ppm) : 7.21 (1H, s, 3-H), 4.76 (2H, brs, NCH₂Ar), 4.35 (1H, hept, J=7Hz, NCHMe₂), 3.86 (2H, s, COCH₂Cl), 1.84 (3H, s, 5-CH₃).
MASS (EI) m/z : 330(M⁺).

### (Example 703)

### Compound of Formula No. C-40

¹H-NMR (CDCl₃) δ(ppm) : 7.22 (1H, s, 3-H), 4.76 (2H, brs, NCH₂Ar), 4.35 (1H, hept, J=7Hz, NCHMe₂), 3.69 (2H, s, COCH₂Br), 1.86 (3H, s, 5-CH₃).
MASS (EI) m/z : 374(M⁺).

### (Example 704)

### Compound of Formula No. C-41

¹H-NMR (CDCl₃) δ(ppm) : 7.17 (1H, s, 3-H), 4.79 (2H, brs, NCH₂Ar), 4.33 (1H, hept, J=7Hz, NCHMe₂), 3.78 (2H, brd, J=4Hz, COCH₂O), 1.81 (3H, s, 5-CH₃).
MASS (EI) m/z : 312(M⁺), 253, 239, 211, 166, 138, 116.

### (Example 705)

### Compound of Formula No. C-42

¹H-NMR (CDCl₃) δ(ppm) : 7.17(1H, s, 3-H), 4.75 (2H, brs, NCH₂Ar), 4.33 (1H, hept, J=7Hz, NCHMe₂), 3.74 (2H, s, COCH₂O), 1.79 (3H, s, 5-CH₃).
MASS (EI) m/z : 326(M⁺), 311, 281, 267, 253, 239, 211, 180.

### (Example 706)

### Compound of Formula No. C-43

¹H-NMR (CDCl₃) δ(ppm) : 7.23 (1H, s, 3-H), 4.78 (2H, s, NCH₂Ar), 4.37 (2H, s, COCH₂O) , 4.33 (1H, hept, J=7Hz, NCHMe₂), 1.85 (3H, s, 5-CH₃).
MASS (EI) m/z : 388(M⁺), 295, 272, 253, 211, 166.

### (Example 707)

### Compound of Formula No. C-44

¹H-NMR (CDCl₃) δ(ppm) : 7.22 (1H, s, 3-H), 4.75 (2H, brs, NCH₂Ar), 4.35 (1H, hept, J=7Hz, NCHMe₂), 4.34 (2H, s, COCH₂O), 1.88 (3H, s, 5-CH₃).
MASS (EI) m/z : 354(M⁺), 252, 238, 211, 166, 138, 116.

### (Example 708)

### Compound of Formula No. C-45

¹H-NMR (CDCl₃) δ(ppm) : 7.17 (1H, brs, 3-H), 4.70 (2H, s, NCH₂Ar), 4.32 (1H, hept, J=7Hz; NCHMe₂), 3.70 (3H, brs, COOCH₃), 1.85 (3H, s, 5-CH₃).
MASS (EI) m/z : 312(M⁺), 297, 196, 154, 122, 116.

### (Example 709)

### Compound of Formula No. C-46

¹H-NMR (CDCl₃) δ(ppm) : 7.20 (1H, s, 3-H), 4.83 (2H, s, NCH₂Ar), 4.33 (1H, hept, J=7Hz, NCHMe₂), 1.88 (3H, s, COCH₃), 1.86 (3H, s, 5-CH₃).
MASS (EI) m/z : 316(M⁺), 274, 180, 138.

### (Example 710)

### Compound of Formula No. C-47

¹H-NMR (CDCl₃) δ(ppm) : 7.29 (1H, s, 3-H), 4.86 (2H, brs, NCH₂Ar), 4.36 (1H, hept, J=7Hz, NCHMe₂), 1.91 (3H, s, 5-CH₃), 1.54-1.36 (1H, m, COCH(CH₂)₂).
MASS (EI) m/z : 342(M⁺), 274, 257, 227, 206, 185, 149, 138.

### (Example 711)

### Compound of Formula No. C-48

¹H-NMR (CDCl₃) δ(ppm) : 7.18 (1H, s, 3-H), 4.87 (2H, brs, NCH₂Ar), 4.33 (1H, hept, J=7Hz, NCHMe₂), 3.79 (2H, d, J=4Hz, COCH₂O), 1.84 (3H, s, 5-CH₃).
MASS (EI) m/z : 332(M⁺), 301, 273, 259, 196, 166.

### (Example 712)

### Compound of Formula No. C-49

¹H-NMR (CDCl₃) δ(ppm) : 7.19 (1H, s, 3-H), 4.83 (2H, brs, NCH₂Ar), 4.33 (1H, hept, J=7Hz, NCHMe₂), 3.76 (2H, s, COCH₂O), 1.83 (3H, s, 5-CH₃).
MASS (EI) m/z : 346(M⁺), 331, 301, 273, 259, 231, 136.

### (Example 713)

### Compound of Formula No. C-50

¹H-NMR (CDCl₃) δ(ppm) : 7.25 (1H, s, 3-H), 4.86 (2H, brs, NCH₂Ar), 4.39 (2H, s, COCH₂O), 4.33 (1H, hept, J=7Hz, NCHMe₂), 1.89 (3H, s, 5-CH₃).
MASS (EI) m/z : 408(M⁺), 315, 287, 273, 259, 166.

### (Example 714)

### Compound of Formula No. C-51

¹H-NMR (CDCl₃) δ(ppm) : 7.24 (1H, s, 3-H), 4.82 (2H, brs, NCH₂Ar), 4.35 (2H, s, COCH₂O), 4.35 (1H, hept, J=7Hz, NCHMe₂), 1.91 (3H, s, 5-CH₃).
MASS (EI) m/z : 374(M⁺), 272, 257, 231, 185, 166, 136.

### (Example 715)

### Compound of Formula No. C-52

¹H-NMR (CDCl₃) δ(ppm) : 7.20 (1H, s, 3-H), 4.78 (2H, s, NCH₂Ar), 4.32 (1H, hept, J=7Hz, NCHMe₂), 3.71 (3H, brs, COOCH₃), 1.90 (3H, s, 5-CH₃).
MASS (EI) m/z : 332(M⁺), 196, 154, 136, 122.

### (Example 716)

### Compound of Formula No. C-53

¹H-NMR (CDCl₃) δ(ppm) : 7.20 (1H, s, 3-H), 4.75 (2H, s, NCH₂Ar), 4.30 (1H, hept, J=7Hz, NCHMe₂), 1.86 (3H, s, COCH₃), 1.74 (3H, s, 5-CH₃).
MASS (EI) m/z : 355(M⁺), 313, 270, 180, 149, 138, 124.

### (Example 717)

### Compound of Formula No. C-54

¹H-NMR (CDCl₃) δ(ppm) : 7.30 (1H, s, 3-H), 4.78 (2H, brs, NCH₂Ar), 4.32 (1H, hept, J=7Hz, NCHMe₂), 1.78 (3H, s, 5-CH₃), 1.53-1.36 (1H, m, COCH(CH₂)₂).
MASS (EI) m/z : 381(M⁺), 313, 270, 206, 193, 175, 164, 138.

### (Example 718)

### Compound of Formula No. C-55

¹H-NMR (CDCl₃) δ(ppm) : 7.21 (1H, s, 3-H), 4.78 (2H, brs, NCH₂Ar), 4.30 (1H, hept, J=7Hz, NCHMe₂), 3.76 (2H, brd, J=4Hz, COCH₂O), 1.72 (3H, s, 5-CH₃).
MASS (EI) m/z : 371(M⁺), 340, 312, 270, 196, 175, 166.

### (Example 719)

### Compound of Formula No. C-56

¹H-NMR (CDCl₃) δ(ppm) : 7.22 (1H, s, 3-H), 4.83 (2H, brs, NCH₂Ar), 4.30 (1H, hept, J=7Hz, NCHMe₂), 3.74 (2H, s, COCH₂O), 1.71 (3H, s, 5-CH₃).
MASS (EI) m/z : 385(M⁺), 370, 340, 312, 270, 210, 175, 124.

### (Example 720)

### Compound of Formula No. C-57

¹H-NMR (CDCl₃) δ(ppm) : 7.27 (1H, s, 3-H), 4.75 (2H, brs, NCH₂Ar), 4.36 (2H, s, COCH₂O), 4.30 (1H, hept, J=7Hz, NCHMe₂), 1.76 (3H, s, 5-CH₃).
MASS (EI) m/z : 447(M⁺), 354, 312, 272, 175, 166.

### (Example 721)

### Compound of Formula No. C-58

¹H-NMR (CDCl₃) δ(ppm) : 7.26 (1H, s, 3-H), 4.73 (2H, brs, NCH₂Ar), 4.33 (2H, s, COCH₂O), 4.31 (1H, hept, J=7Hz, NCHMe₂), 1.78 (3H, s, 5-CH₃).
MASS (EI) m/z : 413(M⁺), 311, 270, 238, 175, 166, 124.

### (Example 722)

### Compound of Formula No. C-59

¹H-NMR (CDCl₃) δ(ppm) : 7.21 (1H, brs, 3-H), 4.68 (2H, s, NCH₂Ar), 4.30 (1H, hept, J=7Hz, NCHMe₂), 3.69 (3H, brs, COOCH₃), 1.79 (3H, s, 5-CH₃).
MASS (EI) m/z : 371(M⁺), 196, 175, 154.

### (Example 723)

### Compound of Formula No. C-60

¹H-NMR (CDCl₃) δ(ppm) : 7.15 (1H, s, 3-H), 4.75 (2H, s, NCH₂Ar), 3.98 (2H, t, J=7Hz, NCH₂-Pr), 1.86 (3H, s, COCH₃), 1.81 (3H, s, 5-CH₃).
MASS (EI) m/z : 310(M⁺), 267, 194, 152, 116.

### (Example 724)

### Compound of Formula No. C-61

¹H-NMR (CDCl₃) δ(ppm) : 7.24 (1H, s, 3-H), 4.78 (2H, brs, NCH₂Ar), 3.99 (2H, t, J=7Hz, NCH₂-Pr), 1.84 (3H, s, 5-CH₃), 1.51-1.38 (1H, m, COCH(CH₂)₂).
MASS (EI) m/z : 336(M⁺), 268, 220, 152, 116.

### (Example 725)

### Compound of Formula No. C-62

¹H-NMR (CDCl₃) δ(ppm) : 7.14 (1H, s, 3-H), 4.79 (2H, brs, NCH₂Ar), 3.98 (2H, t, J=7Hz, NCH₂-Pr), 3.78 (2H, d, J=5Hz, COCH₂O), 1.79 (3H, s, 5-CH₃).
MASS (EI) m/z : 326(M⁺), 295, 267, 210, 180.

### (Example 726)

### Compound of Formula No. C-63

¹H-NMR (CDCl₃) δ(ppm) : 7.15 (1H, s, 3-H), 4.76 (2H, brs, NCH₂Ar), 3.98 (2H, t, J=7Hz, NCH₂-Pr), 3.74 (2H, s, COCH₂O), 1.78 (3H, s, 5-CH₃).
MASS (EI) m/z : 340(M⁺), 325, 267, 224, 136, 116.

### (Example 727)

### Compound of Formula No. C-64

¹H-NMR (CDCl₃) δ(ppm) : 7.20 (1H, s, 3-H), 4.78 (2H, brs, NCH₂Ar), 4.36 (2H, s, COCH₂O), 3.98 (2H, t, J=7Hz, NCH₂-Pr), 1.83 (3H, s, 5-CH₃).
MASS (EI) m/z : 402(M⁺), 309, 267, 136, 116.

### (Example 728)

### Compound of Formula No. C-65

¹H-NMR (CDCl₃) δ(ppm) : 7.20 (1H, s, 3-H), 4.75 (2H, brs, NCH₂Ar), 4.33 (2H, s, COCH₂O), 3.99 (2H, t, J=7Hz, NCH₂-Pr), 1.86 (3H, s, 5-CH₃).
MASS (EI) m/z : 368(M⁺), 325, 266, 210, 180, 116.

### (Example 729)

### Compound of Formula No. C-66

¹H-NMR (CDCl₃) δ(ppm) : 7.15 (1H, s, 3-H), 4.70 (2H, s, NCH₂Ar), 3.96 (2H, t, J=7Hz, NCH₂-Pr), 3.70 (3H, brs, COOCH₃), 1.84 (3H, s, 5-CH₃).
MASS (EI) m/z : 326(M⁺), 283, 267, 210, 168, 116.

### (Example 730)

### Compound of Formula No. C-67

¹H-NMR (CDCl₃) δ(ppm) : 7.17 (1H, s, 3-H), 4.83 (2H, brs, NCH₂Ar), 3.98 (2H, t, J=7Hz, NCH₂-Pr), 1.89 (3H, s, COCH₃), 1.86 (3H, s, 5-CH₃).
MASS (EI) m/z : 330(M⁺), 287, 271, 194, 152, 136.

### (Example 731)

### Compound of Formula No. C-68

¹H-NMR (CDCl₃) δ(ppm) : 7.25 (1H, s, 3-H), 4.86 (2H, s, NCH₂Ar), 3.98 (2H, t, J=7Hz, NCH₂-Pr), 1.89 (3H, s, 5-CH₃), 1.51-1.43 (1H, m, COCH(CH₂)₂).
MASS (EI) m/z : 356(M⁺), 288, 220, 152, 136.

### (Example 732)

### Compound of Formula No. C-69

¹H-NMR (CDCl₃) δ(ppm) : 7.15 (1H, s, 3-H), 4.87 (2H, brs, NCH₂Ar), 3.98 (2H, t, J=7Hz, NCH₂-Pr), 3.79 (2H, d, J=5Hz, COCH₂O), 1.84 (3H, s, 5-CH₃).
MASS (EI) m/z : 346(M⁺), 315, 287, 180, 152, 136.

### (Example 733)

### Compound of Formula No. C-70

¹H-NMR (CDCl₃) δ(ppm) : 7.16 (1H, s, 3-H), 4.84 (2H, brs, NCH₂Ar), 3.98 (2H, t, J=7Hz, NCH₂-Pr), 3.76 (2H, s, COCH₂O), 1.83 (3H, s, 5-CH₃).
MASS (EI) m/z : 360(M⁺), 345, 315, 287, 224, 136.

### (Example 734)

### Compound of Formula No. C-71

¹H-NMR (CDCl₃) δ(ppm) : 7.22 (1H, s, 3-H), 4.87 (2H, brs, NCH₂Ar), 4.38 (2H, s, COCH₂O), 3.98 (2H, t, J=7Hz, NCH₂-Pr), 1.88 (3H, s, 5-CH₃).
MASS (El) m/z ; 422(M⁺), 329, 315, 287, 136, 107.

### (Example 735)

### Compound of Formula No. C-72

¹H-NMR (CDCl₃) δ(ppm) : 7.21 (1H, s, 3-H), 4.83 (2H, brs, NCH₂Ar), 4.35 (2H, s, COCH₂O), 3.98 (2H, t, J=7Hz, NCH₂-Pr), 1.91 (3H, s, 5-CH₃).
MASS (EI) m/z : 388(M⁺), 345, 286, 252, 180, 136.

### (Example 736)

### Compound of Formula No. C-73

¹H-NMR (CDCl₃) δ(ppm) : 7.17 (1H, s, 3-H), 4.79 (2H, s, NCH₂Ar), 3.95 (2H, t, J=7Hz, NCH₂-Pr), 3.71 (3H, s, COOCH₃), 1.89 (3H, s, 5-CH₃).
MASS (EI) m/z : 346(M⁺), 287, 210, 168, 154, 136.

### (Example 737)

### Compound of Formula No. C-74

¹H-NMR (CDCl₃) δ(ppm) : 7.19 (1H, s, 3-H), 4.74 (2H, brs, NCH₂Ar), 3.95 (2H, t, J=7Hz, NCH₂-Pr), 1.86 (3H, s, COCH₃), 1.73 (3H, s, 5-CH₃).
MASS (EI) m/z : 369(M⁺), 327, 194, 175, 152.

### (Example 738)

### Compound of Formula No. C-75

¹H-NMR (CDCl₃) δ(ppm) : 7.26 (1H, s, 3-H), 4.70 (2H, brs, NCH₂Ar), 3.96 (2H, t, J=7Hz, NCH₂-Pr), 1.77 (3H, s, 5-CH₃), 1.51-1.38 (1H, m, COCH(CH₂)₂).
MASS (EI) m/z : 395(M⁺), 327, 220, 175, 152, 138.

### (Example 739)

### Compound of Formula No. C-76

¹H-NMR (CDCl₃) δ(ppm) : 7.18 (1H, s, 3-H), 4.78 (2H, brs, NCH₂Ar), 3.96 (2H, t, J=7Hz, NCH₂-Pr), 3.77 (2H, d, J=5Hz, COCH₂O), 1.71 (3H, s, 5-CH₃).
MASS (EI) m/z : 385(M⁺), 326, 180, 175, 152.

### (Example 740)

### Compound of Formula No. C-77

¹H-NMR (CDCl₃) δ(ppm) : 7.18 (1H, s, 3-H), 4.76 (2H, brs, NCH₂Ar), 3.95 (2H, t, J=7Hz, NCH₂-Pr), 3.73 (2H, s, COCH₂O), 1.70 (3H, s, 5-CH₃).
MASS (EI) m/z : 399(M⁺), 354, 326, 224, 175, 138.

### (Example 741)

### Compound of Formula No. C-78

¹H-NMR (CDCl₃) δ(ppm) : 7.24 (1H, s, 3-H), 4.78 (2H, brs, NCH₂Ar), 4.35 (2H, s, COCH₂O), 3.96 (2H, t, J=7Hz, NCH₂-Pr), 1.75 (3H, s, 5-CH₃).
MASS (EI) m/z : 461(M⁺), 368, 326, 286, 175, 107.

### (Example 742)

### Compound of Formula No. C-79

¹H-NMR (CDCl₃) δ(ppm) : 7.23 (1H, s, 3-H), 4.75 (2H, brs, NCH₂Ar), 4.33 (2H, s, COCH₂O), 3.96 (2H, t, J=7Hz, NCH₂-Pr), 1.78 (3H, s, 5-CH₃).
MASS (EI) m/z : 427(M⁺), 384, 325, 252, 180, 152.

### (Example 743)

### Compound of Formula No. C-80

¹H-NMR (CDCl₃) δ(ppm) : 7.18 (1H, s, 3-H), 4.69 (2H, s, NCH₂Ar), 3.94 (2H, t, J=7Hz, NCH₂-Pr), 3.70 (3H, brs, COOCH₃), 1.84 (3H, s, 5-CH₃).
MASS (EI) m/z : 385(M⁺), 342, 210, 168, 154, 136.

### (Example 744)

### Compound of Formula No. C-81

¹H-NMR (CDCl₃) δ(ppm) : 7.18 (1H, s, 3-H), 4.76 (2H, brs, NCH₂Ar), 3.77 (2H, d, J=7Hz, NCH₂-iPr), 1.87 (3H, s, COCH₃), 1.78 (3H, s, 5-CH₃).
MASS (EI) m/z : 310(M⁺), 295, 267, 194, 152, 116.

### (Example 745)

### Compound of Formula No. C-82

¹H-NMR (CDCl₃) δ(ppm) : 7.16 (1H, s, 3-H), 4.76 (2H, brs, NCH₂Ar), 3.77 (2H, d, J=7Hz, NCH₂-iPr), 2.06 (2H, q, J=7Hz, COCH₂Me), 1.76 (3H, s, 5-CH₃).
MASS (EI) m/z : 324(M⁺), 268, 208, 152, 116.

### (Example 746)

### Compound of Formula No. C-84

¹H-NMR (CDCl₃) δ(ppm) : 7.20 (1H, s, 3-H), 4.78 (2H, brs, NCH₂Ar), 3.86 (2H, s, COCH₂Cl), 3.78 (2H, d, J=7Hz, NCH₂-iPr), 1.81 (3H, s, 5-CH₃).
MASS (EI) m/z : 344(M⁺), 267, 228, 200, 179, 136, 116.

### (Example 747)

### Compound of Formula No. C-85

¹H-NMR (CDCl₃) δ(ppm) : 7.16 (1H, s, 3-H), 4.80 (2H, brs, NCH₂Ar), 3.78 (2H, d, J=5Hz, COCH₂O), 3.77 (2H, d, J=7Hz, NCH₂-iPr), 1.77 (3H, s, 5-CH₃).
MASS (EI) m/z : 326(M⁺), 310, 267, 180, 152, 116.

### (Example 748)

### Compound of Formula No. C-86

¹H-NMR (CDCl₃) δ(ppm) : 7.19 (1H, s, 3-H), 5.04 (1H, d, J=14Hz, NCH₂Ar), 4.52 (1H, d, J=14Hz, NCH₂Ar), 4.10-3.98 (1H, m, COCHMeO), 3.78 (2H, d, J=7Hz, NCH₂-iPr), 1.77 (3H, brs, 5-CH₃).
MASS (EI) m/z : 340(M⁺), 325, 295, 268, 224, 180, 116.

### (Example 749)

### Compound of Formula No. C-87

¹H-NMR (CDCl₃) δ(ppm) : 7.14 (1H, s, 3-H), 4.83 (1H, d, J=14Hz, NCH₂Ar), 4.70 (1H, d, J=14Hz, NCH₂Ar), 3.78 (2H, d, J=7Hz, NCH₂-iPr), 1.81 (3H, s, 5-CH₃), 1.32 (3H, brs, COC(CH₃)₂O), 1.10 (3H, brs, COC(CH₃)₂O).
MASS (EI) m/z : 354(M⁺), 339, 296, 268, 225, 180, 116.

### (Example 750)

### Compound of Formula No. C-88

¹H-NMR (CDCl₃) δ(ppm) : 7.17 (1H, s, 3-H), 4.77 (2H, brs, NCH₂Ar), 3.77 (2H, d, J=7Hz, NCH₂-iPr), 3.74 (2H, s, COCH₂O), 1.76 (3H, s, 5-CH₃).
MASS (EI) m/z : 340(M⁺), 325, 267, 224, 152, 138, 116.

### (Example 751)

### Compound of Formula No. C-89

¹H-NMR (CDCl₃) δ(ppm) : 7.22 (1H, s, 3-H), 4.79 (2H, brs, NCH₂Ar), 4.36 (2H, s, COCH₂O), 3.78 (2H, d, J=7Hz, NCH₂-iPr), 1.80 (3H, s, 5-CH₃).
MASS (EI) m/z : 402(M⁺), 295, 267, 253, 136, 116.

### (Example 752)

### Compound of Formula No. C-90

¹H-NMR (CDCl₃) δ(ppm) : 7.22 (1H, s, 3-H), 4.76 (2H, brs, NCH₂Ar), 4.33 (2H, s, COCH₂O), 3.78 (2H, d, J=7Hz, NCH₂-iPr), 1.81 (3H, s, 5-CH₃).
MASS (EI) m/z : 368(M⁺), 325, 266, 252, 210, 180, 116.

### (Example 753)

### Compound of Formula No. C-91

¹H-NMR (CDCl₃) δ(ppm) : 7.20 (1H, brs, 3-H), 4.94 (1H, d, J=14Hz, NCH₂Ar), 4.88-4.74 (1H, m, COCHMeO), 4.56 (1H, d, J=14Hz, NCH₂Ar), 3.77 (2H, d, J=7Hz, NCH₂-iPr), 1.89 (3H, s, 5-CH₃).
MASS (EI) m/z : 382(M⁺), 339, 295, 268, 210, 180, 116.

### (Example 754)

### Compound of Formula No. C-92

¹H-NMR (CDCl₃) δ(ppm) : 7.06 (1H, s, 3-H), 4.89 (1H, d, J=14Hz, NCH₂Ar), 4.58 (1H, d, J=14Hz, NCH₂Ar), 3.76 (2H, d, J=7Hz, NCH₂-iPr), 1.87 (3H, s, 5-CH₃), 1.45 (6H, brs, COC(CH₃)₂O).
MASS (EI) m/z : 396(M⁺), 295, 268, 225, 180, 129.

### (Example 755)

### Compound of Formula No. C-93

¹H-NMR (CDCl₃) δ(ppm) : 7.17 (1H, s, 3-H), 4.71 (2H, s, NCH₂Ar), 3.75 (2H, d, J=7Hz, NCH₂-iPr), 3.70 (3H, brs, COOCH₃), 1.80 (3H, s, 5-CH₃).
MASS (EI) m/z : 326(M⁺), 310, 261, 234, 210, 188, 149.

### (Example 756)

### Compound of Formula No. C-94

¹H-NMR (CDCl₃) δ(ppm) : 7.17 (1H, s, 3-H), 4.71 (2H, s, NCH₂Ar), 4.15 (2H, brq, J=7Hz, COOCH₂Me), 3.75 (2H, d, J=7Hz, NCH₂-iPr), 1.81 (3H, s, 5-CH₃).
MASS (EI) m/z : 340(M⁺), 325, 284, 267, 224, 152, 116.

### (Example 757)

### Compound of Formula No. C-95

¹H-NMR (CDCl₃) δ(ppm) : 7.17 (1H, s, 3-H), 4.96 (1H, hept, J=7Hz, COOCHMe₂), 4.70 (2H, s, NCH₂Ar), 3.75 (2H, d, J=7Hz, NCH₂-iPr), 1.81 (3H, s, 5-CH₃).
MASS (EI) m/z : 354(M⁺), 312, 267, 256, 196, 116.

### (Example 758)

### Compound of Formula No. C-96

¹H-NMR (CDCl₃) δ(ppm) : 7.29 (1H, s, 3-H), 4.77 (2H, brs, NCH₂Ar), 3.76 (2H, d, J=7Hz, NCH₂-iPr), 1.89 (3H, s, 5-CH₃).
MASS (EI) m/z : 388(M⁺), 295, 239, 136, 116.

### (Example 759)

### Compound of Formula No. C-98

¹H-NMR (CDCl₃) δ(ppm) : 7.20 (1H, s, 3-H), 4.73 (2H, s, NCH₂Ar), 4.42 (1H, q, J=5Hz, CONHMe), 3.77 (2H, d, J=7Hz, NCH₂-iPr), 2.77 (3H, d, J=5Hz, CONHCH₃), 1.80 (3H, s, 5-CH₃).
MASS (EI) m/z : 325(M⁺), 268, 209, 152, 116.

### (Example 760)

### Compound of Formula No. C-99

¹H-NMR (CDCl₃) δ(ppm) : 7.21 (1H, s, 3-H), 4.73 (2H, s, NCH₂Ar), 4.40 (1H, t, J=7Hz, CONHEt), 3.77 (2H, d, J=7Hz, NCH₂-iPr), 3.62 (2H, qd, J=7Hz, 6Hz, CONHCH₂Me), 1.80 (3H, s, 5-CH₃).
MASS (EI) m/z : 339(M+), 268, 223, 212, 152, 116.

### (Example 761)

### Compound of Formula No. C-100

¹H-NMR (CDCl₃) δ(ppm) : 7.26 (1H, s, 3-H), 6.42 (1H, s, CONHPh), 4.80 (2H, s, NCH₂Ar), 3.82 (2H, d, J=7Hz, NCH₂-iPr), 1.86 (3H, s, 5-CH₃).
MASS (EI) m/z : 387(M⁺), 268, 212, 149, 116.

### (Example 762)

### Compound of Formula No. C-101

¹H-NMR (CDCl₃) δ(ppm) : 7.23 (1H, s, 3-H), 4.63 (2H, s, NCH₂Ar), 3.75 (2H, d, J=7Hz, NCH₂-iPr), 3.23 (4H, t, J=5Hz, CON(CH₂CH₂)₂O), 1.78 (3H, s, 5-CH₃).
MASS (EI) m/z : 381(M⁺), 265, 180, 149, 136, 114.

### (Example 763)

### Compound of Formula No. C-102

¹H-NMR (CDCl₃) δ(ppm) : 7.28 (1H, s, 3-H), 4.87 (2H, brs, NCH₂Ar), 3.78 (2H, d, J=7Hz, NCH₂-iPr), 1.87 (3H, s, 5-CH₃), 1.55-1.41 (1H, m, COCH(CH₂)₂).
MASS (EI) m/z : 356(M⁺), 288, 271, 220, 152, 136.

### (Example 764)

### Compound of Formula No. C-103

¹H-NMR (CDCl₃) δ(ppm) : 7.27 (1H, s, 3-H), 4.88 (2H, s, NCH₂Ar), 3.81 (2H, brs, COCH₂O), 3.77 (2H, d, J=7Hz, NCH₂-iPr), 1.82 (3H, s, 5-CH₃).
MASS (EI) m/z : 346(M⁺), 303, 287, 271, 210, 180, 168.

### (Example 765)

### Compound of Formula No. C-104

¹H-NMR (CDCl₃) δ(ppm) : 7.18 (1H, s, 3-H), 4.85 (2H, brs, NCH₂Ar), 3.77 (2H, d, J=7Hz, NCH₂-iPr), 3.76 (2H, s, COCH₂O), 1.82 (3H, s, 5-CH₃).
MASS (EI) m/z : 360(M⁺), 345, 315, 287, 259, 224, 196, 136.

### (Example 766)

### Compound of Formula No. C-105

¹H-NMR (CDCl₃) δ(ppm) : 7.21 (1H, s, 3-H), 4.85 (2H, s, NCH₂Ar), 4.36 (2H, brs, COCH₂O), 3.75 (2H, d, J=7Hz, NCH₂-iPr), 1.84 (3H, s, 5-CH₃).
MASS (EI) m/z : 422(M⁺), 329, 301, 287, 273, 245, 136

### (Example 767)

### Compound of Formula No. C-106

¹H-NMR (CDCl₃) δ(ppm) : 7.23 (1H, s, 3-H), 4.84 (2H, brs, NCH₂Ar), 4.35 (2H, s, COCH₂O), 3.78 (2H, d, J=7Hz, NCH₂-iPr), 1.89 (3H, s, 5-CH₃).
MASS (EI) m/z : 388(M⁺), 345, 286, 271, 252, 230, 210.

### (Example 768)

### Compound of Formula No. C-107

¹H-NMR (CDCl₃) δ(ppm) : 7.32 (1H, s, 3-H), 4.87 (2H, brs, NCH₂Ar), 3.77 (2H, d, J=7Hz, NCH₂-iPr), 1.96 (3H, s, 5-CH₃).
MASS (EI) m/z : 408(M⁺), 315, 259, 136.

### (Example 769)

### Compound of Formula No. C-108

¹H-NMR (CDCl₃) δ(ppm) : 7.28 (1H, s, 3-H), 4.75 (2H, brs, NCH₂Ar), 3.75 (2H, d, J=7Hz, NCH₂-iPr), 1.75 (3H, s, 5-CH₃), 1.50-1.38 (1H, m, COCH(CH₂)₂).
MASS (EI) m/z : 395(M⁺), 327, 271, 220, 175, 164, 152.

### (Example 770)

### Compound of Formula No. C-109

¹H-NMR (CDCl₃) δ(ppm) : 7.20 (1H, s, 3-H), 4.78 (2H, brs, NCH₂Ar), 3.77 (2H, brs, COCH₂O), 3.75 (2H, d, J=7Hz, NCH₂-iPr), 1.70 (3H, s, 5-CH₃).
MASS (EI) m/z : 385(M⁺), 354, 326, 270, 175, 152.

### (Example 771)

### Compound of Formula No. C-110

¹H-NMR (CDCl₃) δ(ppm) : 7.20 (1H, s, 3-H), 4.74 (2H, brs, NCH₂Ar), 3.75 (2H, d, J=7Hz, NCH₂-ipr), 3.73 (2H, s, COCH₂O), 1.69 (3H, s, 5-CH₃).
MASS (EI) m/z : 399(M⁺), 384, 354, 340, 326, 224, 138.

### (Example 772)

### Compound of Formula No. C-111

¹H-NMR (CDCl₃) δ(ppm) : 7.24 (1H, s, 3-H), 4.70 (2H, brs, NCH₂Ar), 4.33 (2H, s, COCH₂O), 3.76 (2H, d, J=7Hz, NCH₂-iPr), 1.77 (3H, s, 5-CH₃).
MASS (EI) m/z : 427(M⁺), 384, 325, 269, 252, 180, 152.

### (Example 773)

### Compound of Formula No. C-112

¹H-NMR (CDCl₃) δ(ppm) : 7.10 (1H, s, 3-H), 4.74 (2H, s, NCH₂Ar), 1.96 (3H, s, 5-CH₃), 1.86 (3H, s, COCH₃), 1.60 (9H, s, NC(CH₃)₃).
MASS (EI) m/z : 310(M⁺), 254, 212, 149, 116.

### (Example 774)

### Compound of Formula No. C-113

¹H-NMR (CDCl₃) δ(ppm) : 7.18 (1H, s, 3-H), 4.78 (1H, brs, NCH₂Ar), 4.75 (1H, brs, NCH₂Ar), 1.99 (3H, s, 5-CH₃), 1.61 (9H, s, NC(CH₃)₃), 1.49-1.37 (1H, m, COCH(CH₂)₂).
MASS (EI) m/z : 336(M⁺), 268, 212, 164, 116.

### (Example 775)

### Compound of Formula No. C-114

¹H-NMR (CDCl₃) δ(ppm) : 7.08 (1H, s, 3-H), 4.78 (2H, s, NCH₂Ar), 3.76 (2H, brs, COCH₂O), 1.95 (3H, s, 5-CH₃), 1.60 (9H, s, NC(CH₃)₃).
MASS (EI) m/z : 402(M⁺), 346, 253, 225, 211, 116.

### (Example 776)

### Compound of Formula No. C-115

¹H-NMR (CDCl₃) δ(ppm) : 7.09 (1H, s, 3-H), 4.75 (2H, s, NCH₂Ar), 3.76 (1H, brs, COCH₂O), 3.69 (1H, brs, OCH₂CO), 1.94 (3H, s, 5-CH₃), 1.60 (9H, s, NC(CH₃)₃).
MASS (EI) m/z : 340(M⁺), 284, 254, 225, 211, 116.

### (Example 777)

### Compound of Formula No. C-116

¹H-NMR (CDCl₃) δ(ppm) : 7.14 (1H, s, 3-H), 4.78 (2H, s, NCH₂Ar), 4.39 (1H, brs, COCH₂O), 4.31 (1H, brs, COCH₂O), 1.99 (3H, s, 5-CH₃), 1.59 (9H, s, NC(CH₃)₃).
MASS (EI) m/z : 402(M⁺), 346, 253, 225, 211, 116.

### (Example 778)

### Compound of Formula No. C-117

¹H-NMR (CDCl₃) δ(ppm) : 7.13 (1H, s, 3-H), 4.74 (2H, s, NCH₂Ar), 4.32 (2H, s, COCH₂O), 2.02 (3H, s, 5-CH₃), 1.59 (9H, s, NC(CH₃)₃).
MASS (EI) m/z : 368(M⁺), 266, 210, 149, 116.

### (Example 779)

### Compound of Formula No. C-118

¹H-NMR (CDCl₃) δ(ppm) : 7.10 (1H, s, 3-H), 4.68 (2H, s, NCH₂Ar), 3.69 (3H, s, COOCH₃), 1.97 (3H, s, 5-CH₃), 1.59 (9H, s, NC(CH₃)₃).
MASS (EI) m/z : 326(M⁺), 270, 211, 154, 116.

### (Example 780)

### Compound of Formula No. C-119

¹H-NMR (CDCl₃) δ(ppm) : 7.15 (1H, s, 3-H), 4.75 (2H, brs, NCH₂Ar), 3.98 (2H, d, J=7Hz, NCH₂-cBu), 1.86 (3H, s, COCH₃), 1.79 (3H, s, 5-CH₃).
MASS (EI) m/z : 322(M⁺), 279, 254, 212, 206, 178, 164, 150.

### (Example 781)

### Compound of Formula No. C-120

¹H-NMR (CDCl₃) δ(ppm) : 7.23 (1H, s, 3-H), 4.76 (2H, brs, NCH₂Ar), 3.99 (2H, d, J=7Hz, NCH₂-cBu), 1.83 (3H, s, 5-CH₃), 1.50-1.40 (1H, m, COCH(CH₂)₂).
MASS (EI) m/z : 348(M⁺), 280, 232, 212, 164, 150, 116.

### (Example 782)

### Compound of Formula No. C-121

¹H-NMR (CDCl₃) δ(ppm) : 7.13 (1H, s, 3-H), 4.79 (2H, brs, NCH₂Ar), 3.99 (2H, d, J=7Hz, NCH₂-cBu), 3.77 (2H, d, J=5Hz, COCH₂O), 1.78 (3H, s, 5-CH₃).
MASS (EI) m/z : 338(M⁺), 323, 279, 239, 222, 211, 192.

### (Example 783)

### Compound of Formula No. C-122

¹H-NMR (CDCl₃) δ(ppm) : 7.14 (1H, s, 3-H), 4.76 (2H, brs, NCH₂Ar), 3.99 (2H, d, J=7Hz, NCH₂-cBu), 3.74 (2H, s, COCH₂O), 1.77 (3H, s, 5-CH₃).
MASS (EI) m/z : 352(M⁺), 337, 322, 307, 279, 236, 225, 211.

### (Example 784)

### Compound of Formula No. C-123

¹H-NMR (CDCl₃) δ(ppm) : 7.19 (1H, s, 3-H), 4.78 (2H, brs, NCH₂Ar), 4.38 (2H, s, COCH₂O), 3.99 (2H, d, J=7Hz, NCH₂-cBu), 1.82 (3H, s, 5-CH₃).
MASS (EI) m/z : 414(M⁺), 399, 346, 321, 298, 279, 253, 211.

### (Example 785)

### Compound of Formula No. C-124

¹H-NMR (CDCl₃) δ(ppm) : 7.19 (1H, s, 3-H), 4.75 (2H, brs, NCH₂Ar), 4.32 (2H, s, COCH₂O), 3.99 (2H, d, J=7Hz, NCH₂-cBu), 1.85 (3H, s, 5-CH₃).
MASS (EI) m/z : 380(M⁺), 365, 337, 278, 264, 250, 210, 192.

### (Example 786)

### Compound of Formula No. C-125

¹H-NMR (CDCl₃) δ(ppm) : 7.15 (1H, s, 3-H), 4.70 (2H, brs, NCH₂Ar), 3.96 (2H, d, J=7Hz, NCH₂-cBu), 3.70 (3H, brs, COOCH₃), 1.82 (3H, s, 5-CH₃).
MASS (EI) m/z : 338(M⁺), 323, 310, 279, 270, 211, 194.

### (Example 787)

### Compound of Formula No. C-126

¹H-NMR (CDCl₃) δ(ppm) : 7.26 (1H, s, 3-H), 4.78 (2H, brs, NCH₂Ar), 3.98 (2H, d, J=7Hz, NCH₂-cBu), 1.92 (3H, s, 5-CH₃).
MASS (EI) m/z : 400(M⁺), 307, 239, 136, 116.

### (Example 788)

### Compound of Formula No. C-127

¹H-NMR (CDCl₃) δ(ppm) : 7.16 (1H, s, 3-H), 4.83 (2H, s, NCH₂Ar), 4.12 (2H, d, J=7Hz, NCH₂-cBu), 1.87 (3H, s, 5-CH₃), 1.84 (3H, s, COCH₃).
MASS (EI) m/z : 342(M⁺), 300, 232, 206, 164, 136.

### (Example 789)

### Compound of Formula No. C-128

¹H-NMR (CDCl₃) δ(ppm) : 7.25 (1H, s, 3-H), 4.86 (2H, brs, NCH₂Ar), 3.99 (2H, d, J=7Hz, NCH₂-cBu), 1.89 (3H, s, 5-CH₃), 1.52-1.40 (1H, m, COCH(CH₂)₂).
MASS (EI) m/z : 368(M⁺), 300, 283, 232, 164, 150, 136.

### (Example 790)

### Compound of Formula No. C-129

¹H-NMR (CDCl₃) δ(ppm) : 7.14 (1H, s, 3-H), 4.87 (2H, brs, NCH₂Ar), 3.98 (2H, d, J=7Hz, NCH₂-cBu), 3.78 (2H, d, J=5Hz, COCH₂O), 1.82 (3H, s, 5-CH₃).
MASS (EI) m/z : 358(M⁺), 222, 192, 154, 136.

### (Example 791)

### Compound of Formula No. C-130

¹H-NMR (CDCl₃) δ(ppm) : 7.15 (1H, s, 3-H), 4.84 (2H, brs, NCH₂Ar), 3.99 (2H, d, J=7Hz, NCH₂-cBu), 3.76 (2H, s, COCH₂O), 1.83 (3H, s, 5-CH₃).
MASS (EI) m/z : 372(M⁺), 357, 327, 299, 259, 236, 208.

### (Example 792)

### Compound of Formula No. C-131

¹H-NMR (CDCl₃) δ(ppm) : 7.20 (1H, s, 3-H), 4.86 (2H, s, NCH₂Ar), 4.37 (2H, s, COCH₂O), 3.98 (2H, d, J=7Hz, NCH₂-cBu), 1.87(3H, s, 5-CH₃).
MASS (EI) m/z : 434(M⁺), 341, 313, 299, 273, 245, 166.

### (Example 793)

### Compound of Formula No. C-132

¹H-NMR (CDCl₃) δ(ppm) : 7.21 (1H, s, 3-H), 4.84 (2H, s, NCH₂Ar), 4.35 (2H, s, COCH₂O), 4.00 (2H, d, J=7Hz, NCH₂-cBu), 1.91 (3H, s, 5-CH₃).
MASS (El) m/z : 400(M⁺), 357, 298, 264, 230, 214, 192.

### (Example 794)

### Compound of Formula No. C-133

¹H-NMR (CDCl₃) δ(ppm) : 7.17 (1H, s, 3-H), 4.79 (2H, s, NCH₂Ar), 3.97 (2H, d, J=7Hz, NCH₂-cBu), 3.71 (3H, brs, COOCH₃), 1.88 (3H, s, 5-CH₃).
MASS (EI) m/z : 358(M⁺), 343, 299, 290, 222, 154, 136, 69.

### (Example 795)

### Compound of Formula No. C-134

¹H-NMR (CDCl₃) δ(ppm) : 7.29 (1H, s, 3-H), 4.86 (2H, brs, NCH₂Ar), 3.98 (2H, d, J=7Hz, NCH₂-cBu), 1.98 (3H, s, 5-CH₃).
MASS (EI) m/z : 420(M⁺), 327, 259, 136.

### (Example 796)

### Compound of Formula No. C-135

¹H-NMR (CDCl₃) δ(ppm) : 7.18 (1H, s, 3-H), 4.74 (2H, brs, NCH₂Ar), 3.96 (2H, d, J=7Hz, NCH₂-cBu), 1.85 (3H, s, COCH₃), 1.73 (3H, s, 5-CH₃).
MASS (EI) m/z : 381(M⁺), 338, 271, 206, 175, 164, 150, 69.

### (Example 797)

### Compound of Formula No. C-136

¹H-NMR (CDCl₃) δ(ppm) : 7.26 (1H, s, 3-H), 4.74 (2H, brs, NCH₂Ar), 3.97 (2H, d, J=7Hz, NCH₂-cBu), 1.77 (3H, s, 5-CH₃), 1.50-1.37 (1H, m, COCH(CH₂)₂).
MASS (EI) m/z : 407(M⁺), 339, 232, 175, 164, 150.

### (Example 798)

### Compound of Formula No. C-137

¹H-NMR (CDCl₃) δ(ppm) : 7.17 (1H, s, 3-H), 4.78 (2H, brs, NCH₂Ar), 3.80 (2H, d, J=7Hz, NCH₂-cBu), 3.75 (2H, d, J=5Hz, COCH₂O), 1.71 (3H, s, 5-CH₃).
MASS (EI) m/z : 397(M⁺), 338, 270, 192, 175, 164.

### (Example 799)

### Compound of Formula No. C-138

¹H-NMR (CDCl₃) δ(ppm) : 7.17 (1H, s, 3-H), 4.75 (2H, brs, NCH₂Ar), 3.96 (2H, d, J=7Hz, NCH₂-cBu), 3.73 (2H, brs, COCH₂O), 1.70 (3H, s, 5-CH₃).
MASS (EI) m/z : 411(M⁺), 396, 266, 338, 270, 236, 175.

### (Example 800)

### Compound of Formula No. C-139

¹H-NMR (CDCl₃) δ(ppm) : 7.23 (1H, s, 3-H), 4.75 (2H, brs, NCH₂Ar), 4.35 (2H, s, COCH₂O), 3.96 (2H, d, J=7Hz, NCH₂-cBu), 1.75(3H, s, 5-CH₃).
MASS (EI) m/z : 473(M⁺), 380, 338, 312, 298, 175.

### (Example 801)

### Compound of Formula No. C-140

¹H-NMR (CDCl₃) δ(ppm) : 7.22 (1H, s, 3-H), 4.70 (2H, brs, NCH₂Ar), 4.32 (2H, s, COCH₂O), 3.96 (2H, d, J=7Hz, NCH₂-cBu), 1.77 (3H, s, 5-CH₃).
MASS (EI) m/z : 439(M⁺), 337, 309, 269, 192, 175, 164.

### (Example 802)

### Compound of Formula No. C-141

¹H-NMR (CDCl₃) δ (ppm) : 7.17 (1H, s, 3-H), 4.68 (2H, brs, NCH₂Ar), 3.95 (2H, d, J=7Hz, NCH₂-cBu), 3.69 (3H, brs, COOCH₃), 1.78 (3H, s, 5-CH₃).
MASS (EI) m/z : 397(M⁺), 329, 222, 194, 175, 154, 122.

### (Example 803)

### Compound of Formula No. C-142

¹H-NMR (CDCl₃) δ(ppm) : 7.27 (1H, s, 3-H), 4.79 (2H, brs, NCH₂Ar), 3.76 (2H, s, NCH₂-tBu), 1.79 (3H, s, 5-CH₃), 1.50-1.37 (1H, m, COCH(CH₂)₂).
MASS (EI) m/z : 350(M⁺), 335, 293, 266, 234, 225, 210.

### (Example 804)

### Compound of Formula No. C-143

¹H-NMR (CDCl₃) δ(ppm) : 7.20 (1H, s, 3-H), 4.78 (2H, brs, NCH₂Ar), 3.85 (2H, s, COCH₂Cl), 3.77 (2H, s, NCH₂-tBu), 1.80 (3H, s, 5-CH₃).
MASS (EI) m/z : 358(M⁺), 343, 323, 301, 281, 242, 186, 116.

### (Example 805)

### Compound of Formula No. C-144

¹H-NMR (CDCl₃) δ(ppm) : 7.14 (1H, s, 3-H), 4.77 (2H, brs, NCH₂Ar), 3.74 (2H, d, J=4Hz, COCH₂O), 3.73 (2H, s, NCH₂-tBu), 1.73 (3H, s, 5-CH₃).
MASS (EI) m/z : 340(M⁺), 325, 316, 283, 224, 211, 168.

### (Example 806)

### Compound of Formula No. C-145

¹H-NMR (CDCl₃) δ(ppm) : 7.13 (1H, s, 3-H), 4.73 (2H, brs, NCH₂Ar), 3.72 (2H, s, NCH₂-tBu), 3.70 (2H, brs, COCH₂O), 1.71 (3H, s, 5-CH₃).
MASS (EI) m/z : 354(M⁺), 339, 309, 297, 281, 225, 211.

### (Example 807)

### Compound of Formula No. C-146

¹H-NMR (CDCl₃) δ(ppm) : 7.21 (1H, s, 3-H), 4.77 (2H, brs, NCH₂Ar), 4.34 (2H, s, COCH₂O), 3.75 (2H, s, NCH₂-tBu), 1.78 (3H, s, 5-CH₃).
MASS (EI) m/z : 416(M⁺), 401, 359, 323, 309, 300, 253.

### (Example 808)

### Compound of Formula No. C-147

¹H-NMR (CDCl₃) δ(ppm) : 7.22 (1H, s, 3-H), 4.74 (2H, brs, NCH₂Ar), 4.32 (2H, s, COCH₂O), 3.76 (2H, s, NCH₂-tBu), 1.82 (3H, s, 5-CH₃).
MASS (EI) m/z : 382(M⁺), 367, 339, 325, 266, 225, 210.

### (Example 809)

### Compound of Formula No. C-148

¹H-NMR (CDCl₃) δ(ppm) : 7.18 (1H, brs, 3-H), 4.72 (2H, s, NCH₂Ar), 3.74 (3H, brs, COOCH₃), 3.70 (2H, brs, NCH₂-tBu), 1.79 (3H, brs, 5-CH₃).
MASS (EI) m/z : 340(M⁺), 325, 283, 224, 168, 116.

### (Example 810)

### Compound of Formula No. C-149

¹H-NMR (CDCl₃) δ(ppm) : 7.31 (1H, s, 3-H), 4.79 (2H, brs, NCH₂Ar), 3.76 (2H, brs, NCH₂-tBu), 1.90 (3H, s, 5-CH₃).
MASS (EI) m/z : 402(M⁺), 387, 309, 239, 193, 116.

### (Example 811)

### Compound of Formula No. C-150

¹H-NMR (CDCl₃) δ(ppm) : 7.24 (1H, s, 3-H), 4.73 (2H, brs, NCH₂Ar), 4.58 (2H, s, CONH₂), 3.75 (2H, d, J=7Hz, NCH₂-tBu), 1.82 (3H, s, 5-CH₃).
MASS (EI) m/z : 325(M⁺), 310, 281, 268, 225, 192.

### (Example 812)

### Compound of Formula No. C-151

¹H-NMR (CDCl₃) δ(ppm) : 7.25 (1H, s, 3-H), 4.77 (2H, brs, NCH₂Ar), 3.95-3.80 (1H, m, NCH(CH₂)₅), 1.84 (3H, s, 5-CH₃), 1.52-1.33 (1H, m, COCH(CH₂)₂)
MASS (EI) m/z : 362(M⁺), 294, 279, 246, 212, 149, 116.

### (Example 813)

### Compound of Formula No. C-152

¹H-NMR (CDCl₃) δ(ppm) : 7.16 (1H, s, 3-H), 4.79 (2H, s, NCH₂Ar), 3.95-3.78 (1H, m, NCH(CH₂)₅), 3.77 (2H, d, J=4Hz, COCH₂O), 1.80 (3H, s, 5-CH₃).
MASS (EI) m/z : 352(M⁺), 337, 295, 270, 239, 206, 116.

### (Example 814)

### Compound of Formula No. C-153

¹H-NMR (CDCl₃) δ(ppm) : 7.16 (1H, s, 3-H), 4.75 (2H, s, NCH₂Ar), 3.94-3.79 (1H, m, NCH(CH₂)₅), 3.73 (2H, s, COCH₂O), 1.79 (3H, s, 5-CH₃).
MASS (EI) m/z : 366(M⁺), 351, 293, 250, 211, 136, 116.

### (Example 815)

### Compound of Formula No. C-154

¹H-NMR (CDCl₃) δ(ppm) : 7.22 (1H, s, 3-H), 4.78 (2H, s, NCH₂Ar), 4.36 (2H, s, COCH₂O), 3.95-3.78 (1H, m, NCH(CH₂)₅), 1.84 (3H, s, 5-CH₃).
MASS (EI) m/z : 428(M⁺), 346, 312, 293, 253, 211, 136, 116

### (Example 816)

### Compound of Formula No. C-155

¹H-NMR (CDCl₃) δ(ppm) : 7.21 (1H, s, 3-H), 4.74 (2H, brs, NCH₂Ar), 4.33 (2H, s, COCH₂O), 3.96-3.79 (1H, m, NCH(CH₂)₅), 1.87 (3H, s, 5-CH₃).
MASS (EI) m/z : 394(M⁺), 351, 292, 271, 206, 116.

### (Example 817)

### Compound of Formula No. C-156

¹H-NMR (CDCl₃) δ(ppm) : 7.23 (1H, s, 3-H), 5.24 (2H, s, NCH₂Ph), 4.76 (2H, s, NCH₂Ar), 1.87 (3H, s, COCH₃), 1.71 (3H, s, 5-CH₃).
MASS (EI) m/z : 344(M⁺), 302, 228, 172, 116, 91.

### (Example 818)

### Compound of Formula No. C-157

¹H-NMR (CDCl₃) δ(ppm) : 7.31 (1H, s, 3-H), 5.24 (2H, s, NCH₂Ph), 4.78 (2H, brs, NCH₂Ar), 1.74 (3H, s, 5-CH₃), 1.51-1.40 (1H, m, COCH(CH₂)₂).
MASS (EI) m/z : 370(M⁺), 302, 254, 186, 116, 91.

### (Example 819)

### Compound of Formula No. C-158

¹H-NMR (CDCl₃) δ(ppm) : 7.21 (1H, s, 3-H), 5.24 (2H, s, NCH₂Ph), 4.79 (2H, brs, NCH₂Ar), 3.78 (2H, d, J=5Hz, COCH₂O), 1.69 (3H, s, 5-CH₃).
MASS (EI) m/z : 360(M⁺), 301, 214, 172, 116.

### (Example 820)

### Compound of Formula No. C-159

¹H-NMR (CDCl₃) δ(ppm) : 7.22 (1H, s, 3-H), 5.24 (2H, s, NCH₂Ph), 4.76 (2H, brs, NCH₂Ar), 3.75 (2H, s, COCH₂O), 1.68 (3H, s, 5-CH₃).
MASS (EI) m/z : 374(M⁺), 301, 172, 116, 91.

### (Example 821)

### Compound of Formula No. C-160

¹H-NMR (CDCl₃) δ(ppm) : 7.30 (1H, s, 3-H), 5.25 (2H, s, NCH₂Ph), 4.79 (2H, brs, NCH₂Ar), 4.35 (2H, s, COCH₂O), 1.73 (3H, s, 5-CH₃).
MASS (EI) m/z : 436(M⁺), 301, 116, 91.

### (Example 822)

### Compound of Formula No. C-161

¹H-NMR (CDCl₃) δ(ppm) : 7.33 (1H, s, 3-H), 5.24 (2H, s, NCH₂Ph), 4.75 (2H, brs, NCH₂Ar), 4.34 (2H, s, COCH₂O), 1.76 (3H, s, 5-CH₃).
MASS (EI) m/z : 402(M⁺), 300, 214, 116.

### (Example 823)

### Compound of Formula No. C-162

¹H-NMR (CDCl₃) δ(ppm) : 7.30 (1H, s, 3-H), 5.22 (2H, s, NCH₂Ph), 4.71 (2H, s, NCH₂Ar), 3.70 (3H, brs, COOCH₃), 1.75 (3H, s, 5-CH₃).
MASS (EI) m/z : 360(M⁺), 244, 116.

### (Example 824)

### Compound of Formula No. C-163

¹H-NMR (CDCl₃) δ(ppm) : 7.27 (1H, s, 3-H), 5.33 (2H, s, NCH₂OMe), 4.80 (2H, brs, NCH₂Ar), 1.94 (3H, s, 5-CH₃), 1.50-1.37 (1H, m, COCH(CH₂)₂).
MASS (EI) m/z : 324(M⁺), 293, 256, 208, 140, 116.

### (Example 825)

### Compound of Formula No. C-164

¹H-NMR (CDCl₃) δ(ppm) : 7.16 (1H, s, 3-H), 5.33 (2H, s, NCH₂OMe), 4.81 (2H, s, NCH₂Ar), 3.79 (2H, s, COCH₂O), 1.89 (3H, s, 5-CH₃).
MASS (EI) m/z : 314(M⁺), 283, 256, 168, 149, 136, 116.

### (Example 826)

### Compound of Formula No. C-165

¹H-NMR (CDCl₃) δ(ppm) : 7.18 (1H, s, 3-H), 5.33 (2H, s, NCH₂OMe), 4.78 (2H, s, NCH₂Ar), 3.76 (2H, s, COCH₂O), 1.89 (3H, s, 5-CH₃).
MASS (EI) m/z : 328(M⁺), 297, 281, 269, 255, 136, 116.

### (Example 827)

### Compound of Formula No. C-166

¹H-NMR (CDCl₃) δ(ppm) : 7.21 (1H, s, 3-H), 5.32 (2H, s, NCH₂OMe), 4.80 (2H, s, NCH₂Ar), 4.38 (2H, s, COCH₂O), 1.93 (3H, s, 5-CH₃).
MASS (EI) m/z : 390(M⁺), 359, 265, 255, 136, 116.

### (Example 828)

### Compound of Formula No. C-167

¹H-NMR (CDCl₃) δ(ppm) : 7.23 (1H, s, 3-H), 5.33 (2H, s, NCH₂OMe), 4.77 (2H, s, NCH₂Ar), 4.34 (2H, s, COCH₂O), 1.96 (3H, s, 5-CH₃).
MASS (EI) m/z : 356(M⁺), 325, 254, 168, 136, 116.

### (Example 829)

### Compound of Formula No. C-168

¹H-NMR (CDCl₃) δ(ppm) : 7.23 (1H, s, 3-H), 5.31 (2H, s, NCH₂OMe), 4.73 (2H, s, NCH₂Ar), 4.61 (2H, brs, CONH₂), 1.97 (3H, s, 5-CH₃).
MASS (EI) m/z : 299(M⁺), 268, 256, 183, 140, 116.

### (Example 830)

### Compound of Formula No. C-169

¹H-NMR (CDCl₃) δ(ppm) : 7.22 (1H, s, 3-H), 4.84 (2H, brs, NCH₂Ar), 4.37 (2H, brs, COCH₂O), 3.77 (2H, d, J=7Hz, NCH₂-iPr), 1.92 (3H, s, 5-CH₃).
MASS (EI) m/z : 473(M⁺), 382, 380, 340, 338, 107.

### (Example 831)

### Compound of Formula No. C-170

¹H-NMR (CDCl₃) δ(ppm) : 7.27 (1H, s, 3-H), 4.81 (2H, brs, NCH₂Ar), 4.35 (2H, brs, COCH₂O), 3.75 (2H, d, J=7Hz, NCH₂-iPr), 1.70 (3H, s, 5-CH₃).
MASS (EI) m/z : 469(M⁺), 390, 227, 168.

### (Example 832)

### Compound of Formula No. C-171

¹H-NMR (CDCl₃) δ(ppm) : 7.16 (1H, s, 3-H), 4.76 (2H, brs, NCH₂Ar), 3.78 (2H, d, J=7Hz, NCH₂-iPr), 2.02 (2H, brt, J=7Hz, COCH₂Et), 1.77 (3H, s, 5-CH₃).
MASS (EI) m/z : 338(M⁺), 268, 222, 212, 180, 152, 116.

### (Example 833)

### Compound of Formula No. C-172

¹H-NMR (CDCl₃) δ(ppm) : 7.16 (1H, s, 3-H), 4.75 (2H, brs, NCH₂Ar), 3.78 (2H, d, J=7Hz, NCH₂-iPr), 2.49 (1H, hept, J=7Hz, COCHMe₂), 1.78 (3H, s, 5-CH₃).
MASS (EI) m/z : 338(M⁺), 268, 225, 212, 180, 152, 124.

### (Example 834)

### Compound of Formula No. C-173

¹H-NMR (CDCl₃) δ(ppm) : 7.21 (1H, s, 3-H), 5.79 (1H, dd, J=15Hz, 2Hz, COCH=CHMe), 4.80 (2H, brs, NCH₂Ar), 3.79 (2H, d, J=7Hz, NCH₂-iPr), 1.73 (3H, s, 5-CH₃).
MASS (EI) m/z : 336(M⁺), 321, 268, 220, 152, 138, 116.

### (Example 835)

### Compound of Formula No. C-174

¹H-NMR (CDCl₃) δ(ppm) : 7.18 (1H, s, 3-H, 4.76 (2H, brs, NCH₂Ar), 3.78 (2H, d, J=7Hz, NCH₂-iPr), 2.88 (2H, brd, J=7Hz, COCH₂CH=CH₂), 1.77 (3H, s, 5-CH₃).
MASS (EI) m/z : 336(M⁺), 321, 295, 279, 268, 220, 167.

### (Example 836)

### Compound of Formula No. C-175

¹H-NMR (CDCl₃) δ(ppm) : 7.16 (1H, s, 3-H), 4.76 (2H, brs, NCH₂Ar), 3.78 (2H, d, J=7Hz, NCH₂-iPr), 2.05 (2H, brt, J=7Hz, COCH₂-Pr), 1.77 (3H, s, 5-CH₃).
MASS (EI) m/z : 352(M⁺), 337, 310, 268, 236, 152, 138.

### (Example 837)

### Compound of Formula No. C-176

¹H-NMR (CDCl₃) δ(ppm) : 7.14 (1H, s, 3-H), 4.77 (2H, brs, NCH₂Ar), 3.78 (2H, d, J=7Hz, NCH₂-iPr), 1.95 (2H, t, J=7Hz, COCH₂-iPr), 1.77 (3H, s, 5-CH₃).
MASS (EI) m/z : 352(M⁺), 268, 212, 152, 138, 116.

### (Example 838)

### Compound of Formula No. C-177

¹H-NMR (CDCl₃) δ(ppm) : 7.12 (1H, s, 3-H), 4.68 (2H, brs, NCH₂Ar), 3.78 (2H, d, J=7Hz, NCH₂-iPr), 1.83 (3H, s, 5-CH₃), 1.10 (9H, s, COC(CH₃)₃).
MASS (EI) m/z : 352(M⁺), 267, 225, 152, 116.

### (Example 839)

### Compound of Formula No. C-178

¹H-NMR (CDCl₃) δ(ppm) : 7.11 (1H, s, 3-H), 4.74 (2H, brs, NCH₂Ar), 3.78 (2H, d, J=7Hz, NCH₂-iPr), 3.07-2.90 (1H, m, COCH(CH₂)₃), 1.76 (3H, s, 5-CH₃).
MASS (EI) m/z : 350(M⁺), 268, 134, 152, 138, 116.

### (Example 840)

### Compound of Formula No. C-179

¹H-NMR (CDCl₃) δ(ppm) : 7.18 (1H, s, 3-H), 5.52 (1H, brt, J=1Hz, COCH=CMe₂), 4.80 (2H, brs, NCH₂Ar), 3.77 (2H, d, J=7Hz, NCH₂-iPr) , 1.72 (3H, s, 5-CH₃).
MASS (EI) m/z : 350(M⁺), 335, 268, 152, 116.

### (Example 841)

### Compound of Formula No. C-180

¹H-NMR (CDCl₃) δ(ppm) : 7.15 (1H, s, 3-H), 5.90-5.70 (1H, m, COCH(Me)-vinyl), 4.76 (2H, brs, NCH₂Ar), 3.78 (2H, d, J=7Hz, NCH₂-iPr), 1.75 (3H, brs, 5-CH₃).
MASS (EI) m/z : 350(M⁺), 335, 295, 268, 239, 228, 211, 180.

### (Example 842)

### Compound of Formula No. C-181

¹H-NMR (CDCl₃) δ(ppm) : 7.11 (1H, s, 3-H), 4.78 (2H, brs, NCH₂Ar), 3.75 (2H, d, J=7Hz, NCH₂-iPr), 1.98 (2H, brs, COCH₂-tBu), 1.72 (3H, s, 5-CH₃).
MASS (EI) m/z : 366(M⁺), 351, 268, 212, 152, 116.

### (Example 843)

### Compound of Formula No. C-182

¹H-NMR (CDCl₃) δ(ppm) : 7.16 (1H, s, 3-H), 4.76 (2H, brs, NCH₂Ar), 3.78 (2H, d, J=7Hz, NCH₂-iPr), 2.66-2.50 (1H, m, COCH(CH₂)₄), 1.79 (3H, s, 5-CH₃).
MASS (EI) m/z : 364(M+), 268, 212, 152, 116.

### (Example 844)

### Compound of Formula No. C-183

¹H-NMR (CDCl₃) δ(ppm) : 7.16 (1H, s, 3-H), 4.75 (2H, brs, NCH₂Ar), 3.78 (2H, d, J=7Hz, NCH₂-iPr), 2.82 (2H, brd, J=6Hz, COCH₂-C₄H₇), 1.76 (3H, s, 5-CH₃).
MASS (EI) m/z : 364(M⁺), 349, 321, 295, 268, 248, 239, 225.

### (Example 845)

### Compound of Formula No. C-184

¹H-NMR (CDCl₃) δ(ppm) : 7.17 (1H, s, 3-H), 4.76 (2H, brs, NCH₂Ar), 3.79 (2H, d, J=7Hz, NCH₂-iPr), 2.10-1.95 (2H, m, COCH₂-C₄H₇), 1.77 (3H, s, 5-CH₃).
MASS (EI) m/z : 364(M⁺), 310, 268, 248, 225, 212, 194.

### (Example 846)

### Compound of Formula No. C-185

¹H-NMR (CDCl₃) δ(ppm) : 7.14 (1H, s, 3-H), 4.74 (2H, brs, NCH₂Ar), 3.80 (2H, d, J=7Hz, NCH₂-iPr), 2.27-2.10 (1H, m, COCH(CH₂)₅), 1.80 (3H, s, 5-CH₃).
MASS (EI) m/z : 378(M⁺), 268, 225, 152, 116.

### (Example 847)

### Compound of Formula No. C-186

¹H-NMR (CDCl₃) δ(ppm) : 7.16 (1H, s, 3-H), 4.76 (2H, brs, NCH₂Ar), 3.78 (2H, d, J=7Hz, NCH₂-iPr), 2.50-2.35 (1H, m, COCH(C₅H₈)), 1.78 8 (3H, s, 5-CH₃).
MASS (EI) m/z : 376(M⁺), 361, 268, 260, 152, 116.

### (Example 848)

### Compound of Formula No. C-187

¹H-NMR (CDCl₃) δ(ppm) : 7.16 (1H, s, 3-H), 4.77 (2H, brs, NCH₂Ar), 3.79 (2H, d, J=7Hz, NCH₂-iPr), 2.37-2.06 (2H, m, COCH₂-cC₅H₉), 1.77 (3H, s, 5-CH₃).
MASS (EI) m/z : 378(M⁺), 310, 268, 212, 167, 149, 116.

### (Example 849)

### Compound of Formula No. C-188

¹H-NMR (CDCl₃) δ(ppm) : 7.14 (1H, s, 3-H), 4.77 (2H, brs, NCH₂Ar), 3.77 (2H, d, J=7Hz, NCH₂-iPr), 2.30-2.21 (2H, m, COCH₂-C₅H₇), 1.76 (3H, s, 5-CH₃).
MASS (EI) m/z : 376(M⁺), 310, 279, 268, 260, 225, 203.

### (Example 850)

### Compound of Formula No. C-189

¹H-NMR (CDCl₃) δ(ppm) : 7.27 (1H, s, 3-H), 4.96 (2H, brs, NCH₂Ar), 3.57 (2H, d, J=7Hz, NCH₂-iPr), 1.42 (3H, s, 5-CH₃).
MASS (EI) m/z : 372(M⁺), 267, 256, 116.

### (Example 851)

### Compound of Formula No. C-190

¹H-NMR (CDCl₃) δ(ppm) : 7.28 (1H, s, 3-H), 4.96 (2H, brs, NCH₂Ar), 3.58 (2H, d, J=7Hz, NCH₂-iPr), 1.46 (3H, s, 5-CH₃).
MASS (EI) m/z : 373(M⁺), 267, 257, 236, 211, 167, 138.

### (Example 852)

### Compound of Formula No. C-191

¹H-NMR (CDCl₃) δ(ppm) : 7.24 (1H, s, 3-H), 4.92 (2H, brs, NCH₂Ar), 3.65 (2H, d, J=7Hz, NCH₂-iPr), 1.56 (3H, s, 5-CH₃).
MASS (EI) m/z : 441(M⁺), 426, 385, 325, 267, 211, 174.

### (Example 853)

### Compound of Formula No. C-192

¹H-NMR (CDCl₃) δ(ppm) : 7.22 (1H, s, 3-H), 4.74 (2H, s, NCH₂Ar), 3.72 (2H, d, J=7Hz, NCH₂-iPr), 3.48(2H, s, COCH₂Ph), 1.45 (3H, s, 5-CH₃).
MASS (EI) m/z : 386(M⁺), 295, 268, 212, 152, 116.

### (Example 854)

### Compound of Formula No. C-193

¹H-NMR (CDCl₃) δ(ppm) : 7.20 (1H, s, 3-H), 4.77 (2H, brs, NCH₂Ar), 3.76 (2H, d, J=7Hz, NCH₂-iPr), 3.04 (2H, brd, J=5Hz, COCH₂-CH=CHPh), 1.75 (3H, s, 5-CH₃).
MASS (EI) m/z : 378(M⁺), 310, 268, 212, 167, 149, 116.

### (Example 855)

### Compound of Formula No. C-194

¹H-NMR (CDCl₃) δ(ppm) : 7.09 (1H, s, 3-H), 4.80 (2H, s, NCH₂Ar), 3.74 (2H, d, J=7Hz, NCH₂-iPr), 3.68 (2H, s, COCH₂Ar), 1.72 (3H, s, 5-CH₃).
MASS (EI) m/z : 387(M⁺), 268, 228, 212, 169, 152, 120.

### (Example 856)

### Compound of Formula No. C-196

¹H-NMR (CDCl₃) δ(ppm) : 7.16 (1H, s, 3-H), 4.76 (2H, brs, NCH₂Ar), 3.77 (2H, d, J=7Hz, NCH₂-iPr), 3.46 (2H, s, COCH₂Ar), 1.57 (3H, s, 5-CH₃).
MASS (EI) m/z : 387(M⁺), 344, 295, 268, 250, 212, 152.

### (Example 857)

### Compound of Formula No. C-197

¹H-NMR (CDCl₃) δ(ppm) : 7.13 (1H, s, 3-H), 4.75 (2H, brs, NCH₂Ar), 3.75 (2H, d, J=7Hz, NCH₂-iPr), 3.47 (2H, s, COCH₂Ar), 1.55 (3H, s, 5-CH₃).
MASS (EI) m/z : 387(M⁺), 372, 344, 331, 295, 271, 250, 215.

### (Example 858)

### Compound of Formula No. C-198

¹H-NMR (CDCl₃) δ(ppm) : 7.14 (1H, s, 3-H), 4.76 (2H, brs, NCH₂Ar), 3.77 (2H, d, J=7Hz, NCH₂-iPr), 3.66 (2H, s, COCH₂Ar), 1.62 (3H, s, 5-CH₃).
MASS (EI) m/z : 392(M⁺), 295, 276, 267, 239, 152, 116.

### (Example 859)

### Compound of Formula No. C-199

¹H-NMR (CDCl₃) δ(ppm) : 7.10 (1H, s, 3-H), 4.75 (2H, s, NCH₂Ar), 3.75 (2H, d, J=7Hz, NCH₂-iPr), 3.49 (2H, s, COCH₂Ar), 1.56 (3H, s, 5-CH₃).
MASS (EI) m/z : 392(M⁺), 295, 268, 212, 152, 116.

### (Example 860)

### Compound of Formula No. C-200

¹H-NMR (CDCl₃) δ(ppm) : 7.20 (1H, s, 3-H), 4.75 (2H, brs, NCH₂Ar), 3.78 (2H, d, J=7Hz, NCH₂-iPr), 3.46 (2H, s, COCH₂Ar), 1.74 (3H, s, 5-CH₃).
MASS (EI) m/z : 391(M⁺), 348, 275, 267, 211, 180, 152.

### (Example 861)

### Compound of Formula No. C-201

¹H-NMR (CDCl₃) δ(ppm) : 7.17 (1H, s, 3-H), 4.76 (2H, brs, NCH₂Ar), 3.79 (2H, s, COCH₂O), 3.77 (2H, d, J=7Hz, NCH₂-iPr), 1.75 (3H, s, 5-CH₃).
MASS (EI) m/z : 354(M⁺), 339, 325, 310, 295, 267, 194, 116.

### (Example 862)

### Compound of Formula No. C-202

¹H-NMR (CDCl₃) δ(ppm) : 7.18 (1H, s, 3-H), 4.76 (2H, brs, NCH₂Ar), 3.79 (2H, s, COCH₂O), 3.77 (2H, d, J=7Hz, NCH₂-iPr), 1.75 (3H, s, 5-CH₃).
MASS (EI) m/z : 368(M⁺), 353, 325, 310, 295, 267, 138, 116.

### (Example 863)

### Compound of Formula No. C-203

¹H-NMR (CDCl₃) δ(ppm) : 7.16 (1H, s, 3-H), 4.76 (2H, brs, NCH₂Ar), 3.79 (2H, s, COCH₂O), 3.77 (2H, d, J=7Hz, NCH₂-iPr), 1.74 (3H, s, 5-CH₃).
MASS (EI) m/z : 366(M⁺), 351, 325, 310, 295, 267, 194, 116.

### (Example 864)

### Compound of Formula No. C-204

¹H-NMR (CDCl₃) δ(ppm) : 7.16 (1H, s, 3-H), 4.76 (2H, brs, NCH₂Ar), 3.77 (2H, s, COCH₂O), 3.76 (2H, d, J=7Hz, NCH₂-iPr), 1.74 (3H, s, 5-CH₃).
MASS (EI) m/z : 380(M⁺), 325, 310, 267, 256, 218, 190, 116.

### (Example 865)

### Compound of Formula No. C-205

¹H-NMR (CDCl₃) δ(ppm) : 7.19 (1H, s, 3-H), 4.77 (2H, brs, NCH₂Ar), 3.92 (2H, s, COCH₂O), 3.78 (2H, d, J=7Hz, NCH₂-ipr), 1.77 (3H, s, 5-CH₃).
MASS (EI) m/z : 364(M⁺), 349, 325, 310, 295, 267, 211, 116.

### (Example 866)

### Compound of Formula No. C-206

¹H-NMR (CDCl₃) δ(ppm) : 7.27 (1H, s, 3-H), 4.75 (2H, brs, NCH₂Ar), 3.89 (2H, brs, COCH₂O), 3.77 (2H, d, J=7Hz, NCH₂-iPr), 1.74 (3H, s, 5-CH₃).
MASS (EI) m/z : 485(M⁺), 369, 325, 116.

### (Example 867)

### Compound of Formula No. C-207

¹H-NMR (CDCl₃) δ(ppm) : 7.19 (1H, s, 3-H), 4.82 (2H, brs, NCH₂Ar), 3.89 (2H, brs, COCH₂O), 3.77 (2H, d, J=7Hz, NCH₂-iPr), 1.75 (3H, s, 5-CH₃).
MASS (EI) m/z ; 370(M⁺), 339, 325, 310, 267, 194, 116.

### (Example 868)

### Compound of Formula No. C-208

¹H-NMR (CDCl₃) δ(ppm) : 7.19 (1H, s, 3-H), 4.75 (2H, brs, NCH₂Ar), 3.90 (2H, brs, COCH₂O), 3.77 (2H, d, J=7Hz, NCH₂-iPr), 1.75 (3H, S, 5-CH₃).
MASS (EI) m/z : 384(M⁺), 339, 325, 310, 267, 194, 138, 116.

### (Example 869)

### Compound of Formula No. C-209

¹H-NMR (CDCl₃) δ(ppm) : 7.18 (1H, s, 3-H), 4.74 (2H, brs, NCH₂Ar), 3.92 (2H, brs, COCH₂O), 3.77 (2H, d, J=7Hz, NCH₂-iPr), 1.75 (3H, s, 5-CH₃).
MASS (EI) m/z : 414(M⁺), 355, 339, 310, 267, 194, 116.

### (Example 870)

### Compound of Formula No. C-210

¹H-NMR (CDCl₃) δ(ppm) : 7.16 (1H, s, 3-H), 4.76 (2H, brs, NCH₂Ar), 3.92 (2H, brs, COCH₂O), 3.74 (2H, d, J=7Hz, NCH₂-iPr), 1.71 (3H, s, 5-CH₃).
MASS (EI) m/z : 446(M⁺), 355, 340, 325, 310, 267, 116.

### (Example 871)

### Compound of Formula No. C-211

¹H-NMR (CDCl₃) δ(ppm) : 7.14 (1H, s, 3-H), 4.75 (2H, brs, NCH₂Ar), 3.96 (2H, brs, COCH₂O), 3.76 (2H, d, J=7Hz, NCH₂-iPr), 1.75 (3H, s, 5-CH₃).
MASS (EI) m/z : 440(M⁺), 439, 425, 383, 267, 224, 116.

### (Example 872)

### Compound of Formula No. C-212

¹H-NMR (CDCl₃) δ(ppm) : 7.15 (1H, s, 3-H), 4.75 (2H, brs, NCH₂Ar), 3.94 (2H, s, COCH₂O), 3.77 (2H, d, J=7Hz, NCH₂-iPr), 1.74 (3H, s, 5-CH₃).
MASS (EI) m/z : 426(M⁺), 411, 383, 326, 267, 211, 180, 149.

### (Example 873)

### Compound of Formula No. C-213

¹H-NMR (CDCl₃) δ(ppm) : 7.02 (1H, s, 3-H), 4.70 (2H, brs, NCH₂Ar), 3.88 (2H, brs, COCH₂O), 3.69 (2H, d, J=7Hz, NCH₂-iPr), 1.53 (3H, s, 5-CH₃).
MASS (EI) m/z: 459(M-1)⁺, 445, 402, 383, 329, 286, 267.

### (Example 874)

### Compound of Formula No. C-214

¹H-NMR (CDCl₃) δ(ppm) : 7.12 (1H, s, 3-H), 4.75 (2H, brs, NCH₂Ar), 4.02 (2H, brs, COCH₂O), 3.77 (2H, d, J=7Hz, NCH₂-iPr), 1.77 (3H, s, 5-CH₃).
MASS (EI) m/z : 481(M-1), 439, 267, 116.

### (Example 875)

### Compound of Formula No. C-215

¹H-NMR (CDCl₃) δ(ppm) : 6.89 (1H, s, 3-H), 4.68 (2H, brs, NCH₂Ar), 3.92 (2H, brs, COCH₂O), 3.61 (2H, d, J=7Hz, NCH₂-iPr), 1.34 (3H, s, 5-CH₃).
MASS (EI) m/z : 563(M-1), 549, 507, 335, 228, 211, 169.

### (Example 876)

### Compound of Formula No. C-216

¹H-NMR (CDCl₃) δ(ppm) : 7.18 (1H, s, 3-H), 4.82 (2H, brs, NCH₂Ar), 3.89 (2H, brs, COCH₂O), 3.77 (2H, d, J=7Hz, NCH₂-iPr), 1.75 (3H, s, 5-CH₃).
MASS (EI) m/z : 456(M⁺), 383, 339, 310, 267, 194, 116.

### (Example 877)

### Compound of Formula No. C-217

¹H-NMR (CDCl₃) δ(ppm) : 7.19 (1H, s, 3-H), 4.78 (2H, brs, NCH₂Ar), 3.94 (2H, s, COCH₂O), 3.76 (2H, d, J=7Hz, NCH₂-iPr), 1.75 (3H, s, 5-CH₃).
MASS (EI) m/z : 410(M⁺), 326, 310, 267, 180, 116.

### (Example 878)

### Compound of Formula No. C-218

¹H-NMR (CDCl₃) δ(ppm) : 7.17 (1H, s, 3-H), 4.75 (2H, brs, NCH₂Ar), 3.88 (2H, s, COCH₂O), 3.76 (2H, d, J=7Hz, NCH₂-iPr), 1.74 (3H, s, 5-CH₃).
MASS (EI) m/z : 384(M⁺), 352, 325, 309, 295, 267, 210, 116.

### (Example 879)

### Compound of Formula No. C-219

¹H-NMR (CDCl₃) δ(ppm) : 7.17 (1H, s, 3-H), 4.77 (2H, brs, NCH₂Ar), 3.77 (2H, d, J=7Hz, NCH₂-iPr), 2.32-2.21 (2H, m, COCH₂CH₂O), 1.77 (3H, s, 5-CH₃).
MASS (EI) m/z : 340(M⁺), 322, 268, 224, 152, 116.

### (Example 880)

### Compound of Formula No. C-220

¹H-NMR (CDCl₃ δ(ppm) : 7.17 (1H, s, 3-H), 4.78 (2H, brs, NCH₂Ar), 3.76 (2H, d, J=7Hz, NCH₂-iPr), 2.33 (2H, t, J=6Hz, COCH₂CH₂O), 1.79 (3H, s, 5-CH₃).
MASS (EI) m/z : 354(M⁺), 339, 268, 212, 152, 116.

### (Example 881)

### Compound of Formula No. C-221

¹H-NMR (CDCl₃) δ(ppm) : 7.17 (1H, s, 3-H), 4.78 (2H, brs, NCH₂Ar), 3.76 (2H, d, J=7Hz, NCH₂-iPr), 2.34 (2H, t, J=6Hz, COCH₂CH₂O), 1.79 (3H, S, 5-CH₃).
MASS (EI) m/z : 368(M⁺), 339, 268, 212, 152, 116.

### (Example 882)

### Compound of Formula No. C-222

¹H-NMR (CDCl₃) δ(ppm) : 7.17 (1H, s, 3-H), 4.78 (2H, brs, NCH₂Ar), 3.76 (2H, d, J=7Hz, NCH₂-iPr), 2.35 (2H, t, J=6Hz, COCH₂CH₂O), 1.79 (3H, s, 5-CH₃).
MASS (EI) m/z : 380(M⁺), 339, 323, 268, 212, 152, 116.

### (Example 883)

### Compound of Formula No. C-223

¹H-NMR (CDCl₃) δ(ppm) : 7.19 (1H, s, 3-H), 4.80 (2H, brs, NCH₂Ar), 3.77 (2H, d, J=7Hz, NCH₂-iPr), 2.54 (2H, t, J=6Hz, COCH₂CH₂O), 1.80 (3H, s, 5-CH₃).
MASS (EI) m/z : 416(M⁺), 323, 300, 268, 212, 152, 116.

### (Example 884)

### Compound of Formula No. C-224

¹H-NMR (CDCl₃) δ(ppm) : 7.14 (1H, S, 3-H), 4.79 (2H, s, NCH₂Ar), 3.77 (2H, d, J=7Hz, NCH₂-iPr), 3.24 (2H, s, COCH₂CO₂Me), 1.83 (3H, s, 5-CH₃).
MASS (El) m/z : 368(M⁺), 337, 295, 267, 252, 211, 152, 116.

### (Example 885)

### Compound of Formula No. C-225

¹H-NMR (CDCl₃) δ(ppm) : 7.20 (1H, s, 3-H), 4.77 (2H, brs, NCH₂Ar), 3.79(2H, d, J=7Hz, NCH₂-iPr), 3.81 (2H, s, COCH₂CN), 1.84 (3H, s, 5-CH₃).
MASS (EI) m/z : 335(M⁺), 320, 292, 279, 267, 219, 179, 164.

### (Example 886)

### Compound of Formula No. C-227

¹H-NMR (CDCl₃) δ(ppm) : 7,18 (1H, s, 3-H), 4.77 (2H, brs, NCH₂Ar), 4.22 (2H, brs, COCH₂O), 3.75 (2H, d, J=7Hz, NCH₂-iPr), 1.75 (3H, s, 5-CH₃).
MASS (EI) m/z : 409(M⁺), 326, 295, 267, 210, 180, 116.

### (Example 887)

### Compound of Formula No. C-228

¹H-NMR (CDCl₃) δ(ppm) : 7.18 (1H, s, 3-H), 4.76 (2H, brs, NCH₂Ar), 4.18 (2H, brs, COCH₂O), 3.76 (2H, d, J=7Hz, NCH₂-iPr), 1.74 (3H, s, 5-CH₃).
MASS (EI) m/z : 399(M⁺), 368, 325, 309, 267, 211, 180, 116.

### (Example 888)

### Compound of Formula No. C-229

¹H-NMR (CDCl₃) δ(ppm) : 7.17 (1H, s, 3-H), 4.76 (2H, brs, NCH₂Ar), 4.19 (2H, s, COCH₂O), 3.76 (2H, d, J=7Hz, NCH₂-iPr), 1.74 (3H, s, 5-CH₃).
MASS (EI) m/z : 425(M⁺), 368, 325, 309, 267, 180, 116.

### (Example 889)

### Compound of Formula No. C-230

¹H-NMR (CDCl₃) δ(ppm) : 7.18 (1H, s, 3-H), 4.80 (2H, brs, NCH₂Ar), 4.18 (2H, brs, COCH₂O), 3.77 (2H, d, J=7Hz, NCH₂-iPr), 1.76 (3H, s, 5-CH₃).
MASS (EI) m/z : 384(M⁺), 341, 326, 309, 295, 267, 180, 116.

### (Example 890)

### Compound of Formula No. C-231

¹H-NMR (CDCl₃) δ(ppm) : 7.17 (1H, s, 3-H), 4.76 (2H, brs, NCH₂Ar), 4.21 (2H, brs, COCH₂O), 3.75 (2H, d, J=7Hz, NCH₂-iPr), 1.75 (3H, s, 5-CH₃).
MASS (EI) m/z : 412(M⁺), 324, 295, 267, 239, 180, 152, 116.

### (Example 891)

### Compound of Formula No. C-232

¹H-NMR (CDCl₃) δ(ppm) : 7.17 (1H, s, 3-H), 4.76 (2H, brs, NCH₂Ar), 4.28 (2H, brs, COCH₂O), 3.76 (2H, d, J=7Hz, NCH₂-iPr), 1.75 (3H, s, 5-CH₃).
MASS (EI) m/z : 451(M⁺), 324, 295, 267, 239, 116.

### (Example 892)

### Compound of Formula No. C-233

¹H-NMR (CDCl₃) δ(ppm) : 7.19 (1H, s, 3-H), 4.77 (2H, brs, NCH₂Ar), 4.39 (1H, brs, COCH₂O), 4.32 (1H, brs, COCH₂O), 3.77 (2H, d, J=7Hz, NCH₂-iPr), 1.78 (3H, s, 5-CH₃).
MASS (EI) m/z : 367(M⁺), 325, 295, 281, 267, 210, 116.

### (Example 893)

### Compound of Formula No. C-235

¹H-NMR (CDCl₃) δ(ppm) : 7.20 (1H, s, 3-H), 4.78 (2H, brs, NCH₂Ar), 4.37 (2H, s, COCH₂O), 3.76 (2H, d, J=7Hz, NCH₂-iPr), 1.79 (3H, s, 5-CH₃).
MASS (EI) m/z : 407(M⁺), 325, 309, 281, 267, 210, 116.

### (Example 894)

### Compound of Formula No. C-236

¹H-NMR (CDCl₃) δ(ppm) : 7.18 (1H, s, 3-H), 4.77 (2H, brs, NCH₂Ar), 4.33 (2H, s, COCH₂O), 3.76 (2H, d, J=7Hz, NCH₂-iPr), 1.79 (3H, s, 5-CH₃).
MASS (EI) m/z : 407(M⁺), 325, 309, 281, 266, 153, 116.

### (Example 895)

### Compound of Formula No. C-237

¹H-NMR (CDCl₃) δ(ppm) : 7.18 (1H, s, 3-H), 4.75 (2H, brs, NCH₂Ar), 4.24 (2H, brs, COCH₂O), 3.76 (2H, d, J=7Hz, NCH₂-iPr), 1.76 (3H, s, 5-CH₃).
MASS (EI) m/z : 397(M⁺), 325, 309, 281, 267, 210, 116.

### (Example 896)

### Compound of Formula No. C-238

¹H-NMR (CDCl₃) δ(ppm) : 7.18 (1H, s, 3-H), 4.78 (2H, brs, NCH₂Ar), 4.24 (2H, brs, COCH₂O), 3.77 (2H, d, J=7Hz, NCH₂-iPr), 1.80 (3H, s, 5-CH₃).
MASS (EI) m/z : 397(M⁺), 325, 309, 281, 267, 203, 116.

### (Example 897)

### Compound of Formula No. C-239

¹H-NMR (CDCl₃) δ(ppm) : 7.18 (1H, s, 3-H), 4.78 (2H, brs, NCH₂Ar), 4.48 (2H, brs, COCH₂O), 3.74 (2H, d, J=7Hz, NCH₂-iPr), 1.73 (3H, s, 5-CH₃).
MASS (EI) m/z : 511(M⁺), 325, 295, 266, 186, 145, 116.

### (Example 898)

### Compound of Formula No. C-240

¹H-NMR (CDCl₃) δ(ppm) : 7.23 (1H, s, 3-H), 4.78 (2H, brs, NCH₂Ar), 4.53 (2H, s, COCH₂O), 3.78 (2H, d, J=7Hz, NCH₂-iPr), 1.82 (3H, s, 5-CH₃).
MASS (EI) m/z : 511(M⁺), 492, 326, 266, 186, 172, 145, 116.

### (Example 899)

### Compound of Formula No. C-241

¹H-NMR (CDCl₃) δ(ppm) : [7.22 (s) & 7.18 (s), 1H, 3-H], 4.78 (2H, brs, NCH₂Ar), [4.53 (brs) & 4.47 (brs), 2H, COCH₂O], [3.78 (d, J=7Hz) & 3.75 (d, J=7Hz), 2H, NCH₂-iPr], [1.81 (s) & 1.78 (s), 3H, 5-CH₃].
MASS (EI) m/z : 447(M⁺), 492, 325, 295, 281, 266, 239, 186.

### (Example 900)

### Compound of Formula No. C-242

¹H-NMR (CDCl₃) δ(ppm) : [7.18 (s) & 7.13 (s) , 1H, 3-H], 4.78 (2H, brs, NCH₂Ar), [4.49 (brs) & 4.31 (brs), 2H, COCH₂O], [3.75 (d, J=7Hz) & 3.74 (d, J=7Hz), 2H, NCH₂-iPr], [1.77 (s) & 1.75 (s), 3H, 5-CH₃].
MASS (EI) m/z : 473(M⁺), 267, 148, 133, 116.

### (Example 901)

### Compound of Formula No. C-243

¹H-NMR (CDCl₃) δ (ppm) : [7.21 (s) & 7.19 (s), 1H, 3-H], 4.77 (2H, brs, NCH₂Ar), [4.53 (brs) & 4.48 (brs), 2H, COCH₂O], 3.78 (2H, d, J=7Hz, NCH₂-iPr), [1.81 (s) & 1.79 (s) , 3H, 5-CH₃].
MASS (EI) m/z : 447(M⁺), 325, 309, 267, 180, 153, 122, 108.

### (Example 902)

### Compound of Formula No. C-244

¹H-NMR (CDCl₃) δ(ppm) : [7.21 (s) & 7.18 (s), 1H, 3-H], 4.78 (2H, brs, NCH₂Ar), [4.51 (brs) & 4.56 (brs), 2H, COCH₂O], [3.77 (d, J=7Hz) & 3.75 (d, J=7Hz), 2H, NCH₂-iPr], [1.81 (s) & 1.78 (s) , 3H, 5-CH₃].
MASS (EI) m/z : 487(M⁺), 456, 428, 371, 325, 295, 267, 210.

### (Example 903)

### Compound of Formula No. C-245

¹H-NMR (CDCl₃) δ(ppm) : 7.19 (1H, s, 3-H), 4.77 (2H, brs, NCH₂Ar), 4.34 (2H, s, COCH₂O), 3.76 (2H, d, J=7Hz, NCH₂-iPr), 1.78 (3H, s, 5-CH₃).
MASS (EI) m/z : 407(M⁺), 325, 281, 267, 153, 116.

### (Example 904)

### Compound of Formula No. C-246

¹H-NMR (CDCl₃) δ(ppm) : 7.19 (1H, s, 3-H), 4.77 (2H, brs, NCH₂Ar), 4.35 (2H, s, COCH₂O), 3.77 (2H, d, J=7Hz, NCH₂-iPr), 1.79 (3H, s, 5-CH₃).
MASS (EI) m/z : 423(M⁺), 325, 281, 267, 153, 116.

### (Example 905)

### Compound of Formula No. C-248

¹H-NMR (CDCl₃) δ(ppm) : 7.16 (1H, s, 3-H), 4.78 (2H, brs, NCH₂Ar), 3.78 (2H, d, J=7Hz, NCH₂-iPr), 3.04 (2H, brs, COCH₂N), 1.76 (3H, s, 5-CH₃).
MASS (EI) m/z : 339(M⁺), 296, 268, 225, 210, 180, 167.

### (Example 906)

### Compound of Formula No. C-249

¹H-NMR (CDCl₃) δ(ppm) : 7.22 (1H, s, 3-H), 4.81 (2H, brs, NCH₂Ar), 3.82 (2H, d, J=7Hz, NCH₂-iPr), 3.54 (2H, brs, COCH₂N), 1.80 (3H, s, 5-CH₃).
MASS (EI) m/z : 401(M⁺), 268, 225, 180, 152, 116.

### (Example 907)

### Compound of Formula No. C-250

¹H-NMR (CDCl₃) δ(ppm) : 7.23 (1H, s, 3-H), 4.76 (2H, brs, NCH₂Ar), 3.75 (2H, d, J=7Hz, NCH₂-iPr), 3.49 (1H, d, J=16Hz, COCH₂N), 3.37 (1H, d, J=16Hz, COCH₂N), 1.79 (3H, s, 5-CH₃).
MASS (EI) m/z : 439(M⁺), 396, 380, 310, 268, 144, 116, 84.

### (Example 908)

### Compound of Formula No. C-251

¹H-NMR (CDCl₃) δ(ppm) : 7.16 (1H, s, 3-H), 4.75 (2H, brs, NCH₂Ar), 3.76 (2H, d, J=7Hz, NCH₂-iPr), 3.67 (2H, brs, COCH₂N), 1.74 (3H, s, 5-CH₃).
MASS (EI) m/z : 439(M⁺), 396, 380, 310, 268, 210, 180.

### (Example 909)

### Compound of Formula No. C-252

¹H-NMR (CDCl₃) δ(ppm) : 7.11 (1H, s, 3-H), 4.73 (2H, brs, NCH₂Ar), 3.74 (2H, d, J=7Hz, NCH₂-iPr), 2.99 (2H, d, J=6Hz, COCH₂N), 1.67 (3H, s, 5-CH₃).
MASS (EI) m/z : 515 (M⁺), 500, 472, 424, 380, 268, 116.

### (Example 910)

### Compound of Formula No. C-253

¹H-NMR (CDCl₃) δ(ppm) : 7.20 (1H, s, 3-H), 4.78 (2H, brs, NCH₂Ar), 3.80 (2H, d, J=5Hz, COCH₂N), 3.79 (2H, d, J=7Hz, NCH₂-iPr) , 1.79 (3H, s, 5-CH₃).
MASS (EI) m/z : 438(M⁺), 404, 351, 295, 267, 211, 180, 116.

### (Example 911)

### Compound of Formula No. C-254

¹H-NMR (CDCl₃) δ(ppm) : 7.20 (1H, s, 3-H), 4.76 (2H, brs, NCH₂Ar), 3.77 (2H, d, J=7Hz, NCH₂-ipr), 3.76 (2H, d, J=5Hz, COCH₂N), 1.77 (3H, s, 5-CH₃).
MASS (EI) m/z : 353(M⁺), 295, 267, 239, 225, 212, 180.

### (Example 912)

### Compound of Formula No. C-255

¹H-NMR (CDCl₃) δ(ppm) : 7.19 (1H, s, 3-H), 4.76 (2H, brs, NCH₂Ar), 3.78 (2H, d, J=7Hz, NCH₂-iPr), 3.65 (2H, d, J=7Hz, COCH₂N), 1.78 (3H, s, 5-CH₃).
MASS (EI) m/z : 383(M⁺), 352, 295, 268, 239, 225, 212, 180.

### (Example 913)

### Compound of Formula No. C-257

¹H-NMR (CDCl₃) δ(ppm) : 7.19 (1H, s, 3-H), 4.93 (1H, d, J=14Hz, NCH₂Ar), 4.58 (1H, d, J=14Hz, NCH₂Ar), 4.18-4.07 (1H, m, COCH(iPr)N), 3.80 (1H, dd, J=14Hz, 7Hz, NCH₂-iPr), 3.77 (1H, dd, J=14Hz, 7Hz, NCH₂-iPr), 1.79 (3H, brs, 5-CH₃).
MASS (EI) m/z : 453(M+), 393, 351, 295, 268, 180, 116.

### (Example 914)

### Compound of Formula No. C-258

¹H-NMR (CDCl₃) δ(ppm) : 7.21 (1H, s, 3-H), 4.76 (2H, brs, NCH₂Ar), 3.77 (2H, d, J=7Hz, NCH₂-iPr), 3.68 (2H, d, J=5Hz, COCH₂N), 1.81 (3H, s, 5-CH₃).
MASS (EI) m/z : 368(M⁺), 351, 325, 295, 268, 225, 180.

### (Example 915)

### Compound of Formula No. C-259

¹H-NMR (CDCl₃) δ(ppm) : 7.20 (1H, s, 3-H), 4.76 (2H, brs, NCH₂Ar), 3.77 (2H, d, J=7Hz, NCH₂-iPr), 3.70 (2H, d, J=5Hz, COCH₂N), 1.81 (3H, s, 5-CH₃).

### (Example 916)

### Compound of Formula No. C-260

¹H-NMR (CDCl₃) δ(ppm) : 7.22 (1H, s, 3-H), 4.77 (2H, brs, NCH₂Ar), 3.80 (2H, brs, COCH₂N), 3.78 (2H, d, J=7Hz, NCH₂-iPr), 1.78 (3H, s, 5-CH₃).
MASS (EI) m/z : 478(M⁺), 351, 325, 268, 225, 180, 152, 116.

### (Example 917)

### Compound of Formula No. C-261

¹H-NMR (CDCl₃) δ(ppm) : 7.16 (2H, s, 3-H & 3'-H), 5.85 (2H, s, COCH=CHCO), 4.85 (4H, brs, NCH₂Ar & N'CH₂Ar), 3.72 (4H, d, J=7Hz, NCH₂-iPr & N'CH₂-iPr), 1.83 (6H, s, 5-CH₃ & 5'-CH₃).
MASS (EI) m/z : 616(M⁺), 573, 500, 349, 293, 267, 190.

### (Example 918)

### Compound of Formula No. C-262

¹H-NMR (CDCl₃) δ(ppm) : 7.20 (1H, s, 3-H), 4.76 (2H, brs, NCH₂Ar), 3.77 (2H, d, J=7Hz, NCH₂-iPr), 3.04 (2H, s, COCH₂S), 1.84 (3H, s, 5-CH₃).
MASS (EI) m/z : 356(M⁺), 310, 295, 267, 254, 239, 210.

### (Example 919)

### Compound of Formula No. C-263

¹H-NMR (CDCl₃) δ(ppm) : 7.22 (1H, s, 3-H), 4.78 (2H, brs, NCH₂Ar), 3.78 (2H, d, J=7Hz, NCH₂-iPr), 3.62 (1H, d, J=14Hz, COCH₂S), 3.53 (1H, d, J=14Hz, COCH₂S), 1.82 (3H, s, 5-CH₃).
MASS (EI) m/z : 372(M⁺), 356, 324, 309, 295, 267, 253, 239.

### (Example 920)

### Compound of Formula No. C-264

¹H-NMR (CDCl₃) δ(ppm) : 7.22 (1H,s, 3-H), 4.81 (2H,s, NCH₂Ar), 3.87 (2H, s, COCH₂S), 3.80 (1H, brd, J=7Hz, NCH₂-iPr), 3.77 (1H, brd, J=7Hz, NCH₂-iPr), 1.86 (3H, s, 5-CH₃).
MASS (EI) m/z : 388(M⁺), 373, 345, 309, 272, 253, 216, 180.

### (Example 921)

### Compound of Formula No. C-265

¹H-NMR (CDCl₃) δ(ppm) : 7.07 (1H, s, 3-H), 4.75 (2H, brs, NCH₂Ar), 3.76 (2H, d, J=7Hz, NCH₂-iPr), 3.51 (2H, s, COCH₂S), 1.79 (3H, s, 5-CH₃).
MASS (EI) m/z : 418(M⁺), 325, 309, 295, 268, 253, 210.

### (Example 922)

### Compound of Formula No. C-266

¹H-NMR (CDCl₃) δ(ppm) : 6.65 (1H, brs, 3-H), 4.69 (2H, brs, NCH₂Ar), 3.78 (1H, d, J=14Hz, COCH₂S), 3.73 (2H, d, J=7Hz, NCH₂-iPr), 3.50 (1H, d, J=14Hz, COCH₂S), 1.81 (3H, brs, 5-CH₃).
MASS (EI) m/z : 434(M⁺), 418, 324, 309, 267, 239, 210.

### (Example 923)

### Compound of Formula No. C-267

¹H-NMR (CDCl₃) δ(ppm) : 7.07 (1H, s, 3-H), 4.74 (2H, s, NCH₂Ar), 4.01 (2H, s, COCH₂S), 3.81 (1H, brd, J=7Hz, NCH₂-iPr), 3.77 (1H, brd, J=7Hz, NCH₂-iPr), 1.86 (3H, s, 5-CH₃).
MASS (EI) m/z : 450(M⁺), 435, 407, 334, 309, 267, 253.

### (Example 924)

### Compound of Formula No. C-268

¹H-NMR (CDCl₃) δ(ppm) : 7.20 (1H, s, 3-H), 4.76 (2H, brs, NCH₂Ar), 3.77 (2H, d, J=7Hz, NCH₂-iPr), 3.41 (2H, s, SCH₂CO), 3.22 (2H, s, COCH₂S), 1.84 (3H, s, 5-CH₃).
MASS (EI) m/z : 428(M⁺), 383, 341, 310, 295, 267, 254.

### (Example 925)

### Compound of Formula No. C-269

¹H-NMR (CDCl₃) δ(ppm) : 7.20 (1H, s, 3-H), 4.76 (2H, brs, NCH₂Ar), 3.79 (2H, d, J=7Hz, NCH₂-iPr), 3.50 (2H, d, J=15Hz, COCH₂S), 1.84 (3H, s, 5-CH₃).
MASS (EI) m/z : 494(M⁺), 341, 325, 310, 266, 227, 208.

### (Example 926)

### Compound of Formula No. C-270

¹H-NMR (CDCl₃) δ(ppm) : 6.94 (1H, s, 3-H), 5.13 (1H, d, J=14Hz, NCH₂Ar), 4.63 (1H, d, J=14Hz, NCH₂Ar), 4.00 (3H, s, NOCH₃), 3.58 (1H, d, J=7Hz, NCH₂-iPr), 3.57 (1H, d, J=7Hz, NCH₂-iPr), 1.43 (3H, s, 5-CH₃).
MASS (EI) m/z : 429(M⁺), 398, 342, 295, 267, 211, 116.

### (Example 927)

### Compound of Formula No. C-271

¹H-NMR (CDCl₃) δ(ppm) : 6.77 (1H, s, 3-H), 4.82 (2H, brs, NCH₂Ar), 3.86 (3H, s, NOCH₃), 3.64 (2H, d, J=7Hz, NCH₂-iPr), 1.49 (3H, s, 5-CH₃).
MASS (EI) m/z : 429(M⁺), 398, 342, 295, 267, 211, 116.

### (Example 928)

### Compound of Formula No. C-272

¹H-NMR (CDCl₃) δ(ppm) : 7.20 (1H, s, 3-H), 4.83 (2H, brs, NCH₂Ar), 4.09 (3H, s, NOCH₃), 3.77 (2H, d, J=7Hz, NCH₂-iPr), 1.80 (3H, s, 5-CH₃).
MASS (EI) m/z : 378(M⁺), 363, 347, 291, 262, 206, 116.

### (Example 929)

### Compound of Formula No. C-273

¹H-NMR (CDCl₃) δ(ppm) : 7.24 (1H, s, 3-H), 4.92 (2H, brs, NCH₂Ar), 3.78 (2H, d, J=7Hz, NCH₂-iPr), 1.82 (3H, s, 5-CH₃).
MASS (EI) m/z : 394(M⁺), 366, 351, 333, 295, 250, 116.

### (Example 930)

### Compound of Formula No. C-274

¹H-NMR (CDCl₃) δ(ppm) : 7.19 (1H, s, 3-H), 4.61 (2H, s, NCH₂Ar), 3.73 (2H, d, J=7Hz, NCH₂-iPr), 2.69 (6H, s, CON(CH₃)₂), 1.77 (3H, s, 5-CH₃).
MASS (EI) m/z : 339(M⁺), 267, 223, 178, 116.

### (Example 931)

### Compound of Formula No. C-275

¹H-NMR (CDCl₃) δ(ppm) : 7.24 (1H, s, 3-H), 4.74 (2H, s, NCH₂Ar), 3.98 (2H, t, J=5Hz, NHCH₂COOEt), 3.78 (2H, d, J=7Hz, NCH₂-iPr), 1.87 (3H, s, 5-CH₃).
MASS (EI) m/z : 397(M⁺), 352, 295, 281, 268, 212, 152, 116.

### (Example 932)

### Compound of Formula No. C-276

¹H-NMR (CDCl₃) δ(ppm) : 7.19 (1H, s, 3-H), 4.77 (2H, s, NCH₂Ar), 3.79 (2H, d, J=7Hz, NCH₂-iPr), 3.25 (2H, s, NCCH₂NH), 1.83 (3H, s, 5-CH₃).
MASS (EI) m/z : 378(M⁺), 335, 320, 267, 219, 179, 136, 116.

### (Example 933)

### Compound of Formula No. C-279

¹H-NMR (CDCl₃) δ(ppm) : 7.16 (1H, s, 3-H), 4.68 (2H, brs, NCH₂Ar), 4.03 (1H, dd, J=8Hz, 7Hz, COCH(iPr)N), 3.74 (2H, d, J=7Hz, NCH₂-iPr), 1.65 (3H, brs, 5-CH₃).
MASS (EI) m/z : 514(M⁺), 366, 295, 268, 152, 116.

### (Example 934)

### Compound of Formula No. C-280

¹H-NMR (CDCl₃) δ(ppm) : 7.23 (1H, s, 3-H), 4.70 (2H, brs, NCH₂Ar), 4.04 (1H, dd, J=8Hz, 7Hz, COCH(iPr)N), 3.77 (2H, d, J=7Hz, NCH₂-iPr), 1.80 (3H, s, 5-CH₃).
MASS (EI) m/z : 514(M⁺), 366, 295, 268, 152, 116.

### (Example 935)

### Compound of Formula No. C-281

¹H-NMR (CDCl₃) δ(ppm) : 7.21 (1H, s, 3-H), 4.73 (1H, d, J=14Hz, NCH₂Ar), 4.65 (1H, d, J=14Hz, NCH₂Ar), 3.96 (1H, dd, J=8Hz, 7Hz, COCH(iPr)N), 3.78 (2H, d, J=7Hz, NCH₂-iPr), 1.79 (3H, s, 5-CH₃).
MASS (EI) m/z : 574(M⁺), 410, 366, 295, 268, 164, 152, 116.

### (Example 936)

### Compound of Formula No. C-284

¹H-NMR (CDCl₃) δ(ppm) : 7.26 (1H, s, 3-H), 4.81 (2H, s, NCH₂Ar), 3.73 (2H, d, J=7Hz, NCH₂-iPr), 1.91 (3H, s, 5-CH₃).
MASS (EI) m/z : 393(M⁺), 295, 266,251, 223, 210, 152.

### (Example 937)

### Compound of Formula No. C-285

¹H-NMR (CDCl₃) δ(ppm) : 7.20 (1H, s, 3-H), 4.78 (2H, brs, NCH₂Ar), 3.78 (2H, d, J=7Hz, NCH₂-iPr), 2.27 (3H, s, COSCH₃), 1.81 (3H, s, 5-CH₃).
MASS (EI) m/z : 342(M⁺), 295, 267, 239, 226, 179, 123.

### (Example 938)

### Compound of Formula No. C-286

¹H-NMR (CDCl₃) δ(ppm) : 7.23 (1H, s, 3-H), 4.77 (2H, brs, NCH₂Ar), 4.36 (2H, brs, COCH₂O), 3.77 (2H, d, J=7Hz, NCH₂-iPr), 1.78 (3H, s, 5-CH₃).
MASS (EI) m/z : 416(M⁺), 397, 281.

### (Example 939)

### Compound of Formula No. C-287

¹H-NMR (CDCl₃) δ(ppm) : 7.21 (1H, s, 3-H), 4.88 (2H, brs, NCH₂Ar), 4.39 (2H, brs, COCH₂O), 3.79 (2H, d, J=7Hz, NCH₂-iPr), 1.97 (3H, s, 5-CH₃).
MASS (EI) m/z : 420(M⁺), 329, 314, 57.

### (Example 940)

### Compound of Formula No. C-288

¹H-NMR (CDCl₃) δ(ppm) : 7.27 (1H, s, 3-H), 4.76 (2H, brs, NCH₂Ar), 3.79 (2H, d, J=7Hz, NCH₂-iPr), 3.77 (2H, brs, COCH₂O), 1.82 (3H, s, 5-CH₃).
MASS (EI) m/z : 344(M⁺), 279, 265, 149.

### (Example 941)

### Compound of Formula No. C-289

¹H-NMR (CDCl₃) δ(ppm) : 7.26 (1H, s, 3-H), 4.87 (2H, brs, NCH₂Ar), 3.83 (2H, d, J=7Hz, NCH₂-iPr), 2.16 (3H, s, 5-CH₃), 1.69-1.56 (1H, m, COCH(CH₂)₂).
MASS (EI) m/z : 354(M⁺), 333, 297, 229.

### (Example 942)

### Compound of Formula No. C-290

¹H-NMR (CDCl₃) δ(ppm) : 7.22 (1H, s, 3-H), 4.72 (2H, brs, NCH₂Ar), 4.32 (2H, s, COCH₂O), 3.80 (2H, d, J=7Hz, NCH₂-iPr), 1.89 (3H, s, 5-CH₃).
MASS (EI) m/z : 386(M⁺), 343, 284, 228, 180.

### (Example 943)

### Compound of Formula No. C-291

¹H-NMR (CDCl₃) δ(ppm) : 7.27 (1H, s, 3-H), 4.75 (2H, brs, NCH₂Ar), 4.35 (2H, brs, COCH₂O), 3.79 (2H, d, J=7Hz, NCH₂-iPr), 1.85 (3H, s, 5-CH₃).
MASS (EI) m/z : 420(M⁺), 327, 285, 107.

### (Example 944)

### Compound of Formula No. C-292

¹H-NMR (CDCl₃) δ(ppm) : 7.26 (1H, s, 3-H), 4.77 (2H, brs, NCH₂Ar), 4.37 (2H, brs, COCH₂O), 3.78 (2H, d, J=7Hz, NCH₂-iPr), 1.82 (3H, s, 5-CH₃).
MASS (EI) m/z : 494(M⁺), 415, 403, 401, 107.

### (Example 945)

### Compound of Formula No. C-293

¹H-NMR (CDCl₃) δ(ppm) : 7.26 (1H, s, 3-H), 4.80 (2H, brs, NCH₂Ar), 4.38 (2H, brs, COCH₂O), 3.80 (2H, d, J=7Hz, NCH₂-iPr), 1.90 (3H, s, 5-CH₃).
MASS (EI) m/z : 441(M⁺), 426, 385, 306, 107.

### (Example 946)

### Compound of Formula No. C-294

¹H-NMR (CDCl₃) δ(ppm) : 7.22 (1H, s, 3-H), 4.83 (2H, brs, NCH₂Ar), 4.38 (2H, s, COCH₂O), 3.81 (2H, d, J=7Hz, NCH₂-iPr), 1.93 (3H, s, 5-CH₃).
MASS (EI) m/z : 427(M⁺), 371, 334, 292, 121.

### (Example 947)

### Compound of Formula No. C-295

¹H-NMR (CDCl₃) δ(ppm) : 7.18 (1H, s, 3-H), 4.87 (2H, brs, NCH₂Ar), 3.93 (2H, s, COCH₂O), 3.78 (2H, d, J=7Hz, NCH₂-iPr), 1.79 (3H, s, 5-CH₃).
MASS (EI) m/z : 388(M⁺), 286, 269, 219, 189.

### (Example 948)

### Compound of Formula No. C-296

¹H-NMR (CDCl₃) δ(ppm) : 7.27 (2H, s, 3-H), 4.74 (4H, brs, NCH₂Ar), 4.35 (4H, brs, COCH₂O), 3.78 (4H, d, J=7Hz, NCH₂-iPr), 1.87 (6H, s, 5-CH₃).
MASS (EI) m/z : 701(M⁺), 658, 567, 393.

### (Example 949)

### Compound of Formula No. C-297

¹H-NMR (CDCl₃) δ(ppm) : 7.26 (1H, s, 3-H), 4.64 (2H, brs, NCH₂Ar), 4.32 (2H, brs, COCH₂O), 3.78 (2H, d, J=7Hz, NCH₂-iPr), 1.81 (3H, s, 5-CH₃).
MASS (EI) m/z : 521(M⁺), 446, 153, 57.

### (Example 950)

### Compound of Formula No. C-298

¹H-NMR (CDCl₃) δ(ppm) : 7.26 (1H, s, 3-H), 4.42 (2H, brs, NCH₂Ar), 4.32 (2H, brs, COCH₂O), 3.78 (2H, d, J=7Hz, NCH₂-iPr), 1.81 (3H, s, 5-CH₃).
MASS (EI) m/z : 443(M⁺), 417, 287, 131.

### (Example 951)

### Compound of Formula No. C-299

¹H-NMR (CDCl₃) δ(ppm) : 7.17 (1H, s, 3-H), 4.70 (2H, brs, NCH₂Ar), 3.83 (2H, brs, COCH₂O), 3.71 (2H, d, J=7Hz, NCH₂-iPr), 1.61 (3H, s, 5-CH₃).
MASS (EI) m/z : 559(M⁺), 540, 369, 354, 326, 194, 175, 151.

### (Example 952)

### Compound of Formula No. C-300

¹H-NMR (CDCl₃) δ(ppm) : 7.25 (1H, s, 3-H), 4.81 (2H, brs, NCH₂Ar), 4.33 (2H, brs, COCH₂O), 3.77 (2H, d, J=7Hz, NCH₂-iPr), 1.76 (3H, s, 5-CH₃).
MASS (EI) m/z : 485(M⁺), 394, 392, 201, 199.

### (Example 953)

### Compound of Formula No. C-301

¹H-NMR (CDCl₃) δ(ppm) : 7.21 (1H, s, 3-H), 4.69 (2H, brs, NCH₂Ar), 4.34 (2H, brs, COCH₂O), 3.79 (2H, d, J=7Hz, NCH₂-iPr), 1.82 (3H, s, 5-CH₃).
MASS (EI) m/z : 432(M⁺), 339, 297, 286, 107.

### (Example 954)

### Compound of Formula No. C-302

¹H-NMR (CDCl₃) δ(ppm) : 7.27 (1H, s, 3-H), 4.71 (2H, brs, NCH₂Ar), 4.34 (2H, brs, COCH₂O), 3.78 (2H, d, J=7Hz, NCH₂-iPr), 1.81 (3H, s, 5-CH₃).
MASS (EI) m/z : 446(M⁺), 353, 286, 132, 107.

### (Example 955)

### Compound of Formula No. C-303

¹H-NMR (CDCl₃) δ(ppm) : 7.27 (1H, s, 3-H), 4.73 (2H, brs, NCH₂Ar), 4.35 (2H, brs, COCH₂O), 3.80 (2H, d, J=7Hz, NCH₂-iPr), 1.88 (3H, s, 5-CH₃).
MASS (EI) m/z : 494(M⁺), 401, 359, 286, 107.

### (Example 956)

### Compound of Formula No. C-304

¹H-NMR (CDCl₃) δ(ppm) : 7.26 (1H, s, 3-H), 4.98 (2H, brs, NCH₂Ar), 4.42 (2H, s, COCH₂O), 3.75 (2H, d, J=7Hz, NCH₂-iPr), 2.02 (3H, s, 5-CH₃).
MASS (EI) m/z : 494(M⁺), 401, 359, 107.

### (Example 957)

### Compound of Formula No. C-305

¹H-NMR (CDCl₃) δ(ppm) : 7.22 (1H, s, 3-H), 4.72 (2H, brs, NCH₂Ar), 4.34 (2H, brs, COCH₂O), 3.80 (2H, d, J=7Hz, NCH₂-iPr), 1.87 (3H, s, 5-CH₃).
MASS (EI) m/z : 508(M⁺), 415, 373, 286, 222.

### (Example 958)

### Compound of Formula No. C-306

¹H-NMR (CDCl₃) δ(ppm) : 7.26 (1H, s, 3-H), 4.99 (2H, brs, NCH₂Ar), 4.42 (2H, s, COCH₂O), 3.76 (2H, d, J=7Hz, NCH₂-iPr), 2.02 (3H, s, 5-CH₃).
MASS (EI) m/z : 508(M⁺), 494, 401, 287, 121.

### (Example 959)

### Compound of Formula No. C-307

¹H-NMR (CDCl₃) δ(ppm) : 7.24 (1H, s, 3-H), 4.80 (2H, brs, NCH₂Ar), 4.35 (2H, brs, COCH₂O), 3.75 (2H, d, J=7Hz, NCH₂-iPr), 1.72 (3H, s, 5-CH₃).
MASS (EI) m/z : 477(M⁺), 384, 342, 286, 107.

### (Example 960)

### Compound of Formula No. C-308

¹H-NMR (CDCl₃) δ(ppm) : 7.24 (1H, s, 3-H), 4.89 (2H, brs, NCH₂Ar), 4.37 (2H, brs, COCH₂O), 3.79 (2H, d, J=7Hz, NCH₂-iPr), 1.85 (3H, s, 5-CH₃).
MASS (EI) m/z : 509(M⁺), 494, 453, 416, 107.

### (Example 961)

### Compound of Formula No. C-309

¹H-NMR (CDCl₃) δ(ppm) : 7.27 (1H, s, 3-H), 4.92 (2H, brs, NCH₂Ar), 4.40 (2H, s, COCH₂O), 3.75 (2H, d, J=7Hz, NCH₂-iPr), 1.94 (3H, s, 5-CH₃).
MASS (EI) m/z : 448(M⁺), 401, 313, 286, 107.

### (Example 962)

### Compound of Formula No. C-310

¹H-NMR (CDCl₃) δ(ppm) : 7.26 (1H, s, 3-H), 5.27 (2H, brs, NCH₂Ar), 4.50 (2H, brs, COCH₂O), 4.12 (2H, d, J=7Hz, NCH₂-iPr), 2.21 (3H, s, 5-CH₃).
MASS (EI) m/z : 480(M⁺), 401, 359, 345, 168.

### (Example 963)

### Compound of Formula No. C-311

¹H-NMR (CDCl₃) δ(ppm) : 7.22 (1H, s, 3-H), 4.75 (1H, brs, NCH₂Ar), 4.72 (1H, brs, NCH₂Ar), 4.35 (2H, brs, COCH₂O), 3.79 (1H, d, J=7Hz, NCH₂-iPr), 3.78 (1H, d, J=7Hz, NCH₂-iPr), 2.01 (3H, s, 5-CH₃).
MASS (EI) m/z : 510(M⁺), 420, 319, 286, 224.

### (Example 964)

### Compound of Formula No. C-312

¹H-NMR (CDCl₃) δ(ppm) : 7.22 (1H, s, 3-H), 5.03 (2H, brs, NCH₂Ar), 4.44 (2H, brs, COCH₂O), 3.78 (2H, d, J=7Hz, NCH₂-iPr), 2.01 (3H, s, 5-CH₃).
MASS (EI) m/z : 510(M⁺), 401, 375, 222, 107.

### (Example 965)

### Compound of Formula No. C-313

¹H-NMR (CDCl₃) δ(ppm) : 7.26 (1H, s, 3-H), 4.78 (2H, brs, NCH₂Ar), 4.34 (2H, brs, COCH₂O), 3.76 (2H, d, J=7Hz, NCH₂-iPr), 1.75 (3H, s, 5-CH₃).
MASS (EI) m/z : 526(M⁺), 510, 224, 57.

### (Example 966)

### Compound of Formula No. C-314

¹H-NMR (CDCl₃) δ(ppm) : 7.27 (1H, s, 3-H), 5.06 (1H, d, J=15Hz, NCH₂Ar), 4.82 (1H, d, J=15Hz, NCH₂Ar), 4.41 (2H, s, COCH₂O), 3.77 (1H, dd, J=14Hz, 7Hz, NCH₂-iPr), 3.76 (1H, dd, J=14Hz, 7Hz, NCH₂-iPr), 1.92 (3H, s, 5-CH₃).
MASS (EI) m/z : 526(M⁺), 509, 481, 401, 57.

### (Example 967)

### Compound of Formula No. C-315

¹H-NMR (CDCl₃) δ(ppm) : 7.26 (1H, s, 3-H), 4.81 (2H, brs, NCH₂Ar), 4.37 (2H, brs, COCH₂O), 3.79 (2H, d, J=7Hz, NCH₂-iPr), 1.88 (3H, s, 5-CH₃).
MASS (EI) m/z : 542(M⁺), 407, 57.

### (Example 968)

### Compound of Formula No. C-316

¹H-NMR (CDCl₃) δ(ppm) : 7.27 (1H, s, 3-H), 5.17 (2H, brs, NCH₂Ar), 4.47 (2H, s, COCH₂O), 3.76 (2H, d, J=7Hz, NCH₂-iPr), 2.09 (3H, s, 5-CH₃).
MASS (EI) m/z : 542(M⁺), 446, 407, 57.

### (Example 969)

### Compound of Formula No. C-318

¹H-NMR (CDCl₃) δ(ppm) : 7.27 (1H, s, 3-H), 4.81 (2H, brs, NCH₂Ar), 4.38 (2H, brs, COCH₂O), 3.79 (2H, d, J=7Hz, NCH₂-iPr), 1.87 (3H, s, 5-CH₃).
MASS (EI) m/z : 494(M⁺), 486, 444, 57.

### (Example 970)

### Compound of Formula No. C-319

¹H-NMR (CDCl₃) δ(ppm) : 7.13 (1H, s, 3-H), 4.76 (2H, brs, NCH₂Ar), 3.99 (2H, d, J=7Hz, NCH₂-cBu), 2.83-2.49 (1H, m, COCH(C₅H₈)), 1.80 (3H, s, 5-CH₃).
MASS (EI) m/z : 388(M⁺), 280, 272, 211, 164, 149, 116.

### (Example 971)

### Compound of Formula No. C-320

¹H-NMR (CDCl₃) δ(ppm) : 7.15 (1H, s, 3-H), 4.85 (2H, brs, NCH₂Ar), 3.97 (2H, d, J=7Hz, NCH₂-cBu), 3.92 (2H, s, COCH₂O), 1.81 (3H, s, 5-CH₃).
MASS (EI) m/z : 493(M⁺), 342, 299, 259, 231, 206, 136.

### (Example 972)

### Compound of Formula No. C-321

¹H-NMR (CDCl₃) δ(ppm) : 7.15 (1H, s, 3-H), 4.68 (2H, brs, NCH₂Ar), 3.92 (2H, d, J=7Hz, NCH₂-cBu), 3.82 (2H, brs, COCH₂O), 1.62 (3H, s, 5-CH₃).
MASS (EI) m/z : 571(M⁺), 381, 338, 206, 175, 122.

### (Example 973)

### Compound of Formula No. C-322

¹H-NMR (CDCl₃) δ(ppm) : 7.14 (1H, s, 3-H), 4.55 (2H, brs, NCH₂Ar), 3.95 (2H, brs, COCH₂O), 3.76 (2H, s, NCH₂-tBu), 1.75 (3H, s, 5-CH₃).
MASS (EI) m/z : 453(M-1), 439, 397, 281, 224, 116.

### (Example 974)

### Compound of Formula No. C-323

¹H-NMR (CDCl₃) δ(ppm) : 7.21 (1H, s, 3-H), 4.75 (2H, brs, NCH₂Ar), 4.35 (2H, s, COCH₂O), 3.76 (2H, s, NCH₂-tBu), 1.82 (3H, s, 5-CH₃).
MASS (EI) m/z : 422(M⁺), 282, 167, 149, 129.

### (Example 975)

### Compound of Formula No. C-324

¹H-NMR (CDCl₃) δ(ppm) : 7.24 (1H, s, 3-H), 4.76 (2H, brs, NCH₂Ar), 4.36 (2H, s, COCH₂O), 3.78 (2H, s, NCH₂-tBu), 1.84 (3H, s, 5-CH₃).
MASS (EI) m/z : 448(M⁺), 433, 397, 341, 282, 194, 116.

### (Example 976)

### Compound of Formula No. C-325

¹H-NMR (CDCl₃) δ(ppm) : 7.27 (1H, s, 3-H), 4.78 (2H, brs, NCH₂Ar), 4.58 (2H, s, COCH₂O), 3.79 (2H, s, NCH₂-tBu), 1.88 (3H, s, 5-CH₃).
MASS (EI) m/z : 466(M⁺), 451, 350, 323, 281, 253, 223.

### (Example 977)

### Compound of Formula No. C-326

¹H-NMR (CDCl₃) δ(ppm) : 7.22 (1H, s, 3-H), 4.80 (2H, brs, NCH₂Ar), 4.32 (2H, s, COCH₂O), 3.76 (2H, s, NCH₂-tBu), 1.81 (3H, s, 5-CH₃).
MASS (EI) m/z . 383(M⁺), 368, 340, 325, 281, 267, 211.

### (Example 978)

### Compound of Formula No. C-327

¹H-NMR (CDCl₃) δ(ppm) : 7.17 (1H, s, 3-H), 4.76 (2H, brs, NCH₂Ar), 3.51 (2H, s, NCH₂Si), 1.79 (3H, s, 5-CH₃), 1.55-1.40 (1H, m, COCH(CH₂)₂).
MASS (EI) m/z : 366(M⁺), 351, 297, 283, 250, 182, 116.

### (Example 979)

### Compound of Formula No. C-328

¹H-NMR (CDCl₃) δ(ppm) : 7.11 (1H, s, 3-H), 4.77 (2H, brs, NCH₂Ar), 3.87 (2H, s, COCH₂Cl), 3.51 (2H, s, NCH₂Si), 1.80 (3H, s, 5-CH₃).
MASS (EI) m/z : 374(M⁺), 359, 325, 297, 258, 208, 116.

### (Example 980)

### Compound of Formula No. C-329

¹H-NMR (CDCl₃) δ(ppm) : 7.07 (1H, s, 3-H), 4.79 (2H, brs, NCH₂Ar), 3.78 (2H, d, J=5Hz, OCH₂CO), 3.50 (2H, s, NCH₂Si), 1.75 (3H, s, 5-CH₃).
MASS (EI) m/z : 356(M⁺), 341, 325, 297, 240, 210, 182, 167, 116.

### (Example 981)

### Compound of Formula No. C-330

¹H-NMR (CDCl₃) δ(ppm) : 7.08 (1H, s, 3-H), 4.75 (2H, brs, NCH₂Ar), 3.74 (2H, s, COCH₂O), 3.50 (2H, s, NCH₂Si), 1.74 (3H, s, 5-CH₃).
MASS (EI) m/z : 370(M⁺), 355, 325, 297, 254, 208, 167, 116.

### (Example 982)

### Compound of Formula No. C-331

¹H-NMR (CDCl₃) δ(ppm) : 7.13 (1H, s, 3-H), 4.64 (2H, brs, NCH₂Ar), 4.34 (2H, s, COCH₂O), 3.50 (2H, s, NCH₂Si), 1.81 (3H, s, 5-CH₃).
MASS (EI) m/z : 398(M⁺), 383, 325, 297, 282, 210, 116.

### (Example 983)

### Compound of Formula No. C-332

¹H-NMR (CDCl₃) δ(ppm) : 7.15 (1H, s, 3-H), 4.86 (2H, brs, NCH₂Ar), 4.76 (2H, brs, COCH₂O), 3.52 (2H, s, NCH₂Si), 1.94 (3H, s, 5-CH₃).
MASS (EI) m/z : 468(M⁺), 453, 323, 298.

### (Example 984)

### Compound of Formula No. C-333

¹H-NMR (CDCl₃) δ(ppm) : 7.17 (1H, s, 3-H), 4.76 (2H, brs, NCH₂Ar), 4.67 (2H, s, COCH₂N), 3.51 (2H, s, NCH₂Si), 1.83 (3H, s, 5-CH₃).
MASS (EI) m/z : 406(M⁺), 391, 325, 297.

### (Example 985)

### Compound of Formula No. C-334

¹H-NMR (CDCl₃) δ(ppm) : 7.17 (1H, s, 3-H), 4.75 (2H, brs, NCH₂Ar), 4.59 (2H, s, COCH₂N), 3.51 (2H, s, NCH₂Si), 1.81 (3H, s, 5-CH₃).
MASS (EI) m/z : 440(M⁺), 425, 324, 297.

### (Example 986)

### Compound of Formula No. C-335

¹H-NMR (CDCl₃) δ(ppm) : 7.17 (1H, s, 3-H), 4.76 (2H, brs, NCH₂Ar), 4.62 (2H, s, COCH₂N), 3.51 (2H, s, NCH₂Si), 1.81 (3H, s, 5-CH₃).
MASS (EI) m/z : 485(M⁺), 471, 368, 325, 297, 222, 194, 159.

### (Example 987)

### Compound of Formula No. C-336

¹H-NMR (CDCl₃) δ(ppm) : 7.19 (1H, s, 3-H), 4.76 (2H, brs, NCH₂Ar), 4.68 (2H, s, COCH₂N), 3.52 (2H, s, NCH₂Si), 1.83 (3H, s, 5-CH₃).
MASS (EI) m/z : 431(M⁺), 416, 325, 315, 297, 222, 194.

### (Example 988)

### Compound of Formula No. C-337

¹H-NMR (CDCl₃) δ(ppm) : 7.19 (1H, s, 3-H), 4.71 (2H, brs, NCH₂Ar), 4.67 (2H, s, COCH₂N), 3.52 (2H, s, NCH₂Si), 1.84 (3H, s, 5-CH₃)
MASS (EI) m/z : 451(M⁺), 436, 335, 325, 297, 222, 194, 167.

### (Example 989)

### Compound of Formula No. C-338

¹H-NMR (CDCl₃) δ(ppm) : 7.18 (1H, s, 3-H), 4.70 (2H, brs, NCH₂Ar), 4.66 (2H, s, COCH₂N), 3.51 (2H, s, NCH₂Si), 1.82 (3H, s, 5-CH₃).
MASS (EI) m/z : 464(M⁺), 449, 433, 359, 348, 325, 297, 281.

### (Example 990)

### Compound of Formula No. C-339

¹H-NMR (CDCl₃) δ(ppm) : 7.09 (1H, s, 3-H), 4.69 (2H, brs, NCH₂Ar), 3.71 (3H, brs, COOCH₃), 3.48 (2H, s, NCH₂Si), 1.79 (3H, s, 5-CH₃).
MASS (EI) m/z : 356(M⁺), 341, 297, 240, 136, 116.

### (Example 991)

### Compound of Formula No. C-341

¹H-NMR (CDCl₃) δ(ppm) : 7.33 (1H, s, 3-H), 4.86 (2H, s, NCH₂Ar), 3.89 (2H, d, J=4Hz, COCH₂O), 1.92 (3H, s, 5-CH₃).
MASS (EI) m/z : 346(M⁺), 315, 287, 200, 172, 158, 118.

### (Example 992)

### Compound of Formula No. C-342

¹H-NMR (CDCl₃) δ(ppm) : 7.38 (1H, s, 3-H), 4.82 (2H, s, NCH₂Ar), 4.44 (2H, s, COCH₂O), 1.99 (3H, s, 5-CH₃).
MASS (EI) m/z : 388(M⁺), 345, 315, 286, 200, 158, 118.

### (Example 993)

### Compound of Formula No. C-343

¹H-NMR (CDCl₃) δ(ppm) : 4.77 (2H, s, NCH₂Ar), 3.75 (3H, brs, COOCH₃), 1.98 (3H, s, 5-CH₃).
MASS (EI) m/z : 346(M⁺), 287, 230, 198, 155, 118.

### (Example 994)

### Compound of Formula No. C-345

¹H-NMR (CDCl₃) δ (ppm) : 7.39 (1H, s, 3-H), 4.78 (2H, s, NCH₂Ar), 4.69 (2H, brs, CONH₂), 2.01 (3H, s, 5-CH₃).
MASS (EI) m/z : 331(M⁺), 286, 256, 215, 172, 118.

### (Example 995)

### Compound of Formula No. C-346

¹H-NMR (CDCl₃) δ(ppm) : 7.36 (1H, s, 3-H), 4.79 (2H, s, NCH₂Ar), 4.75 (1H, brq, J=5Hz, CONHMe), 2.82 (3H, d, J=5Hz, CONHCH₃), 1.98 (3H, s, 5-CH₃).
MASS (EI) m/z : 345(M⁺), 288, 229, 172, 158, 118.

### (Example 996)

### Compound of Formula No. C-347

¹H-NMR (CDCl₃) δ(ppm) : 7.35 (1H, s, 3-H), 4.82 (2H, s, NCH₂Ar), 2.00 (3H, s, 5-CH₃), 1.96 (3H, s, COCH₃).
MASS (EI) m/z : 348(M⁺), 306, 232, 190, 176, 136, 116.

### (Example 997)

### Compound of Formula No. C-348

¹H-NMR (CDCl₃) δ(ppm) : 7.42 (1H, s, 3-H), 4.84 (2H, s, NCH₂Ar), 2.02 (3H, s, 5-CH₃), 1.58-1.49 (1H, m, COCH(CH₂)₂).
MASS (EI) m/z : 374(M⁺), 306, 258, 190, 136.

### (Example 998)

### Compound of Formula No. C-349

¹H-NMR (CDCl₃) δ(ppm) : 7.33 (1H, s, 3-H), 4.86 (2H, s, NCH₂Ar), 3.88 (2H, d, J=3Hz, COCH₂O), 1.97 (3H, s, 5-CH₃).
MASS (EI) m/z : 364(M⁺), 346, 333, 305, 262, 218, 176, 136.

### (Example 999)

### Compound of Formula No. C-350

¹H-NMR (CDCl₃) δ(ppm) : 7.34 (1H, s, 3-H), 4.81 (2H, s, NCH₂Ar), 3.84 (2H, s, COCH₂O), 1.97 (3H, s, 5-CH₃).
MASS (EI) m/z : 378(M⁺), 333, 305, 262, 176, 136, 116.

### (Example 1000)

### Compound of Formula No. C-351

¹H-NMR (CDCl₃) δ(ppm) : 7.38 (1H, s, 3-H), 4.81 (2H, s, NCH₂Ar), 4.43 (2H, s, COCH₂O), 2.04 (3H, s, 5-CH₃).
MASS (EI) m/z : 406(M+), 388, 363, 304, 218, 176, 136.

### (Example 1001)

### Compound of Formula No. C-352

¹H-NMR (CDCl₃) δ(ppm) : 7.31 (1H, s, 3-H), 4.81 (2H, brs, NCH₂Ar), 4.06 (2H, s, COCH₂O), 1.96 (3H, s, 5-CH₃).
MASS (EI) m/z : 477(M-1), 463, 421, 305, 136, 116.

### (Example 1002)

### Compound of Formula No. C-353

¹H-NMR (CDCl₃) δ(ppm) : 7.37 (1H, s, 3-H), 4.81 (2H, brs, NCH₂Ar), 4.45 (2H, s, COCH₂O), 2.05 (3H, s, 5-CH₃).
MASS (EI) m/z : 446(M⁺), 365, 304, 218, 136, 116.

### (Example 1003)

### Compound of Formula No. C-354

¹H-NMR (CDCl₃) δ(ppm) : 7.39 (1H, s, 3-H), 4.84 (2H, brs, NCH₂Ar), 4.63 (2H, s, COCH₂O), 2.07 (3H, s, 5-CH₃).
MASS (EI) m/z : 445(M⁺), 362, 304, 228, 202, 136, 116.

### (Example 1004)

### Compound of Formula No. C-355

¹H-NMR (CDCl₃) δ(ppm) : 7.35 (1H, s, 3-H), 4.83 (2H, s, NCH₂Ar), 4.43 (2H, s, COCH₂O), 2.00 (3H, s, 5-CH₃).
MASS (EI) m/z : 419(M⁺), 363, 319, 304, 136, 116.

### (Example 1005)

### Compound of Formula No. C-356

¹H-NMR (CDCl₃) δ(ppm) : 7.34 (1H, s, 3-H), 4.76 (2H, s, NCH₂Ar), 3.76 (3H, brs, COOCH₃), 2.02 (3H, s, 5-CH₃).
MASS (EI) m/z : 364(M⁺), 305, 248, 190, 173, 136, 116.

### (Example 1006)

### Compound of Formula No. C-357

¹H-NMR (CDCl₃) δ(ppm) : 4.73 (2H, s, NCH₂Ar), 2.05 (3H, s, 5-CH₃), 1.46 (9H, brs, OC(CH₃)₃).
MASS (EI) m/z : 406(M⁺), 350, 306, 234, 136, 116.

### (Example 1007)

### Compound of Formula No. C-358

¹H-NMR (CDCl₃) δ(ppm) : 7.39 (1H, s, 3-H), 4.78 (2H, s, NCH₂Ar), 4.61 (2H, brs, CONH₂), 2.05 (3H, s, 5-CH₃).
MASS (EI) m/z : 349(M⁺), 304, 233, 190, 149, 136, 116.

### (Example 1008)

### Compound of Formula No. C-359

¹H-NMR (CDCl₃) δ(ppm) : 7.36 (1H, s, 3-H), 4.79 (2H, s, NCH₂Ar), 4.51 (1H, brq, J=5Hz, CONHMe), 2.82 (3H, d, J=5Hz, CONHCH₃), 2.03 (3H, s, 5-CH₃).
MASS (EI) m/z : 363(M⁺), 345, 306, 247, 190, 176, 136, 116.

### (Example 1009)

### Compound of Formula No. C-360

¹H-NMR (CDCl₃) δ(ppm) : 7.32 (1H, s, 3-H), 4.85 (2H, s, NCH₂Ar), 3.88 (2H, d, J=4Hz, COCH₂O), 1.96 (3H, s, 5-CH₃).
MASS (EI) m/z : 380(M⁺), 349, 321, 234, 192, 149, 116.

### (Example 1010)

### Compound of Formula No. C-361

¹H-NMR (CDCl₃) δ(ppm) : 7.38 (1H, s, 3-H), 4.81 (2H, s, NCH₂Ar), 4.42 (2H, s, COCH₂O), 2.04 (3H, s, 5-CH₃).
MASS (EI) m/z : 422(M⁺), 379, 320, 306, 234, 152, 116.

### (Example 1011)

### Compound of Formula No. C-362

¹H-NMR (CDCl₃) δ(ppm) : 7.31 (1H, s, 3-H), 4.80 (2H, s, NCH₂Ar), 4.06 (2H, s, COCH₂O), 1.96 (3H, s, 5-CH₃).
MASS (EI) m/z : 493(M-1), 479, 437, 321, 152, 116.

### (Example 1012)

### Compound of Formula No. C-363

¹H-NMR (CDCl₃) δ(ppm) : 7.37 (1H, s, 3-H), 4.81 (2H, brs, NCH₂Ar), 4.45 (2H, s, COCH₂O), 2.04 (3H, s, 5-CH₃).
MASS (EI) m/z : 462(M⁺), 381, 320, 234, 152, 116.

### (Example 1013)

### Compound of Formula No. C-364

¹H-NMR (CDCl₃) δ(ppm) : 7.39 (1H, s, 3-H), 4.84 (2H, brs, NCH₂Ar), 4.63 (2H, s, COCH₂O), 2.07 (3H, s, 5-CH₃).
MASS (EI) m/z : 461(M⁺), 349, 320, 246, 152, 116.

### (Example 1014)

### Compound of Formula No. C-365

¹H-NMR (CDCl₃) δ(ppm) : 7.37 (1H, s, 3-H), 4.73 (2H, s, NCH₂Ar), 2.05 (3H, s, 5-CH₃), 1.46 (9H, s, OC(CH₃)₃).
MASS (EI) m/z : 422(M⁺), 366, 322, 250, 220, 152, 116.

### (Example 1015)

### Compound of Formula No. C-366

¹H-NMR (CDCl₃) δ(ppm) : 7.42 (1H, s, 3-H), 4.83 (2H, s, NCH₂Ar), 2.07 (3H, s, 5-CH₃), 1.57-1.43 (1H, m, COCH(CH₂)₂).
MASS (EI) m/z : 392(M⁺), 324, 194, 154.

### (Example 1016)

### Compound of Formula No. C-367

¹H-NMR (CDCl₃) δ(ppm) : 7.33 (1H, s, 3-H), 4.85 (2H, s, NCH₂Ar), 3.86 (2H, d, J=4Hz, COCH₂O), 2.09 (3H, s, 5-CH₃).
MASS (EI) m/z : 382(M⁺), 351, 323, 236, 194, 154, 116.

### (Example 1017)

### Compound of Formula No. C-368

¹H-NMR (CDCl₃) δ(ppm) : 7.33 (1H, s, 3-H), 4.81 (2H, s, NCH₂Ar), 3.82 (2H, s, COCH₂O), 2.02 (3H, s, 5-CH₃).
MASS (EI) m/z : 396(M⁺), 381, 351, 323, 194, 154, 116.

### (Example 1018)

### Compound of Formula No. C-369

¹H-NMR (CDCl₃) δ(ppm) : 7.38 (1H, s, 3-H), 4.80 (2H, brs, NCH₂Ar), 4.41 (2H, s, COCH₂O), 2.18 (3H, s), 2.09 (3H, s, 5-CH₃).
MASS (EI) m/z : 424(M⁺), 382, 322, 236, 154, 116.

### (Example 1019)

### Compound of Formula No. C-370

¹H-NMR (CDCl₃) δ(ppm) : 7.31 (1H, s, 3-H), 4.80 (2H, s, NCH₂Ar), 4.04 (2H, s, COCH₂O), 2.02 (3H, s, 5-CH₃).
MASS (EI) m/z : 495(M-1), 481, 439, 323, 154, 116.

### (Example 1020)

### Compound of Formula No. C-371

¹H-NMR (CDCl₃) δ(ppm) : 7.37 (1H, s, 3-H), 4.81 (2H, brs, NCH₂Ar), 4.43 (2H, s, COCH₂O), 2.10 (3H, s, 5-CH₃).
MASS (EI) m/z : 464(M⁺), 383, 322, 236, 154, 116.

### (Example 1021)

### Compound of Formula No. C-372

¹H-NMR (CDCl₃) δ(ppm) : 7.39 (1H, s, 3-H), 4.83 (2H, brs, NCH₂Ar), 4.62 (2H, s, COCH₂O), 2.12 (3H, s, 5-CH₃).
MASS (EI) m/z : 463(M⁺), 364, 322, 248, 154, 116.

### (Example 1022)

### Compound of Formula No. C-373

¹H-NMR (CDCl₃) δ(ppm) : 7.35 (1H, s, 3-H), 4.82 (2H, s, NCH₂Ar), 4.41 (2H, s, COCH₂O), 2.05 (3H, s, 5-CH₃).
MASS (EI) m/z : 437(M⁺), 381, 337, 322, 154, 116.

### (Example 1023)

### Compound of Formula No. C-374

¹H-NMR (CDCl₃) δ(ppm) : 7.35 (1H, s, 3-H), 4.72 (2H, s, NCH₂Ar), 2.09 (3H, s, 5-CH₃), 1.46 (9H, s, OC(CH₃)₃).
MASS (EI) m/z : 424(M⁺), 368, 324, 252, 154, 116.

### (Example 1024)

### Compound of Formula No. C-375

¹H-NMR (CDCl₃) δ(ppm) : 7.31 (1H, s, 3-H), 4.86 (2H, s, NCH₂Ar), 3.89 (2H, d, J=4Hz, COCH₂O), 1.91 (3H, s, 5-CH₃).
MASS (EI) m/z : 360(M⁺), 329, 301, 214, 172, 132, 116.

### (Example 1025)

### Compound of Formula No. C-376

¹H-NMR (CDCl₃) δ(ppm) : 7.37 (1H, s, 3-H), 4.81 (2H, s, NCH₂Ar), 4.40 (2H, d, J=3Hz, COCH₂O), 1.98 (3H, s, 5-CH₃).
MASS (EI) m/z : 402(M⁺), 359, 300, 214, 172, 132.

### (Example 1026)

### Compound of Formula No. C-377

¹H-NMR (CDCl₃) δ(ppm) : 7.35 (1H, s, 3-H), 4.82 (2H, brs, NCH₂Ar), 4.46 (2H, s, COCH₂O), 1.99 (3H, s, 5-CH₃).
MASS (EI) m/z : 441(M⁺), 329, 300, 226, 169, 132.

### (Example 1027)

### Compound of Formula No. C-378

¹H-NMR (CDCl₃) δ(ppm) : 7.37 (1H, s, 3-H), 4.84 (2H, brs, NCH₂Ar), 4.65 (2H, s, COCH₂O), 2.01 (3H, s, 5-CH₃).
MASS (EI) m/z : 442(M⁺), 361, 300, 214, 132, 116.

### (Example 1028)

### Compound of Formula No. C-379

¹H-NMR (CDCl₃) δ(ppm) : 7.34 (1H, s, 3-H), 4.73 (2H, s, NCH₂Ar), 1.99 (3H, s, 5-CH₃), 1.45 (9H, s, OC(CH₃)₃).
MASS (EI) m/z : 402(M⁺), 346, 302, 230, 186, 132, 116.

### (Example 1029)

### Compound of Formula No. C-380

¹H-NMR (CDCl₃) δ(ppm) : 7.13 (1H, s, 3-H), 4.77 (2H, s, NCH₂Ar), 4.45 (2H, s, ArOCH₂CO), 3.77 (3H, s, NCH₃), 1.89 (3H, s, 5-CH₃).
MASS (EI) m/z : 378(M⁺), 267, 239, 225, 125, 116.

### (Example 1030)

### Compound of Formula No. C-381

¹H-NMR (CDCl₃) δ(ppm) : 7.16 (1H, s, 3-H), 4.77 (2H, s, NCH₂Ar), 4.36 (2H, s, ArOCH₂CO), 3.79 (3H, s, NCH₃), 1.90 (3H, s, 5-CH₃).
MASS (EI) m/z : 378(M⁺), 267, 239, 225, 125, 116.

### (Example 1031)

### Compound of Formula No. C-382

¹H-NMR (CDCl₃) δ(ppm) : 7.15 (1H, s, 3-H), 4.77 (2H, s, NCH₂Ar), 4.34 (2H, s, ArOCH₂CO), 3.78 (3H, s, NCH₃), 1.88 (3H, s, 5-CH₃).
MASS (EI) m/z : 378(M⁺), 267, 239, 225, 125, 116.

### (Example 1032)

### Compound of Formula No. C-383

¹H-NMR (CDCl₃) δ(ppm) : 7.15 (1H, s, 3-H), 4.78 (2H, s, NCH₂Ar), 4.47 (2H, s, ArOCH₂CO), 3.77 (3H, s, NCH₃), 1.89 (3H, s, 5-CH₃).
MASS (EI) m/z : 394(M⁺), 359, 267, 239, 225, 141, 116.

### (Example 1033)

### Compound of Formula No. C-384

¹H-NMR (CDCl₃) δ(ppm) : 7.16 (1H, s, 3-H), 4.77 (2H, s, NCH₂Ar), 4.36 (2H, s, ArOCH₂CO), 3.79 (3H, s, NCH₃), 1.91 (3H, s, 5-CH₃).
MASS (EI) m/z : 394(M⁺), 359, 267, 239, 225, 141, 116.

### (Example 1034)

### Compound of Formula No. C-385

¹H-NMR (CDCl₃) δ(ppm) : 7.15 (1H, s, 3-H), 4.77 (2H, s, NCH₂Ar), 4.44 (2H, s, ArOCH₂CO), 3.77 (3H, s, NCH₃), 1.89 (3H, s, 5-CH₃).
MASS (EI) m/z : 394(M⁺), 278, 267, 239, 225, 141, 116.

### (Example 1035)

### Compound of Formula No. C-386

¹H-NMR (CDCl₃) δ(ppm) : 7.14 (1H, s, 3-H), 4.78 (2H, s, NCH₂Ar), 4.41 (2H, s, ArOCH₂CO), 3.77 (3H, s, NCH₃), 1.89 (3H, s, 5-CH₃).
MASS (EI) m/z : 374(M⁺), 267, 239, 225, 121, 116.

### (Example 1036)

### Compound of Formula No. C-387

¹H-NMR (CDCl₃) δ(ppm) : 7.15 (1H, s, 3-H), 4.78 (2H, s, NCH₂Ar), 4.36 (2H, s, ArOCH₂CO), 3.77 (3H, s, NCH₃), 1.89 (3H, s, 5-CH₃).
MASS (EI) m/z : 374(M⁺), 267, 239, 225, 121, 116.

### (Example 1037)

### Compound of Formula No. C-388

¹H-NMR (CDCl₃) δ(ppm) : 7.14 (1H, s, 3-H), 4.77 (2H, s, NCH₂Ar), 4.34 (2H, s, ArOCH₂CO), 3.77 (3H, s, NCH₃), 1.88 (3H, s, 5-CH₃).
MASS (EI) m/z : 374(M⁺), 267, 239, 225, 121, 116.

### (Example 1038)

### Compound of Formula No. C-389

¹H-NMR (CDCl₃) δ(ppm) : 7.12 (1H, s, 3-H), 4.76 (2H, s, NCH₂Ar), 4.43 (2H, s, ArOCH₂CO), 3.76 (3H, s, NCH₃), 1.87 (3H, s, 5-CH₃).
MASS (EI) m/z : 390(M⁺), 374, 267, 239, 225, 137, 116.

### (Example 1039)

### Compound of Formula No. C-390

¹H-NMR (CDCl₃) δ(ppm) : 7.15 (1H, s, 3-H), 4.77 (2H, s, NCH₂Ar), 4.35 (2H, s, ArOCH₂CO), 3.77 (3H, s, NCH₃), 1.89 (3H, s, 5-CH₃).
MASS (EI) m/z : 390(M⁺), 274, 267, 239, 224, 137, 116.

### (Example 1040)

### Compound of Formula No. C-391

¹H-NMR (CDCl₃) δ(ppm) : 7.13 (1H, s, 3-H), 4.77 (2H, s, NCH₂Ar), 4.32 (2H, s, ArOCH₂CO), 3.77 (3H, s, NCH₃), 1.87 (3H, s, 5-CH₃).
MASS (EI) m/z : 390(M⁺), 267, 239, 225, 137, 116.

### (Example 1041)

### Compound of Formula No. C-392

¹H-NMR (CDCl₃) δ(ppm) : 7.14 (1H, s, 3-H), 4.77 (2H, s, NCH₂Ar), 4.55 (2H, s, ArOCH₂CO), 3.78 (3H, s, NCH₃), 1.90 (3H, s, 5-CH₃).
MASS (EI) m/z : 405(M⁺), 359, 225, 152, 122, 116.

### (Example 1042)

### Compound of Formula No. C-393

¹H-NMR (CDCl₃) δ(ppm) : 7.20 (1H, s, 3-H), 4.78 (2H, s, NCH₂Ar), 4.48 (2H, s, ArOCH₂CO), 3.81 (3H, s, NCH₃), 1.97 (3H, s, 5-CH₃).
MASS (EI) m/z : 405(M⁺), 289, 253, 225, 152, 116.

### (Example 1043)

### Compound of Formula No. C-394

¹H-NMR (CDCl₃) δ(ppm) : 7.20 (1H, s, 3-H), 4.78 (2H, s, NCH₂Ar), 4.48 (2H, s, ArOCH₂CO), 3.81 (3H, s, NCH₃), 1.93 (3H, s, 5-CH₃).
MASS (EI) m/z : 405(M⁺), 289, 253, 225, 152, 116.

### (Example 1044)

### Compound of Formula No. C-395

¹H-NMR (CDCl₃) δ(ppm) : 7.15 (1H, s, 3-H), 4.78 (2H, s, NCH₂Ar), 4.49 (2H, s, ArOCH₂CO), 3.77 (3H, s, NCH₃), 1.90 (3H, s, 5-CH₃).
MASS (EI) m/z : 428(M⁺), 312, 267, 225, 175, 116.

### (Example 1045)

### Compound of Formula No. C-396

¹H-NMR (CDCl₃) δ(ppm) : 7.16 (1H, s, 3-H), 4.78 (2H, s, NCH₂Ar), 4.42 (2H, s, ArOCH₂CO), 3.79 (3H, s, NCH₃), 1.91 (3H, s, 5-CH₃).
MASS (EI) m/z : 428(M⁺), 312, 253, 225, 175, 116.

### (Example 1046)

### Compound of Formula No. C-397

¹H-NMR (CDCl₃) δ(ppm) : 7.17 (1H, s, 3-H), 4.78 (2H, s, NCH₂Ar), 4.42 (2H, s, ArOCH₂CO), 3.79 (3H, s, NCH₃), 1.90 (3H, s, 5-CH₃).
MASS (EI) m/z : 428(M⁺), 409, 312, 267, 225, 175, 116.

### (Example 1047)

### Compound of Formula No. C-398

¹H-NMR (CDCl₃) δ(ppm) : 7.15 (1H, s, 3-H), 4.76 (2H, s, NCH₂Ar), 4.56 (2H, s, ArOCH₂CO), 3.79 (3H, s, NCH₃), 1.93 (3H, s, 5-CH₃).
MASS (EI) m/z : 385(M⁺), 361, 269, 225, 132, 116.

### (Example 1048)

### Compound of Formula No. C-399

¹H-NMR (CDCl₃) δ(ppm) : 7.18 (1H, s, 3-H), 4.78 (2H, s, NCH₂Ar), 4.40 (2H, s, ArOCH₂CO), 3.81 (3H, s, NCH₃), 1.93 (3H, s, 5-CH₃).
MASS (EI) m/z : 385(M⁺), 269, 225, 132, 116.

### (Example 1049)

### Compound of Formula No. C-400

¹H-NMR (CDCl₃) δ(ppm) : 7.18 (1H, s, 3-H), 4.77 (2H, s, NCH₂Ar), 4.43 (2H, s, ArOCH₂CO), 3.80 (3H, s, NCH₃), 1.91 (3H, s, 5-CH₃).
MASS (EI) m/z : 385(M⁺), 269, 225, 132, 116.

### (Example 1050)

### Compound of Formula No. C-401

¹H-NMR (CDCl₃) δ(ppm) : 7.12 (1H, s, 3-H), 4.76 (2H, s, NCH₂Ar), 4.41 (2H, s, ArOCH₂CO), 3.77 (3H, s, NCH₃), 1.87 (3H, s, 5-CH₃).
MASS (EI) m/z : 396(M⁺), 280, 267, 239, 225, 143, 116.

### (Example 1051)

### Compound of Formula No. C-402

¹H-NMR (CDCl₃) δ(ppm) : 7.15 (1H, s, 3-H), 4.76 (2H, s, NCH₂Ar), 4.44 (2H, s, ArOCH₂CO), 3.78 (3H, s, NCH₃), 1.89 (3H, s, 5-CH₃).
MASS (EI) m/z : 428(M⁺), 393, 267, 253, 225, 175, 116.

### (Example 1052)

### Compound of Formula No. C-403

¹H-NMR (CDCl₃) δ(ppm) : 7.16 (1H, s, 3-H), 4.77 (2H, s, NCH₂Ar), 4.35 (2H, s, ArOCH₂CO), 3.80 (3H, s, NCH₃), 1.92 (3H, s, 5-CH₃).
MASS (EI) m/z : 428(M⁺), 312, 267, 225, 175, 116.

### (Example 1053)

### Compound of Formula No. C-404

¹H-NMR (CDCl₃) δ(ppm) : 7.17 (1H, s, 3-H), 4.77 (2H, s, NCH₂Ar), 4.45 (2H, s, ArOCH₂CO), 4.04 (2H, q, J=7Hz, NCH₂Me), 1.85 (3H, s, 5-CH₃).
MASS (EI) m/z : 392(M⁺), 281, 267, 239, 125, 116.

### (Example 1054)

### Compound of Formula No. C-405

¹H-NMR (CDCl₃) δ(ppm) : 7.20 (1H, s, 3-H), 4.78 (2H, s, NCH₂Ar), 4.36 (2H, s, ArOCH₂CO), 4.06 (2H, q, J=7Hz, NCH₂Me), 1.87 (3H, S, 5-CH₃).
MASS (EI) m/z : 392(M⁺), 281, 267, 239, 125, 116.

### (Example 1055)

### Compound of Formula No. C-406

¹H-NMR (CDCl₃) δ(ppm) : 7.19 (1H, s, 3-H), 4.78 (2H, s, NCH₂Ar), 4.33 (2H, s, ArOCH₂CO), 4.05 (2H, q, J=7Hz, NCH₂Me), 1.85 (3H, s, 5-CH₃).
MASS (EI) m/z : 392(M⁺), 281, 267, 239, 125, 116.

### (Example 1056)

### Compound of Formula No. C-407

¹H-NMR (CDCl₃) δ(ppm) : 7.19 (1H, s, 3-H), 4.78 (2H, s, NCH₂Ar), 4.46 (2H, s, ArOCH₂CO), 4.05 (2H, q, J=7Hz, NCH₂Me), 1.86 (3H, s, 5-CH₃).
MASS (EI) m/z : 408(M⁺), 373, 281, 267, 239, 141, 116.

### (Example 1057)

### Compound of Formula No. C-408

¹H-NMR (CDCl₃) δ(ppm) : 7.20 (1H, s, 3-H), 4.78 (2H, s, NCH₂Ar), 4.36 (2H, s, ArOCH₂CO), 4.06 (2H, q, J=7Hz, NCH₂Me), 1.87 (3H, s, 5-CH₃).
MASS (EI) m/z : 408(M⁺), 373, 281, 267, 239, 141, 116.

### (Example 1058)

### Compound of Formula No. C-409

¹H-NMR (CDCl₃) δ(ppm) : 7.19 (1H, s, 3-H), 4.77 (2H, s, NCH₂Ar), 4.34 (2H, s, ArOCH₂CO), 4.05 (2H, q, J=7Hz, NCH₂Me), 1.85 (3H, s, 5-CH₃).
MASS (EI) m/z : 408(M⁺), 281, 267, 239, 141, 116.

### (Example 1059)

### Compound of Formula No. C-410

¹H-NMR (CDCl₃) δ(ppm) : 7.18 (1H, s, 3-H), 4.78 (2H, s, NCH₂Ar), 4.39 (2H, s, ArOCH₂CO), 4.05 (2H, q, J=7Hz, NCH₂Me), 1.86 (3H, s, 5-CH₃).
MASS (EI) m/z : 388(M⁺), 281, 267, 239, 121, 116.

### (Example 1060)

### Compound of Formula No. C-411

¹H-NMR (CDCl₃) δ(ppm) : 7.18 (1H, s, 3-H), 4.78 (2H, s, NCH₂Ar), 4.35 (2H, s, ArOCH₂CO), 4.05 (2H, q, J=7Hz, NCH₂Me), 1.86 (3H, s, 5-CH₃).
MASS (EI) m/z : 388(M⁺), 281, 267, 239, 121, 116.

### (Example 1061)

### Compound of Formula No. C-412

¹H-NMR (CDCl₃) δ(ppm) : 7.18 (1H, s, 3-H), 4.78 (2H, s, NCH₂Ar), 4.34 (2H, s, ArOCH₂CO), 4.04 (2H, q, J=7Hz, NCH₂Me), 1.84 (3H, s, 5-CH₃).
MASS (EI) m/z : 388(M⁺), 281, 267, 239, 121, 116.

### (Example 1062)

### Compound of Formula No. C-413

¹H-NMR (CDCl₃) δ(ppm) : 7.16 (1H, s, 3-H), 4.77 (2H, s, NCH₂Ar), 4.43 (2H, s, ArOCH₂CO), 4.04 (2H, q, J=7Hz, NCH₂Me), 1.84 (3H, s, 5-CH₃).
MASS (EI) m/z : 404(M⁺), 281, 267, 239, 137, 116.

### (Example 1063)

### Compound of Formula No. C-414

¹H-NMR (CDCl₃) δ(ppm) : 7.19 (1H, s, 3-H), 4.78 (2H, s, NCH₂Ar), 4.35 (2H, s, ArOCH₂CO), 4.05 (2H, q, J=7Hz, NCH₂Me), 1.86 (3H, s, 5-CH₃).
MASS (EI) m/z : 404(M⁺), 281, 267, 239, 137, 116.

### (Example 1064)

### Compound of Formula No. C-415

¹H-NMR (CDCl₃) δ(ppm) : 7.18 (1H, s, 3-H), 4.78 (2H, s, NCH₂Ar), 4.31 (2H, s, ArOCH₂CO), 4.04 (2H, q, J=7Hz, NCH₂Me), 1.84 (3H, s, 5-CH₃).
MASS (EI) m/z : 404(M⁺), 281, 267, 239, 137, 116.

### (Example 1065)

### Compound of Formula No. C-416

¹H-NMR (CDCl₃) δ(ppm) : 7.18 (1H, s, 3-H), 4.77 (2H, s, NCH₂Ar), 4.55 (2H, s, ArOCH₂CO), 4.05 (2H, q, J=7Hz, NCH₂Me), 1.87 (3H, s, 5-CH₃).
MASS (EI) m/z : 419(M⁺), 393, 373, 281, 267, 238, 152, 116.

### (Example 1066)

### Compound of Formula No. C-417

¹H-NMR (CDCl₃) δ(ppm) : 7.25 (1H, s, 3-H), 4.79 (2H, s, NCH₂Ar), 4.47 (2H, s, ArOCH₂CO), 4.08 (2H, q, J=7Hz, NCH₂Me), 1.94 (3H, s, 5-CH₃).
MASS (EI) m/z : 419(M⁺), 326, 303, 281, 267, 239, 152, 116.

### (Example 1067)

### Compound of Formula No. C-418

¹H-NMR (CDCl₃) δ(ppm) : 7.23 (1H, s, 3-H), 4.78 (2H, s, NCH₂Ar), 4.47 (2H, s, ArOCH₂CO), 4.07 (2H, q, J=7Hz, NCH₂Me), 1.89 (3H, s, 5-CH₃).
MASS (EI) m/z : 419(M⁺), 303, 281, 267, 239, 152, 116.

### (Example 1068)

### Compound of Formula No. C-419

¹H-NMR (CDCl₃) δ(ppm) : 7.20 (1H, s, 3-H), 4.78 (2H, s, NCH₂Ar), 4.48 (2H, s, ArOCH₂CO), 4.05 (2H, q, J=7Hz, NCH₂Me), 1.88 (3H, s, 5-CH₃).
MASS (EI) m/z : 442(M⁺), 423, 326, 281, 267, 239, 175, 116.

### (Example 1069)

### Compound of Formula No. C-420

¹H-NMR (CDCl₃) δ(ppm) : 7.21 (1H, s, 3-H), 4.78 (2H, s, NCH₂Ar), 4.41 (2H, s, ArOCH₂CO), 4.07 (2H, q, J=7Hz, NCH₂Me), 1.89 (3H, s, 5-CH₃).
MASS (EI) m/z : 442(M⁺), 423, 326, 281, 267, 239, 175, 116.

### (Example 1070)

### Compound of Formula No. C-421

¹H-NMR (CDCl₃) δ(ppm) : 7.22 (1H, s, 3-H), 4.78 (2H, s, NCH₂Ar), 4.41 (2H, s, ArOCH₂CO), 4.06 (2H, q, J=7Hz, NCH₂Me), 1.87 (3H, s, 5-CH₃).
MASS (EI) m/z : 442(M⁺), 423, 326, 281, 267, 239, 175, 116.

### (Example 1071)

### Compound of Formula No. C-422

¹H-NMR (CDCl₃) δ(ppm) : 7.20 (1H, s, 3-H), 4.77 (2H, s, NCH₂Ar), 4.55 (2H, s, ArOCH₂CO), 4.07 (2H, q, J=7Hz, NCH₂Me), 1.91 (3H, s, 5-CH₃).
MASS (EI) m/z : 399(M⁺), 313, 283, 267, 239, 132, 116.

### (Example 1072)

### Compound of Formula No. C-423

¹H-NMR (CDCl₃) δ(ppm) : 7.22 (1H, s, 3-H), 4.78 (2H, s, NCH₂Ar), 4.39 (2H, s, ArOCH₂CO), 4.08 (2H, q, J=7Hz, NCH₂Me), 1.89 (3H, s, 5-CH₃).
MASS (EI) m/z : 399(M⁺), 313, 283, 267, 239, 132, 116.

### (Example 1073)

### Compound of Formula No. C-424

¹H-NMR (CDCl₃) δ(ppm) : 7.22 (1H, s, 3-H), 4.78 (2H, s, NCH₂Ar), 4.43 (2H, s, ArOCH₂CO), 4.07 (2H, q, J=7Hz, NCH₂Me), 1.88 (3H, s, 5-CH₃).
MASS (EI) m/z : 399(M⁺), 313, 283, 267, 239, 132, 116.

### (Example 1074)

### Compound of Formula No. C-425

¹H-NMR (CDCl₃) δ(ppm) : 7.17 (1H, s, 3-H), 4.77 (2H, s, NCH₂Ar), 4.40 (2H, s, ArOCH₂CO), 4.05 (2H, q, J=7Hz, NCH₂Me), 1.84 (3H, s, 5-CH₃).
MASS (EI) m/z : 410(M⁺), 392, 281, 267, 239, 143, 116.

### (Example 1075)

### Compound of Formula No. C-426

¹H-NMR (CDCl₃) δ(ppm) : 7.19 (1H, s, 3-H), 4.77 (2H, s, NCH₂Ar), 4.44 (2H, s, ArOCH₂CO), 4.05 (2H, q, J=7Hz, NCH₂Me), 1.86 (3H, s, 5-CH₃).
MASS (EI) m/z : 442(M⁺), 407, 326, 281, 267, 239, 175, 116.

### (Example 1076)

### Compound of Formula No. C-427

¹H-NMR (CDCl₃) δ(ppm) : 7.20 (1H, s, 3-H), 4.78 (2H, s, NCH₂Ar), 4.35 (2H, s, ArOCH₂CO), 4.07 (2H, q, J=7Hz, NCH₂Me), 1.89 (3H, s, 5-CH₃).
MASS (EI) m/z : 442(M⁺), 388, 326, 281, 267, 239, 175, 116.

### (Example 1077)

### Compound of Formula No. C-428

¹H-NMR (CDCl₃) δ(ppm) : 7.24 (1H, s, 3-H), 4.76 (2H, s, NCH₂Ar), 4.68 (2H, s, ArOCH₂CO), 4.08 (2H, q, J=7Hz, NCH₂Me), 1.90 (3H, s, 5-CH₃).
MASS (EI) m/z : 464(M⁺), 404, 348, 281, 267, 238, 167, 116.

### (Example 1078)

### Compound of Formula No. C-429

¹H-NMR (CDCl₃) δ(ppm) : 7.19 (1H, s, 3-H), 4.78 (2H, s, NCH₂Ar), 4.45 (2H, s, ArOCH₂CO), 3.95 (2H, t, J=7Hz, NCH₂Et), 1.84 (3H, s, 5-CH₃).
MASS (EI) m/z : 406(M⁺), 295, 253, 125, 116.

### (Example 1079)

### Compound of Formula No. C-430

¹H-NMR (CDCl₃) δ(ppm) : 7.22 (1H, s, 3-H), 4.78 (2H, s, NCH₂Ar), 4.35 (2H, s, ArOCH₂CO), 3.97 (2H, t, J=7Hz, NCH₂Et), 1.84 (3H, s, 5-CH₃).
MASS (EI) m/z : 406(M⁺), 295, 253, 125, 116.

### (Example 1080)

### Compound of Formula No. C-431

¹H-NMR (CDCl₃) δ(ppm) : 7.21 (1H, s, 3-H), 4.78 (2H, s, NCH₂Ar), 4.32 (2H, s, ArOCH₂CO), 3.96 (2H, t, J=7Hz, NCH₂Et), 1.82 (3H, s, 5-CH₃).
MASS (EI) m/z : 406(M⁺), 295, 253, 125, 116.

### (Example 1081)

### Compound of Formula No. C-432

¹H-NMR (CDCl₃) δ(ppm) : 7.21 (1H, s, 3-H), 4.78 (2H, s, NCH₂Ar), 4.46 (2H, s, ArOCH₂CO), 3.96 (2H, t, J=7Hz, NCH₂Et), 1.84 (3H, s, 5-CH₃).
MASS (EI) m/z : 422(M⁺), 387, 295, 253, 141, 116.

### (Example 1082)

### Compound of Formula No. C-433

¹H-NMR (CDCl₃) δ(ppm) : 7.22 (1H, s, 3-H), 4.78 (2H, s, NCH₂Ar), 4.35 (2H, s, ArOCH₂CO), 3.97 (2H, t, J=7Hz, NCH₂Et), 1.85 (3H, s, 5-CH₃).
MASS (EI) m/z : 422(M⁺), 306, 295, 253, 141, 116.

### (Example 1083)

### Compound of Formula No. C-434

¹H-NMR (CDCl₃) δ(ppm) : 7.21 (1H, s, 3-H), 4.78 (2H, s, NCH₂Ar), 4.33 (2H, s, ArOCH₂CO), 3.96 (2H, t, J=7Hz, NCH₂Et), 1.83 (3H, s, 5-CH₃).
MASS (EI) m/z : 422(M⁺), 306, 295, 253, 141, 116.

### (Example 1084)

### Compound of Formula No. C-435

¹H-NMR (CDCl₃) δ(ppm) : 7.20 (1H, s, 3-H), 4.79 (2H, s, NCH₂Ar), 4.39 (2H, s, ArOCH₂CO), 3.96 (2H, t, J=7Hz, NCH₂Et), 1.84 (3H, s, 5-CH₃).
MASS (EI) m/z : 402(M⁺), 295, 253, 121, 116.

### (Example 1085)

### Compound of Formula No. C-436

¹H-NMR (CDCl₃) δ(ppm) : 7.21 (1H, s, 3-H), 4.78 (2H, s, NCH₂Ar), 4.34 (2H, s, ArOCH₂CO), 3.96 (2H, t, J=7Hz, NCH₂Et), 1.83 (3H, s, 5-CH₃).
MASS (EI) m/z : 402(M⁺), 295, 253, 121.

### (Example 1086)

### Compound of Formula No. C-437

¹H-NMR (CDCl₃) δ(ppm) : 7.20 (1H, s, 3-H), 4.78 (2H, s, NCH₂Ar), 4.33 (2H, s, ArOCH₂CO), 3.95 (2H, t, J=7Hz, NCH₂Et), 1.82 (3H, s, 5-CH₃).
MASS (EI) m/z : 402(M⁺), 295, 267, 253, 121, 116.

### (Example 1087)

### Compound of Formula No. C-438

¹H-NMR (CDCl₃) δ(ppm) : 7.18 (1H, s, 3-H), 4.78 (2H, s, NCH₂Ar), 4.42 (2H, s, ArOCH₂CO), 3.95 (2H, t, J=7Hz, NCH₂Et), 1.82 (3H, s, 5-CH₃).
MASS (EI) m/z : 418(M⁺), 295, 267, 253, 137, 116.

### (Example 1088)

### Compound of Formula No. C-439

¹H-NMR (CDCl₃) δ(ppm) : 7.21 (1H, s, 3-H), 4.78 (2H, s, NCH₂Ar), 4.34 (2H, s, ArOCH₂CO), 3.96 (2H, t, J=7Hz, NCH₂Et), 1.83 (3H, s, 5-CH₃).
MASS (EI) m/z : 418(M⁺), 295, 267, 252, 137, 116.

### (Example 1089)

### Compound of Formula No. C-440

¹H-NMR (CDCl₃) δ(ppm) : 7.19 (1H, s, 3-H), 4.78 (2H, s, NCH₂Ar), 4.31 (2H, s, ArOCH₂CO), 3.95 (2H, t, J=7Hz, NCH₂Et), 1.82 (3H, s, 5-CH₃).
MASS (EI) m/z : 418(M⁺), 295, 253, 137, 116.

### (Example 1090)

### Compound of Formula No. C-441

¹H-NMR (CDCl₃) δ(ppm) : 7.20 (1H, s, 3-H), 4.78 (2H, s, NCH₂Ar), 4.54 (2H, s, ArOCH₂CO), 3.96 (2H, t, J=7Hz, NCH₂Et), 1.85 (3H, s, 5-CH₃).
MASS (EI) m/z : 433(M⁺), 387, 253, 152, 122, 116.

### (Example 1091)

### Compound of Formula No. C-442

¹H-NMR (CDCl₃) δ(ppm) : 7.26 (1H, s, 3-H), 4.79 (2H, s, NCH₂Ar), 4.46 (2H, s, ArOCH₂CO), 3.99 (2H, t, J=7Hz, NCH₂Et), 1.91 (3H, s, 5-CH₃).
MASS (EI) m/z : 433(M⁺), 403, 317, 253, 152, 116.

### (Example 1092)

### Compound of Formula No. C-443

¹H-NMR (CDCl₃) δ(ppm) : 7.25 (1H, s, 3-H), 4.79 (2H, s, NCH₂Ar), 4.47 (2H, s, ArOCH₂CO), 3.98 (2H, t, J=7Hz, NCH₂Et), 1.86 (3H, s, 5-CH₃).
MASS (EI) m/z : 433(M⁺), 403, 317, 253, 152, 116.

### (Example 1093)

### Compound of Formula No. C-444

¹H-NMR (CDCl₃) δ(ppm) : 7.22 (1H, s, 3-H), 4.79 (2H, s, NCH₂Ar), 4.48 (2H, s, ArOCH₂CO), 3.96 (2H, t, J=7Hz, NCH₂Et), 1.86 (3H, s, 5-CH₃).
MASS (EI) m/z : 456(M⁺), 340, 295, 253, 175, 116.

### (Example 1094)

### Compound of Formula No. C-445

¹H-NMR (CDCl₃) δ(ppm) : 7.22 (1H, s, 3-H), 4.79 (2H, s, NCH₂Ar), 4.40 (2H, s, ArOCH₂CO), 3.98 (2H, t, J=7Hz, NCH₂Et), 1.86 (3H, s, 5-CH₃).
MASS (EI) m/z : 456(M⁺), 437, 340, 295, 253, 175, 116.

### (Example 1095)

### Compound of Formula No. C-446

¹H-NMR (CDCl₃) δ(ppm) : 7.23 (1H, s, 3-H), 4.78 (2H, s, NCH₂Ar), 4.41 (2H, s, ArOCH₂CO), 3.97 (2H, t, J=7Hz, NCH₂Et), 1.84 (3H, s, 5-CH₃).
MASS (EI) m/z : 456(M⁺), 437, 340, 295, 253, 175, 116.

### (Example 1096)

### Compound of Formula No. C-447

¹H-NMR (CDCl₃) δ(ppm) : 7.22 (1H, s, 3-H), 4.78 (2H, s, NCH₂Ar), 4.54 (2H, s, ArOCH₂CO), 3.97 (2H, t, J=7Hz, NCH₂Et), 1.88 (3H, s, 5-CH₃).
MASS (EI) m/z : 413(M⁺), 297, 253, 178, 132, 116.

### (Example 1097)

### Compound of Formula No. C-448

¹H-NMR (CDCl₃) δ(ppm) : 7.24 (1H, s, 3-H), 4.79 (2H, s, NCH₂Ar), 4.39 (2H, s, ArOCH₂CO), 3.98 (2H, t, J=7Hz, NCH₂Et), 1.86 (3H, s, 5-CH₃).
MASS (EI) m/z : 413(M⁺), 297, 253, 178, 132, 116.

### (Example 1098)

### Compound of Formula No. C-449

¹H-NMR (CDCl₃) δ(ppm) : 7.24 (1H, s, 3-H), 4.78 (2H, s, NCH₂Ar), 4.42 (2H, s, ArOCH₂CO), 3.98 (2H, t, J=7Hz, NCH₂Et), 1.84 (3H, s, 5-CH₃).
MASS (EI) m/z : 413(M⁺), 297, 253, 178, 132, 116.

### (Example 1099)

### Compound of Formula No. C-450

¹H-NMR (CDCl₃) δ(ppm) : 7.18 (1H, s, 3-H), 4.77 (2H, s, NCH₂Ar), 4.40 (2H, s, ArOCH₂CO), 3.95 (2H, t, J=7Hz, NCH₂Et), 1.81 (3H, s, 5-CH₃).
MASS (EI) m/z : 424(M⁺), 295, 253, 143, 116.

### (Example 1100)

### Compound of Formula No. C-451

¹H-NMR (CDCl₃) δ(ppm) : 7.21 (1H, s, 3-H), 4.77 (2H, s, NCH₂Ar), 4.43 (2H, s, ArOCH₂CO), 3.96 (2H, t, J=7Hz, NCH₂Et), 1.83 (3H, s, 5-CH₃).
MASS (EI) m/z : 456(M⁺), 421, 295, 253, 175, 116.

### (Example 1101)

### Compound of Formula No. C-452

¹H-NMR (CDCl₃) δ(ppm) : 7.22 (1H, s, 3-H), 4.78 (2H, s, NCH₂Ar), 4.33 (2H, s, ArOCH₂CO), 3.98 (2H, t, J=7Hz, NCH₂Et), 1.85 (3H, s, 5-CH₃).
MASS (EI) m/z : 456(M⁺), 340, 295, 253, 175, 116.

### (Example 1102)

### Compound of Formula No. C-453

¹H-NMR (CDCl₃) δ(ppm) : 7.27 (1H, s, 3-H), 4.77 (2H, brs, NCH₂Ar), 4.68 (2H, brs, ArOCH₂CO), 3.95 (2H, t, J=7Hz, NCH₂Et), 1.88 (3H, s, 5-CH₃).
MASS (EI) m/z : 478(M⁺), 362, 254, 197, 138, 116.

### (Example 1103)

### Compound of Formula No. C-454

¹H-NMR (CDCl₃) δ(ppm) : 7.20 (1H, s, 3-H), 4.77 (2H, s, NCH₂Ar), 4.44 (2H, s, ArOCH₂CO), 4.33 (1H, hept, J=7Hz, NCHMe₂), 1.83 (3H, s, 5-CH₃).
MASS (EI) m/z : 406(M⁺), 295, 253, 211, 125, 116.

### (Example 1104)

### Compound of Formula No. C-455

¹H-NMR (CDCl₃) δ(ppm) : 7.24 (1H, s, 3-H), 4.78 (2H, s, NCH₂Ar), 4.35 (2H, s, ArOCH₂CO), 4.34 (1H, hept, J=7Hz, NCHMe₂), 1.86 (3H, s, 5-CH₃).
MASS (EI) m/z : 406(M⁺), 295, 253, 211, 125, 116.

### (Example 1105)

### Compound of Formula No. C-456

¹H-NMR (CDCl₃) δ(ppm) : 7.23 (1H, s, 3-H), 4.78 (2H, s, NCH₂Ar), 4.34 (1H, hept, J=7Hz, NCHMe₂), 4.33 (2H, s, ArOCH₂CO), 1.84 (3H, s, 5-CH₃).
MASS (EI) m/z : 406(M⁺), 295, 253, 211, 125, 116.

### (Example 1106)

### Compound of Formula No. C-457

¹H-NMR (CDCl₃) δ(ppm) : 7.21 (1H, s, 3-H), 4.77 (2H, s, NCH₂Ar), 4.45 (2H, s, ArOCH₂CO), 4.33 (1H, hept, J=7Hz, NCHMe₂), 1.84 (3H, s, 5-CH₃).
MASS (EI) m/z : 422(M⁺), 387, 295, 253, 141, 116.

### (Example 1107)

### Compound of Formula No. C-458

¹H-NMR (CDCl₃) δ(ppm) : 7.24 (1H, s, 3-H), 4.78 (2H, s, NCH₂Ar), 4.35 (2H, s, ArOCH₂CO), 4.35 (1H, hept, J=7Hz, NCHMe₂), 1.87 (3H, s, 5-CH₃).
MASS (EI) m/z : 422(M⁺), 295, 267, 253, 141, 116.

### (Example 1108)

### Compound of Formula No. C-459

¹H-NMR (CDCl₃) δ(ppm) : 7.23 (1H, s, 3-H), 4.77 (2H, s, NCH₂Ar), 4.34 (2H, s, ArOCH₂CO), 4.34 (1H, hept, J=7Hz, NCHMe₂), 1.84 (3H, s, 5-CH₃).
MASS (EI) m/z : 422(M⁺), 308, 295, 253, 141, 116.

### (Example 1109)

### Compound of Formula No. C-460

¹H-NMR (CDCl₃) δ(ppm) : 7.21 (1H, s, 3-H), 4.78 (2H, s, NCH₂Ar), 4.39 (2H, s, ArOCH₂CO), 4.33 (1H, hept, J=7Hz, NCHMe₂), 1.84 (3H, s, 5-CH₃).
MASS (EI) m/z : 402(M⁺), 295, 253, 121, 116.

### (Example 1110)

### Compound of Formula No. C-461

¹H-NMR (CDCl₃) δ(ppm) : 7.23 (1H, s, 3-H), 4.78 (2H, s, NCH₂Ar), 4.34 (2H, s, ArOCH₂CO), 4.33 (1H, hept, J=7Hz, NCHMe₂), 1.84 (3H, s, 5-CH₃).
MASS (EI) m/z : 402(M⁺), 295, 253, 187.

### (Example 1111)

### Compound of Formula No. C-462

¹H-NMR (CDCl₃) δ(ppm) : 7.22 (1H, S, 3-H), 4.78 (2H, s, NCH₂Ar), 4.33 (2H, s, ArOCH₂CO), 4.33 (1H, hept, J=7Hz, NCHMe₂), 1.83 (3H, s, 5-CH₃).
MASS (EI) m/z : 402(M⁺), 295, 267, 253, 121, 116.

### (Example 1112)

### Compound of Formula No. C-463

¹H-NMR (CDCl₃) δ(ppm) : 7.20 (1H, s, 3-H), 4.77 (2H, s, NCH₂Ar), 4.41 (2H, s, ArOCH₂CO), 4.34 (1H, hept, J=7Hz, NCHMe₂), 1.83 (3H, s, 5-CH₃).
MASS (EI) m/z : 418(M⁺), 295, 267, 253, 137, 116.

### (Example 1113)

### Compound of Formula No. C-464

¹H-NMR (CDCl₃) δ(ppm) : 7.23 (1H, s, 3-H), 4.78 (2H, s, NCH₂Ar), 4.34 (2H, s, ArOCH₂CO), 4.34 (1H, hept, J=7Hz, NCHMe₂), 1.85 (3H, s, 5-CH₃).
MASS (EI) m/z : 418(M⁺), 295, 267, 252, 137, 116.

### (Example 1114)

### Compound of Formula No. C-465

¹H-NMR (CDCl₃) δ(ppm) : 7.22 (1H, s, 3-H), 4.78 (2H, s, NCH₂Ar), 4.33 (2H, s, ArOCH₂CO), 4.30 (1H, hept, J=7Hz, NCHMe₂), 1.82 (3H, s, 5-CH₃).
MASS (EI) m/z : 418(M⁺), 295, 253, 137, 116.

### (Example 1115)

### Compound of Formula No. C-466

¹H-NMR (CDCl₃) δ(ppm) : 7.23 (1H, s, 3-H), 4.77 (2H, s, NCH₂Ar), 4.54 (2H, s, ArOCH₂CO), 4.35 (1H, hept, J=7Hz, NCHMe₂), 1.87 (3H, s, 5-CH₃).
MASS (EI) m/z : 433(M⁺), 387, 252, 152, 122, 116.

### (Example 1116)

### Compound of Formula No. C-467

¹H-NMR (CDCl₃) δ(ppm) : 7.28 (1H, s, 3-H), 4.79 (2H, s, NCH₂Ar), 4.46 (2H, brs, ArOCH₂CO), 4.38 (1H, hept, J=7Hz, NCHMe₂), 1.93 (3H, s, 5-CH₃).
MASS (EI) m/z : 433(M⁺), 317, 253, 152, 116.

### (Example 1117)

### Compound of Formula No. C-468

¹H-NMR (CDCl₃) δ(ppm) : 7.28 (1H, s, 3-H), 4.78 (2H, s, NCH₂Ar), 4.48 (2H, brs, ArOCH₂CO), 4.37 (1H, hept, J=7Hz, NCHMe₂), 1.88 (3H, s, 5-CH₃).
MASS (EI) m/z : 433(M⁺), 317, 253, 152, 116.

### (Example 1118)

### Compound of Formula No. C-469

¹H-NMR (CDCl₃) δ(ppm) : 7.24 (1H, s, 3-H), 4.78 (2H, s, NCH₂Ar), 4.48 (2H, brs, ArOCH₂CO), 4.35 (1H, hept, J=7Hz, NCHMe₂), 1.87 (3H, s, 5-CH₃).
MASS (EI) m/z : 456(M⁺), 340, 295, 253, 175, 116.

### (Example 1119)

### Compound of Formula No. C-470

¹H-NMR (CDCl₃) δ(ppm) : 7.24 (1H, s, 3-H), 4.78 (2H, s, NCH₂Ar), 4.41 (2H, brs, ArOCH₂CO), 4.35 (1H, hept, J=7Hz, NCHMe₂), 1.87 (3H, s, 5-CH₃).
MASS (EI) m/z : 456(M⁺), 437, 340, 295, 253, 175, 116.

### (Example 1120)

### Compound of Formula No. C-471

¹H-NMR (CDCl₃) δ(ppm) : 7.25 (1H, s, 3-H), 4.78 (2H, s, NCH₂Ar), 4.41 (2H, brs, ArOCH₂CO), 4.35 (1H, hept, J=7Hz, NCHMe₂), 1.86 (3H, s, 5-CH₃).
MASS (EI) m/z : 456(M⁺), 437, 340, 295, 253, 175, 116.

### (Example 1121)

### Compound of Formula No. C-472

¹H-NMR (CDCl₃) δ(ppm) : 7.24 (1H, s, 3-H), 4.77 (2H, s, NCH₂Ar), 4.54 (2H, s, ArOCH₂CO), 4.36 (1H, hept, J=7Hz, NCHMe₂), 1.89 (3H, s, 5-CH₃).
MASS (EI) m/z : 413(M⁺), 398, 297, 253, 132, 116.

### (Example 1122)

### Compound of Formula No. C-473

¹H-NMR (CDCl₃) δ(ppm) : 7.25 (1H, s, 3-H), 4.78 (2H, s, NCH₂Ar), 4.40 (2H, brs, ArOCH₂CO), 4.37 (1H, hept, J=7Hz, NCHMe₂), 1.88 (3H, s, 5-CH₃).
MASS (EI) m/z : 413(M⁺), 297, 253, 132, 116.

### (Example 1123)

### Compound of Formula No. C-474

¹H-NMR (CDCl₃) δ(ppm) : 7.26 (1H, s, 3-H), 4.78 (2H, s, NCH₂Ar), 4.43 (2H, brs, ArOCH₂CO), 4.36 (1H, hept, J=7Hz, NCHMe₂), 1.87 (3H, s, 5-CH₃).
MASS (EI) m/z : 413(M⁺), 297, 253, 132, 116.

### (Example 1124)

### Compound of Formula No. C-475

¹H-NMR (CDCl₃) δ(ppm) : 7.20 (1H, s, 3-H), 4.76 (2H, brs, NCH₂Ar), 4.40 (2H, brs, ArOCH₂CO), 4.33 (1H, hept, J=7Hz, NCHMe₂), 1.83 (3H, s, 5-CH₃).
MASS (EI) m/z : 424(M⁺), 295, 253, 143, 116.

### (Example 1125)

### Compound of Formula No. C-476

¹H-NMR (CDCl₃) δ(ppm) : 7.22 (1H, s, 3-H), 4.77 (2H, brs, NCH₂Ar), 4.43 (2H, brs, ArOCH₂CO), 4.34 (1H, hept, J=7Hz, NCHMe₂), 1.84 (3H, s, 5-CH₃).
MASS (EI) m/z : 456(M⁺), 421, 295, 253, 175, 116.

### (Example 1126)

### Compound of Formula No. C-477

¹H-NMR (CDCl₃) δ(ppm) : 7.24 (1H, s, 3-H), 4.78 (2H, s, NCH₂Ar), 4.36 (1H, hept, J=7Hz, NCHMe₂), 4.34 (2H, brs, ArOCH₂CO), 1.87 (3H, s, 5-CH₃).
MASS (EI) m/z : 456(M⁺), 340, 295, 253, 175, 116.

### (Example 1127)

### Compound of Formula No. C-478

¹H-NMR (CDCl₃) δ(ppm) : 7.27 (1H, s, 3-H), 4.76 (2H, brs, NCH₂Ar), 4.68 (2H, brs, ArOCH₂CO), 4.37 (1H, hept, J=7Hz, NCHMe₂), 1.88 (3H, s, 5-CH₃).
MASS (EI) m/z : 478(M⁺), 362, 252, 197, 167, 116.

### (Example 1128)

### Compound of Formula No. C-479

¹H-NMR (CDCl₃) δ(ppm) : 7.18 (1H, s, 3-H), 4.78 (2H, s, NCH₂Ar), 4.44 (2H, s, ArOCH₂CO), 3.98 (2H, t, J=7Hz, NCH₂-Pr), 1.84 (3H, s, 5-CH₃).
MASS (EI) m/z : 420(M⁺), 309, 267, 125, 116.

### (Example 1129)

### Compound of Formula No. C-480

¹H-NMR (CDCl₃) δ(ppm) : 7.21 (1H, s, 3-H), 4.78 (2H, s, NCH₂Ar), 4.35 (2H, s, ArOCH₂CO), 4.00 (2H, t, J=7Hz, NCH₂-Pr), 1.84 (3H, s, 5-CH₃).
MASS (EI) m/z : 420(M⁺), 304, 267, 125, 116.

### (Example 1130)

### Compound of Formula No. C-481

¹H-NMR (CDCl₃) δ(ppm) : 7.20 (1H, s, 3-H), 4.78 (2H, s, NCH₂Ar), 4.32 (2H, s, ArOCH₂CO), 3.99 (2H, t, J=7Hz, NCH₂-Pr), 1.83 (3H, s, 5-CH₃).
MASS (EI) m/z : 420(M⁺), 309, 267, 253, 116.

### (Example 1131)

### Compound of Formula No. C-482

¹H-NMR (CDCl₃) δ(ppm) : 7.19 (1H, s, 3-H), 4.79 (2H, brs, NCH₂Ar), 4.45 (2H, s, ArOCH₂CO), 3.98 (2H, t, J=7Hz, NCH₂-Pr), 1.84 (3H, s, 5-CH₃).
MASS (EI) m/z : 436(M⁺), 401, 345, 320, 267, 141, 116.

### (Example 1132)

### Compound of Formula No. C-483

¹H-NMR (CDCl₃) δ(ppm) : 7.21 (1H, s, 3-H), 4.78 (2H, brs, NCH₂Ar), 4.34 (2H, s, ArOCH₂CO), 4.00 (2H, t, J=7Hz, NCH₂-Pr), 1.84 (3H, s, 5-CH₃).
MASS (EI) m/z : 436(M⁺), 393, 309, 295, 267, 141, 116.

### (Example 1133)

### Compound of Formula No. C-485

¹H-NMR (CDCl₃) δ(ppm) : 7.19 (1H, s, 3-H), 4.79 (2H, brs, NCH₂Ar), 4.38 (2H, s, ArOCH₂CO), 3.99 (2H, t, J=7Hz, NCH₂-Pr), 1.84 (3H, s, 5-CH₃).
MASS (EI) m/z : 416(M⁺), 309, 267, 121, 116.

### (Example 1134)

### Compound of Formula No. C-486

¹H-NMR (CDCl₃) δ(ppm) : 7.20 (1H, s, 3-H), 4.78 (2H, brs, NCH₂Ar), 4.34 (2H, s, ArOCH₂CO), 3.99 (2H, t, J=7Hz, NCH₂-Pr), 1.83 (3H, s, 5-CH₃).
MASS (EI) m/z : 416(M⁺), 309, 300, 267, 121, 116.

### (Example 1135)

### Compound of Formula No. C-487

¹H-NMR (CDCl₃) δ(ppm) : 7.19 (1H, s, 3-H), 4.78 (2H, brs, NCH₂Ar), 4.32 (2H, s, ArOCH₂CO), 3.98 (2H, t, J=7Hz, NCH₂-Pr), 1.82 (3H, s, 5-CH₃).
MASS (EI) m/z : 416(M⁺), 309, 267, 121, 116.

### (Example 1136)

### Compound of Formula No. C-488

¹H-NMR (CDCl₃) δ(ppm) : 7.17 (1H, s, 3-H), 4.77 (2H, brs, NCH₂Ar), 4.41 (2H, s, ArOCH₂CO), 3.98 (2H, t, J=7Hz, NCH₂-Pr), 1.81 (3H, s, 5-CH₃).
MASS (EI) m/z : 432(M⁺), 309, 267, 137, 116.

### (Example 1137)

### Compound of Formula No. C-489

¹H-NMR (CDCl₃) δ(ppm) : 7.20 (1H, s, 3-H), 4.78 (2H, brs, NCH₂Ar), 4.33 (2H, s, ArOCH₂CO), 3.99 (2H, t, J=7Hz, NCH₂-Pr), 1.83 (3H, s, 5-CH₃).
MASS (EI) m/z : 432(M⁺), 309, 295, 267, 137, 116.

### (Example 1138)

### Compound of Formula No. C-490

¹H-NMR (CDCl₃) δ(ppm) : 7.19 (1H, s, 3-H), 4.78 (2H, brs, NCH₂Ar), 4.30 (2H, s, ArOCH₂CO), 3.98 (2H, t, J=7Hz, NCH₂-Pr), 1.81 (3H, s, 5-CH₃).
MASS (EI) m/z : 432(M⁺), 309, 267, 137, 116.

### (Example 1139)

### Compound of Formula No. C-491

¹H-NMR (CDCl₃) δ(ppm) : 7.20 (1H, s, 3-H), 4.77 (2H, brs, NCH₂Ar), 4.53 (2H, s, ArOCH₂CO), 3.99 (2H, t, J=7Hz, NCH₂-Pr), 1.85 (3H, s, 5-CH₃).
MASS (EI) m/z : 447(M⁺), 401, 325, 267, 211, 152, 122.

### (Example 1140)

### Compound of Formula No. C-492

¹H-NMR (CDCl₃) δ(ppm) : 7.20 (1H, s, 3-H), 4.78 (2H, brs, NCH₂Ar), 4.53 (2H, s, ArOCH₂CO), 3.99 (2H, t, J=7Hz, NCH₂-Pr), 1.85 (3H, s, 5-CH₃).
MASS (EI) m/z : 447(M⁺), 331, 295, 267, 152, 116.

### (Example 1141)

### Compound of Formula No. C-493

¹H-NMR (CDCl₃) δ(ppm) : 7.24 (1H, s, 3-H), 4.78 (2H, brs, NCH₂Ar), 4.46 (2H, s, ArOCH₂CO), 4.01 (2H, t, J=7Hz, NCH₂-Pr), 1.85 (3H, s, 5-CH₃).
MASS (EI) m/z : 447(M⁺), 331, 295, 267, 152, 116.

### (Example 1142)

### Compound of Formula No. C-494

¹H-NMR (CDCl₃) δ(ppm) : 7.21 (1H, s, 3-H), 4.79 (2H, brs, NCH₂Ar), 4.47 (2H, s, ArOCH₂CO), 3.99 (2H, t, J=7Hz, NCH₂-Pr), 1.86 (3H, s, 5-CH₃).
MASS (EI) m/z : 470(M⁺), 354, 309, 267, 175, 116.

### (Example 1143)

### Compound of Formula No. C-495

¹H-NMR (CDCl₃) δ(ppm) : 7.22 (1H, s, 3-H), 4.79 (2H, brs, NCH₂Ar), 4.40 (2H, s, ArOCH₂CO), 4.00 (2H, t, J=7Hz, NCH₂-Pr), 1.85 (3H, s, 5-CH₃).
MASS (EI) m/z : 470(M⁺), 451, 354, 309, 267, 175, 116.

### (Example 1144)

### Compound of Formula No. C-496

¹H-NMR (CDCl₃) δ(ppm) : 7.22 (1H, s, 3-H), 4.78 (2H, brs, NCH₂Ar), 4.40 (2H, s, ArOCH₂CO), 4.00 (2H, t, J=7Hz, NCH₂-Pr), 1.84 (3H, s, 5-CH₃).
MASS (EI) m/z: 470(M⁺), 451, 354, 309, 267, 175, 116.

### (Example 1145)

### Compound of Formula No. C-497

¹H-NMR (CDCl₃) δ(ppm) : 7.21 (1H, s, 3-H), 4.78 (2H, brs, NCH₂Ar), 4.53 (2H, s, ArOCH₂CO), 4.00 (2H, t, J=7Hz, NCH₂-Pr), 1.89 (3H, s, 5-CH₃).
MASS (EI) m/z : 427(M⁺), 384, 311, 267, 132, 116.

### (Example 1146)

### Compound of Formula No. C-498

¹H-NMR (CDCl₃) δ(ppm) : 7.23 (1H, s, 3-H), 4.78 (2H, brs, NCH₂Ar), 4.33 (2H, s, ArOCH₂CO), 4.01 (2H, t, J=7Hz, NCH₂-Pr), 1.85 (3H, s, 5-CH₃).
MASS (EI) m/z : 427(M⁺), 384, 311, 267, 132, 116.

### (Example 1147)

### Compound of Formula No. C-499

¹H-NMR (CDCl₃) δ(ppm) : 7.23 (1H, s, 3-H), 4.78 (2H, brs, NCH₂Ar), 4.42 (2H, s, ArOCH₂CO), 4.00 (2H, t, J=7Hz, NCH₂-Pr), 1.85 (3H, s, 5-CH₃).
MASS (EI) m/z : 427(M⁺), 384, 311, 267, 132, 116.

### (Example 1148)

### Compound of Formula No. C-500

¹H-NMR (CDCl₃) δ(ppm) : 7.17 (1H, s, 3-H), 4.77 (2H, brs, NCH₂Ar), 4.39 (2H, s, ArOCH₂CO), 3.98 (2H, t, J=7Hz, NCH₂-Pr), 1.81 (3H, s, 5-CH₃).
MASS (EI) m/z : 438(M⁺), 322, 309, 267, 143, 116.

### (Example 1149)

### Compound of Formula No. C-501

¹H-NMR (CDCl₃) δ(ppm) : 7.20 (1H, s, 3-H), 4.77 (2H, brs, NCH₂Ar), 4.42 (2H, s, ArOCH₂CO), 3.99 (2H, t, J=7Hz, NCH₂-Pr), 1.83 (3H, s, 5-CH₃).
MASS (EI) m/z : 470(M⁺), 435, 354, 309, 267, 175, 116.

### (Example 1150)

### Compound of Formula No. C-502

¹H-NMR (CDCl₃) δ(ppm) : 7.21 (1H, s, 3-H), 4.78 (2H, brs, NCH₂Ar), 4.33 (2H, s, ArOCH₂CO), 4.01 (2H, t, J=7Hz, NCH₂-Pr), 1.85 (3H, s, 5-CH₃).
MASS (EI) m/z : 470(M⁺), 435, 354, 309, 267, 175, 116.

### (Example 1151)

### Compound of Formula No. C-503

¹H-NMR (CDCl₃) δ(ppm) : 7.25 (1H, s, 3-H), 4.76 (2H, brs, NCH₂Ar), 4.66 (2H, brs, ArOCH₂CO), 3.98 (2H, t, J=7Hz, NCH₂-Pr), 1.87 (3H, s, 5-CH₃).
MASS (EI) m/z : 492(M⁺), 447, 325, 267, 197, 167, 116.

### (Example 1152)

### Compound of Formula No. C-504

¹H-NMR (CDCl₃) δ(ppm) : 7.20 (1H, s, 3-H), 4.79 (2H, brs, NCH₂Ar), 4.44 (2H, s, ArOCH₂CO), 3.78 (2H, d, J=7Hz, NCH₂-iPr), 1.81 (3H, s, 5-CH₃).
MASS (EI) m/z : 420(M⁺), 309, 267, 151, 125, 116.

### (Example 1153)

### Compound of Formula No. C-505

¹H-NMR (CDCl₃) δ(ppm) : 7.23 (1H, s, 3-H), 4.79 (2H, brs, NCH₂Ar), 4.35 (2H, s, ArOCH₂CO), 3.79 (2H, d, J=7Hz, NCH₂-iPr), 1.82 (3H, s, 5-CH₃).
MASS (EI) m/z : 420(M⁺), 304, 267, 149, 125, 116.

### (Example 1154)

### Compound of Formula No. C-506

¹H-NMR (CDCl₃) δ(ppm) : 7.22 (1H, s, 3-H), 4.79 (2H, brs, NCH₂Ar), 4.32 (2H, s, ArOCH₂CO), 3.78 (2H, d, J = 7Hz, NCH₂-iPr), 1.80 (3H, s, 5-CH₃).
MASS (EI) m/z : 420(M⁺), 309, 267, 253, 125, 116.

### (Example 1155)

### Compound of Formula No. C-507

¹H-NMR (CDCl₃) δ(ppm) : 7.22 (1H, s, 3-H), 4.79 (2H, brs, NCH₂Ar), 4.45 (2H, s, ArOCH₂CO), 3.78 (2H, d, J=7Hz, NCH₂-iPr), 1.82 (3H, s, 5-CH₃).
MASS (EI) m/z : 436(M⁺), 401, 345, 320, 267, 116.

### (Example 1156)

### Compound of Formula No. C-508

¹H-NMR (CDCl₃) δ(ppm) : 7.23 (1H, s, 3-H), 4.79 (2H, brs, NCH₂Ar), 4.34 (2H, s, ArOCH₂CO), 3.79 (2H, d, J=7Hz, NCH₂-iPr), 1.82 (3H, s, 5-CH₃).
MASS (EI) m/z : 436(M⁺), 320, 295, 267, 116.

### (Example 1157)

### Compound of Formula No. C-509

¹H-NMR (CDCl₃) δ(ppm) : 7.23 (1H, s, 3-H), 4.78 (2H, brs, NCH₂Ar), 4.33 (2H, s, ArOCH₂CO), 3.78 (2H, d, J=7Hz, NCH₂-iPr), 1.80 (3H, s, 5-CH₃).
MASS (EI) m/z : 436(M⁺), 320, 309, 295, 267.

### (Example 1158)

### Compound of Formula No. C-510

¹H-NMR (CDCl₃) δ(ppm) : 7.21 (1H, s, 3-H), 4.79 (2H, brs, NCH₂Ar), 4.38 (2H, s, ArOCH₂CO), 3.78 (2H, d, J=7Hz, NCH₂-iPr), 1.81 (3H, s, 5-CH₃).
MASS (EI) m/z : 416(M⁺), 309, 300, 267, 253, 121.

### (Example 1159)

### Compound of Formula No. C-511

¹H-NMR (CDCl₃) δ(ppm) : 7.22 (1H, s, 3-H), 4.79 (2H, brs, NCH₂Ar), 4.34 (2H, s, ArOCH₂CO), 3.78 (2H, d, J=7Hz, NCH₂-iPr), 1.81 (3H, s, 5-CH₃).
MASS (EI) m/z : 416(M⁺), 309, 300, 267, 253, 121.

### (Example 1160)

### Compound of Formula No. C-512

¹H-NMR (CDCl₃) δ(ppm) : 7.21 (1H, s, 3-H), 4.78 (2H, brs, NCH₂Ar), 4.32 (2H, s, ArOCH₂CO), 3.77 (2H, d, J=7Hz, NCH₂-iPr), 1.79 (3H, s, 5-CH₃).
MASS (EI) m/z : 416(M⁺), 309, 300, 267, 253, 121.

### (Example 1161)

### Compound of Formula No. C-513

¹H-NMR (CDCl₃) δ(ppm) : 7.20 (1H, s, 3-H), 4.78 (2H, brs, NCH₂Ar), 4.42 (2H, s, ArOCH₂CO), 3.77 (2H, d, J=7Hz, NCH₂-iPr), 1.79 (3H, s, 5-CH₃).
MASS (EI) m/z : 432(M⁺), 309, 267, 253, 137, 116.

### (Example 1162)

### Compound of Formula No. C-514

¹H-NMR (CDCl₃) δ(ppm) : 7.22 (1H, s, 3-H), 4.79 (2H, brs, NCH₂Ar), 4.34 (2H, s, ArOCH₂CO), 3.78 (2H, d, J=7Hz, NCH₂-iPr), 1.81 (3H, s, 5-CH₃).
MASS (EI) m/z : 432(M⁺), 309, 295, 266, 253, 137, 116.

### (Example 1163)

### Compound of Formula No. C-515

¹H-NMR (CDCl₃) δ(ppm) : 7.21 (1H, s, 3-H), 4.79 (2H, brs, NCH₂Ar), 4.30 (2H, s, ArOCH₂CO), 3.77 (2H, d, J=7Hz, NCH₂-iPr), 1.79 (3H, s, 5-CH₃).
MASS (EI) m/z : 432(M⁺), 309, 267, 253, 137, 116.

### (Example 1164)

### Compound of Formula No. C-516

¹H-NMR (CDCl₃) δ(ppm) : 7.23 (1H, s, 3-H), 4.79 (2H, brs, NCH₂Ar), 4.34 (2H, s, ArOCH₂CO), 3.78 (2H, d, J=7Hz, NCH₂-iPr), 1.80 (3H, s, 5-CH₃).
MASS (EI) m/z : 494(M⁺), 309, 286, 186, 116.

### (Example 1165)

### Compound of Formula No. C-517

¹H-NMR (CDCl₃) δ(ppm) : 7.22 (1H, s, 3-H), 4.78 (2H, brs, NCH₂Ar), 4.54 (2H, s, ArOCH₂CO), 3.79 (2H, d, J=7Hz, NCH₂-iPr), 1.83 (3H, s, 5-CH₃).
MASS (EI) m/z : 447(M⁺), 401, 325, 267, 253, 210, 152.

### (Example 1166)

### Compound of Formula No. C-518

¹H-NMR (CDCl₃) δ(ppm) : 7.28 (1H, s, 3-H), 4.81 (2H, brs, NCH₂Ar), 4.46 (2H, s, ArOCH₂CO), 3.81 (2H, d, J=7Hz, NCH₂-iPr), 1.89 (3H, s, 5-CH₃).
MASS (EI) m/z : 447(M⁺), 331, 267, 210, 167, 116.

### (Example 1167)

### Compound of Formula No. C-519

¹H-NMR (CDCl₃) δ(ppm) : 7.27 (1H, s, 3-H), 4.80 (2H, brs, NCH₂Ar), 4.47 (2H, s, ArOCH₂CO), 3.81 (2H, d, J=7Hz, NCH₂-iPr), 1.84 (3H, s, 5-CH₃).
MASS (EI) m/2 : 447(M+), 267, 210, 116.

### (Example 1168)

### Compound of Formula No. C-520

¹H-NMR (CDCl₃) δ(ppm) : 7.24 (1H, s, 3-H), 4.80 (2H, brs, NCH₂Ar), 4.48 (2H, s, ArOCH₂CO), 3.79 (2H, d, J=7Hz, NCH₂-iPr), 1.83 (3H, s, 5-CH₃).
MASS (EI) m/z : 470(M⁺), 451, 354, 309, 267, 253, 175, 116.

### (Example 1169)

### Compound of Formula No. C-521

¹H-NMR (CDCl₃) δ (ppm) : 7.24 (1H, s, 3-H), 4.80 (2H, brs, NCH₂Ar), 4.40 (2H, s, ArOCH₂CO), 3.80 (2H, d, J=7Hz, NCH₂-iPr), 1.83 (3H, s, 5-CH₃).
MASS (EI) m/z : 470 (M⁺), 451, 354, 309, 267, 253, 175, 116.

### (Example 1170)

### Compound of Formula No. C-522

¹H-NMR (CDCl₃) δ(ppm) : 7.25 (1H, s, 3-H), 4.79 (2H, brs, NCH₂Ar), 4.41 (2H, s, ArOCH₂CO), 3.79 (2H, d, J=7Hz, NCH₂-iPr), 1.82 (3H, s, 5-CH₃).
MASS (EI) m/z : 470(M⁺), 451, 354, 309, 267, 253, 175, 116.

### (Example 1171)

### Compound of Formula No. C-523

¹H-NMR (CDCl₃) δ(ppm) : 7.24 (1H, s, 3-H), 4.78 (2H, brs, NCH₂Ar), 4.53 (2H, s, ArOCH₂CO), 3.79 (2H, d, J=7Hz, NCH₂-iPr), 1.86 (3H, s, 5-CH₃).
MASS (EI) m/z : 427(M⁺), 384, 311, 267, 132, 116.

### (Example 1172)

### Compound of Formula No. C-524

¹H-NMR (CDCl₃) δ(ppm) : 7.26 (1H, s, 3-H), 4.80 (2H, brs, NCH₂Ar), 4.39 (2H, s, ArOCH₂CO), 3.80 (2H, d, J=7Hz, NCH₂-iPr), 1.83 (3H, s, 5-CH₃).
MASS (EI) m/z : 427(M⁺), 371, 311, 267, 132, 116.

### (Example 1173)

### Compound of Formula No. C-525

¹H-NMR (CDCl₃) δ(ppm) : 7.25 (1H, s, 3-H), 4.79 (2H, brs, NCH₂Ar), 4.42 (2H, s, ArOCH₂CO), 3.80 (2H, d, J=7Hz, NCH₂-iPr), 1.82 (3H, s, 5-CH₃).
MASS (EI) m/z : 427(M⁺), 311, 267, 132, 116.

### (Example 1174)

### Compound of Formula No. C-526

¹H-NMR (CDCl₃) δ(ppm) : 7.20 (1H, s, 3-H), 4.78 (2H, brs, NCH₂Ar), 4.40 (2H, s, ArOCH₂CO), 3.77 (2H, d, J=7Hz, NCH₂-iPr), 1.79 (3H, s, 5-CH₃).
MASS (EI) m/z : 438(M⁺), 322, 309, 267, 143, 116.

### (Example 1175)

### Compound of Formula No. C-527

¹H-NMR (CDCl₃) δ(ppm) : 7.23 (1H, s, 3-H), 4.78 (2H, brs, MCH₂Ar), 4.43 (2H, s, ArOCH₂CO), 3.78 (2H, d, J=7Hz, NCH₂-iPr), 1.81 (3H, s, 5-CH₃).
MASS (EI) m/z : 470(M⁺), 435, 416, 354, 309, 267, 116.

### (Example 1176)

### Compound of Formula No. C-528

¹H-NMR (CDCl₃) δ(ppm) : 7.24 (1H, s, 3-H), 4.79 (2H, brs, NCH₂Ar), 4.33 (2H, s, ArOCH₂CO), 3.80 (2H, d, J=7Hz, NCH₂-iPr), 1.83 (3H, s, 5-CH₃).
MASS (EI) m/z : 470(M⁺), 354, 309, 267, 116.

### (Example 1177)

### Compound of Formula No. C-529

¹H-NMR (CDCl₃) δ(ppm) : 7.28 (1H, s, 3-H), 4.77 (2H, brs, NCH₂Ar), 4.67 (2H, s, ArOCH₂CO), 3.81 (2H, d, J=7Hz, NCH₂-iPr), 1.84 (3H, s, 5-CH₃).
MASS (EI) m/z : 492(M⁺), 413, 376, 309, 267, 116.

### (Example 1178)

### Compound of Formula No. C-530

¹H-NMR (CDCl₃) δ(ppm) : 7.18 (1H, s, 3-H), 4.78 (2H, brs, NCH₂Ar), 4.43 (2H, s, ArOCH₂CO), 3.99 (2H, d, J=7Hz, NCH₂-cBu), 1.81 (3H, s, 5-CH₃).
MASS (EI) m/z : 432(M⁺), 321, 279, 253, 125, 116.

### (Example 1179)

### Compound of Formula No. C-531

¹H-NMR (CDCl₃) δ(ppm) : 7.20 (1H, s, 3-H), 4.78 (2H, brs, NCH₂Ar), 4.33 (2H, s, ArOCH₂CO), 4.00 (2H, d, J=7Hz, NCH₂-cBu), 1.82 (3H, s, 5-CH₃).
MASS (EI) m/z : 432(M⁺), 321, 279, 253, 125, 116.

### (Example 1180)

### Compound of Formula No. C-532

¹H-NMR (CDCl₃) δ(ppm) : 7.20 (1H, s, 3-H), 4.78 (2H, brs, NCH₂Ar), 4.32 (2H, s, ArOCH₂CO), 4.00 (2H, d, J=7Hz, NCH₂-cBu), 1.82 (3H, s, 5-CH₃).
MASS (EI) m/z : 432(M⁺), 321, 279, 253, 125, 116.

### (Example 1181)

### Compound of Formula No. C-533

¹H-NMR (CDCl₃) δ(ppm) : 7.21 (1H, s, 3-H), 4.78 (2H, brs, NCH₂Ar), 4.44 (2H, s, ArOCH₂CO), 3.99 (2H, d, J=7Hz, NCH₂-cBu), 1.82 (3H, s, 5-CH₃).
MASS (EI) m/z : 448(M⁺), 413, 321, 279, 253, 149, 116.

### (Example 1182)

### Compound of Formula No. C-534

¹H-NMR (CDCl₃) δ(ppm) : 7.20 (1H, s, 3-H), 4.78 (2H, brs, NCH₂Ar), 4.33 (2H, s, ArOCH₂CO), 4.00 (2H, d, J=7Hz, NCH₂-cBu), 1.83 (3H, s, 5-CH₃).
MASS (EI) m/z : 448(M⁺), 332, 321, 279, 253, 141, 116.

### (Example 1183)

### Compound of Formula No. C-535

¹H-NMR (CDCl₃) δ(ppm) : 7.20 (1H, s, 3-H), 4.77 (2H, brs, NCH₂Ar), 4.32 (2H, s, ArOCH₂CO), 3.99 (2H, d, J=7Hz, NCH₂-cBu), 1.81 (3H, s, 5-CH₃).
MASS (EI) m/z : 448(M⁺), 332, 321, 279, 253, 141, 116.

### (Example 1184)

### Compound of Formula No. C-536

¹H-NMR (CDCl₃) δ(ppm) : 7.18 (1H, s, 3-H), 4.79 (2H, brs, NCH₂Ar), 4.37 (2H, s, ArOCH₂CO), 3.99 (2H, d, J=7Hz, NCH₂-cBu), 1.82 (3H, s, 5-CH₃).
MASS (EI) m/z : 428(M⁺), 321, 280, 253, 121, 116.

### (Example 1185)

### Compound of Formula No. C-537

¹H-NMR (CDCl₃) δ(ppm) : 7.19 (1H, s, 3-H), 4.78 (2H, brs, NCH₂Ar), 4.32 (2H, s, ArOCH₂CO), 3.99 (2H, d, J=7Hz, NCH₂-cBu), 1.82 (3H, s, 5-CH₃).
MASS (EI) m/z : 428(M⁺), 321, 279, 253, 121, 116.

### (Example 1186)

### Compound of Formula No. C-538

¹H-NMR (CDCl₃) δ(ppm) : 7.18 (1H, s, 3-H), 4.78 (2H, brs, NCH₂Ar), 4.31 (2H, s, ArOCH₂CO), 3.98 (2H, d, J=7Hz, NCH₂-cBu), 1.80 (3H, s, 5-CH₃).
MASS (EI) m/z : 428(M⁺), 344, 321, 279, 253, 121, 116.

### (Example 1187)

### Compound of Formula No. C-539

¹H-NMR (CDCl₃) δ(ppm) : 7.17 (1H, s, 3-H), 4.77 (2H, brs, NCH₂Ar), 4.41 (2H, s, ArOCH₂CO), 3.98 (2H, d, J=7Hz, NCH₂-cBu), 1.80 (3H, s, 5-CH₃).
MASS (EI) m/z : 444(M⁺), 321, 279, 253, 137, 116.

### (Example 1188)

### Compound of Formula No. C-540

¹H-NMR (CDCl₃) δ(ppm) : 7.19 (1H, s, 3-H), 4.78 (2H, brs, NCH₂Ar), 4.32 (2H, s, ArOCH₂CO), 3.99 (2H, d, J=7Hz, NCH₂-cBu), 1.82 (3H, s, 5-CH₃).
MASS (EI) m/z : 444(M⁺), 321, 279, 253, 137, 116.

### (Example 1189)

### Compound of Formula No. C-541

¹H-NMR (CDCl₃) δ(ppm) : 7.18 (1H, s, 3-H), 4.78 (2H, brs, NCH₂Ar), 4.29 (2H, s, ArOCH₂CO), 3.99 (2H, d, J=7Hz, NCH₂-cBu), 1.80 (3H, s, 5-CH₃).
MASS (EI) m/z : 444(M⁺), 321, 279, 253, 137, 116.

### (Example 1190)

### Compound of Formula No. C-542

¹H-NMR (CDCl₃) δ(ppm) : 7.19 (1H, s, 3-H), 4.77 (2H, brs, NCH₂Ar), 4.52 (2H, s, ArOCH₂CO), 4.00 (2H, d, J=7Hz, NCH₂-cBu), 1.83 (3H, S, 5-CH₃).
MASS (EI) m/z : 459(M⁺), 413, 279, 253, 152, 116.

### (Example 1191)

### Compound of Formula No. C-543

¹H-NMR (CDCl₃) δ(ppm) : 7.24 (1H, s, 3-H), 4.79 (2H, brs, NCH₂Ar), 4.44 (2H, brs, ArOCH₂CO), 4.02 (2H, d, J=7Hz, NCH₂-cBu), 1.89 (3H, s, 5-CH₃).
MASS (EI) m/z : 459(M⁺), 343, 321, 279, 253, 152, 116.

### (Example 1192)

### Compound of Formula No. C-544

¹H-NMR (CDCl₃) δ(ppm) : 7.24 (1H, s, 3-H), 4.78 (2H, brs, NCH₂Ar), 4.45 (2H, s, ArOCH₂CO), 4.02 (2H, d, J=7Hz, NCH₂-cBu), 1.84 (3H, s, 5-CH₃).
MASS (EI) m/z : 459(M⁺), 343, 321, 279, 253, 152, 116.

### (Example 1193)

### Compound of Formula No. C-545

¹H-NMR (CDCl₃) δ(ppm) : 7.20 (1H, s, 3-H), 4.78 (2H, brs, NCH₂Ar), 4.46 (2H, s, ArOCH₂CO), 4.00 (2H, d, J=7Hz, NCH₂-cBu), 1.84 (3H, s, 5-CH₃).
MASS (EI) m/z : 482(M⁺), 366, 321, 279, 253, 175, 116.

### (Example 1194)

### Compound of Formula No. C-546

¹H-NMR (CDCl₃) δ(ppm) : 7.21 (1H, s, 3-H), 4.78 (2H, brs, NCH₂Ar), 4.38 (2H, s, ArOCH₂CO), 4.00 (2H, d, J=7Hz, NCH₂-cBu), 1.84 (3H, s, 5-CH₃).
MASS (EI) m/z : 482(M⁺), 366, 321, 279, 253, 175, 116.

### (Example 1195)

### Compound of Formula No. C-547

¹H-NMR (CDCl₃) δ(ppm) : 7.22 (1H, s, 3-H), 4.78 (2H, brs, NCH₂Ar), 4.40 (2H, s, ArOCH₂CO), 4.00 (2H, d, J=7Hz, NCH₂-cBu), 1.83 (3H, s, 5-CH₃).
MASS (EI) m/z : 482(M⁺), 366, 321, 279, 253, 175, 116.

### (Example 1196)

### Compound of Formula No. C-548

¹H-NMR (CDCl₃) δ(ppm) : 7.20 (1H, s, 3-H), 4.78 (2H, brs, NCH₂Ar), 4.52 (2H, s, ArOCH₂CO), 4.01 (2H, d, J=7Hz, NCH₂-cBu), 1.87 (3H, S, 5-CH₃).
MASS (EI) m/z : 439(M⁺), 411, 323, 279, 253, 132, 116.

### (Example 1197)

### Compound of Formula No. C-549

¹H-NMR (CDCl₃) δ(ppm) : 7.23 (1H, s, 3-H), 4.78 (2H, brs, NCH₂Ar), 4.37 (2H, s, ArOCH₂CO), 4.02 (2H, d, J=7Hz, NCH₂-cBu), 1.84 (3H, s, 5-CH₃).
MASS (EI) m/z : 439(M⁺), 323, 279, 253, 132, 116.

### (Example 1198)

### Compound of Formula No. C-550

¹H-NMR (CDCl₃) δ(ppm) : 7.22 (1H, s, 3-H), 4.77 (2H, brs, NCH₂Ar), 4.40 (2H, s, ArOCH₂CO), 4.01 (2H, d, J=7Hz, NCH₂-cBu), 1.83 (3H, s, 5-CH₃).
MASS (EI) m/z : 439(M⁺), 323, 279, 253, 132, 116.

### (Example 1199)

### Compound of Formula No. C-551

¹H-NMR (CDCl₃) δ(ppm) : 7.17 (1H, s, 3-H), 4.77 (2H, brs, NCH₂Ar), 4.38 (2H, s, ArOCH₂CO), 3.99 (2H, d, J=7Hz, NCH₂-cBu), 1.80 (3H, s, 5-CH₃).
MASS (EI) m/z : 450(M⁺), 321, 279, 253, 143, 116.

### (Example 1200)

### Compound of Formula No. C-552

¹H-NMR (CDCl₃) δ(ppm) : 7.20 (1H, s, 3-H), 4.77 (2H, brs, NCH₂Ar), 4.41 (2H, s, ArOCH₂CO), 3.99 (2H, d, J=7Hz, NCH₂-cBu), 1.82 (3H, s, 5-CH₃).
MASS (EI) m/z : 482(M⁺), 447, 321, 279, 253, 175, 116.

### (Example 1201)

### Compound of Formula No. C-553

¹H-NMR (CDCl₃) δ(ppm) : 7.21 (1H, s, 3-H), 4.78 (2H, brs, NCH₂Ar), 4.32 (2H, s, ArOCH₂CO), 4.01 (2H, d, J=7Hz, NCH₂-cBu), 1.84 (3H, s, 5-CH₃).
MASS (EI) m/z : 482(M⁺), 366, 321, 279, 253, 175, 116.

### (Example 1202)

### Compound of Formula No. C-554

¹H-NMR (CDCl₃) δ(ppm) : 7.24 (1H, s, 3-H), 4.74 (2H, brs, NCH₂Ar), 4.65 (2H, brs, ArOCH₂CO), 4.02 (2H, d, J=7Hz, NCH₂-cBu), 1.85 (3H, s, 5-CH₃).
MASS (EI) m/z : 504(M⁺), 388, 321, 280, 253, 210, 167, 116.

### (Example 1203)

### Compound of Formula No. C-561

¹H-NMR (CDCl₃) δ(ppm) : 7.24 (1H, s, 3-H), 4.79 (2H, brs, NCH₂Ar), 4.41 (2H, s, ArOCH₂CO), 3.79 (2H, d, J=7Hz, NCH₂-iPr), 1.82 (3H, s, 5-CH₃).
MASS (EI) m/z : 403(M⁺), 388, 360, 287, 267, 136, 116.

### (Example 1204)

### Compound of Formula No. C-563

¹H-NMR (CDCl₃) δ(ppm) : 7.23 (1H, S, 3-H), 4.79 (2H, brs, NCH₂Ar), 4.43 (2H, s, COCH₂O), 4.12 (2H, d, J=7Hz, NCH₂-iPr), 1.83 (3H, s, 5-CH₃).
MASS (EI) m/z : 354(M⁺), 339, 311, 267, 238, 210, 192, 180.

### (Example 1205)

### Compound of Formula No. C-564

¹H-NMR (CDCl₃) δ(ppm) : 7.21 (1H, s, 3-H), 4.75 (2H, brs, NCH₂Ar), 4.34 (2H, s, COCH₂O), 3.78 (2H, d, J=7Hz, NCH₂-iPr), 1.84 (3H, s, 5-CH₃).
MASS (EI) m/z : 382(M⁺), 325, 266, 210, 180, 116.

### (Example 1206)

### Compound of Formula No. C-565

¹H-NMR (CDCl₃) δ(ppm) : 7.20 (1H, s, 3-H), 4.75 (2H, brs, NCH₂Ar), 4.34 (2H, s, COCH₂O), 3.77 (2H, d, J=7Hz, NCH₂-iPr), 1.83 (3H, s, 5-CH₃).
MASS (EI) m/z : 395(M+1), 325, 268, 210, 180, 116.

### (Example 1207)

### Compound of Formula No. C-566

¹H-NMR (CDCl₃) δ(ppm) : 7.22 (1H, s, 3-H), 4.76 (2H, brs, NCH₂Ar), 4.36 (2H, s, COCH₂O), 3.78 (2H, d, J=7Hz, NCH₂-iPr), 1.84 (3H, s, 5-CH₃).
MASS (EI) m/z : 394(M⁺), 379, 325, 278, 266, 210, 152.

### (Example 1208)

### Compound of Formula No. C-567

¹H-NMR (CDCl₃) δ(ppm) : 7.22 (1H, s, 3-H), 4.76 (2H, brs, NCH₂Ar), 4.39 (2H, s, COCH₂O), 3.78 (2H, d, J=7Hz, NCH₂-iPr), 1.84 (3H, s, 5-CH₃).
MASS (EI) m/z : 394(M⁺), 325, 268, 210, 180, 152, 116.

### (Example 1209)

### Compound of Formula No. C-568

¹H-NMR (CDCl₃) δ(ppm) : 7.21 (1H, s, 3-H), 4.75 (2H, brs, NCH₂Ar), 4.33 (2H, s, COCH₂O), 3.78 (2H, d, J=7Hz, NCH₂-iPr), 1.85 (3H, s, 5-CH₃).
MASS (EI) m/z : 408(M⁺), 325, 268, 210, 180, 116.

### (Example 1210)

### Compound of Formula No. C-569

¹H-NMR (CDCl₃) δ(ppm) : 7.21 (1H, s, 3-H), 4.75 (2H, brs, NCH₂Ar), 4.33 (2H, s, COCH₂O), 3.77 (2H, d, J=7Hz, NCH₂-iPr), 1.83 (3H, s, 5-CH₃).
MASS (EI) m/z : 410(M⁺), 395, 326, 268, 210, 180, 116.

### (Example 1211)

### Compound of Formula No. C-570

¹H-NMR (CDCl₃) δ(ppm) : 7.20 (1H, s, 3-H), 4.76 (2H, brs, NCH₂Ar), 4.33 (2H, s, COCH₂O), 3.77 (2H, d, J=7Hz, NCH₂-iPr), 1.86 (3H, s, 5-CH₃).
MASS (EI) m/z : 410(M⁺), 395, 325, 268, 210, 116.

### (Example 1212)

### Compound of Formula No. C-571

¹H-NMR (CDCl₃) δ(ppm) : 7.23 (1H, s, 3-H), 4.76 (2H, brs, NCH₂Ar), 4.36 (2H, s, COCH₂O), 3.78 (2H, d, J=7Hz, NCH₂-iPr), 1.85 (3H, s, 5-CH₃).
MASS (EI) m/z : 408(M⁺), 268, 141, 116.

### (Example 1213)

### Compound of Formula No. C-572

¹H-NMR (CDCl₃) δ(ppm) : 7.23 (1H, s, 3-H), 4.76 (2H, brs, NCH₂Ar), 4.40 (2H, s, COCH₂O), 3.78 (2H, d, J=7Hz, NCH₂-iPr), 1.87 (3H, s, 5-CH₃).
MASS (EI) m/z : 408(M⁺), 325, 268, 141, 116.

### (Example 1214)

### Compound of Formula No. C-573

¹H-NMR (CDCl₃) δ(ppm) : 7.22 (1H, s, 3-H), 4.76 (2H, brs, NCH₂Ar), 4.35 (2H, s, COCH₂O), 3.78 (2H, d, J=7Hz, NCH₂-iPr), 1.83 (3H, s, 5-CH₃).
MASS (EI) m/z : 408(M⁺), 327, 266, 210, 180, 116.

### (Example 1215)

### Compound of Formula No. C-574

¹H-NMR (CDCl₃) δ(ppm) : 7.21 (1H, s, 3-H), 4.75 (2H, brs, NCH₂Ar), 4.35 (2H, s, COCH₂O), 3.77 (2H, d, J=7Hz, NCH₂-iPr), 1.84 (3H, s, 5-CH₃).
MASS (EI) m/z : 408(M⁺), 350, 327, 268, 210, 180, 152.

### (Example 1216)

### Compound of Formula No. C-575

¹H-NMR (CDCl₃) δ(ppm) : 7.21 (1H, s, 3-H), 4.75 (2H, brs, NCH₂Ar), 4.34 (2H, s, COCH₂O), 3.77 (2H, d, J=7Hz, NCH₂-iPr), 1.83 (3H, s, 5-CH₃).
MASS (EI) m/z : 422(M⁺), 344, 327, 266, 228, 210, 152.

### (Example 1217)

### Compound of Formula No. C-576

¹H-NMR (CDCl₃) δ(ppm) : 7.21 (1H, s, 3-H), 4.76 (2H, brs, NCH₂Ar), 4.34 (2H, s, COCH₂O), 3.76 (2H, d, J=7Hz, NCH₂-iPr), 1.83 (3H, s, 5-CH₃).
MASS (EI) m/z : 422(M⁺), 327, 266, 228, 210, 152, 124.

### (Example 1218)

### Compound of Formula No. C-577

¹H-NMR (CDCl₃) δ(ppm) : 7.22 (1H, s, 3-H), 4.76 (2H, brs, NCH₂Ar), 4.34 (2H, s, COCH₂O), 3.78 (2H, d, J=7Hz, NCH₂-iPr), 1.84 (3H, s, 5-CH₃).
MASS (EI) m/z : 422(M⁺), 327, 279, 266, 228, 210, 180.

### (Example 1219)

### Compound of Formula No. C-578

¹H-NMR (CDCl₃) δ(ppm) : 7.20 (1H, s, 3-H), 4.75 (2H, brs, NCH₂Ar), 4.33 (2H, s, COCH₂O), 3.77 (2H, d, J=7Hz, NCH₂-iPr), 1.84 (3H, s, 5-CH₃).
MASS (EI) m/z : 422(M⁺), 326, 268, 210, 180, 116.

### (Example 1220)

### Compound of Formula No. C-579

¹H-NMR (CDCl₃) δ(ppm) : 7.19 (1H, s, 3-H), 4.74 (2H, brs, NCH₂Ar), 4.32 (2H, s, COCH₂O), 3.76 (2H, d, J=7Hz, NCH₂-iPr), 1.83 (3H, s, 5-CH₃).
MASS (EI) m/z : 436(M⁺), 326, 268, 210, 180, 116.

### (Example 1221)

### Compound of Formula No. C-580

¹H-NMR (CDCl₃) δ(ppm) : 7.21 (1H, s, 3-H), 4.75 (2H, brs, NCH₂Ar), 4.35 (2H, s, COCH₂O), 3.77 (2H, d, J=7Hz, NCH₂-iPr), 1.85 (3H, s, 5-CH₃).
MASS (EI) m/z : 434(M⁺), 327, 267, 210, 180, 152, 116.

### (Example 1222)

### Compound of Formula No. C-581

¹H-NMR (CDCl₃) δ(ppm) : 7.27 (1H, s, 3-H), 4.80 (2H, brs, NCH₂Ar), 4.57 (2H, s, COCH₂O), 3.80 (2H, d, J=7Hz, NCH₂-iPr), 1.89 (3H, s, 5-CH₃).
MASS (EI) m/z : 444(M-2), 268, 177, 119.

### (Example 1223)

### Compound of Formula No. C-582

¹H-NMR (CDCl₃) δ(ppm) : 7.21 (1H, s, 3-H), 4.76 (2H, brs, NCH₂Ar), 4.35 (2H, s, COCH₂O), 3.78 (2H, d, J=7Hz, NCH₂-iPr), 1.84 (3H, s, 5-CH₃).
MASS (EI) m/z : 434(M⁺), 368, 327, 267, 210, 180, 152.

### (Example 1224)

### Compound of Formula No. C-583

¹H-NMR (CDCl₃) δ(ppm) : 7.26 (1H, s, 3-H), 4.79 (2H, brs, NCH₂Ar), 4.58 (2H, s, COCH₂O), 3.80 (2H, d, J=7Hz, NCH₂-iPr), 1.89 (3H, s, 5-CH₃).
MASS (EI) m/z : 430(M⁺), 268, 210, 163, 116.

### (Example 1225)

### Compound of Formula No. C-584

¹H-NMR (CDCl₃) δ(ppm) : 7.19 (1H, s, 3-H), 4.74 (2H, brs, NCH₂Ar), 4.35 (2H, s, COCH₂O), 3.75 (2H, d, J=7Hz, NCH₂-iPr), 1.81 (3H, s, 5-CH₃).
MASS (EI) m/z : 444(M⁺), 326, 266, 210, 180, 152, 116.

### (Example 1226)

### Compound of Formula No. C-585

¹H-NMR (CDCl₃) δ(ppm) : 7.22 (1H, s, 3-H), 4.76 (2H, brs, NCH₂Ar), 4.37 (2H, s, COCH₂O), 3.77 (2H, d, J=7Hz, NCH₂-iPr), 1.83 (3H, s, 5-CH₃).
MASS (EI) m/z : 470(M⁺), 422, 326, 268, 180, 152, 117.

### (Example 1227)

### Compound of Formula No. C-586

¹H-NMR (CDCl₃) δ(ppm) : 7.26 (1H, s, 3-H), 4.79 (2H, brs, NCH₂Ar), 4.59 (2H, s, COCH₂O), 3.81 (2H, d, J=7Hz, NCH₂-iPr), 1.89 (3H, s, 5-CH₃).
MASS (EI) m/z : 448(M⁺), 266, 210, 181, 123, 116.

### (Example 1228)

### Compound of Formula No. C-587

¹H-NMR (CDCl₃) δ(ppm) : 7.26 (1H, s, 3-H), 4.77 (2H, brs, NCH₂Ar), 4.58 (2H, s, COCH₂O), 3.81 (2H, d, J=7Hz, NCH₂-iPr), 1.89 (3H, s, 5-CH₃).
MASS (EI) m/z : 448(M⁺), 266, 210, 181, 123, 116.

### (Example 1229)

### Compound of Formula No. C-588

¹H-NMR (CDCl₃) δ(ppm) : 7.26 (1H, s, 3-H), 4.78 (2H, brs, NCH₂Ar), 4.57 (2H, s, COCH₂O), 3.80 (2H, d, J=7Hz, NCH₂-iPr), 1.89 (3H, s, 5-CH₃).
MASS (EI) m/z : 448(M⁺), 268, 210, 181, 123, 116.

### (Example 1230)

### Compound of Formula No. C-589

¹H-NMR (CDCl₃) δ(ppm) : 7.28 (1H, s, 3-H), 4.79 (2H, brs, NCH₂Ar), 4.60 (2H, s, COCH₂O), 3.81 (2H, d, J=7Hz, NCH₂-ipr), 1.89 (3H, s, 5-CH₃).
MASS (EI) m/z : 464(M⁺), 325, 268, 210, 197, 139, 116.

### (Example 1231)

### Compound of Formula No. C-590

¹H-NMR (CDCl₃) δ(ppm) : 7.26 (1H, s, 3-H), 4.79 (2H, brs, NCH₂Ar), 4.58 (2H, s, COCH₂O), 3.81 (2H, d, J=7Hz, NCH₂-iPr), 1.89 (3H, s, 5-CH₃).
MASS (EI) m/Z : 464(M⁺), 266, 197, 139, 116.

### (Example 1232)

### Compound of Formula No. C-591

¹H-NMR (CDCl₃) δ(ppm) : 7.26 (1H, s, 3-H), 4.78 (2H, brs, NCH₂Ar), 4.57 (2H, s, COCH₂O), 3.80 (2H, d, J=7Hz, NCH₂-iPr), 1.89 (3H, s, 5-CH₃).
MASS (EI) m/z : 464(M⁺), 325, 268, 210, 197, 139, 116.

### (Example 1233)

### Compound of Formula No. C-592

¹H-NMR (CDCl₃) δ(ppm) : 7.26 (1H, s, 3-H), 4.78 (2H, brs, NCH₂Ar), 4.57 (2H, s, COCH₂O), 3.80 (2H, d, J=7Hz, NCH₂-iPr), 1.89 (3H, s, 5-CH₃).
MASS (EI) m/z : 444(M⁺), 268, 177, 119.

### (Example 1234)

### Compound of Formula No. C-593

¹H-NMR (CDCl₃) δ(ppm) : 7.22 (1H, s, 3-H), 4.78 (2H, brs, NCH₂Ar), 4.56 (2H, s, COCH₂O), 3.79 (2H, d, J=7Hz, NCH₂-iPr), 1.89 (3H, s, 5-CH₃).
MASS (EI) m/z : 444(M⁺), 268, 177, 119.

### (Example 1235)

### Compound of Formula No. C-594

¹H-NMR (CDCl₃) δ(ppm) : 7.26 (1H, s, 3-H), 4.78 (2H, brs, NCH₂Ar), 4.54 (2H, s, OCH₂CO), 3.79 (2H, d, J=7Hz, NCH₂-iPr), 1.89 (3H, s, 5-CH₃).
MASS (EI) m/z : 460(M⁺), 268, 193, 135, 116.

### (Example 1236)

### Compound of Formula No. C-595

¹H-NMR (CDCl₃) δ(ppm) : 7.27 (1H, s, 3-H), 4.76 (2H, brs, NCH₂Ar), 4.61 (2H, s, OCH₂CO), 3.81 (2H, d, J=7Hz, NCH₂-iPr), 1.89 (3H, s, 5-CH₃).
MASS (EI) m/z : 455(M⁺), 266, 210, 130, 116.

### (Example 1237)

### Compound of Formula No. C-596

¹H-NMR (CDCl₃) δ(ppm) : 7.26 (1H, s, 3-H), 4.78 (2H, brs, NCH₂Ar), 4.60 (2H, s, COCH₂O), 3.80 (2H, d, J=7Hz, NCH₂-iPr), 1.89 (3H, s, 5-CH₃).
MASS (EI) m/z : 455 (M⁺), 266, 210, 188, 130, 116.

### (Example 1238)

### Compound of Formula No. C-597

¹H-NMR (CDCl₃) δ(ppm) : 7.28 (1H, s, 3-H), 4.79 (2H, brs, NCH₂Ar), 4.58 (2H, s, COCH₂O), 3.81 (2H, d, J=7Hz, NCH₂-iPr), 1.89 (3H, s, 5-CH₃).
MASS (EI) m/z : 448(M⁺), 266, 199, 141, 123, 116.

### (Example 1239)

### Compound of Formula No. C-598

¹H-NMR (CDCl₃) δ(ppm) : 7.28 (1H, s, 3-H), 4.78 (2H, brs, NCH₂Ar), 4.58 (2H, s, COCH₂O), 3.81 (2H, d, J=7Hz, NCH₂-iPr), 1.89 (3H, s, 5-CH₃).
MASS (EI) m/z : 448(M⁺), 266, 210, 141, 123, 116.

### (Example 1240)

### Compound of Formula No. C-599

¹H-NMR (CDCl₃) δ(ppm) : 7.30 (1H, s, 3-H), 4.76 (2H, brs, NCH₂Ar), 4.58 (2H, s, COCH₂O), 3.82 (2H, d, J=7Hz, NCH₂-iPr), 1.89 (3H, s, 5-CH₃).
MASS (EI) m/z : 498(M⁺), 266, 210, 173, 149, 116.

### (Example 1241)

### Compound of Formula No. C-600

¹H-NMR (CDCl₃) δ(ppm) : 7.26 (1H, s, 3-H), 4.78. (2H, brs, NCH₂Ar), 4.61 (2H, s, COCH₂O), 3.80 (2H, d, J=7Hz, NCH₂-iPr), 1.90 (3H, s, 5-CH₃).
MASS (EI) m/z : 431(M⁺), 388, 315, 266, 210, 116.

### (Example 1242)

### Compound of Formula No. C-601

¹H-NMR (CDCl₃) δ(ppm) : 7.27 (1H, s, 3-H), 4.80 (2H, brs, NCH₂Ar), 4.61 (2H, s, COCH₂O), 3.80 (2H, d, J=7Hz, NCH₂-iPr), 1.89 (3H, s, 5-CH₃).
MASS (EI) m/z : 431(M⁺), 266, 210, 164, 136, 116.

### (Example 1243)

### Compound of Formula No. C-602

¹H-NMR (CDCl₃) δ(ppm) : 7.26 (1H, s, 3-H), 4.76 (2H, brs, NCH₂Ar), 4.61 (2H, s, COCH₂O), 3.80 (2H, d, J=7Hz, NCH₂-iPr), 1.87 (3H, s, 5-CH₃).
MASS (EI) m/z : 499(M⁺), 383, 266, 210, 174, 116.

### (Example 1244)

### Compound of Formula No. C-603

¹H-NMR (CDCl₃) δ(ppm) : 7.26 (1H, s, 3-H), 4.77 (2H, brs, NCH₂Ar), 4.58 (2H, s, COCH₂O), 3.80 (2H, d, J=7Hz, NCH₂-iPr), 1.88 (3H, s, 5-CH₃).
MASS (EI) m/z : 495(M⁺), 433, 266, 210, 170, 149, 116.

### (Example 1245)

### Compound of Formula No. C-604

¹H-NMR (CDCl₃) δ(ppm) : 7.23 (1H, s, 3-H), 4.77 (2H, brs, NCH₂Ar), 4.46 (2H, s, COCH₂O), 3.78 (2H, d, J=7Hz, NCH₂-iPr), 1.87 (3H, s, 5-CH₃).
MASS (EI) m/z : 461(M⁺), 194, 152, 116.

### (Example 1246)

### Compound of Formula No. C-605

¹H-NMR (CDCl₃) δ(ppm) : 7.26 (1H, s, 3-H), 4.76 (2H, brs, NCH₂Ar), 4.55 (2H, s, COCH₂O), 3.79 (2H, d, J=7Hz, NCH₂-iPr), 1.87 (3H, s, 5-CH₃).
MASS (EI) m/z : 420(M⁺), 266, 210, 153, 116.

### (Example 1247)

### Compound of Formula No. C-606

¹H-NMR (CDCl₃) δ(ppm) : 7.24 (1H, s, 3-H), 4.77 (2H, brs, NCH₂Ar), 4.47 (2H, s, COCH₂O), 3.78 (2H, d, J=7Hz, NCH₂-iPr), 1.87 (3H, s, 5-CH₃).
MASS (EI) m/z : 448(M⁺), 268, 181, 123.

### (Example 1248)

### Compound of Formula No. C-607

¹H-NMR (CDCl₃) δ(ppm) : 7.24 (1H, s, 3-H), 4.78 (2H, brs, NCH₂Ar), 4.25 (2H, s, COCH₂O), 3.79 (2H, d, J=7Hz, NCH₂-iPr), 1.87 (3H, s, 5-CH₃).
MASS (EI) m/z : 490(M⁺), 268, 223, 164, 149, 116.

### (Example 1249)

### Compound of Formula No. C-608

¹H-NMR (CDCl₃) δ(ppm) : 7.23 (1H, s, 3-H), 4.77 (2H, brs, NCH₂Ar), 4.52 (2H, s, COCH₂O), 3.78 (2H, d, J=7Hz, NCH₂-iPr), 1.86 (3H, s, 5-CH₃).
MASS (EI) m/z : 502(M⁺), 266, 235, 210, 177, 116.

### (Example 1250)

### Compound of Formula No. C-609

¹H-NMR (CDCl₃) δ(ppm) : 7.26 (1H, s, 3-H), 4.77 (2H, brs, NCH₂Ar), 4.54 (2H, s, COCH₂O), 3.80 (2H, d, J=7Hz, NCH₂-iPr), 1.88 (3H, s, 5-CH₃).
MASS (EI) m/z : 436(M⁺), 268, 210, 169, 116, 111.

### (Example 1251)

### Compound of Formula No. C-610

¹H-NMR (CDCl₃) δ(ppm) : 7.26 (1H, s, 3-H), 4.78 (2H, brs, NCH₂Ar), 4.58 (2H, s, COCH₂O), 3.80 (2H, d, J=7Hz, NCH₂-iPr), 1.89 (3H, s, 5-CH₃).
MASS (EI) m/z : 452(M⁺), 309, 267, 210, 167, 136.

### (Example 1252)

### Compound of Formula No. C-611

¹H-NMR (CDCl₃ δ(ppm) : 7.22 (1H, s, 3-H), 4.74 (2H, brs, NCH₂Ar), 4.42 (2H, s, COCH₂O), 3.78 (2H, d, J=7Hz, NCH₂-iPr), 1.83 (3H, s, 5-CH₃).
MASS (EI) m/z : 398(M⁺), 383, 325, 282, 266, 210, 116.

### (Example 1253)

### Compound of Formula No. C-612

¹H-NMR (CDCl₃) δ(ppm) :
7.20 (1H, s, 3-H), 4.75 (2H, brs, NCH₂Ar), 4.41 (2H, s, COCH₂O), 3.77 (2H, d, J=7Hz, NCH₂-iPr), 1.82 (3H, s, 5-CH₃).
MASS (EI) m/z : 427(M+1), 310, 266, 210, 180, 116.

### (Example 1254)

### Compound of Formula No. C-613

¹H-NMR (CDCl₃) δ(ppm) : 7.22 (1H, s, 3-H), 4.76 (2H, brs, NCH₂Ar), 4.46 (2H, s, COCH₂O), 3.77 (2H, d, J=7Hz, NCH₂-iPr), 1.82 (3H, s, 5-CH₃).
MASS (EI) m/z : 461(M+1), 367, 344, 286, 266, 210, 116.

### (Example 1255)

### Compound of Formula No. C-614

¹H-NMR (CDCl₃) δ(ppm) : 7.19 (1H, s, 3-H), 4.75 (2H, brs, NCH₂Ar), 4.39 (2H, s, COCH₂O), 3.76 (2H, d, J=7Hz, NCH₂-iPr), 1.82 (3H, s, 5-CH₃).
MASS (EI) m/z : 445(M⁺), 326, 226, 210, 192, 180, 152.

### (Example 1256)

### Compound of Formula No. C-615

¹H-NMR (CDCl₃) δ(ppm) : 7.21 (1H, s, 3-H), 4.75 (2H, brs, NCH₂Ar), 4.38 (2H, s, COCH₂O), 3.77 (2H, d, J=7Hz, NCH₂-iPr), 1.83 (3H, s, 5-CH₃).
MASS (EI) m/z : 445(M⁺), 430, 402, 267, 210, 151, 116.

### (Example 1257)

### Compound of Formula No. C-616

¹H-NMR (CDCl₃) δ(ppm) : 7.20 (1H, s, 3-H), 4.75 (2H, brs, NCH₂Ar), 4.38 (2H, s, COCH₂O), 3.76 (2H, d, J=7Hz, NCH₂-iPr), 1.82 (3H, s, 5-CH₃).
MASS (EI) m/z : 445(M⁺), 402, 326, 266, 210, 180, 152.

### (Example 1258)

### Compound of Formula No. C-617

¹H-NMR (CDCl₃) δ(ppm) : 7.20 (1H, s, 3-H), 4.76 (2H, brs, NCH₂Ar), 4.39 (2H, s, COCH₂O), 3.76 (2H, d, J=7Hz, NCH₂-iPr), 1.82 (3H, s, 5-CH₃).
MASS (EI) m/z : 450(M⁺), 326, 267, 210, 180, 136.

### (Example 1259)

### Compound of Formula No. C-618

¹H-NMR (CDCl₃) δ(ppm) : 7.20 (1H, s, 3-H), 4.75 (2H, brs, NCH₂Ar), 4.37 (2H, s, COCH₂O), 3.76 (2H, d, J=7Hz, NCH₂-iPr), 1.82 (3H, s, 5-CH₃).
MASS (EI) m/z : 450(M⁺), 326, 266, 210, 180, 152, 116.

### (Example 1260)

### Compound of Formula No. C-619

¹H-NMR (CDCl₃) δ(ppm) : 7.21 (1H, s, 3-H), 4.76 (2H, brs, NCH₂Ar), 4.39 (2H, s, COCH₂O), 3.77 (2H, d, J=7Hz, NCH₂-iPr), 1.82 (3H, s, 5-CH₃).
MASS (EI) m/z : 449(M⁺), 325, 267, 246, 228, 210.

### (Example 1261)

### Compound of Formula No. C-620

¹H-NMR (CDCl₃) δ(ppm) : 7.16 (1H, s, 3-H), 4.78 (2H, brs, NCH₂Ar), 4.36 (2H, brs, COCH₂O), 3.82 (2H, d, J=7Hz, NCH₂-iPr), 1.74 (3H, s, 5-CH₃).
MASS (EI) m/z : 483(M⁺), 458, 425, 326, 268, 246, 228.

### (Example 1262)

### Compound of Formula No. C-621

¹H-NMR (CDCl₃) δ(ppm) : 7.19 (1H, s, 3-H), 4.76 (2H, brs, NCH₂Ar), 4.38 (2H, s, COCH₂O), 3.74 (2H, d, J=7Hz, NCH₂-iPr), 1.79 (3H, s, 5-CH₃).
MASS (EI) m/z : 500(M⁺), 267, 174, 147, 116.

### (Example 1263)

### Compound of Formula No. C-622

¹H-NMR (CDCl₃) δ(ppm) : 7.22 (1H, s, 3-H), 4.76 (2H, brs, NCH₂Ar), 4.35 (2H, s, COCH₂O), 3.78 (2H, d, J=7Hz, NCH₂-iPr), 1.82 (3H, s, 5-CH₃).
MASS (EI) m/z : 384(M⁺), 369, 309, 266, 210, 116.

### (Example 1264)

### Compound of Formula No. C-623

¹H-NMR (CDCl₃) δ(ppm) : 7.21 (1H, s, 3-H), 4.76 (2H, brs, NCH₂Ar), 4.34 (2H, s, COCH₂O), 3.78 (2H, d, J=7Hz, NCH₂-iPr), 1.82 (3H, s, 5-CH₃).
MASS (EI) m/z : 398(M⁺), 383, 325, 266, 210, 116.

### (Example 1265)

### Compound of Formula No. C-624

¹H-NMR (CDCl₃) δ(ppm) : 7.21 (1H, s, 3-H), 4.77 (2H, brs, NCH₂Ar), 4.34 (2H, s, COCH₂O), 3.78 (2H, d, J=7Hz, NCH₂-iPr), 1.82 (3H, s, 5-CH₃).
MASS (EI) m/z : 412(M⁺), 325, 266, 210, 180, 116.

### (Example 1266)

### Compound of Formula No. C-625

¹H-NMR (CDCl₃) δ(ppm) : 7.21 (1H, s, 3-H), 4.76 (2H, brs, NCH₂Ar), 4.36 (2H, s, COCH₂O), 3.78 (2H, d, J=7Hz, NCH₂-iPr), 1.82 (3H, s, 5-CH₃).
MASS (EI) m/z : 410(M⁺), 309, 267, 210, 116.

### (Example 1267)

### Compound of Formula No. C-626

¹H-NMR (CDCl₃) δ(ppm) : 7.21 (1H, s, 3-H), 4.76 (2H, brs, NCH₂Ar), 4.34 (2H, s, COCH₂O), 3.78 (2H, d, J=7Hz, NCH₂-iPr), 1.82 (3H, s, 5-CH₃).
MASS (EI) m/z : 426(M⁺), 309, 266, 210, 180, 116.

### (Example 1268)

### Compound of Formula No. C-627

¹H-NMR (CDCl₃) δ(ppm) : 7.21 (1H, s, 3-H), 4.79 (2H, brs, NCH₂Ar), 4.45 (2H, s, COCH₂O), 3.78 (2H, d, J=7Hz, NCH₂-iPr), 1.81 (3H, s, 5-CH₃).
MASS (EI) m/z : 446(M⁺), 353, 309, 267, 253, 116.

### (Example 1269)

### Compound of Formula No. C-628

¹H-NMR (CDCl₃) δ(ppm) : 7.21 (1H, s, 3-H), 4.77 (2H, brs, NCH₂Ar), 4.44 (2H, s, COCH₂O), 3.78 (2H, d, J=7Hz, NCH₂-iPr), 1.82 (3H, s, 5-CH₃).
MASS (EI) m/z : 400(M⁺), 344, 325, 309, 266, 226, 210, 116.

### (Example 1270)

### Compound of Formula No. C-629

¹H-NMR (CDCl₃ δ(ppm) : 7.21 (1H, s, 3-H), 4.78 (2H, brs, NCH₂Ar), 4.32 (2H, s, COCH₂O), 3.77 (2H, d, J=7Hz, NCH₂-iPr), 1.82 (3H, s, 5-CH₃).
MASS (EI) m/z : 369(M⁺), 326, 295, 268, 253, 225, 210.

### (Example 1271)

### Compound of Formula No. C-630

¹H-NMR (CDCl₃) δ(ppm) : 7.21 (1H, s, 3-H), 4.76 (2H, brs, NCH₂Ar), 4.32 (2H, s, COCH₂O), 3.78 (2H, d, J=7Hz, NCH₂-iPr), 1.85 (3H, s, 5-CH₃).
MASS (EI) m/z : 383(M⁺), 326, 268, 210, 180, 152.

### (Example 1272)

### Compound of Formula No. C-631

¹H-NMR (CDCl₃) δ(ppm) : 7.21 (1H, s, 3-H), 4.80 (2H, brs, NCH₂Ar), 4.31 (2H, brs, COCH₂O), 3.78 (2H, d, J=7Hz, NCH₂-iPr), 1.85 (3H, s, 5-CH₃).
MASS (EI) m/z : 397(M⁺), 326, 268, 210, 180, 152, 116.

### (Example 1273)

### Compound of Formula No. C-632

¹H-NMR (CDCl₃) δ(ppm) : 7.20 (1H, s, 3-H), 4.75 (2H, brs, NCH₂Ar), 4.32 (2H, s, COCH₂O), 3.77 (2H, d, J=7Hz, NCH₂-iPr), 1.86 (3H, s, 5-CH₃).
MASS (EI) m/z : 397(M⁺), 325, 268, 210, 130, 116.

### (Example 1274)

### Compound of Formula No. C-633

¹H-NMR (CDCl₃) δ(ppm) : 7.21 (1H, s, 3-H), 4.85 (2H, brs, NCH₂Ar), 4.76 (2H, s, COCH₂O), 3.79 (2H, d, J=7Hz, NCH₂-iPr), 1.94 (3H, s, 5-CH₃).
MASS (EI) m/z : 413(M⁺), 325, 146, 116.

### (Example 1275)

### Compound of Formula No. C-634

¹H-NMR (CDCl₃) δ(ppm) : 7.24 (1H, s, 3-H), 4.80 (2H, brs, NCH₂Ar), 4.58 (2H, s, COCH₂O), 3.79 (2H, d, J=7Hz, NCH₂-iPr), 1.84 (3H, s, 5-CH₃).
MASS (EI) m/z : 451(M⁺), 352, 326, 309, 266, 236, 210.

### (Example 1276)

### Compound of Formula No. C-635

¹H-NMR (CDCl₃) δ(ppm) : 7.18 (1H, s, 3-H), 4.74 (2H, brs, NCH₂Ar), 4.32 (2H, d, J=10Hz, COCH₂O), 3.76 (2H, d, J=7Hz, NCH₂-iPr), 1.78 (3H, s, 5-CH₃).
MASS (EI) m/z : 462(M⁺), 417, 308, 266, 210, 192, 116.

### (Example 1277)

### Compound of Formula No. C-636

¹H-NMR (CDCl₃) δ(ppm) : 7.17 (1H, s, 3-H), 4.75 (2H, brs, NCH₂Ar), 4.38 (2H, d, J=14Hz, COCH₂O), 3.78 (2H, d, J=7Hz, NCH₂-iPr), 1.82 (3H, s, 5-CH₃).
MASS (EI) m/z : 478(M⁺), 433, 308, 266, 211, 116.

### (Example 1278)

### Compound of Formula No. C-637

¹H-NMR (CDCl₃) δ(ppm) : 7.20 (1H, s, 3-H), 4.77 (2H, brs, NCH₂Ar), 4.52 (2H, s, COCH₂O), 3.78 (2H, d, J=7Hz, NCH₂-iPr), 1.80 (3H, s, 5-CH₃).
MASS (EI) m/z : 404(M⁺), 389, 325, 288, 266, 210, 116.

### (Example 1279)

### Compound of Formula No. C-638

¹H-NMR (CDCl₃) δ(ppm) : 7.15 (1H, s, 3-H), 4.72 (2H, brs, NCH₂Ar), 4.40 (2H, s, COCH₂O), 3.77 (2H, d, J=7Hz, NCH₂-iPr), 1.75 (3H, s, 5-CH₃).
MASS (EI) m/z : 466(M⁺), 350, 325, 266, 210, 141, 116.

### (Example 1280)

### Compound of Formula No. C-639

¹H-NMR (CDCl₃) δ(ppm) : 7.15 (1H, s, 3-H), 4.74 (2H, brs, NCH₂Ar), 4.37 (2H, s, COCH₂O), 3.77 (2H, d, J=7Hz, NCH₂-iPr), 1.76 (3H, s, 5-CH₃).
MASS (EI) m/z : 480(M⁺), 465, 364, 325, 266, 210, 155, 116.

### (Example 1281)

### Compound of Formula No. C-640

¹H-NMR (CDCl₃) δ(ppm) : 7.19 (1H, s, 3-H), 5.78 (1H, q, J=5Hz, CSNHMe), 5.60 (1H, d, J=14Hz, NCH₂Ar), 5.23 (1H, d, J=14Hz, NCH₂Ar), 3.77 (2H, brt, J=7Hz, NCH₂-iPr), 3.09 (3H, d, J=5Hz, CSNHCH₃), 1.74 (3H, s, 5-CH₃).
MASS (EI) m/z : 341(M⁺), 268, 212, 170, 152, 116.

### (Example 1282)

### Compound of Formula No. C-641

¹H-NMR (CDCl₃) δ(ppm) : 7.20 (1H, s, 3-H), 5.67 (1H, q, J=5Hz, CSNHEt), 5.61 (1H, d, J=14Hz, NCH₂Ar), 5.23 (1H, d, J=14Hz, NCH₂Ar), 3.77 (2H, d, J=7Hz, NCH₂-iPr), 3.62 (2H, brd, J=5Hz, CSNHCH₂Me), 1.74 (3H, S, 5-CH₃).
MASS (EI) m/z : 355(M⁺), 268, 212, 186, 170, 152, 116.

### (Example 1283)

### Compound of Formula No. C-642

¹H-NMR (CDCl₃) δ(ppm) : 7.33 (1H, s, 3-H), 7.26-7.17 (1H, m, CSNHPh), 5.79 (1H, d, J=14Hz, NCH₂Ar), 5.21 (1H, d, J=14Hz, NCH₂Ar), 3.78 (2H, brt, J=7Hz, NCH₂-iPr), 1.82 (3H, s, 5-CH₃).
MASS (EI) m/z : 403(M⁺), 268, 234, 170, 152, 135, 116.

### (Example 1284)

### Compound of Formula No. C-643

¹H-NMR (CDCl₃) δ(ppm) : 7.39(1H, s,3-H), 6.24(1H, q, J=6.2Hz, NHMe), 3.61-3.80(2H, m, NCH₂CH), 1.51(3H, d, J=6.2Hz, NCHMe).
MASS (EI) m/z : 350(M⁺), 336, 282, 267, 220, 164, 152, 138.

### (Example 1285)

### Compound of Formula No. C-644

¹H-NMR (DMSO-d₆, measured at 110°C) δ(ppm) : 5.92 (1H, q J=7.0Hz, NCHMe), 3.74(2H, d, J=7.6Hz, NCH₂), 1.41(3H, d, J=7.0Hz, NCHMe).
MASS (EI) m/z : 340(M⁺), 281, 211, 180, 168, 152, 138, 130, 103, 96, 57.

### (Example 1286)

### Compound of Formula No. C-646

¹H-NMR (CDCl₃) δ(ppm) : 7.27 (1H, s, 3-H), 4.72 (2H, s, NCH₂Ar), 4.40 (2H, s, COCH₂O), 3.81 (2H, d, J=7Hz, NCH₂-iPr), 1.96 (3H, s, 5-CH₃).
MASS (EI) m/z : 446(M⁺), 353, 325, 311, 107.

### (Example 1287)

### Compound of Formula No. C-647

¹H-NMR (CDCl₃) δ(ppm) : 7.17 (1H, s, 3-H), 4.78 (2H, brs, NCH₂Ar), 4.42 (2H, s, COCH₂O), 3.79 (3H, s, NCH₃), 1.91 (3H, s, 5-CH₃).
MASS (EI) m/z : 361(M⁺), 267, 225, 116.

### (Example 1288)

### Compound of Formula No. C-648

¹H-NMR (CDCl₃) δ(ppm) : 7.21 (1H, s, 3-H), 4.78 (2H, s, NCH₂Ar), 4.42 (2H, s, COCH₂O), 4.06 (2H, q, J=7Hz, NCH₂Me), 1.87 (3H, s, 5-CH₃).
MASS (EI) m/z : 375(M⁺), 360, 281, 267, 239, 116.

### (Example 1289)

### Compound of Formula No. C-649

¹H-NMR (CDCl₃) δ(ppm) : 7.23 (1H, s, 3-H), 4.79 (2H, s, NCH₂Ar), 4.41 (2H, s, COCH₂O), 3.97 (2H, t, J=7Hz, NCH₂Et), 1.84 (3H, s, 5-CH₃).
MASS (EI) m/z : 389(M⁺), 360, 273, 253, 116.

### (Example 1290)

### Compound of Formula No. C-650

¹H-NMR (CDCl₃) δ(ppm) : 7.23 (1H, s, 3-H), 4.80 (2H, s, NCH₂Ar), 3.95 (2H, t, J=7Hz, NCH₂Et), 3.77 (2H, brs, COCH₂S), 1.95 (3H, s, 5-CH₃).
MASS (EI) m/z : 406(M⁺), 295, 254, 153, 125, 116.

### (Example 1291)

### Compound of Formula No. C-651

¹H-NMR (CDCl₃ δ(ppm) : 7.25 (1H, s, 3-H), 4.78 (2H, brs, NCH₂Ar), 4.46 (2H, s, COCH₂O), 4.35 (1H, hept, J=7Hz, NCHMe₂), 1.85 (3H, s, 5-CH₃).
MASS (EI) m/z : 389(M⁺), 374, 295, 253, 116.

### (Example 1292)

### Compound of Formula No. C-652

¹H-NMR (CDCl₃) δ(ppm) : 7.26 (1H, s, 3-H), 4.76 (2H, brs, NCH₂Ar), 4.67 (2H, s, COCH₂N), 4.36 (1H, hept, J=7Hz, NCHMe₂), 1.86 (3H, s, 5-CH₃).
MASS (EI) m/z : 362(M⁺), 347, 312, 294, 252, 211, 178.

### (Example 1293)

### Compound of Formula No. C-653

¹H-NMR (CDCl₃) δ(ppm) : 7.22 (1H, s, 3-H), 4.78 (2H, brs, NCH₂Ar), 4.41 (2H, s, COCH₂O), 4.00 (2H, t, J=7Hz, NCH₂-Pr), 1.84 (3H, s, 5-CH₃).
MASS (EI) m/z : 403(M⁺), 360, 280, 267, 149, 116.

### (Example 1294)

### Compound of Formula No. C-654

¹H-NMR (CDCl₃) δ(ppm) : 7.24 (1H, s, 3-H), 4.79 (2H, brs, NCH₂Ar), 4.41 (2H, s, COCH₂O), 3.79 (2H, d, J=7Hz, NCH₂-iPr), 1.82 (3H, s, 5-CH₃).
MASS (EI) m/z : 403(M⁺), 388, 360, 287, 267, 136, 116.

### (Example 1295)

### Compound of Formula No. C-655

¹H-NMR (CDCl₃) δ(ppm) : 7.27 (1H, s, 3-H), 4.78 (2H, brs, NCH₂Ar), 4.65 (2H, s, COCH₂O), 3.79 (2H, d, J=7Hz, NCH₂-iPr), 1.94 (3H, s, 5-CH₃).
MASS (EI) m/z : 402(M-1), 309, 267.

### (Example 1296)

### Compound of Formula No. C-656

¹H-NMR (CDCl₃) δ(ppm) : 7.26 (1H, s, 3-H), 4.78 (2H, brs, NCH₂Ar), 4.66 (2H, s, COCH₂O), 3.79 (2H, d, J=7Hz, NCH₂-iPr), 1.96 (3H, s, 5-CH₃).
MASS (EI) m/z : 417(M⁺), 308, 268, 150.

### (Example 1297)

### Compound of Formula No. C-657

¹H-NMR (CDCl₃) δ(ppm) : 7.27 (1H, s, 3-H), 4.78 (2H, brs, NCH₂Ar), 4.69 (2H, s, COCH₂O), 3.80 (2H, d, J=7Hz, NCH₂-iPr), 1.93 (3H, s, 5-CH₃).
MASS (EI) m/z : 471(M⁺), 452, 268, 204.

### (Example 1298)

### Compound of Formula No. C-658

¹H-NMR (CDCl₃) δ(ppm) : 7.27 (1H, s, 3-H), 4.77 (2H, brs, NCH₂Ar), 4.77 (2H, s, COCH₂O), 3.80 (2H, d, J=7Hz, NCH₂-iPr), 1.83 (3H, s, 5-CH₃).
MASS (EI) m/z : 475(M⁺), 460, 432, 359.

### (Example 1299)

### Compound of Formula No. C-659

¹H-NMR (CDCl₃) δ(ppm) : 7.27 (1H, s, 3-H), 4.78 (2H, brs, NCH₂Ar), 4.78 (2H, s, COCH₂O), 3.80 (2H, d, J=7Hz, NCH₂-iPr), 1.92 (3H, s, 5-CH₃).
MASS (EI) m/z : 489(M⁺), 268, 222.

### (Example 1300)

### Compound of Formula No. C-660

¹H-NMR (CDCl₃) δ(ppm) : 7.27 (1H, s, 3-H), 4.77 (2H, brs, NCH₂Ar), 4.41 (2H, s, COCH₂O), 3.80 (2H, d, J=7Hz, NCH₂-iPr), 1.93 (3H, s, 5-CH₃).
MASS (EI) m/z : 521(M⁺), 505, 268, 238, 210.

### (Example 1301)

### Compound of Formula No. C-661

¹H-NMR (CDCl₃) δ(ppm) : 7.28 (1H, s, 3-H), 4.81 (2H, s, NCH₂Ar), 4.77 (2H, brs, COCH₂O), 3.80 (2H, d, J=7Hz, NCH₂-iPr), 1.95 (3H, s, 5-CH₃).
MASS (EI) m/z : 438(M⁺), 308, 268, 171.

### (Example 1302)

### Compound of Formula No. C-662

¹H-NMR (CDCl₃) δ(ppm) : 7.27 (1H, s, 3-H), 4.78 (2H, s, NCH₂Ar), 4.70 (2H, brs, COCH₂O), 3.80 (2H, d, J=7Hz, NCH₂-ipr), 1.94 (3H, s, 5-CH₃).
MASS (EI) m/z : 404(M⁺), 268, 137.

### (Example 1303)

### Compound of Formula No. C-663

¹H-NMR (CDCl₃) δ(ppm) : 7.27 (1H, s, 3-H), 4.78 (2H, brs, NCH₂Ar), 4.69 (2H, s, COCH₂O), 3.80 (2H, d, J=7Hz, NCH₂-iPr), 1.93 (3H, s, 5-CH₃).
MASS (EI) m/z : 404(M⁺), 268, 137.

### (Example 1304)

### Compound of Formula No. C-664

¹H-NMR (CDCl₃) δ(ppm) : 7.30 (1H, s, 3-H), 4.74 (2H, brs, NCH₂Ar), 4.69 (2H, s, COCH₂O), 3.81 (2H, d, J=7Hz, NCH₂-iPr), 1.90 (3H, s, 5-CH₃).
MASS (EI) m/z : 460(M⁺), 308, 266, 193.

### (Example 1305)

### Compound of Formula No. C-665

¹H-NMR (CDCl₃) δ(ppm) : 7.27 (1H, s, 3-H), 4.78 (2H, brs, NCH₂Ar), 4.67 (2H, s, COCH₂O), 3.80 (2H, d, J=7Hz, NCH₂-iPr), 1.93 (3H, s, 5-CH₃).
MASS (EI) m/z : 404(M⁺), 268, 137.

### (Example 1306)

### Compound of Formula No. C-666

¹H-NMR (CDCl₃) δ(ppm) : 7.24 (1H, s, 3-H), 4.79 (2H, s, NCH₂Ar), 3.81 (2H, s, COCH₂S), 3.77 (2H, d, J=7Hz, NCH₂-iPr), 1.91 (3H, s, 5-CH₃).
MASS (EI) m/z : 419(M⁺), 309, 268, 152, 124, 116.

### (Example 1307)

### Compound of Formula No. C-667

¹H-NMR (CDCl₃) δ(ppm) : 7.23 (1H, s, 3-H), 4.77 (2H, s, NCH₂Ar), 3.76 (2H, d, J=7Hz, NCH₂-iPr), 3.60 (2H, s, COCH₂S), 1.85 (3H, s, 5-CH₃).
MASS (EI) m/z : 419(M⁺), 404, 376, 309, 267, 253.

### (Example 1308)

### Compound of Formula No. C-668

¹H-NMR (CDCl₃) δ(ppm) : 7.25 (1H, s, 3-H), 4.80 (2H, s, NCH₂Ar), 3.79 (2H, s, COCH₂S), 3.78 (2H, d, J=7Hz, NCH₂-iPr), 1.82 (3H, s, 5-CH₃).
MASS (EI) m/z : 453(M⁺), 310, 268, 210, 186, 129, 116.

### (Example 1309)

### Compound of Formula No. C-669

¹H-NMR (CDCl₃) δ(ppm) : 7.24 (1H, s, 3-H), 4.80 (2H, s, NCH₂Ar), 3.84 (2H, brs, COCH₂S), 3.78 (2H, d, J=7Hz, NCH₂-iPr), 1.93 (3H, s, 5-CH₃).
MASS (EI) m/z : 487(M⁺), 468, 268, 220, 192, 116.

### (Example 1310)

### Compound of Formula No. C-670

¹H-NMR (CDCl₃) δ(ppm) : 7.26 (1H, s, 3-H), 4.80 (2H, s, NCH₂Ar), 3.80 (2H, brs, COCH₂S), 3.80 (2H, d, J=7Hz, NCH₂-iPr), 1.97 (3H, s, 5-CH₃).
MASS (EI) m/z : 464(M⁺), 439, 416, 385, 325, 268, 210.

### (Example 1311)

### Compound of Formula No. C-671

¹H-NMR (CDCl₃) δ(ppm) : 7.25 (1H, s, 3-H), 4.79 (2H, s, NCH₂Ar), 3.83 (2H, s, COCH₂S), 3.78 (2H, d, J=7Hz, NCH₂-iPr), 1.91 (3H, s, 5-CH₃).
MASS (EI) m/z : 487(M⁺), 468, 310, 268, 220, 192.

### (Example 1312)

### Compound of Formula No. C-672

¹H-NMR (CDCl₃) δ(ppm) : 7.26 (1H, s, 3-H), 4.80 (2H, brs, NCH₂Ar), 3.81 (2H, brs, COCH₂S), 3.79 (2H, d, J=7Hz, NCH₂-iPr), 1.92 (3H, s, 5-CH₃).
MASS (EI) m/z : 521(M⁺), 502, 268, 254, 226, 152, 116.

### (Example 1313)

### Compound of Formula No. C-673

¹H-NMR (CDCl₃) δ(ppm) : 7.24 (1H, s, 3-H), 4.81 (2H, s, NCH₂Ar), 3.78 (2H, brs, COCH₂S), 3.78 (2H, d, J=7Hz, NCH₂-iPr), 1.92 (3H, s, 5-CH₃).
MASS (EI) m/z : 420(M⁺), 268, 210, 153, 116.

### (Example 1314)

### Compound of Formula No. C-674

¹H-NMR (CDCl₃) δ(ppm) : 7.24 (1H, s, 3-H), 4.80 (2H, s, NCH₂Ar), 3.77 (2H, brs, COCH₂S), 3.77 (2H, d, J=7Hz, NCH₂-iPr), 1.91 (3H, s, 5-CH₃).
MASS (EI) m/z : 434(M⁺), 268, 167, 152, 116.

### (Example 1315)

### Compound of Formula No. C-675

¹H-NMR (CDCl₃) δ(ppm) : 7.26 (1H, s, 3-H), 4.80 (2H, brs, NCH₂Ar), 3.77 (2H, s, COCH₂S), 3.77 (2H, d, J=7Hz, NCH₂-iPr), 1.89 (3H, s, 5-CH₃).
MASS (EI) m/z : 448(M⁺), 256, 181, 149, 116.

### (Example 1316)

### Compound of Formula No. C-676

¹H-NMR (CDCl₃) δ(ppm) : 7.27 (1H, s, 3-H), 4.82 (2H, s, NCH₂Ar), 3.84 (2H, brs, COCH₂S), 3.79 (2H, d, J=7Hz, NCH₂-iPr), 1.94 (3H, s, 5-CH₃).
MASS (EI) m/z : 496(M⁺), 268, 229, 201, 152, 116.

### (Example 1317)

### Compound of Formula No. C-677

¹H-NMR (CDCl₃) δ(ppm) : 7.14 (1H, s, 3-H), 4.84 (1H, d, J=14Hz, NCH₂Ar), 4.75 (1H, d, J=14Hz, NCH₂Ar), 4.00 (1H, d, J=14Hz, COCH₂S), 3.75 (2H, d, J=7Hz, NCH₂-iPr), 3.68 (1H, d, J=14Hz, COCH₂S), 1.83 (3H, s, 5-CH₃).
MASS (EI) m/z : 435(M⁺), 417, 387, 357, 324, 309, 295, 268.

### (Example 1318)

### Compound of Formula No. C-678

¹H-NMR (CDCl₃) δ(ppm) : 7.22 (1H, s, 3-H), 4.69 (2H, brs, NCH₂Ar), 4.43 (1H, brs, COCH₂S), 4.38 (1H, brs, COCH₂S), 3.83-3.77 (2H, m, NCH₂-iPr), 1.89 (3H, s, 5-CH₃).
MASS (EI) m/z : 451(M⁺), 309, 268, 212, 184, 152, 116.

### (Example 1319)

### Compound of Formula No. C-679

¹H-NMR (CDCl₃) δ(ppm) : 7.17 (1H, s, 3-H), 4.77 (2H, S, NCH₂Ar), 4.13 (1H, d, J=14Hz, COCH₂S), 3.87 (1H, d, J=14Hz, COCH₂S), 3.76 (2H, d, J=7Hz, NCH₂-iPr), 1.87 (3H, s, 5-CH₃).
MASS (EI) m/z : 436(M⁺), 420, 388, 370, 342, 324, 310.

### (Example 1320)

### Compound of Formula No. C-680

¹H-NMR (CDCl₃) δ(ppm) : 7.22 (1H, s, 3-H), 4.69 (2H, s, NCH₂Ar), 4.57 (1H, brs, COCH₂S), 4.41 (1H, brs, COCH₂S), 3.83-3.77 (2H, m, NCH₂-iPr), 1.91 (3H, s, 5-CH₃).
MASS (EI) m/z : 452(M⁺), 336, 309, 268, 230, 212, 194.

### (Example 1321)

### Compound of Formula No. C-681

¹H-NMR (CDCl₃) δ(ppm) : 7.24 (1H, s, 3-H), 4.79 (2H, brs, NCH₂Ar), 4.58 (2H, s, COCH₂O), 3.78 (2H, d, J=7Hz, NCH₂-iPr), 1.86 (3H, s, 5-CH₃).
MASS (EI) m/z : 393(M⁺), 277, 266, 252, 210, 164.

### (Example 1322)

### Compound of Formula No. C-682

¹H-NMR (CDCl₃) δ(ppm) : 7.22 (1H, s, 3-H), 4.78 (2H, brs, NCH₂Ar), 4.54 (2H, s, COCH₂O), 3.78 (2H, d, J=7Hz, NCH₂-iPr), 1.85 (3H, s, 5-CH₃).
MASS (EI) m/z : 407(M⁺), 266, 252, 223, 210, 192, 116

### (Example 1323)

### Compound of Formula No. C-683

¹H-NMR (CDCl₃) δ(ppm) : 7.22 (1H, s, 3-H), 4.79 (2H, brs, NCH₂Ar), 4.54 (2H, s, COCH₂O), 3.78 (2H, d, J=7Hz, NCH₂-iPr), 1.86 (3H, s, 5-CH₃).
MASS (EI) m/z : 435(M⁺), 266, 210, 192, 168, 116.

### (Example 1324)

### Compound of Formula No. C-684

¹H-NMR (CDCl₃) δ(ppm) ; 7.23 (1H, s, 3-H), 4.77 (2H, brs, NCH₂Ar), 4.58 (2H, s, COCH₂O), 3.78 (2H, d, J=7Hz, NCH₂-iPr), 1.87 (3H, s, 5-CH₃).
MASS (EI) m/z : 421(M⁺), 308, 266, 210, 192, 154, 136.

### (Example 1325)

### Compound of Formula No. C-685

¹H-NMR (CDCl₃) δ(ppm) : 7.23 (1H, s, 3-H), 4.77 (2H, brs, MCH₂Ar), 4.59 (2H, brs, COCH₂O), 3.78 (2H, d, J=7Hz, NCH₂-iPr), 1.86 (3H, s, 5-CH₃).
MASS (EI) m/z : 441(M⁺), 325, 266, 252, 210, 192.

### (Example 1326)

### Compound of Formula No. C-686

¹H-NMR (CDCl₃) δ(ppm) : 7.26 (1H, s, 3-H), 4.80 (2H, brs, NCH₂Ar), 4.66 (2H, brs, COCH₂O), 3.79 (2H, d, J=7Hz, NCH₂-iPr), 1.89 (3H, s, 5-CH₃).
MASS (EI) m/z : 503(M⁺), 468, 266, 105.

### (Example 1327)

### Compound of Formula No. C-687

¹H-NMR (CDCl₃) δ(ppm) : 7.24 (1H, s, 3-H), 4.78 (2H, brs, NCH₂Ar), 4.61 (2H, s, COCH₂O), 3.79 (2H, d, J=7Hz, NCH₂-iPr), 1.86 (3H, s, 5-CH₃).
MASS (EI) m/z : 451(M⁺), 436, 420, 335, 266, 252, 210.

### (Example 1328)

### Compound of Formula No. C-688

¹H-NMR (CDCl₃) δ(ppm) : 7.27 (1H, s, 3-H), 4.78 (2H, brs, NCH₂Ar), 4.72 (2H, s, COCH₂O), 3.78 (2H, d, J=7Hz, NCH₂-iPr), 1.89 (3H, s, 5-CH₃).
MASS (EI) m/z : 409(M⁺), 309, 266, 142.

### (Example 1329)

### Compound of Formula No. C-689

¹H-NMR (CDCl₃) δ(ppm) : 7.14 (1H, s, 3-H), 4.74 (2H, brs, NCH₂Ar), 4.65 (2H, s, COCH₂O), 3.75 (2H, d, J=7Hz, NCH₂-iPr), 1.68 (3H, s, 5-CH₃).
MASS (EI) m/z : 392(M⁺), 324, 309, 295, 266, 253, 210.

### (Example 1330)

### Compound of Formula No. C-690

¹H-NMR (CDCl₃) δ(ppm) 7.14 (1H, s, 3-H), 4.76 (2H, brs, NCH₂Ar), 4.62 (2H, s, COCH₂O), 3.75 (2H, d, J=7Hz, NCH₂-iPr), 1.70 (3H, s, 5-CH₃).
MASS (EI) m/z : 406(M⁺), 324, 309, 295, 267, 253, 210.

### (Example 1331)

### Compound of Formula No. C-691

¹H-NMR (CDCl₃) δ(ppm) : 7.28 (1H, s, 3-H), 4.80 (2H, brs, NCH₂Ar), 4.76 (2H, s, COCH₂O), 3.80 (2H, d, J=7Hz, NCH₂-iPr), 1.91 (3H, s, 5-CH₃).
MASS (EI) m/z : 443(M⁺), 423, 266, 252, 223, 210, 165.

### (Example 1332)

### Compound of Formula No. C-692

¹H-NMR (CDCl₃) δ(ppm) : 7.26 (1H, s, 3-H), 4.80 (2H, brs, NCH₂Ar), 4.80 (2H, s, COCH₂O), 3.81 (2H, d, J=7Hz, NCH₂-iPr), 2.01 (3H, s, 5-CH₃).
MASS (EI) m/z : 453(M⁺), 267, 186.

### (Example 1333)

### Compound of Formula No. C-693

¹H-NMR (CDCl₃) δ(ppm) : 7.30 (1H, s, 3-H), 4.79 (2H, brs, NCH₂Ar), 4.77 (2H, s, COCH₂O), 3.80 (2H, d, J=7Hz, NCH₂-iPr), 1.98 (3H, s, 5-CH₃).
MASS (EI) m/z : 453(M⁺), 267, 186.

### (Example 1334)

### Compound of Formula No. C-694

¹H-NMR (CDCl₃) δ(ppm) : 7.27 (1H, s, 3-H), 4.80 (2H, brs, NCH₂Ar), 4.75 (2H, s, COCH₂O), 3.80 (2H, d, J=7Hz, NCH₂-iPr), 1.91 (3H, s, 5-CH₃).
MASS (EI) m/z : 457(M⁺), 341, 308, 266.

### (Example 1335)

### Compound of Formula No. C-695

¹H-NMR (CDCl₃) δ(ppm) : 7.27 (1H, s, 3-H), 4.80 (2H, brs, NCH₂Ar), 4.73 (2H, s, COCH₂O), 3.80 (2H, d, J=7Hz, NCH₂-iPr), 1.90 (3H, s, 5-CH₃).
MASS (EI) m/z : 477(M⁺), 423, 326, 266.

### (Example 1336)

### Compound of Formula No. C-696

¹H-NMR (CDCl₃) δ(ppm) : 7.28 (1H, s, 3-H), 4.80 (2H, brs, NCH₂Ar), 4.80 (2H, s, COCH₂O), 3.59 (2H, d, J=7Hz, NCH₂-iPr), 1.94 (3H, s, 5-CH₃).
MASS (EI) m/z : 488(M⁺), 473, 458, 372, 266.

### (Example 1337)

### Compound of Formula No. C-697

¹H-NMR (CDCl₃) δ(ppm) : 7.26 (1H, s, 3-H), 4.80 (2H, brs, NCH₂Ar), 4.74 (2H, s, COCH₂O), 3.79 (2H, d, J=7Hz, NCH₂-iPr), 1.91 (3H, s, 5-CH₃).
MASS (EI) m/z : 458(M⁺), 442, 326, 309, 116.

### (Example 1338)

### Compound of Formula No. C-698

¹H-NMR (CDCl₃) δ(ppm) : 7.26 (1H, s, 3-H), 4.91 (2H, brs, NCH₂Ar), 4.17 (2H, brs, COCH₂O), 3.81 (2H, d, J=7Hz, NCH₂-iPr), 1.89 (3H, s, 5-CH₃).
MASS (EI) m/z : 491(M⁺), 375, 295, 267, 196.

### (Example 1339)

### Compound of Formula No. C-699

¹H-NMR (CDCl₃) δ(ppm) : 7.26 (1H, s, 3-H), 4.80 (2H, brs, NCH₂Ar), 4.41 (2H, brs, COCH₂O), 3.81 (2H, d, J=7Hz, NCH₂-iPr), 1.90 (3H, s, 5-CH₃).
MASS (EI) m/z : 459(M⁺), 423, 267, 164, 136.

### (Example 1340)

### Compound of Formula No. C-701

¹H-NMR (CDCl₃) δ(ppm) : 7.21 (1H, s, 3-H), 4.79 (2H, s, NCH₂Ar), 3.79 (2H, d, J=7Hz, NCH₂-iPr), 3.75 (2H, brs, COCH₂S), 1.88 (3H, s, 5-CH₃).
MASS (EI) m/z : 427(M⁺), 342, 310, 268, 210, 152.

### (Example 1341)

### Compound of Formula No. C-702

¹H-NMR (CDCl₃) δ(ppm) : 7.08 (1H, s, 3-H), 4.80 (2H, s, NCH₂Ar), 3.77 (2H, d, J=7Hz, NCH₂-iPr), 3.50 (2H, s, COCH₂S), 1.80 (3H, s, 5-CH₃).
MASS (EI) m/z : 408 (M⁺), 309, 268, 253, 212, 152, 116.

### (Example 1342)

### Compound of Formula No. C-703

¹H-NMR (CDCl₃) δ(ppm) : 7.17 (1H, s, 3-H), 4.75 (2H, brs, NCH₂Ar), 3.76 (2H, d, J=7Hz, NCH₂-iPr), 3.68 (2H, s, COCH₂S), 1.83 (3H, s, 5-CH₃).
MASS (EI) m/z : 422(M⁺), 309, 267, 210, 155, 127.

### (Example 1343)

### Compound of Formula No. C-704

¹H-NMR (CDCl₃) δ(ppm) : 7.25 (1H, s, 3-H), 4.81 (2H, s, NCH₂Ar), 3.79 (2H, d, J=7Hz, NCH₂-iPr), 3.74 (2H, brs, COCH₂S), 1.89 (3H, s, 5-CH₃).
MASS (EI) m/z : 409(M⁺), 391, 309, 275, 266, 253, 210.

### (Example 1344)

### Compound of Formula No. C-705

¹H-NMR (CDCl₃) δ(ppm) : 7.23 (1H, s, 3-H), 4.81 (2H, s, NCH₂Ar), 3.78 (2H, d, J=7Hz, NCH₂-iPr), 3.68 (2H, brs, COCH₂S), 1.90 (3H, s, 5-CH₃).
MASS (EI) m/z : 465(M⁺), 447, 331, 268, 253, 210, 198.

### (Example 1345)

### Compound of Formula No. C-706

¹H-NMR (CDCl₃) δ(ppm) : 7.22 (1H, s, 3-H), 4.81 (2H, s, NCH₂Ar), 3.78 (2H, d, J=7Hz, NCH₂-iPr), 3.70 (2H, brs, COCH₂S), 1.90 (3H, s, 5-CH₃).
MASS (EI) m/z : 491(M⁺), 473, 447, 357, 331, 268.

### (Example 1346)

### Compound of Formula No. C-707

¹H-NMR (CDCl₃) δ(ppm) : 7.23 (1H, s, 3-H), 4.76 (2H, brs, NCH₂Ar), 3.92 (2H, s, COCH₂S), 3.78 (2H, d, J=7Hz, NCH₂-iPr), 1.87 (3H, s, 5-CH₃).
MASS (EI) m/z : 423(M⁺), 324, 308, 266, 210, 128.

### (Example 1347)

### Compound of Formula No. C-708

¹H-NMR (CDCl₃) δ(ppm) : 7.26 (1H, s, 3-H), 4.79 (2H, brs, NCH₂Ar), 3.96 (2H, s, COCH₂S), 3.79 (2H, d, J=7Hz, NCH₂-iPr), 1.91 (3H, s, 5-CH₃).
MASS (EI) m/z : 440(M⁺), 308, 266, 210, 192, 152.

### (Example 1348)

### Compound of Formula No. C-709

¹H-NMR (CDCl₃) δ(ppm) : 7.27 (1H, s, 3-H), 4.82 (2H, brs, NCH₂Ar), 3.97 (2H, s, COCH₂S), 3.79 (2H, d, J=7Hz, NCH₂-iPr), 1.93 (3H, s, 5-CH₃).
MASS (EI) m/z : 459(M⁺), 309, 266, 210, 192, 151.

### (Example 1349)

### Compound of Formula No. C-710

¹H-NMR (CDCl₃) δ(ppm) : 7.30 (1H, s, 3-H), 4.81 (2H, brs, NCH₂Ar), 4.02 (2H, s, COCH₂S), 3.76 (2H, d, J=7Hz, NCH₂-iPr), 1.93 (3H, s, 5-CH₃).
MASS (EI) m/z : 475(M⁺), 309, 268, 208, 180, 167, 116.

### (Example 1350)

### Compound of Formula No. C-711

¹H-NMR (CDCl₃) δ(ppm) : 7.27 (1H, s, 3-H), 4.83 (2H, brs, NCH₂Ar), 3.77 (2H, d, J=7Hz, NCH₂-iPr), 3.75 (2H, brs, COCH₂S), 1.88 (3H, s, 5-CH₃).
MASS (EI) m/z : 458(M⁺), 309, 268, 210, 191, 163, 150.

### (Example 1351)

### Compound of Formula No. C-712

¹H-NMR (CDCl₃) δ(ppm) : 7.30 (1H, s, 3-H), 4.83 (2H, brs, NCH₂Ar), 3.94 (2H, s, COCH₂S), 3.79 (2H, d, J=7Hz, NCH₂-iPr), 1.93 (3H, s, 5-CH₃).
MASS (EI) m/z : 459(M⁺), 309, 268, 210, 192, 152.

### (Example 1352)

### Compound of Formula No. C-713

¹H-NMR (CDCl₃) δ(ppm) : 7.26 (1H, s, 3-H), 4.77 (2H, brs, NCH₂Ar), 4.67 (2H, s, COCH₂N), 3.79 (2H, d, J=7Hz, NCH₂-iPr), 1.83 (3H, s, 5-CH₃).
MASS (EI) m/z : 376(M⁺), 308, 266, 210, 192, 152.

### (Example 1353)

### Compound of Formula No. C-714

¹H-NMR (CDCl₃) δ(ppm) : 7.26 (1H, s, 3-H), 4.74 (2H, brs, NCH₂Ar), 4.60 (2H, brs, COCH₂N), 3.80 (2H, d, J=7Hz, NCH₂-iPr), 1.82 (3H, s, 5-CH₃).
MASS (EI) m/z : 410(M⁺), 295, 266, 210.

### (Example 1354)

### Compound of Formula No. C-715

¹H-NMR (CDCl₃) δ(ppm) : 7.26 (1H, s, 3-H), 4.76 (2H, brs, NCH₂Ar), 4.62 (2H, brs, COCH₂N), 3.80 (2H, d, J=7Hz, NCH₂-iPr), 1.82 (3H, s, 5-CH₃).
MASS (EI) m/z : 454(M⁺), 266, 210, 192, 116.

### (Example 1355)

### Compound of Formula No. C-716

¹H-NMR (CDCl₃) δ(ppm) : 7.26 (1H, s, 3-H), 4.79 (2H, brs, NCH₂Ar), 4.65 (2H, brs, COCH₂N), 3.80 (2H, d, J=7Hz, NCH₂-iPr), 1.81 (3H, s, 5-CH₃).
MASS (EI) m/z : 502(M⁺), 266, 207.

### (Example 1356)

### Compound of Formula No. C-717

¹H-NMR (CDCl₃) δ(ppm) : 7.25 (1H, s, 3-H), 4.73 (2H, brs, NCH₂Ar), 4.58 (2H, brs, COCH₂N), 3.79 (2H, d, J=7Hz, NCH₂-iPr), 1.82 (3H, s, 5-CH₃).
MASS (EI) m/z : 390(M⁺), 266, 210.

### (Example 1357)

### Compound of Formula No. C-718

¹H-NMR (CDCl₃) δ(ppm) : 7.24 (1H, s, 3-H), 4.76 (2H, brs, NCH₂Ar), 4.48 (2H, brs, COCH₂N), 3.79 (2H, d, J=7Hz, NCH₂-iPr), 1.85 (3H, s, 5-CH₃).
MASS (EI) m/z : 404(M⁺), 361, 266, 109.

### (Example 1358)

### Compound of Formula No. C-719

¹H-NMR (CDCl₃) δ(ppm) : 7.38 (1H, s, 3-H), 4.83 (2H, brs, NCH₂Ar), 4.65 (2H, brs, COCH₂N), 3.82(3H, s, OMe), 3.80 (2H, d, J=7Hz, NCH₂-iPr), 1.84 (3H, s, 5-CH₃).
MASS (EI) m/z : 434(M⁺), 403, 318, 266, 223, 210, 139.

### (Example 1359)

### Compound of Formula No. C-720

¹H-NMR (CDCl₃) δ(ppm) : 7.27 (1H, s, 3-H), 4.80 (2H, brs, NCH₂Ar), 4.65 (2H, brs, COCH₂N), 3.80 (2H, d, J=7Hz, NCH₂-iPr), 1.84 (3H, s, 5-CH₃).
MASS (EI) m/z : 448(M⁺), 403, 266.

### (Example 1360)

### Compound of Formula No. C-721

¹H-NMR (CDCl₃) δ(ppm) : 7.28 (1H, s, 3-H), 4.83 (2H, s, NCH₂Ar), 4.68 (2H, brs, COCH₂N), 3.81 (2H, d, J=7Hz, NCH₂-iPr), 1.84 (3H, s, 5-CH₃).
MASS (EI) m/z : 401(M⁺), 285, 267, 210, 192, 116.

### (Example 1361)

### Compound of Formula No. C-722

¹H-NMR (CDCl₃) δ(ppm) : 7.26 (1H, s, 3-H), 4.71 (2H, brs, NCH₂Ar), 4.52 (2H, brs, COCH₂N), 3.79 (2H, d, J=7Hz, NCH₂-iPr), 1.81 (3H, s, 5-CH₃).
MASS (EI) m/z : 391(M⁺), 281, 266.

### (Example 1362)

### Compound of Formula No. C-723

¹H-NMR (CDCl₃) δ(ppm) : 7.27 (1H, s, 3-H), 4.76 (2H, brs, NCH₂Ar), 4.31 (2H, brs, COCH₂N), 3.79 (2H, d, J=7Hz, NCH₂-iPr), 1.85 (3H, s, 5-CH₃).
MASS (EI) m/z : 336, 228.

### (Example 1363)

### Compound of Formula No. C-724

¹H-NMR (CDCl₃) δ(ppm) : 7.25 (1H, s, 3-H), 4.75 (2H, brs, NCH₂Ar), 4.43 (2H, brs, COCH₂N), 3.79 (2H, d, J=7Hz, NCH₂-iPr), 1.85 (3H, s, 5-CH₃).
MASS (EI) m/z : 419(M⁺), 282, 228, 169.

### (Example 1364)

### Compound of Formula No. C-725

¹H-NMR (CDCl₃) δ(ppm) : 7.30 (1H, s, 3-H), 4.83 (2H, brs, NCH₂Ar), 4.68 (2H, brs, COCH₂N), 3.81 (2H, d, J=7Hz, NCH₂-iPr), 1.86 (3H, s, 5-CH₃).
MASS (EI) m/z : 421(M⁺), 305, 267.

### (Example 1365)

### Compound of Formula No. C-726

¹H-NMR (CDCl₃) δ(ppm) : 7.29 (1H, s, 3-H), 4.76 (2H, brs, NCH₂Ar), 4.62 (2H, brs, COCH₂N), 3.82 (2H, d, J=7Hz, NCH₂-iPr), 1.87 (3H, s, 5-CH₃).
MASS (EI) m/z : 435(M⁺), 418, 266, 140.

### (Example 1366)

### Compound of Formula No. C-727

¹H-NMR (CDCl₃) δ(ppm) : 7.32 (1H, s, 3-H), 4.82 (2H, brs, NCH₂Ar), 4.62 (2H, brs, COCH₂N), 3.82 (2H, d, J=7Hz, NCH₂-iPr), 1.89 (3H, s, 5-CH₃).
MASS (EI) m/z : 435(M⁺), 418, 266, 140.

### (Example 1367)

### Compound of Formula No. C-728

¹H-NMR (CDCl₃) δ(ppm) : 7.30 (1H, s, 3-H), 4.72 (2H, brs, NCH₂Ar), 4.55 (2H, brs, COCH₂N), 3.82 (2H, d, J=7Hz, NCH₂-iPr), 1.88 (3H, s, 5-CH₃).
MASS (EI) m/z : 449(M⁺), 432, 226.

### (Example 1368)

### Compound of Formula No. C-729

¹H-NMR (CDCl₃) δ(ppm) : 7.25 (1H, s, 3-H), 4.78 (2H, brs, NCH₂Ar), 4.44 (2H, brs, COCH₂N), 3.80 (2H, d, J=7Hz, NCH₂-iPr), 1.72 (3H, s, 5-CH₃).
MASS (EI) m/z : 376(M⁺), 361, 333, 320, 295, 268, 239.

### (Example 1369)

### Compound of Formula No. C-730

¹H-NMR (CDCl₃) δ(ppm) : 7.28 (1H, s, 3-H), 4.76 (2H, brs, NCH₂Ar), 4.39 (2H, brs, COCH₂N), 3.82 (2H, d, J=7Hz, NCH₂-iPr), 1.87 (3H, s, 5-CH₃).
MASS (EI) m/z : 444(M⁺), 409, 401, 353, 295, 267.

### (Example 1370)

### Compound of Formula No. C-731

¹H-NMR (CDCl₃) δ(ppm) : 7.27 (1H, s, 3-H), 4.78 (2H, brs, NCH₂Ar), 4.69 (2H, brs, COCH₂N), 3.80 (2H, d, J=7Hz, NCH₂-iPr), 1.98 (3H, s, 5-CH₃).
MASS (EI) m/z : 403(M⁺), 322, 268.

### (Example 1371)

### Compound of Formula No. C-732

¹H-NMR (CDCl₃) δ(ppm) : 7.29 (1H, s, 3-H), 4.76 (2H, brs, NCH₂Ar), 4.62 (2H, brs, COCH₂N), 3.81 (2H, d, J=7Hz, NCH₂-iPr), 1.96 (3H, s, 5-CH₃).
MASS (EI) m/z : 471(M⁺), 452, 268, 204.

### (Example 1372)

### Compound of Formula No. C-733

¹H-NMR (CDCl₃) δ(ppm) : 7.27 (1H, s, 3-H), 4.76 (2H, brs, NCH₂Ar), 4.61 (2H, brs, COCH₂N), 3.80 (2H, d, J=7Hz, NCH₂-iPr), 1.99 (3H, s, 5-CH₃).
MASS (EI) m/z : 417(M⁺), 268, 150.

### (Example 1373)

### Compound of Formula No. C-734

¹H-NMR (CDCl₃) δ(ppm) : 7.30 (1H, s, 3-H), 4.82 (2H, brs, NCH₂Ar), 4.52 (2H, brs, COCH₂N), 3.81 (2H, d, J=7Hz, NCH₂-iPr), 1.95 (3H, s, 5-CH₃).
MASS (EI) m/z : 404(M⁺), 268, 137.

### (Example 1374)

### Compound of Formula No. C-735

¹H-NMR (CDCl₃) δ(ppm) : 7.34 (1H, s, 3-H), 4.87 (2H, brs, NCH₂Ar), 4.73 (2H, brs, COCH₂N), 3.82 (2H, d, J=7Hz, NCH₂-iPr), 1.98 (3H, s, 5-CH₃).
MASS (EI) m/z : 454(M⁺), 268, 187.

### (Example 1375)

### Compound of Formula No. C-736

¹H-NMR (CDCl₃) δ(ppm) : 7.20 (1H, s, 3-H), 4.77 (2H, brs, NCH₂Ar), 4.35 (2H, brs, COCH₂N), 3.79 (2H, d, J=7Hz, NCH₂-iPr), 1.80 (3H, s, 5-CH₃).
MASS (EI) m/z : 406(M⁺), 309, 253.

### (Example 1376)

### Compound of Formula No. C-737

¹H-NMR (CDCl₃) δ(ppm) : 7.23 (1H, s, 3-H), 4.79 (2H, s, NCH₂Ar), 4.52 (2H, brs, COCH₂N), 3.77 (2H, d, J=7Hz, NCH₂-iPr), 1.89 (3H, s, 5-CH₃).
MASS (EI) m/z : 406(M⁺), 309, 266, 210.

### (Example 1377)

### Compound of Formula No. C-738

¹H-NMR (CDCl₃) δ(ppm) : 7.21 (1H, s, 3-H), 4.78 (2H, brs, NCH₂Ar), 4.32 (2H, s, COCH₂N), 3.79 (2H, d, J=7Hz, NCH₂-iPr), 1.80 (3H, s, 5-CH₃).
MASS (EI) m/z : 420(M⁺), 309, 267, 253.

### (Example 1378)

### Compound of Formula No. C-739

¹H-NMR (CDCl₃) δ(ppm) : 7.28 (1H, s, 3-H), 4.80 (2H, brs, NCH₂Ar), 4.72 (2H, brs, COCH₂N), 3.81 (2H, d, J=7Hz, NCH₂-iPr), 1.85 (3H, s, 5-CH₃).
MASS (EI) m/z : 377(M⁺), 267, 223, 210, 192, 136.

### (Example 1379)

### Compound of Formula No. C-740

¹H-NMR (CDCl₃) δ(ppm) : 7.22 (1H, s, 3-H), 4.74 (2H, brs, NCH₂Ar), 4.40 (2H, s, COCH₂O), 4.00 (2H, d, J=7Hz, NCH₂-cBu), 1.83 (3H, s, 5-CH₃).
MASS (EI) m/z : 415(M⁺), 360, 321, 279, 253, 212, 116.

### (Example 1380)

### Compound of Formula No. C-741

¹H-NMR (CDCl₃) δ(ppm) : 7.20 (1H, s, 3-H), 4.78 (2H, brs, NCH₂Ar), 4.57 (2H, s, COCH₂O), 4.00 (2H, s, NCH₂-cBu), 1.87 (3H, s, 5-CH₃).
MASS (EI) m/z : 405(M⁺), 289, 278, 210, 136, 116.

### (Example 1381)

### Compound of Formula No. C-742

¹H-NMR (CDCl₃) δ(ppm) : 7.20 (1H, s, 3-H), 4.77 (2H, brs, NCH₂Ar), 4.52 (2H, s, COCH₂O), 3.99 (2H, s, NCH₂-cBu), 1.88 (3H, s, 5-CH₃).
MASS (EI) m/z : 419(M⁺), 278, 250, 210, 176, 150, 136.

### (Example 1382)

### Compound of Formula No. C-743

¹H-NMR (CDCl₃) δ(ppm) : 7.23 (1H, s, 3-H), 4.76 (2H, brs, NCH₂Ar), 4.62 (2H, brs, COCH₂N), 4.01 (2H, d, J=7Hz, NCH₂-cBu), 1.84 (3H, s, 5-CH₃).
MASS (EI) m/z : 466(M⁺), 350, 320, 278, 250, 159.

### (Example 1383)

### Compound of Formula No. C-744

¹H-NMR (CDCl₃) δ(ppm) : 7.25 (1H, s, 3-H), 4.77 (2H, brs, NCH₂Ar), 4.63 (2H, s, COCH₂O), 3.76 (2H, s, NCH₂-tBu), 1.92 (3H, s, 5-CH₃).
MASS (EI) m/z : 417(M⁺), 402, 282, 136, 116.

### (Example 1384)

### Compound of Formula No. C-745

¹H-NMR (CDCl₃) δ(ppm) : 7.28 (1H, s, 3-H), 4.81 (2H, brs, NCH₂Ar), 4.81 (2H, s, COCH₂O), 3.79 (2H, s, NCH₂-tBu), 1.95 (3H, s, 5-CH₃).
MASS (EI) m/z : 452(M⁺), 322, 307, 282, 171.

### (Example 1385)

### Compound of Formula No. C-746

¹H-NMR (CDCl₃) δ(ppm) : 7.27 (1H, s, 3-H), 4.78 (2H, brs, NCH₂Ar), 4.70 (2H, s, COCH₂O), 3.79 (2H, s, NCH₂-tBu), 1.94 (3H, s, 5-CH₃).
MASS (EI) m/z : 418(M⁺), 403, 282, 223, 116.

### (Example 1386)

### Compound of Formula No. C-747

¹H-NMR (CDCl₃) δ(ppm) : 7.24 (1H, s, 3-H), 4.80 (2H, brs, NCH₂Ar), 4.58 (2H, s, COCH₂O), 3.77 (2H, s, NCH₂-tBu), 1.85 (3H, S, 5-CH₃).
MASS (EI) m/z : 407(M⁺), 392, 291, 280, 206, 136, 116.

### (Example 1387)

### Compound of Formula No. C-748

¹H-NMR (CDCl₃) δ(ppm) : 7.23 (1H, s, 3-H), 4.78 (2H, brs, NCH₂Ar), 4.53 (2H, s, COCH₂O), 3.76 (2H, s, NCH₂-tBu), 1.84 (3H, s, 5-CH₃).
MASS (EI) m/z : 421(M⁺), 406, 340, 280, 265, 251, 228.

### (Example 1388)

### Compound of Formula No. C-749

¹H-NMR (CDCl₃) δ(ppm) : 7.12 (1H, s, 3-H), 4.75 (2H, brs, NCH₂Ar), 4.62 (2H, brs, COCH₂O), 3.71 (2H, s, NCH₂-tBu), 1.64 (3H, s, 5-CH₃).
MASS (EI) m/z : 406(M⁺), 388, 323, 281, 116.

### (Example 1389)

### Compound of Formula No. C-750

¹H-NMR (CDCl₃) δ(ppm) : 7.26 (1H, s, 3-H), 4.80 (2H, brs, NCH₂Ar), 4.66 (2H, brs, COCH₂N), 3.78 (2H, s, NCH₂-tBu), 1.83 (3H, s, 5-CH₃).
MASS (EI) m/z : 390(M⁺), 375, 280, 265, 223, 116.

### (Example 1390)

### Compound of Formula No. C-751

¹H-NMR (CDCl₃) δ(ppm) : 7.24 (1H, s, 3-H), 4.85 (2H, brs, NCH₂Ar), 4.57 (2H, brs, COCH₂N), 3.75 (2H, s, NCH₂-tBu), 1.79 (3H, s, 5-CH₃).
MASS (EI) m/z : 424(M⁺), 409, 280.

### (Example 1391)

### Compound of Formula No. C-752

¹H-NMR (CDCl₃) δ(ppm) : 7.26 (1H, s, 3-H), 4.88 (2H, brs, NCH₂Ar), 4.62 (2H, brs, COCH₂N), 3.78 (2H, s, NCH₂-tBu), 1.81 (3H, s, 5-CH₃).
MASS (EI) m/z : 468(M⁺), 453, 296, 280, 206, 159, 136.

### (Example 1392)

### Compound of Formula No. C-753

¹H-NMR (CDCl₃) δ(ppm) : 7.29 (1H, s, 3-H), 4.90 (2H, brs, NCH₂Ar), 4.68 (2H, brs, COCH₂N), 3.79 (2H, s, NCH₂-tBu), 1.83 (3H, s, 5-CH₃).
MASS (EI) m/z : 415(M⁺), 400, 359, 299, 281, 243, 136, 116.

### (Example 1393)

### Compound of Formula No. C-754

¹H-NMR (CDCl₃) δ(ppm) : 7.38 (1H, s, 3-H), 4.90 (2H, brs, NCH₂Ar), 4.67 (2H, brs, COCH₂N), 3.85(3H, s, OMe), 3.79 (2H, s NCH₂-tBu), 1.83 (3H, s, 5-CH₃).
MASS (EI) m/z : 448(M⁺), 433, 417, 359, 332, 280, 223.

### (Example 1394)

### Compound of Formula No. C-755

¹H-NMR (CDCl₃) δ(ppm) : 7.30 (1H, s, 3-H), 4.92 (2H, brs, NCH₂Ar), 4.66 (2H, brs, COCH₂N), 3.80 (2H, s, NCH₂-tBu), 1.85 (3H, s, 5-CH₃).

### (Example 1395)

### Compound of Formula No. C-756

¹H-NMR (DMSO-d₆) δ(ppm) : 12.15 (1H, brs, NH), 7.33 (1H, s, 3-H), 4.77 (2H, s, NCH₂Ar), 4.50 (2H, s, OCH₂), 3.78 (2H, s, NCH₂-tBu), 1.90 (3H, s, 5-CH₃).
MASS (EI) m/z : 527(M⁺), 484, 450, 435, 416, 401, 341 325, 283.

### (Example 1396)

### Compound of Formula No. C-757

¹H-NMR (CDCl₃) δ(ppm) : 7.20 (1H, s, 3-H), 4.80 (2H, brs, NCH₂Ar), 4.17 (2H, s, OCH₂CO₂H), 3.99 (2H, brs, NCOCH₂O), 3.78 (2H, d, J = 7.6 Hz, NCH₂CH), 1.75 (3H, s, 5-CH₃).
MASS (EI) m/z : 384 (M⁺), 369, 341, 325, 310, 295, 267, 180, 116, 96, 83, 57.

### (Example 1397)

### Compound of Formula No. C-758

¹H-NMR (CDCl₃) δ(ppm) : 7.40 (1H, brs, NH), 7.18 (1H, s, 3-H), 5.80 (1H, brs, NH), 4.76 (2H, brs, NCH₂Ar), 4.08(2H, s, OCH₂CONH₂), 3.91 (2H, brs, NCOCH₂O), 3.78 (2H, d, J = 7.6 Hz, NCH₂CH), 1.76 (3H, brs, 5-CH₃).
MASS (EI) m/z : 383(M⁺), 365, 325, 310, 295, 267, 225, 212, 194, 180, 166, 152, 116, 88, 57.

### (Example 1398)

### Compound of Formula No. C-759

¹H-NMR (CDCl₃) δ(ppm) : 7.19 (1H,s, 3-H), 4.76 (2H, brs, NCH₂Ar), 4.49 (2H, brs, CH₂CN), 3.95 (2H, brs, COCH₂), 3.78 (2H, d, J = 7.6 Hz, NCH₂CH), 1.78 (3H, s, 5-CH₃).
MASS (EI) m/z : 365 (M⁺), 350, 325, 309, 295, 267, 249, 239, 211, 192, 136.

### (Example 1399)

### Compound of Formula No. C-760

¹H-NMR (CDCl₃) δ(ppm) : 7.28 (1H, s, 3-H), 4.76(2H, brs, NCH₂Ar), 3.90 (2H, d, J = 7.6 Hz, NCH₂CH), 1.88 (3H, s, COCH₃), 1.79 (3H, s, 5-CH₃).
MASS (EI) m/z : 336(M⁺), 321, 293, 268, 254, 220, 212, 178.

### (Example 1400)

### Compound of Formula No. C-761

¹H-NMR (CDCl₃) δ(ppm) : 7.15 (1H, brs, 3-H), 4.70 (2H, s, NCH₂Ar), 3.88 (2H, d, J = 7.6 Hz, NCH₂CH), 3.69 (3H, brs, OCH₃), 1.82 (3H, brs, 5-CH₃).
MASS (EI) m/z : 352(M⁺), 337, 293, 284, 270, 236, 211, 168.

### (Example 1401)

### Compound of Formula No. C-762

¹H-NMR (CDCl₃) δ(ppm) : 7.25 (1H, brs, 3-H), 4.79 (, 2H, brs, NCH₂Ar), 3.91 (2H, d, J = 7.6 Hz, NCH₂CH), 1.82 (3H, s, 5-CH₃), 1.45 (1H, m, CH).
MASS (EI) m/z : 362(M⁺), 294, 280, 246, 225, 212, 178, 116, 69.

### (Example 1402)

### Compound of Formula No. C-763

¹H-NMR (CDCl₃) δ(ppm) : 7.15 (1H, s, 3-H), 4.76 (2H, brs, NCH₂Ar), 3.90 (2H, d, J = 7.6 Hz, NCH₂CH), 3.74 (2H, brs, CH₂O), 3.41 (3H, s, OCH₃), 1.76 (3H, s, 5-CH₃).
MASS (EI) m/z : 366(M⁺), 351, 336, 321, 293, 284, 250, 239.

### (Example 1403)

### Compound of Formula No. C-764 (mixture of 2 rotational isomers)

¹H-NMR (CDCl₃) δ(ppm) : 7.20 and 7.15 (1H, s, 3-H), 4.78 (2H, brs, NCH₂Ar), 4.35 and 3.74 (2H, brs, CH₂O), 3.91 and 3.90 (2H, d, J = 7.6 Hz, NCH₂CH), 1.82 and 1.76 (3H, s, 5-CH₃).
MASS (EI) m/z : 428(M⁺), 413, 366, 346, 335, 321, 312, 293, 253, 244, 225, 211, 116, 107, 77.

### (Example 1404)

### Compound of Formula No. C-765

¹H-NMR (CDCl₃) δ(ppm) : 7.20 (1H, s, 3-H), 4.75 (2H, brs, NCH₂Ar), 4.33 (2H, brs, CH₂O), 3.90 (2H, d, J = 7.6 Hz, NCH₂CH), 2.17 (3H, s, COCH₃), 1.84 (3H, s, 5-CH₃).
MASS (EI) m/z : 394(M⁺), 379, 351, 326, 293, 278, 225, 210, 206, 116, 83, 73.

### (Example 1405)

### Compound of Formula No. C-766

¹H-NMR (CDCl₃) δ(ppm) : 7.14 (1H, s, 3-H), 4.80 (2H, brs, NCH₂Ar), 3.90 (2H, d, J = 7.6 Hz, NCH₂CH), 3.78 (2H, brd, J = 4.6 Hz, CH₂O), 3.29 (1H, brt, J = 4.6 Hz, OH), 1.78 (3H, s, 5-CH₃).
MASS (EI) m/z : 352(M⁺), 337, 295, 293, 284, 270, 236, 225, 211, 206, 178.

### (Example 1406)

### Compound of Formula No. C-767

¹H-NMR (CDCl₃) δ(ppm) : 7.21 (1H, s, 3-H), 4.78 (2H, brs, NCH₂Ar), 3.77 (2H, s, NCH₂-tBu), 3.58 (2H, brs, COCH₂), 3.27 (2H, s, CH₂CN), 1.83 (3H, s, 5-CH₃), 0.95 (9H, s, tBu).
MASS (EI) m/z : 395(M⁺), 380, 355, 388, 323, 309, 281, 223, 206, 116, 96, 86.

### (Example 1407)

### Compound of Formula No. C-768

¹H-NMR (CDCl₃) δ(ppm) : 7.19 (1H, s, 3-H), 4.76 (2H, brs, NCH₂Ar), 3.91 (2H, d, J = 7.6 Hz, NCH₂CH), 3.58 (2H, brs, COCH₂), 3.27 (2H, s, CH₂CN), 1.85 (3H, s, 5-CH₃).
MASS (EI) m/z : 407(M⁺), 367, 336, 326, 293, 254, 239, 223, 206, 136, 116.

### (Example 1408)

### Compound of Formula No. C-769

¹H-NMR (CDCl₃) δ(ppm) : 7.18 (1H, s, 3-H), 4.76 (2H, brs, NCH₂Ar), 3.99 (2H, d, J = 7.6 Hz, NCH₂CH), 3.57 (2H, brs, COCH₂), 3.26 (2H, s, CH₂CN), 1.85 (3H, s, 5-CH₃).
MASS (EI) m/z : 393(M⁺), 353, 279, 253, 239, 204, 150, 136, 116, 86, 69, 55.

### (Example 1409)

### Compound of Formula No. C-770

¹H-NMR (CDCl₃) δ(ppm) : 7.33 - 7.65 (10H, m, Ar and 3-H), 4.83 (2H, brs, NCH₂Ar), 3.60 (2H, brs, COCH₂), 3.38 (2H, s, CH₂CN), 2.00 (3H, s, 5-CH₃).
MASS (EI) m/z : 401(M⁺), 361, 329, 315, 287, 225, 212, 200.

### (Example 1410)

### Compound of Formula No. C-771

¹H-NMR (CDCl₃) δ(ppm) : 7.20 (1H,s, 3-H), 4.76 (2H, brs, NCH₂Ar), 4.50 (2H, brs, CH₂CN), 3.95 (2H, brs, COCH₂), 3.77 (2H, s, NCH₂-tBu), 1.77 (3H, s, 5-CH₃), 0.95 (9H, tBu)
MASS (EI) m/z : 379(M⁺), 364, 339, 322, 309, 281, 263, 207, 193, 136, 166, 71.

### (Example 1411)

### Compound of Formula No. C-772

¹H-NMR (CDCl₃) δ(ppm) : 7.18 (1H, s, 3-H), 4.76 (2H, brs, NCH₂Ar), 4.49 (2H, brs, CH₂CN), 3.95 (2H, brs, COCH₂), 3.91 (2H, d, J = 7.6 Hz, NCH₂CH), 1.80 (3H, s, 5-CH₃).
MASS (EI) m/z : 391(M⁺), 377, 351, 323,3 09, 293, 275,.207, 166, 136, 116, 83.

### (Example 1412)

### Compound of Formula No. C-773

¹H-NMR (CDCl₃) δ(ppm) : 7.16 (1H, s, 3-H), 4.74 (2H, brs, NCH₂Ar), 4.49 (2H, brs, CH₂CN), 400 (2H, d, J = 7.6 Hz, NCH₂CH), 3.94 (2H, brs, COCH₂), 1.80 (3H, s, 5-CH₃).
MASS (EI) m/z : 377(M⁺), 362, 337, 322, 309, 279, 269, 261, 211, 136, 116, 70.

### (Example 1413)

### Compound of Formula No. C-774

¹H-NMR (CDCl₃) δ(ppm) : 8.90 (1H, d, J = 2.3 Hz, N=CH), 4.82 (2H, s, NCH₂Ar), 3.79 (2H, d, J = 7.6 Hz, NCH₂CH), 1.94 (3H, s, 5-CH₃), 0.87 (6H, d, J = 7.6 Hz, CH(CH₃)₂).
MASS (EI) m/z : 439(M⁺), 295, 260, 239, 145, 136, 116, 89, 57.

### (Example 1414)

### Compound of Formula No. C-775

¹H-NMR (CDCl₃) δ(ppm) : 8.90 (1H, dd, J = 4.7 Hz, J = 2.3 Hz, N=CH), 4.81 (2H, s, NCH₂Ar), 4.00 (2H, d, J = 7.6 Hz, NCH₂CH), 1.96 (3H, s, 5-CH₃).
MASS (EI) m/z : 451(M⁺), 307, 279, 260, 239, 123, 89, 69.

### (Example 1415)

### Compound of Formula No. C-776

¹H-NMR (CDCl₃) δ(ppm) : 8.90 (1H, d, J = 2.3 Hz, N=CH), 4.82 (2H, s, NCH₂Ar), 3.78 (2H, s, NCH₂-tBu), 1.94 (3H, s, 5-CH₃), 0.95 (9H, s, tBu).
MASS (EI) m/z : 453(M⁺), 309, 260, 239, 145, 136, 116, 89, 71.

### (Example 1416)

### Compound of Formula No. C-777 (mixture of 2 rotational isomers)

¹H-NMR (CDCl₃) δ(ppm) : 9.07 and 9.01 (1H, brd, J = 2.6 Hz, N=CH), 5.11 and 4.87 (2H, brs, MCH₂Ar), 3.74 (2H, brd, J = 7.6 Hz, NCH₂CH), 2.13 and 1.96 (3H, s, 5-CH₃), 0.83 (6H, d, J = 7.6 Hz, CH(CH₃)₂)
MASS (EI) m/z : 439(M⁺), 395, 295, 267, 259, 239, 172, 158, 129, 116, 89, 57.

### (Example 1417)

### Compound of Formula No. C-778 (mixture of 2 rotational isomers)

¹H-NMR (CDCl₃) δ(ppm) : 9.07 and 9.01 (1H, dd, J = 1.2 Hz, J = 4.0 Hz, N=CH), 5.11 and 4.86 (2H, brs, NCH₂Ar), 3.95 (2H, brd, J = 7.6 Hz, NCH₂CH), 2.18 and 1.98 (3H, s, 5-CH₃).
MASS (EI) m/z : 451(M⁺), 278, 265, 223, 210, 172, 149, 129, 116, 97, 83, 69, 57.

### (Example 1418)

### Compound of Formula No. C-779 (mixture of 2 rotational isomers)

¹H-NMR (CDCl₃) δ(ppm) : 9.07 and 8.99 (1H, brs, N=CH), 5.11 and 4.86 (2H, brs, NCH₂Ar), 3.73 (2H, s, NCH₂-tBu), 2.16 and 1.96 (3H, s, 5-CH₃), 0.91 (6H, s, tBu)
MASS (EI) m/z : 439(M⁺), 395, 295, 267, 259, 239, 172, 158, 129, 116, 89, 57.

### (Example 1419)

### Compound of Formula No. C-780

¹H-NMR (CDCl₃) δ(ppm) : 8.36 (1H, d, J = 4.1 Hz, N=CH), 7.31 (1H, s, 3-H), 4.80 (2H, s, NCH₂Ar), 3.74 (2H, s, NCH₂-tBu), 1.93 (3H, s, 5-CH₃), 0.92 (9H, s, tBu)
MASS (EI) m/z : 403(M⁺), 388, 308, 280, 192, 136, 116, 71, 55.

### (Example 1420)

### Compound of Formula No. C-781

¹H-NMR (CDCl₃) δ(ppm) : 4.99 (1H, brd, J = 13.8 Hz, NCH₂Ar), 4.84 (1H, brd, J = 13.8 Hz, NCH₂Ar), 3.66 (2H, s, NCH₂-tBu), 1.91 (3H, s, 5-CH₃), 0.81 (9H, s, tBu).
MASS (EI) m/z : 403(M⁺), 388, 309, 280, 192, 136, 116, 108.

### (Example 1421)

### Compound of Formula No. C-782

¹H-NMR (CDCl₃) δ(ppm) : 4.99 (1H, brd, J = 13.8 Hz, NCH₂Ar), 4.83 (1H, brd, J = 13.8 Hz, NCH₂Ar), 3.66 (2H, d, J = 7.6 Hz, NCH₂CH), 1.91 (3H, s, 5-CH₃), 0.72 (6H, J = 7.6 Hz, CH(CH₃)₂).
MASS (EI) m/z : 389(M⁺), 295, 266, 239, 210, 178, 136, 123, 116, 89, 78, 57.

### (Example 1422)

### Compound of Formula No. C-783

¹H-NMR (CDCl₃) δ(ppm) : 7.31 (1H, s, 3-H), 4.78 (2H, s, NCH₂Ar), 3.76 (2H, s, NCH₂-tBu), 1.88 (3H, s, 5-CH₃), 0.94 (9H, s, tBu).
MASS (EI) m/z : 403(M⁺), 423, 381, 309, 239, 193, 136, 116.

### (Example 1423)

### Compound of Formula No. C-785

¹H-NMR (CDCl₃) δ(ppm) : 8.63 (1H, brs, N=CH), 7.30 (1H, s, 3-H), 4.81 (2H, s, NCH₂Ar), 3.76 (2H, d, J = 7.6 Hz, NCH₂CH), 1.93 (3H, S, 5-CH₃), 0.89 (6H, d, J = 7.6 Hz, CH(CH₃)₂).
MASS (EI) m/z : 457(M⁺), 341, 295, 285, 266, 239, 210, 178, 136, 116, 89, 57.

### (Example 1424)

### Compound of Formula No. C-786

¹H-NMR (CDCl₃) δ(ppm) : 7.11 (1H, s, 3-H), 6.36 (1H, d, J = 9.9 Hz, COCH), 4.94 (1H, brd, J = 10.1 Hz, NCH₂Ar,), 4.87 (1H, brd, J = 10.1 Hz, NCH₂Ar), 3.68 (1H, brd, J = 7.6 Hz, NCH₂CH), 3.66 (1H, brd, J = 7.6 Hz, NCH₂CH), 1.87 (3H, s, 5-CH₃), 0.73 (6H, brs, CH(CH₃)₂).
MASS (EI) m/z : 457(M⁺), 341, 296, 285, 266, 239, 210, 178, 146, 136, 123, 116, 89, 57.

### (Example 1425)

### Compound of Formula No. C-787

¹H-NMR (CDCl₃) δ(ppm) : 8.30 (1H, d, J = 2.3 Hz, , N=CH), 7.30 (1H, s, 3-H), 4.79 (2H, s, NCH₂Ar), 3.76 (2H, d, J = 7.6 Hz, NCH₂CH), 1.92 (3H, s, 5-CH₃), 0.85 (6H, d, J = 7.6 Hz, CH(CH₃)₂).
MASS (EI) m/z : 423(M⁺), 295, 266, 239, 178, 136, 123, 116, 89, 57.

### (Example 1426)

### Compound of Formula No. C-788

¹H-NMR (CDCl₃) δ(ppm) : 6.23 (1H, d, J = 9.9 Hz, COCH), 5.00 (1H, brd, J = 10.1 Hz, NCH₂Ar,), 4.80 (1H, brd, J = 10.1 Hz, NCH₂Ar), 3.69 (2H, brd, J = 7.6 Hz, NCH₂CH), 1.98 (3H, s, 5-CH₃), 0.75 (6H, brs, CH(CH₃)₂).
MASS (EI) m/z : 423(M⁺), 295, 266, 239, 178, 136, 123, 116, 89, 57.

### (Example 1427)

### Compound of Formula No. C-789

¹H-NMR (CDCl₃) δ(ppm) : 8.29 (1H, d, J = 2.4 Hz, N=CH), 7.27 (1H, s, 3-H), 4.78 (2H, s, NCH₂Ar), 3.97 (2H, d, J = 7.6 Hz, NCH₂CH), 1.94 (3H, s, 5-CH₃).
MASS (EI) m/z : 435(M⁺), 307, 278, 239, 211, 190, 136, 116.

### (Example 1428)

### Compound of Formula No. C-790

¹H-NMR (CDCl₃) δ(ppm) : 6.23 (1H, d, J = 9.9 Hz, COCH), 4.95 (1H, brd, J = 10.1 Hz, NCH₂Ar), 4.76 (1H, brd, J = 10.1 Hz, NCH₂Ar), 3.90 (2H, brd, J = 7.6 Hz, NCH₂CH), 1.91 (3H, s, 5-CH₃).
MASS (EI) m/z : 435(M⁺), 307, 295, 278, 239, 211, 190, 136, 123, 116, 89, 69.

### (Example 1429)

### Compound of Formula No. C-791

¹H-NMR (CDCl₃) δ(ppm) : 8.29 (1H, d, J = 2.4 Hz, N=CH), 7.31 (1H, s, 3-H), 4.79 (2H, s, NCH₂Ar), 3.75 (2H, s NCH₂-tBu), 1.91 (3H, s, 5-CH₃), 0.93 (9H, s, tBu).
MASS (EI) m/z : 437(M⁺), 309, 280, 239, 192, 178, 136, 116, 71.

### (Example 1430)

### Compound of Formula No. C-792

¹H-NMR (CDCl₃) δ(ppm) : 6.23 (1H, d, J = 9.9 Hz, COCH), 4.95 (1H, brd, J = 10.1 Hz, NCH₂Ar,), 4.76 (1H, brd, J = 10.1 Hz, NCH₂Ar), 3.90 (2H, brd, J = 7.6 Hz, NCH₂-tBu), 1.91 (3H, s, 5-CH₃).
MASS (EI) m/z : 435(M⁺), 307, 295, 278, 239, 211, 190, 136, 123, 116, 89, 69.

### (Example 1431)

### Compound of Formula No. C-793

¹H-NMR (CDCl₃) δ(ppm) : 8.63 (1H, brs, N=CH), 7.27 (1H, s, 3-H), 4.80 (2H, s, NCH₂Ar), 3.97 (2H, d, J = 7.6 Hz, NCH₂CH), 1.95 (3H, s, 5-CH₃).
MASS (EI) m/z : 469(M⁺), 307, 278, 239, 210, 190, 136, 116.

### (Example 1432)

### Compound of Formula No. C-794

¹H-NMR (CDCl₃) δ(ppm) : 7.11 (1H, brs, 3-H), 6.36 (1H, d, J = 9.3 Hz, COCH), 4.94 (1H, brd, J = 10.1 Hz, NCH₂Ar,), 4.87 (1H, brd, J = 10.1Hz, NCH₂Ar), 3.89 (2H, brd, J = 7.6 Hz, NCH₂CH), 1.87 (3H, s, 5-CH₃).
MASS (EI) m/z : 469(M⁺), 307, 278, 239, 210, 190, 162, 136, 123, 116, 89, 69.

### (Example 1433)

### Compound of Formula No. C-795

¹H-NMR (CDCl₃) δ(ppm) : 8.63 (1H, brs, N=CH), 7.31 (1H, s, 3-H), 4.82 (2H, s, NCH₂Ar), 3.75 (2H, s, NCH₂-tBu), 1.92 (3H, s, 5-CH₃), 0.92 (9H, s, tBu).
MASS (EI) m/z : 471(M⁺), 356, 309, 281, 265, 239, 224, 192.

### (Example 1434)

### Compound of Formula No. C-796

¹H-NMR (CDCl₃) δ(ppm) : 7.12 (1H, brs, 3-H), 6.36 (1H, d, J = 9.9 Hz, COCH), 4.94 (1H, brd, J = 10.1 Hz, NCH₂Ar,), 4.88 (1H, brd, J = 10.1Hz, NCH₂Ar), 3.66 (2H, brs, NCH₂-tBu), 1.85 (3H, s, 5-CH₃), 0.80 (9H, s, tBu).
MASS (EI) m/z : 471(M⁺), 456, 355, 309, 280, 265, 239, 224, 192, 146, 136, 116, 89, 71, 57.

### (Example 1435)

### Compound of Formula No. C-797

¹H-NMR (CDCl₃) δ(ppm) : 8.57 (2H, d, J = 5.8 Hz, N=CH), 7.28 (1H, s, 3-H), 4.79 (2H, s, NCH₂Ar), 3.78 (2H, d, J = 7.6 Hz, NCH₂CH), 1.87 (3H, s, 5-CH₃), 0.87 (6H, d. J = 7.6 Hz, CH(CH₃)₂).
MASS (EI) m/z : 389(M⁺), 374, 346, 333, 295, 273, 239, 179, 136, 123, 116, 57.

### (Example 1436)

### Compound of Formula No. C-798

¹H-NMR (CDCl₃) δ(ppm) : 8.57 (2H, d, J = 6.4 Hz, N=CH), 7.26 (1H, s, 3-H), 4.78 (2H, s, NCH₂Ar), 3.99 (2H, d, J = 7.6 Hz, NCH₂CH), 1.89 (3H, s, 5-CH₃).
MASS (EI) m/z : 401(M⁺), 386, 333, 307, 285, 239, 191, 116.

### (Example 1437)

### Compound of Formula No. C-799

¹H-NMR (CDCl₃) δ(ppm) : 8.57 (2H, d, J = 5.8 Hz, N=CH), 7.29 (1H, s, 3-H), 4.79 (2H, s, NCH₂Ar), 3.77 (2H, s, NCH₂CH), 1.86 (3H, s, 5-CH₃), 0.94 (9H, s, tBu).
MASS (EI) m/z : 403(M⁺), 388, 346, 309, 287, 239, 231, 211, 190, 136, 116, 57.

### (Example 1438)

### Compound of Formula No. C-802

¹H-NMR (CDCl₃) δ(ppm) : 7.26 (1H, s, 3-H), 4.76 (2H, s, NCH₂Ar), 3.78 (2H, d, J = 7.6 Hz, NCH₂CH), 3.39 (3H, s, NCH₃), 1.86 (3H, 5-CH₃), 8.87 (6H, d, J = 7.6 Hz, CH(CH₃)₂).
MASS (EI) m/z : 406(M⁺), 295, 267, 239, 136, 116, 111, 89, 81, 57.

### (Example 1439)

### Compound of Formula No. C-803

¹H-NMR (CDCl₃) δ(ppm) : 7.05 (1H, s, 3-H), 4.70 (2H, brs, NCH₂Ar), 4.65 (2H, s, COCH₂O), 3.47 (2H, s, NCH₂Si), 1.67 (3H, s, 5-CH₃).
MASS (EI) m/z : 422(M⁺), 407, 339, 73.

### (Example 1440)

### Compound of Formula No. C-804

¹H-NMR (CDCl₃) δ(ppm) : 7.26 (1H, s, 3-H), 4.78 (2H, brs, NCH₂Ar), 4.61 (2H, brs, COCH₂N), 3.79 (2H, d, J=8Hz, NCH₂-iPr), 1.83 (3H, s, 5-CH₃).
MASS (EI) m/z : 495(M⁺), 439, 267, 105.

### (Example 1441)

### Compound of Formula No. C-805

¹H-NMR (CDCl₃) δ(ppm) : 7.05 (1H, s, 3-H), 4.62 (2H, brs, NCH₂Ar), 3.71 (2H, s, NCH₂-tBu), 1.80 (3H, s, 5-CH₃) 1.04 (9H, s, COC(CH₃)₃).
MASS (EI) m/z : 366(M⁺), 351, 282, 57.

### (Example 1442)

### Compound of Formula No. C-806

¹H-NMR (CDCl₃) δ(ppm) : 7.26 (1H, s, 3-H), 4.89 (2H, brs, NCH₂Ar), 4.59 (2H, brs, COCH₂N), 3.78 (2H, s, NCH₂-tBu), 1.82 (3H, s, 5-CH₃).
MASS (EI) m/z : 489(M⁺), 474, 432, 281, 83.

### (Example 1443)

### Compound of Formula No. C-807

¹H-NMR (CDCl₃) δ(ppm) : 7.26 (1H, s, 3-H), 4.72 (2H, brs, NCH₂Ar), 4.58 (2H, brs, COCH₂N), 3.79 (2H, d, J=8Hz, NCH₂-iPr), 1.83 (3H, s, 5-CH₃).
MASS (EI) m/z : 449(M⁺), 417, 266, 210.

### (Example 1444)

### Compound of Formula No. C-808

¹H-NMR (CDCl₃) δ(ppm) : 7.27 (1H, s, 3-H), 4.93 (2H, brs, NCH₂Ar), 4.67 (2H, brs, COCH₂N), 3.71 (2H, brs, NCH₂-tBu), 1.66 (3H, s, 5-CH₃).
MASS (EI) m/z : 604(M⁺), 589, 547, 488, 57.

### (Example 1445)

### Compound of Formula No. C-809

¹H-NMR (CDCl₃) δ(ppm) : 7.23 (1H, s; 3-H), 4.75 (2H, brs, NCH₂Ar), 4.55 (2H, brs, COCH₂N), 3.78 (2H, d, J=7Hz, NCH₂-iPr), 1.82 (3H, s, 5-CH₃).
MASS (EI) m/z : 564(M⁺), 266, 210, 119.

### (Example 1446)

### Compound of Formula No. C-810

¹H-NMR (CDCl₃) δ(ppm) : 7.21 (1H, s, 3-H), 4.90 (1H, d, J=13Hz, NCH₂Ar), 4.71 (1H, d, J=13Hz, NCH₂Ar), 3.78 (2H, d, J=8Hz, NCH₂-iPr) , 1.76 (3H, s, 5-CH₃).
MASS (EI) m/z : 364(M⁺), 349, 321, 308, 248.

### (Example 1447)

### Compound of Formula No. C-811

¹H-NMR (CDCl₃) δ(ppm) : 7.23 (1H, s, 3-H), 4.78 (2H, brs, NCH₂Ar), 3.78 (2H, d, J=8Hz, NCH₂-iPr), 1.79 (3H, s, 5-CH₃).
MASS (EI) m/z : 330(M⁺), 312, 295, 214, 123.

### (Example 1448)

### Compound of Formula No. C-812

¹H-NMR (CDCl₃) δ(ppm) : 7.61-7.37 (9H, m, C₆H₄-3-CN and COSC₆H₅), 7.32 (1H, s, 3-H), 4.85 (1H, brs, NCH₂Ar), 4.69 (1H, brs, NCH₂Ar), 3.82 (2H, d, J=7Hz, NCH₂-iPr), 1.89 (3H, s, 5-CH₃).
MASS (EI) m/z : 404(M⁺), 295, 267, 116.

### (Example 1449)

### Compound of Formula No. C-813

¹H-NMR (CDCl₃) δ(ppm) : 7.63-6.81 (7H, m, C₆H₄-3-CN and COOC₆H₃-2,4-F₂), 7.30 (1H, s, 3-H), 4.78 (2H, s, NCH₂Ar), 3.77 (2H, d, J=7Hz, NCH₂-iPr), 1.88 (3H, s, 5-CH₃).
MASS (EI) m/z : 424(M⁺), 295, 239, 116.

### (Example 1450)

### Compound of Formula No. C-814

¹H-NMR (CDCl₃) δ(ppm) : 8.37 (1H, d, J=4Hz, COO-(6-H-2-Pyr)), 7.31 (1H, s, 3-H), 7.00 (1H, d, J=8Hz, COO-(3-H-2-Pyr)), 4.80 (2H, s, NCH₂Ar), 3.76 (2H, d, J=7Hz, NCH₂-iPr), 1.95 (3H, s, 5-CH₃).
MASS (EI) m/z : 389 (M⁺), 295, 239, 116.

### (Example 1451)

### Compound of Formula No. C-815

¹H-NMR (CDCl₃) δ(ppm) : 7.30 (1H, s, 3-H), 4.79 (2H, s, NCH₂Ar), 3.77 (2H, d, J=8Hz, NCH₂-iPr), 3.33 (2H, s, SCH₂CN), 1.97 (3H, s, 5-CH₃).
MASS (EI) m/z : 459(M⁺), 295, 239, 116.

### (Example 1452)

### Compound of Formula No. C-816

¹H-NMR (CDCl₃) δ(ppm) : 7.16 (1H, s, 3-H), 4.73 (2H, s, NCH₂Ar), 4.48 (2H, q, J=8Hz, COOCH₂CF₃), 3.76 (2H, d, J=8Hz, NCH₂-iPr), 1.79 (3H, s, 5-CH₃).
MASS (EI) m/z : 394(M⁺), 379, 351, 338, 278.

### (Example 1453)

### Compound of Formula No. C-817

¹H-NMR (CDCl₃) δ(ppm) : 7.30 (1H, s, 3-H), 6.21 (1H, brs, CONH₂), 5.92 (1H, brs, CONH₂), 4.79 (2H, s, NCH₂Ar), 3.77 (2H, d, J=7Hz, NCH₂-iPr), 1.93 (3H, s, 5-CH₃).
MASS (EI) m/z : 431(M⁺), 413, 295, 268, 152.

### (Example 1454)

### Compound of Formula No. C-818

¹H-NMR (CDCl₃) δ(ppm) : 7.34 (1H, s, 3-H), 4.80 (2H, s, NCH₂Ar), 3.82 (2H, d, J=7Hz, NCH₂-iPr), 3.74 (2H, s, SO₂CH₂CN), 2.01 (3H, s, 5-CH₃).
MASS (EI) m/z : 491(M⁺), 448, 435, 295, 116.

### (Example 1455)

### Compound of Formula No. C-819

¹H-NMR (CDCl₃) δ(ppm) : 7.30 (1H, s, 3-H), 4.77 (1H, d, J=14Hz, NCH₂Ar), 4.76 (1H, d, J=14Hz, NCH₂Ar), 3.81 (2H, d, J=7Hz, NCH₂-iPr), 3.55 (1H, d, J=16Hz, SOCH₂CN), 3.15 (1H, d, J=16Hz, SOCH₂CN), 1.89 (3H, s, 5-CH₃).
MASS (EI) m/z : 475(M⁺), 459, 420, 295, 116.

### (Example 1456)

### Compound of Formula No. C-820

¹H-NMR (CDCl₃) δ(ppm) : 7.81 (2H, d, J=8Hz, COOC₆H₄-4-CONH₂), 7.31 (1H, s, 3-H), 7.13 (2H, d, J=8Hz, COOC₆H₄-4-CONH₂), 6.07 (2H, brs, CONH₂), 4.79 (2H, s, NCH₂Ar), 3.78 (2H, d, J=7Hz, NCH₂-iPr), 1.90 (3H, s, 5-CH₃).
MASS (EI) m/z : 431(M⁺), 413, 295, 239, 116.

### (Example 1457)

### Compound of Formula No. C-821

¹H-NMR (CDCl₃) δ(ppm) : 7.57 (2H, d, J=9Hz, COOC₆H₄-4-SCH₂CN), 7.30 (1H, s, 3-H), 7.10 (2H, d, J=9Hz, COOC₆H₄-4-SCH₂CN), 4.78 (2H, s, NCH₂Ar), 3.78 (2H, d, J=8Hz, NCH₂-iPr), 3.52 (2H, s, SCH₂CN), 1.89 (3H, s, 5-CH₃).
MASS (EI) m/z : 459(M⁺), 295, 239, 116.

### (Example 1458)

### Compound of Formula No. C-822

¹H-NMR (CDCl₃) δ(ppm) : 8.03 (2H, d, J=9Hz, COOC₆H₄-4-SO₂CH₂CN), 7.36 (2H, d, J=9Hz, COOC₆H₄-4-SO₂CH₂CN), 7.31 (1H, s, 3-H), 4.80 (2H, s, NCH₂Ar), 4.06 (2H, s, SO₂CH₂CN), 3.80 (2H, d, J=7Hz, NCH₂-iPr), 1.89 (3H, s, 5-CH₃).
MASS (EI) m/z : 491(M⁺), 295, 239, 116.

### (Example 1459)

### Compound of Formula No. C-823

¹H-NMR (CDCl₃) δ(ppm) : 7.76 (2H, d, J=9Hz, COOC₆H₄-4-SOCH₂CN), 7.33 (2H, d, J=9Hz, COOC₆H₄-SOCH₂CN), 7.31 (1H, s, 3-H), 4.79 (2H, s, NCH₂Ar), 3.79 (2H, d, J=8Hz, NCH₂-iPr), 3.79 (1H, d, J=16Hz, SOCH₂CN), 3.64 (1H, d, J=16Hz, SOCH₂CN), 1.90 (3H, s, 5-CH₃).
MASS (EI) m/z : 475(M⁺), 459, 420, 295, 116.

### (Example 1460)

### Compound of Formula No. C-824

¹H-NMR (CDCl₃) δ(ppm) : 7.63-6.80 (8H, m, C₆H₄-3-CN and COOC₆H₄-3-F), 7.30 (1H, s, 3-H), 4.78 (2H, s, NCH₂Ar), 3.78 (2H, d, J=8Hz, NCH₂-iPr), 1.89 (3H, s, 5-CH₃).
MASS (EI) m/z : 406(M⁺), 295, 239, 116.

### (Example 1461)

### Compound of Formula No. C-825

¹H-NMR (CDCl₃) δ(ppm) : 7.64-7.23 (8H, m, C₆H₄-3-CN and COOC₆H₄-3-CF₃), 7.27 (1H, s, 3-H), 4.79 (2H, s, NCH₂Ar), 3.78 (2H, d, J=8Hz, NCH₂-iPr), 1.90 (3H, s, 5-CH₃).
MASS (EI) m/z : 456(M⁺), 437, 295, 239, 116.

### (Example 1462)

### Compound of Formula No. C-826

¹H-NMR (CDCl₃) δ(ppm) : 7.67-7.34 (8H, m, C₆H₄-3-CN and COOC₆H₄-3-CO₂H), 7.11 (1H, s, 3-H), 4.19 (2H, s, NCH₂Ar), 3.78 (2H, d, J=8Hz, NCH₂-iPr), 2.11 (3H, s, 5-CH₃).
MASS (EI) m/z : 432(M⁺), 295, 267, 116.

### (Example 1463)

### Compound of Formula No. C-827

¹H-NMR (CDCl₃) δ(ppm) : 7.63-7.20 (8H, m, C₆H₄-3-CN and COOC₆H₄-3-SCF₃), 7.31 (1H, s, 3-H), 4.79 (2H, s, NCH₂Ar), 3.78 (2H, d, J=8Hz, NCH₂-iPr), 1.90 (3H, s, 5-CH₃).
MASS (EI) m/z : 488(M⁺), 295, 267, 239, 116.

### (Example 1464)

### Compound of Formula No. C-828

¹H-NMR (CDCl₃) δ(ppm) : 7.56 (2H, d, J=8Hz, COOC₆H₄-4-SCH₂CF₃), 7.30 (1H, s, 3-H), 7.04 (2H, d, J=8Hz, COOC₆H₄-4-SCH₂CF₃), 4.78 (2H, s, NCH₂Ar), 3.78 (2H, d, J=7Hz, NCH₂-iPr), 3.39 (2H, q, J=10Hz, SCH₂CF₃), 1.89 (3H, s, 5-CH₃).
MASS (EI) m/z : 502(M⁺), 295, 239, 116.

### (Example 1465)

### Compound of Formula No. C-829

¹H-NMR (CDCl₃) δ(ppm) : 8.07-7.25 (8H, m, C₆H₄-3-CN and COOC₆H₄-3-SO₂CF₃), 7.33 (1H, s, 3-H), 4.79 (2H, s, NCH₂Ar), 3.80 (2H, d, J=8Hz, NCH₂-iPr), 1.90 (3H, s, 5-CH₃).
MASS (EI) m/z : 520(M⁺), 504, 295, 239, 116.

### (Example 1466)

### Compound of Formula No. C-830

¹H-NMR (CDCl₃) δ(ppm) : 7.62 (2H, d, J=8Hz, COOC₆H₄-4-CF₃), 7.31 (1H, s, 3-H), 7.18 (2H, d, J=8Hz, COOC₆H₄-4-CF₃), 4.79 (2H, s, NCH₂Ar), 3.78 (2H, d, J=8Hz, NCH₂-iPr), 1.89 (3H, s, 5-CH₃).
MASS (EI) m/z : 456(M⁺), 413, 295, 239, 116.

### (Example 1467)

### Compound of Formula No. C-831

¹H-NMR (CDCl₃) δ (ppm) : 7.64-7.31 (8H, m, C₆H₄-3-CN and COOC₆H₄-2-CF₃), 7.25 (1H, s, 3-H), 4.77 (2H, s, NCH₂Ar), 3.77 (2H, d, J=8Hz, NCH₂-iPr), 1.91 (3H, s, 5-CH₃).
MASS (EI) m/z : 456(M⁺), 437, 295, 239, 116.

### (Example 1468)

### Compound of Formula No. C-832

¹H-NMR (CDCl₃) δ(ppm) : 7.17 (1H, s, 3-H), 4.76 (2H, s, NCH₂Ar), 3.99 (2H, d, J=7Hz, NCH₂-cBu), 3.85 (s, 2H, COCH₂Cl), 1.82 (3H, s, 5-CH₃).
MASS (EI) m/z : 356(M⁺), 321, 288, 279, 123.

### (Example 1469)

### Compound of Formula No. C-833

¹H-NMR (CDCl₃) δ(ppm) : 7.20 (1H, s, 3-H), 4.77 (2H, brs, NCH₂Ar), 3.99 (2H, d, J=7Hz, NCH₂-cBu), 1.80 (3H, s, 5-CH₃).
MASS (EI) m/z : 342(M⁺), 327, 307, 279, 123.

### (Example 1470)

### Compound of Formula No. C-834

¹H-NMR (CDCl₃) δ(ppm) : 7.71 (2H, d, J=9Hz, COOC₆H₄-4-SOCH₂CF₃), 7.55 (2H, d, J=9Hz, COOC₆H₄-4-SOCH₂CF₃), 7.31 (1H, s, 3-H), 4.79 (2H, s, NCH₂Ar), 3.79 (2H, d, J=8Hz, NCH₂-iPr), 3.62-3.32 (2H, m, SOCH₂CF₃), 1.90 (3H, s, 5-CH₃).
MASS (EI) m/z : 518(M⁺), 502, 295, 239, 116.

### (Example 1471)

### Compound of Formula No. C-835

¹H-NMR (CDCl₃) δ(ppm) : 7.96 (2H, d, J=9Hz, COOC₆H₄-4-SO₂CH₂CF₃), 7.33 (1H, s, 3-H), 7.32 (2H, d, J=9Hz, COOC₆H₄-4-SO₂CH₂CF₃), 4.80 (2H, s, NCH₂Ar), 3.91 (2H, q, J=9Hz, SO₂CH₂CF₃), 3.80 (2H, d, J=8Hz, NCH₂-iPr), 1.89 (3H, s, 5-CH₃).
MASS (EI) m/z : 534(M⁺), 295, 239, 116.

### (Example 1472)

### Compound of Formula No. C-836

¹H-NMR (CDCl₃) δ(ppm) : 7.62-7.03 (9H, m, C₆H₄-3-CN and COOC₆H₄), 7.29 (1H, s, 3-H), 4.78 (2H, s, NCH₂Ar), 3.90 (2H, d, J=8Hz, NCH₂-cPent), 1.92 (3H, s, 5-CH₃).
MASS (EI) m/z : 414(M⁺), 370, 321, 239, 116.

### (Example 1473)

### Compound of Formula No. C-837

¹H-NMR (CDCl₃) δ(ppm) : 7.64 (2H, d, J=9Hz, COOC₆H₄-4-SCF₃), 7.30 (1H, s, 3-H), 7.13 (2H, d, J=9Hz, COOC₆H₄-4-SCF₃), 4.79 (2H, s, NCH₂Ar), 3.78 (2H, d, J=7Hz, NCH₂-iPr), 1.89 (3H, s, 5-CH₃).
MASS (EI) m/z : 488(M⁺), 295, 239, 116.

### (Example 1474)

### Compound of Formula No. C-838

¹H-NMR (CDCl₃) δ(ppm) : 7.64 (2H, d, J=8Hz, COOC₆H₄-4-SCF₃), 7.27 (1H, s, 3-H), 7.13 (2H, d, J=8Hz, COOC₆H₄-4-SCF₃), 4.78 (2H, s, NCH₂Ar), 3.99(2H, d, J=7Hz, NCH₂-iPr), 1.91 (3H, s, 5-CH₃).
MASS (EI) m/z : 501((M+1)⁺), 307, 279, 239, 116.

### (Example 1475)

### Compound of Formula No. C-839

¹H-NMR (CDCl₃) δ(ppm) : 7.20 (1H, s, 3-H), 4.89 (1H, d, J=14Hz, NCH₂Ar), 4.70 (1H, d, J=14Hz, NCH₂Ar), 3.76 (2H, s, NCH₂-tBu), 1.76 (3H, s, 5-CH₃).
MASS (EI) m/z : 378(M⁺), 363, 322, 281, 262.

### (Example 1476)

### Compound of Formula No. C-840

¹H-NMR (CDCl₃) δ(ppm) : 7.18 (1H, s, 3-H), 4.88 (1H, d, J=13Hz, NCH₂Ar), 4.69 (1H, d, J=13Hz, NCH₂Ar), 3.98 (2H, d, J=7Hz, NCH₂-cBu), 1.76 (3H, s, 5-CH₃).
MASS (EI) m/z : 376(M⁺), 361, 348, 260, 116.

### (Example 1477)

### Compound of Formula No. C-841

¹H-NMR (CDCl₃) δ(ppm) : 7.19 (1H, s, 3-H), 4.89 (1H, d, J=13Hz, NCH₂Ar), 4.69 (1H, d, J=13Hz, NCH₂Ar), 3.90 (2H, d, J=8Hz, NCH₂-cPent), 1.76 (3H, s, 5-CH₃).
MASS (EI) m/z : 390(M⁺), 375, 349, 322, 116.

### (Example 1478)

### Compound of Formula No. C-842

¹H-NMR (CDCl₃) δ(ppm) : 7.63-6.80 (7H, m, C₆H₄-3-CN and COOC₆H₃-2,4-F₂), 7.27 (1H, s, 3-H), 4.77 (2H, s, NCH₂Ar), 3.98 (2H, d, J=7Hz, NCH₂-cBu), 1.90 (3H, s, 5-CH₃).
MASS (EI) m/z : 436(M⁺), 307, 239, 116.

### (Example 1479)

### Compound of Formula No. C-843

¹H-NMR (CDCl₃) δ(ppm) : 8.37 (1H, d, J=3Hz, COO-(6-H-2-Pyr)), 7.28 (1H, s, 3-H), 7.00 (1H, d, J=8Hz, COO-(3-H-2-Pyr)), 4.79 (2H, s, NCH₂Ar), 3.97 (2H, d, J=7Hz, NCH₂-cBu), 1.96 (3H, s, 5-CH₃).
MASS (EI) m/z : 401(M⁺), 357, 306, 278, 190.

### (Example 1480)

### Compound of Formula No. C-844

¹H-NMR (CDCl₃) δ(ppm) : 7.21 (1H, s, 3-H), 4.78 (2H, brs, NCH₂Ar), 4.40 (2H, s, COCH₂O), 4.10 (2H, s, NCH₂-tBu), 1.78 (3H, s, 5-CH₃).
MASS (EI) m/z : 452(M⁺), 437, 395, 336, 323.

### (Example 1481)

### Compound of Formula No. C-845

¹H-NMR (CDCl₃) δ(ppm) : 7.27 (1H, s, 3-H), 4.77 (2H, s, NCH₂Ar), 4.63 (2H, s, COCH₂O), 4.00 (2H, d, J=7Hz, NCH₂-cBu), 1.95 (3H, s, 5-CH₃).
MASS (EI) m/z : 415(M⁺), 280, 136.

### (Example 1482)

### Compound of Formula No. C-846

¹H-NMR (CDCl₃) δ(ppm) : 8.03 (2H, d, J=9Hz, COOC₆H₄-4-SO₂CF₃), 7.39 (2H, d, J=9Hz, COOC₆H₄-4-SO₂CF₃), 7.32 (1H, s, 3-H), 4.80 (2H, s, NCH₂Ar), 3.80 (2H, d, J=8Hz, NCH₂-iPr), 1.88 (3H, s, 5-CH₃).
MASS (EI) m/z : 520(M⁺), 477, 295, 239, 116.

### (Example 1483)

### Compound of Formula No. C-847

¹H-NMR (CDCl₃) δ(ppm) : 7.79 (2H, d, J=8Hz, COOC₆H₄-4-SOCF₃), 7.59 (2H, d, J=8Hz, COOC₆H₄-4-SOCF₃), 7.27 (1H, s, 3-H), 4.80 (2H, s, NCH₂Ar), 3.80 (2H, d, J=7Hz, NCH₂-iPr), 1.89 (3H, s, 5-CH₃).
MASS (EI) m/z : 504(M⁺), 295, 239, 116.

### (Example 1484)

### Compound of Formula No. C-848

¹H-NMR (CDCl₃) δ(ppm) : 8.03 (2H, d, J=9Hz, COOC₆H₄-4-SO₂CF₃), 7.39 (2H, d, J=9Hz, COOC₆H₄-4-SO₂CF₃), 7.27 (1H, s, 3-H), 4.79 (2H, s, NCH₂Ar), 4.01 (2H, d, J=7Hz, NCH₂-cBu), 1.90 (3H, s, 5-CH₃).
MASS (EI) m/z : 532(M⁺), 488, 307, 239, 116.

### (Example 1485)

### Compound of Formula No. C-849

¹H-NMR (CDCl₃) δ(ppm) : 8.00 (2H, d, J=8Hz, COOC₆H₄-4-SOCF₃), 7.44 (2H, d, J=8Hz, COOC₆H₄-4-SOCF₃), 7.26 (1H, s, 3-H), 4.79 (2H, s, NCH₂Ar), 4.02 (2H, d, J=7Hz, NCH₂-cBu), 1.91 (3H, s, 5-CH₃).
MASS (EI) m/z : 516(M⁺), 500, 307, 239, 116.

### (Example 1486)

### Compound of Formula No. C-850

¹H-NMR (CDCl₃) δ(ppm) : 7.61-7.36 (9H, m, C₆H₄-3-CN and COSC₆H₅), 7.29 (1H, s, 3-H), 4.76 (2H, brs, NCH₂Ar), 4.03 (2H, d, J=7Hz, NCH₂-cBu), 1.90 (3H, s, 5-CH₃).
MASS (EI) m/z : 416(M⁺), 307, 279, 239, 116.

### (Example 1487)

### Compound of Formula No. C-851

¹H-NMR (CDCl₃) δ(ppm) : 7.56 (2H, d, J=8Hz, COOC₆H₄-4-SCH₂CN), 7.28 (1H, s, 3-H), 7.10 (2H, d, J=8Hz, COOC₆H₄-4-SCH₂CN), 4.77 (2H, s, NCH₂Ar), 3.99 (2H, d, J=7Hz, NCH₂-cBu), 3.52 (2H, s, SCH₂CN), 1.92 (3H, s, 5-CH₃).
MASS (EI) m/z : 471(M⁺), 443, 307, 175, 116.

### (Example 1488)

### Compound of Formula No. C-852

¹H-NMR (CDCl₃) δ(ppm) : 7.23 (1H, s, 3-H), 4.78 (2H, brs, NCH₂Ar), 3.76 (2H, s, NCH₂-tBu), 1.78 (3H, s, 5-CH₃).
MASS (EI) m/z : 344(M⁺), 331, 329, 309, 281, 116.

### (Example 1489)

### Compound of Formula No. C-853

¹H-NMR (CDCl₃) δ(ppm) : 7.27 (1H, s, 3-H), 4.78 (2H, s, NCH₂Ar), 3.99 (2H, d, J=7Hz, NCH₂-cBu), 3.29 (2H, s, SCH₂CN), 1.99 (3H, s, 5-CH₃).
MASS (EI) m/z : 471(M⁺), 443, 307, 239, 116.

### (Example 1490)

### Compound of Formula No. C-854

¹H-NMR (CDCl₃) δ(ppm) : 7.27 (1H, s, 3-H), 4.79 (2H, s, NCH₂Ar), 3.76 (2H, s, NCH₂-tBu), 3.33 (2H, s, SCH₂CN), 1.96 (3H, s, 5-CH₃).
MASS (EI) m/z : 473(M⁺), 458, 309, 239, 116.

### (Example 1491)

### Compound of Formula No. C-855

¹H-NMR (CDCl₃) δ(ppm) : 7.57 (2H, d, J=6Hz, COOC₆H₄-4-F), 7.30 (1H, s, 3-H), 7.02 (2H, d, J=6Hz, COOC₆H₄-4-F), 4.77 (2H, s, NCH₂Ar), 3.77 (2H, d, J=8Hz, NCH₂-ipr), 1.89 (3H, s, 5-CH₃).
MASS (EI) m/z : 406(M⁺), 295, 239, 116.

### (Example 1492)

### Compound of Formula No. C-856

¹H-NMR (CDCl₃) δ(ppm) : 7.63-7.12 (8H, m, C₆H₄-3-CN and COOC₆H₄-2-F), 7.31 (1H, s, 3-H), 4.79 (2H, s, NCH₂Ar), 3.77 (2H, d, J=8Hz, NCH₂-iPr), 1.90 (3H, s, 5-CH₃).
MASS (EI) m/z : 406(M⁺), 295, 239, 116.

### (Example 1493)

### Compound of Formula No. C-857

¹H-NMR (CDCl₃) δ (ppm) : 7.63-6.91 (7H, m, C₆H₄-3-CN and COOC₆H₃-2,6-F₂), 7.33 (1H, s, 3-H), 4.79 (2H, s, NCH₂Ar), 3.77 (2H, d, J=8Hz, NCH₂-iPr), 1.89 (3H, s, 5-CH₃).
MASS (EI) m/z : 424(M⁺), 409, 295, 239, 116.

### (Example 1494)

### Compound of Formula No. C-858

¹H-NMR (CDCl₃) δ (ppm) : 7.29 (1H, s, 3-H), 7.13 (2H, d, J=8Hz, COOC₆H₄-4-CH₃), 6.92 (2H, d, J=8Hz, COOC₆H₄-4-CH₃), 4.78 (2H, s, NCH₂Ar), 3.77 (2H, d, J=8Hz, NCH₂-iPr), 2.32 (3H, s, COOC₆H₄-4-CH₃), 1.89 (3H, s, 5-CH₃).
MASS (EI) m/z : 402(M⁺), 295, 116.

### (Example 1495)

### Compound of Formula No. C-859

¹H-NMR (CDCl₃) δ(ppm) : 7.30 (1H, s, 3-H), 4.78 (2H, s, NCH₂Ar), 3.77 (2H, d, J=8Hz, NCH₂-iPr), 2.34 (3H, s, COOC₆H₄-3-CH₃), 1.90 (3H, s, 5-CH₃).
MASS (EI) m/z : 402(M⁺), 295, 239, 116.

### (Example 1496)

### Compound of Formula No. C-860

¹H-NMR (CDCl₃) δ(ppm) : 7.26 (1H, s, 3-H), 4.78 (2H, s, NCH₂Ar), 3.77 (2H, d, J=8Hz, NCH₂-iPr), 2.10 (3H, s, COOC₆H₄-3-CH₃), 1.92 (3H, s, 5-CH₃).
MASS (EI) m/z : 402(M⁺), 295, 239, 116.

### (Example 1497)

### Compound of Formula No. C-861

¹H-NMR (CDCl₃) δ(ppm) : 7.30 (1H, s, 3-H), 6.97 (2H, d, J=9Hz, COOC₆H₄-4-OCH₃), 6.85 (2H, d, J=9Hz, COOC₆H₄-4-OCH₃), 4.78 (2H, s, NCH₂Ar), 3.78 (3H, s, OCH₃), 3.77 (2H, d, J=6Hz, NCH₂-iPr), 1.89 (3H, s, 5-CH₃).
MASS (EI) m/z : 418(M⁺), 295, 239, 123.

### (Example 1498)

### Compound of Formula No. C-862

¹H-NMR (CDCl₃) δ(ppm) : 7.30 (1H, s, 3-H), 4.78 (2H, s, NCH₂Ar), 3.78 (3H, s, OCH₃), 3.77 (2H, d, J=7Hz, NCH₂-iPr), 1.90 (3H, s, 5-CH₃).
MASS (EI) m/z : 418(M⁺), 295, 239, 116.

### (Example 1499)

### Compound of Formula No. C-863

¹H-NMR (CDCl₃) δ(ppm) : 7.29 (1H, s, 3-H), 4.79 (2H, s, NCH₂Ar), 3.85 (3H, s, OCH₃), 3.75 (2H, d, J=7Hz, NCH₂-iPr), 1.95 (3H, s, 5-CH₃).
MASS (EI) m/z : 418(M⁺), 295, 239, 116.

### (Example 1500)

### Compound of Formula No. C-864

¹H-NMR (CDCl₃) δ(ppm) : 7.61 (2H, d, J=8Hz, COOC₆H₄-4-CF₃), 7.45 (2H, d, J=8Hz, COOC₆H₄-4-CF₃), 7.31 (1H, s, 3-H), 4.79 (2H, s, NCH₂Ar), 3.77 (2H, s, NCH₂-tBu), 1.88 (3H, s, 5-CH₃).
MASS (EI) m/z : 470(M⁺), 455, 413, 309, 116.

### (Example 1501)

### Compound of Formula No. C-865

¹H-NMR (CDCl₃) δ(ppm) : 7.63-7.28 (8H, m, C₆H₄-3-CN and COOC₆H₄-3-CF₃), 7.32 (1H, s, 3-H), 4.79 (2H, s, NCH₂Ar), 3.77 (2H, s, NCH₂-tBu), 1.89 (3H, s, 5-CH₃).
MASS (EI) m/z : 470(M⁺), 455, 413, 309, 149.

### (Example 1502)

### Compound of Formula No. C-866

¹H-NMR (CDCl₃) δ(ppm) : 7.63-7.31 (8H, m, C₆H₄-3-CN and COOC₆H₄-2-CF₃), 7.25 (1H, s, 3-H), 4.77 (2H, s, NCH₂Ar), 3.76 (2H, s, NCH₂-tBu), 1.91 (3H, s, 5-CH₃).
MASS (EI) m/z : 470(M⁺), 455, 413, 309, 116.

### (Example 1503)

### Compound of Formula No. C-867

¹H-NMR (CDCl₃) δ(ppm) : 7.62 (2H, d, J=8Hz, COOC₆H₄-4-CF₃), 7.28 (1H, s, 3-H), 7.18 (2H, d, J=8Hz, COOC₆H₄-4-CF₃), 4.78 (2H, s, NCH₂Ar), 3.99 (2H, d, J=7Hz, NCH₂-cBu), 1.91 (3H, s, 5-CH₃).
MASS (EI) m/z : 468(M⁺), 307, 239, 116.

### (Example 1504)

### Compound of Formula No. C-868

¹H-NMR (CDCl₃) δ(ppm) : 7.64-7.27 (8H, m, C₆H₄-3-CN and COOC₆H₄-3-CF₃), 7.28 (1H, s, 3-H), 4.78 (2H, s, NCH₂Ar), 4.00 (2H, d, J=7Hz, NCH₂-cBu), 1.92 (3H, s, 5-CH₃).
MASS (EI) m/z : 468(M⁺), 449, 307, 239, 116.

### (Example 1505)

### Compound of Formula No. C-869

¹H-NMR (CDCl₃) δ(ppm) : 7.64-7.28 (8H, m, C₆H₄-3-CN and COOC₆H₄-2-CF₃), 7.22 (1H, s, 3-H), 4.76 (2H, s, NCH₂Ar), 3.98 (2H, d, J=7Hz, NCH₂-cBu), 1.93 (3H, s, 5-CH₃).
MASS (EI) m/z : 468(M⁺), 307, 239, 116.

### (Example 1506)

### Compound of Formula No. C-870

¹H-NMR (CDCl₃) δ(ppm) : 7.30 (1H, s, 3-H), 7.02 (2H, d, J=6Hz, COOC₆H₄-4-F), 7.02 (2H, d, J=6Hz, COOC₆H₄-4-F), 4.78 (2H, s, NCH₂Ar), 3.76 (2H, s, NCH₂-tBu), 1.88 (3H, s, 5-CH₃).
MASS (EI) m/z : 420(M⁺), 405, 309, 239, 116.

### (Example 1507)

### Compound of Formula No. C-871

¹H-NMR (CDCl₃) δ(ppm) : 7.63-6.80 (8H, m, C₆H₄-3-CN and COOC₆H₄-3-F), 7.31 (1H, s, 3-H), 4.78 (2H, s, NCH₂Ar), 3.77 (2H, s, NCH₂-tBu), 1.88 (3H, s, 5-CH₃).
MASS (EI) m/z : 420(M⁺). 405, 309, 239, 116.

### (Example 1508)

### Compound of Formula No. C-872

¹H-NMR (CDCl₃) δ(ppm) : 7.62-7.10 (8H, m, C₆H₄-3-CN and COOC₆H₄-2-F), 7.32 (1H, s, 3-H), 4.79 (2H, s, NCH₂Ar), 3.75 (2H, s, NCH₂-tBu), 1.89 (3H, s, 5-CH₃).
MASS (EI) m/z : 420(M⁺), 405, 309, 239, 116.

### (Example 1509)

### Compound of Formula No. C-873

¹H-NMR (CDCl₃) δ(ppm) : 7.27 (1H, s, 3-H), 7.02 (2H, d, J=6Hz, COOC₆H₄-4-F), 7.02 (2H, d, J=6Hz, COOC₆H₄-4-F), 4.77 (2H, s, NCH₂Ar), 3.99 (2H, d, J=7Hz, NCH₂-cBu), 1.91 (3H, s, 5-CH₃).
MASS (EI) m/z : 418(M⁺), 307, 239, 116.

### (Example 1510)

### Compound of Formula No. C-874

¹H-NMR (CDCl₃) δ(ppm) : 7.63-6.80 (8H, m, C₆H₄-3-CN and COOC₆H₄-3-F), 7.27 (1H, s, 3-H), 4.77 (2H, s, NCH₂Ar), 3.99 (2H, d, J=7Hz, NCH₂-cBu), 1.90 (3H, s, 5-CH₃).
MASS (EI) m/z : 418(M⁺), 307, 239, 116.

### (Example 1511)

### Compound of Formula No. C-875

¹H-NMR (CDCl₃) δ(ppm) : 7.63-7.09 (8H, m, C₆H₄-3-CN and COOC₆H₄-2-F), 7.28 (1H, s, 3-H), 4.78 (2H, s, NCH₂Ar), 3.98 (2H, d, J=7Hz, NCH₂-cBu), 1.92 (3H, s, 5-CH₃).
MASS (EI) m/z : 418(M⁺), 307, 239, 116.

### (Example 1512)

### Compound of Formula No. C-876

¹H-NMR (CDCl₃) δ(ppm) : 7.30 (1H, s, 3-H), 7.13 (2H, d, J=8Hz, COOC₆H₄-4-CH₃), 6.92 (2H, d, J=8Hz, COOC₆H₄-4-CH₃), 4.78 (2H, s, NCH₂Ar), 3.76 (2H, s, NCH₂-tBu), 2.32 (3H, s, COOC₆H₄-4-CH₃), 1.89 (3H, s, 5-CH₃).
MASS (EI) m/z : 416(M⁺), 401, 309, 239, 116.

### (Example 1513)

### Compound of Formula No. C-877

¹H-NMR (CDCl₃) δ(ppm) : 7.30 (1H, s, 3-H), 4.78 (2H, s, NCH₂Ar), 3.76 (2H, s, NCH₂-tBu), 2.34 (3H, s, COOC₆H₄-3-CH₃), 1.89 (3H, s, 5-CH₃).
MASS (EI) m/z : 416(M⁺), 401, 309, 239, 116.

### (Example 1514)

### Compound of Formula No. C-878

¹H-NMR (CDCl₃) δ(ppm) : 7.29 (1H, s, 3-H), 4.78 (2H, s, NCH₂Ar), 3.76 (2H, s, NCH₂-tBu), 2.10 (3H, s, COOC₆H₄-3-CH₃), 1.91 (3H, s, 5-CH₃).
MASS (EI) m/z : 416(M⁺), 401, 309, 239, 116.

### (Example 1515)

### Compound of Formula No. C-879

¹H-NMR (CDCl₃) δ(ppm) : 7.26 (1H, s, 3-H), 7.13 (2H, d, J=8Hz, COOC₆H₄-4-CH₃), 6.92 (2H, d, J=8Hz, COOC₆H₄-4-CH₃), 4.77 (2H, s, NCH₂Ar), 3.98 (2H, d, J=7Hz, NCH₂-cBu), 2.32 (3H, s, COOC₆H₄-4-CH₃), 1.91 (3H, s, 5-CH₃).
MASS (EI) m/z : 414(M⁺), 307, 239, 116.

### (Example 1516)

### Compound of Formula No. C-880

¹H-NMR (CDCl₃) δ(ppm) : 7.27 (1H, s, 3-H), 4.77 (2H, s, NCH₂Ar), 3.98 (2H, d, J=7Hz, NCH₂-cBu), 2.34 (3H, s, COOC₆H₄-3-CH₃), 1.92 (3H, s, 5-CH₃).
MASS (EI) m/z : 414(M⁺), 307, 239, 116.

### (Example 1517)

### Compound of Formula No. C-881

¹H-NMR (CDCl₃) δ(ppm) : 7.25 (1H, s, 3-H), 4.77 (2H, s, NCH₂Ar), 3.98 (2H, d, J=7Hz, NCH₂-cBu), 2.10 (3H, s, COOC₆H₄-3-CH₃), 1.94 (3H, s, 5-CH₃).
MASS (EI) m/z : 414(M⁺), 307, 239, 116.

### (Example 1518)

### Compound of Formula No. C-882

¹H-NMR (CDCl₃) δ(ppm) : 7.76 (2H, d, J=9Hz, COOC₆H₄-4-SOCH₂CN), 7.33 (2H, d, J=9Hz, COOC₆H₄-4-SOCH₂CN), 7.27 (1H, s, 3-H), 4.78 (2H, s, NCH₂Ar), 4.00 (2H, d, J=7Hz, NCH₂-cBu), 3.77 (1H, d, J=16Hz, SOCH₂CN), 3.64 (1H, d, J=16Hz, SOCH₂CN), 1.91 (3H, s, 5-CH₃).
MASS (EI) m/z : 487(M⁺), 471, 432, 307, 239, 116.

### (Example 1519)

### Compound of Formula No. C-883

¹H-NMR (CDCl₃) δ(ppm) : 7.30 (1H, s, 3-H), 6.97 (2H, d, J=9Hz, COOC₆H₄-4-OCH₃), 6.85 (2H, d, J=9Hz, COOC₆H₄-4-OCH₃), 4.78 (2H, s, NCH₂Ar), 3.78 (3H, s, OCH₃), 3.76 (2H, s, NCH₂-tBu), 1.88 (3H, s, 5-CH₃).
MASS (EI) m/z : 432(M⁺), 417, 309, 239, 123.

### (Example 1520)

### Compound of Formula No. C-884

¹H-NMR (CDCl₃) δ(ppm) : 7.31 (1H, s, 3-H), 4.89 (2H, s, NCH₂Ar), 3.78 (3H, s, OCH₃), 3.76 (2H, s, NCH₂-tBu), 1.89 (3H, s, 5-CH₃).
MASS (EI) m/z : 432(M⁺), 417, 309, 239, 116.

### (Example 1521)

### Compound of Formula No. C-885

¹H-NMR (CDCl₃) δ(ppm) : 7.30 (1H, s, 3-H), 4.79 (2H, s, NCH₂Ar), 3.84 (3H, s, OCH₃), 3.74 (2H, s, NCH₂-tBu), 1.94 (3H, s, 5-CH₃).
MASS (EI) m/z : 432(M⁺), 417, 309, 239, 116.

### (Example 1522)

### Compound of Formula No. C-886

¹H-NMR (CDCl₃) δ(ppm) : 7.27 (1H, s, 3-H), 6.96 (2H, d, J=9Hz, COOC₆H₄-4-OCH₃), 6.85 (2H, d, J=9Hz, COOC₆H₄-4-OCH₃), 4.76 (2H, s, NCH₂Ar), 3.98 (2H, d, J=7Hz, NCH₂-cBu), 3.78 (3H, s, OCH₃), 1.91 (3H, s, 5-CH₃).
MASS (EI) m/z : 430(M⁺), 307, 239, 123.

### (Example 1523)

### Compound of Formula No. C-887

¹H-NMR (CDCl₃) δ(ppm) : 7.27 (1H, s, 3-H), 4.77 (2H, s, NCH₂Ar), 3.98 (2H, d, J=7Hz, NCH₂-cBu), 3.79 (3H, s, OCH₃), 1.91 (3H, s, 5-CH₃).
MASS (EI) m/z : 430(M⁺), 307, 239, 116.

### (Example 1524)

### Compound of Formula No. C-888

¹H-NMR (CDCl₃) δ(ppm) : 7.27 (1H, s, 3-H), 4.78 (2H, s, NCH₂Ar), 3.96 (2H, d, J=7Hz, NCH₂-cBu), 3.85 (3H, s, OCH₃), 1.97 (3H, s, 5-CH₃).
MASS (EI) m/z : 430(M⁺), 307, 239, 116.

### (Example 1525)

### Compound of Formula No. C-889

¹H-NMR (CDCl₃) δ(ppm) : 7.57 (2H, d, J=9Hz, COOC₆H₄-4-Cl), 7.29 (1H, s, 3-H), 6.99 (2H, d, J=9Hz, COOC₆H₄-4-Cl), 4.77 (2H, s, NCH₂Ar), 3.77 (2H, d, J=7Hz, NCH₂-iPr), 1.88 (3H, s, 5-CH₃).
MASS (EI) m/z : 422(M⁺), 295, 239, 116.

### (Example 1526)

### Compound of Formula No. C-890

¹H-NMR (CDCl₃) δ(ppm) : 7.60 (2H, d, J=9Hz, COOC₆H₄-4-Cl), 7.30 (1H, s, 3-H), 7.00 (2H, d, J=9Hz, COOC₆H₄-4-Cl), 4.78 (2H, s, NCH₂Ar), 3.76 (2H, s, NCH₂-tBu), 1.87 (3H, s, 5-CH₃).
MASS (EI) m/z : 436(M⁺), 309, 239, 116.

### (Example 1527)

### Compound of Formula No. C-891

¹H-NMR (CDCl₃) δ(ppm) : 7.30 (2H, d, J=9Hz, COOC₆H₄-4-Cl), 7.27 (1H, s, 3-H), 6.99 (2H, d, J=9Hz, COOC₆H₄-4-Cl), 4.76 (2H, s, NCH₂Ar), 3.99 (2H, d, J=7Hz, NCH₂-cBu), 1.90 (3H, s, 5-CH₃).
MASS (EI) m/z : 434(M⁺), 307, 239, 116.

### (Example 1528)

### Compound of Formula No. C-892

¹H-NMR (CDCl₃) δ(ppm) : 7.63-6.95 (8H, m, C₆H₄-3-CN and COOC₆H₄-3-Cl), 7.29 (1H, s, 3-H), 4.78 (2H, s, NCH₂Ar), 3.78 (2H, d, J=8Hz, NCH₂-iPr), 1.89 (3H, s, 5-CH₃).
MASS (EI) m/z : 422(M⁺), 295, 239, 116.

### (Example 1529)

### Compound of Formula No. C-893

¹H-NMR (CDCl₃) δ(ppm) : 7.63-6.94 (8H, m, C₆H₄-3-CN and COOC₆H₄-3-Cl), 7.28 (1H, s, 3-H), 4.78 (2H, s, NCH₂Ar), 3.76 (2H, s, NCH₂-tBu), 1.87 (3H, s, 5-CH₃).
MASS (EI) m/z : 436(M⁺), 421, 309, 239, 116.

### (Example 1530)

### Compound of Formula No. C-894

¹H-NMR (CDCl₃) δ(ppm) : 7.63-6.95 (8H, m, C₆H₄-3-CN and COOC₆H₄-3-Cl), 7.27 (1H, s, 3-H), 4.77 (2H, s, NCH₂Ar), 3.99 (2H, d, J=7Hz, NCH₂-cBu), 1.90 (3H, s, 5-CH₃).
MASS (EI) m/z : 434(M⁺), 307, 239, 116.

### (Example 1531)

### Compound of Formula No. C-895

¹H-NMR (CDCl₃) δ(ppm) : 7.63-7.12 (8H, m, C₆H₄-3-CN and COOC₆H₄-2-Cl), 7.27 (1H, s, 3-H), 4.79 (2H, s, NCH₂Ar), 3.77 (2H, d, J=8Hz, NCH₂-iPr), 1.95 (3H, s, 5-CH₃).
MASS (EI) m/z : 422(M⁺), 295, 239, 116.

### (Example 1532)

### Compound of Formula No. C-896

¹H-NMR (CDCl₃) δ(ppm) : 7.62-7.12 (8H, m, C₆H₄-3-CN and COOC₆H₄-2-Cl), 7.27 (1H, s, 3-H), 4.79 (2H, s, NCH₂Ar), 3.76 (2H, s, NCH₂-tBu), 1.94 (3H, s, 5-CH₃).
MASS (EI) m/z : 436(M⁺), 421, 309, 239, 116.

### (Example 1533)

### Compound of Formula No. C-897

¹H-NMR (CDCl₃) δ(ppm) : 7.63-7.12 (8H, m, C₆H₄-3-CN and COOC₆H₄-2-Cl), 7.27 (1H, s, 3-H), 4.78 (2H, s, NCH₂Ar), 3.98 (2H, d, J=7Hz, NCH₂-cBu), 1.96 (3H, s, 5-CH₃).
MASS (EI) m/z : 434(M⁺), 307, 279, 239, 116.

### (Example 1534)

### Compound of Formula No. C-898

¹H-NMR (CDCl₃) δ(ppm) : 7.64-7.07 (7H, m, C₆H₄-3-CN and COOC₆H₃-2,4-Cl₂), 7.28 (1H, s, 3-H), 4.77 (2H, s, NCH₂Ar), 3.77 (2H, d, J=7Hz, NCH₂-iPr), 1.93 (3H, s, 5-CH₃).
MASS (EI) m/z : 456(M⁺), 295, 239, 116.

### (Example 1535)

### Compound of Formula No. C-899

¹H-NMR (CDCl₃) δ(ppm) : 7.63-7.07 (7H, m, C₆H₄-3-CN and COOC₆H₃-2,4-Cl₂), 7.29 (1H, s, 3-H), 4.78 (2H, s, NCH₂Ar), 3.76 (2H, s, NCH₂-tBu), 1.92 (3H, s, 5-CH₃).
MASS (EI) m/z : 470(M⁺), 455, 309, 239, 116.

### (Example 1536)

### Compound of Formula No. C-900

¹H-NMR (CDCl₃) δ(ppm) : 7.63-7.07 (7H, m, C₆H₄-3-CN and COOC₆H₃-2,4-Cl₂), 7.26 (1H, s, 3-H), 4.76 (2H, s, NCH₂Ar), 3.98 (2H, d, J=7Hz, NCH₂-cBu), 1.94 (3H, s, 5-CH₃).
MASS (EI) m/z : 468(M⁺), 307, 279, 239, 116.

### (Example 1537)

### Compound of Formula No. C-901

¹H-NMR (CDCl₃) δ(ppm) : 7.30 (1H, s, 3-H), 7.20 (2H, d, J=9Hz, COOC₆H₄-4-OCF₃), 7.08 (2H, d, J=9Hz, COOC₆H₄-4-OCF₃), 4.78 (2H, s, NCH₂Ar), 3.78 (2H, d, J=8Hz, NCH₂-iPr), 1.89 (3H, s, 5-CH₃).
MASS (EI) m/z : 472(M⁺), 295, 116, 57.

### (Example 1538)

### Compound of Formula No. C-902

¹H-NMR (CDCl₃) δ(ppm) : 7.31 (1H, s, 3-H), 7.20 (2H, d, J=9Hz, COOC₆H₄-4-OCF₃), 7.08 (2H, d, J=9Hz, COOC₆H₄-4-OCF₃), 4.78 (2H, s, NCH₂Ar), 3.77 (2H, s, NCH₂-tBu), 1.88 (3H, s, 5-CH₃).
MASS (EI) m/z : 486(M⁺), 471, 309, 239, 116.

### (Example 1539)

### Compound of Formula No. C-903

¹H-NMR (CDCl₃) δ(ppm) : 7.27 (1H, s, 3-H), 7.20 (2H, d, J=9Hz, COOC₆H₄-4-OCF₃), 7.08 (2H, d, J=9Hz, COOC₆H₄-4-OCF₃), 4.77 (2H, s, NCH₂Ar), 3.99 (2H, d, J=7Hz, NCH₂-cBu), 1.91 (3H, s, 5-CH₃).
MASS (EI) m/z : 484(M⁺), 307, 239, 116.

### (Example 1540)

### Compound of Formula No. C-904

¹H-NMR (CDCl₃) δ(ppm) : 7.63-6.96 (8H, m, C₆H₄-3-CN and COOC₆H₄-3-OCF₃), 7.30 (1H, s, 3-H), 4.78 (2H, s, NCH₂Ar), 3.78 (2H, d, J=7Hz, NCH₂-iPr), 1.89 (3H, s, 5-CH₃).
MASS (EI) m/z : 472(M⁺), 295, 239, 116.

### (Example 1541)

### Compound of Formula No. C-905

¹H-NMR (CDCl₃) δ(ppm) : 7.63-6.95 (8H, m, C₆H₄-3-CN and COOC₆H₄-3-OCF₃), 7.31 (1H, s, 3-H), 4.79 (2H, s, NCH₂Ar), 3.77 (2H, s, NCH₂-tBu), 1.88 (3H, s, 5-CH₃).
MASS (EI) m/z : 486(M⁺), 471, 309, 239, 116.

### (Example 1542)

### Compound of Formula No. C-906

¹H-NMR (CDCl₃) δ(ppm) : 7.63-6.95 (8H, m, C₆H₄-3-CN and COOC₆H₄-3-OCF₃), 7.27 (1H, s, 3-H), 4.77 (2H, s, NCH₂Ar), 3.99 (2H, d, J=7Hz, NCH₂-cBu), 1.90 (3H, s, 5-CH₃).
MASS (EI) m/z : 484(M⁺), 307, 239, 116.

### (Example 1543)

### Compound of Formula No. C-907

¹H-NMR (CDCl₃) δ(ppm) : 7.33 (1H, s, 3-H), 4.79 (2H, s, NCH₂Ar), 4.08 (2H, s, SO₂CH₂CN), 4.03(2H, d, J=7Hz, NCH₂-cBu), 1.92 (3H, s, 5-CH₃).
MASS (EI) m/z : 503(M⁺), 488, 307, 239, 116.

### (Example 1544)

### Compound of Formula No. C-908

¹H-NMR (CDCl₃) δ(ppm) : 7.27 (1H, s, 3-H), 4.76 (1H, d, J=14Hz, NCH₂Ar), 4.75 (1H, d, J=14Hz, NCH₂Ar), 4.03 (2H, d, J=7Hz, NCH₂-cBu), 3.51 (1H, d, J=16Hz, SOCH₂CN), 3.07 (1H, d, J=16Hz, SOCH₂CN), 1.92 (3H, s, 5-CH₃).
MASS (EI) m/z : 487(M⁺), 471, 447, 432, 307, 239, 116.

### (Example 1545)

### Compound of Formula No. C-909

¹H-NMR (CDCl₃) δ(ppm) : 7.32 (1H, s, 3-H), 4.78 (1H, d, J=14Hz, NCH₂Ar), 4.76 (1H, d, J=14Hz, NCH₂Ar), 3.80 (2H, s, NCH₂-tBu), 3.55 (1H, d, J=16Hz, SOCH₂CN), 3.14 (1H, d, J=16Hz, SOCH₂CN), 1.87 (3H, s, 5-CH₃).
MASS (EI) m/z : 489(M⁺), 473, 434, 309, 116.

### (Example 1546)

### Compound of Formula No. C-910

¹H-NMR (CDCl₃) δ(ppm) : 7.26 (1H, s, 3-H), 4.80 (2H, s, NCH₂Ar), 3.80 (2H, s, NCH₂-tBu), 3.71(2H, s, SO₂CH₂CN), 1.98 (3H, s, 5-CH₃).
MASS (EI) m/z : 505(M⁺), 490, 309, 116.

### (Example 1547)

### Compound of Formula No. C-911

¹H-NMR (CDCl₃) δ(ppm) : 7.25 (1H, s, 3-H), 4.79 (2H, brs, COCH₂O), 4.73 (2H, s, NCH₂Ar), 3.78 (2H, d, J=8Hz, NCH₂-iPr), 1.82 (3H, s, 5-CH₃).
MASS (EI) m/z : 453(M⁺), 380, 267, 186.

### (Example 1548)

### Compound of Formula No. C-912

¹H-NMR (CDCl₃) δ(ppm) : 7.26 (1H, s, 3-H), 4.78 (2H, brs, COCH₂O), 4.73 (2H, s, NCH₂Ar), 3.76 (2H, s, NCH₂-tBu), 1.82 (3H, s, 5-CH₃).
MASS (EI) m/z : 467(M⁺), 452, 380, 281, 186.

### (Example 1549)

### Compound of Formula No. C-913

¹H-NMR (CDCl₃) δ(ppm) : 7.85-7.33 (11H, m, C₆H₄-3-CN and COOC₁₀H₇), 7.40 (1H, s, 3-H), 4.83 (2H, s, NCH₂Ar), 3.79 (2H, d, J=8Hz, NCH₂-iPr), 1.99 (3H, s, 5-CH₃).
MASS (EI) m/z : 438(M⁺), 295, 116.

### (Example 1550)

### Compound of Formula No. C-914

¹H-NMR (CDCl₃) δ(ppm) : 7.84-7.33 (11H, m, C₆H₄-3-CN and COOC₁₀H₇), 7.41 (1H, s, 3-H), 4.83 (2H, s, NCH₂Ar), 3.78 (2H, s, NCH₂-tBu), 1.98 (3H, s, 5-CH₃).
MASS (EI) m/z : 452(M⁺), 309, 116.

### (Example 1551)

### Compound of Formula No. C-915

¹H-NMR (CDCl₃) δ(ppm) : 7.85-7.33 (11H, m, C₆H₄-3-CN and COOC₁₀H₇), 7.38 (1H, s, 3-H), 4.82 (2H, s, NCH₂Ar), 4.01 (2H, d, J=7Hz, NCH₂-cBu), 2.01 (3H, s, 5-CH₃).
MASS (EI) m/z : 450(M⁺), 307, 116.

### (Example 1552)

### Compound of Formula No. C-916

¹H-NMR (CDCl₃) δ(ppm) : 7.84-7.18 (11H, m, C₆H₄-3-CN and COOC₁₀H₇), 7.34 (1H, s, 3-H), 4.81 (2H, s, NCH₂Ar), 3.79 (2H, d, J=8Hz, NCH₂-iPr), 1.95 (3H, s, 5-CH₃).
MASS (EI) m/z : 438(M⁺), 295, 116.

### (Example 1553)

### Compound of Formula No. C-917

¹H-NMR (CDCl₃) δ(ppm) : 7.84-7.17 (11H, m, C₆H₄-3-CN and COOC₁₀H₇), 7.35 (1H, s, 3-H), 4.81 (2H, s, NCH₂Ar), 3.77 (2H, s, NCH₂-tBu), 1.94 (3H, s, 5-CH₃).
MASS (EI) m/z : 452(M⁺), 309, 116.

### (Example 1554)

### Compound of Formula No. C-918

¹H-NMR (CDCl₃ δ(ppm) : 7.85-7.18 (11H, m, C₆H₄-3-CN and COOC₁₀H₇), 7.31 (1H, s, 3-H), 4.80 (2H, s, NCH₂Ar), 4.00 (2H, d, J=7Hz, NCH₂-cBu), 1.97 (3H, s, 5-CH₃).
MASS (EI) m/z : 450(M⁺), 307, 116.

### (Example 1555)

### Compound of Formula No. C-919

¹H-NMR (CDCl₃) δ(ppm) : 7.63-6.91 (7H, m, C₆H₄-3-CN and COOC₆H₃-2,6-F₂), 7.33 (1H, s, 3-H), 4.80 (2H, s, NCH₂Ar), 3.76 (2H, s, NCH₂-tBu), 1.88 (3H, s, 5-CH₃).
MASS (EI) m/z : 438(M⁺), 309, 116.

### (Example 1556)

### Compound of Formula No. C-920

¹H-NMR (CDCl₃) δ(ppm) : 7.63-6.91 (7H, m, C₆H₄-3-CN and COOC₆H₃-2,6-F₂), 7.30 (1H, s, 3-H), 4.78 (2H, S, NCH₂Ar), 3.98 (2H, d, J=7Hz, NCH₂-cBu), 1.91 (3H, s, 5-CH₃).
MASS (EI) m/z : 436(M⁺), 307, 149, 116.

### (Example 1557)

### Compound of Formula No. C-921

¹H-NMR (CDCl₃) δ(ppm) : 8.89 (1H, d, J=4Hz, COO-(2-H-6-Quin)), 8.12 (1H, d, J=8Hz, COO-(8-H-6-Quin)), 8.10 (1H, d, J=9Hz, COO-(4-H-6-Quin)), 7.35 (1H, s, 3-H), 4.82 (2H, s, NCH₂Ar), 4.12 (2H, d, J=7Hz, NCH₂-iPr), 1.94 (3H, s, 5-CH₃).
MASS (EI) m/z : 439(M⁺), 295, 116.

### (Example 1558)

### Compound of Formula No. C-922

¹H-NMR (CDCl₃) δ(ppm) : 8.89 (1H, d, J=4Hz, COO-(2-H-6-Quin)), 8.12 (1H, d, J=8Hz, COO-(8-H-6-Quin)), 8.10 (1H, d, J=9Hz, COO-(4-H-6-Quin)), 7.35 (1H, s, 3-H), 4.82 (2H, s, NCH₂Ar), 3.78 (2H, s, NCH₂-tBu), 1.94 (3H, s, 5-CH₃).
MASS (EI) m/z : 453(M⁺), 309, 116.

### (Example 1559)

### Compound of Formula No. C-923

¹H-NMR (CDCl₃) δ(ppm) : 8.90 (1H, d, J=4Hz, COO-(2-H-6-Quin)), 8.12 (1H, d, J=7Hz, COO-(8-H-6-Quin)), 8.10 (1H, d, J=9Hz, COO-(4-H-6-Quin)), 7.32 (1H, s, 3-H), 4.81 (2H, s, NCH₂Ar), 4.01 (2H, d, J=7Hz, NCH₂-cBu), 1.97 (3H, S, 5-CH₃).
MASS (EI) m/z : 451 (M⁺), 307, 116.

### (Example 1560)

### Compound of Formula No. C-924

¹H-NMR (CDCl₃) δ(ppm) : 7.65 (2H, d, J=9Hz, COOC₆H₄-4-CN), 7.29 (1H, s, 3-H), 7.19 (2H, d, J=9Hz, COOC₆H₄-4-CN), 4.78 (2H, s, NCH₂Ar), 3.78 (2H, d, J=8Hz, NCH₂-iPr), 1.88 (3H, s, 5-CH₃).
MASS (EI) m/z : 413(M⁺), 295, 239, 116.

### (Example 1561)

### Compound of Formula No. C-925

¹H-NMR (CDCl₃) δ(ppm) : 7.66 (2H, d, J=9Hz, COOC₆H₄-4-CN), 7.31 (1H, s, 3-H), 7.19 (2H, d, J=9Hz, COOC₆H₄-4-CN), 4.79 (2H, s, NCH₂Ar), 3.77 (2H, s, NCH₂-tBu), 1.87 (3H, s, 5-CH₃).
MASS (EI) m/z : 427(M⁺), 412, 309, 239, 116.

### (Example 1562)

### Compound of Formula No. C-926

¹H-NMR (CDCl₃) δ(ppm) : 7.65 (2H, d, J=9Hz, COOC₆H₄-4-CN), 7.27 (1H, s, 3-H), 7.19 (2H, d, J=9Hz, COOC₆H₄-4-CN), 4.78 (2H, s, NCH₂Ar), 3.99 (2H, d, J=7Hz, NCH₂-cBu), 1.90 (3H, s, 5-CH₃).
MASS (EI) m/z : 425(M⁺), 307, 239, 116.

### (Example 1563)

### Compound of Formula No. C-927

¹H-NMR (CDCl₃) δ(ppm) : 8.80 (2H, d, J=5Hz, COS- (4,6-H₂-2-Pym)), 7.32 (1H, s, 3-H), 7.28 (1H, dd, J=5,5Hz, COS-(5-H-2-Pym)), 4.93 (1H, d, J=14Hz, NCH₂Ar), 4.67 (1H, d, J=14Hz, NCH₂Ar), 3.81 (2H, d, J=8Hz, NCH₂-iPr), 1.91 (3H, s, 5-CH₃).
MASS (EI) m/z : 406(M⁺), 373, 346, 295, 116.

### (Example 1564)

### Compound of Formula No. C-928

¹H-NMR (CDCl₃) δ(ppm) : 8.76 (2H, d, J=5Hz, COS-(4,6-H₂-2-Pym)), 7.28 (1H, s, 3-H), 7.25 (1H, dd, J=5,5Hz, COS-(5-H-2-Pym)), 4.91 (1H, d, J=14Hz, NCH₂Ar), 4.63 (1H, d, J=14Hz, NCH₂Ar), 3.76 (2H, s, NCH₂-tBu), 1.88 (3H, s, 5-CH₃).
MASS (EI) m/z : 420(M⁺), 405, 360, 309, 116.

### (Example 1565)

### Compound of Formula No. C-929

¹H-NMR (CDCl₃) δ(ppm) : 8.79 (2H, d, J=5Hz, COS-(4,6-H₂-2-Pym)), 7.29 (1H, s, 3-H), 7.28 (1H, dd, J=5,5Hz, COS-(5-H-2-Pym)), 4.91 (1H, d, J=14Hz, NCH₂Ar), 4.68 (1H, d, J=14Hz, NCH₂Ar), 4.02 (2H, d, J=7Hz, NCH₂-cBu), 1.92 (3H, s, 5-CH₃).
MASS (EI) m/z : 418(M⁺), 358, 307, 279, 116.

### (Example 1566)

### Compound of Formula No. C-930

¹H-NMR (CDCl₃) δ(ppm) : 7.28 (1H, s, 3-H), 4.77 (2H, s, NCH₂Ar), 3.77 (2H, d, J=8Hz, NCH₂-iPr), 2.40 (3H, s, COO-(5-CH₃-3-Isox)), 1.86 (3H, s, 5-CH₃).
MASS (EI) m/z : 393(M⁺), 295, 116.

### (Example 1567)

### Compound of Formula No. C-931

¹H-NMR (CDCl₃) δ(ppm) : 7.28 (1H, s, 3-H), 4.78 (2H, s, NCH₂Ar), 3.76 (2H, s, NCH₂-tBu), 2.40 (3H, s, COO-(5-CH₃-3-Isox)), 1.85 (3H, s, 5-CH₃).
MASS (EI) m/z : 407(M⁺), 392, 309, 282, 116.

### (Example 1568)

### Compound of Formula No. C-932

¹H-NMR (CDCl₃) δ(ppm) : 7.26 (1H, s, 3-H), 4.81 (2H, s, NCH₂Ar), 3.71 (2H, s, NCH₂-tBu), 2.25 (3H, s, CO-(3-oxo-5-CH₃-2-Isox)), 1.90 (3H, s, 5-CH₃).
MASS (EI) m/z : 407(M⁺), 309, 280, 116.

### (Example 1569)

### Compound of Formula No. C-933

¹H-NMR (CDCl₃) δ(ppm) : 7.24 (1H, s, 3-H), 4.76 (2H, s, NCH₂Ar), 3.98 (2H, d, J=7Hz, NCH₂-cBu), 2.40 (3H, s, COO-(5-CH₃-3-Isox)), 1.88 (3H, s, 5-CH₃).
MASS (EI) m/z : 405(M⁺), 307, 190, 116.

### (Example 1570)

### Compound of Formula No. C-934

¹H-NMR (CDCl₃) δ(ppm) : 7.24 (1H, s, 3-H), 4.80 (2H, s, NCH₂Ar), 3.94 (2H, d, J=7Hz, NCH₂-cBu), 2.25 (3H, s, CO-(3-oxo-5-CH₃-2-Isox)), 1.92 (3H, s, 5-CH₃).
MASS (EI) m/z : 405(M⁺), 307, 278, 116.

### (Example 1571)

### Compound of Formula No. C-935

¹H-NMR (CDCl₃) δ(ppm) : 8.90 (1H, d, J=4Hz, COO-(2-H-5-Quin)), 7.98 (1H, d, J=8Hz, COO-(8-H-5-Quin)), 7.95 (1H, d, J=5Hz, COO-(4-H-5-Quin)), 7.41 (1H, s, 3-H), 4.84 (2H, s, NCH₂Ar), 3.81 (2H, d, J=8Hz, NCH₂-iPr), 1.97 (3H, s, 5-CH₃).
MASS (EI) m/z : 439(M⁺), 295, 116.

### (Example 1572)

### Compound of Formula No. C-936

¹H-NMR (CDCl₃) δ(ppm) : 8.90 (1H, d, J=4Hz, COO-(2-H-5-Quin)), 7.98 (1H, d, J=8Hz, COO-(8-H-5-Quin)), 7.94 (1H, d, J=5Hz, COO- (4-H-5-Quin)), 7.41 (1H, s, 3-H), 4.84 (2H, s, NCH₂Ar), 3.79 (2H, s, NCH₂-tBu), 1.97 (3H, s, 5-CH₃).
MASS (EI) m/z : 453(M⁺), 309, 116.

### (Example 1573)

### Compound of Formula No. C-937

¹H-NMR (CDCl₃) δ(ppm) : 8.91 (1H, d, J=4Hz, COO-(2-H-5-Quin)), 7.98 (1H, d, J=9Hz, COO-(8-H-5-Quin)), 7.93 (1H, d, J=8Hz, COO-(4-H-5-Quin)), 7.37 (1H, s, 3-H), 4.83 (2H, s, NCH₂Ar), 4.02 (2H, d, J=7Hz, NCH₂-cBu), 1.99 (3H, s, 5-CH₃).
MASS (EI) m/z : 451(M⁺), 307, 116.

### (Example 1574)

### Compound of Formula No. C-938

¹H-NMR (CDCl₃) δ(ppm) : 8.89 (1H, d, J=5Hz, COO-(2-H-4-Quin)), 8.08 (1H, d, J=9Hz, COO-(8-H-4-Quin)), 7.39 (1H, s, 3-H), 4.85 (2H, s, NCH₂Ar), 3.82 (2H, d, J=8Hz, NCH₂-iPr), 1.95 (3H, s, 5-CH₃).
MASS (EI) m/z : 439(M⁺), 295, 116.

### (Example 1575)

### Compound of Formula No. C-939

¹H-NMR (CDCl₃) δ(ppm) : 8.89 (1H, d, J=5Hz, COO-(2-H-4-Quin)), 8.08 (1H, d, J=89Hz, COO-(8-H-4-Quin)), 7.40 (1H, s, 3-H), 4.85 (2H, s, NCH₂Ar), 3.81 (2H, s, NCH₂-tBu), 1.94 (3H, s, 5-CH₃).
MASS (EI) m/z : 453(M⁺), 438, 309.

### (Example 1576)

### Compound of Formula No. C-940

¹H-NMR (CDCl₃) δ(ppm) : 8.89 (1H, d, J=5Hz, COO-(2-H-4-Quin)), 8.08 (1H, d, J=8Hz, COO-(8-H-4-Quin)), 7.36 (1H, s, 3-H), 4.84 (2H, s, NCH₂Ar), 4.03 (2H, d, J=7Hz, NCH₂-cBu), 1.96 (3H, s, 5-CH₃).
MASS (EI) m/z : 451(M⁺), 307, 116.

### (Example 1577)

### Compound of Formula No. C-941

¹H-NMR (CDCl₃) δ(ppm) : 7.64-7.28 (8H, m, C₆H₄-3-CN and COOC₆H₄-3-CN), 7.30 (1H, s, 3-H), 4.78 (2H, s, NCH₂Ar), 3.79 (2H, d, J=8Hz, NCH₂-iPr), 1.89 (3H, s, 5-CH₃).
MASS (EI) m/z : 413(M⁺), 295, 239, 116.

### (Example 1578)

### Compound of Formula No. C-942

¹H-NMR (CDCl₃) δ(ppm) : 7.64-7.34 (8H, m, C₆H₄-3-CN and COOC₆H₄-3-CN), 7.31 (1H, s, 3-H), 4.79 (2H, s, NCH₂Ar), 3.77 (2H, s, NCH₂-tBu), 1.88 (3H, s, 5-CH₃).
MASS (EI) m/z : 427(M⁺), 412, 309, 116, 89.

### (Example 1579)

### Compound of Formula No. C-943

¹H-NMR (CDCl₃) δ(ppm) : 7.64-7.30 (8H, m, C₆H₄-3-CN and COOC₆H₄-3- CN), 7.27 (1H, s, 3-H), 4.77 (2H, s, NCH₂Ar), 4.00 (2H, d, J=7Hz, NCH₂-cBu), 1.91 (3H, s, 5-CH₃).
MASS (EI) m/z : 425(M⁺), 307, 239, 116.

### (Example 1580)

### Compound of Formula No. C-944

¹H-NMR (CDCl₃) δ(ppm) : 8.45 (1H, d, J=4Hz, COO-(6-H-3-Pyr)), 8.35 (1H, d, J=2Hz, COO-(2-H-3-Pyr)), 7.31 (1H, s, 3-H), 4.79 (2H, s, NCH₂Ar), 3.79 (2H, d, J=8Hz, NCH₂-iPr), 1.90 (3H, s, 5-CH₃).
MASS (EI) m/z : 389(M⁺), 295, 239, 116.

### (Example 1581)

### Compound of Formula No. C-945

¹H-NMR (CDCl₃) δ(ppm) : 8.45 (1H, d, J=5Hz, COO-(6-H-3-Pyr)), 8.34 (1H, d, J=2Hz, COO-(2-H-3-Pyr)), 7.32 (1H, s, 3-H), 4.80 (2H, s, NCH₂Ar), 3.77 (2H, s, NCH₂-tBu), 1.89 (3H, s, 5-CH₃).
MASS (EI) m/z : 403(M⁺), 388, 309, 116.

### (Example 1582)

### Compound of Formula No. C-946

¹H-NMR (CDCl₃) δ(ppm) : 8.45 (1H, d, J=4Hz, COO-(6-H-3-Pyr)), 8.35 (1H, d, J=2Hz, COO-(2-H-3-Pyr)), 7.28 (1H, s, 3-H), 4.78 (2H, s, NCH₂Ar), 4.00 (2H, d, J=7Hz, NCH₂-cBu), 1.92 (3H, s, 5-CH₃).
MASS (EI) m/z : 401(M⁺), 307, 239, 116.

### (Example 1583)

### Compound of Formula No. C-947

¹H-NMR (CDCl₃) δ(ppm) : 8.22 (1H, d, J=3Hz, COO-(6-H-2-Pyr)), 7.99 (1H, d, J=3Hz, COO-(4-H-2-Pyr)), 7.30 (1H, s, 3-H), 4.79 (2H, s, NCH₂Ar), 3.76 (2H, d, J=7Hz, NCH₂-iPr), 1.95 (3H, s, 5-CH₃).
MASS (EI) m/z : 457(M⁺), 295, 116.

### (Example 1584)

### Compound of Formula No. C-948

¹H-NMR (CDCl₃) δ(ppm) : 7.73-7.05 (7H, m, 3-H and C₆H₄-3-CN and CO-(4,6-H₂-2-oxo-1-Pyr)), 4.92 (1H, d, J=14Hz, NCH₂Ar), 4.86 (1H, d, J=14Hz, NCH₂Ar), 3.70 (2H, d, J=8Hz, NCH₂-ipr), 1.95 (3H, s, 5-CH₃).
MASS (EI) m/z : 457(M⁺), 295, 116.

### (Example 1585)

### Compound of Formula No. C-949

¹H-NMR (CDCl₃) δ(ppm) : 8.22 (1H, d, J=3Hz, COO-(6-H-2-Pyr)), 7.79 (1H, d, J=3Hz, COO-(4-H-2-Pyr)), 7.31 (1H, s, 3-H), 4.80 (2H, s, NCH₂Ar), 3.75 (2H, s, NCH₂-tBu), 1.94 (3H, s, 5-CH₃).
MASS (EI) m/z : 471(M⁺), 309, 116.

### (Example 1586)

### Compound of Formula No. C-950

¹H-NMR (CDCl₃) δ(ppm) : 7.74-7.06 (7H, m, 3-H and C₆H₄-3-CN and CO-(4,6-H₂-2-oxo-1-Pyr)), 4.91 (1H, d, J=14Hz, NCH₂Ar), 4.88 (1H, d, J=14Hz, NCH₂Ar), 3.69 (2H, s, NCH₂-tBu), 1.94 (3H, s, 5-CH₃).
MASS (EI) m/z : 471(M⁺), 309, 116.

### (Example 1587)

### Compound of Formula No. C-951

¹H-NMR (CDCl₃) δ(ppm) : 8.22 (1H, d, J=2Hz, COO-(6-H-2-Pyr)), 7.79 (1H, d, J=2Hz, COO-(4-H-2-Pyr)), 7.27 (1H, s, 3-H), 4.78 (2H, s, NCH₂Ar), 3.98 (2H, d, J=7Hz, NCH₂-cBu), 1.97 (3H, s, 5-CH₃).
MASS (EI) m/z : 469(M⁺), 307, 116.

### (Example 1588)

### Compound of Formula No. C-952

¹H-NMR (CDCl₃) δ(ppm) : 7.72-7.04 (7H, m, 3-H and C₆H₄-3-CN and CO-(4,6-H₂-2-oxo-1-Pyr)), 4.91 (1H, d, J=14Hz, NCH₂Ar), 4.87 (1H, d, J=14Hz, NCH₂Ar), 3.91 (2H, d, J=7Hz, NCH₂-cBu), 1.95 (3H, s, 5-CH₃).
MASS (EI) m/z : 469(M⁺), 307, 116.

### (Example 1589)

### Compound of Formula No. C-953

¹H-NMR (CDCl₃) δ(ppm) : 7.22 (1H, s, 3-H), 4.78 (2H, s, NCH₂Ar), 4.72 (2H, s, COCH₂O), 3.99 (2H, d, J=7Hz, NCH₂-cBu), 1.83 (3H, s, 5-CH₃).
MASS (El) m/z : 465(M⁺), 279, 186.

### (Example 1590)

### Compound of Formula No. C-954

¹H-NMR (CDCl₃) δ(ppm) : 7.25 (1H, s, 3-H), 4.78(2H, s, NCH₂Ar), 4.65 (2H, s, COCH₂O), 3.92 (2H, d, J=8Hz, NCH₂-cPent), 1.95 (3H, s, 5-CH₃).
MASS (EI) m/z : 429(M⁺), 294, 136.

### (Example 1591)

### Compound of Formula No. C-955

¹H-NMR (CDCl₃) δ(ppm) : 7.31 (1H, s, 3-H), 4.79 (2H, brs, NCH₂Ar), 4.52 (2H, s, COCH₂O), 3.80 (2H, d, J=7Hz, NCH₂-iPr), 1.83 (3H, s, 5-CH₃).
MASS (EI) m/z : 453(M⁺), 438, 410, 267, 158.

### (Example 1592)

### Compound of Formula No. C-956

¹H-NMR (CDCl₃) δ(ppm) : 7.32 (1H, s, 3-H), 4.78 (2H, brs, NCH₂Ar), 4.51 (2H, s, COCH₂O), 3.79 (2H, s, NCH₂-tBu), 1.82 (3H, s, 5-CH₃).
MASS (EI) m/z : 467(M⁺), 452, 410, 282, 158.

### (Example 1593)

### Compound of Formula No. C-957

¹H-NMR (CDCl₃) δ(ppm) : 7.28 (1H, s, 3-H), 4.81 (2H, brs, NCH₂Ar), 4.50 (2H, s, COCH₂O), 4.02 (2H, d, J=7Hz, NCH₂-cBu), 1.84 (3H, s, 5-CH₃).
MASS (EI) m/z : 465(M⁺), 450, 437, 280, 158.

### (Example 1594)

### Compound of Formula No. C-958

¹H-NMR (CDCl₃) δ(ppm) : 7.35 (1H, s, 3-H), 6.30 (1H, dd, J=2,2Hz, COO-(4-H-1-Pyza)), 4.76 (2H, s, NCH₂Ar), 3.77 (2H, d, J=7Hz, NCH₂-iPr), 1.93 (3H, s, 5-CH₃).
MASS (EI) m/z : 378(M⁺), 295, 116.

### (Example 1595)

### Compound of Formula No. C-959

¹H-NMR (CDCl₃) δ(ppm) : 7.35 (1H, s, 3-H), 6.30 (1H, dd, J=3,2Hz, COO-(4-H-1-Pyza)), 4.77 (2H, s, NCH₂Ar), 3.76 (2H, s, NCH₂-tBu), 1.91 (3H, s, 5-CH₃).
MASS (EI) m/z : 392(M⁺), 309, 116.

### (Example 1596)

### Compound of Formula No. C-960

¹H-NMR (CDCl₃) δ(ppm) : 7.35 (1H, s, 3-H), 6.30 (1H, dd, J=2,2Hz, COO-(4-H-1-Pyza)), 4.76 (2H, s, NCH₂Ar), 3.99 (2H, d, J=7Hz, NCH₂-cBu), 1.94 (3H, s, 5-CH₃).
MASS (EI) m/z : 390(M⁺), 307, 116.

### (Example 1597)

### Compound of Formula No. C-961

¹H-NMR (CDCl₃) δ(ppm) : 7.27 (1H, s, 3-H), 4.79 (2H, s, NCH₂Ar), 3.75 (2H, d, J=7Hz, NCH₂-iPr), 2.51 (3H, s, COO-(6-CH₃-2-Pyr)), 1.95 (3H, s, 5-CH₃).
MASS (EI) m/z : 403(M⁺), 279, 149.

### (Example 1598)

### Compound of Formula No. C-962

¹H-NMR (CDCl₃) δ(ppm) : 7.27 (1H, s, 3-H), 4.79 (2H, s, NCH₂Ar), 3.74 (2H, s, NCH₂-tBu), 2.50 (3H, s, COO-(6-CH₃-2-Pyr)), 1.93 (3H, s, 5-CH₃).
MASS (EI) m/z : 417(M⁺), 280, 192, 149.

### (Example 1599)

### Compound of Formula No. C-963

¹H-NMR (CDCl₃) δ(ppm) : 7.27 (1H, s, 3-H), 4.78 (2H, s, NCH₂Ar), 3.97 (2H, d, J=7Hz, NCH₂-cBu), 2.51 (3H, s, COO-(6-CH₃-2-Pyr)), 1.96 (3H, s, 5-CH₃).
MASS (EI) m/z : 415(M⁺), 278, 190, 116.

### (Example 1600)

### Compound of Formula No. C-964

¹H-NMR (CDCl₃) δ(ppm) : 7.24 (1H, s, 3-H), 4.81 (2H, s, NCH₂Ar), 3.73 (2H, d, J=7Hz, NCH₂-iPr), 2.47 (6H, s, COO-(4,6-(CH₃)₂-2-Pym)), 1.99 (3H, s, 5-CH₃).
MASS (EI) m/z : 418(M⁺), 302, 267, 240, 178.

### (Example 1601)

### Compound of Formula No. C-965

¹H-NMR (CDCl₃) δ(ppm) : 7.24 (1H, s, 3-H), 4.81 (2H, s, NCH₂Ar), 3.72 (2H, s, NCH₂-tBu), 2.46 (6H, s, COO-(4,6-(CH₃)₂-2-Pym)), 1.97 (3H, s, 5-CH₃).
MASS (EI) m/z : 432(M⁺), 418, 280, 240, 192.

### (Example 1602)

### Compound of Formula No. C-966

¹H-NMR (CDCl₃) δ(ppm) : 7.21 (1H, s, 3-H), 4.80 (2H, s, NCH₂Ar), 3.94 (2H, d, J=7Hz, NCH₂-cBu), 2.47 (6H, s, COO-(4,6-(CH₃)₂-2-Pym)), 2.00 (3H, s, 5-CH₃).
MASS (EI) m/z : 430(M⁺), 314, 278, 240, 190.

### (Example 1603)

### Compound of Formula No. C-967

¹H-NMR (CDCl₃) δ(ppm) : 8.42 (1H, d, J=2Hz, COO-(6-H-3-Pyr)), 8.25 (1H, d, J=2Hz, COO-(2-H-3-Pyr)), 7.30 (1H, s, 3-H), 4.79 (2H, s, NCH₂Ar), 3.79 (2H, d, J=7Hz, NCH₂-iPr), 1.89 (3H, s, 5-CH₃).
MASS (EI) m/z : 423(M⁺), 295, 239, 116.

### (Example 1604)

### Compound of Formula No. C-968

¹H-NMR (CDCl₃) δ(ppm) : 8.42 (1H, d, J=2Hz, COO-(6-H-3-Pyr)), 8.24 (1H, d, J=2Hz, COO-(2-H-3-Pyr)), 7.31 (1H, s, 3-H), 4.79 (2H, s, NCH₂Ar), 3.78 (2H, s, NCH₂-tBu), 1.88 (3H, s, 5-CH₃).
MASS (EI) m/z : 437(M⁺), 422, 309, 116.

### (Example 1605)

### Compound of Formula No. C-969

¹H-NMR (CDCl₃) δ(ppm) : 8.42 (1H, d, J=2Hz, COO-(6-H-3-Pyr)), 8.25 (1H, d, J=2Hz, COO-(2-H-3-Pyr)), 7.28 (1H, s, 3-H), 4.78 (2H, s, NCH₂Ar), 4.00 (2H, d, J=7Hz, NCH₂-cBu), 1.91 (3H, s, 5-CH₃).
MASS (EI) m/z : 435(M⁺), 307, 239, 116.

### (Example 1606)

### Compound of Formula No. C-970

¹H-NMR (CDCl₃) δ(ppm) : 7.27 (1H, s, 3-H), 4.77 (2H, s, NCH₂Ar), 4.24 (2H, t, J=7Hz, NCH₂CH₂CF₃), 1.88 (3H, s, 5-CH₃), 1.46-1.34 (m, 1H, COCH(CH₂)₂).
MASS (EI) m/z : 376(M⁺), 308, 69.

### (Example 1607)

### Compound of Formula No. C-971

¹H-NMR (CDCl₃) δ(ppm) : 7.18 (1H, s, 3-H), 4.69 (2H, s, NCH₂Ar), 4.20 (2H, t, J=7Hz, NCH₂CH₂CF₃), 3.70 (3H, s, COOCH₃), 1.87 (3H, s, 5-CH₃).
MASS (EI) m/z : 366(M⁺), 307, 250, 218.

### (Example 1608)

### Compound of Formula No. C-972

¹H-NMR (CDCl₃) δ(ppm) : 7.23 (1H, s, 3-H), 4.78 (2H, s, NCH₂Ar), 4.34 (2H, s, COCH₂O), 4.23 (2H, t, J=7Hz, NCH₂CH₂CF₃), 1.87 (3H, s, 5-CH₃).
MASS (EI) m/z : 442(M⁺), 349, 335, 307, 116.

### (Example 1609)

### Compound of Formula No. C-973

¹H-NMR (CDCl₃) δ(ppm) : 7.30 (1H, s, 3-H), 4.80 (2H, s, NCH₂Ar), 3.76 (2H, d, J=8Hz, NCH₂-iPr), 2.19 (3H, s, COO-(3-CH₃-2-Pyr)), 1.89 (3H, s, 5-CH₃).
MASS (EI) m/z : 403(M⁺), 386, 256, 149, 57.

### (Example 1610)

### Compound of Formula No. C-974

¹H-NMR (CDCl₃) δ(ppm) : 7.11 (1H, s, 3-H), 4.91 (2H, s, NCH₂Ar), 3.65 (2H, d, J=8Hz, NCH₂-iPr), 2.15 (3H, s, CO-(3-CH₃-2-oxo-1-Pyr)), 1.95 (3H, s, 5-CH₃).
MASS (EI) m/z : 403(M⁺), 386, 256, 149, 57.

### (Example 1611)

### Compound of Formula No. C-975

¹H-NMR (CDCl₃) δ(ppm) : 7.31 (1H, s, 3-H), 4.80 (2H, s, NCH₂Ar), 3.75 (2H, s, NCH₂-tBu), 2.19 (3H, s, COO-(3-CH₃-2-Pyr)), 1.88 (3H, s, 5-CH₃).
MASS (EI) m/z : 417(M⁺), 280, 192, 149, 116.

### (Example 1612)

### Compound of Formula No. C-976

¹H-NMR (CDCl₃) δ(ppm) : 7.11 (1H, s, 3-H), 4.93 (1H, d, J=14Hz, NCH₂Ar), 4.91 (1H, d, J=14Hz, NCH₂Ar), 3.64 (2H, s, NCH₂-tBu), 2.15 (3H, s, CO-(3-CH₃-2-oxo-1-Pyr)), 1.94 (3H, s, 5-CH₃).
MASS (EI) m/z : 417(M⁺), 280, 192, 149, 116.

### (Example 1613)

### Compound of Formula No. C-977

¹H-NMR (CDCl₃) δ(ppm) : 7.27 (1H, s, 3-H), 4.79 (2H, s, NCH₂Ar), 3.98 (2H, d, J=7Hz, NCH₂-cBu), 2.21 (3H, s, COO-(3-CH₃-2-Pyr)), 1.89 (3H, s, 5-CH₃).
MASS (EI) m/z : 415(M⁺), 307, 278, 190, 116.

### (Example 1614)

### Compound of Formula No. C-978

¹H-NMR (CDCl₃) δ(ppm) : 7.10 (1H, s, 3-H), 4.93 (1H, d, J=14Hz, NCH₂Ar), 4.90 (1H, d, J=14Hz, NCH₂Ar), 3.86 (2H, d, J=7Hz, NCH₂-cBu), 2.15 (3H, s, CO-(3-CH₃-2-oxo-1-Pyr)), 1.96 (3H, s, 5-CH₃).
MASS (EI) m/z : 415(M⁺), 307, 278, 190, 116.

### (Example 1615)

### Compound of Formula No. C-979

¹H-NMR (CDCl₃) δ(ppm) : 7.28 (1H, s, 3-H), 4.78 (2H, s, NCH₂Ar), 4.59 (2H, s, COCH₂O), 3.80 (2H, d, J=7Hz, NCH₂-iPr), 1.98 (3H, s, 5-CH₃).
MASS (EI) m/z : 421(M⁺), 266, 210, 154.

### (Example 1616)

### Compound of Formula No. C-980

¹H-NMR (CDCl₃) δ(ppm) : 7.28 (1H, s, 3-H), 4.79 (2H, s, NCH₂Ar), 4.59 (2H, s, COCH₂O), 3.79 (2H, s, NCH₂-tBu), 1.97 (3H, s, 5-CH₃).
MASS (EI) m/z : 435(M⁺), 420, 280, 154.

### (Example 1617)

### Compound of Formula No. C-981

¹H-NMR (CDCl₃) δ(ppm) : 7.23 (1H, s, 3-H), 5.83 (1H, t, J=54Hz, COCF₂H), 4.79 (2H, brs, NCH₂Ar), 3.79 (2H, d, J=8Hz, NCH₂-iPr), 1.77 (3H, s, 5-CH₃).
MASS (EI) m/z : 346(M⁺), 331, 303, 290, 202.

### (Example 1618)

### Compound of Formula No. C-982

¹H-NMR (CDCl₃) δ(ppm) : 7.22 (1H, s, 3-H), 5.82 (1H, t, J=54Hz, COCF₂H), 4.80 (2H, brs, NCH₂Ar), 3.77 (2H, s, NCH₂-tBu), 1.76 (3H, s, 5-CH₃).
MASS (EI) m/z : 360(M⁺), 345, 304, 281, 116.

### (Example 1619)

### Compound of Formula No. C-983

¹H-NMR (CDCl₃) δ(ppm) : 7.20 (1H, s, 3-H), 5.80 (1H, t, J=53Hz, COCF₂H), 4.78 (2H, brs, NCH₂Ar), 3.99 (2H, d, J=7Hz, NCH₂-cBu), 1.78 (3H, s, 5-CH₃).
MASS (EI) m/z : 358(M⁺), 343, 330, 303, 116.

### (Example 1620)

### Compound of Formula No. C-984

¹H-NMR (CDCl₃) δ(ppm) : 7.63-7.03 (9H, m, C₆H₄-3-CN and COOC₆H₅), 7.31 (1H, s, 3-H), 4.77 (2H, s, NCH₂Ar), 4.22 (2H, t, J=7Hz, NCH₂CH₂CF₃), 1.97 (3H, s, 5-CH₃).
MASS (EI) m/z : 428(M⁺), 335, 116.

### (Example 1621)

### Compound of Formula No. C-985

¹H-NMR (CDCl₃) δ(ppm) : 7.19 (1H, s, 3-H), 4.75 (2H, s, NCH₂Ar), 4.23 (2H, t, J=7Hz, NCH₂CH₂CF₃), 1.86 (3H, s, COCH₃), 1.85 (3H, s, 5-CH₃).
MASS (EI) m/z : 350(M⁺), 308, 234, 192.

### (Example 1622)

### Compound of Formula No. C-986

¹H-NMR (CDCl₃) δ(ppm) : 7.18 (1H, s, 3-H), 4.75 (2H, s, NCH₂Ar), 4.23 (2H, t, J=7Hz, NCH₂CH₂CF₃), 3.71 (2H, s, COCH₂O), 1.82 (3H, s, 5-CH₃).
MASS (EI) m/z : 380(M⁺), 365, 350, 307, 116.

### (Example 1623)

### Compound of Formula No. C-987

¹H-NMR (CDCl₃) δ(ppm) : 8.02 (1H, s, CO-(3-H-1-Pyza)), 7.20 (1H, s, 3-H), 4.92 (2H, s, NCH₂Ar), 3.72 (2H, d, J=8Hz, NCH₂-iPr), 1.67 (3H, s, 5-CH₃).
MASS (EI) m/z : 440(M⁺), 266, 210, 178.

### (Example 1624)

### Compound of Formula No. C-988

¹H-NMR (CDCl₃) δ(ppm) : 7.30 (1H, s, 3-H), 4.78 (2H, s, NCH₂Ar), 3.86 (6H, s, OCH₃ and OCH₃), 3.78 (2H, d, J=7Hz, NCH₂-iPr), 1.91 (3H, s, 5-CH₃).
MASS (EI) m/z : 448(M⁺), 295, 153.

### (Example 1625)

### Compound of Formula No. C-989

¹H-NMR (CDCl₃) δ(ppm) : 7.32 (1H, s, 3-H), 4.79 (2H, s, NCH₂Ar), 3.86 (6H, s, OCH₃ and OCH₃), 3.77 (2H, s, NCH₂-tBu), 1.90 (3H, s, 5-CH₃).
MASS (EI) m/z : 462(M⁺), 447, 309, 153.

### (Example 1626)

### Compound of Formula No. C-990

¹H-NMR (CDCl₃) δ(ppm) : 7.28 (1H, s, 3-H), 4.77 (2H, s, NCH₂Ar), 3.99 (2H, d, J=7Hz, NCH₂-cBu), 3.85 (6H, s, OCH₃ and OCH₃), 1.93 (3H, s, 5-CH₃).
MASS (EI) m/z : 460(M⁺), 307, 153.

### (Example 1627)

### Compound of Formula No. C-991

¹H-NMR (CDCl₃) δ(ppm) : 7.63-7.18 (8H, m, C₆H₄-3-CN and COOC₆H₄-2-OCF₃), 7.27 (1H, s, 3-H), 4.78 (2H, s, NCH₂Ar), 3.77 (2H, d, J=7Hz, NCH₂-iPr), 1.91 (3H, s, 5-CH₃).
MASS (EI) m/z : 472(M⁺), 295, 116.

### (Example 1628)

### Compound of Formula No. C-992

¹H-NMR (CDCl₃) δ(ppm) : 7.63-7.20 (8H, m, C₆H₄-3-CN and COOC₆H₄-2-OCF₃), 7.27 (1H, s, 3-H), 4.77 (2H, s, NCH₂Ar), 3.98 (2H, d, J=7Hz, NCH₂-cBu), 1.92 (3H, s, 5-CH₃).
MASS (EI) m/z : 484(M⁺), 307, 116.

### (Example 1629)

### Compound of Formula No. C-993

¹H-NMR (CDCl₃) δ(ppm) : 8.00 (1H, s, CO-(3-H-1-Pyza)), 7.20 (1H, s, 3-H), 4.92 (2H, s, NCH₂Ar), 3.71 (2H, s, NCH₂-tBu), 1.67 (3H, s, 5-CH₃).
MASS (EI) m/Z : 454(M⁺), 280, 192, 116.

### (Example 1630)

### Compound of Formula No. C-994

¹H-NMR (CDCl₃) δ(ppm) : 7.98 (1H, s, CO-(3-H-1-Pyza)), 7.19 (1H, s, 3-H), 4.92 (2H, s, NCH₂Ar), 3.93 (2H, d, J=7Hz, NCH₂-cBu), 1.67 (3H, s, 5-CH₃).
MASS (EI) m/z : 452(M⁺), 278, 190, 116.

### (Example 1631)

### Compound of Formula No. C-995

¹H-NMR (CDCl₃) δ(ppm) : 8.09 (1H, d, J=2Hz, CO-(5-H-1-Pyza)), 7.12 (1H, s, 3-H), 6.50 (1H, d, J=2Hz, CO-(4-H-1-Pyza)), 4.91 (2H, s, NCH₂Ar), 3.72 (2H, d, J=7Hz, NCH₂-iPr), 1.78 (3H, s, 5-CH₃).
MASS (EI) m/z : 430(M⁺), 411.

### (Example 1632)

### Compound of Formula No. C-996

¹H-NMR (CDCl₃) δ(ppm) : 8.08 (1H, d, J=2Hz, CO-(5-H-1-Pyza)), 7.17 (1H, s, 3-H), 6.50 (1H, d, J=2Hz, CO-(4-H-1-Pyza)), 4.92 (2H, s, NCH₂Ar), 3.70 (2H, s, NCH₂-tBu), 1.74 (3H, s, 5-CH₃).
MASS (EI) m/z : 444(M⁺), 429, 280, 192, 136.

### (Example 1633)

### Compound of Formula No. C-997

¹H-NMR (CDCl₃) δ(ppm) : 8.07 (1H, d, J=2Hz, CO-(5-H-1-Pyza)), 7.08 (1H, s, 3-H), 6.49 (1H, d, J=2Hz, CO-(4-H-1-Pyza)), 4.91 (2H, s, NCH₂Ar), 3.93 (2H, d, J=7Hz, NCH₂-cBu), 1.79 (3H, s, 5-CH₃).
MASS (EI) m/z : 442(M⁺), 278, 210, 190.

### (Example 1634)

### Compound of Formula No. C-998

¹H-NMR (CDCl₃) δ(ppm) : 10.56 (1H, d, J=11Hz, COO-(2-H-4-Pym)), 8.91 (1H, d, J=10Hz, COO-(5-H-4-Pym)), 7.26 (1H, s, 3-H), 4.95 (1H, d, J=14Hz, NCH₂Ar), 4.89 (1H, d, J=14Hz, NCH₂Ar), 3.83 (2H, d, J=7Hz, NCH₂-iPr), 1.85 (3H, s, 5-CH₃).
MASS (EI) m/z : 408(M⁺), 363, 268.

### (Example 1635)

### Compound of Formula No. C-999

¹H-NMR (CDCl₃) δ(ppm) : 10.59 (1H, d, J=11Hz, COO-(2-H-4-Pym)), 8.91 (1H, d, J=10Hz, COO-(5-H-4-Pym)), 7.26 (1H, s, 3-H), 4.94 (1H, d, J=14Hz, NCH₂Ar), 4.90 (1H, d, J=14Hz, NCH₂Ar), 3.83 (2H, s, NCH₂-tBu), 1.84 (3H, s, 5-CH₃).
MASS (EI) m/z : 422(M⁺), 408, 377, 282.

### (Example 1636)

### Compound of Formula No. C-1000

¹H-NMR (CDCl₃) δ(ppm) : 10.50 (1H, d, J=12Hz, COO-(2-H-4-Pym)), 8.94 (1H, d, J=10Hz, COO-(5-H-4-Pym)), 7.26 (1H, s, 3-H), 4.95 (1H, d, J=14Hz, NCH₂Ar), 4.90 (1H, d, J=14Hz, NCH₂Ar), 4.05 (2H, d, J=8Hz, NCH₂-cBu), 1.86 (3H, s, 5-CH₃).
MASS (EI) m/z : 420(M⁺), 318, 280, 149.

### (Example 1637)

### Compound of Formula No. C-1001

¹H-NMR (CDCl₃) δ(ppm) : 8.01 (1H, d, J=2Hz, CO-(3-H-1-Pyza)), 7.24 (1H, s, 3-H), 6.25 (1H, dd, J=2,2Hz, CO-(4-H-1-Pyza)), 4.96 (2H, s, NCH₂Ar), 3.70 (2H, d, J=8Hz, NCH₂-ipr), 1.64 (3H, s, 5-CH₃).
MASS (EI) m/z : 362(M⁺), 266, 178, 149.

### (Example 1638)

### Compound of Formula No. C-1002

¹H-NMR (CDCl₃) δ(ppm) : 8.01 (1H, d, J=2Hz, CO-(3-H-1-Pyza)), 7.24 (1H, s, 3-H), 6.24 (1H, dd, J=2,2Hz, CO-(4-H-1-Pyza)), 4.97 (2H, s, NCH₂Ar), 3.69 (2H, s, NCH₂-tBu), 1.65 (3H, s, 5-CH₃).
MASS (EI) m/z : 376(M⁺), 362, 280, 149.

### (Example 1639)

### Compound of Formula No. C-1003

¹H-NMR (CDCl₃) δ(ppm) : 7.98 (1H, d, J=2Hz, CO-(3-H-1-Pyza)), 7.22 (1H, s, 3-H), 6.25 (1H, dd, J=2,2Hz, CO-(4-H-1-Pyza)), 4.96 (2H, s, NCH₂Ar), 3.91 (2H, d, J=7Hz, NCH₂-cBu), 1.65 (3H, s, 5-CH₃).
MASS (EI) m/z : 374(M⁺), 278, 149.

### (Example 1640)

### Compound of Formula No. C-1004

¹H-NMR (CDCl₃) δ(ppm) : 7.23 (1H, s, 3-H), 4.95 (2H, s, NCH₂Ar), 3.71 (2H, d, J=7Hz, NCH₂-iPr), 2.00 (3H, s, CO-(4-CH₃-1-Pyza)), 1.65 (3H, s, 5-CH₃).
MASS (EI) m/z : 376(M⁺), 266, 178, 149.

### (Example 1641)

### Compound of Formula No. C-1005

¹H-NMR (CDCl₃) δ(ppm) : 7.22 (1H, s, 3-H), 4.95 (2H, s, NCH₂Ar), 3.70 (2H, s, NCH₂-tBu), 2.00 (3H, s, CO-(4-CH₃-1-Pyza)), 1.65 (3H, s, 5-CH₃).
MASS (EI) m/z : 390(M⁺), 280, 58.

### (Example 1642)

### Compound of Formula No. C-1006

¹H-NMR (CDCl₃) δ(ppm) : 7.21 (1H, s, 3-H), 4.95 (2H, s, NCH₂Ar), 3.92 (2H, d, J=7Hz, NCH₂-cBu), 2.01 (3H, s, CO-(4-CH₃-1-Pyza)), 1.66 (3H, s, 5-CH₃).
MASS (EI) m/z : 388(M⁺), 278, 116.

### (Example 1643)

### Compound of Formula No. C-1007

¹H-NMR (CDCl₃) δ(ppm) : 8.99 (1H, s, COO-(2-H-4-Pym)), 8.71 (1H, d, J=5Hz, COO-(5-H-4-Pym)), 7.27 (1H, s, 3-H), 4.82 (2H, s, NCH₂Ar), 3.75 (2H, d, J=8Hz, NCH₂-iPr), 1.90 (3H, s, 5-CH₃).
MASS (EI) m/z : 390(M⁺), 294, 266, 178.

### (Example 1644)

### Compound of Formula No. C-1008

¹H-NMR (CDCl₃) δ(ppm) : 8.03 (1H, s, CO-(2-H-4-oxo-3-Pym)), 7.27 (1H, s, 3-H), 6.19 (1H, d, J=7Hz, CO-(5-H-4-oxo-3-Pym)), 4.92 (2H, brs, NCH₂Ar), 3.68 (2H, d, J=7Hz, NCH₂-iPr), 2.10 (3H, s, 5-CH₃).
MASS (EI) m/z : 390(M⁺), 294, 266, 178.

### (Example 1645)

### Compound of Formula No. C-1009

¹H-NMR (CDCl₃) δ(ppm) : 8.98 (1H, s, COO-(2-H-4-Rym)), 8.70 (1H, d, J=5Hz, COO-(5-H-4-Pym)), 7.27 (1H, s, 3-H), 4.82 (2H, s, NCH₂Ar), 3.74 (2H, s, NCH₂-tBu), 1.89 (3H, s, 5-CH₃).
MASS (EI) m/z : 404(M⁺), 389, 308, 280, 192.

### (Example 1646)

### Compound of Formula No. C-1010

¹H-NMR (CDCl₃) δ(ppm) : 8.03 (1H, s, CO-(2-H-4-oxo-3-Pym)), 7.28 (1H, s, 3-H), 6.19 (1H, d, J=7Hz, CO-(5-H-4-oxo-3-Pym)), 4.92 (2H, brs, NCH₂Ar), 3.75 (2H, s, NCH₂-tBu), 2.10 (3H, s, 5-CH₃).
MASS (EI) m/z : 404(M⁺), 389, 308, 280, 192.

### (Example 1647)

### Compound of Formula No. C-1011

¹H-NMR (CDCl₃) δ(ppm) : 8.98 (1H, s, COO-(2-H-4-Pym)), 8.71 (1H, d, J=5Hz, COO-(5-H-4-Pym)), 7.27 (1H, s, 3-H), 4.80 (2H, s, NCH₂Ar), 3.97 (2H, d, J=7Hz, NCH₂-cBu), 1.92 (3H, s, 5-CH₃).
MASS (EI) m/z : 402(M⁺), 306, 278, 190.

### (Example 1648)

### Compound of Formula No. C-1012

¹H-NMR (CDCl₃) δ(ppm) : 8.03 (1H, s, CO-(2-H-4-oxo-3-Pym)), 7.24 (1H, s, 3-H), 6.19 (1H, d, J=7Hz, CO-(5-H-4-oxo-3-Pym)), 4.91 (2H, brs, NCH₂Ar), 3.89 (2H, d, J=7Hz, NCH₂-cBu), 2.11 (3H, s, 5-CH₃).
MASS (EI) m/z : 402(M⁺), 306, 278, 190.

### (Example 1649)

### Compound of Formula No. C-1013

¹H-NMR (CDCl₃) δ(ppm) : 8.75 (1H, s, CO-(3-H-1-Pyza)), 7.87 (1H, s, CO-(5-H-1-Pyza)), 7.16 (1H, s, 3-H), 4.93 (2H, s, NCH₂Ar), 3.74 (2H, d, J=7Hz, NCH₂-iPr), 1.75 (3H, s, 5-CH₃).
MASS (EI) m/z : 407(M⁺), 392, 266, 210, 178.

### (Example 1650)

### Compound of Formula No. C-1014

¹H-NMR (CDCl₃) δ(ppm) : 8.74 (1H, s, CO-(3-H-1-Pyza)), 7.88 (1H, s, CO-(5-H-1-Pyza)), 7.14 (1H, s, 3-H), 4.92 (2H, s, NCH₂Ar), 3.95 (2H, d, J=7Hz, NCH₂-cBu), 1.75 (3H, s, 5-CH₃).
MASS (EI) m/z : 419(M⁺), 303, 278, 190, 116.

### (Example 1651)

### Compound of Formula No. C-1015

¹H-NMR (CDCl₃) δ(ppm) : 7.19 (1H, s, 3-H), 4.94 (2H, s, NCH₂Ar), 3.82 (3H, s, CO₂CH₃), 3.71 (2H, d, J=7Hz, NCH₂-iPr), 1.68 (3H, s, 5-CH₃).
MASS (EI) m/z : 420(M⁺), 389, 266, 178.

### (Example 1652)

### Compound of Formula No. C-1016

¹H-NMR (CDCl₃) δ(ppm) : 7.17 (1H, s, 3-H), 4.93 (2H, s, NCH₂Ar), 3.92 (2H, d, J=7Hz, NCH₂-cBu), 3.82 (3H, s, CO₂CH₃), 1.68 (3H, s, 5-CH₃).
MASS (EI) m/z : 432(M⁺), 420, 401, 278.

### (Example 1653)

### Compound of Formula No. C-1017

¹H-NMR (CDCl₃) δ(ppm) : 7.65-7.27 (8H, m, C₆H₄-3-CN and COOC₆H₄-2-CN), 7.26 (1H, s, 3-H), 4.80 (2H, s, NCH₂Ar), 3.78 (2H, d, J=8Hz, NCH₂-iPr), 1.96 (3H, s, 5-CH₃).
MASS (EI) m/z : 413(M⁺), 398, 357, 295, 116.

### (Example 1654)

### Compound of Formula No. C-1018

¹H-NMR (CDCl₃) δ (ppm) : 7.64-7.27 (8H, m, C₆H₄-3-CN and COOC₆H₄-2-CN), 7.29 (1H, s, 3-H), 4.79 (2H, s, NCH₂Ar), 3.99 (2H, d, J=7Hz, NCH₂-cBu), 1.98 (3H, s, 5-CH₃).
MASS (EI) m/z : 425(M⁺), 307, 239, 116.

### (Example 1655)

### Compound of Formula No. C-1019

¹H-NMR (CDCl₃) δ(ppm) : 7.13 (1H, s, 3-H), 4.76 (2H, brs, NCH₂Ar), 4.53 (2H, s, COCH₂O), 3.73 (2H, d, J=8Hz, NCH₂-iPr), 1.65 (3H, s, 5-CH₃).
MASS (EI) m/z : 476(M⁺), 310, 151.

### (Example 1656)

### Compound of Formula No. C-1020

¹H-NMR (CDCl₃) δ(ppm) : 7.22 (1H, s, 3-H), 4.79 (2H, brs, NCH₂Ar), 4.31 (2H, s, COCH₂O), 3.79 (2H, d, J=7Hz, NCH₂-iPr), 1.80 (3H, s, 5-CH₃).
MASS (EI) m/z : 462(M⁺), 309, 116.

### (Example 1657)

### Compound of Formula No. C-1021

¹H-NMR (CDCl₃) δ(ppm) : 7.19 (1H, s, 3-H), 4.78 (2H, brs, NCH₂Ar), 4.30 (2H, s, COCH₂O), 4.00 (2H, d, J=7Hz, NCH₂-cBu), 1.81 (3H, s, 5-CH₃).
MASS (EI) m/z : 474(M⁺), 321, 116.

### (Example 1658)

### Compound of Formula No. C-1022

¹H-NMR (CDCl₃) δ(ppm) : 7.29 (1H, s, 3-H), 4.79 (2H, s, NCH₂Ar), 3.88 (3H, s, OCH₃), 3.75 (2H, d, J=8Hz, NCH₂-iPr), 1.99 (3H, s, 5-CH₃).
MASS (EI) m/z : 419(M⁺), 295, 265, 149.

### (Example 1659)

### Compound of Formula No. C-1023

¹H-NMR (CDCl₃) δ(ppm) : 7.30 (1H, s, 3-H), 4.79 (2H, s, NCH₂Ar), 3.87 (3H, s, OCH₃), 3.74 (2H, s, NCH₂-tBu), 1.97 (3H, s, 5-CH₃).
MASS (EI) m/z : 433(M⁺), 247, 149.

### (Example 1660)

### Compound of Formula No. C-1024

¹H-NMR (CDCl₃) δ(ppm) : 7.27 (1H, s, 3-H), 4.78 (2H, s, NCH₂Ar), 3.96 (2H, d, J=7Hz, NCH₂-cBu), 3.88 (3H, s, OCH₃), 2.01 (3H, s, 5-CH₃).
MASS (EI) m/z : 431(M⁺), 387, 278, 149.

### (Example 1661)

### Compound of Formula No. C-1025

¹H-NMR (CDCl₃) δ(ppm) : 7.27 (1H, s, 3-H), 4.97 (1H, d, J=15Hz, NCH₂Ar), 4.87 (1H, d, J=15Hz, NCH₂Ar), 3.68 (3H, s, OCH₃), 3.66 (2H, d, J=7Hz, NCH₂-iPr), 1.96 (3H, s, 5-CH₃).
MASS (EI) m/z : 419(M⁺), 295, 178, 116.

### (Example 1662)

### Compound of Formula No. C-1026

¹H-NMR (CDCl₃) δ(ppm) : 7.27 (1H, s, 3-H), 4.96 (1H, d, J=14Hz, NCH₂Ar), 4.88 (1H, d, J=14Hz, NCH₂Ar), 3.67 (3H, s, OCH₃), 3.65 (2H, s, NCH₂-tBu), 1.95 (3H, s, 5-CH₃).
MASS (EI) m/z : 433(M⁺), 309, 280, 116.

### (Example 1663)

### Compound of Formula No. C-1027

¹H-NMR (CDCl₃) δ(ppm) : 7.27 (1H, s, 3-H), 4.95 (1H, d, J=14Hz, NCH₂Ar), 4.87 (1H, d, J=14Hz, NCH₂Ar), 3.87 (2H, d, J=7Hz, NCH₂-cBu) , 3.68 (3H, s, OCH₃), 1.97 (3H, s, 5-CH₃).
MASS (EI) m/z : 431(M⁺), 307, 190, 116.

### (Example 1664)

### Compound of Formula No. C-1028

¹H-NMR (CDCl₃) δ(ppm) : 7.29 (1H, s, 3-H), 7.21 (1H, d, J=8Hz, COO-(5-H-2-Pyr)), 6.96 (1H, d, J=8Hz, COO-(3-H-2-Pyr)), 4.79 (2H, s, NCH₂Ar), 3.76 (2H, d, J=8Hz, NCH₂-iPr), 1.94 (3H, s, 5-CH₃).
MASS (EI) m/z : 423(M⁺), 295, 178, 116.

### (Example 1665)

### Compound of Formula No. C-1029

¹H-NMR (CDCl₃) δ(ppm) : 7.30 (1H, s, 3-H), 7.22 (1H, d, J=8Hz, COO-(5-H-2-Pyr)), 6.96 (1H, d, J=8Hz, COO-(3-H-2-Pyr)), 4.79 (2H, s, NCH₂Ar), 3.75 (2H, s, NCH₂-tBu), 1.93 (3H, s, 5-CH₃).
MASS (EI) m/z : 437(M⁺), 309, 192, 116.

### (Example 1666)

### Compound of Formula No. C-1030

¹H-NMR (CDCl₃) δ(ppm) : 7.26 (1H, s, 3-H), 7.22 (1H, d, J=8Hz, COO-(5-H-2-Pyr)), 6.96 (1H, d, J=8Hz, COO-(3-H-2-Pyr)), 4.78 (2H, s, NCH₂Ar), 3.97 (2H, d, J=7Hz, NCH₂-cBu), 1.95 (3H, s, 5-CH₃).
MASS (EI) m/z : 435(M⁺), 307, 190, 116.

### (Example 1667)

### Compound of Formula No. C-1031

¹H-NMR (CDCl₃) δ(ppm) : 7.30 (1H, s, 3-H), 4.78 (2H, s, NCH₂Ar), 3.78 (2H, d, J=7Hz, NCH₂-iPr), 2.54 (3H, s, COO-(6-CH₃-3-Pyr)), 1.89 (3H, s, 5-CH₃).
MASS (El) m/z : 403(M⁺), 295, 116.

### (Example 1668)

### Compound of Formula No. C-1032

¹H-NMR (CDCl₃) δ(ppm) : 7.31 (1H, s, 3-H), 4.79 (2H, s, NCH₂Ar), 3.77 (2H, s, NCH₂-tBu), 2.54 (3H, s, COO-(6-CH₃-3-Pyr)), 1.89 (3H, s, 5-CH₃).
MASS (EI) m/z : 417(M⁺), 309, 116.

### (Example 1669)

### Compound of Formula No. C-1033

¹H-NMR (CDCl₃) δ(ppm) : 7.27 (1H, s, 3-H), 4.78 (2H, s, NCH₂Ar), 4.00 (2H, d, J=7Hz, NCH₂-cBu), 2.54 (3H, s, COO-(6-CH₃-3-Pyr)), 1.91 (3H, s, 5-CH₃).
MASS (EI) m/z : 415(M⁺), 307, 239, 116.

### (Example 1670)

### Compound of Formula No. C-1034

¹H-NMR (CDCl₃) δ(ppm) : 7.33 (1H, s, 3-H), 6.95 (1H, d, J=8Hz, COO-(3-H-2-Pyr)), 4.82 (2H, s, NCH₂Ar), 3.77 (2H, d, J=8Hz, NCH₂-iPr), 1.99 (3H, s, 5-CH₃).
MASS (EI) m/z : 465(M⁺), 421, 178, 116.

### (Example 1671)

### Compound of Formula No. C-1035

¹H-NMR (CDCl₃) δ(ppm) : 7.34 (1H, s, 3-H), 6.96 (1H, d, J=8Hz, COO-(3-H-2-Pyr)), 4.839 (2H, s, NCH₂Ar), 3.76 (2H, s, NCH₂-tBu), 1.98 (3H, s, 5-CH₃).
MASS (EI) m/z : 479(M⁺), 280, 57.

### (Example 1672)

### Compound of Formula No. C-1036

¹H-NMR (CDCl₃) δ(ppm) : 7.30 (1H, s, 3-H), 6.95 (1H, d, J=8Hz, COO-(3-H-2-Pyr)), 4.81 (2H, s, NCH₂Ar), 3.98 (2H, d, J=7Hz, NCH₂-cBu), 2.01 (3H, s, 5-CH₃).
MASS (EI) m/z : 477(M⁺), 278, 57.

### (Example 1673)

### Compound of Formula No. C-1037

¹H-NMR (CDCl₃) δ(ppm) : 7.32 (1H, s, 3-H), 4.80 (2H, s, NCH₂Ar), 3.81 (2H, d, J=8Hz, NCH₂-iPr), 1.86 (3H, s, 5-CH₃).
MASS (EI) m/z : 321(M⁺), 306, 265, 205, 123.

### (Example 1674)

### Compound of Formula No. C-1038

7.26 (1H, s, 3-H), 4.81 (2H, s, NCH₂Ar), 3.76 (2H, d, J=8Hz, NCH₂-iPr), 2.60 (3H, s, COO-(3-CN-6-CH₃-2-Pyr)), 1.98 (3H, s, 5-CH₃).
MASS (EI) m/z : 428(M⁺), 295, 178, 116.

### (Example 1675)

### Compound of Formula No. C-1039

¹H-NMR (CDCl₃) δ(ppm) : 7.33 (1H, s, 3-H), 4.82 (2H, s, NCH₂Ar), 3.75 (2H, s, NCH₂-tBu), 2.60 (3H, s, COO-(3-CN-6-CH₃-2-Pyr)), 1.97 (3H, s, 5-CH₃).
MASS (EI) m/z : 442(M⁺), 282, 166.

### (Example 1676)

### Compound of Formula No. C-1040

¹H-NMR (CDCl₃) δ(ppm) : 7.28 (1H, s, 3-H), 4.80 (2H, s, NCH₂Ar), 3.98 (2H, d, J=7Hz, NCH₂-cBu), 2.60 (3H, s, COO-(3-CN-6-CH₃-2-Pyr)), 2.00 (3H, s, 5-CH₃).
MASS (EI) m/z : 440(M⁺), 307, 190, 116.

### (Example 1677)

### Compound of Formula No. C-1041

¹H-NMR (CDCl₃) δ(ppm) : 7.31 (1H, s, 3-H), 4.80 (2H, s, NCH₂Ar), 3.76 (2H, d, J=7Hz, NCH₂-iPr), 2.54 (3H, s, COO-(3-CN-4-CH₃-6-CH₃-2-Pyr)), 2.50 (3H, s, COO-(3-CN-4-CH₃-6-CH₃-2-Pyr)), 1.99 (3H, s, 5-CH₃).
MASS (EI) m/z : 442(M⁺), 295, 178.

### (Example 1678)

### Compound of Formula No. C-1042

¹H-NMR (CDCl₃) δ(ppm) : 7.33 (1H, s, 3-H), 4.81 (2H, s, NCH₂Ar), 3.75 (2H, s, NCH₂-tBu), 2.54 (3H, s, COO-(3-CN-4-CH₃-6-CH₃-2-Pyr)), 2.50 (3H, s, COO-(3-CN-4-CH₃-6-CH₃-2-Pyr)), 1.97 (3H, s, 5-CH₃).
MASS (EI) m/z : 456(M⁺), 308, 192.

### (Example 1679)

### Compound of Formula No. C-1043

¹H-NMR (CDCl₃) δ(ppm) : 7.28 (1H, s, 3-H), 4.80 (2H, s, NCH₂Ar), 3.97 (2H, d, J=7Hz, NCH₂-cBu), 2.54 (3H, s, COO-(3-CN-4-CH₃-6-CH₃-2-Pyr)), 2.50 (3H, s, COO-(3-CN-4-CH₃-6-CH₃-2-Pyr)), 2.00 (3H, s, 5-CH₃).
MASS (EI) m/z : 454(M⁺), 306, 280, 190, 116.

### (Example 1680)

### Compound of Formula No. C-1044

¹H-NMR (CDCl₃) δ(ppm) : 7.29 (1H, s, 3-H), 4.79 (2H, s, NCH₂Ar), 3.79 (2H, d, J=8Hz, NCH₂-iPr), 2.32 (3H, s, COO-(2-CH₃-3-Pyr)), 1.91 (3H, s, 5-CH₃).
MASS (EI) m/z : 403(M⁺), 295, 239, 116.

### (Example 1681)

### Compound of Formula No. C-1045

¹H-NMR (CDCl₃) δ(ppm) : 7.30 (1H, s, 3-H), 4.79 (2H, s, NCH₂Ar), 3.77 (2H, s, NCH₂-tBu), 2.32 (3H, s, COO-(2-CH₃-3-Pyr)), 1.91 (3H, s, 5-CH₃).
MASS (EI) m/z : 417(M⁺), 309, 239, 116.

### (Example 1682)

### Compound of Formula No. C-1046

¹H-NMR (CDCl₃) δ(ppm) : 7.28 (1H, s, 3-H), 4.78 (2H, s, NCH₂Ar), 4.00 (2H, d, J=7Hz, NCH₂-cBu), 2.31 (3H, s, COO-(2-CH₃-3-Pyr)), 1.93 (3H, s, 5-CH₃).
MASS (EI) m/z : 415(M⁺), 307, 239, 116.

### (Example 1683)

### Compound of Formula No. C-1047

¹H-NMR (CDCl₃) δ(ppm) : 7.32 (1H, s, 3-H), 4.80 (2H, s, NCH₂Ar), 3.96 (3H, s, COOCH₃), 3.79 (2H, d, J=8Hz, NCH₂-iPr), 1.91 (3H, s, 5-CH₃).
MASS (EI) m/z : 447(M⁺), 295, 239, 116.

### (Example 1684)

### Compound of Formula No. C-1048

¹H-NMR (CDCl₃) δ(ppm) : 7.32 (1H, s, 3-H), 4.80 (2H, s, NCH₂Ar), 3.96 (3H, s, COOCH₃), 3.78 (2H, s, NCH₂-tBu), 1.89 (3H, s, 5-CH₃).
MASS (EI) m/z : 461(M⁺), 309, 239, 116.

### (Example 1685)

### Compound of Formula No. C-1049

¹H-NMR (CDCl₃) δ(ppm) : 7.29 (1H, s, 3-H), 4.79 (2H, s, NCH₂Ar), 4.01 (2H, d, J=7Hz, NCH₂-cBu), 3.96 (3H, s, COOCH₃), 1.92 (3H, s, 5-CH₃).
MASS (EI) m/z : 459(M⁺), 307, 239, 116.

### (Example 1686)

### Compound of Formula No. C-1050

¹H-NMR (CDCl₃) δ(ppm) : 7.41 (1H, s, 3-H), 6.94 (1H, brs, COO-(5-H-1-Imid)), 6.86 (1H, brs, COO-(4-H-1-Imid)), 4.90 (2H, brs, NCH₂Ar), 3.70 (2H, d, J=8Hz, NCH₂-iPr), 1.47 (3H, s, 5-CH₃).
MASS (EI) m/z : 362(M⁺), 295, 149, 116.

### (Example 1687)

### Compound of Formula No. C-1051

¹H-NMR (CDCl₃) δ(ppm) : 7.41 (1H, s, 3-H), 6.95 (1H, brs, COO-(5-H-1-Imid)), 6.87 (1H, brs, COO-(4-H-1-Imid)), 4.94 (2H, brs, NCH₂Ar), 3.69 (2H, s, NCH₂-tBu), 1.47 (3H, s, 5-CH₃).
MASS (EI) m/z : 376(M⁺), 309, 116.

### (Example 1688)

### Compound of Formula No. C-1052

¹H-NMR (CDCl₃) δ(ppm) : 7.37 (1H, s, 3-H), 6.91 (1H, brs, COO-(5-H-1-Imid)), 6.86 (1H, brs, COO-(4-H-1-Imid)), 4.90 (2H, brs, NCH₂Ar), 3.92 (2H, d, J=7Hz, NCH₂-cBu), 1.48 (3H, s, 5-CH₃).
MASS (EI) m/z : 374(M⁺), 307, 116.

### (Example 1689)

### Compound of Formula No. C-1053

¹H-NMR (CDCl₃) δ(ppm) : 7.30 (1H, s, 3-H), 4.79 (2H, s, NCH₂Ar), 3.76 (2H, d, J=8Hz, NCH₂-iPr), 2.32 (3H, s, COO-(5-CH₃-2-Pyr)), 1.94 (3H, s, 5-CH₃).
MASS (EI) m/z : 403(M⁺), 359, 178, 116.

### (Example 1690)

### Compound of Formula No. C-1054

¹H-NMR (CDCl₃) δ(ppm) : 7.31 (1H, s, 3-H), 4.79 (2H, s, NCH₂Ar), 3.74 (2H, s, NCH₂-tBu), 2.31 (3H, s, COO-(5-CH₃-2-Pyr)), 1.93 (3H, s, 5-CH₃).
MASS (EI) m/z : 417(M⁺), 280, 192, 116.

### (Example 1691)

### Compound of Formula No. C-1055

¹H-NMR (CDCl₃) δ(ppm) : 7.28 (1H, s, 3-H), 4.78 (2H, s, NCH₂Ar), 3.97 (2H, d, J=7Hz, NCH₂-cBu), 2.31 (3H, s, COO-(5-CH₃-2-Pyr)), 1.96 (3H, s, 5-CH₃).
MASS (EI) m/z : 415(M⁺), 371, 278, 190, 116.

### (Example 1692)

### Compound of Formula No. C-1056

¹H-NMR (CDCl₃) δ(ppm) : 7.27 (1H, s, 3-H), 5.04 (1H, d, J=14Hz, NCH₂Ar), 4.77 (1H, d, J=14Hz, NCH₂Ar), 3.67 (2H, d, J=7Hz, NCH₂-iPr), 1.97 (3H, s, CO-(5-CH₃-2-oxo-1-Pyr)), 1.92 (3H, s, 5-CH₃).
MASS (EI) m/z : 403(M⁺), 295, 266, 178, 116.

### (Example 1693)

### Compound of Formula No. C-1057

¹H-NMR (CDCl₃) δ(ppm) : 7.26 (1H, s, 3-H), 5.03 (1H, d, J=13Hz, NCH₂Ar), 4.79 (1H, d, J=13Hz, NCH₂Ar), 3.66 (2H, s, NCH₂-tBu), 1.96 (3H, s, CO-(5-CH₃-2-oxo-1-Pyr)), 1.91 (3H, s, 5-CH₃).
MASS (EI) m/z : 417(M⁺), 309, 280, 192, 116.

### (Example 1694)

### Compound of Formula No. C-1058

¹H-NMR (CDCl₃) δ(ppm) : 7.27 (1H, s, 3-H), 5.04 (1H, d, J=13Hz, NCH₂Ar), 4.77 (1H, d, J=13Hz, NCH₂Ar), 3.88 (2H, d, J=7Hz, NCH₂-cBu), 1.98 (3H, s, CO-(5-CH₃-2-oxo-1-Pyr)), 1.93 (3H, s, 5-CH₃).
MASS (EI) m/z : 415(M⁺), 307, 278, 190, 116.

### (Example 1695)

### Compound of Formula No. C-1059

¹H-NMR (CDCl₃) δ(ppm) : 8.26 (1H, dd, J=5,1Hz, COO-(6-H-3-Pyr)), 7.30 (1H, s, 3-H), 4.79 (2H, s, NCH₂Ar), 3.78 (2H, d, J=8Hz, NCH₂-iPr), 1.95 (3H, s, 5-CH₃).
MASS (EI) m/z : 423(M⁺), 295, 239, 116.

### (Example 1696)

### Compound of Formula No. C-1060

¹H-NMR (CDCl₃) δ(ppm) : 8.26 (1H, dd, J=5,1Hz, COO-(6-H-3-Pyr)), 7.31 (1H, s, 3-H), 4.80 (2H, s, NCH₂Ar), 3.77 (2H, s, NCH₂-tBu), 1.94 (3H, s, 5-CH₃).
MASS (EI) m/z : 437(M⁺), 309, 116.

### (Example 1697)

### Compound of Formula No. C-1061

¹H-NMR (CDCl₃) δ(ppm) : 8.26 (1H, dd, J=5,2Hz, COO-(6-H-3-Pyr)), 7.27 (1H, s, 3-H), 4.78 (2H, s, NCH₂Ar), 3.99 (2H, d, J=7Hz, NCH₂-cBu), 1.96 (3H, s, 5-CH₃).
MASS (EI) m/z : 435(M⁺), 307, 239, 116.

### (Example 1698)

### Compound of Formula No. C-1062

¹H-NMR (CDCl₃) δ(ppm) : 7.68-7.43 (5H, m, C₆H₄-3-CN and CO-(2-H-1-Imid)), 7.33 (1H, s, 3-H), 4.90 (2H, brs, NCH₂Ar), 3.67 (2H, d, J=7Hz, NCH₂-iPr), 1.60 (3H, s, 5-CH₃).
MASS (EI) m/z : 430(M⁺), 395, 295.

### (Example 1699)

### Compound of Formula No. C-1063

¹H-NMR (CDCl₃) δ(ppm) : 7.68-7.43 (5H, m, C₆H₄-3-CN and CO-(2-H-1-Imid)), 7.33 (1H, s, 3-H), 4.91 (2H, brs, NCH₂Ar), 3.65 (2H, s, NCH₂-tBu), 1.60 (3H, s, 5-CH₃).
MASS (EI) m/z : 444(M⁺), 309, 116.

### (Example 1700)

### Compound of Formula No. C-1064

¹H-NMR (CDCl₃) δ(ppm) : 7.68-7.43 (5H, m, C₆H₄-3-CN and CO-(2-H-1-Imid)), 7.30 (1H, s, 3-H), 4.90 (2H, brs, NCH₂Ar), 3.88 (2H, d, J=7Hz, NCH₂-cBu), 1.62 (3H, s, 5-CH₃).
MASS (EI) m/z : 442(M⁺), 407, 307, 116.

### (Example 1701)

### Compound of Formula No. C-1065

¹H-NMR (CDCl₃) δ(ppm) : 7.28 (1H, s, 3-H), 4.73 (2H, s, NCH₂Ar), 3.72 (2H, d, J=7Hz, 2-H₂-indoline), 3.41 (2H, t, J=9Hz, 3-H₂-indoline), 2.89 (2H, t, J=9Hz, NCH₂-iPr), 1.749 (3H, s, 5-CH₃).
MASS (EI) m/z : 431(M⁺), 295, 136, 116.

### (Example 1702)

### Compound of Formula No. C-1066

¹H-NMR (CDCl₃) δ(ppm) : 7.26 (1H, s, 3-H), 4.73 (2H, s, NCH₂Ar), 3.70 (2H, s, 2-H₂-indoline), 3.42 (2H, t, J=9Hz, 3-H₂-indoline), 2.88 (2H, t, J=9Hz, NCH₂-iPr), 1.74 (3H, s, 5-CH₃).
MASS (EI) m/z : 445(M⁺), 309, 136, 116.

### (Example 1703)

### Compound of Formula No. C-1067

¹H-NMR (CDCl₃) δ(ppm) : 7.26 (1H, s, 3-H), 4.72 (2H, s, NCH₂Ar), 3.93 (2H, d, J=7Hz, 2-H₂-indoline), 3.37 (2H, t, J=9Hz, 3-H₂-indoline), 2.88 (2H, t, J=9Hz, NCH₂-iPr), 1.75 (3H, s, 5-CH₃).
MASS (EI) m/z : 443(M⁺), 133, 89.

### (Example 1704)

### Compound of Formula No. C-1068

¹H-NMR (CDCl₃) δ(ppm) : 8.42 (1H, dd, J=4,2Hz, COO-(6-H-3-Pyr)), 7.28 (1H, s, 3-H), 4.78 (2H, s, NCH₂Ar), 3.77 (2H, d, J=7Hz, NCH₂-iPr), 1.95 (3H, s, 5-CH₃).
MASS (EI) m/z : 434 (M⁺), 419, 378, 295, 116.

### (Example 1705)

### Compound of Formula No. C-1069

¹H-NMR (CDCl₃) δ(ppm) : 7.29 (1H, s, 3-H), 4.81 (2H, brs, NCH₂Ar), 3.94 (3H, s, COOCH₃), 3.75 (2H, d, J=8Hz, NCH₂-i-Pr), 1.93 (3H, s, 5-CH₃).
MASS (EI) m/z : 447(M⁺), 295, 266, 239, 178, 116.

### (Example 1706)

### Compound of Formula No. C-1070

¹H-NMR (CDCl₃) δ(ppm) : 7.31 (1H, s, 3-H), 4.79 (2H, s, NCH₂Ar), 3.97 (3H, s, COOCH₃), 3.76 (2H, d, J=7Hz, NCH₂-i-Pr), 1.95 (3H, s, 5-CH₃).
MASS (EI) m/z : 447(M⁺), 403, 293, 265, 238, 177.

### (Example 1707)

### Compound of Formula No. C-1071

¹H-NMR (CDCl₃ δ(ppm) : 7.48 (1H, s, CONH₂), 7.33 (1H, s, 3-H), 5.68 (1H, s, CONH₂), 4.81 (2H, brs, NCH₂Ar), 3.75 (2H, d, J=8Hz, NCH₂-i-Pr), 1.94 (3H, s, 5-CH₃).
MASS (EI) m/z : 432(M⁺), 388, 295, 266, 239, 178, 116.

### (Example 1708)

### Compound of Formula No. C-1072

¹H-NMR (CDCl₃) δ(ppm) : 7.89 (1H, t, J=8Hz, O-Pyr-4'H), 7.30 (1H, s, 3-H), 4.80 (2H, s, NCH₂Ar), 3.76 (2H, d, J=8Hz, NCH₂-i-Pr), 1.94 (3H, s, 5-CH₃).
MASS (EI) m/z : 414(M⁺), 357, 295, 264, 222, 178, 116.

### (Example 1709)

### Compound of Formula No. C-1073

¹H-NMR (CDCl₃) δ(ppm) : 7.09 (1H, s, 3-H), 5.00 (1H, d, J=15Hz, NCH₂Ar), 4.82 (1H, d, J=15Hz, NCH₂Ar), 3.84 (3H, s, COOCH₃), 3.66 (2H, d, J=8Hz, NCH₂-i-Pr), 1.89 (3H, s, 5-CH₃).
MASS (EI) m/z : 447(M⁺), 416, 331, 295, 266, 239, 178.

### (Example 1710)

### Compound of Formula No. C-1074

¹H-NMR (CDCl₃) δ(ppm) : 7.21 (1H, s, 3-H), 4.76 (3H, brs, CONHCH₂Ar & NCH₂Ar), 3.72 (2H, d, J=8Hz, NCH₂-i-Pr), 1.76 (3H, s, 5-CH₃).
MASS (EI) m/z : 401(M⁺), 268, 225, 212, 152, 116.

### (Example 1711)

### Compound of Formula No. C-1075

¹H-NMR (CDCl₃) δ(ppm) : 7.20 (1H, s, 3-H), 4.75 (3H, brs, CONHCH₂Ar & NCH₂Ar), 3.73 (2H, d, J=8Hz, NCH₂-i-Pr), 1.75 (3H, s, 5-CH₃).
MASS (EI) m/z : 419 (M⁺), 268, 225, 212, 152, 109.

### (Example 1712)

### Compound of Formula No. C-1076

¹H-NMR (CDCl₃) δ(ppm) : 7.17 (1H, s, 3-H), 5.04 (1H, t, J=6Hz, CONHCH₂Ar), 4.72 (2H, s, NCH₂Ar), 3.74 (2H, d, J=8Hz, NCH₂-i-Pr), 1.72 (3H, s, 5-CH₃).
MASS (EI) m/z : 431(M⁺), 286, 268, 225, 212, 152, 121.

### (Example 1713)

### Compound of Formula No. C-1077

¹H-NMR (CDCl₃) δ(ppm) : 7.21 (1H, s, 3-H), 4.76 (3H, brs, CONHCH₂Ar & NCH₂Ar), 3.73 (2H, d, J=7Hz, NCH₂-i-Pr), 1.77 (3H, s, 5-CH₃).
MASS (EI) m/z : 431(M⁺), 315, 268, 225, 212, 152, 121.

### (Example 1714)

### Compound of Formula No. C-1078

¹H-NMR (CDCl₃) δ(ppm) : 7.22 (1H, s, 3-H), 4.76 (1H, d, J=14Hz, NCH₂Ar), 4.67 (1H, d, J=14Hz, NCH₂Ar), 4.66 (1H, d, J=7Hz, CONHCH₂Ar), 3.76 (2H, d, J=7Hz, NCH₂-i-Pr), 1.74 (3H, s, 5-CH₃).
MASS (EI) m/z : 415(M⁺), 400, 268, 212, 152, 105.

### (Example 1715)

### Compound of Formula No. C-1079

¹H-NMR (CDCl₃) δ(ppm) : 7.08 (1H, s, 3-H), 4.70 (2H, s, NCH₂Ar), 4.41 (1H, t, J=6Hz, CONHCH₂CH₂Ar), 3.69 (2H, d, J=8Hz, NCH₂-i-Pr), 1.62 (3H, s, 5-CH₃).
MASS (EI) m/z : 415(M⁺), 324, 295, 268, 152, 116.

### (Example 1716)

### Compound of Formula No. C-1080

¹H-NMR (CDCl₃) δ(ppm) : 7.13 (1H, s, 3-H), 4.71 (2H, s, NCH₂Ar), 4.45 (1H, t, J=6Hz, CONHCH₂CH₂Ar), 3.70 (2H, d, J=8Hz, NCH₂-i-Pr), 1.63 (3H, s, 5-CH₃).
MASS (EI) m/z : 475(M⁺), 311, 295, 268, 164, 151, 116.

### (Example 1717)

### Compound of Formula No. C-1081

¹H-NMR (CDCl₃) δ(ppm) : 7.31 (1H, s, 3-H), 6.72 (1H, d, J=3Hz, CONHAr), 4.81 (2H, brs, NCH₂Ar), 3.82 (2H, d, J=7Hz, NCH₂-i-Pr), 1.87 (3H, s, 5-CH₃).
MASS (EI) m/z : 405(M⁺), 289, 268, 212, 152, 116.

### (Example 1718)

### Compound of Formula No. C-1082

¹H-NMR (CDCl₃) δ(ppm) : 7.31 (1H, s, 3-H), 6.38 (1H, s, CONHAr), 4.79 (2H, brs, NCH₂Ar), 3.81 (2H, d, J=7Hz, NCH₂-i-Pr), 1.85 (3H, s, 5-CH₃).
MASS (EI) m/z : 405(M⁺), 289, 268, 212, 152, 116.

### (Example 1719)

### Compound of Formula No. C-1083

¹H-NMR (CDCl₃) δ(ppm) : 7.30 (1H, s, 3-H), 6.43 (1H, s, CONHAr), 4.80 (2H, brs, NCH₂Ar), 3.82 (2H, d, J=8Hz, NCH₂-i-Pr), 1.85 (3H, s, 5-CH₃).
MASS (EI) m/z : 417(M⁺), 301, 268, 212, 152, 116.

### (Example 1720)

### Compound of Formula No. C-1084

¹H-NMR (CDCl₃) δ(ppm) : 7.31 (1H, s, 3-H), 6.28 (1H, s, CONHAr), 4.79 (2H, brs, NCH₂Ar), 3.81 (2H, d, J=7Hz, NCH₂-i-Pr), 1.86 (3H, s, 5-CH₃).
MASS (EI) m/z : 417(M⁺), 301, 268, 222, 152, 122.

### (Example 1721)

### Compound of Formula No. C-1085

¹H-NMR (CDCl₃) δ(ppm) : 7.31 (1H, s, 3-H), 7.18 (1H, s, CONHAr), 4.81 (2H, brs, NCH₂Ar), 3.80 (2H, d, J=7Hz, NCH₂-i-Pr), 1.83 (3H, s, 5-CH₃).
MASS (EI) m/z : 388(M⁺), 268, 225, 212, 152, 120.

### (Example 1722)

### Compound of Formula No. C-1086

¹H-NMR (CDCl₃) δ(ppm) : 7.32 (1H, s, 3-H), 6.54 (1H, s, CONHAr), 4.80 (2H, brs, NCH₂Ar), 3.82 (2H, d, J=7Hz, NCH₂-i-Pr), 1.86 (3H, s, 5-CH₃).
MASS (EI) m/z : 388(M⁺), 295, 272, 212, 152, 116.

### (Example 1723)

### Compound of Formula No. C-1087

¹H-NMR (CDCl₃) δ(ppm) : 7.31 (1H, s, 3-H), 6.59 (1H, s, CONHAr), 4.79 (2H, brs, NCH₂Ar), 3.83 (2H, d, J=7Hz, NCH₂-i-Pr), 1.84 (3H, s, 5-CH₃).
MASS (EI) m/z : 388(M⁺), 272, 212, 152, 116.

### (Example 1724)

### Compound of Formula No. C-1088

¹H-NMR (CDCl₃) δ(ppm) : 7.18 (2H, s, 3-H & CONHOMe), 4.71 (2H, s, NCH₂Ar), 3.76 (2H, d, J=7Hz, NCH₂-i-Pr), 1.79 (3H, s, 5-CH₃).
MASS (EI) m/z : 441(M⁺), 295, 267, 239, 225, 116.

### (Example 1725)

### Compound of Formula No. C-1089

¹H-NMR (CDCl₃) δ(ppm) : 7.26 (1H, s, 3-H), 4.81 (2H, s, NCH₂Ar), 3.97 (2H, d, J=8Hz, NCH₂-c-Bu), 3.95 (3H, s, COOCH₃), 1.94 (3H, s, 5-CH₃).
MASS (EI) m/z : 459(M⁺), 280, 210, 190, 129.

### (Example 1726)

### Compound of Formula No. C-1090

¹H-NMR (CDCl₃) δ(ppm) : 7.28 (1H, s, 3-H), 4.78 (2H, s, NCH₂Ar), 3.97 (3H, s, COOCH₃), 3.97 (2H, d, J=8Hz, NCH₂-c-Bu), 1.97 (3H, s, 5-CH₃).
MASS (EI) m/z : 458((M-1)⁺), 414, 399, 305, 277, 189, 135.

### (Example 1727)

### Compound of Formula No. C-1091

¹H-NMR (CDCl₃) δ(ppm) : 7.58 (1H, s, CONH₂), 7.30 (1H, s, 3-H), 5.63 (1H, s, CONH₂), 4.80 (2H, brs, NCH₂Ar), 3.99 (2H, d, J=7Hz, NCH₂-c-Bu), 1.95 (3H, s, 5-CH₃).
MASS (EI) m/z : 444(M⁺), 400, 306, 278, 239, 190, 116.

### (Example 1728)

### Compound of Formula No. C-1092

¹H-NMR (CDCl₃) δ(ppm) : 7.17 (1H, s, 3-H), 4.79 (3H, brs, CONHCH₂Ar & NCH₂Ar), 3.94 (2H, d, J=7Hz, NCH₂-c-Bu), 1.78 (3H, s, 5-CH₃).
MASS (EI) m/z : 413(M⁺), 280, 212, 164, 116.

### (Example 1729)

### Compound of Formula No. C-1093

¹H-NMR (CDCl₃) δ(ppm) : 7.17 (1H, s, 3-H), 4.74 (3H, brs, CONHCH₂Ar & NCH₂Ar), 3.94 (2H, d, J=7Hz, NCH₂-c-Bu), 1.77 (3H, s, 5-CH₃).
MASS (EI) m/z : 431(M⁺), 315, 280, 212, 164, 109.

### (Example 1730)

### Compound of Formula No. C-1094

¹H-NMR (CDCl₃) δ(ppm) : 7.14 (1H, s, 3-H), 5.02 (1H, t, J=6Hz, CONHCH₂Ar), 4.71 (2H, s, NCH₂Ar), 3.96 (2H, d, J=7Hz, NCH₂-c-Bu), 1.74 (3H, s, 5-CH₃).
MASS (EI) m/z : 443(M⁺), 280, 212, 164, 121.

### (Example 1731)

### Compound of Formula No. C-1095

¹H-NMR (CDCl₃) δ(ppm) : 7.18 (1H, s, 3-H), 4.75 (3H, brs, CONHCH₂Ar & NCH₂Ar), 3.97 (2H, d, J=7Hz, NCH₂-c-Bu), 1.79 (3H, s, 5-CH₃).
MASS (EI) m/z : 443(M⁺), 327, 280, 212, 164, 121.

### (Example 1732)

### Compound of Formula No. C-1096

¹H-NMR (CDCl₃) δ(ppm) : 7.19 (1H, s, 3-H), 4.75 (1H, d, J=15Hz, NCH₂Ar), 4.66 (1H, d, J=15Hz, NCH₂Ar), 4.65 (1H, d, J=7Hz, CONHCH₂Ar), 3.97 (2H, d, J=7Hz, NCH₂-c-Bu), 1.76 (3H, s, 5-CH₃).
MASS (EI) m/z : 427(M⁺), 280, 212, 164, 116, 105.

### (Example 1733)

### Compound of Formula No. C-1097

¹H-NMR (CDCl₃) δ(ppm) : 7.06 (1H, s, 3-H), 4.69 (2H, s, NCH₂Ar), 4.41 (1H, t, J=6Hz, CONHCH₂CH₂Ar), 3.91 (2H, d, J=7Hz, NCH₂-c-Bu), 1.64 (3H, s, 5-CH₃).
MASS (EI) m/z : 427(M⁺), 336, 307, 280, 239, 164, 116.

### (Example 1734)

### Compound of Formula No. C-1098

¹H-NMR (CDCl₃) δ(ppm) : 7.10 (1H, s, 3-H), 4.70 (2H, s, NCH₂Ar), 4.45 (1H, t, J=6Hz, CONHCH₂CH₂Ar), 3.91 (2H, d, J=7Hz, NCH₂-c-Bu), 1.65 (3H, s, 5-CH₃).
MASS (EI) m/z : 487(M⁺), 323, 307, 280, 239, 164, 151, 116.

### (Example 1735)

### Compound of Formula No. C-1099

¹H-NMR (CDCl₃) δ(ppm) : 7.28 (1H, s, 3-H), 6.79 (1H, d, J=3Hz, CONHAr), 4.80 (2H, brs, NCH₂Ar), 4.04 (2H, d, J=7Hz, NCH₂-c-Bu), 1.89 (3H, s, 5-CH₃).
MASS (EI) m/z : 417(M⁺), 301, 280, 239, 164, 116.

### (Example 1736)

### Compound of Formula No. C-1100

¹H-NMR (CDCl₃) δ(ppm) : 7.28 (1H, s, 3-H), 6.36 (1H, s, CONHAr), 4.78 (2H, s, NCH₂Ar), 4.03 (2H, d, J=7Hz, NCH₂-c-Bu), 1.87 (3H, S, 5-CH₃).
MASS (EI) m/z : 417(M⁺), 389, 301, 280, 212, 164, 116.

### (Example 1737)

### Compound of Formula No. C-1101

¹H-NMR (CDCl₃) δ(ppm) : 7.27 (1H, s, 3-H), 6.41 (1H, s, CONHAr), 4.78 (2H, s, NCH₂Ar), 4.03 (2H, d, J=7Hz, NCH₂-c-Bu), 1.87 (3H, s, 5-CH₃).
MASS (EI) m/z : 429(M⁺), 401, 313, 280, 212, 164, 116.

### (Example 1738)

### Compound of Formula No. C-1102

¹H-NMR (CDCl₃) δ(ppm) : 7.28 (1H, s, 3-H), 6.28 (1H, s, CONHAr), 4.78 (2H, s, NCH₂Ar), 4.02 (2H, d, J=7Hz, NCH₂-c-Bu), 1.87 (3H, s, 5-CH₃).
MASS (EI) m/z : 429(M⁺), 401, 313, 280, 222, 164, 122.

### (Example 1739)

### Compound of Formula No. C-1103

¹H-NMR (CDCl₃) δ(ppm) : 7.27 (1H, s, 3-H), 7.18 (1H, s, CONHAr), 4.80 (2H, brs, NCH₂Ar), 4.01 (2H, d, J=7Hz, NCH₂-c-Bu), 1.86 (3H, s, 5-CH₃).
MASS (EI) m/z : 400(M⁺), 280, 252, 212, 164, 120.

### (Example 1740)

### Compound of Formula No. C-1104

¹H-NMR (CDCl₃) δ(ppm) : 7.30 (1H, s, 3-H), 6.47 (1H, s, CONHAr), 4.80 (2H, s, NCH₂Ar), 4.04 (2H, d, J=7Hz, NCH₂-c-Bu), 1.88 (3H, s, 5-CH₃).
MASS (EI) m/z : 400(M⁺), 280, 239, 212, 164, 116.

### (Example 1741)

### Compound of Formula No. C-1105

¹H-NMR (CDCl₃) δ(ppm) : 7.28 (1H, s, 3-H), 6.58 (1H, s, CONHAr), 4.78 (2H, s, NCH₂Ar), 4.04 (2H, d, J=7Hz, NCH₂-c-Bu), 1.86 (3H, s, 5-CH₃).
MASS (EI) m/z : 400(M⁺), 388, 372, 284, 256, 164, 116.

### (Example 1742)

### Compound of Formula No. C-1106

¹H-NMR (CDCl₃) δ(ppm) : 7.18 (1H, s, 3-H), 7.15 (1H, s, CONHOMe), 4.70 (2H, s, NCH₂Ar), 3.98 (2H, d, J=7Hz, NCH₂-c-Bu), 1.81 (3H, s, 5-CH₃).
MASS (EI) m/z : 353(M⁺), 323, 307, 279, 239, 116.

### (Example 1743)

### Compound of Formula No. C-1107

¹H-NMR (CDCl₃) δ(ppm) : 7.17 (1H, s, 3-H), 4.76 (2H, brs, NCH₂Ar), 3.86 (2H, d, J=7Hz, NCH₂CHEt₂), 1.87 (3H, s, COCH₃), 1.78 (3H, s, 5-CH₃).
MASS (EI) m/z : 338(M⁺), 323, 309, 295, 268, 255, 212, 116.

### (Example 1744)

### Compound of Formula No. C-1108

¹H-NMR (CDCl₃) δ(ppm) : 7.25 (1H, s, 3-H), 4.75 (2H, brs, NCH₂Ar), 3.86 (2H, d, J=7Hz, NCH₂CHEt₂), 1.81 (3H, s, 5-CH₃), 1.50-1.38 (1H, m, COCH(CH₂)₂).
MASS (EI) m/Z : 364(M⁺), 349, 335, 296, 281, 248, 116.

### (Example 1745)

### Compound of Formula No. C-1109

¹H-NMR (CDCl₃) δ(ppm) : 7.15 (1H, s, 3-H), 4.80 (2H, brs, NCH₂Ar), 3.86 (2H, d, J=7Hz, NCH₂CHEt₂), 3.78 (2H, s, COCH₂O), 1.77 (3H, s, 5-CH₃).
MASS (EI) m/z : 354(M⁺), 339, 325, 295, 284, 270, 208, 116.

### (Example 1746)

### Compound of Formula No. C-1110

¹H-NMR (CDCl₃) δ(ppm) : 7.16 (1H, s, 3-H), 4.76 (2H, brs, NCH₂Ar), 3.86 (2H, d, J=7Hz, NCH₂CHEt₂), 3.74 (2H, s, COCH₂O), 1.76 (3H, s, 5-CH₃).
MASS (EI) m/z : 368(M⁺), 353, 339, 295, 284, 252, 116.

### (Example 1747)

### Compound of Formula No. C-1111

¹H-NMR (CDCl₃) δ(ppm) : 7.21 (1H, s, 3-H), 4.80 (2H, brs, NCH₂Ar), 4.35 (2H, s, COCH₂O), 3.87 (2H, d, J=7Hz, NCH₂CHEt₂), 1.81 (3H, s, 5-CH₃).
MASS (EI) m/z : 430(M⁺), 415, 401, 360, 295, 244, 225, 116.

### (Example 1748)

### Compound of Formula No. C-1112

¹H-NMR (CDCl₃) δ(ppm) : 7.20 (1H, s, 3-H), 4.75 (2H, brs, NCH₂Ar), 4.33 (2H, s, COCH₂O), 3.76 (2H, d, J=7Hz, NCH₂CHEt₂), 1.83 (3H, s, 5-CH₃).
MASS (EI) m/z : 396(M⁺), 381, 367, 326, 295, 210, 116.

### (Example 1749)

### Compound of Formula No. C-1113

¹H-NMR (CDCl₃) δ(ppm) : 7.17 (1H, s, 3-H), 4.71 (2H, s, NCH₂Ar), 3.84 (2H, d, J=7Hz, NCH₂CHEt₂), 3.70 (3H, brs, COOCH₃), 1.81 (3H, s, 5-CH₃).
MASS (EI) m/z : 354(M⁺), 339, 325, 283, 270, 238, 168, 116.

### (Example 1750)

### Compound of Formula No. C-1114

¹H-NMR (CDCl₃) δ(ppm) : 7.13 (1H, s, 3-H), 4.67 (2H, s, NCH₂Ar), 3.83 (2H, d, J=8Hz, NCH₂CHEt₂), 1.84 (3H, s, 5-CH₃), 1.40 (9H, s, COOC(CH₃)₃).

MASS (EI) m/z : 396(M⁺), 340, 325, 296, 270, 256, 225, 116.

### (Example 1751)

### Compound of Formula No. C-1115

¹H-NMR (CDCl₃) δ(ppm) : 7.39-7.03 (5H, m, COOC₆H₅), 7.30 (1H, s, 3-H), 4.79 (2H, brs, NCH₂Ar), 3.86 (2H, d, J=8Hz, NCH₂CHEt₂), 1.91 (3H, s, 5-CH₃).
MASS (EI) m/z : 416(M⁺), 401, 387, 346, 323, 239, 116.

### (Example 1752)

### Compound of Formula No. C-1116

¹H-NMR (CDCl₃) δ(ppm) : 7.18 (1H, s, 3-H), 4.77 (2H, brs, NCH₂Ar), 3.76 (2H, s, NCH₂-t-Bu), 1.87 (3H, s, COCH₃), 1.77 (3H, s, 5-CH₃).
MASS (EI) m/z : 324(M⁺), 309, 281, 267, 225, 208, 152, 116.

### (Example 1753)

### Compound of Formula No. C-1117

¹H-NMR (CDCl₃) δ(ppm) : 7.32 (1H, s, 3-H), 4.79 (2H, s, NCH₂Ar), 3.97 (3H, s, COOCH₃), 3.75 (2H, s, NCH₂-t-Bu), 1.94 (3H, s, 5-CH₃).
MASS (EI) m/z : 461(M⁺), 446, 417, 307, 279, 191, 135, 116.

### (Example 1754)

### Compound of Formula No. C-1118

¹H-NMR (CDCl₃) δ(ppm) : 7.48 (1H, s, CONH₂), 7.34 (1H, s, 3-H), 5.74 (1H, s, CONH₂), 4.82 (2H, brs, NCH₂Ar), 3.77 (2H, s, NCH₂-t-Bu), 1.93 (3H, s, 5-CH₃).
MASS (EI) m/z : 446(M⁺), 431, 402, 308, 280, 192, 136, 116.

### (Example 1755)

### Compound of Formula No. C-1119

¹H-NMR (CDCl₃) δ(ppm) : 7.89 (1H, t, J=8Hz, O-Pyr-4'H), 7.31 (1H, s, 3-H), 4.80 (2H, s, NCH₂Ar), 3.76 (2H, s, NCH₂-t-Bu), 1.93 (3H, s, 5-CH₃).
MASS (EI) m/z : 428(M⁺), 413, 309, 281, 224, 192, 136, 116.

### (Example 1756)

### Compound of Formula No. C-1120

¹H-NMR (CDCl₃) δ(ppm) : 7.21 (1H, s, 3-H), 4.76 (3H, brs, CONHCH₂Ar & NCH₂Ar), 3.71 (2H, s, NCH₂-t-Bu), 1.74 (3H, s, 5-CH₃).
MASS (EI) m/z : 415(M⁺), 400, 282, 225, 166.

### (Example 1757)

### Compound of Formula No. C-1121

¹H-NMR (CDCl₃) δ(ppm) : 7.21 (1H, s, 3-H), 4.75 (3H, brs, CONHCH₂Ar & NCH₂Ar), 3.71 (2H, s, NCH₂-t-Bu), 1.73 (3H, s, 5-CH₃).
MASS (EI) m/z : 433(M⁺), 418, 282, 225, 166.

### (Example 1758)

### Compound of Formula No. C-1122

¹H-NMR (CDCl₃) δ(ppm) : 7.18 (1H, s, 3-H), 5.03 (1H, t, J=6Hz, CONHCH₂Ar), 4.72 (2H, brs, NCH₂Ar), 3.73 (2H, s, NCH₂-t-Bu), 1.71 (3H, s, 5-CH₃).
MASS (EI) m/z : 445(M⁺), 430, 282, 225, 166, 121.

### (Example 1759)

### Compound of Formula No. C-1123

¹H-NMR (CDCl₃) δ(ppm) : 7.22 (1H, s, 3-H), 4.76 (3H, brs, CONHCH₂Ar & NCH₂Ar), 3.71 (2H, s, NCH₂-t-Bu), 1.75 (3H, s, 5-CH₃).
MASS (EI) m/z : 445(M⁺), 430, 329, 282, 225, 166, 121.

### (Example 1760)

### Compound of Formula No. C-1124

¹H-NMR (CDCl₃) δ(ppm) : 7.22 (1H, s, 3-H), 4.76 (1H, d, J=14Hz, NCH₂Ar), 4.66 (1H, d, J=14Hz, NCH₂Ar), 4.65 (1H, d, J=7Hz, CONHCH₂Ar), 3.74 (2H, s, NCH₂-t-Bu), 1.73 (3H, s, 5-CH₃).
MASS (EI) m/z : 429(M⁺), 414, 282, 268, 225, 166, 105.

### (Example 1761)

### Compound of Formula No. C-1125

¹H-NMR (CDCl₃) δ(ppm) : 7.10 (1H, s, 3-H), 4.71 (2H, brs, NCH₂Ar), 4.40 (1H, t, J=6H2, CONHCH₂CH₂Ar), 3.67 (2H, s, NCH₂-t-Bu), 1.60 (3H, s, 5-CH₃).
MASS (EI) m/z : 429(M⁺), 414, 338, 309, 282, 224, 166, 116.

### (Example 1762)

### Compound of Formula No. C-1126

¹H-NMR (CDCl₃) δ(ppm) : 7.14 (1H, s, 3-H), 4.71 (2H, brs, NCH₂Ar), 4.44 (1H, t, J=6Hz, CONHCH₂CH₂Ar), 3.68 (2H, s, NCH₂-t-Bu), 1.61 (3H, s, 5-CH₃).
MASS (EI) m/z : 489(M⁺), 474, 325, 309, 282, 164, 151, 116.

### (Example 1763)

### Compound of Formula No. C-1127

¹H-NMR (CDCl₃) δ(ppm) : 7.30 (1H, s, 3-H), 6.72 (1H, d, J=4Hz, CONHAr), 4.82 (2H, brs, NCH₂Ar), 3.80 (2H, s, NCH₂-t-Bu), 1.87 (3H, s, 5-CH₃).
MASS (EI) m/z : 419(M⁺), 404, 362, 303, 282, 225, 166, 116.

### (Example 1764)

### Compound of Formula No. C-1128

¹H-NMR (CDCl₃) δ(ppm) : 7.32 (1H, s, 3-H), 6.37 (1H, s, CONHAr), 4.80 (2H, brs, NCH₂Ar), 3.80 (2H, s, NCH₂-t-Bu), 1.84 (3H, s, 5-CH₃).
MASS (EI) m/z : 419(M⁺), 404, 362, 303, 282, 225, 166, 116.

### (Example 1765)

### Compound of Formula No. C-1129

¹H-NMR (CDCl₃) δ(ppm) : 7.31 (1H, s, 3-H), 6.42 (1H, s, CONHAr), 4.80 (2H, brs, NCH₂Ar), 3.79 (2H, s, NCH₂-t-Bu), 1.84 (3H, s, 5-CH₃).
MASS (EI) m/z : 431(M⁺), 416, 315, 282, 225, 166, 116.

### (Example 1766)

### Compound of Formula No. C-1130

¹H-NMR (CDCl₃) δ(ppm) : 7.31 (1H, s, 3-H), 6.28 (1H, s, CONHAr), 4.79 (2H, s, NCH₂Ar), 3.79 (2H, s, NCH₂-t-Bu), 1.84 (3H, s, 5-CH₃).
MASS (EI) m/z : 431(M⁺), 416, 374, 315, 282, 222, 166, 122.

### (Example 1767)

### Compound of Formula No. C-1131

¹H-NMR (CDCl₃) δ(ppm) : 7.32 (1H, s, 3-H), 7.17 (1H, s, CONHAr), 4.80 (2H, brs, NCH₂Ar), 3.78 (2H, s, NCH₂-t-Bu), 1.81 (3H, s, 5-CH₃).
MASS (EI) m/z : 402(M⁺), 282, 267, 225, 166, 120.

### (Example 1768)

### Compound of Formula No. C-1132

¹H-NMR (CDCl₃) δ(ppm) : 7.33 (1H, s, 3-H), 6.47 (1H, s, CONHAr), 4.81 (2H, brs, NCH₂Ar), 3.81 (2H, s, NCH₂-t-Bu), 1.85 (3H, s, 5-CH₃).
MASS (EI) m/z : 402(M⁺), 387, 345, 309, 282, 225, 166, 116.

### (Example 1769)

### Compound of Formula No. C-1133

¹H-NMR (CDCl₃) δ(ppm) : 7.32 (1H, s, 3-H), 6.58 (1H, s, CONHAr), 4.80 (2H, brs, NCH₂Ar), 3.81 (2H, s, NCH₂-t-Bu), 1.83 (3H, s, 5-CH₃).
MASS (EI) m/z : 402(M⁺), 387, 345, 286, 225, 166, 116.

### (Example 1770)

### Compound of Formula No. C-1134

¹H-NMR (CDCl₃) δ(ppm) : 7.20 (1H, s, 3-H), 7.17 (1H, s, CONHOMe), 4.72 (2H, s, NCH₂Ar), 3.75 (2H, s, NCH₂-t-Bu), 1.77 (3H, s, 5-CH₃).
MASS (EI) m/z : 355(M⁺), 340, 309, 282, 239, 223, 116.

### (Example 1771)

### Compound of Formula No. C-1135

¹H-NMR (CDCl₃) δ(ppm) : 7.27 (1H, s, 3-H), 4.80 (2H, brs, NCH₂Ar), 4.37 (2H, s, COCH₂O), 3.88 (2H, s, NCH₂C(CH₂)₂Me), 1.82 (3H, s, 5-CH₃).
MASS (EI) m/z : 414(M⁺), 399, 298, 279, 253, 211, 116.

### (Example 1772)

### Compound of Formula No. C-1136

¹H-NNR (CDCl₃) δ(ppm) : 7.22 (1H, s, 3-H), 4.79 (2H, brs, NCH₂Ar), 4.35 (2H, s, COCH₂O), 3.89 (2H, s, NCH₂C(CH₂)₃Me), 1.78 (3H, s, 5-CH₃).
MASS (EI) m/z : 428(M⁺), 413, 400, 359, 335, 293, 253.

### (Example 1773)

### Compound of Formula No. C-1137

¹H-NMR (CDCl₃) δ(ppm) : 7.18 (1H, s, 3-H), 4.72 (2H, s, NCH₂Ar), 3.87 (2H, s, NCH₂C(CH₂)₃Me), 3.70 (3H, s, COOCH₃), 1.78 (3H, s, 5-CH₃).
MASS (EI) m/z : 352(M⁺), 337, 324, 283, 270, 208, 149.

### (Example 1774)

### Compound of Formula No. C-1138

¹H-NMR (CDCl₃) δ(ppm) : 8.37 (1H, d, J=3Hz, O-Pyr-6'H), 7.32 (1H, s, 3-H), 4.81 (2H, brs, NCH₂Ar), 3.87 (2H, s, NCH₂C(CH₂)₃Me), 1.92 (3H, s, 5-CH₃).
MASS (EI) m/z : 415(M⁺), 387, 371, 320, 292, 204, 116.

### (Example 1775)

### Compound of Formula No. C-1139

¹H-NMR (CDCl₃) δ(ppm) : 7.17 (1H, s, 3-H), 4.76 (2H, brs, NCH₂Ar), 3.85 (2H, s, NCH₂-C(CH₂)₄Me), 1.87 (3H, s, COCH₃), 1.77 (3H, s, 5-CH₃).
MASS (EI) m/z : 350(M⁺), 335, 307, 268, 225, 149.

### (Example 1776)

### Compound of Formula No. C-1140

¹H-NMR (CDCl₃) δ(ppm) : 7.15 (1H, s, 3-H), 4.80 (2H, brs, NCH₂Ar), 3.86 (2H, s, NCH₂-C(CH₂)₄Me), 3.77 (2H, d ,J=4Hz, COCH₂O), 1.76 (3H, s, 5-CH₃).
MASS (EI) m/z : 366(M⁺), 351, 309, 284, 168, 149.

### (Example 1777)

### Compound of Formula No. C-1141

¹H-NMR (CDCl₃) δ(ppm) : 7.22 (1H, s, 3-H), 4.79 (2H, brs, NCH₂Ar), 4.35 (2H, s, COCH₂O), 3.86 (2H, s, NCH₂-C(CH₂)₄Me), 1.80 (3H, s, 5-CH₃).
MASS (EI) m/z : 442(M⁺), 427, 360, 349, 307, 244, 149.

### (Example 1778)

### Compound of Formula No. C-1142

¹H-NMR (CDCl₃) δ(ppm) : 7.21 (1H, s, 3-H), 4.77 (2H, brs, NCH₂Ar), 4.32 (2H, s, COCH₂O), 3.86 (2H, s, NCH₂-C(CH₂)₄Me), 1.82 (3H, s, 5-CH₃).
MASS (EI) m/z : 408(M⁺), 393, 365, 326, 307, 271, 220, 210.

### (Example 1779)

### Compound of Formula No. C-1143

¹H-NMR (CDCl₃) δ(ppm) : 7.17 (1H, s, 3-H), 4.71 (2H, s, NCH₂Ar), 3.83 (2H, s, NCH₂-C(CH₂)₄Me), 3.69 (3H, s, COOCH₃), 1.79 (3H, s, 5-CH₃).
MASS (EI) m/z : 366(M⁺), 351, 307, 284, 270, 168.

### (Example 1780)

### Compound of Formula No. C-1144

¹H-NMR (CDCl₃) δ(ppm) : 7.13 (1H, s, 3-H), 4.67 (2H, s, NCH₂Ar), 3.84 (2H, s, NCH₂-C(CH₂)₄Me), 1.84 (3H, s, 5-CH₃), 1.40 (9H, s, COOC(CH₃)₃).
MASS (EI) m/z : 408(M⁺), 352, 337, 308, 270, 225, 116.

### (Example 1781)

### Compound of Formula No. C-1145

¹H-NMR (CDCl₃) δ(ppm) : 7.39-7.03 (5H, m, COOC₆H₅), 7.30 (1H, s, 3-H), 4.79 (2H, brs, NCH₂Ar), 3.86 (2H, s, NCH₂-C(CH₂)₄Me), 1.90 (3H, s, 5-CH₃).
MASS (EI) m/z : 428(M⁺), 413, 346, 335, 239, 136, 116.

### (Example 1782)

### Compound of Formula No. C-1146

¹H-NMR (CDCl₃) δ(ppm) : 8.37 (1H, d, J=4Hz, O-Pyr-6'H), 7.31 (1H, s, 3-H), 4.80 (2H, brs, NCH₂Ar), 3.85 (2H, s, NCH₂-C(CH₂)₄Me), 1.94 (3H, s, 5-CH₃).
MASS (EI) m/z : 429(M⁺), 414, 385, 347, 306, 291, 218, 116.

### (Example 1783)

### Compound of Formula No. C-1147

¹H-NMR (CDCl₃) δ(ppm) : 7.31 (1H, s, 3-H), 4.80 (2H, brs, NCH₂Ar), 3.84 (2H, s, NCH₂-C(CH₂)₄Me), 2.50 (3H, s, O-Pyr-6'CH₃), 1.94 (3H, s, 5-CH₃).
MASS (EI) m/z : 443(M⁺), 428, 399, 361, 306, 218, 116.

### (Example 1784)

### Compound of Formula No. C-1148

¹H-NMR (CDCl₃) δ(ppm) : 8.43 (1H, s, O-Pyr-6'H), 7.30 (1H, s, 3-H), 4.79 (2H, brs, NCH₂Ar), 3.87 (2H, s, NCH₂-C(CH₂)₄Me), 1.88 (3H, s, 5-CH₃).
MASS (EI) m/z : 463(M⁺), 448, 419, 381, 335, 239, 116.

### (Example 1785)

### Compound of Formula No. C-1149

¹H-NMR (CDCl₃) δ(ppm) : 7.18 (1H, s, 3-H), 4.76 (2H, brs, NCH₂Ar), 3.79 (2H, d, J=7Hz, NCH₂-c-Hex), 1.87 (3H, s, COCH₃), 1.77 (3H, s, 5-CH₃).
MASS (EI) m/z : 350(M⁺), 335, 307, 268, 254, 234, 212.

### (Example 1786)

### Compound of Formula No. C-1150

¹H-NMR (CDCl₃) δ(ppm) : 7.16 (1H, s, 3-H), 4.80 (2H, brs, NCH₂Ar), 3.79 (2H, d, J=7Hz, NCH₂-c-Hex), 3.77 (2H, brs, COCH₂O), 1.76 (3H, s, 5-CH₃).
MASS (EI) m/z : 366(M+), 309, 284, 270, 220, 168, 116.

### (Example 1787)

### Compound of Formula No. C-1151

¹H-NMR (CDCl₃) δ(ppm) : 7.17 (1H, s, 3-H), 4.77 (2H, brs, NCH₂Ar), 3.78 (2H, d, J=7Hz, NCH₂-c-Hex), 3.74 (2H, brs, COCH₂O), 1.74 (3H, s, 5-CH₃).
MASS (EI) m/z : 380(M⁺), 365, 335, 307, 298, 264, 225.

### (Example 1788)

### Compound of Formula No. C-1152

¹H-NMR (CDCl₃) δ(ppm) : 7.22 (1H, s, 3-H), 4.80 (2H, brs, NCH₂Ar), 4.35 (2H, brs, COCH₂O), 3.80 (2H, d, J=7Hz, NCH₂-c-Hex), 1.80 (3H, s, 5-CH₃).
MASS (EI) m/z : 442(M⁺), 359, 346, 326, 307, 253, 210, 116.

### (Example 1789)

### Compound of Formula No. C-1153

¹H-NMR (CDCl₃) δ(ppm) : 7.22 (1H, s, 3-H), 4.75 (2H, brs, NCH₂Ar), 4.33 (2H, s, COCH₂O), 3.79 (2H, d, J=7Hz, NCH₂-c-Hex), 1.82 (3H, s, 5-CH₃).
MASS (EI) m/z : 408(M⁺), 365, 326, 307, 271, 220, 210.

### (Example 1790)

### Compound of Formula No. C-1154

¹H-NMR (CDCl₃) δ(ppm) : 7.17 (1H, s, 3-H), 4.71 (2H, s, NCH₂Ar), 3.76 (2H, d, J=7Hz, NCH₂-c-Hex), 3.70 (3H, brs, COOCH₃), 1.80 (3H, s, 5-CH₃).
MASS (EI) m/z : 366(M⁺), 351, 335, 307, 283, 270, 168.

### (Example 1791)

### Compound of Formula No. C-1155

¹H-NMR (CDCl₃) δ(ppm) : 7.39-7.03 (5H, m, COOC₆H₅), 7.31 (1H, s, 3-H), 4.79 (2H, brs, NCH₂Ar), 3.79 (2H, d, J=7Hz, NCH₂-c-Hex), 1.90 (3H, s, 5-CH₃).
MASS (EI) m/z : 428(M⁺), 384, 346, 335, 307, 239, 116.

### (Example 1792)

### Compound of Formula No. C-1156

¹H-NMR (CDCl₃) δ(ppm) : 8.37 (1H, d, J=3Hz, O-Pyr-6'H), 7.31 (1H, s, 3-H), 4.80 (2H, brs, NCH₂Ar), 3.77 (2H, d, J=8Hz, NCH₂-c-Hex), 1.94 (3H, s, 5-CH₃).
MASS (EI) m/z : 429(M⁺), 385, 347, 306, 239, 218, 136.

### (Example 1793)

### Compound of Formula No. C-1157

¹H-NMR (CDCl₃) δ(ppm) : 7.18 (1H, s, 3-H), 4.77 (2H, brs, NCH₂Ar), 3.78 (2H, s, NCH₂-C(CH₂)₅Me), 1.87 (3H, s, COCH₃), 1.77 (3H, s, 5-CH₃).
MASS (EI) m/z : 364(M⁺), 349, 321, 268, 212, 149, 116.

### (Example 1794)

### Compound of Formula No. C-1158

¹H-NMR (CDCl₃) δ(ppm) : 7.22 (1H, s, 3-H), 4.80 (2H, brs, NCH₂Ar), 4.35 (2H, brs, COCH₂O), 3.79 (2H, s, NCH₂-C(CH₂)₅Me), 1.80 (3H, s, 5-CH₃).
MASS (EI) m/z : 456(M⁺), 441, 360, 346, 321, 244, 116.

### (Example 1795)

### Compound of Formula No. C-1159

¹H-NMR (CDCl₃) δ(ppm) : 7.18 (1H, s, 3-H), 4.71 (2H, brs, NCH₂Ar), 3.76 (2H, s, NCH₂-C(CH₂)₅Me), 3.70 (3H, brs, COOCH₃), 1.79 (3H, s, 5-CH₃).
MASS (EI) m/z : 380(M⁺), 365, 284, 270, 168, 149, 116.

### (Example 1796)

### Compound of Formula No. C-1160

¹H-NMR (CDCl₃) δ(ppm) : 7.13 (1H, s, 3-H), 4.67 (2H, s, NCH₂Ar), 3.75 (2H, s, NCH₂-C(CH₂)₅Me), 1.84 (3H, s, 5-CH₃), 1.40 (9H, s, COOC(CH₃)₃).
MASS (EI) m/z : 422(M⁺), 366, 351, 322, 270, 256, 225.

### (Example 1797)

### Compound of Formula No. C-1161

¹H-NMR (CDCl₃) δ(ppm) : 7.38-7.03 (5H, m, COOC₆H₅), 7.31 (1H, s, 3-H), 4.79 (2H, brs, NCH₂Ar), 3.78 (2H, s, NCH₂-C(CH₂)₅Me), 1.89 (3H, s, 5-CH₃).
MASS (EI) m/z : 442(M⁺), 427, 346, 239, 230, 136, 116.

### (Example 1798)

### Compound of Formula No. C-1162

¹H-NMR (CDCl₃) δ(ppm) : 8.38 (1H, d, J=3Hz, O-Pyr-6'H), 7.33 (1H, s, 3-H), 4.81 (2H, brs, NCH₂Ar), 3.77 (2H, s, NCH₂-C(CH₂)₅Me), 1.94 (3H, s, 5-CH₃).
MASS (EI) m/z : 443(M⁺), 347, 320, 305, 232, 211, 136.

### (Example 1799)

### Compound of Formula No. C-1163

¹H-NMR (CDCl₃) δ(ppm) : 7.31 (1H, s, 3-H), 4.80 (2H, brs, NCH₂Ar), 3.76 (2H, s, NCH₂-C(CH₂)₅Me), 2.51 (3H, s, O-Pyr-6'CH₃), 1.94 (3H, s, 5-CH₃).
MASS (EI) m/z : 457(M⁺), 442, 413, 361, 320, 232, 136, 116.

### (Example 1800)

### Compound of Formula No. C-1164

¹H-NMR (CDCl₃) δ(ppm) : 7.33 (1H, s, 3-H), 5.48 (2H, s, NCH₂OH), 4.78 (2H, s, NCH₂Ar), 2.01 (3H, s, 5-CH₃), 1.52-1.41 (1H, m, COCH(CH₂)₂).
MASS (EI) m/z : 310(M⁺), 280, 212, 116.

### (Example 1801)

### Compound of Formula No. C-1165

¹H-NMR (CDCl₃) δ(ppm) : 7.27 (1H, s, 3-H), 5.46 (2H, s, NCH₂OH), 4.74 (2H, s, NCH₂Ar), 3.73 (3H, s, COOCH₃), 2.00 (3H, S, 5-CH₃). MASS (EI) m/z : 270((M-CH₂O)⁺), 211, 154, 116.

### (Example 1802)

### Compound of Formula No. C-1166

¹H-NMR (CDCl₃) δ(ppm) : 7.42 (1H, s, 3-H), 5.79 (2H, s, NCH₂Cl), 4.78 (2H, brs, NCH₂Ar), 1.98 (3H, s, 5-CH₃), 1.52-1.40 (1H, m, COCH(CH₂)₂).
MASS (EI) m/z : 328(M⁺), 293, 260, 224, 116.

### (Example 1803)

### Compound of Formula No. C-1167

¹H-NMR (CDCl₃) δ(ppm) : 7.27 (1H, s, 3-H), 5.77 (2H, s, NCH₂Cl), 4.71 (2H, s, NCH₂Ar), 3.74 (3H, s, COOCH₃), 2.01 (3H, s, 5-CH₃). MASS (EI) m/z : 318(M⁺), 283, 202, 170, 116.

### (Example 1804)

### Compound of Formula No. C-1168

¹H-NMR (CDCl₃) δ(ppm) : 7.27 (1H, s, 3-H), 4.70 (2H, s, NCH₂Ar), 4.42 (2H, d, J=12Hz, NCH₂PO₃Et₂), 3.71 (3H, s, COOCH₃), 1.94 (3H, s, 5-CH₃).
MASS (EI) m/z : 420(M⁺), 361, 304, 283, 216, 116.

### (Example 1805)

### Compound of Formula No. C-1169

¹H-NMR (CDCl₃) δ(ppm) : 7.28 (1H, s, 3-H), 4.80 (2H, brs, NCH₂Ar), 4.41 (2H, brs, NCH₂C(=CH₂)Me), 1.78 (3H, s, 5-CH₃), 1.54-1.44 (1H, m, COCH(CH₂)₂).
MASS (EI) m/z : 334(M⁺), 266, 218, 150, 116.

### (Example 1806)

### Compound of Formula No. C-1170

¹H-NMR (CDCl₃) δ(ppm) : 7.19 (1H, s, 3-H), 4.72 (2H, s, NCH₂Ar), 4.41 (2H, brs, NCH₂C(=CH₂)Me), 3.72 (3H, s, COOCH₃), 1.78 (3H, s, 5-CH₃).
MASS (EI) m/z : 324(M⁺), 309, 265, 208, 116.

### (Example 1807)

### Compound of Formula No. C-1171

¹H-NMR (CDCl₃) δ(ppm) : 7.25 (1H, s, 3-H), 6.50 (1H, s, NCH=CMe₂), 4.73 (2H, s, NCH₂Ar), 3.73 (3H, brs, COOCH₃), 1.87 (3H, s, 5-CH₃).
MASS (EI) m/z : 324(M⁺), 283, 265, 208, 176, 116.

### (Example 1808)

### Compound of Formula No. C-1172

¹H-NMR (CDCl₃) δ(ppm) : 7.23 (1H, s, 3-H), 6.69 (1H, s, NCH=C(CH₂)₄), 4.71 (2H, s, NCH₂Ar), 3.73 (3H, brs, COOCH₃), 1.85 (3H, s, 5-CH₃).
MASS (EI) m/z : 350(M⁺), 322, 309, 283, 234, 202, 116.

### (Example 1809)

### Compound of Formula No. C-1173

¹H-NMR (CDCl₃) δ(ppm) : 7.25 (1H, s, 3-H), 4.78 (2H, brs, NCH₂Ar), 4.05 (2H, t, J=6Hz, NCH₂(CH₂)₂CF₃), 1.87 (3H, s, 5-CH₃), 1.54-1.41 (1H, m, COCH(CH₂)₂).
MASS (EI) m/z : 390(M⁺), 371, 322, 274, 206, 116.

### (Example 1810)

### Compound of Formula No. C-1174

¹H-NMR (CDCl₃) δ(ppm) : 7.16 (1H, s, 3-H), 4.71 (2H, s, NCH₂Ar), 4.02 (2H, t, J=6Hz, NCH₂(CH₂)₂CF₃), 3.73 (3H, brs, COOCH₃), 1.87 (3H, s, 5-CH₃).
MASS (EI) m/z : 380(M⁺), 361, 321, 264, 232, 116.

### (Example 1811)

### Compound of Formula No. C-1175

¹H-NMR (CDCl₃) δ(ppm) : 7.28 (1H, s, 3-H), 4.77 (2H, brs, NCH₂Ar), 4.23 (2H, d, J=7Hz, NCH₂(C₃H₇O₂)), 1.86 (3H, s, 5-CH₃), 1.52-1.38 (1H, m, COCH(CH₂)₂).
MASS (EI) m/z : 368(M⁺), 300, 281, 252, 225, 116.

### (Example 1812)

### Compound of Formula No. C-1176

¹H-NMR (CDCl₃) δ(ppm) : 7.18 (1H, s, 3-H), 4.70 (2H, s, NCH₂Ar), 4.20 (2H, d, J=7Hz, NCH₂(C₃H₇O₂)), 3.72 (3H, brs, COOCH₃), 1.86 (3H, s, 5-CH₃).
MASS (EI) m/z : 358(M⁺), 327, 284, 271, 168, 116.

### (Example 1813)

### Compound of Formula No. C-1177

¹H-NMR (CDCl₃) δ(ppm) : 7.27 (1H, s, 3-H), 4.79 (2H, brs, NCH₂Ar), 4.17 (2H, d, J=7Hz, NCH₂(C₄H₉O₂)), 1.88 (3H, s, 5-CH₃), 1.55-1.37 (1H, m, COCH(CH₂)₂).
MASS (EI) m/z : 382(M⁺), 367, 313, 294, 281, 149.

### (Example 1814)

### Compound of Formula No. C-1178

¹H-NMR (CDCl₃) δ(ppm) : 7.17 (1H, s, 3-H), 4.71 (2H, s, NCH₂Ar), 4.14 (2H, d, J=7Hz, NCH₂(C₄H₉O₂)), 3.72 (3H, brs, COOCH₃), 1.87 (3H, s, 5-CH₃).
MASS (EI) m/z : 372(M⁺), 357, 311, 284, 168, 116.

### (Example 1815)

### Compound of Formula No. C-1179

¹H-NMR (CDCl₃) δ(ppm) : 7.25 (1H, s, 3-H), 4.79 (2H, brs, NCH₂Ar), 4.06 (2H, d, J=7Hz, NCH₂(C₅H₁₁O₂)), 1.87 (3H, s, 5-CH₃), 1.58-1.42 (1H, m, COCH(CH₂)₂).
MASS (EI) m/z : 396(M⁺), 381, 349, 327, 294, 225, 178, 116.

### (Example 1816)

### Compound of Formula No. C-1180

¹H-NMR (CDCl₃) δ(ppm) : 7.17 (1H, s, 3-H), 4.70 (2H, s, NCH₂Ar), 4.02 (2H, d, J=7Hz, NCH₂(C₅H₁₁O₂)), 3.72 (3H, brs, COOCH₃), 1.86 (3H, s, 5-CH₃).
MASS (EI) m/z : 386(M⁺), 371, 339, 284, 168, 116.

### (Example 1817)

### Compound of Formula No. C-1181

¹H-NMR (CDCl₃) δ(ppm) : 7.27 (1H, s, 3-H), 4.78 (2H, brs, NCH₂Ar), 4.07 (2H, d, J=7Hz, NCH₂(C₇H₁₁O₄)), 1.84 (3H, s, 5-CH₃), 1.51-1.37 (1H, m, COCH(CH₂)₂).
MASS (EI) m/z : 452(M⁺), 109, 384, 323, 116.

### (Example 1818)

### Compound of Formula No. C-1182

¹H-NMR (CDCl₃) δ(ppm) : 7.17 (1H, s, 3-H), 4.70 (2H, s, NCH₂Ar), 4.04 (2H, d, J=7Hz, NCH₂(C₇H₁₁O₄)), 3.72 (3H, brs, COOCH₃), 1.84 (3H, s, 5-CH₃).
MASS (EI) m/z : 442(M⁺), 399, 383, 339, 283, 168, 116.

### (Example 1819)

### Compound of Formula No. C-1183

¹H-NMR (CDCl₃) δ(ppm) : 7.24 (1H, s, 3-H), 4.78 (2H, brs, NCH₂Ar), 4.24 (2H, d, J=8Hz, NCH₂(C₆H₁₁O₂)), 1.94 (3H, s, 5-CH₃), 1.51-1.40 (1H, m, COCH(CH₂)₂).
MASS (EI) m/z : 408(M⁺), 393, 350, 321, 281, 212, 116.

### (Example 1820)

### Compound of Formula No. C-1184

¹H-NMR (CDCl₃) δ(ppm) : 7.16 (1H, s, 3-H), 4.70 (2H, s, NCH₂Ar), 4.22 (2H, d, J=8Hz, NCH₂(C₆H₁₁O₂)), 3.71 (3H, s, COOCH₃), 1.93 (3H, s, 5-CH₃).
MASS (EI) m/z : 398(M⁺), 383, 340, 311, 270, 236, 168, 116.

### (Example 1821)

### Compound of Formula No. C-1185

¹H-NMR (CDCl₃) δ(ppm) : 7.29 (1H, s, 3-H), 4.78 (2H, brs, NCH₂Ar), 4.11 (2H, s, NCH₂(C₄H₉O₂)), 1.85 (3H, s, 5-CH₃), 1.53-1.37 (1H, m, COCH(CH₂)₂).
MASS (EI) m/z : 382(M⁺), 313, 294, 281, 266, 225, 212, 116.

### (Example 1822)

### Compound of Formula No. C-1186

¹H-NMR (CDCl₃) δ(ppm) : 7.19 (1H, s, 3-H), 4.72 (2H, s, NCH₂Ar), 4.09 (2H, s, NCH₂(C₄H₉O₂)), 3.73 (3H, s, COOCH₃), 1.85 (3H, s, 5-CH₃).
MASS (EI) m/z : 372(M⁺), 341, 284, 270, 168, 116.

### (Example 1823)

### Compound of Formula No. C-1187

¹H-NMR (CDCl₃) δ(ppm) : 7.27 (1H, s, 3-H), 4.77 (2H, brs, NCH₂Ar), 4.06 (1H, d, J=14Hz, NCH₂(C₅H₁₁O₂)), 4.00 (1H, d, J=14Hz, NCH₂(C₅H₁₁O₂)), 1.85 (3H, s, 5-CH₃), 1.54-1.35 (1H, m, COCH(CH₂)₂).
MASS (EI) m/z : 396(M⁺), 381, 327, 294, 281, 225, 178, 116.

### (Example 1824)

### Compound of Formula No. C-1188

¹H-NMR (CDCl₃) δ(ppm) : 7.17 (1H, s, 3-H), 4.71 (2H, s, NCH₂Ar), 4.03 (1H, d, J=14Hz, NCH₂(C₅H₁₁O₂)), 3.96 (1H, d, J=14Hz, NCH₂(C₅H₁₁O₂)), 3.72 (3H, s, COOCH₃), 1.84 (3H, s, 5-CH₃).
MASS (EI) m/z : 386(M⁺), 371, 325, 284, 271, 168, 138.

### (Example 1825)

### Compound of Formula No. C-1189

¹H-NMR (CDCl₃) δ(ppm) : 7.24 (1H, s, 3-H), 4.77 (2H, brs, NCH₂Ar), 3.97 (2H, s, NCH₂(C₆H₁₃O₂)), 1.84 (3H, s, 5-CH₃), 1.55-1.40 (1H, m, COCH(CH₂)₂).
MASS (EI) m/z : 410(M⁺), 395, 363, 341, 294, 225, 178, 116.

### (Example 1826)

### Compound of Formula No. C-1190

¹H-NMR (CDCl₃) δ (ppm) : 7.16 (1H, s, 3-H), 4.70 (2H, s, NCH₂Ar), 3.93 (2H, s, NCH₂(C₆H₁₃O₂)), 3.72 (3H, s, COOCH₃), 1.84 (3H, s, 5-CH₃).
MASS (EI) m/z : 400(M⁺), 385, 353, 284, 168, 116.

### (Example 1827)

### Compound of Formula No. C-1191

¹H-NMR (CDCl₃) δ(ppm) : 7.29 (1H, s, 3-H), 4.77 (2H, brs, NCH₂Ar), 4.01 (1H, d, J=14Hz, NCH₂(C₆H₁₁O₃)), 3.87 (1H, d, J=14Hz, NCH₂(C₆H₁₁O₃)), 1.83 (3H, s, 5-CH₃), 1.51-1.35 (1H, m, COCH(CH₂)₂).
MASS (EI) m/z : 424(M⁺), 398, 381, 356, 294, 281, 225, 116.

### (Example 1828)

### Compound of Formula No. C-1192

¹H-NMR (CDCl₃) δ(ppm) : 7.19 (1H, s, 3-H), 4.70 (2H, s, NCH₂Ar), 4.01 (1H, d, J=14Hz, NCH₂(C₆H₁₁O₃)), 3.87 (1H, d, J=14Hz, NCH₂(C₆H₁₁O₃)), 3.72 (3H, s, COOCH₃), 1.82 (3H, s, 5-CH₃).
MASS (EI) m/z : 414(M⁺), 371, 325, 284, 271, 168, 116.

### (Example 1829)

### Compound of Formula No. C-1193

¹H-NMR (CDCl₃) δ(ppm) : 7.25 (1H, s, 3-H), 4.78 (2H, brs, NCH₂Ar), 4.18 (2H, s, NCH₂(C₇H₁₃O₂)), 1.92 (3H, s, 5-CH₃), 1.52-1.40 (1H, m, COCH(CH₂)₂).
MASS (EI) m/z : 422(M⁺), 407, 335, 295, 280, 212, 116.

### (Example 1830)

### Compound of Formula No. C-1194

¹H-NMR (CDCl₃) δ(ppm) : 7.16 (1H, s, 3-H), 4.70 (2H, s, NCH₂Ar), 4.15 (2H, s, NCH₂(C₇H₁₃O₂)), 3.71 (3H, s, COOCH₃), 1.92 (3H, s, 5-CH₃).
MASS (EI) m/z : 412(M⁺), 397, 325, 270, 116.

### (Example 1831)

### Compound of Formula No. C-1195

¹H-NMR (CDCl₃) δ(ppm) : 7.29 (1H, s, 3-H), 4.78 (2H, brs, NCH₂Ar), 4.19 (2H, d, J=4Hz, NCH₂(C₃H₅O₂)), 1.89 (3H, s, 5-CH₃), 1.56-1.38 (1H, m, COCH(CH₂)₂).
MASS (EI) m/z : 366(M⁺), 298, 225, 116.

### (Example 1832)

### Compound of Formula No. C-1196

¹H-NMR (CDCl₃) δ(ppm) : 7.21 (1H, s, 3-H), 4.71 (2H, s, NCH₂Ar), 4.17 (2H, d, J=4Hz, NCH₂(C₃H₅O₂)), 3.72 (3H, s, COOCH₃), 1.89 (3H, s, 5-CH₃).
MASS (EI) m/z : 356(M⁺), 325, 283, 168, 116.

### (Example 1833)

### Compound of Formula No. C-1197

¹H-NMR (CDCl₃) δ(ppm) : 7.63-7.40 (5H, m, NC₆H₅), 7.38 (1H, s, 3-H), 4.85 (2H, s, NCH₂Ar), 4.48 (2H, s, COCH₂O), 1.96 (3H, s, 5-CH₃).
MASS (EI) in/z : 422(M⁺), 329, 306, 287, 158, 118.

### (Example 1834)

### Compound of Formula No. C-1198

¹H-NMR (CDCl₃) δ(ppm) : 8.09 (1H, dd, J=5Hz, 2Hz, O-Pyr-6'H), 7.65-7.45 (5H, m, NC₆H₅), 7.43 (1H, s, 3-H), 4.85 (2H, brs, NCH₂Ar), 4.75 (2H, s, COCH₂O), 2.11 (3H, s, 5-CH₃).
MASS (EI) m/z : 423(M⁺), 328, 288, 136.

### (Example 1835)

### Compound of Formula No. C-1199

¹H-NMR (CDCl₃) δ(ppm) : 7.65-7.43 (5H, m, NC₆H₅), 7.43 (1H, s, 3-H), 7.40-7.08 (5H, m, COOC₆H₅), 4.85 (2H, s, NCH₂Ar), 2.07 (3H, s, 5-CH₃).
MASS (EI) m/z : 408(M⁺), 315, 199, 116.

### (Example 1836)

### Compound of Formula No. C-1200

¹H-NMR (CDCl₃) δ(ppm) : 8.39 (1H, d, J=4Hz, O-Pyr-6'H), 7.67-7.38 (5H, m, NC₆H₅), 7.43 (1H, s, 3-H), 4.87 (2H, s, NCH₂Ar), 2.12 (3H, s, 5-CH₃).
MASS (EI) m/z : 409(M⁺), 315, 286, 198, 116.

### (Example 1837)

### Compound of Formula No. C-1201

¹H-NMR (CDCl₃) δ(ppm) : 7.47-7.21 (5H, m, NC₆H₅), 7.28 (1H, s, 3-H), 6.33 (1H, dd, J=7Hz, 1Hz, O-Pyr-3'H), 5.04 (1H, d, J=14Hz, NCH₂Ar), 4.93 (1H, d, J=14Hz, NCH₂Ar), 2.03 (3H, s, 5-CH₃).
MASS (EI) m/z : 409(M⁺), 315, 286, 198, 116.

### (Example 1838)

### Compound of Formula No. C-1202

¹H-NMR (CDCl₃) δ(ppm) : 7.37 (1H, s, 3-H), 7.31-7.10 (4H, m, NC₆H₄F), 4.85 (2H, brs, NCH₂Ar), 4.47 (2H, s, COCH₂O), 2.00 (3H, s, 5-CH₃).
MASS (EI) m/z : 440(M⁺), 422, 347, 305, 217, 176, 136, 116.

### (Example 1839)

### Compound of Formula No. C-1203

¹H-NMR (CDCl₃) δ(ppm) : 7.37 (1H, s, 3-H), 7.04-6.87 (3H, m, NC₆H₃F₂), 4.84 (2H, brs, NCH₂Ar), 4.47 (2H, s, COCH₂O), 2.04 (3H, s, 5-CH₃).
MASS (EI) m/z : 458(M⁺), 365, 323, 235, 154, 116.

### (Example 1840)

### Compound of Formula No. C-1204

¹H-NMR (CDCl₃) δ(ppm) : 8.10 (1H, dd, J=5Hz, 2Hz, O-Pyr-6'H), 7.44 (1H, s, 3-H), 7.08-6.87 (3H, m, NC₆H₃F₂), 4.84 (2H, brs, NCH₂Ar), 4.72 (2H, s, COCH₂O), 2.20 (3H, s, 5-CH₃).
MASS (EI) m/z : 459(M⁺), 364, 322, 154, 136.

### (Example 1841)

### Compound of Formula No. C-1205

¹H-NMR (CDCl₃) δ(ppm) : 8.39 (1H, d, J=5Hz, O-Pyr-6'H), 7.48 (1H, s, 3-H), 7.07-6.80 (3H, m, NC₆H₃F₂), 4.86 (2H, brs, NCH₂Ar), 2.20 (3H, s, 5-CH₃).
MASS (EI) m/z : 445(M⁺), 351, 322, 234, 154, 116.

### (Example 1842)

### Compound of Formula No. C-1206

¹H-NMR (CDCl₃) δ(ppm) : 7.28 (1H, s, 3-H), 7.06-6.80 (3H, m, NC₆H₃F₂), 6.31 (1H, dd, J=7Hz, 1Hz, O-Pyr-3'H), 5.04 (1H, d, J=14Hz, NCH₂Ar), 4.90 (1H, d, J=14Hz, NCH₂Ar), 2.12 (3H, s, 5-CH₃).
MASS (EI) m/z : 445(M⁺), 351, 322, 234, 154, 116.

### (Example 1843)

### Compound of Formula No. C-1207

¹H-NMR (CDCl₃) δ(ppm) : 7.26 (1H, s, 3-H), 4.81 (2H, brs, NCH₂Ar), 3.77 (2H, d, J=7Hz, NCH₂-i-Pr), 1.82 (3H, s, 5-CH₃), 1.49-1.39 (1H, m, COCH(CH₂)₂).
MASS (EI) m/z : 336(M⁺), 268, 220, 152, 116.

### (Example 1844)

### Compound of Formula No. C-1208

¹H-NMR (CDCl₃) δ(ppm) : 7.18 (1H, s, 3-H), 4.73 (2H, s, NCH₂Ar), 3.75 (2H, d, J=7Hz, NCH₂-i-Pr), 3.70 (3H, brs, COOCH₃), 1.82 (3H, s, 5-CH₃).
MASS (EI) m/z : 326(M⁺), 311, 283, 270, 210, 154, 116.

### (Example 1845)

### Compound of Formula No. C-1209

¹H-NMR (CDCl₃) δ(ppm) : 7.27 (1H, s, 3-H), 4.80 (2H, brs, NCH₂Ar), 3.76 (2H, s, NCH₂-t-Bu), 1.81 (3H, s, 5-CH₃), 1.48-1.38 (1H, m, COCH(CH₂)₂).
MASS (EI) m/z : 350(M⁺), 335, 282, 234, 166, 116.

### (Example 1846)

### Compound of Formula No. C-1210

¹H-NMR (CDCl₃) δ(ppm) : 7.18 (1H, s, 3-H), 4.73 (2H, s, NCH₂Ar), 3.73 (2H, s, NCH₂-t-Bu), 3.69 (3H, brs, COOCH₃), 1.80 (3H, s, 5-CH₃).
MASS (EI) m/z : 340(M⁺), 325, 283, 224, 168, 116.

### (Example 1847)

### Compound of Formula No. C-1211

¹H-NMR (CDCl₃) δ(ppm) : 7.28 (1H, s, 3-H), 4.75 (2H, brs, NCH₂Ar), 3.75 (2H, s, NCH₂-t-Bu), 1.77 (3H, s, 5-CH₃), 1.49-1.37 (1H, m, COCH(CH₂)₂).
MASS (EI) m/z : 359(M⁺), 344, 291, 234, 166, 125.

### (Example 1848)

### Compound of Formula No. C-1212

¹H-NMR (CDCl₃) δ(ppm) : 7.26 (1H, s, 3-H), 4.65 (2H, s, NCH₂Ar), 3.73 (2H, s, NCH₂-t-Bu), 3.69 (3H, brs, COOCH₃), 1.77 (3H, s, 5-CH₃).
MASS (EI) m/z : 349(M⁺), 334, 292, 224, 168, 125.

### (Example 1849)

### Compound of Formula No. C-1213

¹H-NMR (CDCl₃) δ(ppm) : 7.27 (1H, s, 3-H), 4.85 (2H, brs, NCH₂Ar), 3.76 (2H, s, NCH₂-t-Bu), 1.85 (3H, s, 5-CH₃), 1.52-1.39 (1H, m, COCH(CH₂)₂).
MASS (EI) m/z : 370(M⁺), 355, 302, 285, 234, 166, 136.

### (Example 1850)

### Compound of Formula No. C-1214

¹H-NMR (CDCl₃) δ(ppm) : 7.20 (1H, s, 3-H), 4.80 (2H, s, NCH₂Ar), 3.74 (2H, s, NCH₂-t-Bu), 3.73 (3H, brs, COOCH₃), 1.84 (3H, s, 5-CH₃).
MASS (EI) m/z : 360(M⁺), 345, 303, 287, 224, 168, 136.

### (Example 1851)

### Compound of Formula No. C-1215

¹H-NMR (CDCl₃) δ(ppm) : 7.28 (1H, s, 3-H), 4.80 (2H, brs, NCH₂Ar), 4.63 (2H, s, COCH₂O), 3.79 (2H, d, J=8Hz, 1-NCH₂-i-Pr), 1.90 (3H, s, 5-CH₃).
MASS (EI) m/z : 475(M⁺), 266, 210, 178, 150, 116, 104.

### (Example 1852)

### Compound of Formula No. C-1216

¹H-NMR (CDCl₃) δ(ppm) : 7.21 (1H, s, 3-H), 4.80 (2H, brs, NCH₂Ar), 3.78 (2H, d, J=7Hz, 1-NCH₂-i-Pr), 3.58 (2H, brs, SCH₂CN), 3.28 (2H, s, COCH₂S), 1.84 (3H, s, 5-CH₃).
MASS (EI) m/z : 381(M⁺), 366, 341, 309, 295, 285, 267, 253.

### (Example 1853)

### Compound of Formula No. C-1217

¹H-NMR (CDCl₃) δ(ppm) : 7.22 (1H, s, 3-H), 4.76 (2H, brs, NCH₂Ar), 4.42 (2H, s, COCH₂O), 3.78 (2H, d, J=8Hz, 1-NCH₂-i-Pr), 3.71 (2H, brs, SCH₂CN), 3.54 (2H, s, OCOCH₂S), 1.84 (3H, s, 5-CH₃).
MASS (EI) m/z : 439(M⁺), 368, 323, 312, 266, 210, 180, 152.

### (Example 1854)

### Compound of Formula No. C-1218

¹H-NMR (CDCl₃) δ(ppm) : 8.70 (1H, brs, NH), 7.23 (1H, s, 3-H), 4.77 (2H, brs, NCH₂Ar), 4.48 (2H, s, COCH₂O), 3.79 (2H, d, J=7Hz, 1-NCH₂-i-Pr), 1.84 (3H, s, 5-CH₃).
MASS (EI) m/z : 513(M⁺), 470, 369, 326, 295.

### (Example 1855)

### Compound of Formula No. C-1219

¹H-NMR (CDCl₃) δ(ppm) : 8.54 (1H, brs, NH), 7.30 (1H, s, 3-H), 5.46 (2H, brs, NCH₂Ar), 3.71 (2H, d, J=8Hz, 1-NCH₂-i-Pr), 1.93 (3H, s, 5-CH₃).
MASS (EI) m/z : 431(M⁺), 415, 399, 372, 268, 225, 212, 152.

### (Example 1856)

### Compound of Formula No. C-1220

¹H-NMR (CDCl₃) δ(ppm) : 7.22 (1H, s, 3-H), 4.76 (2H, brs, NCH₂Ar), 4.39 (2H, s, COCH₂O), 4.26 (2H, q, J=7Hz, OCH₂CH₃), 3.78 (2H, d, J=7Hz, 1-NCH₂-i-Pr), 1.84 (3H, s, 5-CH₃), 1.31 (3H, t, J=7Hz, OCH₂CH₃).
MASS (EI) m/z : 441(M⁺), 395, 326, 311, 283, 267, 239, 225.

### (Example 1857)

### Compound of Formula No. C-1221

¹H-NMR (CDCl₃) δ(ppm) : 7.23 (1H, s, 3-H), 6.83 (1H, brs, NH), 4.70 (2H, brs, NCH₂Ar), 4.15 (2H, q, J=7Hz, OCH₂CH₃), 3.76 (2H, brd, J=7Hz, 1-NCH₂-i-Pr), 1.76 (3H, s, 5-CH₃), 1.22 (3H, t, J=7Hz, OCH₂CH₃).
MASS (EI) m/z : 383(M⁺), 337, 322, 294, 281, 267, 221, 211.

### (Example 1858)

### Compound of Formula No. C-1222

¹H-NMR (CDCl₃) δ(ppm) : 7.74 (1H, brs, NH), 7.21 (1H, s, 3-H), 4.74 (2H, brs, NCH₂Ar), 4.37 (2H, brs, COCH₂O), 4.23 (2H, q, J=7Hz, OCH₂CH₃), 3.74 (2H, d, J=7Hz, 1-NCH₂), 1.81 (3H, s, 5-CH₃), 1.28 (3H, t, J=7Hz, OCH₂CH₃).
MASS (EI) m/z : 455(M⁺), 409, 340, 325, 309, 283, 223, 211.

### (Example 1859)

### Compound of Formula No. C-1223

¹H-NMR (CDCl₃) δ(ppm) : 7.27 (1H, s, 3-H), 6.85 (1H, brs, NH), 4.73 (2H, brs, NCH₂Ar), 4.19 (2H, q, J=7Hz, OCH₂CH₃), 3.79 (2H, brd, J=7Hz, 1-NCH₂), 1.76 (3H, s, 5-CH₃), 1.25 (3H, t, J=7Hz, OCH₂CH₃).
MASS (EI) m/z : 397(M⁺), 351, 336, 294, 281, 267, 235, 225.

### (Example 1860)

### Compound of Formula No. C-1224

¹H-NMR (CDCl₃) δ(ppm) : 7.86 (1H, brs, NH), 7.28 (1H, s, 3-H), 4.69 (2H, brs, NCH₂Ar), 4.59 (2H, s, COCH₂Cl), 3.78 (2H, s, 1-NCH₂), 1.78 (3H, s, 5-CH₃).
MASS (EI) m/z : 401(M⁺), 365, 350, 309, 282, 267, 249.

### (Example 1861)

### Compound of Formula No. C-1225

¹H-NMR (CDCl₃) δ(ppm) : 7.33 (1H, s, 3-H), 4.80 (2H, brs, NCH₂Ar), 3.79 (2H, s, 1-NCH₂), 1.85 (3H, s, 5-CH₃).
MASS (EI) m/z : 429(M⁺), 413, 365, 350, 282, 267, 225, 212.

### (Example 1862)

### Compound of Formula No. C-1226

¹H-NMR (CDCl₃) δ(ppm) : 8.67 (1H, brs, NH), 7.30 (1H, s, 3-H), 5.46 (2H, s, NCH₂Ar), 3.71 (2H, s, 1-NCH₂-i-Pr), 1.93 (3H, s, 5-CH₃).
MASS (EI) m/z : 445(M⁺), 413, 397, 386, 371, 282, 267, 225.

### (Example 1863)

### Compound of Formula No. C-1227

¹H-NMR (CDCl₃) δ(ppm) : 7.91 (1H, brs, NH), 7.26 (1H, s, 3-H), 4.73 (2H, br, NCH₂Ar), 4.57 (2H, s, COCH₂Cl), 3.79 (2H, br, 1-NCH₂-i-Pr), 1.81 (3H, s, 5-CH₃).
MASS (EI) m/z : 387(M⁺), 351, 336, 311, 295, 268, 235, 225.

### (Example 1864)

### Compound of Formula No. C-1228

¹H-NMR (CDCl₃) δ(ppm) : 7.33 (1H, s, 3-H), 6.23 (1H, br, NH), 4.79 (2H, brs, NCH₂Ar), 3.80 (2H, d, J=7Hz, 1-NCH₂-i-Pr), 1.85 (3H, s, 5-CH₃).
MASS (EI) m/z : 415(M⁺), 399, 339, 311, 268, 253, 225, 212.

### (Example 1865)

### Compound of Formula No. C-1229

¹H-NMR (CDCl₃) δ(ppm) : 9.00 (1H, brs, NH), 7.26 (1H, s, 3-H), 4.77 (2H, brs, NCH₂Ar), 4.51 (2H, s, COCH₂O), 4.43 (2H, s, COCH₂Cl), 3.79 (2H, d, J=8Hz, 1-NCH₂-i-Pr) , 1.86 (3H, s, 5-CH₃). MASS (EI) m/z : 445(M⁺), 402, 369, 326, 311, 283, 267.

### (Example 1866)

### Compound of Formula No. C-1230

¹H-NMR (CDCl₃) δ(ppm) : 7.23 (1H, s, 3-H), 6.25 (1H, br, NH), 4.75 (2H,br, NCH₂Ar), 4.45 (2H, brs, COCH₂O), 3.78 (2H, d, J=7Hz, 1-NCH₂-i-Pr), 1.85 (3H, s, 5-CH₃).
MASS (EI) m/z : 473(M⁺), 460, 442, 431, 403, 326, 311, 283, 267.

### (Example 1867)

### Compound of Formula No. C-1231

¹H-NMR (CDCl₃) δ(ppm) : 8.62 (1H, brs, NH), 7.25 (1H, s, 3-H), 4.75 (2H, br, NCH₂Ar), 4.49 (2H, s, COCH₂O), 4.42 (2H, s, COCH₂Cl), 3.78 (2H, s, 1-NCH₂), 1.84 (3H, s, 5-CH₃).

### (Example 1868)

### Compound of Formula No. C-1232

¹H-NMR (CDCl₃) δ(ppm) : 8.70 (1H, brs, NH), 7.24 (1H, s, 3-H), 4.70 (2H, br, NCH₂Ar), 4.45 (2H, brs, COCH₂O), 3.77 (2H, s, 1-NCH₂), 1.84 (3H, s, 5-CH₃).
MASS (EI) m/z : 487(M⁺), 456, 417, 340, 325, 309, 283, 269.

### (Example 1869)

### Compound of Formula No. C-1233

¹H-NMR (CDCl₃) δ(ppm) : 8.61 (1H, brs, NH), 7.31 (1H, s, 3-H), 4.83 (2H, brs, NCH₂Ar), 3.81 (2H, s, 1-NCH₂), 1.86 (3H, s, 5-CH₃).
MASS (EI) m/z : 469(M⁺), 435, 325, 310.

### (Example 1870)

### Compound of Formula No. C-1234

¹H-NMR (CDCl₃) δ(ppm) : 7.21 (1H, s, 3-H), 4.88 (2H,brs, NCH₂Ar), 4.76 (2H, brs, COCH₂O), 3.79 (2H, d, J=8Hz, 1-NCH₂-i-Pr), 1.87 (3H, s, 5-CH₃).
MASS (EI) m/z : 446((M-43)⁺), 430, 414, 342, 326, 295, 281.

### (Example 1871)

### Compound of Formula No. C-1235

¹H-NMR (CDCl₃) δ(ppm) : 7.19 (1H, s, 3-H), 4.84 (2H, brs, NCH₂Ar), 4.76 (2H, brs, COCH₂O), 3.76 (2H, s, 1-NCH₂), 1.84 (3H, s, 5-CH₃).
MASS (EI) m/z : 460((M-43)⁺), 444, 429, 387, 356, 340, 325.

### (Example 1872)

### Compound of Formula No. D-1

¹H-NMR (CDCl₃) δ(ppm) : 4.93 (1H, d, J=14Hz, NCH₂Ar), 4.50 (1H, d, J=14Hz, NCH₂Ar), 3.70 (3H, s, NCH₃), 3.67 (2H, s, COCH₂O), 1.86 (3H, s, 3-CH₃), 1.72 (3H, s, 5-CH₃).
MASS (EI) m/z : 312(M⁺), 297, 267, 239, 196, 166.

### (Example 1873)

### Compound of Formula No. D-2

¹H-NMR (CDCl₃) δ(ppm) : 4.76 (1H, d, J=14Hz, NCH₂Ar), 4.52 (1H, d, J=14Hz, NCH₂Ar), 3.68 (3H, s, NCH₃), 3.67 (3H, brs, COOCH₃), 1.88 (3H, s, 3-CH₃), 1.78 (3H, s, 5-CH₃).
MASS (EI) m/z : 298(M⁺), 267, 239, 182, 150, 116.

### (Example 1874)

### Compound of Formula No. D-3

¹H-NMR (CDCl₃) δ(ppm) : 4.98 (1H, d, J=14Hz, NCH₂Ar), 4.45 (1H, d, J=14Hz, NCH₂Ar), 3.97 (2H, q, J=7Hz, NCH₂Me), 1.91 (3H, s, 3-CH₃), 1.82 (3H, s, COCH₃), 1.71 (3H, s, 5-CH₃).
MASS (EI) m/z : 296(M⁺), 253, 180, 152, 138, 124, 116.

### (Example 1875)

### Compound of Formula No. D-4

¹H-NMR (CDCl₃) δ(ppm) : 4.81 (1H, d, J=14Hz, NCH₂Ar), 4.49 (1H, d, J=14Hz, NCH₂Ar), 3.96 (2H, q, J=7Hz, NCH₂Me), 3.68 (3H, brs, COOCH₃), 1.91 (3H, s, 3-CH₃), 1.75 (3H, s, 5-CH₃).
MASS (EI) m/z : 312(M⁺), 279, 196, 164, 149, 116.

### (Example 1876)

### Compound of Formula No. D-5

¹H-NMR (CDCl₃) δ(ppm) : 5.02 (1H, d, J=14Hz, NCH₂Ar), 4.41 (1H, d, J=14Hz, NCH₂Ar), 3.88 (2H, t, J=7Hz, NCH₂Et), 1.92 (3H, s, 3-CH₃), 1.82 (3H, s, COCH₃), 1.68 (3H, s, 5-CH₃).
MASS (EI) m/z : 310(M⁺), 281, 267, 194, 152, 138, 116.

### (Example 1877)

### Compound of Formula No. D-6

¹H-NMR (CDCl₃) δ(ppm) : 4.84 (1H, d, J=14Hz, NCH₂Ar), 4.46 (1H, d, J=14Hz, NCH₂Ar), 3.86 (1H, td, J=7Hz, 3Hz, NCH₂Et), 3.68 (3H, brs, COOCH₃), 1.92 (3H, s, 3-CH₃, 1.72 (3H, s, 5-CH₃).
MASS (EI) m/z : 326(M⁺), 297, 267, 210, 168.

### (Example 1878)

### Compound of Formula No. D-7

¹H-NMR (CDCl₃) δ(ppm) : 5.01 (1H, d, J=14Hz, NCH₂Ar), 4.41 (1H, d, J=14Hz, NCH₂Ar), 4.26 (1H, hept, J=7Hz, NCHMe₂), 1.93 (3H, s, 3-CH₃), 1.81 (3H, s, COCH₃), 1.68 (3H, s, 5-CH₃).
MASS (EI) m/z : 310(M⁺), 295, 267, 225, 194, 152, 138, 123.

### (Example 1879)

### Compound of Formula No. D-8

¹H-NMR (CDCl₃) δ(ppm) : 4.83 (1H, d, J=14Hz, NCH₂Ar), 4.46 (1H, d, J=14Hz, NCH₂Ar), 4.26 (1H, hept, J=7Hz, NCHMe₂), 3.67 (3H, brs, COOCH₃), 1.94 (3H, s, 3-CH₃), 1.72 (3H, s, 5-CH₃).
MASS (EI) m/z : 326(M⁺), 311, 210, 168, 136.

### (Example 1880)

### Compound of Formula No. D-9

¹H-NMR (CDCl₃) δ(ppm) : 5.02 (1H, d, J=14Hz, NCH₂Ar), 4.58 (2H, td, J=5Hz, 2Hz, NCH₂CH=CH₂), 4.42 (1H, d, J=14Hz, NCH₂Ar), 1.93 (3H, s, 3-CH₃), 1.83 (3H, s, COCH₃), 1.67 (3H, s, 5-CH₃).
MASS (EI) m/z : 308(M⁺), 265, 192, 164, 150, 136, 116.

### (Example 1881)

### Compound of Formula No. D-10

¹H-NMR (CDCl₃) δ(ppm) : 4.84 (1H, d, J=14Hz, NCH₂Ar), 4.56 (2H, td, J=5Hz, 2Hz, NCH₂CH=CH₂), 4.48 (1H, d, J=14Hz, NCH₂Ar), 3.68 (3H, brs, COOCH₃), 1.93 (3H, s, 3-CH₃), 1.71 (3H, s, 5-CH₃). MASS (EI) m/z : 324(M⁺), 208, 176, 149, 116.

### (Example 1882)

### Compound of Formula No. D-11

¹H-NMR (CDCl₃) δ(ppm) : 5.03 (1H, d, J=14Hz, NCH₂Ar), 4.74 (2H, d, J=3Hz, NCH₂CCH), 4.39 (1H, d, J=14Hz, NCH₂Ar), 1.94 (3H, s, 3-CH₃), 1.83 (3H, s, COCH₃), 1.77 (3H, s, 5-CH₃).
MASS (EI) m/z : 306(M⁺), 263, 225, 190, 157, 148, 116.

### (Example 1883)

### Compound of Formula No. D-12

¹H-NMR (CDCl₃) δ(ppm) : 4.83 (1H, d, J=14Hz, NCH₂Ar), 4.73 (2H, d, J=3Hz, NCH₂CCH), 4.46 (1H, d, J=14Hz, NCH₂Ar), 3.68 (3H, brs, COOCH₃), 1.92 (3H, s, 3-CH₃), 1.81 (3H, s, 5-CH₃).
MASS (EI) m/z : 322(M⁺), 263, 206, 174, 146, 116.

### (Example 1884)

### Compound of Formula No. D-13

¹H-NMR (CDCl₃) δ(ppm) : 5.01 (1H, d, J=14Hz, NCH₂Ar), 4.42 (1H, d, J=14Hz, NCH₂Ar), 3.90 (2H, t, J=7Hz, NCH₂-Pr), 1.92 (3H, s, 3-CH₃), 1.82 (3H, s, COCH₃), 1.69 (3H, s, 5-CH₃).
MASS (EI) m/z : 324(M⁺), 309, 281, 268, 254, 239, 208, 166.

### (Example 1885)

### Compound of Formula No. D-14

¹H-NMR (CDCl₃) δ(ppm) : 4.84 (1H, d, J=14Hz, NCH₂Ar), 4.45 (1H, d, J=14Hz, NCH₂Ar), 3.89 (2H, t, J=7Hz, NCH₂-Pr), 3.88 (2H, t, J=7Hz, NCH₂-Pr), 3.68 (3H, brs, COOCH₃), 1.92 (3H, s, 3-CH₃), 1.71 (3H, s, 5-CH₃).
MASS (EI) m/z : 340(M⁺), 325, 297, 284, 224, 192, 182, 168.

### (Example 1886)

### Compound of Formula No. D-15

¹H-NMR (CDCl₃) δ(ppm) : [5.10 (d, J=14Hz) & 5.01 (d, J=14Hz), 1H, NCH₂Ar], [4.42 (d, J=14Hz) & 4.35 (d, J=14Hz), 1H, NCH₂Ar], 4.40-3.85 (1H, m, NCH(Me)Et), [1.95 (s) & 1.94 (s), 3H, 3-CH₃], [1.82 (s) & 1.81 (s), 3H, COCH₃], [1.68 (s) & 1.63 (s) , 3H, 5-CH₃].
MASS (EI) m/z : 324(M⁺), 309, 295, 281, 268, 225, 208.

### (Example 1887)

### Compound of Formula No. D-16

¹H-NMR (CDCl₃) δ(ppm) : [4.89 (d, J=14Hz) & 4.85 (d, J=14Hz), 1H, NCH₂Ar], [4.45 (d, J=14Hz) & 4.42 (d, J=14Hz), 1H, NCH₂Ar], 4.04-3.77 (1H, m, NCH(Me)Et), [3.67 (brs) & 3.66 (brs), 3H, COOCH₃], 1.94 (3H, s, 3-CH₃), [1.70 (s) & 1.67 (s), 3H, 5-CH₃]. MASS (EI) m/z : 340(M⁺), 325, 311, 297, 284, 224, 168.

### (Example 1888)

### Compound of Formula No. D-17

¹H-NMR (CDCl₃) δ(ppm) : 5.04 (1H, d, J=14Hz, NCH₂Ar), 4.29 (1H, d, J=14Hz, NCH₂Ar), 3.71 (1H, dd, J=14Hz, 7Hz, NCH₂-iPr), 3.65 (1H, dd, J=14Hz, 7Hz, NCH₂-iPr), 1.94 (3H, s, 3-CH₃), 1.81 (3H, s, COCH₃), 1.65 (3H, s, 5-CH₃).
MASS (EI) m/z : 333(M⁺), 290, 208.

### (Example 1889)

### Compound of Formula No. D-18

¹H-NMR (CDCl₃) δ(ppm) : 4.84 (1H, d, J=14Hz, NCH₂Ar), 4.35 (1H, d, J=14Hz, NCH₂Ar), 3.73 (1H, dd, J=14Hz, 7Hz, NCH₂-iPr), 3.66 (3H, s, COOCH₃), 3.60 (1H, dd, J=14Hz, 7Hz, NCH₂-iPr), 1.94 (3H, s, 3-CH₃), 1.68 (3H, s, 5-CH₃).
MASS (EI) m/z : 349(M⁺), 306, 293, 224.

### (Example 1890)

### Compound of Formula No. D-19

¹H-NMR (CDCl₃) δ(ppm) : 5.06 (1H, d, J=14Hz, NCH₂Ar), 4.38 (1H, d, J=14Hz, NCH₂Ar), 3.71 (1H, dd, J=14Hz, 7Hz, NCH₂-iPr), 3.68 (1H, dd, J=14Hz, 7Hz, NCH₂-iPr), 1.93 (3H, s, 3-CH₃), 1.82 (3H, s, COCH₃), 1.66 (3H, s, 5-CH₃).
MASS (EI) m/z : 324(M⁺), 309, 281, 268, 239, 226, 208, 166.

### (Example 1891)

### Compound of Formula No. D-20

¹H-NMR (CDCl₃) δ(ppm) : 5.08 (1H, d, J=14Hz, NCH₂Ar), 4.46 (1H, d, J=14Hz, NCH₂Ar), 3.72 (1H, dd, J=16H, 5Hz, COCH₂O), 3.68 (1H, dd, J=16Hz, 5Hz, COCH₂O), 3.69 (2H, d, J=7Hz, NCH₂-iPr), 1.90 (3H, s, 3-CH₃), 1.64 (3H, s, 5-CH₃).
MASS (EI) m/z : 340(M⁺), 325, 297, 281, 194.

### (Example 1892)

### Compound of Formula No. D-21

¹H-NMR (CDCl₃) δ(ppm) : 5.09 (1H, d, J=14Hz, NCH₂Ar), 4.43 (1H, d, J=14Hz, NCH₂Ar), 4.29 (1H, d, J=15Hz, COCH₂O), 4.28 (1H, d, J=15Hz, COCH₂O), 3.70 (2H, d, J=7Hz, NCH₂-iPr), 1.96 (3H, s, 3-CH₃), 1.69 (3H, s, 5-CH₃).
MASS (EI) m/z : 416(M⁺), 401, 323, 300, 107.

### (Example 1893)

### Compound of Formula No. D-22

¹H-NMR (CDCl₃) δ(ppm) : 5.06 (1H, d, J=14Hz, NCH₂Ar), 4.37 (1H, d, J=14Hz, NCH₂Ar), 3.88 (2H, d, J=3Hz, COCH₂O), 3.69 (2H, d, J=7Hz, NCH₂-iPr), 1.90 (3H, s, 3-CH₃), 1.62 (3H, s, 5-CH₃).
MASS (EI) m/z : 453(M-1), 439, 397, 341, 281, 238, 225, 116.

### (Example 1894)

### Compound of Formula No. D-23

¹H-NMR (CDCl₃) δ(ppm) : 5.07 (1H, d, J=14Hz, NCH₂Ar), 4.46 (2H, s, COCH₂O), 4.42 (1H, d, J=14Hz, NCH₂Ar), 3.72 (1H, dd, J=14Hz, 7Hz, NCH₂-iPr), 3.67 (1H, dd, J=14Hz, 7Hz, NCH₂-ipr), 1.97 (3H, s, 3-CH₃), 1.72 (3H, s, 5-CH₃).
MASS (EI) m/z : 421 (M⁺), 305, 280, 265, 224, 206, 150, 116.

### (Example 1895)

### Compound of Formula No. D-24

¹H-NMR (CDCl₃) δ(ppm) : 5.04 (1H, d, J=14Hz, NCH₂Ar), 4.41 (1H, d, J=14Hz, NCH₂Ar), 4.26 (1H, d, J=15Hz, COCH₂O), 4.25 (1H, d, J=15Hz, COCH₂O), 3.73 (1H, dd, J=14Hz, 7Hz, NCH₂-iPr), 3.67 (1H, dd, J=14Hz, 7Hz, NCH₂-iPr), 1.96 (3H, s, 3-CH₃), 1.71 (3H, s, 5-CH₃).
MASS (EI) m/z : 382(M⁺).

### (Example 1896)

### Compound of Formula No. D-25

¹H-NMR (CDCl₃) δ (ppm) : 5.07 (1H, d, J=14Hz, NCH₂Ar), 4.67 (1H, d, J=12Hz, COCH₂O), 4.58 (1H, d, J=12Hz, COCH₂O), 4.41 (1H, d, J=14Hz, NCH₂Ar), 3.72 (1H, dd, J=14Hz, 7Hz, NCH₂-iPr), 3.66 (1H, dd, J=14Hz, 7Hz, NCH₂-iPr), 1.89 (3H, s, 3-CH₃), 1.61 (3H, s, 5-CH₃).
MASS (EI) m/z : 455(M⁺).

### (Example 1897)

### Compound of Formula No. D-26

¹H-NMR (CDCl₃) δ(ppm) : 4.95 (1H, d, J=14Hz, NCH₂Ar), 4.58 (2H, s, CONH₂), 4.42 (1H, d, J=14Hz, NCH₂Ar), 3.71 (1H, dd, J=14Hz, 7Hz, NCH₂-iPr), 3.67 (1H, dd, J=14Hz, 7Hz, NCH₂-iPr), 1.96 (3H, s, 3-CH₃), 1.71 (3H, s, 5-CH₃).
MASS (EI) m/z : 325(M⁺), 310, 282, 209, 192, 166, 116.

### (Example 1898)

### Compound of Formula No. D-27

¹H-NMR (CDCl₃) δ(ppm) : 5.04 (1H, d, J=14Hz, NCH₂Ar), 4.59 (2H, s, COCH₂N), 4.44 (1H, d, J=14Hz, NCH₂Ar), 3.74 (1H, dd, J=14Hz, 7Hz, NCH₂-iPr), 3.70 (1H, dd, J=14Hz, 7Hz, NCH₂-iPr), 1.98 (3H, s, 3-CH₃), 1.72 (3H, s, 5-CH₃).
MASS (EI) m/z : 390(M⁺), 280, 224.

### (Example 1899)

### Compound of Formula No. D-28

¹H-NMR (CDCl₃) δ(ppm) : 5.03 (1H, d, J=14Hz, NCH₂Ar), 4.52 (2H, s, COCH₂N), 4.44 (1H, d, J=14Hz, NCH₂Ar), 3.74 (1H, dd, J=14Hz, 7Hz, NCH₂-iPr), 3.70 (1H, dd, J=14Hz, 7Hz, NCH₂-iPr), 1.97 (3H, s, 3-CH₃), 1.71 (3H, s, 5-CH₃).
MASS (EI) m/z : 424(M⁺), 308, 281, 115.

### (Example 1900)

### Compound of Formula No. D-29

¹H-NMR (CDCl₃) δ(ppm) : 4.87 (1H, d, J=14Hz, NCH₂Ar), 4.41 (1H, d, J=14Hz, NCH₂Ar), 3.71 (1H, dd, J=14Hz, 7Hz, NCH₂-iPr), 3.66 (3H, s, COOCH₃), 3.62 (1H, dd, J=14Hz, 7Hz, NCH₂-iPr), 1.91 (3H, s, 3-CH₃), 1.67 (3H, s, 5-CH₃).
MASS (EI) m/z : 340(M⁺), 325, 297, 284, 224, 182, 168, 150, 136.

### (Example 1901)

### Compound of Formula No. D-30

¹H-NMR (CDCl₃) δ(ppm) : 5.02 (1H, d, J=13Hz, NCH₂Ar), 4.16 (1H, d, J=13Hz, NCH₂Ar), 3.71 (1H, dd, J=14Hz, 7Hz, NCH₂-iPr), 3.65 (1H, dd, J=14Hz, 7Hz, NCH₂-iPr), 1.95 (3H, s, 3-CH₃), 1.80 (3H, s, COCH₃), 1.64 (3H, s, 5-CH₃).
MASS (EI) m/z : 314 (M⁺), 271, 208, 153.

### (Example 1902)

### Compound of Formula No. D-31

¹H-NMR (CDCl₃) δ(ppm) : 4.81 (1H, d, J=14Hz, NCH₂Ar), 4.23 (1H, d, J=14Hz, NCH₂Ar), 3.72 (1H, dd, J=14Hz, 7Hz, NCH₂-iPr), 3.65 (3H, s, COOCH₃), 3.60 (1H, dd, J=14Hz, 7Hz, NCH₂-iPr), 1.95 (3H, s, 3-CH₃), 1.67 (3H, s, 5-CH₃).
MASS (EI) m/z : 330(M⁺), 315, 287, 224.

### (Example 1903)

### Compound of Formula No. D-32

¹H-NMR (CDCl₃) δ(ppm) : 5.07 (1H, d, J=14Hz, NCH₂Ar), 4.25 (1H, d, J=14Hz, NCH₂Ar), 3.70 (1H, dd, J=14Hz, 7Hz, NCH₂-iPr), 3.64 (1H, dd, J=14Hz, 7Hz, NCH₂-iPr), 1.94 (3H, s, 3-CH₃), 1.81 (3H, s, COCH₃), 1.63 (3H, s, 5-CH₃).
MASS (EI) m/z : 372(M⁺), 340, 329, 208.

### (Example 1904)

### Compound of Formula No. D-33

¹H-NMR (CDCl₃) δ(ppm) : 4.86 (1H, d, J=14Hz, NCH₂Ar), 4.33 (1H, d, J=14Hz, NCH₂Ar), 3.71 (1H, dd, J=14Hz, 7Hz, NCH₂-iPr), 3.66 (3H, s, COOCH₃), 3.60 (1H, dd, J=14Hz, 7Hz, NCH₂-ipr), 1.94 (3H, s, 3-CH₃), 1.66 (3H, s, 5-CH₃).
MASS (EI) m/z 388(M⁺), 356, 332, 224.

### (Example 1905)

### Compound of Formula No. D-34

¹H-NMR (CDCl₃) δ(ppm) : 4.80 (1H, d, J=14Hz, NCH₂Ar), 4.34 (1H, d, J=14Hz, NCH₂Ar), 3.70 (1H, dd, J=14Hz, 7Hz, NCH₂-iPr), 3.65 (3H, s, COOCH₃), 3.60 (1H, dd, J=14Hz, 7Hz, NCH₂-iPr), 1.93 (3H, s, 3-CH₃), 1.68 (3H, s, 5-CH₃).
MASS (EI) m/z : 401(M⁺), 356, 330, 224.

### (Example 1906)

### Compound of Formula No. D-35

¹H-NMR (CDCl₃) δ(ppm) : 4.73 (1H, d, J=14Hz, NCH₂Ar), 4.51 (1H, d, J=14Hz, NCH₂Ar), 3.76 (3H, s, COOCH₃), 3.73 (1H, dd, J=14Hz, 7Hz, NCH₂-iPr), 3.64 (1H, dd, J=14Hz, 7Hz, NCH₂-iPr), 1.89 (3H, s, 3-CH₃), 1.76 (3H, s, 5-CH₃).
MASS (EI) m/z : 408(M⁺), 393, 365, 224.

### (Example 1907)

### Compound of Formula No. D-36

¹H-NMR (CDCl₃) δ(ppm) : 5.10 (1H, d, J=14Hz, NCH₂Ar), 4.50 (1H, d, J=14Hz, NCH₂Ar), 3.72 (1H, dd, J=14Hz, 7Hz, NCH₂-iPr), 3.66 (1H, dd, J=14Hz, 7Hz, NCH₂-iPr), 1.94 (3H, s, 3-CH₃), 1.84 (3H, s, COCH₃), 1.71 (3H, s, 5-CH₃).
MASS (EI) m/z : 344(M⁺), 329, 301, 149.

### (Example 1908)

### Compound of Formula No. D-37

¹H-NMR (CDCl₃) δ (ppm) : 4.94 (1H, d, J=14Hz, NCH₂Ar), 4.55 (1H, d, J=14Hz, NCH₂Ar), 3.73 (1H, dd, J=14Hz, 7Hz, NCH₂-iPr), 3.68 (3H, s, COOCH₃), 3.62 (1H, dd, J=14Hz, 7Hz, NCH₂-iPr), 1.95 (3H, s, 3-CH₃), 1.75 (3H, s, 5-CH₃).
MASS (EI) m/z : 360(M⁺), 345, 317, 224.

### (Example 1909)

### Compound of Formula No. D-38

¹H-NMR (CDCl₃) δ(ppm) : 5.09 (1H, d, J=14Hz, NCH₂Ar), 4.32 (1H, d, J=14Hz, NCH₂Ar), 3.68 (2H, d, J=7Hz, NCH₂-iPr), 1.93 (3H, s, 3-CH₃), 1.82 (3H, s, COCH₃), 1.63 (3H, s, 5-CH₃).
MASS (EI) m/z : 383(M⁺), 368, 340, 208.

### (Example 1910)

### Compound of Formula No. D-39

¹H-NMR (CDCl₃) δ(ppm) : 4.90 (1H, d, J=14Hz, NCH₂Ar), 4.39 (1H, d, J=14Hz, NCH₂Ar), 3.71 (1H, dd, J=14Hz, 7Hz, NCH₂-ipr), 3.67 (3H, s, COOCH₃), 3.61 (1H, dd, J=14Hz, 7Hz, NCH₂-ipr), 1.93 (3H, s, 3-CH₃), 1.67 (3H, s, 5-CH₃).
MASS (EI) m/z : 399(M⁺), 384, 356, 343, 224.

### (Example 1911)

### Compound of Formula No. D-40

¹H-NMR (CDCl₃) δ(ppm) : 5.06 (1H, d, J=14Hz, NCH₂Ar), 4.29 (1H, d, J=14Hz, NCH₂Ar), 3.62 (2H, d, J=7Hz, NCH₂-iPr), 1.87 (3H, s, 3-CH₃), 1.77 (3H, s, COCH₃), 1.57 (3H, s, 5-CH₃).
MASS (EI) m/z : 399(M⁺), 356, 229, 208.

### (Example 1912)

### Compound of Formula No. D-41

¹H-NMR (CDCl₃) δ(ppm) : 4.91 (1H, d, J=14Hz, NCH₂Ar), 4.41 (1H, d, J=14Hz, NCH₂Ar), 3.70 (1H, dd, J=14Hz, 7Hz, NCH₂-iPr), 3.67 (3H, s, COOCH₃), 3.61 (1H, dd, J=14Hz, 7Hz, NCH₂-iPr), 1.92 (3H, s, 3-CH₃), 1.65 (3H, s, 5-CH₃).
MASS (EI) m/z : 415(M⁺), 372, 359, 224.

### (Example 1913)

### Compound of Formula No. D-42

¹H-NMR (CDCl₃) δ(ppm) : 5.05 (1H, d, J=14Hz, NCH₂Ar), 4.23 (1H, d, J=14Hz, NCH₂Ar), 3.73 (1H, dd, J=14Hz, 7Hz, NCH₂-iPr), 3.65 (1H, dd, J=14Hz, 7Hz, NCH₂-iPr), 1.96 (3H, s, 3-CH₃), 1.81 (3H, s, COCH₃), 1.66 (3H, s, 5-CH₃).
MASS (EI) m/z : 395(M⁺), 354, 352, 208.

### (Example 1914)

### Compound of Formula No. D-43

¹H-NMR (CDCl₃) δ(ppm) : 5.09 (1H, d, J=14Hz, NCH₂Ar), 4.29 (1H, d, J=15Hz, COCH₂O), 4.27 (1H, d, J=14Hz, NCH₂Ar), 4.25 (1H, d, J=15Hz, COCH₂O), 3.73 (1H, dd, J=14Hz, 7Hz, NCH₂-iPr), 3.67 (1H, dd, J=14Hz, 7Hz, NCH₂-iPr), 2.00 (3H, s, 3-CH₃), 1.69 (3H, s, 5-CH₃).
MASS (EI) m/z : 487(M⁺), 300, 265.

### (Example 1915)

### Compound of Formula No. D-44

¹H-NMR (CDCl₃) δ(ppm) : 4.85 (1H, d, J=14Hz, NCH₂Ar), 4.30 (1H, d, J=14Hz, NCH₂Ar), 3.74 (1H, dd, J=14Hz, 7Hz, NCH₂-iPr), 3.67 (3H, s, COOCH₃), 3.61 (1H, dd, J=14Hz, 7Hz, NCH₂-iPr), 1.96 (3H, s, 3-CH₃), 1.69 (3H, s, 5-CH₃).
MASS (EI) m/z : 411(M⁺), 357, 355, 224.

### (Example 1916)

### Compound of Formula No. D-45

¹H-NMR (CDCl₃) δ(ppm) : 4.83 (1H, d, J=14Hz, NCH₂Ar), 4.40 (1H, d, J=14Hz, NCH₂Ar), 3.74 (1H, dd, J=14Hz, 7Hz, NCH₂-iPr), 3.67 (3H, s, COOCH₃), 3.63 (1H, dd, J=14Hz, 7Hz, NCH₂-iPr), 1.94 (3H, s, 3-CH₃), 1.72 (3H, s, 5-CH₃).
MASS (EI) m/z : 358(M⁺), 343, 315, 302, 224.

### (Example 1917)

### Compound of Formula No. D-46

¹H-NMR (CDCl₃) δ(ppm) : 5.03 (1H, d, J=14Hz, NCH₂Ar), 4.41 (1H, d, J=14Hz, NCH₂Ar), 4.29 (1H, d, J=15Hz, COCH₂O), 4.27 (1H, d, J=15Hz, COCH₂O), 3.71 (2H, d, J=7Hz, NCH₂-iPr), 1.97 (3H, s, 3-CH₃), 1.73 (3H, s, 5-CH₃).
MASS (EI) m/z : 434(M⁺), 341, 300, 107.

### (Example 1918)

### Compound of Formula No. D-47

¹H-NMR (CDCl₃) δ(ppm) : 5.12 (1H, d, J=14Hz, NCH₂Ar), 4.78 (1H, d, J=14Hz, NCH₂Ar), 3.72 (1H, dd, J=14Hz, 7Hz, NCH₂-ipr), 3.66 (1H, dd, J=14Hz, 7Hz, NCH₂-ipr), 1.94 (3H, s, 3-CH₃), 1.86 (3H, s, COCH₃), 1.79 (3H, s, 5-CH₃).
MASS (EI) m/z : 402(M⁺), 361, 359, 323, 208.

### (Example 1919)

### Compound of Formula No. D-48

¹H-NMR (CDCl₃) δ(ppm) : 5.00 (1H, d, J=15Hz, NCH₂Ar), 4.76 (1H, d, J=15Hz, NCH₂Ar), 3.71 (3H, s, COOCH₃), 3.78-3.56 (2H, m, NCH₂-iPr), 1.96 (3H, s, 3-CH₃), 1.82 (3H, s, 5-CH₃).
MASS (EI) m/z : 418(M⁺), 377, 375, 346, 362, 339, 224.

### (Example 1920)

### Compound of Formula No. D-49

¹H-NMR (CDCl₃) δ(ppm) : 4.83 (1H, d, J=14Hz, NCH₂Ar), 4.26 (1H, d, J=14Hz, NCH₂Ar), 3.73 (1H, dd, J=14Hz, 7Hz, NCH₂-iPr), 3.66 (3H, s, COOCH₃), 3.61 (1H, dd, J=14Hz, 7Hz, NCH₂-iPr), 1.95 (3H, s, 3-CH₃), 1.67 (3H, s, 5-CH₃).
MASS (EI) m/z : 423(M⁺), 224, 201, 199.

### (Example 1921)

### Compound of Formula No. D-50

¹H-NMR (CDCl₃) δ(ppm) : 4.99 (1H, d, J=14Hz, NCH₂Ar), 4.57 (1H, d, J=14Hz, NCH₂Ar), 3.69 (1H, dd, J=14Hz, 7Hz, NCH₂-iPr), 3.62 (1H, dd, J=14Hz, 7Hz, MCH₂-iPr), 1.96 (3H, s, 3-CH₃), 1.82 (3H, s, COCH₃), 1.78 (3H, s, 5-CH₃).
MASS (EI) m/z : 407(M⁺), 366, 364, 208.

### (Example 1922)

### Compound of Formula No. D-51

¹H-NMR (CDCl₃) δ(ppm) : 4.86 (1H, d, J=14Hz, NCH₂Ar), 4.58 (1H, d, J=14Hz, NCH₂Ar), 3.71 (1H, dd, J=14Hz, 7Hz, NCH₂-ipr), 3.65 (3H, s, COOCH₃), 3.60 (1H, dd, J=14Hz, 7Hz, NCH₂-iPr), 1.97 (3H, s, 3-CH₃), 1.80 (3H, s, 5-CH₃).
MASS (EI) m/z : 423(M⁺), 224.

### (Example 1923)

### Compound of Formula No. D-52

¹H-NMR (CDCl₃) δ(ppm) : 5.03 (1H, d, J=14Hz, NCH₂Ar), 4.57 (1H, d, J=14Hz, NCH₂Ar), 3.65 (2H, d, J=7Hz, NCH₂-iPr), 1.97 (3H, s, 3-CH₃), 1.82 (3H, s, COCH₃), 1.75 (3H, s, 5-CH₃).
MASS (EI) m/z : 421(M⁺), 380, 378, 208.

### (Example 1924)

### Compound of Formula No. D-53

¹H-NMR (CDCl₃) δ(ppm) : 4.89 (1H, d, J=14Hz, NCH₂Ar), 4.58 (1H, d, J=14Hz, NCH₂Ar), 3.71 (1H, dd, J=14Hz, 7Hz, NCH₂-iPr), 3.67 (3H, s, COOCH₃), 3.58 (1H, dd, J=14Hz, 7Hz, NCH₂-iPr), 1.98 (3H, s, 3-CH₃), 1.77 (3H, s, 5-CH₃).
MASS (EI) m/z : 437(M⁺), 224.

### (Example 1925)

### Compound of Formula No. D-54

¹H-NMR (CDCl₃) δ(ppm) : 4.85 (1H, d, J=14Hz, NCH₂Ar), 4.37 (1H, d, J=14Hz, NCH₂Ar), 3.74 (1H, dd, J=14Hz, 7Hz, NCH₂-iPr), 3.68 (3H, s, COOCH₃), 3.61 (1H, dd, J=14Hz, 7Hz, NCH₂-iPr), 1.94 (3H, s, 3-CH₃), 1.69 (3H, s, 5-CH₃).
MASS (EI) m/z : 354(M⁺), 224, 126.

### (Example 1926)

### Compound of Formula No. D-55

¹H-NMR (CDCl₃) δ(ppm) : 4.89 (1H, d, J=14Hz, NCH₂Ar), 4.39 (1H, d, J=14Hz, NCH₂Ar), 3.73 (1H, dd, J=14Hz, 7Hz, NCH₂-iPr), 3.68 (3H, s, COOCH₃), 3.63 (1H, dd, J=14Hz, 7Hz, NCH₂-iPr), 1.95 (3H, s, 3-CH₃), 1.71 (3H, S, 5-CH₃).
MASS (EI) m/z : 411(M⁺), 357, 355, 224.

### (Example 1927)

### Compound of Formula No. D-56

¹H-NMR (CDCl₃) δ(ppm) : 5.21 (1H, d, J=14Hz, NCH₂Ar), 4.81 (1H, d, J=14Hz, NCH₂Ar), 3.73 (2H, d, J=7Hz, NCH₂-iPr), 1.95 (3H, s, 3-CH₃), 1.87 (3H, s, COCH₃), 1.74 (3H, s, 5-CH₃).
MASS (EI) m/z : 349(M⁺), 334, 323, 306, 208.

### (Example 1928)

### Compound of Formula No. D-57

¹H-NMR (CDCl₃) δ(ppm) : 5.07 (2H, s, NCH₂Ar), 3.71 (3H, s, COOCH₃), 3.69 (1H, d, J=7Hz, NCH₂-iPr), 1.92 (3H, s, 3-CH₃), 1.90 (3H, s, 5-CH₃).
MASS (EI) m/z : 385(M⁺), 370, 342, 329, 224.

### (Example 1929)

### Compound of Formula No. D-58

¹H-NMR (CDCl₃) δ(ppm) : 4.79 (2H, d, J=14Hz, NCH₂Ar), 4.41 (2H, d, J=14Hz, NCH₂Ar), 3.72 (2H, dd, J=14Hz, 7Hz, NCH₂-iPr), 3.67 (6H, s, COOCH₃), 3.62 (2H, dd, J=14Hz, 7Hz, NCH₂-iPr), 1.91 (3H, s, 3-CH₃), 1.71 (3H, s, 5-CH₃).
MASS (EI) m/z : 577(M⁺), 562, 534, 478, 224.

### (Example 1930)

### Compound of Formula No. D-59

¹H-NMR (CDCl₃) δ(ppm) : 4.96 (1H, d, J=14Hz, NCH₂Ar), 4.64 (1H, d, J=14Hz, NCH₂Ar), 3.70 (1H, dd, J=14Hz, 7Hz, NCH₂-iPr), 3.64 (1H, dd, J=14Hz, 7Hz, NCH₂-iPr), 1.94 (3H, s, 3-CH₃), 1.83 (3H, s, COCH₃), 1.80 (3H, s, 5-CH₃).
MASS (EI) m/z : 355 (M+1), 311, 208.

### (Example 1931)

### Compound of Formula No. D-61

¹H-NMR (CDCl₃) δ(ppm) : 5.00 (1H, d, J=14Hz, NCH₂Ar), 4.65 (1H, d, J=14Hz, NCH₂Ar), 3.67 (2H, d, J=7Hz, NCH₂-iPr), 1.95 (3H, s, 3-CH₃), 1.83 (3H, s, COCH₃), 1.77 (3H, s, 5-CH₃).
MASS (EI) m/z : 368(M⁺), 353, 325, 208.

### (Example 1932)

### Compound of Formula No. D-62

¹H-NMR (CDCl₃) δ(ppm) : 4.88 (1H, d, J=14Hz, NCH₂Ar), 4.63 (1H, d, J=14Hz, NCH₂Ar), 3.75-3.50 (2H, m, NCH₂-iPr), 3.68 (3H, s, COOCH₃), 1.96 (3H, s, 3-CH₃), 1.78 (3H, s, 5-CH₃).
MASS (EI) m/z : 384(M⁺), 224.

### (Example 1933)

### Compound of Formula No. D-66

¹H-NMR (CDCl₃) δ(ppm) : 4.87 (1H, d, J=14Hz, NCH₂Ar); 4.55 (1H, d, J=14Hz, NCH₂Ar), 3.75 (1H, dd, J=14Hz, 7Hz, NCH₂-iPr), 3.65 (1H, dd, J=14Hz, 7Hz, NCH₂-iPr), 1.94 (3H, s, 3-CH₃), 1.84 (3H, s, COCH₃), 1.81 (3H, s, 5-CH₃).
MASS (EI) m/z : 385(M⁺), 208, 177.

### (Example 1934)

### Compound of Formula No. D-67

¹H-NMR (CDCl₃) δ(ppm) : 4.91 (1H, d, J=14Hz, NCH₂Ar), 4.58 (1H, d, J=14Hz, NCH₂Ar), 4.28 (1H, d, J=15Hz, COCH₂O), 4.27 (1H, d, J=15Hz, COCH₂O), 3.75 (1H, dd, J=14Hz, 8Hz, NCH₂-iPr), 3.67 (1H, dd, J=14Hz, 8Hz, NCH₂-iPr), 1.99 (3H, s, 3-CH₃), 1.87 (3H, s, 5-CH₃).
MASS (EI) m/z : 477(M⁺), 342, 177.

### (Example 1935)

### Compound of Formula No. D-68

¹H-NMR (CDCl₃) δ(ppm) : 4.76 (1H, d, J=14Hz, NCH₂Ar), 4.54 (1H, d, J=14Hz, NCH₂Ar), 3.75 (1H, dd, J=14Hz, 7Hz, NCH₂-iPr), 3.66 (3H, s, COOCH₃), 3.61 (1H, dd, J=14Hz, 7Hz, NCH₂-iPr), 1.93 (3H, s, 3-CH₃), 1.84 (3H, s, 5-CH₃).
MASS (EI) m/z : 401(M⁺), 224, 177.

### (Example 1936)

### Compound of Formula No. D-69

¹H-NMR (CDCl₃) δ(ppm) : 4.94 (1H, d, J=14Hz, NCH₂Ar), 4.48 (1H, d, J=14Hz, NCH₂Ar), 1.89 (3H, s, 3-CH₃), 1.84 (3H, s, COCH₃), 1.81 (3H, s, 5-CH₃), 1.57 (9H, s, NC(CH₃)₃).
MASS (EI) m/z : 324(M⁺), 309, 226, 208, 152.

### (Example 1937)

### Compound of Formula No. D-70

¹H-NMR (CDCl₃) δ(ppm) : 5.04 (1H, d, J=14Hz, NCH₂Ar), 4.41 (1H, d, J=14Hz, NCH₂Ar), 3.84 (1H, dd, J=14Hz, 7Hz, NCH₂-cPr), 3.81 (1H, dd, J=14Hz, 7Hz, NCH₂-cPr), 1.93 (3H, s, 3-CH₃), 1.83 (3H, s, COCH₃), 1.69 (3H, s, 5-CH₃).
MASS (EI) m/z : 322(M⁺), 279, 225, 206, 164, 150, 135, 123.

### (Example 1938)

### Compound of Formula No. D-71

¹H-NMR (CDCl₃) δ(ppm) : 4.86 (1H, d, J=14Hz, NCH₂Ar), 4.45 (1H, d, J=14Hz, NCH₂Ar), 3.82 (1H, dd, J=14Hz, 7Hz, NCH₂-cPr), 3.97 (1H, dd, J=14Hz, 7Hz, NCH₂-cPr), 3.68 (3H, brs, COOCH₃), 1.93 (3H, s, 3-CH₃), 1.73 (3H, s, 5-CH₃).
MASS (EI) m/z : 338(M⁺), 351, 323, 293, 281, 250, 166, 116.

### (Example 1939)

### Compound of Formula No. D-72

¹H-NMR (CDCl₃) δ(ppm) : 5.07 (1H, d, J=14Hz, NCH₂Ar), 4.48 (2H, s, NCH₂C₃H₅), 4.39 (1H, d, J=14Hz, NCH₂Ar), 1.94 (3H, s, 3-CH₃), 1.83 (3H, s, COCH₃), 1.64 (3H, s, 5-CH₃).
MASS (EI) m/z : 322(M⁺), 279, 206, 164, 149, 135, 116.

### (Example 1940)

### Compound of Formula No. D-73

¹H-NMR (CDCl₃) δ(ppm) : 4.88 (1H, d, J=14Hz, NCH₂Ar), 4.55-4.42 (2H, m, NCH₂-C₃H₅), 4.45 (1H, d, J=14Hz, NCH₂Ar), 3.68 (3H, brs, COOCH₃), 1.93 (3H, S, 3-CH₃), 1.67 (3H, s, 5-CH₃).
MASS (EI) m/z : 338(M⁺), 323, 279, 222, 190, 162, 116.

### (Example 1941)

### Compound of Formula No. D-74

¹H-NMR (CDCl₃) δ(ppm) : [4.99 (d, J=14Hz) & 4.97 (d, J=14Hz), 1H, NCH₂Ar], 4.43 (1H, d, J=14Hz, NCH₂Ar), 4.63-4.48 (2H, m, NCH₂-C₃H₅), [1.92 (s) & 1.91 (s), 3H, 3-CH₃], [1.83 (s) & 1.82 (s), 3H, COCH₃], [1.69 (s) & 1.68 (s), 3H, 5-CH₃].
MASS (EI) m/z : 322(M⁺), 307, 279, 225, 206, 164, 152.

### (Example 1942)

### Compound of Formula No. D-75

¹H-NMR (CDCl₃) δ(ppm) : [4.82 (d, J=14Hz) & 4.80 (d, J=14Hz), 1H, NCH₂Ar], [4.60 (brd, J=7Hz) & 4.49 (brd, J=7Hz), 2H, NCH₂-C₃H₅], [4.48 (d, J=14Hz) & 4.46 (d, J=14Hz), 1H, NCH₂Ar], 3.68 (3H, brs, COOCH₃), 1.92 (3H, s, 3-CH₃), 1.72 (3H, s, 5-CH₃). MASS (EI) m/z : 338(M⁺), 323, 284, 222, 190, 168, 116.

### (Example 1943)

### Compound of Formula No. D-76

¹H-NMR (CDCl₃) δ(ppm) : 5.04 (1H, d, J=14Hz, NCH₂Ar), 4.39 (1H, d, J=14Hz, NCH₂Ar), 3.92 (2H, d, J=7Hz, NCH₂-cBu), 1.92 (3H, s, 3-CH₃), 1.81 (3H, s, COCH₃), 1.68 (3H, s, 5-CH₃).
MASS (EI) m/z : 336(M⁺), 321, 308, 293, 281, 268, 226.

### (Example 1944)

### Compound of Formula No. D-78

¹H-NMR (CDCl₃) δ(ppm) : 5.08 (1H, d, J=14Hz, NCH₂Ar), 4.38 (1H, d, J=14Hz, NCH₂Ar), 3.69 (2H, s, NCH₂-tBu), 1.93 (3H, s, 3-CH₃), 1.82 (3H, s, COCH₃), 1.66 (3H, s, 5-CH₃).
MASS (EI) m/z : 338(M⁺), 323, 307, 293, 281, 222, 167, 149.

### (Example 1945)

### Compound of Formula No. D-79

¹H-NMR (CDCl₃) δ(ppm) : 4.91 (1H, d, J=14Hz, NCH₂Ar), 4.42 (1H, d, J=14Hz, NCH₂Ar), 3.69 (1H, d, J=14Hz, NCH₂-tBu), 3.68 (3H, brs, COOCH₃), 3.65 (1H, d, J=14Hz, NCH₂-tBu), 1.94 (3H, s, 3-CH₃), 1.68 (3H, s, 5-CH₃).
MASS (EI) m/z : 354(M⁺), 339, 297, 238, 182, 168.

### (Example 1946)

### Compound of Formula No. D-80

¹H-NMR (CDCl₃) δ(ppm) : 5.13 (1H, d, J=14Hz, NCH₂Ar), 4.34 (1H, d, J=14Hz, NCH₂Ar), 3.65 (1H, hept, J=5Hz, NCHEt₂), 1.97 (3H, s, 3-CH₃), 1.81 (3H, s, COCH₃), 1.61 (3H, s, 5-CH₃).
MASS (EI) m/z : 338(M⁺), 323, 309, 295, 281, 268, 222.

### (Example 1947)

### Compound of Formula No. D-81

¹H-NMR (CDCl₃) δ(ppm) : 4.91 (1H, d, J=14Hz, NCH₂Ar), 4.41 (1H, d, J=14Hz, NCH₂Ar), 3.67 (3H, brs, COOCH₃), 3.64 (1H, hept, J=5Hz, NCHEt₂), 1.95 (3H, s, 3-CH₃), 1.65 (3H, s, 5-CH₃).
MASS (EI) m/z : 354(M⁺), 339, 325, 297, 284, 238, 168.

### (Example 1948)

### Compound of Formula No. D-82

¹H-NMR (CDCl₃) δ(ppm) : 4.97 (1H, d, J=14Hz, NCH₂Ar), 4.44 (1H, d, J=14Hz, NCH₂Ar), 4.38 (1H, *pent, J=7Hz, NCH(CH₂)₄), 1.91 (3H, s, 3-CH₃), 1.82 (3H, s, COCH₃), 1.71 (3H, s, 5-CH₃).
MASS (EI) m/z : 336(M⁺), 293, 281, 268, 220, 178, 152.

### (Example 1949)

### Compound of Formula No. D-83

¹H-NMR (CDCl₃) δ(ppm) : 4.96 (1H, d, J=14Hz, NCH₂Ar), 4.45 (1H, d, J=14Hz, NCH₂Ar), 3.98-3.89 (2H, m, NCH₂CH₂-tBu), 1.90 (3H, s, 3-CH₃), 1.82 (3H, s, COCH₃), 1.71 (3H, s, 5-CH₃).
MASS (EI) m/z : 352(M⁺), 337, 309, 295, 281, 269, 253, 236.

### (Example 1950)

### Compound of Formula No. D-84

¹H-NMR (CDCl₃) δ(ppm) : 4.80 (1H, d, J=14Hz, NCH₂Ar), 4.47 (1H, d, J=14Hz, NCH₂Ar), 3.98 (1H, d, J=6Hz, NCH₂CH₂-tBu), 3.88 (1H, d, J=6Hz, NCH₂CH₂-tBu), 3.78 (3H, s, COOCH₃), 1.90 (3H, s, 3-CH₃), 1.74 (3H, s, 5-CH₃).
MASS (EI) m/z : 368(M⁺), 353, 311, 297, 284, 252, 168, 116.

### (Example 1951)

### Compound of Formula No. D-85

¹H-NMR (CDCl₃) δ(ppm) : 5.05 (1H, d, J=14Hz, NCH₂Ar), 4.39 (1H, d, J=14Hz, NCH₂Ar), 3.78 (1H, dd, J=14Hz, 7Hz, NCH₂CHEt₂), 3.77 (1H, dd, J=14Hz, 7Hz, NCH₂CHEt₂), 1.92 (3H, s, 3-CH₃), 1.82 (3H, s, COCH₃), 1.66 (3H, s, 5-CH₃).
MASS (EI) m/z : 352(M⁺), 337, 323, 309, 293, 281, 269, 254.

### (Example 1952)

### Compound of Formula No. D-86

¹H-NMR (CDCl₃) δ(ppm) : 4.88 (1H, d, J=14Hz, NCH₂Ar), 4.44 (1H, d, J=14Hz, NCH₂Ar), 3.78 (1H, dd, J=14Hz, 7Hz, NCH₂CHEt₂), 3.74 (1H, dd, J=14Hz, 7Hz, NCH₂CHEt₂), 3.67 (3H, brs, COOCH₃), 1.93 (3H, s, 3-CH₃), 1.69 (3H, s, 5-CH₃).
MASS (EI) m/z : 368(M⁺), 353, 339, 297, 284, 252, 168.

### (Example 1953)

### Compound of Formula No. D-87

¹H-NMR (CDCl₃) δ(ppm) : 4.96 (1H, d, J=14Hz, NCH₂Ar), 4.54 (2H, brd, J=7Hz, NCH₂CH=CMe₂), 4.44 (1H, d, J=14Hz, NCH₂Ar), 1.91 (3H, s, 3-CH₃), 1.82 (3H, s, COCH₃), 1.68 (3H, S, 5-CH₃).
MASS (EI) m/z : 336(M⁺), 321, 298, 268, 225, 152, 116.

### (Example 1954)

### Compound of Formula No. D-88

¹H-NMR (CDCl₃) δ(ppm) : 4.79 (1H, d, J=14Hz, NCH₂Ar), 4.54 (2H, d, J=7Hz, NCH₂CH=CMe₂), 4.49 (1H, d, J=14Hz, NCH₂Ar), 3.68 (3H, brs, COOCH₃), 1.91 (3H, s, 3-CH₃), 1.75 (3H, s, 5-CH₃).
MASS (EI) m/z : 352(M⁺), 337, 284, 236, 225, 168.

### (Example 1955)

### Compound of Formula No. D-89

¹H-NMR (CDCl₃) δ(ppm) : [5.11 (d, J=14Hz) & 5.03 (d, J=14Hz), 1H, NCH₂Ar], [4.39 (d, J=14Hz) & 4.32 (d, J=14Hz), 1H, NCH₂Ar], 4.18-4.05 (1H, m, NCH(Me)-iBu), [1.95 (s) & 1.94 (s), 3H, 3-CH₃], [1.82 (s) & 1.80 (s), 3H, COCH₃], [1.67 (s) & 1.64 (s), 3H, 5-CH₃].
MASS (EI) m/z : 352(M⁺), 337, 309, 296, 281, 269, 236, 225.

### (Example 1956)

### Compound of Formula No. D-90

¹H-NMR (CDCl₃) δ(ppm) : 5.52 (1H, d, J=14Hz, NCH₂Ar), 4.40 (1H, d, J=14Hz, NCH₂Ar), 3.88-3.71 (1H, m, NCH(CH₂)₅), 1.93 (3H, s, 3-CH₃), 1.81 (3H, s, COCH₃), 1.68 (3H, s, 5-CH₃).
MASS (EI) m/z : 350(M⁺), 335, 307, 295, 281, 268, 234, 225.

### (Example 1957)

### Compound of Formula No. D-91

¹H-NMR (CDCl₃) δ(ppm) : 5.01 (1H, d, J=14Hz, NCH₂Ar), 4.45 (1H, d, J=14Hz, NCH₂Ar), 3.90-3.68 (1H, m, NCH(CH₂)₅), 2.24 (3H, s, COSCH₃), 1.94 (3H, s, 3-CH₃), 1.70 (3H, s, 5-CH₃).
MASS (EI) m/z : 382(M⁺), 335, 307, 300, 266, 253, 225, 219.

### (Example 1958)

### Compound of Formula No. D-92

¹H-NMR (CDCl₃) δ (ppm) : 4.98 (1H, d, J=14Hz, NCH₂Ar), 4.43 (1H, d, J=14Hz, NCH₂Ar), 3.89 (2H, t, J=7Hz, NCH₂-C₆H₁₃), 1.91 (3H, s, 3-CH₃), 1.82 (3H, s, COCH₃), 1.69 (3H, s, 5-CH₃).
MASS (EI) m/z : 366(M⁺), 351, 337, 323, 295, 281, 268.

### (Example 1959)

### Compound of Formula No. D-93

¹H-NMR (CDCl₃) δ(ppm) : 4.82 (1H, d, J=14Hz, NCH₂Ar), 4.47 (1H, d, J=14Hz, NCH₂Ar), 3.88 (2H, td, J=7Hz, 2Hz, NCH₂-C₆H₁₃), 3.68 (3H, brs, COOCH₃), 1.91 (3H, s, 3-CH₃), 1.73 (3H, s, 5-CH₃). MASS (EI) m/z : 382(M⁺), 353, 339, 312, 297, 284, 266.

### (Example 1960)

### Compound of Formula No. D-94

¹H-NMR (CDCl₃) δ(ppm) : 5.08 (1H, d, J=14Hz, NCH₂Ar), 4.36 (1H, d, J=14Hz, NCH₂Ar), 3.71 (1H, dd, J=14Hz, 7Hz, NCH₂-cPr), 3.70 (1H, dd, J=14Hz, 7Hz, NCH₂-cPr), 1.94 (3H, s, 3-CH₃), 1.82 (3H, s, COCH₃), 1.65 (3H, s, 5-CH₃).
MASS (EI) m/z : 364(M⁺), 349, 321, 307, 293, 281, 268.

### (Example 1961)

### Compound of Formula No. D-95

¹H-NMR (CDCl₃) δ(ppm) : 4.90 (1H, d, J=14Hz, NCH₂Ar), 4.42 (1H, d, J=14Hz, NCH₂Ar), 3.72 (1H, dd, J=14Hz, 7Hz, NCH₂-cHex), 3.68 (3H, brs, COOCH₃), 3.65 (1H, dd, J=14Hz, 7Hz, NCH₂-cPr), 1.95 (3H, s, 3-CH₃), 1.68 (3H, s, 5-CH₃).
MASS (EI) m/z : 380(M⁺), 365, 349, 321, 297, 284, 264.

### (Example 1962)

### Compound of Formula No. D-96

¹H-NMR (CDCl₃) δ(ppm) : [5.05 (d, J=14Hz) & 5.03 (d, J=14Hz), 1H, NCH₂Ar], [4.40 (d, J=14Hz) & 4.36 (d, J=14Hz), 1H, NCH₂Ar], [3.88 (d, J=8Hz) & 3.87 (d, J=8Hz), 1H, NCH₂-C₇H₁₁], [3.87 (d, J=8Hz) & 3.65 (d, J=8Hz), 1H, NCH₂-C₇H₁₁], [1.92 (s) & 1.91 (s), 3H, 3-CH₃], [1.83 (s) & 1.81 (s), 3H, COCH₃], [1.68 (s) & 1.67 (s), 3H, 5-CH₃].
MASS (EI) m/z : 376(M⁺), 361, 352, 333, 309, 281, 268, 226.

### (Example 1963)

### Compound of Formula No. D-97

¹H-NMR (CDCl₃) δ(ppm) : [4.87 (d, J=14Hz) & 4.85 (d, J=14Hz), 1H, NCH₂Ar], [4.44 (d, J=14Hz) & 4.42 (d, J=14Hz), 1H, NCH₂Ar], 3.97-3.52 (2H, m, NCH₂-C₇H₁₁), 3.68 (3H, brs, COOCH₃), [1.92 (s) & 1.91 (s), 3H, 3-CH₃], [1.71 (s) & 1.69 (s), 3H, 5-CH₃].
MASS (EI) m/z : 392(M⁺), 377, 333, 325, 297, 284, 276, 168.

### (Example 1964)

### Compound of Formula No. D-98

¹H-NMR (CDCl₃) δ(ppm) : 5.17 (2H, brs, 1-NCH₂Ph), 5.02 (1H, d, J=14Hz, NCH₂Ph), 4.42 (1H, d, J=14Hz, NCH₂Ar), 1.96 (3H, s, 3-CH₃), 1.83 (3H, s, COCH₃), 1.59 (3H, s, 5-CH₃).
MASS (EI) m/z : 358(M⁺), 315, 242, 200, 186, 116.

### (Example 1965)

### Compound of Formula No. D-99

¹H-NMR (CDCl₃) δ(ppm) : 5.18 (1H, d, J=16Hz, 1-NCH₂Ph), 5.12 (1H, d, J=16Hz, 1-NCH₂Ph), 4.83 (1H, d, J=14Hz, NCH₂Ar), 4.48 (1H, d, J=14Hz, NCH₂Ar), 3.68 (3H, brs, COOCH₃), 1.95 (3H, s, 3-CH₃), 1.64 (3H, S, 5-CH₃).
MASS (EI) m/z : 374(M⁺), 279, 258, 167, 149, 116.

### (Example 1966)

### Compound of Formula No. D-100

¹H-NMR (CDCl₃) δ(ppm) : 5.17 (1H, d, J=16Hz, 1-NCH₂Ph), 5.15 (1H, d, J=16Hz, 1-NCH₂Ph), 4.85 (1H, d, J=14Hz, NCH₂Ar), 4.47 (1H, d, J=14Hz, NCH₂Ar), 4.14 (2H, brq, J=7Hz, COOCH₂Me), 1.97 (3H, s, 3-CH₃), 1.63 (3H, s, 5-CH₃).
MASS (EI) m/z : 388(M⁺), 315, 272, 226, 200, 116.

### (Example 1967)

### Compound of Formula No. D-102

¹H-NMR (CDCl₃) δ(ppm) : 5.19 (2H, s, 1-NCH₂Ar), 5.07 (1H, d, J=14Hz, NCH₂Ar), 4.41 (1H, d, J=14Hz, NCH₂Ar), 1.98 (3H, s, 3-CH₃), 1.84 (3H, s, COCH₃), 1.63 (3H, s, 5-CH₃).
MASS (EI) m/z : 383(M⁺), 340, 267, 255, 211, 196.

### (Example 1968)

### Compound of Formula No. D-103

¹H-NMR (CDCl₃) δ(ppm) : 5.19 (1H, d, J=17Hz, 1-NCH₂Ar), 5.17 (1H, d, J=17Hz, 1-NCH₂Ar), 4.89 (1H, d, J=14Hz, NCH₂Ar), 4.47 (1H, d, J=14Hz, NCH₂Ar), 3.71 (3H, brs, COOCH₃), 1.97 (3H, s, 3-CH₃), 1.65 (3H, s, 5-CH₃).
MASS (EI) m/z : 399(M⁺), 340, 283, 251, 116.

### (Example 1969)

### Compound of Formula No. D-104

¹H-NMR (CDCl₃) δ(ppm) : 5.19 (2H, s, 1-NCH₂Ar), 5.01 (1H, d, J=14Hz, NCH₂Ar), 4.44 (1H, d, J=14Hz, NCH₂Ar), 1.96 (3H, s, 3-CH₃), 1.83 (3H, s, COCH₃), 1.62 (3H, s, 5-CH₃).
MASS (EI) m/z : 442(M⁺), 399, 326, 284, 270, 255, 116.

### (Example 1970)

### Compound of Formula No. D-105

¹H-NMR (CDCl₃) δ(ppm) : 5.18 (1H, d, J=16Hz, 1-NCH₂Ar), 5.15 (1H, d, J=16Hz, 1-NCH₂Ar), 4.84 (1H, d, J=14Hz, NCH₂Ar), 4.49 (1H, d, J=14Hz, NCH₂Ar), 3.69 (3H, brs, COOCH₃), 1.95 (3H, s, 3-CH₃), 1.65 (3H, s, 5-CH₃).
MASS (EI) m/z : 458(M⁺), 342, 310, 175, 116.

### (Example 1971)

### Compound of Formula No. D-106

¹H-NMR (CDCl₃) δ(ppm) : 5.72 (2H, s, NCH₂Cl), 5.04 (1H, d, J=14Hz, NCH₂Ar), 4.39 (1H, d, J=14Hz, NCH₂Ar), 1.95 (3H, s, 3-CH₃), 1.83 (3H, s, COCH₃), 1.82 (3H, s, 5-CH₃).
MASS (EI) m/z : 316(M⁺), 281, 274, 268, 237, 225, 200, 164.

### (Example 1972)

### Compound of Formula No. D-107

¹H-NMR (CDCl₃) δ(ppm) : 5.73 (1H, d, J=11Hz, NCH₂Cl), 5.72 (1H, d, J=11Hz, NCH₂Cl), 4.80 (1H, d, J=14Hz, NCH₂Ar), 4.49 (1H, d, J=14Hz, NCH₂Ar), 3.69 (3H, brs, COOCH₃), 1.93 (3H, s, 3-CH₃), 1.86 (3H, s, 5-CH₃).
MASS (EI) m/z : 332(M⁺), 297, 284, 237, 216, 180, 116.

### (Example 1973)

### Compound of Formula No. D-108

¹H-NMR (CDCl₃) δ(ppm) : 5.06 (1H, d, J=14Hz, NCH₂Ar), 4.52 (2H, q, J=8Hz, NCH₂CF₃), 4.38 (1H, d, J=14Hz, NCH₂Ar), 1.95 (3H, s, 3-CH₃), 1.82 (3H, s, COCH₃), 1.73 (3H, s, 5-CH₃).
MASS (EI) m/z : 350(M⁺), 331, 308, 234, 206, 192, 178.

### (Example 1974)

### Compound of Formula No. D-109

¹H-NMR (CDCl₃) δ(ppm) : 4.85 (1H, d, J=14Hz, NCH₂Ar), 4.45 (2H, qd, J=16Hz, 8Hz, NCH₂CF₃), 4.45 (1H, d, J=14Hz, NCH₂Ar), 3.69 (3H, brs, COOCH₃), 1.94 (3H, s, 3-CH₃), 1.76 (3H, s, 5-CH₃). MASS (EI) m/z : 366(M⁺), 347, 307, 265, 250, 218, 178.

### (Example 1975)

### Compound of Formula No. D-110

¹H-NMR (CDCl₃) δ(ppm) : 6.37 (1H, d, J=5Hz, NCH=CFI), 5.12 (1H, d, J=14Hz, NCH₂Ar), 4.38 (1H, d, J=14Hz, NCH₂Ar), 2.01 (3H, s, 3-CH₃), 1.87 (3H, s, COCH₃), 1.77 (3H, s, 5-CH₃).
MASS (EI) m/z : 438(M⁺), 396, 322, 280, 266, 212, 168.

### (Example 1976)

### Compound of Formula No. D-111

¹H-NMR (CDCl₃) δ(ppm) : 6.31 (1H, d, J=5Hz, NCH=CFI), 4.88 (1H, d, J=14Hz, NCH₂Ar), 4.47 (1H, d, J=14Hz, NCH₂Ar), 3.70 (3H, brs, COOCH₃), 1.97 (3H, s, 3-CH₃), 1.80 (3H, s, 5-CH₃).
MASS (EI) m/z : 454(M⁺), 338, 266, 212, 168, 149, 116.

### (Example 1977)

### Compound of Formula No. D-112

¹H-NMR (CDCl₃) δ(ppm) : 5.92 (1H, d, J=28Hz, NCH=CFI), 4.89 (1H, d, J=14Hz, NCH₂Ar), 4.54 (1H, d, J=14Hz, NCH₂Ar), 1.91 (3H, s, 3-CH₃) 1.88 (3H, s, COCH₃), 1.84 (3H, s, 5-CH₃).
MASS (EI) m/z : 438(M⁺), 396, 322, 312, 280, 266, 212.

### (Example 1978)

### Compound of Formula No. D-113

¹H-NMR (CDCl₃) δ(ppm) : 5.86 (1H, d, J=28Hz, NCH=CFI), 4.71 (1H, d, J=14Hz, NCH₂Ar), 4.58 (1H, d, J=14Hz, NCH₂Ar), 3.70 (3H, brs, COOCH₃), 1.90 (3H, s, 3-CH₃), 1.88 (3H, s, 5-CH₃).
MASS (EI) m/z : 454(M⁺), 338, 266, 212, 168, 149, 116.

### (Example 1979)

### Compound of Formula No. D-114

¹H-NMR (CDCl₃) δ(ppm) : [4.98 (d, J=14Hz) & 4.96 (d, J=14Hz), 1H, NCH₂Ar], [4.48 (d, J=14Hz) & 4.46 (d, J=14Hz), 1H, NCH₂Ar], 4.14-3.97 (2H, m, NCH₂CHFMe), [1.91 (s) & 1.90 (s), 3H, 3-CH₃], 1.82 (3H, s, COCH₃), [1.74 (s) & 1.71 (s), 3H, 5-CH₃].
MASS (EI) m/z : 328(M⁺), 308, 285, 265, 212, 184, 170.

### (Example 1980)

### Compound of Formula No. D-116

¹H-NMR (CDCl₃) δ(ppm) : [5.07 (d, J=14Hz) & 5.01 (d, J=14Hz), 1H, NCH₂Ar], [4.43 (d, J=14Hz) & 4.36 (d, J=14Hz), 1H, NCH₂Ar], 4.20-3.91 (2H, m, NCH₂CHClMe), [1.93 (s) & 1.92 (s), 3H, 3-CH₃], 1.82 (3H, s, COCH₃), [1.76 (s) & 1.71 (s), 3H, 5-CH₃].
MASS (EI) m/z : 344(M⁺), 309, 301, 281, 228, 186.

### (Example 1981)

### Compound of Formula No. D-117

¹H-NMR (CDCl₃) δ(ppm) : 4.85 (1H, d, J=14Hz, NCH₂Ar), [4.46 (d, J=14Hz) & 4.39 (d, J=14Hz), 1H, NCH₂Ar], 4.18-4.50 (1H, m, NCH₂CHClMe), 3.98(1H, dd, J=14Hz, 9Hz, NCH₂CHClMe), 3.68 (3H, brs, COOCH₃), 1.91 (3H, s, 3-CH₃), [1.78 (s) & 1.75 (s), 3H, 5-CH₃].
MASS (EI) m/z : 360(M⁺), 325, 297, 284, 244, 208, 168.

### (Example 1982)

### Compound of Formula No. D-118

¹H-NMR (CDCl₃) δ(ppm) : 4.96 (1H, d, J=14Hz, NCH₂Ar), 4.49 (1H, d, J=14Hz, NCH₂Ar), 4.07 (2H, d, J=21Hz, NCH₂CFMe₂), 1.89 (3H, s, 3-CH₃), 1.83 (3H, s, COCH₃), 1.74 (3H, s, 5-CH₃).
MASS (EI) m/z : 342(M⁺), 322, 299, 281, 226, 206, 184.

### (Example 1983)

### Compound of Formula No. D-119

¹H-NMR (CDCl₃) δ(ppm) : 4.78 (1H, d, J=14Hz, NCH₂Ar), 4.53 (1H, d, J=14Hz, NCH₂Ar), 4.05 (2H, d, J=21Hz, NCH₂CFMe₂), 3.68 (3H, brs, COOCH₃), 1.87 (3H, s, 3-CH₃), 1.76 (3H, s, 5-CH₃).
MASS (EI) m/z : 358(M⁺), 338, 297, 242, 222, 149, 116.

### (Example 1984)

### Compound of Formula No. D-120

¹H-NMR (CDCl₃) δ(ppm) : 5.38 (2H, s, NCH₂OH), 4.86 (1H, d, J=14Hz, NCH₂Ar), 4.57 (1H, d, J=14Hz, NCH₂Ar), 1.89 (3H, s, 3-CH₃), 1.87 (3H, s, COCH₃), 1.83 (3H, s, 5-CH₃).
MASS (EI) m/z : 268(M⁺), 225, 152, 124, 116.

### (Example 1985)

### Compound of Formula No. D-121

¹H-NMR (CDCl₃) δ(ppm) : 5.35 (2H, s, NCH₂OH), 4.71 (1H, d, J=14Hz, NCH₂Ar), 4.58 (1H, d, J=14Hz, NCH₂Ar), 3.68 (3H, brs, COOCH₃), 1.93 (3H, s, 3-CH₃), 1.85 (3H, s, 5-CH₃).
MASS (EI) m/z : 298, 284, 253, 225, 168.

### (Example 1986)

### Compound of Formula No. D-122

¹H-NMR (CDCl₃) δ(ppm) : 5.08 (1H, d, J=14Hz, NCH₂Ar), 4.35 (1H, d, J=14Hz, NCH₂Ar), 4.09 (1H, d, J=14Hz, NCH₂-C₄H₇O), 4.06 (1H, d, J=14Hz, NCH₂-C₄H₇O), 1.91 (3H, s, 3-CH₃), 1.81 (3H, s, COCH₃), 1.65 (3H, s, 5-CH₃).
MASS (EI) m/z : 352(M⁺), 337, 323, 309, 281, 269, 254, 236.

### (Example 1987)

### Compound of Formula No. D-123

¹H-NMR (CDCl₃) δ(ppm) : 4.89 (1H, d, J=14Hz, NCH₂Ar), 4.40 (1H, d, J=14Hz, NCH₂Ar), 4.12 (1H, d, J=14Hz, NCH₂-C₄H₇O), 3.99 (1H, d, J=14Hz, NCH₂-C₄H₇O), 3.67 (3H, brs, COOCH₃), 1.91 (3H, s, 3-CH₃), 1.67 (3H, s, 5-CH₃).
MASS (EI) m/z : 368(M⁺), 353, 339, 309, 297, 284, 252, 222.

### (Example 1988)

### Compound of Formula No. D-124

¹H-NMR (CDCl₃) δ(ppm) : 4.98 (1H, d, J=14Hz, NCH₂Ar), 4.44 (1H, d, J=14Hz, NCH₂Ar), 3.74 (2H, td, J=13Hz, 7Hz, NCH₂THP), 1.90 (3H, s, 3-CH₃), [1.83 (s) & 1.82 (s), 3H, COCH₃], [1.76 (s) & 1.74(s), 3H, 5-CH₃].
MASS (EI) m/z : 352(M⁺), 309, 281, 268, 236, 226.

### (Example 1989)

### Compound of Formula No. D-125

¹H-NMR (CDCl₃) δ(ppm) : 4.81 (1H, d, J=14Hz, NCH₂Ar), [4.50 (d, J=14Hz) & 4.49 (d, J=14Hz), 1H, NCH₂Ar], 4.27-3.70 (2H, m, NCH₂THF), 3.67 (3H, brs, COOCH₃), 1.90 (3H, s, 3-CH₃), [1.80 (s) & 1.77 (s), 3H, 5-CH₃].
MASS (EI) m/z : 368(M⁺), 325, 297, 284, 252, 182, 168.

### (Example 1990)

### Compound of Formula No. D-126

¹H-NMR (CDCl₃) δ(ppm) : 5.00 (1H, dd, J=14Hz, 2Hz, NCH₂Ar), 4.43 (1H, d, J=14Hz, NCH₂Ar), 3.89 (2H, d, J=7Hz, NCH₂THP), 1.91 (3H, s, 3-CH₃), 1.81 (3H, s, COCH₃), 1.71 (3H, s, 5-CH₃).
MASS (EI) m/z : 366(M⁺), 338, 323, 282, 268, 250, 166.

### (Example 1991)

### Compound of Formula No. D-127

¹H-NMR (CDCl₃) δ(ppm) : 4.81 (1H, d, J=14Hz, NCH₂Ar), [4.49 (d, J=14Hz) & 4.48 (d, J=14Hz), 1H, NCH₂Ar], 3.96 (1H, dd, J=14Hz, 7Hz, NCH₂THP), 3.89-3.79 (1H, m, NCH₂THP), 3.67 (3H, brs, COOCH₃), [1.91 (s) & 1.90 (s), 3H, 3-CH₃], 1.75 (3H, s, 5-CH₃). MASS (EI) m/z : 382(M⁺), 354, 323, 297, 284, 266, 182.

### (Example 1992)

### Compound of Formula No. D-128

¹H-NMR (CDCl₃) δ(ppm) : 5.36 (2H, s, NCH₂O), 4.94 (1H, d, J=14Hz, NCH₂Ar), 4.50 (1H, d, J=14Hz, NCH₂Ar), 1.89 (3H, s, 3-CH₃), 1.83 (6H, s, COCH₃ & 5-CH₃).
MASS (EI) m/z : 356(M⁺), 297, 281, 268, 237, 164, 149, 116.

### (Example 1993)

### Compound of Formula No. D-129

¹H-NMR (CDCl₃) δ(ppm) : 5.37 (2H, s, NCH₂OCH₂Ph), 4.95 (1H, d, J=14Hz, NCH₂Ar), 4.47 (1H, d, J=14Hz, NCH₂Ar), 1.92 (3H, s, 3-CH₃), 1.81 (6H, s, COCH₃ & 5-CH₃).
MASS (EI) m/z : 388(M⁺), 358, 345, 315, 282, 239.

### (Example 1994)

### Compound of Formula No. D-130

¹H-NMR (CDCl₃) δ(ppm) : 5.36 (2H, s, NCH₂OCH₂Ph), 4.78 (1H, d, J=14Hz, NCH₂Ar), 4.52 (1H, d, J=14Hz, NCH₂Ar), 3.69 (3H, brs, COOCH₃), 1.92 (3H, s, 3-CH₃), 1.85 (3H, s, 5-CH₃).
MASS (EI) m/z : 404(M⁺), 373, 298, 258, 182, 168.

### (Example 1995)

### Compound of Formula No. D-131

¹H-NMR (CDCl₃) δ(ppm) : 4.96 (1H, d, J=14Hz, NCH₂Ar), 4.47 (1H, d, J=14Hz, NCH₂Ar), 4.46-3.95 (4H, m, NCH₂CH₂OH), 1.90 (3H, s, 3-CH₃), 1.82 (3H, s, COCH₃), 1.74 (3H, s, 5-CH₃).
MASS (EI) m/z : 312(M⁺), 293, 281, 269, 196, 149.

### (Example 1996)

### Compound of Formula No. D-132

¹H-NMR (CDCl₃) δ(ppm) : 4.92 (1H, d, J=14Hz, NCH₂Ar), 4.50 (1H, d, J=14Hz, NCH₂Ar), 4.08 (2H, t, J=5Hz, NCH₂CH₂OEt), 1.88 (3H, s, 3-CH₃), 1.82 (3H, s, COCH₃), 1.78 (3H, s, 5-CH₃).
MASS (EI) m/z : 340(M⁺), 325, 311, 296, 281, 268, 253.

### (Example 1997)

### Compound of Formula No. D-133

¹H-NMR (CDCl₃) δ(ppm) : 4.76 (1H, d, J=14Hz, NCH₂Ar), 4.52 (1H, d, J=14Hz, NCH₂Ar), 4.06 (2H, t, J=5Hz, NCH₂CH₂OEt), 3.67 (3H, brs, COOCH₃), 1.88 (3H, s, 3-CH₃), 1.81 (3H, s, 5-CH₃).
MASS (EI) m/z : 356(M⁺), 312, 297, 284, 253, 240, 196.

### (Example 1998)

### Compound of Formula No. D-134

¹H-NMR (CDCl₃) δ(ppm) : 4.92 (1H, d, J=14Hz, NCH₂Ar), 4.44 (1H, d, J=14Hz, NCH₂Ar), 4.30 (4H, s, NCH₂CH₂OPh), 1.89 (3H, s, 3-CH₃), 1.84 (3H, s, COCH₃), 1.77 (3H, s, 5-CH₃).
MASS (EI) m/z : 388(M⁺), 345, 295, 281, 272, 268, 230.

### (Example 1999)

### Compound of Formula No. D-135

¹H-NMR (CDCl₃ δ(ppm) : 4.75 (1H, d, J=14Hz, NCH₂Ar), 4.47 (1H, d, J=14Hz, NCH₂Ar), 4.29 (4H, brs, NCH₂CH₂OPh), 3.66 (3H, brs, COOCH₃), 1.89 (3H, s, 3-CH₃), 1.87 (3H, s, 5-CH₃).
MASS (EI) m/z : 404(M⁺), 373, 345, 311, 297, 284, 256.

### (Example 2000)

### Compound of Formula No. D-136

¹H-NMR (CDCl₃) δ(ppm) : [4.89 (d, J=14Hz) & 4.87 (d, J=14Hz), 1H, NCH₂Ar], [4.55 (d, J=14Hz) & 4.54 (d, J=14Hz), 1H, NCH₂Ar], 4.14 (2H, t, J=5Hz, NCH₂CH₂OTHP), [1.87 (s) & 1.86 (s), 3H, 3-CH₃], [1.83 (s) & 1.82 (s), 3H, COCH₃], [1.82 (s) & 1.81 (s), 3H, 5-CH₃].
MASS (EI) m/z : 397(M⁺), 367, 353, 323, 313, 296, 268, 253.

### (Example 2001)

### Compound of Formula No. D-137

¹H-NMR (CDCl₃) δ(ppm) : [5.03 (d, J=14Hz) & 4.98 (d, J=14Hz), 1H, NCH₂Ar], [4.45 (d, J=14Hz) & 4.41 (d, J=14Hz), 1H, NCH₂Ar], [3.94 (d, J=14Hz) & 3.93 (d, J=14Hz), 1H, NCH₂CH(OH)Me], [3.80 (dd, J=14Hz, 7Hz) & 3.79 (dd, J=14Hz, 7Hz), 1H, NCH₂CH(OH)Me], [1.92 (s) & 1.91 (s), 3H, 3-CH₃], 1.82 (3H, s, COCH₃), [1.72 (s) & 1.69 (s), 3H, 5-CH₃].
MASS (EI) m/z : 326(M⁺), 311, 283, 269, 254, 239, 226.

### (Example 2002)

### Compound of Formula No. D-139

¹H-NMR (CDCl₃) δ(ppm) : [4.98 (d, J=14Hz) & 4.96 (d, J=14Hz), 1H, NCH₂Ar], [4.47 (d, J=14Hz) & 4.46 (d, J=14Hz), 1H, NCH₂Ar], 3.88 (2H, brd, J=6Hz, NCH₂CH(OMe)Me), [1.90 (s) & 1.89 (s), 3H, 3-CH₃], [1.82 (s) & 1.80 (s), 3H, COCH₃], [1.75 (s) & 1.74 (s), 3H, 5-CH₃].
MASS (EI) m/z : 340(M⁺), 310, 297, 282, 268, 224, 166.

### (Example 2003)

### Compound of Formula No. D-141

¹H-NMR (CDCl₃) δ(ppm) : 5.07 (1H, d, J=14Hz, NCH₂Ar), 4.38 (1H, d, J=14Hz, NCH₂Ar), 3.84 (2H, s, NCH₂C(OH)Me₂), 1.95 (3H, s, 3-CH₃), 1.82 (3H, s, COCH₃), 1.67 (3H, s, 5-CH₃).
MASS (EI) m/z : 340(M⁺), 325, 297, 282, 254, 239, 206, 198.

### (Example 2004)

### Compound of Formula No. D-143

¹H-NMR (CDCl₃) δ(ppm) : 5.00 (1H, d, J=14Hz, NCH₂Ar), 4.45 (1H, d, J=14Hz, NCH₂Ar), 3.91 (2H, s, NCH₂C(OMe)Me₂), 1.89 (3H, s, 3-CH₃), 1.82 (3H, s, COCH₃), 1.73 (3H, s, 5-CH₃).
MASS (EI) m/z 354(M⁺), 323, 282, 239, 166, 125, 116.

### (Example 2005)

### Compound of Formula No. D-144

¹H-NMR (CDCl₃) δ(ppm) : 4.82 (1H, d, J=14Hz, NCH₂Ar), 4.49 (1H, d, J=14Hz, NCH₂Ar), 3.89 (2H, s, NCH₂C(OMe)Me₂), 3.68 (3H, s, COOCH₃), 1.89 (3H, s, 3-CH₃), 1.76 (3H, s, 5-CH₃).
MASS (EI) m/z : 370(M⁺), 355, 338, 298, 222, 182, 149.

### (Example 2006)

### Compound of Formula No. D-145

¹H-NMR (CDCl₃) δ(ppm) : 4.81 (1H, d, J=14Hz, NCH₂Ar), 4.63 (1H, d, J=14Hz, NCH₂Ar), 3.85 (2H, s, NCH₂C(OSi)Me₂), 1.85 (3H, s, 3-CH₃), 1.84 (6H, s, COCH₃ & 5-CH₃).
MASS (EI) m/z : 412(M⁺), 397, 354, 325, 282, 238, 197.

### (Example 2007)

### Compound of Formula No. D-147

¹H-NMR (CDCl₃) δ(ppm) : [4.93 (d, J=14Hz) & 4.83 (d, J=14Hz), 1H, NCH₂Ar], [4.58 (d, J=14Hz) & 4.48 (d, J=14Hz), 1H, NCH₂Ar], 4.03 (2H, brd, J=7Hz, NCH₂CH(OAc)Me), [1.88 (s) & 1.83 (s), 3H, 3-CH₃], [1.82 (s) & 1.80 (s), 3H, COCH₃], [1.81 (s) & 1.80 (s), 3H, 5-CH₃].
MASS (EI) m/z : 368(M⁺), 325, 306, 281, 265, 252, 210.

### (Example 2008)

### Compound of Formula No. D-149

¹H-NMR (CDCl₃) δ(ppm) : 5.03 (1H, d, J=14Hz, NCH₂Ar), 4.42 (1H, d, J=14Hz, NCH₂Ar), 4.03 (2H, d, J=7Hz, NCH₂C(OAc)Me₂), 1.91 (3H, s, 3-CH₃), 1.82 (3H, s, COCH₃), 1.74 (3H, s, 5-CH₃).
MASS (EI) m/z : 382(M⁺), 322, 281, 239, 206, 166.

### (Example 2009)

### Compound of Formula No. D-150

¹H-NMR (CDCl₃) δ(ppm) : 4.87 (1H, d, J=14Hz, NCH₂Ar), 4.45 (1H, d, J=14Hz, NCH₂Ar), 4.19 (1H, d, J=14Hz, NCH₂C(OAc)Me₂), 4.17 (1H, d, J=14Hz, NCH₂C(OAc)Me₂), 3.68 (3H, brs, COOCH₃), 1.91 (3H, s, 3-CH₃), 1.76 (3H, s, 5-CH₃).
MASS (EI) m/z : 398(M⁺), 338, 297, 222, 182, 150, 116.

### (Example 2010)

### Compound of Formula No. D-151

¹H-NMR (CDCl₃) δ(ppm) : 5.17-5.08 (1H, m, NCH(C₄H₈O)), [4.95 (d, J=14Hz) & 4.91 (d, J=14Hz), 1H, NCH₂Ar], [4.55 (d, J=14Hz) & 4.50 (d, J=14Hz), 1H, NCH₂Ar], [1.92 (s) & 1.91 (s), 3H, 3-CH₃], [1.83 (s) & 1.81 (s), 3H, COCH₃], [1.81 (s) & 1.80 (s), 3H, 5-CH₃].
MASS (EI) m/z : 352(M⁺), 281, 268, 226, 152, 116.

### (Example 2011)

### Compound of Formula No. D-152

¹H-NMR (CDCl₃) δ(ppm) : 5.02 (1H, d, J=14Hz, NCH₂Ar), 4.76 (2H, s, NCH₂COMe), 4.45 (1H, d, J=14Hz, NCH₂Ar), 1.91 (3H, s, 3-CH₃), 1.86 (3H, S, COCH₃), 1.61 (3H, S, 5-CH₃).
MASS (EI) m/z : 324(M⁺), 281, 239, 208, 166, 152.

### (Example 2012)

### Compound of Formula No. D-155

¹H-NMR (CDCl₃) δ(ppm) : 5.40 (2H, s, NCH₂COAr), 5.04 (1H, d, J=14Hz, NCH₂Ar), 4.46 (1H, d, J=14Hz, NCH₂Ar), 1.93 (3H, s, 3-CH₃), 1.89 (3H, s, COCH₃), 1.62 (3H, s, 5-CH₃).
MASS (EI) m/z : 404(M⁺), 376, 361, 293, 281, 149, 123.

### (Example 2013)

### Compound of Formula No. D-156

¹H-NMR (CDCl₃) δ(ppm) : 4.94 (1H, d, J=14Hz, NCH₂Ar), 4.49 (1H, d, J=14Hz, NCH₂Ar), 4.02 (2H, t, J=7Hz, NCH₂CH₂N(CH₂CH₂)₂O), 1.89 (3H, s, 3-CH₃), 1.82 (3H, s, COCH₃), 1.76 (3H, s, 5-CH₃).
MASS (EI) m/z : 381(M⁺), 364, 339, 320, 295, 282, 250.

### (Example 2014)

### Compound of Formula No. D-157

¹H-NMR (CDCl₃) δ(ppm) : 4.78 (1H, d, J=14Hz, NCH₂Ar), 4.51 (1H, d, J=14Hz, NCH₂Ar), 4.01 (2H, t, J=7Hz, NCH₂CH₂N(CH₂CH₂)₂O), 3.67 (3H, brs, COOCH₃), 1.89 (3H, s, 3-CH₃), 1.80 (3H, s, 5-CH₃).
MASS (EI) m/z : 397(M⁺), 379, 366, 354, 310, 113.

### (Example 2015)

### Compound of Formula No. D-158

¹H-NMR (CDCl₃) δ(ppm) : 4.95 (1H, d, J=14Hz, NCH₂Ar), 4.90 (2H, s, NCH₂CN), 4.47 (1H, d, J=14Hz, NCH₂Ar), 1.91 (3H, s, 3-CH₃), 1.85 (3H, s, COCH₃), 1.84 (3H, s, 5-CH₃).
MASS (EI) m/z : 307(M⁺), 265, 238, 225, 191, 163.

### (Example 2016)

### Compound of Formula No. D-159

¹H-NMR (CDCl₃) δ(ppm) : [5.05 (d, J=14Hz) & 5.04 (d, J=14Hz), 1H, NCH₂Ar], [4.97 (s) & 4.67 (d, J=14Hz), 1H, NCH₂C(=N)Me], [4.97 (s) & 4.66 (d, J=14Hz), 1H, NCH₂C(=N)Me], [4.42 (d, J=14Hz) & 4.40 (d, J=14Hz), 1H, NCH₂Ar], [1.94 (s) & 1.93 (s), 3H, 3-CH₃], [1.84 (s) & 1.82 (s), 3H, COCH₃], [1.69 (s) & 1.67 (s), 3H, 5-CH₃].
MASS (EI) m/z : 339(M⁺), 323, 296, 281, 268, 239, 225.

### (Example 2017)

### Compound of Formula No. D-161

¹H-NMR (CDCl₃) δ(ppm) : [5.02 (d, J=14Hz) & 4.94 (d, J=14Hz), 1H, NCH₂Ar], [4.87 (s) & 4.63 (s), 2H, NCH₂C(=N)Me], [4.50 (d, J=14Hz) & 4.42 (d, J=14Hz), 1H, NCH₂Ar], [1.93 (s) & 1.89 (s), 3H, 3-CH₃], [1.83 (s) & 1.82 (s), 3H, COCH₃], [1.66 (s) & 1.58 (s), 3H, 5-CH₃].
MASS (EI) m/z : 353(M⁺), 322, 310, 281, 237, 206, 195.

### (Example 2018)

### Compound of Formula No. D-162

¹H-NMR (CDCl₃) δ(ppm) : [4.88 (d, J=14Hz) & 4.77 (d, J=14Hz), 1H, NCH₂Ar], [4.85 (s) & 4.61 (s) , 2H, NCH₂C(=N)Me], [4.53 (d, J=14Hz) & 4.46 (d, J=14Hz), 1H, NCH₂Ar], 3.68 (3H, brs, COOCH₃), [1.93 (s) & 1.89 (s), 3H, 3-CH₃], [1.76 (s) & 1.69 (s), 3H, 5-CH₃].
MASS (EI) m/z : 369(M⁺), 338, 310, 297, 283, 253, 237.

### (Example 2019)

### Compound of Formula No. D-163

¹H-NMR (CDCl₃) δ(ppm) : [5.00 (d, J=14Hz) & 4.93 (d, J=14Hz), 1H, NCH₂Ar], [4.90 (s) & 4.63 (s), 2H, NCH₂C(=N)Me], [4.59 (d, J=14Hz) & 4.41 (d, J=14Hz), 1H, NCH₂Ar], [1.92 (s) & 1.86 (s), 3H, 3-CH₃], [1.80 (s) & 1.78 (s), 3H, COCH₃], 1.58 (3H, s, 5-CH₃).
MASS (EI) m/z : 429(M⁺), 412, 386, 357, 322, 282, 242.

### (Example 2020)

### Compound of Formula No. D-164

¹H-NMR (CDCl₃) δ(ppm) : 4.89 (2H, s, NCH₂C(=N)Me), 4.85 (1H, d, J=14Hz, NCH₂Ar), 4.45 (1H, d, J=14Hz, NCH₂Ar), 3.65 (3H, brs, COOCH₃), 1.92 (3H, s, 3-CH₃), 1.61 (3H, s, 5-CH₃).
MASS (EI) m/z : 445(M⁺), 428, 387, 373, 338, 297, 258.

### (Example 2021)

### Compound of Formula No. D-165

¹H-NMR (CDCl₃) δ(ppm) : 4.69 (1H, d, J=14Hz, NCH₂Ar), 4.61 (2H, s, NCH₂C(=N)Me), 4.54 (1H, d, J=14Hz, NCH₂Ar), 3.67 (3H, brs, COOCH₃), 1.86 (3H, s, 3-CH₃), 1.64 (3H, s, 5-CH₃).
MASS (EI) m/z : 445(M⁺), 428, 387, 373, 354, 324, 311.

### (Example 2022)

### Compound of Formula No. D-166

¹H-NMR (CDCl₃) δ(ppm) : [5.08 (d, J=14Hz) & 4.94 (d, J=14Hz), 1H, NCH₂Ar], [4.99 (s) & 4.81 (s), 2H, NCH₂C(=N)Me], [4.49 (d, J=14Hz) & 4.39 (d, J=14Hz), 1H, NCH₂Ar], [1.94 (s) & 1.90 (s), 3H, 3-CH₃], [1.82 (s) & 1.80 (s), 3H, COCH₃], [1.74 (s) & 1.68 (s), 3H, 5-CH₃].
MASS (EI) m/z : 381(M⁺), 339, 323, 296, 281, 237, 223.

### (Example 2023)

### Compound of Formula No. D-167

¹H-NMR (CDCl₃) δ(ppm) : [4.96 (s) & 4.79 (s), 2H, NCH₂C(=N)Me], [4.83 (d, J=14Hz) & 4.77 (d, J=14Hz), 1H, NCH₂Ar], [4.53 (d, J=14Hz) & 4.44 (d, J=14Hz), 1H, NCH₂Ar], 3.68 (3H, brs, COOCH₃), [1.95 (s) & 1.88 (s), 3H, 3-CH₃], [1.77 (s) & 1.69 (s), 3H, 5-CH₃].
MASS (EI) m/z : 397(M⁺), 355, 338, 297, 284, 237, 221.

### (Example 2024)

### Compound of Formula No. D-168

¹H-NMR (CDCl₃) δ(ppm) : 5.00 (1H, d, J=14Hz, NCH₂Ar), 4.74 (2H, s, NCH₂CO₂Me), 4.45 (1H, d, J=14Hz, NCH₂Ar), 1.91 (3H, s, 3-CH₃), 1.85 (3H, s, COCH₃), 1.68 (3H, s, 5-CH₃).
MASS (EI) m/z : 340(M⁺), 297, 281, 224, 168.

### (Example 2025)

### Compound of Formula No. D-169

¹H-NMR (CDCl₃) δ(ppm) : 4.98 (1H, d, J=14Hz, NCH₂Ar), 4.72 (2H, s, NCH₂CO₂Et), 4.46 (1H, d, J=14Hz, NCH₂Ar), 1.91 (3H, s, 3-CH₃), 1.85 (3H, s, COCH₃), 1.69 (3H, s, 5-CH₃).
MASS (EI) m/z : 354(M⁺), 311, 281, 238, 210, 196, 164.

### (Example 2026)

### Compound of Formula No. D-170

¹H-NMR (CDCl₃) δ(ppm) : 4.78 (1H, d, J=14Hz, NCH₂Ar), 4.75 (1H, d, J=18Hz, NCH₂CO₂Et), 4.67 (1H, d, J=18Hz, NCH₂CO₂Et), 4.53 (1H, d, J=14Hz, NCH₂Ar), 3.68 (3H, brs, COOCH₃), 1.88 (3H, s, 3-CH₃), 1.75 (3H, s, 5-CH₃).
MASS (EI) m/z : 370(M⁺), 311, 297, 254, 182, 150, 116.

### (Example 2027)

### Compound of Formula No. D-171

¹H-NMR (CDCl₃) δ(ppm) : 5.06 (2H, s, NCH₂S), 4.94 (1H, d, J=14Hz, NCH₂Ar), 4.49 (1H, d, J=14Hz, NCH₂Ar), 1.88 (3H, s, 3-CH₃), 1.82 (6H, s, COCH₃ & 5-CH₃).
MASS (EI) m/z : 356(M⁺), 313, 282, 269, 239, 198, 157.

### (Example 2028)

### Compound of Formula No. D-172

¹H-NMR (CDCl₃) δ(ppm) : 5.13 (2H, s, NCH₂S), 4.89 (1H, d, J=14Hz, NCH₂Ar), 4.52 (1H, d, J=14Hz, NCH₂Ar), 1.85 (6H, s, 3-CH₃ & COCH₃), 1.81 (3H, s, 5-CH₃).
MASS (EI) m/z : 370(M⁺), 314, 281, 116.

### (Example 2029)

### Compound of Formula No. D-173

¹H-NMR (CDCl₃) δ(ppm) : 5.06 (2H, s, NCH₂S), 4.93 (1H, d, J=14Hz, NCH₂Ar), 4.50 (1H, d, J=14Hz, NCH₂Ar), 1.88 (3H, s, 3-CH₃), 1.82 (6H, s, COCH₃ & 5-CH₃).
MASS (EI) m/z : 396(M⁺), 353, 314, 282, 269, 239, 166.

### (Example 2030)

### Compound of Formula No. D-174

¹H-NMR (CDCl₃) δ(ppm) : 5.13 (1H, d, J=15Hz, NCH₂S), 5.09 (1H, d, J=15Hz, NCH₂S), 4.81 (1H, d, J=14Hz, NCH₂Ar), 4.63 (1H, d, J=14Hz, NCH₂Ar), 1.93 (3H, s, 3-CH₃), 1.83 (6H, s, COCH₃ & 5-CH₃).
MASS (EI) m/z : 388(M⁺), 293, 281, 167, 149, 116.

### (Example 2031)

### Compound of Formula No. D-175

¹H-NMR (CDCl₃) δ(ppm) : 5.27 (1H, d, J=15Hz, NCH₂S), 5.22 (1H, d, J=15Hz, NCH₂S), 4.79 (1H, d, J=14Hz, NCH₂Ar), 4.64 (1H, d, J=14Hz, NCH₂Ar), 1.95 (3H, s, 3-CH₃), 1.82 (3H, s, COCH₃), 1.81 (3H, s, 5-CH₃).
MASS (EI) m/z : 402(M⁺), 346, 338, 281, 166, 116.

### (Example 2032)

### Compound of Formula No. D-176

¹H-NMR (CDCl₃) δ(ppm) : 5.10 (1H, d, J=15Hz, NCH₂S), 5.08 (1H, d, J=15Hz, NCH₂S), 4.83 (1H, d, J=14Hz, NCH₂Ar), 4.61 (1H, d, J=14Hz, NCH₂Ar), 1.92 (3H, s, 3-CH₃), 1.85 (3H, s, COCH₃), 1.83 (3H, s, 5-CH₃).
MASS (EI) m/z : 428(M⁺), 364, 281, 237, 166, 116.

### (Example 2033)

### Compound of Formula No. D-177

¹H-NMR (CDCl₃) δ(ppm) : 4.99 (1H, d, J=14Hz/ NCH₂Ar), 4.43 (1H, d, J=14Hz, NCH₂Ar), 3.46 (2H, s, NCH₂Si), 1.87 (3H, s, 3-CH₃), 1.81 (3H, s, COCH₃), 1.65 (3H, s, 5-CH₃).
MASS (EI) m/z : 354(M⁺), 339, 311, 238, 208, 197, 181, 155.

### (Example 2034)

### Compound of Formula No. D-178

¹H-NMR (CDCl₃) δ(ppm) : 4.85 (1H, d, J=14Hz, NCH₂Ar), 4.44 (1H, d, J=14Hz, NCH₂Ar), 3.67 (3H, brs, COOCH₃), 3.47 (1H, d, J=16Hz, NCH₂Si), 3.43 (1H, d, J=16Hz, NCH₂Si), 1.89 (3H, s, 3-CH₃), 1.67 (3H, s, 5-CH₃).
MASS (EI) m/z : 370(M⁺), 355, 339, 311, 297, 254, 222, 150.

### (Example 2035)

### Compound of Formula No. D-179

¹H-NMR (CDCl₃) δ(ppm) : 5.18 (2H, s, 1-NCH₂Ar), 5.01 (1H, d, J=14Hz, NCH₂Ar), 4.42 (1H, d, J=14Hz, NCH₂Ar), 1.95 (3H, s, 3-CH₃), 1.83 (3H, s, COCH₃), 1.64 (3H, s, 5-CH₃).
MASS (EI) m/z : 359(M⁺), 316, 243, 201, 186, 116.

### (Example 2036)

### Compound of Formula No. D-180

¹H-NMR (CDCl₃) δ(ppm) : 5.41 (2H, s, 1-NCH₂Ar), 5.00 (1H, d, J=14Hz, NCH₂Ar), 4.55 (1H, d, J=14Hz, NCH₂Ar), 1.93 (3H, s, 3-CH₃), 1.85 (3H, s, COCH₃), 1.83 (3H, s, 5-CH₃).
MASS (EI) m/z : 399(M⁺), 356, 293, 283, 253, 225, 167, 149.

### (Example 2037)

### Compound of Formula No. D-181

¹H-NMR (CDCl₃) δ(ppm) : 5.57 (2H, s, 1-NCH₂Ar), 5.00 (1H, d, J=14Hz, NCH₂Ar), 4.48 (1H, d, J=14Hz, NCH₂Ar), 1.96 (3H, s, 3-CH₃), 1.86 (3H, s, COCH₃), 1.75 (3H, s, 5-CH₃).
MASS (EI) m/z : 415(M⁺), 372, 299, 257, 243, 225, 148.

### (Example 2038)

### Compound of Formula No. D-182

¹H-NMR (CDCl₃) δ(ppm) : 5.19 (2H, s, 1-NCH₂Ar), 5.02 (1H, d, J=14Hz, NCH₂Ar), 4.40 (1H, d, J=14Hz, NCH₂Ar), 1.94 (3H, s, 3-CH₃), 1.82 (3H, s, COCH₃), 1.69 (3H, s, 5-CH₃).
MASS (EI) m/z : 398(M⁺), 282, 226, 149, 131.

### (Example 2039)

### Compound of Formula No. D-183

¹H-NMR (CDCl₃) δ(ppm) : 4.83 (1H, d, J=14Hz, NCH₂Ar), 4.68 (1H, d, J=14Hz, NCH₂Ar), 2.66 (3H, s, 1-COMe), 2.11 (3H, s, 3-CH₃), 1.90 (6H, s, COCH₃ & 5-CH₃), 1.81 (3H, s, NCOMe).
MASS (EI) m/z : 310(M⁺), 293, 268, 226, 152, 138, 116.

### (Example 2040)

### Compound of Formula No. D-184

¹H-NMR (CDCl₃) δ(ppm) : 4.85 (1H, d, J=14Hz, NCH₂Ar), 4.69 (1H, d, J=14Hz, NCH₂Ar), 2.24 (3H, s, 3-CH₃), 1.90 (6H, s, COCH₃ & 5-CH₃).
MASS (EI) m/z : 372(M⁺), 330, 307, 293, 267, 225, 199.

### (Example 2041)

### Compound of Formula No. D-185

¹H-NMR (CDCl₃) δ(ppm) : 4.75 (1H, d, J=14Hz, NCH₂Ar), 4.64 (1H, d, J=14Hz, NCH₂Ar), 3.73 (3H, brs, COOCH₃), 2.29 (3H, s, 3-CH₃), 1.85 (3H, s, 5-CH₃).
MASS (EI) m/z 388(M⁺), 284, 168, 116.

### (Example 2042)

### Compound of Formula No. D-186

¹H-NMR (CDCl₃) δ(ppm) : 4.86 (1H, d, J=14Hz, NCH₂Ar), 4.66 (1H, d, J=14Hz, NCH₂Ar), 2.23 (3H, s, 3-CH₃), 1.92 (3H, s, COCH₃), 1.89 (3H, s, 5-CH₃).
MASS (EI) m/z : 390(M⁺), 361, 348, 331, 308, 149.

### (Example 2043)

### Compound of Formula No. D-187

¹H-NMR (CDCl₃) δ(ppm) : 4.74 (1H, d, J=14Hz, NCH₂Ar), 4.64 (1H, d, J=14Hz, NCH₂Ar), 3.74 (3H, brs, COOCH₃), 2.29 (3H, s, 3-CH₃), 1.86 (3H, s, 5-CH₃).
MASS (EI) m/z : 406(M⁺), 378, 347, 290, 123.

### (Example 2044)

### Compound of Formula No. D-188

¹H-NMR (CDCl₃) δ(ppm) : 4.89 (1H, d, J=14Hz, NCH₂Ar), 4.64 (1H, d, J=14Hz, NCH₂Ar), 2.23 (3H, s, 3-CH₃), 1.93 (3H, s, COCH₃), 1.90 (3H, s, 5-CH₃).
MASS (EI) m/z : 397 (M⁺), 355, 281, 267, 239, 225, 197.

### (Example 2045)

### Compound of Formula No. D-189

¹H-NMR (CDCl₃) δ(ppm) : 4.73 (1H, d, J=15Hz, NCH₂Ar), 4.66 (1H, d, J=15Hz, NCH₂Ar), 3.74 (3H, brs, COOCH₃), 2.30 (3H, s, 3-CH₃), 1.87 (3H, s, 5-CH₃).
MASS (EI) m/z : 413(M⁺), 365, 297, 284, 228, 168.

### (Example 2046)

### Compound of Formula No. D-191

¹H-NMR (CDCl₃) δ(ppm) : 4.83 (1H, d, J=14Hz, NCH₂Ar), 4.59 (1H, d, J=14Hz, NCH₂Ar), 2.05 (3H, s, 3-CH₃), 1.92 (3H, s, COCH₃), 1.83 (3H, s, 5-CH₃), 1.66 (9H, s, NC(CH₃)₃).
MASS (EI) m/z : 368(M⁺), 309, 268, 226, 152.

### (Example 2047)

### Compound of Formula No. D-192

¹H-NMR (CDCl₃) δ(ppm) : 5.04 (1H, d, J=14Hz, NCH₂Ar), 4.46 (1H, d, J=14Hz, NCH₂Ar), 1.96 (3H, s, 3-CH₃), 1.88 (3H, s, COCH₃), 1.59 (3H, s, 5-CH₃).
MASS (EI) m/z : 368(M⁺), 339, 325, 293, 149.

### (Example 2048)

### Compound of Formula No. D-193

¹H-NMR (CDCl₃) δ(ppm) : 5.14 (1H, d, J=14Hz, NCH₂Ar), 4.35 (1H, d, J=14Hz, NCH₂Ar), 2.00 (3H, s, 3-CH₃), 1.87 (3H, s, COCH₃), 1.51 (3H, s, 5-CH₃).
MASS (EI) m/z : 382(M⁺), 354, 339, 266.

### (Example 2049)

### Compound of Formula No. D-194

¹H-NMR (CDCl₃) δ(ppm) : 5.11 (1H, d, J=14Hz, NCH₂Ar), 4.39 (1H, d, J=14Hz, NCH₂Ar), 2.01 (3H, s, 3-CH₃), 1.89 (3H, s, COCH₃), 1.61 (3H, s, 5-CH₃).
MASS (EI) m/z : 369(M⁺), 353, 326, 116.

### (Example 2050)

### Compound of Formula No. D-195

¹H-NMR (CDCl₃) δ(ppm) : 5.14 (1H, d, J=14Hz, NCH₂Ar), 4.35 (1H, d, J=14Hz, NCH₂Ar), 2.02 (3H, s, 3-CH₃), 1.87 (3H, s, COCH₃), 1.57 (3H, s, 5-CH₃).
MASS (EI) m/z : 383(M⁺), 340, 310, 267, 116.

### (Example 2051)

### Compound of Formula No. D-196

¹H-NMR (CDCl₃) δ(ppm) : 4.94 (1H, d, J=14Hz, NCH₂Ar), 4.32 (1H, d, J=14Hz, NCH₂Ar), 1.98 (3H, s, 3-CH₃), 1.92 (3H, s, COCH₃), 1.74 (3H, s, 5-CH₃).
MASS (EI) m/z : 422(M⁺), 380, 306, 267, 242, 225, 186, 155.

### (Example 2052)

### Compound of Formula No. D-197

¹H-NMR (CDCl₃) δ(ppm) : 4.92 (1H, d, J=14Hz, NCH₂Ar), 4.52 (1H, d, J=14Hz, NCH₂Ar), 3.02 (6H, s, N(CH₃)₂), 1.97 (3H, s, 3-CH₃), 1.92 (3H, s, COCH₃), 1.83 (3H, s, 5-CH₃).
MASS (EI) m/z : 375(M⁺), 333, 268, 259, 225, 217, 166.

### (Example 2053)

### Compound of Formula No. D-19B

¹H-NMR (CDCl₃) δ(ppm) : 5.07 (1H, d, J=14Hz, NCH₂Ar), 4.46 (1H, d, J=14Hz, NCH₂Ar), 1.99 (3H, s, 3-CH₃), 1.89 (3H, s, COCH₃), 1.73 (3H, s, 5-CH₃).
MASS (EI) m/z : 319(M⁺), 276, 228, 186, 172, 145, 118.

### (Example 2054)

### Compound of Formula No. D-199

¹H-NMR (CDCl₃) δ(ppm) : 5.02 (1H, d, J=14Hz, NCH₂Ar), 4.47 (1H, d, J=14H2, NCH₂Ar), 2.01 (3H, s, 3-CH₃), 1.90 (3H, s, COCH₃), 1.80 (3H, s, 5-CH₃).
MASS (EI) m/z : 337(M⁺), 294, 228, 186, 172, 145, 118.

### (Example 2055)

### Compound of Formula No. D-200

¹H-NMR (CDCl₃) δ(ppm) : 5.03 (1H, d, J=14Hz, NCH₂Ar), 4.47 (1H, d, J=14Hz, NCH₂Ar), 3.76 (2H, s, COCH₂O), 1.99 (3H, s, 3-CH₃), 1.76 (3H, s, 5-CH₃).
MASS (EI) m/z : 367(M⁺), 352, 322, 294, 258, 228, 213, 185.

### (Example 2056)

### Compound of Formula No. D-201

¹H-NMR (CDCl₃) δ(ppm) : 4.83 (1H, d, J=14Hz, NCH₂Ar), 4.52 (1H, d, J=14Hz, NCH₂Ar), 3.71 (3H, brs, COOCH₃), 2.00 (3H, s, 3-CH₃), 1.84 (3H, s, 5-CH₃).
MASS (EI) m/z : 353(M⁺), 322, 294, 244, 212, 172, 159.

### (Example 2057)

### Compound of Formula No. D-202

¹H-NMR (CDCl₃) δ(ppm) : 5.05 (1H, d, J=14Hz, NCH₂Ar), 4.42 (1H, d, J=14Hz, NCH₂Ar), 2.03 (3H, s, 3-CH₃), 1.90 (3H, s, COCH₃), 1.78 (3H, s, 5-CH₃).
MASS (EI) m/z : 353(M⁺), 310, 228, 186, 172.

### (Example 2058)

### Compound of Formula No. D-203

¹H-NMR (CDCl₃) δ(ppm) : 5.06 (1H, d, J=14Hz, NCH₂Ar), 4.42 (1H, d, J=14Hz, NCH₂Ar), 3.76 (2H, s, COCH₂O), 2.01 (3H, s, 3-CH₃), 1.75 (3H, s, 5-CH₃).
MASS (EI) m/z : 383(M⁺), 368, 338, 310, 293, 258, 228.

### (Example 2059)

### Compound of Formula No. D-204

¹H-NMR (CDCl₃) δ(ppm) : 4.84 (1H, d, J=14Hz, NCH₂Ar), 4.48 (1H, d, J=14Hz, NCH₂Ar), 3.71 (3H, brs, COOCH₃), 2.02 (3H, s, 3-CH₃), 1.82 (3H, s, 5-CH₃).
MASS (EI) m/z : 369(M⁺), 244, 212, 169.

### (Example 2060)

### Compound of Formula No. D-205

¹H-NMR (CDCl₃) δ(ppm) : 5.06 (1H, d, J=14Hz, NCH₂Ar), 4.39 (1H, d, J=14Hz, NCH₂Ar), 2.03 (3H, s, 3-CH₃), 1.90 (3H, s, COCH₃), 1.77 (3H, s, 5-CH₃).
MASS (EI) m/z : 397(M⁺), 356, 228, 186, 172.

### (Example 2061)

### Compound of Formula No. D-206

¹H-NMR (CDCl₃) δ(ppm) : 5.07 (1H, d, J=14Hz, NCH₂Ar), 4.39 (1H, d, J=14Hz, NCH₂Ar), 3.76 (2H, s, COCH₂O), 2.02 (3H, s, 3-CH₃), 1.74 (3H, s, 5-CH₃).
MASS (EI) m/z : 427(M⁺), 412, 382, 354, 258, 228, 172.

### (Example 2062)

### Compound of Formula No. D-207

¹H-NMR (CDCl₃) δ(ppm) : 4.85 (1H, d, J=14Hz, NCH₂Ar), 4.45 (1H, d, J=14Hz, NCH₂Ar), 3.71 (3H, brs, COOCH₃), 20.3 (3H, s, 3-CH₃), 1.81 (3H, s, 5-CH₃).
MASS (EI) m/z : 413(M⁺), 244, 212, 171, 118.

### (Example 2063)

### Compound of Formula No. D-208

¹H-NMR (CDCl₃) δ(ppm) : 4.96 (1H, d, J=14Hz, NCH₂Ar), 4.47 (1H, d, J=14Hz, NCH₂Ar), 2.04 (3H, s, 3-CH₃), 1.91 (3H, s, COCH₃), 1.87 (3H, s, 5-CH₃).
MASS (EI) m/z : 355(M⁺), 312, 228, 186, 172, 145, 127.

### (Example 2064)

### Compound of Formula No. D-209

¹H-NMR (CDCl₃) δ(ppm) : 4.97 (1H, d, J=14Hz, NCH₂Ar), 4.48 (1H, d, J=14Hz, NCH₂Ar), 3.77 (2H, s, COCH₂O), 2.02 (3H, s, 3-CH₃), 1.85 (3H, s, 5-CH₃).
MASS (EI) m/z : 385(M⁺), 370, 340, 312, 258, 228, 213, 172.

### (Example 2065)

### Compound of Formula No. D-210

¹H-NMR (CDCl₃) δ(ppm) : 4.79 (1H, d, J=14Hz, NCH₂Ar), 4.51 (1H, d, J=14Hz, NCH₂Ar), 3.72 (3H, brs, COOCH₃), 2.03 (3H, s, 3-CH₃), 1.91 (3H, s, 5-CH₃).
MASS (EI) m/z : 371(M⁺), 340, 312, 244, 212, 172, 159, 127.

### (Example 2066)

### Compound of Formula No. D-211

¹H-NMR (CDCl₃) δ(ppm) : 5.00 (1H, d, J=14Hz, NCH₂Ar), 4.39 (1H, d, J=14H2, NCH₂Ar), 2.06 (3H, s, 3-CH₃), 1.91 (3H, s, COCH₃), 1.83 (3H, s, 5-CH₃).
MASS (EI) m/z : 387(M⁺), 344, 228, 186, 172.

### (Example 2067)

### Compound of Formula No. D-212

¹H-NMR (CDCl₃) δ(ppm) : 5.01 (1H, d, J=14Hz, NCH₂Ar), 4.39 (1H, d, J=14Hz, NCH₂Ar), 3.76 (2H, s, COCH₂O), 2.04 (3H, s, 3-CH₃), 1.81 (3H, s, 5-CH₃).
MASS (EI) m/z : 417(M⁺), 402, 372, 344, 258, 228, 213, 172.

### (Example 2068)

### Compound of Formula No. D-213

¹H-NMR (CDCl₃) δ(ppm) : 4.82 (1H, d, J=14Hz, NCH₂Ar), 4.44 (1H, d, J=14Hz, NCH₂Ar), 3.72 (3H, brs, COOCH₃), 2.05 (3H, s, 3-CH₃), 1.87 (3H, s, 5-CH₃).
MASS (EI) m/z : 403(M⁺), 244, 212, 159, 118.

### (Example 2069)

### Compound of Formula No. D-215

¹H-NMR (CDCl₃) δ(ppm) : 5.04 (1H, d, J=14Hz, NCH₂Ar), 4.35 (1H, d, J=14Hz, NCH₂Ar), 3.76 (2H, s, COCH₂O), 2.05 (3H, s, 3-CH₃), 1.79 (3H, s, 5-CH₃).
MASS (EI) m/z : 505(M⁺), 492, 462, 434, 396, 258, 249, 228.

### (Example 2070)

### Compound of Formula No. D-216

¹H-NMR (CDCl₃) δ(ppm) : 4.83 (1H, d, J=14Hz, NCH₂Ar), 4.41 (1H, d, J=14Hz, NCH₂Ar), 3.72 (3H, brs, COOCH₃), 2.06 (3H, s, 3-CH₃), 1.86 (3H, s, 5-CH₃).
MASS (EI) m/z : 491(M⁺), 293, 244, 212, 172.

### (Example 2071)

### Compound of Formula No. D-217

¹H-NMR (CDCl₃) δ(ppm) : 5.05 (1H, d, J=14Hz, NCH₂Ar), 4.34 (1H, d, J=14Hz, NCH₂Ar), 2.04 (3H, s, 3-CH₃), 1.90 (3H, s, COCH₃), 1.75 (3H, s, 5-CH₃).
MASS (EI) m/z : 445(M⁺), 402, 228, 217, 186, 172, 145, 118.

### (Example 2072)

### Compound of Formula No. D-218

5.09(1H, d, J=13.3Hz, COCH₂), 5.07(1H, d, J=14.0Hz, NCH₂), 4.49(1H, d, J=13.3Hz, COCH₂), 4.41(1H, d, J=14.0Hz, NCH₂), 4.20(2H, m, OCH₂),
MASS (EI) m/z : 517 (M⁺), 472, 402, 391, 300, 213, 172.

### (Example 2073)

### Compound of Formula No. D-219

¹H-NMR (CDCl₃) δ(ppm) : 4.80(1H, brd, J=14.5Hz, NCH₂), 4.37(1H, d, J=14.5Hz, NCH₂), 1.56 and 1.26(9H, s x 2, tBu)
MASS (EI) m/z : 503(M⁺), 447, 403, 230, 186.

### (Example 2074)

### Compound of Formula No. D-220

¹H-NMR (CDCl₃) δ(ppm) : 5.02 (1H, d, J=14Hz, NCH₂Ar), 4.42 (1H, d, J=14Hz, NCH₂Ar), 1.99 (3H, s, 3-CH₃), 1.89 (3H, s, COCH₃), 1.73 (3H, s, 5-CH₃).
MASS (EI) m/z : 333(M⁺), 318, 290, 228, 213, 186, 172, 145.

### (Example 2075)

### Compound of Formula No. D-221

¹H-NMR (CDCl₃) δ(ppm) : 5.05 (1H, d, J=14Hz, NCH₂Ar), 4.42 (1H, d, J=14Hz, NCH₂Ar), 3.75 (2H, s, COCH₂O), 1.98 (3H, s, 3-CH₃), 1.71 (3H, s, 5-CH₃).
MASS (EI) m/z : 363(M⁺), 348, 333, 318, 290, 258, 228, 172.

### (Example 2076)

### Compound of Formula No. D-222

¹H-NMR (CDCl₃) δ(ppm) : 4.83 (1H, d, J=14Hz, NCH₂Ar), 4.48 (1H, d, J=14Hz, NCH₂Ar), 3.71 (3H, brs, COOCH₃), 1.99 (3H, s, 3-CH₃), 1.79 (3H, s, 5-CH₃).
MASS (EI) m/z : 349(M⁺), 318, 290, 244, 212, 172.

### (Example 2077)

### Compound of Formula No. D-223

¹H-NMR (CDCl₃) δ(ppm) : 4.99 (1H, d, J=14Hz, NCH₂Ar), 4.38 (1H, d, J=14Hz, NCH₂Ar), 2.00 (3H, s, 3-CH₃), 1.89 (3H, s, COCH₃), 1.74 (3H, s, 5-CH₃).

MASS (EI) m/z : 347(M⁺), 332, 304, 228, 186, 172, 145, 119-

### (Example 2078)

### Compound of Formula No. D-224

¹H-NMR (CDCl₃) δ(ppm) : 5.11 (1H, d, J=14Hz, NCH₂Ar), 4.45 (1H, d, J=14Hz, NCH₂Ar), 2.01 (3H, s, 3-CH₃), 1.90 (3H, s, COCH₃), 1.72 (3H, s, 5-CH₃).
MASS (EI) m/z : 351(M⁺), 308, 228, 172, 145.

### (Example 2079)

### Compound of Formula No. D-225

¹H-NMR (CDCl₃) δ(ppm) : 5.15 (1H, d, J=14Hz, NCH₂Ar), 4.49 (1H, d, J=14Hz, NCH₂Ar), 2.02 (3H, s, 3-CH₃), 1.91 (3H, s, COCH₃), 1.73 (3H, s, 5-CH₃).
MASS (EI) m/z : 351(M⁺), 336, 311, 253, 105.

### (Example 2080)

### Compound of Formula No. D-226

¹H-NMR (CDCl₃) δ(ppm) : 5.01 (1H, d, J=14Hz, NCH₂Ar), 4.49 (1H, d, J=14Hz, NCH₂Ar), 1.97 (3H, s, 3-CH₃), 1.88 (3H, s, COCH₃), 1.76 (3H, s, 5-CH₃).
MASS (EI) m/z : 349(M⁺), 306, 228, 172, 118.

### (Example 2081)

### Compound of Formula No. D-227

¹H-NMR (CDCl₃) δ(ppm) : 5.18 (1H, d, J=14Hz, NCH₂Ar), 4.47 (1H, d, J=14Hz, NCH₂Ar), 2.03 (3H, s, 3-CH₃), 1.91 (3H, s, COCH₃), 1.71 (3H, s, 5-CH₃).
MASS (EI) m/z : 395(M⁺), 352, 228, 172, 118.

### (Example 2082)

### Compound of Formula No. D-228

¹H-NMR (CDCl₃) δ(ppm) : 5.46 (1H, d, J=14Hz, NCH₂Ar), 4.64 (1H, d, J=14Hz, NCH₂Ar), 2.01 (3H, s, 3-CH₃), 1.95 (3H, s, COCH₃), 1.64 (3H, s, 5-CH₃).
MASS (EI) m/z : 344(M⁺), 329, 301, 228, 186, 172, 157, 145.

### (Example 2083)

### Compound of Formula No. D-230

¹H-NMR (CDCl₃) δ(ppm) : 4.97 (1H, d, J=14Hz, N-CH₂-Ph), 4.64 (1H, d, J=14Hz, N-CH₂-Ph), 3.88(2H, s, COCH₂Cl), 1.98(3H, s, 3-Me), 1.85(3H, 5-Me)
MASS (EI) m/z : 378(M⁺), 343, 301, 262, 234, 213, 185.

### (Example 2084)

### Compound of Formula No. D-231

¹H-NMR (CDCl₃) δ(ppm) : 5.00 (1H, d, J=14Hz, NCH₂Ar), 4.43 (1H, d, J=14Hz, NCH₂Ar), 1.99 (3H, s, 3-CH₃), 1.89 (3H, s, COCH₃), 1.75 (3H, s, 5-CH₃).
MASS (EI) m/z : 333(M⁺), 318, 290, 228, 213, 186, 172.

### (Example 2085)

### Compound of Formula No. D-232

¹H-NMR (CDCl₃) δ(ppm) : 4.99 (1H, d, J=14Hz, N-CH₂-Ph), 4.47 (1H, d, J=14Hz, N-CH₂-Ph), 3.87(2H, s, COCH₂Cl), 1.98(3H, s, 3-Me), 1.78(3H, 5-Me)
MASS (EI) m/z : 383(M⁺), 334, 279, 213, 172, 149, 121.

### (Example 2086)

### Compound of Formula No. D-233

¹H-NMR (CDCl₃) δ(ppm) : 5.01 (1H, d, J=14Hz, NCH₂Ar), 4.45 (1H, d, J=14Hz, NCH₂Ar), 2.00 (3H, s, 3-CH₃), 1.89 (3H, s, COCH₃), 1.79 (3H, s, 5-CH₃).
MASS (EI) m/z : 353(M⁺), 310, 228, 213, 186, 172, 145.

### (Example 2087)

### Compound of Formula No. D-234

¹H-NMR (CDCl₃) δ(ppm) : 4.99 (1H, d, J=14Hz, NCH₂Ar), 4.41 (1H, d, J=14Hz, NCH₂Ar), 1.99 (3H, s, 3-CH₃), 1.88 (3H, s, COCH₃), 1.76 (3H, s, 5-CH₃).
MASS (EI) m/z : 349(M⁺), 279, 228, 172, 149, 121, 104.

### (Example 2088)

### Compound of Formula No. D-235

¹H-NMR (CDCl₃) δ(ppm) : 5.01 (1H, d, J=14Hz, NCH₂Ar), 4.44 (1H, d, J=14Hz, NCH₂Ar), 2.13-2.00 (2H, m, COCH₂Me), 1.98 (3H, s, 3-CH₃), 1.77 (3H, s, 5-CH₃).
MASS (EI) m/z : 367(M⁺), 310., 242, 214, 186, 172, 145.

### (Example 2089)

### Compound of Formula No. D-236

¹H-NMR (CDCl₃) δ(ppm) : 5.00 (1H, d, J=14Hz, NCH₂Ar), 4.57 (1H, d, J=14Hz, NCH₂Ar), 2.09 (2H, qd, J=8Hz, 2Hz, COCH₂Me), 1.97 (3H, S, 3-CH₃), 1.81 (3H, S, 5-CH₃).
MASS (EI) m/z : 358(M⁺), 301, 242, 172, 118.

### (Example 2090)

### Compound of Formula No. D-237

¹H-NMR (CDCl₃) δ(ppm) : 5.00 (1H, d, J=14Hz, NCH₂Ar), 4.57 (1H, d, J=14Hz, NCH₂Ar), 2.06 (1H, t, J=8Hz, COCH₂Et), 2.04 (1H, t, J=7Hz, COCH₂Et), 1.97 (3H, s, 3-CH₃), 1.81 (3H, s, 5-CH₃).
MASS (EI) m/z : 372(M⁺), 302, 256, 172, 118.

### (Example 2091)

### Compound of Formula No. D-238

¹H-NMR (CDCl₃) δ(ppm) : 4.98 (1H, d, J=14Hz, NCH₂Ar), 4.62 (1H, d, J=14Hz, NCH₂Ar), 2.02 (3H, s, 3-CH₃), 1.87 (3H, s, 5-CH₃), 1.54-1.46 (1H, m, COCH(CH₂)₂).
MASS (EI) m/z : 370(M⁺), 302, 254, 172, 118.

### (Example 2092)

### Compound of Formula No. D-239

¹H-NMR (CDCl₃) δ(ppm) : 4.98 (1H, d, J=14Hz, NCH₂Ar), 4.57 (1H, d, J=14Hz, NCH₂Ar), 2.60-2.46 (1H, m, COCHMe₂), 1.98 (3H, s, 3-CH₃), 1.82 (3H, s, 5-CH₃).
MASS (EI) m/z : 372(M⁺), 301, 214, 172, 118.

### (Example 2093)

### Compound of Formula No. D-240

¹H-NMR (CDCl₃) δ(ppm) : 5.00 (1H, d, J=14Hz, NCH₂Ar), 4.57 (1H, d, J=14Hz, NCH₂Ar), 2.11-2.00 (2H, m, COCH₂-C₆H₁₃), 1.97 (3H, s, 3-CH₃), 1.80 (3H, s, 5-CH₃).
MASS (EI) m/z : 428(M⁺), 357, 344, 302, 118.

### (Example 2094)

### Compound of Formula No. D-241

¹H-NMR (CDCl₃) δ(ppm) : 5.00 (1H, d, J=14Hz, NCH₂Ar), 4.57 (1H, d, J=14Hz, NCH₂Ar), 2.07-2.02 (2H, m, COCH₂-C₁₄H₂₉), 1.97 (3H, s, 3-CH₃), 1.80 (3H, s, 5-CH₃).
MASS (EI) m/z : 540(M⁺), 424, 344, 302, 172.

### (Example 2095)

### Compound of Formula No. D-242

¹H-NMR (CDCl₃) δ(ppm) : 5.02 (1H, d, J=14Hz, NCH₂Ar), 4.64 (1H, d, J=14Hz, NCH₂Ar), 3.91-3.69 (2H, m, COCH₂O), 1.96 (3H, s, 3-CH₃), 1.80 (3H, s, 5-CH₃).
MASS (EI) m/z : 360(M⁺), 329, 301, 214, 118.

### (Example 2096)

### Compound of Formula No. D-243

¹H-NMR (CDCl₃) δ(ppm) : 5.02 (1H, d, J=14Hz, NCH₂Ar), 4.58 (1H, d, J=14Hz, NCH₂Ar), 3.76 (2H, s, COCH₂O), 1.97 (3H, s, 3-CH₃), 1.80 (3H, s, 5-CH₃).
MASS (EI) m/z : 374(M⁺), 359, 329, 301, 258, 228.

### (Example 2097)

### Compound of Formula No. D-244

¹H-NMR (CDCl₃) δ(ppm) : 5.02 (1H, d, J=14Hz, NCH₂Ar), 4.64 (1H, d, J=14Hz, NCH₂Ar), 4.41 (1H, d, J=15Hz, COCH₂O), 4.40 (1H, d, J=15Hz, COCH₂O), 2.02 (3H, s, 3-CH₃), 1.84 (3H, s, 5-CH₃).
MASS (EI) m/z : 436(M⁺), 343, 320, 107.

### (Example 2098)

### Compound of Formula No. D-245

¹H-NMR (CDCl₃) δ(ppm) : 4.98 (1H, d, J=14Hz, NCH₂Ar), 4.59 (1H, d, J=14Hz, NCH₂Ar), 4.37 (1H, d, J=15Hz, COCH₂O), 4.36 (1H, d, J=15Hz, COCH₂O), 2.02 (3H, s, 3-CH₃), 1.87 (3H, s, 5-CH₃).
MASS (EI) m/z : 402(M⁺), 359, 300, 214, 118.

### (Example 2099)

### Compound of Formula No. D-246

¹H-NMR (CDCl₃) δ(ppm) : 4.94 (1H, d, J=14Hz, NCH₂Ar), 4.63 (1H, d, J=14Hz, NCH₂Ar), 3.06 (2H, s, COCH₂S), 1.99 (3H, s, 3-CH₃), 1.87 (3H, s, 5-CH₃).
MASS (EI) m/z : 390(M⁺), 375, 344, 329, 301, 274, 228, 185.

### (Example 2100)

### Compound of Formula No. D-247

¹H-NMR (CDCl₃) δ(ppm) : [4.99 (d, J=14Hz) & 4.92 (d, J=14Hz), 1H, NCH₂Ar], [4.68 (d, J=14Hz) & 4.62 (d, J=14Hz), 1H, NCH₂Ar], [3.75 (d, J=12Hz) & 3.68 (d, J=12Hz), 1H, COCH₂S], [3.58 (d, J=12Hz) & 3.53 (d, J=12Hz), 1H, COCH₂S], [2.05 (s) & 2.01 (s), 3H, 3-CH₃], [1.88 (s) & 1.85 (s), 3H, 5-CH₃].
MASS (EI) m/z : 406(M⁺), 391, 358, 343, 329, 301, 227.

### (Example 2101)

### Compound of Formula No. D-248

¹H-NMR (CDCl₃) δ(ppm) : 4.94 (1H, d, J=14Hz, NCH₂Ar), 4.73 (1H, d, J=14Hz, NCH₂Ar), 3.93 (1H, d, J=12Hz, COCH₂S), 3.88 (1H, d, J=12Hz, COCH₂S), 1.95 (3H, s, 3-CH₃), 1.89 (3H, s, 5-CH₃).
MASS (EI) m/z : 422(M⁺), 343, 306, 227, 213, 186, 169.

### (Example 2102)

### Compound of Formula No. D-249

¹H-NMR (CDCl₃) δ(ppm) : 4.93 (1H, d, J=14Hz, NCH₂Ar), 4.63 (1H, d, J=14Hz, NCH₂Ar), 3.10 (2H, s, COCH₂S), 1.99 (3H, s, 3-CH₃), 1.87 (3H, s, 5-CH₃).
MASS (EI) m/z : 404(M⁺), 375, 344, 329, 301, 228, 185.

### (Example 2103)

### Compound of Formula No. D-250

¹H-NMR (CDCl₃) δ(ppm) : [4.98 (d, J=13Hz) & 4.82 (d, J=13Hz), 1H, NCH₂Ar], [4.63 (d, J=13Hz) & 4.62 (d, J=13Hz), 1H, NCH₂Ar], [3.64 (d, J=14Hz) & 3.61 (s), 1H, COCH₂S], [3.61 (s) & 3.55 (d, J=14Hz), 1H, COCH₂S], [2.01 (s) & 1.96 (s), 3H, 3-CH₃], [1.89 (s) & 1.85 (s), 3H, 5-CH₃].
MASS (EI) m/z : 420(M⁺), 403, 391, 358, 343, 329, 227.

### (Example 2104)

### Compound of Formula No. D-251

¹H-NMR (CDCl₃) δ(ppm) : 4.92 (1H, d, J=14Hz, NCH₂Ar), 4.74 (1H, d, J=14Hz, NCH₂Ar), 3.90 (1H, d, J=14Hz, COCH₂S), 3.87 (1H, d, J=14Hz, COCH₂S), 1.96 (3H, s, 3-CH₃), 1.91 (3H, s, 5-CH₃).
MASS (EI) m/z : 436(M⁺), 354, 343, 320, 301, 238, 214.

### (Example 2105)

### Compound of Formula No. D-252

¹H-NMR (CDCl₃) δ(ppm) : 4.92 (1H, d, J=14Hz, NCH₂Ar), 4.64 (1H, d, J=14Hz, NCH₂Ar), 3.13 (1H, d, J=14Hz, COCH₂S), 3.11 (1H, d, J=14Hz, COCH₂S), 1.99 (3H, s, 3-CH₃), 1.85 (3H, s, 5-CH₃).
MASS (EI) m/z : 418(M⁺), 403, 375, 344, 329, 301, 228, 185.

### (Example 2106)

### Compound of Formula No. D-253

¹H-NMR (CDCl₃) δ(ppm) : [4.95 (d, J=14Hz) & 4.93 (d, J=14Hz), 1H, NCH₂Ar], [4.70 (d, J=13Hz) & 4.67 (d, J=14Hz), 1H, NCH₂Ar], [3.55 (d, J=14Hz) & 3.49 (s), 1H, COCH₂S], [3.49 (s) & 3.47 (d, J=14Hz), 1H, COCH₂S], [2.01 (s) & 1.96 (s), 3H, 3-CH₃], [1.90 (s) & 1.87 (s), 3H, 5-CH₃].
MASS (EI) m/z : 434(M⁺), 392, 375, 343, 329, 301, 228.

### (Example 2107)

### Compound of Formula No. D-254

¹H-NMR (CDCl₃) δ(ppm) : 4.91 (1H, d, J=14Hz, NCH₂Ar), 4.76 (1H, d, J=14Hz, NCH₂Ar), [3.95 (d, J=14Hz) & 3.93 (d, J=14Hz), 1H, COCH₂S], [3.90 (d, J=14Hz) & 3.88 (d, J=14Hz), 1H, COCH₂S], 1.95 (3H, s, 3-CH₃), 1.92 (3H, s, 5-CH₃).
MASS (EI) m/z : 450(M⁺), 343, 301, 227, 214, 185, 172.

### (Example 2108)

### Compound of Formula No. D-255

¹H-NMR (CDCl₃) δ(ppm) : 4.93 (1H, d, J=14Hz, NCH₂Ar), 4.63 (1H, d, J=14Hz, NCH₂Ar), 3.08 (2H, s, COCH₂S), 1.99 (3H, s, 3-CH₃), 1.87 (3H, s, 5-CH₃).
MASS (EI) m/z : 460(M⁺), 375, 344, 301, 228, 213, 185.

### (Example 2109)

### Compound of Formula No. D-256

¹H-NMR (CDCl₃) δ(ppm) : [4.98 (d, J=14Hz) & 4.92 (d, J=14Hz), 1H, NCH₂Ar], [4.68 (d, J=14Hz) & 4.63 (d, J=14Hz), 1H, NCH₂Ar], [3.63 (d, J=14Hz) & 3.58 (s), 1H, COCH₂S], [3.58 (s) & 3.52 (d, J=14Hz), 1H, COCH₂S], [2.01 (s) & 1.96 (s), 3H, 3-CH₃], [1.88 (s) & 1.85 (s), 3H, 5-CH₃].
MASS (EI) m/z : 476(M⁺), 459, 391, 343, 329, 301, 228.

### (Example 2110)

### Compound of Formula No. D-257

¹H-NMR (CDCl₃) δ(ppm) : 4.92 (1H, d, J=14Hz, NCH₂Ar), 4.73 (1H, d, J=14Hz, NCH₂Ar), 3.89 (1H, d, J=14Hz, COCH₂S), 3.86 (1H, d, J=14Hz, COCH₂S), 1.95 (3H, s, 3-CH₃), 1.90 (3H, s, 5-CH₃).
MASS (EI) m/z : 492(M⁺), 376, 343, 301, 227, 214, 185.

### (Example 2111)

### Compound of Formula No. D-258

¹H-NMR (CDCl₃) δ(ppm) : 4.93 (1H, d, J=14Hz, NCH₂Ar), 4.63 (1H, d, J=14Hz, NCH₂Ar), 3.54 (1H, d, J=14Hz, COCH₂S), 3.52 (1H, d, J=14Hz, COCH₂S), 1.95 (3H, s, 3-CH₃), 1.82 (3H, s, 5-CH₃).
MASS (EI) m/z : 452(M⁺), 359, 343, 329, 301, 212, 186, 172.

### (Example 2112)

### Compound of Formula No. D-259

¹H-NMR (CDCl₃) δ(ppm) : [5.02 (d, J=14Hz) & 4.83 (d, J=14Hz), 1H, NCH₂Ar], [4.58 (d, J=14Hz) & 4.48 (d, J=14Hz), 1H, NCH₂Ar], [3.85 (d, J=14Hz) & 3.81 (d, J=14Hz), 1H, COCH₂S], 3.53 (1H, d, J=14Hz, COCH₂S), [2.02 (s) & 1.90 (s), 3H, 3-CH₃], 1.57 (3H, S, 5-CH₃).
MASS (EI) m/z : 468(M⁺), 452, 420, 343, 301, 227, 214.

### (Example 2113)

### Compound of Formula No. D-260

¹H-NMR (CDCl₃) δ (ppm) : 4.88 (1H, d, J=14Hz, NCH₂Ar), 4.67 (1H, d, J=14Hz, NCH₂Ar), 4.14 (1H, d, J=12Hz, COCH₂S), 4.06 (1H, d, J=12Hz, COCH₂S), 1.90 (6H, brs, 3-CH₃ & 5-CH₃).
MASS (EI) m/z : 484(M⁺), 451, 368, 343, 301, 226, 214.

### (Example 2114)

### Compound of Formula No. D-261

¹H-NMR (CDCl₃) δ(ppm) : [5.27 (d, J=14Hz) & 4.79 (d, J=14Hz), 1H, NCH₂Ar], [4.77 (d, J=14Hz) & 4.31 (d, J=14Hz), 1H, NCH₂Ar], 4.24 (1H, dd, J=10Hz, 5Hz, COCH(iPr)N), [2.13 (s) & 1.92 (s), 3H, 3-CH₃], [1.91 (s) & 1.69 (s), 3H, 5-CH₃].
MASS (EI) m/z : 487(M⁺), 428, 358, 330, 302, 214.

### (Example 2115)

### Compound of Formula No. D-262

¹H-NMR (CDCl₃) δ(ppm) : [5.25 (d, J=14Hz) & 4.78 (d, J=14Hz), 1H, NCH₂Ar], [4.77 (d, J=14Hz) & 4.32 (d, J=14Hz), 1H, NCH₂Ar], 4.15 (1H, dd, J=10Hz, 6Hz, COCH(iPr)N), [2.11 (s) & 1.91 (s), 3H, 3-CH₃], [1.89 (s) & 1.67 (s), 3H, 5-CH₃].
MASS (EI) m/z : 501(M⁺), 445, 428, 302, 57.

### (Example 2116)

### Compound of Formula No. D-263

¹H-NMR (CDCl₃) δ(ppm) : [5.25 (d, J=14Hz) & 4.77 (d, J=14Hz), 1H, NCH₂Ar], [4.76 (d, J=14Hz) & 4.31 (d, J=14Hz), 1H, NCH₂Ar], 4.15 (1H, dd, J=10Hz, 6Hz, COCH(iPr)N), [2.11 (s) & 1.91 (s), 3H, 3-CH₃], [1.89 (s) & 1.66 (s), 3H, 5-CH₃].
MASS (EI) m/z : 501(M⁺), 445, 427, 302, 57.

### (Example 2117)

### Compound of Formula No. D-264

¹H-NMR (CDCl₃) δ(ppm) : 4.88 (1H, d, J=14Hz, NCH₂Ar), 4.71 (1H, d, J=14Hz, NCH₂Ar), 4.47 (2H, d, J=1Hz, COCH₂N), 1.98 (3H, s, 3-CH₃), 1.73 (3H, s, 5-CH₃).
MASS (EI) m/z : 410(M⁺), 329, 301, 294, 252, 226, 186.

### (Example 2118)

### Compound of Formula No. D-265

¹H-NMR (CDCl₃) δ(ppm) : 4.97 (1H, d, J=14Hz, NCH₂Ar), 4.67 (1H, d, J=14Hz, NCH₂Ar), 4.75 (2H, s, COCH₂N), 2.05 (3H, s, 3-CH₃), 1.89 (3H, s, 5-CH₃).
MASS (EI) m/z : 410(M⁺), 329, 301, 294, 252, 226, 186.

### (Example 2119)

### Compound of Formula No. D-266

¹H-NMR (CDCl₃) δ(ppm) : 5.30 (1H, d, J=14Hz, NCH₂Ar), 4.63 (1H, d, J=14Hz, NCH₂Ar), 2.09 (3H, s, 3-CH₃), 1.54 (3H, s, 5-CH₃). MASS (EI) m/z : 406(M⁺), 301, 290, 172, 105.

### (Example 2120)

### Compound of Formula No. D-267

¹H-NMR (CDCl₃) δ(ppm) : 4.83 (1H, d, J=14Hz, NCH₂Ar), 4.59 (1H, d, J=14Hz, NCH₂Ar), 3.73 (3H, brs, COOCH₃), 1.99 (3H, s, 3-CH₃), 1.86 (3H, s, 5-CH₃).
MASS (EI) m/z : 360(M⁺), 293, 244, 212, 167, 149.

### (Example 2121)

### Compound of Formula No. D-268

¹H-NMR (CDCl₃) δ(ppm) : 4.83 (1H, d, J=14Hz, NCH₂Ar), 4.59 (1H, d, J=14Hz, NCH₂Ar), 4.18 (2H, q, J=7Hz, COOCH₂Me), 1.99 (3H, s, 3-CH₃), 1.87 (3H, s, 5-CH₃).
MASS (EI) m/z : 374(M⁺), 329, 301, 258, 118.

### (Example 2122)

### Compound of Formula No. D-269

¹H-NMR (CDCl₃) δ(ppm) : 5.10-4.88 (1H, m, COOCHMe₂), 4.84 (1H, d, J=14Hz, NCH₂Ar), 4.56 (1H, d, J=14Hz, NCH₂Ar), 1.99 (3H, s, 3-CH₃), 1.85 (3H, s, 5-CH₃).
MASS (EI) m/z : 388(M⁺), 346, 329, 230, 118.

### (Example 2123)

### Compound of Formula No. D-270

¹H-NMR (CDCl₃, measured at 100°C) δ(ppm) : 4.67(2H, brs, NCH₂), 2.01(3H, s, 3-Me), 1.87(3H, s, 5-Me), 1.43(9H, s, tBu).
MASS (EI) m/z : 402(M⁺), 346, 329, 302, 230.

### (Example 2124)

### Compound of Formula No. D-271

¹H-NMR (CDCl₃) δ(ppm) : 4.92 (1H, d, J=14Hz, NCH₂Ar), 4.67 (1H, d, J=14Hz, NCH₂Ar), 2.11 (3H, s, 3-CH₃), 1.97 (3H, s, 5-CH₃). MASS (EI) m/z : 422(M⁺), 329, 213, 116.

### (Example 2125)

### Compound of Formula No. D-272

¹H-NMR (CDCl₃) δ(ppm) : 5.00 (1H, d, J=14Hz, NCH₂Ar), 4.60 (1H, d, J=14Hz, NCH₂Ar), 2.30 (3H, s, COSCH₃), 2.00 (3H, s, 3-CH₃), 1.83 (3H, s, 5-CH₃).
MASS (EI) m/z : 376(M⁺), 329, 301, 293, 260, 213, 172.

### (Example 2126)

### Compound of Formula No. D-273

¹H-NMR (CDCl₃) δ(ppm) : 4.90 (1H, d, J=14Hz, NCH₂Ar), 4.59 (1H, d, J=14Hz, NCH₂Ar), 4.77 (2H, s, H₂NCO), 2.02 (3H, s, 3-CH₃), 1.89 (3H, s, 5-CH₃).
MASS (EI) m/z : 345(M⁺), 301, 229, 212, 172, 118.

### (Example 2127)

### Compound of Formula No. D-274

¹H-NMR (CDCl₃) δ(ppm) : 4.90 (1H, d, J=14Hz, NCH₂Ar), 4.60 (1H, d, J=14Hz, NCH₂Ar), 4.55 (1H, q, J=5Hz, CONHMe), 2.82 (3H, d, J=5Hz, CONHCH₃), 1.98 (3H, s, 3-CH₃), 1.87 (3H, s, 5-CH₃).
MASS (EI) m/z : 359(M⁺), 301, 243, 172, 118.

### (Example 2128)

### Compound of Formula No. D-275

¹H-NMR (CDCl₃) δ(ppm) : 4.61 (2H, brs, NCH₂Ar), 2.72 (6H, s, CON(CH₃)₂), 2.09 (3H, s, 3-CH₃), 1.82 (3H, s, 5-CH₃).
MASS (EI) m/z : 373(M⁺), 301, 257, 118.

### (Example 2129)

### Compound of Formula No. D-276

¹H-NMR (CDCl₃) δ(ppm) : 4.89 (1H, d, J=14Hz, NCH₂Ar), 4.84 (1H, d, J=14Hz, NCH₂Ar), 4.11 (3H, s, NOCH₃), 1.88 (6H, s, 3-CH₃ & 5-CH₃).
MASS (EI) m/z : 412(M⁺), 381, 355, 296, 239, 213, 118.

### (Example 2130)

### Compound of Formula No. D-277

¹H-NMR (CDCl₃) δ(ppm) : 5.23 (1H, d, J=14Hz, NCH₂Ar), 4.65 (1H, d, J=14Hz, NCH₂Ar), 2.04 (3H, s, 3-CH₃), 1.62 (3H, s, 5-CH₃). MASS (EI) m/z : 441(M⁺), 325, 172, 118.

### (Example 2131)

### Compound of Formula No. D-278

¹H-NMR (CDCl₃) δ(ppm) : 5.16 (1H, d, J=14Hz, NCH₂Ar), 4.69 (1H, d, J=14Hz, NCH₂Ar), 2.00 (3H, s, 3-CH₃), 1.68 (3H, s, 5-CH₃). MASS (EI) m/z : 475(M⁺), 359, 301, 172, 118.

### (Example 2132)

### Compound of Formula No. D-279

¹H-NMR (CDCl₃) δ(ppm) : 5.03 (1H, d, J=14Hz, NCH₂Ar), 4.58 (1H, d, J=14Hz, NCH₂Ar), 1.99 (3H, s, 3-CH₃), 1.91 (3H, s, COCH₃), 1.82 (3H, s, 5-CH₃).
MASS (EI) m/z : 344(M⁺), 301, 228, 186, 172, 145, 118.

### (Example 2133)

### Compound of Formula No. D-280

¹H-NMR (CDCl₃) δ(ppm) : 4.93 (1H, d, J=14Hz, NCH₂Ar), 4.69 (1H, d, J=14Hz, NCH₂Ar), 2.01 (3H, s, 3-CH₃), 1.93 (3H, s, COCH₃), 1.92 (3H, s, 5-CH₃).
MASS (EI) m/z : 369(M⁺), 326, 291, 228, 186, 172, 145.

### (Example 2134)

### Compound of Formula No. D-282

¹H-NMR (CDCl₃) δ(ppm) : 5.11 (1H, d, J=14Hz, NCH₂Ar), 4.35 (1H, d, J=14Hz, NCH₂Ar), 1.97 (3H, s, 3-CH₃), 1.85 (3H, s, COCH₃), 1.69 (3H, brs, 5-CH₃).
MASS (EI) m/z : 433(M⁺), 401, 358, 172, 145.

### (Example 2135)

### Compound of Formula No. D-283

¹H-NMR (CDCl₃) δ(ppm) : 5.05 (1H, d, J=14Hz, NCH₂Ar), 4.60 (1H, d, J=14Hz, NCH₂Ar), 1.99 (3H, s, 3-CH₃), 1.91 (3H, s, COCH₃), 1.79 (3H, s, 5-CH₃).
MASS (EI) m/z : 361(M⁺), 344, 318, 293, 149.

### (Example 2136)

### Compound of Formula No. D-285

¹H-NMR (CDCl₃) δ(ppm) : 5.12 (1H, d, J=14Hz, NCH₂Ar), 4.48 (1H, d, J=14Hz, NCH₂Ar), 2.01 (3H, s, 3-CH₃), 1.90 (3H, s, COCH₃), 1.73 (3H, s, 5-CH₃).
MASS (EI) m/z : 376(M⁺), 362, 344, 228, 118.

### (Example 2137)

### Compound of Formula No. D-286

¹H-NMR (CDCl₃) δ(ppm) : 5.11 (1H, d, J=14Hz, NCH₂Ar), 4.54 (1H, d, J=14Hz, NCH₂Ar), 2.02 (3H, s, 3-CH₃), 1.91 (3H, s, COCH₃), 1.78 (3H, s, 5-CH₃).
MASS (EI) m/z : 391(M⁺), 348, 228, 172, 118.

### (Example 2138)

### Compound of Formula No. D-287

¹H-NMR (CDCl₃) δ(ppm) : 5.12 (1H, d, J=14Hz, NCH₂Ar), 4.50 (1H, d, J=14Hz, NCH₂Ar), 2.03 (3H, s, 3-CH₃), 1.91 (3H, s, COCH₃), 1.76 (3H, s, 5-CH₃).
MASS (EI) m/z : 405(M⁺), 362, 319, 228, 118.

### (Example 2139)

### Compound of Formula No. D-288

¹H-NMR (CDCl₃) δ(ppm) : 5.09 (1H, d, J=14Hz, NCH₂Ar), 4.53 (1H, d, J=14Hz, NCH₂Ar), 2.00 (3H, s, 3-CH₃), 1.90 (3H, s, COCH₃), 1.77 (3H, s, 5-CH₃).
MASS (EI) m/z : 362(M⁺), 344, 319, 228.

### (Example 2140)

### Compound of Formula No. D-290

¹H-NMR (CDCl₃) δ(ppm) : 5.08 (1H, d, J=14Hz, NCH₂Ar), 4.52 (1H, d, J=14Hz, NCH₂Ar), 2.00 (3H, s, 3-CH₃), 1.90 (3H, s, COCH₃), 1.77 (3H, s, 5-CH₃).
MASS (EI) m/z : 404(M⁺), 377, 361, 118.

### (Example 2141)

### Compound of Formula No. D-291

¹H-NMR (CDCl₃) δ(ppm) : 5.04 (1H, d, J=14Hz, NCH₂Ar), 4.54 (1H, d, J=14Hz, NCH₂Ar), 1.98 (3H, s, 3-CH₃), 1.91 (3H, s, COCH₃), 1.78 (3H, s, 5-CH₃).
MASS (EI) m/z : 401(M⁺), 358, 228, 172, 118.

### (Example 2142)

### Compound of Formula No. D-292

¹H-NMR (CDCl₃) δ(ppm) : 5.06 (1H, d, J=14Hz, NCH₂Ar), 4.57 (1H, d, J=14Hz, NCH₂Ar), 2.00 (3H, s, 3-CH₃), 1.91 (3H, s, COCH₃), 1.80 (3H, s, 5-CH₃).
MASS (EI) m/z : 468(M⁺), 425, 346, 228, 118.

### (Example 2143)

### Compound of Formula No. D-293

¹H-NMR (CDCl₃) δ(ppm) : 5.11 (1H, d, J=14Hz, NCH₂Ar), 4.50 (1H, d, J=14Hz, NCH₂Ar), 2.02 (3H, s, 3-CH₃), 1.91 (3H, s, COCH₃), 1.75 (3H, s, 5-CH₃).
MASS (EI) m/z : 390(M⁺), 362, 344, 228, 118.

### (Example 2144)

### Compound of Formula No. D-294

¹H-NMR (CDCl₃) δ(ppm) : 5.10 (1H, d, J=14Hz, NCH₂Ar), 4.48 (1H, d, J=14Hz, NCH₂Ar), 2.02 (3H, s, 3-CH₃), 1.90 (3H, s, COCH₃), 1.78 (3H, s, 5-CH₃).
MASS (EI) m/z : 378(M⁺), 344, 228, 172, 118.

### (Example 2145)

### Compound of Formula No. D-295

¹H-NMR (CDCl₃ δ(ppm) : [5.16 (d, J=14Hz) & 5.12 (d, J=14Hz), 1H, NCH₂Ar], [4.46 (d, J=14Hz) & 4.40 (d, J=14Hz), 1H, NCH₂Ar], [2.03 (s) & 2.02 (s), 3H, 3-CH₃], 1.90 (3H, s, COCH₃), [1.75 (s) & 1.73 (s), 3H, 5-CH₃].
MASS (EI) m/z : 420(M⁺), 359, 131.

### (Example 2146)

### Compound of Formula No. D-296

¹H-NMR (CDCl₃) δ(ppm) : [5.20 (d, J=14Hz) & 5.11 (d, J=14Hz), 1H, NCH₂Ar], [4.49 (d, J=14Hz) & 4.34 (d, J=14Hz), 1H, NCH₂Ar], [2.04 (s) & 2.01 (s), 3H, 3-CH₃], 1.89 (3H, s, COCH₃), [1.75 (s) & 1.71 (s), 3H, 5-CH₃].
MASS (EI) m/z : 434(M⁺), 392, 359, 131.

### (Example 2147)

### Compound of Formula No. D-297

¹H-NMR (CDCl₃) δ(ppm) : [5.13 (d, J=14Hz) & 5.11 (d, J=14Hz), 1H, NCH₂Ar], 4.45 (1H, d, J=14Hz, NCH₂Ar), [2.05 (s) & 2.00 (s), 3H, 3-CH₃], 1.89 (3H, s, COCH₃), [1.77 (s) & 1.74 (s), 3H, 5-CH₃].
MASS (EI) m/z : 507(M⁺), 392, 376, 359.

### (Example 2148)

### Compound of Formula No. D-298

¹H-NMR (CDCl₃) δ(ppm) : [5.15 (d, J=14Hz) & 5.07 (d, J=14Hz), 1H, NCH₂Ar], [4.52 (d, J=14Hz) & 4.41 (d, J=14Hz), 1H, NCH₂Ar], 2.02 (3H, s, 3-CH₃), 1.90 (3H, s, COCH₃), [1.81 (s) & 1.74 (s), 3H, 5-CH₃].
MASS (EI) m/z : 434(M⁺), 387, 345, 118.

### (Example 2149)

### Compound of Formula No. D-299

¹H-NMR (CDCl₃) δ(ppm) : 5.01 (1H, d, J=14Hz, NCH₂Ar), 4.64 (1H, d, J=14Hz, NCH₂Ar), 1.97 (3H, s, 3-CH₃), 1.91 (3H, s, COCH₃), 1.82 (3H, s, 5-CH₃).
MASS (EI) m/z : 418(M⁺), 390, 375, 228, 118.

### (Example 2150)

### Compound of Formula No. D-300

¹H-NMR (CDCl₃) δ(ppm) : 5.09 (1H, d, J=14Hz, NCH₂Ar), 4.48 (1H, d, J=14Hz, NCH₂Ar), 2.00 (3H, s, 3-CH₃), 1.90 (3H, s, COCH₃), 1.74 (3H, s, 5-CH₃).
MASS (EI) m/z : 377(M⁺), 361, 318, 228, 118.

### (Example 2151)

### Compound of Formula No. D-301

¹H-NMR (CDCl₃) δ(ppm) : 5.16 (1H, d, J=14Hz, NCH₂Ar), 4.43 (1H, d, J=14Hz, NCH₂Ar), 2.04 (3H, s, 3-CH₃), 1.90 (3H, s, COCH₃), 1.71 (3H, s, 5-CH₃).
MASS (EI) m/z : 399(M⁺), 356, 228, 172, 118.

### (Example 2152)

### Compound of Formula No. D-302

¹H-NMR (CDCl₃) δ(ppm) : 4.99 (1H, d, J=14Hz, NCH₂Ar), 4.58 (1H, d, J=14Hz, NCH₂Ar), 1.99 (3H, s, 3-CH₃), 1.91 (3H, s, COCH₃), 1.83 (3H, s, 5-CH₃).
MASS (EI) m/z : 387(M⁺), 344, 301, 172, 118.

### (Example 2153)

### Compound of Formula No. D-303

¹H-NMR (CDCl₃) δ(ppm) : 5.11 (1H, d, J=14Hz, NCH₂Ar), 4.56 (1H, d, J=14Hz, NCH₂Ar), 2.02 (3H, s, 3-CH₃), 1.91 (3H, s, COCH₃), 1.79 (3H, s, 5-CH₃).
MASS (EI) m/z : 401(M⁺), 358, 228, 172, 118.

### (Example 2154)

### Compound of Formula No. D-304

¹H-NMR (CDCl₃) δ(ppm) : 5.08 (1H, d, J=14Hz, NCH₂Ar), 4.63 (1H, d, J=14Hz, NCH₂Ar), 2.01 (3H, s, 3-CH₃), 1.93 (3H, S, COCH₃), 1.83 (3H, s, 5-CH₃).
MASS (EI) m/z : 455(M⁺), 436, 412, 228, 172.

### (Example 2155)

### Compound of Formula No. D-305

¹H-NMR (CDCl₃) δ(ppm) : 5.15 (1H, d, J=14Hz, NCH₂Ar), 4.52 (1H, d, J=14Hz, NCH₂Ar), 2.04 (3H, s, 3-CH₃), 1.91 (3H, s, COCH₃), 1.77 (3H, s, 5-CH₃).
MASS (EI) m/z : 402(M⁺), 359, 228, 118.

### (Example 2156)

### Compound of Formula No. D-306

¹H-NMR (CDCl₃) δ(ppm) : 4.99 (1H, d, J=14Hz, NCH₂Ar), 4.32 (1H, d, J=14Hz, NCH₂Ar), 2.02 (3H, s, 3-CH₃), 1.89 (3H, s, COCH₃), 1.78 (3H, s, 5-CH₃).
MASS (EI) m/z : 334(M⁺), 293, 228, 186, 173, 149, 127, 118.

### (Example 2157)

### Compound of Formula No. D-307

¹H-NMR (CDCl₃) δ(ppm) : 5.07 (1H, d, J=14Hz, NCH₂Ar), 4.65 (1H, d, J=14Hz, NCH₂Ar), 2.02 (3H, s, 3-CH₃), 1.92 (3H, s, COCH₃), 1.85 (3H, s, 5-CH₃).
MASS (EI) m/z : 364(M⁺), 334, 321, 305, 228, 176, 172.

### (Example 2158)

### Compound of Formula No. D-308

¹H-NMR (CDCl₃) δ(ppm) : 5.08 (1H, d, J=14Hz, NCH₂Ar), 4.65 (1H, d, J=14Hz, NCH₂Ar), 3.78 (2H, s, COCH₂O), 2.00 (3H, s, 3-CH₃), 1.83 (3H, s, 5-CH₃).
MASS (EI) m/z : 394(M⁺), 379, 364, 349, 321, 293, 258, 228.

### (Example 2159)

### Compound of Formula No. D-309

¹H-NMR (CDCl₃) δ(ppm) : 4.91 (1H, d, J=14Hz, NCH₂Ar), 4.68 (1H, d, J=14Hz, NCH₂Ar), 3.73 (3H, brs, COOCH₃), 2.02 (3H, s, 3-CH₃), 1.90 (3H, s, 5-CH₃).
MASS (EI) m/z : 380(M⁺), 350, 321, 244, 212, 159, 118.

### (Example 2160)

### Compound of Formula No. D-310

¹H-NMR (CDCl₃) δ(ppm) : 5.08 (1H, d, J=14Hz, NCH₂Ar), 4.82 (1H, d, J=14Hz, NCH₂Ar), 2.08 (3H, s, 3-CH₃), 1.99 (3H, s, COCH₃), 1.96 (3H, s, 5-CH₃).
MASS (EI) m/z : 409(M⁺), 367, 350, 307, 293, 278, 228.

### (Example 2161)

### Compound of Formula No. D-311

¹H-NMR (CDCl₃) δ (ppm) : [5.08 (d, J=14Hz) & 4.94 (d, J=14Hz), 1H, NCH₂Ar], [4.55 (d, J=14Hz) & 4.46 (d, J=14Hz), 1H, NCH₂Ar], [2.00 (s) & 1.98 (s), 3H, 3-CH₃], [1.91 (s) & 1.90 (s), 3H, COCH₃], [1.84 (s) & 1.80 (s), 3H, 5-CH₃].
MASS (EI) m/z : 362(M⁺), 319, 228, 172, 118.

### (Example 2162)

### Compound of Formula No. D-312

¹H-NMR (CDCl₃) δ(ppm) : 4.99 (1H, d, J=14Hz, NCH₂Ar), 4.46 (1H, d, J=14Hz, NCH₂Ar), 1.99 (3H, s, 3-CH₃), 1.90 (3H, s, COCH₃), 1.80 (3H, s, 5-CH₃).
MASS (EI) m/z : 376(M⁺), 333, 307, 293, 228, 200, 186, 172.

### (Example 2163)

### Compound of Formula No. D-313

¹H-NMR (CDCl₃) δ(ppm) : [5.07 (d, J=14Hz) & 5.02 (d, J=14Hz), 1H, NCH₂Ar], [4.44 (d, J=14Hz) & 4.38 (d, J=14Hz), 1H, NCH₂Ar], [2.03 (s) & 2.01 (s), 3H, 3-CH₃], 1.90 (3H, s, COCH₃), [1.79 (s) & 1.77 (s), 3H, 5-CH₃].
MASS (EI) m/z : 519(M⁺), 476, 460, 361, 228.

### (Example 2164)

### Compound of Formula No. D-314

¹H-NMR (CDCl₃) δ(ppm) : 5.88 (1H, d, J=14Hz, NCH₂Ar), 5.04 (1H, d, J=14Hz, NCH₂Ar), 2.15 (3H, s, 3-CH₃), 2.00 (3H, s, COCH₃), 1.75 (3H, s, 5-CH₃).
MASS (EI) m/z : 392(M⁺), 349, 333, 212, 203, 189, 118.

### (Example 2165)

### Compound of Formula No. D-315

¹H-NMR (CDCl₃) δ(ppm) : 4.97 (1H, d, J=14Hz, NCH₂Ar), 4.54 (1H, d, J=14Hz, NCH₂Ar), 2.00 (3H, s, 3-CH₃), 1.91 (3H, s, COCH₃), 1.83 (3H, s, 5-CH₃).
MASS (EI) m/z : 344(M⁺), 301, 228, 172, 118.

### (Example 2166)

### Compound of Formula No. D-316

¹H-NMR (CDCl₃) δ(ppm) : 5.11 (1H, d, J=14Hz, NCH₂Ar), 4.27 (1H, d, J=14Hz, NCH₂Ar), 2.04 (3H, s, 3-CH₃), 1.91 (3H, s, COCH₃), 1.71 (3H, s, 5-CH₃).
MASS (EI) m/z : 335(M⁺), 292, 228, 186, 172, 145, 118.

### (Example 2167)

### Compound of Formula No. D-317

¹H-NMR (CDCl₃) δ(ppm) : 5.04 (1H, d, J=14Hz, NCH₂Ar), 4.43 (1H, d, J=14Hz, NCH₂Ar), 2.01 (3H, s, 3-CH₃), 1.90 (3H, s, COCH₃), 1.77 (3H, s, 5-CH₃).
MASS (EI) m/z : 349(M⁺), 306, 228, 186, 172, 145, 118.

### (Example 2168)

### Compound of Formula No. D-318

¹H-NMR (CDCl₃) δ(ppm) : 4.96 (1H, d, J=14Hz, NCH₂Ar), 4.49 (1H, d, J=14Hz, NCH₂Ar), 2.00 (3H, s, 3-CH₃), 1.89 (3H, s, COCH₃), 1.83 (3H, s, 5-CH₃).
MASS (EI) m/z : 411(M⁺), 368, 228, 183, 172, 118.

### (Example 2169)

### Compound of Formula No. D-319

¹H-NMR (CDCl₃) δ(ppm) : 5.07 (1H, d, J=14Hz, NCH₂Ar), 4.47 (1H, d, J=14Hz, NCH₂Ar), 2.01 (3H, s, 3-CH₃), 1.90 (3H, s, COCH₃), 1.76 (3H, s, 5-CH₃).
MASS (EI) m/z : 403(M⁺), 360, 318, 228, 186, 172, 145, 118.

### (Example 2170)

### Compound of Formula No. D-320

¹H-NMR (CDCl₃) δ (ppm) : 5.07 (1H, d, J=14Hz, NCH₂Ar), 4.46 (1H, d, J=14Hz, NCH₂Ar), 3.75 (2H, s, COCH₂O), 1.98 (3H, s, 3-CH₃), 1.73 (3H, s, 5-CH₃).
MASS (EI) m/z : 433(M⁺), 418, 388, 360, 258, 228, 185.

### (Example 2171)

### Compound of Formula No. D-321

¹H-NMR (CDCl₃) δ(ppm) : 4.88 (1H, d, J=14Hz, NCH₂Ar), 4.52 (1H, d, J=14Hz, NCH₂Ar), 3.71 (3H, brs, COOCH₃), 2.00 (3H, s, 3-CH₃), 1.81 (3H, s, 5-CH₃).
MASS (EI) m/z : 419(M⁺), 388, 360, 334, 244, 212, 175, 118.

### (Example 2172)

### Compound of Formula No. D-322

¹H-NMR (CDCl₃) δ(ppm) : 5.02 (1H, d, J=14Hz, NCH₂Ar), 4.45 (1H, d, J=14Hz, NCH₂Ar), 2.00 (3H, s, 3-CH₃), 1.90 (3H, s, COCH₃), 1.76 (3H, s, 5-CH₃).
MASS (EI) m/z : 417(M⁺), 374, 228, 189, 172, 145, 118.

### (Example 2173)

### Compound of Formula No. D-323

¹H-NMR (CDCl₃) δ(ppm) : 5.02 (1H, d, J=14Hz, NCH₂Ar), 4.45 (1H, d, J=14Hz, NCH₂Ar), 2.00 (3H, s, 3-CH₃), 1.90 (3H, s, COCH₃), 1.77 (3H, s, 5-CH₃).
MASS (EI) m/z : 449(M⁺), 430, 406, 228, 186, 172, 118.

### (Example 2174)

### Compound of Formula No. D-324

¹H-NMR (CDCl₃) δ(ppm) : 5.03 (1H, d, J=14Hz, NCH₂Ar), 4.49 (1H, d, J=14Hz, NCH₂Ar), 2.01 (3H, s, 3-CH₃), 1.90 (3H, s, COCH₃), 1.80 (3H, s, 5-CH₃).
MASS (EI) m/z : 505(M⁺), 462, 417, 379, 228, 186, 172.

### (Example 2175)

### Compound of Formula No. D-325

¹H-NMR (CDCl₃) δ(ppm) : 5.12 (1H, d, J=14Hz, NCH₂Ar), 4.40 (1H, d, J=14Hz, NCH₂Ar), 2.04 (3H, s, 3-CH₃), 1.90 (3H, s, COCH₃), 1.76 (3H, s, 5-CH₃).
MASS (EI) m/z : 505(M⁺), 462, 417, 379, 228, 186, 172.

### (Example 2176)

### Compound of Formula No. D-326

¹H-NMR (CDCl₃) δ(ppm) : 4.99 (1H, d, J=14Hz, NCH₂Ar), 4.38 (1H, d, J=14HZ, NCH₂Ar), 2.04 (3H, s, 3-CH₃), 1.90 (3H, s, COCH₃), 1.82 (3H, s, 5-CH₃).
MASS (EI) m/z : 379(M⁺), 364, 336, 293, 228, 207, 186, 172.

### (Example 2177)

### Compound of Formula No. D-327

¹H-NMR (CDCl₃) δ(ppm) : 5.06 (1H, d, J=14Hz, NCH₂Ar), 4.39 (1H, d, J=14Hz, NCH₂Ar), 2.02 (3H, s, 3-CH₃), 1.90 (3H, s, COCH₃), 1.74 (3H, s, 5-CH₃).
MASS (EI) m/z : 365(M⁺), 322, 228, 172.

### (Example 2178)

### Compound of Formula No. D-328

¹H-NMR (CDCl₃) δ(ppm) : [5.00 (d, J=14Hz) & 4.98 (d, J=14Hz), 1H, NCH₂Ar], [4.67 (d, J=14Hz) & 4.65 (d, J=14Hz), 1H, NCH₂Ar], 1.95 (3H, s, 3-CH₃), 1.91 (3H, s, COCH₃), [1.84 (s) & 1.83 (s), 3H, 5-CH₃].
MASS (EI) m/z : 381(M⁺), 364, 322, 228, 118.

### (Example 2179)

### Compound of Formula No. D-329

¹H-NMR (CDCl₃) δ(ppm) : 5.06 (1H, d, J=14Hz, NCH₂Ar), 4.65 (1H, d, J=14Hz, NCH₂Ar), 1.99 (3H, s, 3-CH₃), 1.92 (3H, s, COCH₃), 1.84 (3H, s, 5-CH₃).
MASS (EI) m/z : 397(M⁺), 354, 228, 172, 118.

### (Example 2180)

### Compound of Formula No. D-330

¹H-NMR (CDCl₃) δ(ppm) : 4.90 (1H, d, J=14Hz, NCH₂Ar), 4.53 (1H, d, J=14Hz, NCH₂Ar), 3.71 (3H, s, COOCH₃), 1.99 (3H, s, 3-CH₃), 1.80 (3H, s, 5-CH₃).
MASS (EI) m/z : 435(M⁺), 244, 118.

### (Example 2181)

### Compound of Formula No. D-331

¹H-NMR (CDCl₃) δ(ppm) : 4.95 (1H, d, J=14Hz, NCH₂Ar), 4.69 (1H, d, J=14Hz, NCH₂Ar), 3.73 (3H, s, COOCH₃), 2.00 (3H, s, 3-CH₃), 1.87 (3H, s, 5-CH₃).
MASS (EI) m/z : 467(M⁺), 435, 244, 118.

### (Example 2182)

### Compound of Formula No. D-332

¹H-NMR (CDCl₃) δ(ppm) : 5.09 (1H, d, J=14Hz, NCH₂Ar), 4.48 (1H, d, J=14Hz, NCH₂Ar), 2.02 (3H, s, 3-CH₃), 1.91 (3H, s, COCH₃), 1.64 (3H, d, J=2Hz, 5-CH₃).
MASS (EI) m/z : 362(M⁺), 319, 246, 204, 190, 163, 149, 136.

### (Example 2183)

### Compound of Formula No. D-333

¹H-NMR (CDCl₃) δ(ppm) : 5.08 (1H, d, J=14Hz, NCH₂Ar), 4.53 (1H, d, J=14Hz, NCH₂Ar), 2.30 (3H, s, COSCH₃), 2.02 (3H, s, 3-CH₃), 1.66 (3H, d, J=2Hz, 5-CH₃).
MASS (EI) m/z : 394(M⁺), 347, 319, 278, 231, 190, 162.

### (Example 2184)

### Compound of Formula No. D-334

¹H-NMR (CDCl₃) δ(ppm) : 4.96 (1H, d, J=14Hz, NCH₂Ar), 4.59 (1H, d, J=14Hz, NCH₂Ar), 1.98 (3H, s, 3-CH₃), 1.90 (3H, s, COCH₃), 1.87 (3H, s, 5-CH₃).
MASS (EI) m/z : 362(M⁺), 319, 277, 246, 231, 204, 190, 163.

### (Example 2185)

### Compound of Formula No. D-335

¹H-NMR (CDCl₃) δ(ppm) : 4.94 (1H, d, J=14Hz, NCH₂Ar), 4.66 (1H, d, J=14Hz, NCH₂Ar), 3.87 (2H, s, COCH₂Cl), 1.97 (3H, s, 3-CH₃), 1.90 (3H, s, 5-CH₃).
MASS (EI) m/z : 396(M⁺), 361, 319, 280, 252, 231, 190.

### (Example 2186)

### Compound of Formula No. D-336

¹H-NMR (CDCl₃) δ(ppm) : 4.97 (1H, d, J=14Hz, NCH₂Ar), 4.60 (1H, d, J=14Hz, NCH₂Ar), 3.75 (2H, s, COCH₂O), 1.96 (3H, s, 3-CH₃), 1.85 (3H, s, 5-CH₃).
MASS (EI) m/z : 392(M⁺), 377, 347, 319, 276, 246, 231, 203.

### (Example 2187)

### Compound of Formula No. D-337

¹H-NMR (CDCl₃) δ(ppm) : 4.91 (1H, d, J=14Hz, NCH₂Ar), 4.65 (1H, d, J=14Hz, NCH₂Ar), 3.04 (2H, s, COCH₂S), 1.97 (3H, s, 3-CH₃), 1.91 (3H, s, 5-CH₃).
MASS (EI) m/z : 408(M⁺), 393, 362, 347, 318, 293, 246, 231.

### (Example 2188)

### Compound of Formula No. D-339

¹H-NMR (CDCl₃) δ(ppm) : 4.98 (1H, d, J=14Hz, NCH₂Ar), 4.62 (1H, d, J=14Hz, NCH₂Ar), 2.30 (3H, s, COSCH₃), 1.99 (3H, s, 3-CH₃), 1.87 (3H, s, 5-CH₃).
MASS (EI) m/z : 394(M⁺), 347, 319, 293, 278, 231, 190, 162.

### (Example 2189)

### Compound of Formula No. D-340

¹H-NMR (CDCl₃) δ(ppm) : 4.99 (1H, d, J=14Hz, NCH₂Ar), 4.57 (1H, d, J=14Hz, NCH₂Ar), 1.98 (3H, s, 3-CH₃), 1.91 (3H, s, COCH₃), 1.80 (3H, s, 5-CH₃).
MASS (EI) m/z : 362(M⁺), 319, 246, 204, 190, 163, 136, 116.

### (Example 2190)

### Compound of Formula No. D-341

¹H-NMR (CDCl₃) δ(ppm) : 5.00 (1H, d, J=14Hz, NCH₂Ar), 4.60 (1H, d, J=14Hz, NCH₂Ar), 2.30 (3H, s, COSCH₃), 1.99 (3H, s, 3-CH₃), 1.80 (3H, s, 5-CH₃).
MASS (EI) m/z : 394(M⁺), 347, 319, 293, 278, 231, 190, 162.

### (Example 2191)

### Compound of Formula No. D-342

¹H-NMR (CDCl₃) δ(ppm) : 5.16 (1H, d, J=14Hz, NCH₂Ar), 4.43 (1H, d, J=14Hz, NCH₂Ar), 2.04 (3H, s, 3-CH₃), 1.92 (3H, s, COCH₃), 1.55 (3H, s, 5-CH₃).
MASS (EI) m/z : 378(M⁺), 335, 262, 220, 206, 179, 152.

### (Example 2192)

### Compound of Formula No. D-343

¹H-NMR (CDCl₃) δ(ppm) : 5.17 (1H, d, J=14Hz, NCH₂Ar), 4.46 (1H, d, J=14Hz, NCH₂Ar), 2.30 (3H, s, COSCH₃), 2.05 (3H, s, 3-CH₃), 1.56 (3H, s, 5-CH₃).
MASS (EI) m/z : 410(M⁺), 363, 335, 294, 247, 206, 178.

### (Example 2193)

### Compound of Formula No. D-344

¹H-NMR (CDCl₃) δ(ppm) : 4.95 (1H, d, J=14Hz, NCH₂Ar), 4.60 (1H, d, J=14Hz, NCH₂Ar), 1.97 (3H, s, 3-CH₃), 1.90 (3H, s, COCH₃), 1.87 (3H, s, 5-CH₃).
MASS (EI) m/z : 378(M⁺), 335, 262, 220, 206, 179, 152.

### (Example 2194)

### Compound of Formula No. D-345

¹H-NMR (CDCl₃) δ(ppm) : 4.97 (1H, d, J=14Hz, NCH₂Ar), 4.62 (1H, d, J=14Hz, NCH₂Ar), 2.30 (3H, s, COSCH₃), 1.98 (3H, s, 3-CH₃), 1.87 (3H, s, 5-CH₃).
MASS (EI) m/z : 410(M⁺), 363, 335, 294, 247, 206, 178.

### (Example 2195)

### Compound of Formula No. D-346

¹H-NMR (CDCl₃) δ(ppm) : 4.97 (1H, d, J=14Hz, NCH₂Ar), 4.57 (1H, d, J=14Hz, NCH₂Ar), 1.98 (3H, s, 3-CH₃), 1.90 (3H, s, COCH₃), 1.83 (3H, s, 5-CH₃).
MASS (EI) m/z : 378(M⁺), 335, 262, 220, 206, 179, 152.

### (Example 2196)

### Compound of Formula No. D-347

¹H-NMR (CDCl₃) δ(ppm) : 4.99 (1H, d, J=14Hz, NCH₂Ar), 4.60 (1H, d, J=14Hz, NCH₂Ar), 2.30 (3H, s, COSCH₃), 1.98 (3H, s, 3-CH₃), 1.83 (3H, s, 5-CH₃).
MASS (EI) m/z : 410(M⁺), 363, 335, 294, 247, 206, 178.

### (Example 2197)

### Compound of Formula No. D-348

¹H-NMR (CDCl₃) δ(ppm) : 5.07 (1H, d, J=14Hz, NCH₂Ar), 4.50 (1H, d, J=14Hz, NCH₂Ar), 2.01 (3H, s, 3-CH₃), 1.91 (3H, s, COCH₃), 1.68 (3H, d, J=2Hz, 5-CH₃).
MASS (EI) m/z : 380(M⁺), 337, 264, 222, 208, 181, 154.

### (Example 2198)

### Compound of Formula No. D-350

¹H-NMR (CDCl₃) δ(ppm) : 4.93 (1H, d, J=14Hz, NCH₂Ar), 4.61 (1H, d, J=14Hz, NCH₂Ar), 1.96 (3H, s, 3-CH₃), 1.93 (3H, s, COCH₃), 1.89 (3H, s, 5-CH₃)
MASS (EI) m/z : 380(M⁺), 337, 264, 222, 208, 154.

### (Example 2199)

### Compound of Formula No. D-351

¹H-NMR (CDCl₃) δ(ppm) : 4.91 (1H, d, J=14Hz, NCH₂Ar), 4.62 (1H, d, J=14Hz, NCH₂Ar), 1.95 (3H, s, 3-CH₃), 1.92 (3H, s, COCH₃), 1.89 (3H, s, 5-CH₃).
MASS (EI) m/z : 412(M⁺), 369, 296, 254, 240.

### (Example 2200)

### Compound of Formula No. D-352

¹H-NMR (CDCl₃) δ(ppm) : 4.94 (1H, d, J=14Hz, NCH₂Ar), 4.64 (1H, d, J=14Hz, NCH₂Ar), 2.30 (3H, s, COSCH₃), 1.97 (3H, s, 3-CH₃), 1.91 (3H, s, 5-CH₃).
MASS (EI) m/z : 444(M⁺), 397, 369, 328, 281, 240, 186.

### (Example 2201)

### Compound of Formula No. D-353

¹H-NMR (CDCl₃) δ(ppm) : 5.18 (1H, d, J=14Hz, NCH₂Ar), 4.42 (1H, d, J=14Hz, NCH₂Ar), 1.98 (3H, s, 3-CH₃), 1.92 (3H, s, COCH₃), 1.49 (3H, s, 5-CH₃).
MASS (EI) m/z : 358(M⁺), 315, 242, 200, 186, 132, 116.

### (Example 2202)

### Compound of Formula No. D-354

¹H-NMR (CDCl₃) δ(ppm) : 4.98 (1H, d, J=14Hz, NCH₂Ar), 4.55 (1H, d, J=14Hz, NCH₂Ar), 1.98 (3H, s, 3-CH₃), 1.91 (3H, s, COCH₃), 1.81 (3H, s, 5-CH₃).
MASS (EI) m/z : 358(M⁺), 315, 242, 200, 186, 132, 116.

### (Example 2203)

### Compound of Formula No. D-355

¹H-NMR (CDCl₃) δ(ppm) : 4.99 (1H, d, J=14Hz, NCH₂Ar), 4.56 (1H, d, J=14Hz, NCH₂Ar), 1.98 (3H, s, 3-CH₃), 1.91 (3H, s, COCH₃), 1.79 (3H, s, 5-CH₃).
MASS (EI) m/z : 358(M⁺), 315, 242, 200, 186, 159, 132, 116.

### (Example 2204)

### Compound of Formula No. D-356

¹H-NMR (CDCl₃) δ(ppm) : 5.00 (1H, d, J=14Hz, NCH₂Ar), 4.60 (1H, d, J=14Hz, NCH₂Ar), 2.30 (3H, s, COSCH₃), 1.99 (3H, s, 3-CH₃), 1.80 (3H, s, 5-CH₃).
MASS (EI) m/z : 390 (M⁺), 343, 315, 293, 274, 227, 186, 158.

### (Example 2205)

### Compound of Formula No. D-357

¹H-NMR (CDCl₃) δ(ppm) : 4.96 (1H, d, J=14Hz, NCH₂Ar), 4.58 (1H, d, J=14Hz, NCH₂Ar), 1.97 (3H, s, 3-CH₃), 1.90 (3H, s, COCH₃), 1.80 (3H, s, 5-CH₃),
MASS (EI) m/z : 372(M⁺), 329, 256, 214, 200, 173, 157, 146.

### (Example 2206)

### Compound of Formula No. D-358

¹H-NMR (CDCl₃) δ(ppm) : 4.95 (1H, d, J=14Hz, NCH₂Ar), 4.62 (1H, d, J=14Hz, NCH₂Ar), 1.98 (3H, s, 3-CH₃), 1.91 (3H, s, COCH₃), 1.89 (3H, s, 5-CH₃).
MASS (EI) m/z : 412(M⁺), 393, 369, 296, 254, 240, 213, 186.

### (Example 2207)

### Compound of Formula No. D-359

¹H-NMR (CDCl₃) δ(ppm) : 4.97 (1H, d, J=14Hz, NCH₂Ar), 4.59 (1H, d, J=14Hz, NCH₂Ar), 1.99 (3H, s, 3-CH₃), 1.91 (3H, s, COCH₃), 1.90 (3H, s, 5-CH₃).
MASS (EI) m/z : 412(M⁺), 393, 369, 296, 268, 254, 240, 213.

### (Example 2208)

### Compound of Formula No. D-360

¹H-NMR (CDCl₃) δ(ppm) : 4.99 (1H, d, J=14Hz, NCH₂Ar), 4.62 (1H, d, J=14Hz, NCH₂Ar), 2.31 (3H, s, COSCH₃), 2.00 (3H, s, 3-CH₃), 1.90 (3H, s, 5-CH₃).
MASS (EI) m/z : 444(M⁺), 425, 397, 369, 328, 281, 240, 212.

### (Example 2209)

### Compound of Formula No. D-361

¹H-NMR (CDCl₃) δ(ppm) : 4.89 (1H, d, J=14Hz, NCH₂Ar), 4.68 (1H, d, J=14Hz, NCH₂Ar), 1.98 (3H, s, 3-CH₃), 1.97 (3H, s, COCH₃), 1.91 (3H, s, 5-CH₃).
MASS (EI) m/z : 480(M⁺), 461, 438, 364, 322, 308, 281, 254.

### (Example 2210)

### Compound of Formula No. D-362

¹H-NMR (CDCl₃) δ(ppm) : 5.32 (1H, d, J=14Hz, NCH₂Ar), 4.30 (1H, d, J=14Hz, NCH₂Ar), 2.11 (3H, s, 3-CH₃), 1.91 (3H, s, COCH₃), 1.44 (3H, s, 5-CH₃).
MASS (EI) m/z : 480(M⁺), 461, 438, 364, 322, 308, 281, 265.

### (Example 2211)

### Compound of Formula No. D-363

¹H-NMR (CDCl₃) δ(ppm) : 5.02 (1H, d, J=14Hz, NCH₂Ar), 4.45 (1H, d, J=14Hz, NCH₂Ar), 3.73 (3H, brs, COOCH₃), 2.07 (3H, s, 3-CH₃), 1.51 (3H, s, 5-CH₃).
MASS (EI) m/z : 496(M⁺), 477, 437, 380, 348, 308, 295.

### (Example 2212)

### Compound of Formula No. D-364

¹H-NMR (CDCl₃) δ(ppm) : 4.96 (1H, d, J=14Hz, NCH₂Ar), 4.60 (1H, d, J=14Hz, NCH₂Ar), 1.98 (3H, s, 3-CH₃), 1.90 (6H, s, COCH₃ & 5-CH₃).
MASS (EI) m/z : 369(M⁺), 326, 253, 211, 197, 170, 143, 116.

### (Example 2213)

### Compound of Formula No. D-365

¹H-NMR (CDCl₃) δ(ppm) : 4.96 (1H, d, J=14Hz, NCH₂Ar), 4.60 (1H, d, J=14Hz, NCH₂Ar), 1.98 (3H, s, 3-CH₃), 1.94 (3H, s, COCH₃), 1.90 (3H, s, 5-CH₃).
MASS (EI) m/z : 369(M⁺), 326, 253, 211, 197, 143, 116.

### (Example 2214)

### Compound of Formula No. D-366

¹H-NMR (CDCl₃) δ (ppm) : 4.98 (1H, d, J=14Hz, NCH₂Ar), 4.59 (1H, d, J=14Hz, NCH₂Ar), 1.98 (3H, s, 3-CH₃), 1.91 (3H, s, COCH₃), 1.85 (3H, S, 5-CH₃).
MASS (EI) m/z : 387(M⁺), 369, 326, 271, 253, 229, 211, 197.

### (Example 2215)

### Compound of Formula No. D-367

¹H-NMR (CDCl₃) δ(ppm) : 4.95 (1H, d, J=14Hz, NCH₂Ar), 4.64 (1H, d, J=14Hz, NCH₂Ar), 1.99 (3H, s, 3-CH₃), 1.93 (3H, s, COCH₃), 1.90 (3H, s, 5-CH₃).
MASS (EI) m/z : 388(M⁺), 345, 272, 256, 230, 216, 189, 162.

### (Example 2216)

### Compound of Formula No. D-368

¹H-NMR (CDCl₃) δ(ppm) : 4.94 (1H, d, J=14Hz, NCH₂Ar), 4.63 (1H, d, J=14Hz, NCH₂Ar), 1.97 (3H, s, 3-CH₃), 1.92 (3H, s, COCH₃), 1.88 (3H, s, 5-CH₃).
MASS (EI) m/z : 402(M⁺), 359, 286, 244, 230, 203, 176.

### (Example 2217)

### Compound of Formula No. D-369

¹H-NMR (CDCl₃) δ(ppm) : 4.95 (1H, d, J=14Hz, NCH₂Ar), 4.65 (1H, d, J=14Hz, NCH₂Ar), 1.99 (3H, s, 3-CH₃), 1.97 (3H, s, COCH₃), 1.92 (3H, s, 5-CH₃).
MASS (EI) m/z : 389(M⁺), 346, 300, 273, 231, 217, 163, 117.

### (Example 2218)

### Compound of Formula No. D-370

¹H-NMR (CDCl₃) δ(ppm) : 4.96 (1H, d, J=14Hz, NCH₂Ar), 4.67 (1H, d, J=14Hz, NCH₂Ar), 1.98 (3H, s, 3-CH₃), 1.96 (3H, s, COCH₃), 1.90 (3H, s, 5-CH₃).
MASS (EI) m/z : 389(M⁺), 346, 300, 273, 231, 217, 163, 117.

### (Example 2219)

### Compound of Formula No. D-371

¹H-NMR (CDCl₃) δ(ppm) : 4.98 (1H, d, J=14Hz, NCH₂Ar), 4.58 (1H, d, J=14Hz, NCH₂Ar), 1.98 (3H, s, 3-CH₃), 1.91 (3H, s, COCH₃), 1.83 (3H, s, 5-CH₃).
MASS (EI) m/z : 374(M⁺), 331, 293, 258, 216, 202, 175, 157.

### (Example 2220)

### Compound of Formula No. D-372

¹H-NMR (CDCl₃) δ(ppm) : 4.99 (1H, d, J=14Hz, NCH₂Ar), 4.57 (1H, d, J=14Hz, NCH₂Ar), 1.98 (3H, s, 3-CH₃), 1.90 (3H, s, COCH₃), 1.84 (3H, s, 5-CH₃).
MASS (EI) m/z : 428(M⁺), 385, 343, 312, 270, 256, 202, 116.

### (Example 2221)

### Compound of Formula No. D-373

¹H-NMR (CDCl₃) δ(ppm) : 4.82 (1H, d, J=14Hz, NCH₂Ar), 4.72 (1H, d, J=14Hz, NCH₂Ar), 2.25 (3H, s, 5-CH₃), 1.93 (3H, s, 3-CH₃), 1.90 (3H, s, COCH₃).
MASS (EI) m/z : 345(M⁺), 302, 229, 187, 173, 119.

### (Example 2222)

### Compound of Formula No. D-374

¹H-NMR (CDCl₃) δ(ppm) : 4.81 (1H, d, J=14Hz, NCH₂Ar), 4.77 (1H, d, J=14Hz, NCH₂Ar), 2.29 (3H, s, COSCH₃), 2.26 (3H, s, 5-CH₃), 1.93 (3H, s, 3-CH₃).
MASS (EI) m/z : 377(M⁺), 330, 302, 261, 214, 173, 116.

### (Example 2223)

### Compound of Formula No. D-375

¹H-NMR (CDCl₃) δ(ppm) : 4.82 (1H, d, J=14Hz, NCH₂Ar), 4.70 (1H, d, J=14Hz, NCH₂Ar), 2.27 (3H, s, 5-CH₃), 1.92 (3H, s, 3-CH₃), 1.88 (3H, s, COCH₃).
MASS (EI) m/z : 413 (M⁺), 394, 370, 297, 255, 214, 187.

### (Example 2224)

### Compound of Formula No. D-376

¹H-NMR (CDCl₃) δ(ppm) : 4.77 (2H, s, NCH₂Ar), 2.32 (3H, s, 5-CH₃), 1.91 (3H, s, 3-CH₃), 1.89 (3H, s, COCH₃).
MASS (EI) m/z : 413(M⁺), 394, 370, 297, 241, 187, 146.

### (Example 2225)

### Compound of Formula No. D-377

¹H-NMR (CDCl₃) δ(ppm) : 4.86 (1H, d, J=14Hz, NCH₂Ar), 4.68 (1H, d, J=14Hz, NCH₂Ar), 2.27 (3H, s, 5-CH₃), 1.95 (3H, s, 3-CH₃), 1.89 (3H, s, COCH₃).
MASS (EI) m/z : 413(M⁺), 394, 370, 297, 255, 214, 187.

### (Example 2226)

### Compound of Formula No. D-378

¹H-NMR (CDCl₃) δ(ppm) : 4.97 (1H, d, J=14Hz, NCH₂Ar), 4.61 (1H, d, J=14Hz, NCH₂Ar), 1.99 (3H, s, 3-CH₃), 1.91 (3H, s, COCH₃), 1.89 (3H, s, 5-CH₃).
MASS (EI) m/z : 345(M⁺), 302, 229, 187, 173, 119.

### (Example 2227)

### Compound of Formula No. D-379

¹H-NMR (CDCl₃) δ(ppm) : 4.89 (1H, d, J=14Hz, NCH₂Ar), 4.62 (1H, d, J=14Hz, NCH₂Ar), 2.06 (3H, s, 3-CH₃), 1.96 (3H, s, COCH₃), 1.87 (3H, s, 5-CH₃).
MASS (EI) m/z : 413(M⁺), 371, 320, 297, 255, 241, 187.

### (Example 2228)

### Compound of Formula No. D-380

¹H-NMR (CDCl₃) δ(ppm) : 4.93 (1H, d, J=14Hz, NCH₂Ar), 4.78 (1H, d, J=14Hz, NCH₂Ar), 2.09 (3H, s, 3-CH₃), 2.01 (3H, s, COCH₃), 1.99 (3H, s, 5-CH₃).
MASS (EI) m/z : 396(M⁺), 353, 280, 223, 197, 170, 129, 116.

### (Example 2229)

### Compound of Formula No. D-381

¹H-NMR (CDCl₃) δ(ppm) : 4.93 (1H, d, J=14Hz, NCH₂Ar), 4.62 (1H, d, J=14Hz, NCH₂Ar), 2.23 (3H, s, 5-CH₃), 2.03 (3H, s, 3-CH₃), 1.89 (3H, s, COCH₃).
MASS (EI) m/z : 346(M⁺), 303, 230, 188, 174, 120.

### (Example 2230)

### Compound of Formula No. D-382

¹H-NMR (CDCl₃) δ(ppm) : 4.90 (1H, d, J=14Hz, NCH₂Ar), 4.63 (1H, d, J=14Hz, NCH₂Ar), 2.23 (3H, s, 5-CH₃), 2.02 (3H, s, 3-CH₃), 1.89 (3H, s, COCH₃).
MASS (EI) m/z : 360(M⁺), 317, 244, 202, 188, 161, 116.

### (Example 2231)

### Compound of Formula No. D-383

¹H-NMR (CDCl₃) δ(ppm) : 4.88 (1H, d, J=14Hz, NCH₂Ar), 4.65 (1H, d, J=14Hz, NCH₂Ar), 2.23 (3H, s, 5-CH₃), 2.01 (3H, s, 3-CH₃), 1.88 (3H, s, COCH₃).
MASS (EI) m/z : 374(M⁺), 331, 293, 258, 228, 216, 175.

### (Example 2232)

### Compound of Formula No. D-384

¹H-NMR (CDCl₃) δ(ppm) : 4.90 (1H, d, J=14Hz, NCH₂Ar), 4.62 (1H, d, J=14Hz, NCH₂Ar), 2.24 (3H, s, 5-CH₃), 2.02 (3H, s, 3-CH₃), 1.87 (3H, s, COCH₃).
MASS (EI) m/z : 414(M⁺), 371, 307, 293, 256, 215.

### (Example 2233)

### Compound of Formula No. D-385

¹H-NMR (CDCl₃) δ(ppm) : 4.91 (1H, d, J=14Hz, NCH₂Ar), 4.61 (1H, d, J=14Hz, NCH₂Ar), 2.23 (3H, s, 5-CH₃), 2.02 (3H, s, 3-CH₃), 1.87 (3H, s, COCH₃).
MASS (EI) m/z : 410(M⁺), 367, 293, 252, 238, 211.

### (Example 2234)

### Compound of Formula No. D-386

¹H-NMR (CDCl₃) δ(ppm) : 4.89 (1H, d, J=14Hz, NCH₂Ar), 4.62 (1H, d, J=14Hz, NCH₂Ar), 2.25 (3H, s, 5-CH₃), 2.01 (3H, s, 3-CH₃), 1.88 (3H, s, COCH₃).
MASS (EI) m/z : 406(M⁺), 363, 290, 248, 234, 180.

### (Example 2235)

### Compound of Formula No. D-387

¹H-NMR (CDCl₃) δ(ppm) : [4.91 (d, J=14Hz) & 4.88 (d, J=14Hz), 1H, N'CH₂Ar], 4.85 (1H, d, J=14Hz, NCH₂Ar), 4.67 (1H, d, J=14Hz, NCH₂Ar), [4.64 (d, J=14Hz) & 4.62 (d, J=14Hz), 1H, N'CH₂Ar], 2.41 (3H, s, 5-CH₃), [2.25 (s) & 2.23 (s), 3H, 5'-CH₃], [1.98 (s) & 1.97 (s), 3H, 3'-CH₃], 1.95 (3H, s, 3-CH₃), 1.88 (6H, s, COCH₃).
MASS (EI) m/z : 646(M⁺), 603, 530, 488, 472, 447, 403, 316.

### (Example 2236)

### Compound of Formula No. D-388

¹H-NMR (CDCl₃) δ(ppm) : 4.84 (1H, d, J=14Hz, NCH₂Ar), 4.65 (1H, d, J=14Hz, NCH₂Ar), 2.29 (3H, s, 5-CH₃), 1.93 (3H, s, 3-CH₃), 1.87 (3H, s, COCH₃).
MASS (EI) m/z : 406(M⁺), 363, 290, 248, 234, 207, 180.

### (Example 2237)

### Compound of Formula No. D-389

¹H-NMR (CDCl₃) δ(ppm) : 4.92 (1H, d, J=14Hz, NCH₂Ar), 4.59 (1H, d, J=14Hz, NCH₂Ar), 2.27 (3H, s, 5-CH₃), 2.01 (3H, s, 3-CH₃), 1.87 (3H, s, COCH₃).
MASS (EI) m/z : 407(M⁺), 364, 291, 249, 235, 208, 181.

### (Example 2238)

### Compound of Formula No. D-390

¹H-NMR (CDCl₃) δ(ppm) : 4.92 (1H, d, J=14Hz, NCH₂Ar), 4.62 (1H, d, J=14Hz, NCH₂Ar), 2.37 (3H, s, 5-CH₃), 1.98 (3H, s, 3-CH₃), 1.90 (3H, s, COCH₃).
MASS (EI) m/z : 401(M⁺), 358, 285, 243, 229, 175, 149.

### (Example 2239)

### Compound of Formula No. D-391

¹H-NMR (CDCl₃) δ(ppm) : 4.91 (1H, d, J=14Hz, NCH₂Ar), 4.69 (1H, d, J=14Hz, MCH₂Ar), 2.39 (3H, s, 5-CH₃), 2.31 (3H, s, COSCH₃). 1.98 (3H, s, 3-CH₃).
MASS (EI) m/z : 433(M⁺), 386, 358, 317, 270, 229, 175.

### (Example 2240)

### Compound of Formula No. D-392

¹H-NMR (CDCl₃) δ(ppm) : 6.34 (1H, q, J=7Hz, NCH(Me)Ar), [2.30 (s) & 2.29 (s), 3H, 3-CH₃], [1.80 (s) & 1.78 (s), 3H, COCH₃], [1.13 (s) & 1.06 (s), 3H, 5-CH₃].
MASS (EI) m/z : 333(M⁺), 290, 229, 187, 172.

### (Example 2241)

### Compound of Formula No. D-393

¹H-NMR (CDCl₃) δ(ppm) : [6.36 (q, J=7Hz) & 6.32 (q, J=7Hz), 1H, NCH(Me)Ar], [2.30 (s) & 2.29 (s), 3H, 3-CH₃], [1.80 (s) & 1.79 (s), 3H, COCH₃], [1.21 (s) & 1.14 (s), 3H, 5-CH₃].
MASS (EI) m/z : 358(M⁺), 315, 228, 172, 145.

### (Example 2242)

### Compound of Formula No. D-394

¹H-NMR (CDCl₃) δ(ppm) : [5.69 (d, J=11Hz) & 5.30 (d, J=11Hz), 1H, NCH(iPr)Ar], [2.32 (s) & 2.14 (s), 3H, 3-CH₃], [1.83 (s) & 1.82 (s), 3H, COCH₃], [1.32 (s) & 1.05 (s), 3H, 5-CH₃].
MASS (EI) m/z : 386(M⁺), 343, 301, 228, 187.

### (Example 2243)

### Compound of Formula No. D-395

¹H-NMR (CDCl₃) δ(ppm) : 2.46 and 2.44(3H, s), 1090 and 1.88(3H, s), 1.14 and 1.13(3H, s).
MASS (EI) m/z : 344(M⁺), 301, 228, 186, 145.

### (Example 2244)

### Compound of Formula No. D-396

¹H-NMR (CDCl₃) δ(ppm) : 2.44 and 2.44(3H, s), 1.91 and 1.90(3H, s), 1.22 and 1.21(3H, s).
MASS (EI) m/z : 369(M⁺), 326, 300, 228, 186.

### (Example 2245)

### Compound of Formula No. D-397

¹H-NMR (CDCl₃) δ(ppm) : [6.36 (q, J=7Hz) & 6.32 (q, J=7Hz), 1H, NCH(Me)Ar], [2.32 (s) & 2.30 (s), 3H, 3-CH₃], [1.79 (s) & 1.78 (s), 3H, COCH₃], [1.50 (s) & 1.48 (s), 3H, 5-CH₃].
MASS (EI) m/z : 376(M⁺), 333, 319, 293, 246.

### (Example 2246)

### Compound of Formula No. D-398

¹H-NMR (CDCl₃) δ(ppm) : 5.86 (1H, d, J=14Hz, NCH₂Ar), 5.19 (1H, d, J=14Hz, NCH₂Ar), 2.45 (3H, s, CSCH₃), 2.00 (3H, s, 3-CH₃), 1.78 (3H, s, 5-CH₃).
MASS (EI) m/z : 360(M⁺), 307, 293, 149, 116.

### (Example 2247)

### Compound of Formula No. D-400

¹H-NMR (CDCl₃) δ(ppm) : 5.96 (1H, d, J=14Hz, NCH₂Ar), 5.12 (1H, d, J=14Hz, NCH₂Ar), 2.45 (3H, s, CSCH₃), 2.02 (3H, s, 3-CH₃), 1.73 (3H, s, 5-CH₃).
MASS (EI) m/z : 407(M⁺), 391, 362, 163.

### (Example 2248)

### Compound of Formula No. D-401

¹H-NMR (CDCl₃) δ(ppm) : 5.99 (1H, d, J=14Hz, NCH₂Ar), 5.05 (1H, d, J=14Hz, NCH₂Ar), 2.44 (3H, s, CSCH₃), 2.03 (3H, s, 3-CH₃), 1.74 (3H, s, 5-CH₃).
MASS (EI) m/z : 394(M⁺), 360, 335, 150, 116.

### (Example 2249)

### Compound of Formula No. D-403

¹H-NMR (CDCl₃) δ(ppm) : 5.98 (1H, d, J=14Hz, NCH₂Ar), 4.99 (1H, d, J=14Hz, NCH₂Ar), 2.42 (3H, s, CSCH₃), 2.02 (3H, s, 3-CH₃), 1.73 (3H, s, 5-CH₃).
MASS (EI) m/z : 436(M⁺), 377, 318, 192.

### (Example 2250)

### Compound of Formula No. D-404

¹H-NMR (CDCl₃) δ(ppm) : 6.04 (1H, d, J=14Hz, NCH₂Ar), 5.01 (1H, d, J=14Hz, NCH₂Ar), 2.44 (3H, s, CSCH₃), [2.05 (s) & 2.04 (s), 3H, 3-CH₃], [1.72 (s) & 1.71 (s), 3H, 5-CH₃].
MASS (EI) m/z : 422(M⁺), 407, 375, 212.

### (Example 2251)

### Compound of Formula No. D-405

¹H-NMR (CDCl₃) δ(ppm) : [6.08 (d, J=14Hz) & 6.05 (d, J=14Hz), 1H, NCH₂Ar], [5.00 (d, J=14Hz) & 4.94 (d, J=14Hz) , 1H, NCH₂Ar], 2.43 (3H, s, CSCH₃), 2.04 (3H, s, 3-CH₃), 1.70 (3H, s, 5-CH₃). MASS (EI) m/z : 362(M⁺), 319, 228, 172, 118.

### (Example 2252)

### Compound of Formula No. D-406

¹H-NMR (CDCl₃) δ(ppm) : [6.14 (d, J=13Hz) & 6.01 (d, J=13Hz), 1H, NCH₂Ar], [5.04 (d, J=13Hz) & 4.88 (d, J=13Hz), 1H, NCH₂Ar], 2.43 (3H, s, COCH₃), [2.06 (s) & 2.04 (s), 3H, 3-CH₃], [1.71 (s) & 1.67 (s), 3H, 5-CH₃].
MASS (EI) m/z : 450(M⁺), 407, 375, 212, 131.

### (Example 2253)

### Compound of Formula No. D-407

¹H-NMR (CDCl₃) δ(ppm) : [6.03 (d, J=13Hz) & 6.01 (d, J=13Hz), 1H, NCH₂Ar], [5.02 (d, J=13Hz) & 5.00 (d, J=13Hz), 1H, NCH₂Ar], [2.44 (s) & 2.43 (s), 3H, CSCH₃], [2.07 (s) & 2.02 (s), 3H, 3-CH₃], [1.73 (s) & 1.71 (s), 3H, 5-CH₃].
MASS (EI) m/z : 523(M⁺), 407, 279, 212, 164.

### (Example 2254)

### Compound of Formula No. D-408

¹H-NMR (CDCl₃) δ(ppm) : 6.04 (1H, d, J=14Hz, NCH₂Ar), 5.08 (1H, d, J=14Hz, NCH₂Ar), 2.45 (3H, s, CSCH₃), 2.06 (3H, s, 3-CH₃), 1.73 (3H, s, 5-CH₃).
MASS (EI) m/z : 418(M⁺), 402, 377, 174.

### (Example 2255)

### Compound of Formula No. D-409

¹H-NMR (CDCl₃) δ(ppm) : 6.05 (1H, d, J=14Hz, NCH₂Ar), 5.03 (1H, d, J=14Hz, NCH₂Ar), 2.45 (3H, s, CSCH₃), 2.06 (3H, s, 3-CH₃), 1.71 (3H, s, 5-CH₃).
MASS (EI) m/z : 432(M⁺), 391, 373, 188.

### (Example 2256)

### Compound of Formula No. D-410

¹H-NMR (CDCl₃) δ(ppm) : [5.13 (d, J=14Hz) & 4.86 (d, J=14Hz), 1H, NCH₂Ar], [4.67 (d, J=14Hz) & 4.38 (d, J=14Hz), 1H, NCH₂Ar], [2.71 (s) & 2.46 (s), 3H, CSCH₃], [2.02 (s) & 1.94 (s), 3H, 3-CH₃], [1.87 (s) & 1.78 (s), 3H, 5-CH₃].
MASS (EI) m/z : 392(M⁺), 377, 359, 349, 333, 315, 243.

### (Example 2257)

### Compound of Formula No. D-411

¹H-NMR (CDCl₃) δ(ppm) : [6.07 (d, J=13Hz) & 5.69 (d, J=13Hz), 1H, NCH₂Ar], [5.34 (d, J=13Hz) & 4.94 (d, J=13Hz), 1H, NCH₂Ar], [2.72 (s) & 2.48 (s), 3H, CSCH₃], [2.04 (s) & 1.95 (s), 3H, 3-CH₃], [1.85 (s) & 1.74 (s), 3H, 5-CH₃].
MASS (EI) m/z : 408(M⁺), 375, 349, 239, 212, 118.

### (Example 2258)

### Compound of Formula No. D-412

¹H-NMR (CDCl₃) δ(ppm) : 5.01 (1H, d, J=14Hz, NCH₂Ar), 4.29 (1H, d, J=14Hz, NCH₂Ar), 3.72 (2H, d, J=8Hz, NCH₂-iPr), 2.02 (3H, s, 3-CH₃), 1.85 (3H, s, COCH₃), 1.80 (3H, s, 5-CH₃).
MASS (EI) m/z : 368(M⁺), 325, 312, 270, 208.

### (Example 2259)

### Compound of Formula No. D-413

¹H-MMR (CDCl₃) δ(ppm) : 4.84 (1H, d, J=15Hz, NCH₂Ar), 4.35 (1H, d, J=15Hz, NCH₂Ar), 3.75 (1H, dd, J=14Hz, 7Hz, NCH₂-iPr), 3.70 (3H, s, COOCH₃), 3.65 (1H, dd, J=14Hz, 7Hz, NCH₂-iPr), 2.03 (3H, s, 3-CH₃), 1.82 (3H, s, 5-CH₃).
MASS (EI) m/z : 384(M⁺), 369, 341, 328.

### (Example 2260)

### Compound of Formula No. D-414

¹H-NMR (CDCl₃) δ(ppm) : 5.00 (1H, d, J=14Hz, NCH₂Ar), 4.41 (1H, d, J=14Hz, NCH₂Ar), 3.72 (2H, d, J=7Hz, NCH₂-iPr), 1.98 (3H, s, 3-CH₃), 1.81 (3H, s, COCH₃ & 5-CH₃).
MASS (EI) m/z : 339(M⁺), 296, 208.

### (Example 2261)

### Compound of Formula No. D-415

¹H-NMR (CDCl₃) δ(ppm) : 4.85 (1H, d, J=15Hz, NCH₂Ar), 4.46 (1H, d, J=15Hz, NCH₂Ar), 3.75 (1H, dd, J=14Hz, 8Hz, NCH₂-iPr), 3.67 (3H, s, COOCH₃), 3.65 (1H, dd, J=14Hz, 8Hz, NCH₂-iPr), 1.97 (3H, s, 3-CH₃), 1.82 (3H, s, 5-CH₃).
MASS (EI) m/z : 355(M⁺), 224.

### (Example 2262)

### Compound of Formula No. D-416

¹H-NMR (CDCl₃) δ(ppm) : 5.03 (1H, d, J=15Hz, NCH₂Ar), 4.55 (1H, d, J=15Hz, NCH₂Ar), 3.74 (2H, d, J=7Hz, NCH₂-iPr), 1.99 (3H, s, 3-CH₃), 1.86 (3H, s, COCH₃), 1.84 (3H, s, 5-CH₃).
MASS (EI) m/z : 350(M⁺), 335, 307, 208.

### (Example 2263)

### Compound of Formula No. D-417

¹H-NMR (CDCl₃) δ(ppm) : 4.93 (1H, d, J=15Hz, NCH₂Ar), 4.57 (1H, d, J=15Hz, NCH₂Ar), 3.75 (1H, dd, J=14Hz, 7Hz, NCH₂-iPr), 3.70 (3H, s, COOCH₃), 3.63 (1H, dd, J=14Hz, 7Hz, NCH₂-iPr), 1.98 (3H, s, 3-CH₃), 1.86 (3H, s, 5-CH₃).
MASS (EI) m/z : 366(M⁺), 323, 310, 224.

### (Example 2264)

### Compound of Formula No. D-418

¹H-NMR (CDCl₃) δ(ppm) : 4.96 (1H, d, J=15Hz, NCH₂Ar), 4.77 (1H, d, J=15Hz, NCH₂Ar), 3.73 (2H, d, J=7Hz, NCH₂-iPr), 1.90 (3H, s, 3-CH₃), 1.84 (3H, s, COCH₃), 1.81 (3H, s, 5-CH₃).
MASS (EI) m/z : 330(M⁺), 315, 287, 208.

### (Example 2265)

### Compound of Formula No. D-419

¹H-NMR (CDCl₃) δ(ppm) : 4.90 (1H, d, J=14Hz, NCH₂Ar), 4.72 (1H, d, J=14Hz, NCH₂Ar), 3.74 (1H, dd, J=14Hz, 7Hz, NCH₂-iPr), 3.69 (3H, s, COOCH₃), 3.66 (1H, dd, J=14Hz, 7Hz, NCH₂-iPr), 1.91 (3H, s, 3-CH₃), 1.80 (3H, s, 5-CH₃).
MASS (EI) m/z : 346(M⁺), 331, 303, 290, 224.

### (Example 2266)

### Compound of Formula No. D-420

¹H-NMR (CDCl₃) δ(ppm) : 5.28 (1H, d, J=14H2, NCH₂Ar), 4.58 (1H, d, J=14Hz, NCH₂Ar), 2.00 (3H, s, 3-CH₃), 1.92 (3H, s, COCH₃), 1.66 (3H, s, 5-CH₃).
MASS (EI) m/z : 369(M⁺), 326, 228, 172, 149, 118.

### (Example 2267)

### Compound of Formula No. D-421

¹H-NMR (CDCl₃) δ(ppm) : 5.13 (1H, d, J=14Hz, NCH₂Ar), 4.67 (1H, d, J=14Hz, NCH₂Ar), 1.97 (3H, s, 3-CH₃), 1.91 (3H, s, COCH₃), 1.77 (3H, s, 5-CH₃).
MASS (EI) m/z : 390(M⁺), 347, 293, 228, 186, 172, 162.

### (Example 2268)

### Compound of Formula No. D-422

¹H-NMR (CDCl₃) δ(ppm) : 5.13 (1H, d, J=14Hz, NCH₂Ar), 4.67 (1H, d, J=14Hz, NCH₂Ar), 1.97 (3H, s, 3-CH₃), 1.91 (3H, s, COCH₃), 1.77 (3H, s, 5-CH₃).
MASS (EI) m/z : 404(M⁺), 361, 228, 186, 176, 145.

### (Example 2269)

### Compound of Formula No. D-423

¹H-NMR (CDCl₃) δ(ppm) : 5.20 (1H, d, J=14Hz, NCH₂Ar), 4.54 (1H, d, J=14Hz, NCH₂Ar), 2.01 (3H, s, 3-CH₃), 1.91 (3H, s, COCH₃), 1.70 (3H, s, 5-CH₃).
MASS (EI) m/z : 432(M⁺), 389, 228, 204, 189, 172, 145, 118.

### (Example 2270)

### Compound of Formula No. D-424

¹H-NMR (CDCl₃) δ(ppm) : 5.43 (1H, d, J=14Hz, NCH₂Ar), 4.63 (1H, d, J=14Hz, NCH₂Ar), 1.97 (3H, s, 3-CH₃), 1.92 (3H, s, COCH₃), 1.50 (3H, s, 5-CH₃).
MASS (EI) m/z : 390(M⁺), 347, 228, 186, 172, 162, 118.

### (Example 2271)

### Compound of Formula No. D-425

¹H-NMR (CDCl₃) δ(ppm) : 5.45 (1H, d, J=14Hz, NCH₂Ar), 4.63 (1H, d, J=14Hz, NCH₂Ar), 1.98 (3H, s, 3-CH₃), 1.92 (3H, s, COCH₃), 1.50 (3H, s, 5-CH₃).
MASS (EI) m/z : 404(M⁺), 361, 228, 186, 176, 145.

### (Example 2272)

### Compound of Formula No. D-426

¹H-NMR (CDCl₃) δ(ppm) : 5.39 (1H, d, J=14Hz, NCH₂Ar), 4.85 (1H, d, J=14Hz, NCH₂Ar), 1.93 (3H, s, 3-CH₃), 1.90 (3H, s, COCH₃), 1.56 (3H, s, 5-CH₃).
MASS (EI) m/z : 377(M⁺), 349, 334, 316, 290, 274, 258.

### (Example 2273)

### Compound of Formula No. D-427

¹H-NMR (CDCl₃) δ(ppm) : 5.07 (1H, d, J=14Hz, NCH₂Ar), 4.50 (1H, d, J=14Hz, NCH₂Ar), 2.02 (3H, s, 3-CH₃), 1.90 (3H, s, COCH₃), 1.78 (3H, s, 5-CH₃).
MASS (EI) m/z : 320(M⁺), 277, 228, 186, 172, 145, 118.

### (Example 2274)

### Compound of Formula No. D-428

¹H-NMR (CDCl₃) δ(ppm) : 5.08 (1H, d, J=14Hz, NCH₂Ar), 4.50 (1H, d, J=14Hz, NCH₂Ar), 3.76 (2H, s, COCH₂O), 2.00 (3H, s, 3-CH₃), 1.76 (3H, s, 5-CH₃).
MASS (EI) m/z : 350(M⁺), 335, 305, 277, 258, 228, 214.

### (Example 2275)

### Compound of Formula No. D-429

¹H-NMR (CDCl₃) δ(ppm) : 4.88 (1H, d, J=14Hz, NCH₂Ar), 4.55 (1H, d, J=14Hz, NCH₂Ar), 3.71 (3H, brs, COOCH₃), 2.01 (3H, s, 3-CH₃), 1.82 (3H, s, 5-CH₃).
MASS (EI) m/z : 336(M⁺), 305, 293, 277, 244, 212, 149.

### (Example 2276)

### Compound of Formula No. D-430

¹H-NMR (CDCl₃) δ(ppm) : 5.02 (1H, d, J=14Hz, NCH₂Ar), 4.48 (1H, d, J=14Hz, NCH₂Ar), 2.04 (3H, s, 3-CH₃), 1.90 (3H, s, COCH₃), 1.84 (3H, s, 5-CH₃).
MASS (EI) m/z : 354(M⁺), 311, 228, 186, 172, 145, 126.

### (Example 2277)

### Compound of Formula No. D-431

¹H-NMR (CDCl₃) δ(ppm) : 4.80 (1H, d, J=14Hz, NCH₂Ar), 4.52 (1H, d, J=14Hz, NCH₂Ar), 3.74 (3H, brs, COOCH₃), 2.10 (3H, s, 3-CH₃), 1.99 (3H, s, 5-CH₃).
MASS (EI) m/z : 336(M⁺), 305, 293, 277, 244, 212, 149.

### (Example 2278)

### Compound of Formula No. D-432

¹H-NMR (CDCl₃) δ(ppm) : 4.91 (1H, d, J=14Hz, NCH₂Ar), 4.52 (1H, d, J=14Hz, NCH₂Ar), 2.08 (3H, s, 3-CH₃), 1.99 (3H, s, COCH₃), 1.90 (3H, s, 5-CH₃).
MASS (EI) m/z : 393(M⁺), 350, 228, 186, 172.

### (Example 2279)

### Compound of Formula No. D-433

¹H-NMR (CDCl₃) δ(ppm) : 4.96 (1H, d, J=14Hz, NCH₂Ar), 4.76 (1H, d, J=14Hz, NCH₂Ar), 2.06 (3H, s, 3-CH₃), 1.95 (3H, s, COCH₃), 1.90 (3H, s, 5-CH₃).
MASS (EI) m/z : 355(M⁺), 312, 228, 172, 145, 118.

### (Example 2280)

### Compound of Formula No. D-434

¹H-NMR (CDCl₃) δ(ppm) : 5.02 (1H, d, J=14Hz, NCH₂Ar), 4.53 (1H, d, J=14Hz, NCH₂Ar), 2.06 (3H, s, 3-CH₃), 1.93 (3H, s, COCH₃), 1.89 (3H, s, 5-CH₃).
MASS (EI) m/z : 355(M⁺), 312, 228, 172, 145, 118.

### (Example 2281)

### Compound of Formula No. D-435

¹H-NMR (CDCl₃) δ(ppm) : 4.96 (1H, d, J=14Hz, NCH₂Ar), 4.48 (1H, d, J=14Hz, NCH₂Ar), 2.06 (3H, s, 3-CH₃), 1.94 (3H, s, COCH₃), 1.89 (3H, s, 5-CH₃).
MASS (EI) m/z : 355(M⁺), 312, 229, 183, 172, 145, 118.

### (Example 2282)

### Compound of Formula No. D-436

¹H-NMR (CDCl₃) δ(ppm) : 4.99 (1H, d, J = 13.4 Hz, NCH₂Ar), 4.47 (1H, d, J = 13.4 Hz, NCH₂Ar), 3.73 (1H, dd, J = 5.8 Hz, J = 12.2 Hz, NCH₂CH), 3.68 (1H, dd, J = 5.8 Hz, J = 12.2 Hz, NCH₂CH), 3.60 (1H, d, J = 8.2 Hz, COCH₂), 3.58 (1H, d, J = 8.2 Hz, COCH₂), 3.22 (2H, s, SCH₂CN), 1.93 (3H, s, 3-CH₃), 1.72 (3H, s, 5-CH₃).
MASS (EI) m/z : 395(M⁺), 380, 355, 323, 281, 267, 256, 194, 150, 137, 129, 116, 97, 86, 72, 59.

### (Example 2283)

### Compound of Formula No. D-437

¹H-NMR (CDCl₃) δ(ppm) : 4.95 (1H, d, J = 14.0 Hz, NCH₂Ar), 4.64 (1H, d, J = 14.0 Hz, NCH₂Ar), 3.63 (1H, d, J = 7.9 Hz, COCH₂), 3.60 (1H, d, J = 7.9 Hz, COCH₂), 3.31 (2H, s, SCH₂CN), 1.99 (3H, s, 3-CH₃), 1.87 (3H, s, 5-CH₃).
MASS (EI) m/z : 415(M⁺), 375, 343, 329, 299, 214, 186, 172, 144, 129, 118, 86, 77.

### (Example 2284)

### Compound of Formula No. D-438

¹H-NMR (CDCl₃) δ(ppm) : 4.95 (1H, d, J=14Hz, NCH₂Ar), 4.50 (1H, d, J=14Hz, NCH₂Ar), 4.17 (2H, t, J=7Hz, NCH₂CH₂CF₃), 1.93 (3H, s, 3-CH₃), 1.77 (3H, s, 5-CH₃), 1.41-1.26 (m, 1H, COCH(CH₂)₂).
MASS (EI) m/z : 390(M⁺), 322, 274, 192, 69.

### (Example 2285)

### Compound of Formula No. D-439

¹H-NMR (CDCl₃) δ(ppm) : 4.79 (1H, d, J=14Hz, NCH₂Ar), 4.48 (1H, d, J=14Hz, NCH₂Ar), 4.13 (2H, t, J=7Hz, NCH₂CH₂CF₃), 3.68 (3H, s, COOCH₃), 1.89 (3H, s, 3-CH₃), 1.76 (3H, s, 5-CH₃).
MASS (EI) m/z : 380(M⁺), 321, 264, 232.

### (Example 2286)

### Compound of Formula No. D-440

¹H-NMR (CDCl₃) δ(ppm) : 5.02 (1H, d, J=14Hz, NCH₂Ar), 4.68 (2H, s, COCH₂O), 4.61 (1H, d, J=14Hz, NCH₂Ar), 2.10 (3H, s, 3-CH₃), 1.96 (3H, s, 5-CH₃).
MASS (EI) m/z : 437(M⁺), 300, 136.

### (Example 2287)

### Compound of Formula No. D-441

¹H-NMR (CDCl₃) δ(ppm) : 4.98 (1H, d, J=14Hz, NCH₂Ar), 4.67 (1H, d, J=14Hz, NCH₂Ar), 4.63 (1H, d, J=16Hz, COCH₂N), 4.16 (1H, d, J=16Hz, COCH₂N), 2.08 (3H, s, 3-CH₃), 2.02 (3H, s, 5-CH₃).
MASS (EI) m/z : 437(M⁺), 302, 136.

### (Example 2288)

### Compound of Formula No. D-442

¹H-NMR (CDCl₃) δ(ppm) : 8.38 (1H, dd, J=5,2Hz, COO-(6-H-2-Pyr)), 7.02 (1H, d, J=8Hz, COO-(3-H-2-Pyr)), 4.94 (1H, d, J=14Hz, NCH₂Ar), 4.68 (1H, d, J=14Hz, NCH₂Ar), 2.13 (3H, s, 3-CH₃), 2.00 (3H, s, 5-CH₃).
MASS (EI) m/z : 423(M⁺), 379, 329, 300, 212.

### (Example 2289)

### Compound of Formula No. D-443

¹H-NMR (CDCl₃) δ(ppm) : 6.29 (1H, d, J=9Hz, CO-(3-H-2-oxo-1-Pyr)), 6.10 (1H, dd, J=7,7Hz, CO-(5-H-2-oxo-1-Pyr)), 5.15 (2H, brs, NCH₂Ar), 2.19 (3H, s, 3-CH₃), 1.70 (3H, s, 5-CH₃).
MASS (EI) m/z : 423(M⁺), 329, 300, 212.

### (Example 2290)

### Compound of Formula No. D-444

¹H-NMR (CDCl₃) δ(ppm) : 7.68 (1H, s, N-C₆H₄-3'-OH), 6.98 (1H, t, J=2Hz, N-C₆H₄-2'-H), 4.99 (1H, d, J=14Hz, NCH₂Ar), 4.57 (1H, d, J=14Hz, NCH₂Ar), 1.99 (3H, s, 3-CH₃), 1.91 (3H, s, COCH₃), 1.82 (3H, s, 5-CH₃).
MASS (EI) m/z : 360(M⁺), 265, 244, 217, 188, 149.

### (Example 2291)

### Compound of Formula No. E-1

¹H-NMR (CDCl₃) δ(ppm) : 7.04 (1H, s, 3-H), 4.75 (2H, brs, NCH₂Ar), 4.05 (2H, q, J=7Hz, NCH₂Me), 3.79 (2H, brs, COCH₂O), 2.30 (2H, t, J=8Hz, 5-CH₂Et).
MASS (EI) m/z : 340(M⁺), 325, 310, 295, 267, 224.

### (Example 2292)

### Compound of Formula No. E-2

¹H-NMR (CDCl₃) δ(ppm) : 7.06 (1H, s, 3-H), 4.83 (2H, brs, NCH₂Ar), 4.05 (2H, q, J=7Hz, NCH₂Me), 3.81 (2H, brs, COCH₂O), 2.34 (2H, t, J=8Hz, 5-CH₂Et).
MASS (EI) m/z : 360(M⁺), 345, 330, 315, 287, 259.

### (Example 2293)

### Compound of Formula No. E-3

¹H-NMR (CDCl₃) δ(ppm) : 7.09 (1H, s, 3-H), 4.75 (2H, brs, NCH₂Ar), 4.04 (2H, q, J=7Hz, NCH₂Me), 3.80 (2H, brs, COCH₂O), 2.24 (2H, t, J=8Hz, 5-CH₂Et).
MASS (EI) m/z : 399(M⁺), 384, 371, 354, 326, 298, 194.

### (Example 2294)

### Compound of Formula No. E-4

¹H-NMR (CDCl₃) δ(ppm) : 7.12 (1H, s, 3-H), 4.83 (2H, brs, NCH₂Ar), 4.41 (2H, brs, COCH₂O), 3.95 (2H, t, J=7Hz, NCH₂Et), 2.37 (2H, t, J=7Hz, 5-CH₂-Pr).
MASS (EI) m/z : 450(M⁺), 357, 343, 314, 273, 220, 150.

### (Example 2295)

### Compound of Formula No. E-5

¹H-NMR (CDCl₃) δ(ppm) : 7.15 (1H, s, 3-H), 5.00 (1H, s, 5-CH₂F), 4.79 (2H, s, NCH₂Ar), 4.75 (1H, s, 5-CH₂F), 3.92 (2H, d, J=7Hz, NCH₂-iPr), 1.90 (3H, s, COCH₃).
MASS (EI) m/z : 328(M⁺), 308, 295, 286, 266, 243, 210.

### (Example 2296)

### Compound of Formula No. E-6

¹H-NMR (CDCl₃) δ(ppm) : 7.19 (1H, s, 3-H), 5.14 (1H, brs, 5-CH₂F), 4.89 (1H, brs, 5-CH₂F), 4.76 (2H, s, NCH₂Ar), 3.89 (2H, d, J=7Hz, NCH₂-iPr), 3.71 (3H, s, COOCH₃).
MASS (EI) m/z : 344(M⁺), 311, 301, 288, 268, 228, 186.

### (Example 2297)

### Compound of Formula No. E-7

¹H-NMR (CDCl₃) δ(ppm) : 7.15 (1H, s, 3-H), 4.80 (2H, s, NCH₂Ar), 4.13 (2H, s, 5-CH₂Cl), 3.92 (2H, d, J=7Hz, NCH₂-iPr), 1.93 (3H, s, COCH₃).
MASS (EI) m/z : 344(M⁺), 309, 302, 267, 246, 228, 186.

### (Example 2298)

### Compound of Formula No. E-8

¹H-NMR (CDCl₃) δ(ppm) : 7.13 (1H, s, 3-H), 4.76 (2H, s, NCH₂Ar), 4.27 (2H, s, 5-CH₂Cl), 3.90 (2H, d, J=7Hz, NCH₂-iPr), 3.73 (3H, brs, COOCH₃).
MASS (EI) m/z : 360(M⁺), 325, 311, 304, 281, 268.

### (Example 2299)

### Compound of Formula No. E-9

¹H-NMR (CDCl₃) δ(ppm) : 7.35 (1H, brs, 3-H), 4.89 (2H, s, NCH₂Ar), 4.01 (2H, d, J=7Hz, NCH₂-iPr), 3.82 (3H, s, COOCH₃). MASS (EI) m/z : 337(M⁺), 322, 294, 281, 221, 116.

### (Example 2300)

### Compound of Formula No. E-10

¹H-NMR (CDCl₃) δ(ppm) : 7.19 (1H, s, 3-H), 4.80 (2H, s, NCH₂Ar), 4.22 (2H, brs, 5-CH₂OH), 3.92 (2H, d, J=7Hz, NCH₂-iPr), 1.91 (3H, s, COCH₃).
MASS (EI) m/z : 326(M⁺), 308, 295, 284, 266, 241, 223.

### (Example 2301)

### Compound of Formula No. E-11

¹H-NMR (CDCl₃) δ(ppm) : 7.22 (1H, s, 3-H), 4.77 (2H, s, NCH₂Ar), 4.20 (2H, brs, 5-CH₂OH), 3.92 (2H, d, J=7Hz, NCH₂-iPr), 3.85 (3H, brs, COOCH₃).
MASS (EI) m/z : 342(M⁺), 311, 281, 268, 226, 209.

### (Example 2302)

### Compound of Formula No. E-12

¹H-NMR (CDCl₃) δ(ppm) : 7.13 (1H, s, 3-H), 4.76 (2H, s, NCH₂Ar), 3.94 (2H, s, 5-CH₂OMe), 3.87 (2H, d, J=7Hz, NCH₂-iPr), 1.89 (3H, s, COCH₃).
MASS (EI) m/z : 340(M⁺), 325, 308, 298, 283, 266, 224.

### (Example 2303)

### Compound of Formula No. E-13

¹H-NMR (CDCl₃) δ(ppm) : 7.15 (1H, s, 3-H), 4.73 (2H, brs, NCH₂Ar), 4.07 (2H, s, 5-CH₂OMe), 3.88 (2H, d, J=7Hz, NCH₂-iPr), 3.69 (3H, brs, COOCH₃).
MASS (EI) m/z : 356(M⁺), 341, 325, 311, 281, 268, 253.

### (Example 2304)

### Compound of Formula No. E-14

¹H-NMR (CDCl₃) δ(ppm) : 7.15 (1H, s, 3-H), 4.80 (2H, brs, NCH₂Ar), 4.74 (2H, s, 5-CH₂OSO₃Me), 3.85 (2H, d, J=7Hz, NCH₂-iPr), 3.69 (3H, brs, COOCH₃).
MASS (EI) m/z : 420(M⁺), 384, 341, 311, 281, 268, 253.

### (Example 2305)

### Compound of Formula No. E-15

¹H-NMR (CDCl₃) δ(ppm) : 7.20 (1H, s, 3-H), 4.81 (2H, s, NCH₂Ar), 4.35 (2H, d, J=7Hz, NCH₂-iPr), 4.01 (3H, s, COOCH₃).
MASS (EI) m/z : 356(M⁺), 312, 297, 279, 241, 196, 116.

### (Example 2306)

### Compound of Formula No. E-16

¹H-NMR (CDCl₃) δ(ppm) : 7.21 (1H, s, 3-H), 4.78 (2H, brs, NCH₂Ar), 4.33 (2H, d, J=7Hz, NCH₂-iPr), 3.73 (3H, s, 5-COOCH₃), 1.90 (3H, s, COCH₃).
MASS (EI) m/z : 354(M⁺), 312, 279, 269, 253, 223, 154.

### (Example 2307)

### Compound of Formula No. E-17

¹H-NMR (CDCl₃) δ(ppm) : 7.19 (1H, s, 3-H), 4.75 (2H, s, NCH₂Ar), 4.30 (2H, d, J=7Hz, NCH₂-iPr), 3.76 (3H, s, 5-COOCH₃), 3.66 (3H, s, COOCH₃).
MASS (EI) m/z : 370(M⁺), 339, 327, 311, 279, 254, 116.

### (Example 2308)

### Compound of Formula No. E-19

¹H-NMR (CDCl₃) δ(ppm) : 6.91 (1H, s, 3-H), 4.83 (2H, s, NCH₂), 4.00 (2H, d, J=4.9Hz, CH₂OH), 3.72 (2H, d, J=7.6Hz, CH₂-iPr), 3.68 (3H, s, OMe).
MASS (EI) m/z : 342(M⁺), 311, 283, 269, 226, 196, 168.

### (Example 2309)

### Compound of Formula No. E-20

¹H-NMR (CDCl₃) δ(ppm) : 6.92 (1H, s, 3-H), 4.80 (2H, brs, NCH₂), 3.95 (2H, s, CH₂OMe), 3.72 (2H, d, J=7.6Hz, CH₂-iPr), 3.70 (3H, s, OMe), 3.41 (3H, s, CH2OMe).
MASS (EI) m/z : 356(M⁺), 341, 325, 283, 255, 240, 227.

### (Example 2310)

### Compound of Formula No. E-21

¹H-NMR (CDCl₃) δ(ppm) : 6.98 (1H, s, 3-H), 4.83 (2H, brs, NCH₂), 4.55 (2H, s, CH₂OPh), 3.73 (2H, d, J=7.6Hz, CH₂-iPr), 3.72 (3H, s, OMe).
MASS (EI) m/z : 418(M⁺), 399, 387, 325, 302.

### (Example 2311)

### Compound of Formula No. E-22

¹H-NMR (CDCl₃) δ(ppm) : 6.95 (1H, s, 3-H), 4.79 (2H, br s, NCH₂), 4.53 (2H, s, CH₂OAc), 3.81 (3H, s, OMe), 3.72 (2H, d, J=7.6Hz, CH₂-iPr), 2.15 (3H, s, COMe).
MASS (EI) m/z : 384(M⁺), 353, 341, 311, 284, 268, 196.

### (Example 2312)

### Compound of Formula No. E-23

¹H-NMR (CDCl₃) δ(ppm) : 6.98 (1H, brs, 3-H), 4.71 (2H, br s, NCH₂), 3.73 (6H, brs, OMe x 2), 3.65 (2H, d, J=7.0Hz, CH₂-iPr). MASS (EI) m/z : 342(M⁺), 311, 286, 226, 170.

### (Example 2313)

### Compound of Formula No. E-24

¹H-NMR (CDCl₃) δ(ppm) : 6.97 (1H, s, 3-H), 5.73 (1H, m, isoxazole), 4.81 (2H, brs, NCH₂), 4.74 (2H, s, CH₂O), 3.85 (3H, s, OMe), 3.73 (2H, d, J=7.6Hz, CH₂-iPr), 2.32 (3H, s, isoxazole-Me)
MASS (EI) m/z : 423(M⁺), 407, 342, 307, 282, 226, 208.

### (Example 2314)

### Compound of Formula No. E-25

¹H-NMR (CDCl₃) δ(ppm) : 6.86 (1H, s, 5-H), 4.84 (2H, brs, NCH₂Ar), 4.38 (2H, s, 3-CH₂Cl), 3.80 (2H, d, J=7Hz, NCH₂-iPr), 1.96 (3H, s, COCH₃).
MASS (EI) m/z : 344(M⁺), 309, 302, 267, 223, 210, 186.

### (Example 2315)

### Compound of Formula No. E-26

¹H-NMR (CDCl₃) δ(ppm) : 6.83 (1H, s, 5-H), 4.79 (2H, s, NCH₂Ar), 4.45 (2H, brs, 3-CH₂Cl), 3.76 (2H, d, J=7Hz, NCH₂-iPr), 3.74 (3H, brs, COOCH₃).
MASS (EI) m/z : 360(M⁺), 325, 317, 304, 281, 265.

### (Example 2316)

### Compound of Formula No. E-27

¹H-NMR (CDCl₃) δ(ppm) : 7.16 (1H, brs, 5-H), 4.89 (2H, s, NCH₂Ar), 3.87 (2H, d, J=7Hz, NCH₂-iPr), 3.81 (3H, s, COOCH₃). MASS (EI) m/z : 337(M⁺), 292, 278, 221, 191, 116.

### (Example 2317)

### Compound of Formula No. E-28

¹H-NMR (CDCl₃) δ(ppm) : 6.89 (1H, s, 5-H), 4.82 (2H, brs, NCH₂Ar), 4.44 (2H, d, J=5Hz, 3-CH₂OH), 3.79 (2H, d, J=7Hz, NCH₂-iPr), 1.95 (3H, s, COCH₃).
MASS (EI) m/z : 326(M⁺), 308, 284, 265, 241, 210, 174.

### (Example 2318)

### Compound of Formula No. E-29

¹H-NMR (CDCl₃) δ(ppm) : 6.92 (1H, brs, 5-H), 4.76 (2H, s, NCH₂Ar), 4.44 (2H, d, J=5Hz, 3-CH₂OH), 3.78 (3H, brs, COOCH₃), 3.77 (2H, d, J=7Hz, NCH₂-iPr).
MASS (EI) m/z : 342(M⁺), 324, 313, 299, 281, 265.

### (Example 2319)

### Compound of Formula No. E-30

¹H-NMR (CDCl₃) δ(ppm) : 6.89 (1H, s, 5-H), 4.81 (2H, brs, NCH₂Ar), 4.22 (2H, s, 3-CH₂OMe), 3.79 (2H, d, J=7Hz, NCH₂-iPr), 1.94 (3H, s, COCH₃).
MASS (EI) m/z : 340(M⁺), 325, 308, 298, 265, 223.

### (Example 2320)

### Compound of Formula No. E-31

¹H-NMR (CDCl₃) δ(ppm) : 6.90 (1H, brs, 5-H), 4.77 (2H, brs, NCH₂Ar), 4.30 (2H, s, 3-CH₂OMe), 3.77 (2H, d, J=7Hz, NCH₂-iPr), 3.71 (3H, brs, COOCH₃).
MASS (EI) m/z : 356(M⁺), 341, 324, 309, 281, 267, 223.

### (Example 2321)

### Compound of Formula No. E-32

¹H-NMR (CDCl₃) δ(ppm) : 7.04 (1H, brs, 5-H), 4.84 (2H, s, NCH₂Ar), 3.88 (2H, d, J=7Hz, NCH₂-iPr), 3.71 (3H, brs, COOCH₃). MASS (EI) m/z : 356(M⁺), 338, 310, 297, 279, 223, 116.

### (Example 2322)

### Compound of Formula No. E-33

¹H-NMR (CDCl₃) δ(ppm) : 7.00 (1H, s, 5-H), 4.82 (2H, brs, NCH₂Ar), 3.90 (3H, s, 3-COOCH₃), 3.90 (2H, d, J=7Hz, NCH₂-iPr), 1.93 (3H, s, COCH₃).
MASS (EI) m/z : 354(M⁺), 323, 311, 279, 223, 150, 116.

### (Example 2323)

### Compound of Formula No. E-34

¹H-NMR (CDCl₃) δ(ppm) : 6.99 (1H, brs, 5-H), 4.81 (2H, s, NCH₂Ar), 3.90 (3H, s, 3-COOCH₃), 3.87 (2H, d, J=7Hz, NCH₂-iPr), 3.68 (3H, brs, COOCH₃).
MASS (EI) m/z : 370(M⁺), 338, 310, 295, 279, 223, 116.

### (Example 2324)

### Compound of Formula No. E-35

¹H-NMR (CDCl₃) δ(ppm) : 4.82 (1H, d, J=14Hz, NCH₂Ar), 4.80 (1H, d, J=14Hz, NCH₂Ar), 1.97 (3H, s, 3-CH₃), 1.96 (3H, s, COCH₃). MASS (EI) m/z : 364(M⁺), 329, 322, 248, 206, 192, 116.

### (Example 2325)

### Compound of Formula No. E-36

¹H-NMR (CDCl₃) δ(ppm) : 4.83 (1H, d, J=14Hz, NCH₂Ar), 4.69 (1H, d, J=14Hz, NCH₂Ar), 3.75 (3H, brs, COOCH₃), 1.95 (3H, s, 3-CH₃). MASS (EI) m/z : 380(M⁺), 264, 192, 138, 116.

### (Example 2326)

### Compound of Formula No. E-37

¹H-NMR (CDCl₃) δ(ppm) : 5.02 (1H, d, J=14Hz, NCH₂Ar), 4.72 (1H, d, J=14Hz, NCH₂Ar), 2.06 (3H, s, 3-CH₃), 2.01 (3H, s, COCH₃). MASS (EI) m/z : 355(M⁺), 313, 197, 183, 129, 116.

### (Example 2327)

### Compound of Formula No. E-38

¹H-NMR (CDCl₃) δ(ppm) : 4.85 (2H, s, NCH₂Ar), 3.81 (3H, brs, COOCH₃), 2.06 (3H, s, 3-CH₃).
MASS (EI) m/z : 371(M⁺), 312, 255, 224, 183, 129, 116.

### (Example 2328)

### Compound of Formula No. E-39

¹H-NMR (CDCl₃) δ(ppm) : 5.21 (1H, d, J=14Hz, NCH₂Ar), 4.52 (1H, d, J=14Hz, NCH₂Ar), 1.98 (3H, s, COCH₃).
MASS (EI) m/z : 452(M⁺), 410, 294, 280, 172, 116.

### (Example 2329)

### Compound of Formula No. E-40

¹H-NMR (CDCl₃) δ(ppm) : 4.93 (1H, d, J=14Hz, NCH₂Ar), 4.67 (1H, d, J=14Hz, NCH₂Ar), 2.36-2.22 (4H, m, 3-CH₂CH₃ & 5-CH₂CH₃), 1.95 (3H, s, COCH₃).
MASS (EI) m/z : 372(M⁺), 329, 256, 200, 132, 116.

### (Example 2330)

### Compound of Formula No. E-41

¹H-NMR (CDCl₃) δ(ppm) : 4.83 (2H, brs, NCH₂Ar), 2.47 (2H, q, J=8Hz, 5-CH₂Me), 2.00 (3H, s, COCH₃).
MASS (EI) m/z : 344(M⁺), 302, 228, 186, 172, 132, 116.

### (Example 2331)

### Compound of Formula No. E-42

¹H-NMR (CDCl₃) δ(ppm) : 4.76 (2H, s, NCH₂Ar), 3.75 (3H, brs, COOCH₃), 2.44 (2H, q, J=8Hz, 5-CH₂Me).
MASS (EI) m/z : 360(M⁺), 344, 302, 244, 228, 132, 116.

### (Example 2332)

### Compound of Formula No. E-43

¹H-NMR (CDCl₃) δ(ppm) : 4.73 (2H, s, NCH₂Ar), 2.47 (2H, brq, J=8Hz, 5-CH₂Me), 1.45 (9H, brs, OC(CH₃)₃).
MASS (EI) m/z : 402(M⁺), 346, 302, 186, 132, 116.

### (Example 2333)

### Compound of Formula No. E-44

¹H-NMR (CDCl₃) δ(ppm) : 7.25 (1H, s, 3-H), 4.83 (2H, brs, NCH₂Ar), 2.40 (2H, brt, J=8Hz, 5-CH₂Et), 2.00 (3H, s, COCH₃).
MASS (EI) m/z : 358(M⁺), 316, 242, 200, 173, 149, 116.

### (Example 2334)

### Compound of Formula No. E-46

¹H-NMR (CDCl₃) δ(ppm) : 7.26 (1H, s, 3-H), 4.76 (2H, s, NCH₂Ar), 3.75 (3H, brs, COOCH₃), 2.39 (2H, brt, J=8Hz, 5-CH₂Et).
MASS (EI) m/z : 374(M⁺), 258, 226, 198, 146, 116.

### (Example 2335)

### Compound of Formula No. E-47

¹H-NMR (CDCl₃) δ(ppm) : 4.72 (2H, s, NCH₂Ar), 2.42 (2H, brt, J=8Hz, 5-CH₂Et), 1.45 (9H, s, OC(CH₃)₃).
MASS (EI) m/z : 416(M⁺), 360, 316, 244, 146, 116.

### (Example 2336)

### Compound of Formula No. E-48

¹H-NMR (CDCl₃) δ(ppm) : 7.08 (1H, s, 3-H), 5.49 (1H, d, J=14Hz, NCH₂Ar), 4.16 (1H, d, J=14Hz, NCH₂Ar), 3.05 (1H, hept, J=7Hz, 5-CHMe₂), 2.02 (3H, s, COCH₃).
MASS (EI) m/z : 358(M⁺), 316, 242, 200, 173, 146, 116.

### (Example 2337)

### Compound of Formula No. E-49

¹H-NMR (CDCl₃) δ(ppm) : 7.08 (1H, brs, 3-H), 4.74 (2H, brs, NCH₂Ar), 3.75 (3H, brs, COOCH₃), 2.98 (1H, hept, J=7Hz, 5-CHMe₂). MASS (EI) m/z : 374(M⁺), 258, 226, 146, 116.

### (Example 2338)

### Compound of Formula No. E-50

¹H-NMR (CDCl₃) δ(ppm) : 7.06 (1H, brs, 3-H), 4.69 (2H, brs, NCH₂Ar), 2.98 (1H, hept, J=7Hz, 5-CHMe₂), 1.45 (9H, s, OC(CH₃)₃) MASS (EI) m/z : 416(M⁺), 360, 316, 244, 200, 186, 146, 116.

### (Example 2339)

### Compound of Formula No. E-51

¹H-NMR (CDCl₃) δ(ppm) : 4.85 (2H, s, NCH₂Ar), 2.03 (3H, s, COCH₃).
MASS (EI) m/z : 350(M⁺), 308, 234, 192, 138, 116.

### (Example 2340)

### Compound of Formula No. E-54

¹H-NMR (CDCl₃) δ(ppm) : 4.94 (2H, s, NCH₂Ar), 2.11 (3H, s, COCH₃).
MASS (EI) m/z : 341(M⁺), 299, 183, 170, 129, 116.

### (Example 2341)

### Compound of Formula No. E-57

¹H-NMR (CDCl₃) δ(ppm) : 6.84 (1H, s, 5-H), 4.76 (2H, brs, NCH₂Ar), 3.75 (2H, d, J = 7.6 Hz, NCH₂CH), 2.39 (2H, q, J = 7.6 Hz, 3-CH₂), 1.91 (3H, s, COCH₃).
MASS (EI) m/z : 324(M⁺), 282, 239, 282, 239, 226, 208.

### (Example 2342)

### Compound of Formula No. E-58

¹H-NMR (CDCl₃) δ(ppm) : 7.07 (1H, s, 3H) , 4.76 (2H, brs, NCH₂Ar), 3.78 (2H, d, J = 7.6 Hz, NCH₂CH), 2.35 (2H, q, J = 7.6 Hz, 5-CH₂), 1.90 (3H, s, COCH₃).
MASS (EI) m/z : 324(M⁺), 282, 239, 282, 239, 226, 208.

### (Example 2343)

### Compound of Formula No. E-59

¹H-NMR (CDCl₃) δ(ppm) : 6.78 (1H, s, 5-H), 5.34 (2H, brs, NCH₂Ar), 3.75 (2H, d, J = 7.6 Hz, NCH₂CH), 2.75 (1H, hep, J = 7.0 Hz, 3-CH), 1.92 (3H, s, COCH₃).
MASS (EI) m/z : 338(M⁺), 296, 253, 222, 180, 165, 124.

### (Example 2344)

### Compound of Formula No. E-60

¹H-NMR (CDCl₃) δ(ppm) : 6.90 (1H, s, 3H), 5.35 (1H, d, J = 14.0 Hz, NCH₂Ar), 4.14 (1H, d, J = 14.0 Hz, NCH₂Ar), 3.83 (2H, d, J = 7.6 Hz, NCH₂CH), 3.01 (1H, hep, J = 7.0 Hz, 5-CH), 1.92 (3H, s, COCH₃).
MASS (EI) m/z : 338(M⁺), 295, 253, 240, 222, 180, 166, 116, 70, 57.

### (Example 2345)

### Compound of Formula No. E-61

¹H-NMR (CDCl₃) δ(ppm) : 6.90 (1H, s, 5-H), 5.20 (2H, brs, NCH₂Ar), 4.42 (2H, s, CH₂O), 3.76 (2H, d, J = 7.6 Hz, NCH₂CH), 2.43 (2H, q, J = 7.6 Hz, 3-CH₂).
MASS (EI) m/z : 416(M⁺), 397, 323, 309, 300, 281, 267.

### (Example 2346)

### Compound of Formula No. E-62

¹H-NMR (CDCl₃) δ(ppm) : 7.11 (1H, s, 3H), 5.00 (1H, brs, NCH₂Ar), 4.65 (1H, brs, NCH₂Ar), 4.41 (2H, CH₂O), 3.80 (2H, d, J = 7.6 Hz, NCH₂CH), 2.41 (2H, brq, J = 7.6 Hz, 5-CH₂).
MASS (EI) m/z : 416(M⁺), 323, 309, 281, 267, 253, 239, 224, 206.

### (Example 2347)

### Compound of Formula No. E-63

¹H-NMR (CDCl₃) δ(ppm) : 6.83 (1H, s, 5-H), 5.44 (1H, brs, NCH₂Ar), 4.42 (2H, brs, CH₂O), 4.16 (1H, brs, NCH₂Ar), 3.77 (2H, d, J = 7.6 Hz, NCH₂CH), 2.82 (1H, hep, J = 7.0 Hz, 3-CH).
MASS (EI) m/z : 430(M⁺), 416, 337, 314, 295, 281, 165, 116, 107.

### (Example 2348)

### Compound of Formula No. E-64

¹H-NMR (CDCl₃) δ(ppm) : 6.95 (1H, s, 3H), 5.39 (1H, d, J = 14.0 Hz, NCH₂Ar), 4.45 (1H, d, J = 15.1 Hz, CH₂O), 4.18 (1H, d, J = 14.0 Hz, NCH₂Ar), 3.85 (2H, d, J = 7.6 Hz, NCH₂CH), 3.04 (1H, hep, J = 7.0 Hz, 5-CH).
MASS (EI) m/z : 430(M⁺), 387, 337, 323, 314, 295, 166, 116, 107, 77, 57.

### (Example 2349)

### Compound of Formula No. E-65

¹H-NMR (CDCl₃) δ(ppm) : 6.83 (1H, brs, 5-H), 4.68 (2H, brs, NCH₂Ar), 3.73 (2H, d, J = 7.6 Hz, NCH₂CH), 3.72 (3H, brs, OCH₃), 2.38 (2H, brq, J = 7.6 Hz, 3-CH₂).
MASS (EI) m/z : 340(M⁺), 325, 297, 284, 224, 182, 168.

### (Example 2350)

### Compound of Formula No. E-66

¹H-NMR (CDCl₃) δ(ppm) : 7.11 (1H, s, 3H) , 4.77 (2H, s, NCH₂Ar), 3.76 (2H, d, J = 7.6 Hz, NCH₂CH), 3.72, (3H, brs, OCH₃), 2.26 (2H, brq, J = 7.6 Hz, 5-CH₂).
MASS (EI) m/z : 340(M⁺), 325, 311, 297, 284, 239, 224.

### (Example 2351)

### Compound of Formula No. E-67

¹H-NMR (CDCl₃) δ(ppm) : 6.79 (1H, brs, 5-H), 4.68 (2H, s, NCH₂Ar), 3.73 (2H, d, J = 7.6 Hz, NCH₂CH), 3.70 (3H, brs, OCH₃), 2.68 (2H, brhep, J = 7.6 Hz, 3-CH).
MASS (EI) m/z : 354(M⁺), 339, 311, 298, 238, 196, 116.

### (Example 2352)

### Compound of Formula No. E-68

¹H-NMR (CDCl₃) δ(ppm) : 6.94 (1H, s, 3H), 4.67 (2H, brs, NCH₂Ar), 3.79 (2H, d, J = 7.6 Hz, NCH₂CH), 3.69 (3H, brs, OCH₃), 2.95 (2H, hep, J = 7.0 Hz, 5-CH).
MASS (EI) m/z : 354(M⁺), 339, 311, 298, 238, 182, 116.

### (Example 2353)

### Compound of Formula No. E-69

¹H-NMR (CDCl₃) δ(ppm) : 8.37 (1H, d, J = 2.9 Hz, N=CH), 6.98 (1H, s, 5-H), 4.78 (2H, brs, NCH₂Ar), 3.73 (2H, d, J = 7.0 Hz, NCH₂CH), 2.53 (2H, q, J = 7.6 Hz, 3-CH₂).
MASS (EI) m/z : 403(M⁺), 359, 308, 280. 192, 137, 116.

### (Example 2354)

### Compound of Formula No. E-70

¹H-NMR (CDCl₃) δ(ppm) : 8.38 (1H, brs, N=CH), 7.24 (1H, s, 5-H), 4.80 (2H, brs, NCH₂Ar), 3.76 (2H, d, J = 7.6 Hz, NCH₂CH), 2.43 (2H, q, J = 7.6 Hz, 3-CH₂).
MASS (EI) m/z : 403(M⁺), 359, 308, 280. 192, 137, 116.

### (Example 2355)

### Compound of Formula No. E-71

¹H-NMR (CDCl₃) δ(ppm) : 8.37 (1H, brd, J = 2.9 Hz, N=CH), 6.95 (1H, s, 5-H), 4.78 (2H, brs, NCH₂Ar), 3.75 (2H, d, J = 7.6 Hz, NCH₂CH), 2.88 (1H, hep, J = 7.0 Hz, 3-CH).
MASS (EI) m/z : 417(M⁺), 373, 322, 294, 279, 206, 116.

### (Example 2356)

### Compound of Formula No. E-72

¹H-NMR (CDCl₃) δ(ppm) : 8.38 (1H, brs, NCH), 7.09 (1H, s, 3H), 4.90 (2H, brs, NCH₂Ar), 3.80 (2H, d, J = 7.6 Hz, NCH₂CH), 3.06 (1H, hep, J = 7.0 Hz, 5-CH).
MASS (EI) m/z : 417(M⁺), 322, 294, 266, 206, 116, 57.

### (Example 2357)

### Compound of Formula No. E-73

¹H-NMR (CDCl₃) δ(ppm) : 6.97 (1H, s, 5-H), 4.76 (2H, brs, NCH₂Ar), 3.76 (2H, d, J = 7.6 Hz, NCH₂CH), 2.49 (2H, q, J = 7.6 Hz, 3-CH₂).
MASS (EI) m/z : 402(M⁺), 309, 281, 253, 192, 150, 116.

### (Example 2358)

### Compound of Formula No. E-74

¹H-NMR (CDCl₃) δ(ppm) : 4.78 (2H, brs, NCH₂Ar), 3.77 (2H, d, J = 7.6 Hz, NCH₂CH), 2.39 (2H, q, J = 7.6 Hz, 3-CH₂), 0.87 (6H, d, J = 7.0 Hz, CH(CH₃)₂).
MASS (EI) m/z : 402(M⁺), 309, 253, 192, 150, 137, 116.

### (Example 2359)

### Compound of Formula No. E-75

¹H-NMR (CDCl₃) δ(ppm) : 6.92 (1H, s, 5-H), 4.76 (2H, brs, NCH₂Ar), 3.77 (2H, d, J = 7.6 Hz, NCH₂CH), 2.84 (2H, hep, J = 7.0 Hz, 3-CH).
MASS (EI) m/z : 416(M⁺), 323, 281, 267, 206, 164, 116.

### (Example 2360)

### Compound of Formula No. E-76

¹H-NMR (CDCl₃) δ(ppm) : 4.85 (2H, brs, NCH₂Ar), 3.81 (2H, d, J = 7.6 Hz, NCH₂CH), 3.03 (2H, hep, J = 7.0 Hz, 3-CH₂), 0.90 (6H, d, J = 7.0 Hz, CH(CH₃)₂).
MASS (EI) m/z : 416(M⁺), 323, 267, 164, 149, 116, 57.

### (Example 2361)

### Compound of Formula No. F-1

¹H-NMR (CDCl₃) δ(ppm) : 0.95(3H, d, J=6Hz, CHCH₃), 0.97(3H, d, J=5Hz, CHCH₃), 1.99(3H, s, 3-Me), 2.01 (3H, s, 5-Me), 2.28(1H, m, CH), 2.91(2H, t, J=6Hz, COCH₂), 3.19(2H, J=6Hz, CONCH₂), 3.83(2H, m, NCH₂CH), 6.61(1H, d, J=8Hz, CHCHCNO₂), 8.00(1H, dd, J=2Hz, J=8Hz, CHCHNO₂), 8.13(1H, d, J=2Hz, CCHNO₂).
MASS (EI) m/z : 342(M⁺), 327, 299, 286, 253.

### (Example 2362)

### Compound of Formula No. F-3

¹H-NMR (CDCl₃) δ(ppm) : 2.07(3H, s, 3-Me), 2.10(3H, s, 5-Me), 2.90(2H, m, COCH₂), 3.14(2H, m, CONCH₂), 6.89(1H, d, J=8.7Hz, CHCHCCN), 7.34-7.56(8H, m).
MASS (EI) m/z : 342(M⁺), 314, 299, 285, 171, 118, 77.

### (Formulation Example 1)

### Powder

The compound of Example 1 (Compound No. D-229) (0.5 parts by mass), Hymexazol (free acid, 4 parts by mass), Karplex #80-D (manufactured by SIONOGI & CO., LTD., 5 parts by mass) and Keiwa Clay Huhi (manufactured by Keiwarozai Co., Ltd., 90.5 parts by mass) are mixed, and ground in a Sample Mill KII-1 type (manufactured by Fuji Paudal Co., Ltd.) to obtain a powder.

### (Formulation Example 2)

### Emulsion

The compound of Example 2 (Compound No. C-83) (10 parts by mass) is dissolved in a mixed solution of xylene (manufactured by Wako Pure Chemical Industries, Ltd. 40 parts by mass) and DMSO (manufactured by Wako Pure Chemical Industries, Ltd. 35 parts by mass), and Parakol KPS (manufactured by Nippon Nyukazai Co., Ltd. 25 parts by mass) is added thereto to mix them, to obtain an emulsion.

### (Formulation Example 3)

### Wettable Powder

The compound of Example 747 (Compound No. C-85) (10 parts by mass), Karplex #80-D (manufactured by SIONOGI & CO., LTD., 10 parts by mass), Gosenol GL05 (manufactured by THE NIPPON SYNTHETIC CHEMICAL INDUSTRY CO., LTD., 2 parts by mass), Newcoal 291 PG (manufactured by Nippon Nyukazai Co., Ltd., 0.5 part by mass), Neogen Powder (manufactured by Dai-ichi Kogyo Seiyaku Co., Ltd., 5 parts by mass), Radiolite #200 (manufactured by SHOWA CHEMICAL INDUSTRY CO., LTD., 10 parts by mass) and H fine powder (manufactured by Keiwarozai Co.,Ltd., 62.5 parts by mass) are sufficiently mixed, and ground in a Sample Mill KII-1 type (Fuji Paudal Co., Ltd.) to obtain a wettable powder.

### (Formulation Example 4)

### Granules

The compound of Example 124 (Compound No. A-66) (2 parts by mass), sodium tripolyphosphate (manufactured by Mitsui Chemicals, Inc., 2 parts by mass), Amycol No.1 (manufactured by NIPPON STARCH CHEMICAL CO., LTD., 1.5 parts by mass), bentonite (manufactured by Houjun Mining Co., Ltd., 25 parts by mass) and calcium carbonate Calhin 600 (manufactured by Adachisekkai Co., Ltd., 69.5 parts by mass) are mixed, and extruded using a Dome Gran (manufactured by Fuji Paudal Co., Ltd., screen 0.9 mmφ) to granulate the mixture. The resulting granules are dried with a shelf type dryer (manufactured by Tabai Co., Ltd., PERFECT OVEN PS-222 type, drying temperature: 60°C) and sieved to 600-1180µm to obtain granules.

### (Test Example 1)

### Antifungal activity against potato late blight fungus (Phytophthora infestans)

10µl of a solution of 10,000ppm of a compound of the present invention in dimethyl sulfoxide was added to 10ml of corn meal agar medium to prepare an agar medium in which the concentration of the compound of the present invention was 10ppm. On this was seeded hypha of Phytophthora infestans, which was cultured at 28°C for 2 days. The length of hypha was measured, and compared with a control to calculate the hyphal growth inhibiting rate.

As a result, the following compounds showed a hyphal growth inhibiting rate of 50% or higher: Example 2 (Compound No. C-83), Example 3 (Compound No. B-89), Example 6 (Compound No. C-484), Example 8 (Compound No. D-338), Example 19 (Compound No. D-77), Example 23 (Compound No. D-117), Example 29 (Compound No. D-101), Example 36 (Compound No. D-63), Example 39 (Compound No. C-317), Example 41 (Compound No. C-700), Example 49 (Compound No. C-97), Example 50 (Compound No. C-282), Example 51 (Compound No. C-283), Example 54 (Compound No. F-2), Example 57 (Compound No. C-800), Example 58 (Compound No. C-801), Example 63 (Compound No. A-5), Example 75 (Compound No. A-17), Example 77 (Compound No. A-19), Example 90 (Compound No. A-32), Example 91 (Compound No. A-33), Example 115 (Compound No. A-57), Example 116 (Compound No. A-58), Example 117 (Compound No. A-59), Example 264 (Compound No. B-18), Example 266 (Compound No. B-20), Example 271 (Compound No. B-25), Example 278 (Compound No. B-32), Example 308 (Compound No. B-62), Example 310 (Compound No. B-64), Example 315 (Compound No. B-69), Example 317 (Compound No. B-71), Example 318 (Compound No. B-72), Example 327 (Compound No. B-81), Example 329 (Compound No. B-83), Example 330 (Compound No. B-84), Example 331 (Compound No. B-85), Example 332 (Compound No. B-86), Example 333 (Compound No. B-87), Example 334 (Compound No. B-88), Example 338 (Compound No. B-94), Example 340 (Compound No. B-96), Example 341 (Compound No. B-97), Example 346 (Compound No. B-102), Example 347 (Compound No. B-103), Example 348 (Compound No. B-104), Example 349 (Compound No. B-105), Example 350 (Compound No. B-106), Example 351 (Compound No. B-107), Example 352 (Compound No. B-108), Example 353 (Compound No. B-109), Example 354 (Compound No. B-110), Example 355 (Compound No. B-111), Example 356 (Compound No. B-112), Example 357 (Compound No. B-113), Example 358 (Compound No. B-114), Example 359 (Compound No. B-115), Example 364 (Compound No. B-120), Example 371 (Compound No. B-127), Example 372 (Compound No. B-128), Example 412 (Compound No. B-168), Example 415 (Compound No. B-171), Example 425 (Compound No. B-181), Example 426 (Compound No. B-182), Example 427 (Compound No. B-183), Example 428 (Compound No. B-184), Example 429 (Compound No. B-185), Example 431 (Compound No. B-187), Example 432 (Compound No. B-188), Example 434 (Compound No. B-190), Example 435 (Compound No. B-191), Example 436 (Compound No. B-192), Example 437 (Compound No. B-193), Example 438 (Compound No. B-194), Example 439 (Compound No. B-195), Example 440 (Compound No. B-196), Example 441 (Compound No. B-197), Example 443 (Compound No. B-199), Example 444 (Compound No. B-200), Example 445 (Compound No. B-201), Example 446 (Compound No. B-202), Example 447 (Compound No. B-203), Example 452 (Compound No. B-208), Example 456 (Compound No. B-212), Example 459 (Compound No. B-215), Example 464 (Compound No. B-220), Example 471 (Compound No. B-227), Example 473 (Compound No. B-229), Example 474 (Compound No. B-230), Example 475 (Compound No. B-231), Example 476 (Compound No. B-232), Example 477 (Compound No. B-233), Example 478 (Compound No. B-234), Example 479 (Compound No. B-235), Example 480 (Compound No. B-236), Example 481 (Compound No. B-237), Example 482 (Compound No. B-238), Example 483 (Compound No. B-239), Example 484 (Compound No. B-240), Example 485 (Compound No. B-241), Example 486 (Compound No. B-242), Example 487 (Compound No. B-243), Example 488 (Compound No. B-244), Example 489 (Compound No. B-245), Example 490 (Compound No. B-246), Example 492 (Compound No. B-248), Example 493 (Compound No. B-249), Example 494 (Compound No. B-250), Example 495 (Compound No. B-251), Example 496 (Compound No. B-252), Example 497 (Compound No. B-253), Example 498 (Compound No. B-254), Example 499 (Compound No. B-255), Example 500 (Compound No. B-256), Example 501 (Compound No. B-257), Example 502 (Compound No. B-258), Example 503 (Compound No. B-259), Example 504 (Compound No. B-260), Example 505 (Compound No. B-261), Example 506 (Compound No. B-262), Example 507 (Compound No. B-263), Example 508 (Compound No. B-264), Example 509 (Compound No. B-265), Example 510 (Compound No. B-266), Example 511 (Compound No. B-267), Example 512 (Compound No. B-268), Example 513 (Compound No. B-269), Example 514 (Compound No. B-270), Example 515 (Compound No. B-271), Example 516 (Compound No. B-272), Example 517 (Compound No. B-273), Example 518 (Compound No. B-274), Example 519 (Compound No. B-275), Example 520 (Compound No. B-276), Example 521 (Compound No. B-277), Example 522 (Compound No. B-278), Example 523 (Compound No. B-279), Example 524 (Compound No. B-280), Example 525 (Compound No. B-281), Example 526 (Compound No. B-282), Example 527 (Compound No. B-283), Example 528 (Compound No. B-284), Example 529 (Compound No. B-285), Example 530 (Compound No. B-286), Example 531 (Compound No. B-287), Example 532 (Compound No. B-288), Example 533 (Compound No. B-289), Example 534 (Compound No. B-290), Example 535 (Compound No. B-291), Example 536 (Compound No. B-292), Example 537 (Compound No. B-293), Example 538 (Compound No. B-294), Example 539 (Compound No. B-295), Example 540 (Compound No. B-296), Example 541 (Compound No. B-297), Example 542 (Compound No. B-298), Example 543 (Compound No. B-299), Example 544 (Compound No. B-300), Example 545 (Compound No. B-301), Example 546 (Compound No. B-302), Example 547 (Compound No. B-303), Example 550 (Compound No. B-306), Example 551 (Compound No. B-307), Example 553 (Compound No. B-309), Example 554 (Compound No. B-310), Example 555 (Compound No. B-311), Example 610 (Compound No. B-368), Example 690 (Compound No. C-27), Example 724 (Compound No. C-61), Example 725 (Compound No. C-62), Example 726 (Compound No. C-63), Example 727 (Compound No. C-64), Example 728 (Compound No. C-65), Example 731 (Compound No. C-68), Example 732 (Compound No. C-69), Example 733 (Compound No. C-70), Example 734 (Compound No. C-71), Example 735 (Compound No. C-72), Example 742 (Compound No. C-79), Example 744 (Compound No. C-81), Example 745 (Compound No. C-82), Example 746 (Compound No. C-84), Example 747 (Compound No. C-85), Example 748 (Compound No. C-86), Example 749 (Compound No. C-87), Example 750 (Compound No. C-88), Example 751 (Compound No. C-89), Example 752 (Compound No. C-90), Example 753 (Compound No. C-91), Example 754 (Compound No. C-92), Example 755 (Compound No. C-93), Example 756 (Compound No. C-94), Example 757 (Compound No. C-95), Example 758 (Compound No. C-96), Example 760 (Compound No. C-99), Example 761 (Compound No. C-100), Example 762 (Compound No. C-101), Example 763 (Compound No. C-102), Example 764 (Compound No. C-103), Example 765 (Compound No. C-104), Example 766 (Compound No. C-105), Example 767 (Compound No. C-106), Example 768 (Compound No. C-107), Example 772 (Compound No. C-111), Example 778 (Compound No. C-117), Example 781 (Compound No. C-120), Example 783 (Compound No. C-122), Example 784 (Compound No. C-123), Example 785 (Compound No. C-124), Example 786 (Compound No. C-125), Example 787 (Compound No. C-126), Example 788 (Compound No. C-127), Example 789 (Compound No. C-128), Example 790 (Compound No. C-129), Example 791 (Compound No. C-130), Example 792 (Compound No. C-131), Example 793 (Compound No.C-132), Example 794 (Compound No. C-133), Example 795 (Compound No. C-134), Example 797 (Compound No. C-136), Example 798 (Compound No. C-137), Example 799 (Compound No. C-138), Example 800 (Compound No. C-139), Example 801 (Compound No. C-140), Example 806 (Compound No. C-145), Example 809 (Compound No. C-148), Example 810 (Compound No. C-149), Example 812 (Compound No. C-151), Example 814 (Compound No. C-153), Example 815 (Compound No. C-154), Example 818 (Compound No. C-157), Example 821 (Compound No. C-160), Example 822 (Compound No. C-161), Example 834 (Compound No. C-173), Example 840 (Compound No. C-179), Example 861 (Compound No. C-201), Example 862 (Compound No. C-202), Example 863 (Compound No. C-203), Example 864 (Compound No. C-204), Example 865 (Compound No. C-205), Example 880 (Compound No. C-220), Example 881 (Compound No. C-221), Example 882 (Compound No. C-222), Example 883 (Compound No. C-223), Example 891 (Compound No. C-232), Example 898 (Compound No. C-240), Example 900 (Compound No. C-242), Example 902 (Compound No. C-244), Example 906 (Compound No. C-249), Example 908 (Compound No. C-251), Example 909 (Compound No. C-252), Example 910 (Compound No. C-253), Example 912 (Compound No. C-255), Example 917 (Compound No. C-261), Example 918 (Compound No. C-262), Example 920 (Compound No. C-264), Example 921 (Compound No. C-265), Example 922 (Compound No. C-266), Example 923 (Compound No. C-267), Example 924 (Compound No. C-268), Example 925 (Compound No. C-269), Example 927 (Compound No. C-271), Example 947 (Compound No. C-295), Example 953 (Compound No. C-301), Example 956 (Compound No. C-304), Example 971 (Compound No. C-320), Example 993 (Compound No. C-343), Example 996 (Compound No. C-347), Example 999 (Compound No. C-350), Example 1005 (Compound No. C-356), Example 1053 (Compound No. C-404), Example 1054 (Compound No. C-405), Example 1059 (Compound No. C-410), Example 1065 (Compound No. C-416), Example 1070 (Compound No. C-421), Example 1078 (Compound No. C-429), Example 1079 (Compound No. C-430), Example 1080 (Compound No. C-431), Example 1081 (Compound No. C-432), Example 1082 (Compound No. C-433), Example 1084 (Compound No. C-435), Example 1085 (Compound No. C-436), Example 1087 (Compound No. C-438), Example 1088 (Compound No. C-439), Example 1089 (Compound No. C-440), Example 1090 (Compound No. C-441), Example 1091 (Compound No. C-442), Example 1092 (Compound No. C-443), Example 1093 (Compound No. C-444), Example 1096 (Compound No. C-447), Example 1097 (Compound No. C-448), Example 1098 (Compound No. C-449), Example 1099 (Compound No. C-450), Example 1100 (Compound No. C-451), Example 1102 (Compound No. C-453), Example 1106 (Compound No. C-457), Example 1109 (Compound No. C-460), Example 1112 (Compound No. C-463), Example 1115 (Compound No. C-466), Example 1118 (Compound No. C-469), Example 1121 (Compound No. C-472), Example 1123 (Compound No.C-474), Example 1125 (Compound No. C-476), Example 1127 (Compound No. C-478), Example 1128 (Compound No. C-479), Example 1129 (Compound No. C-480), Example 1130 (Compound No. C-481), Example 1131 (Compound No. C-482), Example 1132 (Compound No. C-483), Example 1133 (Compound No. C-485), Example 1134 (Compound No. C-486), Example 1136 (Compound No. C-488), Example 1137 (Compound No. C-489), Example 1138 (Compound No. C-490), Example 1139 (Compound No. C-491), Example 1140 (Compound No. C-492), Example 1141 (Compound No. C-493), Example 1142 (Compound No. C-494), Example 1145 (Compound No. C-497), Example 1146 (Compound No. C-498), Example 1147 (Compound No. C-499), Example 1148 (Compound No. C-500), Example 1149 (Compound No. C-501), Example 1151 (Compound No. C-503), Example 1153 (Compound No. C-505), Example 1154 (Compound No. C-506), Example 1155 (Compound No. C-507), Example 1156 (Compound No. C-508), Example 1157 (Compound No. C-509), Example 1158 (Compound No. C-510), Example 1159 (Compound No. C-511), Example 1160 (Compound No. C-512), Example 1161 (Compound No. C-513), Example 1162 (Compound No. C-514), Example 1163 (Compound No. C-515), Example 1164 (Compound No. C-516), Example 1165 (Compound No. C-517), Example 1166 (Compound No. C-518), Example 1167 (Compound No. C-519), Example 1168 (Compound No. C-520), Example 1169 (Compound No. C-521), Example 1170 (Compound No. C-522), Example 1172 (Compound No. C-524), Example 1173 (Compound No. C-525), Example 1174 (Compound No. C-526), Example 1175 (Compound No. C-527), Example 1176 (Compound No. C-528), Example 1177 (Compound No. C-529), Example 1178 (Compound No. C-530), Example 1179 (Compound No. C-531), Example 1180 (Compound No. C-532), Example 1181 (Compound No. C-533), Example 1182 (Compound No. C-534), Example 1183 (Compound No. C-535), Example 1184 (Compound No. C-536), Example 1185 (Compound No. C-537), Example 1186 (Compound No. C-538), Example 1187 (Compound No. C-539), Example 1188 (Compound No. C-540), Example 1189 (Compound No. C-541), Example 1190 (Compound No. C-542), Example 1191 (Compound No. C-543), Example 1192 (Compound No. C-544), Example 1193 (Compound No.C-545), Example 1194 (Compound No. C-546), Example 1195 (Compound No. C-547), Example 1196 (Compound No. C-548), Example 1197 (Compound No. C-549), Example 1198 (Compound No. C-550), Example 1199 (Compound No. C-551), Example 1200 (Compound No. C-552), Example 1202 (Compound No. C-554), Example 1203 (Compound No. C-561), Example 1205 (Compound No. C-564), Example 1206 (Compound No. C-565), Example 1207 (Compound No. C-566), Example 1208 (Compound No. C-567), Example 1209 (Compound No. C-568), Example 1210 (Compound No. C-569), Example 1211 (Compound No. C-570), Example 1215 (Compound No. C-574), Example 1219 (Compound No. C-578), Example 1220 (Compound No. C-579), Example 1224 (Compound No. C-583), Example 1227 (Compound No. C-586), Example 1228 (Compound No. C-587), Example 1229 (Compound No. C-588), Example 1230 (Compound No. C-589), Example 1231 (Compound No. C-590), Example 1232 (Compound No. C-591), Example 1233 (Compound No. C-592), Example 1234 (Compound No. C-593), Example 1235 (Compound No. C-594), Example 1236 (Compound No. C-595), Example 1237 (Compound No. C-596), Example 1238 (Compound No. C-597), Example 1239 (Compound No. C-598), Example 1240 (Compound No. C-599), Example 1241 (Compound No. C-600), Example 1242 (Compound No. C-601), Example 1246 (Compound No. C-605), Example 1250 (Compound No. C-609), Example 1252 (Compound No. C-611), Example 1253 (Compound No. C-612), Example 1254 (Compound No. C-613), Example 1263 (Compound No. C-622), Example 1264 (Compound No. C-623), Example 1265 (Compound No. C-624), Example 1266 (Compound No. C-625), Example 1267 (Compound No. C-626), Example 1268 (Compound No. C-627), Example 1269 (Compound No. C-628), Example 1271 (Compound No. C-630), Example 1273 (Compound No. C-632), Example 1274 (Compound No. C-633), Example 1276 (Compound No. C-635), Example 1277 (Compound No. C-636), Example 1278 (Compound No. C-637), Example 1279 (Compound No. C-638), Example 1280 (Compound No. C-639), Example 1283 (Compound No. C-642), Example 1289 (Compound No. C-649), Example 1290 (Compound No. C-650), Example 1293 (Compound No. C-653), Example 1294 (Compound No. C-654), Example 1306 (Compound No. C-666), Example 1307 (Compound No. C-667), Example 1308 (Compound No. C-668), Example 1309 (Compound No. C-669), Example 1310 (Compound No. C-670), Example 1311 (Compound No. C-671), Example 1312 (Compound No. C-672), Example 1313 (Compound No. C-673), Example 1314 (Compound No. C-674), Example 1315 (Compound No. C-675), Example 1316 (Compound No. C-676), Example 1317 (Compound No. C-677), Example 1318 (Compound No. C-678), Example 1320 (Compound No. C-680), Example 1322 (Compound No. C-682), Example 1340 (Compound No. C-701), Example 1341 (Compound No. C-702), Example 1342 (Compound No. C-703), Example 1343 (Compound No. C-704), Example 1345 (Compound No. C-706), Example 1347 (Compound No. C-708), Example 1348 (Compound No. C-709), Example 1349 (Compound No. C-710), Example 1350 (Compound No. C-711), Example 1351 (Compound No. C-712), Example 1352 (Compound No. C-713), Example 1378 (Compound No. C-739), Example 1379 (Compound No. C-740), Example 1413 (Compound No. C-774), Example 1414 (Compound No. C-775), Example 1415 (Compound No. C-776), Example 1416 (Compound No. C-777), Example 1419 (Compound No. C-780), Example 1423 (Compound No. C-785), Example 1425 (Compound No. C-787), Example 1427 (Compound No. C-789), Example 1430 (Compound No. C-792), Example 1450 (Compound No. C-814), Example 1479 (Compound No. C-843), Example 1508 (Compound No. C-872), Example 1561 (Compound No. C-925), Example 1566 (Compound No. C-930), Example 1567 (Compound No. C-931), Example 1569 (Compound No. C-933), Example 1572 (Compound No. C-936), Example 1578 (Compound No. C-942), Example 1579 (Compound No. C-943), Example 1581 (Compound No. C-945), Example 1583 (Compound No. C-947), Example 1585 (Compound No. C-949), Example 1586 (Compound No. C-950), Example 1587 (Compound No. C-951), Example 1589 (Compound No. C-953), Example 1597 (Compound No. C-961), Example 1598 (Compound No. C-962), Example 1599 (Compound No. C-963), Example 1600 (Compound No. C-964), Example 1601 (Compound No. C-965), Example 1602 (Compound No. C-966), Example 1613 (Compound No. C-977), Example 1653 (Compound No. C-1017), Example 1654 (Compound No. C-1018), Example 1664 (Compound No. C-1028), Example 1665 (Compound No. C-1029), Example 1666 (Compound No. C-1030), Example 1667 (Compound No. C-1031), Example 1668 (Compound No. C-1032), Example 1669 (Compound No. C-1033), Example 1674 (Compound No. C-1038), Example 1675 (Compound No. C-1039), Example 1676 (Compound No. C-1040), Example 1677 (Compound No. C-1041), Example 1679 (Compound No. C-1043), Example 1681 (Compound No. C-1045), Example 1689 (Compound No. C-1053), Example 1690 (Compound No. C-1054), Example 1691 (Compound No. C-1055), Example 1696 (Compound No. C-1060), Example 1708 (Compound No. C-1072), Example 1754 (Compound No. C-1118), Example 1755 (Compound No. C-1119), Example 1776 (Compound No. C-1140), Example 1778 (Compound No. C-1142), Example 1783 (Compound No. C-1147), Example 1798 (Compound No. C-1162), Example 1885 (Compound No. D-14), Example 1890 (Compound No. D-19), Example 1891 (Compound No. D-20), Example 1892 (Compound No. D-21), Example 1895 (Compound No. D-24), Example 1896 (Compound No. D-25), Example 1900 (Compound No. D-29), Example 1907 (Compound No. D-36), Example 1908 (Compound No. D-37), Example 1914 (Compound No. D-43), Example 1917 (Compound No. D-46), Example 1919 (Compound No. D-48), Example 1925 (Compound No. D-54), Example 1934 (Compound No. D-67), Example 1943 (Compound No. D-76), Example 1945 (Compound No. D-79), Example 1948 (Compound No. D-82), Example 1949 (Compound No. D-83), Example 1950 (Compound No. D-84), Example 1951 (Compound No. D-85), Example 1952 (Compound No. D-86), Example 1953 (Compound No. D-87), Example 1956 (Compound No. D-90), Example 1957 (Compound No. D-91), Example 1960 (Compound No. D-94), Example 1961 (Compound No. D-95), Example 1962 (Compound No. D-96), Example 1963 (Compound No. D-97), Example 1965 (Compound No. D-99), Example 1991 (Compound No. D-127), Example 2027 (Compound No. D-171), Example 2028 (Compound No. D-172), Example 2029 (Compound No. D-173), Example 2033 (Compound No. D-177), Example 2034 (Compound No. D-178), Example 2043 (Compound No. D-187), Example 2058 (Compound No. D-203), Example 2059 (Compound No. D-204), Example 2060 (Compound No. D-205), Example 2062 (Compound No. D-207), Example 2069 (Compound No. D-215), Example 2072 (Compound No. D-218), Example 2089 (Compound No. D-236), Example 2090 (Compound No. D-237), Example 2091 (Compound No. D-238), Example 2092 (Compound No. D-239), Example 2095 (Compound No. D-242), Example 2096 (Compound No. D-243), Example 2097 (Compound No. D-244), Example 2098 (Compound No. D-245), Example 2099 (Compound No. D-246), Example 2105 (Compound No. D-252), Example 2113 (Compound No. D-260), Example 2121 (Compound No. D-268), Example 2124 (Compound No. D-271), Example 2125 (Compound No. D-272), Example 2152 (Compound No. D-302), Example 2157 (Compound No. D-307), Example 2159 (Compound No. D-309), Example 2165 (Compound No. D-315), Example 2169 (Compound No. D-319), Example 2171 (Compound No. D-321), Example 2184 (Compound No. D-334), Example 2185 (Compound No. D-335), Example 2186 (Compound No. D-336), Example 2187 (Compound No. D-337), Example 2188 (Compound No. D-339), Example 2189 (Compound No. D-340), Example 2190 (Compound No. D-341), Example 2191 (Compound No. D-342), Example 2192 (Compound No. D-343), Example 2193 (Compound No. D-344), Example 2202 (Compound No. D-354), Example 2203 (Compound No. D-355), Example 2206 (Compound No. D-358), Example 2245 (Compound No. D-397), Example 2296 (Compound No. E-6), Example 2325 (Compound No. E-36) and Example 2327 (Compound No. E-38).

### (Test Example 2)

### Antifungal activity against Pythium fungus (Pythium graminicola)

10µl of a solution of 10,000ppm of a compound of the present invention in dimethyl sulfoxide was added to 10ml of corn meal agar medium to prepare an agar medium in which the concentration of the compound of the present invention was 10ppm. On this was seeded hypha of Pythium graminicola, which was cultured at 28°C for 2 days. The length of hypha was measured, and compared with a control to calculate the hyphal growth inhibiting rate.

As a result, the following compounds showed a hyphal growth inhibiting rate of 50% or higher:
Example 2 (Compound No. C-83), Example 3 (Compound No. B-89), Example 4 (Compound No. B-93), Example 5 (Compound No. C-645), Example 6 (Compound No. C-484), Example 7 (Compound No. D-349), Example 8 (Compound No. D-338), Example 19 (Compound No. D-77), Example 23 (Compound No. D-117), Example 25 (Compound No. D-140), Example 28 (Compound No. D-146), Example 29 (Compound No. D-101), Example 30 (Compound No. D-154), Example 36 (Compound No. D-63), Example 38 (Compound No. D-64), Example 39 (Compound No. C-317), Example 41 (Compound No. C-700), Example 42 (Compound No. C-226), Example 43 (Compound No. C-234), Example 44 (Compound No. C-256), Example 46 (Compound No. C-278), Example 49 (Compound No. C-97), Example 50 (Compound No. C-282), Example 51 (Compound No. C-283), Example 54 (Compound No. F-2), Example 57 (Compound No. C-800), Example 58 (Compound No. C-801), Example 63 (Compound No. A-5), Example 70 (Compound No. A-12), Example 75 (Compound No. A-17), Example 76 (Compound No. A-18), Example 77 (Compound No. A-19), Example 79 (Compound No. A-21), Example 81 (Compound No. A-23), Example 83 (Compound No. A-25), Example 85 (Compound No. A-27), Example 90 (Compound No. A-32), Example 91 (Compound No. A-33), Example 115 (Compound No. A-57), Example 116 (Compound No. A-58), Example 117 (Compound No. A-59), Example 118 (Compound No. A-60), Example 143 (Compound No. A-87), Example 146 (Compound No. A-90), Example 264 (Compound No. B-18), Example 266 (Compound No. B-20), Example 271 (Compound No. B-25), Example 273 (Compound No. B-27), Example 278 (Compound No. B-32), Example 307 (Compound No. B-61), Example 308 (Compound No. B-62), Example 309 (Compound No. B-63), Example 310 (Compound No. B-64), Example 311 (Compound No. B-65), Example 317 (Compound No. B-71), Example 318 (Compound No. B-72), Example 325 (Compound No. B-79), Example 327 (Compound No. B-81), Example 329 (Compound No. B-83), Example 331 (Compound No. B-85), Example 332 (Compound No. B-86), Example 333 (Compound No. B-87), Example 334 (Compound No. B-88), Example 335 (Compound No. B-90), Example 336 (Compound No. B-91), Example 338 (Compound No. B-94), Example 339 (Compound No. B-95), Example 340 (Compound No. B-96), Example 346 (Compound No. B-102), Example 347 (Compound No. B-103), Example 348 (Compound No. B-104), Example 349 (Compound No. B-105), Example 350 (Compound No. B-106), Example 351 (Compound No. B-107), Example 352 (Compound No. B-108), Example 353 (Compound No. B-109), Example 354 (Compound No. B-110), Example 355 (Compound No. B-111), Example 356 (Compound No. B-112), Example 357 (Compound No. B-113), Example 358 (Compound No. B-114), Example 359 (Compound No. B-115), Example 371 (Compound No. B-127), Example 412 (Compound No. B-168), Example 415 (Compound No. B-171), Example 425 (Compound No. B-181), Example 426 (Compound No. B-182), Example 427 (Compound No. B-183), Example 428 (Compound No. B-184), Example 429 (Compound No. B-185), Example 430 (Compound No. B-186), Example 431 (Compound No. B-187), Example 432 (Compound No. B-188), Example 433 (Compound No. B-189), Example 434 (Compound No. B-190), Example 435 (Compound No. B-191), Example 436 (Compound No. B-192), Example 437 (Compound No. B-193), Example 438 (Compound No. B-194), Example 439 (Compound No. B-195), Example 440 (Compound No. B-196), Example 441 (Compound No. B-197), Example 443 (Compound No. B-199), Example 444 (Compound No. B-200), Example 445 (Compound No. B-201), Example 446 (Compound No. B-202), Example 447 (Compound No. B-203), Example 452 (Compound No. B-208), Example 456 (Compound No. B-212), Example 459 (Compound No. B-215), Example 464 (Compound No. B-220), Example 471 (Compound No. B-227), Example 472 (Compound No. B-228), Example 474 (Compound No. B-230), Example 475 (Compound No. B-231), Example 476 (Compound No. B-232), Example 477 (Compound No. B-233), Example 478 (Compound No. B-234), Example 479 (Compound No. B-235), Example 480 (Compound No. B-236), Example 481 (Compound No. B-237), Example 482 (Compound No. B-238), Example 483 (Compound No. B-239), Example 484 (Compound No. B-240), Example 485 (Compound No. B-241), Example 486 (Compound No. B-242), Example 487 (Compound No. B-243), Example 488 (Compound No. B-244), Example 489 (Compound No. B-245), Example 490 (Compound No. B-246), Example 491 (Compound No. B-247), Example 492 (Compound No. B-248), Example 493 (Compound No. B-249), Example 494 (Compound No. B-250), Example 495 (Compound No. B-251), Example 496 (Compound No. B-252), Example 498 (Compound No. B-254), Example 499 (Compound No. B-255), Example 500 (Compound No. B-256), Example 501 (Compound No. B-257), Example 502 (Compound No. B-258), Example 503 (Compound No. B-259), Example 504 (Compound No. B-260), Example 505 (Compound No. B-261), Example 506 (Compound No. B-262), Example 507 (Compound No. B-263), Example 508 (Compound No. B-264), Example 509 (Compound No. B-265), Example 510 (Compound No. B-266), Example 511 (Compound No. B-267), Example 512 (Compound No. B-268), Example 513 (Compound No. B-269), Example 514 (Compound No. B-270), Example 515 (Compound No. B-271), Example 516 (Compound No. B-272), Example 517 (Compound No. B-273), Example 518 (Compound No. B-274), Example 519 (Compound No. B-275), Example 520 (Compound No. B-276), Example 521 (Compound No. B-277), Example 522 (Compound No. B-278), Example 523 (Compound No. B-279), Example 524 (Compound No. B-280), Example 525 (Compound No. B-281), Example 526 (Compound No. B-282), Example 527 (Compound No. B-283), Example 528 (Compound No. B-284), Example 529 (Compound No. B-285), Example 530 (Compound No. B-286), Example 531 (Compound No. B-287), Example 532 (Compound No. B-288), Example 533 (Compound No. B-289), Example 534 (Compound No. B-290), Example 535 (Compound No. B-291), Example 536 (Compound No. B-292), Example 537 (Compound No. B-293), Example 538 (Compound No. B-294), Example 539 (Compound No. B-295), Example 540 (Compound No. B-296), Example 541 (Compound No. B-297), Example 542 (Compound No. B-298), Example 543 (Compound No. B-299), Example 544 (Compound No. B-300), Example 545 (Compound No. B-301), Example 546 (Compound No. B-302), Example 547 (Compound No. B-303), Example 550 (Compound No. B-306), Example 551 (Compound No. B-307), Example 553 (Compound No. B-309), Example 554 (Compound No. B-310), Example 555 (Compound No. B-311), Example 610 (Compound No. B-368), Example 678 (Compound No. C-15), Example 681 (Compound No. C-18), Example 682 (Compound No. C-19), Example 683 (Compound No. C-20), Example 684 (Compound No. C-21), Example 691 (Compound No. C-28), Example 718 (Compound No. C-55), Example 723 (Compound No. C-60), Example 724 (Compound No. C-61), Example 725 (Compound No. C-62), Example 726 (Compound No. C-63), Example 727 (Compound No. C-64), Example 728 (Compound No. C-65), Example 729 (Compound No. C-66), Example 731 (Compound No. C-68), Example 733 (Compound No. C-70), Example 734 (Compound No. C-71), Example 735 (Compound No. C-72), Example 742 (Compound No. C-79), Example 744 (Compound No. C-81), Example 745 (Compound No. C-82), Example 746 (Compound No. C-84), Example 747 (Compound No. C-85), Example 748 (Compound No. C-86), Example 749 (Compound No. C-87), Example 750 (Compound No. C-88), Example 751 (Compound No. C-89), Example 752 (Compound No. C-90), Example 753 (Compound No. C-91), Example 754 (Compound No. C-92), Example 755 (Compound No. C-93), Example 756 (Compound No. C-94), Example 757 (Compound No. C-95), Example 758 (Compound No. C-96), Example 760 (Compound No. C-99), Example 761 (Compound No. C-100), Example 762 (Compound No. C-101), Example 763 (Compound No. C-102), Example 764 (Compound No. C-103), Example 765 (Compound No. C-104), Example 766 (Compound No. C-105), Example 767 (Compound No. C-106), Example 768 (Compound No. C-107), Example 772 (Compound No. C-111), Example 777 (Compound No. C-116), Example 778 (Compound No. C-117), Example 781 (Compound No. C-120), Example 783 (Compound No. C-122), Example 784 (Compound No. C-123), Example 785 (Compound No. C-124), Example 786 (Compound No. C-125), Example 787 (Compound No. C-126), Example 788 (Compound No. C-127), Example 789 (Compound No. C-128), Example 790 (Compound No. C-129), Example 791 (Compound No. C-130), Example 792 (Compound No. C-131), Example 793 (Compound No.C-132), Example 794 (Compound No. C-133), Example 795 (Compound No. C-134), Example 798 (Compound No. C-137), Example 799 (Compound No. C-138), Example 800 (Compound No. C-139), Example 801 (Compound No. C-140), Example 803 (Compound No. C-142), Example 805 (Compound No. C-144), Example 806 (Compound No. C-145), Example 807 (Compound No. C-146), Example 808 (Compound No. C-147), Example 809 (Compound No. C-148), Example 810 (Compound No. C-149), Example 812 (Compound No. C-151), Example 813 (Compound No. C-152), Example 814 (Compound No. C-153), Example 815 (Compound No. C-154), Example 816 (Compound No. C-155), Example 818 (Compound No. C-157), Example 820 (Compound No. C-159), Example 822 (Compound No. C-161), Example 830 (Compound No. C-169), Example 833 (Compound No. C-172), Example 834 (Compound No. C-173), Example 837 (Compound No. C-176), Example 838 (Compound No. C-177), Example 839 (Compound No. C-178), Example 841 (Compound No. C-180), Example 842 (Compound No. C-181), Example 843 (Compound No. C-182), Example 845 (Compound No. C-184), Example 846 (Compound No. C-185), Example 847 (Compound No. C-186), Example 851 (Compound No. C-190), Example 853 (Compound No. C-192), Example 855 (Compound No. C-194), Example 860 (Compound No. C-200), Example 861 (Compound No. C-201), Example 862 (Compound No. C-202), Example 863 (Compound No. C-203), Example 864 (Compound No. C-204), Example 865 (Compound No. C-205), Example 866 (Compound No. C-206), Example 867 (Compound No. C-207), Example 868 (Compound No. C-208), Example 869 (Compound No. C-209), Example 870 (Compound No. C-210), Example 871 (Compound No. C-211), Example 872 (Compound No. C-212), Example 873 (Compound No. C-213), Example 876 (Compound No. C-216), Example 877 (Compound No. C-217), Example 878 (Compound No. C-218), Example 879 (Compound No. C-219), Example 880 (Compound No. C-220), Example 881 (Compound No. C-221), Example 882 (Compound No. C-222), Example 883 (Compound No. C-223), Example 884 (Compound No. C-224), Example 886 (Compound No. C-227), Example 887 (Compound No. C-228), Example 889 (Compound No. C-230), Example 890 (Compound No. C-231), Example 891 (Compound No. C-232), Example 892 (Compound No. C-233), Example 895 (Compound No. C-237), Example 896 (Compound No. C-238), Example 897 (Compound No. C-239), Example 898 (Compound No. C-240), Example 899 (Compound No. C-241), Example 900 (Compound No. C-242), Example 901 (Compound No. C-243), Example 902 (Compound No. C-244), Example 903 (Compound No. C-245), Example 905 (Compound No. C-248), Example 906 (Compound No. C-249), Example 907 (Compound No. C-250), Example 908 (Compound No. C-251), Example 909 (Compound No. C-252), Example 910 (Compound No. C-253), Example 912 (Compound No. C-255), Example 915 (Compound No. C-259), Example 916 (Compound No. C-260), Example 918 (Compound No. C-262), Example 919 (Compound No. C-263), Example 920 (Compound No. C-264), Example 921 (Compound No. C-265), Example 922 (Compound No. C-266), Example 923 (Compound No. C-267), Example 924 (Compound No. C-268), Example 925 (Compound No. C-269), Example 926 (Compound No. C-270), Example 927 (Compound No. C-271), Example 928 (Compound No. C-272), Example 931 (Compound No. C-275), Example 932 (Compound No. C-276), Example 933 (Compound No. C-279), Example 934 (Compound No. C-280), Example 935 (Compound No. C-281), Example 937 (Compound No. C-285), Example 939 (Compound No. C-287), Example 942 (Compound No. C-290), Example 947 (Compound No. C-295), Example 950 (Compound No. C-298), Example 951 (Compound No. C-299), Example 953 (Compound No. C-301), Example 956 (Compound No. C-304), Example 958 (Compound No. C-306), Example 961 (Compound No. C-309), Example 964 (Compound No. C-312), Example 965 (Compound No. C-313), Example 966 (Compound No. C-314), Example 967 (Compound No. C-315), Example 968 (Compound No. C-316), Example 970 (Compound No. C-319), Example 971 (Compound No. C-320), Example 972 (Compound No. C-321), Example 991 (Compound No. C-341), Example 992 (Compound No. C-342), Example 993 (Compound No. C-343), Example 996 (Compound No. C-347), Example 997 (Compound No. C-348), Example 998 (Compound No. C-349), Example 999 (Compound No. C-350), Example 1000 (Compound No. C-351), Example 1002 (Compound No. C-353), Example 1003 (Compound No. C-354), Example 1004 (Compound No. C-355), Example 1005 (Compound No. C-356), Example 1012 (Compound No. C-363), Example 1013 (Compound No. C-364), Example 1015 (Compound No. C-366), Example 1017 (Compound No. C-368), Example 1020 (Compound No. C-371), Example 1021 (Compound No. C-372), Example 1022 (Compound No. C-373), Example 1026 (Compound No. C-377), Example 1027 (Compound No. C-378), Example 1053 (Compound No. C-404), Example 1054 (Compound No. C-405), Example 1059 (Compound No. C-410), Example 1065 (Compound No. C-416), Example 1068 (Compound No. C-419), Example 1070 (Compound No. C-421), Example 1078 (Compound No. C-429), Example 1079 (Compound No. C-430), Example 1080 (Compound No. C-431), Example 1081 (Compound No. C-432), Example 1082 (Compound No. C-433), Example 1083 (Compound No. C-434), Example 1084 (Compound No. C-435), Example 1085 (Compound No. C-436), Example 1087 (Compound No. C-438), Example 1088 (Compound No. C-439), Example 1089 (Compound No. C-440), Example 1090 (Compound No. C-441), Example 1091 (Compound No. C-442), Example 1092 (Compound No. C-443), Example 1093 (Compound No. C-444), Example 1095 (Compound No. C-446), Example 1096 (Compound No. C-447), Example 1097 (Compound No. C-448), Example 1098 (Compound No. C-449), Example 1099 (Compound No. C-450), Example 1100 (Compound No. C-451), Example 1102 (Compound No. C-453), Example 1105 (Compound No. C-456), Example 1106 (Compound No. C-457), Example 1107 (Compound No. C-458), Example 1109 (Compound No. C-460), Example 1111 (Compound No. C-462), Example 1112 (Compound No. C-463), Example 1115 (Compound No. C-466), Example 1117 (Compound No. C-468), Example 1118 (Compound No. C-469), Example 1121 (Compound No. C-472), Example 1125 (Compound No. C-476), Example 1127 (Compound No. C-478), Example 1128 (Compound No. C-479), Example 1129 (Compound No. C-480), Example 1130 (Compound No. C-481), Example 1131 (Compound No. C-482), Example 1132 (Compound No. C-483), Example 1133 (Compound No. C-485), Example 1134 (Compound No. C-486), Example 1135 (Compound No. C-487), Example 1136 (Compound No. C-488), Example 1137 (Compound No. C-489), Example 1138 (Compound No. C-490), Example 1139 (Compound No. C-491), Example 1140 (Compound No. C-492), Example 1141 (Compound No. C-493), Example 1142 (Compound No. C-494), Example 1144 (Compound No. C-496), Example 1145 (Compound No. C-497), Example 1146 (Compound No. C-498), Example 1147 (Compound No. C-499), Example 1148 (Compound No. C-500), Example 1149 (Compound No. C-501), Example 1151 (Compound No. C-503), Example 1153 (Compound No. C-505), Example 1154 (Compound No. C-506), Example 1155 (Compound No. C-507), Example 1156 (Compound No. C-508), Example 1157 (Compound No. C-509), Example 1158 (Compound No. C-510), Example 1159 (Compound No. C-511), Example 1160 (Compound No. C-512), Example 1161 (Compound No. C-513), Example 1162 (Compound No. C-514), Example 1163 (Compound No. C-515), Example 1164 (Compound No. C-516), Example 1165 (Compound No. C-517), Example 1166 (Compound No. C-518), Example 1167 (Compound No. C-519), Example 1168 (Compound No. C-520), Example 1169 (Compound No. C-521), Example 1170 (Compound No. C-522), Example 1171 (Compound No. C-523), Example 1172 (Compound No. C-524), Example 1173 (Compound No. C-525), Example 1174 (Compound No. C-526), Example 1175 (Compound No. C-527), Example 1176 (Compound No. C-528), Example 1177 (Compound No. C-529), Example 1178 (Compound No. C-530), Example 1179 (Compound No. C-531), Example 1180 (Compound No. C-532), Example 1181 (Compound No. C-533), Example 1182 (Compound No. C-534), Example 1183 (Compound No. C-535), Example 1184 (Compound No. C-536), Example 1185 (Compound No. C-537), Example 1186 (Compound No. C-538), Example 1187 (Compound No. C-539), Example 1188 (Compound No. C-540), Example 1189 (Compound No. C-541), Example 1190 (Compound No. C-542), Example 1191 (Compound No. C-543), Example 1192 (Compound No. C-544), Example 1193 (Compound No.C-545), Example 1194 (Compound No. C-546), Example 1195 (Compound No. C-547), Example 1196 (Compound No. C-548), Example 1197 (Compound No. C-549), Example 1198 (Compound No. C-550), Example 1199 (Compound No. C-551), Example 1200 (Compound No. C-552), Example 1201 (Compound No. C-553), Example 1202 (Compound No. C-554), Example 1203 (Compound No. C-561), Example 1205 (Compound No. C-564), Example 1206 (Compound No. C-565), Example 1207 (Compound No. C-566), Example 1208 (Compound No. C-567), Example 1209 (Compound No. C-568), Example 1210 (Compound No. C-569), Example 1211 (Compound No. C-570), Example 1212 (Compound No. C-571), Example 1213 (Compound No. C-572), Example 1214 (Compound No. C-573), Example 1215 (Compound No. C-574), Example 1216 (Compound No. C-575), Example 1217 (Compound No. C-576), Example 1218 (Compound No. C-577), Example 1219 (Compound No. C-578), Example 1220 (Compound No. C-579), Example 1221 (Compound No. C-580), Example 1222 (Compound No. C-581), Example 1223 (Compound No. C-582), Example 1224 (Compound No. C-583), Example 1225 (Compound No. C-584), Example 1226 (Compound No. C-585), Example 1227 (Compound No. C-586), Example 1228 (Compound No. C-587), Example 1229 (Compound No. C-588), Example 1230 (Compound No. C-589), Example 1231 (Compound No. C-590), Example 1232 (Compound No. C-591), Example 1233 (Compound No. C-592), Example 1234 (Compound No. C-593), Example 1235 (Compound No. C-594), Example 1236 (Compound No. C-595), Example 1237 (Compound No. C-596), Example 1238 (Compound No. C-597), Example 1239 (Compound No. C-598), Example 1240 (Compound No. C-599), Example 1241 (Compound No. C-600), Example 1242 (Compound No. C-601), Example 1243 (Compound No. C-602), Example 1244 (Compound No. C-603), Example 1245 (Compound No. C-604), Example 1246 (Compound No. C-605), Example 1247 (Compound No. C-606), Example 1248 (Compound No. C-607), Example 1249 (Compound No. C-608), Example 1250 (Compound No. C-609), Example 1252 (Compound No. C-611), Example 1253 (Compound No. C-612), Example 1254 (Compound No. C-613), Example 1255 (Compound No. C-614), Example 1256 (Compound No. C-615), Example 1257 (Compound No. C-616), Example 1258 (Compound No. C-617), Example 1259 (Compound No. C-618), Example 1260 (Compound No. C-619), Example 1261 (Compound No. C-620), Example 1262 (Compound No. C-621), Example 1263 (Compound No. C-622), Example 1264 (Compound No. C-623), Example 1265 (Compound No. C-624), Example 1266 (Compound No. C-625), Example 1267 (Compound No. C-626), Example 1268 (Compound No. C-627), Example 1269 (Compound No. C-628), Example 1270 (Compound No. C-629), Example 1271 (Compound No. C-630), Example 1272 (Compound No. C-631), Example 1273 (Compound No. C-632), Example 1274 (Compound No. C-633), Example 1276 (Compound No. C-635), Example 1278 (Compound No. C-637), Example 1280 (Compound No. C-639), Example 1281 (Compound No. C-640), Example 1282 (Compound No. C-641), Example 1283 (Compound No. C-642), Example 1289 (Compound No. C-649), Example 1290 (Compound No. C-650), Example 1291 (Compound No. C-651), Example 1293 (Compound No. C-653), Example 1294 (Compound No. C-654), Example 1295 (Compound No. C-655), Example 1297 (Compound No. C-657), Example 1298 (Compound No. C-658), Example 1299 (Compound No. C-659), Example 1300 (Compound No. C-660), Example 1301 (Compound No. C-661), Example 1302 (Compound No. C-662), Example 1303 (Compound No. C-663), Example 1304 (Compound No. C-664), Example 1305 (Compound No. C-665), Example 1306 (Compound No. C-666), Example 1307 (Compound No. C-667), Example 1308 (Compound No. C-668), Example 1309 (Compound No. C-669), Example 1310 (Compound No. C-670), Example 1311 (Compound No. C-671), Example 1312 (Compound No. C-672), Example 1313 (Compound No. C-673), Example 1314 (Compound No. C-674), Example 1315 (Compound No. C-675), Example 1316 (Compound No. C-676), Example 1317 (Compound No. C-677), Example 1318 (Compound No. C-678), Example 1322 (Compound No. C-682), Example 1326 (Compound No. C-686), Example 1328 (Compound No. C-688), Example 1329 (Compound No. C-689), Example 1330 (Compound No. C-690), Example 1332 (Compound No. C-692), Example 1333 (Compound No. C-693), Example 1334 (Compound No. C-694), Example 1335 (Compound No. C-695), Example 1336 (Compound No. C-696), Example 1337 (Compound No. C-697), Example 1338 (Compound No. C-698), Example 1339 (Compound No. C-699), Example 1340 (Compound No. C-701), Example 1341 (Compound No. C-702), Example 1342 (Compound No. C-703), Example 1343 (Compound No. C-704), Example 1344 (Compound No. C-705), Example 1345 (Compound No. C-706), Example 1346 (Compound No. C-707), Example 1347 (Compound No. C-708), Example 1348 (Compound No. C-709), Example 1349 (Compound No. C-710), Example 1350 (Compound No. C-711), Example 1351 (Compound No. C-712), Example 1352 (Compound No. C-713), Example 1354 (Compound No. C-715), Example 1356 (Compound No. C-717), Example 1357 (Compound No. C-718), Example 1359 (Compound No. C-720), Example 1364 (Compound No. C-725), Example 1365 (Compound No. C-726), Example 1368 (Compound No. C-729), Example 1369 (Compound No. C-730), Example 1370 (Compound No. C-731), Example 1371 (Compound No. C-732), Example 1373 (Compound No. C-734), Example 1374 (Compound No. C-735), Example 1375 (Compound No. C-736), Example 1376 (Compound No. C-737), Example 1377 (Compound No. C-738), Example 1378 (Compound No. C-739), Example 1379 (Compound No. C-740), Example 1381 (Compound No. C-742), Example 1382 (Compound No. C-743), Example 1387 (Compound No. C-748), Example 1389 (Compound No. C-750), Example 1391 (Compound No. C-752), Example 1413 (Compound No. C-774), Example 1414 (Compound No. C-775), Example 1415 (Compound No. C-776), Example 1416 (Compound No. C-777), Example 1419 (Compound No. C-780), Example 1423 (Compound No. C-785), Example 1425 (Compound No. C-787), Example 1427 (Compound No. C-789), Example 1430 (Compound No. C-792), Example 1450 (Compound No. C-814), Example 1479 (Compound No. C-843), Example 1508 (Compound No. C-872), Example 1561 (Compound No. C-925), Example 1566 (Compound No. C-930), Example 1567 (Compound No. C-931), Example 1569 (Compound No. C-933), Example 1572 (Compound No. C-936), Example 1578 (Compound No. C-942), Example 1579 (Compound No. C-943), Example 1581 (Compound No. C-945), Example 1583 (Compound No. C-947), Example 1585 (Compound No. C-949), Example 1586 (Compound No. C-950), Example 1587 (Compound No. C-951), Example 1589 (Compound No. C-953), Example 1597 (Compound No. C-961), Example 1598 (Compound No. C-962), Example 1599 (Compound No. C-963), Example 1600 (Compound No. C-964), Example 1601 (Compound No. C-965), Example 1602 (Compound No. C-966), Example 1613 (Compound No. C-977), Example 1653 (Compound No. C-1017), Example 1654 (Compound No. C-1018), Example 1664 (Compound No. C-1028), Example 1665 (Compound No. C-1029), Example 1666 (Compound No. C-1030), Example 1667 (Compound No. C-1031), Example 1668 (Compound No. C-1032), Example 1669 (Compound No. C-1033), Example 1674 (Compound No. C-1038), Example 1675 (Compound No. C-1039), Example 1676 (Compound No. C-1040), Example 1677 (Compound No. C-1041), Example 1679 (Compound No. C-1043), Example 1681 (Compound No. C-1045), Example 1689 (Compound No. C-1053), Example 1690 (Compound No. C-1054), Example 1691 (Compound No. C-1055), Example 1696 (Compound No. C-1060), Example 1708 (Compound No. C-1072), Example 1754 (Compound No. C-1118), Example 1755 (Compound No. C-1119), Example 1776 (Compound No. C-1140), Example 1778 (Compound No. C-1142), Example 1783 (Compound No. C-1147), Example 1798 (Compound No. C-1162), Example 1883 (Compound No. D-12), Example 1884 (Compound No. D-13), Example 1885 (Compound No. D-14), Example 1890 (Compound No. D-19), Example 1891 (Compound No. D-20), Example 1892 (Compound No. D-21), Example 1895 (Compound No. D-24), Example 1896 (Compound No. D-25), Example 1897 (Compound No. D-26), Example 1900 (Compound No. D-29), Example 1907 (Compound No. D-36), Example 1908 (Compound No. D-37), Example 1914 (Compound No. D-43), Example 1916 (Compound No. D-45), Example 1917 (Compound No. D-46), Example 1918 (Compound No. D-47), Example 1919 (Compound No. D-48), Example 1922 (Compound No. D-51), Example 1924 (Compound No. D-53), Example 1925 (Compound No. D-54), Example 1928 (Compound No. D-57), Example 1934 (Compound No. D-67), Example 1937 (Compound No. D-70), Example 1938 (Compound No. D-71), Example 1939 (Compound No. D-72), Example 1940 (Compound No. D-73), Example 1943 (Compound No. D-76), Example 1944 (Compound No. D-78), Example 1945 (Compound No. D-79), Example 1946 (Compound No. D-80), Example 1947 (Compound No. D-81), Example 1948 (Compound No. D-82), Example 1949 (Compound No. D-83), Example 1950 (Compound No. D-84), Example 1951 (Compound No. D-85), Example 1952 (Compound No. D-86), Example 1953 (Compound No. D-87), Example 1954 (Compound No. D-88), Example 1956 (Compound No. D-90), Example 1957 (Compound No. D-91), Example 1960 (Compound No. D-94), Example 1961 (Compound No. D-95), Example 1962 (Compound No. D-96), Example 1963 (Compound No. D-97), Example 1964 (Compound No. D-98), Example 1965 (Compound No. D-99), Example 1966 (Compound No. D-100), Example 1967 (Compound No. D-102), Example 1979 (Compound No. D-114), Example 1980 (Compound No. D-116), Example 1982 (Compound No. D-118), Example 1983 (Compound No. D-119), Example 1986 (Compound No. D-122), Example 1987 (Compound No. D-123), Example 1989 (Compound No. D-125), Example 1990 (Compound No. D-126), Example 1991 (Compound No. D-127), Example 2004 (Compound No. D-143), Example 2005 (Compound No. D-144), Example 2007 (Compound No. D-147), Example 2009 (Compound No. D-150), Example 2027 (Compound No. D-171), Example 2028 (Compound No. D-172), Example 2029 (Compound No. D-173), Example 2030 (Compound No. D-174), Example 2031 (Compound No. D-175), Example 2033 (Compound No. D-177), Example 2034 (Compound No. D-178), Example 2052 (Compound No. D-197), Example 2060 (Compound No. D-205), Example 2062 (Compound No. D-207), Example 2064 (Compound No. D-209), Example 2065 (Compound No. D-210), Example 2072 (Compound No. D-218), Example 2089 (Compound No. D-236), Example 2090 (Compound No. D-237), Example 2091 (Compound No. D-238), Example 2092 (Compound No. D-239), Example 2093 (Compound No. D-240), Example 2095 (Compound No. D-242), Example 2096 (Compound No. D-243), Example 2097 (Compound No. D-244), Example 2098 (Compound No. D-245), Example 2099 (Compound No. D-246), Example 2101 (Compound No. D-248), Example 2105 (Compound No. D-252), Example 2106 (Compound No. D-253), Example 2107 (Compound No. D-254), Example 2109 (Compound No. D-256), Example 2113 (Compound No. D-260), Example 2117 (Compound No. D-264), Example 2118 (Compound No. D-265), Example 2121 (Compound No. D-268), Example 2122 (Compound No. D-269), Example 2124 (Compound No. D-271), Example 2125 (Compound No. D-272), Example 2127 (Compound No. D-274), Example 2128 (Compound No. D-275), Example 2139 (Compound No. D-288), Example 2143 (Compound No. D-293), Example 2152 (Compound No. D-302), Example 2157 (Compound No. D-307), Example 2158 (Compound No. D-308), Example 2159 (Compound No. D-309), Example 2165 (Compound No. D-315), Example 2169 (Compound No. D-319), Example 2170 (Compound No. D-320), Example 2171 (Compound No. D-321), Example 2178 (Compound No. D-328), Example 2179 (Compound No. D-329), Example 2182 (Compound No. D-332), Example 2184 (Compound No. D-334), Example 2185 (Compound No. D-335), Example 2186 (Compound No. D-336), Example 2187 (Compound No. D-337), Example 2188 (Compound No. D-339), Example 2189 (Compound No. D-340), Example 2191 (Compound No. D-342), Example 2192 (Compound No. D-343), Example 2193 (Compound No. D-344), Example 2197 (Compound No. D-348), Example 2198 (Compound No. D-350), Example 2201 (Compound No. D-353), Example 2202 (Compound No. D-354), Example 2203 (Compound No. D-355), Example 2206 (Compound No. D-358), Example 2213 (Compound No. D-365), Example 2214 (Compound No. D-366), Example 2241 (Compound No. D-393), Example 2245 (Compound No. D-397), Example 2296 (Compound No. E-6) and Example 2332 (Compound No. E-43).

### [Industrial filed of applicability]

As described above, the compounds of the present invention have excellent antifungal activity against fungi, which cause various diseases and result in some agricultural and horticultural problems, in particular, Phytophthora or Pythium. Therefore, the compounds of the present invention can be used as plant disease controlling agents.

## Claims

1. A 4-acylaminopyrazole derivative represented by the following general formula: 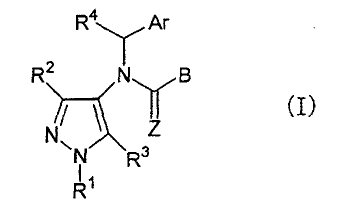 wherein R¹ represents a hydrogen atom, an optionally substituted C₁-C₁₆ alkyl group (substituent(s) being selected from the substituent group a below), a C₃-C₈ cycloalkyl group, an optionally substituted C₂-C₆ alkenyl group (substituent(s) being selected from the substituent group f below), a C₄-C₆ cycloalkenyl group, a C₂-C₆ alkynyl group, an optionally substituted C₆-C₁₄ aryl group (substituent(s) being selected from the substituent group g below), a 4-6 membered saturated heterocyclic group (the heterocycle contains 1-2 same or different heteroatom(s) selected from the group consisting of an oxygen atom, a sulfur atom and a nitrogen atom), an optionally substituted 5-6 membered unsaturated heterocyclic group (substituent(s) being selected from the substituent group i below; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a C₂-C₇ alkylcarbonyl group, an optionally substituted benzoyl group (substituent(s) being selected from the substituent group b below), a C₂-C₇ alkoxycarbonyl group, a C₈-C₁₀ aralkyloxycarbonyl group, a C₃-C₇ alkenyloxycarbonyl group, an optionally substituted phenylsulfonyl group (substituent(s) being C₁-C₆ alkyl group) or a di(C₁-C₆ alkyl)sulfamoyl group; R² and R³ represent, independently, a hydrogen atom, a halogen atom, an optionally substituted C₁-C₆ alkyl group (substituent(s) being selected from the substituent group j below), a carboxyl group, a carbamoyl group, a C₂-C₇ alkoxycarbonyl group, a C₂-C₇ alkylcarbamoyl group, a cyano group, a C₁-C₆ alkoxy group or a phenyl group;
R⁴ represents a hydrogen atom, a C₁-C₆ alkyl group or a cyano group;
Z represents an oxygen atom or a sulfur atom; Ar represents an optionally substituted C₆-C₁₄ aryl group (substituent(s) being selected from the substituent group k below) or an optionally substituted 5-6 membered unsaturated heterocyclic group (substituent(s) being selected from the substituent group b below; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s));
B represents a hydrogen atom, a halogen atom, an optionally substituted C₁-C₁₆ alkyl group (substituent(s) being selected from the substituent group m below), a C₃-C₈ cycloalkyl group, an optionally substituted C₂-C₆ alkenyl group (substituent(s) being selected from the substituent group n below), a C₄-C₈ cycloalkenyl group, a C₂-C₆ alkynyl group, an optionally substituted phenyl group (substituent(s) being selected from the substituent group b below), a 4-6 membered saturated heterocyclic group (the heterocycle contains 1-2 same or different heteroatom(s) selected from the group consisting of an oxygen atom, a sulfur atom and a nitrogen atom), an optionally substituted 5-6 membered unsaturated heterocyclic group (substituent(s) being selected from the substituent group b below; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a C₁-C₆ alkoxy group, a C₇-C₉ aralkyloxy group, a C₃-C₆ cycloalkoxy group, a C₂-C₆ alkenyloxy group, an optionally substituted phenoxy group (substituent(s) being selected from the substituent group b below), an optionally substituted 5-6 membered unsaturated (heterocyclyl)oxy group (substituent(s) being selected from the substituent group b below; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a C₁-C₆ alkylthio group, a C₇-C₉ aralkylthio group, a C₃-C₆ cycloalkylthio group, a C₂-C₆ alkenylthio group, an optionally substituted phenylthio group (substituent(s) being selected from the substituent group b below) or an optionally substituted amino group (substituent(s) being selected from the substituent group d below);
or Ar and B are taken together to represent an optionally substituted o-phenylene group (substituent(s) being selected from the substituent group b below, and one methylene group may be inserted between the phenylene group and the carbon atom to which B binds);
provided that, when R¹, R² and R³ are all a methyl group, then B is neither an unsubstituted alkyl group nor a cycloalkyl group;
the substituent group a is the group consisting of halogen atom, a C₂-C₇ alkylcarbonyl group, an optionally substituted benzoyl group (substituent(s) being selected from the substituent group b below), a carboxyl group, a C₂-C₇ alkoxycarbonyl group, a phenoxycarbonyl group, an optionally substituted carbamoyl group (substituent(s) being selected from the substituent group c below), a cyano group, a nitro group, an optionally substituted C₃-C₆ cycloalkyl group (substituent(s) being C₁-C₆ alkyl group or halogen atom), a C₇-C₈ bicycloalkyl group, an optionally substituted C₆-C₁₄ aryl group (substituent(s) being selected from the substituent group b below), an optionally substituted 4-6 membered saturated heterocyclic group (substituent(s) being C₁-C₆ alkyl group or halogen atom; the heterocycle contains 1-2 same or different heteroatom(s) selected from the group consisting of an oxygen atom, a sulfur atom and a nitrogen atom), an optionally substituted 5-6 membered unsaturated heterocyclic group (substituent(s) being selected from the substituent group b below; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a hydroxyl group, an optionally substituted C₁-C₆ alkoxy group (substituent(s) being cyano group, C₁-C₆ alkoxy group or tri(C₁-C₆ alkyl)silyl group), a C₇-C₉ aralkyloxy group, a C₂-C₆ alkenyloxy group, a C₂-C₆ alkynyloxy group, an optionally substituted phenoxy group (substituent(s) being selected from the substituent group b below), an optionally substituted 4-6 membered saturated (heterocyclyl)oxy group (substituent(s) being C₁-C₆ alkyl group; the heterocycle contains 1-2 same or different heteroatom(s) selected from the group consisting of an oxygen atom, a sulfur atom and a nitrogen atom), an optionally substituted 5-6 membered unsaturated (heterocyclyl)oxy group (substituent(s) being selected from the substituent group b below; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a tri(C₁-C₆ alkyl)silyloxy group, a di(C₁-C₆ alkyl) (phenyl)silyloxy group, a mono(C₁-C₆ alkyl) (diphenyl)silyloxy group, a triphenylsilyloxy group, an optionally substituted C₂-C₁₇ alkylcarbonyloxy group {substituent(s) being halogen atom, substituted C₁-C₆ alkoxy group (substituent(s) being cyano group) or substituted C₁-C₆ alkylthio group (substituent(s) being cyano group)}, a C₄-C₉ cycloalkylcarbonyloxy group, a C₃-C₇ alkenylcarbonyloxy group, a C₅-C₇ cycloalkenylcarbonyloxy group, a C₃-C₇ alkynylcarbonyloxy group, an optionally substituted benzoyloxy group (substituent(s) being selected from the substituent group b below), an optionally substituted 5-6 membered unsaturated (heterocyclyl)carbonyloxy group (substituent(s) being selected from the substituent group b below; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a C₂-C₇ alkoxycarbonyloxy group, a C₈-C₁₀ aralkyloxycarbonyloxy group, a C₃-C₇ alkenyloxycarbonyloxy group, an optionally substituted phenoxycarbonyloxy group (substituent(s) being selected from the substituent group b below), an optionally substituted carbamoyloxy group (substituent(s) being selected from the substituent group c below), a C₂-C₇ alkylthiocarbonyloxy group, a phenyl(thiocarbonyl)oxy group, an optionally substituted thiocarbamoyloxy group (substituent(s) being selected from the substituent group c below), a C₂-C₇ (alkylthio)(thiocarbonyloxy) group, an optionally substituted aminooxy group (substituent(s) being selected from the substituent group d below), an optionally substituted C₁-C₆ alkylthio group (substituent(s) being cyano group), a C₇-C₉ aralkylthio group, a C₃-C₆ cycloalkylthio group, an optionally substituted phenylthio group (substituent(s) being selected from the substituent group b below), a 5-6 membered unsaturated (heterocyclyl)thio group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted C₁-C₆ alkylsulfinyl group (substituent(s) being cyano group), a C₇-C₉ aralkylsulfinyl group, a C₃-C₆ cycloalkylsulfinyl group, a phenylsulfinyl group, a 5-6 membered unsaturated (heterocyclyl)sulfinyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted C₁-C₆ alkylsulfonyl group (substituent(s) being cyano group), a C₇-C₉ aralkylsulfonyl group, a C₃-C₆ cycloalkylsulfonyl group, a phenylsulfonyl group, a 5-6 membered unsaturated (heterocyclyl)sulfonyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted amino group (substituent(s) being selected from the substituent group d below), an optionally substituted imino group {substituent(s) being optionally substituted C₁-C₆ alkyl group (substituent(s) being halogen atom or cyano group), cyano group, nitro group, hydroxyl group, C₂-C₇ alkoxycarbonyloxy group, optionally substituted C₁-C₆ alkoxy group (substituent(s) being cyano group or C₂-C₇ alkoxycarbonyl group), C₇-C₉ aralkyloxy group or C₂-C₇ alkylcarbonyloxy group}, a C₁-C₆ alkylhydrazono group and a tri(C₁-C₆ alkyl)silyl group;
the substituent group b is the group consisting of a halogen atom, an optionally substituted C₁-C₆ alkyl group (substituent(s) being halogen atom), a hydroxyl group, an optionally substituted C₁-C₆ alkoxy group (substituent(s) being halogen atom), a benzoyloxy group, a carboxyl group, a C₂-C₇ alkoxycarbonyl group, a cyano group, a nitro group, an optionally substituted amino group (substituent(s) being C₁-C₆ alkyl group, C₂-C₇ alkylcarbonyl group or benzoyl group) and a phenyl group;
the substituent group c is the group consisting of an optionally substituted C₁-C₆ alkyl group {substituent(s) being halogen atom, C₂-C₇ (alkylthio)carbonyloxy group,
(phenylthio)carbonyloxy group, C₂-C₇ alkyl(thiocarbonyl)oxy group, phenyl(thiocarbonyl)oxy group, cyano group or C₁-C₆ alkoxyimino group}, an optionally substituted C₂-C₇ alkylcarbonyl group (substituent(s) being halogen atom, cyano group or C₁-C₆ alkoxyimino group), a C₂-C₇ alkoxycarbonyl group, a benzoyl group, a benzoylamino group and an optionally substituted phenyl group (substituent(s) being selected from
the above substituent group b);
the substituent group d is the group consisting of an optionally substituted C₁-C₆ alkyl group {substituent(s) being cyano atom, optionally substituted C₆-C₁₄ aryl group (substituent(s) being selected from the above substituent group b), carboxyl group, C₂-C₇ alkoxycarbonyl group, C₈-C₁₀ aralkyloxycarbonyl group, phenoxycarbonyl group or optionally substituted carbamoyl group (substituent(s) being selected from the above substituent group c)}, a C₃-C₆ cycloalkyl group, a C₂-C₆ alkenyl group, a formyl group, an optionally substituted C₂-C₁₇ alkylcarbonyl group (substituent(s) being halogen atom, C₂-C₇ alkoxycarbonylamino group, cyano group or C₁-C₆ alkoxyimino group), a C₄-C₉ cycloalkylcarbonyl group, a C₃-C₇ alkenylcarbonyl group, a C₅-C₇ cycloalkenylcarbonyl group, a C₃-C₇ alkynylcarbonyl group, a C₂-C₇ alkoxycarbonyl group, a C₈-C₁₀ aralkyloxycarbonyl group, a C₃-C₇ alkenyloxycarbonyl group, a phenoxycarbonyl group, a C₂-C₇ (alkylthio)carbonyl group, a C₂-C₇ (alkylthio)(thiocarbonyl) group, a phenyl(thiocarbonyl) group, an optionally substituted benzoyl group (substituent(s) being selected from the above substituent group b), an optionally substituted 5-6 membered unsaturated (heterocyclyl) carbonyl group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted carbamoyl group (substituent(s) being selected from the above substituent group c), a C₂-C₇ alkyl(thiocarbonyl) group, an optionally substituted thiocarbamoyl group (substituent(s) being selected from the above substituent group c), an optionally substituted C₁-C₆ alkylidene group (substituent(s) being selected from the substituent group e below), a C₄-C₆ cycloalkylidene group (an alkylene chain of the cycloalkylidene group may be interrupted with one oxygen atom), a phenyl group, a C₁-C₆ alkylsulfonyl group and a phenylsulfonyl group;
the substituent group e is the group consisting of a C₃-C₆ cycloalkyl group, a C₂-C₇ alkoxycarbonyl group, an optionally substituted 5-6 membered unsaturated (heterocyclyl)carbonyl group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted phenyl group (substituent(s) being selected from the above substituent group b), an optionally substituted 5-6 membered unsaturated heterocyclic group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)) and a C₁-C₆ alkoxy group; the substituent group f is the group consisting of a halogen atom, a hydroxyl group, a C₂-C₇ alkoxycarbonyl group and a C₁-C₆ alkoxy group;
the substituent group g is the group consisting of a halogen atom, an optionally substituted C₁-C₆ alkyl group (substituent(s) being selected from the substituent group h), an optionally substituted C₃-C₆ cycloalkyl group (substituent(s) being halogen atom or C₁-C₆ alkyl group), an optionally substituted C₂-C₆ alkenyl group (substituent(s) being phenyl group, C₁-C₆ alkoxy group or C₂-C₇ alkoxycarbonyl group), a C₂-C₆ alkynyl group, a C₂-C₇ alkylcarbonyl group, an optionally substituted benzoyl group (substituent(s) being selected from the above substituent group b), a carboxyl group, a C₂-C₇ alkoxycarbonyl group, an optionally substituted carbamoyl group (substituent(s) being selected from the above substituent group c), an optionally substituted thiocarbamoyl group (substituent(s) being selected from the above substituent group c), a cyano group, an optionally substituted phenyl group (substituent(s) being selected from the above substituent group b), a hydroxyl group, an optionally substituted C₁-C₆ alkoxy group (substituent(s) being halogen atom), an optionally substituted C₂-C₆ alkenyloxy group (substituent(s) being halogen atom), an optionally substituted phenoxy group (substituent(s) being selected from the above substituent group b), a C₂-C₇ alkylcarbonyloxy group, a benzoyloxy group, a C₂-C₇ alkoxycarbonyloxy group, a phenoxycarbonyloxy group, a mercapto group, a C₁-C₆ alkylthio group, a phenylthio group, a C₁-C₆ alkylsulfinyl group, a phenylsulfinyl group, a C₁-C₆ alkylsulfonyl group, a phenylsulfonyl group, a C₂-C₇ alkylcarbonylthio group, a benzoylthio group, a C₂-C₇ (alkylthio)(thiocarbonylthio) group, an azido group, an optionally substituted amino group (substituent(s) being selected from the above substituent group d), an isocyano group, a nitro group, a tri(C₁-C₆ alkyl)silyl group, and an optionally substituted 5-6 membered unsaturated heterocyclic group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s));
the substituent group h is the group consisting of a halogen atom, an optionally substituted C₁-C₆ alkylimino group {substituent(s) being C₁-C₆ alkylthio group, optionally substituted C₇-C₉ aralkylthio group (substituent(s) being selected from the above substituent group b) or phenylthio group} and an optionally substituted C₁-C₆ alkoxyimino group (substituent(s) being C₁-C₆ alkoxycarbonyl group, cyano group, amino group or phenyl group);
the substituent group i is the group consisting of the above substituent group b and an optionally substituted 1-pyrazolyl group {substituent(s) being C₁-C₆ alkyl group or N-acetyl-N-(cyanobenzyl)amino group};
the substituent group j is the group consisting of a halogen atom, a hydroxyl group, a C₁-C₆ alkoxy group, a C₂-C₇ alkylcarbonyloxy group, a C₂-C₇ alkoxycarbonyloxy group, a C₂-C₇ alkylcarbamoyloxy group and a C₁-C₆ alkylsulfonyloxy group; the substituent group k is the group consisting of a halogen atom, an optionally substituted C₁-C₆ alkyl group (substituent(s) being selected from the substituent group 1 below), an optionally substituted C₃-C₆ cycloalkyl group (substituent(s) being halogen atom or C₁-C₆ alkyl group), an optionally substituted C₂-C₆ alkenyl group (substituent(s) being phenyl group, C₁-C₆ alkoxy group or C₂-C₇ alkoxycarbonyl group), a C₂-C₆ alkynyl group, a C₂-C₇ alkylcarbonyl group, an optionally substituted benzoyl group (substituent(s) being selected from the above substituent group b), a carboxyl group, a C₂-C₇ alkoxycarbonyl group, an optionally substituted carbamoyl group (substituent(s) being selected from the above substituent group c), an optionally substituted thiocarbamoyl group (substituent(s) being selected from the above substituent group c), a cyano group, an optionally substituted phenyl group (substituent(s) being selected from the above substituent group b), an optionally substituted 5-6 membered unsaturated heterocyclic group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a hydroxyl group, an optionally substituted C₁-C₆ alkoxy group (substituent(s) being halogen atom), an optionally substituted C₂-C₆ alkenyloxy group (substituent(s) being halogen atom), an optionally substituted phenoxy group (substituent(s) being selected from the above substituent group b), a C₂-C₇ alkylcarbonyloxy group, a benzoyloxy group, a C₂-C₇ alkoxycarbonyloxy group, a phenoxycarbonyloxy group, a mercapto group, an optionally substituted C₁-C₆ alkylthio group (substituent(s) being halogen atom), a phenylthio group, an optionally substituted C₁-C₆ alkylsulfinyl group (substituent(s) being halogen atom), a phenylsulfinyl group, an optionally substituted C₁-C₆ alkylsulfonyl group (substituent(s) being halogen atom), a phenylsulfonyl group, a C₂-C₇ alkylcarbonylthio group, a benzoylthio group, a C₂-C₇ (alkylthio)(thiocarbonylthio) group, an azido group, an isocyano group, a nitro group, an optionally substituted amino group (substituent(s) being selected from the above substituent group d) and a tri(C₁-C₆ alkyl)silyl group;
the substituent group 1 is the group consisting of a halogen atom, a cyano group, a hydroxyl group, a C₁-C₆ alkoxy group, a C₁-C₆ alkylthio group, a C₁-C₆ alkylsulfinyl group, a C₁-C₆ alkylsulfonyl group, an optionally substituted amino group {substituent(s) being phenoxy(C₂-C₇ alkylcarbonyl) group or optionally substituted 4-pyrazolyl group (substituent(s) being C₁-C₆ alkyl group)}, an imino group, an optionally substituted C₁-C₆ alkylimino group {substituent(s) being C₁-C₆ alkylthio group, optionally substituted C₇-C₉ aralkylthio group (substituent(s) being selected from the above substituent group b) or phenylthio group}, a hydroxyimino group, a C₁-C₆ alkoxyimino group, a C₂-C₇ alkoxycarbonyl group and a phenyl group;
the substituent group m is the group consisting of a halogen atom, a carboxyl group, a C₂-C₇ alkoxycarbonyl group, a phenoxycarbonyl group, an optionally substituted carbamoyl group (substituent(s) being selected from the above substituent group c), a cyano group, a C₃-C₆ cycloalkyl group, a C₄-C₆ cycloalkenyl group, an optionally substituted C₆-C₁₄ aryl group (substituent(s) being selected from the above substituent group b), a 4-6 membered saturated heterocyclic group (the heterocycle contains 1-2 same or different heteroatom(s) selected from the group consisting of an oxygen atom, a sulfur atom and a nitrogen atom), an optionally substituted 5-6 membered unsaturated heterocyclic group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a hydroxyl group, an optionally substituted C₁-C₆ alkoxy group {substituent(s) being optionally substituted 5-6 membered unsaturated heterocyclic group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), cyano group, optionally substituted C₁-C₆ alkoxy group (substituent (s) being C₁-C₆ alkoxy group or tri (C₁-C₆ alkyl)silyl group) or C₇-C₉ aralkyloxy group}, an optionally substituted C₇-C₉ aralkyloxy group (substituent(s) being selected from the above substituent group b), a C₂-C₆ alkenyloxy group, a C₂-C₆ alkynyloxy group, an optionally substituted phenoxy group (substituent(s) being selected from the above substituent group b), a 4-6 membered saturated (heterocyclyl)oxy group (the heterocycle contains 1-2 same or different heteroatom(s) selected from the group consisting of an oxygen atom, a sulfur atom and a nitrogen atom), an optionally substituted 5-6 membered unsaturated (heterocyclyl)oxy group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a tri(C₁-C₆ alkyl)silyloxy group, a di(C₁-C₆ alkyl) (monophenyl)silyloxy group, a mono(C₁-C₆ alkyl) (diphenyl)silyloxy group, a triphenylsilyloxy group, a formyloxy group, an optionally substituted C₂-C₁₇ alkylcarbonyloxy group {substituent(s) being halogen atom, C₄-C₆ cycloalkenyl group, optionally substituted 5-6 membered unsaturated heterocyclic group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), optionally substituted C₁-C₆ alkoxy group (substituent(s) being cyano group), phenoxy group, C₂-C₇ alkylcarbonyloxy group or optionally substituted C₁-C₆ alkylthio group (substituent(s) being cyano group)}, an optionally substituted C₈-C₁₀ aralkylcarbonyloxy group (substituent(s) being selected from the above substituent group b), a C₄-C₉ cycloalkylcarbonyloxy group, an optionally substituted C₃-C₇ alkenylcarbonyloxy group (substituent(s) being phenyl group), a C₅-C₇ cycloalkenylcarbonyloxy group, an optionally substituted cyclohexadienylcarbonyloxy group (substituent(s) being C₁-C₆ alkyl group), a C₃-C₇ alkynylcarbonyloxy group, an optionally substituted benzoyloxy group (substituent(s) being selected from the above substituent group b), an optionally substituted 5-6 membered unsaturated hetero carbonyloxy group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a C₂-C₇ alkoxycarbonyloxy group, a C₃-C₇ alkenyloxycarbonyloxy group, a C₈-C₁₀ aralkyloxycarbonyloxy group, a phenoxycarbonyloxy group, an optionally substituted carbamoyloxy group (substituent(s) being selected from the above substituent group c), an optionally substituted thiocarbamoyloxy group (substituent(s) being selected from the above substituent group c), a C₂-C₇ (alkylthio)carbonyloxy group, a C₂-C₇ (alkylthio)(thiocarbonyloxy) group, an optionally substituted aminooxy group (substituent(s) being selected from the above substituent group d), a di(C₁-C₆ alkoxy)phosphoryloxy group, a di(C₁-C₆ alkoxy)thiophosphoryloxy group, a C₁-C₆ alkylsulfonyloxy group, an optionally substituted phenylsulfonyloxy group (substituent(s) being selected from the above substituent group b), an optionally substituted C₁-C₆ alkylthio group (substituent(s) being C₂-C₇ alkoxycarbonyl group or cyano group), a C₇-C₉ aralkylthio group, a phenylthio group, an optionally substituted 5-6 membered unsaturated (heterocyclyl)thio group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted C₁-C₆ alkylsulfinyl group (substituent(s) being cyano group), a C₇-C₉ aralkylsulfinyl group, a phenylsulfinyl group, a 5-6 membered unsaturated (heterocyclyl)sulfinyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), an optionally substituted C₁-C₆ alkylsulfonyl group (substituent(s) being cyano group), a C₇-C₉ aralkylsulfonyl group, a phenylsulfonyl group, a 5-6 membered unsaturated (heterocyclyl)sulfonyl group (the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)), a di(C₁-C₆ alkoxy)thiophosphorylthio group, an optionally substituted amino group (substituent(s) being selected from the above substituent group d) and an optionally substituted C₁-C₆ alkoxyimino group (substituent(s) being phenyl group or cyano group);
the substituent group n is the group consisting of a phenyl group and an optionally substituted N-aralkyl-N-(5-6 membered unsaturated heterocyclyl)aminocarbonyl group (substituent(s) being selected from the above substituent group b; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring or a pyridine ring, and the heterocycle may be substituted with 1-2 oxo group(s)); or a salt thereof.

2. The 4-acylaminopyrazole derivative according to claim 1, wherein R¹ is a C₁-C₁₀ alkyl group, a halo(C₁-C₈ alkyl) group (the halogen substituent(s) is (are) 1-3 same or different substituent(s) selected from the group consisting of a fluorine atom, a chlorine atom and a bromine atom), a (C₂-C₄ alkylcarbonyl) (C₁-C₃ alkyl) group, a benzoyl(C₁-C₄ alkyl) group wherein the phenyl part may be substituted (substituent(s) being fluorine atom, chlorine atom or bromine atom), a (C₂-C₄ alkoxycarbonyl)(C₁-C₃ alkyl) group, a phenoxycarbonyl(C₁-C₃ alkyl) group, a cyano(C₁-C₃ alkyl) group, a (C₃-C₆ cycloalkyl)(C₁-C₃ alkyl) group wherein the cycloalkyl part may be substituted (substituent(s) being C₁-C₃ alkyl group, fluorine atom or chlorine atom), a norbornyl(C₁-C₃ alkyl) group, a benzyl group wherein the phenyl part may be substituted {substituent(s) being fluorine atom, chlorine atom, bromine atom, cyano group or halo(C₁-C₃ alkoxy) group (the halogen substituent(s) is (are) 1-3 same or different fluorine atom(s), chlorine atom(s) or bromine atom(s))}, a (5-6 membered saturated heterocyclyl) (C₁-C₃ alkyl) group wherein the heterocycle part may be substituted (the substituent(s) is (are) 1-2 same or different C₁-C₃ alkyl group(s), and the heterocycle contains 1-2 same or different heteroatom(s) selected from the group consisting of an oxygen atom and a nitrogen atom), a (5-6 membered unsaturated heterocyclyl) (C₁-C₃ alkyl) group wherein the heterocycle part may be substituted (substituent(s) being fluorine atom, chlorine atom or bromine atom; the heterocycle contains an oxygen atom, a sulfur atom or a nitrogen atom, and may contain one further nitrogen atom, and the heterocycle may be fused with a benzene ring), a hydroxy(C₁-C₄ alkyl) group, a (C₁-C₃ alkoxy)(C₁-C₄ alkyl) group, a (C₁-C₃ alkoxy)(C₁-C₃ alkoxy) (C₁-C₃ alkyl) group, a phenoxy(C₁-C₃ alkyl) group, a (5-6 membered saturated heterocyclyl)oxy(C₁-C₃ alkyl) group (the heterocycle contains 1-2 same or different heteroatom(s) selected from the group consisting of an oxygen atom and a nitrogen atom), a tri(C₁-C₃ alkyl)silyloxy(C₁-C₄ alkyl) group, a (C₂-C₄ alkylcarbonyloxy)(C₁-C₄ alkyl) group (the alkylcarbonyloxy substituent(s) is (are) 1-2 same or different alkylcarbonyloxy group(s)), a benzoyloxy(C₁-C₃ alkyl) group wherein the phenyl part may be substituted (the substituent(s) is (are) 1-2 same or different substituent(s) selected from the group consisting of a fluorine atom, a chlorine atom and a bromine atom), a (C₁-C₄ alkylthio) (C₁-C₃ alkyl) group, a (C₃-C₆ cycloalkylthio) (C₁-C₃ alkyl) group, a (C₁-C₄ alkylsulfonyl) (C₁-C₃ alkyl) group, a (C₃-C₆ cycloalkylsulfonyl) (C₁-C₃ alkyl) group, a hydroxyimino (C₁-C₃ alkyl) group, a (C₂-C₄ alkoxycarbonyloxyimino) (C₁-C₃ alkyl) group, a (C₁-C₃ alkoxyimino) (C₁-C₃ alkyl) group wherein the alkyl part may be substituted (substituent(s) being C₂-C₄ alkoxycarbonyl group), a (C₇-C₈ aralkyloxyimino) (C₁-C₃ alkyl) group, a (C₂-C₄ alkylcarbonyloxyimino) (C₁-C₃ alkyl) group, a tri (C₁-C₃ alkyl) silyl (C₁-C₃ alkyl) group, a C₃-C₆ cycloalkyl group, a C₂-C₆ alkenyl group, a halo(C₂-C₄ alkenyl) group (the halogen substituent(s) is (are) 1-2 same or different substituent(s) selected from the group consisting of a fluorine atom, a chlorine atom, a bromine atom and an iodine atom), a hydroxy(C₂-C₃ alkenyl) group wherein the alkenyl part may be substituted (substituent(s) being C₂-C₄ alkoxycarbonyl group), a (C₁-C₃ alkoxy) (C₂-C₃ alkenyl) group wherein the alkenyl part may be substituted (substituent(s) being C₂-C₄ alkoxycarbonyl group), a C₂-C₃ alkynyl group, an optionally substituted phenyl group {the substituent(s) is (are) 1-3 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a carboxyl group, a C₂-C₄ alkoxycarbonyl group, a carbamoyl group, a cyano group, a C₁-C₃ alkoxy group, a halo(C₁-C₃ alkoxy) group (the halogen substituent(s) is (are) 1-3 same or different substituent(s) selected from the group consisting of a fluorine atom, a chlorine atom and a bromine atom), a halo(C₂-C₃ alkenyl)oxy group (the halogen substituent(s) is (are) 1-2 same or different halogen atom(s) selected from the group consisting of a fluorine atom, a chlorine atom, a bromine atom and an iodine atom) and a nitro group}, a 5-6 membered saturated heterocyclic group (the heterocycle contains 1-2 same or different heteroatom(s) selected from the group consisting of an oxygen atom and a nitrogen atom), an optionally substituted 5-6 membered unsaturated heterocyclic group {the substituent(s) is (are) 1-2 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom) and a C₁-C₃ alkoxy group; the heterocycle contains a sulfur atom or a nitrogen atom, and may contain 1-2 further nitrogen atom(s), and the heterocycle may be fused with a benzene ring}, a C₂-C₆ alkylcarbonyl group, an optionally substituted benzoyl group {substituent(s) being fluorine atom, chlorine atom, bromine atom, cyano group or halo(C₁-C₃ alkoxy) group (the halogen substituent(s) is (are) 1-3 same or different substituent(s) selected from the group consisting of a fluorine atom, a chlorine atom and a bromine atom)}, a C₂-C₅ alkoxycarbonyl group, an optionally substituted phenylsulfonyl group (substituent(s) being C₁-C₃ alkyl group) or a di(C₁-C₃ alkyl)sulfamoyl group, or a salt thereof.

3. The 4-acylaminopyrazole derivative according to claim 1, wherein R¹ is a C₂-C₈ alkyl group, a halo(C₃-C₆ alkyl) group (substituent(s) being fluorine atom or chlorine atom), a (C₃-C₆ cycloalkyl)(C₁-C₂ alkyl) group wherein the cycloalkyl part may be substituted (substituent(s) being methyl group, fluorine atom or chlorine atom), a norbornyl(C₁-C₂ alkyl) group, a benzyl group wherein the phenyl part may be substituted (substituent(s) being fluorine atom, chlorine atom, bromine atom or cyano group), a (5-6 membered saturated heterocyclyl) (C₁-C₂ alkyl) group (the heterocycle contains one oxygen atom), a methoxy(C₃-C₄ alkyl) group, a trimethylsilyloxy(C₃-C₄ alkyl) group, an acetoxy(C₃-C₄ alkyl) group (the number of acetoxy substituent(s) is 1-2), a (C₃-C₄ alkylthio) (C₁-C₂ alkyl) group, a (C₅-C₆ cycloalkylthio) (C₁-C₂ alkyl) group, a (C₃-C₄ alkylsulfonyl) (C₁-C₂ alkyl) group, a trimethylsilyl (C₁-C₂ alkyl) group, a C₅-C₆ cycloalkyl group, a C₄-C₅ alkenyl group, a C₂-C₃ alkynyl group, an optionally substituted phenyl group {the substituent(s) is (are) 1-2 same or different substituent(s) selected from the group consisting of a fluorine atom, a chlorine atom, a methyl group, a halo(C₁-C₂ alkyl) group (the halogen substituent(s) is (are) 1-3 fluorine atom(s)), a carbamoyl group and a cyano group}, a C₃-C₆ alkylcarbonyl group, an optionally substituted benzoyl group (substituent(s) being fluorine atom) or a dimethylsulfamoyl group, or a salt thereof.

4. The acylaminopyrazole derivative according to claim 1, wherein R¹ is a C₂-C₆ alkyl group, a halo(C₃-C₄ alkyl) group (halogen substituent(s) being fluorine atom or chlorine atom), a (C₄-C₆ cycloalkyl)methyl group wherein the cycloalkyl part may be substituted (substituent(s) being methyl group or fluorine atom), a norbornylmethyl group, a benzyl group, a 3-fluorobenzyl group, a tetrahydropyranylmethyl group, a (C₃-C₄ alkylthio)methyl group, a (C₃-C₄ alkylsulfonyl)methyl group, a trimethylsilylmethyl group, a cyclohexyl group, a pentenyl group, a phenyl group, a 2-fluorophenyl group, a 3-fluorophenyl group, a 4-fluorophenyl group, a 2-chlorophenyl group, a 3-chlorophenyl group, a 2,3-difluorophenyl group, a 2,5-difluorophenyl group, a 3,5-difluorophenyl group, a 2-methylphenyl group, a 3-methylphenyl group, a 4-methylphenyl group or a C₄-C₆ alkylcarbonyl group, or a salt thereof.

5. The 4-acylaminopyrazole derivative according to claim 1, wherein R¹ is a C₃-C₆ alkyl group, a cyclobutylmethyl group, a cyclopentylmethyl group, a phenyl group, a 3-fluorophenyl group, a 3,5-difluorophenyl group, or a 3-methylphenyl group, or a salt thereof.

6. The 4-acylaminopyrazole derivative according to claim 1, wherein R¹ is a C₄-C₅ branched alkyl group, a cyclobutylmethyl group, a phenyl group, a 3-fluorophenyl group or a 3-methylphenyl group, or a salt thereof.

7. The 4-acylaminopyrazole derivative according to any one of claims 1-6, wherein R² is a hydrogen atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a hydroxy(C₁-C₃ alkyl) group, a (C₁-C₃ alkoxy) (C₁-C₃ alkyl) group, a carboxyl group, a C₂-C₄ alkoxycarbonyl group or a cyano group, or a salt thereof.

8. The 4-acylaminopyrazole derivative according to any one of claims 1-6, wherein R² is a hydrogen atom or a methyl group, or a salt thereof.

9. The 4-acylaminopyrazole derivative according to any one of claims 1-8, wherein R³ is a hydrogen atom, a fluorine atom, a chlorine atom, a bromine atom, a C₁-C₄ alkyl group, a halo(C₁-C₃ alkyl) group (the halogen substituent(s) is (are) 1-3 same or different substituent(s) selected from the group consisting of a fluorine atom, a chlorine atom and a bromine atom), a hydroxy(C₁-C₃ alkyl) group, a (C₁-C₃ alkoxy) (C₁-C₃ alkyl) group, a (C₁-C₃ alkylsulfonyloxy) (C₁-C₃ alkyl) group, a carboxyl group, a C₂-C₄ alkoxycarbonyl group, a cyano group or a C₁-C₃ alkoxy group, or a salt thereof.

10. The 4-acylaminopyrazole derivative according to any one of claims 1-8, wherein R³ is a hydrogen atom, a chlorine atom, a C₁-C₂ alkyl group, a halo (C₁-C₂ alkyl) group (the halogen substituent(s) is (are) 1-3 fluorine atom(s)) or a cyano group, or a salt thereof.

11. The 4-acylaminopyrazole derivative according to any one of claims 1-8, wherein R³ is a hydrogen atom, a methyl group or a fluoromethyl group, or a salt thereof.

12. The 4-acylaminopyrazole derivative according to any one of claims 1-8, wherein R³ is a hydrogen atom or a methyl group, or a salt thereof.

13. The 4-acylaminopyrazole derivative according to any one of claims 1-12, wherein R⁴ is a hydrogen atom, a C₁-C₃ alkyl group or a cyano group, or a salt thereof.

14. The 4-acylaminopyrazole derivative according to any one of claims 1-12, wherein R⁴ is a hydrogen atom, or a C₁-C₂ alkyl group, or a salt thereof.

15. The 4-acylaminopyrazole derivative according to any one of claims 1-12, wherein R⁴ is a hydrogen atom, or a methyl group, or a salt thereof.

16. The 4-acylaminopyrazole derivative according to any one of claims 1-12, wherein R⁴ is a hydrogen atom, or a salt thereof.

17. The 4-acylaminopyrazole derivative according to any one of claims 1-16, wherein Z is an oxygen atom, or a salt thereof.

18. The 4-acylaminopyrazole derivative according to any one of claims 1-17, wherein Ar is an optionally substituted C₆-C₁₀ aryl group [the substituent(s) is (are) 1-3 same or different substituent(s) selected from the group consisting of a fluorine atom, a chlorine atom, a bromine atom, an iodine atom, a C₁-C₃ alkyl group, a halo(C₁-C₃ alkyl) group (the halogen substituent(s) is (are) 1-2 same or different substituent(s) selected from the group consisting of a fluorine atom, a chlorine atom and a bromine atom), a cyano(C₁-C₃ alkyl) group, a hydroxy(C₁-C₃ alkyl) group, a (C₁-C₃ alkylthio) (C₁-C₃ alkyl) group, a (C₁-C₃ alkylsulfonyl) (C₁-C₃ alkyl) group, an imino(C₁-C₃ alkyl) group wherein the alkyl part is substituted (substituent(s) being C₁-C₃ alkoxy group), a (C₁-C₃ alkylimino) (C₁-C₃ alkyl) group wherein the alkyl part is substituted {substituent(s) being C₁-C₃ alkylthio group or C₇-C₈ aralkylthio group wherein the aryl part may be substituted (substituent(s) being cyano group)}, a hydroxyimino(C₁-C₃ alkyl) group wherein the alkyl part may be substituted (substituent(s) being amino group), an optionally substituted C₂-C₅ alkenyl group (the substituent(s) is (are) 1-2 different substituent(s) selected from the group consisting of a C₁-C₃ alkoxy group and a C₂-C₄ alkoxycarbonyl group), a C₂-C₄ alkylcarbonyl group, a carboxyl group, a C₂-C₄ alkoxycarbonyl group, an optionally substituted carbamoyl group {substituent (s) being C₁-C₃ alkyl group, cyano(C₁-C₃ alkyl) group, C₂-C₄ alkylcarbonyl group or optionally substituted phenyl group (substituent(s) being C₁-C₃ alkoxy group)}, an optionally substituted thiocarbamoyl group (substituent(s) being C₁-C₃ alkyl group), a cyano group, a phenyl group, an optionally substituted 5 membered unsaturated heterocyclic group {substituent(s) being C₁-C₃ alkyl group or halo(C₁-C₃ alkyl) group (the halogen substituent(s) is (are) 1-3 same or different substituent(s) selected from the group consisting of a fluorine atom, a chlorine atom and a bromine atom); the heterocycle contains an oxygen atom, a sulfur atom or a nitrogen atom, and contains 1-2 further nitrogen atom(s)}, a hydroxyl group, a C₁-C₃ alkoxy group, a halo(C₁-C₃ alkoxy) group (the halogen substituent(s) is (are) 1-5 same or different substituent(s) selected from the group consisting of a fluorine atom, a chlorine atom and a bromine atom), a halo(C₂-C₄ alkenyloxy) group (the halogen substituent(s) is (are) 1-2 same or different substituent(s) selected from the group consisting of a fluorine atom, a chlorine atom, a bromine atom and an iodine atom), an optionally substituted phenoxy group (substituent(s) being C₁-C₃ alkyl group), a C₁-C₃ alkylthio group, a halo(C₁-C₃ alkylthio) group (the halogen substituent(s) is (are) 1-3 same or different substituent(s) selected from the group consisting of a fluorine atom, a chlorine atom and a bromine atom), a phenylthio group, a C₁-C₃ alkylsulfinyl group, a phenylsulfinyl group, a C₁-C₃ alkylsulfonyl group, a halo(C₁-C₃ alkylsulfonyl) group (the halogen substituent(s) is (are) 1-3 same or different substituent(s) selected from the group consisting of a fluorine atom, a chlorine atom and a bromine atom), a phenylsulfonyl group, an azido group, a nitro group and an optionally substituted amino group {the substituent(s) is (are) 1-2 same or different substituent(s) selected from the group consisting of a C₁-C₃ alkyl group, a C₇-C₈ aralkyl group, a formyl group, a C₂-C₅ alkylcarbonyl group, a (C₂-C₄ alkoxycarbonylamino) (C₂-C₅ alkylcarbonyl) group, a C₂-C₄ alkoxycarbonyl group, a C₂-C₄ alkyl(thiocarbonyl) group, an optionally substituted carbamoyl group (substituent(s) being C₁-C₃ alkyl group) and a C₁-C₃ alkylsulfonyl group}] or an optionally substituted 5 membered unsaturated heterocyclic group (the substituent(s) is (are) 1-2 same or different substituent(s) selected from the group consisting of a fluorine atom, a chlorine atom, a bromine atom, an iodine atom, a C₁-C₃ alkyl group, a hydroxyl group, a C₁-C₃ alkoxy group, a cyano group, a nitro group and an amino group; the heterocycle contains a sulfur atom or a nitrogen atom, and may contain one further nitrogen atom, and the heterocycle may be fused with a benzene ring),
B is a chlorine atom, a bromine atom, a C₁-C₁₅ alkyl group, a halo(C₁-C₃ alkyl) group (the halogen substituent(s) is (are) 1-3 same or different substituent(s) selected from the group consisting of a fluorine atom, a chlorine atom and a bromine atom), a (C₂-C₄ alkoxycarbonyl) (C₁-C₃ alkyl) group, a cyano(C₁-C₃ alkyl) group, a (C₃-C₆ cycloalkyl) (C₁-C₃ alkyl) group, a (C₄-C₆ cycloalkenyl) (C₁-C₃ alkyl) group, a (C₇-C₈ aralkyl) group, a (5-6 membered saturated heterocyclyl) (C₁-C₃ alkyl) group (the heterocycle contains 1-2 same or different heteroatom(s) selected from the group consisting of an oxygen atom and an nitrogen atom), a (5-6 membered unsaturated heterocyclyl) (C₁-C₃ alkyl) group wherein the heterocycle part may be substituted {the substituent(s) is (are) 1-3 same or different substituent(s) selected from the group consisting of a fluorine atom, a chlorine atom, a bromine atom, a C₁-C₃ alkyl group, a halo(C₁-C₃ alkyl) group (the halogen substituent (s) is (are) 1-3 same or different substituent (s) selected from the group consisting of a fluorine atom, a chlorine atom and a bromine atom), a C₂-C₄ alkoxycarbonyl group, a cyano group, a nitro group and an amino group; the heterocycle contains an oxygen atom, a sulfur atom or a nitrogen atom, and may contain 1-2 further nitrogen atom(s), the heterocycle may be fused with a benzene ring, and the heterocycle may be substituted with one oxo group}, a hydroxy(C₁-C₃ alkyl) group, a (C₁-C₃ alkoxy) (C₁-C₃ alkyl) group, a (C₇-C₈ aralkyloxy) (C₁-C₃ alkyl) group wherein the aryl part may be substituted {substituent(s) being halo(C₁-C₃ alkyl) group (the halogen substituent(s) is (are) 1-3 same or different substituent(s) selected from the group consisting of a fluorine atom, a chlorine atom and a bromine atom), cyano group or nitro group}, a (5-6 membered unsaturated heterocyclyl) (C₁-C₆ alkoxy) (C₁-C₆ alkyl) group wherein the heterocycle part may be substituted (the substituent (s) is (are) 1-2 same or different substituent (s) selected from the group consisting of a fluorine atom, a chlorine atom, a bromine atom and a C₁-C₃ alkyl group; the heterocycle contains an oxygen atom, a sulfur atom or a nitrogen atom, and may contain one further nitrogen atom), a cyano(C₁-C₃ alkoxy) (C₁-C₃ alkyl) group, a (C₁-C₃ alkoxy) (C₁-C₃ alkoxy) (C₁-C₃ alkyl) group, a (C₇-C₈ aralkyloxy) (C₁-C₃ alkoxy) (C₁-C₃ alkyl) group, a (C₁-C₆ alkoxy) (C₁-C₆ alkoxy) (C₁-C₆ alkoxy) (C₁-C₆ alkyl) group, a tri(C₁-C₃ alkyl)silyl(C₁-C₃ alkoxy) (C₁-C₃ alkoxy) (C₁-C₃ alkyl) group, a (C₂-C₅ alkenyloxy) (C₁-C₃ alkyl) group, a (C₂-C₃ alkynyloxy) (C₁-C₃ alkyl) group, a phenoxy(C₁-C₃ alkyl) group wherein the phenyl part may be substituted {the substituent(s) is (are) 1-2 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a C₁-C₃ alkoxy group, a cyano group and a nitro group}, a (5-6 membered saturated heterocyclyl)oxy(C₁-C₃ alkyl) group (the heterocycle contains 1-2 same or different heteroatom(s) selected from the group consisting of an oxygen atom and a nitrogen atom), a (5-6 membered unsaturated heterocyclyl)oxy(C₁-C₃ alkyl) group wherein the heterocycle part may be substituted {the substituent(s) is (are) 1-4 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a C₂-C₄ alkoxycarbonyl group, a cyano group, a nitro group, an amino group and a phenyl group; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring, and the heterocycle may be substituted with 1-2 oxo group(s)}, a tri(C₁-C₄ alkyl) silyloxy (C₁-C₃ alkyl) group, a di(C₁-C₃ alkyl) (monophenyl) silyloxy (C₁-C₃ alkyl) group, a mono(C₁-C₄ alkyl) (diphenyl)silyloxy(C₁-C₃ alkyl) group, a formyloxyt (C₁-C₃ alkyl) group, a (C₂-C₅ alkylcarbonyloxy) (C₁-C₃ alkyl) group, a (C₅-C₆ cycloalkenyl) (C₂-C₄ alkylcarbonyloxy) (C₁-C₃ alkyl) group, a (C₈-C₉ aralkylcarbonyloxy) (C₁-C₃ alkyl) group, a (5-6 membered unsaturated heterocyclyl) (C₂-C₇ alkylcarbonyloxy) (C₁-C₆ alkyl) group wherein the heterocycle part may be substituted (substituent(s) being C₁-C₃ alkyl group; the heterocycle contains an oxygen atom, a sulfur atom or a nitrogen atom, and may contain one further nitrogen atom, and the heterocycle may be fused with a benzene ring), a (C₁-C₃ alkoxy) (C₂-C₄ alkylcarbonyloxy) (C₁-C₃ alkyl) group, a phenoxy(C₂-C₄ alkylcarbonyloxy) (C₁-C₃ alkyl) group, a (C₂-C₄ alkylcarbonyloxy) (C₂-C₄ alkylcarbonyloxy) (C₁-C₃ alkyl) group, a (C₄-C₇ cycloalkylcarbonyloxy) (C₁-C₃ alkyl) group, a (C₄-C₆ alkenylcarbonyloxy) (C₁-C₃ alkyl) group, a phenyl(C₄-C₆ alkenylcarbonyloxy) (C₁-C₃ alkyl) group, a (C₆-C₇ cycloalkenylcarbonyloxy) (C₁-C₃ alkyl) group, a cyclohexadienylcarbonyloxy(C₁-C₃ alkyl) group wherein the cyclohexadiene part may be substituted (substituent(s) being C₁-C₃ alkyl group), a benzoyloxy(C₁-C₃ alkyl) group wherein the phenyl part may be substituted (the substituent(s) is (are) 1-2 same or different substituent(s) selected from the group consisting of a fluorine atom, a chlorine atom, a bromine atom, a C₁-C₃ alkyl group, a C₁-C₃ alkoxy group and a cyano group), a (5-6 membered unsaturated heterocyclyl)carbonyloxy(C₁-C₃ alkyl) group wherein the heterocycle part may be substituted {the substituent(s) is (are) 1-3 same or different substituent(s) selected from the group consisting of a fluorine atom, a chlorine atom, a bromine atom, a C₁-C₄ alkyl group, a halo(C₁-C₃ alkyl) group (the halogen substituent(s) is (are) 1-3 same or different substituent(s) selected from the group consisting of a fluorine atom, a chlorine atom, and a bromine atom), a C₁-C₃ alkoxy group and a phenyl group; the heterocycle contains an oxygen atom, a sulfur atom or a nitrogen atom, and may contain 1-2 further nitrogen atom(s), the heterocycle may be fused with a benzene ring, and the heterocycle may be substituted with one oxo group}, a (C₂-C₅ alkoxycarbonyloxy) (C₁-C₃ alkyl) group, a (C₄-C₅ alkenyloxycarbonyloxy) (C₁-C₃ alkyl) group, a phenoxycarbonyloxy(C₁-C₃ alkyl) group, a carbamoyloxy(C₁-C₃ alkyl) group wherein the carbamoyl part may be substituted (the substituent(s) is (are) 1-2 same or different C₁-C₃ alkyl group(s)), a thiocarbamoyloxy(C₁-C₃ alkyl) group wherein the thiocarbamoyl part may be substituted (the substituent(s) is (are) 1-2 same or different C₁-C₃ alkyl group(s)), a [C₂-C₅ (alkylthio)carbonyloxy](C₁-C₃ alkyl) group, an aminooxy(C₁-C₃ alkyl) group wherein the amino part may be substituted [substituent(s) being C₄-C₆ cycloalkylcarbonyl group, C₂-C₄ alkoxycarbonyl group, C₄-C₅ alkenyloxycarbonyl group, optionally substituted carbamoyl group (the substituent(s) is (are) 1-2 same or different substituent(s) selected from the group consisting of a C₁-C₃ alkyl group and a (C₁-C₃ alkoxyimino) (C₂-C₄ alkylcarbonyl) group wherein the alkyl part is cyano-substituted), optionally substituted C₁-C₆ alkylidene group <<the substituent(s) is (are) 1-2 same or different substituent(s) selected from the group consisting of a C₃-C₆ cycloalkyl group, a C₂-C₄ alkoxycarbonyl group, an optionally substituted phenyl group {substituent(s) being halo(C₁-C₃ alkyl) group (the halogen substituent(s) is (are) 1-3 same or different substituent(s) selected from the group consisting of a fluorine atom, a chlorine atom and a bromine atom) or C₁-C₃ alkoxy group}, an optionally substituted 5-6 membered unsaturated heterocyclic group (substituent(s) being C₁-C₃ alkyl group; the heterocycle contains an oxygen atom, a sulfur atom or a nitrogen atom, and may contain one further nitrogen atom, and the heterocycle may be fused with a benzene ring) and a C₁-C₃ alkoxy group>> or C₅-C₆ cycloalkylidene group (an alkylene chain of the cycloalkylidene group may be interrupted with one oxygen atom)], a di(C₁-C₃ alkoxy)phosphoryloxy(C₁-C₃ alkyl) group, a di (C₁-C₃ alkoxy) thiophosphoryloxy (C₁-C₃ alkyl) group, a (C₁-C₃ alkylsulfonyloxy) (C₁-C₃ alkyl) group, a phenylsulfonyloxy(C₁-C₃ alkyl) group wherein the phenyl part may be substituted (substituent(s) being C₁-C₃ alkyl group), a (C₁-C₆ alkylthio)(C₁-C₃alkyl) group wherein the alkyl part of the alkylthio part may be substituted (substituent(s) being C₂-C₄ alkoxycarbonyl group or cyano group), a phenylthio(C₁-C₃ alkyl) group, a (5-6 membered unsaturated heterocyclyl)thio(C₁-C₃ alkyl) group wherein the heterocycle part may be substituted {the substituent(s) is (are) 1-2 same or different substituent(s) selected from the group consisting of a fluorine atom, a chlorine atom, a bromine atom, a C₁-C₄ alkyl group, a halo(C₁-C₃ alkyl) group (the halogen substituent(s) is (are) 1-3 same or different substituent(s) selected from the group consisting of a fluorine atom, a chlorine atom and a bromine atom), a C₃-C₆ cycloalkyl group, a nitro group and a phenyl group; the heterocycle contains an oxygen atom, a sulfur atom or a nitrogen atom, and may contain 1-2 further nitrogen atom(s), and the heterocycle may be fused with a benzene ring or a pyridine ring}, a (C₁-C₆ alkylsulfinyl)(C₁-C₃ alkyl) group, a phenylsulfinyl(C₁-C₃ alkyl) group, a (5-6 membered unsaturated heterocyclyl)sulfinyl(C₁-C₃ alkyl) group (the heterocycle contains an oxygen atom, a sulfur atom or a nitrogen atom, and may contain 1-2 further nitrogen atom(s), and the heterocycle may be fused with a benzene ring or a pyridine ring), a (C₁-C₆ alkylsulfonyl)(C₁-C₃ alkyl) group, a phenylsulfonyl(C₁-C₃ alkyl) group, a (5-6 membered unsaturated heterocyclyl)sulfonyl(C₁-C₃ alkyl) group (the heterocycle contains an oxygen atom, a sulfur atom or a nitrogen atom, and may contain further 1-2 nitrogen atom(s), and the heterocycle may be fused with a benzene ring or a pyridine ring), a di(C₁-C₃ alkoxy)thiophosphorylthio(C₁-C₃ alkyl) group, an amino (C₁-C₄ alkyl) group wherein the amino part may be substituted {substituent (s) being C₁-C₃ alkyl group, carboxyl (C₁-C₄ alkyl) group, (C₂-C₄ alkoxycarbonyl) (C₁-C₄ alkyl) group, (C₈-C₉ aralkyloxycarbonyl) (C₁-C₄ alkyl) group, formyl group, (C₁-C₃ alkoxyimino) (C₂-C₄ alkylcarbonyl) group wherein the alkyl part is cyano-substituted, C₂-C₅ alkoxycarbonyl group, C₈-C₁₀ aralkyloxycarbonyl group or optionally substituted carbamoyl group (substituent(s) being C₁-C₃ alkyl group or (C₁-C₃ alkoxyimino)(C₂-C₄ alkylcarbonyl) group wherein the alkyl part is cyano-substituted)}, an anilino(C₁-C₃ alkyl) group, a (C₁-C₃ alkoxyimino)(C₁-C₃ alkyl) group wherein the alkyl part may be substituted (substituent(s) being phenyl group or cyano group), a C₃-C₆ cycloalkyl group, a C₃-C₅ alkenyl group, a phenyl (C₃-C₅ alkenyl) group, an N-(cyanoaralkyl)-N-{1,5-di(C₁-C₄ alkyl)-4-pyrazolyl}aminocarbonyl (C₂-C₆ alkenyl) group, a C₅-C₆ cycloalkenyl group, an optionally substituted phenyl group (the substituent(s) is (are) 1-2 same or different substituent(s) selected from the group consisting of a fluorine atom, a chlorine atom and a bromine atom), a 5-6 membered saturated heterocyclic group (the heterocycle contains 1-2 same or different heteroatom(s) selected from the group consisting of an oxygen atom and a nitrogen atom), an optionally substituted 5-6 membered unsaturated heterocyclic group (the substituent(s) is (are) 1-2 same or different substituent(s) selected from the group consisting of a fluorine atom, a chlorine atom, a bromine atom and a C₁-C₆ alkyl group; the heterocycle contains an oxygen atom, a sulfur atom or a nitrogen atom, and may contain 1-2 further nitrogen atom(s), and the heterocycle may be fused with a benzene ring), a C₁-C₄ alkoxy group, an optionally substituted phenoxy group {the substituent(s) is (are) 1-2 same or different substituent(s) selected from the group consisting of a fluorine atom, a chlorine atom, a bromine atom, a straight or branched alkyl group having a carbon number of 1-3, a straight or branched haloalkyl group having a carbon number of 1-3 (the halogen substituent(s) is (are) 1-3 same or different halogen atom(s) selected from the group consisting of a fluorine atom, a chlorine atom and a bromine atom), an alkoxy group having a carbon number of 1-3, a cyano group and a nitro group}, an optionally substituted 5-6 membered unsaturated (heterocyclyl)oxy group {the substituent(s) is (are) 1-4 same or different substituent(s) selected from the group consisting of a fluorine atom, a chlorine atom, a bromine atom, a straight or branched alkyl group having a carbon number of 1-3, a straight or branched haloalkyl group having a carbon number of 1-3 (the halogen substituent(s) is (are) 1-3 same or different halogen atom(s) selected from the group consisting of a fluorine atom, a chlorine atom and a bromine atom), a straight or branched alkoxy group having a carbon number of 1-3, a carbamoyl group, a straight or branched alkoxycarbonyl group having a carbon number of 2-4, a cyano group, a nitro group, an amino group and a phenyl group; the heteroatom for the heterocycle is an oxygen atom, a sulfur atom or a nitrogen atom, and may be 1-2 further nitrogen atom(s), two oxygen atoms may bind to the sulfur atom, and the heterocycle may be fused with a benzene ring, and may be substituted with 1-2 oxo group(s)}, a C₁-C₃ alkylthio group, an optionally substituted amino group [the substituent(s) is (are) 1-2 same or different substituent(s) selected from the group consisting of a C₁-C₃ alkyl group, a cyano(C₁-C₃ alkyl) group, a carboxyl(C₁-C₄ alkyl) group, a (C₂-C₄ alkoxycarbonyl) (C₁-C₄ alkyl) group, a (C₈-C₉ aralkyloxycarbonyl) (C₁-C₄ alkyl) group, a carbamoyl (C₁-C₆ alkyl) group wherein the carbamoyl part may be substituted {substituent(s) being optionally substituted C₇-C₈ aralkyl group (the substituent(s) is (are) 1-2 same or different C₁-C₃ alkoxy group(s))} and a (C₁-C₆ alkoxyimino) (C₂-C₇ alkylcarboyl) group wherein the alkyl part is cyano-substituted] or an aniline group;
or Ar and B are taken together to be an optionally substituted o-phenylene group (substituent(s) being fluorine atom, chlorine atom, bromine atom, hydroxyl group, cyano group, nitro group or amino group, and one methylene group is inserted between the phenylene group and the carbon atom to which B binds), or a salt thereof.

19. The 4-acylaminopyrazole derivative according to any one of claims 1-17, wherein Ar is an optionally substituted phenyl group {the substituent(s) is (are) 1-2 same or different substituent(s) selected from the group consisting of a fluorine atom, a chlorine atom, a bromine atom, an iodine atom, a methyl group, a (C₁-C₂ alkylthio)methyl group, an iminomethyl group wherein the alkyl part is substituted (substituent(s) being C₁-C₂ alkoxy group), a carbamoyl group, a cyano group, a 1,2, 4-oxadiazolyl group, a C₁-C₂ alkoxy group, a halo(C₁-C₂ alkoxy) group (the halogen substituent(s) is (are) 1-3 fluorine atom(s)), an optionally substituted phenoxy group (substituent(s) being methyl group), a C₁-C₂ alkylthio group, a C₁-C₂ alkylsulfinyl group, a phenylsulfinyl group, a C₁-C₂ alkylsulfonyl group, a phenylsulfonyl group, an azido group, a nitro group and an optionally substituted amino group (substituent(s) being methyl group or C₇-C₈ aralkyl group)}, B is a chlorine atom, a C₁-C₇ alkyl group, a halo(C₁-C₂ alkyl) group (halogen substituent(s) being fluorine atom or chlorine atom), a (C₂-C₃ alkoxycarbonyl) (C₁-C₂ alkyl) group, a benzyl group, a morpholyl(C₁-C₂ alkyl) group, a (5-6 membered unsaturated heterocyclyl)(C₁-C₃ alkyl) group wherein the heterocycle part may be substituted {the substituent(s) is (are) 1-2 same or different substituent(s) selected from the group consisting of a chlorine atom, a bromine atom, a methyl group, a halo(C₁-C₂ alkyl) group (the halogen substituent(s) is (are) 1-3 fluorine atom(s)), a C₂-C₃ alkoxycarbonyl group and a nitro group; the heterocycle contains an oxygen atom or a nitrogen atom, and may contain 1-2 further nitrogen atom(s), the heterocycle may be fused with a benzene ring, and the heterocycle may be substituted with one oxo group}, a hydroxy(C₁-C₃ alkyl) group, a (C₁-C₃ alkoxy) (C₁-C₂ alkyl) group, a benzyloxy(C₁-C₂ alkyl) group wherein the phenyl part may be substituted {substituent(s) being halo(C₁-C₂ alkyl) group (the halogen substituent(s) is (are) 1-3 fluorine atom(s)), cyano group or nitro group}, a (5-6 membered unsaturated heterocyclyl) (C₁-C₆ alkoxy) (C₁-C₆ alkyl) group wherein the heterocycle part may be substituted (the substituent(s) is (are) 1-2 same or different substituent(s) selected from the group consisting of a fluorine atom, a chlorine atom, a bromine atom and a methyl group; the heterocycle contains an oxygen atom or a nitrogen atom, and may contain one further nitrogen atom), a cyano(C₁-C₃ alkoxy) (C₁-C₂ alkyl) group, a (C₁-C₂ alkoxy) (C₁-C₂ alkoxy) (C₁-C₂ alkyl) group, a benzyloxy(C₁-C₂ alkoxy)(C₁-C₂ alkyl) group, a trimethylsilyl (C₁-C₂ alkoxy) (C₁-C₂ alkoxy) (C₁-C₂ alkyl) group, a (C₃-C₅ alkenyloxy) (C₁-C₂ alkyl) group, a propynyloxy(C₁-C₂ alkyl) group, a phenoxy(C₁-C₂ alkyl) group wherein the phenyl part may be substituted {the substituent(s) is (are) 1-2 same or different substituent(s) selected from the group consisting of a fluorine atom, a chlorine atom, a methyl group, a halo(C₁-C₂ alkyl) group (the halogen substituent(s) is (are) 1-3 fluorine atom(s)), a methoxy group and a nitro group}, a (5-6 membered unsaturated heterocyclyl)oxy(C₁-C₂ alkyl) group wherein the heterocycle part may be substituted {the substituent(s) is (are) 1-4 same or different substituent(s) selected from the group consisting of a fluorine atom, a chlorine atom, a bromine atom, a C₁-C₃ alkyl group, a halo(C₁-C₃ alkyl) group (the halogen substituent(s) is (are) 1-3 same or different substituent(s) selected from the group consisting of a fluorine atom, a chlorine atom and a bromine atom), a nitro group and an amino group; the heterocycle contains an oxygen atom, a sulfur atom or a nitrogen atom, and may contain 1-2 further nitrogen atom(s), two oxygen atoms may bind to the sulfur atom, the heterocycle may be fused with a benzene ring, and the heterocycle may be substituted with 1-2 oxo group(s)}, a tri(C₁-C₄ alkyl)silyloxy(C₁-C₂ alkyl) group, a dimethylmonophenylsilyloxy(C₁-C₂ alkyl) group, a (C₂-C₅ alkylcarbonyloxy) (C₁-C₃ alkyl) group, a cyclopentenyl (C₂-C₃ alkylcarbonyloxy) (C₁-C₂ alkyl) group, a benzylcarbonyloxy (C₁-C₂ alkyl) group, a (5-6 membered unsaturated heterocyclyl) (C₂-C₇ alkylcarbonyloxy)(C₁-C₆ alkyl) group wherein the heterocycle part may be substituted (substituent(s) being methyl group; the heterocycle contains an oxygen atom, a sulfur atom or a nitrogen atom, and may contain one further nitrogen atom, and the heterocycle may be fused with a benzene ring), a (C₁-C₂ alkoxy) (C₂-C₃ alkylcarbonyloxy) (C₁-C₂ alkyl) group, a phenoxy(C₂-C₃ alkylcarbonyloxy) (C₁-C₂ alkyl) group, a (C₂-C₃ alkylcarbonyloxy) (C₂-C₃ alkylcarbonyloxy) (C₁-C₂ alkyl) group, a (C₄-C₇ cycloalkylcarbonyloxy) (C₁-C₂ alkyl) group, a (C₄-C₆ alkenylcarbonyloxy) (C₁-C₂ alkyl) group, a phenyl(C₁-C₆ alkenylcarbonyloxy) (C₁-C₂ alkyl) group, a 1,4-cyclohexadienylcarbonyloxy(C₁-C₂ alkyl) group wherein the cyclohexadiene part may be substituted (substituent(s) being methyl group), a benzoyloxy(C₁-C₂ alkyl) group wherein the phenyl part may be substituted (the substituent(s) is (are) 1-2 same or different substituent(s) selected from the group consisting of a fluorine atom, a chlorine atom, a methyl group, a methoxy group and a cyano group), a (5-6 membered unsaturated hetero carbonyloxy) (C₁-C₃ alkyl) group wherein the heterocycle part may be substituted {the substituent(s) is (are) 1-3 same or different substituent(s) selected from the group consisting of a fluorine atom, a chlorine atom, a C₁-C₄ alkyl group, a halo(C₁-C₂ alkyl) group (the halogen substituent(s) is (are) 1-3 fluorine atom(s)) and a methoxy group; the heterocycle contains an oxygen atom, a sulfur atom or a nitrogen atom, and the heterocycle may be fused with a benzene ring}, a (C₂-C₅ alkoxycarbonyloxy) (C₁-C₂ alkyl) group, an allyloxycarbonyloxy(C₁-C₂ alkyl) group, a phenoxycarbonyloxy(C₁-C₂ alkyl) group, a carbamoyloxy(C₁-C₂ alkyl) group wherein the carbamoyl part may be substituted (the substituent(s) is (are) 1-2 same or different C₁-C₂ alkyl group(s)), a thiocarbamoyloxy(C₁-C₂ alkyl) group wherein the thiocarbamoyl part may be substituted (the substituent(s) is (are) 1-2 same or different C₁-C₂ alkyl group(s)), a [C₂-C₃ (alkylthio)carbonyloxy] (C₁-C₂ alkyl) group, an aminooxy (C₁-C₂ alkyl) group wherein the amino part may be substituted [substituent(s) being optionally substituted carbamoyl group (the substituent(s) is (are) 1-2 same or different C₁-C₂ alkyl group(s)), optionally substituted C₁-C₃ alkylidene group <<the substituent(s) is (are) 1-2 same or different substituent(s) selected from the group consisting of a C₂-C₃ alkoxycarbonyl group, an optionally substituted phenyl group {substituent(s) being halo(C₁-C₂ alkyl) group (the halogen substituent(s) is (are) 1-3 fluorine atom(s)) or methoxy group}, an optionally substituted 5-6 membered unsaturated heterocyclic group (substituent(s) being methyl group; the heterocycle contains an oxygen atom, a sulfur atom or a nitrogen atom, and the heterocycle may be fused with a benzene ring) and a methoxy group>> or C₅-C₆ cycloalkylidene group], a di (C₁-C₂ alkoxy)phosphoryloxy(C₁-C₂ alkyl) group, a di(C₁-C₂ alkoxy) thiophosphoryloxy (C₁-C₂ alkyl) group, a (C₁-C₂ alkylsulfonyloxy) (C₁-C₂ alkyl) group, a phenylsulfonyloxy (C₁-C₂ alkyl) group wherein the phenyl part may be substituted (substituent(s) being methyl group), a (C₁-C₃ alkylthio) (C₁-C₂ alkyl) group wherein the alkyl part of the alkylthio part may be substituted (substituent(s) being C₂-C₃ alkoxycarbonyl group or cyano group), a phenylthio(C₁-C₂ alkyl) group, a (5-6 membered unsaturated heterocyclyl)thio(C₁-C₂ alkyl) group wherein the heterocycle part may be substituted {the substituent(s) is (are) 1-2 same or different substituent(s) selected from the group consisting of a fluorine atom, a chlorine atom, a C₁-C₄ alkyl group, a halo(C₁-C₂ alkyl) group (the halogen substituent(s) is (are) 1-3 fluorine atom(s)), a cyclohexyl group, a nitro group and a phenyl group; the heterocycle contains an oxygen atom, a sulfur atom or a nitrogen atom, and the heterocycle may be fused with a benzene ring or a pyridine ring}, a (C₁-C₃ alkylsulfinyl) (C₁-C₂ alkyl) group, a phenylsulfinyl (C₁-C₂ alkyl) group, a (6 membered unsaturated heterocyclyl)sulfinyl(C₁-C₂ alkyl) group (the heterocycle contains 1-2 nitrogen atom(s)), a (C₁-C₃ alkylsulfonyl) (C₁-C₂ alkyl) group, a phenylsulfonyl (C₁-C₂ alkyl) group, a (6 membered unsaturated heterocyclyl)sulfonyl(C₁-C₂ alkyl) group (the heterocycle contains 1-2 nitrogen atom(s)), a di(C₁-C₂ alkoxy)thiophosphorylthio(C₁-C₂ alkyl) group, an amino(C₁-C₂ alkyl) group wherein the amino part may be substituted {substituent(s) being methyl group, carboxyl(C₁-C₄ alkyl) group, (C₂-C₃ alkoxycarbonyl)(C₁-C₄ alkyl) group, benzyloxycarbonyl(C₁-C₄ alkyl) group, (C₁-C₂ alkoxyimino)methylcarbonyl group wherein the methyl part is cyano-substituted, C₂-C₃ alkoxycarbonyl group, benzyloxycarbonyl group or optionally substituted carbamoyl group (substituent(s) being C₁-C₂ alkyl group or (C₁-C₂ alkoxyimino)methylcarbonyl group wherein the methyl part is cyano-substituted)}, an anilino(C₁-C₂ alkyl) group, a (C₁-C₂ alkoxyimino)methyl group wherein the alkyl part may be substituted (substituent(s) being phenyl group or cyano group), a C₃-C₅ cycloalkyl group, a C₃-C₅ alkenyl group, an N- (3-cyanobenzyl)-N-(1-isobutyl-5-methyl-4-pyrazolyl)aminocarbonyl(C₂-C₆ alkenyl) group, a cyclohexenyl group, an optionally substituted phenyl group (the substituent(s) is (are) 1-2 same or different substituent(s) selected from the group consisting of a fluorine atom and a chlorine atom), a 5-6 membered unsaturated heterocyclic group (the heterocycle contains 1-2 nitrogen atom(s)), a C₁-C₃ alkoxy group, an optionally substituted phenoxy group (the substituent(s) is (are) 1-2 same or different substituent (s) selected from the group consisting of a fluorine atom, a chlorine atom, a methyl group, an alkyl group having a carbon number of 1-2 substituted with 1-3 fluorine atom(s), a methoxy group and a nitro group), an optionally substituted 5-6 membered unsaturated (heterocyclyl)oxy group {the substituent(s) is (are) 1-4 same or different substituent(s) selected from the group consisting of a fluorine atom, a chlorine atom, a bromine atom, a straight or branched alkyl group having a carbon number of 1-3, a straight or branched haloalkyl group having a carbon number of 1-3 (the halogen substituent(s) is (are) 1-3 same or different halogen atom(s) selected from the group consisting of a fluorine atom, a chlorine atom and a bromine atom), an alkoxy group having a carbon number of 1-2, a carbamoyl group, an alkoxycarbonyl group having a carbon number of 2-3, a cyano group, a nitro group, an amino group and a phenyl group; the heteroatom for the heterocycle is an oxygen atom, a sulfur atom or a nitrogen atom, and may be 1-2 further nitrogen atom(s), two oxygen atoms may bind to the sulfur atom, and the heterocycle may be fused with a benzene ring and may be substituted with 1-2 oxo group(s)}, a C₁-C₂ alkylthio group, an optionally substituted amino group {the substituent(s) is(are) 1-2 same or different substituent(s) selected from the group consisting of a C₁-C₂ alkyl group, a cyano (C₁-C₂ alkyl) group, a (C₂-C₃ alkoxycarbonyl) (C₁-C₄ alkyl) group, a benzyloxycarbonyl (C₁-C₄ alkyl) group, a carbamoyl(C₁-C₄ alkyl) group wherein the carbamoyl part may be substituted (substituents(s) being phenethyl group) and a (C₁-C₂ alkoxyimino)methylcarbonyl group wherein the alkyl part is cyano-substituted} or an anilino group,
or Ar and B are taken together to be an optionally substituted o-phenylene group (substituents(s) being bromine atom, cyano group or nitro group, and one methylene group is inserted between the phenylene group and the carbon atom to which B binds), or a salt thereof.

20. The 4-acylaminopyrazole derivative according to any one of claims 1-17, wherein Ar is an optionally substituted phenyl group {the substituent(s) is (are) 1-2 same or different substituent(s) selected from the group consisting of a fluorine atom, a bromine atom, an iodine atom, a methyl group, a methylthiomethyl group, a cyano group, a 1,2,4-oxadiazolyl group, a methoxy group, a trifluoromethoxy group, a phenoxy group, a methylthio group, a phenylsulfinyl group, a phenylsulfonyl group, a nitro group and a dimethylamino group}, B is a C₁-C₇ alkyl group, a chloromethyl group, a (C₂-C₃ alkoxycarbonyl)methyl group, a benzyl group, a morpholinomethyl group, a pyrazol-1-ylmethyl group wherein the pyrazole part may be substituted (the substituent(s) is (are) 1-2 same or different substituent(s) selected from the group consisting of a chlorine atom, a bromine atom, a methyl group, an ethoxycarbonyl group and a nitro group), a (methyl-isooxazol-3-yl)methyl group, an imidazol-1-ylmethyl group, a (dichloro-imidazol-l-yl)methyl group, a 2-oxo-1-pyridylmethyl group, a (trifluoromethyl-2-oxo-l-pyridyl)methyl group, a 6-oxo-pyrimidin-1-ylmethyl group, a 4-oxo-quinazolin-3-ylmethyl group, a 3-oxo-pyrazol-1-ylmethyl group wherein the pyrazole part may be substituted (the substituent(s) is (are) 1-2 methyl group(s)), a 2-pyridylmethyl group, a hydroxy(C₁-C₃ alkyl) group, a (C₁-C₃ alkoxy) (C₁-C₂ alkyl) group, a nitrobenzyloxymethyl group, a cyanobenzyloxymethyl group, a (dichloro-4-pyridyl)methoxymethyl group, a (methyl-isooxazol-3-yl)methoxymethyl group, a 3-pyridylmethoxymethyl group, a cyano(C₁-C₃ alkoxy) (C₁-C₂ alkyl) group, a (C₁-C₂ alkoxy) (C₁-C₂ alkoxy)methyl group, a benzyloxymethoxymethyl group, a trimethylsilylethoxymethoxymethyl group, a (C₃-C₅ alkenyloxy)(C₁-C₂ alkyl) group, a propynyloxymethyl group, a phenoxy(C₁-C₂ alkyl) group wherein the phenyl part may be substituted (the substituent(s) is (are) 1-2 same substituent(s) selected from the group consisting of a fluorine atom, a chlorine atom, a methyl group, a trifluoromethyl group, a methoxy group and a nitro group), a (methyl-isooxazol-5-yl)oxymethyl group, a pyrazol-1-yloxymethyl group wherein the pyrazole part may be substituted (the substituent is a bromine atom or a methyl group), a 2-pyridyloxymethyl group wherein the pyridine part may be substituted (the substituent (s) is (are) 1-2 same or different substituent(s) selected from the group consisting of a fluorine atom, a chlorine atom and a trifluoromethyl group, or 3-4 fluorine atom(s)), a 3-pyridyloxymethyl group, a pyrimidin-2-yloxymethyl group, a pyrimidin-4-yloxymethyl group, an (isopropyl-methyl-pyrimidin-4-yl)oxymethyl group, a benzisooxazol-3-yloxymethyl group wherein the benzisooxazole part is substituted (substituent(s) being chlorine atom, methyl group, nitro group or amino group), an imidazol-1-yloxymethyl group, a 1,2,4-triazol-1-yloxymethyl group, a thiazol-2-yloxymethyl group, a 2-quinolyloxymethyl group, a 3-isoquinolyloxymethyl group, a benzisothiazol-1,1-dioxide-3-yloxymethyl group, a benzothiazol-2-yloxymethyl group, an N,N-phthaloyloxymethyl group, a mono(C₃-C₄ alkyl)dimethylsilyloxymethyl group, a dimethylmonophenylsilyloxymethyl group, a (C₂-C₅ alkylcarbonyloxy) (C₁-C₂ alkyl) group, a cyclopentenylacetoxymethyl group, a benzylcarbonyloxymethyl group, a 2-thienylacetoxymethyl group, a 3-thienylacetoxymethyl group, a 3-indolylacetoxymethyl group, a 3-benzothienylacetoxymethyl group, a 2-pyridylacetoxymethyl group, a 3-pyridylacetoxymethyl group, a 4-pyridylacetoxymethyl group, a (methyl-isooxazol-5-yl)acetoxymethyl group, a methoxyacetoxymethyl group, a phenoxyacetoxymethyl group, an acetoxyacetoxymethyl group, a (C₄-C₇ cycloalkylcarbonyloxy)methyl group, a (C₄-C₆ alkenylcarbonyloxy)methyl group, a 3-ene-4-phenylbutyryloxymethyl group, a methyl-1,4-cyclohexadienylcarbonyloxymethyl group, a benzoyloxymethyl group wherein the phenyl part may be substituted (the substituent(s) is (are) 1-2 fluorine atom(s), 1-2 chlorine atom(s), one methyl group, one methoxy group or one cyano group), a 4-pyridylcarbonyloxymethyl group, a (dichloro-4-pyridyl)carbonyloxymethyl group, a (chloro-methoxy-4-pyridyl)carbonyloxymethyl group, a (trimethyl-pyrrol-3-yl)carbonyloxymethyl group, a (dimethyl-3-furyl)carbonyloxymethyl group, a (t-butyl-methyl-3-furyl)carbonyloxymethyl group, a (trifluoromethyl-3-furyl)carbonyloxymethyl group, a 2-furylcarbonyloxymethyl group, a 2-thienylcarbonyloxymethyl group, a 3-pyridylcarbonyloxymethyl group, a (C₂-C₅ alkoxycarbonyloxy)methyl group, an allyloxycarbonyloxymethyl group, a phenoxycarbonyloxymethyl group, a carbamoyloxymethyl group wherein the carbamoyl part is substituted (the substituent(s) is (are) 1-2 same or different C₁-C₂ alkyl group(s)), a thiocarbamoyloxymethyl group wherein the thiocarbamoyl part is substituted (the substituent(s) is (are) 1-2 methyl group(s)), a (methylthio)carbonyloxymethyl group, an aminooxymethyl group wherein the amino part may be substituted [substituents(s) being optionally substituted carbamoyl group (the substituent(s) is (are) 1-2 methyl group(s)), optionally substituted C₁-C₃ alkylidene group {the substituent(s) is (are) 1-2 same or different substituent(s) selected from the group consisting of a methoxycarbonyl group, an optionally substituted phenyl group (substituents(s) being trifluoromethyl group or methoxy group), a methyl-2-furyl group and a methoxy group} or cyclopentylidene group], a diethoxyphosphoryloxymethyl group, a diethoxy(thiophosphoryl)oxymethyl group, a methylsulfonyloxymethyl group, a phenylsulfonyloxymethyl group, a tolylsulfonyloxymethyl group, a (C₁-C₃ alkylthio)methyl group, a cyano(C₁-C₃ alkylthio)methyl group, a (C₂-C₃ alkoxycarbonyl)methylthiomethyl group, a phenylthiomethyl group, a 2-pyridylthiomethyl group, a (chloro-2-pyridyl)thiomethyl group, a (trifluoromethyl-2-pyridyl)thiomethyl group, a (nitro-2-pyridyl)thiomethyl group, a (chloro-trifluoromethyl-2-pyridyl)thiomethyl group, a pyrimidin-2-ylthiomethyl group, a (methyl-pyrimidin-2-yl)thiomethyl group, a (dimethyl-pyrimidin-2-yl)thiomethyl group, a (phenyl-pyrimidin-2-yl)thiomethyl group, an imidazol-2-ylthiomethyl group, a (methyl-imidazol-2-yl)thiomethyl group, a 1,3,4-triazol-2-ylthiomethyl group, a (methyl-1,3,4-triazol-2-yl)thiomethyl group, a (t-butyl-1,3,4-triazol-2-yl)thiomethyl group, a (cyclohexyl-1,3,4-triazol-2-yl)thiomethyl group, a (methyl-1,3,4-isothiadiazol-2-yl)thiomethyl group, a 4-pyridylthiomethyl group, a benzooxazol-2-ylthiomethyl group, a benzothiazol-2-ylthiomethyl group, a benzimidazol-2-ylthiomethyl group, a 1H-imidazo[4,5-b]pyridin-2-ylthiomethyl group, a 4,5-dihydrothiazol-2-ylthiomethyl group, a (C₁-C₃ alkylsulfinyl)methyl group, a phenylsufinylmethyl group, a 2-pyridylsulfinylmethyl group, a (C₁-C₃ alkylsulfonyl)methyl group, a phenylsulfonylmethyl group, a 2-pyridylsulfonylmethyl group, a diethoxy(thiophosphoryl)thiomethyl group, an aminomethyl group wherein the amino part may be substituted (substituents(s) being methyl group, methoxycarbonyl(C₁-C₄ alkyl) group, benzyloxycarbonyl (C₁-C₄ alkyl) group, methoxyiminomethylcarbonyl group wherein the methyl part is cyano-substituted, methoxycarbonyl group, benzyloxycarbonyl group or
methoxyiminomethylcarbonylcarbamoyl group wherein the methyl part is cyano-substituted), an anilinomethyl group, a methoxyiminomethyl group wherein the methyl part may be substituted (substituents(s) being phenyl group or cyano group), a C₃-C₅ cycloalkyl group, a C₃-C₅ alkenyl group, a cyclohexenyl group, a fluorophenyl group, a chlorophenyl group, a difluorophenyl group, a dichlorophenyl group, a C₁-C₃ alkoxy group, an optionally substituted phenoxy group (the substituent(s) is (are) 1-2 same or different substituent(s) selected from the group consisting of a fluorine atom, a chlorine atom, a methyl group, a trifluoromethyl group, a methoxy group and a cyano group), an optionally substituted pyrazol-1-yloxy group (the substituent is one bromine atom or methyl group), an optionally substituted 2-pyridyloxy group (the substituent(s) is (are) 1-3 same or different substituent(s) selected from the group consisting of a fluorine atom, a chlorine atom, a methyl group, a trifluoromethyl group, a methoxy group, a carbamoyl group, a methoxycarbonyl group, a cyano group and a phenyl group, or 3-4 fluorine atom(s)), a 3-pyridyloxy group, an optionally substituted pyrimidin-2-yloxy group (the substituent(s) is (are) 1-3 same or different substituent(s) selected from the group consisting of a fluorine atom, a chlorine atom, a methyl group, a trifluoromethyl group, a methoxy group, a carbamoyl group, a methoxycarbonyl group, a cyano group and a phenyl group, or 3-4 fluorine atom(s)), a pyrimidin-4-yloxy group, an (isopropyl-methyl-pyrimidin-4-yl)oxy group, an optionally substituted benzisooxazol-3-yloxy group (the substituent is one chlorine atom, methyl group, nitro group or amino group), an imidazol-1-yloxy group, a 1,2,4-triazol-1-yloxy group, a thiazol-2-yloxy group, a 2-quinolyloxy group, a 3-isoquinolyloxy group, a benzisothiazol-1,1-dioxide-3-yloxy group, a benzisothiazol-2-yloxy group, an N,N-phthaloyloxy group, an optionally substituted 3-isooxazolyloxy group (the substituent is one methyl group), a methylthio group, an optionally substituted amino group (substituents(s) being C₁-C₂ alkyl group, cyanomethyl group, (C₂-C₃ alkoxy)carbonylmethyl group or methoxyiminomethylcarbonyl group wherein the methyl part is cyano-substituted) or an anilino group, or a salt thereof.

21. The 4-acylaminopyrazole derivative according to any one of clams 1-17, wherein Ar is a 3-cyanophenyl group, a 3-nitrophenyl group or a 2-fluoro-5-cyanophenyl group; B is a methyl group, a 4-chloro-1-pyrazolylmethyl group, a 4-bromo-1-pyrazolylmethyl group, a hydroxymethyl group, a methoxymethyl group, a 3-nitrobenzyloxymethyl group, a 3-pyridylmethoxymethyl group, a cyanomethoxymethyl group, an allyloxymethyl group, a 2-methyl-2-butenyloxymethyl group, a phenoxymethyl group, a (fluorophenoxy)methyl group, a (chlorophenoxy)methyl group, a (difluorophenoxy)methyl group, a (dichlorophenoxy)methyl group, a (methylphenoxy)methyl group, a (trifluoromethylphenoxy)methyl group, a (methoxyphenoxy)methyl group, a (nitrophenoxy)methyl group, a (dinitrophenoxy)methyl group, a (cyanophenoxy)methyl group, a 2-(phenoxy)ethyl group, a 3-pyridyloxymethyl group, a 3-isooxazolyloxymethyl group, a 5-methyl-3-isooxazolyloxymethyl group, an isovaleryloxymethyl group, a pivaloyloxymethyl group, a (C₅-C₇ cycloalkylcarbonyloxy)methyl group, a (fluorobenzoyl)oxymethyl group, a (chlorobenzoyl)oxymethyl group, a (difluorobenzoyl)oxymethyl group, a (methylbenzoyl)oxymethyl group, a (methoxybenzoyl)oxymethyl group, a (cyanobenzoyl)oxymethyl group, a 4-pyridylcarbonyloxymethyl group, a 2-thienylcarbonyloxymethyl group, a carbamoyloxymethyl group, a cyanomethylthiomethyl group, a phenylthiomethyl group, a (3-chloro-2-pyridyl)thiomethyl group, a (4-methyl-pyrimidin-2-yl)thiomethyl group, an imidazol-2-ylthiomethyl group, a 4-pyridylthiomethyl group, a benzimidazol-2-ylthiomethyl group, a lH-imidazo[4,5-b]pyridin-2-ylthiomethyl group, a 2-pyrimidinylthiomethyl group, an aminomethyl group wherein the amino part is substituted (substituents(s) being methoxyiminomethylcarbonyl group wherein the methyl part is cyano-substituted or methoxycarbonyl group), an anilinomethyl group, a cyclopropyl group, a 2-chlorophenyl group, a methoxy group, a phenoxy group, a fluorophenoxy group, a chlorophenoxy group, a difluorophenoxy group, a dichlorophenoxy group, a methylphenoxy group, a trifluoromethylphenoxy group, a methoxyphenoxy group, a nitrophenoxy group, a dinitrophenoxy group, a cyanophenoxy group, an optionally substituted 2-pyridyloxy group (the substituent(s) is (are) 1-3 same or different substituent(s) selected from the group consisting of a fluorine atom, a chlorine atom, a methyl group, a trifluoromethyl group and a cyano group), an optionally substituted pyrimidin-2-yloxy group (the substituent(s) is (are) 1-3 same or different substituent(s) selected from the group consisting of a fluorine atom, a chlorine atom, a methyl group, a trifluoromethyl group and a cyano group), a 3-isooxazolyloxy group, a 5-methyl-3-isooxazolyloxy group or an amino group, or a salt thereof.

22. The 4-acylaminopyrazole derivative according to any one of claims 1-17, wherein Ar is a 3-cyanophenyl group; B is a methyl group, a 4-chloro-1-pyrazolylmethyl group, a 4-bromo-1-pyrazolylmethyl group, a hydroxymethyl group, a methoxymethyl group, a 3-pyridylmethoxymethyl group, a cyanomethoxymethyl group, a phenoxymethyl group, a (4-fluorophenoxy)methyl group, a (4-chlorophenoxy)methyl group, a 3-isooxazolyloxymethyl group, a 5-methyl-3-isooxazolyloxymethyl group, a carbamoyloxymethyl group, a cyanomethylthiomethyl group, a 2-pyrimidinylthiomethyl group, a cyclopropyl group, a methoxy group, a phenoxy group, a 2-fluorophenoxy group, a 3-fluorophenoxy group, a 4-fluorophenoxy group, a 4-chlorophenoxy group, a 2-methylphenoxy group, a 2-cyanophenoxy group, a 3-cyanophenoxy group, an optionally substituted 2-pyridyloxy group (the substituent(s) is (are) 1-3 same or different substituent(s) selected from the group consisting of a chlorine atom, a methyl group, a trifluoromethyl group and a cyano group), an optionally substituted pyrimidin-2-yloxy group (the substituent(s) is (are) 1-3 same or different substituent(s) selected from the group consisting of a chlorine atom, a methyl group, a trifluoromethyl group and a cyano group), a 5-methyl-3-isooxazolyloxy group or an amino group, or a salt thereof.

23. Agricultural chemicals which contain as an active ingredient the 4-acylaminopyrazole derivative as defined in any one of claims 1-22, or a salt thereof.
